(19) Europäisches Patentamt
European Patent Office
Office européen des brevets

(11) EP 2 578 679 A1

(12) **EUROPEAN PATENT APPLICATION**

(43) Date of publication:
**10.04.2013 Bulletin 2013/15**

(51) Int Cl.:
***C12N 9/54*** *(2006.01)* ***A23K 1/165*** *(2006.01)*

(21) Application number: **12183920.3**

(22) Date of filing: **03.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **06.06.2008 US 59695 P**

(62) Document number(s) of the earlier application(s) in accordance with Art. 76 EPC:
**09759359.4 / 2 297 316**

(71) Applicant: **Danisco US Inc.**
**Palo Alto, CA 94304-1013 (US)**

(72) Inventors:
- **Cascao-Pereira, Luis**
**PA, CA 94304 (US)**
- **Kellis, James T., Jr.**
**PA, CA 94304 (US)**
- **Estell, David A.**
**PA, CA 94304 (US)**

(74) Representative: **Forrest, Graham Robert et al**
**Mewburn Ellis LLP**
**33 Gutter Lane**
**London**
**EC2V 8AS (GB)**

Remarks:
•This application was filed on 11-09-2012 as a divisional application to the application mentioned under INID code 62.
•Claims filed after the date of receipt of the divisional application (Rule 68(4) EPC).

(54) **Compositions and methods comprising variant microbial proteases**

(57) The present invention provides methods for protein engineering. Specifically, the invention provides methods utilizing site evaluation libraries to design libraries that optimize two or more properties of a protein. The present invention also provides variant subtilisins suitable for various uses.

EP 2 578 679 A1

**Description**

## FIELD OF THE INVENTION

The present invention provides methods for protein engineering and variant proteases. Specifically, the invention provides methods utilizing site evaluation libraries. The present invention also provides subtilisin variants suitable for various uses.

## BACKGROUND OF THE INVENTION

Serine proteases are a subgroup of carbonyl hydrolases comprising a diverse class of enzymes having a wide range of specificities and biological functions. Much research has been conducted on the subtilisins, due largely to their usefulness in cleaning and feed applications. Additional work has been focused on the adverse environmental conditions (e.g., exposure to oxidative agents, chelating agents, extremes of temperature and/or pH) which can adversely impact the functionality of these enzymes in various applications. Nonetheless, there remains a need in the art for enzyme systems that are able to resist these adverse conditions and retain or have improved activity over those currently known in the art.

Various protein engineering methods are known to those in the art. In general, proteins are modified in order to obtain desired protein properties. In most methods, the nucleotide sequence of a cloned gene encoding a protein is mutated and the modified gene is expressed to produce mutants, which are screened for activities of interest. Often, the mutant properties are compared with the properties of wild-type protein.

Historically, the protein design process has been approached as equivalent to the problem of finding in all of protein space the one best sequence for the desired application. This problem is extremely difficult and is "NP hard." In complexity theory, problems defined as being in class P, are considered easy and efficient, polynomial-time algorithms exist for their solution. NP-hard problems are problems for which efficient polynomial-time algorithms are not currently known, and if any NP-hard problem could be solved, all NP-hard problems could be solved *(See e.g.,* Pierce and Winfree, Protein Engineer., 15:779-782 [2002]). Current strategies for building and screening libraries generally involve generating protein sequence diversity randomly across the whole sequence or in controlled random fashion at defined positions within the protein. These libraries generally have a large number of members that are "negative" with respect to the primary property of interest, and require large numbers be screened in order to find the relatively small numbers of positive mutations. Generally, negative mutations are ignored, and sequence information is only obtained for the positive members.

Saturation mutagenesis (Estell et al., in World Biotech Report 1984, vol. 2: USA, Online Publications, London [1984], pages 181-187; and Wells et al., Gene 34:315-323 [1985]) is one technique that can be used to search protein space for mutations that optimize several properties in a protein. Several groups have developed strategies for identifying sites to be changed by saturation mutagenesis (Reetz et al., Agnew. Chem. Int. Edn., 44:4192-4196 [2005]; Kato et al., J. Mol. Biol., 351:683-692 [2005]; and Sandberg et al., Proc. Natl. Acad. Sci., 90:8367-8371 [1993]), but no general system for site identification has been proposed.

In addition, because most protein engineering methods produce a great number of amino acid mutation options, screening of a large number of variants generally is required to produce a desired protein property. Generally, screening is repeated over and over to produce a beneficial variant. Thus, most methods are laborious and time-consuming. There is a continuing need in the art for protein engineering methods that are efficient and produce the desired results.

## SUMMARY OF THE INVENTION

The present invention provides methods for protein engineering. Specifically, the invention provides methods utilizing site evaluation libraries. In particular, the present invention provides means to use information obtained about a number of desired properties, in order to rationally and efficiently design libraries that will optimize those properties. In some embodiments, the present invention provides means to design libraries that are improved for at least two desired properties.

The present invention provides means to identify positions within an amino acid sequence of a protein that are relevant in improving desired properties of the protein. In some particularly preferred embodiments, the present invention provides means to determine which mutations are desirable in order to produce proteins with these desired properties, as well as improved properties. In some additional particularly preferred embodiments, the present invention provides means to identify amino acid positions and mutations that have improvements of a particular percentage better than the wild-type protein (e.g., better than 110% of the wild-type for one property). In still further preferred embodiments, the present invention provides means to identify mutations that provide at least one much improved property and at least one additional property that is not significantly worse than the wild-type protein (e.g., better than 110% of wild-type for one property, yet not worse than 90% of wild-type for another property). In yet further preferred embodiments, libraries are constructed based on this information. In some embodiments, the libraries are constructed using all of the identified mutations, while in some other embodiments, the libraries are constructed using a subset of the identified mutations. Indeed, it is not intended that the libraries be constrained to any particular number and/or type of mutations.

The present invention provides methods for protein engineering comprising the steps of: providing a library of protein variants; testing the library of protein variants for at least one property of interest in a test of interest; identifying a range of values for said the at least one property of interest; identifying a minimum within the range of values that is associated with a favorable outcome in the test of interest; and providing a plurality of protein variants having at least one mutation above said minimum in the range of the at least one property of interest, thereby providing a library of protein variants comprising at least one mutation, and wherein the library is enriched in members having a favorable outcome in the test of interest. In some embodiments, the favorable outcome corresponds to a value of greater than about 50%, about 60%, about 70%, about 80%, about 90%, or about 95% of a maximal value observed in the test set forth in the first step above. In some alternative embodiments, more than one test of interest is used in the methods of the present invention. In some preferred embodiments, the protein is an enzyme. In some particularly preferred embodiments, the enzyme is selected from proteases, transferases, metalloproteases, esterases, amylases, cellulases, oxidases, cutinases, and lipases.

The present invention also provides methods for protein engineering comprising the steps of: providing a library of protein variants; testing the library of protein variants for at least two properties of interest in a test of interest; identifying a range of values for the at least two properties of interest; identifying a minimum within the range of values that is associated with a favorable outcome in the test of interest; and providing a plurality of protein variants above the minimum of the range of the at least two properties of interest, thereby providing a library of protein variants enriched in members having the favorable outcome in the test of interest. In some preferred embodiments, the favorable outcome corresponds to a value of greater than about 50%, about 60%, about 70%, about 80%, about 90%, or about 95% of a maximal value observed in the test set forth in the first step above. In some preferred embodiments, the protein is an enzyme. In some particularly preferred embodiments, the enzyme is selected from proteases, transferases, metalloproteases, esterases, amylases, cellulases, oxidases, cutinases, and lipases.

The present invention also provides methods for protein engineering comprising the steps of: providing a wild-type protein and a library of protein variants of the wild-type protein; testing the library of protein variants and the wild-type protein for at least one property of interest in a test of interest; identifying a range of values for the at least one property of interest; identifying a minimum within the range of values that is associated with a favorable outcome in the test of interest; identifying the protein variants having a favorable outcome as compared to the results obtained for the wild-type, wherein the favorable outcome is an improved property of interest; and providing a plurality of protein variants above the minimum of the range of the at least one property of interest, thereby providing a library of improved protein variants enriched in members having the favorable outcome in the test of interest. In some preferred embodiments, the methods further comprise the step of determining the performance index, wherein the performance index is determined by dividing the value obtained for each of the improved protein variants and the value obtained for the wild-type protein. In some particularly preferred embodiments, the methods further comprise the step of identifying the improved protein variants, wherein the improved protein variants achieve performance index values greater than 1.1 in the test of interest. In some additional embodiments, the protein is an enzyme. In some particularly preferred embodiments, the enzyme is selected from proteases, transferases, metalloproteases, esterases, amylases, cellulases, oxidases, cutinases, and lipases. In some alternative embodiments, the protein is selected from antibodies and growth factors. In still additional preferred embodiments, the wild-type protein is a mature form an enzyme selected from proteases, transferases, metalloproteases, esterases, amylases, cellulases, oxidases, cutinases, and lipases. In some preferred embodiments, the property of interest is selected from charge, wash performance, hard surface cleaning performance, solubility, thermal stability, storage stability, detergent stability, substrate binding, enzyme inhibition, expression level, reaction rate, and substrate degradation. In some embodiments, the wild-type protein and the protein variant are components of at least one detergent composition. In some preferred embodiments, wash performance is tested in a detergent composition formulated into a powdered or liquid detergent having a pH of between 5 and 12.0.

The present invention also provides methods for producing an improved variant of a parent protein within a protein fold, comprising: assaying multiple variants of a test protein within the protein fold spanning a range of a property of interest in an assay of interest; identifying a minimum within the range of the property of interest that is associated with a favorable outcome in the assay of interest; assaying a parent protein of the protein fold in the assay of interest; and producing an improved variant of the parent protein by introducing an amino acid substitution is the parent protein such that the improved variant is above the minimum of the range of the property of interest. In some preferred embodiments, the parent protein and the test protein are different. In some embodiments, the methods further comprise the step of determining the performance index, wherein the performance index is determined by dividing the value obtained for the improved protein variant and the value obtained for the parent protein. In some embodiments, the test proteins and the parent proteins are enzymes. In some particularly preferred embodiments, the enzymes are selected from proteases, transferases, metalloproteases, esterases, amylases, cellulases, oxidases, cutinases, and lipases. In some alternative embodiments, the test and parent proteins are selected from antibodies and growth factors. In still additional preferred embodiments, the parent protein is a mature form an enzyme selected from proteases, transferases, metalloproteases, esterases, amylases, cellulases, oxidases, cutinases, and lipases. In some preferred embodiments, the property of interest is selected from charge, wash performance, hard surface cleaning performance, thermal stability, solubility, storage stability, detergent stability, substrate binding, enzyme inhibition, expression level, reaction rate, and substrate degradation. In some embodiments, the test and parent proteins are components of at least one detergent composition. In some alternative embodiment, the improved protein variant is a component of a detergent composition. In some preferred embodiments, wash performance is tested in a detergent composition formulated into a powdered or liquid detergent having a pH of between about 5 and about 12.0.

The present invention also provides isolated subtilisin variants of a *Bacillus* subtilisin, wherein the subtilisin variant is a mature form having proteolytic activity and comprising a substitution at one or more positions selected from positions: 1, 2, 3, 4, 5, 6, 7, 8, 9, 10, 11, 12, 13, 14, 15, 16, 17, 18, 19, 20, 21, 21, 22, 23, 24, 25, 26, 27, 28, 29, 30, 31, 33, 35, 36, 37, 39, 40, 41, 42, 43, 44, 45, 46, 47, 48, 49, 50, 51, 52, 53, 54, 55, 56, 57, 58, 59, 60, 62, 65, 66, 67, 71, 72, 73, 76, 77, 78, 79, 81, 82, 83, 84, 85, 86, 87, 88, 89, 90, 91, 92, 93, 94, 95, 96, 97, 98, 99, 101, 102, 103, 104, 105, 106, 107, 109, 110, 111, 112, 113, 114, 115, 116, 117, 118, 119, 122, 123, 124, 125, 126, 127, 128, 129, 130, 131, 132, 133, 134, 135, 136, 137, 139, 141, 143, 144, 145, 146, 147, 148, 149, 150, 151, 152, 153, 156, 157, 158, 159, 160, 162, 163, 164, 165, 166, 167, 169, 170, 171, 172, 173, 174, 175, 176, 177, 178, 179, 180, 181, 182, 183, 184, 185, 186, 187, 188, 189, 190, 191, 192, 194, 195, 196, 197, 198, 199, 200, 201, 203, 204, 206, 207, 209, 210, 212, 213, 214, 215, 216, 217, 218, 219, 220, 222, 223, 224, 225, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 267, 268, 269, 270, 271, 272, 273 and 274, wherein the positions are numbered by correspondence with the amino acid sequence of *B. amyloliquefaciens* subtilisin BPN' set forth as SEQ ID NO:2. In some embodiments, the one or more positions are selected from: 1, 2, 5, 6, 7, 8, 9, 10, 11, 12, 13, 14, 15, 16, 17, 18, 19, 20, 21, 21, 22, 23, 24, 25, 26, 28, 29, 30, 31, 33, 35, 36, 37, 39, 40, 41, 42, 43, 44, 45, 46, 47, 48, 49, 50, 51, 52, 53, 54, 55, 56, 57, 58, 59, 60, 62, 65, 66, 67, 71, 72, 73, 77, 78, 79, 81, 82, 83, 84, 85, 86, 87, 88, 89, 90, 91, 92, 93, 94, 95, 96, 97, 98, 99, 101, 102, 103, 104, 105, 106, 107, 109, 110, 111, 112, 113, 114, 115, 116, 118, 119, 122, 123, 124, 125, 126, 127, 128, 129, 131, 133, 134, 135, 136, 137, 139, 141, 143, 144, 145, 146, 147, 148, 149, 150, 151, 152, 153, 156, 157, 158, 159, 160, 162, 163, 164, 165, 166, 167, 169, 170, 171, 172, 173, 174, 175, 176, 177, 178, 179, 180, 181, 182, 183, 184, 185, 186, 187, 188, 189, 190, 191, 192, 194, 195, 196, 197, 198, 199, 200, 201, 203, 204, 206, 207, 212, 213, 214, 215, 216, 217, 218, 219, 220, 222, 223, 224, 225, 228, 229, 230, 231, 232, 233, 234, 235, 236, 237, 238, 239, 240, 241, 242, 243, 244, 245, 246, 247, 248, 249, 250, 251, 253, 254, 255, 256, 257, 258, 259, 260, 261, 262, 263, 264, 265, 267, 268, 269, 270, 271, 272, 273 and 274, and wherein the positions are numbered by correspondence with the amino acid sequence of *B. amyloliquefaciens* subtilisin BPN' set forth as SEQ ID NO:2. In some further embodiments, the one or more positions are selected from: 2, 3, 5, 6, 7, 8, 9, 10, 11, 12, 13, 14, 15, 16, 17, 19, 20, 21, 21, 22, 23, 25, 26, 28, 29, 30, 31, 33, 35, 36, 37, 39, 40, 41, 42, 44, 46, 47, 48, 49, 50, 51, 53, 54, 55, 56, 57, 58, 59, 60, 62, 65, 66, 67, 71, 73, 77, 78, 79, 81, 82, 83, 84, 85, 86, 88, 89, 90, 91, 92, 93, 94, 95, 96, 102, 105, 106, 110, 111, 112, 113, 114, 115, 116, 122, 123, 124, 125, 126, 128, 129, 131, 133, 134, 135, 136, 137, 139, 141, 143, 145, 146, 147, 148, 149, 150, 151, 152, 153, 156, 157, 158, 159, 160, 162, 165, 166, 167, 169, 170, 171, 173, 174, 175, 176, 177, 178, 179, 180, 181, 182, 183, 184, 186, 187, 189, 190, 191, 192, 194, 195, 196, 197, 198, 199, 200, 201, 203, 204, 206, 207, 212, 214, 216, 217, 218, 219, 220, 222, 223, 224, 225, 228, 229, 230, 231, 232, 233, 234, 235, 236, 237, 238, 239, 240, 241, 242, 243, 244, 245, 246, 247, 249, 250, 251, 253, 254, 255, 257, 258, 259, 260, 261, 262, 263, 264, 265, 267, 268, 269, 270, 271, 272, 273, and 274, and wherein the positions are numbered by correspondence with the amino acid sequence *of B. amyloliquefaciens* subtilisin BPN' set forth as SEQ ID NO:2. In some embodiments, the subtilisin variant further comprises a substitution at one or more positions selected from 18, 52, 72, 117, 119, 127, 144, 185, 209 and 213, and wherein the positions are numbered by correspondence with the amino acid sequence *of B. amyloliquefaciens* subtilisin BPN' set forth as SEQ ID NO:2. In some preferred embodiments, the substitution comprises one or more of the positions: A001C, A001D, A001E, A001 F, A001 G, A001 H, A001I, A001 K, A001 L, A001 M, A001 N, A001 Q, A001R, A001 S, A001V, A001W, Q002A, Q002D, Q002E, Q002G, Q002S, S003A, S003D, S003F, S003G, S003I, S003K, S003L, S003M, S003N, S003P, S003Q, S003R, S003T, S003V, S003W, S003Y, P005C, P005E, P005G, P005N, P005Q, P005T, Y006A, Y006C, Y006E, Y006F, Y006G, Y006K, Y006M, Y006N, Y006P, Y006Q, Y006R, Y006T, Y006V, Y006W, S009A, S009C, S009E, S009F, S009G, S009H, S009K, S009L, S009M, S009P, S009Q, S009R, S009T, S009V, S009W, Q010A, Q010C, Q010E, Q010F, Q010G, Q010H, Q010I, Q010K, Q010L, Q010M, Q010R, Q010S, Q010T, Q010V, Q010W, I011C, I011L, I011T, I011V, I011Y, K012A, K012C, K012D, K012E, K012F, K012G, K012H, K012I, K012N, K012Q, K012S, K012T, K012V, K012W, K012Y, A013C, A013F, A013G, A013H, A013L, A013R, A013S, A013T, A013V, P014A, P014C, P014D, P014E, P014F, P014G, P014I, P014K, P014L, P014M, P014N, P014Q, P014R, P014S, P014T, P014V, P014W, P014Y, A015C, A015D, A015E, A015F, A015G, A015H, A0151, A015K, A015L, A015M, A015P, A015Q, A015R, A015T, A015V, A015Y, L016A, L016C, L016I, L016M, L016Q, L016S, L016T, H017F, H017I, H017L, H017M, H017V, H017W, H017Y, S018A, S018E, S018F, S018G, S018H, S018I, S018K, S018L, S018M, S018N, S018P, S018Q, S018R, S018T, S018V, S018W, S018Y, Q019A, Q019C, Q019D, Q019E, Q019G, Q019I, Q019K, Q019M, Q019R, Q019T, Q019V, G020A, G020C, G020D, G020E, G020F, G020H, G020K, G020M, G020N, G020P, G020Q, G020R, G020S, G020W, Y021A, Y021C, Y021D, Y021E, Y021F, Y021G, Y021H, Y021K, Y021M, Y021R, Y021S, Y021T, Y021V, Y021W, T022A, T022C, T022E, T022F, T022G, T022H, T022I, T022K, T022L, T022M, T022N, T022Q, T022S, T022V, T022W, T022Y, S024C, S024F, S024G, S024H, S024I, S024K, S024L, S024M, S024N, S024Q, S024R, S024T, S024W, S024Y, N025C, N025E, N025F, N025G, N025H, N0251, N025K, N025L, N025M, N025P, N025R, N025S, N025T, N025V, N025W, K027A, K027D, K027E, K027F, K027G, K027H, K027L, K027M, K027P, K027R, K027S, K027W, K027Y, S033A, S033F, S033G, S033H, S033P, S033T, S033W, I035A, I035C, I035D, I035R, I035S, I035T, 1035V, D036A, D036C, D036E, D036Q, D036S, D036W, S037A, S037C, S037E, S037F, S037G, S037H, S037K, S037L, S037M, S037P, S037Q, S037R, S037T, S037W, H039C, H039Q, H039T, H039V, P040A, P040C, P040E, P040F, P040G, P040G, P040H, P040I, P040K, P040L , P040M, P040N, P040Q, P040R, P040S, P040T, P040V, P040W, D041E, L042I, L042M, L042V, K043A, K043C, K043D, K043E, K043F, K043G, K043I, K043L, K043M, K043N, K043R, K043S, K043T, K043V, K043W, K043Y, V044A, V044C, V044I, V044L, V044M, V044P, V044S, V044T, A045C, A045E, A045F, A045H, A045I, A045K, A045L, A045M, A045N, A045P, A045Q, A045S, A045T, A045V, A045Y, G046A, G046C, G046E, G046H, G046K, G046M, G046N, G046Q, G046T, G046W, G046Y, A048C, A048D, A048E, A048F, A048H, A048I, A048K, A048L, A048M, A048Q, A048R, A048S, A048T, A048V, A048W, A048Y, M050A, M050C, M050F, M050H, M050I, M050K, M050L, M050Q, M050R, M050S, M050T, M050V, M050W, M050Y, V051A, V051C, V051D, V051E, V051H, V051I, V051L, V051P, V051Q, P052C, P052D, P052E, P052F, P052H, P052I, P052K, P052L, P052Q, P052R, P052S, P052T, P052V, P052W, P052Y, S053E, S053F, S053G, S053H, S053I, S053K, S053L, S053M, S053N, S053Q, S053R, S053T, S053V, S053W, E054A, E054N, E054Q, E054S, T055A, T055C, T055D, T055F, T055G, T055H, T055I, T055K, T055M, T055P, T055Q, T055R, T055S, T055V, T055W, T055Y, N056D, N056M, N056P, N056Q, N056S, N056T, N056V, P057N, P057Q, P057T, P057V, P057W, F058C, F058E, F058G, F058H, F058L, F058M, F058N, F058Q, F058S, F058V, F058Y, Q059A, Q059C, Q059D, Q059E, Q059F, Q059G, Q059H, Q059K, Q059L, Q059M, Q059N, Q059P, Q059R, Q059S, Q059T, Q059V, Q059W, Q059Y, D060E, D060G, N062A, N062C, N062D, N062E, N062F, N062G, N062H, N0621, N062K, N062L, N062M, N062Q, N062R, N062S, N062T, N062V, N062W, N062Y, T066S, T066W, T066Y, H067A, H067C, H067F, H067I, H067L, H067M, H067N, H067P, H067R, H067S, H067T, T071I, T071Y, N077S, S078A, S078C, S078D, S078F, S078G, S078I, S078K, S078L, S078M, S078N, S078P, S078Q, S078R, S078T, S078V, S078W, S078Y, 1079A, 1079C, 1079E, 1079F, 1079G, 1079H, 1079K, 1079L, 1079N, 1079Q, 1079R, I079S, 1079T, 1079V, 1079W, 1079Y, V081F, V081I, V081L, V081M, V081T, G083F, G083P, Q084A, Q084C, Q084H, Q084I, Q084N, Q084T, A085C, A085F, A085G, A085R, A085S, P086A, P086D, P086E, P086G, P086M, P086N, P086Q, P086R, P086S, P086T, P086W, P086Y, S089C, S089D, S089E, S089F, S089G, S089H, S089K, S089L, S089V, S089W, S089Y, L090A, L090D, L090E, L090G, L090H, L090M, L090Q, L090T, L090V, Y091A, Y091C, Y091D, Y091E, Y091F, Y091H, Y091I, Y091L, Y091M, Y091Q, Y091S, Y091T, Y091V, L096F, L096H, L096I, L096K, L096M, L096T, L096V, G097A, G097C, G097D, G097E, G097H, G097K, G097L, G097M, G097P, G097Q, G097R, G097S, G097T, G097V, A098C, A098D, A098F, A098G, A098H, A098I, A098L, A098P, A098Q, A098R, A098S, A098T, A098V, A098Y, D099C, D099E, D099I, D099K, D099L, D099N, D099P, D099Q, D099R, D099S, D099T, S101A, S101C, S101E, S101F, S101G, S101I, S101K, S101L, S101M, S101N, S101P, S101Q, S101R, S101T, S101V, S101Y, G102A, G102C, G102E, G102F, G102I, G102N, G102S, G102V, G102Y, Q103A, Q103C, Q103E, Q103F, Q103G, Q103I, Q103K, Q103L, Q103M, Q103N, Q103R, Q103S, Q103T, Q103V, Q103W, S105A, S105C, S105D, S105E, S105F, S105G, S105I, S105K, S105L, S105M, S105N, S105P, S105R, S105V, S105W, W106A, W106C, W106E, W106F, W106G, W106H, W106I, W106L, W106M, W106N, W106R, W106S, W106T, W106V, W106Y, N109A, N109C, N109E, N109F, N109G, N109H, N109L, N109M, N109P, N109Q, N109R, N109S, N109T, N109V, N109W, N109Y, I111C, I111F, I111L, I111M, I111N, I111P, I111T, I111V, I111W, E112A, E112C, E112D, E112F, E112G, E112I, E112L, E112M, E112N, E112Q, E112R, E112S, E112T, E112V, E112W, E112Y, W113D, W113E, W113F, W113N, W113P, W113Q, W113S, W113V, W113Y, I115E, I115H, I115N, I115R, I115T, I115V, I115W, I115Y, A116C, A116D, A116E, A116F, A116G, A116H, A116I, A116K, A116L, A116M, A116N, A116P, A116Q, A116R, A116S, A116T, A116V, A116W, A116Y, N118A, N118C, N118D, N118E, N118F, N118G, N118H, N118I, N118K, N118L, N118M, N118Q, N118R, N118S, N118T, N118V, N118W, N118Y, I122A, I122C, I122H, I122K, I122L, I122M, I122N, I122P, N123A, N123C, N123D, N123E, N123G, N123I, N123L, N123M, N123Q, N123S, N123T, N123V, M124C, M124D, M124I, M124L, M124S, M124T, M124V, M124W, S125A, S125C, S125E, S125F, S125I, S125K, S125M, S125N, S125P, S125Q, S125R, S125T, S125W, L126A, L126C, L126I, L126K, L126N, L126R, L126S, L126T, L126V, L126W, L126Y, G127A, G127C, G127E, G127F, G127I, G127K, G127M, G127N, G127P, G127Q, G127S, G127T, G127V, G127W, G127Y, G128A, G128D, G128F, G128H, G128L, G128N, G128P, G128S, G128T, G128V, G128W, G128Y, P129A, P129C, P129D, P129E, P129F, P129G, P129I, P129K, P129L, P129M, P129N, P129Q, P129S, P129T, P129V, P129Y, G131A, G131C, G131E, G131F, G131K, G131L, G131M, G131N, G131P, G131Q, G131R, G131S, G131T, G131V, G131W, G131Y, A133C, A133D, A133E, A133F, A133G, A133I, A133K, A133L, A133M, A133P, A133R, A133S, A133T, A133V, A133W, A133Y, A134D, A134E, A134F, A134G, A134I, A134K, A134L, A134M, A134P, A134Q, A134R, A134S, A134T, A134V, A134W, L135D, L135E, L135M, L135R, L135W, L135Y, K136E, K136H, K136L, K136M, K136N, K136R, K136S, K136T, K136V, K136W, K136Y, A137C, A137E, A137F, A137H, A137K, A137L, A137M, A137N, A137P, A137Q, A137R, A137S, A137T, A137V, A137W, A137Y, V139C, V139I, V139L, V139N, V139S, V139T, K141A, K141C, K141D, K141E, K141F, K141G, K141H, K141I, K141L, K141M, K141N , K141Q, K141R, K141S, K141V, K141W, K141Y, V143A, V143C, V143D, V143E, V143F, V143G, V143K, V143L, V143M, V143N, V143Q, V143R, V143S, V143T, V143W, A144C, A144D, A144E, A144G, A144I, A144K, A144L, A144M, A144R, A144S, A144T, A144V, A144W, S145A, S145C, S145D, S145E, S145F, S145G, S145H, S145I, S145L, S145M, S145Q, S145R, S145T, S145V, S145W, S145Y, G146A, G146C, G146D, G146E, G146M, G146Q, G146R, G146S, G146T, G146Y, A153S, G154L, G154P, G154T, E156A, E156C, E156F, E156K, E156L, E156N, E156Q, E156R, E156S, E156T, E156V, E156W, E156Y, T158A, T158D, T158E, T158G, T158H, T158I, T158K, T158L, T158M, T158N, T158Q, T158S, T158V, T158Y, S159A, S159C, S159D, S159E, S159G, S159H, S159I, S159K, S159M, S159Q, S159R, S159T, G160D, G160E, G160K, G160N, G160P, G160Q, G160S, G160T, S162A, S162C, S162E, S162H, S162K, S162L, S162M, S162N, S162Q, S162R, S162T, S162V, S163G, S163P, Y167A, Y167F, Y167H, Y167I, P168D, P168G, P168I, P168M, P168S, P168Y, G169A, G169E, G169F, G169H, G169I, G169M, G169N, G169R, G169T, G169V, G169Y, K170A, K170C, K170F, K170G, K170H, K170R, K170V, K170W, K170Y, Y171G, Y171K, Y171P, P172A, P172C, P172E, P172K, P172L, P172M, P172N, P172Q, P172R, P172S, P172T, P172V, P172Y, S173A, S173C, S173E, S173I, S173T, S173V, V174C, V174F, V174H, V174R, V174T, I175G, I175L, I175M, I175R, I175T, I175V, A176C, A176E, A176F, A176K, A176M, A176S, A176Y, A179G , V180A, V180C, V180L, V180N, V180S, V180T, D181C, D181E, D181G, D181H, D181M, D181N, D181S, D181T, D181W, S182A, S182C, S182D, S182F, S182G, S182H, S182I, S182K, S182M, S182P, S182Q, S182R, S182V, S182Y, S183A, S183C, S183E, S183F, S183G, S183H, S183I, S183K, S183L, S183M, S183N, S183Q, S183R, S183T, S183V, S183W, S183Y, N184C, N184D, N184E, N184G, N184H, N184K, N184L, N184M, N184Q, N184S, N184T, N184V, N184W, Q185A, Q185C, Q185E, Q185F, Q185G, Q185H, Q185I, Q185K, Q185L, Q185M, Q185N, Q185S, Q185T, Q185V, Q185W, R186I, R186L, R186W, A187C, A187D, A187E, A187F, A187G, A187P, A187S, A187W, A187Y, S188A, S188C, S188D, S188E, S188F, S188G, S188H, S188I, S188K, S188L, S188M, S188P, S188Q, S188T, S188V, S188W, S188Y, S190F, S190H, S190I, S190K, S191A, S191G, S191N, S191P, V192A, V192S, V192T, V192Y, G193F, G193H, G193I, G193N, G193P, G193R, G193T, P194C, P194E, P194H, P194I, P194K, P194L, P194M, P194Q, P194T, P194V, P194W, L196A, M199P, M199S, A200C, A200G, A200K, A200Y, G202D, G202E, G202F, G202L, G202P, G202V, G202Y, Q206A, Q206C, Q206D, Q206E, Q206F, Q206G, Q206H, Q206I, Q206L, Q206M, Q206N, Q206P, Q206R, Q206S, Q206T, Q206V, Q206W, Q206Y, S207D, S207E, S207K, S207Q, S207T, S207V, P210A, P210C, P210S, P210T, N212A, N212C, N212E, N212F, N212G, N212H, N212K, N212L, N212M, N212P, N212Q, N212R, N212S, N212V, K213A, K213C, K213D, K213E, K213F, K213H, K213I, K213L, K213M, K213N, K213Q, K213R, K213S, K213T, K213V, K213Y, Y214W, G215A, G215C, G215D, G215E, G215I, G215M, G215N, G215Q, G215S, G215T, G215V, A216C, A216D, A216E, A216G, A216K, A216L, A216M, A216N, A216P, A216Q, A216R, A216S, A216V, A216W, Y217A, Y217C, Y217D, Y217E, Y217F, Y217G, Y217H, Y217I, Y217K, Y217L, Y217M, Y217N, Y217Q, Y217R, Y217S, Y217T, Y217V, Y217W, N218A, N218C, N218E, N218F, N218G, N218H, N218K, N218M, N218R, N218S, N218T, N218W, N218Y, G219A, G219C, G219D, G219H, G219I, G219M, G219P, G219Q, G219R, G219S, G219T, G219V, G219W, T220D, T220E, T220F, T220G, T220K, T220M, T220S, T220Y, A223E, A223F, A223L, A223M, A223R, A223S, A223V, A223W, A223Y, S224A, S224C, S224F, S224G, S224H, S224M, S224N, S224Q, S224R, S224T, P225A, P225C, P225F, P225G, P225H, P225I, P225K, P225L, P225M, P225R, P225S, P225T, P225V, P225Y, A228P, A228R, A228S, A228T, A228W, G229A, G229H, G229I, G229S, A230C, A230E, A230G , A230Q, A230R, A230S, A230T, A230V, A231C, A231I, A231P, A231R, I234A, I234C, I234L, I234M, I234N, I234P, I234Q, I234S, I234T, I234V, L235A, L235C, L235G, L235I, L235K, L235M, L235N, L235Q, L235R, L235S, L235T, L235V, L235W, L235Y, S236A, S236C, S236D, S236E, S236G, S236H, S236N, S236Q, S236T, S236V, S236Y, K237A, K237E , K237F, K237G, K237H, K237I, K237L, K237M, K237N, K237Q, K237R, K237S, K237T, K237V, K237W, K237Y, H238C, H238D, H238E, H238F, H238M, H238R, H238S, P239C, P239D, P239E, P239F, P239H, P239L, P239M, P239N, P239Q, P239R, P239S, P239T, P239V, P239W, P239Y, N240A, N240C, N240D, N240F, N240G, N240K, N240L, N240Q, N240R, N240S, N240T, N240V, N240W, N240Y, W241A, W241F, W241G, W241H, W241I, W241K, W241M, W241Q, W241T, W241V, T242A, T242C, T242E, T242F, T242G, T242K, T242M, T242N, T242P, T242R, T242S, T242W, T242Y, N243C, N243E, N243F, N243G, N2431, N243Q, N243S, N243T, N243V, N243W, N243Y, T244A, T244D, T244F, T244G, T244H, T244K, T244L, T244M, T244N, T244P, T244Q, T244R, T244S, T244V, T244W, T244Y, Q245A, Q245C, Q245D, Q245E, Q245H, Q245I, Q245K, Q245L, Q245M, Q245R, Q245T, Q245V, Q245Y, R247W, S248A, S248E, S248F, S248G, S248H, S248I, S248L, S248M, S248N, S248Q, S248R, S248T, S248V, S248W, S248Y, S249C, S249D, S249H, S249I, S249K, S249L, S249M, S249N, S249Q, S249R, S249T, S249V, S249W, S249Y, L250D, L250F, L250H, L250I, L250M, L250T, L250V, E251A, E251T, E251W, N252A, N252C, N252E, N252G, N252H, N2521, N252L, N252M, N252Q, N252R, N252S, N252T, N252V, N252Y, T253A, T253C, T253E, T253G, T253H, T253K, T253M, T253S, T253V, T254A, T254C, T254L, T254M, T254R, T254S, T254V, T255A, T255C, T255D, T255E, T255F, T255G, T255H, T255I, T255K, T255L, T255M, T255R, T255S, T255V, K256A, K256C, K256D, K256E, K256F, K256H, K256I, K256L, K256M, K256N, K256P, K256Q, K256R, K256S, K256T, K256V, K256W, K256Y, L257A, L257C, L257F, L257G, L257H, L257I, L257K, L257M, L257N, L257R, L257S, L257T, L257V, L257W, L257Y, G258Q, D259A, D259E, D259F, D259G, D259L, D259N, D259P, D259Q, D259R, D259S, D259T, D259V, D259W, D259Y, S260A, S260C, S260D, S260E, S260F, S260G, S260H, S260L, S260M, S260N, S260P, S260R, S260V, S260W, S260Y, F261H, F261W, Y262A, Y262C, Y262E, Y262F, Y262H, Y262L, Y262M, Y262N, Y263F, Y263M, Y263T, G264F, G264I, G264L, K265A, K265C, K265E, K265G, K265H, K265L, K265M, K265N, K265Q, K265R, K265S, K265W, K265Y, G266C, G266F, G266L, G266M, G266P, G266R, G266V, G266W, G266Y, L267A, L267C, L267E, L267G, L267H, L267I, L267M, L267N, L267Q, L267S, L267T, L267V, I268A, I268C, I268K, I268L, I268M, I268P, I268R, 1268V, N269D, N269E, N269K, N269L, N269P, N269Q, N269S, Q271A, Q271C, Q271D, Q271E, Q271F, Q271G, Q271H, Q271I, Q271K, Q271L, Q271M, Q271N, Q271P, Q271R, Q271S, Q271T, Q271V, Q271W, Q271Y, A272E, A272F, A272G, A272H, A272K, A272L, A272M, A272N, A272Q, A272R, A272S, A272T, A272V, A272W, A272Y, A274C, A274D, A274F, A274G, A274H, A274I, A274K, A274L, A274Q, A274S, A274T, and A274V.

In some preferred embodiments, the substitution comprises a combination selected from: Y021H-A045V-Y217E, Y021W-S101E-G128R-Y217Q, Y021H-Y217E, Y021H-A045V-S101N-Y217Q, Y021H-A045I-Y217E, Y021H-A045I-S101E-Y217Q, Y021W-A045I-S101E-Y217E, D036N-S101E-Y217L, Y021H-A045V-Y217Q, Y021H-A045I-S101E-Y217L, Y021H-A045I-Y217E, Y021H-Y217Q, Y021W-S101E-Y217L, Y021W-A045V-S101E-Y217L, Y021H-A045V-S101E-Y217E, S101E-Y217L, Y021H-A045V-S101E-Y217Q, Y021H-Y217L, Y021W-A045I-S101N-Y217L, S101N-K213I-Y217Q, Y021W-S101N-Y217L, Y021H-S101E-Y217E, M119F-K213K-Y217Q, Y021W-A045V-Y217E, Y021W-A045I-Y217E, M119F-K213I-Y217Q, Y021W-Y217E-N212S, M119F-K213L-Y217E, K213I-Y217Q, A045I-Y217Q, Y021W-A045V-S101E-Y217Q, Y021H-A045V-S101E-Y217L, S101S-M119F-K213I-Y217Q, K213N-Y217Q, M119F-Y217Q, S024H-A092G-A114G, S101N-M119F-K213I-Y217Q, S101N-K213L-Y217Q, S101N-M119F-K213K-Y217L, S101N-M119F-K213I-Y217L, Y021HA045V-S101E, S101N-K213L-Y217E, A045V-Y217L, S101N-K213I-Y217L, S101S-M119M-K213K-Y217L, Y021H-Y217L, S101E-K213L-Y217L, K213I-Y217E, S101N-M119F-Y217E, Y021W-A045V-Y217L, A092G-A114G, S024H-A092G-Q103E, Y021W-S101E, V26Q-K213I, Y021H-S101N, S101E-K213N-Y217E. In some embodiments, the substitution comprises a combination selected from: S024S-V028V-M050M-A092A-Q103E-A114G-V246V, S024S-V028V-M050V-A092A-Q103Q-A114A-V246V, S024S-V028V-M050V-A092A-Q103Q-A114G-V246V, S024H-V028V-M050V-A092A-Q103Q-A114A-V246T, S024H-V028V-M050V-A092A-Q103E-A114A-V246V, S024H-V028V-M050V-A092A-Q103Q-A114G-V246V, S024H-V028V-M050M-A092A-Q103E-A114A-V246V, S024H-V028V-M050M-A092A-Q103E-A114A-V246T, S024S-V028V-M050M-A092G-Q103Q-A114A-V246T, S024H-V028V-M050M-A092A-Q103Q-A114G-V246T, S101E-M119N-K213N, S101N-K213I, S101N-M119H, M119H-K213I-Y217L, S101N-M119H-K213N-Y217L, S101N-K213L-Y217E, M119N-K213N-Y217L, M119F-K213L-Y217E, S101P-K213N, S101N-M119H-K213N, S101N-M119F-K213I-Y217L, K213L, M119N-K213N, K213N, K213I-Y217E, S101N-M119H-Y217Q, S101P-K213N-Y217L, M119N-K213I, K213N-Y217Q, M119H-K213N, S101N-K213L-Y217Q, S101P-K213N, S101E-K213N-Y217E, S101E-M119H-K213I-Y217Q, S101E-M119H-K213N, K213I-Y217Q, S101N-K213I-Y217Q, M119H-Y217Q, S101N-M119N-K213N-Y217Q, M119H-K213I-Y217Q, K213I-Y217Q, S101E-M119N-Y217L, M119F-K213L, M119H-K213N-Y217E, S101N-M119N-K213N-Y217Q, S101N-M119H-K213N-Y217Q, M119H-K213I-Y217Q, Y217Q, M119H-K213N-Y217Q, S101N-M119F-Y217E, M119F-Y217Q, S101N-M119F-K213I-Y217Q, S101N-K213I-Y217L, S101N-M119H-K213N-Y217L, S101E-K213L-Y217L, S101N-K213N-Y217Q, S101N-M119H-K213L, S101N-M119H-K213I, S101P-K213N-Y217L, M119F-K213I-Y217Q, S101N-K213I-Y217Q, S101EM119H-K213I-Y217L, S101N-M119H-K213I-Y217Q, M119H-K213N-Y217E, S101N-M119N-K213N-Y217L, A048E-K213L, A048H-K213L, V026Q-A048Y-K213L, K213N, V026N-K213L, V026N-K213L, V026Y-K213N, A048D-K213N, V026Q-A048E-K213L, A048H-K213N, V026Q-A048H-K213L, A048H-K213L, K213L, K213N, V147D-K213L, K213I, V026Q-A048E-K213N, V026N-A048E-K213N, A048E-K213L, V026Y-A048E-K213I, A048D-K213N, K213I, A048H-K213N, V147D-K213N, Y021H-A045V-S101E-Y217L, Y021H-Y217L, Y021H-A045V-S101E-Y217Q, Y021H-A045I-S101E-Y217L, Y021H-Y217Q, Y021H-A045V-Y217E, A045V-Y217L, Y021H-A045V-S101N-Y217Q, Y021W-S101P-Y217L, Y021W-A045I-Y217E, Y021H-A045V-S101E-Y217E, Y021HA045I-S101E-Y217L, Y021H-A045I-S101E-Y217Q, Y021H-A045V-Y217E, Y021W-S101E-Y217L, S101E-Y217L, Y021H-A045V-Y217Q, Y021H-Y217E, Y021W-Y217E-(N0212S), A045I-Y217Q, Y021 H-A045 V-Y217E, Y021W-A045V-S101E-Y217Q, Y021H-S101E-Y217E, Y021W-S101N-Y217L, S024S-V028V-M050M-A092A-Q103Q-A114A-V246T, Y021H-A045V-S101E, Y021W-A045V-S101E-Y217L, S101N-M119H-K213K-Y217Q, S101S-M119N-K213N-Y217Q, Y021HA045V-S101P-Y217L, S024S-V028V-M050M-A092A-Q103Q-A114G-V246T, S101S-M119F-K213I-Y217Q, S101S-M119M-K213K-Y217L, Y021H-A045I-S101E-Y217Q, Y021H-A045V-S101E-Y217E, Y021H-A045V-S101E, Y021H-Y217L, Y021H-A045I-Y217E, Y021W-A045I-S101E-Y217E, S101N-M119F-K213K-Y217L, S101E-M119H-K213N-Y217E, Y021H-A045I-Y217E, S101S-M119H-K213L-Y217L, Y021H-Y217E, S101S-M119F-K213K-Y217Q, and Y021H-A045I-S101E-Y217L. In some embodiments, the substitution comprises a combination selected from: S024S-V028T-M050V-A092G-Q103E-A114G-V246T, S101N-M119H, M119N-K213N-Y217L, Y217L, M119N-K213N, K213N, K213N-Y217Q, S101E-K213N-Y217E, S101E-M119N-Y217L, Y217Q, S101N-M119N-K213N-Y217E, S101N-M119N-K213N-Y217L, Y021H-A045I-S101E-Y217L, S101E-Y217L, A045I-Y217Q, S101S-M119M-K213K-Y217L, Y021H-A045I-S101E-Y217L.

The present invention further provides the variants set forth in the Tables included in the Examples, as well as compositions comprising these variants.

Also provided by the present invention are isolated nucleic acids encoding the subtilisin variant, expression vectors comprising the nucleic acids, and host cells comprising the expression vector. Moreover the present invention provides cleaning compositions comprising the subtilisin variant. In some embodiments, the cleaning composition is a laundry detergent. In some embodiments, the laundry detergent is a cold water detergent, a low pH detergent, or a compact detergent. Moreover the present invention provides methods for producing a subtilisin variant of a *Bacillus* subtilisin, comprising: transforming a host cell with an expression vector comprising a nucleic acid encoding the subtilisin variant; and cultivating the transformed host cell under conditions suitable for the production of the subtilisin variant. In some embodiments, the methods further comprise the step of harvesting the produced subtilisin variant. In some embodiments, the host cell is a *Bacillus* species, and in a subset of these embodiments, the *Bacillus* species is *B. subtilis.* Moreover the present invention provides a method of cleaning, comprising the step of contacting a surface and/or an article comprising a fabric with a cleaning composition comprising an isolated subtilisin variant.

Additionally the present invention provides methods for protease engineering comprising the steps of: a) providing a plurality of site evaluation libraries (SELs) each comprising a plurality of protease variants having distinct substitutions at an identical amino acid position of the protease; b) testing the protease variants of the SELs and a standard protease in a test of a property of interest; c) determining a performance index (PI) for each of the protease variants for the test; d) identifying two or more of the amino acid positions as non-restrictive positions, wherein at least one of the plurality of protease variants in each of two of the SELs has a PI greater than 0.5; and f) providing a multiple mutation library comprising a plurality of multiply-substituted protease variants each comprising substitutions in the two or more non-restrictive positions. In some embodiments, the test comprises two or more different assays selected from the group consisting of stain removal assays (microswatch), LAS stability assays, detergent stability assays, and specific activity assays. In some embodiments the protease is selected from the group consisting of a bacterial serine protease, a bacterial subtilisin, and a bacterial neutral metalloprotease.

In further embodiments, the present invention provides methods for producing a multiply substituted subtilisin variant of a *Bacillus* subtilisin, comprising: testing a plurality of singly-substituted subtilisin variants in a first test of a first property and a second test of a second property, wherein the property of a parent subtilisin is given a value of 1.0 in each test, a favorable first or second property has a value greater than 1.0, and an unduly unfavorable first or second property has a value less than about 0.80 or in some preferred embodiments, less than about 0.60; identifying a substitution in at least one of the singly-substituted subtilisin variants that is associated with a favorable first property and which is not associated with an unduly unfavorable second property; identifying a substitution in at least one of the singly-substituted subtilisin variants that is associated with a favorable second property and which is not associated with an unduly unfavorable first property; and introducing the substitution from the previous steps into a subtilisin to yield a multiply-substituted subtilisin variant. In some embodiments, the methods further comprise testing the multiply-substituted subtilisin variant in the first test and the second test, wherein an improved subtilisin variant achieves a value of greater than 1.0 in both of the first and second tests, or a value of greater than 1.0 in the first test and a value of 0.80 to 1.0 in the second test. In some embodiments, the methods further comprise producing the improved subtilisin variant(s). In some embodiments, the first and second properties are negatively correlated. In some embodiments, a favorable first or second property has a value greater than about 1.2. In some embodiments, an unduly unfavorable first or second property has a value less than about 0.40. In some embodiments, the first property is stability, and the second property is wash performance. In a subset of these the stability comprises stability in detergent and wash performance comprises blood milk ink (BMI) wash performance in detergent. In some embodiments, the parent bacterial subtilisin is a wild type mature form of a *B. amyloliquefaciens* subtilisin BPN' having an amino acid sequence set forth as SEQ ID NO:2. In other embodiments, the parent bacterial subtilisin is a wild type of a *B. lentus* GG36 subtilisin having an amino acid sequence set forth as SEQ ID NO:562, or a BPN" comprising a Y217L substitution (SEQ IDNO:565), alone or in combination with other modifications. In some embodiments of the present invention, wash performance is tested in a powder or liquid detergent composition having a pH of between 5 and 12.0. It is not intended that the steps be limited to the exact order listed above, as any suitable order finds use in the present invention. However in some preferred embodiments, the substitutions are in positions in a parent subtilisin having a solvent accessible surface (SAS) of greater than about 50% or greater than about 65%.

## BRIEF DESCRIPTION OF THE DRAWINGS

FIG. 1A provides a map of pHPLT-VAAc1, while FIG. 1B provides a map of pHPLT-BPN'.

FIG. 2A depicts BMI cleaning performance of a BPN'-Y217L CCL in North American laundry detergent as a function of charge change. Similarly FIG. 2B depicts BMI cleaning performance of a GG36 CCL in North American laundry detergent as a function of charge change.

FIG. 3A depicts BMI cleaning performance of a BPN'-Y217L CCL in Western European liquid laundry detergent as a function of charge change. Similarly FIG. 3B depicts BMI cleaning performance of a GG36 CCL in Western European liquid laundry detergent as a function of charge change.

FIG. 4A depicts BMI cleaning performance of a BPN'-Y217L CCL in Japanese powdered laundry detergent as a function of charge change. Similarly FIG. 4B depicts BMI cleaning performance of a GG36 CCL in Japanese powdered laundry detergent as a function of charge change.

FIG. 5A depicts baked egg yolk cleaning performance of a BPN'-Y217L CCL in automatic dish washing detergent as a function of charge change. Similarly FIG. 5B depicts baked egg yolk cleaning performance of a GG36 CCL in automatic dish washing detergent as a function of charge change.

FIG. 6 depicts LAS/EDTA stability as a function of net charge change relative to parent BPN'-Y217L, for a library containing 80 variants.

## DESCRIPTION OF THE INVENTION

The present invention provides methods for protein engineering. Specifically, the invention provides methods utilizing site evaluation libraries.

For practical purposes, it is not usually necessary to find the best sequence in a protein space in order to create a protein that is optimum for a particular application. For most applications, the problem to be solved is to identify at least one protein sequence that meets or exceeds the minimum value required for a number of properties. This requires knowledge of mutations that are good for a particular property, as well as knowledge of those mutations that are bad for any of the desired properties. The present invention provides means to meet the goal by identifying those positions in the protein that can be altered to improve the primary property and keep the values for other properties within desired limits.

The present invention provides means to evaluate all positions in a protein for all the properties of interest by building "site evaluation libraries" at each site. In preferred embodiments, these libraries contain 9-19 mutations at each position, and are used to evaluate each position for use in engineering the protein and constructing libraries. Each property is measured relative to the parent enzyme and an apparent free energy difference for each mutant vs. wild type is calculated. These delta delta G ("*i.e.,* $\Delta\Delta$G") apparent values are then used to determine additivity.

An ideal way to analyze variants would be through the difference in free energy for the variant versus the parent protein in the process of interest. The Gibbs Free Energy for a process represents the maximum amount of work that can be performed by a system. The change in Free energy relative to the parent enzyme ($\Delta\Delta$ G) is given as follows;

$$\Delta\Delta\ G = -RT \ln\ (k_{variant}/k_{parent})$$

where $k_{variant}$ is the rate constant for the variant enzyme, and $k_{parent}$ is the rate constant for the parent enzyme, R is the Gas law constant and T is the absolute temperature. Most assays are not constructed to allow determination of true Free Energies, so we utilized a quantity

$$\Delta\Delta\ G_{app} = -RT \ln\ (P_{variant}/P_{parent})$$

where $P_{variant}$ is the performance value for the variant and $P_{parent}$ is the performance value for the parent enzyme under the same conditions. The $\Delta\Delta\ G_{app}$ values may be expected to behave in a similar fashion as to $\Delta\Delta$ G for data distributions and additivity. However, since $\Delta\Delta$ G is the maximum amount of work that can be carried out by the variant compared to the parent enzyme, the quantity $\Delta\Delta\ G_{app}$ will generally underestimate the $\Delta\Delta$ G and lead to results that appear synergistic in that the properties of two additive positions may be greater than the value predicted by adding their $\Delta\Delta\ G_{app}$ values together.

The methods of the present invention used to design efficient libraries that were used to engineer multiple properties in parallel. Although certain enzymes are described herein, the methods apply to any protein of interest for engineering. Site evaluation libraries (SELs) were built as described herein by introducing from 12 to 19 substitutions at each of the positions. The resulting mutations were analyzed using activity and stability assays. The wild type amino acid is listed as a reference point for every position. For each property, measurements were used to determine the mean and standard deviation of $\Delta\Delta\ G_{app}$ for the parent enzyme. The parent mean ($\mu_{parent}$) was normalized to 0, and the standard deviation ($\sigma_{parent}$) for $\Delta\Delta\ G_{app}$ was determined. These values were used as the reference for each property at each position of the molecule. The site evaluation data were tested for evidence of correlation between properties. The $\Delta\Delta\ G_{app}$ values for each property were plotted versus each other property and correlation coefficients were calculated. The two activity measurements on protein substrates were correlated.

In order to analyze the positions within the amino acid sequence, two types of sites were defined. "Unproductive" sites have no mutant that is better than the parent enzyme, while "Productive" sites have at least one substitution that is better than the parent enzyme. The probability that a site will be Productive is given by the number of Productive sites divided by the total number of sites. Although the probability that any mutation will be better than the parent enzyme is low (i.e., 6%-28%) the probability that a given site will have at least one Up mutation is quite high.

It was of interest to determine how the Productive and Unproductive sites were distributed with respect to structural features (e.g., buried amino acids, interacting amino acids, positions near the active site, etc.) in the protease, as well as sequence sites that are conserved or changeable in evolution. To make this determination, the structure is examined and the sequence aligned with non-redundant homologs.

As indicated in the Examples, deleterious mutations for any property are correlated with deleterious mutations for every other property, regardless of correlations of the properties. Only a small number of positions (5-10%) have mutations that are bad for all properties. These positions define the "fold" and are conserved in evolution. The implication of this is that although identification of beneficial mutations for any property requires a truly predictive screen for that property, identification of mutations likely to be deleterious for any property can be accomplished using ANY screen. A simplified protein engineering strategy is to build SELs and screen using a simple activity and/or stability screen. The deleterious mutations are identified and those positions that have few deleterious mutations are used to build libraries and combinatorial mutations to improve multiple properties. Also, picking sites that are on the surface of the protein, have few interactions and are variable in sequence alignments provides a high proportion of productive sites. Sites that are on the interior of the molecule, have many contacts and are strongly conserved in evolution will have a high probability of having deleterious mutations and should be avoided. It is contemplated that any suitable method for analyzing sequence and/or structural information will find use in the present invention, including but not limited to computer and/or electronic methods and/or programs.

The methods provide pairwise comparisons of the numbers of variants with more than 5% wt activity and less than 5% activity for each of two properties, along with correlation coefficients for the two properties.

**Library Design**

In some particularly preferred embodiments, the site evaluation library data are used for combinatorial library design. Traditional directed evolution builds random libraries and screens large numbers of library for single properties, combines these and repeats the process. As several investigators have found *(See e.g.,* Bloom et al., Curr. Opin. Struct. Biol., 15:447-452 [2005]; Bloom et al., Proc. Natl. Acad. Sci. USA 103:5869-5874 [2006]; and Guo et al., Proc. Natl. Acad. Sci. USA 101:9205-9210 [2004]), the accumulation of positive mutations for one property commonly leads to decreases in other properties. The probability that any mutation will be Up for any property is small, and the probability that any mutation will be Down is high (> 85%), and the probability that accumulating more than three (3) mutations that increase activity will result in a decrease in several other properties is quite high. This problem is avoided by using the site evaluation data to build libraries that would be good for multiple properties. Unproductive sites were not included in combinatorial libraries, and productive sites were further classified by the percentage of mutations that were up.

**Cleaning Compositions**

The cleaning composition of the present invention are advantageously employed for example, in laundry applications, hard surface cleaning, automatic dishwashing applications, as well as cosmetic applications such as dentures, teeth, hair and skin. However, due to the unique advantages of increased effectiveness in lower temperature solutions, the enzymes of the present invention are ideally suited for laundry applications. Furthermore, the enzymes of the present invention may be employed in both granular and liquid compositions.

The variant proteases of the present invention also find use cleaning additive products. In some embodiments, low temperature solution cleaning applications find use. The additive product may be, in its simplest form, one or more proteases. In some embodiments, the additive is packaged in dosage form for addition to a cleaning process. Any suitable single dosage form also finds use with the present invention, including but not limited to pills, tablets, gelcaps, or other single dosage units such as premeasured powders or liquids. In some embodiments, filler(s) or carrier material (s) are included to increase the volume of such composition. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials. Acidic fillers find use to reduce pH. Alternatively, the cleaning additives include adjunct ingredients as more fully described below.

The present cleaning compositions and cleaning additives require an effective amount of at least one of the protease variants provided herein, alone or in combination with other proteases and/or additional enzymes. The required level of enzyme is achieved by the addition of one or more protease variants of the present invention. Typically the present cleaning compositions will comprise at least about 0.0001 weight percent, from about 0.0001 to about 1, from about 0.001 to about 0.5, or even from about 0.01 to about 0.1 weight percent of at least one of the variant proteases of the present invention.

The cleaning compositions herein are typically formulated such that, during use in aqueous cleaning operations, the wash water will have a pH of from about 5.0 to about 11.5 or even from about 7.5 to about 10.5. Liquid product formulations are typically formulated to have a neat pH from about 3.0 to about 9.0 or even from about 3 to about 5. Granular laundry products are typically formulated to have a pH from about 9 to about 11. Techniques for controlling pH at recommended usage levels include the use of buffers, alkalis, acids, etc., and are well known to those skilled in the art.

Suitable low pH cleaning compositions typically have a neat pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine or hydrochloric acid, to provide such cleaning composition with a neat pH of from about 3 to about 5. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C.

In some embodiments, when the variant protease(s) is/are employed in a granular composition or liquid, it is desirable for the variant protease to be in the form of an encapsulated particle to protect the variant protease from other components of the granular composition during storage. In addition, encapsulation is also a means of controlling the availability of the variant protease during the cleaning process. In some embodiments, encapsulation enhances the performance of the variant protease(s) and/or additional enzymes. In this regard, the variant proteases of the present invention are encapsulated with any suitable encapsulating material known in the art. In some embodiments, the encapsulating material typically encapsulates at least part of the catalyst for the variant protease(s) of the present invention. Typically, the encapsulating material is water-soluble and/or water-dispersible. In some embodiments, the encapsulating material has a glass transition temperature (Tg) of 0°C or higher. Glass transition temperature is described in more detail in WO 97/11151. The encapsulating material is selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. Typically, the encapsulating material is a starch. Suitable starches are described in EP 0 922 499; US 4,977,252; US 5,354,559, and US 5,935,826. In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

As described herein, the variant proteases of the present invention find particular use in the cleaning industry, including, but not limited to laundry and dish detergents. These applications place enzymes under various environmental stresses. The variant proteases of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, a European detergent typically has about 4500-5000 ppm of detergent components in the wash water, while a Japanese detergent typically has approximately 667 ppm of detergent components in the wash water. In North America, particularly the United States, detergents typically have about 975 ppm of detergent components present in the wash water.

A low detergent concentration system includes detergents where less than about 800 ppm of detergent components are present in the wash water. Japanese detergents are typically considered low detergent concentration system as they have approximately 667 ppm of detergent components present in the wash water.

A medium detergent concentration includes detergents where between about 800 ppm and about 2000ppm of detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies" ), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between 10 and 30°C (e.g., about 20°C), whereas the temperature of wash water in Europe is typically between 30 and 60°C (e.g., about 40°C).

As a further example, different geographies typically have different water hardness. Water hardness is usually described in terms of the grains per gallon mixed $Ca^{2+}/Mg^{2+}$. Hardness is a measure of the amount of calcium ($Ca^{2+}$) and magnesium ($Mg^{2+}$) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| Water | Grains per gallon | Parts per million |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than 10.5 (for example 10.5-20.0) grains per gallon mixed $Ca^{2+}/Mg^{2+}$ (e.g., about 15 grains per gallon mixed $Ca^{2+}/Mg^{2+}$). North American water hardness is typically greater than Japanese water hardness, but less than European water hardness. For example, North American water hardness can be between 3 to 10 grains, 3-8 grains or about 6 grains. Japanese water hardness is typically lower than North American water hardness, usually less than 4, for example 3 grains per gallon mixed $Ca^{2+}/Mg^{2+}$.

Accordingly, in some embodiments, the present invention provides variant proteases that show surprising wash performance in at least one set of wash conditions (e.g., water temperature, water hardness, and/or detergent concentration). In some embodiments, the variant proteases of the present invention are comparable in wash performance to other subtilisin proteases. In some embodiments, the variant proteases of the present invention exhibit enhanced wash performance as compared to subtilisin proteases currently commercially available. Thus, in some preferred embodiments of the present invention, the variant proteases provided herein exhibit enhanced oxidative stability, enhanced thermal stability, and/or enhanced chelator stability. In addition, the variant proteases of the present invention find use in cleaning compositions that do not include detergents, again either alone or in combination with builders and stabilizers.

In some embodiments of the present invention, the cleaning compositions comprise at least one variant protease of the present invention at a level from about 0.00001 % to about 10% by weight of the composition and the balance (e.g., about 99.999% to about 90.0%) comprising cleaning adjunct materials by weight of composition. In other aspects of the present invention, the cleaning compositions of the present invention comprises at least one variant protease at a level of about 0.0001 % to about 10%, about 0.001 % to about 5%, about 0.001 % to about 2%, about 0.005% to about 0.5% by weight of the composition and the balance of the cleaning composition (e.g., about 99.9999% to about 90.0%, about 99.999 % to about 98%, about 99.995% to about 99.5% by weight) comprising cleaning adjunct materials.

In some embodiments, preferred cleaning compositions comprise one or more additional enzymes or enzyme derivatives which provide cleaning performance and/or fabric care benefits, in addition to one or more of the variant proteases provided herein. Such enzymes include, but are not limited to other proteases, lipases, cutinases, amylases, cellulases, peroxidases, oxidases (e.g. laccases), and/or mannanases.

Any other suitable protease finds use in the compositions of the present invention. Suitable proteases include those of animal, vegetable or microbial origin. In some particularly preferred embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus (e.g.,* subtilisin, *lentus, amyloliquefaciens,* subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628, all of which are incorporated herein by reference. Additional protease examples include, but are not limited to trypsin *(e.g.,* of porcine or bovine origin), and the *Fusarium* protease described in WO 89/06270. Preferred commercially available protease enzymes include MAXATASE®, MAXACAL™, MAXAPEM™, OPTICLEAN®, OPTIMASE®, PROPERASE®, PURAFECT® and PURAFECT® OXP (Genencor); ALCALASE®, SAVINASE®, PRIMASE®, DURAZYM™, RELASE® and ESPERASE® (Novozymes); and BLAP™ (Henkel Kommanditgesellschaft auf Aktien, Duesseldorf, Germany. Various proteases are described in WO95/23221, WO 92/21760, and U.S. Pat. Nos. 5,801,039, 5,340,735, 5,500,364, 5,855,625, US RE 34,606, 5,955,340, 5,700,676, 6,312,936, and 6,482,628, and various other patents.

In addition, any suitable lipase finds use in the present invention. Suitable lipases include, but are not limited to those of bacterial or fungal origin. Chemically or genetically modified mutants are encompassed by the present invention. Examples of useful lipases include *Humicola lanuginosa* lipase *(See e.g.,* EP 258 068, and EP 305 216), *Rhizomucor miehei* lipase *(See e.g.,* EP 238 023), *Candida* lipase, such as C. *antarctica* lipase *(e.g.,* the C. *antarctica* lipase A or B; *See e.g.,* EP 214 761), a *Pseudomonas* lipase such as *P. alcaligenes* and *P. pseudoalcaligenes* lipase *(See e.g.,* EP 218 272), *P. cepacia* lipase *(See e.g.,* EP 331 376), *P. stutzeri* lipase *(See e.g.,* GB 1,372,034), *P. fluorescens* lipase, *Bacillus* lipase *(e.g., B. subtilis* lipase [Dartois et al., Biochem. Biophys. Acta 1131:253-260 [1993]); *B. stearothermophilus* lipase [*See e.g.,* JP 64/744992]; and *B. pumilus* lipase [*See e.g.,* WO 91/16422]).

Furthermore, a number of cloned lipases find use in some embodiments of the present invention, including but not limited to *Penicillium camembertii* lipase *(See,* Yamaguchi et al., Gene 103:61-67 [1991*]), Geotricum candidum* lipase *(See,* Schimada et al., J. Biochem., 106:383-388 [1989]), and various *Rhizopus* lipases such as *R. delemar* lipase *(See,* Hass et al., Gene 109:117-113 [1991]), a *R. niveus* lipase (Kugimiya et al., Biosci. Biotech. Biochem. 56:716-719 [1992]) and *R. oryzae* lipase.

Other types of lipolytic enzymes such as cutinases also find use in some embodiments of the present invention, including but not limited to the cutinase derived from *Pseudomonas mendocina (See,* WO 88/09367), and the cutinase derived from *Fusarium solani pisi (See,* WO 90/09446).

Additional suitable lipases include commercially available lipases such as M1 LIPASE™, LUMA FAST™, and LIPOMAX™ (Genencor); LIPOLASE® and LIPOLASE® ULTRA (Novozymes); and LIPASE P™ "Amano" (Amano Pharmaceutical Co. Ltd., Japan).

In some embodiments of the present invention, the cleaning compositions of the present invention further comprise lipases at a level from about 0.00001 % to about 10% of additional lipase by weight of the composition and the balance of cleaning adjunct materials by weight of composition. In other aspects of the present invention, the cleaning compositions of the present invention also comprise, lipases at a level of about 0.0001 % to about 10%, about 0.001% to about 5%, about 0.001 % to about 2%, about 0.005% to about 0.5% lipase by weight of the composition.

Any amylase (alpha and/or beta) suitable for use in alkaline solutions also find use in some embodiments of the present invention. Suitable amylases include, but are not limited to those of bacterial or fungal origin. Chemically or genetically modified mutants are included in some embodiments. Amylases that find use in the present invention, include, but are not limited to $\alpha$-amylases obtained from *B. licheniformis (See e.g.,* GB 1,296,839). Commercially available amylases that find use in the present invention include, but are not limited to DURAMYL®, TERMAMYL®, FCTNGAMYL® and BAN™ (Novozymes) and RAPIDASE® and MAXAMYL® P (Genencor).

In some embodiments of the present invention, the cleaning compositions of the present invention further comprise amylases at a level from about 0.00001 % to about 10% of additional amylase by weight of the composition and the balance of cleaning adjunct materials by weight of composition. In other aspects of the present invention, the cleaning compositions of the present invention also comprise, amylases at a level of about 0.0001 % to about 10%, about 0.001% to about 5%, about 0.001 % to about 2%, about 0.005% to about 0.5% amylase by weight of the composition.

In some further embodiments, any suitable cellulase finds used in the cleaning compositions of the present invention. Suitable cellulases include, but are not limited to those of bacterial or fungal origin. Chemically or genetically modified mutants are included in some embodiments. Suitable cellulases include, but are not limited to *Humicola insolens* cellulases *(See e.g.,* U.S. Pat. No. 4,435,307). Especially suitable cellulases are the cellulases having color care benefits *(See e.g.,* EP 0 495 257). Commercially available cellulases that find use in the present include, but are not limited to CELLUZYME® (Novozymes), and KAC-500(B)™ (Kao Corporation). In some embodiments, cellulases are incorporated as portions or fragments of mature wild-type or variant cellulases, wherein a portion of the N-terminus is deleted (*See e.g.,* U.S. Pat. No. 5,874,276). In some embodiments, the cleaning compositions of the present invention further comprise cellulases at a level from about 0.00001 % to about 10% of additional cellulase by weight of the composition and the balance of cleaning adjunct materials by weight of composition. In other aspects of the present invention, the cleaning compositions of the present invention also comprise cellulases at a level of about 0.0001% to about 10%, about 0.001% to about 5%, about 0.001% to about 2%, about 0.005% to about 0.5% cellulase by weight of the composition.

Any mannanase suitable for use in detergent compositions also finds use in the present invention. Suitable mannanases include, but are not limited to those of bacterial or fungal origin. Chemically or genetically modified mutants are included in some embodiments. Various mannanases are known which find use in the present invention *(See e.g.,* U.S. Pat. No. 6,566,114, U.S. Pat. No.6,602,842, and US Patent No. 6,440,991, all of which are incorporated herein by reference). In some embodiments, the cleaning compositions of the present invention further comprise mannanases at a level from about 0.00001 % to about 10% of additional mannanase by weight of the composition and the balance of cleaning adjunct materials by weight of composition. In other aspects of the present invention, the cleaning compositions of the present invention also comprise, mannanases at a level of about 0.0001 % to about 10%, about 0.001% to about 5%, about 0.001% to about 2%, about 0.005% to about 0.5% mannanase by weight of the composition.

In some embodiments, peroxidases are used in combination with hydrogen peroxide or a source thereof (e.g., a percarbonate, perborate or persulfate) in the compositions of the present invention. In some alternative embodiments, oxidases are used in combination with oxygen. Both types of enzymes are used for "solution bleaching" *(i.e.,* to prevent transfer of a textile dye from a dyed fabric to another fabric when the fabrics are washed together in a wash liquor), preferably together with an enhancing agent (*See e.g.,* WO 94/12621 and WO 95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001% to about 10% of additional peroxidase and/or oxidase by weight of the composition and the balance of cleaning adjunct materials by weight of composition. In other aspects of the present invention, the cleaning compositions of the present invention also comprise, peroxidase and/or oxidase enzymes at a level of about 0.0001% to about 10%, about 0.001% to about 5%, about 0.001% to about 2%, about 0.005% to about 0.5% peroxidase and/or oxidase enzymes by weight of the composition.

In some embodiments, additional enzymes find use, including but not limited to perhydrolases *(See e.g.,* WO 05/056782). In addition, in some particularly preferred embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the variant protease(s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (e.g., through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents *(See e.g.,* U.S. Pat. Nos. 6,610,642, 6,605,458, 5,705,464, 5,710,115, 5,698,504, 5,695,679, 5,686,014 and 5,646,101, all of which are incorporated herein by reference). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated *(i.e.,* not in contact with each other) until combination of the two components is appropriate are used. Such separation methods include any suitable method known in the art (e.g., gelcaps, encapulation, tablets, physical separation, etc.).

In some preferred embodiments, an effective amount of one or more variant protease(s) provided herein are included in compositions useful for cleaning a variety of surfaces in need of proteinaceous stain removal. Such cleaning compositions include cleaning compositions for such applications as cleaning hard surfaces, fabrics, and dishes. Indeed, in some embodiments, the present invention provides fabric cleaning compositions, while in other embodiments, the present invention provides non-fabric cleaning compositions. Notably, the present invention also provides cleaning compositions suitable for personal care, including oral care (including dentrifices, toothpastes, mouthwashes, etc., as well as denture cleaning compositions), skin, and hair cleaning compositions. It is intended that the present invention encompass detergent compositions in any form *(i.e.,* liquid, granular, bar, semi-solid, gels, emulsions, tablets, capsules, etc.).

By way of example, several cleaning compositions wherein the variant proteases of the present invention find use are described in greater detail below. In embodiments in which the cleaning compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents *(i.e.,* as additional cleaning adjunct materials). The compositions of the present invention also find use detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments formulated as compositions for use in manual dishwashing methods, the compositions of the invention preferably contain at least one surfactant and preferably at least one additional cleaning adjunct material selected from organic polymeric compounds, suds enhancing agents, group II metal ions, solvents, hydrotropes and additional enzymes.

In some embodiments, various cleaning compositions such as those provided in U.S, Pat. No. 6,605,458 find use with the variant proteases of the present invention. Thus, in some embodiments, the compositions comprising at least one variant protease of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one variant protease of the present invention are fabric cleaning compositions such as those described in U.S. Pat. Nos. 6,610,642 and 6,376,450. In addition, the variant proteases of the present invention find use in granular laundry detergent compositions of particular utility under European or Japanese washing conditions *(See e.g.,* U.S. Pat. No. 6,610,642).

In some alternative embodiments, the present invention provides hard surface cleaning compositions comprising at least one variant protease provided herein. Thus, in some embodiments, the compositions comprising at least one variant protease of the present invention is a hard surface cleaning composition such as those described in U.S. Pat. Nos. 6,610,642, 6,376,450, and 6,376,450.

In yet further embodiments, the present invention provides dishwashing compositions comprising at least one variant protease provided herein. Thus, in some embodiments, the compositions comprising at least one variant protease of the present invention is a hard surface cleaning composition such as those in U.S. Pat. Nos. 6,610,642 and 6,376,450. In still further embodiments, the present invention provides dishwashing compositions comprising at least one variant protease provided herein. In some further embodiments, the compositions comprising at least one variant protease of the present invention comprise oral care compositions such as those in U.S. Pat. No. 6,376,450, and 6,376,450. The formulations and descriptions of the compounds and cleaning adjunct materials contained in the aforementioned US Pat. Nos. 6,376,450, 6,605,458, 6,605,458, and 6,610,642, find use with the variant proteases provided herein.

The cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,565,422, 5,516,448, 5,489,392, and 5,486,303, all of which are incorporated herein by reference. When a low pH cleaning composition is desired, the pH of such composition is adjusted via the addition of a material such as monoethanolamine or an acidic material such as HCl.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348, that are incorporated by reference. The aforementioned adjunct ingredients may constitute the balance of the cleaning compositions of the present invention.

In some embodiments, the cleaning compositions according to the present invention comprise a surfactant or surfactant system wherein the surfactant is selected from nonionic surfactants, anionic surfactants, cationic surfactants, ampholytic surfactants, zwitterionic surfactants, semi-polar nonionic surfactants and mixtures thereof.

In some additional embodiments, the cleaning compositions of the present invention comprise one or more detergent builders or builder systems. Builders include, but are not limited to, the alkali metal, ammonium and alkanolammonium salts of polyphosphates, alkali metal silicates, alkaline earth and alkali metal carbonates, aluminosilicate builders polycarboxylate compounds. ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof.

In some further embodiments, the cleaning compositions herein contain a chelating agent. Suitable chelating agents include copper, iron and/or manganese chelating agents and mixtures thereof.

In some still further embodiments, the cleaning compositions provided herein contain a deposition aid. Suitable deposition aids include, polyethylene glycol, polypropylene glycol, polycarboxylate, soil release polymers such as polytelephthalic acid, clays such as Kaolinite, montmorillonite, atapulgite, illite, bentonite, halloysite, and mixtures thereof.

In some additional embodiments, the cleaning compositions of the present invention also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof.

In some still additional embodiments, the cleaning compositions of the present invention also contain dispersants. Suitable water-soluble organic materials include the homo- or co-polymeric acids or their salts, in which the polycarboxylic acid comprises at least two carboxyl radicals separated from each other by not more than two carbon atoms.

In some particularly preferred embodiments, the cleaning compositions comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratinases, reductases, oxidases, phenol oxidases, lipoxygenases, ligninases, pullulanases, tannases, pentosanases, malanases, ß-glucanases, arabinosidases, hyaluronidase, chondroitinase, laccase, and amylases, or mixtures thereof. A typical combination is cocktail of conventional applicable enzymes including at least one protease, at least one lipase, at least one cutinase, and/or at least one cellulase in conjunction with at least one amylase.

In some further embodiments, the enzymes used in the cleaning compositions are stabilized any suitable technique. In some embodiments, the enzymes employed herein are stabilized by the presence of water-soluble sources of calcium and/or magnesium ions in the finished compositions that provide such ions to the enzymes.

In some still further embodiments, the cleaning compositions of the present invention include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methylenephosphonic acid) and water-soluble salts thereof. Such catalysts are disclosed in U.S. Pat. No. 4,430,243.

In some embodiments, the compositions herein are catalyzed by means of a manganese compound. Such compounds and levels of use are well known in the art and include, for example, the manganese-based catalysts disclosed in U.S. Pat. No. 5,576,282. In addition, cobalt bleach catalysts useful herein are known, and are described, for example, in U.S. Pat. Nos. 5,597,936, and 5,595,967. Such cobalt catalysts are readily prepared by known procedures, such as taught for example in U.S. Pat. Nos. 5,597,936, and 5,595,967. In some embodiments, the compositions provided herein also suitably include a transition metal complex of a macropolycyclic rigid ligand *(i.e.,* "MRL"). As a practical matter, and not by way of limitation, the compositions and cleaning processes herein are adjustable, to provide on the order of at least one part per hundred million of the active MRL species in the aqueous washing medium, and will preferably provide from about 0.005 ppm to about 25 ppm, more preferably from about 0.05 ppm to about 10 ppm, and most preferably from about 0.1 ppm to about 5 ppm, of the MRL in the wash liquor. Preferred transition-metals in the instant transition-metal bleach catalyst include manganese, iron and chromium. Preferred MRLs herein are a special type of ultra-rigid ligand that is cross-bridged such as 5,12-diethyl-1,5,8,12-tetraazabicyclo[6.6.2]hexadecane. Suitable transition metal MRLs are readily prepared by known procedures, such as taught for example in WO 00/332601, and U.S. Pat. No. 6,225,464.

As indicated above, the cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,516,448, 5,489,392, and 5,486,303, all of which are incorporated herein by reference.

The cleaning compositions disclosed herein of find use in cleaning a situs (e.g., a surface, dishware, or fabric). Typically, at least a portion of the situs is contacted with an embodiment of the present cleaning composition, in neat form or diluted in a wash liquor, and then the situs is optionally washed and/or rinsed. For purposes of the present invention, "washing" includes but is not limited to, scrubbing, and mechanical agitation. In some embodiments, the cleaning compositions are typically employed at concentrations of from about 500 ppm to about 15,000 ppm in solution. When the wash solvent is water, the water temperature typically ranges from about 5°C to about 90°C and, when the situs comprises a fabric, the water to fabric mass ratio is typically from about 1:1 to about 30:1.

In additional embodiments, the present invention also provides compositions and methods utilizing various surfactants and surfactant blends to assess and improve the performance of proteases in detergent compositions. Using experimental design software, a simplex lattice mixture experiment was designed to evaluate the activity of a protease in the presence of the most commonly used surfactants in liquid laundry detergents; namely linear alkylbenzene sulfonate (LAS), alkylethoxy sulfate (AES), and alcohol ethoxylate (AE). LAS and AES are anionic surfactants, while AE is a nonionic surfactant.

Surprisingly, it was determined that selecting a surfactant composition for optimum performance of a protease was not simply a matter of selecting the surfactant in which the protease was most stable. Rather, selecting an optimum surfactant composition was based on combining different surfactants in a ratio that provided the best overall cleaning results, wherein the mixture included surfactants that if used in the absence of other surfactants, would destabilize the protease.

For example, it was determined that in some embodiments, a protease is able to function better in the presence of a first surfactant when a second surfactant is present but less well if a third surfactant is present. In additional embodiments, a protease tolerates much higher levels of a first surfactant, depending on the ratio of a second and third surfactant. The observation that enzymes can be used in the presence of different surfactants if the ratios of the surfactants are carefully selected is contrary to the conventional belief that enzymes must be used only in combination with surfactants that do not adversely affect their activity, limiting the use of enzymes to certain detergent compositions.

Based, in part, on the observations described herein, the present compositions and methods provide detergent composition comprising at least one protease and a mixture of surfactants in a ratio preselected to promote the activity of the protease, wherein the surfactants are not selected simply by measuring the stability of the protease in each surfactant. In some cases, the protease is inactivated or exhibits reduced activity in the presence of a different ratio of the same surfactants, or in the presence of any subset of the surfactants but in the absence of at least one of the surfactants in the mixture. In some cases, the protease is inactivated or exhibits reduced activity in the presence of any one of the surfactants, in the absence of other surfactant present in the mixture. Similarly, in some cases, the presence of a first surfactant allows the use of an increased amount of a second surfactant, wherein the same amount of the second surfactant in the absence of the first surfactant would inactivate or reduce the activity of the protease.

In addition, the presence of a first type of surfactant (e.g., a non-ionic or anionic detergent) permits the use of a second type of detergent, where the second type of detergent in the absence of the first type of surfactant would inactivate or reduce the activity of the protease.

In these experiments, *Bacillus amyloliquefaciens* subtilisin BPN'-Y217L ("FNA"; Genencor) was used, as well as variants of this enzyme. At low temperatures (i.e., 16°C) the protease was active in a composition comprising a relatively small amount of AE and a larger amount of either AES or LAS, or both. In comparison, the protease was less active in a composition comprising only AES, only LAS, and particularly, only AE. Therefore, the preferred composition was not one that included only the single surfactant in which the protease was most active, but rather one that included at least two, and preferably all three surfactants, any one of which would inactivate or reduce the activity of the protease if used individually at a sufficient level. The particular ratio of AES>LAS>AE appeared to produce the best result for this protease at the low temperature.

In contrast, at high temperatures (i.e., 32°C) the protease was active in a composition comprising only AES, or AES in combination with LAS and a relatively small amount of AE. Thus, the tolerance for AES increased at the higher temperature, making the inclusion of the other surfactants less important.

Several additional tests were also conducted. The protease activity after incubation was determined using AAPF-pNA substrate. The melting point, Tm, was measured of the enzyme in each detergent formulation. Finally, four formulations were selected and tested in the Terg-O-Tometer for correlation to the 96-well assay results *(See,* Example 22).

Definitions

Unless otherwise indicated, the practice of the present invention involves conventional techniques commonly used in molecular biology, protein engineering, microbiology, and recombinant DNA, which are within the skill of the art. Such techniques are known to those of skill in the art and are described in numerous texts and reference works (*See e.g.,* Sambrook et al., "Molecular Cloning: A Laboratory Manual", Second Edition (Cold Spring Harbor), [1989]); and Ausubel et al., "Current Protocols in Molecular Biology" [1987]). All patents, patent applications, articles and publications mentioned herein, both *supra* and *infra,* are hereby expressly incorporated herein by reference.

Unless defined otherwise herein, all technical and scientific terms used herein have the same meaning as commonly understood by one of ordinary skill in the art to which this invention pertains. There are various dictionaries available and known to those in the art that provide definitions of these terms. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular "a", "an" and "the" includes the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

The practice of the present invention employs, unless otherwise indicated, conventional techniques of protein purification, molecular biology, microbiology, recombinant DNA techniques and protein sequencing, all of which are within the skill of those in the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, a number of terms are defined below.

As used herein, the terms "protease," and "proteolytic activity" refer to a protein or peptide exhibiting the ability to hydrolyze peptides or substrates having peptide linkages. Many well known procedures exist for measuring proteolytic activity (Kalisz, "Microbial Proteinases," In: Fiechter (ed.), Advances in Biochemical Engineering/Biotechnology, [1988]). For example, proteolytic activity may be ascertained by comparative assays which analyze the respective protease's ability to hydrolyze a commercial substrate. Exemplary substrates useful in the analysis of protease or proteolytic activity, include, but are not limited to di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (*See e.g.,* WO 99/34011; and U.S. Pat. No. 6,376,450, both of which are incorporated herein by reference). The pNA assay *(See e.g.,* Del Mar et al., Anal. Biochem., 99:316-320 [1979]) also finds use in determining the active enzyme concentration for fractions collected during gradient elution. This assay measures the rate at which *p*-nitroaniline is released as the enzyme hydrolyzes the soluble synthetic substrate, succinyl-alanine-alanine-proline-phenylalanine-*p*-nitroanilide (sAAPF-*p*NA). The rate of production of yellow color from the hydrolysis reaction is measured at 410 nm on a spectrophotometer and is proportional to the active enzyme concentration. In addition, absorbance measurements at 280 nm can be used to determine the total protein concentration. The active enzyme/total-protein ratio gives the enzyme purity.

As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named "*Geobacillus stearothermophilus*." The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid", used interchangeably herein, refer to a polymeric form of nucleotides of any length, either ribonucleotides or deoxyribonucleotides. These terms include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, ESTs, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In some embodiments, polynucleotides comprise modified nucleotides, such as methylated nucleotides and nucleotide analogs, uracyl, other sugars and linking groups such as fluororibose and thioate, and nucleotide branches. In alternative embodiments, the sequence of nucleotides is interrupted by non-nucleotide components.

As used herein, the terms "DNA construct" and "transforming DNA" are used interchangeably to refer to DNA used to introduce sequences into a host cell or organism. The DNA may be generated *in vitro* by PCR or any other suitable technique(s) known to those in the art. In particularly preferred embodiments, the DNA construct comprises a sequence of interest (e.g., as an incoming sequence). In some embodiments, the sequence is operably linked to additional elements such as control elements (e.g., promoters, etc.). The DNA construct may further comprise a selectable marker. It may further comprise an incoming sequence flanked by homology boxes. In a further embodiment, the transforming DNA comprises other non-homologous sequences, added to the ends (e.g., stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell, and/or 2) mutagenize a region of the host cell chromosome (i.e., replace an endogenous sequence with a heterologous sequence), 3) delete target genes, and/or 4) introduce a replicating plasmid into the host.

As used herein, the terms "expression cassette" and "expression vector" refer to nucleic acid constructs generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In preferred embodiments, expression vectors have the ability to incorporate and express heterologous DNA fragments in a host cell. Many prokaryotic and eukaryotic expression vectors are commercially available. Selection of appropriate expression vectors is within the knowledge of those of skill in the art. The term "expression cassette" is used interchangeably herein with "DNA construct," and their grammatical equivalents. Selection of appropriate expression vectors is within the knowledge of those of skill in the art.

As used herein, the term "vector" refers to a polynucleotide construct designed to introduce nucleic acids into one or more cell types. Vectors include cloning vectors, expression vectors, shuttle vectors, plasmids, cassettes and the like. In some embodiments, the polynucleotide construct comprises a DNA sequence encoding the protease (e.g., precursor or mature protease) that is operably linked to a suitable prosequence (e.g., secretory, etc.) capable of effecting the expression of the DNA in a suitable host.

As used herein, the term "plasmid" refers to a circular double-stranded (ds) DNA construct used as a cloning vector, and which forms an extrachromosomal self-replicating genetic element in some eukaryotes or prokaryotes, or integrates into the host chromosome.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, conjugation, and transduction *(See e.g.,* Ferrari et al., "Genetics," in Hardwood et al, (eds.), Bacillus, Plenum Publishing Corp., pages 57-72, [1989]).

As used herein, the terms "transformed" and "stably transformed" refers to a cell that has a non-native (heterologous) polynucleotide sequence integrated into its genome or as an episomal plasmid that is maintained for at least two generations.

A nucleic acid is "operably linked" when it is placed into a functional relationship with another nucleic acid sequence. For example, DNA encoding a secretory leader *(i.e.,* a signal peptide), is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein the term "gene" refers to a polynucleotide (e.g., a DNA segment), that encodes a polypeptide and includes regions preceding and following the coding regions as well as intervening sequences (introns) between individual coding segments (exons).

As used herein, "homologous genes" refers to a pair of genes from different, but usually related species, which correspond to each other and which are identical or very similar to each other. The term encompasses genes that are separated by speciation *(i.e.,* the development of new species) *(e.g.,* orthologous genes), as well as genes that have been separated by genetic duplication (e.g., paralogous genes).

As used herein, proteins are defined as having a common "fold" if they have the same major secondary structures in the same arrangement and with the same topological connections. Different proteins with the same fold often have peripheral elements of secondary structure and turn regions that differ in size and conformation. In some cases, these differing peripheral regions may comprise half the structure. Proteins placed together in the same fold category do not necessarily have a common evolutionary origin (e.g., structural similarities arising from the physics and chemistry of proteins favoring certain packing arrangements and chain topologies).

As used herein, "homology" refers to sequence similarity or identity, with identity being preferred. This homology is determined using standard techniques known in the art *(See e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

As used herein, an "analogous sequence" is one wherein the function of the gene is essentially the same as the gene based on the BPN' protease. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps.

Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). A particularly useful BLAST program is the WU-BLAST-2 program *(See,* Altschul et al., Meth. Enzymol., 266:460-480 [1996]). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical with the nucleotide residues of the starting sequence *(i.e.,* the sequence of interest). A preferred method utilizes the BLASTN module of WU-BLAST-2 set to the default parameters, with overlap span and overlap fraction set to 1 and 0.125, respectively.

As used herein, "recombinant" includes reference to a cell or vector, that has been modified by the introduction of a heterologous nucleic acid sequence or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention. "Recombination," "recombining," and generating a "recombined" nucleic acid are generally the assembly of two or more nucleic acid fragments wherein the assembly gives rise to a chimeric gene.

In some preferred embodiments, mutant DNA sequences are generated with site saturation mutagenesis in at least one codon. In another preferred embodiment, site saturation mutagenesis is performed for two or more codons. In a further embodiment, mutant DNA sequences have more than about 50%, more than about 55%, more than about 60%, more than about 65%, more than about 70%, more than about 75%, more than about 80%, more than about 85%, more than about 90%, more than about 95%, or more than about 98% homology with the wild-type sequence. In alternative embodiments, mutant DNA is generated *in vivo* using any known mutagenic procedure such as, for example, radiation, nitrosoguanidine and the like. The desired DNA sequence is then isolated and used in the methods provided herein.

As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (e.g., an inhibitor of an inhibitable enzyme) results in the amplification of either the endogenous gene encoding the gene product required for growth in the presence of the drug or by amplification of exogenous (i.e., input) sequences encoding this gene product, or both.

"Amplification" is a special case of nucleic acid replication involving template specificity. It is to be contrasted with non-specific template replication (i.e., replication that is template-dependent but not dependent on a specific template). Template specificity is here distinguished from fidelity of replication (i.e., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, *(i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195 4,683,202, and 4,965,188, hereby incorporated by reference, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times *(i.e.*, denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

As used herein, the term "RT-PCR" refers to the replication and amplification of RNA sequences. In this method, reverse transcription is coupled to PCR, most often using a one enzyme procedure in which a thermostable polymerase is employed, as described in U.S. Patent No. 5,322,770, herein incorporated by reference. In RT-PCR, the RNA template is converted to cDNA due to the reverse transcriptase activity of the polymerase, and then amplified using the polymerizing activity of the polymerase (*i.e.*, as in other PCR methods).

As used herein, the terms "restriction endonucleases" and "restriction enzymes" refer to bacterial enzymes, each of which cut double-stranded DNA at or near a specific nucleotide sequence.

A "restriction site" refers to a nucleotide sequence recognized and cleaved by a given restriction endonuclease and is frequently the site for insertion of DNA fragments. In certain embodiments of the invention restriction sites are engineered into the selective marker and into 5' and 3' ends of the DNA construct.

"Homologous recombination" means the exchange of DNA fragments between two DNA molecules or paired chromosomes at the site of identical or nearly identical nucleotide sequences. In a preferred embodiment, chromosomal integration is homologous recombination.

As used herein "amino acid" refers to peptide or protein sequences or portions thereof. The terms "protein," "peptide," and "polypeptide" are used interchangeably.

As used herein, "protein of interest" and "polypeptide of interest" refer to a protein/polypeptide that is desired and/or being assessed. In some embodiments, the protein of interest is expressed intracellularly, while in other embodiments, it is a secreted polypeptide. In particularly preferred embodiments, these enzymes include the serine proteases of the present invention. In some embodiments, the protein of interest is a secreted polypeptide which is fused to a signal peptide *(i.e.,* an amino-terminal extension on a protein to be secreted). Nearly all secreted proteins use an amino-terminal protein extension which plays a crucial role in the targeting to and translocation of precursor proteins across the membrane. This extension is proteolytically removed by a signal peptidase during or immediately following membrane transfer.

A polynucleotide is said to "encode" an RNA or a polypeptide if, in its native state or when manipulated by methods known to those of skill in the art, it can be transcribed and/or translated to produce the RNA, the polypeptide or a fragment thereof. The anti-sense strand of such a nucleic acid is also said to encode the sequences. As is known in the art, a DNA can be transcribed by an RNA polymerase to produce RNA, but an RNA can be reverse transcribed by reverse transcriptase to produce a DNA. Thus a DNA can encode a RNA and vice versa.

"Host strain" or "host cell" refers to a suitable host for an expression vector comprising DNA according to the present invention.

An enzyme is "overexpressed" in a host cell if the enzyme is expressed in the cell at a higher level that the level at which it is expressed in a corresponding wild-type cell.

The terms "protein" and "polypeptide" are used interchangeability herein. The 3-letter code for amino acids as defined in conformity with the IUPAC-IUB Joint Commission on Biochemical Nomenclature (JCBN) is used through out this disclosure. It is also understood that a polypeptide may be coded for by more than one nucleotide sequence due to the degeneracy of the genetic code.

A "prosequence" is an amino acid sequence between the signal sequence and mature protease that is necessary for the secretion of the protease. Cleavage of the pro sequence results in a mature active protease.

The term "signal sequence" or "signal peptide" refers to any sequence of nucleotides and/or amino acids which may participate in the secretion of the mature or precursor forms of the protein. This definition of signal sequence is a functional one, meant to include all those amino acid sequences encoded by the N-terminal portion of the protein gene, which participate in the effectuation of the secretion of protein. They are often, but not universally, bound to the N-terminal portion of a protein or to the N-terminal portion of a precursor protein. The signal sequence may be endogenous or exogenous. The signal sequence may be that normally associated with the protein (*e.g.*, protease), or may be from a gene encoding another secreted protein. One exemplary exogenous signal sequence comprises the first seven amino acid residues of the signal sequence from *Bacillus subtilis* subtilisin fused to the remainder of the signal sequence of the subtilisin from *Bacillus lentus* (ATCC 21536).

The term "hybrid signal sequence" refers to signal sequences in which part of sequence is obtained from the expression host fused to the signal sequence of the gene to be expressed. In some embodiments, synthetic sequences are utilized.

The term "mature" form of a protein or peptide refers to the final functional form of the protein or peptide. For example, a mature form of the protease of the present invention includes at least the amino acid sequence identical to residue positions 1-275 of SEQ ID NO:2.

The term "precursor" form of a protein or peptide refers to a mature form of the protein having a prosequence operably linked to the amino or carbonyl terminus of the protein. The precursor may also have a "signal" sequence operably linked, to the amino terminus of the prosequence. The precursor may also have additional polynucleotides that are involved in post-translational activity (e.g., polynucleotides cleaved therefrom to leave the mature form of a protein or peptide).

"Naturally occurring enzyme" refers to an enzyme having the unmodified amino acid sequence identical to that found in nature. Naturally occurring enzymes include native enzymes, those enzymes naturally expressed or found in the particular microorganism.

The terms "derived from" and "obtained from" refer to not only a protease produced or producible by a strain of the organism in question, but also a protease encoded by a DNA sequence isolated from such strain and produced in a host organism containing such DNA sequence. Additionally, the term refers to a protease which is encoded by a DNA sequence of synthetic and/or cDNA origin and which has the identifying characteristics of the protease in question.

A "derivative" within the scope of this definition generally retains the characteristic proteolytic activity observed in the wild-type, native or parent form to the extent that the derivative is useful for similar purposes as the wild-type, native or parent form. Functional derivatives of serine protease encompass naturally occurring, synthetically or recombinantly produced peptides or peptide fragments which have the general characteristics of the serine protease of the present invention.

The term "functional derivative" refers to a derivative of a nucleic acid which has the functional characteristics of a nucleic acid which encodes serine protease. Functional derivatives of a nucleic acid which encode serine protease of the present invention encompass naturally occurring, synthetically or recombinantly produced nucleic acids or fragments and encode serine protease characteristic of the present invention. Wild type nucleic acid encoding serine proteases according to the invention include naturally occurring alleles and homologues based on the degeneracy of the genetic code known in the art.

The term "identical" in the context of two nucleic acids or polypeptide sequences refers to the residues in the two sequences that are the same when aligned for maximum correspondence, as measured using one of the following sequence comparison or analysis algorithms.

The term "optimal alignment" refers to the alignment giving the highest percent identity score.

"Percent sequence identity," "percent amino acid sequence identity," "percent gene sequence identity," and/or "percent nucleic acid/polynucleotide sequence identity," with respect to two amino acid, polynucleotide and/or gene sequences (as appropriate), refer to the percentage of residues that are identical in the two sequences when the sequences are optimally aligned. Thus, 80% amino acid sequence identity means that 80% of the amino acids in two optimally aligned polypeptide sequences are identical.

The phrase "substantially identical" in the context of two nucleic acids or polypeptides thus refers to a polynucleotide or polypeptide that comprising at least about 70% sequence identity, preferably at least about 75%, preferably at least about 80%, preferably at least about 85%, preferably at least about 90%, preferably at least about 95% , preferably at least about 97% , preferably at least about 98%, and preferably at least about 99% sequence identity as compared to a reference sequence using the programs or algorithms (e.g., BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (e.g., within a range of medium to high stringency).

The phrase "equivalent," in this context, refers to serine proteases enzymes that are encoded by a polynucleotide capable of hybridizing to the polynucleotide having the sequence as shown in SEQ ID NO:1, under conditions of medium to maximum stringency. For example, being equivalent means that an equivalent mature serine protease comprises at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98%,and/or at least about 99% sequence identity to the mature BPN' serine protease having the amino acid sequence of SEQ ID NO:2.

The term "isolated" or "purified" refers to a material that is removed from its original environment (e.g., the natural environment if it is naturally occurring). For example, the material is said to be "purified" when it is present in a particular composition in a higher or lower concentration than exists in a naturally occurring or wild type organism or in combination with components not normally present upon expression from a naturally occurring or wild type organism. For example, a naturally-occurring polynucleotide or polypeptide present in a living animal is not isolated, but the same polynucleotide or polypeptide, separated from some or all of the coexisting materials in the natural system, is isolated. In some embodiments, such polynucleotides are part of a vector, and/or such polynucleotides or polypeptides are part of a composition, and still be isolated in that such vector or composition is not part of its natural environment. In preferred embodiments, a nucleic acid or protein is said to be purified, for example, if it gives rise to essentially one band in an electrophoretic gel or blot.

The term "isolated", when used in reference to a DNA sequence, refers to a DNA sequence that has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art *(See e.g.,* Dynan and Tijan, Nature 316:774-78 [1985]). The term "an isolated DNA sequence" is alternatively referred to as "a cloned DNA sequence".

The term "isolated," when used in reference to a protein, refers to a protein that is found in a condition other than its native environment. In a preferred form, the isolated protein is substantially free of other proteins, particularly other homologous proteins. An isolated protein is more than about 10% pure, preferably more than about 20% pure, and even more preferably more than about 30% pure, as determined by SDS-PAGE. Further aspects of the invention encompass the protein in a highly purified form (i.e., more than about 40% pure, more than about 60% pure, more than about 80% pure, more than about 90% pure, more than about 95% pure, more than about 97% pure, and even more than about 99% pure), as determined by SDS-PAGE.

As used herein, the term, "combinatorial mutagenesis" refers to methods in which libraries of variants of a starting sequence are generated. In these libraries, the variants contain one or several mutations chosen from a predefined set of mutations. In addition, the methods provide means to introduce random mutations which were not members of the predefined set of mutations. In some embodiments, the methods include those set forth in U.S. Patent Appln. Ser. No. 09/699.250, filed October 26, 2000, hereby incorporated by reference. In alternative embodiments, combinatorial mutagenesis methods encompass commercially available kits (*e.g.*, QuikChange® Multisite, Stratagene, San Diego, CA).

As used herein, the term "library of mutants" refers to a population of cells which are identical in most of their genome but include different homologues of one or more genes. Such libraries can be used, for example, to identify genes or operons with improved traits.

As used herein, the term "starting gene" refers to a gene of interest that encodes a protein of interest that is to be improved and/or changed using the present invention.

As used herein, the term "multiple sequence alignment" ("MSA") refers to the sequences of multiple homologs of a starting gene that are aligned using an algorithm (e.g., Clustal W).

As used herein, the terms "consensus sequence" and "canonical sequence" refer to an archetypical amino acid sequence against which all variants of a particular protein or sequence of interest are compared. The terms also refer to a sequence that sets forth the nucleotides that are most often present in a DNA sequence of interest. For each position of a gene, the consensus sequence gives the amino acid that is most abundant in that position in the MSA.

As used herein, the term "consensus mutation" refers to a difference in the sequence of a starting gene and a consensus sequence. Consensus mutations are identified by comparing the sequences of the starting gene and the consensus sequence resulting from an MSA. In some embodiments, consensus mutations are introduced into the starting gene such that it becomes more similar to the consensus sequence. Consensus mutations also include amino acid changes that change an amino acid in a starting gene to an amino acid that is more frequently found in an MSA at that position relative to the frequency of that amino acid in the starting gene. Thus, the term consensus mutation comprises all single amino acid changes that replace an amino acid of the starting gene with an amino acid that is more abundant than the amino acid in the MSA.

As used herein, the term "initial hit" refers to a variant that was identified by screening a combinatorial consensus mutagenesis library. In preferred embodiments, initial hits have improved performance characteristics, as compared to the starting gene.

As used herein, the term "improved hit" refers to a variant that was identified by screening an enhanced combinatorial consensus mutagenesis library.

As used herein, the terms "improving mutation" and "performance-enhancing mutation" refer to a mutation that leads to improved performance when it is introduced into the starting gene. In some preferred embodiments, these mutations are identified by sequencing hits that were identified during the screening step of the method. In most embodiments, mutations that are more frequently found in hits are likely to be improving mutations, as compared to an unscreened combinatorial consensus mutagenesis library.

As used herein, the term "enhanced combinatorial consensus mutagenesis library" refers to a CCM library that is designed and constructed based on screening and/or sequencing results from an earlier round of CCM mutagenesis and screening. In some embodiments, the enhanced CCM library is based on the sequence of an initial hit resulting from an earlier round of CCM. In additional embodiments, the enhanced CCM is designed such that mutations that were frequently observed in initial hits from earlier rounds of mutagenesis and screening are favored. In some preferred embodiments, this is accomplished by omitting primers that encode performance-reducing mutations or by increasing the concentration of primers that encode performance-enhancing mutations relative to other primers that were used in earlier CCM libraries.

As used herein, the term "performance-reducing mutations" refer to mutations in the combinatorial consensus mutagenesis library that are less frequently found in hits resulting from screening as compared to an unscreened combinatorial consensus mutagenesis library. In preferred embodiments, the screening process removes and/or reduces the abundance of variants that contain "performance-reducing mutations."

As used herein, the term "functional assay" refers to an assay that provides an indication of a protein's activity. In particularly preferred embodiments, the term refers to assay systems in which a protein is analyzed for its ability to function in its usual capacity. For example, in the case of enzymes, a functional assay involves determining the effectiveness of the enzyme in catalyzing a reaction.

As used herein, the term "target property" refers to the property of the starting gene that is to be altered. It is not intended that the present invention be limited to any particular target property. However, in some preferred embodiments, the target property is the stability of a gene product (e.g., resistance to denaturation, proteolysis or other degradative factors), while in other embodiments, the level of production in a production host is altered. Indeed, it is contemplated that any property of a starting gene will find use in the present invention.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (*e.g.,* promoter strength, promoter recognition, promoter regulation, enhancer function), a property affecting RNA processing (*e.g.,* RNA splicing, RNA stability, RNA conformation, and post-transcriptional modification), a property affecting translation (*e.g.,* level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide (including proteins), as used herein, refer to any characteristic or attribute of a polypeptide that can be selected or detected. These properties include, but are not limited to oxidative stability, substrate specificity, catalytic activity, thermal stability, alkaline stability, pH activity profile, resistance to proteolytic degradation, $K_M$, $k_{cat}$, $k_{cat}/k_M$ ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat disease, etc.

As used herein, the term "screening" has its usual meaning in the art and is, in general a multistep process. In the first step, a mutant nucleic acid or variant polypeptide therefrom is provided. In the second step, a property of the mutant nucleic acid or variant polypeptide is determined. In the third step, the determined property is compared to a property of the corresponding precursor nucleic acid, to the property of the corresponding naturally occurring polypeptide or to the property of the starting material (*e.g.*, the initial sequence) for the generation of the mutant nucleic acid.

It will be apparent to the skilled artisan that the screening procedure for obtaining a nucleic acid or protein with an altered property depends upon the property of the starting material the modification of which the generation of the mutant nucleic acid is intended to facilitate. The skilled artisan will therefore appreciate that the invention is not limited to any specific property to be screened for and that the following description of properties lists illustrative examples only. Methods for screening for any particular property are generally described in the art. For example, one can measure binding, pH, specificity, etc., before and after mutation, wherein a change indicates an alteration. Preferably, the screens are performed in a high-throughput manner, including multiple samples being screened simultaneously, including, but not limited to assays utilizing chips, phage display, and multiple substrates and/or indicators.

As used herein, in some embodiments, screens encompass selection steps in which variants of interest are enriched from a population of variants. Examples of these embodiments include the selection of variants that confer a growth advantage to the host organism, as well as phage display or any other method of display, where variants can be captured from a population of variants based on their binding or catalytic properties. In a preferred embodiment, a library of variants is exposed to stress (heat, protease, denaturation) and subsequently variants that are still intact are identified in a screen or enriched by selection. It is intended that the term encompass any suitable means for selection. Indeed, it is not intended that the present invention be limited to any particular method of screening.

As used herein, the term "targeted randomization" refers to a process that produces a plurality of sequences where one or several positions have been randomized. In some embodiments, randomization is complete (*i.e.,* all four nucleotides, A, T, G, and C can occur at a randomized position. In alternative embodiments, randomization of a nucleotide is limited to a subset of the four nucleotides. Targeted randomization can be applied to one or several codons of a sequence, coding for one or several proteins of interest. When expressed, the resulting libraries produce protein populations in which one or more amino acid positions can contain a mixture of all 20 amino acids or a subset of amino acids, as determined by the randomization scheme of the randomized codon. In some embodiments, the individual members of a population resulting from targeted randomization differ in the number of amino acids, due to targeted or random insertion or deletion of codons. In further embodiments, synthetic amino acids are included in the protein populations produced. In some preferred embodiments, the majority of members of a population resulting from targeted randomization show greater sequence homology to the consensus sequence than the starting gene. In some embodiments, the sequence encodes one or more proteins of interest. In alternative embodiments, the proteins have differing biological functions. In some preferred embodiments, the incoming sequence comprises at least one selectable marker. This sequence can code for one or more proteins of interest. It can have other biological function. In many cases the incoming sequence will include a selectable marker, such as a gene that confers resistance to an antibiotic.

The terms "modified sequence" and "modified genes" are used interchangeably herein to refer to a sequence that includes a deletion, insertion or interruption of naturally occurring nucleic acid sequence. In some preferred embodiments, the expression product of the modified sequence is a truncated protein (*e.g.,* if the modification is a deletion or interruption of the sequence). In some particularly preferred embodiments, the truncated protein retains biological activity. In alternative embodiments, the expression product of the modified sequence is an elongated protein (*e.g.,* modifications comprising an insertion into the nucleic acid sequence). In some embodiments, an insertion leads to a truncated protein (*e.g.,* when the insertion results in the formation of a stop codon). Thus, an insertion may result in either a truncated protein or an elongated protein as an expression product.

As used herein, the terms "mutant sequence" and "mutant gene" are used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity (e.g., enhanced enzymatic activity).

The terms "mutagenic primer" or "mutagenic oligonucleotide" (used interchangeably herein) are intended to refer to oligonucleotide compositions which correspond to a portion of the template sequence and which are capable of hybridizing thereto. With respect to mutagenic primers, the primer will not precisely match the template nucleic acid, the mismatch or mismatches in the primer being used to introduce the desired mutation into the nucleic acid library. As used herein, "non-mutagenic primer" or "non-mutagenic oligonucleotide" refers to oligonucleotide compositions which will match precisely to the template nucleic acid. In one embodiment of the invention, only mutagenic primers are used. In another preferred embodiment of the invention, the primers are designed so that for at least one region at which a mutagenic primer has been included, there is also non-mutagenic primer included in the oligonucleotide mixture. By adding a mixture of mutagenic primers and non-mutagenic primers corresponding to at least one of the mutagenic primers, it is possible to produce a resulting nucleic acid library in which a variety of combinatorial mutational patterns are presented. For example, if it is desired that some of the members of the mutant nucleic acid library retain their precursor sequence at certain positions while other members are mutant at such sites, the non-mutagenic primers provide the ability to obtain a specific level of non-mutant members within the nucleic acid library for a given residue. The methods of the invention employ mutagenic and non-mutagenic oligonucleotides which are generally between 10-50 bases in length, more preferably about 15-45 bases in length. However, it may be necessary to use primers that are either shorter than 10 bases or longer than 50 bases to obtain the mutagenesis result desired. With respect to corresponding mutagenic and non-mutagenic primers, it is not necessary that the corresponding oligonucleotides be of identical length, but only that there is overlap in the region corresponding to the mutation to be added. Primers may be added in a pre-defined ratio according to the present invention. For example, if it is desired that the resulting library have a significant level of a certain specific mutation and a lesser amount of a different mutation at the same or different site, by adjusting the amount of primer added, it is possible to produce the desired biased library. Alternatively, by adding lesser or greater amounts of non-mutagenic primers, it is possible to adjust the frequency with which the corresponding mutation(s) are produced in the mutant nucleic acid library.

As used herein, the phrase "contiguous mutations" refers to mutations which are presented within the same oligonucleotide primer. For example, contiguous mutations may be adjacent or nearby each other, however, they will be introduced into the resulting mutant template nucleic acids by the same primer.

As used herein, the phrase "discontiguous mutations" refers to mutations which are presented in separate oligonucleotide primers. For example, discontiguous mutations will be introduced into the resulting mutant template nucleic acids by separately prepared oligonucleotide primers.

The terms "wild-type sequence," or "wild-type gene" are used interchangeably herein, to refer to a sequence that is native or naturally occurring in a host cell. In some embodiments, the wild-type sequence refers to a sequence of interest that is the starting point of a protein engineering project. The wild-type sequence may encode either a homologous or heterologous protein. A homologous protein is one the host cell would produce without intervention. A heterologous protein is one that the host cell would not produce but for the intervention.

Unless otherwise indicated, the amino acid position numbers refer to those assigned to the mature *Bacillus amyloliquefaciens* subtilisin sequence of SEQ ID NO:2. The invention, however, is not limited to the mutation of this particular subtilisin but extends to precursor proteases containing amino acid residues at positions which are "equivalent" to the particular identified residues in the *Bacillus amyloliquefaciens* subtilisin.

As used herein, the terms "modification" and "mutation" refers to any change or alteration in an amino acid sequence. It is intended that the term encompass substitutions, deletions, insertions, and/or replacement of amino acid side chains in an amino acid sequence of interest (*e.g.,* a subtilisin sequence). It is also intended that the term encompass chemical modification of an amino acid sequence of interest (*e.g.,* a subtilisin sequence).

As used herein, the term "antibodies" refers to immunoglobulins. Antibodies include but are not limited to immunoglobulins obtained directly from any species from which it is desirable to produce antibodies. In addition, the present invention encompasses modified antibodies. The term also refers to antibody fragments that retain the ability to bind to the epitope that the intact antibody binds and include polyclonal antibodies, monoclonal antibodies, chimeric antibodies, anti-idiotype (anti-ID) antibodies. Antibody fragments include, but are not limited to the complementarity-determining regions (CDRs), single-chain fragment variable regions (scFv), heavy chain variable region (VH), light chain variable region (VL). Polyclonal and monoclonal antibodies are also encompassed by the present invention. Preferably, the antibodies are monoclonal antibodies.

The term "oxidation stable" refers to proteases of the present invention that retain a specified amount of enzymatic activity over a given period of time under conditions prevailing during the proteolytic, hydrolyzing, cleaning or other process of the invention, for example while exposed to or contacted with bleaching agents or oxidizing agents. In some embodiments, the proteases retain at least about 50%, about 60%, about 70%, about 75%, about 80%, about 85%, about 90%, about 92%, about 95%, about 96%, about 97%, about 98%,or about 99% proteolytic activity after contact with a bleaching or oxidizing agent over a given time period, for example, at least about 1 minute, about 3 minutes, about 5 minutes, about 8 minutes, about 12 minutes, about 16 minutes, about 20 minutes, etc. In some embodiments, the stability is measured as described in the Examples.

The term "chelator stable" refers to proteases of the present invention that retain a specified amount of enzymatic activity over a given period of time under conditions prevailing during the proteolytic, hydrolyzing, cleaning or other process of the invention, for example while exposed to or contacted with chelating agents. In some embodiments, the proteases retain at least about 50%, about 60%, about 70%, about 75%, about 80%, about 85%, about 90%, about 92%, about 95%, about 96%, about 97%, about 98%,or about 99% proteolytic activity after contact with a chelating agent over a given time period, for example, at least about 10 minutes, about 20 minutes, about 40 minutes, about 60 minutes, about 100 minutes, etc. In some embodiments, the chelator stability is measured as described in the Examples.

The terms "thermally stable" and "thermostable" refer to proteases of the present invention that retain a specified amount of enzymatic activity after exposure to identified temperatures over a given period of time under conditions prevailing during the proteolytic, hydrolyzing, cleaning or other process of the invention, for example while exposed altered temperatures. Altered temperatures includes increased or decreased temperatures. In some embodiments, the proteases retain at least about 50%, about 60%, about 70%, about 75%, about 80%, about 85%, about 90%, about 92%, about 95%, about 96%, about 97%, about 98%,or about 99% proteolytic activity after exposure to altered temperatures over a given time period, for example, at least about 60 minutes, about 120 minutes, about 180 minutes, about 240 minutes, about 300 minutes, etc. In some embodiments, the thermostability is determined as described in the Examples.

The term "enhanced stability" in the context of an oxidation, chelator, thermal and/or pH stable protease refers to a higher retained proteolytic activity over time as compared to other serine proteases (*e.g.,* subtilisin proteases) and/or wild-type enzymes.

The term "diminished stability" in the context of an oxidation, chelator, thermal and/or pH stable protease refers to a lower retained proteolytic activity over time as compared to other serine proteases (*e.g.,* subtilisin proteases) and/or wild-type enzymes.

The term "cleaning activity" refers to the cleaning performance achieved by the protease under conditions prevailing during the proteolytic, hydrolyzing, cleaning or other process of the invention. In some embodiments, cleaning performance is determined by the application of various cleaning assays concerning enzyme sensitive stains, for example grass, blood, milk, or egg protein as determined by various chromatographic, spectrophotometric or other quantitative methodologies after subjection of the stains to standard wash conditions. Exemplary assays include, but are not limited to those described in WO 99/34011, and U.S. Pat. 6,605,458 (both of which are herein incorporated by reference), as well as those methods included in the Examples.

The term "cleaning effective amount" of a protease refers to the quantity of protease described hereinbefore that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g.,* granular, bar) composition is required, etc.

The term "cleaning adjunct materials," as used herein, means any liquid, solid or gaseous material selected for the particular type of cleaning composition desired and the form of the product (*e.g.,* liquid, granule, powder, bar, paste, spray, tablet, gel; or foam composition), which materials are also preferably compatible with the protease enzyme used in the composition. In some embodiments, granular compositions are in "compact" form, while in other embodiments, the liquid compositions are in a "concentrated" form.

The term "enhanced performance" in the context of cleaning activity refers to an increased or greater cleaning activity of certain enzyme sensitive stains such as egg, milk, grass or blood, as determined by usual evaluation after a standard wash cycle and/or multiple wash cycles.

The term "diminished performance" in the context of cleaning activity refers to an decreased or lesser cleaning activity of certain enzyme sensitive stains such as egg, milk, grass or blood, as determined by usual evaluation after a standard wash cycle.

The term "comparative performance" in the context of cleaning activity refers to at least about 60%, at least about 70%, at least about 80% at least about 90%, or at least about 95% of the cleaning activity of a comparative subtilisin protease (e.g., commercially available proteases), including but not limited to OPTIMASE™ protease (Genencor), PURAFECT™protease products (Genencor), SAVINASE™ protease (Novozymes), BPN'-variants (*See e.g.,* U.S. Pat. No. Re 34,606), RELASE™, DURAZYME™, EVERLASE™, KANNASE™ protease (Novozymes), MAXACAL™, MAXAPEM™, PROPERASE™ proteases (Genencor; *See also,* U.S. Pat. No. Re 34,606, and U.S. Pat. Nos. 5,700,676; 5,955,340; 6,312,936; and 6,482,628), and *B. lentus* variant protease products (e.g., those described in WO 92/21760, WO 95/23221 and/or WO 97/07770). Exemplary subtilisin protease variants include, but are not limited to those having substitutions or deletions at residue positions equivalent to positions 76, 101, 103, 104, 120, 159, 167, 170, 194, 195, 217, 232, 235, 236, 245, 248, and/or 252 of BPN'. Cleaning performance can be determined by comparing the proteases of the present invention with those subtilisin proteases in various cleaning assays concerning enzyme sensitive stains such as grass, blood or milk as determined by usual spectrophotometric or analytical methodologies after standard wash cycle conditions.

As used herein, "fabric cleaning compositions" include hand and machine laundry detergent compositions including laundry additive compositions and compositions suitable for use in the soaking and/or pretreatment of stained fabrics (*e.g.*, clothes, linens, and other textile materials).

As used herein, "non-fabric cleaning compositions" include non-textile (*i.e.*, fabric) surface cleaning compositions, including but not limited to dishwashing detergent compositions, oral cleaning compositions, denture cleaning compositions, and personal cleansing compositions.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. A preferred filler salt is sodium sulfate.

As used herein, the term "surfactant" refers to any compound generally recognized in the art as having surface active qualities. Surfactants generally include anionic, cationic, nonionic, and zwitterionic compounds, which are further described, herein.

As used herein, the terms "contacting" and "exposing" refer to placing a surfactant and lipolytic enzyme in sufficient proximity an oily stain or oily soil to enable the enzyme and surfactant to at least partially decrease the amount of the stain or soil by producing fatty acids that are solubilized in the surfactant. Contacting may occur in a washing machine, a sink, on a body surface, etc.

**EXPERIMENTAL**

The present invention is described in further detail in the following Examples which are not in any way intended to limit the scope of the invention as claimed. The attached Figures are meant to be considered as integral parts of the specification and description of the invention. The following Examples are offered to illustrate, but not to limit the claimed invention

In the experimental disclosure which follows, the following abbreviations apply: ppm (parts per million); M (molar); mM (millimolar); μM (micromolar); nM (nanomolar); mol (moles); mmol (millimoles); μmol (micromoles); nmol (nanomoles); gm (grams); mg (milligrams); μg (micrograms); pg (picograms); L (liters); ml and mL (milliliters); μl and μL (microliters); cm (centimeters); mm (millimeters); μm (micrometers); nm (nanometers); U (units); V (volts); MW (molecular weight); sec (seconds); min(s) (minute/minutes); h(s) and hr(s) (hour/hours); °C. (degrees Centigrade); QS (quantity sufficient); ND (not done); NA (not applicable); rpm (revolutions per minute); $H_2O$ (water); $dH_2O$ (deionized water); (HCl (hydrochloric acid); aa (amino acid); bp (base pair); kb (kilobase pair); kD (kilodaltons); cDNA (copy or complementary DNA); DNA (deoxyribonucleic acid); ssDNA (single stranded DNA); dsDNA (double stranded DNA); dNTP (deoxyribonucleotide triphosphate); RNA (ribonucleic acid); $MgCl_2$ (magnesium chloride); NaCl (sodium chloride); w/v (weight to volume); v/v (volume to volume); g (gravity); OD (optical density); PI (performance index); Dulbecco's phosphate buffered solution (DPBS); SOC (2% Bacto-Tryptone, 0.5% Bacto Yeast Extract, 10 mM NaCl, 2.5 mM KCl); Terrific Broth (TB; 12 g/l Bacto Tryptone, 24 g/l glycerol, 2.31 g/l $KH_2PO_4$, and 12.54 g/l $K_2HPO_4$); $OD_{280}$ (optical density at 280 nm); $OD_{600}$ (optical density at 600 nm); $A_{405}$ (absorbance at 405 nm); Vmax (the maximum initial velocity of an enzyme catalyzed reaction); PAGE (polyacrylamide gel electrophoresis); PBS (phosphate buffered saline [150 mM NaCl, 10 mM sodium phosphate buffer, pH 7.2]); PBST (PBS+0.25% TWEEN® 20); PEG (polyethylene glycol); PCR (polymerase chain reaction); RT-PCR (reverse transcription PCR); SDS (sodium dodecyl sulfate); Tris (tris(hydroxymethyl)aminomethane); HEPES (N-[2-Hydroxyethyl]piperazine-N-[2-ethanesulfonic acid]); HBS (HEPES buffered saline); Tris-HCl (tris[Hydroxymethyl]aminomethane-hydrochloride); Tricine (N-[tris-(hydroxymethyl)-methyl]-glycine); CHES (2-(N-cyclo-hexylamino) ethane-sulfonic acid); TAPS (3-{[tris-(hydroxymethyl)-methyl]-amino}-propanesulfonic acid); CAPS (3-(cyclo-hexylamino)-propane-sulfonic acid; DMSO (dimethyl sulfoxide); DTT (1,4-dithio-DL-threitol); SA (sinapinic acid (s,5-dimethoxy-4-hydroxy cinnamic acid); TCA (trichloroacetic acid); Glut and GSH (reduced glutathione); GSSG (oxidized glutathione); TCEP (Tris[2-carboxyethyl] phosphine); Ci (Curies); mCi (milliCuries); μCi (microCuries); HPLC (high pressure liquid chromatography); RP-HPLC (reverse phase high pressure liquid chromatography); TLC (thin layer chromatography); MALDI-TOF (matrix-assisted laser desorption/ionization--time of flight); Ts (tosyl); Bn (benzyl); Ph (phenyl); Ms (mesyl); Et (ethyl), Me (methyl); *Taq* (*Thermus aquaticus* DNA polymerase); Klenow (DNA polymerase I large (Klenow) fragment); EGTA (ethylene glycol-bis(ß-aminoethyl ether) N, N, N', N'-tetraacetic acid); EDTA (ethylenediaminetetracetic acid); bla (β-lactamase or ampicillin-resistance gene); HDL (high density liquid); MJ Research (MJ Research, Reno, NV); Baseclear (Baseclear BV, Inc., Leiden, the Netherlands); PerSeptive (PerSeptive Biosystems, Framingham, MA); ThermoFinnigan (ThermoFinnigan, San Jose, CA); Argo (Argo BioAnalytica, Morris Plains, NJ);Seitz EKS (SeitzSchenk Filtersystems GmbH, Bad Kreuznach, Germany); Pall (Pall Corp., East Hills, NY); Spectrum (Spectrum Laboratories, Dominguez Rancho, CA); Molecular Structure (Molecular Structure Corp., Woodlands, TX); Accelrys (Accelrys, Inc., San Diego, CA); Chemical Computing (Chemical Computing Corp., Montreal, Canada); New Brunswick (New Brunswick Scientific, Co., Edison, NJ); CFT (Center for Test Materials, Vlaardingen, the Netherlands); Test Fabrics (Test Fabrics, Inc., West Pittiston, PA), Procter & Gamble (Procter & Gamble, Inc., Cincinnati, OH); GE Healthcare (GE Healthcare, Chalfont St. Giles, United Kingdom); OXOID (Oxoid, Basingstoke, Hampshire, UK); Megazyme (Megazyme International Ireland Ltd., Bray Business Park, Bray, Co., Wicklow, Ireland); Finnzymes (Finnzymes Oy, Espoo, Finland); Kelco (CP Kelco, Wilmington, DE); Corning (Corning Life Sciences, Corning, NY); (NEN (NEN Life Science Products, Boston, MA); Pharma AS (Pharma AS, Oslo, Norway); Dynal (Dynal, Oslo, Norway); Bio-Synthesis (Bio-Synthesis, Lewisville, TX); ATCC (American Type Culture Collection, Rockville, MD); Gibco/BRL (Gibco/BRL, Grand Island , NY); Sigma (Sigma Chemical Co., St. Louis, MO); Pharmacia (Pharmacia Biotech, Piscataway, NJ); NCBI (National Center for Biotechnology Information); Applied Biosystems (Applied Biosystems, Foster City, CA); BD Biosciences and/or Clontech (BD Biosciences CLONTECH Laboratories, Palo Alto, CA); Operon Technologies (Operon Technologies, Inc., Alameda, CA); MWG Biotech (MWG Biotech, High Point, NC); Oligos Etc (Oligos Etc. Inc, Wilsonville, OR); Bachem (Bachem Bioscience, Inc., King of Prussia, PA); Difco (Difco Laboratories, Detroit, MI); Mediatech (Mediatech, Herndon, VA; Santa Cruz (Santa Cruz Biotechnology, Inc., Santa Cruz, CA); Oxoid (Oxoid Inc., Ogdensburg, NY); Worthington (Worthington Biochemical Corp., Freehold, NJ); GIBCO BRL or Gibco BRL (Life Technologies, Inc., Gaithersburg, MD); Millipore (Millipore, Billerica, MA); Bio-Rad (Bio-Rad, Hercules, CA); Invitrogen (Invitrogen Corp., San Diego, CA); NEB (New England Biolabs, Ipswich, MA); Sigma (Sigma Chemical Co., St. Louis, MO); Pierce (Pierce Biotechnology, Rockford, IL); Takara (Takara Bio Inc. Otsu, Japan); Roche (Hoffmann-La Roche, Basel, Switzerland); EM Science (EM Science, Gibbstown, NJ); Qiagen (Qiagen, Inc., Valencia, CA); Biodesign (Biodesign Intl., Saco, Maine); Aptagen (Aptagen, Inc., Herndon, VA); Sorvall (Sorvall brand, from Kendro Laboratory Products, Asheville, NC); United States Testing (United States Testing Co., Hoboken, NJ);Molecular Devices (Molecular Devices, Corp., Sunnyvale, CA); R&D Systems (R&D Systems, Minneapolis, MN); Stratagene (Stratagene Cloning Systems, La Jolla, CA); Marsh (Marsh Biosciences, Rochester, NY); Geneart (Geneart GmbH, Regensburg, Germany); DNA2.0 (DNA2.0, Menlo Park, CA); Gene Oracle (Gene Oracle, Mountain View, CA); Bio-Tek (Bio-Tek Instruments, Winooski, VT); Biacore (Biacore, Inc., Piscataway, NJ); Bioké (Bioké, Leiden, The Netherlands); PeproTech (PeproTech, Rocky Hill, NJ); SynPep (SynPep, Dublin, CA); New Objective (New Objective brand; Scientific Instrument Services, Inc., Ringoes, NJ); Waters (Waters, Inc., Milford, MA); Matrix Science (Matrix Science, Boston, MA); Dionex (Dionex, Corp., Sunnyvale, CA); Monsanto (Monsanto Co., St. Louis, MO); Wintershall (Wintershall AG, Kassel, Germany); BASF (BASF Co., Florham Park, NJ); Huntsman (Huntsman Petrochemical Corp., Salt Lake City, UT); Enichem (Enichem Iberica, Barcelona, Spain); Fluka Chemie AG (Fluka Chemie AG, Buchs, Switzerland); Gist-Brocades (Gist-Brocades, NV, Delft, the Netherlands); Dow Corning (Dow Corning Corp., Midland, MI); and Microsoft (Microsoft, Inc., Redmond, WA).

**EXAMPLE** 1

**Assays**

In the following Examples, various assays were used as set forth below for ease in reading. Any deviations from the protocols provided below are indicated in the Examples.

**A. TCA Assay for Protein Content Determination in 96-well Microtiter Plates**

For BPN' (*e.g.*, reference protease) and BPN' variants, this assay was started using filtered culture supernatant from microtiter plates grown 3-4 days at 33 °C with shaking at 230 rpm and humidified aeration. A fresh 96-well flat bottom microtiter plate (MTP) was used for the assay. First, 100 μL/well of 0.25 N HCl was placed in each well. Then, 50 μL of filtered culture broth was added. The light scattering/absorbance at 405 nm (use 5 sec mixing mode in the plate reader) was then determined, in order to provide the "blank" reading. For the test, 100 μL/well of 15% (w/v) trichloroacetic acid (TCA) was placed in the plates and incubated between 5 and 30 min at room temperature. The light scattering/absorbance at 405 nm (use 5 sec mixing mode in the plate reader) was then determined.

For GG36 (*e.g.,* reference protease) and variants thereof, this assay was performed using filtered culture supernatant from microtiter plates grown approximately 3 days at 37 °C with shaking at 300 rpm and humidified aeration. In this assay 100 μL of a 0.25 M HCl solution was added to each well of a 96-well flat bottom microtiter plate. Subsequently, 25 μL aliquots of the filtered culture supernatants (containing the proteases) were added to wells. The light scattering/absorbance at 405 nm (using the 5 sec mixing mode in the plate reader) was then determined, in order to provide the "blank" reading. After this measurement, 100 μL of a 30% (w/v) TCA solution was added to each well and the microtiter plates were incubated between 5 and 15 minutes at room temperature. Finally, the resulting light scattering/absorbance at 405 nm (using the 5 sec mixing mode in the plate reader) was determined.

The equipment used was a Biomek FX Robot (Beckman Coulter) and a SpectraMAX (type 340; Molecular Devices) MTP Reader; the MTP's were from Costar (type 9017). The equipment used was a Biomek FX Robot (Beckman Coulter) and a SpectraMAX type 340 (Molecular Devices) MTP Reader; and the MTPs were type 9017 (Costar).

The calculations were performed by subtracting the blank (no TCA) from the test reading with TCA to provide a relative measure of the protein content in the samples. If desired, a standard curve can be created by calibrating the TCA readings with AAPF assays of clones with known conversion factors. However, the TCA results are linear with respect to protein concentration from 50 to 500 protein per ml (ppm) and can thus be plotted directly against enzyme performance for the purpose of choosing good-performing variants. The turbidity/light scatter increase in the samples correlates to the total amount of precipitable protein in the culture supernatant.

**B. AAPF Protease Assay in 96-well Microtiter Plates**

In order to determine the protease activity of the proteases and variants thereof of the present invention, the hydrolysis of N-succinyl-L-alanyl-L-alanyl-L-prolyl-L-phenyl-p-nitroanilide (suc-AAPF-pNA) was measured. The reagent solutions used were: 100 mM Tris/HCl, pH 8.6, containing 0.005% TWEEN®-80 (Tris dilution buffer); 100 mM Tris buffer, pH 8.6, containing 10 mM $CaCl_2$ and 0.005% TWEEN®-80 (Tris/Ca buffer); and 160 mM suc-AAPF-pNA in DMSO (suc-AAPF-pNA stock solution) (Sigma: S-7388). To prepare a suc-AAPF-pNA working solution, 1 ml suc-AAPF-pNA stock solution was added to 100 ml Tris/Ca buffer and mixed well for at least 10 seconds. The assay was performed by adding 10 μl of diluted protease solution to each well, immediately followed by the addition of 190 μl 1 mg/ml suc-AAPF-pNA working solution. The solutions were mixed for 5 sec., and the absorbance change in kinetic mode (20 readings in 5 minutes) was read at 410 nm in an MTP reader, at 25°C. The protease activity was expressed as AU (activity = $\Delta OD \cdot min^{-1}$ $ml^{-1}$).

**C. Surfactant and Chelant Stability Assays**

LAS and LAS/EDTA stability was measured after incubation of the test protease in the presence of LAS and LAS/EDTA respectively, as a function of residual activity determined using the AAPF assay.

**LAS Stability Method**

**Reagents:**

Dodecylbenzenesulfonate, Sodium salt (=LAS): Sigma D-2525
TWEEN®-80: Sigma P-8074
TRIS buffer (free acid): Sigma T-1378); 6.35 g is dissolved in about 960 ml water; pH is adjusted to 8.2 with 4N HCl. Final concentration of TRIS is 52.5 mM
LAS stock solution: Prepare a 10.5 % LAS solution in MQ water (=10.5 g per 100 ml MQ)
TRIS buffer-100 mM / pH 8.6 (100mM Tris/0.005% Tween®-80)
TRIS-Ca buffer, pH 8.6 (100mM Tris/10mM CaCl2/0.005% Tween-®80)

**Hardware:**

Flat bottom MTPs (Costar No. 9017)
Biomek FX
ASYS Multipipettor
Spectramax MTP Reader
iEMS Incubator/Shaker
Innova 4330 Incubator/Shaker
Biohit multichannel pipette
BMG Thermostar Shaker

A 0.063% LAS solution was prepared in 52.5 mM Tris buffer pH 8.2. The suc-AAPF-pNA working solution was prepared by adding 1 ml of 100 mg/ml suc-AAPF-pNA stock solution (in DMSO) to 100 ml (100 mM) TRIS buffer, pH 8.6. To dilute the supernatants, flat-bottomed plates were filled with dilution buffer and an aliquot of the supernatant was added and mixed well. The dilution ratio depended on the concentration of the protease controls in the growth plates (AAPF activity). The desired protein concentration was 80 ppm.

Ten μl of the diluted supernatant were added to 190 μl 0.063% LAS buffer/well. The MTP was covered with tape, shaken for a few seconds and placed in an incubator (Innova 4230) at 45°C for BPN' or GG36, for 60 minutes at 200 rpm agitation. The initial activity (*t*=10 minutes) was determined after 10 minutes of incubation by transferring 10 μl of the mixture in each well to a fresh MTP containing 190μl suc-AAPF-pNA work solution. These solutions were mixed well and the AAPF activity was measured using a MTP Reader (20 readings in 5 minutes and 25°C).

The final activity (*t*=60 minutes) was determined by removing another 10 μl of solution from the incubating plate after 60 minutes of incubation. The AAPF activity was then determined as described above. The stability of the samples was determined by calculating the ration of the residual and initial AAPF activity as follows:

$$\text{Residual Activity (\%)} = [t\text{-60 value}]*100 / [t\text{-10 value}].$$

### LAS/EDTA Stability Method

The stability of protease variants in the presence of a representative anionic surfactant (LAS=linear alkylbene sulfonate, sodium dodecylbenzenesulfonate-DOBS) and di-sodium EDTA was measured after incubation under defined conditions and the residual activity was determined using the AAPF assay. The reagents used were dodecyllbenzene sulfonate, sodium salt (DOBS, Sigma No. D-2525), TWEEN®-80 (Sigma No. P-8074), di-sodium EDTA (Siegfried Handel No. 164599-02), HEPES (Sigma No. H-7523), unstress buffer: 50 mM HEPES (11.9 g/l) + 0.005% TWEEN®-80, pH 8.0, Stress buffer: 50 mM HEPES (11.9 g/l), 0.1% (w/v) DOBS (1 g/l), 10 mM EDTA (3.36 g/l), pH 8.0, reference protease and protease variant culture supernatants, containing 200 - 400 μg/ml protein. The equipment used was V- or U-bottom MTP as dilution plates (Greiner 651101 and 650161 respectively), F-bottom MTP (Corning 9017) for unstress and LAS/EDTA buffer as well as for suc-AAPF-pNA plates, Biomek FX (Beckman Coulter), Spectramax Plus 384 MTP Reader (Molecular Devices), iEMS Incubator/Shaker (1 mm amplitude) (Thermo Electron Corporation), sealing tape: Nunc (236366)

The iEMS incubator/shaker (Thermo/Labsystems) was set at 29°C. Culture supernatants were diluted into plates containing unstress buffer to a concentration of ~ 25 ppm (master dilution plate). 20 μl of sample from the master dilution plate was added to plates containing 180 μl unstress buffer to give a final incubation concentration of 2.5 ppm. The contents were mixed and kept at room temperature and a AAPF assay was performed on this plate. 20 μl of sample from the master dilution plate was also added to plates containing 180 μl stress buffer (50 mM HEPES (11.9 g/l), 0.1% (w/v) DOBS (1 g/l), 10 mM EDTA (3.36 g/l), pH 8.0). The solutions were mixed and immediately placed in 29°C iEMS shaker for 30 min at 400 rpm. Following 30 minutes of incubation, a AAPF assay was performed on the stress plate. The stability of the samples was determined by calculating the ratio of the residual and initial AAPF activity as follows: Residual Activity (%) = [mOD.min-1 stressed]*100 / [mOD. min-1 unstressed].

### <u>D. Cleaning Performance Assays</u>

The stain removal performance of the protease variants was determined in commercially available detergents. Heat inactivation of commercial detergent formulas serves to destroy the enzymatic activity of any protein components while retaining the properties of non-enzymatic components. Thus this method was suitable for preparing commercially purchased detergents for use in testing the enzyme variants of the present invention.

### Microswatches:

Microswatches of ¼" circular diameter were obtained from CFT. Single microswatches or two microswatches were placed vertically in each well of a 96-well MTP to expose the whole surface area (*i.e.,* not flat on the bottom of the well).

### BMI Microswatch Assay

Microswatches containing blood milk and ink (BMI) of 0.25 inch circular diameter were obtained from CFT. Before cutting of the swatches, the fabric (EMPA 116) was washed with water. One microswatch was vertically placed in each well of a 96-well microtiter plate in order to expose the whole surface area (*i.e.,* not flat on the bottom of the well). The desired detergent solution was prepared as described herein. After equilibrating the Thermomixer at 25°C, 190 μl of detergent solution was added to each well of the MTP, containing microswatches. To this mixture, 10 μl of the diluted enzyme solution was added so that the final enzyme concentration was 1 μg/ml (determined from BCA assay). The MTP was sealed with tape and placed in the incubator for 30 minutes, with agitation at 1400 rpm. Following incubation under the appropriate conditions, 100 μl of the solution from each well was transferred into a fresh MTP. The new MTP containing 100 μl of solution/well was read at 405 nm using a MTP SpectraMax reader. Blank controls, as well as a control containing two microswatches and detergent but no enzyme were also included.

### Baked Egg Microswatch Assay

The 96-well baked egg yolk substrate plates used in these assays were prepared from chicken egg yolks. Chicken egg yolks were separated from the whites, released from the membrane sac, and diluted 20% (vol/weight) with Milli-Q water. The diluted yolk was stirred for 15 min at room temperature using a magnetic stirrer. Five μL were carefully pipetted into the center of each well of a 96-well V-bottom plate (Costar #3894) using an 8-channel pipette. The plates were baked at 90°C for 1 hour and cooled at room temperature. The baked egg yolk substrate plates were stored at room temperature and used within one week of preparation. Automatic dish detergents were prepared as described elsewhere in this document and pre-heated to 50°C. A 190 μL aliquot of detergent was added to each well of the 96-well plate using an 8-channel pipette. Ten μL of diluted enzyme was added to each well using a 96-channel pipetting device. The plate was carefully sealed with an adhesive foil sealer and incubated at 50°C with shaking for 30 min. Then, 120 μL of the reaction mixture was transferred to a new 96-well flat-bottom plate, and the absorbance/light scattering was determined at 405 nm. The absorbance/light scattering at 405 nm is proportional to egg yolk removal.

**Egg Yolk Microswatch Assay**

Automatic dish detergents were prepared as described elsewhere in this document. The equipment used included a New Brunswick Innova 4230 shaker/incubator and a SpectraMAX (type 340) MTP reader. The MTPs were obtained from Costar (type 9017). Aged egg yolk with pigment swatches (CS-38) were obtained from Center for Test Materials. Before cutting 0.25-inch circular microswatches, the fabric was washed with water. One microswatch was placed in each well of a 96-well microtiter plate. The test detergent was equilibrated at 50°C. 190 μl of detergent solution was added to each well of the MTP, containing microswatches. To this mixture, 10 μl of the diluted enzyme solution was added. The MTP was sealed with adhesive foil and placed in the incubator for 30 minutes, with agitation. Following incubation, 100 μl of the solution from each well was transferred into a fresh MTP. This MTP was read at 405 nm using a SpectraMax MTP reader. Blank controls, as well as controls containing microswatches and detergent but no enzyme were also included.

**"3K" Swatch Fixation**

This type of microswatch was pretreated using the fixation method described below. In a 10 liter beaker, 8 liters of distilled water were mixed well with 80 ml of 30% hydrogen peroxide using a ladle. Forty pieces of EMPA 116 swatches were laid down in a fan-type distribution prior to being added into the solution to ensure uniform fixation. Using the ladle, the swatches were swirled in the solution for a total of 30 minutes, continuously for the first five minutes and occasionally for the remaining 25 minutes. After the fixation process, the solution was discarded and the swatches were rinsed 6 times with approximately 6 liters of distilled water per rinse. After the rinse the swatches were put on top of paper towels to dry. The air-dried swatches were then punched using a ¼" circular die on an expulsion press. Finally, a single microswatch was put into each well of a 96-well MTP vertically to expose the whole surface area (*i.e.* not flat on the bottom of the well).

**"Pre-washed" Swatch**

This type of microswatch was pre-washed in deionised water for 20 minutes at ambient temperature. After the pre-washing step, the swatches were put on top of paper towels to dry. The air-dried swatches were then punched using a ¼" circular die on an expulsion press. Finally two microswatches were put into each well of a 96-well MTP vertically to expose the whole surface area (*i.e.* not flat on the bottom of the well).

**Detergents**

For North American (NA) and Western European (WE) heavy duty liquid laundry (HDL) detergents, heat inactivation was performed by placing pre-weighed liquid detergent (in a glass bottle) in a water bath at 95°C for 2 hours. The incubation time for heat inactivation of North American (NA) and Japanese (JPN) heavy duty granular laundry (HDG) detergent was 8 hours and that for Western European (WE) HDG detergent was 5 hours. The incubation time for heat inactivation of NA and WE auto dish washing (ADW) detergents was 8 hours. The detergents were purchased from local supermarket stores. Both un-heated and heated detergents were assayed within 5 minutes of dissolving the detergent to accurately determine percentage deactivated. Enzyme activity was tested by the AAPF assay.

Working solutions of detergents were made from the heat inactivated stocks. All of the detergents were commercially purchased detergents. Appropriate amounts of water hardness (6 gpg or 12 gpg) and buffer were added to the detergent solutions to match the desired conditions (Table 1-1). The solutions were mixed by vortexing or inverting the bottles.

**Table 1-1. Laundry and Dish Washing Conditions**

| Region | Form | Dose | Detergent* | Buffer | Gpg | pH | T (°C) |
|---|---|---|---|---|---|---|---|
| Laundry (heavy duty liquid and granular) | | | | | | | |
| NA | HDL | 0.78 g/l | P&G TIDE@ 2X | 5 mM HEPES | 6 | 8.0 | 20 |
| WE | HDL | 5.0 g/L | Henkel Persil | 5 mM HEPES | 12 | 8.2 | 40 |

(continued)

| Table 1-1. Laundry and Dish Washing Conditions | | | | | | | |
|---|---|---|---|---|---|---|---|
| **Region** | **Form** | **Dose** | **Detergent*** | **Buffer** | **Gpg** | **pH** | **T (°C)** |
| WE | HDG | 8.0 g/L | P&G Ariel | 2 mM $Na_2 CO_3$ | 12 | 10.5 | 40 |
| JPN | HDG | 0.7 g/L | P&G TIDE@ | 2 mM $Na_2 CO_3$ | 6 | 10.0 | 20 |
| NA | HDG | 1.0 g/L | P&G TIDE@ | 2 mM $Na_2 CO_3$ | 6 | 10.0 | 20 |
| Automatic Dish Washing | | | | | | | |
| WE | ADW | 3.0 g/L | RB Calgonit | 2 mM $Na_2 CO_3$ | 21 | 10.0 | 40 |
| NA | ADW | 3.0 g/L | P&G Cascade | 2 mM $Na_2 CO_3$ | 9 | 10.0 | 40 |
| * Abbreviations: Procter & Gamble (P&G); and Reckitt Benckiser (RB). | | | | | | | |

The stain removal performance of reference serine proteases and variants therefrom on microswatches was determined on a MTP scale in commercially available detergent (Calgonit 5 in1). The reagents used were: 5 mM HEPES, pH 8.0 or 5 mM MOPS, pH 7 buffer, 3:1 Ca: Mg for medium water hardness. ($CaCl_2$: MgCl2●6H2O); 15000 grains per gallon (gpg) stock diluted to 6 gpg, 2 BMI (blood/milk/ink) swatches per plate: EMPA-116 BMI cotton swatches processed by CFT: pre-rinsed and punched 2 swatches per well, and heat inactivated TIDE@ 2X Cold off-the-shelf detergent in which lack of protease activity was confirmed.

| Table 1-2 Working Detergent Solutions | | | | | | |
|---|---|---|---|---|---|---|
| Detergent | Temp (C) | Detergent g/L | pH | Buffer | gpg | Protease |
| TIDE@ 2X Cold | 16 | 0.98 | 8 | 5mM HEPES | 6 | BPN' |
| TIDE@ 2X Cold | 32 | 0.98 | 8 | 5mM HEPES | 6 | GG36, BPN' |
| TIDE@ 2X Cold | 16 | 0.98 | 7 | 5mM MOPS | 6 | BPN' |

The incubator was set at the desired temperature (16°C or 32°C). 10 μL samples from the master dilution plate of ~10 ppm enzyme was added to BMI 2-swatch plates with 190 μL working detergent solutions listed above. The volume was adjusted to give final concentration of 0.5 ppm for variants in the assay plates. The plates were immediately transferred to iEMS incubators and incubated for 30 minutes with 1400 rpm shaking at given temperature. Following incubation, 100 μL of supernatant was transferred into a new 96-well plate and the absorbance was measured in MTP Reader at 405nm and/or 600nm. Control wells, containing 1 or 2 microswatches and detergent without the addition of protease samples were also included in the test. The measurement at 405 nm provides a higher value and tracks pigment removal, while the measurement at 600 nm tracks turbidity and cleaning.

**Calculation of the Stain Removal Activity for All Microswatch Assay Methods:**

The absorbance value obtained was corrected for the blank value (substrate without enzyme), providing a measure of hydrolytic activity. For each sample (variant) the performance index was calculated. The performance index compares the performance of the variant (actual value) and the standard enzyme (theoretical value) at the same protein concentration. In addition, the theoretical values can be calculated, using the parameters of the Langmuir equation of the standard enzyme.

**Enzymes and Equipment**

Samples of reference serine proteases variants thereof were obtained from filtered culture broth of cultures grown in MTP plates. The equipment used was a Biomek FX Robot (Beckman Coulter), a SpectraMAX MTP Reader (type 340; Molecular Devices), an iEMS incubator/shaker (Thermo/Labsystems); F-bottom MTPs (Costar type 9017 used for reading reaction plates after incubation); and V-bottom MTPs (Greiner 651101 used for pre-dilution of supernatant). In this assay, the proteases hydrolyze the substrate and liberate pigment and insoluble particles from the substrate. Thus the rate of turbidity is a measure of enzyme activity.

## E. Relative Specific Activity of Proteases and Variants Thereof

In order to discriminate the protease variants, the apparent relative specific activity was calculated using suc-AAPF-pNA as a substrate, which enabled the comparison and ranking of the variants versus the wild-type or standard protease. The specific activity on the suc-AAPF-pNA substrate was determined by dividing the proteolytic activity by the measured TCA-values of each sample, using the assays described above. Using these values, the relative specific activity was calculated (specific activity of variant/specific activity of reference protease).

## F. Dishwashing Performance

The performance of the protease variants was tested under various automatic dishwashing conditions. The compositions of the dish detergents are shown in the following Tables. These detergents are commercially available from wfk Testmaterials (www.testgewebe.de/en/products/detergents/) and are referred to by their wfk Testmaterials designations. These detergents were obtained from the source without the presence of enzymes, to permit analysis of the protease variants.

| Table 1-3. Phosphate-Free Detergent IEC-60436 WFK Type B (pH=10.4 in 3g/l) | |
|---|---|
| **Component** | **Wt %** |
| Sodium citrate dehydrate | 30.0 |
| Maleic acid/ Acrylic acid copolymer sodium Salt | 12.0 |
| Sodium perborate monohydrate | 5.0 |
| TAED | 2.0 |
| Sodium disilicate: Protil A (Cognis) | 25.0 |
| Linear fatty alcohol ethoxylate | 2.0 |
| Sodium carbonate anhydrous | add to 100 |

| Table 1-4. Phosphate-Containing Detergent: IEC-60436 WFK Type C (pH=10.5 in 3 g/l) | |
|---|---|
| **Component** | **Wt %** |
| Sodium tripolyphosphate | 23.0 |
| Sodium citrate dehydrate | 22.3 |
| Maleic acid/ Acrylic acid copolymer sodium salt | 4.0 |
| Sodium perborate monohydrate | 6.0 |
| TAED | 2.0 |
| Sodium disilicate: Protil A (Cognis) | 5.0 |
| Linear fatty alcohol ethoxylate | 2.0 |
| Sodium carbonate anhydrous | add to 100 |

The protocols for preparation of each of the stain types (egg yolk, minced meat and egg, and egg with milk) are provided below. Before the individual soil types were applied to the test dishes, the dishes were thoroughly washed. This was particularly necessary, as residues of certain persistent stains may still be present on the dishes from previous tests. New dishes were also subjected to three thorough washes before being used for the first time in a test.

### Preparation of Egg Yolk Stains on Stainless Steel

The stainless steel sheets (10 x 15 cm; brushed on one side) used in these experiments were thoroughly washed at 95°C in a laboratory dishwasher with a high-alkalinity commercial detergent (*e.g.,* ECOLAB® detergent; Henkel) to provide sheets that were clean and grease-free. These sheets were deburred prior to their first use. The sheets were dried for 30 minutes at 80°C in a thermal cabinet before being soiled with egg yolk. The surfaces to be brushed were not touched prior to soiling. Also, no water stains or fluff on the surfaces were permitted. The cooled sheets were weighed before soiling.

The egg yolks were prepared by separating the yolks of approximately 10-11 eggs (200 g of egg yolk) from the whites. The yolks were stirred with a fork in a glass beaker to homogenize the yolk suspension. The yolks were then strained (approximately 0.5 mm mesh) to remove coarse particles and any egg shell fragments.

A flat brush (2.5") was used to apply 2.0 ± 0.1 g egg yolk suspension as uniformly as possible over an area of 140 $cm^2$ on the brushed sides of each of the stainless steel sheets, leaving an approximately 1 cm wide unsoiled rim (adhesive tape was used if needed). The soiled sheets were dried horizontally (to prevent formation of droplets on the edges of the sheets), at room temperature for 4 hours (max. 24 hr).

To denaturate the egg yolk proteins, the sheets were immersed for 30 seconds in boiling, demineralized water (using a holding device if necessary). Then the sheets were dried again for 30 min at 80°C. After drying and cooling, the sheets were weighed. After weighing, the sheets were left for at least 24 hrs (20°C, 40-60% relatively humidity) before submitting them to the wash test. In order to meet the testing requirements, only sheets with 1000 ± 100 mg/ 140 $cm^2$ (egg yolk after denaturation) were used in the testing. After the wash tests were conducted, the sheets were dried for 30 min at 80°C in the thermal cabinet and weighed again after cooling. The percent cleaning performance was determined by dividing the mg of egg yolk released upon washing by the mg of denatured egg yolk applied and multiplying by 100.

**Preparation of Minced Meat and Egg Stains on Porcelain Plates**

For these experiments, dessert plates (Arzberg, 19 cm diameter, white, glazed porcelain) conforming to EN 50242, form 1495, No. 0219, were used. A total of 225 g lean pork and beef (50:50 ratio) was finely chopped and maintained cool. The mixture was twice run through a mincer. Temperatures above 35°C were avoided. The 225 g of the minced meat was then mixed with 75 g of egg (white and yolk mixed together). The preparation was then frozen for up to three months at -18°C, prior to use. If pork was not available, 100% beef was used, as these are interchangeable.

The minced meat and egg mixture (300 g) was brought to room temperature and mixed with 80 ml demineralized water. The mixture was then homogenized for 2 min using a kitchen hand blender. A fork was used to spread 3 g of the minced meat/egg/water mixture on each white porcelain plate, leaving an approximately 2 cm wide unsoiled margin around the rim. The amount applied was 11.8 ± 0.5 mg/$cm^2$. The plates were dried for 2 hours at 120°C in a preheated thermal cabinet. As soon as the plates were cooled, they were ready for use.

After conducting the dishwashing tests, the plates were sprayed with ninhydrin solution (prepared to 1% in ethanol) for better identification of the minced meat protein residues. To promote the color reaction, the plates were heated for 10 min at 80°C in the thermal cabinet. Evaluation of the washing performance was done by visually inspecting the color reactions of the minced meat residue with reference to the IKW photographic catalogue (IKW - The German Cosmetic, Toiletry, Perfumery and Detergent Association).

**Preparation of Egg/Milk Stains on Stainless Steel**

The stainless steel sheets (10 x 15 cm; brushed on one side) used in these experiments were thoroughly washed at 95°C in a laboratory dishwasher with a high-alkalinity commercial detergent to remove grease and clean the sheets. The sheets were polished dry with a cellulose cloth. The surfaces to be brushed were not touched prior to soiling. Also, no water stains or fluff on the surfaces were permitted. Before soiling, the sheets were placed in a thermal cabinet at 80°C, for 30 min. The cooled sheets were weighed before soiling.

The egg yolks and whites of whole raw eggs (3-4 eggs; approximately 160 g/egg) were placed in a bowl and beaten with an egg whisk. Then, 50 ml semi-skimmed milk (1.5% fat, ultra-high-temperature, homogenized) were added to the mixture. The milk and egg were mixed without generating froth. A flat brush was used to uniformly distribute 1.0 ± 0.1 g of the egg/milk mixture on the brushed side of the stainless steel sheets, using a balance to check the distribution. A margin of approximately 1.0 cm was left around the short sides of the sheets. The soiled sheets were dried horizontally (to prevent formation of droplets on the edges of the sheets), at room temperature for 4 hours (max. 24 hr).

The sheets were then immersed for 30 seconds in boiling, demineralized water (using a holding device if necessary). Then the sheets were dried again for 30 min at 80°C. After drying and cooling the sheets were weighed. After weighing the sheets were left to sit for at least 24 hours (20°C, 40-60% relatively humidity) before submitting them to the wash test. In order to meet the testing requirements, only sheets with 190 ± 10 mg egg yolk/milk were used.

After the wash tests were conducted, the sheets were dried for 30 min at 80°C, in the thermal cabinet, and weighed again after cooling. The percentage cleaning performance was determined by dividing the mg of egg/milk released upon washing by the mg of egg/milk applied and multiplying by 100.

**Washing Equipment and Conditions**

The washing tests were performed in an automatic dishwasher (Miele model G690SC), equipped with soiled dishes and stainless steel sheets, prepared as described above. A defined amount of the detergent was used. The temperature tested was 50°C. The water hardness was 21° GH (German hardness). As described above, after washing the plates soiled with minced meat were visually assessed using a photo rating scale of 0 to 10, wherein "0" designated a completely dirty plate and "10" designated a clean plate. These values correspond to the stain or soil removal (SR) capability of the enzyme-containing detergent. The washed stainless steel plates soiled with egg yolk or egg yolk/milk were analyzed gravimetrically to determine the amount of residual stain after washing. The variant proteases were tested at a level of between 0 and 30 mg/active protein per wash.

**G. Eglin C Inhibition Assay**

As described herein, serine protease concentration and specific activity was determined by titration with an inhibitor. Eglin c from the leech *Hirudo medicinalis* is a tight-binding protein inhibitor of subtilisins and ASP protease (Heinz et al., Biochemistry, 31: 8755-66, 1992), and can therefore be used to measure enzyme concentration, which in turn permits specific activity to be calculated. Briefly, one measures the amount of enzyme inhibition produced by several known concentrations of eglin c. From this information, the concentration of eglin c required for complete inhibition is calculated. This is equivalent to the enzyme concentration in the sample.

Protease activity was measured using the chromogenic AAPF assay described above. The gene for eglin c was synthesized and expressed in *E. coli* by standard methods. Its properties and inhibitory potency were the same as eglin c purchased from Sigma. The concentration of an eglin c stock solution was determined by measuring the inhibition of a sample of *Bacillus lentus* subtilisin of known specific activity. Then the calibrated eglin c sample was used to determine the concentration and specific activity of subtilisin variants. These values were used to create normalized 96-well enzyme stock plates, where all of the variants were diluted to a common concentration.

**H. Performance Index**

The performance index compares the performance of the variant (actual value) and the standard or reference protease (theoretical value) at the same protein concentration. In addition, the theoretical values can be calculated, using the parameters of the binding curve (*i.e.*, Langmuir equation) of the standard protease. A performance index (PI) that is greater than 1 (PI>1) identifies a better variant as compared to the standard (*e.g.,* wild-type), while a PI of 1 (PI=1) identifies a variant that performs the same as the standard, and a PI that is less than 1 (PI<1) identifies a variant that performs worse than the standard. Thus, the PI identifies winners, as well as variants that are less desirable for use under certain circumstances.

**EXAMPLE** 2

**Production of BPN' Protease in *B. subtilis***

In this Example, experiments conducted to produce BPN' protease in *B. subtilis,* are described. Transformation of plasmid pHPLT-BPN' into *B. subtilis* was performed as known in the art (See e.g., WO 02/14490, incorporated herein by reference). The DNA sequence (aprE-BPN' hybrid leader, BPN' pro and BPN' mature DNA sequence from *B. amyloliquefaciens)* provided below, encodes the BPN' precursor protein:

GTGAGAAGCAAAAAATTGTGGATCAGTTTGCTGTTTGCTTTAGCGTTAATCTTTACGATGGC
GTTCGGCAGCACATCCTCTGCCCAGGCGGCAGGGAAATCAAACGGGGAAAAGAAATATATT
GTCGGGTTTAAACAGACAATGAGCACGATGAGCGCCGCTAAGAAGAAAGATGTCATTTCTG
AAAAAGGCGGGAAAGTGCAAAAGCAATTCAAATATGTAGACGCAGCTTCAGCTACATTAAA
CGAAAAAGCTGTAAAAGAATTGAAAAAAGACCCGAGCGTCGCTTACGTTGAAGAAGATCAC
GTAGCACATGCGTAC**GCGCAGTCCGTGCCTTACGGCGTATCACAAATTAAAGCCCCTGC**
**TCTGCACTCTCAAGGCTACACTGGATCAAATGTTAAAGTAGCGGTTATCGACAGCGGT**
**ATCGATTCTTCTCATCCTGATTTAAAGGTAGCAGGCGGAGCCAGCATGGTTCCTTCTG**
**AAACAAATCCTTTCCAAGACAACAACTCTCACGGAACTCACGTTGCCGGCACAGTTGC**
**GGCTCTTAATAACTCAATCGGTGTATTAGGCGTTGCGCCAAGCGCATCACTTTACGCT**
**GTAAAAGTTCTCGGTGCTGACGGTTCCGGCCAATACAGCTGGATCATTAACGGAATCG**
**AGTGGGCGATCGCAAACAATATGGACGTTATTAACATGAGCCTCGGCGGACCTTCTGG**
**TTCTGCTGCTTTAAAAGCGGCAGTTGATAAAGCCGTTGCATCCGGCGTCGTAGTCGTT**
**GCGGCAGCCGGTAACGAAGGCACTTCCGGCAGCTCAAGCACAGTGGGCTACCCTGGT**
**AAATACCCTTCTGTCATTGCAGTAGGCGCTGTTGACAGCAGCAACCAAAGAGCATCTT**
**TCTCAAGCGTAGGACCTGAGCTTGATGTCATGGCACCTGGCGTATCTATCCAAAGCAC**
**GCTTCCTGGAAACAAATACGGGGCGTACAACGGTACGTCAATGGCATCTCCGCACGTT**
**GCCGGAGCGGCTGCTTTGATTCTTTCTAAGCACCCGAACTGGACAAACACTCAAGTCC**
**GCAGCAGTTTAGAAAACACCACTACAAAACTTGGTGATTCTTTCTACTATGGAAAAGG**
**GCTGATCAACGTACAGGCGGCAGCTCAGTAA** (SEQ ID NO:1).

In the above sequence, bold indicates the DNA that encodes the mature protease, standard font indicates the leader sequence (aprE-BPN' hybrid leader), and the underlined indicates the pro sequences (BPN'). The amino acid sequence (aprE-BPN' hybrid leader, BPN' pro and BPN' mature DNA sequence) of the BPN' precursor protein is provided below. In this sequence, bold and underlined indicates the mature BPN' protease.

VRSKKLWISLLFALALIFTMAFGSTSSAQAAGKSNGEKKYIVGFKQTMSTMSAAKKKDVISEKG
GKVQKQFKYVDAASATLNEKAVKELKKDPSVAYVEEDHVAHAY**AQSVPYGVSQIKAPALHS**
**QGYTGSNVKVAVIDSGIDSSHPDLKVAGGASMVPSETNPFQDNNSHGTHVAGTVAALNNSI**
**GVLGVAPSASLYAVKVLGADGSGQYSWIINGIEWAIANNMDVINMSLGGPSGSAALKAAVD**
**KAVASGVVVVAAAGNEGTSGSSSTVGYPGKYPSVIAVGAVDSSNQRASFSSVGPELDVMAP**
**GVSIQSTLPGNKYGAYNGTSMASPHVAGAAALILSKHPNWTNTQVRSSLENTTTKLGDSFY**
**YGKGLINVQAAAQ** (SEQ ID NO:2)

**Construction of BPN' expression vector (pHPLT-BPN')**

PCR fragments were obtained using standard conditions with TGO polymerase (Roche Diagnostics) using pJH101term. Plasmid pJH101 corresponds to pJH101 (Ferrari et al., J Bacteriol, 154:1513-5 [1983]) with the following fragment inserted in the EcoRI/BamHI site:

GAATTCCTCCATTTTCTTCTGCTATCAAAATAACAGACTCGTGATTTTCCAAACGAGCTTTCA
AAAAAGCCTCTGCCCCTTGCAAATCGGATGCCTGTCTATAAAATTCCCGATATTGGCTTAAA
CAGCGGCGCAATGGCGGCCGCATCTGATGTCTTTGCTTGGCGAATGTTCATCTTATTTCTTCC
TCCCTCTCAATAATTTTTTCATTCTATCCCTTTTCTGTAAAGTTTATTTTTCAGAATACTTTTA
TCATCATGCTTTGAAAAAATATCACGATAATATCCATTGTTCTCACGGAAGCACACGCAGGT
CATTTGAACGAATTTTTTCGACAGGAATTTGCCGGGACTCAGGAGCATTTAACCTAAAAAAG
CATGACATTTCAGCATAATGAACATTTACTCATGTCTATTTTCGTTCTTTTCTGTATGAAAAT
AGTTATTTCGAGTCTCTACGGAAATAGCGAGAGATGATATACCTAAATAGAGATAAAATCA
TCTCAAAAAAATGGGTCTACTAAAATATTATTCCATCTATTACAATAAATTCACAGAATAGT
CTTTTAAGTAAGTCTACTCTGAATTTTTTTAAAAGGAGAGGGTAAAGAGTGAGAAGCAAAA
AATTGTGGATCAGTTTGCTGTTTGCTTTAGCGTTAATCTTTACGATGGCGTTCGGCAGCACAT
CCTCTGCCCAGGCGGCAGGGAAATCAAACGGGGAAAAGAAATATATTGTCGGGTTTAAACA
GACAATGAGCACGATGAGCGCCGCTAAGAAGAAAGATGTCATTTCTGAAAAAGGCGGGAA
AGTGCAAAAGCAATTCAAATATGTAGACGCAGCTTCAGCTACATTAAACGAAAAAGCTGTA
AAAGAATTGAAAAAAGACCCGAGCGTCGCTTACGTTGAAGAAGATCACGTAGCACATGCGT
ACGCGCAGTCCGTGCCTTACGGCGTATCACAAATTAAAGCCCCTGCTCTGCACTCTCAAGGC
TACACTGGATCAAATGTTAAAGTAGCGGTTATCGACAGCGGTATCGATTCTTCTCATCCTGA
TTTAAAGGTAGCAGGCGGAGCCAGCATGGTTCCTTCTGAAACAAATCCTTTCCAAGACAAC
AACTCTCACGGAACTCACGTTGCCGGCACAGTTGCGGCTCTTAATAACTCAATCGGTGTATT
AGGCGTTGCGCCAAGCGCATCACTTTACGCTGTAAAAGTTCTCGGTGCTGACGGTTCCGGCC
AATACAGCTGGATCATTAACGGAATCGAGTGGGCGATCGCAAACAATATGGACGTTATTAA
CATGAGCCTCGGCGGACCTTCTGGTTCTGCTGCTTTAAAAGCGGCAGTTGATAAAGCCGTTG
CATCCGGCGTCGTAGTCGTTGCGGCAGCCGGTAACGAAGGCACTTCCGGCAGCTCAAGCAC
AGTGGGCTACCCTGGTAAATACCCTTCTGTCATTGCAGTAGGCGCTGTTGACAGCAGCAACC
AAAGAGCATCTTTCTCAAGCGTAGGACCTGAGCTTGATGTCATGGCACCTGGCGTATCTATC
CAAAGCACGCTTCCTGGAAACAAATACGGGGCGTACAACGGTACGTCAATGGCATCTCCGC
ACGTTGCCGGAGCGGCTGCTTTGATTCTTTCTAAGCACCCGAACTGGACAAACACTCAAGTC
CGCAGCAGTTTAGAAAACACCACTACAAAACTTGGTGATTCTTTCTACTATGGAAAAGGGCT
GATCAACGTACAGGCGGCAGCTCAGTAAAACATAAAAAACCGGCCTTGGCCCCGCCGGTTT
TTTATTATTTTTCTTCCTCCGCATGTTCAATCCGCTCCATAATCGACGGATGGCTCCCTCTGA
AAATTTTAACGAGAAACGGCGGGTTGACCCGGCTCAGTCCCGTAACGGCCAAGTCCTGAAA
CGTCTCAATCGCCGCTTCCCGGTTTCCGGTCAGCTCAATGCCGTAACGGTCGGCGGCGTTTT
CCTGATACCGGGAGACGGCATTCGTAATCGGATCC (SEQ ID NO:3).

This fragment contains the BPN' gene with a fusion signal sequence (containing the first eight amino acids of the aprE signal sequence of *B. subtilis* followed by the BPN' signal sequence of *B. amyloliquefaciens* starting at the 9th amino acid). This fragment was used as a template for a PCR reaction employing the following primers: AK04-14: GtcctctgttaacTTACTGAGCTGCCGCCTGTAC (SEQ ID NO:4) annealing to the 3' end of the BPN' mature gene, introducing a HpaI site downstream of the translational stop codon; and AK04-21.1: TTATGCGAGgctagcaaaaggagagggtaaagagtgagaagc (SEQ ID NO:5) annealing to the 5' end of the BPN' signal sequence introducing a NheI site at the 5' end. [01] The PCR fragment obtained from this reaction was cleaned over a Qiagen PCR cleaning kit using standard conditions. After digesting the cleaned fragment with the restriction enzymes NheI and HpaI under standard conditions, it was ligated into a NheI/HpaI digested vector containing the LAT promoter (pHPLT-VAAc1, described inUS2006/0014265 and See FIG. 3A).
Subsequently, the pHPLT-BPN' ligation mixture was transformed into *B. subtilis* (ΔaprE, ΔnprE, oppA, ΔspoIIE, degUHy32, ΔamyE::(xylR,pxylA-comK) as described in WO 02/14490, incorporated herein by reference. Selective growth of *B. subtilis* transformants harboring the pHPLT-BPN' vector (See, FIG. 3B) was performed in shake flasks containing 25 ml MBD medium (a MOPS based defined medium), with 20 mg/L neomycin. Incubation of the transformed cells resulted in the production of secreted BPN' protease having proteolytic activity. Gel analysis was performed using NuPage Novex 10% Bis-Tris gels (Invitrogen®, Catalog No. NP0301BOX). To prepare samples for analysis, 2 volumes of supernatant were mixed with 1 volume 1M HCl, 1 volume 4X LDS sample buffer (Invitrogen®, Catalog No. NP0007), and 1% PMSF (20 mg/ml), and subsequently heated for 10 minutes at 70°C. Then, 25 μL of each sample was loaded onto the gel, together with 10 μL of SeeBlue plus 2 pre-stained protein standards (Invitrogen®, Catalog No.LC5925). The results clearly demonstrated that the BPN' cloning strategy described in this Example yielded active BPN' produced by *B. subtilis.*

## EXAMPLE 3

### Generation of BPN' Site Evaluation Libraries and Multiple Mutation Libraries

In this Example, the construction of BPN' variants is described.

### BPN' Site Evaluation Library (SEL) Construction

The pHPLT-BPN' vector containing the BPN' expression cassette served as template DNA. This vector contains a unique *Bgl*II restriction site, which was utilized in SEL construction. Primers synthesized by Invitrogen® (desalted, 50 nmol scale) and listed in Table 7-1, were used to generate the libraries.

To construct BPN' SELs, three PCR amplifications were performed: two mutagenesis PCRs to introduce the mutated codon of interest in the mature BPN' DNA sequence and a third PCR to fuse the two mutagenesis PCRs in order to construct the pHPLT-BPN' expression vector having the desired mutated codon in the mature BPN' sequence. The method of mutagenesis was based on the codon-specific mutation approach. In this method, the creation of all possible mutations at a time in a specific DNA triplet is performed using forward and reverse oligonucleotide primers with a length of 25 to 45 nucleotides enclosing a specific designed triple DNA sequence (NNS with N = A, C, T or G, and S = C or G) that corresponds with the sequence of the codon to be mutated. This guarantees random incorporation of nucleotides at that specific BPN' mature codon. The number listed in the primer name (Table 3-1) corresponds with the specific BPN' mature codon position. Two additional primers that were used to construct the SELs contained a *Bgl*II restriction site together with a portion of the pHPLT-BPN' DNA sequence flanking the *Bgl*II restriction site.

Construction of each SEL began with two primary PCR amplifications using the pHPLT-BglII-FW primer and a specific BPN' reverse mutagenesis primer, and for the second PCR amplification the pHPLT-BglII-RV primer and a specific BPN' forward mutagenesis primer (equal BPN' mature codon positions for the forward and reverse mutagenesis primers). The introduction of the mutations in the mature BPN' sequence was performed using Finnzymes Phusion High-Fidelity DNA Polymerase (Catalog No. F-530L). All PCR amplifications were executed according to Finnzymes protocol supplied with the polymerase. The PCR conditions were as follows:
For primary PCR 1:
pHPLT-BglII-FW primer and a specific BPN' reverse mutagenesis primer-both 1 μL (10 μM); and for primary PCR 2: pHPLT-BglII-RV primer and a specific BPN' forward mutagenesis primer - both 1 μL (10 μM); in

| | |
|---|---|
| 5X Phusion HF buffer | 10 μL |
| 10 mM dNTP mixture | 1 μL |
| Phusion DNA polymerase | 0.75 μL (2 units/ μL) |
| DMSO, 100% | 1 μL |
| pHPLT-BPN' template DNA | 1 μL (0.1 - 1 ng/ μL) |
| Distilled, autoclaved water | up to 50 μL |

PCR was completed using a MJ Research (Location) PTC-200 Peltier thermal cycler with the following PCR program: 30 seconds 98°C, 30X (10 seconds 98°C, 20 seconds 55°C, 1 minute 72°C) and 5 min 72°C. The reactions resulted in two fragments of approximately 2 to 3 kB in length, which had about 30 nucleotide base overlap around the BPN' mature codon of interest. Fragments were fused in a third reaction using the two aforementioned fragments and the forward and reverse BglII primers. The fusion PCR reaction was carried out as follows:
pHPLT-BglII-FW primer and pHPLT-BglII-RV primer-both 1 μL (10 μM)

| | |
|---|---|
| 5X Phusion HF buffer | 10 μL |
| 10 mM dNTP mixture | 1 μL |
| Phusion DNA polymares | 0.75 μL (2 units/ μL) |
| DMSO, 100% | 1 μL |
| primary PCR 1 reaction mix | 1 μL |
| primary PCR 2 reaction mix | 1 μL |
| Distilled, autoclaved water | up to 50 μL |

The PCR fusion program was as follows: 30 seconds 98°C, 30X (10 seconds 98°C, 20 seconds 55°C, 2:05 minute 72°C) and 5 min 72°C using a MJ Research® PTC-200 Peltier thermal cycler.

The amplified linear 4.8 Kb fragment was purified using the Qiagen® Qiaquick PCR purification kit (Catalog No. 28106) and digested with the BglII restriction enzyme to create cohesive ends on both sides of the fusion fragment. The digestion reactions contained:

- 35 μL purified linear DNA fragment
- 4 μL React® 3 buffer (Invitrogen®)
- 1 μL BglII, 10 units/ml (Invitrogen®)

Reaction conditions: 1 hour, 30°C.

Ligation of the BglII digested and purified fragment yielded circular and multimeric DNA products containing the desired mutation, which were subsequently directly transformed into competent *Bacillus subtilis:*

- 30 μL of purified BglII digested DNA fragment
- 8 μL T4 DNA Ligase buffer (Invitrogen® Catalog No. 46300-018)
- 1 μL T4 DNA Ligase, 1 unit/μL (Invitrogen® Catalog No. 15224-017)

Reaction conditions: 16-20 hours, 16°C

The ligation mixture was used to transform *B. subtilis* (ΔaprE, ΔnprE, oppA, ΔspoIIE, degUHy32, ΔamyE::(xylR, pxylA-comK) as described in WO 02/14490, incorporated herein by reference. For each library, 96 single colonies were picked and grown in MOPS media containing neomycin and 1.25 g/L yeast extract, for sequence analysis (BaseClear) and screening purposes. The library numbers ranged from 1 to 275. Each number represents the codon of the mature bpn' sequence that was randomly mutated. Each library contained a maximum of 19 BPN' variants.

**Table 3-1 BPN' Primers**

| Primer Name | Oligonucleotide Sequence | SEQ ID NO: |
|---|---|---|
| **pHPLT-BPN *Bgl*II containing primers** | | |
| pHPLT-BgLII-FW | GCAATCAGATCTTCCTTCAGGTTATGACC | 6 |
| pHPLT-BglII-RV | GCATCGAAGATCTGATTGCTTAACTGCTTC | 7 |
| **BPN' Mutagenesis Primers (F = Forward and R = Reverse)** | | |
| BPN1F | CACGTAGCACATGCATACNNSCAGTCCGTGCCTTACGGC | 8 |
| BPN2F | GTAGCACATGCATACGCGNNSTCCGTGCCTTACGGCGTA | 9 |
| BPN3F | GCACATGCATACGCGCAGNNSGTGCCTTACGGCGTATCA | 10 |
| BPN4F | CATGCATACGCGCAGTCCNNSCCTTACGGCGTATCACAA | 11 |
| BPN5F | GCATACGCGCAGTCCGTGNNSTACGGCGTATCACAAATT | 12 |
| BPN6F | TACGCGCAGTCCGTGCCTNNSGGCGTATCACAAATTAAA | 13 |
| BPN7F | GCGCAGTCCGTGCCTTACNNSGTATCACAAATTAAAGCC | 14 |
| BPN8F | CAGTCCGTGCCTTACGGCNNSTCACAAATTAAAGCCCCT | 15 |
| BPN9F | TCCGTGCCTTACGGCGTANNSCAAATTAAAGCCCCTGCT | 16 |
| BPN10F | GTGCCTTACGGCGTATCANNSATTAAAGCCCCTGCTCTG | 17 |
| BPN11F | CCTTACGGCGTATCACAANNSAAAGCCCCTGCTCTGCAC | 18 |
| BPN12F | TACGGCGTATCACAAATTNNSGCCCCTGCTCTGCACTCT | 19 |
| BPN13F | GGCGTATCACAAATTAAANNSCCTGCTCTGCACTCTCAA | 20 |
| BPN14F | GTATCACAAATTAAAGCCNNSGCTCTGCACTCTCAAGGC | 21 |
| BPN15F | TCACAAATTAAAGCCCCTNNSCTGCACTCTCAAGGCTAC | 22 |
| BPN16F | CAAATTAAAGCCCCTGCTNNSCACTCTCAAGGCTACACT | 23 |
| BPN17F | ATTAAAGCCCCTGCTCTGNNSTCTCAAGGCTACACTGGA | 24 |
| BPN18F | AAAGCCCCTGCTCTGCACNNSCAAGGCTACACTGGATCA | 25 |

(continued)

| Table 3-1 BPN' Primers | | |
|---|---|---|
| **Primer Name** | **Oligonucleotide Sequence** | **SEQ ID NO:** |
| BPN19F | GCCCCTGCTCTGCACTCTNNSGGCTACACTGGATCAAAT | 26 |
| BPN20F | CCTGCTCTGCACTCTCAANNSTACACTGGATCAAATGTT | 27 |
| BPN21F | GCTCTGCACTCTCAAGGCNNSACTGGATCAAATGTTAAA | 28 |
| BPN22F | CTGCACTCTCAAGGCTACNNSGGATCAAATGTTAAAGTA | 29 |
| BPN23F | CACTCTCAAGGCTACACTNNSTCAAATGTTAAAGTAGCG | 30 |
| BPN24F | TCTCAAGGCTACACTGGANNSAATGTTAAAGTAGCGGTT | 31 |
| BPN25F | CAAGGCTACACTGGATCANNSGTTAAAGTAGCGGTTATC | 32 |
| BPN26F | GGCTACACTGGATCAAATNNSAAAGTAGCGGTTATCGAC | 33 |
| BPN27F | TACACTGGATCAAATGTTNNSGTAGCGGTTATCGACAGC | 34 |
| BPN28F | ACTGGATCAAATGTTAAANNSGCGGTTATCGACAGCGGT | 35 |
| BPN29F | GGATCAAATGTTAAAGTANNSGTTATCGACAGCGGTATC | 36 |
| BPN30F | TCAAATGTTAAAGTAGCGNNSATCGACAGCGGTATCGAT | 37 |
| BPN31F | AATGTTAAAGTAGCGGTTNNSGACAGCGGTATCGATTCT | 38 |
| BPN32F | GTTAAAGTAGCGGTTATCNNSAGCGGTATCGATTCTTCT | 39 |
| BPN33F | AAAGTAGCGGTTATCGACNNSGGTATCGATTCTTCTCAT | 40 |
| BPN34F | GTAGCGGTTATCGACAGCNNSATCGATTCTTCTCATCCT | 41 |
| BPN35F | GCGGTTATCGACAGCGGTNNSGATTCTTCTCATCCTGAT | 42 |
| BPN36F | GTTATCGACAGCGGTATCNNSTCTTCTCATCCTGATTTA | 43 |
| BPN37F | ATCGACAGCGGTATCGATNNSTCTCATCCTGATTTAAAG | 44 |
| BPN38F | GACAGCGGTATCGATTCTNNSCATCCTGATTTAAAGGTA | 45 |
| BPN39F | AGCGGTATCGATTCTTCTNNSCCTGATTTAAAGGTAGCA | 46 |
| BPN40F | GGTATCGATTCTTCTCATNNSGATTTAAAGGTAGCAGGC | 47 |
| BPN41F | ATCGATTCTTCTCATCCTNNSTTAAAGGTAGCAGGCGGA | 48 |
| BPN42F | GATTCTTCTCATCCTGATNNSAAGGTAGCAGGCGGAGCC | 49 |
| BPN43F | TCTTCTCATCCTGATTTANNSGTAGCAGGCGGAGCCAGC | 50 |
| BPN44F | TCTCATCCTGATTTAAAGNNSGCAGGCGGAGCCAGCATG | 51 |
| BPN45F | CATCCTGATTTAAAGGTANNSGGCGGAGCCAGCATGGTT | 52 |
| BPN46F | CCTGATTTAAAGGTAGCANNSGGAGCCAGCATGGTTCCT | 53 |
| BPN47F | GATTTAAAGGTAGCAGGCNNSGCCAGCATGGTTCCTTCT | 54 |
| BPN48F | TTAAAGGTAGCAGGCGGANNSAGCATGGTTCCTTCTGAA | 55 |
| BPN49F | AAGGTAGCAGGCGGAGCCNNSATGGTTCCTTCTGAAACA | 56 |
| BPN50F | GTAGCAGGCGGAGCCAGCNNSGTTCCTTCTGAAACAAAT | 57 |
| BPN51F | GCAGGCGGAGCCAGCATGNNSCCTTCTGAAACAAATCCT | 58 |
| BPN52F | GGCGGAGCCAGCATGGTTNNSTCTGAAACAAATCCTTTC | 59 |
| BPN53F | GGAGCCAGCATGGTTCCTNNSGAAACAAATCCTTTCCAA | 60 |
| BPN54F | GCCAGCATGGTTCCTTCTNNSACAAATCCTTTCCAAGAC | 61 |
| BPN55F | AGCATGGTTCCTTCTGAANNSAATCCTTTCCAAGACAAC | 62 |

(continued)

| Table 3-1 BPN' Primers | | |
|---|---|---|
| **Primer Name** | **Oligonucleotide Sequence** | **SEQ ID NO:** |
| BPN56F | ATGGTTCCTTCTGAAACANNSCCTTTCCAAGACAACAAC | 63 |
| BPN57F | GTTCCTTCTGAAACAAATNNSTTCCAAGACAACAACTCT | 64 |
| BPN58F | CCTTCTGAAACAAATCCTNNSCAAGACAACAACTCTCAC | 65 |
| BPN59F | TCTGAAACAAATCCTTTCNNSGACAACAACTCTCACGGA | 66 |
| BPN60F | GAAACAAATCCTTTCCAANNSAACAACTCTCACGGAACT | 67 |
| BPN61F | ACAAATCCTTTCCAAGACNNSAACTCTCACGGAACTCAC | 68 |
| BPN62F | AATCCTTTCCAAGACAACNNSTCTCACGGAACTCACGTT | 69 |
| BPN63F | CCTTTCCAAGACAACAACNNSCACGGAACTCACGTTGCC | 70 |
| BPN64F | TTCCAAGACAACAACTCTNNSGGAACTCACGTTGCCGGC | 71 |
| BPN65F | CAAGACAACAACTCTCACNNSACTCACGTTGCCGGCACA | 72 |
| BPN66F | GACAACAACTCTCACGGANNSCACGTTGCCGGCACAGTT | 73 |
| BPN67F | AACAACTCTCACGGAACTNNSGTTGCCGGCACAGTTGCG | 74 |
| BPN68F | AACTCTCACGGAACTCACNNSGCCGGCACAGTTGCGGCT | 75 |
| BPN69F | TCTCACGGAACTCACGTTNNSGGCACAGTTGCGGCTCTT | 76 |
| BPN70F | CACGGAACTCACGTTGCCNNSACAGTTGCGGCTCTTAAT | 77 |
| BPN71F | GGAACTCACGTTGCCGGCNNSGTTGCGGCTCTTAATAAC | 78 |
| BPN72F | ACTCACGTTGCCGGCACANNSGCGGCTCTTAATAACTCA | 79 |
| BPN73F | CACGTTGCCGGCACAGTTNNSGCTCTTAATAACTCAATC | 80 |
| BPN74F | GTTGCCGGCACAGTTGCGNNSCTTAATAACTCAATCGGT | 81 |
| BPN75F | GCCGGCACAGTTGCGGCTNNSAATAACTCAATCGGTGTA | 82 |
| BPN76F | GGCACAGTTGCGGCTCTTNNSAACTCAATCGGTGTATTA | 83 |
| BPN77F | ACAGTTGCGGCTCTTAATNNSTCAATCGGTGTATTAGGC | 84 |
| BPN78F | GTTGCGGCTCTTAATAACNNSATCGGTGTATTAGGCGTT | 85 |
| BPN79F | GCGGCTCTTAATAACTCANNSGGTGTATTAGGCGTTGCG | 86 |
| BPN80F | GCTCTTAATAACTCAATCNNSGTATTAGGCGTTGCGCCA | 87 |
| BPN81F | CTTAATAACTCAATCGGTNNSTTAGGCGTTGCGCCAAGC | 88 |
| BPN82F | AATAACTCAATCGGTGTANNSGGCGTTGCGCCAAGCGCA | 89 |
| BPN83F | AACTCAATCGGTGTATTANNSGTTGCGCCAAGCGCATCA | 90 |
| BPN84F | TCAATCGGTGTATTAGGCNNSGCGCCAAGCGCATCACTT | 91 |
| BPN85F | ATCGGTGTATTAGGCGTTNNSCCAAGCGCATCACTTTAC | 92 |
| BPN86F | GGTGTATTAGGCGTTGCGNNSAGCGCATCACTTTACGCT | 93 |
| BPN87F | GTATTAGGCGTTGCGCCANNSGCATCACTTTACGCTGTA | 94 |
| BPN88F | TTAGGCGTTGCGCCAAGCNNSTCACTTTACGCTGTAAAA | 95 |
| BPN89F | GGCGTTGCGCCAAGCGCANNSCTTTACGCTGTAAAAGTT | 96 |
| BPN90F | GTTGCGCCAAGCGCATCANNSTACGCTGTAAAAGTTCTC | 97 |
| BPN91F | GCGCCAAGCGCATCACTTNNSGCTGTAAAAGTTCTCGGT | 98 |
| BPN92F | CCAAGCGCATCACTTTACNNSGTAAAAGTTCTCGGTGCT | 99 |

(continued)

| Table 3-1 BPN' Primers | | |
|---|---|---|
| Primer Name | Oligonucleotide Sequence | SEQ ID NO: |
| BPN93F | AGCGCATCACTTTACGCTNNSAAAGTTCTCGGTGCTGAC | 100 |
| BPN94F | GCATCACTTTACGCTGTANNSGTTCTCGGTGCTGACGGT | 101 |
| BPN95F | TCACTTTACGCTGTAAAANNSCTCGGTGCTGACGGTTCC | 102 |
| BPN96F | CTTTACGCTGTAAAAGTTNNSGGTGCTGACGGTTCCGGC | 103 |
| BPN97F | TACGCTGTAAAAGTTCTCNNSGCTGACGGTTCCGGCCAA | 104 |
| BPN98F | GCTGTAAAAGTTCTCGGTNNSGACGGTTCCGGCCAATAC | 105 |
| BPN99F | GTAAAAGTTCTCGGTGCTNNSGGTTCCGGCCAATACAGC | 106 |
| BPN100F | AAAGTTCTCGGTGCTGACNNSTCCGGCCAATACAGCTGG | 107 |
| BPN101F | GTTCTCGGTGCTGACGGTNNSGGCCAATACAGCTGGATC | 108 |
| BPN102F | CTCGGTGCTGACGGTTCCNNSCAATACAGCTGGATCATT | 109 |
| BPN103F | GGTGCTGACGGTTCCGGCNNSTACAGCTGGATCATTAAC | 110 |
| BPN104F | GCTGACGGTTCCGGCCAANNSAGCTGGATCATTAACGGA | 111 |
| BPN105F | GACGGTTCCGGCCAATACNNSTGGATCATTAACGGAATC | 112 |
| BPN106F | GGTTCCGGCCAATACAGCNNSATCATTAACGGAATCGAG | 113 |
| BPN107F | TCCGGCCAATACAGCTGGNNSATTAACGGAATCGAGTGG | 114 |
| BPN108F | GGCCAATACAGCTGGATCNNSAACGGAATCGAGTGGGCG | 115 |
| BPN109F | CAATACAGCTGGATCATTNNSGGAATCGAGTGGGCGATC | 116 |
| BPN110F | TACAGCTGGATCATTAACNNSATCGAGTGGGCGATCGCA | 117 |
| BPN111F | AGCTGGATCATTAACGGANNSGAGTGGGCGATCGCAAAC | 118 |
| BPN112F | TGGATCATTAACGGAATCNNSTGGGCGATCGCAAACAAT | 119 |
| BPN113F | ATCATTAACGGAATCGAGNNSGCGATCGCAAACAATATG | 120 |
| BPN114F | ATTAACGGAATCGAGTGGNNSATCGCAAACAATATGGAC | 121 |
| BPN115F | AACGGAATCGAGTGGGCGNNSGCAAACAATATGGACGTT | 122 |
| BPN116F | GGAATCGAGTGGGCGATCNNSAACAATATGGACGTTATT | 123 |
| BPN117F | ATCGAGTGGGCGATCGCANNSAATATGGACGTTATTAAC | 124 |
| BPN118F | GAGTGGGCGATCGCAAACNNSATGGACGTTATTAACATG | 125 |
| BPN119F | TGGGCGATCGCAAACAATNNSGACGTTATTAACATGAGC | 126 |
| BPN120F | GCGATCGCAAACAATATGNNSGTTATTAACATGAGCCTC | 127 |
| BPN121F | ATCGCAAACAATATGGACNNSATTAACATGAGCCTCGGC | 128 |
| BPN122F | GCAAACAATATGGACGTTNNSAACATGAGCCTCGGCGGA | 129 |
| BPN123F | AACAATATGGACGTTATTNNSATGAGCCTCGGCGGACCT | 130 |
| BPN124F | AATATGGACGTTATTAACNNSAGCCTCGGCGGACCTTCT | 131 |
| BPN125F | ATGGACGTTATTAACATGNNSCTCGGCGGACCTTCTGGT | 132 |
| BPN126F | GACGTTATTAACATGAGCNNSGGCGGACCTTCTGGTTCT | 133 |
| BPN127F | GTTATTAACATGAGCCTCNNSGGACCTTCTGGTTCTGCT | 134 |
| BPN128F | ATTAACATGAGCCTCGGCNNSCCTTCTGGTTCTGCTGCT | 135 |
| BPN129F | AACATGAGCCTCGGCGGANNSTCTGGTTCTGCTGCTTTA | 136 |

(continued)

| Table 3-1 BPN' Primers | | |
|---|---|---|
| **Primer Name** | **Oligonucleotide Sequence** | **SEQ ID NO:** |
| BPN130F | ATGAGCCTCGGCGGACCTNNSGGTTCTGCTGCTTTAAAA | 137 |
| BPN131F | AGCCTCGGCGGACCTTCTNNSTCTGCTGCTTTAAAAGCG | 138 |
| BPN132F | CTCGGCGGACCTTCTGGTNNSGCTGCTTTAAAAGCGGCA | 139 |
| BPN133F | GGCGGACCTTCTGGTTCTNNSGCTTTAAAAGCGGCAGTT | 140 |
| BPN134F | GGACCTTCTGGTTCTGCTNNSTTAAAAGCGGCAGTTGAT | 141 |
| BPN135F | CCTTCTGGTTCTGCTGCTNNSAAAGCGGCAGTTGATAAA | 142 |
| BPN136F | TCTGGTTCTGCTGCTTTANNSGCGGCAGTTGATAAAGCC | 143 |
| BPN137F | GGTTCTGCTGCTTTAAAANNSGCAGTTGATAAAGCCGTT | 144 |
| BPN138F | TCTGCTGCTTTAAAAGCGNNSGTTGATAAAGCCGTTGCA | 145 |
| BPN139F | GCTGCTTTAAAAGCGGCANNSGATAAAGCCGTTGCATCC | 146 |
| BPN140F | GCTTTAAAAGCGGCAGTTNNSAAAGCCGTTGCATCCGGC | 147 |
| BPN141F | TTAAAAGCGGCAGTTGATNNSGCCGTTGCATCCGGCGTC | 148 |
| BPN142F | AAAGCGGCAGTTGATAAANNSGTTGCATCCGGCGTCGTA | 149 |
| BPN143F | GCGGCAGTTGATAAAGCCNNSGCATCCGGCGTCGTAGTC | 150 |
| BPN144F | GCAGTTGATAAAGCCGTTNNSTCCGGCGTCGTAGTCGTT | 151 |
| BPN145F | GTTGATAAAGCCGTTGCANNSGGCGTCGTAGTCGTTGCG | 152 |
| BPN146F | GATAAAGCCGTTGCATCCNNSGTCGTAGTCGTTGCGGCA | 153 |
| BPN147F | AAAGCCGTTGCATCCGGCNNSGTAGTCGTTGCGGCAGCC | 154 |
| BPN148F | GCCGTTGCATCCGGCGTCNNSGTCGTTGCGGCAGCCGGT | 155 |
| BPN149F | GTTGCATCCGGCGTCGTANNSGTTGCGGCAGCCGGTAAC | 156 |
| BPN150F | GCATCCGGCGTCGTAGTCNNSGCGGCAGCCGGTAACGAA | 157 |
| BPN151F | TCCGGCGTCGTAGTCGTTNNSGCAGCCGGTAACGAAGGC | 158 |
| BPN152F | GGCGTCGTAGTCGTTGCGNNSGCCGGTAACGAAGGCACT | 159 |
| BPN153F | GTCGTAGTCGTTGCGGCANNSGGTAACGAAGGCACTTCC | 160 |
| BPN154F | GTAGTCGTTGCGGCAGCCNNSAACGAAGGCACTTCCGGC | 161 |
| BPN155F | GTCGTTGCGGCAGCCGGTNNSGAAGGCACTTCCGGCAGC | 162 |
| BPN156F | GTTGCGGCAGCCGGTAACNNSGGCACTTCCGGCAGCTCA | 163 |
| BPN157F | GCGGCAGCCGGTAACGAANNSACTTCCGGCAGCTCAAGC | 164 |
| BPN158F | GCAGCCGGTAACGAAGGCNNSTCCGGCAGCTCAAGCACA | 165 |
| BPN159F | GCCGGTAACGAAGGCACTNNSGGCAGCTCAAGCACAGTG | 166 |
| BPN160F | GGTAACGAAGGCACTTCCNNSAGCTCAAGCACAGTGGGC | 167 |
| BPN161F | AACGAAGGCACTTCCGGCNNSTCAAGCACAGTGGGCTAC | 168 |
| BPN162F | GAAGGCACTTCCGGCAGCNNSAGCACAGTGGGCTACCCT | 169 |
| BPN163F | GGCACTTCCGGCAGCTCANNSACAGTGGGCTACCCTGGT | 170 |
| BPN164F | ACTTCCGGCAGCTCAAGCNNSGTGGGCTACCCTGGTAAA | 171 |
| BPN165F | TCCGGCAGCTCAAGCACANNSGGCTACCCTGGTAAATAC | 172 |
| BPN166F | GGCAGCTCAAGCACAGTGNNSTACCCTGGTAAATACCCT | 173 |

(continued)

| Table 3-1 BPN' Primers | | |
|---|---|---|
| **Primer Name** | **Oligonucleotide Sequence** | **SEQ ID NO:** |
| BPN167F | AGCTCAAGCACAGTGGGCNNSCCTGGTAAATACCCTTCT | 174 |
| BPN168F | TCAAGCACAGTGGGCTACNNSGGTAAATACCCTTCTGTC | 175 |
| BPN169F | AGCACAGTGGGCTACCCTNNSAAATACCCTTCTGTCATT | 176 |
| BPN170F | ACAGTGGGCTACCCTGGTNNSTACCCTTCTGTCATTGCA | 177 |
| BPN171F | GTGGGCTACCCTGGTAAANNSCCTTCTGTCATTGCAGTA | 178 |
| BPN172F | GGCTACCCTGGTAAATACNNSTCTGTCATTGCAGTAGGC | 179 |
| BPN173F | TACCCTGGTAAATACCCTNNSGTCATTGCAGTAGGCGCT | 180 |
| BPN174F | CCTGGTAAATACCCTTCTNNSATTGCAGTAGGCGCTGTT | 181 |
| BPN175F | GGTAAATACCCTTCTGTCNNSGCAGTAGGCGCTGTTGAC | 182 |
| BPN176F | AAATACCCTTCTGTCATTNNSGTAGGCGCTGTTGACAGC | 183 |
| BPN177F | TACCCTTCTGTCATTGCANNSGGCGCTGTTGACAGCAGC | 184 |
| BPN178F | CCTTCTGTCATTGCAGTANNSGCTGTTGACAGCAGCAAC | 185 |
| BPN179F | TCTGTCATTGCAGTAGGCNNSGTTGACAGCAGCAACCAA | 186 |
| BPN180F | GTCATTGCAGTAGGCGCTNNSGACAGCAGCAACCAAAGA | 187 |
| BPN181F | ATTGCAGTAGGCGCTGTTNNSAGCAGCAACCAAAGAGCA | 188 |
| BPN182F | GCAGTAGGCGCTGTTGACNNSAGCAACCAAAGAGCATCT | 189 |
| BPN183F | GTAGGCGCTGTTGACAGCNNSAACCAAAGAGCATCTTTC | 190 |
| BPN184F | GGCGCTGTTGACAGCAGCNNSCAAAGAGCATCTTTCTCA | 191 |
| BPN185F | GCTGTTGACAGCAGCAACNNSAGAGCATCTTTCTCAAGC | 192 |
| BPN186F | GTTGACAGCAGCAACCAANNSGCATCTTTCTCAAGCGTA | 193 |
| BPN187F | GACAGCAGCAACCAAAGANNSTCTTTCTCAAGCGTAGGA | 194 |
| BPN188F | AGCAGCAACCAAAGAGCANNSTTCTCAAGCGTAGGACCT | 195 |
| BPN189F | AGCAACCAAAGAGCATCTNNSTCAAGCGTAGGACCTGAG | 196 |
| BPN190F | AACCAAAGAGCATCTTTCNNSAGCGTAGGACCTGAGCTT | 197 |
| BPN191F | CAAAGAGCATCTTTCTCANNSGTAGGACCTGAGCTTGAT | 198 |
| BPN192F | AGAGCATCTTTCTCAAGCNNSGGACCTGAGCTTGATGTC | 199 |
| BPN193F | GCATCTTTCTCAAGCGTANNSCCTGAGCTTGATGTCATG | 200 |
| BPN194F | TCTTTCTCAAGCGTAGGANNSGAGCTTGATGTCATGGCA | 201 |
| BPN195F | TTCTCAAGCGTAGGACCTNNSCTTGATGTCATGGCACCT | 202 |
| BPN196F | TCAAGCGTAGGACCTGAGNNSGATGTCATGGCACCTGGC | 203 |
| BPN197F | AGCGTAGGACCTGAGCTTNNSGTCATGGCACCTGGCGTA | 204 |
| BPN198F | GTAGGACCTGAGCTTGATNNSATGGCACCTGGCGTATCT | 205 |
| BPN199F | GGACCTGAGCTTGATGTCNNSGCACCTGGCGTATCTATC | 206 |
| BPN200F | CCTGAGCTTGATGTCATGNNSCCTGGCGTATCTATCCAA | 207 |
| BPN201F | GAGCTTGATGTCATGGCANNSGGCGTATCTATCCAAAGC | 208 |
| BPN202F | CTTGATGTCATGGCACCTNNSGTATCTATCCAAAGCACG | 209 |
| BPN203F | GATGTCATGGCACCTGGCNNSTCTATCCAAAGCACGCTT | 210 |

(continued)

| Table 3-1 BPN' Primers | | |
|---|---|---|
| **Primer Name** | **Oligonucleotide Sequence** | **SEQ ID NO:** |
| BPN204F | GTCATGGCACCTGGCGTANNSATCCAAAGCACGCTTCCT | 211 |
| BPN205F | ATGGCACCTGGCGTATCTNNSCAAAGCACGCTTCCTGGA | 212 |
| BPN206F | GCACCTGGCGTATCTATCNNSAGCACGCTTCCTGGAAAC | 213 |
| BPN207F | CCTGGCGTATCTATCCAANNSACGCTTCCTGGAAACAAA | 214 |
| BPN208F | GGCGTATCTATCCAAAGCNNSCTTCCTGGAAACAAATAC | 215 |
| BPN209F | GTATCTATCCAAAGCACGNNSCCTGGAAACAAATACGGG | 216 |
| BPN210F | TCTATCCAAAGCACGCTTNNSGGAAACAAATACGGGGCG | 217 |
| BPN211F | ATCCAAAGCACGCTTCCTNNSAACAAATACGGGGCGTAC | 218 |
| BPN212F | CAAAGCACGCTTCCTGGANNSAAATACGGGGCGTACAAC | 219 |
| BPN213F | AGCACGCTTCCTGGAAACNNSTACGGGGCGTACAACGGT | 220 |
| BPN214F | ACGCTTCCTGGAAACAAANNSGGGGCGTACAACGGTACG | 221 |
| BPN215F | CTTCCTGGAAACAAATACNNSGCGTACAACGGTACGTCA | 222 |
| BPN216F | CCTGGAAACAAATACGGGNNSTACAACGGTACGTCAATG | 223 |
| BPN217F | GGAAACAAATACGGGGCGNNSAACGGTACGTCAATGGCA | 224 |
| BPN218F | AACAAATACGGGGCGTACNNSGGTACGTCAATGGCATCT | 225 |
| BPN219F | AAATACGGGGCGTACAACNNSACGTCAATGGCATCTCCG | 226 |
| BPN220F | TACGGGGCGTACAACGGTNNSTCAATGGCATCTCCGCAC | 227 |
| BPN221F | GGGGCGTACAACGGTACGNNSATGGCATCTCCGCACGTT | 228 |
| BPN222F | GCGTACAACGGTACGTCANNSGCATCTCCGCACGTTGCC | 229 |
| BPN223F | TACAACGGTACGTCAATGNNSTCTCCGCACGTTGCCGGA | 230 |
| BPN224F | AACGGTACGTCAATGGCANNSCCGCACGTTGCCGGAGCG | 231 |
| BPN225F | GGTACGTCAATGGCATCTNNSCACGTTGCCGGAGCGGCT | 232 |
| BPN226F | ACGTCAATGGCATCTCCGNNSGTTGCCGGAGCGGCTGCT | 233 |
| BPN227F | TCAATGGCATCTCCGCACNNSGCCGGAGCGGCTGCTTTG | 234 |
| BPN228F | ATGGCATCTCCGCACGTTNNSGGAGCGGCTGCTTTGATT | 235 |
| BPN229F | GCATCTCCGCACGTTGCCNNSGCGGCTGCTTTGATTCTT | 236 |
| BPN230F | TCTCCGCACGTTGCCGGANNSGCTGCTTTGATTCTTTCT | 237 |
| BPN231F | CCGCACGTTGCCGGAGCGNNSGCTTTGATTCTTTCTAAG | 238 |
| BPN232F | CACGTTGCCGGAGCGGCTNNSTTGATTCTTTCTAAGCAC | 239 |
| BPN233F | GTTGCCGGAGCGGCTGCTNNSATTCTTTCTAAGCACCCG | 240 |
| BPN234F | GCCGGAGCGGCTGCTTTGNNSCTTTCTAAGCACCCGAAC | 241 |
| BPN235F | GGAGCGGCTGCTTTGATTNNSTCTAAGCACCCGAACTGG | 242 |
| BPN236F | GCGGCTGCTTTGATTCTTNNSAAGCACCCGAACTGGACA | 243 |
| BPN237F | GCTGCTTTGATTCTTTCTNNSCACCCGAACTGGACAAAC | 244 |
| BPN238F | GCTTTGATTCTTTCTAAGNNSCCGAACTGGACAAACACT | 245 |
| BPN239F | TTGATTCTTTCTAAGCACNNSAACTGGACAAACACTCAA | 246 |
| BPN240F | ATTCTTTCTAAGCACCCGNNSTGGACAAACACTCAAGTC | 247 |

(continued)

| Table 3-1 BPN' Primers | | |
|---|---|---|
| **Primer Name** | **Oligonucleotide Sequence** | **SEQ ID NO:** |
| BPN241F | CTTTCTAAGCACCCGAACNNSACAAACACTCAAGTCCGC | 248 |
| BPN242F | TCTAAGCACCCGAACTGGNNSAACACTCAAGTCCGCAGC | 249 |
| BPN243F | AAGCACCCGAACTGGACANNSACTCAAGTCCGCAGCAGT | 250 |
| BPN244F | CACCCGAACTGGACAAACNNSCAAGTCCGCAGCAGTTTA | 251 |
| BPN245F | CCGAACTGGACAAACACTNNSGTCCGCAGCAGTTTAGAA | 252 |
| BPN246F | AACTGGACAAACACTCAANNSCGCAGCAGTTTAGAAAAC | 253 |
| BPN247F | TGGACAAACACTCAAGTCNNSAGCAGTTTAGAAAACACC | 254 |
| BPN248F | ACAAACACTCAAGTCCGCNNSAGTTTAGAAAACACCACT | 255 |
| BPN249F | AACACTCAAGTCCGCAGCNNSTTAGAAAACACCACTACA | 256 |
| BPN250F | ACTCAAGTCCGCAGCAGTNNSGAAAACACCACTACAAAA | 257 |
| BPN251F | CAAGTCCGCAGCAGTTTANNSAACACCACTACAAAACTT | 258 |
| BPN252F | GTCCGCAGCAGTTTAGAANNSACCACTACAAAACTTGGT | 259 |
| BPN253F | CGCAGCAGTTTAGAAAACNNSACTACAAAACTTGGTGAT | 260 |
| BPN254F | AGCAGTTTAGAAAACACCNNSACAAAACTTGGTGATTCT | 261 |
| BPN255F | AGTTTAGAAAACACCACTNNSAAACTTGGTGATTCTTTC | 262 |
| BPN256F | TTAGAAAACACCACTACANNSCTTGGTGATTCTTTCTAC | 263 |
| BPN257F | GAAAACACCACTACAAAANNSGGTGATTCTTTCTACTAT | 264 |
| BPN258F | AACACCACTACAAAACTTNNSGATTCTTTCTACTATGGA | 265 |
| BPN259F | ACCACTACAAAACTTGGTNNSTCTTTCTACTATGGAAAA | 266 |
| BPN260F | ACTACAAAACTTGGTGATNNSTTCTACTATGGAAAAGGG | 267 |
| BPN261F | ACAAAACTTGGTGATTCTNNSTACTATGGAAAAGGGCTG | 268 |
| BPN262F | AAACTTGGTGATTCTTTCNNSTATGGAAAAGGGCTGATC | 269 |
| BPN263F | CTTGGTGATTCTTTCTACNNSGGAAAAGGGCTGATCAAC | 270 |
| BPN264F | GGTGATTCTTTCTACTATNNSAAAGGGCTGATCAACGTA | 271 |
| BPN265F | GATTCTTTCTACTATGGANNSGGGCTGATCAACGTACAG | 272 |
| BPN266F | TCTTTCTACTATGGAAAANNSCTGATCAACGTACAGGCG | 273 |
| BPN267F | TTCTACTATGGAAAAGGGNNSATCAACGTACAGGCGGCA | 274 |
| BPN268F | TACTATGGAAAAGGGCTGNNSAACGTACAGGCGGCAGCT | 275 |
| BPN269F | TATGGAAAAGGGCTGATCNNSGTACAGGCGGCAGCTCAG | 276 |
| BPN270F | GGAAAAGGGCTGATCAACNNSCAGGCGGCAGCTCAGTAA | 277 |
| BPN271F | AAAGGGCTGATCAACGTANNSGCGGCAGCTCAGTAAAGC | 278 |
| BPN272F | GGGCTGATCAACGTACAGNNSGCAGCTCAGTAAAGCTTA | 279 |
| BPN273F | CTGATCAACGTACAGGCGNNSGCTCAGTAAAGCTTACTG | 280 |
| BPN274F | ATCAACGTACAGGCGGCANNSCAGTAAAGCTTACTGGCC | 281 |
| BPN275F | AACGTACAGGCGGCAGCTNNSTAAAGCTTACTGGCCGTC | 282 |
| BPN1R | GCCGTAAGGCACGGACTGSNNGTATGCATGTGCTACGTG | 283 |
| BPN2R | TACGCCGTAAGGCACGGASNNCGCGTATGCATGTGCTAC | 284 |

(continued)

| Table 3-1 BPN' Primers | | |
|---|---|---|
| **Primer Name** | **Oligonucleotide Sequence** | **SEQ ID NO:** |
| BPN3R | TGATACGCCGTAAGGCACSNNCTGCGCGTATGCATGTGC | 285 |
| BPN4R | TTGTGATACGCCGTAAGGSNNGGACTGCGCGTATGCATG | 286 |
| BPN5R | AATTTGTGATACGCCGTASNNCACGGACTGCGCGTATGC | 287 |
| BPN6R | TTTAATTTGTGATACGCCSNNAGGCACGGACTGCGCGTA | 288 |
| BPN7R | GGCTTTAATTTGTGATACSNNGTAAGGCACGGACTGCGC | 289 |
| BPN8R | AGGGGCTTTAATTTGTGASNNGCCGTAAGGCACGGACTG | 290 |
| BPN9R | AGCAGGGGCTTTAATTTGSNNTACGCCGTAAGGCACGGA | 291 |
| BPN10R | CAGAGCAGGGGCTTTAATSNNTGATACGCCGTAAGGCAC | 292 |
| BPN11R | GTGCAGAGCAGGGGCTTTSNNTTGTGATACGCCGTAAGG | 293 |
| BPN12R | AGAGTGCAGAGCAGGGGCSNNAATTTGTGATACGCCGTA | 294 |
| BPN13R | TTGAGAGTGCAGAGCAGGSNNTTTAATTTGTGATACGCC | 295 |
| BPN14R | GCCTTGAGAGTGCAGAGCSNNGGCTTTAATTTGTGATAC | 296 |
| BPN15R | GTAGCCTTGAGAGTGCAGSNNAGGGGCTTTAATTTGTGA | 297 |
| BPN16R | AGTGTAGCCTTGAGAGTGSNNAGCAGGGGCTTTAATTTG | 298 |
| BPN17R | TCCAGTGTAGCCTTGAGASNNCAGAGCAGGGGCTTTAAT | 299 |
| BPN18R | TGATCCAGTGTAGCCTTGSNNGTGCAGAGCAGGGGCTTT | 300 |
| BPN19R | ATTTGATCCAGTGTAGCCSNNAGAGTGCAGAGCAGGGGC | 301 |
| BPN20R | AACATTTGATCCAGTGTASNNTTGAGAGTGCAGAGCAGG | 302 |
| BPN21R | TTTAACATTTGATCCAGTSNNGCCTTGAGAGTGCAGAGC | 303 |
| BPN22R | TACTTTAACATTTGATCCSNNGTAGCCTTGAGAGTGCAG | 304 |
| BPN23R | CGCTACTTTAACATTTGASNNAGTGTAGCCTTGAGAGTG | 305 |
| BPN24R | AACCGCTACTTTAACATTSNNTCCAGTGTAGCCTTGAGA | 306 |
| BPN25R | GATAACCGCTACTTTAACSNNTGATCCAGTGTAGCCTTG | 307 |
| BPN26R | GTCGATAACCGCTACTTTSNNATTTGATCCAGTGTAGCC | 308 |
| BPN27R | GCTGTCGATAACCGCTACSNNAACATTTGATCCAGTGTA | 309 |
| BPN28R | ACCGCTGTCGATAACCGCSNNTTTAACATTTGATCCAGT | 310 |
| BPN29R | GATACCGCTGTCGATAACSNNTACTTTAACATTTGATCC | 311 |
| BPN30R | ATCGATACCGCTGTCGATSNNCGCTACTTTAACATTTGA | 312 |
| BPN31R | AGAATCGATACCGCTGTCSNNAACCGCTACTTTAACATT | 313 |
| BPN32R | AGAAGAATCGATACCGCTSNNGATAACCGCTACTTTAAC | 314 |
| BPN33R | ATGAGAAGAATCGATACCSNNGTCGATAACCGCTACTTT | 315 |
| BPN34R | AGGATGAGAAGAATCGATSNNGCTGTCGATAACCGCTAC | 316 |
| BPN35R | ATCAGGATGAGAAGAATCSNNACCGCTGTCGATAACCGC | 317 |
| BPN36R | TAAATCAGGATGAGAAGASNNGATACCGCTGTCGATAAC | 318 |
| BPN37R | CTTTAAATCAGGATGAGASNNATCGATACCGCTGTCGAT | 319 |
| BPN38R | TACCTTTAAATCAGGATGSNNAGAATCGATACCGCTGTC | 320 |
| BPN39R | TGCTACCTTTAAATCAGGSNNAGAAGAATCGATACCGCT | 321 |

(continued)

| Table 3-1 BPN' Primers | | |
|---|---|---|
| **Primer Name** | **Oligonucleotide Sequence** | **SEQ ID NO:** |
| BPN40R | GCCTGCTACCTTTAAATCSNNATGAGAAGAATCGATACC | 322 |
| BPN41R | TCCGCCTGCTACCTTTAASNNAGGATGAGAAGAATCGAT | 323 |
| BPN42R | GGCTCCGCCTGCTACCTTSNNATCAGGATGAGAAGAATC | 324 |
| BPN43R | GCTGGCTCCGCCTGCTACSNNTAAATCAGGATGAGAAGA | 325 |
| BPN44R | CATGCTGGCTCCGCCTGCSNNCTTTAAATCAGGATGAGA | 326 |
| BPN45R | AACCATGCTGGCTCCGCCSNNTACCTTTAAATCAGGATG | 327 |
| BPN46R | AGGAACCATGCTGGCTCCSNNTGCTACCTTTAAATCAGG | 328 |
| BPN47R | AGAAGGAACCATGCTGGCSNNGCCTGCTACCTTTAAATC | 339 |
| BPN48R | TTCAGAAGGAACCATGCTSNNTCCGCCTGCTACCTTTAA | 330 |
| BPN49R | TGTTTCAGAAGGAACCATSNNGGCTCCGCCTGCTACCTT | 331 |
| BPN50R | ATTTGTTTCAGAAGGAACSNNGCTGGCTCCGCCTGCTAC | 332 |
| BPN51R | AGGATTTGTTTCAGAAGGSNNCATGCTGGCTCCGCCTGC | 333 |
| BPN52R | GAAAGGATTTGTTTCAGASNNAACCATGCTGGCTCCGCC | 334 |
| BPN53R | TTGGAAAGGATTTGTTTCSNNAGGAACCATGCTGGCTCC | 335 |
| BPN54R | GTCTTGGAAAGGATTTGTSNNAGAAGGAACCATGCTGGC | 336 |
| BPN55R | GTTGTCTTGGAAAGGATTSNNTTCAGAAGGAACCATGCT | 337 |
| BPN56R | GTTGTTGTCTTGGAAAGGSNNTGTTTCAGAAGGAACCAT | 338 |
| BPN57R | AGAGTTGTTGTCTTGGAASNNATTTGTTTCAGAAGGAAC | 339 |
| BPN58R | GTGAGAGTTGTTGTCTTGSNNAGGATTTGTTTCAGAAGG | 340 |
| BPN59R | TCCGTGAGAGTTGTTGTCSNNGAAAGGATTTGTTTCAGA | 341 |
| BPN60R | AGTTCCGTGAGAGTTGTTSNNTTGGAAAGGATTTGTTTC | 342 |
| BPN61R | GTGAGTTCCGTGAGAGTTSNNGTCTTGGAAAGGATTTGT | 343 |
| BPN62R | AACGTGAGTTCCGTGAGASNNGTTGTCTTGGAAAGGATT | 344 |
| BPN63R | GGCAACGTGAGTTCCGTGSNNGTTGTTGTCTTGGAAAGG | 345 |
| BPN64R | GCCGGCAACGTGAGTTCCSNNAGAGTTGTTGTCTTGGAA | 346 |
| BPN65R | TGTGCCGGCAACGTGAGTSNNGTGAGAGTTGTTGTCTTG | 347 |
| BPN66R | AACTGTGCCGGCAACGTGSNNTCCGTGAGAGTTGTTGTC | 348 |
| BPN67R | CGCAACTGTGCCGGCAACSNNAGTTCCGTGAGAGTTGTT | 349 |
| BPN68R | AGCCGCAACTGTGCCGGCSNNGTGAGTTCCGTGAGAGTT | 350 |
| BPN69R | AAGAGCCGCAACTGTGCCSNNAACGTGAGTTCCGTGAGA | 351 |
| BPN70R | ATTAAGAGCCGCAACTGTSNNGGCAACGTGAGTTCCGTG | 352 |
| BPN71R | GTTATTAAGAGCCGCAACSNNGCCGGCAACGTGAGTTCC | 353 |
| BPN72R | TGAGTTATTAAGAGCCGCSNNTGTGCCGGCAACGTGAGT | 354 |
| BPN73R | GATTGAGTTATTAAGAGCSNNAACTGTGCCGGCAACGTG | 355 |
| BPN74R | ACCGATTGAGTTATTAAGSNNCGCAACTGTGCCGGCAAC | 356 |
| BPN75R | TACACCGATTGAGTTATTSNNAGCCGCAACTGTGCCGGC | 357 |
| BPN76R | TAATACACCGATTGAGTTSNNAAGAGCCGCAACTGTGCC | 358 |

(continued)

| Table 3-1 BPN' Primers | | |
|---|---|---|
| **Primer Name** | **Oligonucleotide Sequence** | **SEQ ID NO:** |
| BPN77R | GCCTAATACACCGATTGASNNATTAAGAGCCGCAACTGT | 359 |
| BPN78R | AACGCCTAATACACCGATSNNGTTATTAAGAGCCGCAAC | 360 |
| BPN79R | CGCAACGCCTAATACACCSNNTGAGTTATTAAGAGCCGC | 361 |
| BPN80R | TGGCGCAACGCCTAATACSNNGATTGAGTTATTAAGAGC | 362 |
| BPN81R | GCTTGGCGCAACGCCTAASNNACCGATTGAGTTATTAAG | 363 |
| BPN82R | TGCGCTTGGCGCAACGCCSNNTACACCGATTGAGTTATT | 364 |
| BPN83R | TGATGCGCTTGGCGCAACSNNTAATACACCGATTGAGTT | 365 |
| BPN84R | AAGTGATGCGCTTGGCGCSNNGCCTAATACACCGATTGA | 366 |
| BPN85R | GTAAAGTGATGCGCTTGGSNNAACGCCTAATACACCGAT | 367 |
| BPN86R | AGCGTAAAGTGATGCGCTSNNCGCAACGCCTAATACACC | 368 |
| BPN87R | TACAGCGTAAAGTGATGCSNNTGGCGCAACGCCTAATAC | 369 |
| BPN88R | TTTTACAGCGTAAAGTGASNNGCTTGGCGCAACGCCTAA | 370 |
| BPN89R | AACTTTTACAGCGTAAAGSNNTGCGCTTGGCGCAACGCC | 371 |
| BPN90R | GAGAACTTTTACAGCGTASNNTGATGCGCTTGGCGCAAC | 372 |
| BPN91R | ACCGAGAACTTTTACAGCSNNAAGTGATGCGCTTGGCGC | 373 |
| BPN92R | AGCACCGAGAACTTTTACSNNGTAAAGTGATGCGCTTGG | 374 |
| BPN93R | GTCAGCACCGAGAACTTTSNNAGCGTAAAGTGATGCGCT | 375 |
| BPN94R | ACCGTCAGCACCGAGAACSNNTACAGCGTAAAGTGATGC | 376 |
| BPN95R | GGAACCGTCAGCACCGAGSNNTTTTACAGCGTAAAGTGA | 377 |
| BPN96R | GCCGGAACCGTCAGCACCSNNAACTTTTACAGCGTAAAG | 378 |
| BPN97R | TTGGCCGGAACCGTCAGCSNNGAGAACTTTTACAGCGTA | 379 |
| BPN98R | GTATTGGCCGGAACCGTCSNNACCGAGAACTTTTACAGC | 380 |
| BPN99R | GCTGTATTGGCCGGAACCSNNAGCACCGAGAACTTTTAC | 381 |
| BPN100R | CCAGCTGTATTGGCCGGASNNGTCAGCACCGAGAACTTT | 382 |
| BPN101R | GATCCAGCTGTATTGGCCSNNACCGTCAGCACCGAGAAC | 383 |
| BPN102R | AATGATCCAGCTGTATTGSNNGGAACCGTCAGCACCGAG | 384 |
| BPN103R | GTTAATGATCCAGCTGTASNNGCCGGAACCGTCAGCACC | 385 |
| BPN104R | TCCGTTAATGATCCAGCTSNNTTGGCCGGAACCGTCAGC | 386 |
| BPN105R | GATTCCGTTAATGATCCASNNGTATTGGCCGGAACCGTC | 387 |
| BPN106R | CTCGATTCCGTTAATGATSNNGCTGTATTGGCCGGAACC | 388 |
| BPN107R | CCACTCGATTCCGTTAATSNNCCAGCTGTATTGGCCGGA | 389 |
| BPN108R | CGCCCACTCGATTCCGTTSNNGATCCAGCTGTATTGGCC | 390 |
| BPN109R | GATCGCCCACTCGATTCCSNNAATGATCCAGCTGTATTG | 391 |
| BPN110R | TGCGATCGCCCACTCGATSNNGTTAATGATCCAGCTGTA | 392 |
| BPN111R | GTTTGCGATCGCCCACTCSNNTCCGTTAATGATCCAGCT | 393 |
| BPN112R | ATTGTTTGCGATCGCCCASNNGATTCCGTTAATGATCCA | 394 |
| BPN113R | CATATTGTTTGCGATCGCSNNCTCGATTCCGTTAATGAT | 395 |

(continued)

| Table 3-1 BPN' Primers | | |
|---|---|---|
| **Primer Name** | **Oligonucleotide Sequence** | **SEQ ID NO:** |
| BPN114R | GTCCATATTGTTTGCGATSNNCCACTCGATTCCGTTAAT | 396 |
| BPN115R | AACGTCCATATTGTTTGCSNNCGCCCACTCGATTCCGTT | 397 |
| BPN116R | AATAACGTCCATATTGTTSNNGATCGCCCACTCGATTCC | 398 |
| BPN117R | GTTAATAACGTCCATATTSNNTGCGATCGCCCACTCGAT | 399 |
| BPN118R | CATGTTAATAACGTCCATSNNGTTTGCGATCGCCCACTC | 401 |
| BPN119R | GCTCATGTTAATAACGTCSNNATTGTTTGCGATCGCCCA | 402 |
| BPN120R | GAGGCTCATGTTAATAACSNNCATATTGTTTGCGATCGC | 403 |
| BPN121R | GCCGAGGCTCATGTTAATSNNGTCCATATTGTTTGCGAT | 404 |
| BPN122R | TCCGCCGAGGCTCATGTTSNNAACGTCCATATTGTTTGC | 405 |
| BPN123R | AGGTCCGCCGAGGCTCATSNNAATAACGTCCATATTGTT | 406 |
| BPN124R | AGAAGGTCCGCCGAGGCTSNNGTTAATAACGTCCATATT | 407 |
| BPN125R | ACCAGAAGGTCCGCCGAGSNNCATGTTAATAACGTCCAT | 408 |
| BPN126R | AGAACCAGAAGGTCCGCCSNNGCTCATGTTAATAACGTC | 409 |
| BPN127R | AGCAGAACCAGAAGGTCCSNNGAGGCTCATGTTAATAAC | 410 |
| BPN128R | AGCAGCAGAACCAGAAGGSNNGCCGAGGCTCATGTTAAT | 411 |
| BPN129R | TAAAGCAGCAGAACCAGASNNTCCGCCGAGGCTCATGTT | 412 |
| BPN130R | TTTTAAAGCAGCAGAACCSNNAGGTCCGCCGAGGCTCAT | 413 |
| BPN131R | CGCTTTTAAAGCAGCAGASNNAGAAGGTCCGCCGAGGCT | 414 |
| BPN132R | TGCCGCTTTTAAAGCAGCSNNACCAGAAGGTCCGCCGAG | 415 |
| BPN133R | AACTGCCGCTTTTAAAGCSNNAGAACCAGAAGGTCCGCC | 416 |
| BPN134R | ATCAACTGCCGCTTTTAASNNAGCAGAACCAGAAGGTCC | 417 |
| BPN135R | TTTATCAACTGCCGCTTTSNNAGCAGCAGAACCAGAAGG | 418 |
| BPN136R | GGCTTTATCAACTGCCGCSNNTAAAGCAGCAGAACCAGA | 419 |
| BPN137R | AACGGCTTTATCAACTGCSNNTTTTAAAGCAGCAGAACC | 420 |
| BPN138R | TGCAACGGCTTTATCAACSNNCGCTTTTAAAGCAGCAGA | 421 |
| BPN139R | GGATGCAACGGCTTTATCSNNTGCCGCTTTTAAAGCAGC | 422 |
| BPN140R | GCCGGATGCAACGGCTTTSNNAACTGCCGCTTTTAAAGC | 423 |
| BPN141R | GACGCCGGATGCAACGGCSNNATCAACTGCCGCTTTTAA | 424 |
| BPN142R | TACGACGCCGGATGCAACSNNTTTATCAACTGCCGCTTT | 425 |
| BPN143R | GACTACGACGCCGGATGCSNNGGCTTTATCAACTGCCGC | 426 |
| BPN144R | AACGACTACGACGCCGGASNNAACGGCTTTATCAACTGC | 427 |
| BPN145R | CGCAACGACTACGACGCCSNNTGCAACGGCTTTATCAAC | 428 |
| BPN146R | TGCCGCAACGACTACGACSNNGGATGCAACGGCTTTATC | 429 |
| BPN147R | GGCTGCCGCAACGACTACSNNGCCGGATGCAACGGCTTT | 430 |
| BPN148R | ACCGGCTGCCGCAACGACSNNGACGCCGGATGCAACGGC | 431 |
| BPN149R | GTTACCGGCTGCCGCAACSNNTACGACGCCGGATGCAAC | 432 |
| BPN150R | TTCGTTACCGGCTGCCGCSNNGACTACGACGCCGGATGC | 433 |

(continued)

| Table 3-1 BPN' Primers | | |
|---|---|---|
| **Primer Name** | **Oligonucleotide Sequence** | **SEQ ID NO:** |
| BPN151R | GCCTTCGTTACCGGCTGCSNNAACGACTACGACGCCGGA | 434 |
| BPN152R | AGTGCCTTCGTTACCGGCSNNCGCAACGACTACGACGCC | 435 |
| BPN153R | GGAAGTGCCTTCGTTACCSNNTGCCGCAACGACTACGAC | 436 |
| BPN154R | GCCGGAAGTGCCTTCGTTSNNGGCTGCCGCAACGACTAC | 437 |
| BPN155R | GCTGCCGGAAGTGCCTTCSNNACCGGCTGCCGCAACGAC | 438 |
| BPN156R | TGAGCTGCCGGAAGTGCCSNNGTTACCGGCTGCCGCAAC | 439 |
| BPN157R | GCTTGAGCTGCCGGAAGTSNNTTCGTTACCGGCTGCCGC | 440 |
| BPN158R | TGTGCTTGAGCTGCCGGASNNGCCTTCGTTACCGGCTGC | 441 |
| BPN159R | CACTGTGCTTGAGCTGCCSNNAGTGCCTTCGTTACCGGC | 442 |
| BPN160R | GCCCACTGTGCTTGAGCTSNNGGAAGTGCCTTCGTTACC | 443 |
| BPN161R | GTAGCCCACTGTGCTTGASNNGCCGGAAGTGCCTTCGTT | 444 |
| BPN162R | AGGGTAGCCCACTGTGCTSNNGCTGCCGGAAGTGCCTTC | 445 |
| BPN163R | ACCAGGGTAGCCCACTGTSNNTGAGCTGCCGGAAGTGCC | 446 |
| BPN164R | TTTACCAGGGTAGCCCACSNNGCTTGAGCTGCCGGAAGT | 447 |
| BPN165R | GTATTTACCAGGGTAGCCSNNTGTGCTTGAGCTGCCGGA | 448 |
| BPN166R | AGGGTATTTACCAGGGTASNNCACTGTGCTTGAGCTGCC | 449 |
| BPN167R | AGAAGGGTATTTACCAGGSNNGCCCACTGTGCTTGAGCT | 450 |
| BPN168R | GACAGAAGGGTATTTACCSNNGTAGCCCACTGTGCTTGA | 451 |
| BPN169R | AATGACAGAAGGGTATTTSNNAGGGTAGCCCACTGTGCT | 452 |
| BPN170R | TGCAATGACAGAAGGGTASNNACCAGGGTAGCCCACTGT | 453 |
| BPN171R | TACTGCAATGACAGAAGGSNNTTTACCAGGGTAGCCCAC | 454 |
| BPN172R | GCCTACTGCAATGACAGASNNGTATTTACCAGGGTAGCC | 455 |
| BPN173R | AGCGCCTACTGCAATGACSNNAGGGTATTTACCAGGGTA | 456 |
| BPN174R | AACAGCGCCTACTGCAATSNNAGAAGGGTATTTACCAGG | 457 |
| BPN175R | GTCAACAGCGCCTACTGCSNNGACAGAAGGGTATTTACC | 458 |
| BPN176R | GCTGTCAACAGCGCCTACSNNAATGACAGAAGGGTATTT | 459 |
| BPN177R | GCTGCTGTCAACAGCGCCSNNTGCAATGACAGAAGGGTA | 460 |
| BPN178R | GTTGCTGCTGTCAACAGCSNNTACTGCAATGACAGAAGG | 461 |
| BPN179R | TTGGTTGCTGCTGTCAACSNNGCCTACTGCAATGACAGA | 462 |
| BPN180R | TCTTTGGTTGCTGCTGTCSNNAGCGCCTACTGCAATGAC | 463 |
| BPN181R | TGCTCTTTGGTTGCTGCTSNNAACAGCGCCTACTGCAAT | 464 |
| BPN182R | AGATGCTCTTTGGTTGCTSNNGTCAACAGCGCCTACTGC | 465 |
| BPN183R | GAAAGATGCTCTTTGGTTSNNGCTGTCAACAGCGCCTAC | 466 |
| BPN184R | TGAGAAAGATGCTCTTTGSNNGCTGCTGTCAACAGCGCC | 467 |
| BPN185R | GCTTGAGAAAGATGCTCTSNNGTTGCTGCTGTCAACAGC | 468 |
| BPN186R | TACGCTTGAGAAAGATGCSNNTTGGTTGCTGCTGTCAAC | 469 |
| BPN187R | TCCTACGCTTGAGAAAGASNNTCTTTGGTTGCTGCTGTC | 470 |

(continued)

| Table 3-1 BPN' Primers | | |
|---|---|---|
| **Primer Name** | **Oligonucleotide Sequence** | **SEQ ID NO:** |
| BPN188R | AGGTCCTACGCTTGAGAASNNTGCTCTTTGGTTGCTGCT | 471 |
| BPN189R | CTCAGGTCCTACGCTTGASNNAGATGCTCTTTGGTTGCT | 472 |
| BPN190R | AAGCTCAGGTCCTACGCTSNNGAAAGATGCTCTTTGGTT | 473 |
| BPN191R | ATCAAGCTCAGGTCCTACSNNTGAGAAAGATGCTCTTTG | 474 |
| BPN192R | GACATCAAGCTCAGGTCCSNNGCTTGAGAAAGATGCTCT | 475 |
| BPN193R | CATGACATCAAGCTCAGGSNNTACGCTTGAGAAAGATGC | 476 |
| BPN194R | TGCCATGACATCAAGCTCSNNTCCTACGCTTGAGAAAGA | 477 |
| BPN195R | AGGTGCCATGACATCAAGSNNAGGTCCTACGCTTGAGAA | 478 |
| BPN196R | GCCAGGTGCCATGACATCSNNCTCAGGTCCTACGCTTGA | 479 |
| BPN197R | TACGCCAGGTGCCATGACSNNAAGCTCAGGTCCTACGCT | 480 |
| BPN198R | AGATACGCCAGGTGCCATSNNATCAAGCTCAGGTCCTAC | 481 |
| BPN199R | GATAGATACGCCAGGTGCSNNGACATCAAGCTCAGGTCC | 482 |
| BPN200R | TTGGATAGATACGCCAGGSNNCATGACATCAAGCTCAGG | 483 |
| BPN201R | GCTTTGGATAGATACGCCSNNTGCCATGACATCAAGCTC | 484 |
| BPN202R | CGTGCTTTGGATAGATACSNNAGGTGCCATGACATCAAG | 485 |
| BPN203R | AAGCGTGCTTTGGATAGASNNGCCAGGTGCCATGACATC | 486 |
| BPN204R | AGGAAGCGTGCTTTGGATSNNTACGCCAGGTGCCATGAC | 487 |
| BPN205R | TCCAGGAAGCGTGCTTTGSNNAGATACGCCAGGTGCCAT | 488 |
| BPN206R | GTTTCCAGGAAGCGTGCTSNNGATAGATACGCCAGGTGC | 489 |
| BPN207R | TTTGTTTCCAGGAAGCGTSNNTTGGATAGATACGCCAGG | 490 |
| BPN208R | GTATTTGTTTCCAGGAAGSNNGCTTTGGATAGATACGCC | 491 |
| BPN209R | CCCGTATTTGTTTCCAGGSNNCGTGCTTTGGATAGATAC | 492 |
| BPN210R | CGCCCCGTATTTGTTTCCSNNAAGCGTGCTTTGGATAGA | 493 |
| BPN211R | GTACGCCCCGTATTTGTTSNNAGGAAGCGTGCTTTGGAT | 494 |
| BPN212R | GTTGTACGCCCCGTATTTSNNTCCAGGAAGCGTGCTTTG | 495 |
| BPN213R | ACCGTTGTACGCCCCGTASNNGTTTCCAGGAAGCGTGCT | 496 |
| BPN214R | CGTACCGTTGTACGCCCCSNNTTTGTTTCCAGGAAGCGT | 497 |
| BPN215R | TGACGTACCGTTGTACGCSNNGTATTTGTTTCCAGGAAG | 498 |
| BPN216R | CATTGACGTACCGTTGTASNNCCCGTATTTGTTTCCAGG | 499 |
| BPN217R | TGCCATTGACGTACCGTTSNNCGCCCCGTATTTGTTTCC | 500 |
| BPN218R | AGATGCCATTGACGTACCSNNGTACGCCCCGTATTTGTT | 501 |
| BPN219R | CGGAGATGCCATTGACGTSNNGTTGTACGCCCCGTATTT | 502 |
| BPN220R | GTGCGGAGATGCCATTGASNNACCGTTGTACGCCCCGTA | 503 |
| BPN221R | AACGTGCGGAGATGCCATSNNCGTACCGTTGTACGCCCC | 504 |
| BPN222R | GGCAACGTGCGGAGATGCSNNTGACGTACCGTTGTACGC | 505 |
| BPN223R | TCCGGCAACGTGCGGAGASNNCATTGACGTACCGTTGTA | 506 |
| BPN224R | CGCTCCGGCAACGTGCGGSNNTGCCATTGACGTACCGTT | 507 |

(continued)

| Table 3-1 BPN' Primers | | |
|---|---|---|
| **Primer Name** | **Oligonucleotide Sequence** | **SEQ ID NO:** |
| BPN225R | AGCCGCTCCGGCAACGTGSNNAGATGCCATTGACGTACC | 508 |
| BPN226R | AGCAGCCGCTCCGGCAACSNNCGGAGATGCCATTGACGT | 509 |
| BPN227R | CAAAGCAGCCGCTCCGGCSNNGTGCGGAGATGCCATTGA | 510 |
| BPN228R | AATCAAAGCAGCCGCTCCSNNAACGTGCGGAGATGCCAT | 511 |
| BPN229R | AAGAATCAAAGCAGCCGCSNNGGCAACGTGCGGAGATGC | 512 |
| BPN230R | AGAAAGAATCAAAGCAGCSNNTCCGGCAACGTGCGGAGA | 513 |
| BPN231R | CTTAGAAAGAATCAAAGCSNNCGCTCCGGCAACGTGCGG | 514 |
| BPN232R | GTGCTTAGAAAGAATCAASNNAGCCGCTCCGGCAACGTG | 515 |
| BPN233R | CGGGTGCTTAGAAAGAATSNNAGCAGCCGCTCCGGCAAC | 516 |
| BPN234R | GTTCGGGTGCTTAGAAAGSNNCAAAGCAGCCGCTCCGGC | 517 |
| BPN235R | CCAGTTCGGGTGCTTAGASNNAATCAAAGCAGCCGCTCC | 518 |
| BPN236R | TGTCCAGTTCGGGTGCTTSNNAAGAATCAAAGCAGCCGC | 519 |
| BPN237R | GTTTGTCCAGTTCGGGTGSNNAGAAAGAATCAAAGCAGC | 520 |
| BPN238R | AGTGTTTGTCCAGTTCGGSNNCTTAGAAAGAATCAAAGC | 521 |
| BPN239R | TTGAGTGTTTGTCCAGTTSNNGTGCTTAGAAAGAATCAA | 522 |
| BPN240R | GACTTGAGTGTTTGTCCASNNCGGGTGCTTAGAAAGAAT | 523 |
| BPN241R | GCGGACTTGAGTGTTTGTSNNGTTCGGGTGCTTAGAAAG | 524 |
| BPN242R | GCTGCGGACTTGAGTGTTSNNCCAGTTCGGGTGCTTAGA | 525 |
| BPN243R | ACTGCTGCGGACTTGAGTSNNTGTCCAGTTCGGGTGCTT | 526 |
| BPN244R | TAAACTGCTGCGGACTTGSNNGTTTGTCCAGTTCGGGTG | 527 |
| BPN245R | TTCTAAACTGCTGCGGACSNNAGTGTTTGTCCAGTTCGG | 528 |
| BPN246R | GTTTTCTAAACTGCTGCGSNNTTGAGTGTTTGTCCAGTT | 529 |
| BPN247R | GGTGTTTTCTAAACTGCTSNNGACTTGAGTGTTTGTCCA | 530 |
| BPN248R | AGTGGTGTTTTCTAAACTSNNGCGGACTTGAGTGTTTGT | 531 |
| BPN249R | TGTAGTGGTGTTTTCTAASNNGCTGCGGACTTGAGTGTT | 532 |
| BPN250R | TTTTGTAGTGGTGTTTTCSNNACTGCTGCGGACTTGAGT | 533 |
| BPN251R | AAGTTTTGTAGTGGTGTTSNNTAAACTGCTGCGGACTTG | 534 |
| BPN252R | ACCAAGTTTTGTAGTGGTSNNTTCTAAACTGCTGCGGAC | 535 |
| BPN253R | ATCACCAAGTTTTGTAGTSNNGTTTTCTAAACTGCTGCG | 536 |
| BPN254R | AGAATCACCAAGTTTTGTSNNGGTGTTTTCTAAACTGCT | 537 |
| BPN255R | GAAAGAATCACCAAGTTTSNNAGTGGTGTTTTCTAAACT | 538 |
| BPN256R | GTAGAAAGAATCACCAAGSNNTGTAGTGGTGTTTTCTAA | 539 |
| BPN257R | ATAGTAGAAAGAATCACCSNNTTTTGTAGTGGTGTTTTC | 540 |
| BPN258R | TCCATAGTAGAAAGAATCSNNAAGTTTTGTAGTGGTGTT | 541 |
| BPN259R | TTTTCCATAGTAGAAAGASNNACCAAGTTTTGTAGTGGT | 542 |
| BPN260R | CCCTTTTCCATAGTAGAASNNATCACCAAGTTTTGTAGT | 543 |
| BPN261R | CAGCCCTTTTCCATAGTASNNAGAATCACCAAGTTTTGT | 544 |

(continued)

| Table 3-1 BPN' Primers | | |
|---|---|---|
| **Primer Name** | **Oligonucleotide Sequence** | **SEQ ID NO:** |
| BPN262R | GATCAGCCCTTTTCCATASNNGAAAGAATCACCAAGTTT | 545 |
| BPN263R | GTTGATCAGCCCTTTTCCSNNGTAGAAAGAATCACCAAG | 546 |
| BPN264R | TACGTTGATCAGCCCTTTSNNATAGTAGAAAGAATCACC | 547 |
| BPN265R | CTGTACGTTGATCAGCCCSNNTCCATAGTAGAAAGAATC | 548 |
| BPN266R | CGCCTGTACGTTGATCAGSNNTTTCCATAGTAGAAAGA | 549 |
| BPN267R | TGCCGCCTGTACGTTGATSNNCCCTTTTCCATAGTAGAA | 550 |
| BPN268R | AGCTGCCGCCTGTACGTTSNNCAGCCCTTTTCCATAGTA | 551 |
| BPN269R | CTGAGCTGCCGCCTGTACSNNGATCAGCCCTTTTCCATA | 552 |
| BPN270R | TTACTGAGCTGCCGCCTGSNNGTTGATCAGCCCTTTTCC | 553 |
| BPN271R | GCTTTACTGAGCTGCCGCSNNTACGTTGATCAGCCCTTT | 554 |
| BPN272R | TAAGCTTTACTGAGCTGCSNNCTGTACGTTGATCAGCCC | 555 |
| BPN273R | CAGTAAGCTTTACTGAGCSNNCGCCTGTACGTTGATCAG | 556 |
| BPN274R | GGCCAGTAAGCTTTACTGSNNTGCCGCCTGTACGTTGAT | 557 |
| BPN275R | GACGGCCAGTAAGCTTTASNNAGCTGCCGCCTGTACGTT | 558 |
| **BPN' Multiple Mutation Library Construction** | | |

Synthetic BPN' multiple mutation libraries (or combinatorial libraries) were produced by Geneart, Baseclear, and Bioké. Each synthetic BPN' multiple mutation library contained a mix of BPN' genes in which two or more selected codons of the mature sequence were randomly replaced by specific DNA sequences. For example: a BPN' combinatorial library could be designed in such a way that codon 22 of the mature sequence would be randomly substituted for DNA triplets coding for Thr, Gln, Val or Tyr, codon 26 for Val, Gln, Asn or Tyr, codon 31 for Ile, His, Tyr, or Asn and codon 48 for Ala, Glu, His or Asp. In this example, the combinatorial library would contain a maximum of 256 BPN' combinatorial variants. However, a typical BPN' multiple mutation library could contain up to thousands of unique BPN' variant genes. Multiple mutation library fragments having terminal *Ava*I and *Hind*III sites (*See* e.g., wild type BPN') were digested with *Ava*I and *Hind*III, gel-purified and cloned into the pHPLT vector by ligase reaction using Invitrogen® T4 DNA Ligase (Catalog No. 15224-025) as recommended by the manufacturer for general cloning of cohesive ends. The wild type BPN'*Ava*I / *Hind*III fragment:

```
AAGACCCGAG CGTCGCTTAC GTTGAAGAAG ATCACGTAGC ACATGCGTAC GCGCAGTCCG
TGCCTTACGG CGTATCACAA ATTAAAGCCC CTGCTCTGCA CTCTCAAGGC TACACTGGAT
CAAATGTTAA AGTAGCGGTT ATCGACAGCG GTATCGATTC TTCTCATCCT GATTTAAAGG
TAGCAGGCGG AGCCAGCATG GTTCCTTCTG AAACAAATCC TTTCCAAGAC AACAACTCTC
ACGGAACTCA CGTTGCCGGC ACAGTTGCGG CTCTTAATAA CTCAATCGGT GTATTAGGCG
TTGCGCCAAG CGCATCACTT TACGCTGTAA AAGTTCTCGG TGCTGACGGT TCCGGCCAAT
ACAGCTGGAT CATTAACGGA ATCGAGTGGG CGATCGCAAA CAATATGGAC GTTATTAACA
TGAGCCTCGG CGGACCTTCT GGTTCTGCTG CTTTAAAAGC GGCAGTTGAT AAAGCCGTTG
CATCCGGCGT CGTAGTCGTT GCGGCAGCCG GTAACGAAGG CACTTCCGGC AGCTCAAGCA
CAGTGGGCTA CCCTGGTAAA TACCCTTCTG TCATTGCAGT AGGCGCTGTT GACAGCAGCA
ACCAAAGAGC ATCTTTCTCA AGCGTAGGAC CTGAGCTTGA TGTCATGGCA CCTGGCGTAT
CTATCCAAAG CACGCTTCCT GGAAACAAAT ACGGGGCGTA CAACGGTACG TCAATGGCAT
CTCCGCACGT TGCCGGAGCG GCTGCTTTGA TTCTTTCTAA GCACCCGAAC TGGACAAACA
CTCAAGTCCG CAGCAGTTTA GAAAACACCA CTACAAAACT TGGTGATTCT TTCTACTATG
GAAAAGGGCT GATCAACGTA CAGGCGGCAG CTCAGTAAGT TAACAGAGGA GGATTTCCT
GAAGGAAATC CGTTTTTTTA TTTTAAGCTT GGAGA  (SEQ ID NO:559)
```

To transform the ligation reaction mix, the library DNA (BPN' library fragment mix cloned in pHPLT) was amplified using the TempliPhi kit (Amersham Catalog No. 25-6400). For this purpose, 1μL of the ligation reaction mix was mixed

with 5μL of sample buffer from the TempliPhi kit and heated for 3 minutes at 95°C to denature the DNA. The reaction was placed on ice to cool for 2 minutes and then spun down briefly. Next, 5μL of reaction buffer and 0.2μL of phi29 polymerase from the TempliPhi kit were added, and the reactions were incubated at 30°C in an MJ Research PCR machine for 4 hours. The phi29 enzyme was heat inactivated by incubation at 65°C for 10 minutes.

[02] For transformation of the libraries 0.1 μL of the TempliPhi amplification reaction product was mixed with 500 μL of competent *B. subtilis* cells (ΔaprE, ΔnprE, oppA, ΔspoIIE, degUHy32, ΔamyE::(xylR,pxylA-comK) followed by vigorous shaking at 37°C for 1 hour. After this time aliquots of 100 and 500 μL were plated on HI-agar plates containing 20 ppm neomycin and 0.5% skim milk.

The following Table provides a list of some of the single substitutions used in the present invention.

| Table 3-2. Mutations | |
|---|---|
| **POS** | **Variant** |
| 1 | A001T |
| 1 | A001V |
| 3 | S003F |
| 4 | V004M |
| 8 | V008I |
| 10 | Q010L |
| 10 | Q010R |
| 12 | K012N |
| 12 | K012R |
| 13 | A013T |
| 13 | A013V |
| 14 | P014L |
| 14 | P014S |
| 15 | A015T |
| 16 | L016M |
| 17 | H017Q |
| 17 | H017R |
| 17 | H017Y |
| 19 | Q019H |
| 20 | G020D |
| 25 | N025Y |
| 29 | A029V |
| 33 | S033G |
| 33 | S033I |
| 38 | S038F |
| 44 | V044I |
| 45 | A045V |
| 50 | M050I |
| 53 | S053F |
| 53 | S053T |
| 54 | E054D |

(continued)

| Table 3-2. Mutations | |
|---|---|
| POS | Variant |
| 55 | T055I |
| 55 | T055S |
| 59 | Q059H |
| 59 | Q059K |
| 59 | Q059L |
| 60 | D060N |
| 61 | N061D |
| 63 | S063T |
| 65 | G065R |
| 66 | T066G |
| 66 | T066I |
| 66 | T066S |
| 67 | H067R |
| 67 | H067S |
| 68 | V068C |
| 68 | V068D |
| 68 | V068G |
| 68 | V068I |
| 68 | V068L |
| 68 | V068N |
| 68 | V068S |
| 69 | A069G |
| 69 | A069S |
| 71 | T071G |
| 71 | T071I |
| 75 | L075G |
| 75 | L075I |
| 75 | L075R |
| 76 | N076D |
| 76 | N076G |
| 77 | N077Z |
| 79 | 1079F |
| 81 | V081A |
| 81 | V081I |
| 84 | V084D |
| 84 | V084I |
| 85 | A085V |

(continued)

| Table 3-2. Mutations | |
|---|---|
| POS | Variant |
| 86 | P086S |
| 86 | P086T |
| 88 | A088S |
| 88 | A088T |
| 88 | A088V |
| 92 | A092G |
| 92 | A092L |
| 92 | A092S |
| 92 | A092V |
| 93 | V093C |
| 93 | V093G |
| 93 | V093I |
| 93 | V093L |
| 93 | V093R |
| 93 | V093S |
| 94 | K094E |
| 94 | K094G |
| 94 | K094R |
| 94 | K094S |
| 94 | K094V |
| 94 | K094Z |
| 95 | V095D |
| 96 | L096I |
| 96 | L096V |
| 97 | G097C |
| 97 | G097D |
| 97 | G097H |
| 97 | G097L |
| 97 | G097N |
| 97 | G097R |
| 97 | G097S |
| 97 | G097V |
| 97 | G097Y |
| 97 | G097Z |
| 98 | A098C |
| 98 | A098D |
| 98 | A098F |

(continued)

| Table 3-2. Mutations | |
|---|---|
| POS | Variant |
| 98 | A098G |
| 98 | A098H |
| 98 | A098I |
| 98 | A098L |
| 98 | A098N |
| 98 | A098R |
| 98 | A098S |
| 98 | A098T |
| 98 | A098V |
| 98 | A098Y |
| 98 | A098Z |
| 99 | D099C |
| 99 | D099F |
| 99 | D099G |
| 99 | D099H |
| 99 | D099I |
| 99 | D099L |
| 99 | D099N |
| 99 | D099R |
| 99 | D099S |
| 99 | D099V |
| 99 | D099Y |
| 99 | D099Z |
| 100 | G 100C |
| 100 | G 100D |
| 100 | G 100H |
| 100 | G 100L |
| 100 | G 100N |
| 100 | G 100R |
| 100 | G100S |
| 100 | G100V |
| 100 | G100Z |
| 101 | S101C |
| 101 | S101D |
| 101 | S101F |
| 101 | S101G |
| 101 | S101H |

(continued)

| Table 3-2. Mutations | |
|---|---|
| POS | Variant |
| 101 | S101I |
| 101 | S101L |
| 101 | S101N |
| 101 | S101P |
| 101 | S101R |
| 101 | S101V |
| 101 | S101Y |
| 101 | S101Z |
| 102 | G102C |
| 102 | G102D |
| 102 | G102I |
| 102 | G102L |
| 102 | G102R |
| 102 | G102S |
| 102 | G102V |
| 102 | G102Z |
| 103 | Q103C |
| 103 | Q103D |
| 103 | Q103F |
| 103 | Q103G |
| 103 | Q103H |
| 103 | Q103I |
| 103 | Q103L |
| 103 | Q103N |
| 103 | Q103R |
| 103 | Q103S |
| 103 | Q103V |
| 103 | Q103Y |
| 103 | Q103Z |
| 104 | Y104C |
| 104 | Y104F |
| 104 | Y104G |
| 104 | Y104L |
| 104 | Y104N |
| 104 | Y104R |
| 104 | Y104S |
| 104 | Y104Z |

(continued)

| Table 3-2. Mutations | |
|---|---|
| POS | Variant |
| 105 | S105G |
| 106 | W106C |
| 109 | N109D |
| 109 | N109S |
| 116 | A116D |
| 116 | A116G |
| 116 | A116H |
| 116 | A116T |
| 117 | N117C |
| 117 | N117G |
| 117 | N117H |
| 117 | N117I |
| 117 | N117L |
| 117 | N117R |
| 117 | N117S |
| 117 | N117V |
| 117 | N117Y |
| 117 | N117Z |
| 118 | N118C |
| 118 | N118D |
| 118 | N118G |
| 118 | N118H |
| 118 | N118R |
| 118 | N118S |
| 119 | M119C |
| 119 | M119H |
| 119 | M119I |
| 119 | M119L |
| 119 | M119N |
| 119 | M119S |
| 119 | M119V |
| 120 | D120C |
| 120 | D120G |
| 120 | D120H |
| 120 | D120L |
| 120 | D120N |
| 120 | D120R |

(continued)

| Table 3-2. Mutations | |
|---|---|
| POS | Variant |
| 120 | D120S |
| 121 | V121C |
| 121 | V121I |
| 121 | V121L |
| 122 | I122C |
| 122 | I122D |
| 122 | I122G |
| 122 | I122H |
| 122 | I122L |
| 122 | I122S |
| 122 | I122V |
| 123 | N123C |
| 123 | N123D |
| 123 | N123G |
| 123 | N123I |
| 123 | N123S |
| 123 | N123V |
| 124 | M124G |
| 124 | M124I |
| 124 | M124L |
| 124 | M124S |
| 124 | M124V |
| 126 | L126C |
| 126 | L126F |
| 126 | L126G |
| 126 | L126H |
| 126 | L126I |
| 126 | L126R |
| 126 | L126S |
| 126 | L126V |
| 126 | L126Y |
| 127 | G127C |
| 127 | G127D |
| 127 | G127H |
| 127 | G127L |
| 127 | G127N |
| 127 | G127R |

(continued)

| Table 3-2. Mutations | |
|---|---|
| POS | Variant |
| 127 | G127S |
| 127 | G127V |
| 128 | G128C |
| 128 | G128D |
| 128 | G128H |
| 128 | G128N |
| 128 | G128R |
| 128 | G128S |
| 128 | G128Y |
| 129 | P129C |
| 129 | P129D |
| 129 | P129F |
| 129 | P129G |
| 129 | P129H |
| 129 | P129L |
| 129 | P129R |
| 129 | P129S |
| 129 | P129V |
| 129 | P129Y |
| 129 | P129Z |
| 129 | P129R |
| 130 | S130C |
| 130 | S130D |
| 130 | S130F |
| 130 | S130G |
| 130 | S130H |
| 130 | S130I |
| 130 | S130L |
| 130 | S130N |
| 130 | S130R |
| 130 | S130V |
| 130 | S130Y |
| 130 | S130Z |
| 131 | G131C |
| 131 | G131D |
| 131 | G131H |
| 131 | G131N |

(continued)

| Table 3-2. Mutations | |
|---|---|
| POS | Variant |
| 131 | G131R |
| 131 | G131S |
| 131 | G131V |
| 131 | G131Y |
| 131 | G131Z |
| 132 | S132C |
| 132 | S132D |
| 132 | S132G |
| 132 | S132H |
| 132 | S132I |
| 132 | S132L |
| 132 | S132N |
| 132 | S132R |
| 132 | S132T |
| 132 | S132V |
| 132 | S132Z |
| 133 | A133D |
| 133 | A133S |
| 133 | A133T |
| 133 | A133V |
| 134 | A134T |
| 135 | L135F |
| 136 | K136N |
| 137 | A137T |
| 138 | A138V |
| 139 | V139I |
| 142 | A142V |
| 143 | V143I |
| 144 | A144E |
| 144 | A144T |
| 144 | A144V |
| 145 | S145T |
| 146 | G146D |
| 147 | V147I |
| 151 | A151V |
| 153 | A153T |
| 153 | A153V |

(continued)

| Table 3-2. Mutations | |
|---|---|
| POS | Variant |
| 154 | G154S |
| 156 | E156D |
| 156 | E156G |
| 157 | G157D |
| 157 | G157S |
| 160 | G160D |
| 160 | G160S |
| 161 | S161N |
| 166 | G166D |
| 166 | G166S |
| 169 | G169A |
| 175 | I175T |
| 179 | A179V |
| 180 | V180A |
| 182 | S182N |
| 183 | S183G |
| 183 | S183N |
| 187 | A187V |
| 192 | V192A |
| 192 | V192I |
| 194 | P194L |
| 200 | A200T |
| 200 | A200V |
| 204 | S204P |
| 206 | Q206R |
| 209 | L209F |
| 211 | G211V |
| 213 | K213E |
| 213 | K213N |
| 213 | K213R |
| 216 | A216E |
| 216 | A216T |
| 216 | A216V |
| 217 | L217S |
| 217 | L217Y |
| 218 | N218I |
| 228 | A228T |

(continued)

| Table 3-2. Mutations | |
|---|---|
| POS | Variant |
| 230 | A230V |
| 231 | A231T |
| 233 | L233S |
| 236 | S236Y |
| 237 | K237E |
| 238 | H238Q |
| 238 | H238Y |
| 241 | W241R |
| 243 | N243D |
| 244 | T244I |
| 244 | T244N |
| 244 | T244S |
| 245 | Q245H |
| 248 | S248G |
| 248 | S248N |
| 248 | S248R |
| 249 | S249N |
| 249 | S249R |
| 250 | L250I |
| 251 | E251Q |
| 253 | T253I |
| 260 | S260F |
| 261 | F261L |
| 267 | L267G |
| 267 | L267M |
| 267 | L267R |
| 267 | L267V |
| 269 | N269D |
| 270 | V270I |
| 271 | Q271H |
| 272 | A272S |
| 273 | A273T |
| 273 | A273V |
| 275 | Q275Z |

**Preparation of Crude Enzyme Samples Containing BPN' Variants**

The BPN' variant proteins were produced by growing the *B. subtilis* transformants in 96 well MTP at 37°C for 68 hours in MBD medium (a MOPS based defined medium). MBD medium was made essentially as known in the art (*See,* Neidhardt et al., J. Bacteriol., 119: 736-747 [1974]), except that $NH_4Cl_2$, $FeSO_4$, and $CaCl_2$ were omitted from the base medium, 3 mM $K_2HPO_4$ was used, and the base medium was supplemented with 60 mM urea, 75 g/L glucose, and 1 % soytone. The micronutrients were made up as a 100X stock solution containing in one liter, 400 mg $FeSO_4$ $7H_2O$, 100 mg $MnSO_4$ $.H_2O$, 100 mg $ZnSO_4$ $7H_2O$, 50 mg $CuCl_2$ $2H_2O$, 100 mg $CoCl_2$ $6H_2O$, 100 mg $NaMoO_4$ $2H_2O$, 100 mg $Na_2B_4O_7$ $10H_2O$, 10 ml of 1M $CaCl_2$, and 10 ml of 0.5 M sodium citrate.

## EXAMPLE 4

### LAS Stability of BPN' Variants

In this Example, experiments conducted to assess the stability of various single-substitution BPN' variants and multiple-substitution BPN' variants in the presence of LAS are described. LAS stability was measured by determining the AAPF activity before and after incubation in the presence of LAS at elevated temperature (45°C), using the methods described in Example 1. The results of the following tables are shown as relative stability values in which the stability of the variant BPN' is compared to the wild-type BPN' enzyme. In particular the relative stability is the ratio of the variant residual activity to the wild-type residual activity. A value of greater than one indicates greater stability in the presence of LAS.

Table 4-1 contains the relative stability values of BPN' variants as compared to wild-type BPN'. As determined during development of the present invention, numerous BPN' variants had demonstrably higher stability in the presence of LAS as compared to the wild-type BPN'. In particular in the LAS stability assay of 92 BPN' sites examined, 40 sites (43%) were poor (deleterious) and 52 sites (57%) were good (beneficial). Moreover of the 1508 variants tested, 96 (6%) were superior (up), 196 (13%) were neutral (same), and 1216 (81%) were inferior (down).

**Table 4-1 LAS Stability Results for Singly-Substituted BPN' Variants**

| Variant Code | PI | Variant Code | PI | Variant Code | PI |
|---|---|---|---|---|---|
| A1C | 2.14 | P14T | 1.68 | P40F | 1.43 |
| A1D | 2 | P14V | 1.33 | P40H | 1.28 |
| A1E | 2.06 | A15C | 2.12 | P40I | 1.42 |
| A1M | 1.37 | A15D | 2.02 | P40L | 1.25 |
| A1N | 1.57 | A15E | 2.26 | P40Q | 1.32 |
| A1Q | 1.73 | A15P | 1.9 | P40R | 1.77 |
| A1S | 1.32 | L16I | 1.27 | P40W | 1.68 |
| Q2E | 1.22 | S18E | 2.08 | K43A | 1.24 |
| S3D | 2.5 | S18H | 1.15 | K43C | 1.32 |
| S3Q | 1.24 | S18I | 1.24 | K43D | 1.33 |
| Y6E | 1.84 | S18M | 1.27 | K43E | 1.26 |
| Y6W | 1.56 | S18Q | 1.12 | K43G | 1.23 |
| S9C | 1.84 | S18T | 1.13 | K43L | 1.23 |
| S9E | 2.87 | S18V | 1.28 | K43M | 1.25 |
| S9G | 1.23 | S18Y | 1.07 | K43R | 1.1 |
| S9P | 2.15 | Q19C | 1.54 | K43S | 1.32 |
| S9T | 1.28 | Q19D | 1.81 | K43W | 1.22 |

**Table 4-1 LAS Stability Results for Singly-Substituted BPN' Variants**

| | | | | | |
|---|---|---|---|---|---|
| Q10E | 1.26 | Q19E | 1.73 | M50I | 1.27 |

(continued)

| Table 4-1 LAS Stability Results for Singly-Substituted BPN' Variants | | | | | |
|---|---|---|---|---|---|
| I11V | 1.37 | G20C | 1.37 | M50K | 1.36 |
| K12A | 2.13 | G20D | 1.36 | M50K | 1.4 |
| K12C | 2.49 | G20E | 1.57 | P52K | 1.47 |
| K12D | 2.11 | G20H | 1.34 | P52R | 1.55 |
| K12E | 2.47 | T22C | 1.23 | S53E | 1.23 |
| K12F | 2.49 | T22E | 1.44 | S53K | 1.36 |
| K12G | 1.87 | T22F | 1.69 | S53R | 1.41 |
| K12H | 2.29 | T22L | 1.21 | N56D | 1.29 |
| K12I | 2.38 | T22W | 1.52 | F58C | 1.24 |
| K12N | 2.56 | T22Y | 1.68 | Q59C | 1.32 |
| K12Q | 2.15 | N25E | 1.35 | Q59D | 1.51 |
| K12S | 2.13 | N25F | 1.24 | Q59E | 1.43 |
| K12T | 1.78 | N25W | 1.27 | Q59N | 1.13 |
| K12V | 2.3 | K27G | 1.22 | H67P | 2.06 |
| K12W | 2.12 | K27P | 1.64 | S78C | 1.8 |
| K12Y | 2.41 | I35A | 1.56 | S78D | 1.69 |
| P14C | 2.23 | I35C | 1.53 | S78F | 1.39 |
| P14D | 2.89 | I35S | 1.23 | S78I | 1.28 |
| P14E | 2.55 | I35T | 1.63 | S78K | 1.61 |
| P14G | 1.11 | I35V | 1.5 | S78L | 1.21 |
| P14I | 1.37 | S37E | 1.22 | S78N | 1.28 |
| P14L | 1.44 | S37R | 1.89 | S78Q | 1.26 |
| P14N | 1.72 | P40C | 1.6 | S78R | 2.52 |
| P14S | 1.36 | P40E | 1.86 | S78W | 1.41 |
| S78Y | 1.21 | W106G | 1.36 | M124I | 1.93 |
| I79E | 1.76 | W106H | 1.29 | M124L | 1.29 |
| P86Y | 1.31 | W106I | 1.42 | L126I | 1.69 |
| S89C | 1.26 | W106L | 1.59 | L126V | 1.51 |
| S89D | 1.26 | W106M | 1.39 | L126W | 1.26 |
| S89G | 1.24 | W106R | 1.48 | G128A | 1.84 |
| Y91I | 1.29 | W106S | 1.42 | G128S | 1.63 |
| Y91V | 1.28 | W106T | 1.59 | G131P | 1.52 |
| L96I | 1.3 | W106V | 1.23 | G131R | 1.22 |
| L96V | 1.32 | W106Y | 1.38 | A133C | 1.7 |
| A98C | 1.41 | N109C | 1.42 | A133D | 1.88 |
| A98D | 1.3 | N109F | 2.04 | A133E | 1.61 |
| A98I | 1.2 | N109H | 1.36 | A133F | 1.37 |
| A98S | 1.1 | N109L | 1.84 | A133I | 1.23 |

(continued)

| Table 4-1 LAS Stability Results for Singly-Substituted BPN' Variants | | | | | |
|---|---|---|---|---|---|
| A98T | 1.08 | N109P | 1.5 | A133L | 1.33 |
| D99R | 1.25 | N109Q | 1.18 | A133P | 1.62 |
| S101C | 1.29 | N109R | 2.25 | A133R | 1.83 |
| S101E | 1.4 | N109V | 1.56 | A133W | 1.34 |
| S101G | 1.61 | N109W | 1.65 | A134D | 1.4 |
| S101P | 1.19 | N109Y | 1.22 | A134E | 1.47 |
| G102A | 1.42 | E112A | 2.9 | A134G | 1.22 |
| G102S | 1.27 | E112C | 2.08 | K136E | 2.28 |
| Q103C | 1.39 | E112F | 2.75 | K136H | 1.41 |
| Q103F | 1.42 | E112G | 2.84 | A137C | 1.22 |
| Q103G | 1.65 | E112I | 2.85 | A137F | 1.86 |
| Q103I | 1.4 | E112L | 2.68 | A137H | 1.27 |
| Q103K | 1.63 | E112M | 2.86 | A137K | 1.55 |
| Q103M | 1.22 | E112N | 1.98 | A137L | 1.35 |
| Q103N | 1.25 | E112Q | 2.53 | A137M | 1.25 |
| Q103R | 1.58 | E112S | 2.68 | A137R | 2.01 |
| Q103T | 1.5 | E112T | 2.56 | A137W | 1.92 |
| Q103W | 1.69 | E112V | 2.59 | A137Y | 1.51 |
| S105A | 1.29 | E112W | 2.84 | V139C | 2.09 |
| S105C | 1.27 | E112Y | 2.53 | K141C | 1.54 |
| S105D | 1.46 | W113N | 1.22 | K141D | 1.31 |
| S105E | 1.38 | I115N | 1.45 | K141E | 1.29 |
| S105K | 1.89 | I115R | 1.7 | K141F | 1.5 |
| S105L | 1.25 | I115T | 1.31 | K141G | 1.44 |
| W106A | 1.31 | A116D | 1.23 | K141H | 1.58 |
| W106C | 1.53 | A116K | 1.55 | K141I | 1.54 |
| W106E | 1.69 | A116R | 1.62 | K141L | 1.46 |
| W106F | 1.43 | N118C | 1.23 | K141N | 1.43 |
| K141Q | 1.39 | S 183C | 1.92 | Y217L | 1.54 |
| K141S | 1.28 | S183E | 2.48 | Y217M | 2.3 |
| K141W | 1.64 | S183N | 1.82 | Y217N | 1.26 |
| K141Y | 1.28 | S183Q | 1.46 | Y217Q | 1.55 |
| V143D | 1.28 | N184C | 1.61 | Y217R | 1.26 |
| V143E | 1.37 | Q185C | 1.65 | N218C | 1.29 |
| A144C | 1.52 | Q185E | 2.41 | N218S | 2.38 |

(continued)

| Table 4-1 LAS Stability Results for Singly-Substituted BPN' Variants | | | | | |
|---|---|---|---|---|---|
| A144D | 1.58 | S188C | 1.52 | N218T | 1.45 |
| A144E | 1.47 | S188D | 2.58 | S224C | 1.61 |
| A144I | 1.24 | S188E | 2.55 | P225A | 2.82 |
| A144K | 1.59 | S188P | 1.36 | P225C | 1.43 |
| A144L | 1.29 | P194C | 1.5 | P225G | 2.86 |
| A144R | 2.42 | P194E | 1.73 | P225I | 1.69 |
| A144W | 1.8 | Q206C | 1.91 | P225S | 2.39 |
| S145C | 1.41 | Q206D | 2.47 | P225V | 2.14 |
| S145D | 1.26 | Q206E | 2.77 | G229A | 1.59 |
| S145E | 1.27 | Q206L | 1.23 | A230G | 1.29 |

| Table 4-1 LAS Stability Results for Singly-Substituted BPN' Variants | | | | | |
|---|---|---|---|---|---|
| S145F | 1.29 | Q206M | 1.23 | 1234V | 1.26 |
| S145L | 1.26 | Q206W | 1.39 | L235G | 1.46 |
| S 145M | 1.36 | Q206Y | 1.43 | L235I | 1.54 |
| S145R | 1.65 | N212C | 1.22 | L235K | 1.21 |
| E156C | 1.3 | N212E | 1.51 | L235M | 1.58 |
| E156Y | 1.26 | K213A | 2.14 | L235N | 1.39 |
| T158D | 1.8 | K213C | 2.29 | L235Q | 1.63 |
| T158E | 2.16 | K213D | 2.59 | L235S | 1.51 |
| T158V | 1.31 | K213E | 2.44 | L235W | 1.92 |
| S159C | 1.84 | K213H | 1.72 | L235Y | 1.95 |
| S159D | 1.68 | K213I | 1.3 | S236E | 1.35 |
| S159Q | 1.22 | K213L | 1.58 | S236H | 1.29 |
| S162C | 1.77 | K213M | 2.1 | H238F | 1.42 |
| S162E | 2.1 | K213N | 2.21 | P239N | 1.22 |
| S162M | 1.31 | K213Q | 2.12 | P239T | 1.32 |
| S162N | 1.45 | K213S | 2.34 | P239V | 1.26 |
| S162Q | 1.52 | K213T | 2.31 | T244D | 1.47 |
| G169A | 2.88 | K213V | 1.3 | T244F | 1.3 |
| K170A | 1.54 | A216C | 2.07 | T244H | 1.45 |
| P172E | 2.3 | A216D | 2.09 | T244K | 1.29 |
| P172R | 1.23 | A216E | 2.62 | T244R | 1.73 |
| A176C | 2 | Y217C | 2.31 | Q245C | 1.31 |
| S182C | 1.64 | Y217D | 1.46 | Q245D | 1.37 |
| S182D | 2.02 | Y217E | 2.11 | Q245E | 1.32 |
| S182Q | 1.39 | Y217K | 1.42 | S248E | 1.47 |

(continued)

| Table 4-1 LAS Stability Results for Singly-Substituted BPN' Variants | | | | | |
|---|---|---|---|---|---|
| N252C | 1.36 | K256M | 2.23 | K265C | 1.92 |
| N252E | 1.81 | K256N | 2.5 | K265E | 1.83 |
| T254A | 2.1 | K256P | 2.82 | K265H | 2.28 |
| T254C | 2.32 | K256Q | 2.3 | K265L | 1.38 |
| T255C | 1.24 | K256S | 2.23 | K265M | 1.48 |
| T255D | 1.54 | K256T | 2.22 | K265N | 2.19 |
| T255E | 2.29 | K256V | 2.4 | K265Q | 2.1 |
| K256A | 2.61 | K256W | 2.79 | K265S | 1.49 |
| K256C | 2.44 | K256Y | 2.71 | K265Y | 1.7 |
| K256D | 2.81 | S260C | 1.25 | N269D | 1.95 |
| K256E | 2.81 | S260D | 1.86 | Q271C | 1.53 |
| K256F | 2.4 | S260E | 2.6 | Q271D | 1.86 |
| K256H | 2.42 | S260P | 2.49 | Q271E | 2.24 |
| K256I | 2.37 | K265A | 1.71 | A272E | 1.5 |
| K256L | 1.99 | S101Y | 1.26 | G166S | 2.41 |
| V008I | 1.41 | G102A | 1.41 | G169A | 1.59 |
| T022E | 1.62 | G102S | 1.57 | K170A | 1.26 |

| Table 4-1 LAS Stability Results for Singly-Substituted BPN' Variants | | | | | |
|---|---|---|---|---|---|
| T022F | 1.57 | Q103A | 1.18 | K170E | 1.58 |
| T022M | 1.23 | Q103C | 1.67 | V203C | 1.19 |
| T022S | 1.18 | Q103E | 1.48 | S101Q | 1.19 |
| T022W | 1.65 | Q103F | 1.47 | S101T | 1.21 |
| T022Y | 1.69 | Q103I | 1.3 | S101V | 1.55 |
| V0026F | 1.18 | Q103L | 1.2 | G166N | 2.21 |
| V0026G | 1.18 | Q103S | 1.2 | G166Q | 2.53 |
| I031E | 1.25 | Q103T | 1.26 | G166R | 1.63 |
| I031F | 1.17 | Q103V | 1.18 | S101I | 1.17 |
| I031H | 1.24 | Q103W | 1.34 | S101L | 1.28 |
| I031N | 1.23 | Y104F | 1.28 | S101N | 1.17 |
| I031S | 1.2 | Y104W | 1.58 | G166H | 2.19 |
| I031T | 1.32 | M124I | 2.02 | G166K | 1.17 |
| A0045E | 1.28 | V147A | 1.17 | G166M | 2.65 |
| H067P | 1.32 | V147E | 1.26 | G097E | 1.2 |
| V072A | 1.38 | V147S | 1.35 | S101C | 1.25 |
| V072C | 1.25 | V149A | 1.82 | S101E | 1.42 |
| S087D | 1.32 | V149C | 1.43 | V149S | 1.49 |

(continued)

| Table 4-1 LAS Stability Results for Singly-Substituted BPN' Variants | | | | | |
|---|---|---|---|---|---|
| A092S | 1.22 | V149D | 1.39 | V149T | 1.22 |
| L096I | 1.39 | V149E | 2.06 | G166C | 2.3 |
| L096M | 1.34 | V149F | 1.68 | G097D | 1.43 |
| | | | | V149P | 1.23 |

| Table 4-2 LAS Stability Results for Multiply-Substituted BPN' Variants | |
|---|---|
| **Variant Code** | **PI** |
| S024S-V028V-M050M-A092A-Q103E-A114G-V246V | 1.21 |
| S024S-V028V-M0S0V-A092A-Q103Q-A114A-V246V | 1.10 |
| S024S-V028V-M050V-A092A-Q103Q-A114G-V246V | 1.22 |
| S024H-V028V-M050V-A092A-Q103Q-A114A-V246T | 1.20 |
| S024H-V028V-M050V-A092A-Q103E-A114A-V246V | 1.24 |
| S024H-V028V-M050V-A092A-Q103Q-A114G-V246V | 1.18 |
| S024H-V028V-M050M-A092A-Q103E-A114A-V246V | 1.11 |
| S024H-V028V-M050M-A092A-Q103E-A114A-V246T | 1.22 |
| S024S-V028V-M050M-A092G-Q103Q-A114A-V246T | 1.11 |
| S024H-V028V-M050M-A092A-Q103Q-A114G-V246T | 1.14 |
| S101E-M119N-K213N | 1.65 |
| S101N-K213I | 1.22 |
| S101N-M119H | 1.12 |
| M119H-K213I-Y217L | 1.43 |
| S101N-M119H-K213N-Y217L | 1.53 |
| S101N-K213L-Y217E | 1.79 |
| M119N-K213N-Y217L | 1.71 |
| M119F-K213L-Y217E | 1.62 |
| S101P-K213N | 1.64 |
| S101N-M119H-K213N | 1.63 |
| S101N-M119F-K213I-Y217L | 1.43 |
| K213L | 1.38 |
| M119N-K213N | 1.70 |
| K213N | 1.51 |
| K213I-Y217E | 1.36 |
| S101N-M119H-Y217Q | 1.41 |
| S101P-K213N-Y217L | 1.66 |
| M119N-K213I | 1.25 |
| K213N-Y217Q | 1.66 |
| M119H-K213N | 1.62 |

(continued)

| Table 4-2 LAS Stability Results for Multiply-Substituted BPN' Variants | |
|---|---|
| Variant Code | PI |
| S101N-K213L-Y217Q | 1.49 |
| S101P-K213N | 1.66 |
| S101E-K213N-Y217E | 1.81 |
| S101E-M119H-K213I-Y217Q | 1.52 |
| S101E-M119H-K213N | 1.65 |
| K213I-Y217Q | 1.27 |
| S101N-K213I-Y217Q | 1.53 |
| M119H-Y217Q | 1.39 |
| S101N-M119N-K213N-Y217Q | 1.89 |
| M119H-K213I-Y217Q | 1.44 |
| K213I-Y217Q | 1.24 |
| S101E-M119N-Y217L | 1.45 |
| M119F-K213L | 1.34 |
| M119H-K213N-Y217E | 1.91 |
| S101N-M119N-K213N-Y217Q | 1.75 |
| S101N-M119H-K213N-Y217Q | 1.75 |
| M119H-K213I-Y217Q | 1.93 |
| Y217Q | 1.18 |
| M119H-K213N-Y217Q | 1.73 |
| S101N-M119F-Y217E | 1.39 |
| M119F-Y217Q | 1.17 |
| S101N-M119F-K213I-Y217Q | 1.22 |
| S101N-K213I-Y217L | 1.19 |
| S101N-M119H-K213N-Y217L | 1.44 |
| S101E-K213L-Y217L | 1.54 |
| S101N-K213N-Y217Q | 1.45 |
| S101N-M119H-K213L | 1.29 |
| S101N-M119H-K213I | 1.22 |
| S101P-K213N-Y217L | 1.46 |
| M119F-K213I-Y217Q | 1.24 |
| S101N-K213I-Y217Q | 1.24 |
| S101E-M119H-K213I-Y217L | 1.92 |
| S101N-M119H-K213I-Y217Q | 1.58 |
| M119H-K213N-Y217E | 1.49 |
| S101N-M119N-K213N-Y217L | 1.46 |
| A048E-K213L | 1.28 |
| A048H-K213L | 1.25 |

(continued)

| Table 4-2 LAS Stability Results for Multiply-Substituted BPN' Variants | |
|---|---|
| Variant Code | PI |
| V026Q-A048Y-K213L | 1.19 |
| K213N | 1.34 |
| V026N-K213L | 1.24 |
| V026N-K213L | 1.23 |
| V026Y-K213N | 1.44 |
| A048D-K213N | 1.22 |
| V026Q-A048E-K213L | 1.24 |
| A048H-K213N | 1.34 |
| V026Q-A048H-K213L | 1.24 |
| A048H-K213L | 1.28 |
| K213L | 1.25 |
| K213N | 1.51 |
| V147D-K213L | 1.31 |
| K213I | 1.10 |
| V026Q-A048E-K213N | 1.28 |
| V026N-A048E-K213N | 1.48 |
| A048E-K213L | 1.92 |
| V026Y-A048E-K213I | 1.71 |
| A048D-K213N | 1.46 |
| K213I | 1.10 |
| A048H-K213N | 1.54 |
| V147D-K213N | 1.54 |
| Y021H-A045V-S101E-Y217L | 1.21 |
| Y021H-Y217L | 1.15 |
| Y021H-A045V-S101E-Y217Q | 1.29 |
| Y021H-A0451-S101E-Y217L | 1.20 |
| Y021H-Y217Q | 1.14 |
| Y021H-A045V-Y217E | 1.36 |
| A045V-Y217L | 1.14 |
| Y021H-A045V-S101N-Y217Q | 1.25 |
| Y021W-S101P-Y217L | 1.13 |
| Y021W-A045I-Y217E | 1.32 |
| Y021H-A045V-S101E-Y217E | 1.50 |
| Y021H-A0451-S101E-Y217L | 1.25 |
| Y021H-A045I-S101E-Y217Q | 1.36 |
| Y021H-A045V-Y217E | 1.31 |
| Y021W-S101E-Y217L | 1.27 |

(continued)

| Table 4-2 LAS Stability Results for Multiply-Substituted BPN' Variants | |
|---|---|
| **Variant Code** | **PI** |
| S101E-Y217L | 1.32 |
| Y021H-A045V-Y217Q | 1.19 |
| Y021H-Y217E | 1.46 |
| Y021W-Y217E-(N0212S) | 1.24 |
| A045I-Y217Q | 1.15 |
| Y021H-A045V-Y217E | 1.43 |
| Y021W-A045V-S101E-Y217Q | 1.25 |
| Y021H-S101E-Y217E | 1.49 |
| Y021W-S101N-Y217L | 1.16 |
| S024S-V028V-M050M-A092A-Q103Q-A114A-V246T | 1.17 |
| Y021H-A045V-S101E | 1.15 |
| Y021W-A045V-S101E-Y217L | 1.25 |
| S101N-M119H-K213K-Y217Q | 1.38 |
| S101S-M119N-K213N-Y217Q | 1.68 |
| Y021H-A045V-S101P-Y217L | 1.23 |
| S024S-V028V-M050M-A092A-Q103Q-A114G-V246T | 1.29 |
| S101S-M119F-K213I-Y217Q | 1.34 |
| S101S-M119M-K213K-Y217L | 1.35 |
| Y021H-A045I-S101E-Y217Q | 1.32 |
| Y021H-A045V-S101E-Y217E | 1.57 |
| Y021H-A045V-S101E | 1.19 |
| Y021H-Y217L | 1.18 |
| Y021H-A045I-Y217E | 1.54 |
| Y021W-A045I-S101E-Y217E | 1.47 |
| S101N-M119F-K213K-Y217L | 1.26 |
| S101E-M119H-K213N-Y217E | 1.37 |
| Y021H-A045I-Y217E | 1.44 |
| S101S-M119H-K213L-Y217L | 1.56 |
| Y021H-Y217E | 1.67 |
| S101S-M119F-K213K-Y217Q | 1.25 |
| Y021H-A045I-S101E-Y217L | 1.46 |

**EXAMPLE** 5

**Specific Activity of BPN'-Variants**

In this Example, experiments conducted to determine the relative specific activity of BPN' and BPN'-variants is described. Specific activity towards AAPF was measured using methods provided in Example 1. In Table 5-1, results are shown as relative specific activity values in which the activity of BPN' variants is compared to activity of wild-type BPN'.

In particular the relative specific activity is the ratio of the variant specific activity to the wild-type specific activity. A value greater than one (PI>1) indicates higher specific activity towards the AAPF substrate.

**Table 5-1 Specific Activity of Singly-Substituted BPN' Variants**

| Variant Code | PI | Variant Code | PI | Variant Code | PI |
|---|---|---|---|---|---|
| Y006N | 1.25 | I115R | 1.30 | I175V | 2.81 |
| Y006T | 1.21 | G131P | 1.29 | Y217C | 1.33 |
| Q019E | 1.26 | G131R | 1.21 | Y217L | 3.96 |
| 1035T | 1.17 | A137W | 1.18 | Y217M | 1.85 |
| L042V | 1.19 | A137Y | 1.18 | Y217S | 1.48 |
| M050K | 1.32 | V139C | 1.23 | N218S | 1.22 |
| M050R | 1.51 | K141F | 1.18 | S236V | 1.21 |
| M050T | 1.35 | K141I | 1.39 | H238R | 1.46 |
| Q059W | 1.59 | K141S | 1.19 | K256F | 1.17 |
| P086M | 1.44 | K141Y | 1.25 | K256P | 1.26 |
| P086Y | 1.22 | V143D | 1.21 | K256Q | 1.32 |
| A098I | 1.26 | V143N | 1.35 | K256W | 1.20 |
| A098T | 1.34 | V143Q | 1.41 | K256Y | 1.27 |
| A098V | 1.49 | S 145M | 1.25 | | |
| N109Q | 1.19 | S145R | 1.36 | | |
| N109T | 1.28 | G146M | 1.39 | | |
| N109V | 1.45 | K170A | 1.32 | | |

**EXAMPLE 6**

**Comparative Evaluation of BPN'-Variant Data**

In this Example, results of experiments conducted to determine protein expression, stain removal activity, LAS stability, and AAPF activity (tests of properties of interest) of BPN' and BPN'-variants are described. The results were obtained using the methods described in Example 1. As described throughout, functionality of the BPN' variants was quantified as a performance index (PI), which is the ratio of performance of a variant to a parent protein. PI gradations used herein include: Up mutations (PI > 1.0); Neutral mutations (PI > 0.5); Non-deleterious mutations (PI > 0.05); and Deleterious mutations (PI ≤ 0.05). "Combinable mutations" are those mutations for which the variant has Performance index values = 0.5 for at least one property, and >0.05 for all properties. Combinable mutations are mutations that can be combined to deliver proteins with appropriate Performance indices for one or more desired properties. Positions at which mutations occur are classed as follows: Non-restrictive positions have ≥ 20% neutral mutations for at least one property; and Restrictive positions have < 20% neutral mutations for activity and stability.

These data find use in engineering any subtilisin. Even if the subtilisin to be engineered has an amino acid different from that of subtilisin BPN' at a particular position, this data find use in identifying at least one substitution that will alter the desired properties by identifying the best choice substitution, including substitution to the BPN' wild type amino acid.

Table 6-1 provides Performance index values (Pi) for 5,004 variants of subtilisin BPN' at 275 positions. Performance indices less than or equal to 0.05 were fixed to 0.05 and indicated in bold italics in the table. Also, for the stability measure, if the Performance index of activity in the stability assays was less than or equal to 0.05, the associated stability performance index was fixed to 0.05.

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 1 | A001C | 0.74 | 0.84 | 1.10 | 0.79 | 1.1 | 0.87 |
| 1 | A001D | 1.34 | 0.93 | 1.12 | 0.88 | 1.1 | 0.88 |
| 1 | A001E | 0.82 | 0.94 | 0.85 | 0.84 | 1.5 | 0.94 |
| 1 | A001F | 1.39 | 0.94 | 1.01 | 0.84 | 0.4 | 0.90 |
| 1 | A001G | 1.39 | 0.90 | 1.12 | 1.06 | 1.1 | 1.04 |
| 1 | A001H | 1.26 | 0.99 | 1.04 | 0.98 | 0.9 | 0.97 |
| 1 | A001I | 1.50 | 0.72 | 1.00 | 0.92 | 0.6 | 0.88 |
| 1 | A001K | 1.23 | 0.83 | 0.79 | 1.11 | 0.2 | 0.96 |
| 1 | A001L | 0.96 | 0.86 | 0.81 | 0.94 | 0.6 | 0.94 |
| 1 | A001M | 1.34 | 1.02 | 1.07 | 1.08 | 0.9 | 0.99 |
| 1 | A001N | 1.29 | 1.02 | 0.81 | 0.98 | 1.0 | 1.05 |
| 1 | A001P | 0.27 | 1.16 | 1.46 | 1.14 | 0.7 | 0.66 |
| 1 | A001Q | 0.88 | 1.00 | 0.97 | 0.98 | 1.2 | 1.02 |
| 1 | A001R | 1.00 | 0.89 | 0.72 | 0.91 | 0.1 | 1.00 |
| 1 | A001S | 1.09 | 0.89 | 1.00 | 1.05 | 0.9 | 1.08 |
| 1 | A001T | 1.13 | 1.00 | 1.16 | 1.02 | 1.0 | 1.01 |
| 1 | A001V | 1.32 | 0.83 | 0.68 | 0.96 | 0.8 | 0.96 |
| 1 | A001W | 1.19 | 0.76 | 0.67 | 0.88 | 0.2 | 0.92 |
| 1 | A001Y | ***0.05*** | ***0.05*** | ***0.05*** | ***0.05*** | ***0.05*** | ***0.05*** |
| 2 | Q002A | 0.78 | 0.88 | 0.99 | 1.01 | ***0.05*** | 0.94 |
| 2 | Q002C | 0.55 | 0.81 | 1.26 | 0.92 | 0.1 | 0.74 |
| 2 | Q002D | 1.01 | 0.94 | 0.96 | 1.02 | ***0.05*** | 0.96 |
| 2 | Q002E | 1.18 | 0.87 | 1.01 | 0.79 | ***0.05*** | 0.85 |
| 2 | Q002F | 0.44 | 1.18 | 0.91 | 0.97 | ***0.05*** | 0.78 |
| 2 | Q002G | 0.80 | 1.11 | 0.31 | 1.02 | ***0.05*** | 0.71 |
| 2 | Q002H | ***0.05*** | ***0.05*** | ***0.05*** | ***0.05*** | ***0.05*** | ***0.05*** |
| 2 | Q002I | 0.37 | 1.16 | 0.99 | 1.08 | ***0.05*** | 0.77 |
| 2 | Q002K | 0.40 | 1.02 | 0.75 | 1.03 | ***0.05*** | 0.86 |
| 2 | Q002L | 0.34 | 0.90 | 1.24 | 1.15 | ***0.05*** | 0.70 |
| 2 | Q002M | 0.47 | 0.96 | 0.92 | 0.92 | ***0.05*** | 0.79 |
| 2 | Q002N | ***0.05*** | ***0.05*** | ***0.05*** | ***0.05*** | ***0.05*** | ***0.05*** |
| 2 | Q002P | 0.76 | 0.84 | 0.82 | 1.12 | ***0.05*** | 0.94 |
| 2 | Q002R | 0.35 | 0.79 | 0.84 | 1.11 | ***0.05*** | 0.78 |
| 2 | Q002T | 0.77 | 0.93 | 1.23 | 0.80 | ***0.05*** | 0.88 |
| 2 | Q002T | 0.38 | 0.93 | 0.95 | 0.93 | ***0.05*** | 0.82 |
| 2 | Q002V | 0.42 | 1.17 | 0.96 | 1.17 | ***0.05*** | 0.86 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 2 | Q002W | 0.17 | 3.77 | 6.93 | 2.44 | ***0.05*** | 0.47 |
| 2 | Q002Y | 0.44 | 0.93 | 0.85 | 1.03 | ***0.05*** | 0.86 |
| 3 | S003A | 1.19 | 1.20 | 0.97 | 0.89 | 0.3 | 0.93 |
| 3 | S003C | ***0.05*** | ***0.05*** | ***0.05*** | ***0.05*** | ***0.05*** | ***0.05*** |
| 3 | S003D | 1.24 | 1.11 | 1.08 | 0.97 | 1.0 | 1.02 |
| 3 | S003E | ***0.05*** | ***0.05*** | ***0.05*** | ***0.05*** | ***0.05*** | ***0.05*** |
| 3 | S003F | 1.20 | 0.90 | 0.95 | 0.86 | 0.2 | 1.02 |
| 3 | S003G | 1.45 | 0.96 | 1.03 | 1.05 | ***0.05*** | 0.91 |
| 3 | S003H | ***0.05*** | ***0.05*** | ***0.05*** | ***0.05*** | ***0.05*** | ***0.05*** |
| 3 | S003I | 1.24 | 0.80 | 0.90 | 1.05 | 1.0 | 0.92 |
| 3 | S003K | 1.28 | 0.97 | 0.80 | 0.95 | 0.1 | 1.09 |
| 3 | S003L | 1.13 | 0.90 | 0.84 | 1.06 | 0.6 | 0.92 |
| 3 | S003M | 1.35 | 0.83 | 1.04 | 0.92 | 1.1 | 0.84 |
| 3 | S003N | 1.18 | 0.98 | 1.15 | 0.85 | 0.5 | 1.01 |
| 3 | S003P | 1.01 | 1.08 | 1.09 | 0.89 | ***0.05*** | 0.90 |
| 3 | S003Q | 1.38 | 0.97 | 0.96 | 1.08 | 1.3 | 1.00 |
| 3 | S003R | 1.37 | 0.75 | 0.62 | 1.00 | ***0.05*** | 0.98 |
| 3 | S003T | 1.31 | 1.09 | 1.01 | 0.87 | 1.2 | 0.90 |
| 3 | S003V | 1.19 | 0.84 | 0.80 | 1.06 | 1.0 | 0.99 |
| 3 | S003W | 1.50 | 0.71 | 0.65 | 0.91 | 0.1 | 0.84 |
| 3 | S003Y | 1.25 | 0.76 | 0.72 | 0.92 | 0.2 | 0.88 |
| 4 | V004A | 0.83 | 0.90 | 1.07 | 1.00 | ***0.05*** | 0.95 |
| 4 | V004C | 0.87 | 1.03 | 1.22 | 0.92 | 0.3 | 1.09 |
| 4 | V004D | 0.78 | 0.87 | 1.06 | 0.98 | ***0.05*** | 0.91 |
| 4 | V004E | 1.00 | 1.04 | 1.22 | 0.92 | 0.1 | 0.99 |
| 4 | V004F | ***0.05*** | ***0.05*** | ***0.05*** | ***0.05*** | ***0.05*** | ***0.05*** |
| 4 | V004G | 0.45 | 0.86 | 1.15 | 1.14 | ***0.05*** | 0.78 |
| 4 | V004H | ***0.05*** | ***0.05*** | ***0.05*** | ***0.05*** | ***0.05*** | ***0.05*** |
| 4 | V004I | ***0.05*** | ***0.05*** | ***0.05*** | ***0.05*** | ***0.05*** | ***0.05*** |
| 4 | V004K | 0.89 | 0.83 | 1.01 | 1.08 | ***0.05*** | 1.24 |
| 4 | V004L | 1.07 | 0.97 | 1.12 | 0.94 | ***0.05*** | 1.13 |
| 4 | V004M | 1.14 | 1.06 | 1.03 | 0.98 | ***0.05*** | 0.87 |
| 4 | V004N | 0.98 | 1.22 | 1.02 | 1.09 | ***0.05*** | 0.93 |
| 4 | V004P | 0.75 | 1.19 | 1.16 | 0.92 | ***0.05*** | 1.00 |
| 4 | V004Q | 0.91 | 0.85 | 1.16 | 0.78 | ***0.05*** | 1.30 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 4 | V004R | 1.01 | 0.83 | 0.90 | 0.85 | ***0.05*** | 1.20 |
| 4 | V004S | 0.80 | 0.91 | 1.14 | 0.77 | ***0.05*** | 1.00 |
| 4 | V004T | 1.02 | 0.92 | 1.27 | 0.97 | 1.2 | 1.20 |
| 4 | V004W | 0.93 | 0.99 | 0.92 | 0.99 | ***0.05*** | 1.16 |
| 4 | V004Y | 0.79 | 0.93 | 1.03 | 1.08 | ***0.05*** | 0.89 |
| 5 | P005A | 0.53 | 0.86 | 0.73 | 0.79 | ***0.05*** | 0.60 |
| 5 | P005C | 0.70 | 0.94 | 0.80 | 0.92 | ***0.05*** | 0.60 |
| 5 | P005D | ***0.05*** | ***0.05*** | ***0.05*** | ***0.05*** | ***0.05*** | ***0.05*** |
| 5 | P005E | 0.79 | 1.02 | 0.79 | 0.94 | ***0.05*** | 0.84 |
| 5 | P005F | 0.22 | 0.55 | 0.44 | 0.61 | 0.1 | 0.23 |
| 5 | P005G | 0.98 | 1.09 | 1.10 | 0.98 | 0.3 | 1.13 |
| 5 | P005H | 0.38 | 0.96 | 0.88 | 1.09 | ***0.05*** | 0.64 |
| 5 | P005I | 0.21 | 0.49 | 0.96 | 1.02 | 0.1 | 0.20 |
| 5 | P005K | 0.16 | 4.53 | 2.60 | 0.65 | 0.6 | 0.06 |
| 5 | P005L | 0.21 | 1.50 | 1.40 | 1.49 | ***0.05*** | 0.47 |
| 5 | P005M | 0.41 | 1.02 | 1.11 | 1.13 | ***0.05*** | 0.84 |
| 5 | P005N | 0.68 | 0.91 | 0.91 | 1.00 | ***0.05*** | 0.78 |
| 5 | P005Q | 0.70 | 1.08 | 0.84 | 0.97 | ***0.05*** | 0.60 |
| 5 | P005R | 0.16 | ***0.05*** | ***0.05*** | 1.56 | 0.3 | 0.07 |
| 5 | P005S | 0.74 | 0.98 | 1.09 | 1.01 | ***0.05*** | 0.81 |
| 5 | P005T | 0.73 | 1.16 | 0.91 | 0.97 | ***0.05*** | 1.46 |
| 5 | P005V | 0.27 | 0.72 | 1.30 | 1.14 | ***0.05*** | 0.73 |
| 5 | P005W | 0.43 | 0.79 | 0.82 | 0.76 | ***0.05*** | 0.60 |
| 5 | P005Y | 0.17 | 3.33 | 3.27 | 2.19 | ***0.05*** | 0.35 |
| 6 | Y006A | 1.02 | 1.14 | 1.03 | 1.01 | 0.8 | 0.97 |
| 6 | Y006C | 1.04 | 1.03 | 0.95 | 0.83 | 0.7 | 0.91 |
| 6 | Y006D | ***0.05*** | ***0.05*** | ***0.05*** | ***0.05*** | ***0.05*** | ***0.05*** |
| 6 | Y006E | 1.49 | 1.13 | 0.76 | 1.00 | 1.1 | 0.81 |
| 6 | Y006F | 1.17 | 0.84 | 0.88 | 0.81 | 0.6 | 0.92 |
| 6 | Y006G | 1.00 | 1.20 | 1.09 | 0.78 | 0.7 | 0.86 |
| 6 | Y006H | ***0.05*** | ***0.05*** | ***0.05*** | ***0.05*** | ***0.05*** | ***0.05*** |
| 6 | Y006I | ***0.05*** | ***0.05*** | ***0.05*** | ***0.05*** | ***0.05*** | ***0.05*** |
| 6 | Y006K | 1.36 | 0.90 | 0.70 | 0.92 | ***0.05*** | 0.91 |
| 6 | Y006L | 0.15 | ***0.05*** | ***0.05*** | ***0.05*** | 0.7 | 0.16 |
| 6 | Y006M | 1.31 | 1.16 | 1.04 | 0.96 | 0.6 | 0.86 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 6 | Y006N | 1.12 | 1.20 | 0.97 | 0.81 | 0.6 | 0.93 |
| 6 | Y006P | 0.92 | 1.10 | 0.77 | 0.96 | 0.7 | 1.13 |
| 6 | Y006Q | 1.36 | 1.29 | 1.19 | 0.87 | 0.9 | 0.94 |
| 6 | Y006R | 0.75 | 0.76 | 0.61 | 0.93 | ***0.05*** | 0.92 |
| 6 | Y006S | 0.83 | 1.08 | 1.20 | 0.96 | 0.5 | 0.96 |
| 6 | Y006T | 1.13 | 1.02 | 1.14 | 1.12 | 0.7 | 0.85 |
| 6 | Y006V | 1.39 | 1.02 | 0.97 | 0.91 | 0.7 | 1.06 |
| 6 | Y006W | 1.96 | 1.06 | 1.00 | 0.96 | 1.1 | 0.82 |
| 7 | G007A | 0.73 | 0.88 | 0.77 | 1.07 | ***0.05*** | 0.60 |
| 7 | G007C | 0.20 | 0.75 | 0.37 | 1.11 | 0.2 | 0.08 |
| 7 | G007F | 0.22 | 0.08 | ***0.05*** | ***0.05*** | ***0.05*** | ***0.05*** |
| 7 | G0071 | 0.34 | 0.06 | ***0.05*** | ***0.05*** | ***0.05*** | ***0.05*** |
| 7 | G007K | 0.35 | 0.11 | ***0.05*** | ***0.05*** | ***0.05*** | ***0.05*** |
| 7 | G007L | 0.37 | *0.05* | ***0.05*** | ***0.05*** | ***0.05*** | ***0.05*** |
| 7 | G007M | 0.27 | ***0.05*** | ***0.05*** | ***0.05*** | ***0.05*** | ***0.05*** |
| 7 | G007N | 0.95 | 0.85 | 0.85 | 0.95 | ***0.05*** | 0.66 |
| 7 | G007Q | 0.15 | ***0.05*** | ***0.05*** | ***0.05*** | ***0.05*** | ***0.05*** |
| 7 | G007R | 0.25 | 0.16 | ***0.05*** | ***0.05*** | ***0.05*** | ***0.05*** |
| 7 | G007S | 0.85 | 0.72 | 0.70 | 0.96 | ***0.05*** | 0.67 |
| 7 | G007T | 0.19 | 2.98 | 2.43 | 3.02 | 0.1 | 0.21 |
| 7 | G007V | 0.34 | ***0.05*** | ***0.05*** | ***0.05*** | ***0.05*** | ***0.05*** |
| 7 | G007W | 0.16 | ***0.05*** | ***0.05*** | 4.88 | ***0.05*** | ***0.05*** |
| 7 | G007Y | 0.20 | 0.39 | ***0.05*** | ***0.05*** | ***0.05*** | ***0.05*** |
| 8 | V008A | 0.57 | 1.02 | 0.78 | 0.98 | ***0.05*** | 0.69 |
| 8 | V008C | 0.81 | 0.87 | 0.93 | 1.10 | 0.1 | 0.83 |
| 8 | V008D | 0.27 | ***0.05*** | ***0.05*** | ***0.05*** | ***0.05*** | ***0.05*** |
| 8 | V008F | 0.46 | 0.77 | 0.76 | 1.07 | ***0.05*** | 0.70 |
| 8 | V008G | 0.26 | 1.38 | 0.49 | 1.41 | ***0.05*** | 0.41 |
| 8 | V008H | 0.39 | 0.88 | 0.66 | 1.25 | ***0.05*** | 0.43 |
| 8 | V008I | 0.91 | 1.06 | 0.87 | 1.01 | 1.0 | 0.93 |
| 8 | V008K | 0.19 | 3.07 | 3.04 | 3.06 | 0.2 | 0.45 |
| 8 | V008L | 0.83 | 0.91 | 0.98 | 1.03 | 0.2 | 0.80 |
| 8 | V008M | 0.85 | 0.95 | 0.98 | 0.92 | ***0.05*** | 0.74 |
| 8 | V008P | 0.19 | 2.71 | 3.00 | 2.94 | ***0.05*** | 0.55 |
| 8 | V008Q | 0.30 | 0.94 | 0.74 | 1.43 | ***0.05*** | 0.63 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 8 | V008R | 0.21 | 0.83 | 0.14 | 1.14 | 0.2 | 0.06 |
| 8 | V008S | 0.30 | 1.14 | 0.89 | 1.37 | ***0.05*** | 0.62 |
| 8 | V008T | 0.74 | 0.76 | 0.69 | 1.10 | ***0.05*** | 0.79 |
| 8 | V008Y | 0.13 | ***0.05*** | ***0.05*** | ***0.05*** | 0.3 | 0.07 |
| 9 | S009A | 1.22 | 0.84 | 1.00 | 0.98 | 0.8 | 0.97 |
| 9 | S009C | 1.32 | 0.94 | 0.80 | 0.79 | 1.2 | 1.15 |
| 9 | S009D | 0.43 | ***0.05*** | ***0.05*** | ***0.05*** | ***0.05*** | ***0.05*** |
| 9 | S009E | 1.89 | 0.91 | 0.76 | 0.92 | 1.5 | 0.91 |
| 9 | S009F | 1.43 | 1.06 | 0.79 | 1.04 | ***0.05*** | 0.96 |
| 9 | S009G | 1.19 | 0.88 | 0.92 | 0.88 | 0.7 | 0.91 |
| 9 | S009H | 1.35 | 0.70 | 1.08 | 1.00 | 0.8 | 0.95 |
| 9 | S009I | ***0.05*** | ***0.05*** | ***0.05*** | ***0.05*** | ***0.05*** | ***0.05*** |
| 9 | S009K | 0.96 | 0.76 | 0.66 | 0.95 | ***0.05*** | 1.13 |
| 9 | S009L | 1.30 | 0.99 | 0.76 | 0.86 | 0.6 | 1.05 |
| 9 | S009M | 1.32 | 0.98 | 0.95 | 0.99 | 0.8 | 1.17 |
| 9 | S009N | ***0.05*** | ***0.05*** | ***0.05*** | ***0.05*** | ***0.05*** | ***0.05*** |
| 9 | S009P | 1.38 | 1.00 | 0.87 | 0.94 | 0.7 | 1.06 |
| 9 | S009Q | 1.02 | 0.92 | 0.77 | 0.83 | 0.8 | 1.15 |
| 9 | S009R | 1.21 | 0.98 | 0.82 | 0.77 | ***0.05*** | 0.98 |
| 9 | S009T | 1.16 | 1.07 | 1.11 | 0.80 | 1.3 | 0.94 |
| 9 | S009V | 1.51 | 0.99 | 0.75 | 0.86 | 0.9 | 1.03 |
| 9 | S009W | 1.31 | 0.86 | 0.84 | 0.82 | ***0.05*** | 1.34 |
| 9 | S009Y | ***0.05*** | ***0.05*** | ***0.05*** | ***0.05*** | ***0.05*** | ***0.05*** |
| 10 | Q010A | 1.06 | 0.90 | 1.06 | 1.00 | 0.5 | 0.87 |
| 10 | Q010C | 1.25 | 0.92 | 0.94 | 0.93 | 0.7 | 0.86 |
| 10 | Q010D | ***0.05*** | ***0.05*** | ***0.05*** | ***0.05*** | ***0.05*** | ***0.05*** |
| 10 | Q010E | 1.07 | 0.70 | 1.01 | 0.99 | 1.5 | 0.79 |
| 10 | Q010F | 1.18 | 0.89 | 0.84 | 0.98 | ***0.05*** | 1.10 |
| 10 | Q010G | 1.34 | 0.95 | 0.93 | 0.90 | 0.3 | 1.29 |
| 10 | Q010H | 1.20 | 1.04 | 1.07 | 0.92 | 0.9 | 1.29 |
| 10 | Q010I | 1.22 | 0.80 | 0.70 | 0.72 | ***0.05*** | 0.75 |
| 10 | Q010K | 1.16 | 0.83 | 0.49 | 0.89 | 0.1 | 0.96 |
| 10 | Q010L | 1.19 | 0.89 | 0.68 | 0.85 | *0.05* | 0.91 |
| 10 | Q010M | 1.26 | 0.82 | 0.97 | 1.07 | 0.5 | 0.90 |
| 10 | Q010N | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 10 | Q010P | 0.18 | ***0.05*** | 1.50 | 0.09 | ***0.05*** | ***0.05*** |
| 10 | Q010R | 1.15 | 0.82 | 0.60 | 0.73 | ***0.05*** | 0.95 |
| 10 | Q010S | 1.00 | 0.88 | 0.90 | 0.87 | 0.5 | 1.10 |
| 10 | Q010T | 1.25 | 1.04 | 1.00 | 0.85 | 0.8 | 0.93 |
| 10 | Q010V | 1.24 | 0.96 | 0.80 | 1.00 | 0.2 | 1.18 |
| 10 | Q010W | 1.04 | 0.97 | 0.81 | 1.00 | ***0.05*** | 0.97 |
| 10 | Q010Y | 0.44 | 0.97 | 0.79 | 0.99 | 0.4 | 0.82 |
| 11 | 1011A | 0.28 | 0.90 | 1.10 | 1.10 | 0.3 | 0.65 |
| 11 | I011C | 1.02 | 1.07 | 0.88 | 0.90 | 0.7 | 1.00 |
| 11 | I011D | 0.34 | ***0.05*** | ***0.05*** | ***0.05*** | ***0.05*** | ***0.05*** |
| 11 | I011E | 0.17 | ***0.05*** | ***0.05*** | 0.53 | ***0.05*** | ***0.05*** |
| 11 | I011F | ***0.05*** | ***0.05*** | ***0.05*** | ***0.05*** | ***0.05*** | ***0.05*** |
| 11 | I011G | 0.18 | 2.21 | 2.17 | 1.57 | 0.4 | 0.27 |
| 11 | I011H | 0.16 | 36.45 | 114.23 | 1.88 | 0.8 | 0.35 |
| 11 | I011K | 0.34 | ***0.05*** | 0.07 | ***0.05*** | ***0.05*** | ***0.05*** |
| 11 | 1011L | 0.76 | 1.07 | 1.13 | 0.79 | 0.6 | 1.15 |
| 11 | I011M | ***0.05*** | ***0.05*** | ***0.05*** | ***0.05*** | ***0.05*** | ***0.05*** |
| 11 | I011N | 0.28 | ***0.05*** | ***0.05*** | ***0.05*** | ***0.05*** | ***0.05*** |
| 11 | I011P | 0.28 | ***0.05*** | ***0.05*** | 0.07 | ***0.05*** | ***0.05*** |
| 11 | I011Q | 0.14 | ***0.05*** | ***0.05*** | ***0.05*** | ***0.05*** | ***0.05*** |
| 11 | I011R | 0.30 | ***0.05*** | ***0.05*** | ***0.05*** | ***0.05*** | ***0.05*** |
| 11 | I011S | 0.26 | 1.34 | 1.73 | 1.26 | 0.3 | 0.97 |
| 11 | I011T | 1.05 | 1.02 | 0.87 | 0.93 | 0.7 | 1.00 |
| 11 | I011V | 1.28 | 1.17 | 0.91 | 0.86 | 1.0 | 1.10 |
| 11 | I011W | 0.30 | ***0.05*** | 0.07 | ***0.05*** | ***0.05*** | ***0.05*** |
| 11 | I011Y | 0.21 | ***0.05*** | 0.11 | ***0.05*** | ***0.05*** | ***0.05*** |
| 12 | K012A | 1.48 | 0.85 | 0.82 | 1.02 | 1.2 | 0.84 |
| 12 | K012C | 1.24 | 0.73 | 0.94 | 0.93 | 1.4 | 0.94 |
| 12 | K012D | 1.26 | 0.49 | 1.06 | 0.89 | 1.4 | 0.96 |
| 12 | K012E | 1.35 | 0.62 | 0.71 | 0.85 | 1.4 | 0.90 |
| 12 | K012F | 1.04 | 0.60 | 0.80 | 0.68 | 1.3 | 0.94 |
| 12 | K012G | 1.50 | 0.94 | 0.88 | 0.90 | 1.4 | 0.88 |
| 12 | K012H | 1.24 | 0.94 | 1.03 | 0.92 | 1.3 | 1.01 |
| 12 | K012I | 1.24 | 0.68 | 0.65 | 0.96 | 1.2 | 0.92 |
| 12 | K012L | 0.17 | 3.09 | 4.59 | 1.99 | 0.6 | 0.28 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 12 | K012M | 0.34 | ***0.05*** | ***0.05*** | 0.06 | ***0.05*** | ***0.05*** |
| 12 | K012N | 1.13 | 0.86 | 0.93 | 0.85 | 1.3 | 1.01 |
| 12 | K012P | 0.31 | ***0.05*** | ***0.05*** | ***0.05*** | ***0.05*** | ***0.05*** |
| 12 | K012Q | 1.35 | 0.96 | 0.61 | 1.01 | 1.3 | 1.10 |
| 12 | K012R | 0.73 | 0.83 | 0.84 | 0.83 | 0.9 | 0.75 |
| 12 | K012S | 1.07 | 0.71 | 0.74 | 0.72 | 1.0 | 0.93 |
| 12 | K012T | 1.13 | 1.07 | 0.95 | 0.84 | 1.1 | 0.85 |
| 12 | K012V | 1.57 | 0.80 | 0.83 | 0.98 | 1.1 | 0.76 |
| 12 | K012W | 1.35 | 0.54 | 0.85 | 0.94 | 1.2 | 0.90 |
| 12 | K012Y | 1.38 | 0.52 | 0.90 | 0.67 | 1.3 | 0.95 |
| 13 | A013C | 1.05 | 0.99 | 0.77 | 0.83 | 0.6 | 1.12 |
| 13 | A013D | ***0.05*** | ***0.05*** | ***0.05*** | ***0.05*** | ***0.05*** | ***0.05*** |
| 13 | A013E | 0.35 | ***0.05*** | ***0.05*** | ***0.05*** | ***0.05*** | ***0.05*** |
| 13 | A013F | 0.33 | ***0.05*** | 0.08 | ***0.05*** | ***0.05*** | ***0.05*** |
| 13 | A013G | 1.56 | 1.02 | 1.05 | 0.84 | 0.7 | 0.92 |
| 13 | A013H | 0.33 | ***0.05*** | ***0.05*** | ***0.05*** | ***0.05*** | ***0.05*** |
| 13 | A013I | 0.19 | 1.12 | 1.48 | 1.44 | ***0.05*** | 0.31 |
| 13 | A013K | 0.37 | ***0.05*** | ***0.05*** | ***0.05*** | ***0.05*** | ***0.05*** |
| 13 | A013L | 0.26 | 1.12 | 1.04 | 1.19 | 1.1 | 0.80 |
| 13 | A013M | 0.16 | 1.38 | ***0.05*** | 0.81 | ***0.05*** | ***0.05*** |
| 13 | A013N | ***0.05*** | ***0.05*** | ***0.05*** | ***0.05*** | ***0.05*** | ***0.05*** |
| 13 | A013P | 0.19 | 0.15 | ***0.05*** | 0.07 | ***0.05*** | ***0.05*** |
| 13 | A013Q | 0.15 | 0.17 | ***0.05*** | ***0.05*** | ***0.05*** | ***0.05*** |
| 13 | A013R | 0.30 | ***0.05*** | 0.07 | ***0.05*** | ***0.05*** | ***0.05*** |
| 13 | A013S | 0.91 | 1.05 | 1.10 | 1.05 | 1.1 | 1.23 |
| 13 | A013T | 0.51 | 1.12 | 0.91 | 0.96 | 0.6 | 0.82 |
| 13 | A013V | 0.82 | 1.01 | 0.74 | 0.97 | 0.1 | 0.76 |
| 13 | A013W | 0.27 | ***0.05*** | ***0.05*** | 0.07 | ***0.05*** | ***0.05*** |
| 13 | A013Y | ***0.05*** | ***0.05*** | ***0.05*** | ***0.05*** | ***0.05*** | ***0.05*** |
| 14 | P014A | 1.21 | 0.97 | 1.13 | 1.04 | 0.1 | 1.07 |
| 14 | P014C | 1.05 | 0.78 | 1.22 | 0.84 | 0.7 | 0.99 |
| 14 | P014D | 1.07 | 0.70 | 1.21 | 0.92 | 0.4 | 1.02 |
| 14 | P014E | 1.27 | 0.55 | 1.14 | 0.95 | 0.9 | 1.14 |
| 14 | P014F | 0.93 | 0.57 | 0.89 | 0.90 | ***0.05*** | 1.07 |
| 14 | P014G | 1.35 | 0.92 | 1.20 | 0.94 | 0.2 | 1.18 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 14 | P014H | ***0.05*** | ***0.05*** | ***0.05*** | ***0.05*** | ***0.05*** | ***0.05*** |
| 14 | P014I | 0.89 | 0.85 | 1.19 | 0.94 | 0.7 | 1.19 |
| 14 | P014K | 1.36 | 0.51 | 0.90 | 0.95 | ***0.05*** | 1.01 |
| 14 | P014L | 0.43 | 0.74 | 1.58 | 1.02 | 0.6 | 1.16 |
| 14 | P014M | 1.50 | 0.77 | 1.17 | 0.93 | 0.3 | 1.08 |
| 14 | P014N | 1.10 | 0.80 | 0.98 | 0.91 | 0.1 | 1.00 |
| 14 | P014Q | 1.34 | 0.62 | 0.94 | 0.81 | 0.6 | 1.12 |
| 14 | P014R | 1.19 | 0.41 | 0.67 | 0.98 | ***0.05*** | 1.15 |
| 14 | P014S | 0.96 | 0.79 | 1.06 | 0.96 | 0.1 | 1.13 |
| 14 | P014T | 0.98 | 0.99 | 1.08 | 0.83 | 0.9 | 1.35 |
| 14 | P014V | 1.32 | 0.88 | 1.10 | 0.90 | 0.7 | 1.12 |
| 14 | P014W | 1.17 | 0.84 | 1.18 | 0.94 | ***0.05*** | 0.92 |
| 14 | P014Y | 1.16 | 0.92 | 0.95 | 1.01 | ***0.05*** | 0.93 |
| 15 | A015C | 1.72 | 0.83 | 0.87 | 0.90 | 1.3 | 0.83 |
| 15 | A015D | 1.60 | 0.66 | 0.77 | 0.75 | 1.3 | 1.00 |
| 15 | A015E | 1.68 | 0.62 | 0.77 | 1.11 | 1.5 | 0.87 |
| 15 | A015F | 1.39 | 0.78 | 0.68 | 0.85 | 0.9 | 0.99 |
| 15 | A015G | 1.63 | 0.77 | 0.66 | 0.76 | 0.9 | 0.79 |
| 15 | A015H | 0.90 | 0.64 | 0.77 | 0.93 | 1.0 | 1.01 |
| 15 | A015I | 1.52 | 0.46 | 0.88 | 0.86 | 1.1 | 0.95 |
| 15 | A015K | 1.50 | 0.74 | 0.59 | 0.69 | 0.3 | 0.98 |
| 15 | A015L | 1.52 | 0.89 | 0.77 | 0.88 | 1.1 | 0.85 |
| 15 | A015M | 0.96 | 0.95 | 0.62 | 1.01 | 1.0 | 0.99 |
| 15 | A015N | ***0.05*** | ***0.05*** | ***0.05*** | ***0.05*** | ***0.05*** | ***0.05*** |
| 15 | A015P | 1.41 | 0.57 | 0.64 | 0.73 | 0.9 | 0.95 |
| 15 | A015Q | 1.39 | 0.55 | 0.73 | 1.05 | 1.1 | 1.06 |
| 15 | A015R | 1.44 | 0.67 | 0.53 | 0.84 | 0.1 | 0.95 |
| 15 | A015S | 1.17 | 0.74 | 0.80 | 1.07 | 0.9 | 1.08 |
| 15 | A015T | 1.53 | 0.85 | 0.68 | 0.93 | 1.0 | 1.00 |
| 15 | A015V | 1.50 | 0.77 | 0.73 | 0.89 | 1.0 | 0.93 |
| 15 | A015W | 0.45 | 0.59 | 0.88 | 1.18 | 0.8 | 0.93 |
| 15 | A015Y | 1.43 | 0.63 | 0.70 | 0.90 | 1.0 | 0.94 |
| 16 | L016A | 1.14 | 0.79 | 1.22 | 0.96 | 0.8 | 0.84 |
| 16 | L016C | 1.01 | 0.71 | 1.15 | 1.08 | 1.0 | 1.13 |
| 16 | L016D | 0.20 | ***0.05*** | 0.39 | 0.36 | 0.3 | 0.09 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 16 | L016E | 1.02 | 0.57 | 1.18 | 1.09 | 0.9 | 1.03 |
| 16 | L016F | 0.45 | 0.63 | 0.93 | 0.89 | 0.5 | 0.58 |
| 16 | L016G | 0.29 | 0.54 | 1.19 | 0.97 | 0.8 | 0.47 |
| 16 | L016H | 0.29 | 0.70 | 1.11 | 1.12 | 0.5 | 0.53 |
| 16 | L016I | 1.22 | 0.51 | 1.21 | 1.05 | 1.0 | 0.89 |
| 16 | L016K | 0.36 | 0.62 | 1.09 | 0.99 | 0.5 | 0.73 |
| 16 | L016M | 1.63 | 0.76 | 0.97 | 1.13 | 1.0 | 0.89 |
| 16 | L016N | 0.59 | 0.71 | 1.10 | 1.05 | 0.8 | 0.96 |
| 16 | L016P | 0.50 | 0.73 | 1.05 | 1.07 | 0.8 | 0.90 |
| 16 | L016Q | 0.90 | 0.88 | 1.02 | 1.04 | 0.9 | 0.88 |
| 16 | L016R | 0.15 | ***0.05*** | ***0.05*** | ***0.05*** | ***0.05*** | 0.21 |
| 16 | L016S | 0.92 | 0.81 | 1.09 | 0.96 | 0.7 | 0.82 |
| 16 | L016T | 1.30 | 0.63 | 1.02 | 1.07 | 0.9 | 0.93 |
| 16 | L016V | 0.17 | ***0.05*** | 1.28 | 0.30 | ***0.05*** | ***0.05*** |
| 16 | L016W | 0.17 | 3.37 | 6.22 | 3.16 | 0.1 | 0.35 |
| 16 | L016Y | 0.25 | 0.76 | 1.58 | 1.43 | ***0.05*** | 0.47 |
| 17 | H017A | 0.37 | 0.41 | 0.95 | 0.94 | 0.4 | 0.40 |
| 17 | H017C | 0.33 | 0.80 | 0.73 | 1.01 | 0.7 | 0.39 |
| 17 | H017D | 0.18 | ***0.05*** | 0.22 | 0.26 | ***0.05*** | ***0.05*** |
| 17 | H017E | 0.30 | 0.81 | 1.09 | 1.00 | 0.4 | 0.63 |
| 17 | H017F | 1.40 | 0.72 | 1.01 | 0.85 | 0.3 | 0.86 |
| 17 | H017G | 0.19 | 1.02 | 0.45 | 1.57 | 0.6 | 0.25 |
| 17 | H017I | 1.36 | 0.57 | 0.99 | 1.10 | 0.8 | 0.94 |
| 17 | H017K | 0.13 | ***0.05*** | ***0.05*** | ***0.05*** | 0.2 | 0.10 |
| 17 | H017L | 1.15 | 0.90 | 1.06 | 0.93 | 0.5 | 0.82 |
| 17 | H017M | 1.29 | 0.60 | 1.24 | 0.94 | 0.7 | 0.84 |
| 17 | H017N | ***0.05*** | ***0.05*** | ***0.05*** | ***0.05*** | ***0.05*** | ***0.05*** |
| 17 | H017P | 0.14 | ***0.05*** | ***0.05*** | ***0.05*** | ***0.05*** | ***0.05*** |
| 17 | H017Q | ***0.05*** | ***0.05*** | ***0.05*** | ***0.05*** | ***0.05*** | ***0.05*** |
| 17 | H017R | 0.15 | ***0.05*** | ***0.05*** | ***0.05*** | ***0.05*** | 0.11 |
| 17 | H017S | 0.20 | 1.15 | 1.18 | 1.54 | 0.4 | 0.34 |
| 17 | H017T | 0.47 | 0.97 | 1.25 | 1.13 | 0.2 | 0.89 |
| 17 | H017V | 0.80 | 0.67 | 0.96 | 1.02 | 0.4 | 0.67 |
| 17 | H017W | 1.54 | 0.60 | 1.13 | 1.03 | ***0.05*** | 1.07 |
| 17 | H017Y | 0.49 | 0.77 | 1.33 | 1.06 | ***0.05*** | 0.67 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 18 | S018A | 0.89 | 0.57 | 1.16 | 0.91 | 0.9 | 0.95 |
| 18 | S018C | ***0.05*** | ***0.05*** | ***0.05*** | ***0.05*** | ***0.05*** | ***0.05*** |
| 18 | S018D | 1.72 | 0.78 | 1.03 | 0.86 | 0.8 | 0.86 |
| 18 | S018E | 1.65 | 0.75 | 1.15 | 0.99 | 1.3 | 0.85 |
| 18 | S018F | 1.74 | 0.80 | 1.14 | 0.95 | 0.4 | 0.87 |
| 18 | S018G | 1.37 | 0.80 | 0.96 | 1.04 | 1.0 | 0.85 |
| 18 | S018H | 1.72 | 0.83 | 1.07 | 1.05 | 1.0 | 0.93 |
| 18 | S018I | 1.89 | 0.66 | 1.33 | 0.90 | 0.6 | 0.88 |
| 18 | S018K | 1.75 | 0.63 | 1.14 | 0.94 | 0.1 | 0.99 |
| 18 | S018L | 1.48 | 0.73 | 1.25 | 0.96 | 0.7 | 0.96 |
| 18 | S018M | 1.72 | 0.93 | 1.28 | 1.04 | 0.9 | 0.91 |
| 18 | S018N | 1.68 | 0.59 | 1.12 | 0.94 | 1.1 | 0.91 |
| 18 | S018P | 1.19 | 0.65 | 1.06 | 0.83 | 1.3 | 0.75 |
| 18 | S018Q | 1.68 | 0.80 | 1.12 | 0.99 | 1.0 | 0.92 |
| 18 | S018R | 1.61 | 0.71 | 0.99 | 1.02 | ***0.05*** | 0.93 |
| 18 | S018T | 1.64 | 0.67 | 1.25 | 0.99 | 1.1 | 0.89 |
| 18 | S018V | 1.67 | 0.57 | 1.17 | 1.14 | 0.8 | 0.89 |
| 18 | S018W | 1.59 | 0.73 | 1.06 | 1.00 | 0.4 | 0.95 |
| 18 | S018Y | 1.86 | 0.81 | 1.25 | 1.08 | 0.8 | 1.13 |
| 19 | Q019A | 1.82 | 1.07 | 0.99 | 1.05 | 0.8 | 0.92 |
| 19 | Q019C | 0.88 | 1.08 | 0.90 | 0.81 | 1.0 | 0.91 |
| 19 | Q019D | 0.87 | 0.98 | 0.95 | 0.67 | 1.1 | 0.98 |
| 19 | Q019E | 1.02 | 0.97 | 0.61 | 0.90 | 1.2 | 0.95 |
| 19 | Q019F | ***0.05*** | ***0.05*** | ***0.05*** | ***0.05*** | ***0.05*** | ***0.05*** |
| 19 | Q019G | 1.33 | 0.99 | 1.15 | 0.87 | 0.8 | 1.13 |
| 19 | Q019H | 0.82 | 1.05 | 0.98 | 0.72 | 0.9 | 1.08 |
| 19 | Q019I | 0.89 | 1.06 | 0.87 | 1.03 | 0.9 | 1.22 |
| 19 | Q019K | 0.86 | 0.87 | 0.78 | 0.77 | 0.6 | 1.15 |
| 19 | Q019L | 0.98 | 0.95 | 0.61 | 0.87 | 1.0 | 1.01 |
| 19 | Q019M | 0.90 | 1.02 | 1.01 | 0.90 | 0.7 | 0.96 |
| 19 | Q019N | ***0.05*** | ***0.05*** | ***0.05*** | ***0.05*** | ***0.05*** | ***0.05*** |
| 19 | Q019P | 0.38 | ***0.05*** | 0.08 | ***0.05*** | ***0.05*** | ***0.05*** |
| 19 | Q019R | 1.09 | 1.03 | 0.64 | 0.74 | 0.4 | 0.99 |
| 19 | Q019S | 0.82 | 1.03 | 1.14 | 0.92 | 0.8 | 1.07 |
| 19 | Q019T | 0.93 | 0.94 | 1.00 | 0.84 | 0.8 | 1.15 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 19 | Q019V | 0.88 | 1.12 | 1.04 | 0.97 | 0.7 | 1.22 |
| 19 | Q019W | ***0.05*** | ***0.05*** | ***0.05*** | ***0.05*** | ***0.05*** | ***0.05*** |
| 19 | Q019Y | ***0.05*** | ***0.05*** | ***0.05*** | ***0.05*** | ***0.05*** | ***0.05*** |
| 20 | G020A | 1.12 | 0.90 | 0.99 | 0.93 | 0.8 | 0.80 |
| 20 | G020C | 0.96 | 0.81 | 1.12 | 0.99 | 1.0 | 0.95 |
| 20 | G020D | 1.00 | 0.82 | 1.19 | 1.02 | 1.1 | 0.99 |
| 20 | G020E | 1.27 | 0.91 | 1.04 | 1.01 | 1.0 | 1.01 |
| 20 | G020F | 0.88 | 0.64 | 1.15 | 1.06 | 1.0 | 0.84 |
| 20 | G020H | 1.00 | 0.66 | 1.20 | 1.08 | 1.0 | 0.92 |
| 20 | G020I | 0.22 | 0.44 | 1.36 | 1.12 | 0.9 | 0.34 |
| 20 | G020K | 0.90 | 0.70 | 0.95 | 0.93 | 0.4 | 1.06 |
| 20 | G020L | 0.75 | 0.74 | 1.14 | 0.89 | 1.0 | 0.92 |
| 20 | G020M | 0.98 | 0.83 | 1.08 | 0.97 | 0.9 | 0.89 |
| 20 | G020N | 0.96 | 0.91 | 1.18 | 0.90 | 1.0 | 1.03 |
| 20 | G020P | 0.26 | 0.63 | 1.11 | 0.98 | 0.9 | 0.49 |
| 20 | G020Q | 0.95 | 0.53 | 1.13 | 1.01 | 0.9 | 1.06 |
| 20 | G020R | 0.86 | 0.70 | 0.99 | 0.92 | 0.2 | 0.99 |
| 20 | G020S | 1.00 | 0.63 | 1.07 | 1.05 | 0.7 | 0.88 |
| 20 | G020T | 0.54 | 0.82 | 1.24 | 1.08 | 0.9 | 1.02 |
| 20 | G020V | 0.22 | 0.97 | 1.37 | 1.53 | 0.8 | 0.40 |
| 20 | G020W | 0.63 | 0.88 | 1.15 | 1.05 | 0.8 | 0.93 |
| 20 | G020Y | 0.70 | 0.57 | 1.03 | 1.04 | 0.9 | 0.92 |
| 21 | Y021A | 1.15 | 0.85 | 0.77 | 0.94 | 0.9 | 0.81 |
| 21 | Y021C | 1.15 | 0.79 | 1.02 | 0.86 | 0.9 | 1.05 |
| 21 | Y021D | 0.84 | 0.83 | 0.76 | 1.02 | 0.9 | 0.73 |
| 21 | Y021E | 0.71 | 0.82 | 0.77 | 0.89 | 1.0 | 0.91 |
| 21 | Y021F | 1.13 | 0.59 | 0.77 | 0.97 | 1.0 | 0.85 |
| 21 | Y021G | 0.46 | 0.60 | 0.76 | 0.89 | 1.0 | 0.52 |
| 21 | Y021H | 1.22 | 0.78 | 0.69 | 0.98 | 1.1 | 0.93 |
| 21 | Y021K | 0.72 | 0.61 | 0.47 | 1.00 | 0.8 | 0.91 |
| 21 | Y021L | 0.92 | 0.78 | 0.75 | 0.99 | 1.0 | 0.95 |
| 21 | Y021M | 1.17 | 0.88 | 0.87 | 0.95 | 1.0 | 0.78 |
| 21 | Y021P | 0.47 | 0.50 | 0.88 | 1.08 | 1.2 | 0.69 |
| 21 | Y021Q | 0.74 | 0.88 | 0.87 | 1.02 | 0.9 | 0.96 |
| 21 | Y021R | 0.95 | 0.70 | 0.77 | 0.94 | 0.7 | 0.91 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 21 | Y021S | 0.98 | 0.71 | 0.84 | 0.93 | 0.9 | 0.80 |
| 21 | Y021T | 1.33 | 0.81 | 0.76 | 0.93 | 1.0 | 0.90 |
| 21 | Y021V | 1.15 | 0.70 | 0.99 | 1.00 | 1.1 | 0.87 |
| 21 | Y021W | 0.83 | 0.68 | 0.70 | 1.04 | 1.0 | 1.14 |
| 22 | T022A | 0.67 | 0.85 | 0.92 | 0.86 | 1.2 | 0.80 |
| 22 | T022C | 1.21 | 1.12 | 0.74 | 1.08 | 1.3 | 1.13 |
| 22 | T022D | 0.76 | 0.98 | 0.74 | 1.03 | 1.3 | 0.97 |
| 22 | T022E | 1.33 | 1.01 | 0.89 | 0.90 | 1.6 | 1.04 |
| 22 | T022F | 1.37 | 1.09 | 0.73 | 1.06 | 1.2 | 0.90 |
| 22 | T022G | 1.60 | 1.00 | 0.92 | 1.02 | 1.2 | 0.87 |
| 22 | T022H | 1.17 | 1.09 | 0.88 | 1.14 | 1.1 | 0.99 |
| 22 | T022I | 1.26 | 1.17 | 0.91 | 1.28 | 1.0 | 1.08 |
| 22 | T022K | 1.19 | 0.78 | 0.73 | 1.02 | 0.4 | 1.25 |
| 22 | T022L | 0.92 | 0.90 | 0.73 | 1.07 | 0.9 | 0.92 |
| 22 | T022M | 1.34 | 0.92 | 0.85 | 1.07 | 1.0 | 1.13 |
| 22 | T022N | 1.13 | 0.90 | 0.95 | 1.03 | 1.2 | 1.07 |
| 22 | T022P | 0.58 | 0.78 | 0.62 | 0.89 | 1.3 | 0.52 |
| 22 | T022Q | 1.42 | 1.15 | 0.75 | 1.14 | 1.1 | 0.97 |
| 22 | T022S | 1.10 | 0.95 | 0.91 | 0.98 | 1.3 | 0.93 |
| 22 | T022V | 1.32 | 1.18 | 0.75 | 1.10 | 1.3 | 1.11 |
| 22 | T022W | 1.52 | 0.93 | 0.79 | 1.07 | 1.7 | 0.87 |
| 22 | T022Y | 1.15 | 0.96 | 0.95 | 1.03 | 1.4 | 0.95 |
| 23 | G023A | 0.63 | 1.05 | 0.84 | 1.02 | 1.0 | 0.79 |
| 23 | G023C | 0.19 | 0.17 | ***0.05*** | 0.13 | ***0.05*** | ***0.05*** |
| 23 | G023D | 0.23 | ***0.05*** | ***0.05*** | ***0.05*** | ***0.05*** | ***0.05*** |
| 23 | G023F | 0.22 | 0.11 | 0.06 | ***0.05*** | ***0.05*** | ***0.05*** |
| 23 | G023H | 0.21 | 0.11 | ***0.05*** | 0.08 | ***0.05*** | ***0.05*** |
| 23 | G023I | 0.19 | ***0.05*** | ***0.05*** | 0.15 | ***0.05*** | ***0.05*** |
| 23 | G023K | 0.38 | ***0.05*** | ***0.05*** | ***0.05*** | ***0.05*** | ***0.05*** |
| 23 | G023L | 0.20 | 0.26 | 0.06 | 0.15 | ***0.05*** | ***0.05*** |
| 23 | G023M | 0.31 | ***0.05*** | ***0.05*** | ***0.05*** | ***0.05*** | ***0.05*** |
| 23 | G023N | 0.19 | ***0.05*** | ***0.05*** | 0.14 | ***0.05*** | ***0.05*** |
| 23 | G023P | 0.30 | 0.08 | ***0.05*** | 0.06 | ***0.05*** | ***0.05*** |
| 23 | G023Q | 0.26 | 0.08 | ***0.05*** | 0.10 | ***0.05*** | ***0.05*** |
| 23 | G023R | 0.22 | 0.14 | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 23 | G023S | 0.17 | 10.83 | 4.09 | 2.61 | 1.1 | 0.13 |
| 23 | G023T | 0.21 | 0.16 | ***0.05*** | ***0.05*** | ***0.05*** | ***0.05*** |
| 23 | G023V | 0.14 | ***0.05*** | 0.26 | ***0.05*** | ***0.05*** | ***0.05*** |
| 23 | G023W | 0.25 | 0.10 | ***0.05*** | ***0.05*** | ***0.05*** | ***0.05*** |
| 23 | G023Y | 0.15 | ***0.05*** | 0.50 | ***0.05*** | ***0.05*** | ***0.05*** |
| 24 | S024C | 1.12 | 1.15 | 0.86 | 0.94 | 1.1 | 0.97 |
| 24 | S024F | 1.07 | 1.15 | 0.93 | 1.05 | 1.0 | 1.22 |
| 24 | S024G | 0.56 | 0.92 | 0.68 | 1.01 | 1.2 | 0.68 |
| 24 | S024H | 0.97 | 1.11 | 0.98 | 1.18 | 1.1 | 1.28 |
| 24 | S024I | 1.02 | 1.16 | 1.02 | 1.15 | 1.1 | 0.98 |
| 24 | S024K | 1.17 | 1.03 | 0.82 | 1.04 | 0.9 | 0.97 |
| 24 | S024L | 1.17 | 1.02 | 1.01 | 1.02 | 1.2 | 1.16 |
| 24 | S024M | 1.15 | 1.07 | 0.98 | 1.02 | 1.2 | 0.88 |
| 24 | S024N | 1.16 | 0.99 | 0.88 | 1.11 | 1.2 | 1.01 |
| 24 | S024P | 0.44 | 1.10 | 0.76 | 0.96 | 1.1 | 0.72 |
| 24 | S024Q | 0.90 | 0.96 | 0.94 | 1.11 | 1.0 | 1.10 |
| 24 | S024R | 0.86 | 1.19 | 0.82 | 1.12 | 1.0 | 1.19 |
| 24 | S024T | 0.57 | 1.07 | 0.92 | 1.06 | 1.1 | 0.96 |
| 24 | S024V | 0.45 | 1.21 | 0.93 | 1.19 | 1.1 | 1.22 |
| 24 | S024W | 1.13 | 1.09 | 0.62 | 1.09 | 1.2 | 0.94 |
| 24 | S024Y | 1.06 | 0.95 | 0.83 | 0.96 | 1.1 | 1.19 |
| 25 | N025A | 0.73 | 0.63 | 1.09 | 1.00 | 0.9 | 0.93 |
| 25 | N025C | 0.23 | ***0.05*** | 0.29 | 0.10 | 1.4 | 0.07 |
| 25 | N025D | ***0.05*** | ***0.05*** | ***0.05*** | ***0.05*** | ***0.05*** | ***0.05*** |
| 25 | N025E | 1.31 | 0.61 | 1.16 | 1.03 | 1.2 | 1.13 |
| 25 | N025F | 1.09 | 0.69 | 1.14 | 1.12 | 1.1 | 0.97 |
| 25 | N025G | 1.21 | 0.65 | 1.20 | 0.99 | 1.0 | 1.05 |
| 25 | N025H | 1.16 | 0.57 | 1.06 | 1.07 | 1.1 | 0.98 |
| 25 | N0251 | 0.95 | 0.72 | 1.29 | 0.83 | 1.0 | 1.00 |
| 25 | N025K | 0.96 | 0.64 | 1.04 | 0.96 | 1.0 | 0.99 |
| 25 | N025L | 0.64 | 0.62 | 1.02 | 1.09 | 1.1 | 0.98 |
| 25 | N025M | 1.31 | 0.82 | 1.18 | 1.04 | 1.0 | 1.01 |
| 25 | N025P | 0.71 | 0.53 | 1.10 | 0.81 | 0.8 | 0.82 |
| 25 | N025Q | 1.10 | 0.61 | 1.21 | 1.09 | 1.0 | 1.08 |
| 25 | N025R | 1.34 | 0.68 | 1.02 | 1.12 | 1.0 | 1.03 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 25 | N025S | 0.85 | 0.60 | 1.18 | 1.01 | 1.0 | 0.93 |
| 25 | N025T | 1.06 | 0.73 | 1.21 | 1.12 | 0.8 | 1.22 |
| 25 | N025V | 0.59 | 0.45 | 1.27 | 1.11 | 0.9 | 1.01 |
| 25 | N025W | 1.42 | 0.53 | 1.30 | 1.00 | 0.9 | 0.99 |
| 25 | N025Y | ***0.05*** | ***0.05*** | ***0.05*** | ***0.05*** | ***0.05*** | ***0.05*** |
| 26 | V026C | 1.14 | 0.96 | 0.91 | 1.02 | 1.0 | 0.88 |
| 26 | V026D | 0.16 | ***0.05*** | ***0.05*** | 31.85 | 1.1 | 0.33 |
| 26 | V026E | 0.36 | 1.12 | 0.79 | 1.19 | 1.0 | 0.87 |
| 26 | V026F | 0.35 | 0.97 | 0.90 | 1.19 | 1.1 | 0.68 |
| 26 | V026G | 0.54 | 0.91 | 0.75 | 1.07 | 1.0 | 0.82 |
| 26 | V026H | 0.46 | 1.45 | 0.86 | 1.19 | 1.1 | 0.89 |
| 26 | V026I | 0.91 | 0.75 | 0.70 | 1.09 | 1.2 | 0.90 |
| 26 | V026K | 0.77 | 1.03 | 0.67 | 1.11 | 1.1 | 0.95 |
| 26 | V026L | 0.59 | 0.92 | 0.85 | 0.96 | 1.0 | 1.03 |
| 26 | V026M | 0.80 | 0.90 | 0.85 | 0.98 | 1.0 | 0.90 |
| 26 | V026N | 0.44 | 1.00 | 0.79 | 1.21 | 1.0 | 0.96 |
| 26 | V026P | 0.14 | ***0.05*** | ***0.05*** | ***0.05*** | 0.8 | 0.16 |
| 26 | V026Q | 0.61 | 0.96 | 0.78 | 1.02 | 1.0 | 1.08 |
| 26 | V026S | 0.82 | 0.82 | 0.85 | 0.95 | 0.9 | 0.86 |
| 26 | V026T | 0.92 | 0.98 | 0.98 | 1.02 | 0.9 | 1.04 |
| 26 | V026W | 0.25 | 2.33 | 1.16 | 2.13 | 1.1 | 0.95 |
| 26 | V026Y | 0.31 | 1.34 | 1.28 | 1.50 | 1.0 | 0.98 |
| 27 | K027A | 1.08 | 0.76 | 1.13 | 1.05 | 1.0 | 1.07 |
| 27 | K027C | ***0.05*** | ***0.05*** | ***0.05*** | ***0.05*** | ***0.05*** | ***0.05*** |
| 27 | K027D | 1.15 | 0.72 | 1.10 | 1.00 | 1.1 | 0.93 |
| 27 | K027E | 0.45 | 0.93 | 1.11 | 1.09 | 1.0 | 1.06 |
| 27 | K027F | 0.25 | 0.76 | 1.59 | 1.33 | 1.1 | 0.58 |
| 27 | K027G | 0.76 | 0.65 | 1.27 | 0.95 | 1.0 | 1.15 |
| 27 | K027H | 1.03 | 0.72 | 1.19 | 0.99 | 1.1 | 1.10 |
| 27 | K027I | 0.18 | 0.49 | 1.78 | 0.91 | 1.4 | 0.20 |
| 27 | K027L | 0.76 | 0.72 | 1.29 | 0.87 | 1.1 | 0.89 |
| 27 | K027M | 0.64 | 0.71 | 1.31 | 0.91 | 1.0 | 1.06 |
| 27 | K027N | 0.17 | ***0.05*** | 2.56 | 0.87 | 0.9 | 0.09 |
| 27 | K027P | 0.18 | 1.31 | 3.37 | 2.05 | 1.1 | 0.46 |
| 27 | K027Q | 0.15 | 0.58 | ***0.05*** | ***0.05*** | 1.5 | 0.08 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 27 | K027R | 1.09 | 0.64 | 1.16 | 0.96 | 1.0 | 1.19 |
| 27 | K027S | 0.93 | 0.92 | 1.11 | 1.04 | 1.0 | 1.11 |
| 27 | K027T | 0.56 | 0.85 | 1.39 | 1.09 | 1.0 | 1.09 |
| 27 | K027V | 0.23 | ***0.05*** | 0.18 | 0.06 | ***0.05*** | ***0.05*** |
| 27 | K027W | 0.36 | 0.69 | 1.47 | 1.33 | 0.9 | 0.93 |
| 27 | K027Y | 0.45 | 1.00 | 1.19 | 1.03 | 0.9 | 0.96 |
| 28 | V028A | 0.46 | 0.95 | 0.84 | 1.02 | 0.9 | 0.72 |
| 28 | V028C | 0.65 | 1.20 | 0.99 | 1.08 | 1.0 | 0.94 |
| 28 | V028D | 0.58 | ***0.05*** | ***0.05*** | ***0.05*** | ***0.05*** | ***0.05*** |
| 28 | V028E | 0.16 | ***0.05*** | ***0.05*** | 3.97 | 1.0 | 0.17 |
| 28 | V028G | 0.16 | ***0.05*** | ***0.05*** | 2.42 | ***0.05*** | ***0.05*** |
| 28 | V028H | 0.20 | 1.08 | 0.79 | 1.03 | 1.1 | 0.22 |
| 28 | V028I | 1.02 | 1.27 | 1.00 | 1.06 | 1.1 | 0.87 |
| 28 | V028M | 1.05 | 0.99 | 0.68 | 0.91 | 0.6 | 0.70 |
| 28 | V028N | 0.21 | 0.31 | 0.67 | 0.50 | 1.1 | 0.13 |
| 28 | V028P | 0.53 | 0.06 | ***0.05*** | ***0.05*** | ***0.05*** | ***0.05*** |
| 28 | V028Q | 0.18 | 2.79 | 1.99 | 1.43 | 1.6 | 0.25 |
| 28 | V028R | 0.40 | ***0.05*** | ***0.05*** | ***0.05*** | ***0.05*** | ***0.05*** |
| 28 | V028S | 0.18 | 5.70 | 5.17 | 3.40 | 1.1 | 0.37 |
| 28 | V028T | 0.58 | 1.31 | 1.18 | 1.13 | 1.0 | 1.21 |
| 28 | V028W | 0.31 | 0.07 | ***0.05*** | ***0.05*** | ***0.05*** | ***0.05*** |
| 28 | V028Y | 0.20 | 0.68 | 0.48 | 0.81 | ***0.05*** | 0.08 |
| 29 | A029C | 0.76 | 1.32 | 1.19 | 1.13 | 0.9 | 0.77 |
| 29 | A029D | 0.19 | 2.95 | 2.74 | 2.34 | 0.9 | 0.42 |
| 29 | A029E | 0.23 | 0.06 | ***0.05*** | 0.09 | ***0.05*** | ***0.05*** |
| 29 | A029F | 0.42 | ***0.05*** | ***0.05*** | ***0.05*** | ***0.05*** | ***0.05*** |
| 29 | A029G | 0.94 | 1.08 | 0.88 | 1.06 | 1.0 | 1.11 |
| 29 | A029H | 0.47 | ***0.05*** | ***0.05*** | ***0.05*** | ***0.05*** | ***0.05*** |
| 29 | A029K | 0.47 | 0.06 | ***0.05*** | ***0.05*** | ***0.05*** | ***0.05*** |
| 29 | A029L | 0.24 | 0.35 | 0.12 | 0.26 | ***0.05*** | ***0.05*** |
| 29 | A029N | 0.32 | 0.09 | ***0.05*** | 0.07 | ***0.05*** | ***0.05*** |
| 29 | A029P | 0.52 | ***0.05*** | ***0.05*** | ***0.05*** | ***0.05*** | ***0.05*** |
| 29 | A029Q | 0.33 | 0.12 | ***0.05*** | ***0.05*** | ***0.05*** | ***0.05*** |
| 29 | A029R | 0.42 | ***0.05*** | ***0.05*** | ***0.05*** | ***0.05*** | ***0.05*** |
| 29 | A029S | 0.47 | 1.64 | 1.13 | 1.23 | 1.1 | 1.01 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 29 | A029T | 0.24 | 2.02 | 1.55 | 1.57 | 1.1 | 0.47 |
| 29 | A029V | 0.38 | 1.85 | 1.52 | 1.62 | 1.0 | 0.83 |
| 29 | A029W | 0.34 | ***0.05*** | ***0.05*** | ***0.05*** | ***0.05*** | ***0.05*** |
| 29 | A029Y | 0.41 | ***0.05*** | ***0.05*** | ***0.05*** | ***0.05*** | ***0.05*** |
| 30 | V030A | 0.38 | 0.98 | 1.04 | 0.87 | 0.9 | 0.41 |
| 30 | V030C | 0.91 | 0.83 | 0.85 | 0.99 | 1.0 | 0.37 |
| 30 | V030D | 0.20 | 2.34 | 2.31 | 1.96 | 0.9 | 0.22 |
| 30 | V030E | 0.17 | 30.23 | 33.27 | 4.25 | 0.8 | 0.15 |
| 30 | V030F | 0.33 | 1.08 | 1.27 | 1.17 | ***0.05*** | ***0.05*** |
| 30 | V030G | 0.17 | 7.55 | 3.71 | 1.39 | ***0.05*** | ***0.05*** |
| 30 | V030H | 0.26 | 0.57 | 0.61 | 0.60 | ***0.05*** | ***0.05*** |
| 30 | V030I | 1.15 | 1.16 | 1.03 | 1.07 | 1.1 | 0.82 |
| 30 | V030L | 1.24 | 1.09 | 0.86 | 0.87 | 1.0 | 0.24 |
| 30 | V030M | 0.89 | 0.66 | 0.80 | 0.79 | 0.8 | 0.11 |
| 30 | V030N | 0.25 | 1.06 | 1.30 | 1.50 | 1.1 | 0.15 |
| 30 | V030P | 0.27 | 0.13 | 0.13 | 0.09 | ***0.05*** | ***0.05*** |
| 30 | V030Q | 0.21 | 0.82 | 0.99 | 1.10 | ***0.05*** | ***0.05*** |
| 30 | V030R | 0.20 | 0.13 | ***0.05*** | 0.22 | ***0.05*** | ***0.05*** |
| 30 | V030S | 0.28 | 1.25 | 1.21 | 1.43 | 0.3 | 0.12 |
| 30 | V030T | 0.30 | 1.39 | 1.27 | 1.38 | 0.9 | 0.37 |
| 30 | V030W | 0.26 | 0.11 | ***0.05*** | 0.08 | ***0.05*** | ***0.05*** |
| 30 | V030Y | 0.21 | 0.43 | ***0.05*** | 0.21 | ***0.05*** | ***0.05*** |
| 31 | I031A | 0.90 | 0.96 | 0.85 | 0.97 | 1.1 | 1.64 |
| 31 | I031C | 0.94 | 1.16 | 0.87 | 1.02 | 1.1 | 1.53 |
| 31 | I031D | 0.20 | 1.18 | 1.43 | 1.13 | 1.2 | 0.28 |
| 31 | I031E | 0.21 | 2.56 | 1.98 | 2.40 | 1.1 | 1.12 |
| 31 | I031F | 0.92 | 1.26 | 0.92 | 1.00 | 1.1 | 1.89 |
| 31 | I031G | 0.17 | 5.42 | 3.74 | 2.40 | 1.1 | 0.40 |
| 31 | I031H | 0.26 | 1.98 | 1.57 | 1.79 | 1.3 | 1.22 |
| 31 | I031K | 0.32 | 1.84 | 1.28 | 1.39 | 1.2 | 1.03 |
| 31 | I031L | 1.12 | 0.89 | 0.88 | 0.85 | 1.2 | 1.70 |
| 31 | I031M | 1.21 | 0.98 | 0.83 | 0.99 | 1.1 | 1.60 |
| 31 | I031N | 0.23 | 2.34 | 2.01 | 1.96 | 1.3 | 0.74 |
| 31 | I031P | 0.24 | 0.16 | 0.06 | 0.17 | 1.5 | 0.09 |
| 31 | I031Q | 0.19 | 5.38 | 3.51 | 3.08 | 1.1 | 0.64 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 31 | I031R | 0.31 | 0.10 | ***0.05*** | ***0.05*** | ***0.05*** | ***0.05*** |
| 31 | I031S | 0.21 | 3.71 | 2.83 | 2.91 | 1.1 | 0.86 |
| 31 | I031T | 0.38 | 1.21 | 1.04 | 0.91 | 1.1 | 1.17 |
| 31 | I031V | 0.83 | 1.03 | 1.09 | 1.03 | 0.9 | 1.20 |
| 31 | I031W | 0.29 | 0.13 | ***0.05*** | ***0.05*** | ***0.05*** | ***0.05*** |
| 31 | I031Y | 0.21 | 4.14 | 3.02 | 3.08 | 0.9 | 0.90 |
| 32 | D032A | 0.36 | ***0.05*** | ***0.05*** | 0.13 | ***0.05*** | ***0.05*** |
| 32 | D032C | 0.46 | 0.23 | 0.09 | 0.14 | ***0.05*** | ***0.05*** |
| 32 | D032E | 0.36 | 0.24 | ***0.05*** | 0.12 | ***0.05*** | ***0.05*** |
| 32 | D032G | 0.56 | ***0.05*** | ***0.05*** | ***0.05*** | ***0.05*** | ***0.05*** |
| 32 | D032I | 0.26 | 0.14 | ***0.05*** | 0.06 | ***0.05*** | ***0.05*** |
| 32 | D032K | 0.27 | 0.15 | ***0.05*** | ***0.05*** | ***0.05*** | ***0.05*** |
| 32 | D032L | 0.28 | 0.06 | ***0.05*** | ***0.05*** | ***0.05*** | ***0.05*** |
| 32 | D032M | 0.30 | 0.12 | ***0.05*** | 0.06 | ***0.05*** | ***0.05*** |
| 32 | D032N | 0.29 | 0.12 | ***0.05*** | ***0.05*** | ***0.05*** | ***0.05*** |
| 32 | D032P | 0.28 | 0.12 | 0.10 | 0.08 | ***0.05*** | ***0.05*** |
| 32 | D032Q | 0.27 | 0.16 | 0.14 | 0.10 | ***0.05*** | ***0.05*** |
| 32 | D032R | 0.27 | 0.17 | ***0.05*** | ***0.05*** | ***0.05*** | ***0.05*** |
| 32 | D032S | 0.40 | ***0.05*** | ***0.05*** | ***0.05*** | ***0.05*** | ***0.05*** |
| 32 | D032T | 0.27 | ***0.05*** | ***0.05*** | ***0.05*** | ***0.05*** | ***0.05*** |
| 32 | D032V | 0.30 | 0.20 | ***0.05*** | ***0.05*** | ***0.05*** | ***0.05*** |
| 32 | D032Y | 0.28 | 0.13 | 0.11 | ***0.05*** | ***0.05*** | ***0.05*** |
| 33 | S033A | 0.82 | 0.84 | 0.84 | 0.83 | 0.7 | 0.29 |
| 33 | S033C | 0.73 | 0.66 | 0.55 | 0.71 | 1.0 | 0.19 |
| 33 | S033E | 0.44 | 1.21 | 0.81 | 0.92 | 0.8 | 0.07 |
| 33 | S033F | 0.53 | 1.38 | 0.59 | 0.95 | ***0.05*** | ***0.05*** |
| 33 | S033G | 0.96 | 1.14 | 0.80 | 1.01 | 0.9 | 0.17 |
| 33 | S033H | 0.48 | 1.65 | 0.82 | 1.17 | ***0.05*** | ***0.05*** |
| 33 | S033K | 0.25 | 1.24 | 0.81 | 0.77 | ***0.05*** | ***0.05*** |
| 33 | S033L | 0.25 | 1.02 | 0.56 | 0.39 | ***0.05*** | ***0.05*** |
| 33 | S033N | 0.45 | 1.14 | 0.90 | 1.03 | 0.6 | 0.38 |
| 33 | S033P | 0.27 | 0.18 | ***0.05*** | ***0.05*** | ***0.05*** | ***0.05*** |
| 33 | S033Q | 0.36 | 0.69 | 0.72 | 0.72 | 0.5 | 0.13 |
| 33 | S033R | 0.33 | 1.10 | 0.53 | 0.80 | ***0.05*** | ***0.05*** |
| 33 | S033T | 0.82 | 0.74 | 0.64 | 0.85 | 1.0 | 1.40 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 33 | S033V | 0.50 | 0.56 | 0.57 | 0.54 | 0.9 | 0.09 |
| 33 | S033W | 0.34 | 1.15 | 0.39 | 0.51 | ***0.05*** | ***0.05*** |
| 34 | G034A | 0.27 | 0.81 | 0.91 | 0.81 | 0.5 | 0.09 |
| 34 | G034C | 0.26 | ***0.05*** | ***0.05*** | 0.12 | ***0.05*** | ***0.05*** |
| 34 | G034D | 0.28 | ***0.05*** | ***0.05*** | 0.06 | ***0.05*** | ***0.05*** |
| 34 | G034E | 0.27 | ***0.05*** | 0.06 | 0.13 | ***0.05*** | ***0.05*** |
| 34 | G034F | 0.29 | ***0.05*** | ***0.05*** | 0.12 | ***0.05*** | ***0.05*** |
| 34 | G034H | 0.32 | ***0.05*** | ***0.05*** | 0.07 | ***0.05*** | ***0.05*** |
| 34 | G034I | 0.26 | ***0.05*** | 0.07 | 0.10 | ***0.05*** | ***0.05*** |
| 34 | G034K | 0.29 | ***0.05*** | ***0.05*** | ***0.05*** | ***0.05*** | ***0.05*** |
| 34 | G034L | 0.30 | ***0.05*** | ***0.05*** | ***0.05*** | ***0.05*** | ***0.05*** |
| 34 | G034M | 0.30 | ***0.05*** | ***0.05*** | 0.12 | ***0.05*** | ***0.05*** |
| 34 | G034N | ***0.05*** | ***0.05*** | ***0.05*** | ***0.05*** | ***0.05*** | ***0.05*** |
| 34 | G034P | 0.45 | ***0.05*** | ***0.05*** | ***0.05*** | ***0.05*** | ***0.05*** |
| 34 | G034Q | 0.28 | ***0.05*** | 0.08 | 0.06 | ***0.05*** | ***0.05*** |
| 34 | G034R | 0.27 | ***0.05*** | ***0.05*** | ***0.05*** | ***0.05*** | ***0.05*** |
| 34 | G034S | 0.17 | ***0.05*** | 1.23 | 0.73 | ***0.05*** | ***0.05*** |
| 34 | G034T | 0.25 | ***0.05*** | ***0.05*** | ***0.05*** | ***0.05*** | ***0.05*** |
| 34 | G034V | 0.20 | ***0.05*** | 0.16 | 0.21 | ***0.05*** | ***0.05*** |
| 34 | G034W | 0.18 | 1.21 | 2.19 | 1.78 | ***0.05*** | ***0.05*** |
| 34 | G034Y | 0.21 | ***0.05*** | 0.13 | 0.11 | ***0.05*** | ***0.05*** |
| 35 | 1035A | 0.59 | 0.82 | 1.14 | 0.92 | 0.9 | 0.69 |
| 35 | 1035C | 0.95 | 0.65 | 1.41 | 1.06 | 1.0 | 0.95 |
| 35 | 1035D | 0.29 | ***0.05*** | 0.08 | ***0.05*** | ***0.05*** | ***0.05*** |
| 35 | 1035E | 0.24 | ***0.05*** | 0.12 | ***0.05*** | ***0.05*** | ***0.05*** |
| 35 | 1035F | 0.17 | ***0.05*** | 1.52 | 0.33 | ***0.05*** | ***0.05*** |
| 35 | 1035G | 0.18 | 0.21 | 1.85 | 1.07 | 1.0 | 0.16 |
| 35 | 1035H | 0.26 | ***0.05*** | ***0.05*** | ***0.05*** | ***0.05*** | ***0.05*** |
| 35 | 1035K | 0.16 | ***0.05*** | ***0.05*** | 2.01 | ***0.05*** | ***0.05*** |
| 35 | 1035L | 0.61 | 0.68 | 1.14 | 1.03 | 0.7 | 0.74 |
| 35 | 1035M | 0.33 | 0.71 | 1.27 | 1.07 | 0.8 | 0.59 |
| 35 | 1035N | 0.20 | ***0.05*** | 0.34 | 0.09 | ***0.05*** | ***0.05*** |
| 35 | 1035P | 0.38 | ***0.05*** | ***0.05*** | ***0.05*** | ***0.05*** | ***0.05*** |
| 35 | 1035Q | 0.16 | ***0.05*** | ***0.05*** | 1.69 | 1.0 | 0.14 |
| 35 | 1035R | 0.31 | ***0.05*** | 0.06 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 35 | I035S | 0.24 | 1.04 | 2.03 | 1.68 | 0.7 | 0.96 |
| 35 | 1035T | 0.42 | 0.89 | 1.31 | 1.15 | 0.8 | 1.40 |
| 35 | 1035V | 0.83 | 0.85 | 1.17 | 1.03 | 0.9 | 1.13 |
| 35 | I035W | 0.17 | ***0.05*** | 0.61 | ***0.05*** | ***0.05*** | ***0.05*** |
| 35 | 1035Y | 0.16 | ***0.05*** | 10.64 | ***0.05*** | ***0.05*** | ***0.05*** |
| 36 | D036A | 1.00 | 0.58 | 0.60 | 0.94 | 0.4 | 0.78 |
| 36 | D036C | 0.98 | 0.91 | 0.75 | 0.87 | 0.3 | 0.71 |
| 36 | D036E | 1.30 | 1.13 | 0.83 | 0.94 | 0.6 | 0.91 |
| 36 | D036F | 0.47 | 0.58 | 0.72 | 1.03 | ***0.05*** | 0.70 |
| 36 | D036G | 0.37 | 0.79 | 0.58 | 0.76 | ***0.05*** | 0.71 |
| 36 | D036H | 0.56 | 0.49 | 0.52 | 1.00 | 0.6 | 0.95 |
| 36 | D036I | 0.27 | 1.27 | 1.02 | 1.41 | ***0.05*** | 0.46 |
| 36 | D036K | 0.18 | ***0.05*** | ***0.05*** | ***0.05*** | 0.1 | 0.29 |
| 36 | D036L | 0.25 | 1.87 | 1.26 | 1.40 | ***0.05*** | 0.65 |
| 36 | D036M | 0.56 | 0.61 | 0.47 | 0.76 | ***0.05*** | 0.70 |
| 36 | D036N | 0.84 | 0.52 | 0.58 | 0.91 | ***0.05*** | 0.85 |
| 36 | D036P | 0.19 | ***0.05*** | ***0.05*** | ***0.05*** | 0.1 | 0.07 |
| 36 | D036Q | 0.67 | 0.62 | 0.54 | 0.99 | 0.9 | 0.98 |
| 36 | D036R | 0.17 | ***0.05*** | 0.14 | ***0.05*** | ***0.05*** | 0.14 |
| 36 | D036S | 1.03 | 0.72 | 0.70 | 0.87 | 0.1 | 0.93 |
| 36 | D036T | 0.72 | 0.64 | 0.45 | 0.87 | 0.1 | 0.85 |
| 36 | D036V | 0.54 | 0.92 | 0.69 | 0.88 | ***0.05*** | 0.83 |
| 36 | D036W | 0.71 | 0.80 | 0.70 | 0.82 | ***0.05*** | 0.85 |
| 37 | S037A | 1.25 | 1.14 | 1.07 | 0.73 | 0.9 | 1.03 |
| 37 | S037C | 1.09 | 0.90 | 0.87 | 0.93 | 1.0 | 0.94 |
| 37 | S037D | ***0.05*** | ***0.05*** | ***0.05*** | ***0.05*** | ***0.05*** | ***0.05*** |
| 37 | S037E | 0.72 | 0.83 | 1.06 | 0.84 | 1.0 | 0.92 |
| 37 | S037F | 0.99 | 1.05 | 0.88 | 0.91 | 1.0 | 0.83 |
| 37 | S037G | 1.21 | 1.19 | 0.95 | 0.95 | 1.0 | 1.25 |
| 37 | S037H | 1.04 | 1.06 | 0.98 | 0.90 | 0.9 | 1.04 |
| 37 | S037I | ***0.05*** | ***0.05*** | ***0.05*** | ***0.05*** | ***0.05*** | ***0.05*** |
| 37 | S037K | 1.31 | 0.98 | 0.63 | 0.84 | 1.1 | 1.02 |
| 37 | S037L | 0.98 | 1.04 | 0.91 | 0.78 | 0.8 | 0.87 |
| 37 | S037M | 1.31 | 1.00 | 1.07 | 0.82 | 0.9 | 0.87 |
| 37 | S037N | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 37 | S037P | 0.48 | 1.48 | 0.90 | 1.13 | 0.4 | 0.70 |
| 37 | S037Q | 1.31 | 1.09 | 1.24 | 0.87 | 0.9 | 1.05 |
| 37 | S037R | 1.26 | 0.98 | 0.63 | 0.85 | 1.2 | 1.05 |
| 37 | S037T | 0.94 | 1.05 | 1.00 | 0.87 | 0.7 | 0.95 |
| 37 | S037V | 0.98 | 1.09 | 0.86 | 0.76 | 0.7 | 1.39 |
| 37 | S037W | 1.07 | 1.01 | 1.01 | 0.88 | 0.8 | 0.86 |
| 37 | S037Y | 0.19 | 0.21 | 0.16 | 0.13 | ***0.05*** | ***0.05*** |
| 38 | S038A | 1.17 | 1.14 | 1.06 | 1.03 | 0.9 | 0.90 |
| 38 | S038C | 1.27 | 0.96 | 1.02 | 1.03 | 1.2 | 0.82 |
| 38 | S038D | 0.67 | 1.26 | 1.19 | 1.07 | 1.2 | 0.97 |
| 38 | S038E | 1.31 | 1.02 | 1.11 | 0.97 | 1.3 | 0.84 |
| 38 | S038F | 0.97 | 0.99 | 0.97 | 1.07 | 1.0 | 1.22 |
| 38 | S038G | 1.43 | 1.01 | 1.04 | 1.13 | 0.9 | 0.97 |
| 38 | S038H | ***0.05*** | ***0.05*** | ***0.05*** | ***0.05*** | ***0.05*** | ***0.05*** |
| 38 | S038I | 1.07 | 0.98 | 1.08 | 1.01 | 1.1 | 0.89 |
| 38 | S038K | 1.25 | 0.75 | 0.80 | 1.02 | 1.3 | 0.93 |
| 38 | S038L | 1.06 | 0.90 | 1.11 | 1.12 | 1.1 | 1.02 |
| 38 | S038M | 1.33 | 1.10 | 1.07 | 1.00 | 1.0 | 0.91 |
| 38 | S038N | ***0.05*** | ***0.05*** | ***0.05*** | ***0.05*** | ***0.05*** | ***0.05*** |
| 38 | S038P | 1.16 | 1.12 | 0.93 | 0.99 | 0.5 | 0.76 |
| 38 | S038Q | 1.11 | 1.06 | 0.87 | 1.08 | 1.1 | 0.96 |
| 38 | S038R | 2.01 | 0.65 | 0.89 | 1.01 | 1.4 | 0.89 |
| 38 | S038T | 1.20 | 0.86 | 1.07 | 1.05 | 1.1 | 0.93 |
| 38 | S038V | 1.28 | 1.05 | 1.07 | 1.02 | 1.1 | 1.02 |
| 38 | S038W | 1.15 | 0.75 | 0.77 | 1.04 | 1.3 | 0.91 |
| 38 | S038Y | ***0.05*** | ***0.05*** | ***0.05*** | ***0.05*** | ***0.05*** | ***0.05*** |
| 39 | H039A | 0.29 | 0.81 | 1.17 | 1.04 | ***0.05*** | 0.76 |
| 39 | H039C | 0.96 | 0.80 | 1.08 | 0.99 | ***0.05*** | 1.00 |
| 39 | H039D | 0.20 | ***0.05*** | 0.11 | ***0.05*** | ***0.05*** | ***0.05*** |
| 39 | H039E | 0.18 | ***0.05*** | 0.22 | 0.09 | ***0.05*** | ***0.05*** |
| 39 | H039F | 0.26 | 0.70 | 1.65 | 1.18 | ***0.05*** | 0.73 |
| 39 | H039G | 0.15 | ***0.05*** | ***0.05*** | ***0.05*** | 0.1 | 0.10 |
| 39 | H039I | 0.50 | 1.02 | 1.03 | 0.97 | ***0.05*** | 1.18 |
| 39 | H039K | 0.33 | ***0.05*** | ***0.05*** | ***0.05*** | ***0.05*** | ***0.05*** |
| 39 | H039L | 0.27 | 0.87 | 1.31 | 1.42 | ***0.05*** | 0.84 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 39 | H039M | 0.24 | 0.98 | 1.54 | 1.16 | ***0.05*** | 0.64 |
| 39 | H039N | 0.77 | 0.82 | 1.02 | 1.32 | ***0.05*** | 0.92 |
| 39 | H039P | 0.23 | 0.12 | 0.09 | ***0.05*** | ***0.05*** | ***0.05*** |
| 39 | H039Q | 1.07 | 0.90 | 0.95 | 0.93 | 0.4 | 1.19 |
| 39 | H039R | 0.32 | ***0.05*** | ***0.05*** | ***0.05*** | ***0.05*** | ***0.05*** |
| 39 | H039S | 0.48 | 0.80 | 0.90 | 1.02 | ***0.05*** | 0.96 |
| 39 | H039T | 0.37 | 1.07 | 1.35 | 1.31 | ***0.05*** | 1.20 |
| 39 | H039V | 0.69 | 0.85 | 1.10 | 1.17 | ***0.05*** | 1.13 |
| 39 | H039W | 0.87 | 0.88 | 1.00 | 1.04 | ***0.05*** | 1.10 |
| 39 | H039Y | 0.14 | 0.50 | ***0.05*** | ***0.05*** | ***0.05*** | ***0.05*** |
| 40 | P040A | 1.01 | 1.23 | 1.09 | 1.08 | 0.8 | 1.22 |
| 40 | P040C | 0.99 | 1.04 | 1.19 | 1.15 | 1.1 | 1.12 |
| 40 | P040D | ***0.05*** | ***0.05*** | ***0.05*** | ***0.05*** | ***0.05*** | ***0.05*** |
| 40 | P040E | 1.21 | 1.05 | 1.34 | 1.05 | 1.6 | 1.01 |
| 40 | P040F | 0.92 | 1.12 | 1.21 | 1.09 | 0.9 | 1.02 |
| 40 | P040G | 1.06 | 1.04 | 1.11 | 1.14 | 0.1 | 1.11 |
| 40 | P040H | 0.89 | 0.97 | 0.98 | 1.17 | 0.5 | 1.09 |
| 40 | P040I | 0.83 | 1.12 | 1.30 | 1.10 | 0.7 | 1.23 |
| 40 | P040K | 1.20 | 0.78 | 0.96 | 0.99 | ***0.05*** | 1.02 |
| 40 | P040L | 1.06 | 1.20 | 1.05 | 1.10 | 1.0 | 1.09 |
| 40 | P040M | 1.01 | 1.07 | 1.24 | 1.10 | 0.6 | 0.98 |
| 40 | P040N | 0.88 | 1.12 | 1.14 | 1.07 | 0.5 | 1.18 |
| 40 | P040Q | 1.01 | 1.16 | 1.13 | 1.11 | 1.1 | 1.01 |
| 40 | P040R | 1.10 | 0.76 | 0.84 | 1.11 | ***0.05*** | 1.03 |
| 40 | P040S | 0.84 | 1.15 | 1.06 | 0.81 | 0.3 | 1.12 |
| 40 | P040T | 0.81 | 1.29 | 1.68 | 1.22 | 0.1 | 1.20 |
| 40 | P040V | 0.91 | 1.04 | 1.07 | 1.09 | 0.7 | 1.06 |
| 40 | P040W | 1.09 | 0.87 | 0.90 | 1.13 | 1.0 | 1.12 |
| 40 | P040Y | 0.89 | 1.07 | 1.07 | 1.06 | 0.5 | 1.02 |
| 41 | D041A | 0.22 | 2.48 | ***0.05*** | 0.77 | 0.1 | 0.18 |
| 41 | D041C | 0.26 | 1.70 | 0.30 | 1.07 | ***0.05*** | 0.48 |
| 41 | D041E | 0.67 | 1.07 | 1.03 | 0.91 | ***0.05*** | 0.92 |
| 41 | D041F | 0.29 | 0.13 | ***0.05*** | ***0.05*** | ***0.05*** | ***0.05*** |
| 41 | D041G | 0.17 | ***0.05*** | ***0.05*** | ***0.05*** | 0.1 | 0.10 |
| 41 | D041H | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 41 | D041I | ***0.05*** | ***0.05*** | ***0.05*** | ***0.05*** | ***0.05*** | ***0.05*** |
| 41 | D041K | 0.23 | 1.13 | 0.27 | 0.22 | ***0.05*** | ***0.05*** |
| 41 | D041L | 0.26 | 0.10 | ***0.05*** | 0.09 | ***0.05*** | ***0.05*** |
| 41 | D041M | 0.21 | 10.40 | ***0.05*** | 0.63 | ***0.05*** | ***0.05*** |
| 41 | D041N | 0.25 | 2.10 | 1.29 | 1.42 | ***0.05*** | 0.45 |
| 41 | D041P | 0.40 | ***0.05*** | ***0.05*** | ***0.05*** | ***0.05*** | ***0.05*** |
| 41 | D041Q | 0.19 | ***0.05*** | ***0.05*** | 13.41 | ***0.05*** | 0.15 |
| 41 | D041R | 0.41 | ***0.05*** | ***0.05*** | ***0.05*** | ***0.05*** | ***0.05*** |
| 41 | D041S | 0.23 | 3.12 | 1.52 | 1.20 | 0.5 | 0.33 |
| 41 | D041T | 0.19 | ***0.05*** | 0.19 | ***0.05*** | ***0.05*** | ***0.05*** |
| 41 | D041V | 0.18 | ***0.05*** | ***0.05*** | ***0.05*** | ***0.05*** | ***0.05*** |
| 41 | D041W | 0.14 | ***0.05*** | ***0.05*** | ***0.05*** | ***0.05*** | ***0.05*** |
| 41 | D041Y | 0.19 | ***0.05*** | ***0.05*** | 11.39 | ***0.05*** | ***0.05*** |
| 42 | L042A | 0.18 | ***0.05*** | 0.58 | 0.48 | 0.4 | 0.13 |
| 42 | L042C | 0.41 | 0.71 | 0.95 | 1.06 | 0.2 | 1.08 |
| 42 | L042D | 0.29 | ***0.05*** | ***0.05*** | ***0.05*** | ***0.05*** | ***0.05*** |
| 42 | L042E | ***0.05*** | ***0.05*** | ***0.05*** | ***0.05*** | ***0.05*** | ***0.05*** |
| 42 | L042F | 0.32 | 0.81 | 0.79 | 0.99 | ***0.05*** | 0.57 |
| 42 | L042G | 0.16 | ***0.05*** | 12.92 | ***0.05*** | ***0.05*** | ***0.05*** |
| 42 | L042H | 0.23 | 0.98 | 1.26 | 1.11 | 0.3 | 0.38 |
| 42 | L042I | 1.15 | 0.87 | 0.56 | 0.92 | 0.8 | 0.77 |
| 42 | L042K | ***0.05*** | ***0.05*** | ***0.05*** | ***0.05*** | ***0.05*** | ***0.05*** |
| 42 | L042M | 0.80 | 1.20 | 0.99 | 1.00 | 0.7 | 0.92 |
| 42 | L042N | 0.23 | 0.80 | 0.86 | 1.21 | 0.6 | 0.48 |
| 42 | L042P | 0.38 | ***0.05*** | ***0.05*** | ***0.05*** | ***0.05*** | ***0.05*** |
| 42 | L042Q | 0.14 | ***0.05*** | ***0.05*** | ***0.05*** | 0.2 | 0.07 |
| 42 | L042R | 0.33 | ***0.05*** | ***0.05*** | ***0.05*** | ***0.05*** | ***0.05*** |
| 42 | L042S | 0.16 | 32.78 | ***0.05*** | 0.37 | ***0.05*** | ***0.05*** |
| 42 | L042T | ***0.05*** | ***0.05*** | ***0.05*** | ***0.05*** | ***0.05*** | ***0.05*** |
| 42 | L042V | 0.97 | 0.96 | 0.88 | 0.98 | 0.5 | 0.82 |
| 42 | L042W | 0.14 | 0.18 | ***0.05*** | ***0.05*** | ***0.05*** | ***0.05*** |
| 42 | L042Y | 0.17 | 3.62 | 4.35 | 1.94 | ***0.05*** | 0.35 |
| 43 | K043A | 0.87 | 0.67 | 1.08 | 0.90 | 1.2 | 1.01 |
| 43 | K043C | 0.83 | 0.60 | 1.17 | 0.89 | 1.4 | 0.99 |
| 43 | K043D | 0.88 | 0.70 | 1.16 | 0.71 | 1.3 | 1.03 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 43 | K043E | 0.80 | 0.56 | 1.04 | 0.74 | 1.3 | 1.13 |
| 43 | K043F | 0.74 | 0.72 | 1.24 | 0.78 | 1.3 | 1.02 |
| 43 | K043G | 0.96 | 0.88 | 0.82 | 0.93 | 1.3 | 1.06 |
| 43 | K043H | ***0.05*** | ***0.05*** | ***0.05*** | ***0.05*** | ***0.05*** | ***0.05*** |
| 43 | K043I | 0.72 | 0.57 | 1.12 | 0.95 | 0.7 | 1.17 |
| 43 | K043L | 0.96 | 0.58 | 0.88 | 0.84 | 1.3 | 0.88 |
| 43 | K043M | 1.02 | 0.79 | 1.19 | 0.97 | 1.1 | 1.07 |
| 43 | K043N | 0.80 | 0.85 | 1.31 | 0.81 | 1.2 | 1.32 |
| 43 | K043P | 0.77 | 0.67 | 1.11 | 0.88 | ***0.05*** | 1.02 |
| 43 | K043Q | 0.95 | 0.61 | 1.09 | 0.90 | 1.3 | 1.05 |
| 43 | K043R | 1.14 | 0.56 | 1.30 | 0.87 | 0.9 | 1.33 |
| 43 | K043S | 0.98 | 0.77 | 1.14 | 0.92 | 1.3 | 0.99 |
| 43 | K043T | 0.93 | 0.92 | 1.06 | 0.96 | 1.1 | 1.07 |
| 43 | K043V | 1.10 | 0.59 | 0.98 | 0.93 | 0.6 | 1.26 |
| 43 | K043W | 0.85 | 0.67 | 0.98 | 0.90 | 1.3 | 1.03 |
| 43 | K043Y | 0.87 | 0.67 | 1.12 | 0.82 | 1.1 | 1.60 |
| 44 | V044A | 0.71 | 0.91 | 1.16 | 1.01 | 0.7 | 0.81 |
| 44 | V044C | 0.93 | 0.94 | 1.26 | 0.79 | 0.8 | 0.90 |
| 44 | V044D | ***0.05*** | ***0.05*** | ***0.05*** | ***0.05*** | ***0.05*** | ***0.05*** |
| 44 | V044E | 0.18 | 0.71 | 2.18 | 1.98 | 0.5 | 0.19 |
| 44 | V044F | 0.22 | 0.77 | 2.45 | 1.34 | ***0.05*** | ***0.05*** |
| 44 | V044G | 0.21 | 0.75 | 1.66 | 1.12 | 0.5 | 0.34 |
| 44 | V044H | 0.19 | 1.63 | 2.57 | 1.57 | 0.5 | 0.38 |
| 44 | V044I | 0.92 | 0.76 | 0.98 | 1.07 | 0.9 | 1.08 |
| 44 | V044K | 0.22 | ***0.05*** | 0.70 | 0.36 | 0.6 | 0.13 |
| 44 | V044L | 0.85 | 0.67 | 1.03 | 0.85 | 0.8 | 0.88 |
| 44 | V044M | 0.65 | 0.95 | 1.33 | 1.03 | 0.6 | 0.82 |
| 44 | V044N | 0.16 | ***0.05*** | ***0.05*** | 1.41 | 0.5 | 0.13 |
| 44 | V044P | 0.67 | 0.83 | 1.25 | 1.03 | 0.4 | 0.78 |
| 44 | V044Q | 0.20 | 1.48 | 2.85 | 1.49 | 0.6 | 0.33 |
| 44 | V044R | 0.19 | 0.79 | 1.62 | 0.69 | 0.2 | 0.13 |
| 44 | V044S | 0.33 | 1.03 | 1.51 | 1.13 | 0.5 | 0.86 |
| 44 | V044T | 0.30 | 1.32 | 1.94 | 1.30 | 0.7 | 1.26 |
| 44 | V044W | 0.13 | ***0.05*** | ***0.05*** | ***0.05*** | 0.4 | 0.06 |
| 44 | V044Y | 0.62 | 0.79 | 1.22 | 1.13 | 0.7 | 1.04 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 45 | A045C | 1.09 | 0.89 | 0.76 | 0.89 | 1.2 | 1.05 |
| 45 | A045E | 1.06 | 1.10 | 0.97 | 0.82 | 1.2 | 1.07 |
| 45 | A045F | 1.04 | 0.88 | 0.81 | 1.00 | 1.0 | 1.11 |
| 45 | A045H | 1.04 | 0.86 | 0.76 | 0.91 | 1.1 | 1.11 |
| 45 | A045I | 1.07 | 1.08 | 0.81 | 1.16 | 1.2 | 1.03 |
| 45 | A045K | 1.05 | 1.00 | 0.69 | 1.06 | 0.9 | 1.03 |
| 45 | A045L | 1.03 | 0.72 | 0.82 | 0.99 | 1.0 | 1.19 |
| 45 | A045M | 1.15 | 1.02 | 0.87 | 1.00 | 1.2 | 1.09 |
| 45 | A045N | 1.35 | 1.03 | 0.91 | 0.88 | 1.1 | 1.03 |
| 45 | A045P | 0.87 | 0.82 | 0.90 | 0.96 | 0.7 | 1.20 |
| 45 | A045Q | 1.07 | 0.84 | 0.95 | 1.09 | 1.1 | 1.16 |
| 45 | A045R | 0.18 | ***0.05*** | ***0.05*** | ***0.05*** | 1.0 | 0.11 |
| 45 | A045S | 1.08 | 0.93 | 0.87 | 0.86 | 1.2 | 1.02 |
| 45 | A045T | 0.99 | 1.04 | 1.31 | 0.90 | 1.2 | 1.01 |
| 45 | A045V | 1.28 | 0.85 | 0.81 | 1.03 | 1.1 | 1.12 |
| 45 | A045Y | 1.03 | 0.81 | 0.67 | 0.73 | 1.1 | 1.13 |
| 46 | G046A | 0.92 | 1.09 | 0.97 | 1.00 | 1.0 | 0.90 |
| 46 | G046C | 0.39 | 1.28 | 0.81 | 0.90 | 1.1 | 0.82 |
| 46 | G046E | 0.57 | 1.10 | 0.70 | 0.95 | 1.0 | 1.16 |
| 46 | G046F | 0.39 | 1.40 | 0.95 | 1.03 | 1.0 | 0.88 |
| 46 | G046H | 0.48 | 0.98 | 0.99 | 1.25 | 1.0 | 1.20 |
| 46 | G046I | 0.20 | ***0.05*** | ***0.05*** | 5.77 | 0.9 | 0.43 |
| 46 | G046K | 0.44 | 0.88 | 0.72 | 1.09 | 0.8 | 1.20 |
| 46 | G046L | 0.27 | 1.57 | 1.44 | 1.42 | 0.8 | 0.78 |
| 46 | G046M | 0.42 | 1.30 | 0.84 | 1.14 | 0.9 | 0.96 |
| 46 | G046N | 0.69 | 0.79 | 0.72 | 1.05 | 1.1 | 0.94 |
| 46 | G046P | 0.17 | ***0.05*** | ***0.05*** | ***0.05*** | 0.9 | 0.06 |
| 46 | G046Q | 0.15 | ***0.05*** | ***0.05*** | ***0.05*** | 1.5 | 0.23 |
| 46 | G046S | 0.74 | 0.86 | 0.78 | 0.94 | 0.9 | 1.16 |
| 46 | G046T | 0.39 | 1.21 | 0.96 | 0.79 | 0.8 | 1.04 |
| 46 | G046V | 0.23 | 4.92 | 3.26 | 2.09 | 0.8 | 0.74 |
| 46 | G046W | 0.44 | 1.08 | 0.66 | 0.90 | 1.0 | 1.13 |
| 46 | G046Y | 0.36 | 1.02 | 1.27 | 1.12 | 0.8 | 1.23 |
| 47 | G047A | 0.32 | 0.98 | 0.96 | 1.04 | 0.5 | 0.30 |
| 47 | G047C | 0.24 | 0.64 | 1.09 | 0.88 | 0.6 | 0.15 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 47 | G047D | ***0.05*** | ***0.05*** | ***0.05*** | ***0.05*** | ***0.05*** | ***0.05*** |
| 47 | G047E | 0.23 | 0.72 | 0.99 | 1.02 | 0.6 | 0.14 |
| 47 | G047F | 0.25 | 0.96 | 0.86 | 1.15 | 0.5 | 0.15 |
| 47 | G047H | 0.23 | 1.04 | 1.17 | 1.20 | 1.1 | 0.47 |
| 47 | G047I | 0.17 | 1.25 | 1.59 | 1.31 | ***0.05*** | ***0.05*** |
| 47 | G047K | 0.16 | ***0.05*** | ***0.05*** | 2.16 | ***0.05*** | ***0.05*** |
| 47 | G047L | 0.17 | ***0.05*** | 2.78 | 0.82 | ***0.05*** | ***0.05*** |
| 47 | G047M | 0.22 | 0.63 | 0.89 | 0.87 | 0.5 | 0.10 |
| 47 | G047N | 0.16 | ***0.05*** | ***0.05*** | 2.54 | 0.5 | 0.06 |
| 47 | G047P | 0.39 | ***0.05*** | ***0.05*** | ***0.05*** | ***0.05*** | ***0.05*** |
| 47 | G047Q | 0.16 | ***0.05*** | ***0.05*** | 6.49 | 0.6 | 0.14 |
| 47 | G047R | 0.16 | ***0.05*** | 7.73 | 1.41 | ***0.05*** | ***0.05*** |
| 47 | G047S | 0.21 | 1.19 | 1.04 | 1.24 | 0.5 | 0.15 |
| 47 | G047T | 0.17 | 4.45 | 4.77 | 2.27 | 0.5 | 0.12 |
| 47 | G047V | 0.16 | ***0.05*** | ***0.05*** | 5.71 | 0.6 | 0.13 |
| 47 | G047W | 0.18 | 2.60 | 2.43 | 2.22 | 0.5 | 0.17 |
| 47 | G047Y | ***0.05*** | ***0.05*** | ***0.05*** | ***0.05*** | ***0.05*** | ***0.05*** |
| 48 | A048C | 1.15 | 1.14 | 1.16 | 0.85 | 1.1 | 0.95 |
| 48 | A048D | 1.13 | 1.01 | 1.00 | 0.97 | 1.1 | 0.89 |
| 48 | A048E | 1.17 | 0.84 | 0.93 | 1.02 | 1.2 | 0.90 |
| 48 | A048F | 1.08 | 1.08 | 0.92 | 0.96 | 1.0 | 0.89 |
| 48 | A048H | 1.08 | 0.87 | 1.16 | 1.04 | 1.1 | 0.87 |
| 48 | A048I | 1.02 | 0.79 | 0.91 | 1.02 | 1.2 | 1.00 |
| 48 | A048K | 1.13 | 0.90 | 0.55 | 0.91 | 1.1 | 0.90 |
| 48 | A048L | 0.99 | 0.87 | 0.97 | 0.99 | 1.0 | 0.91 |
| 48 | A048M | 0.59 | 1.21 | 0.99 | 0.87 | 1.1 | 0.92 |
| 48 | A048P | 0.17 | ***0.05*** | ***0.05*** | ***0.05*** | 1.0 | 0.22 |
| 48 | A048Q | 1.10 | 0.83 | 0.69 | 1.03 | 1.0 | 1.02 |
| 48 | A048R | 1.11 | 0.83 | 0.51 | 0.98 | 0.9 | 1.05 |
| 48 | A048S | 0.98 | 1.08 | 0.47 | 1.06 | 1.0 | 1.03 |
| 48 | A048T | 0.98 | 1.06 | 0.77 | 0.84 | 1.1 | 0.97 |
| 48 | A048V | 1.47 | 0.99 | 0.61 | 0.92 | 1.0 | 0.94 |
| 48 | A048W | 0.81 | 0.69 | 0.74 | 0.81 | 0.9 | 1.04 |
| 48 | A048Y | 1.03 | 0.84 | 0.81 | 0.86 | 1.0 | 0.90 |
| 49 | S049A | 0.35 | 0.54 | 0.61 | 1.02 | 0.9 | 0.30 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 49 | S049C | 0.61 | 0.48 | 0.69 | 0.97 | 0.5 | 0.38 |
| 49 | S049D | 0.23 | 1.09 | 1.53 | 1.69 | 0.5 | 0.36 |
| 49 | S049E | 0.27 | ***0.05*** | ***0.05*** | ***0.05*** | ***0.05*** | ***0.05*** |
| 49 | S049F | 0.33 | ***0.05*** | ***0.05*** | ***0.05*** | ***0.05*** | ***0.05*** |
| 49 | S049G | 0.28 | 0.78 | 0.75 | 1.64 | 1.2 | 0.50 |
| 49 | S049H | 0.23 | 0.12 | 0.39 | 0.39 | ***0.05*** | ***0.05*** |
| 49 | S049I | 0.18 | 2.22 | 2.52 | 2.14 | 0.5 | 0.11 |
| 49 | S049K | 0.23 | ***0.05*** | ***0.05*** | 0.17 | ***0.05*** | ***0.05*** |
| 49 | S049L | 0.23 | ***0.05*** | ***0.05*** | 0.10 | ***0.05*** | ***0.05*** |
| 49 | S049N | 0.35 | 1.04 | 0.88 | 1.32 | 0.5 | 0.64 |
| 49 | S049P | 0.30 | ***0.05*** | ***0.05*** | 0.09 | ***0.05*** | ***0.05*** |
| 49 | S049Q | 0.23 | ***0.05*** | 0.07 | 0.18 | ***0.05*** | ***0.05*** |
| 49 | S049R | 0.33 | ***0.05*** | ***0.05*** | ***0.05*** | ***0.05*** | ***0.05*** |
| 49 | S049T | 0.29 | 0.95 | 1.23 | 1.37 | 0.5 | 0.46 |
| 49 | S049V | 0.21 | 1.58 | 1.39 | 2.10 | 0.4 | 0.30 |
| 49 | S049W | 0.20 | ***0.05*** | ***0.05*** | ***0.05*** | ***0.05*** | ***0.05*** |
| 49 | S049Y | 0.27 | ***0.05*** | ***0.05*** | ***0.05*** | ***0.05*** | ***0.05*** |
| 50 | M050A | 0.78 | 1.16 | 0.85 | 0.89 | 0.9 | 1.09 |
| 50 | M050C | 0.89 | 1.17 | 0.89 | 0.80 | 1.1 | 1.01 |
| 50 | M050D | 0.17 | 6.35 | 0.87 | 0.90 | 1.0 | 0.16 |
| 50 | M050F | 0.95 | 1.01 | 0.96 | 0.81 | 1.1 | 1.09 |
| 50 | M050H | 0.85 | 1.22 | 0.98 | 0.95 | 1.2 | 1.01 |
| 50 | M050I | 0.33 | 1.61 | 0.91 | 1.09 | 0.9 | 0.55 |
| 50 | M050K | 0.49 | 0.80 | 0.67 | 0.79 | 1.1 | 1.07 |
| 50 | M050L | 0.91 | 0.76 | 1.02 | 0.96 | 1.1 | 1.00 |
| 50 | M050N | 0.35 | 1.03 | 0.86 | 1.07 | 1.0 | 1.11 |
| 50 | M050P | 0.20 | 0.64 | ***0.05*** | 0.16 | ***0.05*** | ***0.05*** |
| 50 | M050Q | 0.76 | 1.23 | 0.93 | 1.03 | 1.1 | 1.01 |
| 50 | M050R | 0.25 | 1.38 | 0.73 | 1.07 | 1.0 | 0.82 |
| 50 | M050S | 0.81 | 1.08 | 0.89 | 1.05 | 1.0 | 1.08 |
| 50 | M050T | 0.87 | 1.05 | 0.90 | 1.04 | 1.1 | 1.38 |
| 50 | M050V | 0.70 | 1.08 | 0.82 | 1.02 | 1.1 | 0.82 |
| 50 | M050W | 1.06 | 1.10 | 0.80 | 0.78 | 1.0 | 1.16 |
| 50 | M050Y | 0.70 | 1.03 | 0.87 | 0.89 | 1.0 | 1.19 |
| 51 | V051A | 0.38 | 1.41 | 1.25 | 1.22 | 0.9 | 0.43 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 51 | V051C | 0.91 | 1.02 | 1.16 | 1.02 | 1.1 | 0.41 |
| 51 | V051D | 0.34 | 1.02 | 1.32 | 1.12 | 1.0 | 0.50 |
| 51 | V051E | 0.39 | 1.17 | 1.59 | 1.26 | 1.0 | 0.59 |
| 51 | V051F | ***0.05*** | ***0.05*** | ***0.05*** | ***0.05*** | ***0.05*** | ***0.05*** |
| 51 | V051G | 0.22 | 3.43 | 4.27 | 1.35 | ***0.05*** | ***0.05*** |
| 51 | V051H | 0.86 | 0.59 | 1.06 | 0.98 | 1.2 | 0.79 |
| 51 | V051I | 0.69 | 0.79 | 1.23 | 1.12 | 1.1 | 0.95 |
| 51 | V051K | ***0.05*** | ***0.05*** | ***0.05*** | ***0.05*** | ***0.05*** | ***0.05*** |
| 51 | V051L | 0.48 | 0.73 | 1.21 | 0.87 | 1.0 | 0.59 |
| 51 | V051M | 0.60 | 0.97 | 1.07 | 1.10 | 1.0 | 0.67 |
| 51 | V051N | ***0.05*** | ***0.05*** | ***0.05*** | ***0.05*** | ***0.05*** | ***0.05*** |
| 51 | V051P | 0.20 | ***0.05*** | ***0.05*** | 4.63 | ***0.05*** | ***0.05*** |
| 51 | V051Q | 0.28 | 1.26 | 1.81 | 1.14 | 0.9 | 0.40 |
| 51 | V051R | 0.18 | ***0.05*** | ***0.05*** | ***0.05*** | 1.3 | 0.10 |
| 51 | V051S | 0.32 | 1.84 | 1.95 | 1.43 | 0.9 | 0.35 |
| 51 | V051T | 0.46 | 0.98 | 1.16 | 1.03 | 0.9 | 0.75 |
| 51 | V051W | 0.18 | ***0.05*** | ***0.05*** | ***0.05*** | ***0.05*** | ***0.05*** |
| 51 | V051Y | 0.25 | 1.20 | 1.58 | 1.76 | 0.9 | 0.31 |
| 52 | P052A | 0.56 | 1.22 | 1.25 | 1.17 | 0.7 | 0.59 |
| 52 | P052C | 0.34 | 1.36 | 1.56 | 0.94 | 1.0 | 0.44 |
| 52 | P052D | 0.79 | 1.27 | 1.50 | 1.21 | 0.9 | 0.76 |
| 52 | P052E | 0.89 | 1.12 | 1.54 | 1.20 | 0.9 | 0.75 |
| 52 | P052F | 0.41 | 0.87 | 1.13 | 1.14 | 0.9 | 0.51 |
| 52 | P052G | 0.44 | 1.05 | 1.42 | 1.24 | 0.8 | 0.53 |
| 52 | P052H | 0.45 | 0.97 | 1.28 | 1.04 | 0.9 | 0.47 |
| 52 | P052I | 0.45 | 1.33 | 1.50 | 1.11 | 0.8 | 0.61 |
| 52 | P052K | 0.38 | 0.86 | 1.09 | 1.35 | 0.8 | 0.53 |
| 52 | P052L | 0.46 | 1.33 | 1.24 | 1.32 | 0.8 | 0.61 |
| 52 | P052M | 0.43 | 1.40 | 1.30 | 1.06 | 0.7 | 0.54 |
| 52 | P052N | ***0.05*** | ***0.05*** | ***0.05*** | ***0.05*** | ***0.05*** | ***0.05*** |
| 52 | P052Q | 0.43 | 0.83 | 1.48 | 1.20 | 0.8 | 0.63 |
| 52 | P052R | 0.33 | 1.02 | 1.31 | 1.26 | 0.7 | 0.58 |
| 52 | P052S | 0.51 | 1.02 | 1.55 | 1.26 | 0.8 | 0.60 |
| 52 | P052T | 0.47 | 1.23 | 1.73 | 1.09 | 0.8 | 0.61 |
| 52 | P052V | 0.31 | 1.79 | 1.63 | 1.03 | 1.0 | 0.50 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 52 | P052W | 0.36 | 1.49 | 1.34 | 1.43 | 1.0 | 0.77 |
| 52 | P052Y | 0.35 | 1.55 | 1.42 | 1.32 | 1.0 | 0.55 |
| 53 | S053A | 1.37 | 1.07 | 1.15 | 1.03 | 1.0 | 0.87 |
| 53 | S053C | ***0.05*** | ***0.05*** | ***0.05*** | ***0.05*** | ***0.05*** | ***0.05*** |
| 53 | S053D | ***0.05*** | ***0.05*** | ***0.05*** | ***0.05*** | ***0.05*** | ***0.05*** |
| 53 | S053E | 1.52 | 1.05 | 1.16 | 1.12 | 1.0 | 0.98 |
| 53 | S053F | 1.19 | 0.91 | 1.05 | 0.87 | 1.0 | 1.11 |
| 53 | S053G | 1.72 | 1.15 | 1.19 | 0.94 | 1.0 | 0.86 |
| 53 | S053H | 1.35 | 0.96 | 1.02 | 0.96 | 1.1 | 0.84 |
| 53 | S053I | 1.22 | 0.97 | 1.10 | 0.80 | 1.0 | 0.90 |
| 53 | S053K | 1.36 | 0.89 | 0.93 | 0.76 | 1.1 | 0.95 |
| 53 | S053L | 1.18 | 0.92 | 0.97 | 1.00 | 1.1 | 0.90 |
| 53 | S053M | 1.54 | 1.01 | 0.98 | 0.97 | 0.8 | 0.80 |
| 53 | S053N | 1.48 | 0.95 | 1.04 | 1.15 | 1.1 | 0.87 |
| 53 | S053P | 0.46 | 1.01 | 1.39 | 0.94 | 0.9 | 0.85 |
| 53 | S053Q | 0.76 | 0.85 | 1.30 | 1.09 | 1.1 | 1.05 |
| 53 | S053R | 1.06 | 0.68 | 0.82 | 0.87 | 1.1 | 1.00 |
| 53 | S053T | 1.33 | 1.05 | 1.23 | 0.85 | 1.0 | 0.94 |
| 53 | S053V | 1.13 | 1.11 | 1.20 | 1.10 | 1.1 | 0.95 |
| 53 | S053W | 1.49 | 0.86 | 1.02 | 0.80 | 0.9 | 0.98 |
| 53 | S053Y | ***0.05*** | ***0.05*** | ***0.05*** | ***0.05*** | ***0.05*** | ***0.05*** |
| 54 | E054A | 0.89 | 0.97 | 1.07 | 0.83 | 0.6 | 0.66 |
| 54 | E054C | 0.71 | 0.94 | 1.05 | 0.85 | 0.8 | 0.68 |
| 54 | E054D | ***0.05*** | ***0.05*** | ***0.05*** | ***0.05*** | ***0.05*** | ***0.05*** |
| 54 | E054F | 0.51 | 0.86 | 0.97 | 0.93 | 0.7 | 0.63 |
| 54 | E054G | 0.38 | 1.27 | 0.59 | 1.04 | 0.5 | 0.42 |
| 54 | E054H | 0.73 | 0.69 | 0.80 | 0.96 | 0.8 | 0.67 |
| 54 | E054I | ***0.05*** | ***0.05*** | ***0.05*** | ***0.05*** | ***0.05*** | ***0.05*** |
| 54 | E054K | 0.35 | 1.03 | 1.14 | 1.07 | 0.8 | 0.60 |
| 54 | E054L | 0.49 | 1.00 | 1.02 | 0.87 | 0.8 | 0.71 |
| 54 | E054M | 0.63 | 1.06 | 0.99 | 1.03 | 0.7 | 0.61 |
| 54 | E054N | 0.97 | 0.99 | 1.03 | 1.06 | 0.8 | 0.76 |
| 54 | E054P | 0.19 | 0.47 | 1.13 | 1.34 | 0.6 | 0.17 |
| 54 | E054Q | 0.97 | 1.00 | 0.99 | 1.04 | 0.8 | 0.90 |
| 54 | E054R | 0.24 | 1.47 | 1.83 | 1.68 | 0.6 | 0.55 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 54 | E054S | 0.98 | 0.97 | 0.97 | 0.96 | 0.8 | 0.69 |
| 54 | E054T | 0.52 | 1.02 | 1.11 | 0.93 | 0.6 | 0.70 |
| 54 | E054V | 0.41 | 1.12 | 1.34 | 1.11 | 0.6 | 0.78 |
| 54 | E054W | 0.31 | 1.18 | 1.18 | 1.01 | 0.6 | 0.71 |
| 54 | E054Y | 0.42 | 1.03 | 0.99 | 1.00 | 0.8 | 0.69 |
| 55 | T055A | 1.11 | 0.63 | 0.98 | 1.00 | 1.0 | 0.96 |
| 55 | T055C | 1.41 | 0.64 | 1.06 | 0.89 | 1.1 | 0.86 |
| 55 | T055D | 0.76 | 1.01 | 1.26 | 0.94 | 1.1 | 1.06 |
| 55 | T055E | ***0.05*** | ***0.05*** | ***0.05*** | ***0.05*** | ***0.05*** | ***0.05*** |
| 55 | T055F | 0.67 | 0.62 | 0.78 | 0.84 | 0.9 | 0.92 |
| 55 | T055G | 1.21 | 1.00 | 0.90 | 0.97 | 1.1 | 0.85 |
| 55 | T055H | 1.06 | 1.14 | 0.98 | 0.96 | 1.1 | 1.04 |
| 55 | T055I | 0.96 | 1.08 | 0.97 | 0.89 | 1.0 | 1.02 |
| 55 | T055K | 1.27 | 0.59 | 0.87 | 0.98 | 1.0 | 1.09 |
| 55 | T055L | 1.39 | 0.80 | 1.30 | 0.96 | 1.1 | 0.95 |
| 55 | T055M | 0.92 | 0.85 | 1.19 | 0.99 | 1.0 | 1.09 |
| 55 | T055N | ***0.05*** | ***0.05*** | ***0.05*** | ***0.05*** | ***0.05*** | ***0.05*** |
| 55 | T055P | 0.94 | 0.90 | 1.20 | 0.86 | 1.0 | 1.23 |
| 55 | T055Q | 1.06 | 0.87 | 1.20 | 0.91 | 1.1 | 1.06 |
| 55 | T055R | 1.14 | 0.54 | 0.81 | 0.90 | 1.0 | 1.01 |
| 55 | T055S | 1.00 | 1.03 | 1.32 | 0.94 | 1.1 | 0.99 |
| 55 | T055V | 0.98 | 0.54 | 1.20 | 0.97 | 1.0 | 1.10 |
| 55 | T055W | 1.06 | 0.68 | 0.81 | 0.92 | 1.0 | 0.95 |
| 55 | T055Y | 0.97 | 0.90 | 0.97 | 0.91 | 1.0 | 0.91 |
| 56 | N056A | 0.31 | 1.18 | 1.27 | 1.14 | 0.4 | 0.34 |
| 56 | N056C | ***0.05*** | ***0.05*** | ***0.05*** | ***0.05*** | ***0.05*** | ***0.05*** |
| 56 | N056D | 1.09 | 0.79 | 0.89 | 0.84 | 1.2 | 0.99 |
| 56 | N056E | 0.67 | 0.91 | 0.98 | 0.91 | 0.7 | 0.69 |
| 56 | N056F | 0.27 | 1.02 | 1.00 | 1.31 | 0.6 | 0.31 |
| 56 | N056G | 0.29 | 1.61 | 1.06 | 1.22 | 0.4 | 0.46 |
| 56 | N056H | 0.39 | 1.08 | 1.07 | 0.95 | 0.8 | 0.81 |
| 56 | N0561 | 0.24 | 1.69 | 1.26 | 1.41 | 0.6 | 0.47 |
| 56 | N056K | 0.21 | 2.07 | 1.45 | 1.86 | 0.5 | 0.38 |
| 56 | N056L | 0.23 | 1.26 | 1.81 | 1.66 | 0.7 | 0.34 |
| 56 | N056M | 0.26 | 1.50 | 1.24 | 1.23 | 0.5 | 0.36 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 56 | N056P | 0.26 | 0.95 | 1.32 | 0.94 | 0.8 | 0.62 |
| 56 | N056Q | 0.29 | 1.74 | 1.29 | 1.22 | 0.8 | 0.41 |
| 56 | N056R | 0.17 | 4.90 | 3.96 | 2.20 | 0.5 | 0.27 |
| 56 | N056S | 0.99 | 0.78 | 0.97 | 0.92 | 0.9 | 0.77 |
| 56 | N056T | 0.58 | 0.90 | 1.08 | 1.04 | 0.6 | 0.65 |
| 56 | N056V | 0.27 | 1.36 | 1.29 | 0.99 | 0.5 | 0.49 |
| 56 | N056W | 0.36 | 1.28 | 1.33 | 1.16 | 0.6 | 0.48 |
| 56 | N056Y | 0.25 | 1.30 | 1.25 | 1.30 | 0.7 | 0.41 |
| 57 | P057A | 0.61 | 0.95 | 1.08 | 1.03 | 0.5 | 0.66 |
| 57 | P057C | 0.50 | 1.04 | 1.18 | 0.95 | 0.8 | 0.65 |
| 57 | P057D | 0.52 | 1.11 | 1.42 | 1.14 | 0.9 | 0.87 |
| 57 | P057E | 0.38 | 1.23 | 1.53 | 1.23 | 0.8 | 0.87 |
| 57 | P057F | 0.59 | 0.93 | 0.98 | 0.92 | 0.3 | 0.75 |
| 57 | P057G | 0.42 | 1.29 | 1.19 | 1.12 | 0.3 | 0.69 |
| 57 | P057H | ***0.05*** | ***0.05*** | ***0.05*** | ***0.05*** | ***0.05*** | ***0.05*** |
| 57 | P057I | 0.25 | 2.20 | 2.00 | 1.60 | 0.4 | 0.29 |
| 57 | P057K | 0.24 | 1.51 | 1.84 | 1.09 | 0.3 | 0.34 |
| 57 | P057L | 0.24 | 2.59 | 2.00 | 1.46 | 0.4 | 0.28 |
| 57 | P057M | 0.36 | 1.08 | 1.11 | 0.97 | 0.4 | 0.40 |
| 57 | P057N | 0.35 | 1.17 | 1.67 | 1.11 | 0.7 | 0.73 |
| 57 | P057Q | 0.35 | 1.25 | 1.38 | 1.04 | 0.5 | 0.65 |
| 57 | P057R | 0.23 | 2.31 | 3.14 | 1.52 | 0.4 | 0.42 |
| 57 | P057S | 0.39 | 0.91 | 1.10 | 0.97 | 0.5 | 0.65 |
| 57 | P057T | 0.25 | 2.53 | 1.97 | 2.27 | 0.5 | 0.56 |
| 57 | P057V | 0.22 | 3.61 | 5.95 | 3.50 | 0.3 | 0.48 |
| 57 | P057W | 1.03 | 0.61 | 1.05 | 0.94 | 0.7 | 0.86 |
| 57 | P057Y | ***0.05*** | ***0.05*** | ***0.05*** | ***0.05*** | ***0.05*** | ***0.05*** |
| 58 | F058A | 0.55 | 1.18 | 1.32 | 1.15 | 0.6 | 0.61 |
| 58 | F058C | 0.95 | 1.08 | 1.21 | 0.93 | 1.1 | 0.92 |
| 58 | F058D | 0.52 | 1.18 | 1.32 | 0.97 | 1.0 | 0.93 |
| 58 | F058E | 0.87 | 0.87 | 1.20 | 1.09 | 0.9 | 1.05 |
| 58 | F058G | 0.91 | 1.21 | 1.26 | 1.00 | 1.1 | 0.76 |
| 58 | F058H | 0.98 | 0.96 | 1.34 | 0.89 | 0.9 | 0.99 |
| 58 | F058I | 0.64 | 0.88 | 1.07 | 0.94 | 0.8 | 1.00 |
| 58 | F058K | 0.44 | 1.15 | 1.36 | 1.09 | 0.6 | 0.63 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 58 | F058L | 0.89 | 0.99 | 1.16 | 0.89 | 0.9 | 0.86 |
| 58 | F058M | 1.01 | 1.08 | 1.24 | 0.91 | 0.8 | 0.82 |
| 58 | F058N | 0.86 | 1.18 | 1.25 | 0.87 | 0.9 | 0.91 |
| 58 | F058P | 0.23 | 0.71 | 1.00 | 0.89 | 0.6 | 0.22 |
| 58 | F058Q | 0.60 | 1.22 | 1.20 | 0.93 | 0.6 | 0.80 |
| 58 | F058R | 0.49 | 0.75 | 1.06 | 0.95 | 1.0 | 0.73 |
| 58 | F058S | 0.61 | 1.21 | 1.10 | 1.03 | 0.6 | 0.81 |
| 58 | F058T | 0.36 | 1.36 | 1.76 | 1.16 | 0.7 | 1.01 |
| 58 | F058V | 0.73 | 1.01 | 1.09 | 1.05 | 0.7 | 0.98 |
| 58 | F058W | ***0.05*** | ***0.05*** | ***0.05*** | ***0.05*** | ***0.05*** | ***0.05*** |
| 58 | F058Y | 1.16 | 1.19 | 0.90 | 1.00 | 0.9 | 1.09 |
| 59 | Q059A | 1.27 | 0.91 | 1.18 | 0.96 | 0.9 | 0.96 |
| 59 | Q059C | 0.89 | 1.08 | 1.26 | 1.06 | 1.2 | 0.92 |
| 59 | Q059D | 1.42 | 1.09 | 1.31 | 0.88 | 1.2 | 1.01 |
| 59 | Q059E | 1.42 | 1.11 | 1.15 | 0.85 | 1.3 | 0.94 |
| 59 | Q059F | 1.18 | 0.97 | 1.13 | 0.85 | 0.9 | 0.93 |
| 59 | Q059G | 0.92 | 1.06 | 1.05 | 1.00 | 0.7 | 0.75 |
| 59 | Q059H | 1.24 | 0.91 | 1.00 | 1.14 | 1.0 | 0.91 |
| 59 | Q059I | ***0.05*** | ***0.05*** | ***0.05*** | ***0.05*** | ***0.05*** | ***0.05*** |
| 59 | Q059K | 1.31 | 0.83 | 0.87 | 0.87 | 0.9 | 0.94 |
| 59 | Q059L | 1.21 | 1.10 | 1.12 | 1.04 | 1.1 | 1.01 |
| 59 | Q059M | 1.26 | 1.18 | 1.08 | 0.95 | 1.1 | 0.95 |
| 59 | Q059N | 1.15 | 1.12 | 1.16 | 1.01 | 1.0 | 0.98 |
| 59 | Q059P | 0.27 | 1.86 | 2.01 | 1.47 | 0.8 | 0.43 |
| 59 | Q059R | 0.82 | 0.78 | 0.71 | 0.81 | 1.0 | 0.95 |
| 59 | Q059S | 1.25 | 1.13 | 1.15 | 0.94 | 0.8 | 0.97 |
| 59 | Q059T | 1.37 | 1.10 | 1.04 | 1.02 | 0.9 | 0.90 |
| 59 | Q059V | 1.38 | 1.14 | 1.14 | 1.14 | 1.0 | 1.21 |
| 59 | Q059W | 0.24 | 1.36 | 1.62 | 1.16 | 1.0 | 0.46 |
| 59 | Q059Y | 1.12 | 1.08 | 1.21 | 1.08 | 0.9 | 0.86 |
| 60 | D060A | 0.43 | 1.01 | 1.21 | 0.99 | 0.2 | 0.68 |
| 60 | D060C | 0.49 | 0.81 | 0.94 | 0.89 | 0.5 | 0.56 |
| 60 | D060E | 0.32 | 0.89 | 1.23 | 0.94 | 0.5 | 0.45 |
| 60 | D060F | 0.25 | 0.35 | 1.00 | 0.71 | 0.1 | 0.34 |
| 60 | D060G | 1.09 | 0.80 | 0.93 | 0.89 | 0.4 | 0.91 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 60 | D060H | 0.31 | 0.66 | 1.29 | 0.95 | 0.2 | 0.50 |
| 60 | D060I | 0.33 | 0.50 | 1.15 | 0.83 | 0.1 | 0.61 |
| 60 | D060K | ***0.05*** | ***0.05*** | ***0.05*** | ***0.05*** | ***0.05*** | ***0.05*** |
| 60 | D060L | 0.27 | 0.54 | 1.07 | 0.88 | 0.1 | 0.41 |
| 60 | D060M | 0.27 | 0.87 | 1.26 | 1.16 | 0.2 | 0.43 |
| 60 | D060N | 0.32 | 0.77 | 1.19 | 1.11 | 0.2 | 0.55 |
| 60 | D060P | 0.85 | 0.64 | 0.83 | 0.73 | 0.1 | 0.59 |
| 60 | D060Q | 0.26 | 0.97 | 1.35 | 1.03 | 0.2 | 0.57 |
| 60 | D060R | 0.35 | 0.31 | 0.65 | 0.63 | 0.1 | 0.62 |
| 60 | D060S | 0.40 | 0.83 | 0.96 | 1.00 | 0.2 | 0.74 |
| 60 | D060T | 0.30 | 0.95 | 1.72 | 1.28 | 0.2 | 0.78 |
| 60 | D060V | 0.39 | 0.77 | 0.91 | 0.98 | 0.1 | 0.74 |
| 60 | D060W | 0.21 | 0.57 | 1.17 | 1.22 | 0.1 | 0.35 |
| 60 | D060Y | 0.28 | 0.58 | 0.93 | 0.73 | 0.2 | 0.47 |
| 61 | N061A | 1.66 | 1.22 | 1.11 | 1.09 | 1.0 | 0.95 |
| 61 | N061C | 1.41 | 0.95 | 0.96 | 0.90 | 1.1 | 1.11 |
| 61 | N061D | 1.25 | 1.19 | 1.21 | 0.85 | 1.2 | 1.14 |
| 61 | N061E | 1.53 | 1.21 | 1.08 | 1.08 | 1.2 | 1.08 |
| 61 | N061F | 1.50 | 1.00 | 0.90 | 0.80 | 1.0 | 0.89 |
| 61 | N061G | 1.45 | 1.01 | 1.06 | 1.01 | 1.0 | 0.80 |
| 61 | N061H | 1.46 | 1.15 | 1.00 | 0.93 | 1.2 | 0.89 |
| 61 | N061I | 1.32 | 1.25 | 1.00 | 0.71 | 1.2 | 1.10 |
| 61 | N061K | 1.20 | 0.89 | 1.00 | 0.76 | 0.9 | 1.11 |
| 61 | N061L | 1.34 | 0.96 | 1.00 | 0.88 | 1.1 | 1.07 |
| 61 | N061M | 1.28 | 0.98 | 1.01 | 1.04 | 1.0 | 1.06 |
| 61 | N061P | 1.42 | 1.47 | 1.04 | 0.78 | 1.0 | 1.40 |
| 61 | N061Q | 1.27 | 0.90 | 1.08 | 0.88 | 1.0 | 1.14 |
| 61 | N061R | 1.42 | 0.67 | 0.71 | 0.86 | 0.8 | 1.09 |
| 61 | N061S | 1.06 | 1.00 | 0.98 | 0.88 | 0.9 | 1.51 |
| 61 | N061T | 1.14 | 0.98 | 0.98 | 0.99 | 1.1 | 1.19 |
| 61 | N061V | 1.43 | 1.02 | 1.03 | 0.77 | 1.0 | 1.19 |
| 61 | N061W | 1.42 | 1.12 | 0.80 | 0.83 | 1.1 | 0.94 |
| 61 | N061Y | 1.31 | 1.04 | 0.97 | 0.98 | 1.2 | 0.90 |
| 62 | N062A | 1.20 | 0.77 | 0.76 | 0.88 | 1.1 | 1.18 |
| 62 | N062C | 1.57 | 1.18 | 1.16 | 0.82 | 0.3 | 0.63 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 62 | N062D | 1.48 | 1.40 | 1.18 | 0.91 | 1.1 | 0.67 |
| 62 | N062E | 1.42 | 1.19 | 1.19 | 0.92 | 1.1 | 0.59 |
| 62 | N062F | 2.06 | 0.75 | 1.07 | 0.87 | 1.1 | 0.37 |
| 62 | N062G | 1.41 | 1.03 | 0.83 | 0.84 | 1.3 | 0.78 |
| 62 | N062H | ***0.05*** | ***0.05*** | ***0.05*** | ***0.05*** | ***0.05*** | ***0.05*** |
| 62 | N0621 | 0.95 | 0.81 | 0.87 | 0.69 | 1.3 | 0.74 |
| 62 | N062K | 1.23 | 1.05 | 0.97 | 1.02 | 0.9 | 0.48 |
| 62 | N062L | 1.79 | 0.87 | 0.80 | 1.07 | 1.4 | 0.63 |
| 62 | N062M | 1.76 | 1.30 | 1.12 | 1.07 | 1.1 | 0.58 |
| 62 | N062P | ***0.05*** | ***0.05*** | ***0.05*** | ***0.05*** | ***0.05*** | ***0.05*** |
| 62 | N062Q | 1.45 | 1.32 | 1.23 | 1.06 | 1.2 | 0.60 |
| 62 | N062R | 1.33 | 1.13 | 0.73 | 0.94 | 0.9 | 0.29 |
| 62 | N062S | 0.65 | 1.14 | 0.97 | 0.91 | 1.0 | 1.06 |
| 62 | N062T | 0.96 | 1.21 | 0.97 | 0.86 | 1.1 | 1.01 |
| 62 | N062V | 1.43 | 0.86 | 1.00 | 0.57 | 1.1 | 0.56 |
| 62 | N062W | 1.56 | 1.27 | 1.25 | 0.79 | 1.1 | 0.11 |
| 62 | N062Y | 1.70 | 0.92 | 0.79 | 1.09 | 1.2 | 0.37 |
| 63 | S063A | 1.12 | 0.94 | 1.12 | 0.98 | 1.1 | 0.88 |
| 63 | S063C | 1.56 | 1.31 | 0.97 | 0.69 | 1.3 | 0.77 |
| 63 | S063D | 1.42 | 0.90 | 0.86 | 0.84 | 1.4 | 0.81 |
| 63 | S063E | 1.50 | 1.13 | 1.03 | 0.93 | 1.3 | 1.00 |
| 63 | S063F | 1.26 | 0.91 | 0.73 | 0.88 | 1.0 | 0.77 |
| 63 | S063G | 1.46 | 0.67 | 0.79 | 0.83 | 0.9 | 1.21 |
| 63 | S063H | 1.41 | 0.92 | 0.95 | 0.80 | 1.0 | 0.90 |
| 63 | S063I | ***0.05*** | ***0.05*** | ***0.05*** | ***0.05*** | ***0.05*** | ***0.05*** |
| 63 | S063K | 1.62 | 1.03 | 0.75 | 0.89 | 0.3 | 0.82 |
| 63 | S063L | 1.46 | 1.22 | 0.86 | 1.02 | 1.0 | 0.92 |
| 63 | S063M | 1.48 | 1.02 | 0.86 | 1.17 | 0.9 | 1.04 |
| 63 | S063N | 1.42 | 0.97 | 1.00 | 0.97 | 1.2 | 0.85 |
| 63 | S063P | 0.84 | 0.62 | 0.71 | 0.71 | 0.6 | 0.67 |
| 63 | S063Q | 1.55 | 1.36 | 0.81 | 1.05 | 1.0 | 0.98 |
| 63 | S063R | 1.58 | 0.93 | 0.65 | 0.83 | 0.1 | 1.03 |
| 63 | S063T | 1.03 | 1.33 | 1.03 | 1.01 | 1.0 | 0.93 |
| 63 | S063V | 1.19 | 1.13 | 0.88 | 0.98 | 0.7 | 1.35 |
| 63 | S063W | 1.26 | 0.84 | 0.93 | 0.67 | 0.9 | 2.18 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 63 | S063Y | 1.38 | 1.02 | 0.72 | 0.93 | 0.8 | 0.90 |
| 64 | H064C | 0.48 | 0.06 | ***0.05*** | ***0.05*** | ***0.05*** | ***0.05*** |
| 64 | H064D | 0.34 | 0.11 | ***0.05*** | ***0.05*** | ***0.05*** | ***0.05*** |
| 64 | H064F | 0.57 | ***0.05*** | ***0.05*** | ***0.05*** | ***0.05*** | ***0.05*** |
| 64 | H064I | 0.56 | 0.09 | ***0.05*** | ***0.05*** | ***0.05*** | ***0.05*** |
| 64 | H064K | 0.38 | 0.07 | ***0.05*** | ***0.05*** | ***0.05*** | ***0.05*** |
| 64 | H064L | 0.54 | 0.08 | ***0.05*** | ***0.05*** | ***0.05*** | ***0.05*** |
| 64 | H064M | 0.45 | *0.05* | *0.05* | *0.05* | *0.05* | *0.05* |
| 64 | H064Q | 0.57 | *0.05* | *0.05* | *0.05* | *0.05* | *0.05* |
| 64 | H064R | 0.44 | *0.05* | *0.05* | *0.05* | *0.05* | *0.05* |
| 64 | H064S | 0.81 | *0.05* | *0.05* | *0.05* | *0.05* | *0.05* |
| 64 | H064T | 0.70 | ***0.05*** | ***0.05*** | ***0.05*** | ***0.05*** | ***0.05*** |
| 64 | H064V | 0.49 | ***0.05*** | ***0.05*** | ***0.05*** | ***0.05*** | ***0.05*** |
| 64 | H064Y | 0.49 | ***0.05*** | ***0.05*** | ***0.05*** | ***0.05*** | ***0.05*** |
| 65 | G065A | 0.28 | 0.26 | 0.55 | 0.99 | ***0.05*** | ***0.05*** |
| 65 | G065D | 0.51 | ***0.05*** | ***0.05*** | ***0.05*** | ***0.05*** | ***0.05*** |
| 65 | G065F | 0.56 | ***0.05*** | ***0.05*** | ***0.05*** | ***0.05*** | ***0.05*** |
| 65 | G065H | 0.37 | 0.09 | ***0.05*** | ***0.05*** | ***0.05*** | ***0.05*** |
| 65 | G065I | 0.56 | ***0.05*** | ***0.05*** | ***0.05*** | ***0.05*** | ***0.05*** |
| 65 | G065K | 0.49 | ***0.05*** | ***0.05*** | ***0.05*** | ***0.05*** | ***0.05*** |
| 65 | G065L | 0.59 | ***0.05*** | ***0.05*** | ***0.05*** | ***0.05*** | ***0.05*** |
| 65 | G065M | 0.49 | ***0.05*** | ***0.05*** | ***0.05*** | ***0.05*** | ***0.05*** |
| 65 | G065N | 0.57 | ***0.05*** | ***0.05*** | ***0.05*** | ***0.05*** | ***0.05*** |
| 65 | G065P | 0.51 | ***0.05*** | ***0.05*** | ***0.05*** | ***0.05*** | ***0.05*** |
| 65 | G065Q | 0.23 | 1.74 | 2.07 | 2.34 | 1.2 | 0.66 |
| 65 | G065R | 0.48 | ***0.05*** | ***0.05*** | ***0.05*** | ***0.05*** | ***0.05*** |
| 65 | G065S | 0.38 | 0.07 | ***0.05*** | 0.24 | ***0.05*** | ***0.05*** |
| 65 | G065T | 0.52 | ***0.05*** | ***0.05*** | ***0.05*** | ***0.05*** | ***0.05*** |
| 65 | G065V | 0.43 | ***0.05*** | ***0.05*** | ***0.05*** | ***0.05*** | ***0.05*** |
| 65 | G065W | 0.53 | ***0.05*** | ***0.05*** | ***0.05*** | ***0.05*** | ***0.05*** |
| 65 | G065Y | 0.52 | ***0.05*** | ***0.05*** | ***0.05*** | ***0.05*** | ***0.05*** |
| 66 | T066A | 0.22 | ***0.05*** | 0.12 | ***0.05*** | ***0.05*** | ***0.05*** |
| 66 | T066C | ***0.05*** | ***0.05*** | ***0.05*** | ***0.05*** | ***0.05*** | ***0.05*** |
| 66 | T066D | 0.26 | ***0.05*** | ***0.05*** | ***0.05*** | ***0.05*** | ***0.05*** |
| 66 | T066E | 0.31 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 66 | T066F | 0.37 | ***0.05*** | ***0.05*** | ***0.05*** | ***0.05*** | ***0.05*** |
| 66 | T066G | 0.27 | ***0.05*** | ***0.05*** | ***0.05*** | ***0.05*** | ***0.05*** |
| 66 | T066H | 0.26 | ***0.05*** | ***0.05*** | ***0.05*** | ***0.05*** | ***0.05*** |
| 66 | T066I | 0.21 | ***0.05*** | 0.06 | 0.11 | ***0.05*** | ***0.05*** |
| 66 | T066K | 0.31 | ***0.05*** | ***0.05*** | ***0.05*** | ***0.05*** | ***0.05*** |
| 66 | T066L | 0.21 | ***0.05*** | 0.08 | ***0.05*** | ***0.05*** | ***0.05*** |
| 66 | T066M | 0.22 | ***0.05*** | ***0.05*** | 0.06 | ***0.05*** | ***0.05*** |
| 66 | T066N | 0.18 | ***0.05*** | 0.25 | ***0.05*** | ***0.05*** | ***0.05*** |
| 66 | T066P | 0.30 | ***0.05*** | ***0.05*** | ***0.05*** | ***0.05*** | ***0.05*** |
| 66 | T066Q | 0.22 | ***0.05*** | ***0.05*** | ***0.05*** | ***0.05*** | ***0.05*** |
| 66 | T066R | 0.30 | ***0.05*** | ***0.05*** | ***0.05*** | ***0.05*** | ***0.05*** |
| 66 | T066S | 1.10 | 0.87 | 0.95 | 0.89 | 0.6 | 0.81 |
| 66 | T066V | 0.15 | 0.14 | ***0.05*** | ***0.05*** | ***0.05*** | ***0.05*** |
| 66 | T066W | 0.32 | ***0.05*** | ***0.05*** | ***0.05*** | ***0.05*** | ***0.05*** |
| 66 | T066Y | 0.28 | ***0.05*** | ***0.05*** | ***0.05*** | ***0.05*** | ***0.05*** |
| 67 | H067A | 1.37 | 0.43 | 0.24 | 0.50 | 0.5 | 0.34 |
| 67 | H067C | 1.36 | 0.46 | 0.24 | 0.52 | 0.3 | 0.29 |
| 67 | H067D | 0.29 | 0.11 | ***0.05*** | ***0.05*** | ***0.05*** | ***0.05*** |
| 67 | H067F | 1.13 | 0.64 | 0.50 | 0.81 | 0.1 | 0.33 |
| 67 | H067I | 1.55 | 0.51 | 0.20 | 0.47 | 0.2 | 0.42 |
| 67 | H067K | 0.54 | 0.17 | ***0.05*** | 0.24 | ***0.05*** | ***0.05*** |
| 67 | H067L | 1.31 | 0.37 | 0.14 | 0.47 | 0.3 | 0.16 |
| 67 | H067M | 1.34 | 0.56 | 0.31 | 0.66 | 0.6 | 0.42 |
| 67 | H067N | 1.12 | 0.76 | 0.31 | 0.65 | 0.1 | 0.62 |
| 67 | H067P | 1.01 | 0.43 | 0.18 | 0.50 | 0.8 | 0.51 |
| 67 | H067R | 1.33 | 0.22 | 0.06 | 0.30 | ***0.05*** | ***0.05*** |
| 67 | H067S | 1.26 | 0.55 | 0.28 | 0.55 | 0.1 | 0.39 |
| 67 | H067T | 1.23 | 0.57 | 0.36 | 0.69 | 0.1 | 0.52 |
| 67 | H067W | 0.24 | 0.76 | 0.08 | 0.51 | ***0.05*** | 0.95 |
| 68 | V068A | 1.00 | 1.79 | 1.11 | 1.30 | 1.0 | 0.06 |
| 68 | V068C | 1.05 | 1.11 | 1.10 | 1.07 | 0.8 | 0.70 |
| 68 | V068D | 0.26 | 0.49 | 0.55 | 0.55 | ***0.05*** | ***0.05*** |
| 68 | V068E | ***0.05*** | ***0.05*** | ***0.05*** | ***0.05*** | ***0.05*** | ***0.05*** |
| 68 | V068F | 0.46 | ***0.05*** | ***0.05*** | ***0.05*** | ***0.05*** | ***0.05*** |
| 68 | V068G | 0.52 | 1.83 | 1.33 | 1.36 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 68 | V068H | 0.78 | ***0.05*** | ***0.05*** | ***0.05*** | ***0.05*** | ***0.05*** |
| 68 | V068I | 1.19 | 1.15 | 0.96 | 0.82 | 1.2 | 0.32 |
| 68 | V068K | 0.34 | ***0.05*** | 0.06 | ***0.05*** | ***0.05*** | ***0.05*** |
| 68 | V068L | 0.62 | 1.22 | 0.81 | 0.95 | ***0.05*** | ***0.05*** |
| 68 | V068M | 1.17 | 1.31 | 0.92 | 1.16 | ***0.05*** | ***0.05*** |
| 68 | V068N | 0.77 | 0.45 | 0.48 | 0.55 | ***0.05*** | ***0.05*** |
| 68 | V068P | 0.28 | 0.06 | 0.19 | 0.10 | ***0.05*** | ***0.05*** |
| 68 | V068Q | 1.26 | 0.14 | 0.19 | 0.25 | ***0.05*** | ***0.05*** |
| 68 | V068R | 0.33 | ***0.05*** | ***0.05*** | ***0.05*** | ***0.05*** | ***0.05*** |
| 68 | V068S | 1.25 | 1.74 | 1.26 | 1.12 | ***0.05*** | ***0.05*** |
| 68 | V068T | 0.94 | 1.17 | 1.06 | 0.79 | 0.6 | 0.42 |
| 68 | V068W | 0.38 | ***0.05*** | ***0.05*** | ***0.05*** | ***0.05*** | ***0.05*** |
| 68 | V068Y | 0.44 | ***0.05*** | ***0.05*** | ***0.05*** | ***0.05*** | ***0.05*** |
| 69 | A069C | 0.36 | 1.66 | 0.92 | 1.19 | 0.7 | 0.45 |
| 69 | A069D | 0.25 | 2.04 | ***0.05*** | ***0.05*** | ***0.05*** | ***0.05*** |
| 69 | A069E | 0.34 | 1.08 | ***0.05*** | ***0.05*** | ***0.05*** | ***0.05*** |
| 69 | A069F | 0.18 | 13.66 | 1.33 | 0.48 | ***0.05*** | ***0.05*** |
| 69 | A069G | 0.88 | 0.73 | 0.95 | 1.04 | 0.9 | 1.02 |
| 69 | A069H | 0.17 | 39.03 | 2.24 | 0.72 | 0.7 | 0.08 |
| 69 | A069I | 0.16 | ***0.05*** | ***0.05*** | 0.95 | ***0.05*** | ***0.05*** |
| 69 | A069K | 0.20 | ***0.05*** | 0.06 | ***0.05*** | ***0.05*** | ***0.05*** |
| 69 | A069L | 0.15 | 0.89 | ***0.05*** | 0.25 | ***0.05*** | ***0.05*** |
| 69 | A069M | 0.19 | 7.17 | 0.69 | 0.55 | 0.5 | 0.06 |
| 69 | A069N | 0.21 | 2.48 | 0.06 | ***0.05*** | ***0.05*** | ***0.05*** |
| 69 | A069P | 0.32 | 1.77 | ***0.05*** | ***0.05*** | ***0.05*** | ***0.05*** |
| 69 | A069Q | 0.22 | 5.22 | 0.06 | ***0.05*** | ***0.05*** | ***0.05*** |
| 69 | A069R | 0.35 | 2.01 | ***0.05*** | ***0.05*** | ***0.05*** | ***0.05*** |
| 69 | A069S | 0.85 | 0.61 | 0.70 | 1.17 | 0.9 | 0.93 |
| 69 | A069T | 0.48 | 1.58 | 0.96 | 1.02 | 1.0 | 0.72 |
| 69 | A069V | 0.15 | 0.31 | ***0.05*** | ***0.05*** | ***0.05*** | ***0.05*** |
| 69 | A069W | 0.15 | ***0.05*** | ***0.05*** | 5.05 | 1.0 | 0.12 |
| 69 | A069Y | 0.16 | ***0.05*** | ***0.05*** | 0.08 | ***0.05*** | ***0.05*** |
| 70 | G070A | 0.62 | ***0.05*** | ***0.05*** | ***0.05*** | ***0.05*** | ***0.05*** |
| 70 | G070C | 0.41 | ***0.05*** | ***0.05*** | ***0.05*** | ***0.05*** | ***0.05*** |
| 70 | G070D | 0.42 | 0.09 | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 70 | G070F | 0.39 | 0.13 | ***0.05*** | ***0.05*** | ***0.05*** | ***0.05*** |
| 70 | G070H | 0.40 | 0.07 | ***0.05*** | ***0.05*** | ***0.05*** | ***0.05*** |
| 70 | G070I | 0.34 | 0.07 | ***0.05*** | ***0.05*** | ***0.05*** | ***0.05*** |
| 70 | G070K | 0.36 | ***0.05*** | ***0.05*** | ***0.05*** | ***0.05*** | ***0.05*** |
| 70 | G070L | 0.41 | ***0.05*** | ***0.05*** | ***0.05*** | ***0.05*** | ***0.05*** |
| 70 | G070M | 0.44 | ***0.05*** | ***0.05*** | ***0.05*** | ***0.05*** | ***0.05*** |
| 70 | G070N | 0.35 | ***0.05*** | ***0.05*** | ***0.05*** | ***0.05*** | ***0.05*** |
| 70 | G070P | 0.34 | 0.08 | ***0.05*** | ***0.05*** | ***0.05*** | ***0.05*** |
| 70 | G070R | 0.32 | 0.14 | ***0.05*** | ***0.05*** | ***0.05*** | ***0.05*** |
| 70 | G070S | 0.43 | ***0.05*** | ***0.05*** | ***0.05*** | ***0.05*** | ***0.05*** |
| 70 | G070T | 0.33 | 0.07 | ***0.05*** | ***0.05*** | ***0.05*** | ***0.05*** |
| 70 | G070V | 0.43 | ***0.05*** | ***0.05*** | ***0.05*** | ***0.05*** | ***0.05*** |
| 70 | G070W | 0.31 | 0.08 | ***0.05*** | ***0.05*** | ***0.05*** | ***0.05*** |
| 71 | T071A | 0.44 | 1.17 | 1.02 | 1.07 | 0.1 | 0.59 |
| 71 | T071C | ***0.05*** | ***0.05*** | ***0.05*** | ***0.05*** | ***0.05*** | ***0.05*** |
| 71 | T071D | 0.21 | 41.12 | 34.56 | 1.45 | 0.2 | 0.06 |
| 71 | T071E | 0.25 | 2.16 | 1.92 | 1.03 | 0.1 | 0.33 |
| 71 | T071F | 0.20 | 0.24 | 0.40 | ***0.05*** | ***0.05*** | ***0.05*** |
| 71 | T071G | 0.23 | 3.34 | 1.64 | 1.85 | ***0.05*** | 0.36 |
| 71 | T071H | ***0.05*** | ***0.05*** | ***0.05*** | ***0.05*** | ***0.05*** | ***0.05*** |
| 71 | T071I | 0.80 | 0.74 | 1.19 | 1.08 | 0.5 | 1.07 |
| 71 | T071K | 0.21 | 37.02 | 45.54 | 2.17 | 0.1 | 0.17 |
| 71 | T071L | 0.24 | 0.24 | ***0.05*** | ***0.05*** | ***0.05*** | ***0.05*** |
| 71 | T071M | 0.28 | 1.33 | 1.18 | 1.13 | ***0.05*** | 0.39 |
| 71 | T071N | 0.19 | ***0.05*** | ***0.05*** | ***0.05*** | 0.1 | 0.10 |
| 71 | T071P | 0.18 | ***0.05*** | ***0.05*** | ***0.05*** | 0.1 | 0.15 |
| 71 | T071Q | 0.20 | ***0.05*** | ***0.05*** | 2.94 | 0.1 | 0.09 |
| 71 | T071R | 0.18 | ***0.05*** | ***0.05*** | ***0.05*** | ***0.05*** | ***0.05*** |
| 71 | T071S | 1.14 | 0.87 | 1.17 | 1.17 | 0.5 | 0.89 |
| 71 | T071V | 0.74 | 0.85 | 1.00 | 0.99 | 0.5 | 0.86 |
| 71 | T071W | 0.18 | ***0.05*** | ***0.05*** | ***0.05*** | ***0.05*** | ***0.05*** |
| 71 | T071Y | 0.20 | ***0.05*** | ***0.05*** | 0.15 | ***0.05*** | ***0.05*** |
| 72 | V072A | 0.84 | 0.95 | 1.00 | 0.94 | 0.8 | 0.89 |
| 72 | V072C | 0.71 | 1.13 | 1.02 | 1.10 | 0.8 | 0.88 |
| 72 | V072D | 0.19 | 2.54 | 1.66 | 1.93 | 0.7 | 0.19 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 72 | V072E | 0.50 | 1.14 | 1.08 | 1.13 | 0.8 | 0.62 |
| 72 | V072F | 0.71 | 0.96 | 1.06 | 0.96 | 0.7 | 0.74 |
| 72 | V072G | 0.35 | 1.52 | 1.06 | 1.36 | 0.8 | 0.65 |
| 72 | V072H | 0.35 | 1.05 | 1.01 | 1.17 | 0.8 | 0.57 |
| 72 | V072I | 1.16 | 1.12 | 1.03 | 0.97 | 1.0 | 0.79 |
| 72 | V072K | 0.19 | 2.81 | 2.06 | 2.76 | 1.7 | 0.30 |
| 72 | V072L | 1.00 | 1.16 | 1.07 | 0.97 | 0.9 | 0.67 |
| 72 | V072P | 0.21 | 0.33 | ***0.05*** | 0.08 | ***0.05*** | ***0.05*** |
| 72 | V072Q | 0.27 | 1.63 | 1.54 | 1.67 | 0.6 | 0.64 |
| 72 | V072R | 0.37 | ***0.05*** | ***0.05*** | ***0.05*** | ***0.05*** | ***0.05*** |
| 72 | V072S | 0.36 | 1.36 | 1.41 | 1.34 | 0.8 | 0.91 |
| 72 | V072T | 0.63 | 1.37 | 1.01 | 1.09 | 0.9 | 0.99 |
| 72 | V072W | 0.21 | 0.20 | ***0.05*** | 0.13 | ***0.05*** | ***0.05*** |
| 72 | V072Y | 0.15 | ***0.05*** | ***0.05*** | ***0.05*** | 0.8 | 0.28 |
| 73 | A073C | 0.85 | 1.03 | 1.08 | 1.03 | 0.9 | 0.93 |
| 73 | A073E | 0.47 | 1.62 | 1.22 | 1.13 | 0.2 | 0.78 |
| 73 | A073F | 0.23 | ***0.05*** | ***0.05*** | 0.17 | ***0.05*** | ***0.05*** |
| 73 | A073G | 0.96 | 0.85 | 1.01 | 1.14 | 0.8 | 1.00 |
| 73 | A073H | 0.14 | ***0.05*** | ***0.05*** | ***0.05*** | 0.2 | 0.23 |
| 73 | A073I | 0.17 | 22.54 | 15.70 | 6.07 | 0.1 | 0.30 |
| 73 | A073K | 0.18 | 3.45 | 2.48 | 2.56 | 0.1 | 0.27 |
| 73 | A073L | 0.15 | ***0.05*** | ***0.05*** | ***0.05*** | 0.4 | 0.14 |
| 73 | A073M | 0.24 | 1.71 | 1.42 | 1.48 | 0.3 | 0.43 |
| 73 | A073N | 0.71 | 1.15 | 1.05 | 1.13 | ***0.05*** | 0.76 |
| 73 | A073Q | 0.55 | 1.31 | 1.33 | 1.14 | 0.5 | 0.93 |
| 73 | A073R | 0.14 | ***0.05*** | ***0.05*** | ***0.05*** | ***0.05*** | ***0.05*** |
| 73 | A073S | 1.02 | 1.35 | 0.94 | 1.00 | 0.8 | 1.06 |
| 73 | A073T | 0.71 | 1.27 | 1.06 | 1.15 | 0.6 | 1.11 |
| 73 | A073V | 0.38 | 1.42 | 1.19 | 1.46 | 0.1 | 0.83 |
| 73 | A073W | 0.38 | 0.10 | ***0.05*** | ***0.05*** | ***0.05*** | ***0.05*** |
| 73 | A073Y | 0.21 | ***0.05*** | ***0.05*** | ***0.05*** | ***0.05*** | ***0.05*** |
| 74 | A074C | 0.73 | 0.65 | 1.02 | 0.96 | ***0.05*** | 0.98 |
| 74 | A074D | ***0.05*** | ***0.05*** | ***0.05*** | ***0.05*** | ***0.05*** | ***0.05*** |
| 74 | A074E | 0.25 | 4.31 | ***0.05*** | ***0.05*** | ***0.05*** | ***0.05*** |
| 74 | A074F | 0.31 | 2.60 | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 74 | A074G | 1.04 | 0.89 | 0.85 | 1.06 | 0.2 | 1.00 |
| 74 | A074H | 0.30 | 3.15 | ***0.05*** | ***0.05*** | ***0.05*** | ***0.05*** |
| 74 | A074I | 0.33 | 2.09 | ***0.05*** | ***0.05*** | ***0.05*** | ***0.05*** |
| 74 | A074K | 0.35 | 1.19 | 0.07 | ***0.05*** | ***0.05*** | ***0.05*** |
| 74 | A074L | 0.29 | 2.24 | ***0.05*** | ***0.05*** | ***0.05*** | ***0.05*** |
| 74 | A074M | 0.23 | 5.33 | ***0.05*** | ***0.05*** | ***0.05*** | ***0.05*** |
| 74 | A074N | 0.23 | ***0.05*** | 0.10 | ***0.05*** | ***0.05*** | ***0.05*** |
| 74 | A074P | 0.33 | 1.26 | ***0.05*** | ***0.05*** | ***0.05*** | ***0.05*** |
| 74 | A074Q | 0.26 | 2.88 | 0.06 | ***0.05*** | ***0.05*** | ***0.05*** |
| 74 | A074R | 0.29 | 1.85 | ***0.05*** | ***0.05*** | ***0.05*** | ***0.05*** |
| 74 | A074S | 1.11 | 0.77 | 0.91 | 1.14 | ***0.05*** | 0.95 |
| 74 | A074T | 0.15 | ***0.05*** | ***0.05*** | ***0.05*** | 0.1 | 0.12 |
| 74 | A074V | 0.26 | 3.59 | 0.06 | ***0.05*** | ***0.05*** | ***0.05*** |
| 74 | A074W | 0.29 | 2.54 | ***0.05*** | ***0.05*** | ***0.05*** | ***0.05*** |
| 74 | A074Y | 0.31 | 2.83 | 0.09 | ***0.05*** | ***0.05*** | ***0.05*** |
| 75 | L075A | 1.07 | 1.41 | 0.96 | 1.03 | ***0.05*** | 1.01 |
| 75 | L075C | 1.10 | ***0.05*** | 1.09 | 0.99 | 0.2 | 1.09 |
| 75 | L075D | 0.76 | ***0.05*** | 0.77 | 1.09 | ***0.05*** | 1.06 |
| 75 | L075E | 1.09 | ***0.05*** | 0.87 | 1.10 | ***0.05*** | 1.12 |
| 75 | L075F | 0.55 | ***0.05*** | 1.00 | 0.96 | ***0.05*** | 0.93 |
| 75 | L075G | 0.90 | 0.63 | 0.86 | 1.02 | ***0.05*** | 1.12 |
| 75 | L075H | 0.75 | ***0.05*** | 0.82 | 0.95 | ***0.05*** | 1.04 |
| 75 | L075I | 0.97 | ***0.05*** | 0.70 | 1.08 | 0.1 | 1.08 |
| 75 | L075K | 0.93 | ***0.05*** | 0.61 | 0.95 | 0.1 | 1.12 |
| 75 | L075M | 1.03 | 0.67 | 0.71 | 1.06 | ***0.05*** | 0.97 |
| 75 | L075N | 0.82 | ***0.05*** | 0.61 | 0.91 | ***0.05*** | 0.96 |
| 75 | L075P | 0.38 | ***0.05*** | 0.82 | 1.09 | ***0.05*** | 0.87 |
| 75 | L075Q | 1.18 | ***0.05*** | 0.68 | 1.16 | 0.1 | 1.09 |
| 75 | L075R | 0.95 | ***0.05*** | 0.95 | 1.09 | 0.1 | 1.00 |
| 75 | L075S | 0.88 | 0.70 | 0.57 | 1.11 | ***0.05*** | 1.08 |
| 75 | L075T | 0.96 | ***0.05*** | 0.64 | 0.97 | ***0.05*** | 1.13 |
| 75 | L075V | 1.08 | ***0.05*** | 0.67 | 1.17 | 0.1 | 1.04 |
| 75 | L075W | 0.62 | ***0.05*** | 0.97 | 1.06 | ***0.05*** | 1.18 |
| 75 | L075Y | 0.48 | 1.23 | 0.83 | 0.97 | ***0.05*** | 0.95 |
| 76 | N076A | 1.05 | 1.69 | 0.75 | 0.99 | ***0.05*** | 1.03 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 76 | N076C | 0.86 | ***0.05*** | 0.64 | 0.86 | 0.2 | 1.06 |
| 76 | N076D | 1.26 | ***0.05*** | 0.74 | 0.89 | 1.9 | 1.04 |
| 76 | N076E | ***0.05*** | ***0.05*** | ***0.05*** | ***0.05*** | ***0.05*** | ***0.05*** |
| 76 | N076F | 0.95 | 0.51 | 0.61 | 1.03 | ***0.05*** | 0.94 |
| 76 | N076G | 0.97 | 1.00 | 0.63 | 1.00 | ***0.05*** | 1.21 |
| 76 | N076H | 1.29 | ***0.05*** | 0.70 | 1.03 | 0.3 | 1.01 |
| 76 | N076I | 1.08 | ***0.05*** | 0.80 | 0.96 | ***0.05*** | 0.95 |
| 76 | N076K | 0.78 | ***0.05*** | 0.76 | 1.10 | ***0.05*** | 0.99 |
| 76 | N076L | 0.31 | ***0.05*** | 0.94 | 1.22 | ***0.05*** | 0.85 |
| 76 | N076M | ***0.05*** | ***0.05*** | ***0.05*** | ***0.05*** | ***0.05*** | ***0.05*** |
| 76 | N076P | ***0.05*** | ***0.05*** | ***0.05*** | ***0.05*** | ***0.05*** | ***0.05*** |
| 76 | N076Q | 1.20 | ***0.05*** | 0.92 | 0.98 | 0.1 | 1.06 |
| 76 | N076R | 0.93 | ***0.05*** | 0.55 | 1.03 | ***0.05*** | 0.93 |
| 76 | N076S | 1.18 | 0.86 | 0.94 | 0.94 | ***0.05*** | 1.09 |
| 76 | N076T | 0.74 | ***0.05*** | 0.84 | 0.87 | ***0.05*** | 1.11 |
| 76 | N076V | 0.47 | ***0.05*** | 0.75 | 1.21 | 0.4 | 1.01 |
| 76 | N076W | 0.45 | ***0.05*** | 1.40 | 1.20 | ***0.05*** | 1.15 |
| 76 | N076Y | 0.91 | 0.40 | 0.68 | 0.93 | ***0.05*** | 1.06 |
| 77 | N077A | 0.21 | 1.14 | ***0.05*** | 0.27 | 0.2 | 0.06 |
| 77 | N077C | 0.23 | 0.95 | 0.12 | 0.36 | 0.2 | 0.14 |
| 77 | N077D | 0.44 | 1.02 | 0.96 | 1.05 | ***0.05*** | 1.00 |
| 77 | N077E | 0.20 | 1.17 | 0.13 | 0.35 | 0.2 | 0.07 |
| 77 | N077F | 0.20 | 0.90 | ***0.05*** | 0.20 | ***0.05*** | ***0.05*** |
| 77 | N077G | 0.21 | 1.52 | 0.75 | 0.97 | ***0.05*** | 0.12 |
| 77 | N077H | 0.20 | 1.16 | ***0.05*** | 0.38 | 0.2 | 0.09 |
| 77 | N0771 | 0.23 | 0.63 | 0.09 | 0.28 | 0.2 | 0.08 |
| 77 | N077K | 0.18 | 1.28 | 0.06 | 0.36 | 0.2 | 0.12 |
| 77 | N077L | o. i9 | 2.18 | 0.24 | 0.77 | 0.1 | 0.12 |
| 77 | N077M | 0.21 | 0.94 | 0.11 | 0.32 | 0.3 | 0.06 |
| 77 | N077P | 0.19 | 2.17 | ***0.05*** | 0.63 | 0.3 | 0.06 |
| 77 | N077Q | 0.20 | 2.11 | 0.83 | 1.17 | ***0.05*** | 0.38 |
| 77 | N077R | 0.18 | 2.49 | 0.10 | 0.93 | 0.1 | 0.16 |
| 77 | N077S | 0.26 | 1.67 | 1.31 | 1.20 | ***0.05*** | 0.78 |
| 77 | N077T | 0.18 | 4.17 | 1.33 | 1.47 | ***0.05*** | 0.28 |
| 77 | N077V | 0.13 | ***0.05*** | ***0.05*** | ***0.05*** | 0.4 | 0.06 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 77 | N077W | 0.12 | ***0.05*** | ***0.05*** | ***0.05*** | ***0.05*** | ***0.05*** |
| 77 | N077Y | 0.13 | ***0.05*** | ***0.05*** | ***0.05*** | 0.2 | 0.09 |
| 78 | S078A | 1.21 | 1.08 | 1.10 | 1.03 | 1.1 | 0.91 |
| 78 | S078C | 1.23 | 1.03 | 0.77 | 0.88 | 1.5 | 0.83 |
| 78 | S078D | 1.33 | 1.10 | 0.83 | 0.88 | 1.7 | 0.90 |
| 78 | S078E | 1.04 | 1.05 | 0.87 | 0.96 | 1.1 | 0.91 |
| 78 | S078F | 1.11 | 0.95 | 0.64 | 0.98 | 0.5 | 0.97 |
| 78 | S078G | 1.28 | 1.14 | 0.83 | 0.92 | 0.6 | 0.88 |
| 78 | S078H | ***0.05*** | ***0.05*** | ***0.05*** | ***0.05*** | ***0.05*** | ***0.05*** |
| 78 | S078I | 1.00 | 1.03 | 0.70 | 0.96 | 0.3 | 0.99 |
| 78 | S078K | 1.25 | 0.98 | 0.62 | 0.94 | 0.3 | 0.92 |
| 78 | S078L | 0.68 | 1.01 | 0.78 | 0.92 | 0.8 | 0.96 |
| 78 | S078M | 1.03 | 1.06 | 1.09 | 0.99 | 1.2 | 1.04 |
| 78 | S078N | 1.19 | 1.04 | 0.87 | 1.16 | 1.6 | 1.00 |
| 78 | S078P | 1.25 | 1.00 | 0.93 | 0.94 | ***0.05*** | 0.95 |
| 78 | S078Q | 1.12 | 1.11 | 0.94 | 1.09 | 1.4 | 1.36 |
| 78 | S078R | 1.22 | 1.02 | 0.52 | 0.81 | 0.7 | 1.15 |
| 78 | S078T | 1.07 | 1.17 | 1.02 | 0.99 | 1.4 | 1.09 |
| 78 | S078V | 1.13 | 1.16 | 0.97 | 0.91 | 0.5 | 0.90 |
| 78 | S078W | 1.00 | 1.31 | 0.67 | 1.02 | 0.3 | 1.08 |
| 78 | S078Y | 1.06 | 0.97 | 0.68 | 1.12 | 0.6 | 1.06 |
| 79 | I079A | 1.26 | 0.63 | 1.14 | 0.93 | ***0.05*** | 1.13 |
| 79 | I079C | 1.22 | 0.73 | 1.12 | 1.02 | 0.2 | 0.91 |
| 79 | I079D | ***0.05*** | ***0.05*** | ***0.05*** | ***0.05*** | ***0.05*** | ***0.05*** |
| 79 | I079E | 1.28 | 0.71 | 1.02 | 0.98 | 0.1 | 1.00 |
| 79 | I079F | 1.23 | 0.93 | 1.22 | 1.03 | 0.9 | 0.95 |
| 79 | I079G | 0.90 | 0.83 | 1.18 | 0.90 | ***0.05*** | 0.95 |
| 79 | I079H | 1.21 | 0.60 | 1.11 | 1.10 | ***0.05*** | 1.03 |
| 79 | I079K | 1.14 | 0.25 | 0.87 | 0.88 | ***0.05*** | 0.87 |
| 79 | I079L | 1.20 | 0.84 | 1.14 | 1.08 | ***0.05*** | 0.91 |
| 79 | I079M | ***0.05*** | ***0.05*** | ***0.05*** | ***0.05*** | ***0.05*** | ***0.05*** |
| 79 | I079N | 0.92 | 0.76 | 1.03 | 1.17 | ***0.05*** | 0.89 |
| 79 | I079P | 0.17 | ***0.05*** | 1.05 | 0.47 | ***0.05*** | ***0.05*** |
| 79 | I079Q | 1.11 | 0.39 | 1.22 | 0.89 | ***0.05*** | 1.11 |
| 79 | I079R | 1.15 | 0.58 | 0.92 | 0.95 | ***0.05*** | 0.97 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 79 | I079S | 1.03 | 0.68 | 1.00 | 1.00 | ***0.05*** | 0.91 |
| 79 | I079T | 1.10 | 0.63 | 1.22 | 1.11 | ***0.05*** | 0.98 |
| 79 | I079V | 1.10 | 0.68 | 1.15 | 1.02 | 0.1 | 0.97 |
| 79 | I079W | 1.13 | 0.81 | 1.15 | 1.18 | 0.1 | 1.00 |
| 79 | I079Y | 1.19 | 0.76 | 1.26 | 0.92 | 1.0 | 1.25 |
| 80 | G080A | 0.16 | 0.78 | ***0.05*** | 2.12 | 0.1 | 0.16 |
| 80 | G080C | 0.19 | 0.60 | 1.75 | 1.16 | 0.1 | 0.36 |
| 80 | G080D | 0.18 | ***0.05*** | 0.40 | 0.10 | ***0.05*** | ***0.05*** |
| 80 | G080E | 0.18 | ***0.05*** | 0.39 | 0.08 | ***0.05*** | ***0.05*** |
| 80 | G080F | 0.22 | 0.81 | 1.58 | 1.12 | ***0.05*** | 0.61 |
| 80 | G080H | 0.16 | 11.26 | 32.17 | 3.11 | ***0.05*** | 0.32 |
| 80 | G080I | 0.17 | ***0.05*** | 1.18 | 0.12 | ***0.05*** | ***0.05*** |
| 80 | G080K | 0.16 | ***0.05*** | 0.59 | 1.27 | ***0.05*** | ***0.05*** |
| 80 | G080L | 0.15 | 0.37 | ***0.05*** | 4.69 | 0.1 | 0.11 |
| 80 | G080M | ***0.05*** | ***0.05*** | ***0.05*** | ***0.05*** | ***0.05*** | ***0.05*** |
| 80 | G080N | 0.15 | 0.92 | ***0.05*** | 0.73 | ***0.05*** | ***0.05*** |
| 80 | G080P | 0.23 | ***0.05*** | 0.18 | ***0.05*** | ***0.05*** | ***0.05*** |
| 80 | G080Q | 0.15 | 0.40 | ***0.05*** | ***0.05*** | ***0.05*** | ***0.05*** |
| 80 | G080R | 0.15 | 0.38 | ***0.05*** | ***0.05*** | ***0.05*** | ***0.05*** |
| 80 | G080S | 0.16 | 1.10 | ***0.05*** | 0.17 | ***0.05*** | ***0.05*** |
| 80 | G080T | 0.25 | ***0.05*** | 0.13 | ***0.05*** | ***0.05*** | ***0.05*** |
| 80 | G080V | 0.13 | 0.11 | ***0.05*** | ***0.05*** | ***0.05*** | ***0.05*** |
| 80 | G080W | 0.13 | ***0.05*** | ***0.05*** | ***0.05*** | 0.1 | 0.12 |
| 80 | G080Y | 0.14 | ***0.05*** | ***0.05*** | ***0.05*** | ***0.05*** | 0.49 |
| 81 | V081A | 0.66 | 1.15 | 0.91 | 1.16 | ***0.05*** | 0.80 |
| 81 | V081C | 1.10 | 1.23 | 0.92 | 0.95 | ***0.05*** | 0.88 |
| 81 | V081D | 0.24 | 1.35 | 0.77 | 0.60 | 0.1 | 0.14 |
| 81 | V081E | ***0.05*** | ***0.05*** | ***0.05*** | ***0.05*** | ***0.05*** | ***0.05*** |
| 81 | V081F | 0.91 | 1.32 | 0.89 | 1.08 | ***0.05*** | 0.80 |
| 81 | V081G | 0.63 | 1.17 | 0.82 | 1.09 | ***0.05*** | 0.69 |
| 81 | V081H | 0.72 | 1.45 | 0.96 | 1.04 | ***0.05*** | 0.73 |
| 81 | V081I | 1.15 | 1.09 | 0.82 | 1.05 | 0.3 | 0.88 |
| 81 | V081K | 0.45 | 0.86 | 0.60 | 1.19 | ***0.05*** | 0.82 |
| 81 | V081L | 1.09 | 1.16 | 0.84 | 1.18 | ***0.05*** | 0.97 |
| 81 | V081M | 0.94 | 1.16 | 0.75 | 0.99 | ***0.05*** | 0.89 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 81 | V081N | 0.41 | 1.33 | 0.87 | 1.24 | ***0.05*** | 0.70 |
| 81 | V081P | 0.20 | ***0.05*** | ***0.05*** | 3.63 | 0.1 | 0.15 |
| 81 | V081Q | 0.61 | 1.30 | 0.77 | 1.08 | ***0.05*** | 0.91 |
| 81 | V081R | 0.24 | 2.02 | 1.48 | 1.77 | ***0.05*** | 0.61 |
| 81 | V081S | 0.24 | 0.14 | ***0.05*** | ***0.05*** | ***0.05*** | ***0.05*** |
| 81 | V081T | 0.94 | 1.17 | 0.79 | 1.00 | 0.2 | 0.67 |
| 81 | V081 W | 0.63 | 1.36 | 0.81 | 1.19 | ***0.05*** | 0.76 |
| 81 | V081Y | 0.88 | 1.22 | 0.77 | 1.08 | 0.1 | 0.84 |
| 82 | L082A | 0.63 | 1.22 | 1.02 | 1.09 | ***0.05*** | 0.81 |
| 82 | L082C | 0.85 | 1.17 | 0.99 | 0.99 | ***0.05*** | 0.83 |
| 82 | L082D | 0.25 | 0.06 | ***0.05*** | ***0.05*** | ***0.05*** | ***0.05*** |
| 82 | L082E | 0.45 | 1.27 | 1.13 | 1.03 | ***0.05*** | 0.83 |
| 82 | L082F | 0.56 | 1.12 | 1.13 | 1.15 | ***0.05*** | 0.94 |
| 82 | L082G | 0.21 | 2.20 | 1.71 | 1.50 | 0.1 | 0.36 |
| 82 | L082H | 0.67 | 1.17 | 1.07 | 1.12 | ***0.05*** | 0.85 |
| 82 | L082K | 0.80 | 0.89 | 0.89 | 1.02 | ***0.05*** | 1.06 |
| 82 | L082M | 0.98 | 1.16 | 1.03 | 1.04 | ***0.05*** | 0.98 |
| 82 | L082N | 0.26 | 1.75 | 1.47 | 1.66 | ***0.05*** | 0.60 |
| 82 | L082P | 0.30 | 0.06 | ***0.05*** | ***0.05*** | ***0.05*** | ***0.05*** |
| 82 | L082Q | 0.68 | 1.29 | 1.19 | 1.15 | ***0.05*** | 0.93 |
| 82 | L082R | 0.54 | 0.99 | 0.95 | 1.12 | ***0.05*** | 0.75 |
| 82 | L082S | 0.47 | 1.28 | 1.12 | 1.12 | ***0.05*** | 0.89 |
| 82 | L082T | 0.51 | 1.24 | 1.10 | 1.19 | ***0.05*** | 0.82 |
| 82 | L082V | 0.78 | 1.21 | 1.19 | 1.10 | 0.2 | 1.13 |
| 82 | L082W | 0.17 | 10.29 | 8.50 | 4.18 | 0.7 | 0.37 |
| 82 | L082Y | 0.59 | 1.18 | 1.21 | 1.25 | ***0.05*** | 1.15 |
| 83 | G083A | 0.38 | ***0.05*** | ***0.05*** | ***0.05*** | ***0.05*** | ***0.05*** |
| 83 | G083C | 0.35 | ***0.05*** | ***0.05*** | ***0.05*** | ***0.05*** | ***0.05*** |
| 83 | G083D | ***0.05*** | ***0.05*** | ***0.05*** | ***0.05*** | ***0.05*** | ***0.05*** |
| 83 | G083E | 0.38 | ***0.05*** | ***0.05*** | ***0.05*** | ***0.05*** | ***0.05*** |
| 83 | G083F | 0.35 | ***0.05*** | ***0.05*** | ***0.05*** | ***0.05*** | ***0.05*** |
| 83 | G083H | ***0.05*** | ***0.05*** | ***0.05*** | ***0.05*** | ***0.05*** | ***0.05*** |
| 83 | G083I | 0.39 | ***0.05*** | ***0.05*** | ***0.05*** | ***0.05*** | ***0.05*** |
| 83 | G083K | 0.34 | ***0.05*** | 0.08 | ***0.05*** | ***0.05*** | ***0.05*** |
| 83 | G083L | 0.21 | ***0.05*** | 0.20 | 0.07 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 83 | G083M | 0.37 | ***0.05*** | 0.10 | ***0.05*** | ***0.05*** | ***0.05*** |
| 83 | G083N | ***0.05*** | ***0.05*** | ***0.05*** | ***0.05*** | ***0.05*** | ***0.05*** |
| 83 | G083P | 0.39 | ***0.05*** | 0.07 | ***0.05*** | ***0.05*** | ***0.05*** |
| 83 | G083Q | 0.34 | ***0.05*** | ***0.05*** | ***0.05*** | ***0.05*** | ***0.05*** |
| 83 | G083R | 0.32 | ***0.05*** | ***0.05*** | ***0.05*** | ***0.05*** | ***0.05*** |
| 83 | G083S | 0.26 | 0.96 | 1.61 | 1.24 | ***0.05*** | 0.83 |
| 83 | G083T | 0.29 | ***0.05*** | 0.15 | ***0.05*** | ***0.05*** | ***0.05*** |
| 83 | G083V | 0.30 | ***0.05*** | 0.06 | ***0.05*** | ***0.05*** | ***0.05*** |
| 83 | G083W | 0.29 | ***0.05*** | 0.08 | ***0.05*** | ***0.05*** | ***0.05*** |
| 83 | G083Y | 0.14 | ***0.05*** | ***0.05*** | ***0.05*** | 1.1 | 0.33 |
| 84 | V084A | 1.27 | 0.84 | 0.96 | 0.82 | 0.7 | 1.08 |
| 84 | V084C | 1.15 | 0.77 | 0.75 | 0.79 | 0.8 | 0.97 |
| 84 | V084D | 0.17 | ***0.05*** | 0.88 | ***0.05*** | ***0.05*** | ***0.05*** |
| 84 | V084E | 0.19 | ***0.05*** | ***0.05*** | ***0.05*** | ***0.05*** | ***0.05*** |
| 84 | V084F | 0.17 | ***0.05*** | 0.56 | ***0.05*** | ***0.05*** | ***0.05*** |
| 84 | V084G | 0.29 | 0.75 | 0.84 | 0.86 | 0.5 | 0.66 |
| 84 | V084H | 0.24 | ***0.05*** | ***0.05*** | ***0.05*** | ***0.05*** | ***0.05*** |
| 84 | V084I | 1.08 | 0.96 | 0.99 | 0.68 | 0.5 | 0.99 |
| 84 | V084K | 0.28 | ***0.05*** | 0.07 | 0.07 | ***0.05*** | ***0.05*** |
| 84 | V084L | 0.51 | 0.66 | 0.91 | 1.00 | 0.3 | 1.07 |
| 84 | V084M | 0.89 | 0.91 | 0.68 | 0.87 | 0.3 | 1.20 |
| 84 | V084N | 0.67 | 0.75 | 0.98 | 0.88 | 0.8 | 1.09 |
| 84 | V084P | 0.14 | ***0.05*** | ***0.05*** | ***0.05*** | ***0.05*** | ***0.05*** |
| 84 | V084Q | ***0.05*** | ***0.05*** | ***0.05*** | ***0.05*** | ***0.05*** | ***0.05*** |
| 84 | V084R | 0.33 | ***0.05*** | ***0.05*** | ***0.05*** | ***0.05*** | ***0.05*** |
| 84 | V084S | 0.81 | 0.79 | 0.64 | 0.85 | 0.5 | 1.00 |
| 84 | V084T | 1.06 | 0.95 | 0.42 | 0.70 | 0.7 | 0.92 |
| 84 | V084W | 0.29 | ***0.05*** | ***0.05*** | ***0.05*** | ***0.05*** | ***0.05*** |
| 84 | V084Y | 0.20 | ***0.05*** | 0.25 | ***0.05*** | ***0.05*** | ***0.05*** |
| 85 | A085C | 0.64 | 0.84 | 0.85 | 0.99 | 0.8 | 0.73 |
| 85 | A085D | 0.23 | ***0.05*** | 0.18 | 0.07 | ***0.05*** | ***0.05*** |
| 85 | A085E | 0.24 | 0.07 | ***0.05*** | ***0.05*** | ***0.05*** | ***0.05*** |
| 85 | A085F | 0.36 | ***0.05*** | ***0.05*** | 0.08 | ***0.05*** | ***0.05*** |
| 85 | A085G | 0.77 | 0.93 | 0.92 | 0.97 | 0.9 | 0.94 |
| 85 | A085I | 0.17 | 1.69 | 0.66 | 0.71 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 85 | A085K | 0.29 | ***0.05*** | 0.16 | ***0.05*** | ***0.05*** | ***0.05*** |
| 85 | A085L | 0.21 | 0.10 | ***0.05*** | 0.06 | ***0.05*** | ***0.05*** |
| 85 | A085M | 0.54 | ***0.05*** | ***0.05*** | ***0.05*** | ***0.05*** | ***0.05*** |
| 85 | A085N | 0.25 | 0.08 | 0.06 | ***0.05*** | ***0.05*** | ***0.05*** |
| 85 | A085R | 0.39 | ***0.05*** | ***0.05*** | ***0.05*** | ***0.05*** | ***0.05*** |
| 85 | A085S | 0.90 | 0.94 | 0.94 | 0.97 | 0.9 | 0.93 |
| 85 | A085T | 0.43 | 1.35 | 1.11 | 0.88 | 0.6 | 0.95 |
| 85 | A085V | 0.20 | 2.50 | 2.45 | 2.31 | 0.7 | 0.49 |
| 85 | A085Y | 0.24 | 0.12 | ***0.05*** | ***0.05*** | ***0.05*** | ***0.05*** |
| 86 | P086A | 0.45 | 1.30 | 1.13 | 1.29 | 0.7 | 1.08 |
| 86 | P086D | 0.52 | 1.20 | 0.80 | 1.21 | 0.5 | 0.95 |
| 86 | P086E | 0.46 | 0.88 | 1.20 | 1.02 | 0.4 | 1.10 |
| 86 | P086G | 0.37 | 1.41 | 0.91 | 1.28 | 0.1 | 0.76 |
| 86 | P086K | 0.25 | ***0.05*** | ***0.05*** | ***0.05*** | ***0.05*** | ***0.05*** |
| 86 | P086M | 0.27 | 1.95 | 1.84 | 1.83 | 0.1 | 1.02 |
| 86 | P086N | 0.54 | 1.25 | 1.13 | 1.14 | 0.7 | 1.07 |
| 86 | P086Q | 0.25 | 1.54 | 1.60 | 1.99 | 0.1 | 0.88 |
| 86 | P086R | 0.19 | 3.54 | 3.82 | 2.73 | 0.7 | 0.72 |
| 86 | P086S | 0.53 | 1.15 | 1.15 | 1.06 | 0.7 | 1.21 |
| 86 | P086T | 0.28 | 1.73 | 1.80 | 1.87 | 0.2 | 1.05 |
| 86 | P086V | 0.16 | ***0.05*** | ***0.05*** | 36.35 | 0.1 | 0.19 |
| 86 | P086W | 0.42 | 1.35 | 1.52 | 1.25 | 0.4 | 1.41 |
| 86 | P086Y | 0.39 | 1.46 | 1.19 | 1.70 | 0.9 | 1.38 |
| 87 | S087A | 1.11 | 1.20 | 1.08 | 1.01 | 0.9 | 0.82 |
| 87 | S087C | 1.16 | 1.11 | 0.98 | 0.86 | 1.1 | 0.86 |
| 87 | S087D | 1.12 | 1.29 | 1.05 | 0.95 | 1.5 | 0.89 |
| 87 | S087E | 1.19 | 1.03 | 1.04 | 1.00 | 1.4 | 0.96 |
| 87 | S087F | 0.92 | 1.36 | 0.88 | 1.06 | 0.5 | 0.95 |
| 87 | S087G | 1.16 | 1.16 | 0.91 | 1.03 | 0.8 | 0.85 |
| 87 | S087I | 0.56 | 1.40 | 0.81 | 1.01 | 0.4 | 0.90 |
| 87 | S087K | 1.39 | 0.87 | 0.72 | 1.10 | 0.4 | 0.85 |
| 87 | S087L | 1.07 | 1.31 | 0.95 | 1.07 | 0.8 | 0.98 |
| 87 | S087M | 1.02 | 1.34 | 0.99 | 1.08 | 0.7 | 1.04 |
| 87 | S087N | 1.32 | 1.23 | 1.04 | 0.96 | 1.0 | 0.86 |
| 87 | S087Q | 0.81 | 1.34 | 0.93 | 0.96 | 0.8 | 0.87 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 87 | S087R | 1.23 | 1.06 | 0.73 | 0.99 | 0.2 | 0.82 |
| 87 | S087T | 1.08 | 1.17 | 1.00 | 1.10 | 0.9 | 1.16 |
| 87 | S087V | 0.92 | 1.33 | 0.95 | 1.17 | 0.9 | 1.15 |
| 87 | S087W | 0.91 | 1.44 | 1.01 | 1.26 | 0.5 | 1.06 |
| 88 | A088C | 0.78 | 1.12 | 1.30 | 1.05 | 0.9 | 1.02 |
| 88 | A088D | 0.27 | 1.44 | 1.63 | 1.25 | 0.7 | 0.68 |
| 88 | A088E | 0.17 | 11.44 | 17.45 | 1.68 | 0.6 | 0.16 |
| 88 | A088G | 0.65 | 1.19 | 1.28 | 1.06 | 0.9 | 1.07 |
| 88 | A088K | 0.21 | 2.38 | 2.98 | 1.68 | 0.8 | 0.67 |
| 88 | A088L | 0.62 | 1.20 | 1.17 | 0.93 | 0.8 | 1.06 |
| 88 | A088M | 0.45 | 1.03 | 1.50 | 0.98 | 0.4 | 0.73 |
| 88 | A088N | 0.67 | 0.97 | 1.11 | 1.08 | 0.6 | 1.17 |
| 88 | A088P | 0.21 | 2.19 | 3.40 | 2.03 | 0.9 | 0.62 |
| 88 | A088Q | 0.22 | 1.86 | 1.99 | 1.23 | 0.5 | 0.52 |
| 88 | A088R | 0.37 | ***0.05*** | ***0.05*** | ***0.05*** | ***0.05*** | ***0.05*** |
| 88 | A088S | 0.84 | 0.94 | 1.31 | 0.91 | 0.9 | 1.09 |
| 88 | A088T | 0.86 | 1.28 | 1.29 | 0.94 | 1.0 | 1.08 |
| 88 | A088V | 0.87 | 1.22 | 1.41 | 0.94 | 0.9 | 1.11 |
| 88 | A088W | 0.49 | ***0.05*** | 0.06 | ***0.05*** | ***0.05*** | ***0.05*** |
| 88 | A088Y | 0.18 | 0.66 | 2.48 | 0.40 | ***0.05*** | ***0.05*** |
| 89 | S089A | 0.22 | 0.73 | ***0.05*** | ***0.05*** | ***0.05*** | ***0.05*** |
| 89 | S089C | 0.91 | 1.14 | 1.14 | 0.99 | 0.9 | 0.92 |
| 89 | S089D | 1.20 | 1.35 | 1.04 | 1.09 | 0.8 | 0.96 |
| 89 | S089E | 0.92 | 1.07 | 1.17 | 0.95 | 0.8 | 0.93 |
| 89 | S089F | 0.65 | 1.14 | 1.27 | 0.90 | 0.8 | 1.16 |
| 89 | S089G | 0.88 | 1.13 | 1.10 | 1.13 | 0.8 | 1.04 |
| 89 | S089H | 0.99 | 0.86 | 1.23 | 1.09 | 0.6 | 1.09 |
| 89 | S089I | 0.26 | 0.29 | ***0.05*** | 0.11 | ***0.05*** | ***0.05*** |
| 89 | S089K | 0.98 | 0.99 | 1.06 | 1.01 | 0.8 | 0.88 |
| 89 | S089L | 0.41 | 1.08 | 1.52 | 1.40 | 1.3 | 0.93 |
| 89 | S089M | 0.43 | 1.28 | 1.34 | 1.17 | 1.4 | 0.86 |
| 89 | S089N | ***0.05*** | ***0.05*** | ***0.05*** | ***0.05*** | ***0.05*** | ***0.05*** |
| 89 | S089P | 0.20 | ***0.05*** | ***0.05*** | 0.13 | ***0.05*** | ***0.05*** |
| 89 | S089Q | 0.21 | 108.13 | 37.15 | 0.22 | ***0.05*** | ***0.05*** |
| 89 | S089R | 0.71 | 0.67 | 1.08 | 1.06 | 0.8 | 0.94 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 89 | S089T | ***0.05*** | ***0.05*** | ***0.05*** | ***0.05*** | ***0.05*** | ***0.05*** |
| 89 | S089V | 0.66 | 1.31 | 1.27 | 1.17 | 0.7 | 1.34 |
| 89 | S089W | 0.37 | 1.19 | 1.84 | 1.30 | 1.2 | 1.05 |
| 89 | S089Y | 0.75 | 1.22 | 1.34 | 1.00 | 0.7 | 1.13 |
| 90 | L090A | 0.35 | 1.29 | 1.22 | 1.35 | 0.7 | 0.85 |
| 90 | L090D | 0.20 | 2.40 | 2.05 | 2.19 | 0.6 | 0.47 |
| 90 | L090E | 0.23 | 1.61 | 1.38 | 1.49 | 1.0 | 0.52 |
| 90 | L090F | 0.30 | 1.57 | 0.81 | 1.39 | 0.3 | 0.34 |
| 90 | L090G | 0.28 | 0.21 | 0.15 | 0.22 | 0.9 | 0.11 |
| 90 | L090H | 0.23 | 2.00 | 2.18 | 2.26 | 1.0 | 0.88 |
| 90 | L090M | 0.78 | 1.07 | 1.04 | 0.94 | 1.0 | 0.95 |
| 90 | L090P | 0.27 | 1.56 | 1.73 | 1.61 | 0.9 | 0.88 |
| 90 | L090Q | 0.44 | 1.22 | 1.21 | 1.20 | 1.1 | 1.05 |
| 90 | L090R | 0.35 | ***0.05*** | ***0.05*** | ***0.05*** | ***0.05*** | ***0.05*** |
| 90 | L090S | 0.24 | 1.57 | 1.85 | 1.57 | 0.7 | 0.93 |
| 90 | L090T | 0.21 | 3.87 | 3.31 | 2.49 | 0.8 | 0.96 |
| 90 | L090V | 0.77 | 1.20 | 1.08 | 1.18 | 0.8 | 1.12 |
| 90 | L090W | 0.15 | ***0.05*** | ***0.05*** | ***0.05*** | 0.7 | 0.07 |
| 91 | Y091A | 0.25 | 0.60 | 1.29 | 1.22 | 0.9 | 0.67 |
| 91 | Y091C | 0.23 | 0.99 | 2.01 | 1.14 | 1.1 | 0.54 |
| 91 | Y091D | 0.31 | 0.90 | 1.51 | 1.27 | 1.1 | 0.80 |
| 91 | Y091E | 0.16 | ***0.05*** | 3.30 | 0.67 | 1.3 | 0.10 |
| 91 | Y091F | 1.07 | 0.60 | 1.21 | 1.23 | 1.1 | 1.00 |
| 91 | Y091G | 0.19 | ***0.05*** | 0.32 | 0.11 | 0.9 | 0.06 |
| 91 | Y091H | 0.80 | 0.71 | 1.16 | 1.04 | 1.0 | 0.99 |
| 91 | Y091I | 0.22 | 1.01 | 2.17 | 1.25 | 1.2 | 0.58 |
| 91 | Y091K | 0.22 | ***0.05*** | 0.06 | ***0.05*** | ***0.05*** | ***0.05*** |
| 91 | Y091L | 0.18 | 1.59 | 2.82 | 1.67 | 1.0 | 0.49 |
| 91 | Y091M | 0.27 | 1.09 | 1.56 | 1.13 | 1.1 | 0.62 |
| 91 | Y091N | ***0.05*** | ***0.05*** | ***0.05*** | ***0.05*** | ***0.05*** | ***0.05*** |
| 91 | Y091P | 0.31 | ***0.05*** | 0.09 | 0.48 | ***0.05*** | ***0.05*** |
| 91 | Y091Q | 0.16 | 7.64 | 26.89 | 1.98 | 1.2 | 0.25 |
| 91 | Y091R | 0.16 | ***0.05*** | 11.79 | 0.51 | 1.1 | 0.09 |
| 91 | Y091S | 0.25 | 0.90 | 1.68 | 1.44 | 0.8 | 0.86 |
| 91 | Y091T | 0.17 | 3.68 | 8.06 | 2.67 | 0.9 | 0.61 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 91 | Y091V | 0.23 | 0.89 | 1.92 | 1.46 | 1.0 | 0.70 |
| 91 | Y091W | 0.14 | 0.33 | ***0.05*** | ***0.05*** | ***0.05*** | ***0.05*** |
| 92 | A092C | 0.29 | 2.61 | 2.00 | 1.71 | 0.6 | 0.33 |
| 92 | A092E | 0.50 | ***0.05*** | ***0.05*** | 0.08 | ***0.05*** | ***0.05*** |
| 92 | A092F | 0.36 | ***0.05*** | ***0.05*** | 0.08 | ***0.05*** | ***0.05*** |
| 92 | A092G | 0.55 | 1.16 | 1.46 | 1.30 | 0.8 | 0.67 |
| 92 | A092I | 0.33 | 1.73 | 1.53 | 1.55 | 0.7 | 0.31 |
| 92 | A092K | 0.17 | ***0.05*** | ***0.05*** | 4.27 | 0.1 | 0.20 |
| 92 | A092M | 0.21 | 2.49 | 2.62 | 2.92 | 0.6 | 0.16 |
| 92 | A092N | 0.18 | 6.44 | 7.31 | 5.48 | 0.7 | 0.15 |
| 92 | A092P | 0.26 | 1.62 | 2.34 | 2.13 | 0.9 | 0.47 |
| 92 | A092Q | 0.28 | 0.08 | ***0.05*** | 0.07 | ***0.05*** | ***0.05*** |
| 92 | A092R | 0.27 | 0.41 | 0.06 | 0.27 | ***0.05*** | ***0.05*** |
| 92 | A092S | 1.04 | 0.85 | 0.94 | 1.22 | 1.1 | 0.87 |
| 92 | A092T | 0.73 | 1.10 | 1.14 | 0.98 | 0.9 | 0.96 |
| 92 | A092V | 0.30 | 1.40 | 1.31 | 1.73 | 0.6 | 0.50 |
| 92 | A092W | 0.28 | 0.13 | ***0.05*** | 0.09 | ***0.05*** | ***0.05*** |
| 93 | V093A | 0.31 | 1.09 | 1.32 | 1.22 | 0.9 | 0.68 |
| 93 | V093C | 0.61 | 1.02 | 1.24 | 1.00 | 0.9 | 0.86 |
| 93 | V093D | 0.21 | 2.38 | 3.40 | 2.01 | 1.1 | 0.67 |
| 93 | V093E | 0.27 | 0.22 | 0.25 | 0.12 | ***0.05*** | ***0.05*** |
| 93 | V093F | 0.22 | 1.32 | 2.23 | 1.46 | 1.0 | 0.61 |
| 93 | V093G | 0.23 | 0.60 | 1.00 | 0.61 | 1.1 | 0.18 |
| 93 | V093H | 0.48 | ***0.05*** | ***0.05*** | ***0.05*** | ***0.05*** | ***0.05*** |
| 93 | V093K | 0.63 | ***0.05*** | ***0.05*** | ***0.05*** | ***0.05*** | ***0.05*** |
| 93 | V093L | 0.58 | 1.39 | 1.12 | 1.01 | 0.8 | 0.98 |
| 93 | V093N | 0.32 | 0.09 | 0.20 | 0.11 | ***0.05*** | ***0.05*** |
| 93 | V093P | 0.34 | ***0.05*** | 0.19 | ***0.05*** | ***0.05*** | ***0.05*** |
| 93 | V093Q | 0.42 | 0.06 | ***0.05*** | ***0.05*** | ***0.05*** | ***0.05*** |
| 93 | V093R | 0.48 | ***0.05*** | ***0.05*** | ***0.05*** | ***0.05*** | ***0.05*** |
| 93 | V093S | 0.29 | 0.17 | 0.35 | 0.27 | 0.9 | 0.12 |
| 93 | V093T | 0.33 | 1.46 | 1.70 | 1.37 | 1.1 | 1.04 |
| 94 | K094C | 0.22 | 1.33 | 1.37 | 1.05 | 1.0 | 0.17 |
| 94 | K094D | 0.32 | 0.10 | 0.11 | 0.15 | ***0.05*** | ***0.05*** |
| 94 | K094E | 0.21 | 0.66 | 0.92 | 0.67 | 0.8 | 0.06 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 94 | K094F | 0.29 | 0.18 | 0.10 | 0.14 | ***0.05*** | ***0.05*** |
| 94 | K094G | 0.27 | 0.30 | 14.09 | 0.31 | ***0.05*** | ***0.05*** |
| 94 | K094H | 0.33 | 0.16 | ***0.05*** | 0.10 | ***0.05*** | ***0.05*** |
| 94 | K094I | 0.33 | ***0.05*** | 0.12 | 0.07 | ***0.05*** | ***0.05*** |
| 94 | K094L | 0.30 | 0.26 | 0.32 | 0.28 | 1.1 | 0.06 |
| 94 | K094N | 0.28 | 0.69 | 3.31 | 0.56 | 0.8 | 0.08 |
| 94 | K094Q | 0.42 | 1.19 | 1.22 | 0.90 | 1.0 | 0.72 |
| 94 | K094R | 0.41 | 1.36 | 1.48 | 1.32 | 0.8 | 0.39 |
| 94 | K094S | 0.19 | 4.15 | 3.79 | 2.53 | 1.0 | 0.24 |
| 94 | K094T | 0.14 | ***0.05*** | ***0.05*** | ***0.05*** | 0.8 | 0.19 |
| 94 | K094V | 0.21 | 0.75 | 1.10 | 0.67 | 0.8 | 0.12 |
| 94 | K094W | 0.44 | ***0.05*** | ***0.05*** | ***0.05*** | ***0.05*** | ***0.05*** |
| 94 | K094Y | 0.49 | ***0.05*** | ***0.05*** | ***0.05*** | ***0.05*** | ***0.05*** |
| 95 | V095A | 0.44 | 0.85 | 0.53 | 0.71 | 1.0 | 0.87 |
| 95 | V095C | 1.03 | 0.96 | 0.64 | 0.87 | 1.0 | 1.11 |
| 95 | V095D | 0.25 | 0.08 | ***0.05*** | ***0.05*** | ***0.05*** | ***0.05*** |
| 95 | V095E | 0.31 | 0.32 | 0.08 | ***0.05*** | ***0.05*** | ***0.05*** |
| 95 | V095F | 0.32 | ***0.05*** | ***0.05*** | ***0.05*** | ***0.05*** | ***0.05*** |
| 95 | V095G | 0.43 | 0.97 | 0.34 | 0.83 | 1.0 | 0.86 |
| 95 | V095H | 0.26 | ***0.05*** | ***0.05*** | ***0.05*** | ***0.05*** | ***0.05*** |
| 95 | V095I | 0.43 | 2.43 | 1.17 | 1.31 | ***0.05*** | ***0.05*** |
| 95 | V095K | 0.38 | ***0.05*** | ***0.05*** | ***0.05*** | ***0.05*** | ***0.05*** |
| 95 | V095L | 0.32 | 0.09 | ***0.05*** | ***0.05*** | ***0.05*** | ***0.05*** |
| 95 | V095M | 0.27 | 0.13 | ***0.05*** | ***0.05*** | ***0.05*** | ***0.05*** |
| 95 | V095N | 0.24 | 0.18 | ***0.05*** | 0.06 | ***0.05*** | ***0.05*** |
| 95 | V095P | 0.34 | ***0.05*** | ***0.05*** | ***0.05*** | ***0.05*** | ***0.05*** |
| 95 | V095R | 0.24 | 0.26 | 0.06 | ***0.05*** | ***0.05*** | ***0.05*** |
| 95 | V095S | 0.91 | 1.07 | 0.82 | 0.96 | 1.1 | 1.11 |
| 95 | V095T | 0.90 | 1.23 | 0.95 | 1.04 | 1.1 | 0.98 |
| 95 | V095W | 0.17 | 0.62 | ***0.05*** | 0.08 | ***0.05*** | ***0.05*** |
| 95 | V095Y | 0.25 | 0.19 | ***0.05*** | ***0.05*** | ***0.05*** | ***0.05*** |
| 96 | L096C | 0.57 | 1.04 | 0.44 | 0.78 | ***0.05*** | ***0.05*** |
| 96 | L096D | 0.26 | 0.13 | ***0.05*** | ***0.05*** | ***0.05*** | ***0.05*** |
| 96 | L096E | 0.22 | 0.56 | ***0.05*** | ***0.05*** | ***0.05*** | ***0.05*** |
| 96 | L096F | 0.45 | 2.00 | 0.80 | 1.35 | 0.9 | 0.55 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 96 | L096H | 0.20 | 1.89 | 0.18 | 0.37 | ***0.05*** | ***0.05*** |
| 96 | L096I | 0.76 | 1.64 | 0.85 | 1.02 | 1.2 | 0.35 |
| 96 | L096K | 0.19 | 0.54 | ***0.05*** | 0.10 | ***0.05*** | ***0.05*** |
| 96 | L096M | 0.91 | 1.53 | 1.09 | 1.23 | 0.9 | 0.46 |
| 96 | L096N | 0.17 | 6.10 | 0.90 | 0.69 | ***0.05*** | ***0.05*** |
| 96 | L096P | 0.22 | 0.38 | ***0.05*** | ***0.05*** | ***0.05*** | ***0.05*** |
| 96 | L096Q | 0.17 | 31.69 | 6.84 | 3.85 | ***0.05*** | ***0.05*** |
| 96 | L096R | 0.14 | ***0.05*** | 0.20 | ***0.05*** | ***0.05*** | ***0.05*** |
| 96 | L096S | 0.23 | 3.81 | 1.03 | 1.50 | ***0.05*** | ***0.05*** |
| 96 | L096T | 0.44 | 2.29 | 1.26 | 1.38 | ***0.05*** | ***0.05*** |
| 96 | L096V | 0.95 | 1.42 | 0.85 | 1.31 | 1.1 | 0.08 |
| 96 | L096W | 0.20 | 5.01 | 1.57 | 2.17 | 1.0 | 0.33 |
| 96 | L096Y | 0.30 | 2.01 | 0.73 | 1.18 | ***0.05*** | ***0.05*** |
| 97 | G097A | 0.91 | 1.55 | 0.95 | 1.33 | 1.0 | 0.77 |
| 97 | G097C | 1.01 | 1.66 | 1.07 | 1.08 | 1.0 | 0.49 |
| 97 | G097D | 1.55 | 1.43 | 0.79 | 1.20 | 1.0 | 0.76 |
| 97 | G097E | 1.14 | 1.38 | 0.78 | 1.06 | 1.1 | 0.71 |
| 97 | G097F | 0.29 | 1.93 | 1.01 | 1.36 | 0.9 | 0.29 |
| 97 | G097H | 0.85 | 1.26 | 1.10 | 1.01 | 1.0 | 0.47 |
| 97 | G097K | 1.23 | 1.17 | 0.63 | 1.00 | 1.1 | 0.77 |
| 97 | G097L | 0.77 | 1.43 | 1.11 | 1.24 | 1.1 | 0.70 |
| 97 | G097M | 0.84 | 1.32 | 1.04 | 1.18 | 1.1 | 0.91 |
| 97 | G097P | 0.39 | 2.35 | 1.56 | 1.34 | 1.1 | 0.69 |
| 97 | G097Q | 0.97 | 1.36 | 0.77 | 1.03 | 1.2 | 0.98 |
| 97 | G097R | 1.22 | 0.97 | 0.64 | 1.02 | 1.0 | 0.92 |
| 97 | G097S | 0.98 | 1.45 | 1.12 | 1.07 | 0.9 | 0.95 |
| 97 | G097T | 1.02 | 1.23 | 0.93 | 1.10 | 1.0 | 1.09 |
| 97 | G097V | 0.54 | 1.56 | 1.09 | 1.22 | 1.0 | 0.95 |
| 97 | G097W | 0.23 | 2.95 | 1.54 | 1.49 | 1.0 | 0.34 |
| 97 | G097Y | 0.37 | 1.37 | 0.93 | 1.04 | 0.9 | 0.34 |
| 98 | A098C | 1.10 | 0.86 | 1.07 | 1.06 | 1.2 | 0.94 |
| 98 | A098D | 1.18 | 0.87 | 1.36 | 1.19 | 1.2 | 0.91 |
| 98 | A098E | 1.08 | 0.49 | 1.20 | 1.06 | 0.9 | 1.24 |
| 98 | A098F | 0.73 | 0.54 | 1.00 | 1.02 | 1.0 | 1.16 |
| 98 | A098G | 1.20 | 0.94 | 1.27 | 1.09 | 0.9 | 0.93 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 98 | A098H | 1.26 | 0.70 | 1.08 | 0.97 | 1.0 | 0.99 |
| 98 | A098I | 0.98 | 0.93 | 1.00 | 1.08 | 0.9 | 1.39 |
| 98 | A098K | ***0.05*** | ***0.05*** | ***0.05*** | ***0.05*** | ***0.05*** | ***0.05*** |
| 98 | A098L | 0.96 | 0.63 | 1.09 | 1.01 | 1.0 | 1.05 |
| 98 | A098M | ***0.05*** | ***0.05*** | ***0.05*** | ***0.05*** | ***0.05*** | ***0.05*** |
| 98 | A098N | 0.32 | ***0.05*** | ***0.05*** | ***0.05*** | ***0.05*** | ***0.05*** |
| 98 | A098P | 1.13 | 0.87 | 1.19 | 1.09 | 1.0 | 0.79 |
| 98 | A098Q | 0.89 | 0.76 | 1.13 | 1.05 | 1.0 | 1.09 |
| 98 | A098R | 1.22 | 0.70 | 1.00 | 1.11 | 1.0 | 0.95 |
| 98 | A098S | 1.20 | 0.85 | 1.12 | 1.12 | 0.8 | 1.01 |
| 98 | A098T | 0.98 | 0.74 | 1.09 | 0.99 | 1.0 | 1.36 |
| 98 | A098V | 0.99 | 0.91 | 1.17 | 0.97 | 0.9 | 1.70 |
| 98 | A098W | ***0.05*** | ***0.05*** | ***0.05*** | ***0.05*** | ***0.05*** | ***0.05*** |
| 98 | A098Y | 0.89 | 0.75 | 1.20 | 1.01 | 0.8 | 1.37 |
| 99 | D099A | 0.81 | 1.07 | 0.65 | 1.04 | 0.8 | 0.62 |
| 99 | D099C | 0.71 | 1.49 | 0.78 | 1.08 | 1.1 | 0.62 |
| 99 | D099E | 1.04 | 1.37 | 0.80 | 1.03 | 1.0 | 0.80 |
| 99 | D099F | 0.50 | 1.14 | 0.53 | 1.08 | 1.0 | 0.44 |
| 99 | D099H | 0.79 | 1.17 | 0.53 | 0.98 | 0.9 | 0.65 |
| 99 | D099I | 0.44 | 1.59 | 0.64 | 1.10 | 1.0 | 0.55 |
| 99 | D099K | 0.94 | 0.72 | 0.43 | 1.00 | 1.0 | 0.75 |
| 99 | D099L | 0.34 | 1.20 | 0.47 | 0.93 | 1.1 | 0.57 |
| 99 | D099M | 0.59 | 1.38 | 0.65 | 1.18 | 0.9 | 0.66 |
| 99 | D099N | 1.06 | 1.09 | 0.74 | 0.99 | 1.1 | 0.88 |
| 99 | D099P | 0.39 | 1.98 | 0.89 | 1.21 | 1.0 | 0.40 |
| 99 | D099Q | 0.98 | 1.08 | 0.77 | 1.15 | 1.0 | 0.65 |
| 99 | D099R | 0.87 | 0.54 | 0.42 | 0.86 | 1.1 | 0.83 |
| 99 | D099S | 0.87 | 1.00 | 0.60 | 0.97 | 0.9 | 0.94 |
| 99 | D099T | 0.70 | 1.08 | 0.70 | 1.10 | 0.9 | 0.97 |
| 99 | D099V | 0.43 | 1.71 | 0.75 | 1.08 | 0.9 | 0.61 |
| 99 | D099W | 0.58 | 1.22 | 0.57 | 0.89 | 0.8 | 0.88 |
| 99 | D099Y | 0.50 | 1.43 | 0.60 | 1.07 | 0.9 | 0.60 |
| 100 | G 100A | 0.94 | 0.95 | 1.13 | 0.96 | 1.0 | 0.35 |
| 100 | G 100C | ***0.05*** | ***0.05*** | ***0.05*** | ***0.05*** | ***0.05*** | ***0.05*** |
| 100 | G 100D | 0.77 | 1.41 | 1.35 | 1.08 | 0.9 | 0.34 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 100 | G100E | 0.54 | 1.66 | 1.46 | 1.25 | 1.0 | 0.30 |
| 100 | G100F | 0.53 | 1.04 | 1.32 | 1.22 | 0.9 | 0.10 |
| 100 | G 100H | 0.55 | 1.41 | 1.18 | 1.05 | 0.9 | 0.28 |
| 100 | G100I | 0.28 | 1.80 | 1.64 | 1.21 | ***0.05*** | ***0.05*** |
| 100 | G100K | 0.65 | 1.46 | 1.00 | 1.22 | 0.9 | 0.31 |
| 100 | G 100L | 1.23 | 1.17 | 0.89 | 0.97 | ***0.05*** | ***0.05*** |
| 100 | G 100M | 1.13 | 1.42 | 1.16 | 1.20 | 0.8 | 0.17 |
| 100 | G 100N | 1.09 | 1.25 | 1.29 | 1.31 | 1.0 | 0.41 |
| 100 | G100P | 0.40 | ***0.05*** | 0.10 | ***0.05*** | ***0.05*** | ***0.05*** |
| 100 | G 100Q | 0.66 | 1.57 | 1.18 | 1.38 | 1.0 | 0.35 |
| 100 | G 100R | 0.68 | 1.09 | 0.89 | 1.03 | 0.9 | 0.29 |
| 100 | G100S | 0.79 | 1.28 | 1.35 | 1.15 | 0.9 | 0.35 |
| 100 | G 100T | 0.34 | 2.17 | 1.64 | 1.46 | 1.0 | 0.18 |
| 100 | G100V | 0.31 | 1.98 | 1.55 | 1.39 | 1.0 | 0.07 |
| 100 | G100W | 0.50 | 0.92 | 0.90 | 1.04 | ***0.05*** | ***0.05*** |
| 100 | G 100Y | 0.49 | 1.58 | 1.27 | 1.05 | 1.0 | 0.20 |
| 101 | S101A | 1.27 | 1.38 | 1.00 | 1.09 | 1.1 | 1.08 |
| 101 | S101C | 1.24 | 1.14 | 0.67 | 0.90 | 1.2 | 0.61 |
| 101 | S101E | 1.35 | 1.52 | 1.19 | 1.14 | 1.1 | 0.71 |
| 101 | S101F | 0.96 | 1.02 | 0.73 | 0.97 | 1.0 | 1.13 |
| 101 | S101G | 0.38 | 1.30 | 0.46 | 0.67 | 1.1 | 0.21 |
| 101 | S101I | 1.01 | 1.22 | 0.75 | 0.86 | 1.2 | 0.91 |
| 101 | S101K | 1.28 | 1.15 | 0.62 | 1.00 | 1.1 | 0.99 |
| 101 | S101L | 1.10 | 1.21 | 0.80 | 1.00 | 1.0 | 1.21 |
| 101 | S101M | 1.01 | 1.22 | 0.79 | 1.08 | 1.1 | 1.21 |
| 101 | S101N | 1.24 | 1.46 | 0.99 | 1.10 | 1.1 | 1.03 |
| 101 | S101P | 0.25 | 3.64 | 1.39 | 1.83 | 1.1 | 0.30 |
| 101 | S101Q | 0.59 | 1.56 | 0.79 | 1.05 | 1.0 | 1.24 |
| 101 | S101R | 1.20 | 1.04 | 0.65 | 0.90 | 1.0 | 0.94 |
| 101 | S101T | 0.94 | 1.34 | 0.68 | 1.16 | 1.0 | 1.12 |
| 101 | S101V | 0.92 | 1.39 | 0.74 | 1.18 | 0.9 | 1.19 |
| 101 | S101Y | 0.72 | 1.04 | 0.48 | 0.92 | 1.0 | 1.54 |
| 102 | G102A | 1.12 | 1.46 | 1.31 | 1.19 | 1.0 | 0.31 |
| 102 | G102C | 0.82 | 0.99 | 0.39 | 0.74 | ***0.05*** | ***0.05*** |
| 102 | G102E | 0.25 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 102 | G102F | 0.21 | 0.30 | ***0.05*** | 0.07 | ***0.05*** | ***0.05*** |
| 102 | G102I | 0.23 | 1.13 | 0.16 | 0.09 | ***0.05*** | ***0.05*** |
| 102 | G102K | 0.23 | 0.14 | ***0.05*** | ***0.05*** | ***0.05*** | ***0.05*** |
| 102 | G102L | 0.20 | 0.12 | ***0.05*** | ***0.05*** | ***0.05*** | ***0.05*** |
| 102 | G102M | 0.24 | 0.30 | 0.11 | ***0.05*** | ***0.05*** | ***0.05*** |
| 102 | G102N | 0.22 | 1.24 | 0.09 | 0.08 | ***0.05*** | ***0.05*** |
| 102 | G102R | 0.20 | 0.25 | ***0.05*** | ***0.05*** | ***0.05*** | ***0.05*** |
| 102 | G102S | 0.77 | 1.61 | 1.10 | 1.04 | 1.1 | 0.11 |
| 102 | G102V | 0.21 | 0.69 | ***0.05*** | ***0.05*** | ***0.05*** | ***0.05*** |
| 102 | G102W | 0.21 | 0.25 | ***0.05*** | ***0.05*** | ***0.05*** | ***0.05*** |
| 102 | G102Y | 0.29 | 0.15 | ***0.05*** | ***0.05*** | ***0.05*** | ***0.05*** |
| 103 | Q103A | 1.09 | 0.93 | 0.93 | 0.89 | 1.0 | 0.92 |
| 103 | Q103C | 1.19 | 1.10 | 1.05 | 0.97 | 1.0 | 0.63 |
| 103 | Q103E | 1.21 | 1.38 | 1.15 | 1.05 | 1.0 | 0.64 |
| 103 | Q103F | 1.02 | 0.82 | 0.57 | 0.83 | 0.9 | 0.91 |
| 103 | Q103G | 0.39 | 1.48 | 1.06 | 1.34 | 1.1 | 0.43 |
| 103 | Q103H | 0.89 | 1.35 | 0.89 | 0.93 | 0.9 | 0.65 |
| 103 | Q103I | 1.06 | 0.88 | 0.89 | 0.87 | 1.1 | 0.82 |
| 103 | Q103K | 0.17 | 2.63 | 1.36 | 1.25 | 1.0 | 0.45 |
| 103 | Q103L | 0.44 | 0.87 | 0.68 | 0.81 | 1.1 | 0.93 |
| 103 | Q103M | 0.89 | 1.06 | 0.76 | 0.99 | 0.9 | 1.01 |
| 103 | Q103N | 1.44 | 1.44 | 1.23 | 0.88 | 1.0 | 0.74 |
| 103 | Q103R | 1.09 | 0.47 | 0.57 | 0.88 | 1.1 | 1.04 |
| 103 | Q103S | 1.21 | 1.03 | 0.96 | 0.91 | 1.0 | 1.07 |
| 103 | Q103T | 0.84 | 1.01 | 0.94 | 0.95 | 1.0 | 0.89 |
| 103 | Q103V | 0.94 | 1.13 | 1.03 | 0.97 | 1.0 | 0.75 |
| 103 | Q103W | 0.98 | 0.65 | 0.63 | 0.73 | 1.1 | 0.76 |
| 104 | Y104A | 0.37 | 0.90 | 1.00 | 1.03 | ***0.05*** | ***0.05*** |
| 104 | Y104C | 0.34 | 1.24 | 1.33 | 1.31 | 1.1 | 0.06 |
| 104 | Y104F | 0.90 | 0.88 | 0.78 | 1.03 | 1.0 | 0.67 |
| 104 | Y104G | 0.27 | 0.18 | 0.09 | 0.33 | ***0.05*** | ***0.05*** |
| 104 | Y104H | 0.75 | 0.95 | 1.78 | 0.95 | 1.1 | 0.30 |
| 104 | Y104I | 0.30 | 1.10 | 1.10 | 1.48 | 1.3 | 0.08 |
| 104 | Y104K | 0.43 | ***0.05*** | ***0.05*** | ***0.05*** | ***0.05*** | ***0.05*** |
| 104 | Y104L | 0.24 | 1.45 | 1.48 | 1.17 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 104 | Y104M | 0.31 | 1.53 | 1.50 | 1.27 | 1.2 | 0.08 |
| 104 | Y104N | 0.46 | 1.42 | 1.78 | 1.04 | 1.1 | 0.10 |
| 104 | Y104P | 0.48 | ***0.05*** | ***0.05*** | ***0.05*** | ***0.05*** | ***0.05*** |
| 104 | Y104Q | 0.41 | ***0.05*** | 0.34 | ***0.05*** | ***0.05*** | ***0.05*** |
| 104 | Y104R | 0.34 | 0.11 | ***0.05*** | 0.07 | ***0.05*** | ***0.05*** |
| 104 | Y104S | 0.27 | 1.29 | 1.46 | 1.08 | ***0.05*** | ***0.05*** |
| 104 | Y104T | 0.42 | 1.33 | 1.69 | 1.35 | 2.0 | 0.08 |
| 104 | Y104V | 0.30 | 1.73 | 1.19 | 1.43 | ***0.05*** | ***0.05*** |
| 104 | Y104W | 0.62 | 0.82 | 0.89 | 1.17 | 1.2 | 1.38 |
| 105 | S105A | 0.67 | 1.06 | 0.97 | 1.01 | 1.0 | 1.16 |
| 105 | S105C | 0.32 | 0.91 | 1.10 | 0.81 | 1.1 | 0.52 |
| 105 | S105D | 0.39 | 1.33 | 1.03 | 1.21 | 1.1 | 0.90 |
| 105 | S105E | 0.54 | 0.98 | 0.79 | 0.93 | 1.2 | 0.76 |
| 105 | S105F | 0.23 | ***0.05*** | 0.06 | 0.08 | ***0.05*** | ***0.05*** |
| 105 | S105G | 0.68 | 1.12 | 0.98 | 0.98 | 0.9 | 0.93 |
| 105 | S105H | ***0.05*** | ***0.05*** | ***0.05*** | ***0.05*** | ***0.05*** | ***0.05*** |
| 105 | S105I | 0.19 | 1.60 | 1.06 | 0.72 | 1.0 | 0.26 |
| 105 | S105K | 0.21 | 0.13 | 0.10 | 0.13 | 1.2 | 0.09 |
| 105 | S105L | 0.17 | 0.60 | 9.45 | 0.53 | 1.2 | 0.12 |
| 105 | S105M | 0.18 | 1.09 | 1.67 | 1.11 | 0.9 | 0.19 |
| 105 | S105N | 0.32 | 1.10 | 0.97 | 1.17 | 1.1 | 0.81 |
| 105 | S105P | 0.24 | ***0.05*** | ***0.05*** | ***0.05*** | ***0.05*** | ***0.05*** |
| 105 | S105Q | 0.24 | 0.70 | 0.76 | 0.72 | 1.0 | 0.40 |
| 105 | S105R | 0.16 | 3.02 | 3.37 | 0.95 | 1.2 | 0.19 |
| 105 | S105T | 0.87 | 1.19 | 1.11 | 1.21 | 1.0 | 1.07 |
| 105 | S105V | 0.18 | 2.82 | 3.06 | 2.81 | 1.0 | 0.75 |
| 105 | S105W | 0.14 | ***0.05*** | ***0.05*** | ***0.05*** | 1.0 | 0.22 |
| 105 | S105Y | 0.18 | 0.07 | 0.43 | 0.06 | ***0.05*** | ***0.05*** |
| 106 | W106A | 0.38 | 1.42 | 1.15 | 1.06 | 0.9 | 0.36 |
| 106 | W106C | 0.37 | 1.38 | 1.10 | 0.99 | 0.9 | 0.23 |
| 106 | W106D | ***0.05*** | ***0.05*** | ***0.05*** | ***0.05*** | ***0.05*** | ***0.05*** |
| 106 | W106E | 0.39 | 1.73 | 1.34 | 1.15 | 1.1 | 0.35 |
| 106 | W106F | 0.81 | 1.33 | 0.80 | 1.29 | 1.0 | 0.40 |
| 106 | W106G | 0.24 | 1.40 | 1.28 | 1.21 | 0.9 | 0.12 |
| 106 | W106H | 0.78 | 1.02 | 0.97 | 1.16 | 0.9 | 0.72 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 106 | W106I | 0.30 | 1.51 | 1.13 | 1.34 | 1.1 | 0.40 |
| 106 | W106K | 0.49 | ***0.05*** | ***0.05*** | ***0.05*** | ***0.05*** | ***0.05*** |
| 106 | W106L | 0.31 | 1.56 | 1.11 | 1.40 | 1.1 | 0.26 |
| 106 | W106M | 0.41 | 1.36 | 1.11 | 1.13 | 0.9 | 0.30 |
| 106 | W106N | 0.51 | 1.16 | 1.02 | 1.23 | 1.0 | 0.51 |
| 106 | W106P | 0.36 | ***0.05*** | 0.06 | ***0.05*** | ***0.05*** | ***0.05*** |
| 106 | W106Q | ***0.05*** | ***0.05*** | ***0.05*** | ***0.05*** | ***0.05*** | ***0.05*** |
| 106 | W106R | 0.34 | 0.94 | 1.24 | 1.26 | 1.0 | 0.34 |
| 106 | W106S | 0.37 | 1.41 | 0.93 | 1.19 | 2.0 | 0.18 |
| 106 | W106T | 0.39 | 1.41 | 1.15 | 1.25 | 0.9 | 0.30 |
| 106 | W106V | 0.33 | 1.48 | 1.20 | 1.19 | 0.9 | 0.49 |
| 106 | W106Y | 0.90 | 1.19 | 1.05 | 1.22 | 0.9 | 0.43 |
| 107 | I107E | 0.47 | 1.07 | 1.99 | 1.00 | 1.0 | 0.95 |
| 107 | I107F | 0.54 | 0.87 | 0.79 | 1.16 | 1.0 | 0.06 |
| 107 | I107G | 0.27 | 0.82 | 0.43 | 0.72 | ***0.05*** | ***0.05*** |
| 107 | I107H | 0.52 | 0.11 | 0.15 | 0.07 | ***0.05*** | ***0.05*** |
| 107 | I107K | 0.50 | ***0.05*** | ***0.05*** | ***0.05*** | ***0.05*** | ***0.05*** |
| 107 | I107L | 0.84 | 0.57 | 0.33 | 0.48 | 1.0 | 0.95 |
| 107 | I107M | 0.86 | 1.18 | 2.50 | 0.93 | 1.0 | 0.17 |
| 107 | I107N | 0.40 | 0.72 | 0.61 | 0.49 | ***0.05*** | ***0.05*** |
| 107 | I107Q | 0.50 | 0.76 | 0.68 | 0.67 | ***0.05*** | ***0.05*** |
| 107 | I107R | 0.23 | 2.33 | 2.34 | 1.86 | 1.2 | 0.06 |
| 107 | I107S | 0.46 | 1.41 | 0.88 | 1.22 | 1.0 | 0.08 |
| 107 | I107T | 0.70 | 1.35 | 2.33 | 0.93 | 1.0 | 0.13 |
| 107 | I107V | 0.82 | 0.71 | 0.73 | 0.78 | 0.9 | 1.33 |
| 107 | I107W | 0.61 | 1.28 | 1.47 | 1.05 | ***0.05*** | ***0.05*** |
| 107 | I107Y | 0.39 | 0.27 | 0.47 | 0.37 | ***0.05*** | ***0.05*** |
| 108 | I108A | 0.38 | 1.15 | 1.22 | 1.17 | 0.8 | 0.70 |
| 108 | I108C | 0.79 | 1.09 | 1.19 | 1.00 | 0.9 | 0.76 |
| 108 | I108D | 0.32 | ***0.05*** | 0.06 | ***0.05*** | ***0.05*** | ***0.05*** |
| 108 | I108E | 0.21 | 0.23 | 1.19 | 0.59 | 0.9 | 0.13 |
| 108 | I108F | 0.26 | 0.18 | 0.23 | 0.09 | ***0.05*** | ***0.05*** |
| 108 | I108G | 0.21 | ***0.05*** | 0.36 | 0.11 | ***0.05*** | ***0.05*** |
| 108 | I108H | 0.29 | ***0.05*** | 0.07 | ***0.05*** | ***0.05*** | ***0.05*** |
| 108 | I108K | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 108 | 1108L | 0.73 | 1.29 | 1.29 | 1.05 | 1.0 | 0.41 |
| 108 | I108M | 0.80 | 1.10 | 1.21 | 1.10 | 0.7 | 0.73 |
| 108 | I108N | 0.20 | 0.34 | 0.24 | 0.14 | ***0.05*** | ***0.05*** |
| 108 | I108P | 0.22 | ***0.05*** | 0.29 | ***0.05*** | ***0.05*** | ***0.05*** |
| 108 | I108Q | 0.20 | 0.33 | 0.24 | 0.07 | 0.7 | 0.06 |
| 108 | I108R | 0.29 | ***0.05*** | ***0.05*** | 0.06 | ***0.05*** | ***0.05*** |
| 108 | I108S | 0.16 | 2.38 | 8.60 | 1.30 | 0.9 | 0.13 |
| 108 | I108T | 0.30 | 1.75 | 1.66 | 1.51 | 0.9 | 0.77 |
| 108 | I108V | 1.11 | 1.29 | 1.09 | 1.16 | 1.0 | 0.93 |
| 108 | I108W | 0.28 | ***0.05*** | 0.07 | ***0.05*** | ***0.05*** | ***0.05*** |
| 108 | I108Y | 0.22 | 0.32 | 0.15 | ***0.05*** | ***0.05*** | ***0.05*** |
| 109 | N109A | 0.62 | 1.05 | 1.01 | 1.09 | 1.1 | 1.03 |
| 109 | N109C | 0.90 | 0.82 | 0.73 | 0.89 | 1.1 | 0.85 |
| 109 | N109D | ***0.05*** | ***0.05*** | ***0.05*** | ***0.05*** | ***0.05*** | ***0.05*** |
| 109 | N109E | 0.66 | 0.94 | 1.01 | 1.05 | 1.1 | 1.09 |
| 109 | N109F | 0.52 | 1.17 | 0.79 | 1.20 | 1.1 | 0.97 |
| 109 | N109G | 0.66 | 1.13 | 0.93 | 1.01 | 1.1 | 1.12 |
| 109 | N109H | 0.93 | 1.01 | 0.64 | 1.04 | 1.1 | 0.92 |
| 109 | N109I | ***0.05*** | ***0.05*** | ***0.05*** | ***0.05*** | ***0.05*** | ***0.05*** |
| 109 | N109K | ***0.05*** | ***0.05*** | ***0.05*** | ***0.05*** | ***0.05*** | ***0.05*** |
| 109 | N109L | 0.81 | 0.80 | 0.99 | 1.18 | 1.1 | 1.17 |
| 109 | N109M | 0.20 | ***0.05*** | 0.36 | 0.24 | 1.0 | 0.07 |
| 109 | N109P | 0.59 | 1.13 | 0.94 | 1.20 | 1.0 | 0.79 |
| 109 | N109Q | 1.04 | 0.63 | 0.55 | 0.99 | 1.2 | 0.93 |
| 109 | N109R | 0.71 | 0.58 | 0.57 | 1.03 | 1.0 | 1.07 |
| 109 | N109S | 1.03 | 0.93 | 0.90 | 1.13 | 1.0 | 1.05 |
| 109 | N109T | 0.94 | 1.26 | 0.82 | 1.02 | 0.9 | 1.09 |
| 109 | N109V | 0.59 | 0.91 | 0.91 | 1.17 | 1.0 | 1.08 |
| 109 | N109W | 0.63 | 0.92 | 0.76 | 1.15 | 0.9 | 0.92 |
| 109 | N109Y | 0.54 | 0.88 | 0.89 | 1.08 | 0.9 | 0.96 |
| 110 | G110A | 0.79 | 1.28 | 0.75 | 1.05 | 0.9 | 0.78 |
| 110 | G110C | 0.41 | ***0.05*** | ***0.05*** | ***0.05*** | ***0.05*** | ***0.05*** |
| 110 | G110D | 0.53 | ***0.05*** | ***0.05*** | ***0.05*** | ***0.05*** | ***0.05*** |
| 110 | G110E | 0.53 | ***0.05*** | 2.02 | ***0.05*** | ***0.05*** | ***0.05*** |
| 110 | G110F | 0.53 | ***0.05*** | 1.55 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 110 | G110H | 0.45 | ***0.05*** | 0.06 | ***0.05*** | ***0.05*** | ***0.05*** |
| 110 | G110I | 0.47 | ***0.05*** | ***0.05*** | ***0.05*** | ***0.05*** | ***0.05*** |
| 110 | G110K | 0.48 | ***0.05*** | ***0.05*** | ***0.05*** | ***0.05*** | ***0.05*** |
| 110 | G110L | 0.48 | ***0.05*** | 0.40 | ***0.05*** | ***0.05*** | ***0.05*** |
| 110 | G110M | 0.49 | 0.06 | 0.53 | ***0.05*** | ***0.05*** | ***0.05*** |
| 110 | G110N | 0.51 | ***0.05*** | ***0.05*** | ***0.05*** | ***0.05*** | ***0.05*** |
| 110 | G110P | 0.48 | ***0.05*** | 0.08 | ***0.05*** | ***0.05*** | ***0.05*** |
| 110 | G110R | 0.50 | ***0.05*** | 0.94 | ***0.05*** | ***0.05*** | ***0.05*** |
| 110 | G110S | 0.25 | 2.17 | 1.37 | 1.69 | 0.9 | 0.51 |
| 110 | G110T | 0.20 | 4.57 | 3.95 | 2.82 | 0.9 | 0.53 |
| 110 | G110Y | 0.44 | ***0.05*** | ***0.05*** | ***0.05*** | ***0.05*** | ***0.05*** |
| 111 | I111A | 0.24 | 3.80 | 2.39 | 1.92 | 0.9 | 0.30 |
| 111 | I111C | 0.36 | 1.88 | 1.45 | 1.48 | 0.9 | 0.88 |
| 111 | I111D | 0.24 | 0.38 | 0.07 | ***0.05*** | ***0.05*** | ***0.05*** |
| 111 | I111E | 0.22 | 0.52 | 0.21 | 0.06 | ***0.05*** | ***0.05*** |
| 111 | I111F | 0.46 | 1.68 | 1.11 | 1.24 | ***0.05*** | ***0.05*** |
| 111 | I111G | 0.29 | 1.19 | ***0.05*** | ***0.05*** | ***0.05*** | ***0.05*** |
| 111 | I111H | 0.25 | 0.36 | ***0.05*** | 0.06 | ***0.05*** | ***0.05*** |
| 111 | I111K | 0.22 | 0.60 | 0.29 | 0.10 | ***0.05*** | ***0.05*** |
| 111 | I111L | 1.00 | 1.30 | 0.96 | 1.10 | 1.0 | 0.74 |
| 111 | I111M | 0.80 | 1.37 | 0.98 | 1.04 | 1.1 | 0.74 |
| 111 | I111N | 0.21 | ***0.05*** | ***0.05*** | 0.18 | ***0.05*** | ***0.05*** |
| 111 | I111P | 0.25 | 0.24 | 0.18 | 0.07 | ***0.05*** | ***0.05*** |
| 111 | I111Q | 0.19 | ***0.05*** | ***0.05*** | ***0.05*** | ***0.05*** | ***0.05*** |
| 111 | I111R | 0.25 | 0.17 | 0.12 | 0.12 | ***0.05*** | ***0.05*** |
| 111 | I111S | 0.18 | ***0.05*** | ***0.05*** | ***0.05*** | ***0.05*** | ***0.05*** |
| 111 | I111T | 0.31 | 3.10 | 1.56 | 1.67 | 1.0 | 0.85 |
| 111 | I111V | 0.73 | 1.59 | 0.98 | 1.32 | 1.0 | 0.83 |
| 111 | I111W | 0.21 | ***0.05*** | ***0.05*** | 0.13 | ***0.05*** | ***0.05*** |
| 111 | I111Y | 0.20 | ***0.05*** | ***0.05*** | 1.39 | ***0.05*** | ***0.05*** |
| 112 | E112A | 0.33 | 1.05 | 0.84 | 1.31 | 1.1 | 0.77 |
| 112 | E112C | 0.49 | 0.40 | 0.28 | 0.28 | 1.1 | 0.20 |
| 112 | E112D | 0.97 | 1.20 | 0.97 | 1.11 | 1.0 | 0.91 |
| 112 | E112F | 0.17 | ***0.05*** | ***0.05*** | ***0.05*** | 1.0 | 0.24 |
| 112 | E112G | 0.37 | 0.87 | 0.64 | 0.90 | 1.0 | 0.56 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 112 | E112H | ***0.05*** | ***0.05*** | ***0.05*** | ***0.05*** | ***0.05*** | ***0.05*** |
| 112 | E112I | 0.22 | 7.24 | 2.74 | 2.71 | 1.1 | 0.63 |
| 112 | E112K | ***0.05*** | ***0.05*** | ***0.05*** | ***0.05*** | ***0.05*** | ***0.05*** |
| 112 | E112L | 0.21 | 19.65 | 11.59 | 3.83 | 1.1 | 0.57 |
| 112 | E112M | 0.35 | 0.98 | 0.74 | 1.07 | 1.0 | 0.71 |
| 112 | E112N | 0.38 | 1.24 | 0.85 | 1.19 | 1.0 | 0.93 |
| 112 | E112P | 0.27 | 0.26 | ***0.05*** | ***0.05*** | ***0.05*** | ***0.05*** |
| 112 | E112Q | 0.39 | 1.25 | 1.01 | 1.27 | 1.0 | 0.97 |
| 112 | E112R | 0.18 | ***0.05*** | ***0.05*** | ***0.05*** | 1.1 | 0.28 |
| 112 | E112S | 0.38 | 1.22 | 0.98 | 1.05 | 1.0 | 0.85 |
| 112 | E112T | 0.25 | 2.89 | 2.14 | 1.98 | 1.0 | 0.90 |
| 112 | E112V | 0.21 | ***0.05*** | ***0.05*** | 2.81 | 1.1 | 0.35 |
| 112 | E112W | 0.15 | ***0.05*** | ***0.05*** | ***0.05*** | 1.0 | 0.47 |
| 112 | E112Y | 0.19 | ***0.05*** | ***0.05*** | ***0.05*** | 1.0 | 0.68 |
| 113 | W113A | 0.23 | ***0.05*** | 0.24 | 0.17 | 1.0 | 0.08 |
| 113 | W113C | 0.19 | 0.36 | 1.25 | 1.25 | 1.0 | 0.19 |
| 113 | W113D | 0.18 | 0.52 | 1.67 | 0.60 | 0.9 | 0.16 |
| 113 | W113E | 0.19 | 0.26 | 1.58 | 0.31 | 0.9 | 0.16 |
| 113 | W113F | 0.50 | 0.97 | 1.02 | 1.03 | 0.9 | 0.96 |
| 113 | W113G | 0.31 | ***0.05*** | 0.07 | ***0.05*** | ***0.05*** | ***0.05*** |
| 113 | W113H | 0.22 | 1.45 | 1.69 | 1.49 | 1.1 | 0.57 |
| 113 | W113I | ***0.05*** | ***0.05*** | ***0.05*** | ***0.05*** | ***0.05*** | ***0.05*** |
| 113 | W113K | 0.22 | ***0.05*** | ***0.05*** | ***0.05*** | ***0.05*** | ***0.05*** |
| 113 | W113L | 0.19 | ***0.05*** | 0.35 | 0.17 | 1.1 | 0.08 |
| 113 | W113M | 0.19 | 0.40 | 1.19 | 0.61 | 0.9 | 0.18 |
| 113 | W113N | 0.17 | 0.74 | 2.31 | 0.88 | 0.9 | 0.22 |
| 113 | W113P | 0.32 | ***0.05*** | ***0.05*** | ***0.05*** | ***0.05*** | ***0.05*** |
| 113 | W113Q | 0.19 | ***0.05*** | 0.38 | 0.07 | ***0.05*** | ***0.05*** |
| 113 | W113R | 0.22 | ***0.05*** | 0.08 | ***0.05*** | ***0.05*** | ***0.05*** |
| 113 | W113S | 0.19 | ***0.05*** | 0.30 | 0.24 | 1.0 | 0.09 |
| 113 | W113T | 0.17 | 0.12 | 1.00 | 0.51 | 1.1 | 0.10 |
| 113 | W113V | 0.15 | 0.34 | ***0.05*** | ***0.05*** | 1.0 | 0.14 |
| 113 | W113Y | 0.91 | 0.82 | 1.25 | 1.02 | 1.1 | 0.89 |
| 114 | A114C | 0.87 | 1.18 | 1.38 | 1.17 | 1.0 | 1.19 |
| 114 | A114D | 0.39 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 114 | A114E | 0.48 | ***0.05*** | ***0.05*** | ***0.05*** | ***0.05*** | ***0.05*** |
| 114 | A114F | 0.63 | ***0.05*** | ***0.05*** | ***0.05*** | ***0.05*** | ***0.05*** |
| 114 | A114G | 0.72 | 1.15 | 1.41 | 1.31 | 1.1 | 1.14 |
| 114 | A114H | 0.26 | 0.16 | ***0.05*** | 0.15 | ***0.05*** | ***0.05*** |
| 114 | A114I | 0.19 | 3.35 | 2.63 | 2.54 | 0.9 | 0.42 |
| 114 | A114K | 0.51 | ***0.05*** | ***0.05*** | ***0.05*** | ***0.05*** | ***0.05*** |
| 114 | A114L | 0.51 | ***0.05*** | ***0.05*** | ***0.05*** | ***0.05*** | ***0.05*** |
| 114 | A114N | 0.33 | ***0.05*** | ***0.05*** | ***0.05*** | ***0.05*** | ***0.05*** |
| 114 | A114P | 0.28 | 0.07 | ***0.05*** | 0.06 | ***0.05*** | ***0.05*** |
| 114 | A114Q | 0.47 | ***0.05*** | ***0.05*** | ***0.05*** | ***0.05*** | ***0.05*** |
| 114 | A114R | 0.45 | 0.09 | ***0.05*** | ***0.05*** | ***0.05*** | ***0.05*** |
| 114 | A114S | 0.33 | 1.15 | 1.39 | 1.63 | 1.0 | 0.98 |
| 114 | A114T | 0.59 | 1.03 | 1.49 | 1.53 | 0.9 | 1.46 |
| 114 | A114V | 0.31 | 1.57 | 2.18 | 1.56 | 0.9 | 0.97 |
| 114 | A114W | 0.40 | ***0.05*** | ***0.05*** | ***0.05*** | ***0.05*** | ***0.05*** |
| 114 | A114Y | 0.51 | ***0.05*** | ***0.05*** | ***0.05*** | ***0.05*** | ***0.05*** |
| 115 | I115A | 0.40 | 1.58 | 0.99 | 1.09 | 0.9 | 0.79 |
| 115 | I115C | 0.69 | 1.23 | 0.81 | 1.25 | 1.0 | 1.10 |
| 115 | I115D | 0.20 | ***0.05*** | ***0.05*** | 9.72 | 1.1 | 0.28 |
| 115 | I115E | 0.28 | 2.29 | 1.67 | 1.56 | 0.9 | 0.69 |
| 115 | I115F | 0.30 | 1.99 | 1.22 | 1.37 | 0.9 | 0.61 |
| 115 | I115G | 0.17 | ***0.05*** | ***0.05*** | ***0.05*** | 1.1 | 0.21 |
| 115 | I115H | 0.24 | 4.85 | 2.46 | 2.39 | 0.9 | 0.52 |
| 115 | I115K | 0.18 | ***0.05*** | ***0.05*** | ***0.05*** | 0.1 | 0.25 |
| 115 | I115L | 0.83 | 1.04 | 0.96 | 1.08 | 1.1 | 1.06 |
| 115 | I115M | 0.89 | 1.46 | 1.03 | 1.09 | 1.0 | 0.94 |
| 115 | I115N | 0.21 | 8.31 | 8.40 | 2.63 | 1.0 | 0.37 |
| 115 | I115P | 0.21 | 1.81 | 0.08 | 0.17 | ***0.05*** | ***0.05*** |
| 115 | I115Q | 0.58 | 1.30 | 0.80 | 1.24 | 1.0 | 1.00 |
| 115 | I115R | 0.38 | 1.37 | 1.00 | 1.21 | 1.0 | 0.86 |
| 115 | I115S | 0.24 | 4.20 | 2.44 | 2.22 | 0.9 | 0.70 |
| 115 | I115T | 0.59 | 1.46 | 0.89 | 1.08 | 0.9 | 0.91 |
| 115 | 1115V | 0.84 | 1.44 | 0.99 | 1.25 | 0.9 | 1.58 |
| 115 | I115W | 0.16 | ***0.05*** | ***0.05*** | ***0.05*** | 0.9 | 0.34 |
| 115 | I115Y | 0.24 | 4.81 | 4.07 | 2.48 | 0.9 | 0.79 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 116 | A116C | 1.15 | 0.85 | 1.06 | 0.90 | 1.0 | 0.95 |
| 116 | A116D | 1.24 | 1.02 | 0.81 | 1.03 | 1.0 | 1.11 |
| 116 | A116E | 1.16 | 1.21 | 1.09 | 0.89 | 1.0 | 1.08 |
| 116 | A116F | 0.95 | 1.06 | 0.92 | 0.94 | 1.0 | 1.01 |
| 116 | A116G | 0.66 | 0.94 | 1.01 | 0.97 | 1.0 | 1.08 |
| 116 | A116H | 1.07 | 1.09 | 0.92 | 1.13 | 1.0 | 1.30 |
| 116 | A116I | 0.95 | 0.82 | 1.04 | 1.13 | 1.0 | 1.05 |
| 116 | A116K | 1.18 | 0.74 | 0.79 | 1.13 | 1.0 | 0.94 |
| 116 | A116L | 0.82 | 1.05 | 0.89 | 0.83 | 1.0 | 0.98 |
| 116 | A116M | 1.11 | 0.86 | 1.14 | 0.85 | 1.0 | 0.98 |
| 116 | A116N | 0.99 | 0.97 | 0.88 | 1.10 | 1.0 | 1.15 |
| 116 | A116P | 0.14 | ***0.05*** | ***0.05*** | ***0.05*** | 1.0 | 0.34 |
| 116 | A116Q | 1.07 | 0.85 | 0.71 | 1.03 | 1.0 | 1.15 |
| 116 | A116R | 1.15 | 0.86 | 0.73 | 1.09 | 1.0 | 1.15 |
| 116 | A116S | 1.14 | 1.07 | 0.98 | 1.17 | 0.9 | 1.11 |
| 116 | A116T | 1.03 | 1.01 | 1.12 | 1.06 | 0.9 | 1.17 |
| 116 | A116V | 0.88 | 1.03 | 1.03 | 0.96 | 1.0 | 1.09 |
| 116 | A116W | 0.86 | 1.24 | 0.71 | 0.97 | 1.0 | 1.17 |
| 116 | A116Y | 0.88 | 0.89 | 1.03 | 0.93 | 0.9 | 1.19 |
| 117 | N117A | ***0.05*** | ***0.05*** | ***0.05*** | ***0.05*** | ***0.05*** | ***0.05*** |
| 117 | N117C | 0.60 | 1.00 | 0.98 | 1.07 | 1.0 | 1.06 |
| 117 | N117D | ***0.05*** | ***0.05*** | ***0.05*** | ***0.05*** | ***0.05*** | ***0.05*** |
| 117 | N117E | 0.52 | 0.97 | 1.03 | 1.06 | 1.0 | 1.18 |
| 117 | N117F | 0.40 | 1.04 | 1.21 | 1.17 | 0.9 | 1.33 |
| 117 | N117G | 0.67 | 1.18 | 0.85 | 1.29 | 1.1 | 0.96 |
| 117 | N117H | ***0.05*** | ***0.05*** | ***0.05*** | ***0.05*** | ***0.05*** | ***0.05*** |
| 117 | N117I | 0.15 | ***0.05*** | ***0.05*** | 5.36 | 1.0 | 0.27 |
| 117 | N117K | 0.22 | 1.91 | 1.91 | 1.80 | 1.0 | 0.88 |
| 117 | N117L | 0.30 | 1.13 | 1.22 | 1.39 | 1.0 | 1.13 |
| 117 | N117M | 0.57 | 1.09 | 1.21 | 1.12 | 1.0 | 1.24 |
| 117 | N117P | 0.32 | ***0.05*** | ***0.05*** | ***0.05*** | ***0.05*** | ***0.05*** |
| 117 | N117Q | 1.04 | 0.69 | 0.95 | 0.99 | 1.1 | 1.10 |
| 117 | N117R | 0.80 | 0.98 | 0.78 | 0.98 | 1.0 | 0.90 |
| 117 | N117S | 0.53 | 1.18 | 1.24 | 1.06 | 1.3 | 1.14 |
| 117 | N117T | 0.80 | 1.05 | 1.07 | 1.15 | 1.0 | 1.13 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 117 | N117V | 0.17 | 5.36 | 4.11 | 2.10 | 1.0 | 0.46 |
| 117 | N117W | 0.25 | 2.09 | 1.77 | 1.63 | 1.0 | 1.05 |
| 117 | N117Y | 0.45 | 0.99 | 0.89 | 1.22 | 1.0 | 0.98 |
| 118 | N118A | 0.71 | 0.58 | 0.84 | 0.95 | 1.0 | 1.14 |
| 118 | N118C | 0.74 | 0.61 | 1.01 | 0.63 | 1.1 | 0.94 |
| 118 | N118D | 1.12 | 0.67 | 1.05 | 0.86 | 1.2 | 1.01 |
| 118 | N118E | 0.88 | 0.71 | 0.70 | 0.69 | 1.1 | 1.16 |
| 118 | N118F | 0.34 | 1.02 | 1.31 | 0.93 | 1.0 | 1.07 |
| 118 | N118G | 1.07 | 0.83 | 0.50 | 0.79 | 0.9 | 0.92 |
| 118 | N118H | 1.18 | 0.75 | 0.88 | 0.92 | 1.0 | 1.07 |
| 118 | N118I | 0.17 | 4.85 | 5.90 | 1.96 | 1.1 | 0.62 |
| 118 | N118K | 1.17 | 0.80 | 0.81 | 0.81 | 1.1 | 0.95 |
| 118 | N118L | 0.23 | 1.45 | 1.85 | 1.32 | 1.0 | 1.13 |
| 118 | N118M | 0.49 | 0.87 | 0.99 | 1.06 | 1.0 | 1.17 |
| 118 | N118P | 0.18 | ***0.05*** | 0.13 | 0.06 | ***0.05*** | ***0.05*** |
| 118 | N118Q | 1.03 | 0.62 | 1.12 | 0.90 | 1.1 | 1.09 |
| 118 | N118R | 1.35 | 0.82 | 0.62 | 0.80 | 1.0 | 1.01 |
| 118 | N118S | 0.89 | 0.77 | 0.81 | 0.76 | 0.9 | 1.07 |
| 118 | N118T | 0.64 | 0.72 | 0.86 | 1.08 | 0.9 | 1.21 |
| 118 | N118V | 0.23 | 1.35 | 1.92 | 1.53 | 1.0 | 0.99 |
| 118 | N118W | 0.27 | 1.16 | 1.36 | 1.41 | 1.1 | 1.03 |
| 118 | N118Y | 0.84 | 0.98 | 0.72 | 0.70 | 1.0 | 1.13 |
| 119 | M119A | 0.75 | 1.01 | 1.16 | 1.01 | 0.8 | 0.85 |
| 119 | M119C | 0.86 | 1.01 | 1.16 | 0.95 | 1.0 | 0.99 |
| 119 | M119D | 0.25 | 0.21 | 0.49 | 0.19 | ***0.05*** | ***0.05*** |
| 119 | M119E | 0.27 | ***0.05*** | 0.11 | 0.08 | ***0.05*** | ***0.05*** |
| 119 | M119F | 0.29 | 1.64 | 1.87 | 1.60 | 1.3 | 0.78 |
| 119 | M119G | 0.26 | 0.33 | 0.48 | 0.28 | 1.0 | 0.11 |
| 119 | M119H | 0.30 | 1.19 | 1.46 | 1.22 | 1.1 | 0.67 |
| 119 | M119I | 0.74 | 1.20 | 1.16 | 0.94 | 1.1 | 0.97 |
| 119 | M119K | 0.33 | 0.07 | ***0.05*** | 0.07 | ***0.05*** | ***0.05*** |
| 119 | M119L | 0.65 | 1.17 | 1.19 | 0.82 | 1.0 | 0.98 |
| 119 | M119N | 0.23 | 1.95 | 2.59 | 1.79 | 1.1 | 0.83 |
| 119 | M119P | 0.31 | 0.13 | 0.18 | 0.09 | ***0.05*** | ***0.05*** |
| 119 | M119R | 0.43 | ***0.05*** | 0.07 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 119 | M119S | 0.53 | 1.19 | 1.30 | 0.87 | 0.8 | 1.04 |
| 119 | M119T | 0.46 | 1.25 | 1.32 | 1.03 | 1.0 | 1.43 |
| 119 | M119V | 0.75 | 0.95 | 1.13 | 0.91 | 0.7 | 1.14 |
| 119 | M119W | 0.22 | 0.16 | 0.13 | 0.11 | ***0.05*** | ***0.05*** |
| 119 | M119Y | 0.15 | ***0.05*** | ***0.05*** | ***0.05*** | 1.0 | 0.28 |
| 120 | D120A | 1.01 | 1.11 | 0.84 | 0.80 | 1.0 | 0.85 |
| 120 | D120C | 0.67 | 0.84 | 0.95 | 0.76 | 1.0 | 0.92 |
| 120 | D120E | 1.04 | 0.60 | 0.87 | 1.04 | 1.1 | 0.99 |
| 120 | D120F | 0.24 | 0.50 | 1.07 | 0.78 | 1.0 | 0.50 |
| 120 | D120G | 1.00 | 0.99 | 1.04 | 0.97 | 0.8 | 0.92 |
| 120 | D120H | 1.13 | 1.07 | 1.16 | 0.88 | 1.1 | 0.93 |
| 120 | D120I | 0.20 | 0.92 | 1.69 | 1.26 | 1.0 | 0.55 |
| 120 | D120K | 1.15 | 0.88 | 1.01 | 0.95 | 1.1 | 0.81 |
| 120 | D120L | 0.33 | 0.65 | 1.25 | 1.12 | 1.1 | 0.88 |
| 120 | D120M | 0.73 | 0.87 | 0.97 | 0.95 | 0.7 | 0.93 |
| 120 | D120N | 1.11 | 1.01 | 1.00 | 0.92 | 1.1 | 0.90 |
| 120 | D120P | 0.22 | 1.12 | 1.61 | 1.14 | 1.1 | 0.53 |
| 120 | D120Q | 1.05 | 1.21 | 1.09 | 0.96 | 1.1 | 0.87 |
| 120 | D120R | 1.03 | 0.86 | 0.97 | 1.10 | 0.9 | 0.93 |
| 120 | D120S | 1.01 | 0.94 | 1.16 | 0.86 | 1.0 | 0.97 |
| 120 | D120T | 0.77 | 1.08 | 1.09 | 1.00 | 1.0 | 1.06 |
| 120 | D120V | 0.32 | 1.13 | 1.21 | 0.64 | 1.1 | 0.81 |
| 120 | D120W | 0.32 | 1.10 | 1.44 | 1.19 | 1.1 | 0.95 |
| 120 | D120Y | 0.22 | 1.30 | 1.73 | 1.29 | 1.0 | 0.70 |
| 121 | V121A | 0.37 | ***0.05*** | ***0.05*** | ***0.05*** | ***0.05*** | ***0.05*** |
| 121 | V121C | 0.76 | 0.98 | 0.99 | 0.78 | 1.1 | 0.92 |
| 121 | V121D | ***0.05*** | ***0.05*** | ***0.05*** | ***0.05*** | ***0.05*** | ***0.05*** |
| 121 | V121E | 0.19 | 0.45 | 1.16 | 0.85 | 0.7 | 0.21 |
| 121 | V121F | 0.18 | 0.12 | 1.14 | 0.47 | 0.7 | 0.12 |
| 121 | V121G | 0.18 | 0.14 | 0.37 | 0.34 | ***0.05*** | ***0.05*** |
| 121 | V121H | 0.30 | ***0.05*** | ***0.05*** | ***0.05*** | ***0.05*** | ***0.05*** |
| 121 | V121I | 1.15 | 0.83 | 0.88 | 0.81 | 1.0 | 0.89 |
| 121 | V121K | ***0.05*** | ***0.05*** | ***0.05*** | ***0.05*** | ***0.05*** | ***0.05*** |
| 121 | V121L | 0.81 | 0.95 | 0.91 | 0.88 | 0.8 | 0.94 |
| 121 | V121M | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 121 | V121N | 0.25 | ***0.05*** | 0.14 | ***0.05*** | ***0.05*** | ***0.05*** |
| 121 | V121P | 0.32 | ***0.05*** | 0.06 | ***0.05*** | ***0.05*** | ***0.05*** |
| 121 | V121Q | 0.25 | ***0.05*** | 0.11 | ***0.05*** | ***0.05*** | ***0.05*** |
| 121 | V121R | 0.20 | ***0.05*** | 0.65 | 0.08 | ***0.05*** | ***0.05*** |
| 121 | V121S | 0.15 | ***0.05*** | ***0.05*** | ***0.05*** | 1.1 | 0.47 |
| 121 | V121T | 0.26 | 1.28 | 1.66 | 1.24 | 1.1 | 0.85 |
| 121 | V121W | 0.26 | ***0.05*** | ***0.05*** | ***0.05*** | ***0.05*** | ***0.05*** |
| 121 | V121Y | 0.30 | ***0.05*** | ***0.05*** | ***0.05*** | ***0.05*** | ***0.05*** |
| 122 | I122A | 0.92 | 1.06 | 1.04 | 1.00 | 0.8 | 0.88 |
| 122 | I122C | 0.78 | 1.17 | 1.22 | 1.09 | 0.8 | 0.98 |
| 122 | I122D | 0.18 | ***0.05*** | ***0.05*** | ***0.05*** | ***0.05*** | ***0.05*** |
| 122 | I122E | 0.17 | ***0.05*** | ***0.05*** | ***0.05*** | ***0.05*** | ***0.05*** |
| 122 | I122F | 0.37 | 1.16 | 1.45 | 1.19 | 1.4 | 0.66 |
| 122 | I122G | 0.20 | ***0.05*** | ***0.05*** | 4.05 | 2.3 | 0.36 |
| 122 | I122H | 0.16 | ***0.05*** | ***0.05*** | ***0.05*** | ***0.05*** | ***0.05*** |
| 122 | I122K | 0.23 | 0.86 | 0.47 | 0.12 | ***0.05*** | ***0.05*** |
| 122 | I122L | 0.73 | 0.99 | 1.28 | 1.09 | 0.8 | 0.97 |
| 122 | I122M | 0.93 | 1.17 | 1.05 | 1.23 | 0.8 | 0.96 |
| 122 | I122N | 0.17 | ***0.05*** | ***0.05*** | ***0.05*** | ***0.05*** | ***0.05*** |
| 122 | I122P | 0.25 | 0.15 | 0.25 | ***0.05*** | ***0.05*** | ***0.05*** |
| 122 | I122Q | 0.15 | ***0.05*** | ***0.05*** | ***0.05*** | 2.3 | 0.15 |
| 122 | I122R | 0.21 | 2.78 | ***0.05*** | ***0.05*** | ***0.05*** | ***0.05*** |
| 122 | I122S | 0.22 | 6.46 | 6.12 | 2.56 | 1.9 | 0.43 |
| 122 | I122T | 0.34 | 1.78 | 1.79 | 1.13 | 1.4 | 0.90 |
| 122 | I122V | 0.80 | 1.14 | 1.39 | 1.13 | 0.7 | 1.09 |
| 122 | I122W | 0.16 | ***0.05*** | ***0.05*** | ***0.05*** | ***0.05*** | ***0.05*** |
| 122 | I122Y | 0.16 | ***0.05*** | ***0.05*** | ***0.05*** | 1.9 | 0.55 |
| 123 | N123A | 0.75 | 1.71 | 1.36 | 1.06 | ***0.05*** | ***0.05*** |
| 123 | N123C | 0.95 | 1.53 | 1.35 | 1.43 | 0.5 | 0.09 |
| 123 | N123D | 0.53 | 1.03 | 1.07 | 1.04 | ***0.05*** | ***0.05*** |
| 123 | N123E | 0.96 | 1.13 | 0.77 | 0.94 | ***0.05*** | ***0.05*** |
| 123 | N123F | 0.39 | ***0.05*** | 0.17 | ***0.05*** | ***0.05*** | ***0.05*** |
| 123 | N123G | 0.47 | 1.81 | 1.59 | 1.34 | 0.4 | 0.13 |
| 123 | N123H | ***0.05*** | ***0.05*** | ***0.05*** | ***0.05*** | ***0.05*** | ***0.05*** |
| 123 | N123I | 0.55 | 1.20 | 1.11 | 1.06 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 123 | N123K | 0.39 | ***0.05*** | ***0.05*** | ***0.05*** | ***0.05*** | ***0.05*** |
| 123 | N123L | 0.87 | 0.98 | 1.13 | 1.06 | ***0.05*** | ***0.05*** |
| 123 | N123M | 0.43 | 1.24 | 1.01 | 0.96 | ***0.05*** | ***0.05*** |
| 123 | N123P | 0.36 | ***0.05*** | 0.13 | ***0.05*** | ***0.05*** | ***0.05*** |
| 123 | N123Q | 0.87 | 1.22 | 1.30 | 1.03 | 0.7 | 0.09 |
| 123 | N123R | 0.34 | ***0.05*** | 0.10 | ***0.05*** | ***0.05*** | ***0.05*** |
| 123 | N123S | 0.92 | 1.31 | 1.15 | 1.08 | 0.2 | 0.09 |
| 123 | N123T | 0.94 | 1.20 | 1.41 | 0.93 | 0.4 | 0.13 |
| 123 | N123V | 0.89 | 1.27 | 1.32 | 1.25 | ***0.05*** | ***0.05*** |
| 123 | N123W | 0.30 | 0.06 | 0.07 | ***0.05*** | ***0.05*** | ***0.05*** |
| 123 | N123Y | 0.29 | ***0.05*** | 0.09 | ***0.05*** | ***0.05*** | ***0.05*** |
| 124 | M124A | 0.61 | 1.44 | 1.02 | 0.97 | 1.0 | 0.69 |
| 124 | M124C | 0.26 | 0.86 | 0.54 | 0.63 | 1.3 | 0.07 |
| 124 | M124D | 0.22 | 0.22 | ***0.05*** | ***0.05*** | ***0.05*** | ***0.05*** |
| 124 | M124E | 0.23 | 0.59 | 0.08 | 0.13 | ***0.05*** | ***0.05*** |
| 124 | M124F | 1.13 | 1.02 | 0.86 | 1.06 | 0.9 | 0.34 |
| 124 | M124H | 0.20 | 1.36 | 0.26 | 0.31 | ***0.05*** | ***0.05*** |
| 124 | M124I | 1.36 | 1.12 | 1.08 | 1.12 | 1.0 | 0.34 |
| 124 | M124K | 0.18 | 0.15 | ***0.05*** | ***0.05*** | ***0.05*** | ***0.05*** |
| 124 | M124L | 0.86 | 0.69 | 1.01 | 1.03 | 1.2 | 0.92 |
| 124 | M124N | 0.18 | 2.98 | 0.78 | 0.79 | ***0.05*** | ***0.05*** |
| 124 | M124P | 0.20 | 0.36 | 0.22 | 0.08 | ***0.05*** | ***0.05*** |
| 124 | M124Q | 0.18 | 5.22 | 2.64 | 1.40 | ***0.05*** | ***0.05*** |
| 124 | M124R | 0.19 | 0.51 | ***0.05*** | ***0.05*** | ***0.05*** | ***0.05*** |
| 124 | M124S | 0.28 | 2.79 | 1.44 | 1.52 | 0.9 | 0.12 |
| 124 | M124T | 0.49 | 1.82 | 1.32 | 1.35 | 0.9 | 0.23 |
| 124 | M124V | 1.07 | 1.37 | 1.15 | 1.06 | 0.9 | 0.31 |
| 124 | M124W | 0.20 | 0.28 | ***0.05*** | ***0.05*** | ***0.05*** | ***0.05*** |
| 124 | M124Y | 0.20 | 0.71 | 1.12 | 0.13 | ***0.05*** | ***0.05*** |
| 125 | S125A | 0.92 | 1.90 | 1.40 | 1.17 | 1.1 | 0.06 |
| 125 | S125C | 1.04 | 0.19 | ***0.05*** | 0.11 | ***0.05*** | ***0.05*** |
| 125 | S125D | ***0.05*** | ***0.05*** | ***0.05*** | ***0.05*** | ***0.05*** | ***0.05*** |
| 125 | S125E | 0.25 | 0.23 | ***0.05*** | ***0.05*** | ***0.05*** | ***0.05*** |
| 125 | S125F | 0.22 | 0.96 | ***0.05*** | 0.08 | ***0.05*** | ***0.05*** |
| 125 | S125G | 0.33 | 0.23 | ***0.05*** | 0.18 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 125 | S125H | ***0.05*** | ***0.05*** | ***0.05*** | ***0.05*** | ***0.05*** | ***0.05*** |
| 125 | S125I | 0.36 | 0.08 | ***0.05*** | ***0.05*** | ***0.05*** | ***0.05*** |
| 125 | S125K | 0.23 | 0.21 | 0.10 | 0.06 | ***0.05*** | ***0.05*** |
| 125 | S125L | 0.23 | 0.46 | ***0.05*** | ***0.05*** | ***0.05*** | ***0.05*** |
| 125 | S125M | 0.24 | 0.49 | 0.18 | ***0.05*** | ***0.05*** | ***0.05*** |
| 125 | S125N | 1.11 | ***0.05*** | ***0.05*** | ***0.05*** | ***0.05*** | ***0.05*** |
| 125 | S125P | 0.48 | ***0.05*** | ***0.05*** | ***0.05*** | ***0.05*** | ***0.05*** |
| 125 | S125Q | 0.37 | 0.08 | ***0.05*** | ***0.05*** | ***0.05*** | ***0.05*** |
| 125 | S125R | 0.22 | 0.44 | 0.07 | 0.11 | ***0.05*** | ***0.05*** |
| 125 | S125T | 0.56 | 0.07 | ***0.05*** | ***0.05*** | ***0.05*** | ***0.05*** |
| 125 | S125V | 0.20 | ***0.05*** | 0.11 | 0.07 | ***0.05*** | ***0.05*** |
| 125 | S125W | 0.21 | 0.96 | 0.27 | ***0.05*** | ***0.05*** | ***0.05*** |
| 125 | S125Y | ***0.05*** | ***0.05*** | ***0.05*** | ***0.05*** | ***0.05*** | ***0.05*** |
| 126 | L126A | 0.93 | 1.56 | 1.09 | 1.22 | ***0.05*** | ***0.05*** |
| 126 | L126C | 1.32 | 0.64 | 0.37 | 0.77 | ***0.05*** | ***0.05*** |
| 126 | L126D | 0.23 | 0.08 | 0.07 | ***0.05*** | ***0.05*** | ***0.05*** |
| 126 | L126I | 1.11 | 1.17 | 1.03 | 1.17 | 1.1 | 0.11 |
| 126 | L126K | 0.14 | ***0.05*** | ***0.05*** | ***0.05*** | ***0.05*** | ***0.05*** |
| 126 | L126N | 0.37 | 1.14 | 0.23 | 0.67 | ***0.05*** | ***0.05*** |
| 126 | L126P | 0.28 | 0.07 | ***0.05*** | ***0.05*** | ***0.05*** | ***0.05*** |
| 126 | L126Q | 0.25 | 3.05 | 0.91 | 1.13 | ***0.05*** | ***0.05*** |
| 126 | L126R | 0.20 | 0.28 | ***0.05*** | ***0.05*** | ***0.05*** | ***0.05*** |
| 126 | L126S | 0.73 | 1.56 | 0.93 | 0.86 | ***0.05*** | ***0.05*** |
| 126 | L126T | 0.53 | 2.41 | 1.47 | 1.40 | ***0.05*** | ***0.05*** |
| 126 | L126V | 1.09 | 1.26 | 1.34 | 1.32 | 1.0 | 0.08 |
| 126 | L126W | 0.89 | 0.71 | 0.79 | 0.78 | 0.9 | 0.06 |
| 126 | L126Y | 0.58 | 1.47 | 1.10 | 1.27 | ***0.05*** | ***0.05*** |
| 127 | G127A | 1.44 | 0.91 | 0.93 | 0.94 | ***0.05*** | ***0.05*** |
| 127 | G127C | 1.06 | 1.00 | 0.57 | 0.87 | ***0.05*** | ***0.05*** |
| 127 | G127E | 0.91 | 0.80 | 0.90 | 0.93 | ***0.05*** | ***0.05*** |
| 127 | G127F | 1.20 | 0.60 | 0.31 | 0.68 | ***0.05*** | ***0.05*** |
| 127 | G127I | 1.33 | 0.78 | 0.54 | 0.60 | ***0.05*** | ***0.05*** |
| 127 | G127K | 0.58 | 0.70 | 0.47 | 0.88 | ***0.05*** | ***0.05*** |
| 127 | G127L | 0.91 | 0.99 | 0.64 | 0.56 | ***0.05*** | ***0.05*** |
| 127 | G127M | 0.93 | 0.84 | 0.57 | 0.83 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 127 | G127N | 1.06 | 1.03 | 0.81 | 0.82 | ***0.05*** | ***0.05*** |
| 127 | G127P | 0.41 | ***0.05*** | ***0.05*** | ***0.05*** | ***0.05*** | ***0.05*** |
| 127 | G127Q | 1.00 | 1.02 | 0.87 | 0.85 | ***0.05*** | ***0.05*** |
| 127 | G127R | 0.62 | 0.55 | 0.34 | 0.71 | ***0.05*** | ***0.05*** |
| 127 | G127S | 1.06 | 1.10 | 0.80 | 0.93 | ***0.05*** | ***0.05*** |
| 127 | G127T | 0.80 | 1.07 | 0.89 | 0.88 | ***0.05*** | ***0.05*** |
| 127 | G127V | 1.07 | 0.81 | 0.68 | 0.75 | ***0.05*** | ***0.05*** |
| 127 | G127W | 1.48 | 0.55 | 0.27 | 0.50 | ***0.05*** | ***0.05*** |
| 127 | G127Y | 1.00 | 0.68 | 0.54 | 0.60 | ***0.05*** | ***0.05*** |
| 128 | G128A | 1.21 | 1.24 | 1.28 | 1.21 | 1.0 | 0.16 |
| 128 | G128C | ***0.05*** | ***0.05*** | ***0.05*** | ***0.05*** | ***0.05*** | ***0.05*** |
| 128 | G128E | 0.31 | 1.63 | 1.19 | 1.45 | ***0.05*** | ***0.05*** |
| 128 | G128F | 0.50 | 0.70 | 0.46 | 0.80 | ***0.05*** | ***0.05*** |
| 128 | G128H | 0.86 | 1.15 | 1.12 | 0.98 | ***0.05*** | ***0.05*** |
| 128 | G128I | 0.39 | 0.35 | 0.21 | 0.36 | ***0.05*** | ***0.05*** |
| 128 | G128K | 0.45 | 0.25 | 0.08 | 0.33 | ***0.05*** | ***0.05*** |
| 128 | G128L | 0.57 | 0.29 | 0.16 | 0.37 | ***0.05*** | ***0.05*** |
| 128 | G128N | 0.55 | 1.59 | 1.55 | 1.29 | ***0.05*** | ***0.05*** |
| 128 | G128P | 0.39 | 0.17 | 0.12 | 0.18 | ***0.05*** | ***0.05*** |
| 128 | G128Q | 0.39 | 1.05 | 0.84 | 0.92 | ***0.05*** | ***0.05*** |
| 128 | G128R | 0.39 | 0.28 | 0.18 | 0.33 | ***0.05*** | ***0.05*** |
| 128 | G128S | 1.35 | 1.17 | 1.12 | 1.09 | 1.2 | 0.26 |
| 128 | G128T | 0.28 | 2.16 | 2.42 | 1.63 | ***0.05*** | ***0.05*** |
| 128 | G128V | 0.36 | 0.47 | 0.39 | 0.52 | ***0.05*** | ***0.05*** |
| 128 | G128W | 0.37 | 0.33 | 0.15 | 0.48 | ***0.05*** | ***0.05*** |
| 128 | G128Y | 0.39 | 0.24 | 0.16 | 0.36 | ***0.05*** | ***0.05*** |
| 129 | P129A | 1.04 | 1.02 | 0.98 | 0.99 | 0.7 | 0.80 |
| 129 | P129C | 1.37 | 1.09 | 1.11 | 0.91 | 0.9 | 0.56 |
| 129 | P129D | 1.48 | 1.03 | 1.08 | 0.84 | 1.1 | 0.67 |
| 129 | P129E | 1.42 | 1.24 | 1.04 | 1.10 | 1.0 | 0.87 |
| 129 | P129F | 0.65 | 1.10 | 0.71 | 1.00 | 0.7 | 1.27 |
| 129 | P129G | 0.78 | 0.82 | 0.60 | 0.81 | 0.8 | 0.56 |
| 129 | P129H | 0.66 | 0.92 | 0.73 | 0.84 | 0.9 | 0.81 |
| 129 | P129I | 1.14 | 0.95 | 0.99 | 1.11 | 0.9 | 0.40 |
| 129 | P129K | 1.13 | 0.72 | 0.64 | 1.01 | 0.9 | 1.22 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 129 | P129L | 1.12 | 1.13 | 1.21 | 1.06 | 0.9 | 0.53 |
| 129 | P129M | 1.20 | 1.08 | 0.92 | 1.20 | 0.9 | 0.88 |
| 129 | P129N | 0.90 | 1.00 | 1.04 | 0.98 | 0.9 | 0.85 |
| 129 | P129Q | 1.10 | 1.22 | 0.90 | 1.01 | 1.0 | 0.98 |
| 129 | P129R | 0.74 | 0.73 | 0.75 | 0.87 | 0.9 | 1.01 |
| 129 | P129S | 1.07 | 0.99 | 0.87 | 0.98 | 0.9 | 0.84 |
| 129 | P129T | 1.13 | 1.29 | 1.17 | 1.09 | 0.9 | 1.06 |
| 129 | P129V | 0.72 | 1.41 | 1.26 | 1.09 | 0.8 | 0.75 |
| 129 | P129W | 0.38 | 0.97 | 0.79 | 1.18 | 0.4 | 1.18 |
| 129 | P129Y | 0.71 | 1.01 | 0.90 | 1.04 | 0.8 | 1.56 |
| 130 | S130A | 1.14 | 0.92 | 0.76 | 0.86 | 1.0 | 0.92 |
| 130 | S130C | 1.24 | 0.75 | 0.95 | 0.66 | 1.1 | 0.78 |
| 130 | S130D | ***0.05*** | ***0.05*** | ***0.05*** | ***0.05*** | ***0.05*** | ***0.05*** |
| 130 | S130E | 1.36 | 0.73 | 1.29 | 1.03 | 1.1 | 0.88 |
| 130 | S130F | 1.17 | 0.89 | 0.79 | 0.84 | 1.0 | 1.02 |
| 130 | S130G | 0.93 | 0.95 | 0.99 | 0.98 | 0.9 | 0.80 |
| 130 | S130H | 1.33 | 0.76 | 0.97 | 1.05 | 1.1 | 1.03 |
| 130 | S130I | 1.22 | 0.82 | 0.97 | 0.91 | 1.1 | 0.95 |
| 130 | S130K | 1.27 | 0.63 | 0.70 | 0.67 | 1.1 | 1.12 |
| 130 | S130L | 1.04 | 0.90 | 0.79 | 0.80 | 1.1 | 0.98 |
| 130 | S130M | 1.19 | 0.97 | 0.91 | 0.89 | 0.9 | 0.92 |
| 130 | S130N | ***0.05*** | ***0.05*** | ***0.05*** | ***0.05*** | ***0.05*** | ***0.05*** |
| 130 | S130P | 0.60 | 1.32 | 1.32 | 1.00 | 0.7 | 0.07 |
| 130 | S130Q | 1.19 | 0.98 | 0.95 | 0.93 | 1.1 | 1.06 |
| 130 | S130R | 1.12 | 0.47 | 0.63 | 0.77 | 1.1 | 1.11 |
| 130 | S130T | 1.16 | 1.08 | 1.10 | 0.95 | 0.9 | 1.19 |
| 130 | S130V | 1.13 | 0.98 | 0.96 | 0.91 | 1.0 | 1.07 |
| 130 | S 130W | 0.73 | 0.60 | 0.81 | 0.86 | 0.8 | 0.89 |
| 130 | S130Y | 1.24 | 0.73 | 0.94 | 0.95 | 1.0 | 1.04 |
| 131 | G131A | 1.21 | 0.53 | 1.00 | 0.84 | 1.1 | 1.02 |
| 131 | G131C | 0.55 | 0.59 | 0.82 | 0.83 | 0.6 | 0.87 |
| 131 | G131D | 0.93 | 0.50 | 0.80 | 0.78 | 0.9 | 1.32 |
| 131 | G131E | 0.35 | ***0.05*** | 0.34 | 0.64 | 0.9 | 0.67 |
| 131 | G131F | 0.23 | 0.42 | 0.55 | 0.67 | 0.2 | 0.43 |
| 131 | G131H | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 131 | G131I | 0.17 | ***0.05*** | 0.52 | 0.29 | 1.1 | 0.26 |
| 131 | G131K | 1.00 | 0.48 | 1.00 | 0.89 | 1.0 | 1.39 |
| 131 | G131L | 0.27 | ***0.05*** | 0.20 | ***0.05*** | ***0.05*** | ***0.05*** |
| 131 | G131M | 0.53 | 0.47 | 0.89 | 0.75 | 0.6 | 0.90 |
| 131 | G131N | 0.46 | 0.23 | 0.49 | 0.54 | 0.8 | 1.11 |
| 131 | G131P | 0.22 | 0.77 | 1.29 | 1.21 | 0.1 | 0.50 |
| 131 | G131Q | 0.28 | 0.49 | 1.15 | 0.95 | 0.3 | 0.70 |
| 131 | G131R | 0.19 | 0.97 | 2.04 | 1.64 | 0.1 | 0.59 |
| 131 | G131S | 0.21 | 0.12 | 0.47 | 0.83 | 0.2 | 0.45 |
| 131 | G131T | 0.86 | 0.52 | 1.16 | 0.89 | 1.0 | 1.03 |
| 131 | G131V | 0.51 | 0.50 | 0.83 | 0.94 | 0.7 | 0.99 |
| 131 | G131W | ***0.05*** | ***0.05*** | ***0.05*** | ***0.05*** | ***0.05*** | ***0.05*** |
| 131 | G131Y | 0.30 | 0.26 | 0.40 | 0.71 | 0.9 | 1.04 |
| 132 | S132A | 0.60 | 1.01 | 1.03 | 1.03 | 0.5 | 0.77 |
| 132 | S132C | 1.25 | 1.06 | 1.10 | 0.83 | 1.0 | 0.64 |
| 132 | S132D | ***0.05*** | ***0.05*** | ***0.05*** | ***0.05*** | ***0.05*** | ***0.05*** |
| 132 | S132E | 0.33 | 1.04 | 1.40 | 1.01 | 0.9 | 0.50 |
| 132 | S132F | 0.30 | ***0.05*** | 0.10 | 0.08 | ***0.05*** | ***0.05*** |
| 132 | S132G | 0.83 | 0.83 | 0.14 | 0.94 | 1.0 | 0.41 |
| 132 | S132H | ***0.05*** | ***0.05*** | ***0.05*** | ***0.05*** | ***0.05*** | ***0.05*** |
| 132 | S132I | 0.25 | 1.62 | 1.82 | 1.25 | 0.8 | 0.57 |
| 132 | S132K | 0.19 | 0.09 | 0.60 | 0.16 | 0.7 | 0.11 |
| 132 | S132L | 0.25 | 1.27 | 1.65 | 1.22 | 0.8 | 0.78 |
| 132 | S132M | 0.35 | 1.01 | 1.05 | 1.05 | 0.6 | 0.72 |
| 132 | S132N | 0.88 | 1.38 | 1.35 | 1.17 | 1.0 | 0.87 |
| 132 | S132P | 0.17 | 9.36 | 11.70 | 2.50 | 1.0 | 0.23 |
| 132 | S132Q | 0.34 | 1.35 | 1.28 | 1.18 | 0.7 | 0.88 |
| 132 | S132R | 0.15 | ***0.05*** | ***0.05*** | ***0.05*** | 0.8 | 0.15 |
| 132 | S132T | ***0.05*** | ***0.05*** | ***0.05*** | ***0.05*** | ***0.05*** | ***0.05*** |
| 132 | S132V | 0.23 | 3.29 | 2.39 | 2.07 | 0.8 | 0.58 |
| 132 | S132W | 0.24 | 0.08 | 0.06 | ***0.05*** | ***0.05*** | ***0.05*** |
| 132 | S132Y | 0.21 | 0.07 | 0.40 | 0.08 | ***0.05*** | ***0.05*** |
| 133 | A133C | 0.98 | 0.87 | 1.06 | 0.79 | 1.1 | 0.84 |
| 133 | A133D | 0.91 | 0.90 | 0.79 | 0.81 | 1.0 | 0.62 |
| 133 | A133E | 1.07 | 0.93 | 0.96 | 0.84 | 1.0 | 0.99 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 133 | A133F | 0.83 | 0.95 | 0.65 | 0.93 | 0.9 | 1.11 |
| 133 | A133G | 0.78 | 0.95 | 0.95 | 0.92 | 1.0 | 1.12 |
| 133 | A133H | ***0.05*** | ***0.05*** | ***0.05*** | ***0.05*** | ***0.05*** | ***0.05*** |
| 133 | A133I | 0.95 | 0.88 | 0.88 | 0.84 | 1.0 | 1.49 |
| 133 | A133K | 1.11 | 0.75 | 0.65 | 0.72 | 1.1 | 1.02 |
| 133 | A133L | 0.93 | 0.78 | 0.83 | 0.88 | 1.0 | 1.15 |
| 133 | A133M | 0.95 | 0.99 | 0.71 | 0.85 | 1.0 | 1.00 |
| 133 | A133N | ***0.05*** | ***0.05*** | ***0.05*** | ***0.05*** | ***0.05*** | ***0.05*** |
| 133 | A133P | 1.10 | 1.21 | 0.74 | 0.83 | 1.2 | 0.94 |
| 133 | A133Q | 0.85 | 1.25 | 0.98 | 0.79 | 1.1 | 1.07 |
| 133 | A133R | 0.93 | 0.51 | 0.41 | 0.65 | 1.1 | 0.97 |
| 133 | A133S | 1.05 | 1.00 | 0.94 | 0.82 | 1.0 | 1.06 |
| 133 | A133T | 0.96 | 1.03 | 0.78 | 0.82 | 1.1 | 1.14 |
| 133 | A133V | 0.98 | 0.95 | 0.89 | 0.87 | 1.1 | 1.07 |
| 133 | A133W | 0.73 | 0.86 | 0.42 | 0.73 | 1.1 | 1.09 |
| 133 | A133Y | 0.84 | 0.71 | 0.75 | 0.65 | 1.1 | 1.12 |
| 134 | A134C | 0.78 | 0.83 | 1.13 | 0.80 | 1.0 | 0.82 |
| 134 | A134D | 0.19 | ***0.05*** | 0.68 | 0.35 | 1.1 | 0.12 |
| 134 | A134E | 0.23 | ***0.05*** | 0.34 | 0.23 | ***0.05*** | ***0.05*** |
| 134 | A134F | 0.26 | 0.99 | 0.97 | 0.88 | 0.9 | 0.86 |
| 134 | A134G | 0.63 | 1.14 | 0.59 | 0.93 | 1.2 | 0.74 |
| 134 | A134H | 0.12 | ***0.05*** | ***0.05*** | ***0.05*** | 1.1 | 0.28 |
| 134 | A134I | 0.26 | 1.60 | 1.22 | 1.29 | 0.9 | 0.93 |
| 134 | A134K | 0.16 | ***0.05*** | ***0.05*** | 4.71 | 1.2 | 0.33 |
| 134 | A134L | 0.19 | 1.85 | 2.41 | 1.75 | 1.1 | 0.61 |
| 134 | A134M | 0.21 | 1.40 | 1.20 | 1.29 | 1.1 | 0.53 |
| 134 | A134N | ***0.05*** | ***0.05*** | ***0.05*** | ***0.05*** | ***0.05*** | ***0.05*** |
| 134 | A134P | 0.78 | 1.06 | 0.86 | 0.95 | 1.0 | 1.25 |
| 134 | A134Q | 0.19 | 0.12 | 0.36 | 0.32 | 1.1 | 0.10 |
| 134 | A134R | 0.25 | ***0.05*** | ***0.05*** | ***0.05*** | ***0.05*** | ***0.05*** |
| 134 | A134S | 0.97 | 1.04 | 0.91 | 0.92 | 1.1 | 1.12 |
| 134 | A134T | 0.94 | 1.03 | 0.86 | 1.04 | 1.0 | 1.04 |
| 134 | A134V | 0.39 | 1.27 | 1.04 | 0.84 | 0.9 | 1.14 |
| 134 | A134W | 0.19 | ***0.05*** | 0.32 | 0.15 | 1.1 | 0.08 |
| 134 | A134Y | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 135 | L135A | 0.23 | 1.24 | 0.90 | 1.12 | 1.0 | 0.07 |
| 135 | L135D | 0.46 | ***0.05*** | ***0.05*** | ***0.05*** | ***0.05*** | ***0.05*** |
| 135 | L135E | 0.43 | 1.19 | 1.03 | 0.88 | 1.1 | 0.82 |
| 135 | L135I | 0.34 | 1.41 | 0.97 | 1.06 | 0.7 | 0.16 |
| 135 | L135K | 0.35 | ***0.05*** | ***0.05*** | ***0.05*** | ***0.05*** | ***0.05*** |
| 135 | L135M | 1.03 | 1.01 | 1.19 | 0.96 | 1.1 | 0.59 |
| 135 | L135N | 0.34 | ***0.05*** | ***0.05*** | ***0.05*** | ***0.05*** | ***0.05*** |
| 135 | L135P | 0.37 | ***0.05*** | ***0.05*** | ***0.05*** | ***0.05*** | ***0.05*** |
| 135 | L135R | 0.37 | 0.06 | ***0.05*** | ***0.05*** | ***0.05*** | ***0.05*** |
| 135 | L135T | 0.19 | 3.36 | 2.29 | 2.06 | 0.6 | 0.06 |
| 135 | L135V | 0.34 | 2.12 | 1.12 | 1.33 | 0.6 | 0.19 |
| 135 | L135W | 0.27 | 2.14 | 1.96 | 1.47 | 0.8 | 0.23 |
| 135 | L135Y | 0.34 | 1.91 | 2.03 | 1.66 | ***0.05*** | ***0.05*** |
| 136 | K136A | 0.57 | 0.80 | 1.04 | 0.83 | 0.9 | 0.80 |
| 136 | K136C | 0.51 | 1.02 | 1.04 | 0.74 | 0.9 | 0.81 |
| 136 | K136D | 0.21 | 1.62 | 1.47 | 0.92 | 0.4 | 0.47 |
| 136 | K136E | 1.03 | 0.92 | 0.73 | 0.82 | 1.2 | 1.03 |
| 136 | K136F | 0.27 | 1.35 | 1.57 | 1.15 | 0.6 | 0.82 |
| 136 | K136G | 0.44 | 1.09 | 1.12 | 0.91 | 0.7 | 0.82 |
| 136 | K136H | 0.89 | 0.99 | 1.00 | 0.68 | 1.1 | 1.08 |
| 136 | K136I | 0.20 | 2.18 | 2.48 | 1.44 | 0.4 | 0.47 |
| 136 | K136L | 0.65 | 1.05 | 0.71 | 0.90 | 1.0 | 1.16 |
| 136 | K136M | 0.69 | 1.02 | 0.87 | 0.87 | 0.9 | 1.09 |
| 136 | K136N | 0.60 | 1.08 | 0.88 | 0.81 | 1.0 | 0.88 |
| 136 | K136P | 0.20 | 0.23 | 0.74 | 0.27 | ***0.05*** | ***0.05*** |
| 136 | K136Q | ***0.05*** | ***0.05*** | ***0.05*** | ***0.05*** | ***0.05*** | ***0.05*** |
| 136 | K136R | 0.82 | 1.02 | 0.82 | 0.69 | 1.1 | 1.01 |
| 136 | K136S | 0.39 | 1.08 | 0.87 | 0.92 | 0.7 | 0.80 |
| 136 | K136T | 0.16 | ***0.05*** | ***0.05*** | 9.54 | 0.5 | 0.37 |
| 136 | K136V | 0.21 | 2.18 | 2.41 | 1.96 | 0.5 | 0.83 |
| 136 | K136W | 0.44 | 1.03 | 0.93 | 0.80 | 0.6 | 0.91 |
| 136 | K136Y | 0.25 | 1.78 | 1.41 | 1.21 | 0.4 | 0.94 |
| 137 | A137C | 1.00 | 0.91 | 0.95 | 0.71 | 1.1 | 1.08 |
| 137 | A137D | 1.14 | 1.04 | 1.00 | 0.81 | 1.0 | 1.06 |
| 137 | A137E | 0.75 | 0.91 | 1.10 | 0.88 | 1.1 | 1.03 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 137 | A137F | 0.59 | 1.01 | 0.78 | 0.90 | 1.0 | 1.41 |
| 137 | A137G | 0.72 | 1.01 | 0.91 | 0.78 | 1.1 | 1.24 |
| 137 | A137H | 1.02 | 0.95 | 0.78 | 0.91 | 0.9 | 1.03 |
| 137 | A137I | ***0.05*** | ***0.05*** | ***0.05*** | ***0.05*** | ***0.05*** | ***0.05*** |
| 137 | A137K | 0.90 | 0.96 | 0.52 | 0.90 | 1.0 | 1.17 |
| 137 | A137L | 0.84 | 0.85 | 0.82 | 0.89 | 1.1 | 1.07 |
| 137 | A137M | 0.93 | 1.09 | 0.89 | 0.85 | 1.0 | 1.06 |
| 137 | A137N | 1.01 | 1.02 | 0.62 | 0.77 | 1.0 | 1.38 |
| 137 | A137P | 0.18 | 3.85 | 3.42 | 1.76 | 0.2 | 0.10 |
| 137 | A137Q | 1.05 | 1.05 | 0.60 | 0.92 | 1.0 | 1.18 |
| 137 | A137R | 0.65 | 0.65 | 0.49 | 0.70 | 1.0 | 1.17 |
| 137 | A137S | 0.98 | 0.91 | 0.92 | 1.08 | 0.9 | 1.11 |
| 137 | A137T | 0.96 | 0.94 | 0.77 | 0.96 | 1.0 | 1.07 |
| 137 | A137V | 0.62 | 1.27 | 0.85 | 1.06 | 0.8 | 1.23 |
| 137 | A137W | 0.69 | 0.81 | 0.55 | 0.62 | 0.9 | 1.50 |
| 137 | A137Y | 0.83 | 1.05 | 0.73 | 0.66 | 0.9 | 1.33 |
| 138 | A138C | 0.85 | 1.04 | 1.10 | 1.14 | 0.9 | 1.07 |
| 138 | A138D | 0.17 | 1.47 | 4.55 | 2.27 | 0.3 | 0.16 |
| 138 | A138E | 0.36 | 1.44 | 0.96 | 1.13 | 0.9 | 0.83 |
| 138 | A138F | 0.18 | 2.55 | 2.71 | 1.96 | 0.8 | 0.31 |
| 138 | A138G | 0.52 | 1.27 | 1.35 | 1.07 | 0.9 | 1.16 |
| 138 | A138H | 0.20 | 2.04 | 2.24 | 1.84 | 0.7 | 0.52 |
| 138 | A138I | 0.78 | 0.93 | 0.98 | 1.11 | 0.9 | 1.18 |
| 138 | A138K | 0.29 | ***0.05*** | ***0.05*** | ***0.05*** | ***0.05*** | ***0.05*** |
| 138 | A138L | 0.27 | 1.23 | 1.19 | 1.42 | 0.9 | 0.70 |
| 138 | A138M | 0.71 | 0.88 | 0.89 | 1.00 | 1.0 | 0.91 |
| 138 | A138N | 0.16 | ***0.05*** | ***0.05*** | 4.25 | 0.9 | 0.25 |
| 138 | A138P | 0.32 | ***0.05*** | 0.09 | ***0.05*** | ***0.05*** | ***0.05*** |
| 138 | A138Q | 0.17 | 11.86 | 16.19 | 4.99 | 0.9 | 0.75 |
| 138 | A138R | 0.40 | ***0.05*** | 0.07 | ***0.05*** | ***0.05*** | ***0.05*** |
| 138 | A138S | 0.86 | 1.05 | 0.99 | 1.02 | 0.7 | 1.22 |
| 138 | A138T | 0.51 | 1.17 | 1.27 | 1.18 | 0.9 | 1.07 |
| 138 | A138V | 0.76 | 1.08 | 1.11 | 1.05 | 0.9 | 1.12 |
| 138 | A138W | 0.18 | ***0.05*** | 0.40 | 0.11 | ***0.05*** | ***0.05*** |
| 138 | A138Y | 0.17 | 5.98 | 9.05 | 3.30 | 0.6 | 0.51 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 139 | V139A | 0.69 | 1.06 | 1.06 | 0.97 | 0.8 | 0.94 |
| 139 | V139C | 1.19 | 1.03 | 1.09 | 0.95 | 1.0 | 1.30 |
| 139 | V139D | 0.23 | ***0.05*** | 0.07 | ***0.05*** | ***0.05*** | ***0.05*** |
| 139 | V139E | 0.23 | ***0.05*** | ***0.05*** | ***0.05*** | ***0.05*** | ***0.05*** |
| 139 | V139F | ***0.05*** | ***0.05*** | ***0.05*** | ***0.05*** | ***0.05*** | ***0.05*** |
| 139 | V139G | 0.21 | 0.40 | 0.60 | 0.65 | 0.5 | 0.25 |
| 139 | V139H | 0.25 | 0.75 | 0.74 | 0.73 | 0.8 | 0.06 |
| 139 | V139I | 0.85 | 0.84 | 0.73 | 1.02 | 1.0 | 0.43 |
| 139 | V139K | ***0.05*** | ***0.05*** | ***0.05*** | ***0.05*** | ***0.05*** | ***0.05*** |
| 139 | V139L | 0.75 | 0.98 | 0.84 | 1.09 | 0.8 | 0.28 |
| 139 | V139M | 0.61 | 0.97 | 0.67 | 1.01 | 0.6 | 0.49 |
| 139 | V139N | 0.47 | 0.83 | 0.76 | 1.14 | 0.9 | 1.20 |
| 139 | V139P | 0.39 | ***0.05*** | ***0.05*** | ***0.05*** | ***0.05*** | ***0.05*** |
| 139 | V139Q | 0.17 | ***0.05*** | 0.83 | 0.15 | ***0.05*** | ***0.05*** |
| 139 | V139R | 0.37 | ***0.05*** | ***0.05*** | ***0.05*** | ***0.05*** | ***0.05*** |
| 139 | V139S | 0.49 | 0.78 | 1.11 | 1.11 | 0.9 | 0.98 |
| 139 | V139T | 0.42 | 1.27 | 0.83 | 1.19 | 1.0 | 0.63 |
| 139 | V139W | 0.36 | ***0.05*** | ***0.05*** | ***0.05*** | ***0.05*** | ***0.05*** |
| 139 | V139Y | 0.31 | 0.17 | 0.26 | 0.25 | ***0.05*** | ***0.05*** |
| 140 | D140A | 0.45 | 0.70 | 0.72 | 0.81 | 0.2 | 0.61 |
| 140 | D140C | 0.56 | 0.90 | 1.02 | 0.89 | 0.8 | 0.88 |
| 140 | D140E | 1.02 | 1.20 | 0.98 | 0.92 | 0.9 | 1.01 |
| 140 | D140F | ***0.05*** | ***0.05*** | ***0.05*** | ***0.05*** | ***0.05*** | ***0.05*** |
| 140 | D140G | 0.52 | 0.80 | 0.79 | 0.85 | 0.7 | 0.86 |
| 140 | D140H | 0.50 | 0.47 | 0.53 | 0.67 | 0.7 | 0.72 |
| 140 | D140I | ***0.05*** | ***0.05*** | ***0.05*** | ***0.05*** | ***0.05*** | ***0.05*** |
| 140 | D140K | 0.48 | 0.25 | 0.53 | 0.47 | 0.8 | 0.85 |
| 140 | D140L | 0.42 | 0.54 | 0.78 | 0.84 | 0.1 | 0.97 |
| 140 | D140M | 0.48 | 0.77 | 0.81 | 0.82 | 0.3 | 0.95 |
| 140 | D140N | 0.88 | 0.86 | 0.84 | 0.83 | 0.9 | 0.97 |
| 140 | D140P | 0.30 | 0.06 | 0.09 | ***0.05*** | ***0.05*** | ***0.05*** |
| 140 | D140Q | 0.61 | 0.87 | 0.80 | 0.96 | 0.7 | 1.31 |
| 140 | D140R | 0.41 | 0.22 | 0.28 | 0.50 | 0.7 | 0.98 |
| 140 | D140S | 0.55 | 0.81 | 0.77 | 0.92 | 0.4 | 0.83 |
| 140 | D140T | 0.60 | 0.85 | 0.80 | 0.89 | 0.5 | 0.87 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 140 | D140V | 0.42 | 0.98 | 1.00 | 0.96 | 0.2 | 1.07 |
| 140 | D140W | 0.23 | 0.74 | 0.78 | 0.85 | 0.4 | 0.95 |
| 140 | D140Y | 0.50 | 0.79 | 0.74 | 0.83 | 0.6 | 1.05 |
| 141 | K141A | 0.87 | 0.87 | 1.11 | 0.93 | 1.0 | 1.06 |
| 141 | K141C | 0.78 | 0.73 | 1.11 | 1.04 | 1.0 | 1.11 |
| 141 | K141D | 0.82 | 0.95 | 1.23 | 1.04 | 1.0 | 1.14 |
| 141 | K141E | 1.18 | 0.80 | 1.10 | 1.00 | 1.0 | 0.97 |
| 141 | K141F | 0.70 | 0.84 | 1.27 | 1.04 | 1.0 | 1.13 |
| 141 | K141G | 0.94 | 0.84 | 1.15 | 1.07 | 1.1 | 1.09 |
| 141 | K141H | 1.00 | 1.03 | 1.09 | 1.04 | 1.2 | 1.08 |
| 141 | K141I | 0.60 | 0.94 | 1.23 | 1.00 | 1.1 | 1.25 |
| 141 | K141L | 0.66 | 0.87 | 1.29 | 1.01 | 1.1 | 1.14 |
| 141 | K141M | 0.79 | 1.00 | 1.17 | 1.02 | 1.0 | 1.24 |
| 141 | K141N | 1.05 | 0.90 | 1.30 | 1.03 | 1.1 | 1.17 |
| 141 | K141P | 0.28 | ***0.05*** | 0.09 | ***0.05*** | ***0.05*** | ***0.05*** |
| 141 | K141Q | 1.06 | 0.96 | 1.26 | 1.09 | 1.1 | 1.10 |
| 141 | K141R | 1.09 | 0.87 | 1.13 | 1.07 | 1.0 | 0.97 |
| 141 | K141S | 0.95 | 0.96 | 1.06 | 1.22 | 1.0 | 1.15 |
| 141 | K141T | ***0.05*** | ***0.05*** | ***0.05*** | ***0.05*** | ***0.05*** | ***0.05*** |
| 141 | K141V | 0.60 | 1.20 | 1.28 | 1.02 | 1.0 | 1.36 |
| 141 | K141W | 0.70 | 1.14 | 1.32 | 1.18 | 1.2 | 1.27 |
| 141 | K141Y | 0.91 | 1.16 | 1.07 | 1.06 | 1.1 | 1.16 |
| 142 | A142C | 0.79 | 1.03 | 0.92 | 0.89 | 1.1 | 1.17 |
| 142 | A142D | 0.35 | ***0.05*** | ***0.05*** | ***0.05*** | ***0.05*** | ***0.05*** |
| 142 | A142E | 0.34 | ***0.05*** | ***0.05*** | ***0.05*** | ***0.05*** | ***0.05*** |
| 142 | A142F | 0.28 | ***0.05*** | ***0.05*** | ***0.05*** | ***0.05*** | ***0.05*** |
| 142 | A142G | 0.46 | 1.32 | 1.20 | 0.96 | 1.0 | 1.18 |
| 142 | A142H | 0.25 | ***0.05*** | 0.10 | 0.06 | ***0.05*** | ***0.05*** |
| 142 | A142I | 0.25 | 1.60 | 1.39 | 1.46 | 0.6 | 0.81 |
| 142 | A142K | 0.37 | ***0.05*** | ***0.05*** | ***0.05*** | ***0.05*** | ***0.05*** |
| 142 | A142L | 0.29 | 1.24 | 1.40 | 1.19 | 0.6 | 0.70 |
| 142 | A142M | 0.22 | 1.21 | 1.62 | 1.31 | 0.6 | 0.38 |
| 142 | A142N | 0.31 | ***0.05*** | ***0.05*** | ***0.05*** | ***0.05*** | ***0.05*** |
| 142 | A142P | 0.29 | ***0.05*** | ***0.05*** | ***0.05*** | ***0.05*** | ***0.05*** |
| 142 | A142Q | 0.31 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 142 | A142R | 0.36 | ***0.05*** | ***0.05*** | ***0.05*** | ***0.05*** | ***0.05*** |
| 142 | A142S | 0.68 | 1.01 | 0.98 | 1.23 | 1.0 | 1.18 |
| 142 | A142T | 0.31 | 1.32 | 1.55 | 1.46 | 0.8 | 1.11 |
| 142 | A142V | 0.37 | 1.49 | 1.19 | 1.23 | 0.8 | 1.12 |
| 142 | A142W | 0.32 | ***0.05*** | ***0.05*** | ***0.05*** | ***0.05*** | ***0.05*** |
| 142 | A142Y | 0.29 | ***0.05*** | ***0.05*** | ***0.05*** | ***0.05*** | ***0.05*** |
| 143 | V143A | 1.05 | 0.81 | 1.04 | 0.90 | 0.9 | 0.88 |
| 143 | V143C | 0.87 | 1.00 | 1.14 | 0.89 | 1.0 | 1.08 |
| 143 | V143D | 0.95 | 0.95 | 1.03 | 0.97 | 1.0 | 1.05 |
| 143 | V143E | 1.12 | 0.87 | 1.05 | 1.02 | 1.0 | 1.08 |
| 143 | V143F | 0.88 | 0.76 | 1.00 | 0.81 | 0.9 | 0.81 |
| 143 | V143G | 0.71 | 0.90 | 1.16 | 1.15 | 0.9 | 1.11 |
| 143 | V143H | ***0.05*** | ***0.05*** | ***0.05*** | ***0.05*** | ***0.05*** | ***0.05*** |
| 143 | V143I | ***0.05*** | ***0.05*** | ***0.05*** | ***0.05*** | ***0.05*** | ***0.05*** |
| 143 | V143K | 0.84 | 0.61 | 0.72 | 0.96 | 1.1 | 1.00 |
| 143 | V143L | 0.78 | 0.78 | 1.13 | 0.83 | 0.9 | 1.11 |
| 143 | V143M | 0.83 | 0.94 | 1.05 | 1.11 | 0.9 | 0.99 |
| 143 | V143N | 0.91 | 0.90 | 1.00 | 1.20 | 1.0 | 1.13 |
| 143 | V143P | 0.20 | ***0.05*** | ***0.05*** | 5.50 | 0.7 | 0.12 |
| 143 | V143Q | 0.88 | 0.82 | 1.11 | 1.04 | 1.1 | 1.04 |
| 143 | V143R | 0.77 | 0.64 | 0.70 | 0.92 | 1.0 | 0.94 |
| 143 | V143S | 1.03 | 0.94 | 0.97 | 0.93 | 1.0 | 1.02 |
| 143 | V143T | 0.83 | 0.90 | 0.93 | 0.99 | 1.0 | 1.17 |
| 143 | V143W | 0.65 | 1.36 | 0.84 | 1.02 | 0.9 | 0.88 |
| 143 | V143Y | 0.21 | ***0.05*** | 0.51 | 0.21 | ***0.05*** | ***0.05*** |
| 144 | A144C | 1.17 | 0.98 | 0.85 | 0.88 | 1.0 | 1.02 |
| 144 | A144D | 1.29 | 1.18 | 1.11 | 0.99 | 1.0 | 0.96 |
| 144 | A144E | 1.17 | 1.08 | 0.76 | 1.00 | 1.0 | 1.13 |
| 144 | A144F | 0.96 | 1.20 | 0.73 | 0.94 | 1.0 | 1.16 |
| 144 | A144G | 1.09 | 1.08 | 0.98 | 1.02 | 1.0 | 0.99 |
| 144 | A144H | ***0.05*** | ***0.05*** | ***0.05*** | ***0.05*** | ***0.05*** | ***0.05*** |
| 144 | A144I | 0.97 | 1.29 | 0.79 | 0.97 | 1.1 | 1.06 |
| 144 | A144K | 1.28 | 1.00 | 0.57 | 0.77 | 1.1 | 0.90 |
| 144 | A144L | 1.11 | 1.18 | 0.79 | 1.08 | 1.0 | 1.03 |
| 144 | A144M | 1.06 | 1.08 | 0.84 | 1.07 | 1.0 | 1.05 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 144 | A144N | ***0.05*** | ***0.05*** | ***0.05*** | ***0.05*** | ***0.05*** | ***0.05*** |
| 144 | A144P | 0.18 | 1.75 | 2.60 | 1.24 | 0.4 | 0.30 |
| 144 | A144Q | 0.25 | ***0.05*** | 0.07 | ***0.05*** | ***0.05*** | ***0.05*** |
| 144 | A144R | 1.23 | 0.96 | 0.45 | 0.90 | 1.1 | 1.00 |
| 144 | A144S | 1.04 | 1.24 | 0.93 | 0.85 | 1.0 | 1.05 |
| 144 | A144T | 1.08 | 1.16 | 0.89 | 1.04 | 1.1 | 1.14 |
| 144 | A144V | 0.80 | 1.11 | 1.13 | 0.86 | 1.0 | 1.21 |
| 144 | A144W | 0.87 | 1.23 | 0.64 | 0.88 | 1.0 | 1.63 |
| 144 | A144Y | ***0.05*** | ***0.05*** | ***0.05*** | ***0.05*** | ***0.05*** | ***0.05*** |
| 145 | S145A | 1.09 | 0.97 | 1.13 | 0.94 | 1.0 | 0.99 |
| 145 | S145C | 1.10 | 0.72 | 1.03 | 1.00 | 1.1 | 1.03 |
| 145 | S 145D | 1.24 | 1.03 | 1.06 | 1.08 | 1.1 | 1.01 |
| 145 | S145E | 1.24 | 0.90 | 1.20 | 0.90 | 1.1 | 0.95 |
| 145 | S145F | 0.84 | 0.81 | 1.15 | 1.12 | 1.1 | 1.22 |
| 145 | S145G | 1.06 | 0.77 | 1.13 | 1.06 | 1.0 | 1.11 |
| 145 | S 145H | 1.21 | 0.93 | 1.04 | 1.05 | 1.1 | 1.02 |
| 145 | S145I | 0.79 | 0.42 | 1.24 | 1.03 | 1.1 | 1.24 |
| 145 | S 145K | ***0.05*** | ***0.05*** | ***0.05*** | ***0.05*** | ***0.05*** | ***0.05*** |
| 145 | S145L | 0.87 | 1.02 | 1.25 | 1.08 | 1.1 | 1.13 |
| 145 | S 145M | 1.00 | 0.86 | 1.06 | 1.04 | 1.0 | 1.08 |
| 145 | S 145N | ***0.05*** | ***0.05*** | ***0.05*** | ***0.05*** | ***0.05*** | ***0.05*** |
| 145 | S145P | 0.34 | ***0.05*** | 0.07 | ***0.05*** | ***0.05*** | ***0.05*** |
| 145 | S145Q | 1.10 | 0.74 | 1.18 | 1.08 | 1.0 | 1.06 |
| 145 | S 145R | 1.21 | 0.78 | 1.01 | 1.13 | 1.1 | 1.06 |
| 145 | S145T | 1.04 | 0.92 | 1.17 | 1.04 | 1.0 | 1.07 |
| 145 | S145V | 0.83 | 0.87 | 1.25 | 1.06 | 1.0 | 1.17 |
| 145 | S145W | 0.81 | 1.15 | 1.10 | 1.01 | 0.9 | 1.21 |
| 145 | S 145Y | 0.48 | 0.88 | 1.28 | 1.13 | 0.9 | 1.29 |
| 146 | G146A | 0.48 | 1.04 | 1.18 | 1.08 | 1.0 | 1.02 |
| 146 | G146C | 0.50 | 1.01 | 1.15 | 0.98 | 1.1 | 0.97 |
| 146 | G146D | 1.08 | 0.86 | 1.02 | 1.00 | 1.1 | 1.07 |
| 146 | G146E | 0.82 | 0.79 | 1.12 | 0.98 | 1.0 | 1.04 |
| 146 | G146F | 0.50 | 0.88 | 1.03 | 1.06 | 1.0 | 1.02 |
| 146 | G146H | 0.92 | 0.86 | 1.17 | 1.06 | 0.9 | 1.09 |
| 146 | G146I | 0.16 | 0.37 | ***0.05*** | 0.53 | 0.9 | 0.07 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 146 | G146K | 0.89 | 0.72 | 1.09 | 0.98 | 0.9 | 1.07 |
| 146 | G146L | 0.18 | 2.22 | 3.81 | 1.88 | 1.1 | 0.59 |
| 146 | G146M | 0.34 | 1.18 | 1.21 | 1.22 | 1.0 | 1.09 |
| 146 | G146N | ***0.05*** | ***0.05*** | ***0.05*** | ***0.05*** | ***0.05*** | ***0.05*** |
| 146 | G146P | 0.18 | 0.49 | 0.60 | 0.25 | 0.2 | 0.08 |
| 146 | G146Q | 0.85 | 0.95 | 1.16 | 1.05 | 1.0 | 1.45 |
| 146 | G146R | 0.81 | 0.79 | 1.08 | 1.04 | 0.9 | 1.02 |
| 146 | G146S | 0.76 | 0.92 | 1.11 | 0.91 | 0.9 | 1.06 |
| 146 | G146T | 0.25 | 1.52 | 1.80 | 1.57 | 0.7 | 1.12 |
| 146 | G146V | 0.13 | ***0.05*** | ***0.05*** | ***0.05*** | 0.7 | 0.16 |
| 146 | G146W | 0.13 | ***0.05*** | ***0.05*** | ***0.05*** | 0.9 | 0.49 |
| 146 | G146Y | 0.21 | 1.66 | 2.23 | 1.62 | 0.9 | 0.88 |
| 147 | V147A | 1.13 | 1.04 | 1.09 | 0.92 | 1.1 | 0.90 |
| 147 | V147C | 0.87 | 1.19 | 1.25 | 0.99 | 1.1 | 0.90 |
| 147 | V147D | 0.21 | 2.29 | 3.05 | 2.09 | 1.2 | 0.53 |
| 147 | V147E | 0.53 | 0.98 | 1.17 | 0.93 | 1.0 | 1.12 |
| 147 | V147G | 0.64 | 1.25 | 1.42 | 1.04 | 1.0 | 0.93 |
| 147 | V147H | 0.70 | 1.23 | 1.30 | 0.94 | 1.2 | 0.93 |
| 147 | V147I | 0.98 | 1.25 | 0.94 | 0.94 | 1.1 | 1.04 |
| 147 | V147L | 0.70 | 1.16 | 1.29 | 1.15 | 1.0 | 0.97 |
| 147 | V147M | 0.97 | 1.00 | 1.07 | 0.90 | 1.0 | 1.14 |
| 147 | V147P | 0.31 | 1.71 | 2.12 | 1.52 | 0.8 | 1.13 |
| 147 | V147Q | 0.84 | 1.23 | 1.09 | 1.03 | 1.2 | 1.01 |
| 147 | V147R | 0.71 | 1.16 | 1.07 | 0.88 | 1.0 | 0.96 |
| 147 | V147S | 0.87 | 1.06 | 1.22 | 0.87 | 1.0 | 0.98 |
| 147 | V147T | 0.80 | 1.24 | 1.06 | 1.02 | 0.9 | 1.16 |
| 147 | V147W | 0.25 | 1.97 | 2.16 | 1.32 | 0.9 | 0.93 |
| 147 | V147Y | 0.26 | 2.34 | 2.31 | 1.50 | 0.9 | 0.78 |
| 148 | V148A | 0.56 | 1.06 | 0.76 | 1.03 | 0.8 | 0.74 |
| 148 | V148D | 0.29 | 0.07 | 0.29 | 0.21 | ***0.05*** | ***0.05*** |
| 148 | V148E | 0.21 | 0.62 | 0.99 | 0.67 | 0.9 | 0.14 |
| 148 | V148F | 0.73 | 0.96 | 1.18 | 1.06 | 0.9 | 0.85 |
| 148 | V148G | 0.23 | 0.70 | 1.12 | 0.98 | 0.9 | 0.26 |
| 148 | V148H | 0.30 | 1.07 | 1.68 | 1.39 | 0.8 | 0.74 |
| 148 | V148I | 0.70 | 0.98 | 1.20 | 1.02 | 1.1 | 0.91 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 148 | V148K | 0.31 | ***0.05*** | 0.09 | ***0.05*** | ***0.05*** | ***0.05*** |
| 148 | V148L | 0.98 | 1.05 | 1.11 | 1.07 | 1.1 | 0.80 |
| 148 | V148M | 0.64 | 0.94 | 1.06 | 1.01 | 0.8 | 0.74 |
| 148 | V148N | 0.41 | 1.38 | 1.35 | 1.11 | 0.8 | 1.11 |
| 148 | V148P | 0.27 | 0.61 | 0.87 | 0.76 | 1.0 | 0.21 |
| 148 | V148Q | 0.25 | 1.08 | 1.73 | 1.58 | 1.0 | 0.82 |
| 148 | V148R | 0.47 | ***0.05*** | ***0.05*** | ***0.05*** | ***0.05*** | ***0.05*** |
| 148 | V148S | 0.52 | 1.09 | 1.20 | 1.10 | 1.0 | 0.90 |
| 148 | V148T | 0.55 | 1.05 | 1.41 | 1.13 | 1.0 | 0.99 |
| 148 | V148Y | 0.22 | 1.60 | 1.45 | 1.60 | 0.6 | 0.37 |
| 149 | V149A | 0.80 | 0.96 | 1.10 | 0.99 | 1.0 | 0.99 |
| 149 | V149C | 0.86 | 1.11 | 0.96 | 0.99 | 1.2 | 0.89 |
| 149 | V149D | 0.22 | 0.83 | 1.23 | 1.11 | 1.3 | 0.32 |
| 149 | V149E | 0.18 | 2.65 | 3.71 | 2.50 | 1.0 | 0.40 |
| 149 | V149F | 0.92 | 1.15 | 1.31 | 1.07 | 1.0 | 0.23 |
| 149 | V149G | 0.24 | 0.90 | 0.72 | 1.05 | 1.0 | 0.28 |
| 149 | V149H | 0.25 | 0.54 | 0.75 | 0.70 | 1.2 | 0.26 |
| 149 | V149I | 0.84 | 0.96 | 1.08 | 0.85 | 1.1 | 0.80 |
| 149 | V149L | 0.69 | 1.02 | 1.11 | 1.11 | 1.1 | 0.98 |
| 149 | V149M | 0.82 | 1.09 | 1.11 | 1.07 | 1.1 | 0.82 |
| 149 | V149P | 0.46 | 0.94 | 1.23 | 1.04 | 1.2 | 1.25 |
| 149 | V149R | 0.50 | ***0.05*** | ***0.05*** | ***0.05*** | ***0.05*** | ***0.05*** |
| 149 | V149S | 0.37 | 1.12 | 1.42 | 1.31 | 1.1 | 1.18 |
| 149 | V149T | 0.66 | 0.88 | 1.16 | 1.09 | 1.1 | 1.11 |
| 149 | V149W | 0.25 | 0.44 | 0.50 | 0.59 | 1.3 | 0.12 |
| 149 | V149Y | 0.33 | 1.18 | 1.52 | 1.33 | 1.0 | 0.18 |
| 150 | V150A | 0.54 | 0.85 | 1.21 | 1.04 | 0.9 | 0.67 |
| 150 | V 150C | 0.80 | 0.86 | 1.07 | 1.06 | 1.0 | 0.80 |
| 150 | V150E | 0.45 | ***0.05*** | 0.06 | ***0.05*** | ***0.05*** | ***0.05*** |
| 150 | V150F | 0.47 | 0.89 | 1.07 | 1.07 | 0.8 | 0.86 |
| 150 | V150G | 0.30 | ***0.05*** | 0.15 | 0.18 | ***0.05*** | ***0.05*** |
| 150 | V150H | 0.37 | ***0.05*** | ***0.05*** | ***0.05*** | ***0.05*** | ***0.05*** |
| 150 | V150I | 0.83 | 1.14 | 1.05 | 1.07 | 1.0 | 1.15 |
| 150 | V150K | 0.31 | 0.11 | ***0.05*** | 0.06 | ***0.05*** | ***0.05*** |
| 150 | V150L | 0.75 | 0.97 | 0.96 | 1.02 | 0.9 | 0.95 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 150 | V150M | 0.39 | 0.97 | 1.26 | 1.27 | 0.9 | 0.77 |
| 150 | V150N | 0.28 | 0.27 | 0.26 | 0.21 | 1.1 | 0.06 |
| 150 | V150P | 0.43 | ***0.05*** | ***0.05*** | ***0.05*** | ***0.05*** | ***0.05*** |
| 150 | V150Q | 0.31 | 1.25 | 1.67 | 1.46 | 1.0 | 1.05 |
| 150 | V150R | 0.48 | ***0.05*** | ***0.05*** | ***0.05*** | ***0.05*** | ***0.05*** |
| 150 | V150S | 0.33 | 1.08 | 1.22 | 1.17 | 0.8 | 0.52 |
| 150 | V150T | 0.61 | 0.71 | 1.28 | 1.09 | 1.0 | 0.72 |
| 150 | V150W | 0.43 | 0.06 | ***0.05*** | ***0.05*** | ***0.05*** | ***0.05*** |
| 150 | V150Y | 0.32 | ***0.05*** | ***0.05*** | ***0.05*** | ***0.05*** | ***0.05*** |
| 151 | A151C | 0.74 | 1.20 | 0.94 | 0.98 | 0.9 | 0.91 |
| 151 | A151D | 0.50 | ***0.05*** | 0.11 | ***0.05*** | ***0.05*** | ***0.05*** |
| 151 | A151E | 0.47 | ***0.05*** | ***0.05*** | ***0.05*** | ***0.05*** | ***0.05*** |
| 151 | A151F | 0.51 | ***0.05*** | ***0.05*** | ***0.05*** | ***0.05*** | ***0.05*** |
| 151 | A151G | 0.49 | 0.85 | 1.24 | 1.16 | 0.8 | 0.74 |
| 151 | A151I | 0.21 | 0.25 | 0.54 | 0.19 | ***0.05*** | ***0.05*** |
| 151 | A151K | 0.49 | ***0.05*** | ***0.05*** | ***0.05*** | ***0.05*** | ***0.05*** |
| 151 | A151L | 0.47 | ***0.05*** | ***0.05*** | ***0.05*** | ***0.05*** | ***0.05*** |
| 151 | A151M | 0.24 | 1.15 | 1.42 | 1.13 | 1.0 | 0.32 |
| 151 | A151N | 0.21 | 0.93 | 1.88 | 1.11 | 0.7 | 0.13 |
| 151 | A151P | 0.47 | ***0.05*** | ***0.05*** | ***0.05*** | ***0.05*** | ***0.05*** |
| 151 | A151Q | 0.35 | 0.20 | 0.20 | 0.21 | ***0.05*** | ***0.05*** |
| 151 | A151R | 0.48 | ***0.05*** | ***0.05*** | ***0.05*** | ***0.05*** | ***0.05*** |
| 151 | A151S | 0.79 | 0.96 | 1.26 | 1.08 | 1.0 | 0.51 |
| 151 | A151T | 0.46 | 1.36 | 1.20 | 1.18 | 0.6 | 0.56 |
| 151 | A151V | 0.38 | 0.95 | 1.08 | 1.45 | 0.6 | 0.61 |
| 151 | A151W | 0.56 | ***0.05*** | ***0.05*** | ***0.05*** | ***0.05*** | ***0.05*** |
| 152 | A152C | 1.40 | 0.76 | 0.77 | 0.94 | ***0.05*** | ***0.05*** |
| 152 | A152E | 1.63 | ***0.05*** | ***0.05*** | ***0.05*** | ***0.05*** | ***0.05*** |
| 152 | A152F | 0.38 | ***0.05*** | ***0.05*** | ***0.05*** | ***0.05*** | ***0.05*** |
| 152 | A152H | 0.46 | ***0.05*** | ***0.05*** | ***0.05*** | ***0.05*** | ***0.05*** |
| 152 | A152K | 0.64 | ***0.05*** | ***0.05*** | ***0.05*** | ***0.05*** | ***0.05*** |
| 152 | A152L | 0.46 | ***0.05*** | ***0.05*** | ***0.05*** | ***0.05*** | ***0.05*** |
| 152 | A152M | 1.47 | ***0.05*** | ***0.05*** | ***0.05*** | ***0.05*** | ***0.05*** |
| 152 | A152N | 0.67 | 0.11 | ***0.05*** | 0.14 | ***0.05*** | ***0.05*** |
| 152 | A152P | 2.05 | 0.91 | 0.91 | 0.95 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 152 | A152Q | 1.99 | ***0.05*** | ***0.05*** | ***0.05*** | ***0.05*** | ***0.05*** |
| 152 | A152S | 1.11 | 0.74 | 1.00 | 0.85 | 0.9 | 0.32 |
| 152 | A152T | 0.75 | 0.80 | 0.81 | 1.05 | ***0.05*** | ***0.05*** |
| 152 | A152V | 0.44 | 1.24 | 1.09 | 1.35 | ***0.05*** | ***0.05*** |
| 152 | A152W | 0.37 | ***0.05*** | ***0.05*** | ***0.05*** | ***0.05*** | ***0.05*** |
| 152 | A152Y | 0.45 | ***0.05*** | ***0.05*** | ***0.05*** | ***0.05*** | ***0.05*** |
| 153 | A153C | 0.54 | 1.02 | 1.03 | 0.96 | 0.5 | 0.67 |
| 153 | A153D | 0.20 | ***0.05*** | 0.16 | 0.06 | ***0.05*** | ***0.05*** |
| 153 | A153E | 0.36 | ***0.05*** | ***0.05*** | ***0.05*** | ***0.05*** | ***0.05*** |
| 153 | A153F | 0.50 | ***0.05*** | ***0.05*** | ***0.05*** | ***0.05*** | ***0.05*** |
| 153 | A153G | 0.51 | 1.03 | 1.06 | 1.03 | 0.5 | 0.64 |
| 153 | A153H | 0.35 | ***0.05*** | ***0.05*** | ***0.05*** | ***0.05*** | ***0.05*** |
| 153 | A153I | 0.14 | 0.56 | ***0.05*** | ***0.05*** | ***0.05*** | ***0.05*** |
| 153 | A153K | 0.36 | ***0.05*** | ***0.05*** | ***0.05*** | ***0.05*** | ***0.05*** |
| 153 | A153L | 0.22 | ***0.05*** | 0.10 | ***0.05*** | ***0.05*** | ***0.05*** |
| 153 | A153M | ***0.05*** | ***0.05*** | ***0.05*** | ***0.05*** | ***0.05*** | ***0.05*** |
| 153 | A153N | 0.22 | ***0.05*** | 0.11 | ***0.05*** | ***0.05*** | ***0.05*** |
| 153 | A153P | 0.20 | ***0.05*** | 0.27 | 0.07 | ***0.05*** | ***0.05*** |
| 153 | A153Q | 0.20 | ***0.05*** | 0.24 | 0.22 | 0.3 | 0.08 |
| 153 | A153R | 0.31 | ***0.05*** | ***0.05*** | ***0.05*** | ***0.05*** | ***0.05*** |
| 153 | A153S | 0.65 | 1.05 | 1.03 | 1.04 | 0.6 | 0.62 |
| 153 | A153T | 0.17 | 4.36 | 4.31 | 3.03 | ***0.05*** | 0.29 |
| 153 | A153V | 0.28 | 1.34 | 1.24 | 1.39 | 0.3 | 0.88 |
| 153 | A153W | 0.36 | ***0.05*** | 0.10 | ***0.05*** | ***0.05*** | ***0.05*** |
| 153 | A153Y | 0.26 | ***0.05*** | ***0.05*** | ***0.05*** | ***0.05*** | ***0.05*** |
| 154 | G154A | 0.43 | ***0.05*** | ***0.05*** | ***0.05*** | ***0.05*** | ***0.05*** |
| 154 | G154C | ***0.05*** | ***0.05*** | ***0.05*** | ***0.05*** | ***0.05*** | ***0.05*** |
| 154 | G154D | 0.29 | 0.49 | 0.43 | 0.45 | ***0.05*** | ***0.05*** |
| 154 | G154E | 0.47 | ***0.05*** | 0.09 | 0.07 | ***0.05*** | ***0.05*** |
| 154 | G154F | 0.37 | ***0.05*** | ***0.05*** | ***0.05*** | ***0.05*** | ***0.05*** |
| 154 | G154H | 0.25 | 0.21 | 0.35 | 0.56 | ***0.05*** | ***0.05*** |
| 154 | G154I | 0.43 | ***0.05*** | ***0.05*** | ***0.05*** | ***0.05*** | ***0.05*** |
| 154 | G154K | 0.35 | ***0.05*** | ***0.05*** | 0.07 | ***0.05*** | ***0.05*** |
| 154 | G154L | 0.28 | 0.38 | 0.51 | 0.49 | ***0.05*** | ***0.05*** |
| 154 | G154M | 0.27 | 0.24 | 0.66 | 0.49 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 154 | G154N | 0.21 | 0.55 | 0.54 | 0.59 | ***0.05*** | ***0.05*** |
| 154 | G154P | 0.37 | ***0.05*** | ***0.05*** | ***0.05*** | ***0.05*** | ***0.05*** |
| 154 | G154Q | 0.25 | 0.34 | 0.38 | 0.56 | ***0.05*** | ***0.05*** |
| 154 | G154R | 0.24 | ***0.05*** | ***0.05*** | 0.10 | ***0.05*** | ***0.05*** |
| 154 | G154S | 0.22 | 0.58 | 0.78 | 0.76 | 0.2 | 0.06 |
| 154 | G154T | 0.26 | 0.48 | 0.65 | 0.93 | ***0.05*** | ***0.05*** |
| 154 | G154V | 0.28 | ***0.05*** | 0.07 | 0.18 | ***0.05*** | ***0.05*** |
| 154 | G154W | 0.28 | ***0.05*** | ***0.05*** | 0.11 | ***0.05*** | ***0.05*** |
| 154 | G154Y | ***0.05*** | ***0.05*** | ***0.05*** | ***0.05*** | ***0.05*** | ***0.05*** |
| 155 | N155A | 1.36 | 0.08 | ***0.05*** | 0.13 | ***0.05*** | ***0.05*** |
| 155 | N155C | 0.94 | ***0.05*** | ***0.05*** | 0.06 | ***0.05*** | ***0.05*** |
| 155 | N155D | 2.13 | ***0.05*** | ***0.05*** | ***0.05*** | ***0.05*** | ***0.05*** |
| 155 | N155F | 0.86 | 0.07 | ***0.05*** | 0.13 | ***0.05*** | ***0.05*** |
| 155 | N155G | 1.90 | 0.25 | ***0.05*** | 0.49 | ***0.05*** | ***0.05*** |
| 155 | N155H | 1.16 | 0.14 | 0.09 | 0.22 | ***0.05*** | ***0.05*** |
| 155 | N155I | 0.64 | ***0.05*** | ***0.05*** | ***0.05*** | ***0.05*** | ***0.05*** |
| 155 | N155K | 0.73 | 0.09 | 0.08 | 0.18 | ***0.05*** | ***0.05*** |
| 155 | N155L | 0.61 | ***0.05*** | ***0.05*** | 0.08 | ***0.05*** | ***0.05*** |
| 155 | N155M | 0.75 | ***0.05*** | ***0.05*** | ***0.05*** | ***0.05*** | ***0.05*** |
| 155 | N155Q | 0.94 | ***0.05*** | ***0.05*** | 0.06 | ***0.05*** | ***0.05*** |
| 155 | N155R | 0.58 | 0.08 | 0.06 | 0.16 | ***0.05*** | ***0.05*** |
| 155 | N155S | 1.50 | 0.10 | 0.07 | 0.21 | ***0.05*** | ***0.05*** |
| 155 | N155T | 0.81 | ***0.05*** | ***0.05*** | 0.10 | ***0.05*** | ***0.05*** |
| 155 | N155V | 0.69 | ***0.05*** | ***0.05*** | ***0.05*** | ***0.05*** | ***0.05*** |
| 155 | N155W | 0.70 | ***0.05*** | ***0.05*** | 0.11 | ***0.05*** | ***0.05*** |
| 155 | N155Y | 1.10 | 0.11 | ***0.05*** | 0.17 | ***0.05*** | ***0.05*** |
| 156 | E156A | 1.12 | 0.69 | 0.60 | 0.84 | 1.0 | 0.87 |
| 156 | E156C | 1.10 | 0.61 | 0.70 | 0.83 | 1.0 | 0.85 |
| 156 | E156F | 1.11 | 0.41 | 0.57 | 0.89 | 1.0 | 1.12 |
| 156 | E156I | 0.43 | 0.59 | 0.64 | 0.86 | 0.5 | 0.86 |
| 156 | E156K | 0.99 | 0.42 | 0.41 | 0.78 | 0.5 | 1.30 |
| 156 | E156L | 0.82 | 0.55 | 0.60 | 0.83 | 0.8 | 1.31 |
| 156 | E156M | 1.10 | 0.46 | 0.57 | 0.86 | 0.9 | 1.31 |
| 156 | E156N | 1.17 | 0.38 | 0.48 | 0.72 | 1.0 | 0.91 |
| 156 | E156P | 0.26 | ***0.05*** | ***0.05*** | 0.16 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 156 | E156Q | 0.93 | 0.51 | 0.51 | 0.75 | 0.9 | 0.94 |
| 156 | E156R | 1.10 | 0.23 | 0.41 | 0.66 | 0.4 | 1.35 |
| 156 | E156S | 0.93 | 0.62 | 0.52 | 0.91 | 0.8 | 1.01 |
| 156 | E156T | 0.72 | 0.73 | 0.65 | 1.00 | 0.7 | 1.44 |
| 156 | E156V | 0.54 | 0.60 | 0.60 | 0.98 | 0.6 | 1.13 |
| 156 | E156W | 0.79 | 0.24 | 0.21 | 0.60 | 0.7 | 0.79 |
| 156 | E156Y | 1.10 | 0.35 | 0.43 | 0.72 | 0.9 | 1.09 |
| 157 | G157A | 0.48 | 0.83 | 0.87 | 0.92 | 0.6 | 0.62 |
| 157 | G157C | 0.60 | 0.63 | 0.79 | 0.95 | 0.4 | 0.18 |
| 157 | G157D | 0.49 | 0.68 | 0.87 | 0.79 | 0.4 | 0.20 |
| 157 | G157E | 0.58 | 0.62 | 0.82 | 0.69 | 0.2 | 0.12 |
| 157 | G157F | 0.34 | 0.38 | 0.60 | 0.68 | 0.2 | 0.19 |
| 157 | G157H | ***0.05*** | ***0.05*** | ***0.05*** | ***0.05*** | ***0.05*** | ***0.05*** |
| 157 | G157I | 0.34 | 0.34 | 0.53 | 0.54 | 0.1 | 0.06 |
| 157 | G157K | 0.33 | 0.33 | 0.68 | 0.62 | ***0.05*** | 0.21 |
| 157 | G157L | 0.40 | 0.33 | 0.54 | 0.60 | 0.1 | 0.06 |
| 157 | G157M | 0.43 | 0.58 | 0.72 | 0.66 | 0.2 | 0.11 |
| 157 | G157N | ***0.05*** | ***0.05*** | ***0.05*** | ***0.05*** | ***0.05*** | ***0.05*** |
| 157 | G157P | 0.45 | 0.32 | 0.42 | 0.58 | 0.1 | 0.09 |
| 157 | G157Q | 0.41 | 0.55 | 0.80 | 0.71 | 0.2 | 0.13 |
| 157 | G157R | 0.31 | 0.28 | 0.52 | 0.43 | 0.1 | 0.15 |
| 157 | G157S | 0.53 | 1.03 | 0.96 | 0.88 | 0.8 | 0.72 |
| 157 | G157T | 0.37 | 0.51 | 0.81 | 0.97 | 0.2 | 0.12 |
| 157 | G157V | 0.33 | 0.43 | 0.74 | 0.63 | 0.1 | 0.09 |
| 157 | G157W | 0.40 | 0.59 | 0.70 | 0.88 | 0.2 | 0.18 |
| 157 | G157Y | 0.29 | 0.74 | 0.80 | 0.80 | 0.3 | 0.27 |
| 158 | T158A | 0.94 | 0.98 | 0.98 | 0.92 | 0.8 | 1.10 |
| 158 | T158C | ***0.05*** | ***0.05*** | ***0.05*** | ***0.05*** | ***0.05*** | ***0.05*** |
| 158 | T158D | 0.68 | 1.09 | 1.09 | 1.04 | 1.1 | 0.94 |
| 158 | T158E | 1.10 | 1.09 | 1.03 | 0.98 | 1.2 | 0.95 |
| 158 | T158F | ***0.05*** | ***0.05*** | ***0.05*** | ***0.05*** | ***0.05*** | ***0.05*** |
| 158 | T158G | 0.77 | 1.11 | 0.47 | 1.06 | 0.8 | 0.75 |
| 158 | T158H | 0.85 | 1.04 | 0.95 | 0.92 | 0.9 | 0.87 |
| 158 | T158I | 1.02 | 1.08 | 0.75 | 0.91 | 1.0 | 0.96 |
| 158 | T158K | 0.83 | 1.05 | 0.53 | 0.85 | 0.8 | 0.97 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 158 | T158L | 0.71 | 1.06 | 0.65 | 0.83 | 0.8 | 0.73 |
| 158 | T158M | 0.80 | 0.97 | 0.71 | 0.97 | 0.7 | 0.71 |
| 158 | T158N | 0.79 | 1.16 | 0.69 | 0.85 | 1.0 | 0.80 |
| 158 | T158P | 0.58 | 1.12 | 0.88 | 0.86 | 0.7 | 0.66 |
| 158 | T158Q | 0.82 | 1.04 | 0.89 | 0.81 | 1.0 | 0.81 |
| 158 | T158R | 0.56 | 0.84 | 0.54 | 0.83 | 0.5 | 1.02 |
| 158 | T158S | 1.31 | 1.09 | 1.04 | 0.93 | 0.9 | 0.94 |
| 158 | T158V | 0.93 | 1.20 | 0.83 | 0.86 | 0.9 | 0.95 |
| 158 | T158W | 0.61 | 1.01 | 0.75 | 0.89 | 0.6 | 0.82 |
| 158 | T158Y | 0.75 | 1.01 | 0.53 | 1.04 | 0.7 | 0.84 |
| 159 | S159A | 0.92 | 0.93 | 0.90 | 1.07 | 0.7 | 0.78 |
| 159 | S159C | 1.16 | 1.08 | 1.18 | 0.87 | 1.1 | 0.93 |
| 159 | S159D | 1.41 | 1.26 | 1.15 | 1.05 | 1.1 | 0.92 |
| 159 | S159E | 1.07 | 1.04 | 1.13 | 1.07 | 1.0 | 0.90 |
| 159 | S159F | 0.73 | 1.14 | 0.93 | 1.06 | 0.4 | 0.74 |
| 159 | S159G | 0.88 | 1.14 | 1.13 | 0.91 | 0.6 | 0.86 |
| 159 | S159H | 1.10 | 0.95 | 0.85 | 0.88 | 1.0 | 1.05 |
| 159 | S159I | 0.90 | 1.03 | 1.02 | 0.92 | 0.4 | 0.63 |
| 159 | S159K | 1.24 | 1.37 | 0.92 | 1.00 | 1.0 | 1.08 |
| 159 | S159L | 0.77 | 1.09 | 1.15 | 1.25 | 0.4 | 0.77 |
| 159 | S159M | 0.81 | 1.02 | 1.22 | 0.92 | 0.5 | 0.70 |
| 159 | S159N | ***0.05*** | ***0.05*** | ***0.05*** | ***0.05*** | ***0.05*** | ***0.05*** |
| 159 | S159P | 0.72 | 1.20 | 0.94 | 1.05 | 0.3 | 0.62 |
| 159 | S159Q | 1.10 | 1.06 | 1.16 | 1.11 | 1.1 | 1.08 |
| 159 | S159R | 1.09 | 0.78 | 0.87 | 0.93 | 0.9 | 1.07 |
| 159 | S159T | 0.65 | 1.02 | 1.05 | 1.03 | 0.8 | 0.96 |
| 159 | S159V | 0.70 | 1.14 | 1.14 | 1.05 | 0.5 | 0.66 |
| 159 | S159W | 0.43 | 0.93 | 1.04 | 1.06 | 0.2 | 0.54 |
| 159 | S159Y | 0.37 | 1.07 | 1.11 | 1.27 | 0.5 | 0.90 |
| 160 | G160A | 0.51 | 0.83 | 0.96 | 0.81 | 0.6 | 0.63 |
| 160 | G160C | 0.85 | 0.75 | 1.06 | 0.83 | 0.9 | 0.86 |
| 160 | G160D | 1.17 | 0.71 | 1.28 | 0.88 | 1.0 | 0.90 |
| 160 | G160E | 1.05 | 0.85 | 1.31 | 0.92 | 1.0 | 0.92 |
| 160 | G160F | ***0.05*** | ***0.05*** | ***0.05*** | ***0.05*** | ***0.05*** | ***0.05*** |
| 160 | G160H | ***0.05*** | ***0.05*** | ***0.05*** | ***0.05*** | ***0.05*** | ***0.05*** |

(continued)

Table 6-1. Performance Index Values for BPN' Variants

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 160 | G160I | ***0.05*** | ***0.05*** | ***0.05*** | ***0.05*** | ***0.05*** | ***0.05*** |
| 160 | G160K | 0.79 | 0.75 | 0.79 | 0.88 | 0.4 | 0.89 |
| 160 | G160L | 0.36 | 0.92 | 1.00 | 1.00 | 0.5 | 0.52 |
| 160 | G160M | 0.41 | 0.70 | 0.86 | 0.87 | 0.4 | 0.46 |
| 160 | G160N | 0.92 | 1.05 | 1.21 | 0.93 | 0.9 | 1.00 |
| 160 | G160P | 0.51 | 0.82 | 1.15 | 0.69 | 0.7 | 0.63 |
| 160 | G160Q | 0.75 | 0.72 | 1.15 | 0.92 | 0.8 | 0.83 |
| 160 | G160R | 0.54 | 0.69 | 0.83 | 0.87 | 0.3 | 0.78 |
| 160 | G160S | 0.80 | 0.82 | 1.12 | 0.89 | 0.7 | 0.84 |
| 160 | G160T | 0.71 | 0.70 | 1.21 | 1.08 | 0.6 | 0.93 |
| 160 | G160V | 0.48 | 1.12 | 1.25 | 0.88 | 0.5 | 0.60 |
| 160 | G160W | 0.37 | 0.93 | 0.90 | 0.99 | 0.3 | 0.61 |
| 160 | G160Y | 0.46 | 0.90 | 1.11 | 1.06 | 0.4 | 0.61 |
| 161 | S161A | 1.08 | 1.15 | 1.21 | 0.95 | 0.8 | 1.00 |
| 161 | S161C | 1.31 | 0.93 | 1.12 | 0.82 | 1.2 | 1.04 |
| 161 | S161D | ***0.05*** | ***0.05*** | ***0.05*** | ***0.05*** | ***0.05*** | ***0.05*** |
| 161 | S161E | 1.35 | 1.12 | 1.21 | 1.00 | 1.2 | 0.92 |
| 161 | S161F | 0.58 | 0.96 | 1.18 | 1.02 | 0.9 | 1.17 |
| 161 | S161G | 1.37 | 1.13 | 1.14 | 0.96 | 1.1 | 0.93 |
| 161 | S161H | ***0.05*** | ***0.05*** | ***0.05*** | ***0.05*** | ***0.05*** | ***0.05*** |
| 161 | S161I | 1.01 | 1.18 | 1.10 | 0.94 | 0.9 | 0.86 |
| 161 | S161K | 0.97 | 0.88 | 1.00 | 1.02 | 0.9 | 1.04 |
| 161 | S161L | 1.12 | 1.25 | 0.96 | 0.87 | 1.0 | 0.96 |
| 161 | S161M | 1.07 | 1.08 | 1.03 | 0.99 | 1.0 | 0.95 |
| 161 | S161N | ***0.05*** | ***0.05*** | ***0.05*** | ***0.05*** | ***0.05*** | ***0.05*** |
| 161 | S161P | 1.30 | 1.26 | 1.19 | 1.07 | 1.0 | 1.04 |
| 161 | S161Q | 1.10 | 1.16 | 1.13 | 0.98 | 1.1 | 1.15 |
| 161 | S161R | 0.84 | 0.94 | 0.84 | 0.88 | 0.8 | 1.16 |
| 161 | S161T | 1.13 | 1.09 | 1.21 | 1.00 | 1.0 | 1.41 |
| 161 | S161V | 0.93 | 1.29 | 1.07 | 1.05 | 0.9 | 1.10 |
| 161 | S161W | 0.43 | 0.96 | 1.12 | 1.05 | 0.7 | 0.83 |
| 161 | S161Y | 0.70 | 1.05 | 1.05 | 0.95 | 0.8 | 0.99 |
| 162 | S162A | 0.75 | 1.09 | 1.00 | 0.96 | 1.0 | 0.92 |
| 162 | S162C | 1.24 | 0.94 | 0.95 | 0.89 | 1.1 | 0.93 |
| 162 | S162D | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 162 | S162E | 1.41 | 1.16 | 0.93 | 0.89 | 1.1 | 0.99 |
| 162 | S162F | 0.45 | 0.94 | 0.96 | 0.82 | 0.9 | 0.77 |
| 162 | S162G | 0.76 | 1.16 | 0.94 | 0.91 | 1.0 | 1.03 |
| 162 | S162H | 1.29 | 0.95 | 0.71 | 1.02 | 1.0 | 1.02 |
| 162 | S162I | 0.53 | 1.06 | 0.84 | 0.89 | 1.0 | 0.98 |
| 162 | S162K | 1.38 | 1.05 | 0.81 | 0.94 | 1.0 | 1.09 |
| 162 | S162L | 0.66 | 1.25 | 0.55 | 0.91 | 1.0 | 0.84 |
| 162 | S162M | 0.73 | 1.09 | 0.64 | 1.03 | 0.9 | 1.00 |
| 162 | S162N | 1.39 | 0.89 | 0.87 | 1.00 | 1.1 | 1.09 |
| 162 | S162P | 0.56 | 1.06 | 0.87 | 0.89 | 0.7 | 0.66 |
| 162 | S162Q | 1.28 | 1.06 | 1.06 | 0.88 | 1.1 | 1.07 |
| 162 | S162R | 0.90 | 0.80 | 0.69 | 0.89 | 0.9 | 1.26 |
| 162 | S162T | 0.89 | 1.04 | 0.69 | 0.76 | 0.9 | 0.98 |
| 162 | S162V | 0.61 | 1.13 | 0.81 | 0.92 | 0.9 | 1.03 |
| 162 | S162W | 0.50 | 1.03 | 0.55 | 0.68 | 0.7 | 0.71 |
| 162 | S162Y | ***0.05*** | ***0.05*** | ***0.05*** | ***0.05*** | ***0.05*** | ***0.05*** |
| 163 | S163A | 0.32 | 0.71 | 0.78 | 0.89 | 0.2 | 0.30 |
| 163 | S163C | 0.35 | 0.88 | 0.55 | 0.85 | 0.2 | 0.26 |
| 163 | S163D | 0.56 | 0.92 | 0.90 | 0.86 | 0.1 | 0.45 |
| 163 | S163E | 0.38 | 0.93 | 0.84 | 0.83 | ***0.05*** | 0.33 |
| 163 | S163F | 0.29 | 0.77 | 0.70 | 0.68 | ***0.05*** | 0.25 |
| 163 | S163G | 1.10 | 1.02 | 0.86 | 0.93 | 0.6 | 0.85 |
| 163 | S163H | 0.25 | 0.97 | 0.70 | 0.98 | 0.1 | 0.29 |
| 163 | S163I | 0.31 | 0.73 | 0.72 | 0.93 | 0.1 | 0.20 |
| 163 | S163K | 0.28 | 0.73 | 0.79 | 0.81 | ***0.05*** | 0.30 |
| 163 | S163L | 0.34 | 0.76 | 0.53 | 0.79 | ***0.05*** | 0.36 |
| 163 | S163M | 0.33 | 0.71 | 0.71 | 0.76 | ***0.05*** | 0.28 |
| 163 | S163N | 0.34 | 0.81 | 0.91 | 0.71 | ***0.05*** | 0.30 |
| 163 | S163P | 0.99 | 1.05 | 0.78 | 0.95 | 0.4 | 0.63 |
| 163 | S163Q | ***0.05*** | ***0.05*** | ***0.05*** | ***0.05*** | ***0.05*** | ***0.05*** |
| 163 | S163R | 0.27 | 0.67 | 0.55 | 0.77 | 0.1 | 0.24 |
| 163 | S163T | 0.45 | 0.95 | 1.01 | 1.06 | 0.7 | 0.60 |
| 163 | S163V | 0.27 | 1.02 | 1.00 | 0.97 | ***0.05*** | 0.26 |
| 163 | S163W | 0.21 | 1.05 | 0.96 | 0.95 | 0.1 | 0.20 |
| 163 | S163Y | 0.31 | 0.81 | 0.66 | 0.63 | ***0.05*** | 0.36 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 164 | T164A | 0.38 | 0.40 | 0.53 | 0.76 | 0.1 | 0.21 |
| 164 | T164C | 0.29 | 0.51 | 0.70 | 0.74 | 0.1 | 0.11 |
| 164 | T164D | 0.51 | 0.82 | 0.98 | 0.98 | ***0.05*** | 0.18 |
| 164 | T164E | 0.39 | 0.79 | 1.27 | 1.14 | 0.8 | 0.43 |
| 164 | T164F | 0.27 | 0.33 | 0.34 | 0.72 | ***0.05*** | 0.15 |
| 164 | T164G | 0.40 | 0.51 | 0.76 | 0.70 | 0.1 | 0.26 |
| 164 | T164I | 0.19 | 0.44 | 0.67 | 0.86 | 0.3 | 0.07 |
| 164 | T164K | 0.28 | 0.41 | 0.64 | 0.74 | ***0.05*** | 0.29 |
| 164 | T164L | 0.22 | 0.90 | 1.06 | 1.33 | 0.1 | 0.18 |
| 164 | T164M | 0.30 | 0.40 | 0.66 | 0.81 | 0.4 | 0.19 |
| 164 | T164N | 0.36 | 0.71 | 0.78 | 0.92 | 0.4 | 0.51 |
| 164 | T164Q | 0.24 | 0.63 | 1.02 | 1.06 | ***0.05*** | 0.26 |
| 164 | T164R | 0.30 | 0.45 | 0.58 | 0.70 | ***0.05*** | 0.30 |
| 164 | T164S | 0.32 | 0.80 | 0.98 | 0.87 | 0.4 | 0.45 |
| 164 | T164V | 0.26 | 0.55 | 0.80 | 0.88 | 0.1 | 0.19 |
| 164 | T164W | 0.18 | 1.58 | 2.34 | 1.67 | 0.1 | 0.20 |
| 164 | T164Y | 0.20 | 1.10 | 1.76 | 1.62 | 0.1 | 0.19 |
| 165 | V165A | 0.19 | ***0.05*** | ***0.05*** | 0.28 | ***0.05*** | ***0.05*** |
| 165 | V165C | 0.22 | 0.53 | 0.65 | 0.92 | 0.4 | 0.23 |
| 165 | V165E | 0.24 | ***0.05*** | ***0.05*** | 0.08 | ***0.05*** | ***0.05*** |
| 165 | V165F | 0.22 | ***0.05*** | 0.11 | 0.12 | ***0.05*** | ***0.05*** |
| 165 | V165H | 0.15 | ***0.05*** | ***0.05*** | ***0.05*** | 0.2 | 0.16 |
| 165 | V165I | 0.37 | 0.89 | 1.17 | 1.15 | 1.1 | 0.87 |
| 165 | V165K | 0.28 | ***0.05*** | ***0.05*** | ***0.05*** | ***0.05*** | ***0.05*** |
| 165 | V165L | 0.28 | 0.62 | 0.66 | 1.12 | 0.8 | 0.54 |
| 165 | V165M | 0.30 | 0.61 | 0.84 | 1.10 | 0.7 | 0.53 |
| 165 | V165P | 0.46 | 0.87 | 0.80 | 0.84 | 0.8 | 0.70 |
| 165 | V165Q | 0.16 | ***0.05*** | ***0.05*** | 2.10 | ***0.05*** | ***0.05*** |
| 165 | V165R | 0.22 | ***0.05*** | ***0.05*** | 0.07 | ***0.05*** | ***0.05*** |
| 165 | V165S | 0.18 | ***0.05*** | ***0.05*** | 0.23 | ***0.05*** | ***0.05*** |
| 165 | V165T | 0.19 | 1.50 | 1.95 | 1.86 | 0.1 | 0.21 |
| 165 | V165W | 0.29 | ***0.05*** | ***0.05*** | ***0.05*** | ***0.05*** | ***0.05*** |
| 166 | G166A | 0.27 | 0.47 | 0.77 | 0.97 | 1.1 | 0.26 |
| 166 | G166C | 0.86 | 0.61 | 0.78 | 0.79 | 1.1 | 0.33 |
| 166 | G166F | 1.09 | 0.54 | 0.46 | 0.82 | 1.3 | 0.17 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 166 | G166H | 1.04 | 0.80 | 0.77 | 0.78 | 1.2 | 0.41 |
| 166 | G166K | 0.82 | 0.32 | 0.33 | 0.58 | 0.9 | 0.43 |
| 166 | G166L | 1.36 | 0.41 | 0.48 | 0.61 | 1.2 | 0.20 |
| 166 | G166M | 1.13 | 0.56 | 0.59 | 0.66 | 1.1 | 0.40 |
| 166 | G166N | 1.08 | 0.90 | 0.94 | 1.10 | 1.1 | 0.47 |
| 166 | G166P | 0.37 | 0.79 | 0.83 | 0.88 | 0.1 | 0.19 |
| 166 | G166Q | 1.21 | 0.66 | 0.81 | 0.96 | 1.1 | 0.57 |
| 166 | G166R | 0.87 | 0.41 | 0.43 | 0.88 | 0.9 | 0.55 |
| 166 | G166S | 1.09 | 0.80 | 1.04 | 0.75 | 1.0 | 0.26 |
| 166 | G166T | 1.16 | 0.68 | 0.65 | 0.77 | ***0.05*** | ***0.05*** |
| 166 | G166V | 1.01 | 0.44 | 0.85 | 0.75 | ***0.05*** | ***0.05*** |
| 166 | G166W | 1.10 | 0.65 | 0.95 | 0.99 | 1.1 | 0.06 |
| 166 | G166Y | 0.99 | 0.69 | 0.45 | 0.92 | 1.0 | 0.19 |
| 167 | Y167A | 0.68 | 1.35 | 1.13 | 1.28 | 0.7 | 0.57 |
| 167 | Y167C | 0.42 | 1.20 | 1.06 | 1.31 | 0.6 | 0.30 |
| 167 | Y167D | 0.27 | 1.36 | 1.64 | 1.29 | 0.1 | 0.07 |
| 167 | Y167E | 0.37 | 1.46 | 1.71 | 1.31 | 0.5 | 0.08 |
| 167 | Y167F | 1.01 | 0.96 | 1.11 | 0.99 | 0.9 | 0.83 |
| 167 | Y167G | 0.27 | 0.47 | 0.90 | 0.76 | ***0.05*** | ***0.05*** |
| 167 | Y167H | 0.33 | 0.93 | 1.06 | 1.15 | 0.5 | 0.47 |
| 167 | Y167I | 0.66 | 0.98 | 1.15 | 1.14 | 0.9 | 0.34 |
| 167 | Y167K | 0.16 | ***0.05*** | 1.11 | 0.23 | ***0.05*** | ***0.05*** |
| 167 | Y167L | 0.23 | 1.16 | 1.53 | 1.29 | 0.1 | 0.17 |
| 167 | Y167M | 0.23 | 1.69 | 1.28 | 1.35 | 0.4 | 0.22 |
| 167 | Y167N | ***0.05*** | ***0.05*** | ***0.05*** | ***0.05*** | ***0.05*** | ***0.05*** |
| 167 | Y167P | 0.25 | 1.80 | 1.86 | 1.64 | ***0.05*** | ***0.05*** |
| 167 | Y167Q | 0.18 | 1.16 | 1.36 | 1.60 | ***0.05*** | ***0.05*** |
| 167 | Y167R | 0.29 | ***0.05*** | ***0.05*** | ***0.05*** | ***0.05*** | ***0.05*** |
| 167 | Y167S | 0.30 | 1.37 | 1.27 | 1.32 | 0.2 | 0.30 |
| 167 | Y167T | 0.33 | 1.47 | 1.25 | 1.39 | 0.3 | 0.35 |
| 167 | Y167V | 0.60 | 0.96 | 1.06 | 1.07 | 0.8 | 0.44 |
| 167 | Y167W | 0.41 | 1.21 | 1.10 | 1.04 | 0.3 | 0.54 |
| 168 | P168A | 0.23 | 0.38 | 0.54 | 0.32 | ***0.05*** | ***0.05*** |
| 168 | P168C | 0.27 | 0.17 | 0.12 | ***0.05*** | ***0.05*** | ***0.05*** |
| 168 | P168D | 0.29 | 0.22 | 0.06 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 168 | P168E | ***0.05*** | ***0.05*** | ***0.05*** | ***0.05*** | ***0.05*** | ***0.05*** |
| 168 | P168F | 0.24 | 0.07 | ***0.05*** | 0.10 | ***0.05*** | ***0.05*** |
| 168 | P168G | 0.23 | ***0.05*** | 0.22 | ***0.05*** | ***0.05*** | ***0.05*** |
| 168 | P168H | 0.23 | 0.60 | 0.10 | ***0.05*** | ***0.05*** | ***0.05*** |
| 168 | P168I | **0.19** | ***0.05*** | ***0.05*** | ***0.05*** | ***0.05*** | ***0.05*** |
| 168 | P168K | 0.24 | 0.14 | 0.09 | ***0.05*** | ***0.05*** | ***0.05*** |
| 168 | P168L | 0.21 | 1.99 | 0.85 | 0.34 | ***0.05*** | ***0.05*** |
| 168 | P168M | 0.26 | 0.08 | 0.21 | ***0.05*** | ***0.05*** | ***0.05*** |
| 168 | P168N | ***0.05*** | ***0.05*** | ***0.05*** | ***0.05*** | ***0.05*** | ***0.05*** |
| 168 | P168Q | 0.24 | 0.06 | 0.11 | ***0.05*** | ***0.05*** | ***0.05*** |
| 168 | P168R | 0.23 | ***0.05*** | 0.20 | 0.09 | ***0.05*** | ***0.05*** |
| 168 | P168S | 0.25 | 0.30 | 0.12 | ***0.05*** | ***0.05*** | ***0.05*** |
| 168 | P168T | 0.21 | 2.95 | 0.29 | ***0.05*** | ***0.05*** | ***0.05*** |
| 168 | P168V | 0.18 | ***0.05*** | ***0.05*** | ***0.05*** | ***0.05*** | ***0.05*** |
| 168 | P168W | 0.22 | 0.27 | ***0.05*** | 0.07 | ***0.05*** | ***0.05*** |
| 168 | P168Y | 0.24 | ***0.05*** | 0.10 | ***0.05*** | ***0.05*** | ***0.05*** |
| 169 | G169A | 1.32 | 0.86 | 0.90 | 0.89 | 1.0 | 1.23 |
| 169 | G169C | 0.19 | 3.00 | 2.18 | 2.35 | 1.0 | 0.46 |
| 169 | G169E | 0.44 | 0.08 | ***0.05*** | ***0.05*** | ***0.05*** | ***0.05*** |
| 169 | G169F | 0.48 | ***0.05*** | ***0.05*** | ***0.05*** | ***0.05*** | ***0.05*** |
| 169 | G169H | 0.59 | ***0.05*** | ***0.05*** | ***0.05*** | ***0.05*** | ***0.05*** |
| 169 | G169I | 0.75 | ***0.05*** | ***0.05*** | ***0.05*** | ***0.05*** | ***0.05*** |
| 169 | G169K | 0.52 | ***0.05*** | ***0.05*** | ***0.05*** | ***0.05*** | ***0.05*** |
| 169 | G169L | 0.43 | ***0.05*** | ***0.05*** | ***0.05*** | ***0.05*** | ***0.05*** |
| 169 | G169M | 0.61 | ***0.05*** | ***0.05*** | ***0.05*** | ***0.05*** | ***0.05*** |
| 169 | G169N | 0.41 | ***0.05*** | ***0.05*** | ***0.05*** | ***0.05*** | ***0.05*** |
| 169 | G169P | 0.21 | 0.16 | ***0.05*** | 0.10 | ***0.05*** | ***0.05*** |
| 169 | G169Q | 0.30 | 0.20 | ***0.05*** | 0.11 | ***0.05*** | ***0.05*** |
| 169 | G169R | 0.49 | ***0.05*** | ***0.05*** | ***0.05*** | ***0.05*** | ***0.05*** |
| 169 | G169S | 0.38 | 1.02 | 0.83 | 1.03 | 0.9 | 0.63 |
| 169 | G169T | 0.23 | 0.15 | ***0.05*** | 0.09 | ***0.05*** | ***0.05*** |
| 169 | G169V | 0.49 | ***0.05*** | ***0.05*** | ***0.05*** | ***0.05*** | ***0.05*** |
| 169 | G169W | 0.42 | ***0.05*** | ***0.05*** | ***0.05*** | ***0.05*** | ***0.05*** |
| 169 | G169Y | 0.29 | 0.09 | ***0.05*** | ***0.05*** | ***0.05*** | ***0.05*** |
| 170 | K170A | 1.04 | 1.16 | 1.09 | 0.98 | 1.1 | 1.23 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 170 | K170C | 0.81 | 1.19 | 1.03 | 0.95 | 0.9 | 0.73 |
| 170 | K170E | 0.18 | 2.86 | 0.09 | 1.18 | ***0.05*** | ***0.05*** |
| 170 | K170F | 0.50 | 1.09 | 1.09 | 0.95 | 0.9 | 0.69 |
| 170 | K170G | 0.51 | 1.21 | 0.92 | 1.00 | 0.9 | 0.65 |
| 170 | K170H | 0.57 | 1.10 | 1.10 | 0.98 | 0.8 | 0.86 |
| 170 | K170I | 0.47 | 1.19 | 1.14 | 0.93 | 0.7 | 0.43 |
| 170 | K170L | 0.36 | 1.27 | 1.15 | 0.97 | 0.7 | 0.33 |
| 170 | K170M | 0.39 | 1.04 | 0.96 | 0.99 | 0.6 | 0.38 |
| 170 | K170N | 0.34 | 1.59 | 1.37 | 1.18 | 0.5 | 0.58 |
| 170 | K170P | 0.22 | 2.48 | 2.58 | 1.55 | 0.9 | 0.65 |
| 170 | K170Q | 0.51 | 1.32 | 1.22 | 1.05 | 0.8 | 0.63 |
| 170 | K170R | 1.13 | 1.12 | 1.08 | 1.05 | 1.1 | 1.14 |
| 170 | K170S | 0.51 | 1.30 | 1.13 | 0.90 | 0.9 | 0.75 |
| 170 | K170T | 0.29 | 1.48 | 1.50 | 1.06 | 0.5 | 0.48 |
| 170 | K170V | 0.62 | 1.13 | 1.17 | 1.08 | 0.8 | 0.78 |
| 170 | K170W | 0.30 | 1.08 | 1.13 | 0.97 | 0.8 | 0.48 |
| 170 | K170Y | 0.38 | 1.39 | 1.22 | 1.04 | 0.9 | 0.66 |
| 171 | Y171A | 0.22 | 0.15 | ***0.05*** | 0.15 | ***0.05*** | ***0.05*** |
| 171 | Y171C | 0.17 | 12.33 | 8.58 | 1.82 | 0.8 | 0.12 |
| 171 | Y171D | 0.17 | 4.55 | 3.45 | 1.13 | ***0.05*** | ***0.05*** |
| 171 | Y171F | 0.47 | 1.24 | 0.88 | 0.93 | 0.7 | 0.68 |
| 171 | Y171G | 0.43 | ***0.05*** | ***0.05*** | ***0.05*** | ***0.05*** | ***0.05*** |
| 171 | Y171H | 0.16 | ***0.05*** | ***0.05*** | 9.98 | 0.1 | 0.25 |
| 171 | Y171I | 0.20 | 0.18 | ***0.05*** | ***0.05*** | ***0.05*** | ***0.05*** |
| 171 | Y171K | 0.57 | 0.06 | ***0.05*** | ***0.05*** | ***0.05*** | ***0.05*** |
| 171 | Y171L | 0.18 | 4.11 | 3.35 | 2.44 | 0.3 | 0.25 |
| 171 | Y171M | 0.16 | ***0.05*** | 0.18 | 7.20 | ***0.05*** | ***0.05*** |
| 171 | Y171N | 0.17 | 2.21 | ***0.05*** | 0.47 | ***0.05*** | ***0.05*** |
| 171 | Y171P | 0.45 | ***0.05*** | ***0.05*** | ***0.05*** | ***0.05*** | ***0.05*** |
| 171 | Y171Q | 0.23 | 0.32 | ***0.05*** | 0.17 | ***0.05*** | ***0.05*** |
| 171 | Y171R | 0.49 | ***0.05*** | ***0.05*** | ***0.05*** | ***0.05*** | ***0.05*** |
| 171 | Y171S | 0.20 | ***0.05*** | ***0.05*** | 0.06 | ***0.05*** | ***0.05*** |
| 171 | Y171T | 0.18 | 0.37 | ***0.05*** | ***0.05*** | ***0.05*** | ***0.05*** |
| 171 | Y171V | 0.11 | ***0.05*** | ***0.05*** | ***0.05*** | ***0.05*** | ***0.05*** |
| 171 | Y171W | 0.49 | 1.37 | 1.02 | 1.02 | 0.6 | 0.40 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 172 | P172A | 0.83 | 1.13 | 1.08 | 1.09 | 1.0 | 1.14 |
| 172 | P172C | 0.69 | 1.22 | 1.17 | 0.77 | 0.9 | 1.06 |
| 172 | P172E | 0.80 | 1.30 | 1.16 | 1.01 | 1.2 | 1.01 |
| 172 | P172F | 0.40 | 1.25 | 0.99 | 1.14 | 0.8 | 0.92 |
| 172 | P172G | 0.34 | 1.36 | 1.22 | 1.23 | 1.0 | 0.87 |
| 172 | P172H | 0.52 | 1.09 | 0.91 | 1.08 | 0.9 | 1.03 |
| 172 | P172I | 0.46 | 1.38 | 1.11 | 1.11 | 0.9 | 1.07 |
| 172 | P172K | 0.66 | 0.99 | 0.61 | 0.90 | 1.0 | 1.05 |
| 172 | P172L | 0.52 | 1.50 | 1.08 | 1.14 | 1.0 | 1.02 |
| 172 | P172M | 0.55 | 1.23 | 1.14 | 1.08 | 1.0 | 1.02 |
| 172 | P172N | 0.67 | 1.09 | 0.96 | 1.01 | 0.9 | 1.05 |
| 172 | P172Q | 0.75 | 1.17 | 1.02 | 0.90 | 1.0 | 1.19 |
| 172 | P172R | 0.52 | 0.84 | 0.51 | 0.80 | 1.0 | 0.99 |
| 172 | P172S | 0.64 | 1.17 | 1.02 | 1.20 | 1.0 | 1.06 |
| 172 | P172T | 0.58 | 1.27 | 1.04 | 1.15 | 0.8 | 1.24 |
| 172 | P172V | 0.54 | 1.37 | 1.18 | 1.24 | 0.8 | 1.15 |
| 172 | P172Y | 0.29 | 1.67 | 1.29 | 1.41 | 0.8 | 0.88 |
| 173 | S173A | 0.89 | 1.16 | 0.72 | 1.02 | 0.9 | 0.93 |
| 173 | S 173C | 0.81 | 1.19 | 0.89 | 1.05 | 0.9 | 0.98 |
| 173 | S173D | ***0.05*** | ***0.05*** | ***0.05*** | ***0.05*** | ***0.05*** | ***0.05*** |
| 173 | S173E | 0.96 | 0.90 | 0.78 | 0.98 | 1.0 | 0.95 |
| 173 | S173F | 0.51 | 1.07 | 1.17 | 0.87 | 0.6 | 0.82 |
| 173 | S173G | 0.64 | 0.93 | 0.65 | 0.91 | 0.8 | 0.59 |
| 173 | S173H | 0.49 | 1.35 | 0.88 | 1.18 | 0.8 | 0.92 |
| 173 | S173I | 0.44 | 1.16 | 1.05 | 1.30 | 0.8 | 1.10 |
| 173 | S173K | 0.70 | 0.66 | 0.57 | 1.03 | 0.7 | 0.95 |
| 173 | S173L | 0.56 | 1.10 | 1.02 | 1.35 | 0.9 | 0.84 |
| 173 | S173M | 0.69 | 1.11 | 1.16 | 1.00 | 0.8 | 1.01 |
| 173 | S173N | ***0.05*** | ***0.05*** | ***0.05*** | ***0.05*** | ***0.05*** | ***0.05*** |
| 173 | S173P | 0.45 | 1.14 | 1.02 | 1.22 | 0.9 | 0.89 |
| 173 | S173Q | 0.67 | 1.28 | 0.84 | 1.21 | 0.9 | 1.03 |
| 173 | S173R | 0.45 | 0.98 | 0.65 | 1.14 | 0.6 | 0.93 |
| 173 | S173T | 0.82 | 1.08 | 0.81 | 1.15 | 1.0 | 1.00 |
| 173 | S173V | 0.79 | 1.09 | 0.79 | 1.10 | 1.0 | 1.09 |
| 173 | S173W | 0.33 | 1.89 | 1.29 | 1.50 | 0.6 | 0.90 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 173 | S173Y | 0.39 | 1.71 | 1.01 | 1.38 | 0.7 | 0.87 |
| 174 | V174A | 0.45 | 1.44 | 1.21 | 1.10 | 0.8 | 1.00 |
| 174 | V174C | 0.80 | 1.14 | 1.08 | 1.21 | 0.9 | 1.19 |
| 174 | V174D | 0.20 | ***0.05*** | ***0.05*** | 0.65 | ***0.05*** | ***0.05*** |
| 174 | V174E | 0.22 | 0.36 | ***0.05*** | 0.08 | ***0.05*** | ***0.05*** |
| 174 | V174F | 0.22 | 0.58 | 0.18 | 0.16 | ***0.05*** | ***0.05*** |
| 174 | V174G | 0.19 | ***0.05*** | ***0.05*** | ***0.05*** | 0.2 | 0.13 |
| 174 | V174H | 0.23 | 0.50 | 0.12 | ***0.05*** | ***0.05*** | ***0.05*** |
| 174 | V174I | 0.37 | 1.48 | 0.86 | 1.14 | 0.6 | 0.58 |
| 174 | V174K | 0.22 | 0.49 | ***0.05*** | 0.08 | ***0.05*** | ***0.05*** |
| 174 | V174L | 0.21 | 92.96 | 66.79 | 4.24 | 0.5 | 0.34 |
| 174 | V174M | 0.20 | ***0.05*** | ***0.05*** | 2.70 | 0.1 | 0.15 |
| 174 | V174N | 0.17 | ***0.05*** | ***0.05*** | ***0.05*** | 0.2 | 0.13 |
| 174 | V174P | 0.20 | ***0.05*** | ***0.05*** | 14.42 | 0.2 | 0.40 |
| 174 | V174Q | ***0.05*** | ***0.05*** | ***0.05*** | ***0.05*** | ***0.05*** | ***0.05*** |
| 174 | V174R | 0.25 | 0.16 | ***0.05*** | ***0.05*** | ***0.05*** | ***0.05*** |
| 174 | V174S | 0.41 | 1.38 | 1.04 | 1.21 | 0.9 | 0.82 |
| 174 | V174T | 0.78 | 1.29 | 0.82 | 1.13 | 0.9 | 1.11 |
| 174 | V174W | 0.24 | ***0.05*** | ***0.05*** | ***0.05*** | ***0.05*** | ***0.05*** |
| 174 | V174Y | 0.24 | 0.12 | ***0.05*** | ***0.05*** | ***0.05*** | ***0.05*** |
| 175 | I175A | 0.21 | 6.10 | 9.32 | 1.84 | 0.4 | 0.31 |
| 175 | I175C | 0.61 | 1.10 | 1.06 | 1.16 | 0.9 | 0.92 |
| 175 | I175D | 0.23 | ***0.05*** | 0.23 | 0.06 | ***0.05*** | ***0.05*** |
| 175 | I175E | 0.19 | ***0.05*** | ***0.05*** | ***0.05*** | 0.4 | 0.18 |
| 175 | I175F | 0.32 | 1.69 | 1.23 | 1.30 | 0.6 | 0.81 |
| 175 | I175G | 0.25 | 0.39 | 0.13 | ***0.05*** | ***0.05*** | ***0.05*** |
| 175 | I175H | ***0.05*** | ***0.05*** | ***0.05*** | ***0.05*** | ***0.05*** | ***0.05*** |
| 175 | I175K | 0.24 | 0.18 | ***0.05*** | ***0.05*** | ***0.05*** | ***0.05*** |
| 175 | I175L | 0.90 | 1.27 | 0.89 | 1.04 | 0.8 | 1.10 |
| 175 | I175M | 0.92 | 1.17 | 0.82 | 1.05 | 1.0 | 0.98 |
| 175 | I175N | 0.22 | 0.16 | ***0.05*** | 0.15 | ***0.05*** | ***0.05*** |
| 175 | I175P | 0.23 | 0.09 | 0.06 | ***0.05*** | ***0.05*** | ***0.05*** |
| 175 | I175Q | 0.19 | ***0.05*** | ***0.05*** | ***0.05*** | 0.7 | 0.46 |
| 175 | I175R | 0.22 | 1.47 | ***0.05*** | 0.11 | ***0.05*** | ***0.05*** |
| 175 | I175S | 0.17 | ***0.05*** | ***0.05*** | ***0.05*** | 0.5 | 0.09 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 175 | I175T | 0.31 | 1.96 | 1.59 | 1.44 | 0.5 | 0.93 |
| 175 | I175V | 0.33 | 1.96 | 1.32 | 1.36 | 0.9 | 1.06 |
| 175 | I175W | 0.16 | ***0.05*** | ***0.05*** | ***0.05*** | 0.1 | 0.28 |
| 175 | I175Y | ***0.05*** | ***0.05*** | ***0.05*** | ***0.05*** | ***0.05*** | ***0.05*** |
| 176 | A176C | 1.04 | 0.99 | 0.79 | 1.00 | 1.1 | 1.15 |
| 176 | A176D | ***0.05*** | ***0.05*** | ***0.05*** | ***0.05*** | ***0.05*** | ***0.05*** |
| 176 | A176E | 0.31 | ***0.05*** | ***0.05*** | ***0.05*** | ***0.05*** | ***0.05*** |
| 176 | A176F | 0.34 | ***0.05*** | ***0.05*** | ***0.05*** | ***0.05*** | ***0.05*** |
| 176 | A176G | 0.24 | 0.55 | 0.86 | 1.10 | 0.5 | 0.37 |
| 176 | A176H | 0.34 | ***0.05*** | ***0.05*** | ***0.05*** | ***0.05*** | ***0.05*** |
| 176 | A176I | ***0.05*** | ***0.05*** | ***0.05*** | ***0.05*** | ***0.05*** | ***0.05*** |
| 176 | A176K | 0.38 | ***0.05*** | ***0.05*** | ***0.05*** | ***0.05*** | ***0.05*** |
| 176 | A176L | 0.28 | ***0.05*** | 0.07 | 0.10 | ***0.05*** | ***0.05*** |
| 176 | A176M | 0.57 | ***0.05*** | ***0.05*** | ***0.05*** | ***0.05*** | ***0.05*** |
| 176 | A176N | 0.19 | ***0.05*** | 0.41 | 0.53 | ***0.05*** | ***0.05*** |
| 176 | A176P | 0.36 | ***0.05*** | ***0.05*** | ***0.05*** | ***0.05*** | ***0.05*** |
| 176 | A176Q | 0.29 | ***0.05*** | ***0.05*** | ***0.05*** | ***0.05*** | ***0.05*** |
| 176 | A176R | 0.33 | ***0.05*** | ***0.05*** | ***0.05*** | ***0.05*** | ***0.05*** |
| 176 | A176S | 0.78 | 0.89 | 1.00 | 1.12 | 1.0 | 0.71 |
| 176 | A176T | 0.58 | 1.19 | 1.12 | 1.24 | 1.1 | 0.77 |
| 176 | A176V | 0.16 | 1.53 | ***0.05*** | 0.61 | ***0.05*** | ***0.05*** |
| 176 | A176W | 0.38 | ***0.05*** | ***0.05*** | ***0.05*** | ***0.05*** | ***0.05*** |
| 176 | A176Y | 0.34 | ***0.05*** | ***0.05*** | ***0.05*** | ***0.05*** | ***0.05*** |
| 177 | V177A | 0.67 | 0.50 | 0.83 | 0.93 | 0.4 | 0.82 |
| 177 | V177C | 0.49 | 0.77 | 0.95 | 1.10 | 0.7 | 0.83 |
| 177 | V177D | 0.51 | ***0.05*** | ***0.05*** | ***0.05*** | ***0.05*** | ***0.05*** |
| 177 | V177E | 0.49 | ***0.05*** | ***0.05*** | ***0.05*** | ***0.05*** | ***0.05*** |
| 177 | V177F | 0.52 | ***0.05*** | ***0.05*** | ***0.05*** | ***0.05*** | ***0.05*** |
| 177 | V177G | 0.34 | ***0.05*** | ***0.05*** | 0.06 | ***0.05*** | ***0.05*** |
| 177 | V177H | 0.46 | ***0.05*** | ***0.05*** | ***0.05*** | ***0.05*** | ***0.05*** |
| 177 | V177I | 0.37 | 0.89 | 1.25 | 1.25 | 0.5 | 0.89 |
| 177 | V177K | 0.39 | ***0.05*** | ***0.05*** | ***0.05*** | ***0.05*** | ***0.05*** |
| 177 | V177L | 0.42 | ***0.05*** | ***0.05*** | ***0.05*** | ***0.05*** | ***0.05*** |
| 177 | V177M | 0.48 | ***0.05*** | ***0.05*** | ***0.05*** | ***0.05*** | ***0.05*** |
| 177 | V177N | 0.46 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 177 | V177P | 0.43 | ***0.05*** | 0.07 | ***0.05*** | ***0.05*** | ***0.05*** |
| 177 | V177Q | 0.45 | ***0.05*** | ***0.05*** | ***0.05*** | ***0.05*** | ***0.05*** |
| 177 | V177R | 0.41 | ***0.05*** | ***0.05*** | ***0.05*** | ***0.05*** | ***0.05*** |
| 177 | V177S | 0.34 | 0.87 | 0.97 | 1.16 | 0.1 | 0.72 |
| 177 | V177T | 0.56 | 0.75 | 0.73 | 1.09 | 0.8 | 1.00 |
| 177 | V177W | 0.18 | 3.77 | 5.40 | 4.51 | 0.2 | 0.53 |
| 177 | V177Y | 0.40 | ***0.05*** | ***0.05*** | ***0.05*** | ***0.05*** | ***0.05*** |
| 178 | G178C | 0.26 | 0.06 | ***0.05*** | ***0.05*** | ***0.05*** | ***0.05*** |
| 178 | G178D | 0.48 | ***0.05*** | ***0.05*** | ***0.05*** | ***0.05*** | ***0.05*** |
| 178 | G178E | 0.49 | ***0.05*** | ***0.05*** | ***0.05*** | ***0.05*** | ***0.05*** |
| 178 | G178F | 0.46 | ***0.05*** | ***0.05*** | ***0.05*** | ***0.05*** | ***0.05*** |
| 178 | G178H | 0.47 | ***0.05*** | ***0.05*** | ***0.05*** | ***0.05*** | ***0.05*** |
| 178 | G178I | 0.50 | ***0.05*** | ***0.05*** | ***0.05*** | ***0.05*** | ***0.05*** |
| 178 | G178K | 0.68 | ***0.05*** | ***0.05*** | ***0.05*** | ***0.05*** | ***0.05*** |
| 178 | G178L | 0.48 | ***0.05*** | ***0.05*** | ***0.05*** | ***0.05*** | ***0.05*** |
| 178 | G178M | 0.51 | ***0.05*** | ***0.05*** | ***0.05*** | ***0.05*** | ***0.05*** |
| 178 | G178N | 0.47 | ***0.05*** | ***0.05*** | ***0.05*** | ***0.05*** | ***0.05*** |
| 178 | G178P | ***0.05*** | ***0.05*** | ***0.05*** | ***0.05*** | ***0.05*** | ***0.05*** |
| 178 | G178Q | 0.21 | 0.89 | 1.34 | 1.25 | ***0.05*** | 0.36 |
| 178 | G178R | 0.44 | ***0.05*** | ***0.05*** | ***0.05*** | ***0.05*** | ***0.05*** |
| 178 | G178S | 0.31 | 0.68 | 0.85 | 1.28 | ***0.05*** | 0.58 |
| 178 | G178T | 0.38 | ***0.05*** | ***0.05*** | ***0.05*** | ***0.05*** | ***0.05*** |
| 178 | G178V | 0.40 | ***0.05*** | ***0.05*** | ***0.05*** | ***0.05*** | ***0.05*** |
| 178 | G178Y | 0.40 | ***0.05*** | ***0.05*** | ***0.05*** | ***0.05*** | ***0.05*** |
| 179 | A179C | ***0.05*** | ***0.05*** | ***0.05*** | ***0.05*** | ***0.05*** | ***0.05*** |
| 179 | A179D | 0.28 | ***0.05*** | ***0.05*** | 0.06 | ***0.05*** | ***0.05*** |
| 179 | A179E | 0.31 | ***0.05*** | ***0.05*** | ***0.05*** | ***0.05*** | ***0.05*** |
| 179 | A179F | 0.21 | ***0.05*** | 0.14 | 0.24 | ***0.05*** | ***0.05*** |
| 179 | A179G | 0.92 | 1.06 | 0.88 | 1.19 | 0.4 | 0.72 |
| 179 | A179H | 0.34 | ***0.05*** | ***0.05*** | ***0.05*** | ***0.05*** | ***0.05*** |
| 179 | A179I | 0.19 | ***0.05*** | 0.06 | 0.17 | ***0.05*** | ***0.05*** |
| 179 | A179K | 0.30 | ***0.05*** | ***0.05*** | ***0.05*** | ***0.05*** | ***0.05*** |
| 179 | A179L | 0.18 | ***0.05*** | ***0.05*** | 0.33 | ***0.05*** | ***0.05*** |
| 179 | A179M | 0.21 | 0.36 | 0.57 | 0.65 | 0.1 | 0.13 |
| 179 | A179N | 0.19 | 0.09 | 0.42 | 0.55 | ***0.05*** | 0.26 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 179 | A179P | 0.30 | ***0.05*** | 0.07 | ***0.05*** | ***0.05*** | ***0.05*** |
| 179 | A179Q | 0.27 | ***0.05*** | ***0.05*** | 0.07 | ***0.05*** | ***0.05*** |
| 179 | A179R | 0.37 | ***0.05*** | ***0.05*** | ***0.05*** | ***0.05*** | ***0.05*** |
| 179 | A179S | 0.63 | 1.04 | 0.74 | 1.16 | 0.4 | 0.74 |
| 179 | A179T | 0.22 | 0.60 | 0.59 | 1.21 | ***0.05*** | 0.35 |
| 179 | A179V | 0.23 | 0.64 | 1.06 | 1.00 | ***0.05*** | 0.40 |
| 179 | A179W | ***0.05*** | ***0.05*** | ***0.05*** | ***0.05*** | ***0.05*** | ***0.05*** |
| 179 | A179Y | 0.20 | 0.17 | 0.49 | 0.62 | ***0.05*** | ***0.05*** |
| 180 | V180A | 0.70 | 0.98 | 0.76 | 1.05 | 0.1 | 0.78 |
| 180 | V180C | 0.99 | 1.03 | 0.73 | 1.08 | 1.0 | 0.91 |
| 180 | V180D | ***0.05*** | ***0.05*** | ***0.05*** | ***0.05*** | ***0.05*** | ***0.05*** |
| 180 | V180E | ***0.05*** | ***0.05*** | ***0.05*** | ***0.05*** | ***0.05*** | ***0.05*** |
| 180 | V180F | 0.16 | 1.66 | ***0.05*** | 1.51 | ***0.05*** | ***0.05*** |
| 180 | V180G | 0.21 | 1.11 | 1.49 | 1.28 | ***0.05*** | 0.49 |
| 180 | V180H | 0.17 | 2.27 | 2.90 | 1.90 | 0.1 | 0.14 |
| 180 | V180I | ***0.05*** | ***0.05*** | ***0.05*** | ***0.05*** | ***0.05*** | ***0.05*** |
| 180 | V180K | 0.14 | 0.43 | 0.06 | ***0.05*** | ***0.05*** | ***0.05*** |
| 180 | V180L | 1.08 | 0.98 | 0.64 | 1.06 | 0.9 | 1.05 |
| 180 | V180M | 0.58 | 0.77 | 0.80 | 1.00 | ***0.05*** | 0.67 |
| 180 | V180N | 0.36 | 1.12 | 0.91 | 1.09 | ***0.05*** | 0.82 |
| 180 | V180P | 0.38 | ***0.05*** | ***0.05*** | ***0.05*** | ***0.05*** | ***0.05*** |
| 180 | V180Q | 0.17 | 4.63 | 4.63 | 2.08 | ***0.05*** | 0.30 |
| 180 | V180R | 0.29 | ***0.05*** | ***0.05*** | ***0.05*** | ***0.05*** | ***0.05*** |
| 180 | V180S | 0.90 | 0.96 | 0.94 | 1.04 | 0.3 | 1.01 |
| 180 | V180T | 1.07 | 0.93 | 0.87 | 1.01 | 0.9 | 1.13 |
| 180 | V180W | 0.11 | ***0.05*** | ***0.05*** | ***0.05*** | ***0.05*** | ***0.05*** |
| 180 | V180Y | 0.13 | 0.14 | 0.09 | ***0.05*** | ***0.05*** | ***0.05*** |
| 181 | D181A | ***0.05*** | ***0.05*** | ***0.05*** | ***0.05*** | ***0.05*** | ***0.05*** |
| 181 | D181C | 0.80 | 0.88 | 0.79 | 0.89 | 0.5 | 0.84 |
| 181 | D181E | 0.86 | 1.01 | 0.71 | 1.22 | 0.3 | 0.86 |
| 181 | D181F | ***0.05*** | ***0.05*** | ***0.05*** | ***0.05*** | ***0.05*** | ***0.05*** |
| 181 | D181G | 0.89 | 0.84 | 0.71 | 1.06 | ***0.05*** | 1.12 |
| 181 | D181H | 0.86 | 0.70 | 0.54 | 0.95 | ***0.05*** | 0.88 |
| 181 | D181I | 0.21 | 0.63 | 0.66 | 1.28 | ***0.05*** | 0.61 |
| 181 | D181K | 0.47 | 0.18 | 0.30 | 0.65 | ***0.05*** | 0.92 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 181 | D181L | 0.54 | 0.56 | 0.43 | 0.87 | ***0.05*** | 0.91 |
| 181 | D181M | 0.65 | 0.63 | 0.67 | 0.96 | ***0.05*** | 0.90 |
| 181 | D181N | 1.07 | 0.79 | 0.62 | 0.93 | 0.2 | 1.06 |
| 181 | D181P | 0.20 | ***0.05*** | ***0.05*** | 0.07 | ***0.05*** | ***0.05*** |
| 181 | D181Q | 0.55 | 0.71 | 0.60 | 0.89 | ***0.05*** | 0.84 |
| 181 | D181R | 0.34 | 0.06 | 0.22 | 0.53 | ***0.05*** | 0.77 |
| 181 | D181S | 0.88 | 0.73 | 0.73 | 1.02 | ***0.05*** | 1.07 |
| 181 | D181T | 0.93 | 0.78 | 0.63 | 0.85 | ***0.05*** | 0.98 |
| 181 | D181V | 0.20 | 0.81 | 1.44 | 1.58 | ***0.05*** | 0.53 |
| 181 | D181W | 0.41 | 0.38 | 0.50 | 0.74 | ***0.05*** | 0.89 |
| 181 | D181Y | 0.43 | 0.64 | 0.42 | 1.03 | ***0.05*** | 1.00 |
| 182 | S182A | 0.96 | 0.97 | 1.09 | 1.02 | 0.9 | 0.94 |
| 182 | S182C | 1.10 | 1.03 | 1.06 | 0.85 | 1.1 | 1.00 |
| 182 | S182D | 0.92 | 1.22 | 1.07 | 1.01 | 1.1 | 1.06 |
| 182 | S182E | ***0.05*** | ***0.05*** | ***0.05*** | ***0.05*** | ***0.05*** | ***0.05*** |
| 182 | S182F | 0.88 | 1.14 | 1.07 | 1.00 | 0.4 | 1.20 |
| 182 | S182G | 1.16 | 1.09 | 1.03 | 1.00 | 0.9 | 0.81 |
| 182 | S182H | 1.09 | 1.13 | 1.04 | 0.93 | 0.8 | 0.91 |
| 182 | S182I | 0.87 | 1.13 | 1.17 | 1.15 | 0.7 | 1.15 |
| 182 | S182K | 1.14 | 0.50 | 0.66 | 1.08 | ***0.05*** | 0.97 |
| 182 | S182L | 0.88 | 0.88 | 1.07 | 0.98 | 0.4 | 1.00 |
| 182 | S182M | 1.00 | 0.96 | 1.22 | 0.95 | 0.8 | 1.05 |
| 182 | S182N | ***0.05*** | ***0.05*** | ***0.05*** | ***0.05*** | ***0.05*** | ***0.05*** |
| 182 | S182P | 1.26 | 0.77 | 1.17 | 1.00 | 0.9 | 0.77 |
| 182 | S182Q | 0.94 | 1.28 | 1.04 | 0.98 | 1.0 | 1.08 |
| 182 | S 182R | 1.08 | 0.69 | 0.57 | 1.02 | ***0.05*** | 1.18 |
| 182 | S182T | 0.95 | 0.82 | 1.19 | 0.92 | 0.8 | 0.98 |
| 182 | S182V | 0.79 | 1.02 | 1.31 | 1.07 | 0.8 | 1.19 |
| 182 | S182W | 0.32 | 1.48 | 1.36 | 1.36 | 0.3 | 1.04 |
| 182 | S182Y | 0.86 | 1.07 | 1.05 | 0.98 | 0.5 | 1.22 |
| 183 | S183A | 1.09 | 0.88 | 1.12 | 1.03 | 0.9 | 0.99 |
| 183 | S183C | 1.09 | 1.22 | 1.01 | 0.91 | 1.1 | 0.84 |
| 183 | S183D | ***0.05*** | ***0.05*** | ***0.05*** | ***0.05*** | ***0.05*** | ***0.05*** |
| 183 | S183E | 1.06 | 0.84 | 1.01 | 0.93 | 1.2 | 1.04 |
| 183 | S183F | 0.97 | 0.91 | 1.35 | 0.83 | 0.9 | 1.06 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 183 | S183G | 0.98 | 0.77 | 0.97 | 1.04 | 1.0 | 0.84 |
| 183 | S183H | 1.10 | 1.10 | 0.84 | 1.00 | 1.0 | 0.85 |
| 183 | S183I | 1.11 | 1.07 | 1.02 | 1.01 | 0.9 | 1.02 |
| 183 | S183K | 1.08 | 0.68 | 0.67 | 0.95 | 0.2 | 0.93 |
| 183 | S183L | 1.02 | 0.84 | 0.85 | 1.00 | 1.0 | 1.01 |
| 183 | S183M | 1.05 | 1.03 | 1.23 | 1.05 | 1.0 | 0.87 |
| 183 | S183N | 1.08 | 0.85 | 1.02 | 1.02 | 1.1 | 0.95 |
| 183 | S183P | 0.31 | 1.26 | 1.29 | 0.99 | ***0.05*** | 0.58 |
| 183 | S183Q | 0.99 | 0.66 | 1.23 | 0.98 | 1.1 | 0.95 |
| 183 | S183R | 1.06 | 0.67 | 0.70 | 0.95 | 0.1 | 0.93 |
| 183 | S183T | 0.92 | 1.20 | 1.25 | 1.02 | 1.0 | 1.12 |
| 183 | S183V | 0.90 | 1.20 | 1.10 | 0.93 | 1.0 | 1.11 |
| 183 | S183W | 0.71 | 1.03 | 1.00 | 1.06 | 0.7 | 1.26 |
| 183 | S183Y | 0.95 | 0.89 | 0.91 | 1.12 | 1.0 | 1.11 |
| 184 | N184A | 0.27 | 1.40 | 1.89 | 1.40 | 0.7 | 0.71 |
| 184 | N184C | 0.90 | 1.16 | 1.02 | 1.06 | 1.0 | 1.10 |
| 184 | N184D | 0.90 | 1.03 | 1.20 | 0.96 | 1.1 | 0.96 |
| 184 | N184E | 0.83 | 1.18 | 1.09 | 0.90 | 0.9 | 1.06 |
| 184 | N184F | 0.84 | 0.86 | 0.99 | 1.14 | 0.5 | 1.03 |
| 184 | N184G | 0.99 | 0.91 | 1.19 | 0.93 | 0.8 | 0.91 |
| 184 | N184H | 0.94 | 1.06 | 1.11 | 1.02 | 0.6 | 0.97 |
| 184 | N184I | 0.75 | 1.04 | 1.07 | 0.98 | 0.2 | 1.03 |
| 184 | N184K | 0.92 | 0.90 | 0.79 | 0.91 | ***0.05*** | 0.94 |
| 184 | N184L | 0.95 | 1.36 | 0.87 | 1.09 | 0.7 | 0.93 |
| 184 | N184M | 1.05 | 0.99 | 1.06 | 0.97 | 0.7 | 0.97 |
| 184 | N184P | 0.78 | 0.78 | 1.02 | 0.79 | 0.1 | 0.82 |
| 184 | N184Q | 0.82 | 0.78 | 1.10 | 1.10 | 0.6 | 1.05 |
| 184 | N184R | 0.93 | 0.69 | 0.89 | 0.93 | ***0.05*** | 0.93 |
| 184 | N184S | 0.88 | 1.02 | 0.97 | 1.06 | 0.7 | 1.04 |
| 184 | N184T | 0.80 | 1.16 | 1.14 | 0.90 | 0.4 | 1.19 |
| 184 | N184V | 0.72 | 1.30 | 1.07 | 1.02 | 0.3 | 1.02 |
| 184 | N184W | 0.80 | 0.92 | 1.28 | 0.99 | 0.4 | 1.26 |
| 184 | N184Y | 0.80 | 0.93 | 1.06 | 1.16 | 0.6 | 1.10 |
| 185 | Q185A | 1.01 | 0.84 | 1.15 | 1.06 | 0.9 | 1.08 |
| 185 | Q185C | 0.95 | 1.15 | 1.09 | 0.92 | 1.1 | 1.00 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 185 | Q185D | 0.29 | 2.02 | 1.65 | 1.42 | 0.6 | 0.56 |
| 185 | Q185E | 0.77 | 1.37 | 1.07 | 0.91 | 1.2 | 1.03 |
| 185 | Q185F | 0.84 | 1.05 | 1.09 | 0.94 | 0.6 | 1.14 |
| 185 | Q185G | 0.74 | 0.79 | 1.29 | 1.00 | 0.7 | 1.02 |
| 185 | Q185H | 1.00 | 0.88 | 0.91 | 1.02 | 0.9 | 1.09 |
| 185 | Q185I | 0.87 | 1.46 | 0.98 | 0.95 | 0.9 | 1.24 |
| 185 | Q185K | 0.98 | 0.68 | 0.73 | 0.85 | 0.7 | 0.97 |
| 185 | Q185L | 0.87 | 0.99 | 0.96 | 1.04 | 0.7 | 1.00 |
| 185 | Q185M | 0.93 | 0.91 | 1.09 | 1.05 | 1.0 | 1.01 |
| 185 | Q185N | 0.97 | 1.14 | 1.11 | 1.08 | 1.1 | 1.05 |
| 185 | Q185P | 0.17 | ***0.05*** | ***0.05*** | ***0.05*** | ***0.05*** | ***0.05*** |
| 185 | Q185R | 0.91 | 0.75 | 0.80 | 0.99 | 0.4 | 1.07 |
| 185 | Q185S | 0.83 | 1.38 | 1.04 | 1.06 | 0.8 | 1.06 |
| 185 | Q185T | 0.82 | 1.36 | 1.28 | 1.13 | 1.0 | 1.17 |
| 185 | Q185V | 0.94 | 1.04 | 1.30 | 0.99 | 0.8 | 1.16 |
| 185 | Q185W | 0.81 | 0.85 | 0.85 | 0.87 | 0.1 | 1.14 |
| 185 | Q185Y | 0.74 | 0.95 | 1.05 | 1.26 | 0.7 | 1.28 |
| 186 | R186A | 0.38 | 1.03 | 1.51 | 1.09 | 0.7 | 0.45 |
| 186 | R186C | 0.36 | 1.91 | 1.61 | 1.05 | 0.6 | 0.47 |
| 186 | R186D | 0.21 | 11.05 | 16.42 | 0.21 | ***0.05*** | ***0.05*** |
| 186 | R186E | 0.21 | 23.12 | 23.90 | 1.79 | 0.1 | 0.11 |
| 186 | R186F | 0.33 | 1.32 | 1.44 | 0.88 | 0.2 | 0.29 |
| 186 | R186G | 0.31 | 2.21 | 1.88 | 1.19 | 0.2 | 0.60 |
| 186 | R186H | 0.50 | 1.13 | 1.34 | 1.17 | 0.7 | 0.73 |
| 186 | R186I | 0.38 | 1.34 | 2.12 | 1.26 | 0.9 | 0.80 |
| 186 | R186K | 0.62 | 0.95 | 1.19 | 1.00 | 0.6 | 0.92 |
| 186 | R186L | 0.54 | 1.58 | 1.44 | 1.20 | 0.9 | 0.89 |
| 186 | R186M | ***0.05*** | ***0.05*** | ***0.05*** | ***0.05*** | ***0.05*** | ***0.05*** |
| 186 | R186N | 0.25 | 2.46 | 3.74 | 1.52 | 0.3 | 0.43 |
| 186 | R186P | 0.24 | 2.96 | 2.29 | 0.95 | 0.1 | 0.19 |
| 186 | R186Q | 0.37 | 1.48 | 2.05 | 1.32 | 0.8 | 0.85 |
| 186 | R186S | 0.27 | 2.44 | 1.91 | 1.39 | 0.3 | 0.40 |
| 186 | R186T | 0.22 | 8.56 | 6.97 | 2.69 | 0.4 | 0.36 |
| 186 | R186V | 0.42 | 2.00 | 2.04 | 1.42 | 0.9 | 0.75 |
| 186 | R186W | 0.63 | 1.54 | 1.71 | 1.12 | 1.1 | 0.98 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 186 | R186Y | 0.38 | 1.50 | 1.98 | 1.37 | 0.8 | 0.71 |
| 187 | A187C | 0.77 | 0.75 | 1.06 | 0.84 | 0.2 | 0.31 |
| 187 | A187D | 0.93 | 1.51 | 0.99 | 1.03 | 0.4 | 0.17 |
| 187 | A187E | 0.72 | 1.41 | 1.49 | 1.00 | 0.2 | 0.34 |
| 187 | A187F | 0.73 | 1.02 | 1.07 | 0.93 | 0.1 | 0.43 |
| 187 | A187G | 0.90 | 1.04 | 1.12 | 0.93 | 0.6 | 1.05 |
| 187 | A187H | ***0.05*** | ***0.05*** | ***0.05*** | ***0.05*** | ***0.05*** | ***0.05*** |
| 187 | A187I | 0.34 | 1.14 | 1.55 | 0.93 | ***0.05*** | 0.43 |
| 187 | A187K | 0.23 | 1.21 | 1.45 | 1.18 | ***0.05*** | 0.36 |
| 187 | A187L | 0.55 | 0.77 | 1.15 | 0.90 | ***0.05*** | 0.33 |
| 187 | A187M | 0.40 | 1.03 | 1.34 | 0.93 | ***0.05*** | 0.42 |
| 187 | A187N | 0.74 | 1.11 | 1.39 | 1.03 | 0.5 | 1.22 |
| 187 | A187P | 1.08 | 1.15 | 1.12 | 0.92 | 0.7 | 0.69 |
| 187 | A187Q | 0.48 | 1.32 | 1.24 | 0.92 | ***0.05*** | 0.41 |
| 187 | A187R | 0.24 | 0.48 | 0.81 | 0.70 | ***0.05*** | 0.29 |
| 187 | A187S | 0.68 | 1.14 | 1.13 | 1.06 | 0.4 | 0.94 |
| 187 | A187T | ***0.05*** | ***0.05*** | ***0.05*** | ***0.05*** | ***0.05*** | ***0.05*** |
| 187 | A187V | 0.46 | 1.14 | 1.14 | 0.91 | ***0.05*** | 0.60 |
| 187 | A187W | 0.93 | 0.95 | 0.99 | 1.07 | 0.7 | 0.41 |
| 187 | A187Y | 1.02 | 1.05 | 1.25 | 0.79 | 0.5 | 0.40 |
| 188 | S188A | 1.14 | 0.93 | 1.08 | 0.96 | 0.9 | 0.92 |
| 188 | S188C | 1.24 | 1.07 | 1.27 | 0.81 | 1.0 | 0.85 |
| 188 | S188D | 1.08 | 1.03 | 1.33 | 0.94 | 1.2 | 0.86 |
| 188 | S188E | 1.03 | 1.18 | 1.29 | 0.98 | 1.1 | 1.00 |
| 188 | S188F | 0.91 | 0.98 | 1.08 | 0.86 | 0.8 | 0.92 |
| 188 | S188G | 1.06 | 0.99 | 1.09 | 0.94 | 0.9 | 0.89 |
| 188 | S188H | 1.01 | 0.92 | 1.00 | 0.91 | 0.9 | 1.01 |
| 188 | S188I | 0.94 | 0.79 | 1.24 | 1.01 | 0.9 | 0.96 |
| 188 | S188K | 1.14 | 0.64 | 0.89 | 1.00 | 0.5 | 1.05 |
| 188 | S188L | 1.01 | 1.07 | 1.16 | 0.82 | 0.8 | 0.98 |
| 188 | S188M | 0.98 | 0.73 | 1.28 | 0.90 | 0.8 | 0.98 |
| 188 | S188N | ***0.05*** | ***0.05*** | ***0.05*** | ***0.05*** | ***0.05*** | ***0.05*** |
| 188 | S188P | 1.05 | 1.05 | 1.23 | 1.01 | 1.0 | 0.90 |
| 188 | S188Q | 0.97 | 1.01 | 1.17 | 1.00 | 0.9 | 1.03 |
| 188 | S 188R | 0.48 | 0.91 | 1.28 | 1.13 | 0.5 | 1.00 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 188 | S188T | 0.82 | 0.95 | 1.37 | 1.01 | 0.9 | 1.08 |
| 188 | S188V | 0.84 | 0.97 | 1.25 | 0.96 | 0.8 | 1.15 |
| 188 | S188W | 0.80 | 0.63 | 1.04 | 0.70 | 0.8 | 0.81 |
| 188 | S188Y | 0.77 | 0.90 | 1.11 | 0.86 | 0.9 | 0.94 |
| 189 | F189A | 0.79 | 0.82 | 0.85 | 0.96 | ***0.05*** | 0.39 |
| 189 | F189C | 1.16 | 0.82 | 0.77 | 0.86 | 0.3 | 0.15 |
| 189 | F189D | 2.06 | 1.03 | 0.88 | 0.94 | 0.4 | 0.28 |
| 189 | F189H | 0.65 | 0.76 | 0.73 | 0.98 | ***0.05*** | 0.38 |
| 189 | F189I | 0.78 | 0.98 | 0.99 | 1.05 | ***0.05*** | 0.33 |
| 189 | F189K | 0.47 | 0.65 | 0.62 | 1.01 | ***0.05*** | 0.57 |
| 189 | F189L | 1.02 | 0.90 | 0.73 | 0.89 | ***0.05*** | 0.39 |
| 189 | F189M | 1.67 | 1.01 | 0.89 | 1.02 | 0.1 | 0.40 |
| 189 | F189N | 1.31 | 0.83 | 0.77 | 0.82 | 0.1 | 0.83 |
| 189 | F189P | 0.43 | 0.87 | 0.81 | 1.03 | ***0.05*** | 0.23 |
| 189 | F189Q | 0.80 | 0.68 | 0.75 | 0.78 | ***0.05*** | 0.38 |
| 189 | F189R | 0.60 | 0.61 | 0.52 | 0.98 | ***0.05*** | 0.51 |
| 189 | F189S | 0.93 | 0.91 | 1.08 | 1.06 | 0.1 | 0.81 |
| 189 | F189T | 0.74 | 1.01 | 1.10 | 0.99 | 0.1 | 0.92 |
| 189 | F189V | 0.70 | 1.07 | 0.83 | 0.93 | ***0.05*** | 0.36 |
| 189 | F189W | 0.59 | 0.57 | 0.71 | 0.81 | 1.0 | 0.54 |
| 189 | F189Y | 0.66 | 0.62 | 0.86 | 0.96 | 0.5 | 0.53 |
| 190 | S190A | 0.35 | 1.19 | 1.27 | 0.80 | 0.1 | 0.57 |
| 190 | S190C | 0.62 | 0.82 | 1.11 | 0.69 | 0.6 | 0.68 |
| 190 | S190D | 0.19 | ***0.05*** | ***0.05*** | ***0.05*** | ***0.05*** | ***0.05*** |
| 190 | S190E | 0.20 | ***0.05*** | ***0.05*** | 0.23 | ***0.05*** | ***0.05*** |
| 190 | S190F | 0.25 | 0.29 | ***0.05*** | ***0.05*** | ***0.05*** | ***0.05*** |
| 190 | S190G | 0.27 | 1.50 | 2.17 | 1.30 | 0.1 | 0.56 |
| 190 | S190H | 0.22 | ***0.05*** | ***0.05*** | 0.13 | ***0.05*** | ***0.05*** |
| 190 | S190I | 0.20 | 0.72 | 0.41 | 0.11 | ***0.05*** | ***0.05*** |
| 190 | S190K | 0.24 | 0.14 | ***0.05*** | 0.07 | ***0.05*** | ***0.05*** |
| 190 | S190L | 0.19 | 0.18 | ***0.05*** | ***0.05*** | ***0.05*** | ***0.05*** |
| 190 | S190M | 0.19 | ***0.05*** | ***0.05*** | ***0.05*** | ***0.05*** | ***0.05*** |
| 190 | S190N | 0.23 | 1.60 | 2.04 | 1.06 | 0.1 | 0.12 |
| 190 | S190P | 0.18 | 0.09 | ***0.05*** | ***0.05*** | ***0.05*** | ***0.05*** |
| 190 | S190Q | 0.18 | 0.08 | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 190 | S190R | 0.22 | ***0.05*** | ***0.05*** | 0.65 | ***0.05*** | ***0.05*** |
| 190 | S190T | 0.31 | 1.03 | 1.36 | 1.15 | 0.3 | 0.49 |
| 190 | S190V | 0.17 | ***0.05*** | ***0.05*** | ***0.05*** | ***0.05*** | 0.32 |
| 190 | S190W | 0.25 | ***0.05*** | ***0.05*** | ***0.05*** | ***0.05*** | ***0.05*** |
| 190 | S190Y | ***0.05*** | ***0.05*** | ***0.05*** | ***0.05*** | ***0.05*** | ***0.05*** |
| 191 | Q191A | 0.93 | 0.67 | 1.11 | 0.98 | 0.9 | 0.66 |
| 191 | Q191C | 0.44 | 0.72 | 0.91 | 0.96 | 0.3 | 0.32 |
| 191 | Q191D | 0.24 | 0.14 | 0.24 | 0.10 | ***0.05*** | ***0.05*** |
| 191 | Q191E | 0.24 | 0.49 | 0.20 | 0.20 | ***0.05*** | ***0.05*** |
| 191 | Q191F | 0.25 | ***0.05*** | ***0.05*** | ***0.05*** | ***0.05*** | ***0.05*** |
| 191 | Q191G | 0.77 | 0.75 | 0.73 | 0.74 | 0.7 | 0.33 |
| 191 | Q191H | 0.20 | ***0.05*** | 0.46 | 1.53 | ***0.05*** | ***0.05*** |
| 191 | Q191I | 0.18 | ***0.05*** | 0.13 | ***0.05*** | ***0.05*** | ***0.05*** |
| 191 | Q191K | 0.17 | ***0.05*** | ***0.05*** | ***0.05*** | ***0.05*** | ***0.05*** |
| 191 | Q191L | 0.19 | ***0.05*** | ***0.05*** | ***0.05*** | ***0.05*** | ***0.05*** |
| 191 | Q191M | 0.20 | ***0.05*** | 0.15 | 0.41 | ***0.05*** | ***0.05*** |
| 191 | Q191N | 0.72 | 0.70 | 1.34 | 1.14 | 0.1 | 0.56 |
| 191 | Q191P | 1.19 | 0.60 | 0.59 | 0.62 | ***0.05*** | ***0.05*** |
| 191 | Q191R | 0.20 | ***0.05*** | 0.25 | 0.37 | ***0.05*** | ***0.05*** |
| 191 | Q191S | ***0.05*** | ***0.05*** | ***0.05*** | ***0.05*** | ***0.05*** | ***0.05*** |
| 191 | Q191T | 0.32 | 1.03 | 0.92 | 0.82 | 0.1 | 0.10 |
| 191 | Q191V | 0.33 | 0.98 | 0.62 | 0.74 | 0.1 | 0.09 |
| 191 | Q191W | 0.21 | ***0.05*** | ***0.05*** | 0.08 | ***0.05*** | ***0.05*** |
| 191 | Q191Y | 0.18 | ***0.05*** | ***0.05*** | ***0.05*** | ***0.05*** | ***0.05*** |
| 192 | Y192A | 0.86 | 0.94 | 1.08 | 0.97 | 0.8 | 1.08 |
| 192 | Y192C | ***0.05*** | ***0.05*** | ***0.05*** | ***0.05*** | ***0.05*** | ***0.05*** |
| 192 | Y192D | 0.24 | 1.91 | 2.19 | 1.17 | 0.1 | 0.11 |
| 192 | Y192E | 0.35 | 1.44 | 1.58 | 1.09 | ***0.05*** | 0.22 |
| 192 | Y192F | 0.51 | 0.70 | 0.98 | 0.87 | 0.4 | 0.55 |
| 192 | Y192G | 0.43 | 0.66 | 0.98 | 0.99 | 0.2 | 0.47 |
| 192 | Y192H | 0.45 | 0.85 | 0.66 | 0.82 | 0.2 | 0.49 |
| 192 | Y192I | ***0.05*** | ***0.05*** | ***0.05*** | ***0.05*** | ***0.05*** | ***0.05*** |
| 192 | Y192K | 0.34 | 0.53 | 1.15 | 0.70 | ***0.05*** | 0.24 |
| 192 | Y192L | 0.23 | 1.94 | 1.92 | 1.49 | ***0.05*** | 0.19 |
| 192 | Y192M | 0.41 | 0.90 | 0.93 | 0.84 | 0.1 | 0.39 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 192 | Y192N | 0.33 | 1.16 | 1.47 | 1.01 | 0.1 | 0.66 |
| 192 | Y192P | 0.34 | 0.88 | 0.38 | 0.60 | 0.1 | 0.13 |
| 192 | Y192Q | 0.36 | 2.14 | 1.27 | 1.17 | 0.2 | 0.56 |
| 192 | Y192R | 0.31 | 0.47 | 0.75 | 0.66 | ***0.05*** | 0.18 |
| 192 | Y192S | 0.63 | 0.82 | 1.15 | 0.93 | 0.7 | 0.88 |
| 192 | Y192T | 0.72 | 0.73 | 1.08 | 0.98 | 0.9 | 0.93 |
| 192 | Y192V | ***0.05*** | ***0.05*** | ***0.05*** | ***0.05*** | ***0.05*** | ***0.05*** |
| 192 | Y192W | 0.46 | 1.01 | 1.27 | 0.96 | ***0.05*** | 0.54 |
| 193 | G193A | 0.22 | 0.68 | ***0.05*** | 0.06 | ***0.05*** | ***0.05*** |
| 193 | G193C | 0.20 | 0.24 | ***0.05*** | ***0.05*** | ***0.05*** | ***0.05*** |
| 193 | G193D | 0.18 | ***0.05*** | ***0.05*** | ***0.05*** | ***0.05*** | ***0.05*** |
| 193 | G193E | 0.29 | ***0.05*** | ***0.05*** | ***0.05*** | ***0.05*** | ***0.05*** |
| 193 | G193F | 0.22 | ***0.05*** | ***0.05*** | ***0.05*** | ***0.05*** | ***0.05*** |
| 193 | G193H | 0.21 | 2.68 | ***0.05*** | ***0.05*** | ***0.05*** | ***0.05*** |
| 193 | G193I | 0.23 | ***0.05*** | ***0.05*** | ***0.05*** | ***0.05*** | ***0.05*** |
| 193 | G193K | 0.24 | 0.09 | 0.10 | ***0.05*** | ***0.05*** | ***0.05*** |
| 193 | G193L | 0.23 | 0.22 | ***0.05*** | ***0.05*** | ***0.05*** | ***0.05*** |
| 193 | G193M | ***0.05*** | ***0.05*** | ***0.05*** | ***0.05*** | ***0.05*** | ***0.05*** |
| 193 | G193N | 0.21 | ***0.05*** | 23.24 | 0.16 | ***0.05*** | ***0.05*** |
| 193 | G193P | 0.22 | 0.56 | 0.24 | 0.09 | ***0.05*** | ***0.05*** |
| 193 | G193Q | 0.21 | 2.43 | ***0.05*** | 0.09 | ***0.05*** | ***0.05*** |
| 193 | G193R | 0.19 | ***0.05*** | ***0.05*** | ***0.05*** | ***0.05*** | ***0.05*** |
| 193 | G193S | 0.20 | ***0.05*** | 0.84 | ***0.05*** | ***0.05*** | ***0.05*** |
| 193 | G193T | 0.22 | 4.84 | 0.51 | 0.10 | ***0.05*** | ***0.05*** |
| 193 | G193V | 0.21 | 2.28 | 1.17 | ***0.05*** | ***0.05*** | ***0.05*** |
| 193 | G193W | 0.21 | ***0.05*** | ***0.05*** | 0.27 | ***0.05*** | ***0.05*** |
| 193 | G193Y | 0.38 | ***0.05*** | ***0.05*** | ***0.05*** | ***0.05*** | ***0.05*** |
| 194 | P194A | 0.69 | 1.09 | 0.94 | 0.95 | 0.8 | 1.16 |
| 194 | P194C | 0.75 | 1.19 | 1.18 | 1.19 | 1.0 | 1.41 |
| 194 | P194D | ***0.05*** | ***0.05*** | ***0.05*** | ***0.05*** | ***0.05*** | ***0.05*** |
| 194 | P194E | 0.70 | 1.52 | 1.09 | 1.03 | 1.0 | 1.25 |
| 194 | P194F | ***0.05*** | ***0.05*** | ***0.05*** | ***0.05*** | ***0.05*** | ***0.05*** |
| 194 | P194G | 0.48 | 1.23 | 0.92 | 1.12 | 0.7 | 0.81 |
| 194 | P194H | 0.76 | 1.13 | 1.00 | 1.15 | 1.0 | 1.03 |
| 194 | P194I | 0.48 | 1.63 | 1.09 | 1.21 | 0.9 | 1.12 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 194 | P194K | 0.72 | 0.79 | 0.90 | 0.90 | 0.6 | 0.98 |
| 194 | P194L | 0.58 | 1.58 | 0.87 | 1.29 | 0.9 | 1.09 |
| 194 | P194M | 0.60 | 1.36 | 1.09 | 1.06 | 0.8 | 1.47 |
| 194 | P194N | 0.52 | 1.82 | 1.00 | 1.23 | 0.1 | 1.69 |
| 194 | P194Q | 0.60 | 1.24 | 1.02 | 1.16 | 0.8 | 1.09 |
| 194 | P194R | 0.18 | ***0.05*** | ***0.05*** | ***0.05*** | ***0.05*** | ***0.05*** |
| 194 | P194S | 0.69 | 1.19 | 0.94 | 1.13 | 0.8 | 1.10 |
| 194 | P194T | 0.58 | 1.32 | 1.37 | 1.10 | 0.9 | 1.21 |
| 194 | P194V | 0.48 | 1.67 | 1.08 | 1.18 | 0.8 | 1.36 |
| 194 | P194W | 0.57 | 1.27 | 1.17 | 1.10 | 0.9 | 1.16 |
| 194 | P194Y | ***0.05*** | ***0.05*** | ***0.05*** | ***0.05*** | ***0.05*** | ***0.05*** |
| 195 | E195A | 0.26 | 0.78 | 0.71 | 0.64 | 0.1 | 0.26 |
| 195 | E195C | 0.25 | 1.37 | 0.92 | 0.57 | 0.4 | 0.22 |
| 195 | E195D | 0.34 | 1.09 | 0.84 | 0.86 | 0.7 | 0.38 |
| 195 | E195F | 0.17 | ***0.05*** | ***0.05*** | ***0.05*** | 0.5 | 0.09 |
| 195 | E195G | 0.71 | 0.95 | 0.44 | 0.77 | 0.3 | 0.52 |
| 195 | E195H | 0.26 | 0.67 | 0.38 | 0.48 | 0.1 | 0.08 |
| 195 | E195I | 0.17 | ***0.05*** | 0.12 | ***0.05*** | ***0.05*** | ***0.05*** |
| 195 | E195K | 0.22 | 3.69 | 2.57 | 2.17 | 0.3 | 0.47 |
| 195 | E195L | 0.18 | ***0.05*** | ***0.05*** | ***0.05*** | ***0.05*** | ***0.05*** |
| 195 | E195M | 0.20 | ***0.05*** | ***0.05*** | 2.57 | 0.1 | 0.19 |
| 195 | E195N | ***0.05*** | ***0.05*** | ***0.05*** | ***0.05*** | ***0.05*** | ***0.05*** |
| 195 | E195P | 0.18 | ***0.05*** | ***0.05*** | ***0.05*** | 0.2 | 0.06 |
| 195 | E195Q | 0.30 | 1.29 | 0.69 | 1.02 | 0.2 | 0.69 |
| 195 | E195R | 0.18 | ***0.05*** | ***0.05*** | ***0.05*** | ***0.05*** | ***0.05*** |
| 195 | E195S | 0.25 | 1.14 | 0.72 | 0.80 | ***0.05*** | 0.30 |
| 195 | E195T | ***0.05*** | ***0.05*** | ***0.05*** | ***0.05*** | ***0.05*** | ***0.05*** |
| 195 | E195V | 0.16 | ***0.05*** | ***0.05*** | ***0.05*** | ***0.05*** | ***0.05*** |
| 195 | E195W | 0.22 | 4.29 | 1.26 | 1.32 | 0.1 | 0.10 |
| 195 | E195Y | ***0.05*** | ***0.05*** | ***0.05*** | ***0.05*** | ***0.05*** | ***0.05*** |
| 196 | L196A | 0.19 | ***0.05*** | ***0.05*** | ***0.05*** | ***0.05*** | ***0.05*** |
| 196 | L196C | 0.20 | ***0.05*** | ***0.05*** | 7.45 | 0.1 | 0.15 |
| 196 | L196D | 0.24 | 0.43 | 0.07 | 0.10 | ***0.05*** | ***0.05*** |
| 196 | L196E | ***0.05*** | ***0.05*** | ***0.05*** | ***0.05*** | ***0.05*** | ***0.05*** |
| 196 | L196F | 0.17 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 196 | L196G | 0.17 | ***0.05*** | ***0.05*** | ***0.05*** | 0.1 | 0.09 |
| 196 | L196H | 0.30 | 0.29 | ***0.05*** | ***0.05*** | ***0.05*** | ***0.05*** |
| 196 | L196I | 0.38 | 1.68 | 1.15 | 1.17 | 0.8 | 1.03 |
| 196 | L196K | 0.16 | ***0.05*** | 0.09 | ***0.05*** | ***0.05*** | ***0.05*** |
| 196 | L196M | 0.37 | 1.52 | 1.04 | 1.06 | 0.2 | 0.72 |
| 196 | L196N | 0.16 | ***0.05*** | ***0.05*** | ***0.05*** | 0.2 | 0.08 |
| 196 | L196P | 0.24 | 0.54 | ***0.05*** | 0.13 | ***0.05*** | ***0.05*** |
| 196 | L196Q | 0.16 | ***0.05*** | ***0.05*** | ***0.05*** | ***0.05*** | ***0.05*** |
| 196 | L196R | 0.16 | ***0.05*** | ***0.05*** | ***0.05*** | ***0.05*** | ***0.05*** |
| 196 | L196S | 0.15 | ***0.05*** | 0.09 | ***0.05*** | ***0.05*** | ***0.05*** |
| 196 | L196T | 0.24 | 3.86 | 2.91 | 2.68 | 1.0 | 0.69 |
| 196 | L196V | 0.34 | 1.95 | 1.28 | 1.45 | 0.7 | 0.79 |
| 196 | L196W | 0.19 | ***0.05*** | ***0.05*** | ***0.05*** | 2.7 | 0.09 |
| 196 | L196Y | 0.20 | ***0.05*** | ***0.05*** | 0.37 | ***0.05*** | ***0.05*** |
| 197 | D197A | 0.45 | 0.74 | 1.09 | 1.05 | 0.5 | 0.66 |
| 197 | D197C | 0.38 | 1.02 | 1.11 | 0.97 | 0.3 | 0.55 |
| 197 | D197F | 0.16 | 0.38 | 0.51 | 1.24 | ***0.05*** | ***0.05*** |
| 197 | D197G | 0.37 | 1.06 | 1.07 | 1.18 | 0.3 | 0.60 |
| 197 | D197H | 0.15 | ***0.05*** | 0.38 | ***0.05*** | ***0.05*** | ***0.05*** |
| 197 | D197I | 0.18 | 3.13 | 4.06 | 2.64 | ***0.05*** | 0.30 |
| 197 | D197K | 0.14 | ***0.05*** | ***0.05*** | ***0.05*** | 0.1 | 0.10 |
| 197 | D197L | 0.16 | ***0.05*** | ***0.05*** | 19.01 | 0.1 | 0.18 |
| 197 | D197M | 0.18 | 1.52 | 0.68 | 1.10 | 0.1 | 0.09 |
| 197 | D197N | 0.86 | 0.93 | 1.16 | 0.88 | 0.5 | 0.85 |
| 197 | D197P | 0.36 | ***0.05*** | ***0.05*** | ***0.05*** | ***0.05*** | ***0.05*** |
| 197 | D197R | 0.13 | ***0.05*** | ***0.05*** | ***0.05*** | ***0.05*** | ***0.05*** |
| 197 | D197S | 0.28 | 0.18 | 0.28 | 0.35 | 0.9 | 0.11 |
| 197 | D197T | 0.53 | 0.94 | 1.20 | 1.14 | 0.5 | 0.88 |
| 197 | D197Y | 0.24 | ***0.05*** | ***0.05*** | ***0.05*** | ***0.05*** | ***0.05*** |
| 198 | V198A | 0.58 | 0.88 | 0.86 | 1.11 | 0.2 | 0.96 |
| 198 | V198C | 0.84 | 0.78 | 1.11 | 1.00 | 0.8 | 0.94 |
| 198 | V198D | 0.20 | 1.23 | 1.82 | 1.45 | 1.1 | 0.34 |
| 198 | V198E | 0.22 | ***0.05*** | 0.06 | 0.10 | ***0.05*** | ***0.05*** |
| 198 | V198F | 0.76 | 0.78 | 0.72 | 1.00 | 0.9 | 0.76 |
| 198 | V198H | 0.20 | 0.57 | 1.01 | 0.96 | 0.2 | 0.18 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 198 | V198I | 0.83 | 0.41 | 0.94 | 0.98 | 1.0 | 0.87 |
| 198 | V198K | 0.29 | ***0.05*** | ***0.05*** | ***0.05*** | ***0.05*** | ***0.05*** |
| 198 | V198L | 0.90 | 0.67 | 0.73 | 0.94 | 1.0 | 1.13 |
| 198 | V198M | 0.75 | 0.78 | 1.03 | 1.08 | 0.8 | 0.96 |
| 198 | V198N | 0.23 | 0.08 | 0.09 | 0.26 | ***0.05*** | ***0.05*** |
| 198 | V198P | 0.45 | ***0.05*** | ***0.05*** | ***0.05*** | ***0.05*** | ***0.05*** |
| 198 | V198Q | 0.16 | ***0.05*** | ***0.05*** | 12.00 | 0.1 | 0.26 |
| 198 | V198R | 0.46 | ***0.05*** | ***0.05*** | ***0.05*** | ***0.05*** | ***0.05*** |
| 198 | V198S | 0.32 | ***0.05*** | ***0.05*** | ***0.05*** | ***0.05*** | ***0.05*** |
| 198 | V198T | 0.42 | 0.99 | 1.05 | 1.06 | 0.2 | 1.05 |
| 198 | V198W | 0.13 | ***0.05*** | ***0.05*** | ***0.05*** | 0.2 | 0.29 |
| 198 | V198Y | 0.53 | 0.67 | 1.19 | 1.12 | 0.8 | 1.00 |
| 199 | M199A | 0.49 | 1.04 | 0.94 | 1.10 | 0.1 | 0.79 |
| 199 | M199C | 0.64 | 0.88 | 0.85 | 0.80 | 0.7 | 0.66 |
| 199 | M199D | 0.16 | ***0.05*** | ***0.05*** | 0.88 | ***0.05*** | ***0.05*** |
| 199 | M199E | 0.17 | ***0.05*** | 8.86 | 0.21 | ***0.05*** | ***0.05*** |
| 199 | M199F | 0.17 | 28.65 | 28.81 | 2.58 | 0.1 | 0.23 |
| 199 | M199G | 0.18 | 1.09 | 0.55 | 0.93 | 0.1 | 0.12 |
| 199 | M199H | 0.19 | 0.52 | ***0.05*** | 0.41 | ***0.05*** | ***0.05*** |
| 199 | M199I | 0.43 | 1.20 | 1.02 | 1.04 | ***0.05*** | 0.75 |
| 199 | M199K | 0.34 | 0.07 | ***0.05*** | ***0.05*** | ***0.05*** | ***0.05*** |
| 199 | M199L | 0.34 | 1.28 | 0.88 | 1.05 | ***0.05*** | 0.51 |
| 199 | M199N | 0.20 | 0.90 | 0.97 | 0.93 | ***0.05*** | 0.22 |
| 199 | M199P | 0.50 | ***0.05*** | ***0.05*** | ***0.05*** | ***0.05*** | ***0.05*** |
| 199 | M199Q | 0.23 | 2.11 | 1.65 | 1.69 | 0.8 | 0.63 |
| 199 | M199S | 0.54 | 1.12 | 0.88 | 1.00 | 0.3 | 0.93 |
| 199 | M199T | 0.53 | 1.22 | 1.06 | 1.02 | 0.3 | 0.87 |
| 199 | M199V | 0.66 | 1.14 | 0.97 | 0.96 | 0.9 | 1.05 |
| 199 | M199W | 0.13 | ***0.05*** | 0.12 | ***0.05*** | ***0.05*** | ***0.05*** |
| 199 | M199Y | 0.13 | ***0.05*** | 0.09 | ***0.05*** | ***0.05*** | ***0.05*** |
| 200 | A200C | 0.70 | 1.32 | 0.86 | 1.16 | 0.6 | 1.17 |
| 200 | A200D | ***0.05*** | ***0.05*** | ***0.05*** | ***0.05*** | ***0.05*** | ***0.05*** |
| 200 | A200E | 0.32 | ***0.05*** | ***0.05*** | ***0.05*** | ***0.05*** | ***0.05*** |
| 200 | A200F | 0.18 | ***0.05*** | ***0.05*** | ***0.05*** | ***0.05*** | ***0.05*** |
| 200 | A200G | 0.83 | 1.19 | 1.05 | 1.08 | 0.6 | 1.08 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 200 | A200H | 0.21 | 4.98 | ***0.05*** | 0.16 | ***0.05*** | ***0.05*** |
| 200 | A200I | 0.26 | 0.23 | ***0.05*** | 0.09 | ***0.05*** | ***0.05*** |
| 200 | A200K | 0.25 | 0.12 | ***0.05*** | ***0.05*** | ***0.05*** | ***0.05*** |
| 200 | A200L | 0.18 | ***0.05*** | ***0.05*** | ***0.05*** | 1.0 | 0.06 |
| 200 | A200M | 0.18 | ***0.05*** | ***0.05*** | ***0.05*** | ***0.05*** | ***0.05*** |
| 200 | A200N | 0.47 | 1.33 | 1.20 | 1.17 | 0.7 | 1.28 |
| 200 | A200P | 0.23 | 0.61 | ***0.05*** | 0.07 | ***0.05*** | ***0.05*** |
| 200 | A200Q | ***0.05*** | ***0.05*** | ***0.05*** | ***0.05*** | ***0.05*** | ***0.05*** |
| 200 | A200R | 0.20 | ***0.05*** | ***0.05*** | 0.24 | ***0.05*** | ***0.05*** |
| 200 | A200S | 0.72 | 1.23 | 0.80 | 1.07 | 0.5 | 0.91 |
| 200 | A200T | 0.35 | 1.73 | 0.95 | 1.35 | 0.2 | 0.71 |
| 200 | A200V | 0.24 | 5.13 | 2.34 | 2.54 | ***0.05*** | 0.41 |
| 200 | A200W | 0.22 | 0.87 | 0.41 | 0.13 | ***0.05*** | ***0.05*** |
| 200 | A200Y | 0.21 | 2.63 | ***0.05*** | 0.10 | ***0.05*** | ***0.05*** |
| 201 | P201A | 0.65 | 0.61 | 0.87 | 1.08 | 0.9 | 0.79 |
| 201 | P201C | 0.47 | 1.07 | 0.95 | 1.30 | 0.2 | 1.06 |
| 201 | P201D | 0.15 | ***0.05*** | ***0.05*** | ***0.05*** | ***0.05*** | ***0.05*** |
| 201 | P201E | 0.29 | ***0.05*** | ***0.05*** | ***0.05*** | ***0.05*** | ***0.05*** |
| 201 | P201F | 0.44 | 1.13 | 0.94 | 1.21 | ***0.05*** | 1.02 |
| 201 | P201G | 0.54 | 0.42 | 1.08 | 1.21 | 0.6 | 0.91 |
| 201 | P201H | 0.40 | 0.65 | 1.21 | 1.15 | 0.1 | 0.90 |
| 201 | P201I | 0.18 | 1.05 | 0.64 | 1.28 | 0.1 | 0.12 |
| 201 | P201K | 0.16 | ***0.05*** | ***0.05*** | 10.07 | ***0.05*** | 0.41 |
| 201 | P201L | 0.25 | 1.65 | 1.71 | 2.07 | ***0.05*** | 0.63 |
| 201 | P201M | 0.25 | 1.13 | 1.49 | 1.68 | ***0.05*** | 0.54 |
| 201 | P201N | 0.41 | 1.05 | 1.26 | 1.38 | 0.1 | 0.75 |
| 201 | P201R | 0.18 | 0.30 | ***0.05*** | 0.39 | 0.4 | 0.06 |
| 201 | P201S | 0.43 | 0.66 | 1.21 | 1.35 | 0.1 | 1.06 |
| 201 | P201T | 0.20 | 3.99 | 4.09 | 3.50 | ***0.05*** | 0.88 |
| 201 | P201V | 0.18 | 8.32 | 11.79 | 6.01 | ***0.05*** | 0.68 |
| 201 | P201W | 0.14 | ***0.05*** | ***0.05*** | ***0.05*** | 0.1 | 0.18 |
| 201 | P201Y | 0.58 | 0.73 | 0.91 | 1.11 | 0.7 | 1.17 |
| 202 | G202A | 0.33 | 0.40 | 0.49 | 0.61 | ***0.05*** | 0.44 |
| 202 | G202C | 0.39 | ***0.05*** | ***0.05*** | ***0.05*** | ***0.05*** | ***0.05*** |
| 202 | G202D | 0.35 | ***0.05*** | 0.08 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 202 | G202E | 0.41 | ***0.05*** | ***0.05*** | ***0.05*** | ***0.05*** | ***0.05*** |
| 202 | G202F | 0.38 | ***0.05*** | ***0.05*** | ***0.05*** | ***0.05*** | ***0.05*** |
| 202 | G202H | 0.38 | ***0.05*** | 0.07 | ***0.05*** | ***0.05*** | ***0.05*** |
| 202 | G202I | 0.40 | ***0.05*** | ***0.05*** | ***0.05*** | ***0.05*** | ***0.05*** |
| 202 | G202K | 0.37 | ***0.05*** | ***0.05*** | ***0.05*** | ***0.05*** | ***0.05*** |
| 202 | G202L | 0.37 | ***0.05*** | ***0.05*** | ***0.05*** | ***0.05*** | ***0.05*** |
| 202 | G202M | 0.33 | ***0.05*** | 0.06 | ***0.05*** | ***0.05*** | ***0.05*** |
| 202 | G202N | 0.36 | ***0.05*** | ***0.05*** | ***0.05*** | ***0.05*** | ***0.05*** |
| 202 | G202P | 0.37 | ***0.05*** | ***0.05*** | ***0.05*** | ***0.05*** | ***0.05*** |
| 202 | G202Q | 0.34 | ***0.05*** | ***0.05*** | ***0.05*** | ***0.05*** | ***0.05*** |
| 202 | G202R | 0.36 | ***0.05*** | ***0.05*** | ***0.05*** | ***0.05*** | ***0.05*** |
| 202 | G202S | 0.16 | ***0.05*** | ***0.05*** | 1.08 | ***0.05*** | ***0.05*** |
| 202 | G202T | 0.35 | ***0.05*** | ***0.05*** | ***0.05*** | ***0.05*** | ***0.05*** |
| 202 | G202V | 0.35 | ***0.05*** | ***0.05*** | ***0.05*** | ***0.05*** | ***0.05*** |
| 202 | G202W | 0.33 | ***0.05*** | ***0.05*** | ***0.05*** | ***0.05*** | ***0.05*** |
| 202 | G202Y | 0.36 | ***0.05*** | ***0.05*** | ***0.05*** | ***0.05*** | ***0.05*** |
| 203 | V203A | 0.51 | 1.07 | 1.26 | 1.24 | 0.3 | 1.48 |
| 203 | V203C | 0.93 | 1.10 | 1.10 | 0.83 | 1.0 | 0.66 |
| 203 | V203D | 0.54 | 1.12 | 1.62 | 1.23 | 1.1 | 1.09 |
| 203 | V203E | 0.57 | 0.88 | 1.05 | 1.13 | 1.1 | 0.88 |
| 203 | V203F | 0.46 | 1.06 | 1.28 | 1.11 | ***0.05*** | 1.00 |
| 203 | V203G | 0.17 | 8.72 | 7.71 | 4.86 | 0.1 | 0.33 |
| 203 | V203H | 0.59 | 0.87 | 0.81 | 0.89 | 0.1 | 0.30 |
| 203 | V203I | 0.81 | 0.63 | 1.05 | 0.93 | 0.8 | 0.77 |
| 203 | V203K | 0.62 | 0.60 | 0.73 | 0.87 | ***0.05*** | 1.10 |
| 203 | V203M | 0.77 | 0.98 | 1.14 | 1.00 | 0.6 | 0.99 |
| 203 | V203N | 0.47 | 1.05 | 1.38 | 1.22 | 0.1 | 0.99 |
| 203 | V203P | 0.23 | ***0.05*** | ***0.05*** | ***0.05*** | ***0.05*** | ***0.05*** |
| 203 | V203Q | 0.62 | 0.90 | 1.18 | 1.13 | 0.5 | 1.19 |
| 203 | V203R | 0.45 | 0.75 | 0.59 | 0.85 | ***0.05*** | 1.03 |
| 203 | V203S | 0.55 | 0.93 | 1.10 | 1.04 | 0.2 | 1.07 |
| 203 | V203T | 0.65 | 0.90 | 1.31 | 1.07 | 0.6 | 1.01 |
| 203 | V203W | 0.54 | 0.91 | 1.14 | 1.19 | ***0.05*** | 1.03 |
| 203 | V203Y | 0.43 | 0.94 | 1.21 | 1.05 | 0.1 | 0.97 |
| 204 | S204A | 0.97 | 0.86 | 1.00 | 0.81 | 1.2 | 1.06 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 204 | S204C | 0.90 | 1.00 | 1.05 | 0.89 | 1.4 | 0.85 |
| 204 | S204F | 0.86 | 0.39 | 0.60 | 0.82 | 0.7 | 1.06 |
| 204 | S204G | 0.88 | 1.01 | 1.19 | 0.75 | 1.1 | 1.50 |
| 204 | S204H | 0.68 | 0.72 | 0.81 | 0.95 | 1.0 | 1.21 |
| 204 | S204I | 0.66 | 0.79 | 0.99 | 0.83 | 0.7 | 0.91 |
| 204 | S204K | 0.90 | 0.74 | 0.64 | 0.87 | 0.1 | 0.93 |
| 204 | S204L | 0.77 | 0.85 | 0.82 | 0.93 | 0.9 | 0.90 |
| 204 | S204M | 0.90 | 0.75 | 1.10 | 0.95 | 0.8 | 0.90 |
| 204 | S204P | 0.22 | 0.06 | ***0.05*** | 0.12 | ***0.05*** | ***0.05*** |
| 204 | S204Q | 0.87 | 0.84 | 1.06 | 0.94 | 1.3 | 0.96 |
| 204 | S204R | 0.83 | 0.42 | 0.60 | 0.78 | ***0.05*** | 0.97 |
| 204 | S204T | 0.92 | 0.78 | 0.86 | 0.80 | 0.9 | 1.09 |
| 204 | S204V | 0.82 | 0.71 | 0.79 | 0.85 | 0.7 | 1.20 |
| 204 | S204W | 0.74 | 0.39 | 0.67 | 0.79 | 0.6 | 0.97 |
| 204 | S204Y | 0.75 | 0.52 | 1.04 | 0.87 | 0.7 | 1.03 |
| 205 | I205A | 0.40 | 0.78 | 0.92 | 0.93 | ***0.05*** | 0.61 |
| 205 | I205C | ***0.05*** | ***0.05*** | ***0.05*** | ***0.05*** | ***0.05*** | ***0.05*** |
| 205 | I205D | 0.27 | 0.35 | ***0.05*** | ***0.05*** | ***0.05*** | ***0.05*** |
| 205 | I205E | 0.23 | 0.46 | ***0.05*** | ***0.05*** | ***0.05*** | ***0.05*** |
| 205 | I205F | 0.27 | 0.27 | ***0.05*** | ***0.05*** | ***0.05*** | ***0.05*** |
| 205 | I205G | 0.19 | ***0.05*** | 0.15 | ***0.05*** | 0.1 | 0.11 |
| 205 | I205H | 0.20 | ***0.05*** | ***0.05*** | 4.55 | 0.2 | 0.08 |
| 205 | I205K | 0.20 | ***0.05*** | 0.99 | 0.30 | ***0.05*** | ***0.05*** |
| 205 | I205L | 0.30 | 1.37 | 1.00 | 0.86 | ***0.05*** | 0.53 |
| 205 | I205M | 0.20 | ***0.05*** | ***0.05*** | 4.69 | 0.1 | 0.26 |
| 205 | I205N | ***0.05*** | ***0.05*** | ***0.05*** | ***0.05*** | ***0.05*** | ***0.05*** |
| 205 | I205P | 0.23 | 0.79 | ***0.05*** | ***0.05*** | ***0.05*** | ***0.05*** |
| 205 | I205Q | 0.20 | ***0.05*** | ***0.05*** | 3.32 | ***0.05*** | 0.34 |
| 205 | I205R | 0.18 | ***0.05*** | 0.12 | ***0.05*** | ***0.05*** | ***0.05*** |
| 205 | I205S | 0.20 | ***0.05*** | ***0.05*** | 5.15 | ***0.05*** | 0.48 |
| 205 | I205T | 0.77 | 1.60 | 1.20 | 1.11 | 0.8 | 1.29 |
| 205 | I205V | 0.95 | 1.25 | 1.08 | 1.11 | 1.3 | 1.03 |
| 205 | I205W | 0.20 | ***0.05*** | ***0.05*** | 0.13 | ***0.05*** | ***0.05*** |
| 205 | I205Y | 0.19 | ***0.05*** | ***0.05*** | ***0.05*** | ***0.05*** | ***0.05*** |
| 206 | Q206A | 1.09 | 0.84 | 1.24 | 1.05 | 0.7 | 0.72 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 206 | Q206C | 1.10 | 1.13 | 1.03 | 1.12 | 1.4 | 0.94 |
| 206 | Q206D | 1.02 | 1.23 | 1.04 | 1.01 | 1.3 | 0.84 |
| 206 | Q206E | 1.01 | 1.26 | 1.14 | 1.16 | 1.4 | 0.97 |
| 206 | Q206F | 0.98 | 1.00 | 0.66 | 0.95 | ***0.05*** | 0.94 |
| 206 | Q206G | 1.03 | 0.91 | 0.66 | 1.03 | ***0.05*** | 0.72 |
| 206 | Q206H | 1.06 | 1.00 | 0.97 | 1.11 | 1.0 | 0.92 |
| 206 | Q206I | 1.12 | 1.00 | 0.99 | 1.04 | 0.5 | 0.87 |
| 206 | Q206K | 1.11 | 0.84 | 0.75 | 1.13 | 0.1 | 0.86 |
| 206 | Q206L | 1.03 | 0.89 | 0.93 | 0.94 | 0.7 | 1.02 |
| 206 | Q206M | 0.77 | 1.09 | 0.93 | 1.06 | 0.8 | 0.95 |
| 206 | Q206N | 1.03 | 1.07 | 1.08 | 1.06 | 1.1 | 0.91 |
| 206 | Q206P | 0.94 | 1.01 | 1.00 | 1.02 | 0.9 | 0.86 |
| 206 | Q206R | 1.05 | 0.78 | 0.52 | 0.98 | ***0.05*** | 1.03 |
| 206 | Q206S | 0.92 | 1.21 | 0.98 | 1.00 | 0.9 | 0.97 |
| 206 | Q206T | 0.99 | 0.91 | 0.95 | 1.10 | 0.8 | 0.92 |
| 206 | Q206V | 1.01 | 1.04 | 1.19 | 1.11 | 0.6 | 1.07 |
| 206 | Q206W | 0.81 | 0.80 | 0.69 | 1.05 | ***0.05*** | 0.91 |
| 206 | Q206Y | 1.04 | 0.96 | 0.96 | 1.18 | 0.9 | 0.93 |
| 207 | S207A | 0.64 | 0.84 | 0.97 | 0.93 | ***0.05*** | 0.95 |
| 207 | S207C | 0.27 | 0.16 | ***0.05*** | 0.49 | ***0.05*** | ***0.05*** |
| 207 | S207D | 0.28 | ***0.05*** | ***0.05*** | ***0.05*** | ***0.05*** | ***0.05*** |
| 207 | S207E | 0.32 | ***0.05*** | ***0.05*** | ***0.05*** | ***0.05*** | ***0.05*** |
| 207 | S207F | 0.29 | ***0.05*** | ***0.05*** | ***0.05*** | ***0.05*** | ***0.05*** |
| 207 | S207G | 0.86 | 0.64 | 0.62 | 0.92 | ***0.05*** | 0.50 |
| 207 | S207H | ***0.05*** | ***0.05*** | ***0.05*** | ***0.05*** | ***0.05*** | ***0.05*** |
| 207 | S207I | ***0.05*** | ***0.05*** | ***0.05*** | ***0.05*** | ***0.05*** | ***0.05*** |
| 207 | S207K | 0.37 | ***0.05*** | ***0.05*** | ***0.05*** | ***0.05*** | ***0.05*** |
| 207 | S207L | 0.35 | ***0.05*** | ***0.05*** | ***0.05*** | ***0.05*** | ***0.05*** |
| 207 | S207M | 0.33 | 0.21 | 0.07 | 0.38 | ***0.05*** | ***0.05*** |
| 207 | S207N | ***0.05*** | ***0.05*** | ***0.05*** | ***0.05*** | ***0.05*** | ***0.05*** |
| 207 | S207P | 0.44 | 0.06 | ***0.05*** | ***0.05*** | ***0.05*** | ***0.05*** |
| 207 | S207Q | 0.19 | 0.21 | ***0.05*** | ***0.05*** | ***0.05*** | ***0.05*** |
| 207 | S207R | 0.43 | ***0.05*** | ***0.05*** | ***0.05*** | ***0.05*** | ***0.05*** |
| 207 | S207T | 0.65 | 0.20 | 0.14 | 0.42 | ***0.05*** | 0.11 |
| 207 | S207V | 0.21 | 1.19 | 2.46 | 0.17 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 207 | S207W | 0.25 | ***0.05*** | 0.15 | ***0.05*** | ***0.05*** | ***0.05*** |
| 207 | S207Y | 0.25 | 0.18 | ***0.05*** | 0.09 | ***0.05*** | ***0.05*** |
| 208 | T208A | 0.89 | 1.28 | 1.15 | 1.12 | 0.1 | 1.02 |
| 208 | T208C | 0.82 | 1.48 | 1.13 | 1.24 | 0.8 | 1.14 |
| 208 | T208D | 0.27 | 0.78 | 0.73 | 0.35 | 0.2 | 0.20 |
| 208 | T208E | 0.25 | 0.42 | ***0.05*** | ***0.05*** | ***0.05*** | ***0.05*** |
| 208 | T208F | ***0.05*** | ***0.05*** | ***0.05*** | ***0.05*** | ***0.05*** | ***0.05*** |
| 208 | T208G | 0.19 | ***0.05*** | ***0.05*** | ***0.05*** | 0.2 | 0.11 |
| 208 | T208H | 0.18 | ***0.05*** | ***0.05*** | ***0.05*** | ***0.05*** | ***0.05*** |
| 208 | T208I | ***0.05*** | ***0.05*** | ***0.05*** | ***0.05*** | ***0.05*** | ***0.05*** |
| 208 | T208K | 0.24 | 0.73 | 0.06 | ***0.05*** | ***0.05*** | ***0.05*** |
| 208 | T208L | 0.87 | 1.40 | 1.12 | 1.14 | ***0.05*** | 1.18 |
| 208 | T208M | 0.22 | 5.66 | 6.03 | 1.79 | 0.4 | 0.37 |
| 208 | T208N | ***0.05*** | ***0.05*** | ***0.05*** | ***0.05*** | ***0.05*** | ***0.05*** |
| 208 | T208P | 0.41 | 1.68 | 1.07 | 1.20 | ***0.05*** | 0.69 |
| 208 | T208Q | 0.29 | 0.15 | 0.10 | ***0.05*** | ***0.05*** | ***0.05*** |
| 208 | T208R | 0.18 | ***0.05*** | ***0.05*** | ***0.05*** | ***0.05*** | ***0.05*** |
| 208 | T208S | 0.81 | 1.20 | 1.16 | 1.01 | 0.2 | 1.24 |
| 208 | T208V | 0.62 | 1.32 | 1.44 | 1.20 | ***0.05*** | 0.94 |
| 208 | T208W | 0.17 | ***0.05*** | ***0.05*** | ***0.05*** | ***0.05*** | ***0.05*** |
| 208 | T208Y | 0.19 | ***0.05*** | ***0.05*** | 0.73 | ***0.05*** | ***0.05*** |
| 209 | L209A | 1.06 | 0.51 | 0.27 | 0.77 | 0.6 | 0.43 |
| 209 | L209C | 0.82 | 1.41 | 1.04 | 1.05 | 0.9 | 1.53 |
| 209 | L209D | ***0.05*** | ***0.05*** | ***0.05*** | ***0.05*** | ***0.05*** | ***0.05*** |
| 209 | L209E | 0.98 | 0.69 | 0.36 | 0.76 | 0.8 | 0.71 |
| 209 | L209F | 0.72 | 1.26 | 1.05 | 1.15 | 0.2 | 2.25 |
| 209 | L209G | 1.00 | 0.51 | 0.33 | 0.60 | 0.3 | 0.26 |
| 209 | L209H | 0.64 | 0.45 | 0.25 | 0.62 | 0.6 | 0.14 |
| 209 | L209I | ***0.05*** | ***0.05*** | ***0.05*** | ***0.05*** | ***0.05*** | ***0.05*** |
| 209 | L209K | 0.88 | 0.81 | 0.69 | 1.05 | 0.3 | 1.20 |
| 209 | L209M | 0.71 | 1.26 | 1.09 | 1.16 | 0.8 | 1.83 |
| 209 | L209N | ***0.05*** | ***0.05*** | ***0.05*** | ***0.05*** | ***0.05*** | ***0.05*** |
| 209 | L209P | 0.22 | 1.24 | 0.26 | ***0.05*** | ***0.05*** | ***0.05*** |
| 209 | L209Q | 0.84 | 0.97 | 0.44 | 0.89 | 0.7 | 0.99 |
| 209 | L209R | 0.81 | 0.63 | 0.31 | 0.90 | ***0.05*** | 0.65 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 209 | L209S | 0.99 | 0.52 | 0.27 | 0.63 | 0.5 | 0.36 |
| 209 | L209T | 0.81 | 0.77 | 0.35 | 0.82 | 0.5 | 0.88 |
| 209 | L209V | 0.83 | 1.20 | 1.01 | 1.00 | 0.6 | 0.82 |
| 209 | L209W | 0.78 | 1.43 | 0.96 | 1.11 | 0.3 | 1.27 |
| 209 | L209Y | 0.62 | 0.95 | 0.64 | 1.01 | 0.1 | 1.47 |
| 210 | P210A | 0.66 | 1.12 | 0.81 | 0.89 | 0.3 | 0.93 |
| 210 | P210C | 0.42 | 1.52 | 0.90 | 0.96 | 0.2 | 0.75 |
| 210 | P210D | 0.32 | 1.40 | 0.78 | 1.18 | 0.1 | 0.26 |
| 210 | P210E | 0.37 | 1.67 | 1.08 | 1.31 | 0.4 | 0.69 |
| 210 | P210F | 0.21 | 10.28 | 3.47 | 2.00 | 0.1 | 0.11 |
| 210 | P210G | 0.32 | 1.78 | 1.00 | 1.26 | ***0.05*** | 0.58 |
| 210 | P210H | ***0.05*** | ***0.05*** | ***0.05*** | ***0.05*** | ***0.05*** | ***0.05*** |
| 210 | P210I | 0.46 | 1.04 | 0.88 | 1.19 | ***0.05*** | 0.61 |
| 210 | P210K | 0.33 | 1.08 | 0.84 | 1.28 | ***0.05*** | 0.60 |
| 210 | P210L | 0.20 | ***0.05*** | ***0.05*** | 15.17 | ***0.05*** | 0.31 |
| 210 | P210M | 0.29 | 1.15 | 1.11 | 1.48 | ***0.05*** | 0.43 |
| 210 | P210N | ***0.05*** | ***0.05*** | ***0.05*** | ***0.05*** | ***0.05*** | ***0.05*** |
| 210 | P210Q | 0.27 | 2.95 | 2.26 | 2.01 | ***0.05*** | 0.84 |
| 210 | P210R | 0.23 | 3.30 | 2.81 | 2.38 | ***0.05*** | 0.46 |
| 210 | P210S | 0.58 | 1.35 | 1.10 | 1.15 | 0.4 | 1.25 |
| 210 | P210T | 0.55 | 1.15 | 1.10 | 1.34 | 0.3 | 1.10 |
| 210 | P210V | 0.49 | 1.36 | 1.24 | 1.13 | 0.2 | 0.75 |
| 210 | P210W | 0.18 | ***0.05*** | ***0.05*** | ***0.05*** | 0.1 | 0.12 |
| 210 | P210Y | 0.24 | 0.34 | 0.17 | 0.21 | ***0.05*** | ***0.05*** |
| 211 | G211A | 0.90 | 1.54 | 1.09 | 1.04 | 1.0 | 0.92 |
| 211 | G211C | 1.13 | 0.68 | 1.18 | 0.94 | 1.1 | 0.86 |
| 211 | G211D | 0.95 | 1.52 | 1.15 | 0.99 | 1.4 | 0.95 |
| 211 | G211E | 0.92 | 1.31 | 1.08 | 1.10 | 1.3 | 0.90 |
| 211 | G211F | 0.85 | 1.21 | 1.09 | 1.15 | 0.6 | 1.08 |
| 211 | G211H | ***0.05*** | ***0.05*** | ***0.05*** | ***0.05*** | ***0.05*** | ***0.05*** |
| 211 | G211I | ***0.05*** | ***0.05*** | ***0.05*** | ***0.05*** | ***0.05*** | ***0.05*** |
| 211 | G211K | 0.90 | 1.05 | 0.90 | 0.95 | 0.4 | 1.03 |
| 211 | G211L | 0.67 | 1.22 | 1.18 | 1.12 | 0.7 | 0.86 |
| 211 | G211M | 0.88 | 1.22 | 1.21 | 0.99 | 0.9 | 0.96 |
| 211 | G211N | 0.92 | 1.29 | 1.12 | 1.00 | 1.0 | 0.99 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 211 | G211P | 0.86 | 1.32 | 1.66 | 1.01 | 0.6 | 0.99 |
| 211 | G211Q | 0.77 | 1.26 | 1.01 | 1.18 | 1.0 | 1.07 |
| 211 | G211R | 0.76 | 0.67 | 0.81 | 0.96 | 0.2 | 0.92 |
| 211 | G211S | 0.95 | 1.25 | 1.18 | 1.01 | 0.9 | 1.01 |
| 211 | G211T | 0.78 | 1.37 | 1.35 | 1.15 | 0.7 | 1.07 |
| 211 | G211V | 0.76 | 1.33 | 1.20 | 0.92 | 0.5 | 0.93 |
| 211 | G211W | 0.80 | 1.33 | 1.08 | 1.18 | 0.2 | 1.04 |
| 211 | G211Y | 0.86 | 1.01 | 1.27 | 1.16 | 0.8 | 1.04 |
| 212 | N212A | 1.18 | 0.98 | 1.29 | 0.99 | 0.8 | 0.67 |
| 212 | N212C | 1.06 | 1.04 | 1.20 | 0.92 | 0.9 | 1.13 |
| 212 | N212D | ***0.05*** | ***0.05*** | ***0.05*** | ***0.05*** | ***0.05*** | ***0.05*** |
| 212 | N212E | 0.97 | 1.44 | 1.12 | 0.93 | 1.2 | 0.75 |
| 212 | N212F | 1.08 | 0.91 | 1.01 | 0.95 | 0.5 | 0.75 |
| 212 | N212G | 0.80 | 1.14 | 1.32 | 0.98 | 0.9 | 0.92 |
| 212 | N212H | 0.95 | 1.22 | 1.11 | 0.94 | 0.6 | 1.21 |
| 212 | N212I | 0.49 | 0.41 | 0.41 | 0.24 | 0.5 | 0.20 |
| 212 | N212K | 0.79 | 0.82 | 0.75 | 0.99 | 0.1 | 0.85 |
| 212 | N212L | 0.83 | 0.63 | 0.69 | 0.76 | 0.2 | 0.75 |
| 212 | N212M | 1.06 | 1.08 | 1.04 | 1.09 | 0.5 | 0.87 |
| 212 | N212P | 0.99 | 0.96 | 0.85 | 1.00 | 0.3 | 0.78 |
| 212 | N212Q | 0.95 | 0.97 | 0.80 | 0.89 | 0.9 | 0.98 |
| 212 | N212R | 0.83 | 0.74 | 0.79 | 1.06 | 0.2 | 0.83 |
| 212 | N212S | 1.09 | 1.12 | 0.96 | 1.03 | 0.8 | 0.77 |
| 212 | N212T | 0.23 | 6.10 | 4.36 | 2.96 | 0.6 | 1.31 |
| 212 | N212V | 0.77 | 0.97 | 0.99 | 1.08 | 0.6 | 0.83 |
| 212 | N212W | ***0.05*** | ***0.05*** | ***0.05*** | ***0.05*** | ***0.05*** | ***0.05*** |
| 212 | N212Y | 0.93 | 1.05 | 0.85 | 0.96 | 0.4 | 0.98 |
| 213 | K213A | 1.05 | 1.23 | 1.22 | 0.95 | 1.4 | 0.91 |
| 213 | K213C | 0.96 | 1.03 | 1.03 | 0.86 | 1.4 | 0.99 |
| 213 | K213D | 1.01 | 1.13 | 1.20 | 0.89 | 1.4 | 0.86 |
| 213 | K213E | 0.96 | 0.99 | 1.09 | 0.89 | 1.5 | 0.84 |
| 213 | K213F | 0.92 | 1.20 | 1.26 | 0.98 | 1.1 | 0.93 |
| 213 | K213H | 1.01 | 1.29 | 1.07 | 0.97 | 1.3 | 1.09 |
| 213 | K213I | 1.02 | 1.01 | 1.14 | 0.94 | 1.2 | 0.96 |
| 213 | K213L | 1.03 | 1.13 | 1.17 | 1.03 | 1.3 | 1.17 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 213 | K213M | 1.06 | 1.13 | 1.17 | 1.04 | 1.5 | 1.03 |
| 213 | K213N | 1.02 | 1.26 | 1.17 | 1.00 | 1.7 | 0.95 |
| 213 | K213Q | 1.04 | 1.36 | 1.01 | 0.97 | 1.5 | 1.04 |
| 213 | K213R | 0.78 | 1.24 | 1.15 | 0.83 | 1.2 | 0.98 |
| 213 | K213S | 0.82 | 1.17 | 1.07 | 0.96 | 1.5 | 0.85 |
| 213 | K213T | 0.91 | 1.35 | 1.21 | 0.85 | 1.5 | 0.94 |
| 213 | K213V | 0.85 | 1.16 | 1.16 | 1.06 | 1.1 | 0.99 |
| 213 | K213W | 0.80 | 1.11 | 1.10 | 0.98 | 0.9 | 1.13 |
| 213 | K213Y | 0.85 | 1.15 | 1.01 | 0.97 | 1.3 | 0.96 |
| 214 | Y214A | 0.17 | 2.41 | 1.57 | 0.71 | 0.2 | 0.07 |
| 214 | Y214C | 0.54 | 0.95 | 0.82 | 0.84 | ***0.05*** | 0.64 |
| 214 | Y214D | 0.18 | 3.22 | 3.18 | 1.71 | 0.2 | 0.12 |
| 214 | Y214F | 0.97 | 1.15 | 1.14 | 0.95 | ***0.05*** | 0.87 |
| 214 | Y214G | 0.20 | 0.38 | ***0.05*** | 0.07 | ***0.05*** | ***0.05*** |
| 214 | Y214H | 0.36 | 1.23 | 1.03 | 1.28 | ***0.05*** | 0.81 |
| 214 | Y214I | 0.86 | 1.04 | 1.05 | 1.01 | ***0.05*** | 0.95 |
| 214 | Y214K | 0.15 | ***0.05*** | ***0.05*** | ***0.05*** | 0.3 | 0.07 |
| 214 | Y214L | 0.73 | 1.10 | 0.92 | 0.97 | ***0.05*** | 0.84 |
| 214 | Y214M | 0.41 | 0.85 | 0.74 | 1.02 | ***0.05*** | 0.33 |
| 214 | Y214N | 0.19 | 1.65 | 1.59 | 1.29 | 0.7 | 0.18 |
| 214 | Y214P | 0.22 | 2.06 | 2.29 | 1.41 | ***0.05*** | 0.72 |
| 214 | Y214Q | 0.36 | 1.27 | 0.76 | 0.95 | ***0.05*** | 0.51 |
| 214 | Y214R | 0.18 | 0.80 | 0.28 | 0.12 | ***0.05*** | ***0.05*** |
| 214 | Y214S | 0.18 | 2.22 | 1.06 | 1.50 | 0.2 | 0.10 |
| 214 | Y214T | 0.50 | 1.03 | 0.90 | 0.98 | ***0.05*** | 0.81 |
| 214 | Y214V | 0.65 | 1.01 | 0.94 | 0.97 | ***0.05*** | 0.91 |
| 214 | Y214W | 0.69 | 1.24 | 1.01 | 1.15 | ***0.05*** | 0.91 |
| 215 | G215A | 0.99 | 1.16 | 0.99 | 0.98 | 0.6 | 0.58 |
| 215 | G215C | 1.00 | 1.03 | 1.06 | 0.82 | 0.8 | 0.70 |
| 215 | G215D | 0.89 | 1.33 | 1.10 | 1.06 | 1.0 | 0.70 |
| 215 | G215E | 1.04 | 1.22 | 1.16 | 0.84 | 1.0 | 0.75 |
| 215 | G215F | ***0.05*** | ***0.05*** | ***0.05*** | ***0.05*** | ***0.05*** | ***0.05*** |
| 215 | G215H | 0.64 | 1.16 | 0.76 | 1.05 | 0.1 | 0.72 |
| 215 | G215I | 0.56 | 1.23 | 0.85 | 1.09 | ***0.05*** | 0.64 |
| 215 | G215K | 0.95 | 0.95 | 0.77 | 0.95 | ***0.05*** | 0.62 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 215 | G215L | 0.26 | 0.07 | ***0.05*** | 0.12 | ***0.05*** | ***0.05*** |
| 215 | G215M | 0.72 | 1.23 | 1.00 | 0.82 | 0.1 | 0.59 |
| 215 | G215N | 0.70 | 1.11 | 1.07 | 0.97 | ***0.05*** | 0.63 |
| 215 | G215P | 0.34 | ***0.05*** | ***0.05*** | ***0.05*** | ***0.05*** | ***0.05*** |
| 215 | G215Q | 0.80 | 1.37 | 0.96 | 0.97 | 0.1 | 0.67 |
| 215 | G215R | 0.74 | 0.76 | 0.74 | 0.83 | ***0.05*** | 0.66 |
| 215 | G215S | 0.84 | 1.12 | 0.94 | 0.93 | 0.1 | 0.72 |
| 215 | G215T | 0.61 | 1.07 | 0.96 | 1.07 | ***0.05*** | 0.83 |
| 215 | G215V | 0.36 | 1.52 | 0.90 | 1.15 | 0.1 | 0.51 |
| 215 | G215W | 0.48 | 1.00 | 0.88 | 0.91 | 0.1 | 0.71 |
| 215 | G215Y | 0.49 | 1.04 | 0.77 | 1.05 | ***0.05*** | 0.64 |
| 216 | A216C | 0.97 | 1.21 | 1.11 | 0.92 | 1.2 | 0.89 |
| 216 | A216D | 0.76 | 1.22 | 1.28 | 1.19 | 1.2 | 0.95 |
| 216 | A216E | 1.20 | 1.36 | 0.92 | 1.03 | 1.4 | 0.75 |
| 216 | A216F | 1.22 | 1.20 | 1.21 | 0.87 | 1.2 | 0.91 |
| 216 | A216G | 1.00 | 0.99 | 0.78 | 0.88 | 0.8 | 0.90 |
| 216 | A216H | 1.03 | 0.86 | 1.01 | 0.93 | 0.9 | 0.79 |
| 216 | A216I | 1.17 | 0.92 | 0.94 | 1.01 | 1.0 | 0.81 |
| 216 | A216K | 0.94 | 0.78 | 0.98 | 0.87 | 1.0 | 0.55 |
| 216 | A216L | 0.96 | 0.90 | 0.78 | 0.83 | 0.9 | 1.05 |
| 216 | A216M | 0.82 | 0.85 | 1.07 | 0.90 | 1.1 | 0.82 |
| 216 | A216N | 1.03 | 0.97 | 1.11 | 1.07 | 1.1 | 0.72 |
| 216 | A216P | 0.90 | 1.09 | 0.94 | 1.11 | 1.2 | 1.00 |
| 216 | A216Q | 1.03 | 0.96 | 1.14 | 1.09 | 0.9 | 0.94 |
| 216 | A216R | 1.12 | 0.54 | 0.62 | 0.92 | 0.1 | 0.90 |
| 216 | A216S | 0.73 | 1.00 | 1.09 | 1.22 | 0.9 | 1.03 |
| 216 | A216T | ***0.05*** | ***0.05*** | ***0.05*** | ***0.05*** | ***0.05*** | ***0.05*** |
| 216 | A216V | 0.92 | 1.13 | 0.93 | 0.94 | 0.9 | 1.15 |
| 216 | A216W | 0.96 | 0.55 | 0.86 | 0.77 | 0.9 | 0.73 |
| 216 | A216Y | 0.81 | 0.76 | 0.90 | 0.96 | 1.1 | 0.88 |
| 217 | Y217A | 0.38 | 0.16 | 0.08 | 0.14 | ***0.05*** | ***0.05*** |
| 217 | Y217C | 1.03 | 0.98 | 0.86 | 0.95 | 1.6 | 1.34 |
| 217 | Y217D | 1.01 | 1.13 | 1.07 | 1.21 | 1.1 | 0.34 |
| 217 | Y217E | 1.00 | 1.40 | 0.99 | 1.18 | 1.3 | 0.54 |
| 217 | Y217F | 1.04 | 0.99 | 1.03 | 0.97 | 1.0 | 1.35 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 217 | Y217G | 0.85 | 0.81 | 0.72 | 0.95 | 1.0 | 0.97 |
| 217 | Y217H | 0.96 | 0.97 | 0.90 | 1.15 | 1.1 | 0.76 |
| 217 | Y217I | 0.84 | 1.16 | 1.24 | 1.14 | 0.7 | 1.53 |
| 217 | Y217K | 1.02 | 0.80 | 0.58 | 0.97 | 1.0 | 1.06 |
| 217 | Y217L | 0.98 | 1.34 | 0.97 | 1.07 | 1.4 | 3.46 |
| 217 | Y217M | 0.93 | 1.31 | 1.22 | 1.07 | 1.5 | 1.80 |
| 217 | Y217N | 0.78 | 0.94 | 0.65 | 0.91 | 1.0 | 1.21 |
| 217 | Y217P | 0.28 | 0.54 | 0.18 | 0.57 | 0.1 | 0.18 |
| 217 | Y217Q | 0.92 | 1.19 | 1.29 | 1.11 | 1.2 | 1.05 |
| 217 | Y217R | 1.00 | 0.80 | 0.54 | 1.01 | 0.9 | 0.87 |
| 217 | Y217S | 0.83 | 1.11 | 0.95 | 1.01 | 1.0 | 1.63 |
| 217 | Y217T | 0.80 | 1.03 | 0.63 | 1.10 | 0.8 | 1.15 |
| 217 | Y217V | 0.75 | 1.10 | 0.98 | 1.22 | 0.7 | 1.03 |
| 217 | Y217W | 0.80 | 0.76 | 0.68 | 0.79 | 1.0 | 0.71 |
| 218 | N218A | 0.99 | 0.94 | 0.92 | 0.97 | 0.7 | 1.13 |
| 218 | N218C | 1.05 | 1.06 | 0.86 | 0.83 | 1.1 | 0.76 |
| 218 | N218D | ***0.05*** | ***0.05*** | ***0.05*** | ***0.05*** | ***0.05*** | ***0.05*** |
| 218 | N218E | 0.90 | 1.03 | 1.09 | 0.85 | 1.1 | 0.59 |
| 218 | N218F | 0.77 | 0.62 | 0.55 | 0.78 | 0.2 | 0.97 |
| 218 | N218G | 0.88 | 1.06 | 1.07 | 1.01 | 0.5 | 1.23 |
| 218 | N218H | 0.94 | 1.02 | 0.93 | 0.91 | 0.9 | 0.61 |
| 218 | N218I | 0.52 | 0.45 | 0.43 | 0.57 | 0.1 | 0.36 |
| 218 | N218K | 0.76 | 0.55 | 0.59 | 0.93 | ***0.05*** | 1.14 |
| 218 | N218L | 0.46 | 0.66 | 0.69 | 0.64 | ***0.05*** | 0.62 |
| 218 | N218M | 0.76 | 0.95 | 0.74 | 0.75 | 0.4 | 0.94 |
| 218 | N218P | 0.29 | 1.56 | 0.82 | 1.19 | 0.2 | 0.60 |
| 218 | N218Q | ***0.05*** | ***0.05*** | ***0.05*** | ***0.05*** | ***0.05*** | ***0.05*** |
| 218 | N218R | 0.65 | 0.54 | 0.45 | 0.69 | ***0.05*** | 1.16 |
| 218 | N218S | 1.00 | 1.00 | 0.99 | 1.07 | 1.2 | 1.07 |
| 218 | N218T | 0.93 | 0.62 | 0.73 | 0.73 | 0.9 | 0.58 |
| 218 | N218V | 0.48 | 0.51 | 0.64 | 0.50 | 0.2 | 0.77 |
| 218 | N218W | 0.70 | 0.65 | 0.59 | 0.65 | 0.3 | 0.68 |
| 218 | N218Y | 0.70 | 0.62 | 0.75 | 0.70 | 0.2 | 1.08 |
| 219 | G219A | 0.82 | 0.14 | ***0.05*** | 0.08 | 0.3 | 0.19 |
| 219 | G219C | 0.53 | 0.83 | 0.71 | 0.65 | 1.0 | 0.60 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 219 | G219D | 0.46 | ***0.05*** | ***0.05*** | ***0.05*** | 0.4 | 0.23 |
| 219 | G219E | 0.33 | 0.09 | ***0.05*** | ***0.05*** | 3.4 | 0.40 |
| 219 | G219F | 0.35 | ***0.05*** | 0.07 | ***0.05*** | 0.1 | 0.48 |
| 219 | G219H | 0.46 | ***0.05*** | 0.07 | ***0.05*** | 0.7 | 0.08 |
| 219 | G219I | 0.24 | 0.10 | 0.31 | ***0.05*** | 0.4 | 0.12 |
| 219 | G219K | 0.27 | 0.27 | ***0.05*** | 0.08 | ***0.05*** | 0.41 |
| 219 | G219L | 0.54 | 0.68 | 0.70 | 0.70 | 0.2 | 0.89 |
| 219 | G219M | 0.32 | 0.21 | ***0.05*** | 0.06 | 0.7 | 0.44 |
| 219 | G219N | ***0.05*** | ***0.05*** | ***0.05*** | ***0.05*** | ***0.05*** | ***0.05*** |
| 219 | G219P | 0.22 | 0.20 | ***0.05*** | ***0.05*** | 1.0 | 0.65 |
| 219 | G219Q | 0.31 | 0.35 | ***0.05*** | ***0.05*** | 1.3 | 0.42 |
| 219 | G219R | 0.26 | 0.30 | 0.08 | ***0.05*** | 0.7 | 0.43 |
| 219 | G219S | 1.04 | ***0.05*** | ***0.05*** | ***0.05*** | 0.9 | 0.19 |
| 219 | G219T | 0.22 | 0.28 | 0.34 | 0.08 | 0.8 | 0.58 |
| 219 | G219V | 0.25 | 0.22 | ***0.05*** | ***0.05*** | 0.5 | 0.57 |
| 219 | G219W | 0.27 | 0.15 | ***0.05*** | ***0.05*** | 0.6 | 0.55 |
| 219 | G219Y | 0.32 | 0.18 | ***0.05*** | ***0.05*** | 0.5 | 0.45 |
| 220 | T220A | 0.58 | 0.71 | 0.89 | 1.12 | ***0.05*** | ***0.05*** |
| 220 | T220C | 0.63 | 0.69 | 0.53 | 0.71 | ***0.05*** | ***0.05*** |
| 220 | T220D | 0.86 | 0.27 | 0.32 | 0.30 | ***0.05*** | ***0.05*** |
| 220 | T220E | 0.59 | ***0.05*** | ***0.05*** | 0.18 | ***0.05*** | ***0.05*** |
| 220 | T220F | 0.28 | 0.08 | 0.15 | 0.06 | ***0.05*** | ***0.05*** |
| 220 | T220G | 0.53 | 0.19 | 0.06 | 0.40 | ***0.05*** | ***0.05*** |
| 220 | T220H | ***0.05*** | ***0.05*** | ***0.05*** | ***0.05*** | ***0.05*** | ***0.05*** |
| 220 | T220I | 0.28 | ***0.05*** | ***0.05*** | ***0.05*** | ***0.05*** | ***0.05*** |
| 220 | T220K | 0.26 | ***0.05*** | ***0.05*** | ***0.05*** | ***0.05*** | ***0.05*** |
| 220 | T220L | 0.26 | ***0.05*** | 0.29 | 0.14 | ***0.05*** | ***0.05*** |
| 220 | T220M | 0.42 | ***0.05*** | ***0.05*** | ***0.05*** | ***0.05*** | ***0.05*** |
| 220 | T220N | 0.36 | 0.07 | ***0.05*** | 0.13 | ***0.05*** | ***0.05*** |
| 220 | T220P | 0.26 | 0.10 | 0.26 | ***0.05*** | ***0.05*** | ***0.05*** |
| 220 | T220Q | 0.33 | 0.07 | ***0.05*** | ***0.05*** | ***0.05*** | ***0.05*** |
| 220 | T220R | 0.28 | 0.14 | ***0.05*** | 0.07 | ***0.05*** | ***0.05*** |
| 220 | T220S | 0.68 | 1.16 | 1.13 | 0.97 | 0.6 | 0.49 |
| 220 | T220V | 0.38 | 0.39 | 0.41 | 0.76 | ***0.05*** | ***0.05*** |
| 220 | T220W | 0.31 | 0.06 | 0.07 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 220 | T220Y | 0.29 | ***0.05*** | 0.06 | ***0.05*** | ***0.05*** | ***0.05*** |
| 221 | S221A | 1.12 | ***0.05*** | ***0.05*** | ***0.05*** | ***0.05*** | ***0.05*** |
| 221 | S221C | 1.84 | ***0.05*** | ***0.05*** | ***0.05*** | ***0.05*** | ***0.05*** |
| 221 | S221D | 2.07 | ***0.05*** | ***0.05*** | ***0.05*** | ***0.05*** | ***0.05*** |
| 221 | S221E | 1.82 | ***0.05*** | ***0.05*** | ***0.05*** | ***0.05*** | ***0.05*** |
| 221 | S221G | 1.34 | ***0.05*** | ***0.05*** | ***0.05*** | ***0.05*** | ***0.05*** |
| 221 | S221H | 1.05 | ***0.05*** | ***0.05*** | ***0.05*** | ***0.05*** | ***0.05*** |
| 221 | S221K | 0.43 | ***0.05*** | ***0.05*** | ***0.05*** | ***0.05*** | ***0.05*** |
| 221 | S221L | 0.39 | ***0.05*** | ***0.05*** | ***0.05*** | ***0.05*** | ***0.05*** |
| 221 | S221M | 0.41 | ***0.05*** | ***0.05*** | ***0.05*** | ***0.05*** | ***0.05*** |
| 221 | S221N | 1.64 | ***0.05*** | ***0.05*** | ***0.05*** | ***0.05*** | ***0.05*** |
| 221 | S221P | 0.40 | ***0.05*** | ***0.05*** | ***0.05*** | ***0.05*** | ***0.05*** |
| 221 | S221Q | 0.85 | ***0.05*** | ***0.05*** | ***0.05*** | ***0.05*** | ***0.05*** |
| 221 | S221R | 0.36 | 0.08 | ***0.05*** | ***0.05*** | ***0.05*** | ***0.05*** |
| 221 | S221T | 1.09 | ***0.05*** | ***0.05*** | ***0.05*** | ***0.05*** | ***0.05*** |
| 221 | S221V | 0.34 | ***0.05*** | ***0.05*** | ***0.05*** | ***0.05*** | ***0.05*** |
| 221 | S221W | 0.37 | ***0.05*** | ***0.05*** | ***0.05*** | ***0.05*** | ***0.05*** |
| 222 | M222A | 1.49 | 0.94 | 0.68 | 0.92 | 1.0 | 0.48 |
| 222 | M222C | 1.15 | 0.66 | 0.55 | 0.80 | 1.6 | 1.32 |
| 222 | M222D | 0.65 | 0.56 | 0.48 | 0.80 | ***0.05*** | ***0.05*** |
| 222 | M222E | 0.95 | 0.90 | 0.46 | 1.03 | ***0.05*** | ***0.05*** |
| 222 | M222F | 0.86 | 0.62 | 0.73 | 1.00 | 1.4 | 0.06 |
| 222 | M222H | 1.07 | 0.52 | 0.56 | 0.78 | 2.0 | 0.06 |
| 222 | M222I | 1.19 | 0.68 | 0.66 | 1.02 | ***0.05*** | ***0.05*** |
| 222 | M222K | 1.59 | 0.25 | 0.20 | 0.50 | ***0.05*** | ***0.05*** |
| 222 | M222L | 0.98 | 0.48 | 0.60 | 0.76 | 0.6 | 0.13 |
| 222 | M222N | 1.14 | 0.76 | 0.74 | 0.93 | 0.9 | 0.27 |
| 222 | M222P | 1.22 | 0.47 | 0.24 | 0.54 | 0.3 | 0.14 |
| 222 | M222Q | 0.78 | 0.97 | 0.87 | 1.19 | 1.4 | 0.12 |
| 222 | M222R | ***0.05*** | ***0.05*** | ***0.05*** | ***0.05*** | ***0.05*** | ***0.05*** |
| 222 | M222S | 1.15 | 0.77 | 0.62 | 0.87 | 1.0 | 0.95 |
| 222 | M222T | 1.12 | 0.64 | 0.46 | 0.72 | 1.0 | 0.34 |
| 222 | M222V | 1.20 | 0.69 | 0.50 | 0.80 | 0.9 | 0.12 |
| 222 | M222W | 1.89 | 0.36 | 0.21 | 0.74 | 0.7 | 0.09 |
| 222 | M222Y | 1.46 | 0.55 | 0.34 | 0.88 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 223 | A223C | 0.36 | 0.60 | 0.54 | 0.50 | ***0.05*** | 0.14 |
| 223 | A223D | ***0.05*** | ***0.05*** | ***0.05*** | ***0.05*** | ***0.05*** | ***0.05*** |
| 223 | A223E | 0.24 | 0.20 | ***0.05*** | 0.10 | ***0.05*** | ***0.05*** |
| 223 | A223F | 0.23 | 0.42 | ***0.05*** | 0.07 | ***0.05*** | ***0.05*** |
| 223 | A223G | 0.62 | 1.00 | 0.60 | 0.86 | 0.2 | 0.66 |
| 223 | A223H | 0.25 | 0.20 | ***0.05*** | 0.09 | ***0.05*** | ***0.05*** |
| 223 | A223I | ***0.05*** | ***0.05*** | ***0.05*** | ***0.05*** | ***0.05*** | ***0.05*** |
| 223 | A223K | 0.24 | 0.28 | ***0.05*** | 0.16 | ***0.05*** | ***0.05*** |
| 223 | A223L | 0.26 | 0.35 | 0.07 | ***0.05*** | ***0.05*** | ***0.05*** |
| 223 | A223M | 0.27 | 0.26 | ***0.05*** | ***0.05*** | ***0.05*** | ***0.05*** |
| 223 | A223N | ***0.05*** | ***0.05*** | ***0.05*** | ***0.05*** | ***0.05*** | ***0.05*** |
| 223 | A223P | 0.26 | 0.61 | 0.31 | 0.11 | ***0.05*** | ***0.05*** |
| 223 | A223Q | 0.43 | ***0.05*** | ***0.05*** | ***0.05*** | ***0.05*** | ***0.05*** |
| 223 | A223R | 0.24 | 0.30 | ***0.05*** | 0.08 | ***0.05*** | ***0.05*** |
| 223 | A223S | 1.05 | 0.95 | 1.00 | 0.91 | 0.9 | 0.72 |
| 223 | A223T | 0.23 | 1.22 | 0.73 | 0.91 | 0.2 | 0.17 |
| 223 | A223V | 0.17 | ***0.05*** | 0.07 | ***0.05*** | ***0.05*** | ***0.05*** |
| 223 | A223W | 0.22 | 1.54 | 0.83 | 0.23 | ***0.05*** | ***0.05*** |
| 223 | A223Y | 0.20 | ***0.05*** | 0.45 | 0.73 | ***0.05*** | ***0.05*** |
| 224 | S224A | 1.14 | 1.24 | 1.14 | 1.15 | 0.9 | 1.16 |
| 224 | S224C | 1.34 | 1.23 | 1.23 | 1.22 | 1.1 | 0.65 |
| 224 | S224D | 0.22 | 1.59 | 0.58 | 0.29 | ***0.05*** | ***0.05*** |
| 224 | S224E | 0.30 | 0.30 | ***0.05*** | 0.14 | ***0.05*** | ***0.05*** |
| 224 | S224F | 0.23 | 0.62 | ***0.05*** | 0.13 | ***0.05*** | ***0.05*** |
| 224 | S224G | 0.90 | 1.23 | 1.15 | 1.13 | 0.3 | 0.63 |
| 224 | S224H | 0.27 | 0.19 | ***0.05*** | ***0.05*** | ***0.05*** | ***0.05*** |
| 224 | S224I | 0.19 | ***0.05*** | ***0.05*** | 80.86 | ***0.05*** | ***0.05*** |
| 224 | S224K | ***0.05*** | ***0.05*** | ***0.05*** | ***0.05*** | ***0.05*** | ***0.05*** |
| 224 | S224L | 0.20 | ***0.05*** | ***0.05*** | 4.61 | ***0.05*** | ***0.05*** |
| 224 | S224M | 0.26 | 0.30 | ***0.05*** | 0.10 | ***0.05*** | ***0.05*** |
| 224 | S224N | 0.35 | 1.77 | 1.55 | 1.26 | 0.6 | 0.24 |
| 224 | S224P | 0.38 | 0.36 | 0.24 | 0.42 | ***0.05*** | ***0.05*** |
| 224 | S224Q | 0.25 | 2.04 | 0.13 | 0.17 | ***0.05*** | ***0.05*** |
| 224 | S224R | 0.24 | 0.62 | ***0.05*** | ***0.05*** | ***0.05*** | ***0.05*** |
| 224 | S224T | 0.66 | 1.16 | 1.22 | 1.07 | 0.9 | 0.37 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 224 | S224V | 0.97 | 0.89 | 0.90 | 1.05 | 0.9 | 0.06 |
| 224 | S224W | 0.25 | 0.32 | 0.19 | 0.06 | ***0.05*** | ***0.05*** |
| 224 | S224Y | 0.24 | 0.20 | ***0.05*** | 0.13 | ***0.05*** | ***0.05*** |
| 225 | P225A | 1.38 | 0.24 | 0.34 | 0.55 | ***0.05*** | ***0.05*** |
| 225 | P225C | 0.88 | 0.21 | 0.30 | 0.46 | ***0.05*** | ***0.05*** |
| 225 | P225D | ***0.05*** | ***0.05*** | ***0.05*** | ***0.05*** | ***0.05*** | ***0.05*** |
| 225 | P225E | 0.22 | ***0.05*** | ***0.05*** | 0.10 | ***0.05*** | ***0.05*** |
| 225 | P225F | 0.24 | 0.35 | ***0.05*** | 0.12 | ***0.05*** | ***0.05*** |
| 225 | P225G | 1.06 | 0.42 | 0.38 | 0.49 | ***0.05*** | ***0.05*** |
| 225 | P225H | 0.28 | ***0.05*** | ***0.05*** | ***0.05*** | ***0.05*** | ***0.05*** |
| 225 | P225I | 0.61 | 0.19 | 0.23 | 0.34 | ***0.05*** | ***0.05*** |
| 225 | P225K | 0.24 | 0.08 | ***0.05*** | 0.06 | ***0.05*** | ***0.05*** |
| 225 | P225L | 0.24 | ***0.05*** | ***0.05*** | 0.07 | ***0.05*** | ***0.05*** |
| 225 | P225M | 0.30 | 0.26 | 0.34 | 0.26 | ***0.05*** | ***0.05*** |
| 225 | P225N | ***0.05*** | ***0.05*** | ***0.05*** | ***0.05*** | ***0.05*** | ***0.05*** |
| 225 | P225Q | 0.29 | 0.16 | 0.40 | 0.16 | ***0.05*** | ***0.05*** |
| 225 | P225R | 0.23 | 0.16 | ***0.05*** | 0.14 | ***0.05*** | ***0.05*** |
| 225 | P225S | 1.13 | 0.37 | 0.40 | 0.55 | 0.9 | 0.07 |
| 225 | P225T | 0.79 | 0.25 | 0.29 | 0.47 | ***0.05*** | ***0.05*** |
| 225 | P225V | 0.77 | 0.43 | 0.37 | 0.50 | ***0.05*** | ***0.05*** |
| 225 | P225W | 0.22 | 0.27 | ***0.05*** | 0.17 | ***0.05*** | ***0.05*** |
| 225 | P225Y | 0.32 | ***0.05*** | ***0.05*** | 0.07 | ***0.05*** | ***0.05*** |
| 226 | H226A | ***0.05*** | ***0.05*** | ***0.05*** | ***0.05*** | ***0.05*** | ***0.05*** |
| 226 | H226C | 0.75 | 1.15 | 1.26 | 0.89 | ***0.05*** | 1.25 |
| 226 | H226D | 0.31 | ***0.05*** | ***0.05*** | ***0.05*** | ***0.05*** | ***0.05*** |
| 226 | H226E | 0.22 | 1.54 | 1.86 | 1.16 | ***0.05*** | 0.53 |
| 226 | H226F | 0.81 | 1.08 | 1.25 | 1.11 | ***0.05*** | 0.70 |
| 226 | H226G | 0.60 | 1.07 | 0.91 | 1.13 | ***0.05*** | 0.88 |
| 226 | H226I | 0.42 | 1.07 | 1.39 | 1.04 | ***0.05*** | 0.84 |
| 226 | H226K | 0.16 | ***0.05*** | ***0.05*** | 3.21 | 0.1 | 0.15 |
| 226 | H226L | 0.55 | 0.99 | 1.00 | 1.07 | ***0.05*** | 0.94 |
| 226 | H226M | 0.76 | 1.19 | 1.16 | 0.96 | ***0.05*** | 0.97 |
| 226 | H226N | 0.51 | 1.19 | 1.11 | 1.21 | ***0.05*** | 0.84 |
| 226 | H226P | 0.27 | ***0.05*** | ***0.05*** | ***0.05*** | ***0.05*** | ***0.05*** |
| 226 | H226Q | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 226 | H226R | 0.30 | ***0.05*** | 0.09 | ***0.05*** | ***0.05*** | ***0.05*** |
| 226 | H226S | 0.77 | 1.26 | 1.20 | 1.08 | ***0.05*** | 1.06 |
| 226 | H226T | 0.37 | 1.35 | 1.44 | 1.16 | ***0.05*** | 0.90 |
| 226 | H226V | 0.34 | 1.06 | 1.36 | 1.01 | ***0.05*** | 1.20 |
| 226 | H226W | 0.16 | ***0.05*** | ***0.05*** | 3.77 | ***0.05*** | 0.19 |
| 226 | H226Y | 0.73 | 1.22 | 0.97 | 1.06 | ***0.05*** | 0.66 |
| 227 | V227A | 1.03 | 0.99 | 1.26 | 0.83 | 1.0 | 0.81 |
| 227 | V227C | 0.95 | 1.01 | 1.29 | 1.01 | 0.9 | 0.81 |
| 227 | V227D | 0.40 | ***0.05*** | 0.06 | ***0.05*** | ***0.05*** | ***0.05*** |
| 227 | V227E | 0.31 | ***0.05*** | ***0.05*** | ***0.05*** | ***0.05*** | ***0.05*** |
| 227 | V227F | 0.30 | 0.87 | 1.35 | 1.00 | 0.5 | 0.74 |
| 227 | V227G | 0.32 | 1.55 | 1.43 | 1.06 | 0.9 | 0.69 |
| 227 | V227H | 0.34 | ***0.05*** | ***0.05*** | ***0.05*** | ***0.05*** | ***0.05*** |
| 227 | V227I | 0.97 | 1.12 | 1.06 | 0.82 | 0.7 | 0.95 |
| 227 | V227K | 0.34 | ***0.05*** | ***0.05*** | ***0.05*** | ***0.05*** | ***0.05*** |
| 227 | V227L | 0.50 | 1.36 | 1.30 | 1.04 | 0.4 | 0.92 |
| 227 | V227M | 0.36 | 0.76 | 1.18 | 0.87 | 0.7 | 0.75 |
| 227 | V227N | 0.30 | ***0.05*** | ***0.05*** | ***0.05*** | ***0.05*** | ***0.05*** |
| 227 | V227P | 0.27 | ***0.05*** | 0.06 | ***0.05*** | ***0.05*** | ***0.05*** |
| 227 | V227Q | 0.27 | ***0.05*** | 0.07 | ***0.05*** | ***0.05*** | ***0.05*** |
| 227 | V227R | 0.29 | ***0.05*** | 0.08 | ***0.05*** | ***0.05*** | ***0.05*** |
| 227 | V227S | 0.16 | 14.70 | 19.48 | 4.22 | 0.5 | 0.53 |
| 227 | V227T | 0.63 | 1.04 | 1.44 | 1.01 | 0.7 | 0.87 |
| 227 | V227W | 0.25 | ***0.05*** | 0.14 | ***0.05*** | 1.5 | 0.06 |
| 227 | V227Y | ***0.05*** | ***0.05*** | ***0.05*** | ***0.05*** | ***0.05*** | ***0.05*** |
| 228 | A228E | 0.48 | ***0.05*** | ***0.05*** | ***0.05*** | ***0.05*** | ***0.05*** |
| 228 | A228F | 0.46 | ***0.05*** | ***0.05*** | ***0.05*** | ***0.05*** | ***0.05*** |
| 228 | A228G | 0.38 | 1.14 | 1.11 | 1.31 | 1.0 | 0.66 |
| 228 | A228H | 0.51 | ***0.05*** | ***0.05*** | ***0.05*** | ***0.05*** | ***0.05*** |
| 228 | A228I | 0.23 | 1.27 | 0.90 | 1.50 | 0.2 | 0.23 |
| 228 | A228K | 0.45 | ***0.05*** | ***0.05*** | ***0.05*** | ***0.05*** | ***0.05*** |
| 228 | A228L | 0.31 | 0.14 | ***0.05*** | 0.09 | ***0.05*** | ***0.05*** |
| 228 | A228M | 0.22 | 0.86 | 0.93 | 1.15 | 0.4 | 0.20 |
| 228 | A228N | 0.49 | ***0.05*** | ***0.05*** | ***0.05*** | ***0.05*** | ***0.05*** |
| 228 | A228P | 0.58 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 228 | A228R | 0.48 | ***0.05*** | ***0.05*** | ***0.05*** | ***0.05*** | ***0.05*** |
| 228 | A228S | 0.88 | 0.86 | 1.10 | 1.17 | 1.1 | 0.80 |
| 228 | A228T | 0.78 | 1.04 | 1.04 | 1.07 | 1.0 | 0.68 |
| 228 | A228V | 0.37 | 1.18 | 1.37 | 1.39 | 0.9 | 0.51 |
| 228 | A228W | 0.46 | 0.06 | ***0.05*** | ***0.05*** | ***0.05*** | ***0.05*** |
| 228 | A228Y | 0.39 | ***0.05*** | ***0.05*** | ***0.05*** | ***0.05*** | ***0.05*** |
| 229 | G229A | 1.20 | 0.82 | 1.12 | 0.99 | 0.8 | 0.99 |
| 229 | G229C | 0.23 | ***0.05*** | 0.12 | ***0.05*** | ***0.05*** | ***0.05*** |
| 229 | G229D | 0.14 | ***0.05*** | ***0.05*** | ***0.05*** | 0.6 | 0.11 |
| 229 | G229E | 0.29 | ***0.05*** | ***0.05*** | ***0.05*** | ***0.05*** | ***0.05*** |
| 229 | G229F | 0.35 | ***0.05*** | ***0.05*** | ***0.05*** | ***0.05*** | ***0.05*** |
| 229 | G229H | 0.28 | ***0.05*** | ***0.05*** | ***0.05*** | ***0.05*** | ***0.05*** |
| 229 | G229I | 0.27 | ***0.05*** | ***0.05*** | ***0.05*** | ***0.05*** | ***0.05*** |
| 229 | G229K | 0.30 | ***0.05*** | ***0.05*** | 0.07 | ***0.05*** | ***0.05*** |
| 229 | G229L | 0.19 | ***0.05*** | 0.49 | 0.12 | ***0.05*** | ***0.05*** |
| 229 | G229M | 0.21 | ***0.05*** | 0.13 | ***0.05*** | ***0.05*** | ***0.05*** |
| 229 | G229N | 0.25 | ***0.05*** | ***0.05*** | ***0.05*** | ***0.05*** | ***0.05*** |
| 229 | G229P | 0.28 | ***0.05*** | ***0.05*** | ***0.05*** | ***0.05*** | ***0.05*** |
| 229 | G229Q | 0.31 | ***0.05*** | ***0.05*** | ***0.05*** | ***0.05*** | ***0.05*** |
| 229 | G229R | 0.29 | ***0.05*** | 0.13 | ***0.05*** | ***0.05*** | ***0.05*** |
| 229 | G229S | 0.76 | 0.75 | 0.96 | 1.03 | 0.6 | 1.03 |
| 229 | G229T | 0.12 | ***0.05*** | ***0.05*** | ***0.05*** | 0.6 | 0.15 |
| 229 | G229V | 0.20 | ***0.05*** | 0.19 | 0.07 | ***0.05*** | ***0.05*** |
| 229 | G229W | 0.25 | ***0.05*** | ***0.05*** | ***0.05*** | ***0.05*** | ***0.05*** |
| 229 | G229Y | 0.24 | ***0.05*** | ***0.05*** | ***0.05*** | ***0.05*** | ***0.05*** |
| 230 | A230C | 1.06 | 0.88 | 1.21 | 1.12 | 0.8 | 1.06 |
| 230 | A230D | ***0.05*** | ***0.05*** | ***0.05*** | ***0.05*** | ***0.05*** | ***0.05*** |
| 230 | A230E | 0.77 | 0.74 | 1.09 | 1.06 | 0.6 | 0.75 |
| 230 | A230F | 0.50 | 0.52 | 1.30 | 1.06 | 1.1 | 0.94 |
| 230 | A230G | 0.99 | 0.86 | 1.16 | 0.95 | 1.0 | 0.98 |
| 230 | A230H | 0.16 | 10.35 | 16.17 | 3.82 | 0.1 | 0.38 |
| 230 | A230I | 0.24 | 1.02 | 1.83 | 1.31 | ***0.05*** | 0.83 |
| 230 | A230K | 0.26 | ***0.05*** | ***0.05*** | ***0.05*** | ***0.05*** | ***0.05*** |
| 230 | A230L | 0.27 | ***0.05*** | 0.12 | ***0.05*** | ***0.05*** | ***0.05*** |
| 230 | A230M | 0.24 | 1.19 | 1.78 | 1.27 | ***0.05*** | 0.66 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 230 | A230N | 0.19 | 1.80 | 2.19 | 1.72 | 0.6 | 0.58 |
| 230 | A230P | 0.24 | ***0.05*** | 0.10 | ***0.05*** | ***0.05*** | ***0.05*** |
| 230 | A230Q | 0.39 | 0.81 | 1.14 | 1.03 | 0.3 | 0.86 |
| 230 | A230R | 0.31 | ***0.05*** | 0.06 | ***0.05*** | ***0.05*** | ***0.05*** |
| 230 | A230S | 1.00 | 0.86 | 1.00 | 1.00 | 0.9 | 1.04 |
| 230 | A230T | 0.89 | 0.94 | 0.94 | 1.01 | 0.7 | 1.14 |
| 230 | A230V | 0.56 | 0.85 | 0.95 | 1.08 | 0.2 | 1.17 |
| 230 | A230W | 0.15 | 0.58 | ***0.05*** | ***0.05*** | ***0.05*** | ***0.05*** |
| 230 | A230Y | 0.11 | 0.06 | ***0.05*** | ***0.05*** | ***0.05*** | ***0.05*** |
| 231 | A231C | 0.84 | 0.99 | 1.15 | 1.09 | 0.8 | 0.79 |
| 231 | A231D | 0.21 | ***0.05*** | ***0.05*** | 0.07 | ***0.05*** | ***0.05*** |
| 231 | A231E | 0.31 | ***0.05*** | ***0.05*** | ***0.05*** | ***0.05*** | ***0.05*** |
| 231 | A231F | 0.33 | 1.16 | 1.34 | 1.33 | 0.5 | 0.57 |
| 231 | A231G | 0.42 | 1.10 | 1.04 | 1.35 | 1.1 | 0.86 |
| 231 | A231H | 0.32 | ***0.05*** | ***0.05*** | 0.07 | ***0.05*** | ***0.05*** |
| 231 | A231I | 0.86 | 1.08 | 1.16 | 1.12 | 0.8 | 0.71 |
| 231 | A231K | 0.49 | ***0.05*** | ***0.05*** | ***0.05*** | ***0.05*** | ***0.05*** |
| 231 | A231L | 0.45 | 1.07 | 1.27 | 1.32 | 0.7 | 0.83 |
| 231 | A231M | 0.41 | 1.15 | 1.26 | 1.30 | 0.7 | 0.73 |
| 231 | A231N | 0.31 | 0.09 | ***0.05*** | ***0.05*** | ***0.05*** | ***0.05*** |
| 231 | A231P | 0.53 | ***0.05*** | ***0.05*** | ***0.05*** | ***0.05*** | ***0.05*** |
| 231 | A231Q | 0.31 | ***0.05*** | ***0.05*** | ***0.05*** | ***0.05*** | ***0.05*** |
| 231 | A231R | 0.48 | ***0.05*** | ***0.05*** | ***0.05*** | ***0.05*** | ***0.05*** |
| 231 | A231S | 0.55 | 0.96 | 1.28 | 1.14 | 1.0 | 1.03 |
| 231 | A231T | 0.57 | 1.22 | 1.31 | 1.09 | 0.8 | 0.98 |
| 231 | A231V | 0.71 | 1.07 | 1.04 | 1.12 | 0.8 | 0.99 |
| 231 | A231W | 0.18 | 4.36 | 5.85 | 4.42 | 0.3 | 0.16 |
| 231 | A231Y | 0.25 | 1.52 | 2.14 | 1.63 | 0.2 | 0.44 |
| 232 | A232C | 0.92 | 1.09 | 1.13 | 1.00 | 0.9 | 0.99 |
| 232 | A232E | 0.28 | 0.25 | 0.21 | 0.37 | 1.0 | 0.14 |
| 232 | A232H | 0.22 | ***0.05*** | 0.13 | ***0.05*** | ***0.05*** | ***0.05*** |
| 232 | A232K | 0.37 | ***0.05*** | ***0.05*** | ***0.05*** | ***0.05*** | ***0.05*** |
| 232 | A232L | 0.37 | 1.15 | 1.29 | 1.42 | 1.1 | 0.87 |
| 232 | A232M | 0.66 | 1.02 | 1.04 | 0.98 | 1.0 | 0.91 |
| 232 | A232N | 0.20 | 1.64 | 2.16 | 1.52 | 1.1 | 0.44 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 232 | A232P | 0.30 | ***0.05*** | ***0.05*** | ***0.05*** | ***0.05*** | ***0.05*** |
| 232 | A232Q | 0.54 | ***0.05*** | ***0.05*** | ***0.05*** | ***0.05*** | ***0.05*** |
| 232 | A232S | 0.81 | 0.96 | 1.27 | 0.93 | 0.9 | 0.94 |
| 232 | A232T | 0.73 | 0.97 | 1.02 | 1.03 | 1.0 | 1.04 |
| 232 | A232V | 0.77 | 1.00 | 1.35 | 0.99 | 1.0 | 1.09 |
| 232 | A232Y | 0.43 | ***0.05*** | ***0.05*** | ***0.05*** | ***0.05*** | ***0.05*** |
| 233 | L233D | 0.71 | 0.89 | 0.91 | 0.92 | 1.1 | 0.71 |
| 233 | L233F | 0.38 | ***0.05*** | ***0.05*** | ***0.05*** | ***0.05*** | ***0.05*** |
| 233 | L233H | 0.24 | ***0.05*** | ***0.05*** | ***0.05*** | ***0.05*** | ***0.05*** |
| 233 | L233I | 0.85 | 0.89 | 1.11 | 1.03 | 1.0 | 0.90 |
| 233 | L233M | 0.67 | 0.92 | 0.99 | 1.01 | 0.9 | 0.94 |
| 233 | L233P | 0.40 | ***0.05*** | ***0.05*** | ***0.05*** | ***0.05*** | ***0.05*** |
| 233 | L233R | 0.49 | 0.07 | ***0.05*** | ***0.05*** | ***0.05*** | ***0.05*** |
| 233 | L233S | 0.61 | 1.05 | 1.16 | 0.96 | 0.9 | 0.78 |
| 233 | L233T | 0.78 | 0.95 | 1.25 | 0.90 | 1.0 | 0.89 |
| 233 | L233V | 0.94 | 1.15 | 1.09 | 0.89 | 1.1 | 0.91 |
| 233 | L233Y | 0.38 | ***0.05*** | ***0.05*** | ***0.05*** | ***0.05*** | ***0.05*** |
| 234 | I234A | 1.16 | 0.86 | 0.96 | 0.88 | 0.8 | 1.12 |
| 234 | I234C | 1.21 | 1.02 | 0.92 | 0.96 | 1.1 | 0.88 |
| 234 | I234D | 0.21 | 0.70 | 1.52 | 0.91 | 0.6 | 0.44 |
| 234 | I234E | 0.88 | 1.05 | 1.01 | 0.86 | 0.8 | 1.01 |
| 234 | I234F | 0.72 | 1.03 | 1.02 | 0.72 | 0.1 | 1.02 |
| 234 | I234G | 0.33 | 0.93 | 0.96 | 1.15 | 0.7 | 0.86 |
| 234 | I234H | 0.37 | 0.98 | 0.98 | 0.97 | 0.3 | 0.94 |
| 234 | I234K | 0.30 | 0.89 | 0.92 | 1.00 | 0.1 | 0.84 |
| 234 | I234L | 1.22 | 0.82 | 0.84 | 1.08 | 1.1 | 1.07 |
| 234 | I234M | 1.27 | 1.02 | 0.91 | 0.98 | 1.0 | 1.18 |
| 234 | I234N | 0.42 | 1.08 | 0.98 | 1.03 | 0.9 | 0.91 |
| 234 | I234P | 0.29 | ***0.05*** | ***0.05*** | ***0.05*** | ***0.05*** | ***0.05*** |
| 234 | I234Q | 0.87 | 0.72 | 0.94 | 0.93 | 0.9 | 0.96 |
| 234 | I234R | 0.14 | ***0.05*** | ***0.05*** | ***0.05*** | 0.2 | 0.34 |
| 234 | I234S | 0.81 | 0.92 | 0.93 | 0.93 | 0.9 | 0.98 |
| 234 | I234T | 1.14 | 0.87 | 0.92 | 1.02 | 1.1 | 0.99 |
| 234 | 1234V | 1.20 | 1.02 | 1.19 | 0.93 | 1.1 | 1.14 |
| 234 | I234W | 0.17 | 4.77 | 6.83 | 2.41 | 0.1 | 0.19 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 234 | I234Y | 0.39 | 0.88 | 1.06 | 0.82 | ***0.05*** | 0.95 |
| 235 | L235A | 0.67 | 1.05 | 0.95 | 1.05 | 1.0 | 1.15 |
| 235 | L235C | 1.08 | 0.96 | 1.24 | 1.03 | 0.9 | 1.07 |
| 235 | L235D | 0.20 | 0.19 | 1.17 | 0.45 | 1.1 | 0.14 |
| 235 | L235E | 0.42 | 0.81 | 1.03 | 0.94 | 1.1 | 0.25 |
| 235 | L235F | 1.25 | 1.08 | 1.01 | 0.94 | 1.0 | 0.80 |
| 235 | L235G | 0.27 | 1.09 | 0.76 | 1.11 | 1.1 | 0.62 |
| 235 | L235H | ***0.05*** | ***0.05*** | ***0.05*** | ***0.05*** | ***0.05*** | ***0.05*** |
| 235 | L235I | 1.31 | 0.92 | 0.83 | 1.10 | 1.1 | 0.94 |
| 235 | L235K | 1.32 | 0.86 | 1.21 | 0.96 | 1.1 | 0.85 |
| 235 | L235M | 1.34 | 0.99 | 0.99 | 1.03 | 1.1 | 0.89 |
| 235 | L235N | 0.25 | 1.32 | 1.54 | 1.19 | 1.2 | 0.69 |
| 235 | L235P | 0.31 | ***0.05*** | ***0.05*** | ***0.05*** | ***0.05*** | ***0.05*** |
| 235 | L235Q | 1.01 | 0.92 | 0.84 | 1.03 | 1.1 | 1.24 |
| 235 | L235R | 1.29 | 0.79 | 0.86 | 0.95 | 1.0 | 0.90 |
| 235 | L235S | 0.69 | 1.05 | 0.86 | 0.76 | 1.0 | 1.00 |
| 235 | L235T | 0.65 | 1.10 | 0.80 | 0.86 | 1.1 | 1.07 |
| 235 | L235V | 1.17 | 0.92 | 1.07 | 0.83 | 1.1 | 1.24 |
| 235 | L235W | 1.09 | 0.98 | 0.63 | 1.13 | 1.1 | 0.94 |
| 235 | L235Y | 0.99 | 0.99 | 0.98 | 1.04 | 1.1 | 1.02 |
| 236 | S236A | 1.21 | 1.02 | 1.06 | 0.94 | 0.9 | 1.03 |
| 236 | S236C | 1.28 | 0.89 | 0.95 | 0.96 | 1.0 | 0.90 |
| 236 | S236D | 1.22 | 1.02 | 0.95 | 0.98 | 1.2 | 1.07 |
| 236 | S236E | 1.26 | 0.90 | 0.88 | 1.09 | 1.2 | 1.55 |
| 236 | S236F | ***0.05*** | ***0.05*** | ***0.05*** | ***0.05*** | ***0.05*** | ***0.05*** |
| 236 | S236G | 1.20 | 1.06 | 1.09 | 0.97 | 0.9 | 0.99 |
| 236 | S236H | 1.12 | 0.84 | 0.87 | 0.92 | 1.2 | 1.07 |
| 236 | S236I | ***0.05*** | ***0.05*** | ***0.05*** | ***0.05*** | ***0.05*** | ***0.05*** |
| 236 | S236K | 0.31 | 0.96 | 0.80 | 1.18 | 0.7 | 0.55 |
| 236 | S236L | 0.19 | 0.42 | 0.92 | 0.66 | 0.8 | 0.13 |
| 236 | S236M | ***0.05*** | ***0.05*** | ***0.05*** | ***0.05*** | ***0.05*** | ***0.05*** |
| 236 | S236N | 1.24 | 1.03 | 1.18 | 0.86 | 0.6 | 1.13 |
| 236 | S236P | 0.34 | ***0.05*** | 0.09 | ***0.05*** | ***0.05*** | ***0.05*** |
| 236 | S236Q | 1.18 | 0.85 | 0.69 | 0.95 | 1.1 | 1.00 |
| 236 | S236R | 0.34 | 0.96 | 0.72 | 1.13 | 0.7 | 0.60 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 236 | S236T | 0.59 | 0.99 | 0.97 | 1.04 | 0.8 | 1.16 |
| 236 | S236V | 0.91 | 0.97 | 0.86 | 1.06 | 0.9 | 1.21 |
| 236 | S236W | 0.18 | 2.11 | 3.43 | 2.28 | 0.8 | 0.50 |
| 236 | S236Y | 0.48 | 1.10 | 0.66 | 0.91 | 0.8 | 1.09 |
| 237 | K237A | 1.13 | 1.11 | 1.31 | 0.81 | 1.0 | 1.03 |
| 237 | K237C | 0.19 | 1.21 | 1.50 | 0.91 | 1.1 | 0.33 |
| 237 | K237D | ***0.05*** | ***0.05*** | ***0.05*** | ***0.05*** | ***0.05*** | ***0.05*** |
| 237 | K237E | 1.03 | 0.86 | 0.84 | 0.85 | 1.0 | 1.22 |
| 237 | K237F | 1.05 | 0.80 | 0.99 | 0.92 | 1.0 | 1.21 |
| 237 | K237G | 1.05 | 0.90 | 1.08 | 1.06 | 1.0 | 0.94 |
| 237 | K237H | 1.05 | 0.91 | 0.97 | 0.93 | 1.1 | 0.88 |
| 237 | K237I | 1.02 | 1.03 | 0.69 | 0.70 | 1.0 | 1.05 |
| 237 | K237L | 1.04 | 0.96 | 0.89 | 1.10 | 1.0 | 1.12 |
| 237 | K237M | 1.17 | 0.89 | 1.09 | 0.86 | 0.9 | 1.20 |
| 237 | K237N | 1.09 | 1.00 | 0.99 | 0.94 | 1.1 | 0.93 |
| 237 | K237P | 0.19 | 0.50 | 0.92 | 0.59 | 1.1 | 0.15 |
| 237 | K237Q | 1.12 | 0.74 | 0.87 | 0.84 | 1.1 | 1.01 |
| 237 | K237R | 1.13 | 0.82 | 0.95 | 1.02 | 1.0 | 1.08 |
| 237 | K237S | 1.17 | 0.97 | 1.07 | 0.86 | 1.0 | 1.24 |
| 237 | K237T | 1.07 | 0.81 | 0.75 | 0.82 | 1.0 | 0.94 |
| 237 | K237V | 1.08 | 0.85 | 1.09 | 1.10 | 0.9 | 1.09 |
| 237 | K237W | 0.81 | 0.96 | 1.08 | 0.95 | 0.9 | 0.87 |
| 237 | K237Y | 1.00 | 1.10 | 0.98 | 1.08 | 0.9 | 1.10 |
| 238 | H238A | 0.28 | 1.34 | 1.44 | 1.12 | 1.1 | 0.46 |
| 238 | H238C | 0.62 | 1.07 | 1.09 | 0.99 | 1.1 | 0.97 |
| 238 | H238D | 0.35 | 0.93 | 1.18 | 1.19 | 1.1 | 0.78 |
| 238 | H238E | 0.61 | 0.91 | 1.00 | 1.00 | 1.1 | 1.08 |
| 238 | H238F | 0.74 | 0.69 | 1.05 | 0.95 | 1.1 | 1.09 |
| 238 | H238G | 0.22 | 5.21 | 5.98 | 2.44 | 1.0 | 0.44 |
| 238 | H238I | 0.22 | 7.74 | 7.02 | 3.64 | 1.1 | 0.52 |
| 238 | H238K | 0.87 | 0.82 | 1.07 | 0.89 | 1.0 | 1.24 |
| 238 | H238L | 0.20 | ***0.05*** | ***0.05*** | 4.71 | 1.2 | 0.36 |
| 238 | H238M | 0.58 | 1.00 | 1.15 | 0.89 | 1.0 | 0.93 |
| 238 | H238N | ***0.05*** | ***0.05*** | ***0.05*** | ***0.05*** | ***0.05*** | ***0.05*** |
| 238 | H238P | 0.17 | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 238 | H238Q | ***0.05*** | ***0.05*** | ***0.05*** | ***0.05*** | ***0.05*** | ***0.05*** |
| 238 | H238R | 0.79 | 0.96 | 0.76 | 1.04 | 0.9 | 1.17 |
| 238 | H238S | 0.55 | 1.29 | 1.03 | 0.82 | 1.0 | 0.99 |
| 238 | H238T | 0.16 | ***0.05*** | ***0.05*** | ***0.05*** | ***0.05*** | ***0.05*** |
| 238 | H238V | 0.20 | ***0.05*** | ***0.05*** | 12.14 | 0.9 | 0.26 |
| 238 | H238W | 0.41 | 1.14 | 0.92 | 1.16 | 1.1 | 1.10 |
| 238 | H238Y | ***0.05*** | ***0.05*** | ***0.05*** | ***0.05*** | ***0.05*** | ***0.05*** |
| 239 | P239A | 0.57 | 0.98 | 0.94 | 0.88 | 1.0 | 0.86 |
| 239 | P239C | 0.78 | 0.96 | 0.85 | 0.83 | 1.0 | 1.03 |
| 239 | P239D | 0.89 | 1.00 | 0.81 | 0.98 | 1.1 | 0.87 |
| 239 | P239E | 0.80 | 1.16 | 0.81 | 0.97 | 1.1 | 0.96 |
| 239 | P239F | 0.66 | 1.15 | 0.67 | 1.03 | 1.0 | 1.02 |
| 239 | P239G | 0.76 | 0.71 | 0.48 | 0.95 | 0.2 | 0.79 |
| 239 | P239H | 0.89 | 1.41 | 1.02 | 1.12 | 1.1 | 1.09 |
| 239 | P239I | ***0.05*** | ***0.05*** | ***0.05*** | ***0.05*** | ***0.05*** | ***0.05*** |
| 239 | P239K | 0.23 | 0.15 | ***0.05*** | 0.10 | ***0.05*** | ***0.05*** |
| 239 | P239L | 0.65 | 1.04 | 0.90 | 0.95 | 1.1 | 1.09 |
| 239 | P239M | 0.79 | 1.12 | 0.70 | 0.98 | 1.1 | 0.89 |
| 239 | P239N | 0.82 | 1.08 | 0.83 | 1.01 | 1.2 | 0.94 |
| 239 | P239Q | 0.91 | 1.14 | 0.80 | 1.10 | 1.2 | 0.97 |
| 239 | P239R | 0.92 | 1.21 | 0.67 | 1.27 | 1.0 | 1.04 |
| 239 | P239S | 0.87 | 1.31 | 0.96 | 0.99 | 1.1 | 1.33 |
| 239 | P239T | 0.80 | 0.97 | 1.07 | 1.11 | 1.2 | 1.03 |
| 239 | P239V | 0.71 | 1.10 | 1.22 | 1.06 | 1.2 | 1.32 |
| 239 | P239W | 0.65 | 1.06 | 0.75 | 1.11 | 1.0 | 1.28 |
| 239 | P239Y | 0.76 | 0.90 | 0.94 | 1.20 | 1.2 | 1.22 |
| 240 | N240A | 0.80 | 0.96 | 0.99 | 0.96 | 1.0 | 0.91 |
| 240 | N240C | 1.06 | 1.04 | 0.83 | 0.89 | 1.1 | 0.95 |
| 240 | N240D | 1.05 | 1.07 | 0.88 | 1.03 | 1.2 | 0.93 |
| 240 | N240E | 1.03 | 1.27 | 0.83 | 1.00 | 1.2 | 1.05 |
| 240 | N240F | 1.02 | 0.94 | 0.84 | 0.93 | 1.1 | 0.92 |
| 240 | N240G | 1.03 | 1.21 | 0.87 | 0.90 | 1.0 | 1.01 |
| 240 | N240H | ***0.05*** | ***0.05*** | ***0.05*** | ***0.05*** | ***0.05*** | ***0.05*** |
| 240 | N2401 | ***0.05*** | ***0.05*** | ***0.05*** | ***0.05*** | ***0.05*** | ***0.05*** |
| 240 | N240K | 1.09 | 1.04 | 1.04 | 0.99 | 1.1 | 1.02 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 240 | N240L | 0.78 | 1.27 | 0.86 | 1.08 | 1.0 | 1.05 |
| 240 | N240M | 0.94 | 1.03 | 0.76 | 0.92 | 1.0 | 0.94 |
| 240 | N240P | 0.39 | 0.75 | 0.72 | 0.89 | 0.9 | 0.54 |
| 240 | N240Q | 0.91 | 1.09 | 0.88 | 1.11 | 1.1 | 1.04 |
| 240 | N240R | 0.75 | 0.85 | 0.59 | 1.14 | 1.0 | 0.97 |
| 240 | N240S | 1.00 | 1.19 | 1.04 | 1.11 | 1.1 | 0.95 |
| 240 | N240T | 0.90 | 1.02 | 0.77 | 1.14 | 1.1 | 1.02 |
| 240 | N240V | 0.90 | 1.06 | 0.92 | 1.06 | 1.0 | 1.10 |
| 240 | N240W | 1.05 | 1.05 | 0.66 | 1.01 | 1.0 | 1.00 |
| 240 | N240Y | 0.92 | 1.01 | 0.80 | 1.22 | 1.0 | 1.43 |
| 241 | W241A | 0.84 | 0.94 | 0.65 | 1.02 | 1.1 | 0.86 |
| 241 | W241C | 0.63 | 0.96 | 0.72 | 0.98 | 1.1 | 1.09 |
| 241 | W241D | 0.44 | 1.06 | 1.03 | 1.05 | 1.2 | 0.89 |
| 241 | W241E | 0.34 | 1.05 | 0.81 | 1.06 | 1.1 | 0.63 |
| 241 | W241F | 0.81 | 0.94 | 0.86 | 0.75 | 1.0 | 0.87 |
| 241 | W241G | 0.31 | 1.35 | 1.05 | 0.92 | 1.0 | 0.64 |
| 241 | W241H | 0.65 | 1.08 | 0.99 | 1.12 | 1.1 | 1.05 |
| 241 | W241I | 0.75 | 1.11 | 0.99 | 1.13 | 1.0 | 1.15 |
| 241 | W241K | 0.83 | 1.00 | 0.88 | 1.22 | 1.0 | 1.11 |
| 241 | W241L | 0.85 | 0.78 | 1.00 | 1.03 | 1.0 | 0.99 |
| 241 | W241M | 0.91 | 1.01 | 0.82 | 1.04 | 1.1 | 1.07 |
| 241 | W241N | 0.35 | 1.14 | 0.92 | 1.07 | 1.2 | 0.64 |
| 241 | W241P | 0.18 | ***0.05*** | ***0.05*** | ***0.05*** | 0.9 | 0.30 |
| 241 | W241Q | 0.65 | 1.40 | 1.00 | 1.14 | 1.1 | 1.14 |
| 241 | W241R | 0.59 | 0.93 | 0.88 | 1.12 | 0.9 | 1.37 |
| 241 | W241S | 0.66 | 1.14 | 0.68 | 1.04 | 0.9 | 1.06 |
| 241 | W241T | 0.61 | 1.06 | 0.86 | 1.16 | 1.0 | 1.11 |
| 241 | W241V | 0.61 | 1.20 | 1.01 | 1.09 | 1.0 | 1.35 |
| 241 | W241Y | 0.81 | 1.17 | 0.84 | 1.08 | 1.0 | 1.38 |
| 242 | T242A | 1.24 | 0.92 | 1.10 | 0.95 | 1.1 | 0.75 |
| 242 | T242C | 1.10 | 0.89 | 1.21 | 0.84 | 1.1 | 0.86 |
| 242 | T242D | ***0.05*** | ***0.05*** | ***0.05*** | ***0.05*** | ***0.05*** | ***0.05*** |
| 242 | T242E | 1.03 | 1.08 | 1.32 | 0.87 | 1.0 | 0.90 |
| 242 | T242F | 0.71 | 0.86 | 0.97 | 0.81 | 0.9 | 0.99 |
| 242 | T242G | 0.88 | 0.74 | 1.02 | 1.03 | 1.0 | 0.95 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 242 | T242H | 0.95 | 1.14 | 0.98 | 0.90 | 1.1 | 0.92 |
| 242 | T242I | 0.84 | 0.82 | 1.26 | 1.11 | 1.1 | 0.93 |
| 242 | T242K | 0.99 | 0.77 | 1.07 | 0.93 | 1.1 | 0.94 |
| 242 | T242L | 0.97 | 0.99 | 1.12 | 1.01 | 1.1 | 0.89 |
| 242 | T242M | 1.00 | 0.83 | 1.03 | 0.97 | 1.0 | 0.87 |
| 242 | T242N | 1.02 | 0.65 | 1.14 | 0.96 | 1.1 | 0.92 |
| 242 | T242P | 0.93 | 0.83 | 1.22 | 1.06 | 1.1 | 0.97 |
| 242 | T242Q | 0.91 | 0.87 | 1.34 | 0.95 | 1.1 | 1.23 |
| 242 | T242R | 0.91 | 1.04 | 1.18 | 0.91 | 1.1 | 1.05 |
| 242 | T242S | 1.05 | 1.00 | 1.16 | 1.18 | 1.1 | 0.95 |
| 242 | T242V | 0.80 | 0.89 | 1.20 | 0.99 | 1.0 | 1.05 |
| 242 | T242W | 0.62 | 0.72 | 0.97 | 0.91 | 1.1 | 0.96 |
| 242 | T242Y | 0.69 | 0.94 | 0.97 | 1.01 | 1.1 | 1.02 |
| 243 | N243A | ***0.05*** | ***0.05*** | ***0.05*** | ***0.05*** | ***0.05*** | ***0.05*** |
| 243 | N243C | 1.05 | 0.88 | 1.22 | 0.98 | 1.0 | 0.93 |
| 243 | N243D | ***0.05*** | ***0.05*** | ***0.05*** | ***0.05*** | ***0.05*** | ***0.05*** |
| 243 | N243E | 1.14 | 0.94 | 1.22 | 0.98 | 1.1 | 0.79 |
| 243 | N243F | 0.69 | 0.75 | 1.03 | 1.01 | 0.6 | 0.79 |
| 243 | N243G | 0.97 | 1.16 | 0.94 | 1.00 | 1.2 | 0.72 |
| 243 | N243H | 0.26 | ***0.05*** | ***0.05*** | ***0.05*** | ***0.05*** | ***0.05*** |
| 243 | N2431 | 0.94 | 0.79 | 1.61 | 0.79 | 0.8 | 0.91 |
| 243 | N243K | 0.78 | 0.97 | 1.05 | 1.11 | 0.9 | 0.85 |
| 243 | N243L | 0.83 | 0.92 | 1.24 | 1.03 | 0.8 | 0.74 |
| 243 | N243M | ***0.05*** | ***0.05*** | ***0.05*** | ***0.05*** | ***0.05*** | ***0.05*** |
| 243 | N243P | 0.26 | ***0.05*** | ***0.05*** | 0.19 | ***0.05*** | ***0.05*** |
| 243 | N243Q | 0.93 | 0.74 | 1.28 | 1.03 | 1.0 | 0.94 |
| 243 | N243R | 0.73 | 0.79 | 0.92 | 0.90 | 0.8 | 1.09 |
| 243 | N243S | 0.97 | 1.05 | 1.13 | 0.86 | 1.0 | 0.93 |
| 243 | N243T | 0.92 | 0.97 | 1.01 | 0.92 | 1.0 | 0.98 |
| 243 | N243V | 0.89 | 0.87 | 1.13 | 1.13 | 1.0 | 1.29 |
| 243 | N243W | 0.59 | 0.90 | 1.02 | 1.01 | 0.6 | 1.05 |
| 243 | N243Y | 0.81 | 0.82 | 1.20 | 0.82 | 0.9 | 0.92 |
| 244 | T244A | 1.53 | 0.66 | 0.97 | 1.07 | 1.0 | 0.84 |
| 244 | T244C | ***0.05*** | ***0.05*** | ***0.05*** | ***0.05*** | ***0.05*** | ***0.05*** |
| 244 | T244D | 1.13 | 0.97 | 1.23 | 1.13 | 1.0 | 1.11 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 244 | T244E | 0.69 | 0.89 | 1.12 | 0.90 | 1.0 | 0.92 |
| 244 | T244F | 0.83 | 0.97 | 0.97 | 1.01 | 1.0 | 0.96 |
| 244 | T244G | 1.05 | 0.83 | 1.06 | 1.01 | 1.0 | 0.96 |
| 244 | T244H | 1.06 | 0.90 | 1.29 | 1.01 | 1.1 | 0.94 |
| 244 | T244I | ***0.05*** | ***0.05*** | ***0.05*** | ***0.05*** | ***0.05*** | ***0.05*** |
| 244 | T244K | 1.09 | 0.76 | 0.95 | 1.00 | 1.0 | 0.97 |
| 244 | T244L | 0.99 | 1.07 | 1.13 | 0.92 | 1.1 | 1.00 |
| 244 | T244M | 1.15 | 0.94 | 1.05 | 1.07 | 1.0 | 0.96 |
| 244 | T244N | 1.03 | 1.20 | 1.10 | 1.03 | 1.1 | 0.95 |
| 244 | T244P | 0.74 | 0.89 | 1.25 | 1.00 | 1.0 | 1.00 |
| 244 | T244Q | 1.04 | 0.87 | 1.23 | 0.94 | 1.0 | 1.08 |
| 244 | T244R | 0.96 | 0.54 | 1.04 | 0.91 | 0.9 | 1.10 |
| 244 | T244S | 1.04 | 1.02 | 1.23 | 1.07 | 1.0 | 0.99 |
| 244 | T244V | 0.90 | 0.69 | 1.07 | 1.04 | 1.0 | 1.06 |
| 244 | T244W | 0.88 | 0.70 | 1.29 | 0.94 | 1.0 | 1.13 |
| 244 | T244Y | 0.87 | 0.38 | 1.03 | 1.01 | 1.0 | 1.10 |
| 245 | Q245A | 1.00 | 0.92 | 1.10 | 0.93 | 1.1 | 0.92 |
| 245 | Q245C | 0.80 | 0.79 | 1.15 | 0.93 | 1.1 | 0.89 |
| 245 | Q245D | 1.00 | 0.73 | 1.19 | 0.88 | 1.1 | 0.90 |
| 245 | Q245E | 1.03 | 1.00 | 1.15 | 0.79 | 1.2 | 0.89 |
| 245 | Q245F | 0.80 | 0.89 | 1.21 | 1.00 | 1.0 | 0.98 |
| 245 | Q245G | 0.76 | 1.12 | 1.17 | 0.99 | 1.1 | 0.99 |
| 245 | Q245H | 0.93 | 1.01 | 1.04 | 0.97 | 1.1 | 0.94 |
| 245 | Q245I | 0.83 | 0.97 | 1.12 | 1.01 | 1.1 | 0.99 |
| 245 | Q245K | 0.99 | 0.83 | 0.82 | 0.88 | 1.1 | 0.96 |
| 245 | Q245L | 0.88 | 0.77 | 1.18 | 0.85 | 1.1 | 0.97 |
| 245 | Q245M | 1.06 | 0.81 | 1.19 | 1.10 | 1.1 | 0.92 |
| 245 | Q245N | 0.63 | 0.91 | 0.96 | 0.79 | 1.0 | 0.82 |
| 245 | Q245P | 0.27 | 0.47 | 0.63 | 0.50 | 1.0 | 0.25 |
| 245 | Q245R | 0.76 | 0.57 | 0.89 | 0.97 | 1.0 | 1.07 |
| 245 | Q245S | 0.81 | 0.93 | 1.19 | 0.97 | 0.9 | 1.14 |
| 245 | Q245T | 0.82 | 0.90 | 1.18 | 1.03 | 1.0 | 1.37 |
| 245 | Q245V | 0.72 | 0.96 | 1.19 | 1.06 | 1.0 | 1.09 |
| 245 | Q245W | 0.50 | 0.94 | 1.00 | 0.90 | 0.9 | 1.08 |
| 245 | Q245Y | 1.06 | 0.66 | 1.08 | 0.99 | 1.0 | 1.06 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 246 | V246A | 0.53 | 1.06 | 1.22 | 1.01 | 0.9 | 0.92 |
| 246 | V246C | 0.64 | 0.95 | 1.05 | 0.92 | 1.1 | 1.02 |
| 246 | V246D | 0.44 | ***0.05*** | ***0.05*** | ***0.05*** | ***0.05*** | ***0.05*** |
| 246 | V246E | 0.61 | ***0.05*** | ***0.05*** | ***0.05*** | ***0.05*** | ***0.05*** |
| 246 | V246F | 0.31 | 1.18 | 1.59 | 1.59 | 0.8 | 0.81 |
| 246 | V246G | 0.18 | 0.46 | 0.18 | 0.47 | 0.5 | 0.06 |
| 246 | V246H | 0.30 | ***0.05*** | 0.07 | ***0.05*** | ***0.05*** | ***0.05*** |
| 246 | V246I | 1.00 | 1.00 | 1.11 | 0.81 | 1.1 | 0.95 |
| 246 | V246K | 0.38 | ***0.05*** | ***0.05*** | ***0.05*** | ***0.05*** | ***0.05*** |
| 246 | V246L | 0.67 | 1.05 | 1.10 | 0.88 | 1.0 | 0.94 |
| 246 | V246N | 0.20 | 0.32 | 0.16 | 0.32 | 1.2 | 0.08 |
| 246 | V246P | 0.43 | ***0.05*** | ***0.05*** | ***0.05*** | ***0.05*** | ***0.05*** |
| 246 | V246Q | 0.36 | 0.07 | 0.11 | 0.11 | ***0.05*** | ***0.05*** |
| 246 | V246R | 0.23 | 0.08 | 0.17 | 0.19 | ***0.05*** | ***0.05*** |
| 246 | V246S | 0.23 | 0.60 | 0.54 | 0.81 | 0.7 | 0.33 |
| 246 | V246T | 0.74 | 0.95 | 1.23 | 1.04 | 1.0 | 1.10 |
| 246 | V246Y | 0.25 | 0.23 | 0.41 | 0.43 | 0.2 | 0.15 |
| 247 | R247A | 0.55 | 0.86 | 1.05 | 0.74 | 0.2 | 0.70 |
| 247 | R247C | 0.36 | 1.07 | 1.40 | 1.04 | 0.4 | 0.64 |
| 247 | R247D | 0.23 | 0.43 | 0.88 | 0.46 | 0.2 | 0.15 |
| 247 | R247E | 0.38 | 0.77 | 1.02 | 0.88 | 0.3 | 0.59 |
| 247 | R247F | 0.32 | 0.96 | 1.09 | 0.86 | 0.3 | 0.65 |
| 247 | R247G | 0.24 | ***0.05*** | ***0.05*** | ***0.05*** | ***0.05*** | ***0.05*** |
| 247 | R247H | 0.31 | 1.31 | 1.51 | 1.28 | 0.3 | 0.68 |
| 247 | R247I | 0.37 | 1.16 | 1.37 | 1.11 | 0.2 | 0.88 |
| 247 | R247K | 0.50 | 1.01 | 1.17 | 1.02 | 0.5 | 0.75 |
| 247 | R247L | 0.30 | 1.65 | 1.54 | 1.25 | 0.1 | 0.55 |
| 247 | R247M | 0.42 | 1.07 | 1.22 | 0.83 | 0.2 | 0.63 |
| 247 | R247N | ***0.05*** | ***0.05*** | ***0.05*** | ***0.05*** | ***0.05*** | ***0.05*** |
| 247 | R247P | ***0.05*** | ***0.05*** | ***0.05*** | ***0.05*** | ***0.05*** | ***0.05*** |
| 247 | R247Q | ***0.05*** | ***0.05*** | ***0.05*** | ***0.05*** | ***0.05*** | ***0.05*** |
| 247 | R247S | 0.37 | 1.06 | 1.56 | 0.93 | 0.4 | 0.74 |
| 247 | R247T | 0.44 | 1.18 | 1.33 | 1.11 | 0.5 | 0.97 |
| 247 | R247V | 0.40 | 0.84 | 1.31 | 1.01 | 0.4 | 0.76 |
| 247 | R247W | 0.32 | 1.35 | 1.39 | 0.95 | 0.3 | 0.68 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 247 | R247Y | 0.27 | 1.53 | 2.24 | 1.48 | 0.2 | 0.90 |
| 248 | S248A | 1.06 | 1.03 | 1.21 | 1.00 | 1.0 | 1.25 |
| 248 | S248C | 1.02 | 0.82 | 1.09 | 0.96 | 1.0 | 0.91 |
| 248 | S248D | ***0.05*** | ***0.05*** | ***0.05*** | ***0.05*** | ***0.05*** | ***0.05*** |
| 248 | S248E | 1.21 | 0.61 | 1.11 | 0.86 | 1.0 | 1.02 |
| 248 | S248F | 0.98 | 0.97 | 1.11 | 0.96 | 0.9 | 1.52 |
| 248 | S248G | 0.93 | 1.11 | 0.97 | 0.96 | 0.9 | 1.10 |
| 248 | S248H | 0.78 | 1.16 | 1.10 | 0.98 | 1.0 | 0.99 |
| 248 | S248I | 1.00 | 1.01 | 1.23 | 1.13 | 0.9 | 1.20 |
| 248 | S248K | 1.07 | 0.54 | 0.99 | 1.02 | 0.9 | 0.98 |
| 248 | S248L | 1.00 | 0.82 | 1.08 | 1.01 | 0.9 | 1.18 |
| 248 | S248M | 1.00 | 0.75 | 1.31 | 0.96 | 1.0 | 1.05 |
| 248 | S248N | 1.08 | 0.96 | 1.19 | 1.05 | 1.0 | 1.01 |
| 248 | S248P | 0.57 | 1.06 | 1.39 | 1.07 | 0.9 | 1.00 |
| 248 | S248Q | 1.10 | 1.01 | 1.14 | 0.84 | 1.0 | 0.93 |
| 248 | S248R | 1.10 | 0.47 | 0.80 | 0.87 | 0.9 | 0.87 |
| 248 | S248T | 0.95 | 0.93 | 1.19 | 0.96 | 1.0 | 1.07 |
| 248 | S248V | 0.80 | 1.16 | 1.13 | 1.01 | 0.8 | 1.31 |
| 248 | S248W | 0.85 | 0.87 | 1.24 | 1.02 | 1.1 | 0.99 |
| 248 | S248Y | 1.31 | 0.89 | 1.17 | 0.94 | 1.0 | 0.98 |
| 249 | S249A | 1.07 | 0.92 | 1.25 | 1.02 | 1.0 | 0.95 |
| 249 | S249C | 1.14 | 0.97 | 1.21 | 0.92 | 1.0 | 0.86 |
| 249 | S249D | 0.97 | 0.84 | 1.17 | 0.90 | 0.9 | 1.27 |
| 249 | S249E | ***0.05*** | ***0.05*** | ***0.05*** | ***0.05*** | ***0.05*** | ***0.05*** |
| 249 | S249F | 1.14 | 0.75 | 0.97 | 0.95 | 0.8 | 0.89 |
| 249 | S249G | 0.72 | 0.83 | 1.17 | 0.98 | 0.9 | 1.04 |
| 249 | S249H | 1.10 | 0.95 | 0.87 | 1.12 | 0.9 | 0.87 |
| 249 | S249I | 0.92 | 0.76 | 1.30 | 0.99 | 0.9 | 0.93 |
| 249 | S249K | 1.08 | 0.91 | 0.83 | 0.87 | 0.8 | 0.93 |
| 249 | S249L | 0.99 | 0.89 | 1.19 | 0.91 | 0.8 | 1.21 |
| 249 | S249M | 1.07 | 0.99 | 1.24 | 1.11 | 1.0 | 1.00 |
| 249 | S249N | 1.00 | 1.08 | 1.35 | 1.06 | 1.0 | 0.92 |
| 249 | S249P | ***0.05*** | ***0.05*** | ***0.05*** | ***0.05*** | ***0.05*** | ***0.05*** |
| 249 | S249Q | 1.00 | 0.73 | 1.06 | 1.17 | 0.8 | 1.12 |
| 249 | S249R | 1.03 | 0.78 | 0.87 | 0.95 | 0.9 | 1.00 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 249 | S249T | 1.08 | 0.78 | 1.17 | 0.95 | 0.9 | 1.13 |
| 249 | S249V | 0.97 | 0.82 | 1.18 | 1.05 | 0.9 | 0.99 |
| 249 | S249W | 0.95 | 0.92 | 1.09 | 1.17 | 0.8 | 1.06 |
| 249 | S249Y | 0.94 | 0.80 | 1.07 | 0.91 | 0.9 | 1.01 |
| 250 | L250A | ***0.05*** | ***0.05*** | ***0.05*** | ***0.05*** | ***0.05*** | ***0.05*** |
| 250 | L250C | 0.37 | 1.06 | 1.50 | 1.21 | 0.9 | 1.21 |
| 250 | L250D | 0.24 | ***0.05*** | 0.13 | ***0.05*** | ***0.05*** | ***0.05*** |
| 250 | L250E | 0.20 | 2.64 | ***0.05*** | 0.06 | ***0.05*** | ***0.05*** |
| 250 | L250F | 0.61 | 1.05 | 1.44 | 0.90 | 0.7 | 0.96 |
| 250 | L250G | 0.22 | 0.11 | ***0.05*** | ***0.05*** | ***0.05*** | ***0.05*** |
| 250 | L250H | 0.22 | ***0.05*** | ***0.05*** | ***0.05*** | ***0.05*** | ***0.05*** |
| 250 | L250I | 0.98 | 0.88 | 1.22 | 1.00 | 0.9 | 1.13 |
| 250 | L250K | ***0.05*** | ***0.05*** | ***0.05*** | ***0.05*** | ***0.05*** | ***0.05*** |
| 250 | L250M | 0.90 | 1.09 | 1.13 | 1.20 | 1.0 | 1.05 |
| 250 | L250N | 0.22 | ***0.05*** | ***0.05*** | ***0.05*** | ***0.05*** | ***0.05*** |
| 250 | L250P | 0.25 | ***0.05*** | 0.07 | ***0.05*** | ***0.05*** | ***0.05*** |
| 250 | L250Q | 0.17 | ***0.05*** | ***0.05*** | ***0.05*** | 0.8 | 0.13 |
| 250 | L250R | 0.27 | ***0.05*** | ***0.05*** | ***0.05*** | ***0.05*** | ***0.05*** |
| 250 | L250S | 0.19 | ***0.05*** | 0.06 | ***0.05*** | 0.5 | 0.07 |
| 250 | L250T | 0.25 | 1.49 | 2.25 | 1.69 | 0.8 | 0.81 |
| 250 | L250V | 0.80 | 0.64 | 1.06 | 1.02 | 0.9 | 0.98 |
| 250 | L250W | 0.17 | ***0.05*** | ***0.05*** | ***0.05*** | 0.5 | 0.20 |
| 250 | L250Y | ***0.05*** | ***0.05*** | ***0.05*** | ***0.05*** | ***0.05*** | ***0.05*** |
| 251 | E251A | 0.93 | 0.92 | 0.67 | 0.98 | 0.6 | 0.70 |
| 251 | E251C | 0.66 | 0.99 | 1.04 | 1.14 | 0.8 | 0.69 |
| 251 | E251D | 0.76 | 1.08 | 1.07 | 1.12 | 0.8 | 0.95 |
| 251 | E251F | 0.68 | 0.96 | 0.65 | 1.04 | 0.7 | 0.66 |
| 251 | E251G | 0.65 | 1.07 | 0.83 | 1.17 | 0.6 | 0.95 |
| 251 | E251H | 0.64 | 1.03 | 0.74 | 1.04 | 0.5 | 0.74 |
| 251 | E251I | 0.58 | 0.93 | 0.91 | 0.93 | 0.5 | 0.81 |
| 251 | E251K | 0.62 | 0.54 | 0.41 | 0.89 | 0.1 | 0.65 |
| 251 | E251L | 0.60 | 0.85 | 0.54 | 1.04 | 0.7 | 0.72 |
| 251 | E251M | 0.65 | 0.78 | 0.62 | 0.87 | 0.6 | 0.62 |
| 251 | E251N | 0.69 | 0.96 | 0.78 | 1.09 | 0.6 | 0.85 |
| 251 | E251P | 0.20 | ***0.05*** | ***0.05*** | 1.05 | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 251 | E251Q | 0.61 | 1.12 | 0.91 | 1.10 | 0.5 | 0.77 |
| 251 | E251R | 0.43 | 0.66 | 0.74 | 1.23 | 0.2 | 0.79 |
| 251 | E251S | 0.52 | 0.81 | 0.99 | 0.96 | 0.6 | 0.81 |
| 251 | E251T | 0.69 | 1.02 | 0.91 | 1.00 | 0.7 | 0.93 |
| 251 | E251V | 0.61 | 1.05 | 0.78 | 1.19 | 0.6 | 0.88 |
| 251 | E251W | 0.50 | 1.21 | 0.90 | 1.07 | 0.3 | 0.73 |
| 251 | E251Y | 0.58 | 0.92 | 0.57 | 0.97 | 0.4 | 0.74 |
| 252 | N252A | 1.02 | 1.03 | 1.28 | 0.97 | 1.0 | 0.93 |
| 252 | N252C | 1.01 | 0.91 | 0.88 | 1.01 | 1.1 | 0.83 |
| 252 | N252D | ***0.05*** | ***0.05*** | ***0.05*** | ***0.05*** | ***0.05*** | ***0.05*** |
| 252 | N252E | 0.77 | 0.94 | 0.93 | 1.01 | 1.1 | 0.95 |
| 252 | N252F | 0.27 | 1.07 | 0.77 | 1.10 | 0.9 | 0.49 |
| 252 | N252G | 0.88 | 1.23 | 0.86 | 1.08 | 1.1 | 0.97 |
| 252 | N252H | 0.94 | 1.17 | 0.88 | 1.09 | 1.1 | 0.97 |
| 252 | N2521 | 0.81 | 1.07 | 1.11 | 0.94 | 1.0 | 1.10 |
| 252 | N252K | 1.08 | 0.87 | 0.86 | 0.93 | 1.0 | 0.90 |
| 252 | N252L | 0.96 | 1.09 | 0.85 | 1.08 | 1.0 | 1.02 |
| 252 | N252M | 0.89 | 0.98 | 1.11 | 1.10 | 1.1 | 1.03 |
| 252 | N252P | 0.18 | ***0.05*** | ***0.05*** | ***0.05*** | ***0.05*** | ***0.05*** |
| 252 | N252Q | 1.37 | 1.32 | 1.08 | 1.09 | 1.1 | 0.98 |
| 252 | N252R | 0.95 | 0.84 | 0.90 | 1.08 | 0.9 | 0.99 |
| 252 | N252S | 0.95 | 1.05 | 1.13 | 1.03 | 1.0 | 1.16 |
| 252 | N252T | 0.81 | 1.23 | 1.11 | 1.18 | 1.1 | 1.05 |
| 252 | N252V | 0.74 | 0.99 | 0.99 | 1.28 | 1.0 | 1.24 |
| 252 | N252W | 0.80 | 1.12 | 1.04 | 1.10 | 1.0 | 1.21 |
| 252 | N252Y | 0.82 | 1.06 | 0.93 | 1.14 | 1.0 | 1.09 |
| 253 | T253A | 1.02 | 0.94 | 1.16 | 0.98 | 0.9 | 0.82 |
| 253 | T253C | 0.81 | 0.89 | 1.19 | 0.95 | 1.0 | 0.86 |
| 253 | T253D | 0.77 | 1.10 | 1.02 | 1.03 | 0.8 | 0.78 |
| 253 | T253E | 0.91 | 1.10 | 1.08 | 0.92 | 1.0 | 0.87 |
| 253 | T253F | 1.00 | 1.10 | 0.88 | 1.01 | 1.0 | 0.82 |
| 253 | T253G | 0.97 | 0.97 | 1.04 | 1.05 | 0.8 | 0.86 |
| 253 | T253H | 1.25 | 0.99 | 0.94 | 1.04 | 1.0 | 0.89 |
| 253 | T253I | 0.43 | 1.14 | 0.91 | 0.90 | 0.1 | 0.66 |
| 253 | T253K | 0.94 | 0.89 | 0.83 | 1.09 | 0.6 | 0.88 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 253 | T253L | 0.86 | 1.15 | 0.85 | 1.08 | 0.8 | 1.00 |
| 253 | T253M | 1.04 | 0.88 | 1.05 | 1.19 | 0.8 | 0.84 |
| 253 | T253N | 0.92 | 1.10 | 1.14 | 1.03 | 1.0 | 0.88 |
| 253 | T253P | 0.18 | ***0.05*** | ***0.05*** | ***0.05*** | 0.2 | 0.10 |
| 253 | T253Q | 0.88 | 1.05 | 1.09 | 1.08 | 0.8 | 1.27 |
| 253 | T253R | 0.83 | 0.70 | 0.73 | 1.08 | 0.4 | 0.92 |
| 253 | T253S | 1.00 | 1.13 | 1.23 | 1.08 | 1.0 | 0.94 |
| 253 | T253V | 0.49 | 1.28 | 1.10 | 1.09 | 0.2 | 1.10 |
| 253 | T253W | ***0.05*** | ***0.05*** | ***0.05*** | ***0.05*** | ***0.05*** | ***0.05*** |
| 253 | T253Y | 0.21 | 3.47 | 2.00 | 1.61 | 0.2 | 0.10 |
| 254 | T254A | 1.14 | 0.93 | 1.13 | 1.00 | 1.0 | 0.76 |
| 254 | T254C | 1.01 | 0.99 | 1.08 | 1.14 | 1.1 | 0.83 |
| 254 | T254D | 0.26 | 2.25 | 1.81 | 1.66 | ***0.05*** | 0.53 |
| 254 | T254E | 0.25 | 1.23 | 1.03 | 1.06 | 0.1 | 0.39 |
| 254 | T254F | 0.17 | ***0.05*** | 0.11 | ***0.05*** | ***0.05*** | ***0.05*** |
| 254 | T254G | 0.55 | 1.00 | 1.16 | 1.13 | 0.8 | 0.85 |
| 254 | T254H | 0.16 | ***0.05*** | ***0.05*** | ***0.05*** | ***0.05*** | 0.46 |
| 254 | T254I | 0.48 | 1.08 | 1.07 | 1.16 | 0.2 | 0.81 |
| 254 | T254K | 0.21 | 0.88 | ***0.05*** | 0.60 | 0.1 | 0.22 |
| 254 | T254L | 0.37 | 1.14 | 1.00 | 1.33 | ***0.05*** | 0.61 |
| 254 | T254M | 0.27 | 1.06 | 0.92 | 1.30 | 0.1 | 0.34 |
| 254 | T254N | ***0.05*** | ***0.05*** | ***0.05*** | ***0.05*** | ***0.05*** | ***0.05*** |
| 254 | T254P | 0.83 | 1.16 | 0.97 | 1.07 | 0.7 | 0.88 |
| 254 | T254Q | 0.21 | 32.63 | 41.13 | 3.71 | ***0.05*** | 0.47 |
| 254 | T254R | 0.21 | 7.65 | 3.72 | 1.56 | ***0.05*** | 0.60 |
| 254 | T254S | 0.93 | 0.95 | 1.03 | 0.90 | 1.0 | 0.92 |
| 254 | T254V | 1.00 | 1.19 | 0.97 | 1.07 | 0.8 | 1.05 |
| 254 | T254W | 0.17 | ***0.05*** | ***0.05*** | ***0.05*** | ***0.05*** | ***0.05*** |
| 254 | T254Y | 0.18 | ***0.05*** | ***0.05*** | ***0.05*** | ***0.05*** | 0.14 |
| 255 | T255A | 1.09 | 0.98 | 0.97 | 0.96 | #DIV/0! | 0.85 |
| 255 | T255C | 1.06 | 1.04 | 1.12 | 0.95 | #DIV/0! | 0.89 |
| 255 | T255D | 0.96 | 1.13 | 1.10 | 0.99 | #DIV/0! | 0.91 |
| 255 | T255E | 1.00 | 1.12 | 0.97 | 1.08 | #DIV/0! | 0.97 |
| 255 | T255F | 0.84 | 1.12 | 1.12 | 0.93 | #DIV/0! | 1.27 |
| 255 | T255G | 0.90 | 1.06 | 1.10 | 1.04 | #DIV/0! | 0.99 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 255 | T255H | 0.87 | 1.12 | 1.03 | 1.04 | #DIV/0! | 0.97 |
| 255 | T255I | 0.99 | 1.27 | 0.98 | 1.13 | #DIV/0! | 1.02 |
| 255 | T255K | 1.00 | 0.86 | 0.91 | 1.06 | #DIV/0! | 1.11 |
| 255 | T255L | 0.96 | 1.52 | 1.00 | 0.95 | #DIV/0! | 1.02 |
| 255 | T255M | 1.04 | 0.93 | 1.13 | 0.92 | #DIV/0! | 1.02 |
| 255 | T255N | ***0.05*** | ***0.05*** | ***0.05*** | ***0.05*** | ***0.05*** | ***0.05*** |
| 255 | T255P | 0.24 | 1.56 | 0.99 | 1.35 | #DIV/0! | 0.38 |
| 255 | T255Q | ***0.05*** | ***0.05*** | ***0.05*** | ***0.05*** | ***0.05*** | ***0.05*** |
| 255 | T255R | 0.95 | 0.80 | 0.76 | 0.95 | #DIV/0! | 1.06 |
| 255 | T255S | 0.94 | 0.78 | 1.02 | 0.98 | #DIV/0! | 1.08 |
| 255 | T255V | 0.99 | 1.20 | 1.19 | 1.16 | #DIV/0! | 0.96 |
| 255 | T255W | 0.92 | 1.09 | 1.15 | 0.99 | #DIV/0! | 1.41 |
| 255 | T255Y | ***0.05*** | ***0.05*** | ***0.05*** | ***0.05*** | ***0.05*** | ***0.05*** |
| 256 | K256A | 1.00 | 1.06 | 1.22 | 0.97 | #DIV/0! | 0.96 |
| 256 | K256C | 0.97 | 0.94 | 1.00 | 0.89 | #DIV/0! | 1.10 |
| 256 | K256D | 0.90 | 1.22 | 1.24 | 0.95 | #DIV/0! | 0.96 |
| 256 | K256E | 0.99 | 1.15 | 1.06 | 0.88 | #DIV/0! | 0.94 |
| 256 | K256F | 0.96 | 0.95 | 1.21 | 0.96 | #DIV/0! | 0.98 |
| 256 | K256G | 0.72 | 1.30 | 0.91 | 1.03 | #DIV/0! | 1.00 |
| 256 | K256H | 1.04 | 1.20 | 1.20 | 1.03 | #DIV/0! | 1.03 |
| 256 | K256I | 0.93 | 1.18 | 1.22 | 1.03 | #DIV/0! | 1.03 |
| 256 | K256L | 0.97 | 1.21 | 1.12 | 0.92 | #DIV/0! | 1.04 |
| 256 | K256M | 0.94 | 1.17 | 1.29 | 1.10 | #DIV/0! | 1.12 |
| 256 | K256N | 0.90 | 1.12 | 1.15 | 0.94 | #DIV/0! | 1.09 |
| 256 | K256P | 0.91 | 1.10 | 1.09 | 1.00 | #DIV/0! | 1.35 |
| 256 | K256Q | 0.89 | 1.24 | 1.18 | 1.11 | #DIV/0! | 1.02 |
| 256 | K256R | 1.13 | 1.42 | 1.03 | 1.05 | #DIV/0! | 0.94 |
| 256 | K256S | 0.91 | 1.19 | 1.20 | 1.00 | #DIV/0! | 1.11 |
| 256 | K256T | 0.92 | 1.07 | 1.29 | 1.11 | #DIV/0! | 1.52 |
| 256 | K256V | 0.82 | 1.15 | 1.48 | 1.14 | #DIV/0! | 1.10 |
| 256 | K256W | 0.84 | 1.15 | 1.14 | 0.91 | #DIV/0! | 1.15 |
| 256 | K256Y | 0.44 | 1.23 | 1.27 | 1.02 | #DIV/0! | 1.02 |
| 257 | L257A | 0.72 | 1.00 | 1.27 | 0.91 | 0.6 | 0.99 |
| 257 | L257C | 0.78 | 1.09 | 1.04 | 1.05 | 0.8 | 0.83 |
| 257 | L257D | 0.37 | 1.17 | 1.22 | 0.83 | 0.4 | 0.68 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 257 | L257E | 0.50 | 1.05 | 1.27 | 0.92 | 0.6 | 0.81 |
| 257 | L257F | 0.67 | 0.60 | 1.05 | 0.91 | 0.2 | 0.98 |
| 257 | L257G | 0.67 | 1.01 | 0.64 | 1.20 | 0.7 | 0.90 |
| 257 | L257H | 0.56 | 0.83 | 1.00 | 1.01 | 0.4 | 0.87 |
| 257 | L257I | 0.83 | 0.89 | 1.04 | 0.97 | 0.9 | 1.06 |
| 257 | L257K | 0.59 | 0.62 | 0.75 | 0.85 | ***0.05*** | 0.90 |
| 257 | L257M | 0.84 | 1.06 | 0.93 | 0.86 | 0.8 | 0.83 |
| 257 | L257N | 0.52 | 1.16 | 1.00 | 1.00 | 0.5 | 0.87 |
| 257 | L257P | 0.45 | 0.69 | 0.96 | 0.91 | 0.3 | 0.73 |
| 257 | L257Q | ***0.05*** | ***0.05*** | ***0.05*** | ***0.05*** | ***0.05*** | ***0.05*** |
| 257 | L257R | 0.59 | 0.64 | 0.76 | 1.04 | 0.1 | 0.88 |
| 257 | L257S | 0.62 | 0.93 | 1.10 | 0.97 | 0.5 | 1.03 |
| 257 | L257T | 0.62 | 1.05 | 1.29 | 1.00 | 0.7 | 1.06 |
| 257 | L257V | 0.76 | 0.91 | 0.90 | 0.97 | 0.9 | 0.90 |
| 257 | L257W | 0.44 | 1.26 | 1.03 | 1.18 | ***0.05*** | 0.86 |
| 257 | L257Y | 0.62 | 1.17 | 1.00 | 0.98 | 0.4 | 0.84 |
| 258 | G258A | 0.59 | 1.05 | 1.13 | 1.14 | 0.1 | 0.61 |
| 258 | G258C | 0.53 | 0.87 | 1.15 | 1.03 | 0.4 | 0.62 |
| 258 | G258E | 0.57 | 0.98 | 1.11 | 1.12 | 0.9 | 0.71 |
| 258 | G258F | 0.48 | 0.99 | 1.09 | 1.05 | ***0.05*** | 0.75 |
| 258 | G258H | 0.50 | 1.00 | 1.07 | 1.09 | ***0.05*** | 0.68 |
| 258 | G258I | 0.29 | 1.26 | 1.39 | 1.41 | 0.3 | 0.53 |
| 258 | G258K | 0.50 | 0.99 | 0.92 | 1.15 | ***0.05*** | 0.82 |
| 258 | G258L | 0.50 | 0.89 | 1.10 | 1.14 | ***0.05*** | 0.72 |
| 258 | G258M | 0.43 | 1.02 | 1.05 | 1.29 | 0.1 | 0.63 |
| 258 | G258P | 0.31 | 1.41 | 1.63 | 1.56 | ***0.05*** | 0.65 |
| 258 | G258Q | 0.52 | 1.05 | 1.26 | 1.12 | 0.4 | 0.80 |
| 258 | G258R | 0.40 | 0.86 | 0.83 | 1.20 | ***0.05*** | 0.71 |
| 258 | G258S | 0.48 | 0.70 | 1.01 | 1.01 | ***0.05*** | 0.64 |
| 258 | G258T | ***0.05*** | ***0.05*** | ***0.05*** | ***0.05*** | ***0.05*** | ***0.05*** |
| 258 | G258V | 0.38 | 1.26 | 1.57 | 1.45 | 0.6 | 0.81 |
| 258 | G258W | 0.44 | 1.03 | 1.28 | 1.19 | ***0.05*** | 0.73 |
| 258 | G258Y | 0.46 | 1.08 | 1.13 | 1.33 | ***0.05*** | 0.79 |
| 259 | D259A | 0.94 | 0.61 | 0.43 | 0.93 | #DIV/0! | 1.03 |
| 259 | D259C | 1.04 | 0.84 | 0.98 | 0.90 | #DIV/0! | 1.02 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 259 | D259E | 0.99 | 1.15 | 0.97 | 0.98 | #DIV/0! | 1.05 |
| 259 | D259F | 0.77 | 0.54 | 0.41 | 0.66 | #DIV/0! | 1.09 |
| 259 | D259G | 0.91 | 0.58 | 0.58 | 0.94 | #DIV/0! | 1.07 |
| 259 | D259H | ***0.05*** | ***0.05*** | ***0.05*** | ***0.05*** | ***0.05*** | ***0.05*** |
| 259 | D259I | ***0.05*** | ***0.05*** | ***0.05*** | ***0.05*** | ***0.05*** | ***0.05*** |
| 259 | D259K | 1.16 | 0.31 | 0.46 | 0.72 | #DIV/0! | 1.08 |
| 259 | D259L | 0.98 | 0.52 | 0.51 | 0.69 | #DIV/0! | 1.04 |
| 259 | D259M | 0.91 | 0.50 | 0.53 | 0.78 | #DIV/0! | 1.07 |
| 259 | D259N | 1.02 | 0.60 | 0.75 | 1.08 | #DIV/0! | 1.19 |
| 259 | D259P | 0.87 | 0.61 | 0.74 | 0.83 | #DIV/0! | 1.11 |
| 259 | D259Q | 1.02 | 0.78 | 0.69 | 1.01 | #DIV/0! | 1.13 |
| 259 | D259R | 0.89 | 0.32 | 0.40 | 0.80 | #DIV/0! | 1.14 |
| 259 | D259S | 0.92 | 0.53 | 0.66 | 0.93 | #DIV/0! | 1.07 |
| 259 | D259T | 0.77 | 0.73 | 0.75 | 1.00 | #DIV/0! | 1.17 |
| 259 | D259V | 0.72 | 0.51 | 0.63 | 0.89 | #DIV/0! | 1.29 |
| 259 | D259W | 0.71 | 0.36 | 0.50 | 0.80 | #DIV/0! | 1.24 |
| 259 | D259Y | 0.66 | 0.42 | 0.48 | 0.73 | #DIV/0! | 1.28 |
| 260 | S260A | 1.06 | 0.95 | 1.29 | 0.98 | #DIV/0! | 0.91 |
| 260 | S260C | 0.99 | 1.11 | 1.13 | 0.82 | #DIV/0! | 1.05 |
| 260 | S260D | 0.94 | 1.22 | 1.05 | 0.90 | #DIV/0! | 0.92 |
| 260 | S260E | 1.03 | 1.11 | 1.10 | 0.97 | #DIV/0! | 0.88 |
| 260 | S260F | 0.89 | 0.91 | 1.17 | 0.99 | #DIV/0! | 0.93 |
| 260 | S260G | 0.97 | 1.21 | 1.11 | 1.12 | #DIV/0! | 1.27 |
| 260 | S260H | 1.17 | 0.97 | 1.10 | 0.95 | #DIV/0! | 1.08 |
| 260 | S260I | 0.48 | 1.13 | 1.16 | 1.15 | #DIV/0! | 0.98 |
| 260 | S260K | ***0.05*** | ***0.05*** | ***0.05*** | ***0.05*** | ***0.05*** | ***0.05*** |
| 260 | S260L | 0.92 | 1.22 | 1.00 | 1.16 | #DIV/0! | 1.05 |
| 260 | S260M | 0.99 | 1.06 | 1.07 | 1.05 | #DIV/0! | 0.97 |
| 260 | S260N | 0.94 | 1.13 | 1.20 | 1.14 | #DIV/0! | 1.06 |
| 260 | S260P | 1.02 | 1.19 | 1.07 | 0.99 | #DIV/0! | 1.14 |
| 260 | S260Q | ***0.05*** | ***0.05*** | ***0.05*** | ***0.05*** | ***0.05*** | ***0.05*** |
| 260 | S260R | 1.01 | 0.98 | 0.70 | 0.99 | #DIV/0! | 0.98 |
| 260 | S260T | 0.36 | 0.48 | 0.59 | 0.74 | #DIV/0! | 0.10 |
| 260 | S260V | 0.85 | 1.05 | 1.07 | 0.98 | #DIV/0! | 1.28 |
| 260 | S260W | 0.69 | 1.15 | 0.88 | 1.10 | #DIV/0! | 1.04 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 260 | S260Y | 0.86 | 1.28 | 0.92 | 1.07 | #DIV/0! | 1.20 |
| 261 | F261A | 0.55 | 0.82 | 0.95 | 0.91 | 0.5 | 0.59 |
| 261 | F261C | 0.57 | 0.80 | 1.08 | 0.95 | 0.7 | 0.68 |
| 261 | F261D | ***0.05*** | ***0.05*** | ***0.05*** | ***0.05*** | ***0.05*** | ***0.05*** |
| 261 | F261E | 0.65 | 0.65 | 1.05 | 0.94 | 0.7 | 0.68 |
| 261 | F261G | 0.46 | 0.75 | 0.82 | 0.80 | 0.2 | 0.55 |
| 261 | F261H | 0.74 | 0.92 | 1.04 | 0.99 | 0.8 | 0.77 |
| 261 | F261I | ***0.05*** | ***0.05*** | ***0.05*** | ***0.05*** | ***0.05*** | ***0.05*** |
| 261 | F261K | ***0.05*** | ***0.05*** | ***0.05*** | ***0.05*** | ***0.05*** | ***0.05*** |
| 261 | F261L | 0.77 | 0.84 | 0.77 | 0.95 | 0.8 | 0.62 |
| 261 | F261M | 0.73 | 0.79 | 0.90 | 0.76 | 0.7 | 0.73 |
| 261 | F261N | 0.80 | 0.92 | 0.88 | 0.92 | 0.8 | 0.72 |
| 261 | F261P | 0.45 | 0.87 | 0.86 | 0.95 | 0.3 | 0.60 |
| 261 | F261Q | 0.57 | 0.78 | 1.10 | 0.95 | 0.7 | 0.73 |
| 261 | F261R | 0.58 | 0.71 | 0.48 | 0.93 | 0.2 | 0.66 |
| 261 | F261S | 0.54 | 0.89 | 0.87 | 0.98 | 0.5 | 0.68 |
| 261 | F261T | 0.56 | 0.98 | 1.26 | 1.25 | 0.7 | 0.88 |
| 261 | F261V | 0.57 | 0.92 | 1.09 | 0.87 | 0.7 | 0.78 |
| 261 | F261W | 0.89 | 0.70 | 0.98 | 1.18 | 1.1 | 0.80 |
| 261 | F261Y | 0.67 | 0.63 | 0.94 | 0.97 | 0.9 | 0.79 |
| 262 | Y262A | 0.83 | 0.81 | 0.96 | 0.84 | 0.5 | 0.70 |
| 262 | Y262C | 1.10 | 0.91 | 0.98 | 0.83 | 0.9 | 0.78 |
| 262 | Y262D | 0.92 | 0.99 | 0.91 | 0.97 | 0.8 | 0.61 |
| 262 | Y262E | 0.86 | 1.01 | 1.11 | 1.06 | 0.9 | 0.77 |
| 262 | Y262F | 1.11 | 0.77 | 1.10 | 0.93 | 0.9 | 0.93 |
| 262 | Y262G | 0.55 | 0.94 | 0.60 | 1.03 | 0.2 | 0.56 |
| 262 | Y262H | 0.93 | 0.81 | 0.77 | 1.01 | 0.9 | 0.87 |
| 262 | Y262I | 0.72 | 0.59 | 0.86 | 0.90 | 0.6 | 0.53 |
| 262 | Y262K | 0.84 | 0.53 | 0.67 | 0.95 | 0.1 | 0.96 |
| 262 | Y262L | 0.92 | 0.96 | 1.06 | 0.90 | 0.9 | 0.81 |
| 262 | Y262M | 1.00 | 0.60 | 0.88 | 1.02 | 0.9 | 0.75 |
| 262 | Y262N | 0.82 | 1.00 | 1.13 | 0.86 | 0.7 | 0.77 |
| 262 | Y262P | 0.23 | 1.18 | 1.64 | 1.11 | ***0.05*** | 0.24 |
| 262 | Y262Q | 0.82 | 0.86 | 0.96 | 0.91 | 0.8 | 0.72 |
| 262 | Y262R | 0.78 | 0.39 | 0.62 | 0.83 | ***0.05*** | 0.76 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 262 | Y262S | 0.79 | 1.08 | 0.91 | 1.00 | 0.7 | 0.80 |
| 262 | Y262T | 0.65 | 0.94 | 0.95 | 0.86 | 0.7 | 0.71 |
| 262 | Y262V | 0.51 | 1.01 | 0.90 | 0.94 | 0.6 | 0.69 |
| 262 | Y262W | 0.83 | 0.81 | 0.99 | 1.03 | 0.8 | 0.89 |
| 263 | Y263A | 0.56 | 0.84 | 0.93 | 0.93 | 0.1 | 0.76 |
| 263 | Y263C | 0.98 | 0.86 | 0.98 | 0.93 | 0.6 | 0.90 |
| 263 | Y263D | 0.31 | 1.61 | 1.44 | 1.22 | 0.5 | 0.71 |
| 263 | Y263E | 0.47 | 1.02 | 0.98 | 0.86 | 0.5 | 0.80 |
| 263 | Y263F | 1.03 | 1.14 | 0.97 | 0.89 | 0.9 | 0.80 |
| 263 | Y263G | 0.32 | 0.59 | 1.09 | 0.83 | ***0.05*** | 0.41 |
| 263 | Y263H | ***0.05*** | ***0.05*** | ***0.05*** | ***0.05*** | ***0.05*** | ***0.05*** |
| 263 | Y263I | 0.27 | 1.10 | 1.16 | 1.17 | ***0.05*** | 0.41 |
| 263 | Y263K | 0.22 | 2.39 | 2.76 | 1.86 | 0.1 | 0.34 |
| 263 | Y263L | 0.84 | 0.89 | 0.83 | 0.95 | 0.2 | 1.00 |
| 263 | Y263M | 0.96 | 0.99 | 1.08 | 0.97 | 0.6 | 0.74 |
| 263 | Y263N | ***0.05*** | ***0.05*** | ***0.05*** | ***0.05*** | ***0.05*** | ***0.05*** |
| 263 | Y263P | 0.18 | ***0.05*** | ***0.05*** | ***0.05*** | ***0.05*** | ***0.05*** |
| 263 | Y263Q | ***0.05*** | ***0.05*** | ***0.05*** | ***0.05*** | ***0.05*** | ***0.05*** |
| 263 | Y263R | 0.21 | 8.02 | 4.08 | 1.72 | 0.1 | 0.26 |
| 263 | Y263S | 0.47 | 1.06 | 0.82 | 0.95 | 0.2 | 0.77 |
| 263 | Y263T | 0.91 | 0.84 | 0.97 | 1.10 | 0.7 | 0.97 |
| 263 | Y263V | 0.66 | 0.80 | 0.89 | 0.94 | 0.4 | 1.07 |
| 263 | Y263W | 0.35 | 0.96 | 0.90 | 0.95 | 0.1 | 0.64 |
| 264 | G264A | 0.55 | 0.81 | 0.96 | 0.93 | 0.3 | 0.79 |
| 264 | G264C | 0.25 | ***0.05*** | 0.10 | 0.13 | ***0.05*** | ***0.05*** |
| 264 | G264D | 0.20 | ***0.05*** | ***0.05*** | 1.31 | ***0.05*** | ***0.05*** |
| 264 | G264E | 0.19 | ***0.05*** | 0.30 | ***0.05*** | ***0.05*** | ***0.05*** |
| 264 | G264F | 0.28 | 0.08 | ***0.05*** | ***0.05*** | ***0.05*** | ***0.05*** |
| 264 | G264H | 0.19 | ***0.05*** | ***0.05*** | ***0.05*** | ***0.05*** | ***0.05*** |
| 264 | G264I | 0.43 | ***0.05*** | ***0.05*** | ***0.05*** | ***0.05*** | ***0.05*** |
| 264 | G264K | ***0.05*** | ***0.05*** | ***0.05*** | ***0.05*** | ***0.05*** | ***0.05*** |
| 264 | G264L | 0.26 | ***0.05*** | ***0.05*** | 0.09 | ***0.05*** | ***0.05*** |
| 264 | G264M | 0.20 | ***0.05*** | ***0.05*** | 0.85 | ***0.05*** | ***0.05*** |
| 264 | G264N | 0.18 | ***0.05*** | ***0.05*** | ***0.05*** | 0.3 | 0.08 |
| 264 | G264P | 0.18 | ***0.05*** | 0.07 | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 264 | G264Q | 0.20 | ***0.05*** | ***0.05*** | 1.17 | ***0.05*** | ***0.05*** |
| 264 | G264R | 0.22 | 0.32 | 0.51 | 0.07 | ***0.05*** | ***0.05*** |
| 264 | G264S | 0.25 | 1.28 | 1.13 | 1.32 | ***0.05*** | 0.44 |
| 264 | G264T | 0.22 | ***0.05*** | 0.93 | 0.12 | ***0.05*** | ***0.05*** |
| 264 | G264V | 0.17 | ***0.05*** | ***0.05*** | ***0.05*** | ***0.05*** | ***0.05*** |
| 264 | G264W | 0.18 | ***0.05*** | ***0.05*** | ***0.05*** | ***0.05*** | ***0.05*** |
| 264 | G264Y | 0.17 | ***0.05*** | ***0.05*** | ***0.05*** | ***0.05*** | ***0.05*** |
| 265 | K265A | 1.18 | 1.03 | 1.24 | 0.86 | 0.8 | 0.96 |
| 265 | K265C | 0.92 | 0.96 | 1.54 | 1.11 | 0.9 | 0.93 |
| 265 | K265D | 0.59 | 1.05 | 1.24 | 0.85 | 0.9 | 0.77 |
| 265 | K265E | 0.70 | 1.00 | 1.32 | 0.90 | 1.0 | 0.94 |
| 265 | K265F | ***0.05*** | ***0.05*** | ***0.05*** | ***0.05*** | ***0.05*** | ***0.05*** |
| 265 | K265G | 0.89 | 1.22 | 1.38 | 1.04 | 0.7 | 0.97 |
| 265 | K265H | 0.69 | 1.16 | 1.06 | 0.92 | 1.1 | 0.88 |
| 265 | K265I | ***0.05*** | ***0.05*** | ***0.05*** | ***0.05*** | ***0.05*** | ***0.05*** |
| 265 | K265L | 0.59 | 1.11 | 1.44 | 1.11 | 1.2 | 0.97 |
| 265 | K265M | 0.78 | 1.04 | 1.19 | 0.99 | 1.0 | 0.88 |
| 265 | K265N | 1.05 | 1.07 | 1.31 | 1.00 | 1.2 | 0.85 |
| 265 | K265P | 0.25 | 1.61 | 1.92 | 1.59 | 1.0 | 0.80 |
| 265 | K265Q | 0.76 | 1.16 | 1.19 | 1.03 | 1.3 | 0.82 |
| 265 | K265R | 1.16 | 1.11 | 0.88 | 1.17 | 1.1 | 0.92 |
| 265 | K265S | 1.06 | 1.13 | 1.15 | 1.04 | 1.0 | 0.88 |
| 265 | K265T | 0.80 | 1.08 | 1.20 | 1.01 | 1.1 | 0.87 |
| 265 | K265V | 0.51 | 1.12 | 1.33 | 0.96 | 0.9 | 0.87 |
| 265 | K265W | 0.75 | 1.28 | 1.35 | 1.08 | 1.0 | 1.20 |
| 265 | K265Y | 0.85 | 1.07 | 1.25 | 1.02 | 1.0 | 1.26 |
| 266 | G266A | 0.23 | 0.16 | 0.07 | 0.18 | ***0.05*** | ***0.05*** |
| 266 | G266C | 0.21 | 0.22 | 2.27 | 0.36 | ***0.05*** | ***0.05*** |
| 266 | G266D | 0.31 | ***0.05*** | ***0.05*** | ***0.05*** | ***0.05*** | ***0.05*** |
| 266 | G266E | 0.26 | 0.09 | 0.06 | 0.07 | ***0.05*** | ***0.05*** |
| 266 | G266F | 0.26 | 0.08 | ***0.05*** | 0.08 | ***0.05*** | ***0.05*** |
| 266 | G266H | 0.19 | ***0.05*** | ***0.05*** | 20.22 | ***0.05*** | ***0.05*** |
| 266 | G266I | ***0.05*** | ***0.05*** | ***0.05*** | ***0.05*** | ***0.05*** | ***0.05*** |
| 266 | G266K | ***0.05*** | ***0.05*** | ***0.05*** | ***0.05*** | ***0.05*** | ***0.05*** |
| 266 | G266L | 0.26 | ***0.05*** | 0.27 | 0.06 | ***0.05*** | ***0.05*** |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 266 | G266M | 0.32 | ***0.05*** | ***0.05*** | ***0.05*** | ***0.05*** | ***0.05*** |
| 266 | G266N | 0.19 | ***0.05*** | ***0.05*** | ***0.05*** | ***0.05*** | ***0.05*** |
| 266 | G266P | 0.26 | ***0.05*** | ***0.05*** | 0.08 | ***0.05*** | ***0.05*** |
| 266 | G266Q | 0.18 | 0.08 | ***0.05*** | ***0.05*** | ***0.05*** | ***0.05*** |
| 266 | G266R | 0.22 | ***0.05*** | ***0.05*** | 0.12 | ***0.05*** | ***0.05*** |
| 266 | G266S | 0.24 | ***0.05*** | ***0.05*** | 0.11 | ***0.05*** | ***0.05*** |
| 266 | G266T | ***0.05*** | ***0.05*** | ***0.05*** | ***0.05*** | ***0.05*** | ***0.05*** |
| 266 | G266V | 0.22 | 0.51 | 0.33 | 0.16 | ***0.05*** | ***0.05*** |
| 266 | G266W | 0.18 | ***0.05*** | ***0.05*** | ***0.05*** | ***0.05*** | ***0.05*** |
| 266 | G266Y | 0.22 | 0.77 | 0.24 | 0.21 | ***0.05*** | ***0.05*** |
| 267 | L267A | 0.82 | 1.07 | 1.22 | 1.07 | 0.9 | 1.10 |
| 267 | L267C | 0.68 | 1.04 | 1.22 | 1.19 | 0.9 | 1.14 |
| 267 | L267D | 0.13 | ***0.05*** | ***0.05*** | ***0.05*** | 0.1 | 0.17 |
| 267 | L267E | 0.58 | 1.17 | 1.35 | 1.10 | 1.1 | 1.19 |
| 267 | L267F | 0.72 | 0.98 | 1.11 | 1.13 | 0.2 | 0.81 |
| 267 | L267G | 0.62 | 0.71 | 1.13 | 1.16 | 0.3 | 0.97 |
| 267 | L267H | 0.63 | 1.07 | 1.21 | 1.13 | 0.1 | 1.05 |
| 267 | L267I | 0.91 | 1.06 | 1.25 | 1.07 | 1.1 | 0.99 |
| 267 | L267K | 0.57 | 0.98 | 0.89 | 1.04 | ***0.05*** | 1.05 |
| 267 | L267M | 0.77 | 0.94 | 1.22 | 0.95 | 0.9 | 1.00 |
| 267 | L267N | 0.40 | 1.29 | 1.32 | 1.25 | ***0.05*** | 0.88 |
| 267 | L267P | 0.36 | 1.16 | 1.20 | 1.33 | ***0.05*** | 0.82 |
| 267 | L267Q | 0.73 | 1.14 | 1.32 | 1.11 | 0.9 | 1.27 |
| 267 | L267R | 0.53 | 0.96 | 0.91 | 1.06 | ***0.05*** | 0.95 |
| 267 | L267S | 0.71 | 1.03 | 1.07 | 1.16 | 0.5 | 1.15 |
| 267 | L267T | 0.63 | 1.19 | 1.25 | 1.33 | 0.5 | 1.33 |
| 267 | L267V | 0.73 | 1.12 | 1.08 | 1.24 | 0.8 | 1.06 |
| 267 | L267Y | 0.29 | 0.99 | 1.78 | 1.82 | ***0.05*** | 1.02 |
| 268 | I268A | 0.86 | 0.85 | 0.88 | 0.95 | 0.7 | 0.68 |
| 268 | I268C | 0.95 | 0.94 | 0.73 | 1.18 | 0.9 | 0.78 |
| 268 | I268D | ***0.05*** | ***0.05*** | ***0.05*** | ***0.05*** | ***0.05*** | ***0.05*** |
| 268 | I268E | 0.22 | 0.83 | 1.00 | 0.50 | 0.5 | 0.07 |
| 268 | I268F | 0.26 | 1.09 | 1.01 | 1.03 | ***0.05*** | 0.40 |
| 268 | I268G | 0.18 | ***0.05*** | ***0.05*** | ***0.05*** | 0.1 | 0.15 |
| 268 | I268H | 0.19 | 0.15 | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 268 | I268K | 0.22 | 0.07 | 0.40 | 0.20 | ***0.05*** | ***0.05*** |
| 268 | I268L | 0.99 | 0.85 | 1.01 | 1.08 | 0.7 | 0.91 |
| 268 | I268M | 0.88 | 1.03 | 1.06 | 1.04 | 0.3 | 1.08 |
| 268 | I268N | 0.18 | ***0.05*** | ***0.05*** | ***0.05*** | ***0.05*** | ***0.05*** |
| 268 | I268P | 0.69 | 0.69 | 0.76 | 0.82 | 0.4 | 0.60 |
| 268 | I268Q | 0.25 | ***0.05*** | 0.26 | 0.23 | 0.2 | 0.08 |
| 268 | I268R | 0.25 | 0.12 | ***0.05*** | 0.08 | ***0.05*** | ***0.05*** |
| 268 | I268S | 0.30 | 1.40 | 1.31 | 1.55 | ***0.05*** | 0.80 |
| 268 | I268T | 0.25 | 1.90 | 1.96 | 1.76 | 0.8 | 0.44 |
| 268 | I268V | 0.93 | 0.95 | 0.76 | 1.13 | 1.0 | 0.90 |
| 268 | I268W | 0.21 | 2.80 | 2.43 | 0.16 | ***0.05*** | ***0.05*** |
| 268 | I268Y | ***0.05*** | ***0.05*** | ***0.05*** | ***0.05*** | ***0.05*** | ***0.05*** |
| 269 | N269A | 0.48 | 1.02 | 1.00 | 1.07 | 0.3 | 0.82 |
| 269 | N269C | ***0.05*** | ***0.05*** | ***0.05*** | ***0.05*** | ***0.05*** | ***0.05*** |
| 269 | N269D | 1.25 | 1.14 | 0.89 | 1.01 | 1.3 | 0.90 |
| 269 | N269E | 0.83 | 1.26 | 1.08 | 1.05 | 1.1 | 1.01 |
| 269 | N269F | 0.37 | 1.00 | 1.00 | 1.14 | ***0.05*** | 0.71 |
| 269 | N269G | 0.53 | 0.88 | 0.95 | 1.03 | 0.1 | 0.89 |
| 269 | N269H | ***0.05*** | ***0.05*** | ***0.05*** | ***0.05*** | ***0.05*** | ***0.05*** |
| 269 | N2691 | 0.69 | 1.00 | 1.06 | 1.04 | 0.6 | 1.00 |
| 269 | N269K | 0.64 | 0.75 | 0.81 | 1.01 | 0.5 | 0.89 |
| 269 | N269L | 0.39 | 0.88 | 0.96 | 1.17 | 0.1 | 0.84 |
| 269 | N269M | 0.81 | 0.88 | 0.86 | 0.91 | 0.5 | 0.87 |
| 269 | N269P | 0.26 | ***0.05*** | 0.08 | 0.15 | ***0.05*** | ***0.05*** |
| 269 | N269Q | 0.84 | 1.00 | 0.85 | 1.19 | 1.0 | 0.96 |
| 269 | N269R | 0.46 | 0.63 | 0.63 | 0.98 | ***0.05*** | 0.72 |
| 269 | N269S | 0.94 | 1.02 | 1.15 | 1.12 | 0.8 | 1.00 |
| 269 | N269T | 0.56 | 0.85 | 0.91 | 0.91 | 0.3 | 0.88 |
| 269 | N269V | 0.44 | 1.01 | 0.97 | 1.24 | 0.5 | 0.95 |
| 269 | N269W | 0.20 | ***0.05*** | ***0.05*** | 14.66 | 0.1 | 0.41 |
| 269 | N269Y | ***0.05*** | ***0.05*** | ***0.05*** | ***0.05*** | ***0.05*** | ***0.05*** |
| 270 | V270A | 1.21 | 0.98 | 1.07 | 1.02 | 1.0 | 0.77 |
| 270 | V270C | 1.09 | 0.99 | 1.05 | 1.03 | 1.1 | 0.90 |
| 270 | V270E | 0.19 | ***0.05*** | ***0.05*** | ***0.05*** | ***0.05*** | ***0.05*** |
| 270 | V270F | 0.17 | 2.38 | 2.92 | 1.13 | 0.2 | 0.08 |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 270 | V270G | 0.41 | 0.98 | 0.92 | 1.09 | 0.8 | 0.84 |
| 270 | V270H | 0.29 | ***0.05*** | ***0.05*** | ***0.05*** | ***0.05*** | ***0.05*** |
| 270 | V270I | 0.88 | 0.96 | 1.09 | 1.01 | 1.0 | 0.89 |
| 270 | V270K | 0.43 | ***0.05*** | ***0.05*** | ***0.05*** | ***0.05*** | ***0.05*** |
| 270 | V270L | 0.90 | 1.02 | 1.01 | 0.87 | 0.7 | 0.86 |
| 270 | V270M | 0.81 | 0.92 | 1.01 | 0.94 | 0.4 | 0.77 |
| 270 | V270N | 0.64 | 0.94 | 1.31 | 0.89 | 0.7 | 0.93 |
| 270 | V270P | 0.50 | 0.91 | 1.08 | 0.93 | 0.8 | 0.80 |
| 270 | V270Q | 0.20 | 0.31 | ***0.05*** | ***0.05*** | ***0.05*** | ***0.05*** |
| 270 | V270R | 0.51 | ***0.05*** | ***0.05*** | ***0.05*** | ***0.05*** | ***0.05*** |
| 270 | V270S | 1.08 | 0.99 | 0.95 | 0.78 | 1.0 | 0.84 |
| 270 | V270T | 0.94 | 0.96 | 1.07 | 1.01 | 0.8 | 1.06 |
| 270 | V270W | 0.23 | 0.11 | ***0.05*** | ***0.05*** | ***0.05*** | ***0.05*** |
| 270 | V270Y | 0.39 | ***0.05*** | ***0.05*** | ***0.05*** | ***0.05*** | ***0.05*** |
| 271 | Q271A | 1.18 | 0.94 | 1.12 | 1.04 | 1.1 | 0.83 |
| 271 | Q271C | 0.99 | 1.02 | 0.98 | 1.08 | 1.2 | 0.86 |
| 271 | Q271D | 1.00 | 1.03 | 0.84 | 1.00 | 1.1 | 0.89 |
| 271 | Q271E | 1.01 | 0.93 | 1.16 | 0.95 | 1.2 | 1.02 |
| 271 | Q271F | 1.09 | 1.04 | 0.80 | 1.05 | 1.0 | 0.89 |
| 271 | Q271G | 0.92 | 0.97 | 0.86 | 1.06 | 0.7 | 0.96 |
| 271 | Q271H | 0.92 | 1.01 | 0.85 | 1.09 | 1.0 | 1.04 |
| 271 | Q271I | 1.07 | 1.04 | 0.88 | 0.96 | 1.0 | 0.86 |
| 271 | Q271K | 1.03 | 0.60 | 0.57 | 1.09 | 0.5 | 1.01 |
| 271 | Q271L | 1.16 | 0.90 | 0.94 | 1.01 | 1.0 | 0.88 |
| 271 | Q271M | 1.15 | 0.89 | 1.19 | 1.11 | 1.0 | 0.96 |
| 271 | Q271N | 0.99 | 0.91 | 0.91 | 1.09 | 0.9 | 1.01 |
| 271 | Q271P | 1.03 | 0.75 | 1.12 | 1.06 | 1.1 | 0.76 |
| 271 | Q271R | 1.23 | 0.63 | 1.09 | 1.05 | 0.2 | 0.95 |
| 271 | Q271S | 0.97 | 0.85 | 1.02 | 0.98 | 1.0 | 0.96 |
| 271 | Q271T | 0.88 | 0.97 | 0.91 | 1.11 | 0.8 | 0.99 |
| 271 | Q271V | 0.92 | 1.06 | 0.84 | 1.11 | 0.9 | 0.97 |
| 271 | Q271W | 1.00 | 0.90 | 0.94 | 1.14 | 0.9 | 1.07 |
| 271 | Q271Y | 0.96 | 0.94 | 0.95 | 0.98 | 0.9 | 0.97 |
| 272 | A272C | ***0.05*** | ***0.05*** | ***0.05*** | ***0.05*** | ***0.05*** | ***0.05*** |
| 272 | A272D | ***0.05*** | ***0.05*** | ***0.05*** | ***0.05*** | ***0.05*** | ***0.05*** |

(continued)

| Table 6-1. Performance Index Values for BPN' Variants | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 272 | A272E | 1.08 | 0.82 | 1.09 | 0.96 | 1.1 | 0.79 |
| 272 | A272F | 0.88 | 1.02 | 1.08 | 0.99 | 0.9 | 0.91 |
| 272 | A272G | 1.07 | 0.79 | 1.23 | 1.03 | 0.8 | 0.91 |
| 272 | A272H | 1.12 | 0.90 | 1.19 | 1.00 | 0.9 | 0.82 |
| 272 | A272I | 0.76 | 0.72 | 1.23 | 0.98 | 1.0 | 0.94 |
| 272 | A272K | 1.00 | 0.61 | 1.02 | 0.95 | 0.9 | 0.89 |
| 272 | A272L | 0.99 | 0.87 | 1.12 | 0.95 | 1.1 | 0.81 |
| 272 | A272M | 0.98 | 0.77 | 0.98 | 1.08 | 0.9 | 0.86 |
| 272 | A272N | ***0.05*** | ***0.05*** | ***0.05*** | ***0.05*** | ***0.05*** | ***0.05*** |
| 272 | A272P | 0.92 | 0.93 | 1.05 | 0.95 | 0.8 | 0.92 |
| 272 | A272Q | 1.19 | 0.86 | 1.18 | 1.02 | 1.0 | 0.88 |
| 272 | A272R | 0.99 | 0.47 | 0.99 | 0.81 | 0.8 | 1.00 |
| 272 | A272S | 1.00 | 1.04 | 1.19 | 1.09 | 1.0 | 0.97 |
| 272 | A272T | 0.88 | 0.97 | 0.99 | 1.04 | 1.0 | 1.19 |
| 272 | A272V | 0.80 | 1.01 | 1.14 | 0.99 | 1.0 | 1.00 |
| 272 | A272W | 0.91 | 0.52 | 1.04 | 1.07 | 0.9 | 1.21 |
| 272 | A272Y | 0.95 | 0.95 | 1.28 | 1.00 | 1.0 | 0.93 |
| 273 | A273E | 0.59 | 0.92 | 0.99 | 1.05 | 0.2 | 0.57 |
| 273 | A273G | 0.87 | 0.93 | 1.21 | 1.22 | 0.8 | 0.94 |
| 273 | A273H | 0.73 | 1.05 | 0.98 | 1.09 | 0.4 | 0.75 |
| 273 | A273K | 0.23 | 1.63 | 1.61 | 1.93 | ***0.05*** | 0.58 |
| 273 | A273L | 0.70 | 0.88 | 1.13 | 1.08 | 0.3 | 0.71 |
| 273 | A273N | 0.41 | 1.13 | 1.13 | 1.15 | 0.1 | 0.66 |
| 273 | A273P | 0.29 | 1.50 | 1.73 | 1.83 | ***0.05*** | 0.65 |
| 273 | A273Q | 0.44 | 1.09 | 1.19 | 1.42 | 0.1 | 0.75 |
| 273 | A273R | 0.24 | 1.43 | 1.20 | 2.03 | ***0.05*** | 0.63 |
| 273 | A273S | 0.87 | 1.06 | 1.35 | 1.15 | 0.7 | 0.98 |
| 273 | A273T | 0.53 | 0.79 | 1.23 | 1.29 | 0.2 | 0.91 |
| 273 | A273V | 0.53 | 1.41 | 1.24 | 1.15 | 0.3 | 0.84 |
| 273 | A273W | 0.26 | 2.08 | 1.97 | 1.53 | ***0.05*** | 0.81 |
| 274 | A274C | 0.94 | 1.10 | 0.99 | 0.96 | 1.0 | 0.97 |
| 274 | A274D | 0.94 | 0.92 | 0.87 | 0.89 | 0.7 | 1.05 |
| 274 | A274E | 0.45 | ***0.05*** | ***0.05*** | ***0.05*** | ***0.05*** | ***0.05*** |
| 274 | A274F | 0.80 | 0.82 | 0.84 | 0.96 | 0.6 | 0.74 |
| 274 | A274G | 0.85 | 0.94 | 0.88 | 0.92 | 0.9 | 0.96 |

(continued)

| Table 6-1. Performance Index Values for BPN' Variants | | | | | | | |
|---|---|---|---|---|---|---|---|
| **Position** | **BPN' variant** | **TCA PI** | **PI BMI pH 8 16C** | **PI BMI pH 7 16C** | **PI BMI pH 8 32C** | **LAS-EDTA PI** | **AAPF PI** |
| 274 | A274H | 0.79 | 1.17 | 0.89 | 0.95 | 0.5 | 0.98 |
| 274 | A274I | 0.98 | 0.99 | 0.92 | 1.07 | 1.0 | 1.03 |
| 274 | A274K | 0.54 | 0.94 | 0.61 | 0.95 | 0.2 | 0.76 |
| 274 | A274L | 0.78 | 1.21 | 0.92 | 1.03 | 1.1 | 0.83 |
| 274 | A274M | 0.84 | 0.87 | 0.72 | 0.98 | 1.0 | 0.72 |
| 274 | A274N | ***0.05*** | ***0.05*** | ***0.05*** | ***0.05*** | ***0.05*** | ***0.05*** |
| 274 | A274P | 0.54 | 0.99 | 0.70 | 1.02 | 0.4 | 0.63 |
| 274 | A274Q | 0.77 | 1.05 | 0.90 | 1.07 | 0.8 | 0.93 |
| 274 | A274R | 0.41 | 0.85 | 0.58 | 1.12 | 0.1 | 1.12 |
| 274 | A274S | 1.12 | 0.92 | 0.88 | 1.05 | 1.0 | 0.97 |
| 274 | A274T | 0.91 | 1.15 | 0.83 | 1.06 | 1.0 | 1.08 |
| 274 | A274V | 0.96 | 1.14 | 0.76 | 1.19 | 1.0 | 1.10 |
| 274 | A274W | 0.37 | 1.32 | 0.74 | 1.09 | 0.5 | 0.70 |
| 274 | A274Y | 0.57 | 1.06 | 0.86 | 1.05 | 0.6 | 0.93 |
| 275 | Q275A | 0.70 | 0.95 | 1.23 | 0.99 | 1.0 | 0.84 |
| 275 | Q275C | 0.41 | 1.04 | 1.23 | 0.91 | 1.1 | 0.83 |
| 275 | Q275D | 1.04 | 1.21 | 1.03 | 1.01 | 1.1 | 1.08 |
| 275 | Q275E | 1.17 | 1.01 | 1.20 | 1.23 | 1.1 | 0.95 |
| 275 | Q275F | 0.70 | 0.86 | 0.97 | 0.89 | 0.9 | 1.01 |
| 275 | Q275G | 0.90 | 0.90 | 0.81 | 1.05 | 1.1 | 0.77 |
| 275 | Q275H | 1.06 | 0.79 | 0.96 | 1.01 | 1.1 | 0.88 |
| 275 | Q275I | ***0.05*** | ***0.05*** | ***0.05*** | ***0.05*** | ***0.05*** | ***0.05*** |
| 275 | Q275K | 1.02 | 0.70 | 0.80 | 1.06 | 0.9 | 0.92 |
| 275 | Q275L | 0.86 | 0.78 | 1.01 | 0.97 | 1.0 | 1.00 |
| 275 | Q275M | 0.96 | 1.16 | 0.96 | 0.97 | 1.0 | 0.81 |
| 275 | Q275N | ***0.05*** | ***0.05*** | ***0.05*** | ***0.05*** | ***0.05*** | ***0.05*** |
| 275 | Q275P | 0.74 | 1.10 | 1.00 | 1.00 | 1.1 | 0.96 |
| 275 | Q275R | 1.05 | 0.81 | 0.80 | 1.03 | 0.9 | 0.97 |
| 275 | Q275S | 0.91 | 1.12 | 1.13 | 1.01 | 1.0 | 0.97 |
| 275 | Q275T | 0.85 | 1.06 | 1.01 | 1.05 | 1.0 | 0.98 |
| 275 | Q275V | 0.70 | 1.06 | 1.09 | 0.97 | 1.1 | 1.07 |
| 275 | Q275W | 0.96 | 1.02 | 1.08 | 1.20 | 1.0 | 1.09 |
| 275 | Q275Y | ***0.05*** | ***0.05*** | ***0.05*** | ***0.05*** | ***0.05*** | ***0.05*** |

**Combinable mutations:**

Table 6-2 lists subtilisin BPN' variants which are Combinable Mutations for the 275 positions (2,907). These variants have Performance index values ≥0.5 for at least one property, and >0.05 for both properties.

**Table 6-2. Combinable Mutations of BPN'**

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 1 | A001C | 0.74 | 0.84 | 1.10 | 0.79 | 1.1 | 0.87 |
| 1 | A001D | 1.34 | 0.93 | 1.12 | 0.88 | 1.1 | 0.88 |
| 1 | A001E | 0.82 | 0.94 | 0.85 | 0.84 | 1.5 | 0.94 |
| 1 | A001F | 1.39 | 0.94 | 1.01 | 0.84 | 0.4 | 0.90 |
| 1 | A001G | 1.39 | 0.90 | 1.12 | 1.06 | 1.1 | 1.04 |
| 1 | A001H | 1.26 | 0.99 | 1.04 | 0.98 | 0.9 | 0.97 |
| 1 | A001I | 1.50 | 0.72 | 1.00 | 0.92 | 0.6 | 0.88 |
| 1 | A001K | 1.23 | 0.83 | 0.79 | 1.11 | 0.2 | 0.96 |
| 1 | A001L | 0.96 | 0.86 | 0.81 | 0.94 | 0.6 | 0.94 |
| 1 | A001M | 1.34 | 1.02 | 1.07 | 1.08 | 0.9 | 0.99 |
| 1 | A001N | 1.29 | 1.02 | 0.81 | 0.98 | 1.0 | 1.05 |
| 1 | A001P | 0.27 | 1.16 | 1.46 | 1.14 | 0.7 | 0.66 |
| 1 | A001Q | 0.88 | 1.00 | 0.97 | 0.98 | 1.2 | 1.02 |
| 1 | A001R | 1.00 | 0.89 | 0.72 | 0.91 | 0.1 | 1.00 |
| 1 | A001S | 1.09 | 0.89 | 1.00 | 1.05 | 0.9 | 1.08 |
| 1 | A001T | 1.13 | 1.00 | 1.16 | 1.02 | 1.0 | 1.01 |
| 1 | A001V | 1.32 | 0.83 | 0.68 | 0.96 | 0.8 | 0.96 |
| 1 | A001W | 1.19 | 0.76 | 0.67 | 0.88 | 0.2 | 0.92 |
| 2 | Q002C | 0.55 | 0.81 | 1.26 | 0.92 | 0.1 | 0.74 |
| 3 | S003A | 1.19 | 1.20 | 0.97 | 0.89 | 0.3 | 0.93 |
| 3 | S003D | 1.24 | 1.11 | 1.08 | 0.97 | 1.0 | 1.02 |
| 3 | S003F | 1.20 | 0.90 | 0.95 | 0.86 | 0.2 | 1.02 |
| 3 | S003I | 1.24 | 0.80 | 0.90 | 1.05 | 1.0 | 0.92 |
| 3 | S003K | 1.28 | 0.97 | 0.80 | 0.95 | 0.1 | 1.09 |
| 3 | S003L | 1.13 | 0.90 | 0.84 | 1.06 | 0.6 | 0.92 |
| 3 | S003M | 1.35 | 0.83 | 1.04 | 0.92 | 1.1 | 0.84 |
| 3 | S003N | 1.18 | 0.98 | 1.15 | 0.85 | 0.5 | 1.01 |
| 3 | S003Q | 1.38 | 0.97 | 0.96 | 1.08 | 1.3 | 1.00 |
| 3 | S003T | 1.31 | 1.09 | 1.01 | 0.87 | 1.2 | 0.90 |
| 3 | S003V | 1.19 | 0.84 | 0.80 | 1.06 | 1.0 | 0.99 |
| 3 | S003W | 1.50 | 0.71 | 0.65 | 0.91 | 0.1 | 0.84 |
| 3 | S003Y | 1.25 | 0.76 | 0.72 | 0.92 | 0.2 | 0.88 |
| 4 | V004C | 0.87 | 1.03 | 1.22 | 0.92 | 0.3 | 1.09 |
| 4 | V004E | 1.00 | 1.04 | 1.22 | 0.92 | 0.1 | 0.99 |

(continued)

| Table 6-2. Combinable Mutations of BPN' | | | | | | | |
|---|---|---|---|---|---|---|---|
| Position | BPN' variant | TCA PI | PI BMI pH 8 16C | PI BMI pH 7 16C | PI BMI pH 8 32C | LAS-EDTA PI | AAPF PI |
| 4 | V004T | 1.02 | 0.92 | 1.27 | 0.97 | 1.2 | 1.20 |
| 5 | P005F | 0.22 | 0.55 | 0.44 | 0.61 | 0.1 | 0.23 |
| 5 | P005G | 0.98 | 1.09 | 1.10 | 0.98 | 0.3 | 1.13 |
| 5 | P005I | 0.21 | 0.49 | 0.96 | 1.02 | 0.1 | 0.20 |
| 5 | P005K | 0.16 | 4.53 | 2.60 | 0.65 | 0.6 | 0.06 |
| 6 | Y006A | 1.02 | 1.14 | 1.03 | 1.01 | 0.8 | 0.97 |
| 6 | Y006C | 1.04 | 1.03 | 0.95 | 0.83 | 0.7 | 0.91 |
| 6 | Y006E | 1.49 | 1.13 | 0.76 | 1.00 | 1.1 | 0.81 |
| 6 | Y006F | 1.17 | 0.84 | 0.88 | 0.81 | 0.6 | 0.92 |
| 6 | Y006G | 1.00 | 1.20 | 1.09 | 0.78 | 0.7 | 0.86 |
| 6 | Y006M | 1.31 | 1.16 | 1.04 | 0.96 | 0.6 | 0.86 |
| 6 | Y006N | 1.12 | 1.20 | 0.97 | 0.81 | 0.6 | 0.93 |
| 6 | Y006P | 0.92 | 1.10 | 0.77 | 0.96 | 0.7 | 1.13 |
| 6 | Y006Q | 1.36 | 1.29 | 1.19 | 0.87 | 0.9 | 0.94 |
| 6 | Y006S | 0.83 | 1.08 | 1.20 | 0.96 | 0.5 | 0.96 |
| 6 | Y006T | 1.13 | 1.02 | 1.14 | 1.12 | 0.7 | 0.85 |
| 6 | Y006V | 1.39 | 1.02 | 0.97 | 0.91 | 0.7 | 1.06 |
| 6 | Y006W | 1.96 | 1.06 | 1.00 | 0.96 | 1.1 | 0.82 |
| 7 | G007C | 0.20 | 0.75 | 0.37 | 1.11 | 0.2 | 0.08 |
| 7 | G007T | 0.19 | 2.98 | 2.43 | 3.02 | 0.1 | 0.21 |
| 8 | V008C | 0.81 | 0.87 | 0.93 | 1.10 | 0.1 | 0.83 |
| Position | BPN' variant | TCA PI | PI BMI pH 8 16C | PI BMI pH 7 16C | PI BMI pH 8 32C | LAS-EDTA PI | AAPF PI |
| 8 | V008I | 0.91 | 1.06 | 0.87 | 1.01 | 1.0 | 0.93 |
| 8 | V008K | 0.19 | 3.07 | 3.04 | 3.06 | 0.2 | 0.45 |
| 8 | V008L | 0.83 | 0.91 | 0.98 | 1.03 | 0.2 | 0.80 |
| 8 | V008R | 0.21 | 0.83 | 0.14 | 1.14 | 0.2 | 0.06 |
| 9 | S009A | 1.22 | 0.84 | 1.00 | 0.98 | 0.8 | 0.97 |
| 9 | S009C | 1.32 | 0.94 | 0.80 | 0.79 | 1.2 | 1.15 |
| 9 | S009E | 1.89 | 0.91 | 0.76 | 0.92 | 1.5 | 0.91 |
| 9 | S009G | 1.19 | 0.88 | 0.92 | 0.88 | 0.7 | 0.91 |
| 9 | S009H | 1.35 | 0.70 | 1.08 | 1.00 | 0.8 | 0.95 |
| 9 | S009L | 1.30 | 0.99 | 0.76 | 0.86 | 0.6 | 1.05 |
| 9 | S009M | 1.32 | 0.98 | 0.95 | 0.99 | 0.8 | 1.17 |
| 9 | S009P | 1.38 | 1.00 | 0.87 | 0.94 | 0.7 | 1.06 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 9 | S009Q | 1.02 | 0.92 | 0.77 | 0.83 | 0.8 | 1.15 |
| 9 | S009T | 1.16 | 1.07 | 1.11 | 0.80 | 1.3 | 0.94 |
| 9 | S009V | 1.51 | 0.99 | 0.75 | 0.86 | 0.9 | 1.03 |
| 10 | Q010A | 1.06 | 0.90 | 1.06 | 1.00 | 0.5 | 0.87 |
| 10 | Q010C | 1.25 | 0.92 | 0.94 | 0.93 | 0.7 | 0.86 |
| 10 | Q010E | 1.07 | 0.70 | 1.01 | 0.99 | 1.5 | 0.79 |
| 10 | Q010G | 1.34 | 0.95 | 0.93 | 0.90 | 0.3 | 1.29 |
| 10 | Q010H | 1.20 | 1.04 | 1.07 | 0.92 | 0.9 | 1.29 |
| 10 | Q010K | 1.16 | 0.83 | 0.49 | 0.89 | 0.1 | 0.96 |
| 10 | Q010M | 1.26 | 0.82 | 0.97 | 1.07 | 0.5 | 0.90 |
| 10 | Q010S | 1.00 | 0.88 | 0.90 | 0.87 | 0.5 | 1.10 |
| 10 | Q010T | 1.25 | 1.04 | 1.00 | 0.85 | 0.8 | 0.93 |
| 10 | Q010V | 1.24 | 0.96 | 0.80 | 1.00 | 0.2 | 1.18 |
| 10 | Q010Y | 0.44 | 0.97 | 0.79 | 0.99 | 0.4 | 0.82 |
| 11 | I011A | 0.28 | 0.90 | 1.10 | 1.10 | 0.3 | 0.65 |
| 11 | IO11C | 1.02 | 1.07 | 0.88 | 0.90 | 0.7 | 1.00 |
| 11 | I011G | 0.18 | 2.21 | 2.17 | 1.57 | 0.4 | 0.27 |
| 11 | IO11H | 0.16 | 36.45 | 114.23 | 1.88 | 0.8 | 0.35 |
| 11 | I011L | 0.76 | 1.07 | 1.13 | 0.79 | 0.6 | 1.15 |
| 11 | I011S | 0.26 | 1.34 | 1.73 | 1.26 | 0.3 | 0.97 |
| 11 | IO11T | 1.05 | 1.02 | 0.87 | 0.93 | 0.7 | 1.00 |
| 11 | I011V | 1.28 | 1.17 | 0.91 | 0.86 | 1.0 | 1.10 |
| 12 | K012A | 1.48 | 0.85 | 0.82 | 1.02 | 1.2 | 0.84 |
| 12 | K012C | 1.24 | 0.73 | 0.94 | 0.93 | 1.4 | 0.94 |
| 12 | K012D | 1.26 | 0.49 | 1.06 | 0.89 | 1.4 | 0.96 |
| 12 | K012E | 1.35 | 0.62 | 0.71 | 0.85 | 1.4 | 0.90 |
| 12 | K012F | 1.04 | 0.60 | 0.80 | 0.68 | 1.3 | 0.94 |
| 12 | K012G | 1.50 | 0.94 | 0.88 | 0.90 | 1.4 | 0.88 |
| 12 | K012H | 1.24 | 0.94 | 1.03 | 0.92 | 1.3 | 1.01 |
| 12 | K012I | 1.24 | 0.68 | 0.65 | 0.96 | 1.2 | 0.92 |
| 12 | K012L | 0.17 | 3.09 | 4.59 | 1.99 | 0.6 | 0.28 |
| 12 | K012N | 1.13 | 0.86 | 0.93 | 0.85 | 1.3 | 1.01 |
| 12 | K012Q | 1.35 | 0.96 | 0.61 | 1.01 | 1.3 | 1.10 |
| 12 | K012R | 0.73 | 0.83 | 0.84 | 0.83 | 0.9 | 0.75 |
| 12 | K012S | 1.07 | 0.71 | 0.74 | 0.72 | 1.0 | 0.93 |
| 12 | K012T | 1.13 | 1.07 | 0.95 | 0.84 | 1.1 | 0.85 |
| 12 | K012V | 1.57 | 0.80 | 0.83 | 0.98 | 1.1 | 0.76 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 12 | K012W | 1.35 | 0.54 | 0.85 | 0.94 | 1.2 | 0.90 |
| 12 | K012Y | 1.38 | 0.52 | 0.90 | 0.67 | 1.3 | 0.95 |
| 13 | A013C | 1.05 | 0.99 | 0.77 | 0.83 | 0.6 | 1.12 |
| 13 | A013G | 1.56 | 1.02 | 1.05 | 0.84 | 0.7 | 0.92 |
| 13 | A013L | 0.26 | 1.12 | 1.04 | 1.19 | 1.1 | 0.80 |
| 13 | A013S | 0.91 | 1.05 | 1.10 | 1.05 | 1.1 | 1.23 |
| 13 | A013T | 0.51 | 1.12 | 0.91 | 0.96 | 0.6 | 0.82 |
| 13 | A013V | 0.82 | 1.01 | 0.74 | 0.97 | 0.1 | 0.76 |
| 14 | P014A | 1.21 | 0.97 | 1.13 | 1.04 | 0.1 | 1.07 |
| 14 | P014C | 1.05 | 0.78 | 1.22 | 0.84 | 0.7 | 0.99 |
| 14 | P014D | 1.07 | 0.70 | 1.21 | 0.92 | 0.4 | 1.02 |
| 14 | P014E | 1.27 | 0.55 | 1.14 | 0.95 | 0.9 | 1.14 |
| 14 | P014G | 1.35 | 0.92 | 1.20 | 0.94 | 0.2 | 1.18 |
| 14 | P014I | 0.89 | 0.85 | 1.19 | 0.94 | 0.7 | 1.19 |
| 14 | P014L | 0.43 | 0.74 | 1.58 | 1.02 | 0.6 | 1.16 |
| 14 | P014M | 1.50 | 0.77 | 1.17 | 0.93 | 0.3 | 1.08 |
| 14 | P014N | 1.10 | 0.80 | 0.98 | 0.91 | 0.1 | 1.00 |
| 14 | P014Q | 1.34 | 0.62 | 0.94 | 0.81 | 0.6 | 1.12 |
| 14 | P014S | 0.96 | 0.79 | 1.06 | 0.96 | 0.1 | 1.13 |
| 14 | P014T | 0.98 | 0.99 | 1.08 | 0.83 | 0.9 | 1.35 |
| 14 | P014V | 1.32 | 0.88 | 1.10 | 0.90 | 0.7 | 1.12 |
| 15 | A015C | 1.72 | 0.83 | 0.87 | 0.90 | 1.3 | 0.83 |
| 15 | A015D | 1.60 | 0.66 | 0.77 | 0.75 | 1.3 | 1.00 |
| 15 | A015E | 1.68 | 0.62 | 0.77 | 1.11 | 1.5 | 0.87 |
| 15 | A015F | 1.39 | 0.78 | 0.68 | 0.85 | 0.9 | 0.99 |
| 15 | A015G | 1.63 | 0.77 | 0.66 | 0.76 | 0.9 | 0.79 |
| 15 | A015H | 0.90 | 0.64 | 0.77 | 0.93 | 1.0 | 1.01 |
| 15 | A015I | 1.52 | 0.46 | 0.88 | 0.86 | 1.1 | 0.95 |
| 15 | A015K | 1.50 | 0.74 | 0.59 | 0.69 | 0.3 | 0.98 |
| 15 | A015L | 1.52 | 0.89 | 0.77 | 0.88 | 1.1 | 0.85 |
| 15 | A015M | 0.96 | 0.95 | 0.62 | 1.01 | 1.0 | 0.99 |
| 15 | A015P | 1.41 | 0.57 | 0.64 | 0.73 | 0.9 | 0.95 |
| 15 | A015Q | 1.39 | 0.55 | 0.73 | 1.05 | 1.1 | 1.06 |
| 15 | A015R | 1.44 | 0.67 | 0.53 | 0.84 | 0.1 | 0.95 |
| 15 | A015S | 1.17 | 0.74 | 0.80 | 1.07 | 0.9 | 1.08 |
| 15 | A015T | 1.53 | 0.85 | 0.68 | 0.93 | 1.0 | 1.00 |
| 15 | A015V | 1.50 | 0.77 | 0.73 | 0.89 | 1.0 | 0.93 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 15 | A015W | 0.45 | 0.59 | 0.88 | 1.18 | 0.8 | 0.93 |
| 15 | A015Y | 1.43 | 0.63 | 0.70 | 0.90 | 1.0 | 0.94 |
| 16 | L016A | 1.14 | 0.79 | 1.22 | 0.96 | 0.8 | 0.84 |
| 16 | L016C | 1.01 | 0.71 | 1.15 | 1.08 | 1.0 | 1.13 |
| 16 | L016E | 1.02 | 0.57 | 1.18 | 1.09 | 0.9 | 1.03 |
| 16 | L016F | 0.45 | 0.63 | 0.93 | 0.89 | 0.5 | 0.58 |
| 16 | L016G | 0.29 | 0.54 | 1.19 | 0.97 | 0.8 | 0.47 |
| 16 | L016H | 0.29 | 0.70 | 1.11 | 1.12 | 0.5 | 0.53 |
| 16 | L016I | 1.22 | 0.51 | 1.21 | 1.05 | 1.0 | 0.89 |
| 16 | L016K | 0.36 | 0.62 | 1.09 | 0.99 | 0.5 | 0.73 |
| 16 | L016M | 1.63 | 0.76 | 0.97 | 1.13 | 1.0 | 0.89 |
| 16 | L016N | 0.59 | 0.71 | 1.10 | 1.05 | 0.8 | 0.96 |
| 16 | L016P | 0.50 | 0.73 | 1.05 | 1.07 | 0.8 | 0.90 |
| 16 | L016Q | 0.90 | 0.88 | 1.02 | 1.04 | 0.9 | 0.88 |
| 16 | L016S | 0.92 | 0.81 | 1.09 | 0.96 | 0.7 | 0.82 |
| 16 | L016T | 1.30 | 0.63 | 1.02 | 1.07 | 0.9 | 0.93 |
| 16 | L016W | 0.17 | 3.37 | 6.22 | 3.16 | 0.1 | 0.35 |
| 17 | H017A | 0.37 | 0.41 | 0.95 | 0.94 | 0.4 | 0.40 |
| 17 | H017C | 0.33 | 0.80 | 0.73 | 1.01 | 0.7 | 0.39 |
| 17 | H017E | 0.30 | 0.81 | 1.09 | 1.00 | 0.4 | 0.63 |
| 17 | H017F | 1.40 | 0.72 | 1.01 | 0.85 | 0.3 | 0.86 |
| 17 | H017G | 0.19 | 1.02 | 0.45 | 1.57 | 0.6 | 0.25 |
| 17 | H017I | 1.36 | 0.57 | 0.99 | 1.10 | 0.8 | 0.94 |
| 17 | H017L | 1.15 | 0.90 | 1.06 | 0.93 | 0.5 | 0.82 |
| 17 | H017M | 1.29 | 0.60 | 1.24 | 0.94 | 0.7 | 0.84 |
| 17 | H017S | 0.20 | 1.15 | 1.18 | 1.54 | 0.4 | 0.34 |
| 17 | H017T | 0.47 | 0.97 | 1.25 | 1.13 | 0.2 | 0.89 |
| 17 | H017V | 0.80 | 0.67 | 0.96 | 1.02 | 0.4 | 0.67 |
| 18 | S018A | 0.89 | 0.57 | 1.16 | 0.91 | 0.9 | 0.95 |
| 18 | S018D | 1.72 | 0.78 | 1.03 | 0.86 | 0.8 | 0.86 |
| 18 | S018E | 1.65 | 0.75 | 1.15 | 0.99 | 1.3 | 0.85 |
| 18 | S018F | 1.74 | 0.80 | 1.14 | 0.95 | 0.4 | 0.87 |
| 18 | S018G | 1.37 | 0.80 | 0.96 | 1.04 | 1.0 | 0.85 |
| 18 | S018H | 1.72 | 0.83 | 1.07 | 1.05 | 1.0 | 0.93 |
| 18 | S018I | 1.89 | 0.66 | 1.33 | 0.90 | 0.6 | 0.88 |
| 18 | S018K | 1.75 | 0.63 | 1.14 | 0.94 | 0.1 | 0.99 |
| 18 | S018L | 1.48 | 0.73 | 1.25 | 0.96 | 0.7 | 0.96 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 18 | S018M | 1.72 | 0.93 | 1.28 | 1.04 | 0.9 | 0.91 |
| 18 | S018N | 1.68 | 0.59 | 1.12 | 0.94 | 1.1 | 0.91 |
| 18 | S018P | 1.19 | 0.65 | 1.06 | 0.83 | 1.3 | 0.75 |
| 18 | S018Q | 1.68 | 0.80 | 1.12 | 0.99 | 1.0 | 0.92 |
| 18 | S018T | 1.64 | 0.67 | 1.25 | 0.99 | 1.1 | 0.89 |
| 18 | S018V | 1.67 | 0.57 | 1.17 | 1.14 | 0.8 | 0.89 |
| 18 | S018W | 1.59 | 0.73 | 1.06 | 1.00 | 0.4 | 0.95 |
| 18 | S018Y | 1.86 | 0.81 | 1.25 | 1.08 | 0.8 | 1.13 |
| 19 | Q019A | 1.82 | 1.07 | 0.99 | 1.05 | 0.8 | 0.92 |
| 19 | Q019C | 0.88 | 1.08 | 0.90 | 0.81 | 1.0 | 0.91 |
| 19 | Q019D | 0.87 | 0.98 | 0.95 | 0.67 | 1.1 | 0.98 |
| 19 | Q019E | 1.02 | 0.97 | 0.61 | 0.90 | 1.2 | 0.95 |
| 19 | Q019G | 1.33 | 0.99 | 1.15 | 0.87 | 0.8 | 1.13 |
| 19 | Q019H | 0.82 | 1.05 | 0.98 | 0.72 | 0.9 | 1.08 |
| 19 | Q019I | 0.89 | 1.06 | 0.87 | 1.03 | 0.9 | 1.22 |
| 19 | Q019K | 0.86 | 0.87 | 0.78 | 0.77 | 0.6 | 1.15 |
| 19 | Q019L | 0.98 | 0.95 | 0.61 | 0.87 | 1.0 | 1.01 |
| 19 | Q019M | 0.90 | 1.02 | 1.01 | 0.90 | 0.7 | 0.96 |
| 19 | Q019R | 1.09 | 1.03 | 0.64 | 0.74 | 0.4 | 0.99 |
| 19 | Q019S | 0.82 | 1.03 | 1.14 | 0.92 | 0.8 | 1.07 |
| 19 | Q019T | 0.93 | 0.94 | 1.00 | 0.84 | 0.8 | 1.15 |
| 19 | Q019V | 0.88 | 1.12 | 1.04 | 0.97 | 0.7 | 1.22 |
| 20 | G020A | 1.12 | 0.90 | 0.99 | 0.93 | 0.8 | 0.80 |
| 20 | G020C | 0.96 | 0.81 | 1.12 | 0.99 | 1.0 | 0.95 |
| 20 | G020D | 1.00 | 0.82 | 1.19 | 1.02 | 1.1 | 0.99 |
| 20 | G020E | 1.27 | 0.91 | 1.04 | 1.01 | 1.0 | 1.01 |
| 20 | G020F | 0.88 | 0.64 | 1.15 | 1.06 | 1.0 | 0.84 |
| 20 | G020H | 1.00 | 0.66 | 1.20 | 1.08 | 1.0 | 0.92 |
| 20 | G020I | 0.22 | 0.44 | 1.36 | 1.12 | 0.9 | 0.34 |
| 20 | G020K | 0.90 | 0.70 | 0.95 | 0.93 | 0.4 | 1.06 |
| 20 | G020L | 0.75 | 0.74 | 1.14 | 0.89 | 1.0 | 0.92 |
| 20 | G020M | 0.98 | 0.83 | 1.08 | 0.97 | 0.9 | 0.89 |
| 20 | G020N | 0.96 | 0.91 | 1.18 | 0.90 | 1.0 | 1.03 |
| 20 | G020P | 0.26 | 0.63 | 1.11 | 0.98 | 0.9 | 0.49 |
| 20 | G020Q | 0.95 | 0.53 | 1.13 | 1.01 | 0.9 | 1.06 |
| 20 | G020R | 0.86 | 0.70 | 0.99 | 0.92 | 0.2 | 0.99 |
| 20 | G020S | 1.00 | 0.63 | 1.07 | 1.05 | 0.7 | 0.88 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 20 | G020T | 0.54 | 0.82 | 1.24 | 1.08 | 0.9 | 1.02 |
| 20 | G020V | 0.22 | 0.97 | 1.37 | 1.53 | 0.8 | 0.40 |
| 20 | G020W | 0.63 | 0.88 | 1.15 | 1.05 | 0.8 | 0.93 |
| 20 | G020Y | 0.70 | 0.57 | 1.03 | 1.04 | 0.9 | 0.92 |
| 21 | Y021A | 1.15 | 0.85 | 0.77 | 0.94 | 0.9 | 0.81 |
| 21 | Y021C | 1.15 | 0.79 | 1.02 | 0.86 | 0.9 | 1.05 |
| 21 | Y021D | 0.84 | 0.83 | 0.76 | 1.02 | 0.9 | 0.73 |
| 21 | Y021E | 0.71 | 0.82 | 0.77 | 0.89 | 1.0 | 0.91 |
| 21 | Y021F | 1.13 | 0.59 | 0.77 | 0.97 | 1.0 | 0.85 |
| 21 | Y021G | 0.46 | 0.60 | 0.76 | 0.89 | 1.0 | 0.52 |
| 21 | Y021H | 1.22 | 0.78 | 0.69 | 0.98 | 1.1 | 0.93 |
| 21 | Y021K | 0.72 | 0.61 | 0.47 | 1.00 | 0.8 | 0.91 |
| 21 | Y021L | 0.92 | 0.78 | 0.75 | 0.99 | 1.0 | 0.95 |
| 21 | Y021M | 1.17 | 0.88 | 0.87 | 0.95 | 1.0 | 0.78 |
| 21 | Y021P | 0.47 | 0.50 | 0.88 | 1.08 | 1.2 | 0.69 |
| 21 | Y021Q | 0.74 | 0.88 | 0.87 | 1.02 | 0.9 | 0.96 |
| 21 | Y021R | 0.95 | 0.70 | 0.77 | 0.94 | 0.7 | 0.91 |
| 21 | Y021S | 0.98 | 0.71 | 0.84 | 0.93 | 0.9 | 0.80 |
| 21 | Y021T | 1.33 | 0.81 | 0.76 | 0.93 | 1.0 | 0.90 |
| 21 | Y021V | 1.15 | 0.70 | 0.99 | 1.00 | 1.1 | 0.87 |
| 21 | Y021W | 0.83 | 0.68 | 0.70 | 1.04 | 1.0 | 1.14 |
| 22 | T022A | 0.67 | 0.85 | 0.92 | 0.86 | 1.2 | 0.80 |
| 22 | T022C | 1.21 | 1.12 | 0.74 | 1.08 | 1.3 | 1.13 |
| 22 | T022D | 0.76 | 0.98 | 0.74 | 1.03 | 1.3 | 0.97 |
| 22 | T022E | 1.33 | 1.01 | 0.89 | 0.90 | 1.6 | 1.04 |
| 22 | T022F | 1.37 | 1.09 | 0.73 | 1.06 | 1.2 | 0.90 |
| 22 | T022G | 1.60 | 1.00 | 0.92 | 1.02 | 1.2 | 0.87 |
| 22 | T022H | 1.17 | 1.09 | 0.88 | 1.14 | 1.1 | 0.99 |
| 22 | T022I | 1.26 | 1.17 | 0.91 | 1.28 | 1.0 | 1.08 |
| 22 | T022K | 1.19 | 0.78 | 0.73 | 1.02 | 0.4 | 1.25 |
| 22 | T022L | 0.92 | 0.90 | 0.73 | 1.07 | 0.9 | 0.92 |
| 22 | T022M | 1.34 | 0.92 | 0.85 | 1.07 | 1.0 | 1.13 |
| 22 | T022N | 1.13 | 0.90 | 0.95 | 1.03 | 1.2 | 1.07 |
| 22 | T022P | 0.58 | 0.78 | 0.62 | 0.89 | 1.3 | 0.52 |
| 22 | T022Q | 1.42 | 1.15 | 0.75 | 1.14 | 1.1 | 0.97 |
| 22 | T022S | 1.10 | 0.95 | 0.91 | 0.98 | 1.3 | 0.93 |
| 22 | T022V | 1.32 | 1.18 | 0.75 | 1.10 | 1.3 | 1.11 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 22 | T022W | 1.52 | 0.93 | 0.79 | 1.07 | 1.7 | 0.87 |
| 22 | T022Y | 1.15 | 0.96 | 0.95 | 1.03 | 1.4 | 0.95 |
| 23 | G023A | 0.63 | 1.05 | 0.84 | 1.02 | 1.0 | 0.79 |
| 23 | G023S | 0.17 | 10.83 | 4.09 | 2.61 | 1.1 | 0.13 |
| 24 | S024C | 1.12 | 1.15 | 0.86 | 0.94 | 1.1 | 0.97 |
| 24 | S024F | 1.07 | 1.15 | 0.93 | 1.05 | 1.0 | 1.22 |
| 24 | S024G | 0.56 | 0.92 | 0.68 | 1.01 | 1.2 | 0.68 |
| 24 | S024H | 0.97 | 1.11 | 0.98 | 1.18 | 1.1 | 1.28 |
| 24 | S024I | 1.02 | 1.16 | 1.02 | 1.15 | 1.1 | 0.98 |
| 24 | S024K | 1.17 | 1.03 | 0.82 | 1.04 | 0.9 | 0.97 |
| 24 | S024L | 1.17 | 1.02 | 1.01 | 1.02 | 1.2 | 1.16 |
| 24 | S024M | 1.15 | 1.07 | 0.98 | 1.02 | 1.2 | 0.88 |
| 24 | S024N | 1.16 | 0.99 | 0.88 | 1.11 | 1.2 | 1.01 |
| 24 | S024P | 0.44 | 1.10 | 0.76 | 0.96 | 1.1 | 0.72 |
| 24 | S024Q | 0.90 | 0.96 | 0.94 | 1.11 | 1.0 | 1.10 |
| 24 | S024R | 0.86 | 1.19 | 0.82 | 1.12 | 1.0 | 1.19 |
| 24 | S024T | 0.57 | 1.07 | 0.92 | 1.06 | 1.1 | 0.96 |
| 24 | S024V | 0.45 | 1.21 | 0.93 | 1.19 | 1.1 | 1.22 |
| 24 | S024W | 1.13 | 1.09 | 0.62 | 1.09 | 1.2 | 0.94 |
| 24 | S024Y | 1.06 | 0.95 | 0.83 | 0.96 | 1.1 | 1.19 |
| 25 | N025A | 0.73 | 0.63 | 1.09 | 1.00 | 0.9 | 0.93 |
| 25 | N025E | 1.31 | 0.61 | 1.16 | 1.03 | 1.2 | 1.13 |
| 25 | N025F | 1.09 | 0.69 | 1.14 | 1.12 | 1.1 | 0.97 |
| 25 | N025G | 1.21 | 0.65 | 1.20 | 0.99 | 1.0 | 1.05 |
| 25 | N025H | 1.16 | 0.57 | 1.06 | 1.07 | 1.1 | 0.98 |
| 25 | N0251 | 0.95 | 0.72 | 1.29 | 0.83 | 1.0 | 1.00 |
| 25 | N025K | 0.96 | 0.64 | 1.04 | 0.96 | 1.0 | 0.99 |
| 25 | N025L | 0.64 | 0.62 | 1.02 | 1.09 | 1.1 | 0.98 |
| 25 | N025M | 1.31 | 0.82 | 1.18 | 1.04 | 1.0 | 1.01 |
| 25 | N025P | 0.71 | 0.53 | 1.10 | 0.81 | 0.8 | 0.82 |
| 25 | N025Q | 1.10 | 0.61 | 1.21 | 1.09 | 1.0 | 1.08 |
| 25 | N025R | 1.34 | 0.68 | 1.02 | 1.12 | 1.0 | 1.03 |
| 25 | N025S | 0.85 | 0.60 | 1.18 | 1.01 | 1.0 | 0.93 |
| 25 | N025T | 1.06 | 0.73 | 1.21 | 1.12 | 0.8 | 1.22 |
| 25 | N025V | 0.59 | 0.45 | 1.27 | 1.11 | 0.9 | 1.01 |
| 25 | N025W | 1.42 | 0.53 | 1.30 | 1.00 | 0.9 | 0.99 |
| 26 | V026C | 1.14 | 0.96 | 0.91 | 1.02 | 1.0 | 0.88 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 26 | V026E | 0.36 | 1.12 | 0.79 | 1.19 | 1.0 | 0.87 |
| 26 | V026F | 0.35 | 0.97 | 0.90 | 1.19 | 1.1 | 0.68 |
| 26 | V026G | 0.54 | 0.91 | 0.75 | 1.07 | 1.0 | 0.82 |
| 26 | V026H | 0.46 | 1.45 | 0.86 | 1.19 | 1.1 | 0.89 |
| 26 | V026I | 0.91 | 0.75 | 0.70 | 1.09 | 1.2 | 0.90 |
| 26 | V026K | 0.77 | 1.03 | 0.67 | 1.11 | 1.1 | 0.95 |
| 26 | V026L | 0.59 | 0.92 | 0.85 | 0.96 | 1.0 | 1.03 |
| 26 | V026M | 0.80 | 0.90 | 0.85 | 0.98 | 1.0 | 0.90 |
| 26 | V026N | 0.44 | 1.00 | 0.79 | 1.21 | 1.0 | 0.96 |
| 26 | V026Q | 0.61 | 0.96 | 0.78 | 1.02 | 1.0 | 1.08 |
| 26 | V026S | 0.82 | 0.82 | 0.85 | 0.95 | 0.9 | 0.86 |
| 26 | V026T | 0.92 | 0.98 | 0.98 | 1.02 | 0.9 | 1.04 |
| 26 | V026W | 0.25 | 2.33 | 1.16 | 2.13 | 1.1 | 0.95 |
| 26 | V026Y | 0.31 | 1.34 | 1.28 | 1.50 | 1.0 | 0.98 |
| 27 | K027A | 1.08 | 0.76 | 1.13 | 1.05 | 1.0 | 1.07 |
| 27 | K027D | 1.15 | 0.72 | 1.10 | 1.00 | 1.1 | 0.93 |
| 27 | K027E | 0.45 | 0.93 | 1.11 | 1.09 | 1.0 | 1.06 |
| 27 | K027F | 0.25 | 0.76 | 1.59 | 1.33 | 1.1 | 0.58 |
| 27 | K027G | 0.76 | 0.65 | 1.27 | 0.95 | 1.0 | 1.15 |
| 27 | K027H | 1.03 | 0.72 | 1.19 | 0.99 | 1.1 | 1.10 |
| 27 | K027I | 0.18 | 0.49 | 1.78 | 0.91 | 1.4 | 0.20 |
| 27 | K027L | 0.76 | 0.72 | 1.29 | 0.87 | 1.1 | 0.89 |
| 27 | K027M | 0.64 | 0.71 | 1.31 | 0.91 | 1.0 | 1.06 |
| 27 | K027P | 0.18 | 1.31 | 3.37 | 2.05 | 1.1 | 0.46 |
| 27 | K027R | 1.09 | 0.64 | 1.16 | 0.96 | 1.0 | 1.19 |
| 27 | K027S | 0.93 | 0.92 | 1.11 | 1.04 | 1.0 | 1.11 |
| 27 | K027T | 0.56 | 0.85 | 1.39 | 1.09 | 1.0 | 1.09 |
| 27 | K027W | 0.36 | 0.69 | 1.47 | 1.33 | 0.9 | 0.93 |
| 27 | K027Y | 0.45 | 1.00 | 1.19 | 1.03 | 0.9 | 0.96 |
| 28 | V028A | 0.46 | 0.95 | 0.84 | 1.02 | 0.9 | 0.72 |
| 28 | V028C | 0.65 | 1.20 | 0.99 | 1.08 | 1.0 | 0.94 |
| 28 | V028H | 0.20 | 1.08 | 0.79 | 1.03 | 1.1 | 0.22 |
| 28 | V028I | 1.02 | 1.27 | 1.00 | 1.06 | 1.1 | 0.87 |
| 28 | V028M | 1.05 | 0.99 | 0.68 | 0.91 | 0.6 | 0.70 |
| 28 | V028N | 0.21 | 0.31 | 0.67 | 0.50 | 1.1 | 0.13 |
| 28 | V028Q | 0.18 | 2.79 | 1.99 | 1.43 | 1.6 | 0.25 |
| 28 | V028S | 0.18 | 5.70 | 5.17 | 3.40 | 1.1 | 0.37 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 28 | V028T | 0.58 | 1.31 | 1.18 | 1.13 | 1.0 | 1.21 |
| 29 | A029C | 0.76 | 1.32 | 1.19 | 1.13 | 0.9 | 0.77 |
| 29 | A029D | 0.19 | 2.95 | 2.74 | 2.34 | 0.9 | 0.42 |
| 29 | A029G | 0.94 | 1.08 | 0.88 | 1.06 | 1.0 | 1.11 |
| 29 | A029S | 0.47 | 1.64 | 1.13 | 1.23 | 1.1 | 1.01 |
| 29 | A029T | 0.24 | 2.02 | 1.55 | 1.57 | 1.1 | 0.47 |
| 29 | A029V | 0.38 | 1.85 | 1.52 | 1.62 | 1.0 | 0.83 |
| 30 | V030A | 0.38 | 0.98 | 1.04 | 0.87 | 0.9 | 0.41 |
| 30 | V030C | 0.91 | 0.83 | 0.85 | 0.99 | 1.0 | 0.37 |
| 30 | V030D | 0.20 | 2.34 | 2.31 | 1.96 | 0.9 | 0.22 |
| 30 | V030E | 0.17 | 30.23 | 33.27 | 4.25 | 0.8 | 0.15 |
| 30 | V030I | 1.15 | 1.16 | 1.03 | 1.07 | 1.1 | 0.82 |
| 30 | V030L | 1.24 | 1.09 | 0.86 | 0.87 | 1.0 | 0.24 |
| 30 | V030M | 0.89 | 0.66 | 0.80 | 0.79 | 0.8 | 0.11 |
| 30 | V030N | 0.25 | 1.06 | 1.30 | 1.50 | 1.1 | 0.15 |
| 30 | V030S | 0.28 | 1.25 | 1.21 | 1.43 | 0.3 | 0.12 |
| 30 | V030T | 0.30 | 1.39 | 1.27 | 1.38 | 0.9 | 0.37 |
| 31 | I031A | 0.90 | 0.96 | 0.85 | 0.97 | 1.1 | 1.64 |
| 31 | I031C | 0.94 | 1.16 | 0.87 | 1.02 | 1.1 | 1.53 |
| 31 | I031D | 0.20 | 1.18 | 1.43 | 1.13 | 1.2 | 0.28 |
| 31 | I031E | 0.21 | 2.56 | 1.98 | 2.40 | 1.1 | 1.12 |
| 31 | I031F | 0.92 | 1.26 | 0.92 | 1.00 | 1.1 | 1.89 |
| 31 | I031G | 0.17 | 5.42 | 3.74 | 2.40 | 1.1 | 0.40 |
| 31 | I031H | 0.26 | 1.98 | 1.57 | 1.79 | 1.3 | 1.22 |
| 31 | I031K | 0.32 | 1.84 | 1.28 | 1.39 | 1.2 | 1.03 |
| 31 | I031L | 1.12 | 0.89 | 0.88 | 0.85 | 1.2 | 1.70 |
| 31 | I031M | 1.21 | 0.98 | 0.83 | 0.99 | 1.1 | 1.60 |
| 31 | I031N | 0.23 | 2.34 | 2.01 | 1.96 | 1.3 | 0.74 |
| 31 | I031P | 0.24 | 0.16 | 0.06 | 0.17 | 1.5 | 0.09 |
| 31 | I031Q | 0.19 | 5.38 | 3.51 | 3.08 | 1.1 | 0.64 |
| 31 | I031S | 0.21 | 3.71 | 2.83 | 2.91 | 1.1 | 0.86 |
| 31 | I031T | 0.38 | 1.21 | 1.04 | 0.91 | 1.1 | 1.17 |
| 31 | I031V | 0.83 | 1.03 | 1.09 | 1.03 | 0.9 | 1.20 |
| 31 | I031Y | 0.21 | 4.14 | 3.02 | 3.08 | 0.9 | 0.90 |
| 33 | S033A | 0.82 | 0.84 | 0.84 | 0.83 | 0.7 | 0.29 |
| 33 | S033C | 0.73 | 0.66 | 0.55 | 0.71 | 1.0 | 0.19 |
| 33 | S033E | 0.44 | 1.21 | 0.81 | 0.92 | 0.8 | 0.07 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 33 | S033G | 0.96 | 1.14 | 0.80 | 1.01 | 0.9 | 0.17 |
| 33 | S033N | 0.45 | 1.14 | 0.90 | 1.03 | 0.6 | 0.38 |
| 33 | S033Q | 0.36 | 0.69 | 0.72 | 0.72 | 0.5 | 0.13 |
| 33 | S033T | 0.82 | 0.74 | 0.64 | 0.85 | 1.0 | 1.40 |
| 33 | S033V | 0.50 | 0.56 | 0.57 | 0.54 | 0.9 | 0.09 |
| 34 | G034A | 0.27 | 0.81 | 0.91 | 0.81 | 0.5 | 0.09 |
| 35 | I035A | 0.59 | 0.82 | 1.14 | 0.92 | 0.9 | 0.69 |
| 35 | 1035C | 0.95 | 0.65 | 1.41 | 1.06 | 1.0 | 0.95 |
| 35 | 1035G | 0.18 | 0.21 | 1.85 | 1.07 | 1.0 | 0.16 |
| 35 | 1035L | 0.61 | 0.68 | 1.14 | 1.03 | 0.7 | 0.74 |
| 35 | 1035M | 0.33 | 0.71 | 1.27 | 1.07 | 0.8 | 0.59 |
| 35 | I035S | 0.24 | 1.04 | 2.03 | 1.68 | 0.7 | 0.96 |
| 35 | 1035T | 0.42 | 0.89 | 1.31 | 1.15 | 0.8 | 1.40 |
| 35 | 1035V | 0.83 | 0.85 | 1.17 | 1.03 | 0.9 | 1.13 |
| 36 | D036A | 1.00 | 0.58 | 0.60 | 0.94 | 0.4 | 0.78 |
| 36 | D036C | 0.98 | 0.91 | 0.75 | 0.87 | 0.3 | 0.71 |
| 36 | D036E | 1.30 | 1.13 | 0.83 | 0.94 | 0.6 | 0.91 |
| 36 | D036H | 0.56 | 0.49 | 0.52 | 1.00 | 0.6 | 0.95 |
| 36 | D036Q | 0.67 | 0.62 | 0.54 | 0.99 | 0.9 | 0.98 |
| 36 | D036S | 1.03 | 0.72 | 0.70 | 0.87 | 0.1 | 0.93 |
| 36 | D036T | 0.72 | 0.64 | 0.45 | 0.87 | 0.1 | 0.85 |
| 37 | S037A | 1.25 | 1.14 | 1.07 | 0.73 | 0.9 | 1.03 |
| 37 | S037C | 1.09 | 0.90 | 0.87 | 0.93 | 1.0 | 0.94 |
| 37 | S037E | 0.72 | 0.83 | 1.06 | 0.84 | 1.0 | 0.92 |
| 37 | S037F | 0.99 | 1.05 | 0.88 | 0.91 | 1.0 | 0.83 |
| 37 | S037G | 1.21 | 1.19 | 0.95 | 0.95 | 1.0 | 1.25 |
| 37 | S037H | 1.04 | 1.06 | 0.98 | 0.90 | 0.9 | 1.04 |
| 37 | S037K | 1.31 | 0.98 | 0.63 | 0.84 | 1.1 | 1.02 |
| 37 | S037L | 0.98 | 1.04 | 0.91 | 0.78 | 0.8 | 0.87 |
| 37 | S037M | 1.31 | 1.00 | 1.07 | 0.82 | 0.9 | 0.87 |
| 37 | S037P | 0.48 | 1.48 | 0.90 | 1.13 | 0.4 | 0.70 |
| 37 | S037Q | 1.31 | 1.09 | 1.24 | 0.87 | 0.9 | 1.05 |
| 37 | S037R | 1.26 | 0.98 | 0.63 | 0.85 | 1.2 | 1.05 |
| 37 | S037T | 0.94 | 1.05 | 1.00 | 0.87 | 0.7 | 0.95 |
| 37 | S037V | 0.98 | 1.09 | 0.86 | 0.76 | 0.7 | 1.39 |
| 37 | S037W | 1.07 | 1.01 | 1.01 | 0.88 | 0.8 | 0.86 |
| 38 | S038A | 1.17 | 1.14 | 1.06 | 1.03 | 0.9 | 0.90 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 38 | S038C | 1.27 | 0.96 | 1.02 | 1.03 | 1.2 | 0.82 |
| 38 | S038D | 0.67 | 1.26 | 1.19 | 1.07 | 1.2 | 0.97 |
| 38 | S038E | 1.31 | 1.02 | 1.11 | 0.97 | 1.3 | 0.84 |
| 38 | S038F | 0.97 | 0.99 | 0.97 | 1.07 | 1.0 | 1.22 |
| 38 | S038G | 1.43 | 1.01 | 1.04 | 1.13 | 0.9 | 0.97 |
| 38 | S038I | 1.07 | 0.98 | 1.08 | 1.01 | 1.1 | 0.89 |
| 38 | S038K | 1.25 | 0.75 | 0.80 | 1.02 | 1.3 | 0.93 |
| 38 | S038L | 1.06 | 0.90 | 1.11 | 1.12 | 1.1 | 1.02 |
| 38 | S038M | 1.33 | 1.10 | 1.07 | 1.00 | 1.0 | 0.91 |
| 38 | S038P | 1.16 | 1.12 | 0.93 | 0.99 | 0.5 | 0.76 |
| 38 | S038Q | 1.11 | 1.06 | 0.87 | 1.08 | 1.1 | 0.96 |
| 38 | S038R | 2.01 | 0.65 | 0.89 | 1.01 | 1.4 | 0.89 |
| 38 | S038T | 1.20 | 0.86 | 1.07 | 1.05 | 1.1 | 0.93 |
| 38 | S038V | 1.28 | 1.05 | 1.07 | 1.02 | 1.1 | 1.02 |
| 38 | S038W | 1.15 | 0.75 | 0.77 | 1.04 | 1.3 | 0.91 |
| 39 | H039Q | 1.07 | 0.90 | 0.95 | 0.93 | 0.4 | 1.19 |
| 40 | P040A | 1.01 | 1.23 | 1.09 | 1.08 | 0.8 | 1.22 |
| 40 | P040C | 0.99 | 1.04 | 1.19 | 1.15 | 1.1 | 1.12 |
| 40 | P040E | 1.21 | 1.05 | 1.34 | 1.05 | 1.6 | 1.01 |
| 40 | P040F | 0.92 | 1.12 | 1.21 | 1.09 | 0.9 | 1.02 |
| 40 | P040G | 1.06 | 1.04 | 1.11 | 1.14 | 0.1 | 1.11 |
| 40 | P040H | 0.89 | 0.97 | 0.98 | 1.17 | 0.5 | 1.09 |
| 40 | P040I | 0.83 | 1.12 | 1.30 | 1.10 | 0.7 | 1.23 |
| 40 | P040L | 1.06 | 1.20 | 1.05 | 1.10 | 1.0 | 1.09 |
| 40 | P040M | 1.01 | 1.07 | 1.24 | 1.10 | 0.6 | 0.98 |
| 40 | P040N | 0.88 | 1.12 | 1.14 | 1.07 | 0.5 | 1.18 |
| 40 | P040Q | 1.01 | 1.16 | 1.13 | 1.11 | 1.1 | 1.01 |
| 40 | P040S | 0.84 | 1.15 | 1.06 | 0.81 | 0.3 | 1.12 |
| 40 | P040T | 0.81 | 1.29 | 1.68 | 1.22 | 0.1 | 1.20 |
| 40 | P040V | 0.91 | 1.04 | 1.07 | 1.09 | 0.7 | 1.06 |
| 40 | P040W | 1.09 | 0.87 | 0.90 | 1.13 | 1.0 | 1.12 |
| 40 | P040Y | 0.89 | 1.07 | 1.07 | 1.06 | 0.5 | 1.02 |
| 41 | D041S | 0.23 | 3.12 | 1.52 | 1.20 | 0.5 | 0.33 |
| 42 | L042C | 0.41 | 0.71 | 0.95 | 1.06 | 0.2 | 1.08 |
| 42 | L042H | 0.23 | 0.98 | 1.26 | 1.11 | 0.3 | 0.38 |
| 42 | L042I | 1.15 | 0.87 | 0.56 | 0.92 | 0.8 | 0.77 |
| 42 | L042M | 0.80 | 1.20 | 0.99 | 1.00 | 0.7 | 0.92 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 42 | L042N | 0.23 | 0.80 | 0.86 | 1.21 | 0.6 | 0.48 |
| 42 | L042V | 0.97 | 0.96 | 0.88 | 0.98 | 0.5 | 0.82 |
| 43 | K043A | 0.87 | 0.67 | 1.08 | 0.90 | 1.2 | 1.01 |
| 43 | K043C | 0.83 | 0.60 | 1.17 | 0.89 | 1.4 | 0.99 |
| 43 | K043D | 0.88 | 0.70 | 1.16 | 0.71 | 1.3 | 1.03 |
| 43 | K043E | 0.80 | 0.56 | 1.04 | 0.74 | 1.3 | 1.13 |
| 43 | K043F | 0.74 | 0.72 | 1.24 | 0.78 | 1.3 | 1.02 |
| 43 | K043G | 0.96 | 0.88 | 0.82 | 0.93 | 1.3 | 1.06 |
| 43 | K043I | 0.72 | 0.57 | 1.12 | 0.95 | 0.7 | 1.17 |
| 43 | K043L | 0.96 | 0.58 | 0.88 | 0.84 | 1.3 | 0.88 |
| 43 | K043M | 1.02 | 0.79 | 1.19 | 0.97 | 1.1 | 1.07 |
| 43 | K043N | 0.80 | 0.85 | 1.31 | 0.81 | 1.2 | 1.32 |
| 43 | K043Q | 0.95 | 0.61 | 1.09 | 0.90 | 1.3 | 1.05 |
| 43 | K043R | 1.14 | 0.56 | 1.30 | 0.87 | 0.9 | 1.33 |
| 43 | K043S | 0.98 | 0.77 | 1.14 | 0.92 | 1.3 | 0.99 |
| 43 | K043T | 0.93 | 0.92 | 1.06 | 0.96 | 1.1 | 1.07 |
| 43 | K043V | 1.10 | 0.59 | 0.98 | 0.93 | 0.6 | 1.26 |
| 43 | K043W | 0.85 | 0.67 | 0.98 | 0.90 | 1.3 | 1.03 |
| 43 | K043Y | 0.87 | 0.67 | 1.12 | 0.82 | 1.1 | 1.60 |
| 44 | V044A | 0.71 | 0.91 | 1.16 | 1.01 | 0.7 | 0.81 |
| 44 | V044C | 0.93 | 0.94 | 1.26 | 0.79 | 0.8 | 0.90 |
| 44 | V044E | 0.18 | 0.71 | 2.18 | 1.98 | 0.5 | 0.19 |
| 44 | V044G | 0.21 | 0.75 | 1.66 | 1.12 | 0.5 | 0.34 |
| 44 | V044H | 0.19 | 1.63 | 2.57 | 1.57 | 0.5 | 0.38 |
| 44 | V044I | 0.92 | 0.76 | 0.98 | 1.07 | 0.9 | 1.08 |
| 44 | V044L | 0.85 | 0.67 | 1.03 | 0.85 | 0.8 | 0.88 |
| 44 | V044M | 0.65 | 0.95 | 1.33 | 1.03 | 0.6 | 0.82 |
| 44 | V044P | 0.67 | 0.83 | 1.25 | 1.03 | 0.4 | 0.78 |
| 44 | V044Q | 0.20 | 1.48 | 2.85 | 1.49 | 0.6 | 0.33 |
| 44 | V044R | 0.19 | 0.79 | 1.62 | 0.69 | 0.2 | 0.13 |
| 44 | V044S | 0.33 | 1.03 | 1.51 | 1.13 | 0.5 | 0.86 |
| 44 | V044T | 0.30 | 1.32 | 1.94 | 1.30 | 0.7 | 1.26 |
| 44 | V044Y | 0.62 | 0.79 | 1.22 | 1.13 | 0.7 | 1.04 |
| 45 | A045C | 1.09 | 0.89 | 0.76 | 0.89 | 1.2 | 1.05 |
| 45 | A045E | 1.06 | 1.10 | 0.97 | 0.82 | 1.2 | 1.07 |
| 45 | A045F | 1.04 | 0.88 | 0.81 | 1.00 | 1.0 | 1.11 |
| 45 | A045H | 1.04 | 0.86 | 0.76 | 0.91 | 1.1 | 1.11 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 45 | A045I | 1.07 | 1.08 | 0.81 | 1.16 | 1.2 | 1.03 |
| 45 | A045K | 1.05 | 1.00 | 0.69 | 1.06 | 0.9 | 1.03 |
| 45 | A045L | 1.03 | 0.72 | 0.82 | 0.99 | 1.0 | 1.19 |
| 45 | A045M | 1.15 | 1.02 | 0.87 | 1.00 | 1.2 | 1.09 |
| 45 | A045N | 1.35 | 1.03 | 0.91 | 0.88 | 1.1 | 1.03 |
| 45 | A045P | 0.87 | 0.82 | 0.90 | 0.96 | 0.7 | 1.20 |
| 45 | A045Q | 1.07 | 0.84 | 0.95 | 1.09 | 1.1 | 1.16 |
| 45 | A045S | 1.08 | 0.93 | 0.87 | 0.86 | 1.2 | 1.02 |
| 45 | A045T | 0.99 | 1.04 | 1.31 | 0.90 | 1.2 | 1.01 |
| 45 | A045V | 1.28 | 0.85 | 0.81 | 1.03 | 1.1 | 1.12 |
| 45 | A045Y | 1.03 | 0.81 | 0.67 | 0.73 | 1.1 | 1.13 |
| 46 | G046A | 0.92 | 1.09 | 0.97 | 1.00 | 1.0 | 0.90 |
| 46 | G046C | 0.39 | 1.28 | 0.81 | 0.90 | 1.1 | 0.82 |
| 46 | G046E | 0.57 | 1.10 | 0.70 | 0.95 | 1.0 | 1.16 |
| 46 | G046F | 0.39 | 1.40 | 0.95 | 1.03 | 1.0 | 0.88 |
| 46 | G046H | 0.48 | 0.98 | 0.99 | 1.25 | 1.0 | 1.20 |
| 46 | G046K | 0.44 | 0.88 | 0.72 | 1.09 | 0.8 | 1.20 |
| 46 | G046L | 0.27 | 1.57 | 1.44 | 1.42 | 0.8 | 0.78 |
| 46 | G046M | 0.42 | 1.30 | 0.84 | 1.14 | 0.9 | 0.96 |
| 46 | G046N | 0.69 | 0.79 | 0.72 | 1.05 | 1.1 | 0.94 |
| 46 | G046S | 0.74 | 0.86 | 0.78 | 0.94 | 0.9 | 1.16 |
| 46 | G046T | 0.39 | 1.21 | 0.96 | 0.79 | 0.8 | 1.04 |
| 46 | G046V | 0.23 | 4.92 | 3.26 | 2.09 | 0.8 | 0.74 |
| 46 | G046W | 0.44 | 1.08 | 0.66 | 0.90 | 1.0 | 1.13 |
| 46 | G046Y | 0.36 | 1.02 | 1.27 | 1.12 | 0.8 | 1.23 |
| 47 | G047A | 0.32 | 0.98 | 0.96 | 1.04 | 0.5 | 0.30 |
| 47 | G047C | 0.24 | 0.64 | 1.09 | 0.88 | 0.6 | 0.15 |
| 47 | G047E | 0.23 | 0.72 | 0.99 | 1.02 | 0.6 | 0.14 |
| 47 | G047F | 0.25 | 0.96 | 0.86 | 1.15 | 0.5 | 0.15 |
| 47 | G047H | 0.23 | 1.04 | 1.17 | 1.20 | 1.1 | 0.47 |
| 47 | G047M | 0.22 | 0.63 | 0.89 | 0.87 | 0.5 | 0.10 |
| 47 | G047S | 0.21 | 1.19 | 1.04 | 1.24 | 0.5 | 0.15 |
| 47 | G047T | 0.17 | 4.45 | 4.77 | 2.27 | 0.5 | 0.12 |
| 47 | G047W | 0.18 | 2.60 | 2.43 | 2.22 | 0.5 | 0.17 |
| 48 | A048C | 1.15 | 1.14 | 1.16 | 0.85 | 1.1 | 0.95 |
| 48 | A048D | 1.13 | 1.01 | 1.00 | 0.97 | 1.1 | 0.89 |
| 48 | A048E | 1.17 | 0.84 | 0.93 | 1.02 | 1.2 | 0.90 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 48 | A048F | 1.08 | 1.08 | 0.92 | 0.96 | 1.0 | 0.89 |
| 48 | A048H | 1.08 | 0.87 | 1.16 | 1.04 | 1.1 | 0.87 |
| 48 | A048I | 1.02 | 0.79 | 0.91 | 1.02 | 1.2 | 1.00 |
| 48 | A048K | 1.13 | 0.90 | 0.55 | 0.91 | 1.1 | 0.90 |
| 348 | A048L | 0.99 | 0.87 | 0.97 | 0.99 | 1.0 | 0.91 |
| 48 | A048M | 0.59 | 1.21 | 0.99 | 0.87 | 1.1 | 0.92 |
| 48 | A048Q | 1.10 | 0.83 | 0.69 | 1.03 | 1.0 | 1.02 |
| 48 | A048R | 1.11 | 0.83 | 0.51 | 0.98 | 0.9 | 1.05 |
| 48 | A048S | 0.98 | 1.08 | 0.47 | 1.06 | 1.0 | 1.03 |
| 48 | A048T | 0.98 | 1.06 | 0.77 | 0.84 | 1.1 | 0.97 |
| 48 | A048V | 1.47 | 0.99 | 0.61 | 0.92 | 1.0 | 0.94 |
| 48 | A048W | 0.81 | 0.69 | 0.74 | 0.81 | 0.9 | 1.04 |
| 48 | A048Y | 1.03 | 0.84 | 0.81 | 0.86 | 1.0 | 0.90 |
| 49 | S049A | 0.35 | 0.54 | 0.61 | 1.02 | 0.9 | 0.30 |
| 49 | S049C | 0.61 | 0.48 | 0.69 | 0.97 | 0.5 | 0.38 |
| 49 | S049D | 0.23 | 1.09 | 1.53 | 1.69 | 0.5 | 0.36 |
| 49 | S049G | 0.28 | 0.78 | 0.75 | 1.64 | 1.2 | 0.50 |
| 49 | S049I | 0.18 | 2.22 | 2.52 | 2.14 | 0.5 | 0.11 |
| 49 | S049N | 0.35 | 1.04 | 0.88 | 1.32 | 0.5 | 0.64 |
| 49 | S049T | 0.29 | 0.95 | 1.23 | 1.37 | 0.5 | 0.46 |
| 49 | S049V | 0.21 | 1.58 | 1.39 | 2.10 | 0.4 | 0.30 |
| 50 | M050A | 0.78 | 1.16 | 0.85 | 0.89 | 0.9 | 1.09 |
| 50 | M050C | 0.89 | 1.17 | 0.89 | 0.80 | 1.1 | 1.01 |
| 50 | M050D | 0.17 | 6.35 | 0.87 | 0.90 | 1.0 | 0.16 |
| 50 | M050F | 0.95 | 1.01 | 0.96 | 0.81 | 1.1 | 1.09 |
| 50 | M050H | 0.85 | 1.22 | 0.98 | 0.95 | 1.2 | 1.01 |
| 50 | M050I | 0.33 | 1.61 | 0.91 | 1.09 | 0.9 | 0.55 |
| 50 | M050K | 0.49 | 0.80 | 0.67 | 0.79 | 1.1 | 1.07 |
| 50 | M050L | 0.91 | 0.76 | 1.02 | 0.96 | 1.1 | 1.00 |
| 50 | M050N | 0.35 | 1.03 | 0.86 | 1.07 | 1.0 | 1.11 |
| 50 | M050Q | 0.76 | 1.23 | 0.93 | 1.03 | 1.1 | 1.01 |
| 50 | M050R | 0.25 | 1.38 | 0.73 | 1.07 | 1.0 | 0.82 |
| 50 | M050S | 0.81 | 1.08 | 0.89 | 1.05 | 1.0 | 1.08 |
| 50 | M050T | 0.87 | 1.05 | 0.90 | 1.04 | 1.1 | 1.38 |
| 50 | M050V | 0.70 | 1.08 | 0.82 | 1.02 | 1.1 | 0.82 |
| 50 | M050W | 1.06 | 1.10 | 0.80 | 0.78 | 1.0 | 1.16 |
| 50 | M050Y | 0.70 | 1.03 | 0.87 | 0.89 | 1.0 | 1.19 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 51 | V051A | 0.38 | 1.41 | 1.25 | 1.22 | 0.9 | 0.43 |
| 51 | V051C | 0.91 | 1.02 | 1.16 | 1.02 | 1.1 | 0.41 |
| 51 | V051D | 0.34 | 1.02 | 1.32 | 1.12 | 1.0 | 0.50 |
| 51 | V051E | 0.39 | 1.17 | 1.59 | 1.26 | 1.0 | 0.59 |
| 51 | V051H | 0.86 | 0.59 | 1.06 | 0.98 | 1.2 | 0.79 |
| 51 | V051I | 0.69 | 0.79 | 1.23 | 1.12 | 1.1 | 0.95 |
| 51 | V051L | 0.48 | 0.73 | 1.21 | 0.87 | 1.0 | 0.59 |
| 51 | V051M | 0.60 | 0.97 | 1.07 | 1.10 | 1.0 | 0.67 |
| 51 | VOS1Q | 0.28 | 1.26 | 1.81 | 1.14 | 0.9 | 0.40 |
| 51 | V051S | 0.32 | 1.84 | 1.95 | 1.43 | 0.9 | 0.35 |
| 51 | V051T | 0.46 | 0.98 | 1.16 | 1.03 | 0.9 | 0.75 |
| 51 | V051Y | 0.25 | 1.20 | 1.58 | 1.76 | 0.9 | 0.31 |
| 52 | P052A | 0.56 | 1.22 | 1.25 | 1.17 | 0.7 | 0.59 |
| 52 | P052C | 0.34 | 1.36 | 1.56 | 0.94 | 1.0 | 0.44 |
| 52 | P052D | 0.79 | 1.27 | 1.50 | 1.21 | 0.9 | 0.76 |
| 52 | P052E | 0.89 | 1.12 | 1.54 | 1.20 | 0.9 | 0.75 |
| 52 | P052F | 0.41 | 0.87 | 1.13 | 1.14 | 0.9 | 0.51 |
| 52 | P052G | 0.44 | 1.05 | 1.42 | 1.24 | 0.8 | 0.53 |
| 52 | P052H | 0.45 | 0.97 | 1.28 | 1.04 | 0.9 | 0.47 |
| 52 | P052I | 0.45 | 1.33 | 1.50 | 1.11 | 0.8 | 0.61 |
| 52 | P052K | 0.38 | 0.86 | 1.09 | 1.35 | 0.8 | 0.53 |
| 52 | P052L | 0.46 | 1.33 | 1.24 | 1.32 | 0.8 | 0.61 |
| 52 | P052M | 0.43 | 1.40 | 1.30 | 1.06 | 0.7 | 0.54 |
| 52 | P052Q | 0.43 | 0.83 | 1.48 | 1.20 | 0.8 | 0.63 |
| 52 | P052R | 0.33 | 1.02 | 1.31 | 1.26 | 0.7 | 0.58 |
| 52 | P052S | 0.51 | 1.02 | 1.55 | 1.26 | 0.8 | 0.60 |
| 52 | P052T | 0.47 | 1.23 | 1.73 | 1.09 | 0.8 | 0.61 |
| 52 | P052V | 0.31 | 1.79 | 1.63 | 1.03 | 1.0 | 0.50 |
| 52 | P052W | 0.36 | 1.49 | 1.34 | 1.43 | 1.0 | 0.77 |
| 52 | P052Y | 0.35 | 1.55 | 1.42 | 1.32 | 1.0 | 0.55 |
| 53 | S053A | 1.37 | 1.07 | 1.15 | 1.03 | 1.0 | 0.87 |
| 53 | S053E | 1.52 | 1.05 | 1.16 | 1.12 | 1.0 | 0.98 |
| 53 | S053F | 1.19 | 0.91 | 1.05 | 0.87 | 1.0 | 1.11 |
| 53 | S053G | 1.72 | 1.15 | 1.19 | 0.94 | 1.0 | 0.86 |
| 53 | S053H | 1.35 | 0.96 | 1.02 | 0.96 | 1.1 | 0.84 |
| 53 | S053I | 1.22 | 0.97 | 1.10 | 0.80 | 1.0 | 0.90 |
| 53 | S053K | 1.36 | 0.89 | 0.93 | 0.76 | 1.1 | 0.95 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 53 | S053L | 1.18 | 0.92 | 0.97 | 1.00 | 1.1 | 0.90 |
| 53 | S053M | 1.54 | 1.01 | 0.98 | 0.97 | 0.8 | 0.80 |
| 53 | S053N | 1.48 | 0.95 | 1.04 | 1.15 | 1.1 | 0.87 |
| 53 | S053P | 0.46 | 1.01 | 1.39 | 0.94 | 0.9 | 0.85 |
| 53 | S053Q | 0.76 | 0.85 | 1.30 | 1.09 | 1.1 | 1.05 |
| 53 | S053R | 1.06 | 0.68 | 0.82 | 0.87 | 1.1 | 1.00 |
| 53 | S053T | 1.33 | 1.05 | 1.23 | 0.85 | 1.0 | 0.94 |
| 53 | S053V | 1.13 | 1.11 | 1.20 | 1.10 | 1.1 | 0.95 |
| 53 | S053W | 1.49 | 0.86 | 1.02 | 0.80 | 0.9 | 0.98 |
| 54 | E054A | 0.89 | 0.97 | 1.07 | 0.83 | 0.6 | 0.66 |
| 54 | E054C | 0.71 | 0.94 | 1.05 | 0.85 | 0.8 | 0.68 |
| 54 | E054F | 0.51 | 0.86 | 0.97 | 0.93 | 0.7 | 0.63 |
| 54 | E054G | 0.38 | 1.27 | 0.59 | 1.04 | 0.5 | 0.42 |
| 54 | E054H | 0.73 | 0.69 | 0.80 | 0.96 | 0.8 | 0.67 |
| 54 | E054K | 0.35 | 1.03 | 1.14 | 1.07 | 0.8 | 0.60 |
| 54 | E054L | 0.49 | 1.00 | 1.02 | 0.87 | 0.8 | 0.71 |
| 54 | E054M | 0.63 | 1.06 | 0.99 | 1.03 | 0.7 | 0.61 |
| 54 | E054N | 0.97 | 0.99 | 1.03 | 1.06 | 0.8 | 0.76 |
| 54 | E054P | 0.19 | 0.47 | 1.13 | 1.34 | 0.6 | 0.17 |
| 54 | E054Q | 0.97 | 1.00 | 0.99 | 1.04 | 0.8 | 0.90 |
| 54 | E054R | 0.24 | 1.47 | 1.83 | 1.68 | 0.6 | 0.55 |
| 54 | E054S | 0.98 | 0.97 | 0.97 | 0.96 | 0.8 | 0.69 |
| 54 | E054T | 0.52 | 1.02 | 1.11 | 0.93 | 0.6 | 0.70 |
| 54 | E054V | 0.41 | 1.12 | 1.34 | 1.11 | 0.6 | 0.78 |
| 54 | E054W | 0.31 | 1.18 | 1.18 | 1.01 | 0.6 | 0.71 |
| 54 | E054Y | 0.42 | 1.03 | 0.99 | 1.00 | 0.8 | 0.69 |
| 55 | T055A | 1.11 | 0.63 | 0.98 | 1.00 | 1.0 | 0.96 |
| 55 | T055C | 1.41 | 0.64 | 1.06 | 0.89 | 1.1 | 0.86 |
| 55 | T055D | 0.76 | 1.01 | 1.26 | 0.94 | 1.1 | 1.06 |
| 55 | T055F | 0.67 | 0.62 | 0.78 | 0.84 | 0.9 | 0.92 |
| 55 | T055G | 1.21 | 1.00 | 0.90 | 0.97 | 1.1 | 0.85 |
| 55 | T055H | 1.06 | 1.14 | 0.98 | 0.96 | 1.1 | 1.04 |
| 55 | T055I | 0.96 | 1.08 | 0.97 | 0.89 | 1.0 | 1.02 |
| 55 | T055K | 1.27 | 0.59 | 0.87 | 0.98 | 1.0 | 1.09 |
| 55 | T055L | 1.39 | 0.80 | 1.30 | 0.96 | 1.1 | 0.95 |
| 55 | T055M | 0.92 | 0.85 | 1.19 | 0.99 | 1.0 | 1.09 |
| 55 | T055P | 0.94 | 0.90 | 1.20 | 0.86 | 1.0 | 1.23 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 55 | T055Q | 1.06 | 0.87 | 1.20 | 0.91 | 1.1 | 1.06 |
| 55 | T055R | 1.14 | 0.54 | 0.81 | 0.90 | 1.0 | 1.01 |
| 55 | T055S | 1.00 | 1.03 | 1.32 | 0.94 | 1.1 | 0.99 |
| 55 | T055V | 0.98 | 0.54 | 1.20 | 0.97 | 1.0 | 1.10 |
| 55 | T055W | 1.06 | 0.68 | 0.81 | 0.92 | 1.0 | 0.95 |
| 55 | T055Y | 0.97 | 0.90 | 0.97 | 0.91 | 1.0 | 0.91 |
| 56 | N056A | 0.31 | 1.18 | 1.27 | 1.14 | 0.4 | 0.34 |
| 56 | N056D | 1.09 | 0.79 | 0.89 | 0.84 | 1.2 | 0.99 |
| 56 | N056E | 0.67 | 0.91 | 0.98 | 0.91 | 0.7 | 0.69 |
| 56 | N056F | 0.27 | 1.02 | 1.00 | 1.31 | 0.6 | 0.31 |
| 56 | N056G | 0.29 | 1.61 | 1.06 | 1.22 | 0.4 | 0.46 |
| 56 | N056H | 0.39 | 1.08 | 1.07 | 0.95 | 0.8 | 0.81 |
| 56 | N0561 | 0.24 | 1.69 | 1.26 | 1.41 | 0.6 | 0.47 |
| 56 | N056K | 0.21 | 2.07 | 1.45 | 1.86 | 0.5 | 0.38 |
| 56 | N056L | 0.23 | 1.26 | 1.81 | 1.66 | 0.7 | 0.34 |
| 56 | N056M | 0.26 | 1.50 | 1.24 | 1.23 | 0.5 | 0.36 |
| 56 | N056P | 0.26 | 0.95 | 1.32 | 0.94 | 0.8 | 0.62 |
| 56 | N056Q | 0.29 | 1.74 | 1.29 | 1.22 | 0.8 | 0.41 |
| 56 | N056R | 0.17 | 4.90 | 3.96 | 2.20 | 0.5 | 0.27 |
| 56 | N056S | 0.99 | 0.78 | 0.97 | 0.92 | 0.9 | 0.77 |
| 56 | N056T | 0.58 | 0.90 | 1.08 | 1.04 | 0.6 | 0.65 |
| 56 | N056V | 0.27 | 1.36 | 1.29 | 0.99 | 0.5 | 0.49 |
| 56 | N056W | 0.36 | 1.28 | 1.33 | 1.16 | 0.6 | 0.48 |
| 56 | N056Y | 0.25 | 1.30 | 1.25 | 1.30 | 0.7 | 0.41 |
| 57 | P057A | 0.61 | 0.95 | 1.08 | 1.03 | 0.5 | 0.66 |
| 57 | P057C | 0.50 | 1.04 | 1.18 | 0.95 | 0.8 | 0.65 |
| 57 | P057D | 0.52 | 1.11 | 1.42 | 1.14 | 0.9 | 0.87 |
| 57 | P057E | 0.38 | 1.23 | 1.53 | 1.23 | 0.8 | 0.87 |
| 57 | P057F | 0.59 | 0.93 | 0.98 | 0.92 | 0.3 | 0.75 |
| 57 | P057G | 0.42 | 1.29 | 1.19 | 1.12 | 0.3 | 0.69 |
| 57 | P057I | 0.25 | 2.20 | 2.00 | 1.60 | 0.4 | 0.29 |
| 57 | P057K | 0.24 | 1.51 | 1.84 | 1.09 | 0.3 | 0.34 |
| 57 | P057L | 0.24 | 2.59 | 2.00 | 1.46 | 0.4 | 0.28 |
| 57 | P057M | 0.36 | 1.08 | 1.11 | 0.97 | 0.4 | 0.40 |
| 57 | P057N | 0.35 | 1.17 | 1.67 | 1.11 | 0.7 | 0.73 |
| 57 | P057Q | 0.35 | 1.25 | 1.38 | 1.04 | 0.5 | 0.65 |
| 57 | P057R | 0.23 | 2.31 | 3.14 | 1.52 | 0.4 | 0.42 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 57 | P057S | 0.39 | 0.91 | 1.10 | 0.97 | 0.5 | 0.65 |
| 57 | P057T | 0.25 | 2.53 | 1.97 | 2.27 | 0.5 | 0.56 |
| 57 | P057V | 0.22 | 3.61 | 5.95 | 3.50 | 0.3 | 0.48 |
| 57 | P057W | 1.03 | 0.61 | 1.05 | 0.94 | 0.7 | 0.86 |
| 58 | F058A | 0.55 | 1.18 | 1.32 | 1.15 | 0.6 | 0.61 |
| 58 | F058C | 0.95 | 1.08 | 1.21 | 0.93 | 1.1 | 0.92 |
| 58 | F058D | 0.52 | 1.18 | 1.32 | 0.97 | 1.0 | 0.93 |
| 58 | F058E | 0.87 | 0.87 | 1.20 | 1.09 | 0.9 | 1.05 |
| 58 | F058G | 0.91 | 1.21 | 1.26 | 1.00 | 1.1 | 0.76 |
| 58 | F058H | 0.98 | 0.96 | 1.34 | 0.89 | 0.9 | 0.99 |
| 58 | F058I | 0.64 | 0.88 | 1.07 | 0.94 | 0.8 | 1.00 |
| 58 | F058K | 0.44 | 1.15 | 1.36 | 1.09 | 0.6 | 0.63 |
| 58 | F058L | 0.89 | 0.99 | 1.16 | 0.89 | 0.9 | 0.86 |
| 58 | F058M | 1.01 | 1.08 | 1.24 | 0.91 | 0.8 | 0.82 |
| 58 | F058N | 0.86 | 1.18 | 1.25 | 0.87 | 0.9 | 0.91 |
| 58 | F058P | 0.23 | 0.71 | 1.00 | 0.89 | 0.6 | 0.22 |
| 58 | F058Q | 0.60 | 1.22 | 1.20 | 0.93 | 0.6 | 0.80 |
| 58 | F058R | 0.49 | 0.75 | 1.06 | 0.95 | 1.0 | 0.73 |
| 58 | F058S | 0.61 | 1.21 | 1.10 | 1.03 | 0.6 | 0.81 |
| 58 | F058T | 0.36 | 1.36 | 1.76 | 1.16 | 0.7 | 1.01 |
| 58 | F058V | 0.73 | 1.01 | 1.09 | 1.05 | 0.7 | 0.98 |
| 58 | F058Y | 1.16 | 1.19 | 0.90 | 1.00 | 0.9 | 1.09 |
| 59 | Q059A | 1.27 | 0.91 | 1.18 | 0.96 | 0.9 | 0.96 |
| 59 | Q059C | 0.89 | 1.08 | 1.26 | 1.06 | 1.2 | 0.92 |
| 59 | Q059D | 1.42 | 1.09 | 1.31 | 0.88 | 1.2 | 1.01 |
| 59 | Q059E | 1.42 | 1.11 | 1.15 | 0.85 | 1.3 | 0.94 |
| 59 | Q059F | 1.18 | 0.97 | 1.13 | 0.85 | 0.9 | 0.93 |
| 59 | Q059G | 0.92 | 1.06 | 1.05 | 1.00 | 0.7 | 0.75 |
| 59 | Q059H | 1.24 | 0.91 | 1.00 | 1.14 | 1.0 | 0.91 |
| 59 | Q059K | 1.31 | 0.83 | 0.87 | 0.87 | 0.9 | 0.94 |
| 59 | Q059L | 1.21 | 1.10 | 1.12 | 1.04 | 1.1 | 1.01 |
| 59 | Q059M | 1.26 | 1.18 | 1.08 | 0.95 | 1.1 | 0.95 |
| 59 | Q059N | 1.15 | 1.12 | 1.16 | 1.01 | 1.0 | 0.98 |
| 59 | Q059P | 0.27 | 1.86 | 2.01 | 1.47 | 0.8 | 0.43 |
| 59 | Q059R | 0.82 | 0.78 | 0.71 | 0.81 | 1.0 | 0.95 |
| 59 | Q059S | 1.25 | 1.13 | 1.15 | 0.94 | 0.8 | 0.97 |
| 59 | Q059T | 1.37 | 1.10 | 1.04 | 1.02 | 0.9 | 0.90 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 59 | Q059V | 1.38 | 1.14 | 1.14 | 1.14 | 1.0 | 1.21 |
| 59 | Q059W | 0.24 | 1.36 | 1.62 | 1.16 | 1.0 | 0.46 |
| 59 | Q059Y | 1.12 | 1.08 | 1.21 | 1.08 | 0.9 | 0.86 |
| 60 | D060A | 0.43 | 1.01 | 1.21 | 0.99 | 0.2 | 0.68 |
| 60 | D060C | 0.49 | 0.81 | 0.94 | 0.89 | 0.5 | 0.56 |
| 60 | D060E | 0.32 | 0.89 | 1.23 | 0.94 | 0.5 | 0.45 |
| 60 | D060F | 0.25 | 0.35 | 1.00 | 0.71 | 0.1 | 0.34 |
| 60 | D060G | 1.09 | 0.80 | 0.93 | 0.89 | 0.4 | 0.91 |
| 60 | D060H | 0.31 | 0.66 | 1.29 | 0.95 | 0.2 | 0.50 |
| 60 | D060I | 0.33 | 0.50 | 1.15 | 0.83 | 0.1 | 0.61 |
| 60 | D060L | 0.27 | 0.54 | 1.07 | 0.88 | 0.1 | 0.41 |
| 60 | D060M | 0.27 | 0.87 | 1.26 | 1.16 | 0.2 | 0.43 |
| 60 | D060N | 0.32 | 0.77 | 1.19 | 1.11 | 0.2 | 0.55 |
| 60 | D060P | 0.85 | 0.64 | 0.83 | 0.73 | 0.1 | 0.59 |
| 60 | D060Q | 0.26 | 0.97 | 1.35 | 1.03 | 0.2 | 0.57 |
| 60 | D060R | 0.35 | 0.31 | 0.65 | 0.63 | 0.1 | 0.62 |
| 60 | D060S | 0.40 | 0.83 | 0.96 | 1.00 | 0.2 | 0.74 |
| 60 | D060T | 0.30 | 0.95 | 1.72 | 1.28 | 0.2 | 0.78 |
| 60 | D060V | 0.39 | 0.77 | 0.91 | 0.98 | 0.1 | 0.74 |
| 60 | D060W | 0.21 | 0.57 | 1.17 | 1.22 | 0.1 | 0.35 |
| 60 | D060Y | 0.28 | 0.58 | 0.93 | 0.73 | 0.2 | 0.47 |
| 61 | N061A | 1.66 | 1.22 | 1.11 | 1.09 | 1.0 | 0.95 |
| 61 | N061C | 1.41 | 0.95 | 0.96 | 0.90 | 1.1 | 1.11 |
| 61 | N061D | 1.25 | 1.19 | 1.21 | 0.85 | 1.2 | 1.14 |
| 61 | N061E | 1.53 | 1.21 | 1.08 | 1.08 | 1.2 | 1.08 |
| 61 | N061F | 1.50 | 1.00 | 0.90 | 0.80 | 1.0 | 0.89 |
| 61 | N061G | 1.45 | 1.01 | 1.06 | 1.01 | 1.0 | 0.80 |
| 61 | N061H | 1.46 | 1.15 | 1.00 | 0.93 | 1.2 | 0.89 |
| 61 | N061I | 1.32 | 1.25 | 1.00 | 0.71 | 1.2 | 1.10 |
| 61 | N061K | 1.20 | 0.89 | 1.00 | 0.76 | 0.9 | 1.11 |
| 61 | N061L | 1.34 | 0.96 | 1.00 | 0.88 | 1.1 | 1.07 |
| 61 | N061M | 1.28 | 0.98 | 1.01 | 1.04 | 1.0 | 1.06 |
| 61 | N061P | 1.42 | 1.47 | 1.04 | 0.78 | 1.0 | 1.40 |
| 61 | N061Q | 1.27 | 0.90 | 1.08 | 0.88 | 1.0 | 1.14 |
| 61 | N061R | 1.42 | 0.67 | 0.71 | 0.86 | 0.8 | 1.09 |
| 61 | N061S | 1.06 | 1.00 | 0.98 | 0.88 | 0.9 | 1.51 |
| 61 | N061T | 1.14 | 0.98 | 0.98 | 0.99 | 1.1 | 1.19 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 61 | N061V | 1.43 | 1.02 | 1.03 | 0.77 | 1.0 | 1.19 |
| 61 | N061W | 1.42 | 1.12 | 0.80 | 0.83 | 1.1 | 0.94 |
| 61 | N061Y | 1.31 | 1.04 | 0.97 | 0.98 | 1.2 | 0.90 |
| 62 | N062A | 1.20 | 0.77 | 0.76 | 0.88 | 1.1 | 1.18 |
| 62 | N062C | 1.57 | 1.18 | 1.16 | 0.82 | 0.3 | 0.63 |
| 62 | N062D | 1.48 | 1.40 | 1.18 | 0.91 | 1.1 | 0.67 |
| 62 | N062E | 1.42 | 1.19 | 1.19 | 0.92 | 1.1 | 0.59 |
| 62 | N062F | 2.06 | 0.75 | 1.07 | 0.87 | 1.1 | 0.37 |
| 62 | N062G | 1.41 | 1.03 | 0.83 | 0.84 | 1.3 | 0.78 |
| 62 | N0621 | 0.95 | 0.81 | 0.87 | 0.69 | 1.3 | 0.74 |
| 62 | N062K | 1.23 | 1.05 | 0.97 | 1.02 | 0.9 | 0.48 |
| 62 | N062L | 1.79 | 0.87 | 0.80 | 1.07 | 1.4 | 0.63 |
| 62 | N062M | 1.76 | 1.30 | 1.12 | 1.07 | 1.1 | 0.58 |
| 62 | N062Q | 1.45 | 1.32 | 1.23 | 1.06 | 1.2 | 0.60 |
| 62 | N062R | 1.33 | 1.13 | 0.73 | 0.94 | 0.9 | 0.29 |
| 62 | N062S | 0.65 | 1.14 | 0.97 | 0.91 | 1.0 | 1.06 |
| 62 | N062T | 0.96 | 1.21 | 0.97 | 0.86 | 1.1 | 1.01 |
| 62 | N062V | 1.43 | 0.86 | 1.00 | 0.57 | 1.1 | 0.56 |
| 62 | N062W | 1.56 | 1.27 | 1.25 | 0.79 | 1.1 | 0.11 |
| 62 | N062Y | 1.70 | 0.92 | 0.79 | 1.09 | 1.2 | 0.37 |
| 63 | S063A | 1.12 | 0.94 | 1.12 | 0.98 | 1.1 | 0.88 |
| 63 | S063C | 1.56 | 1.31 | 0.97 | 0.69 | 1.3 | 0.77 |
| 63 | S063D | 1.42 | 0.90 | 0.86 | 0.84 | 1.4 | 0.81 |
| 63 | S063E | 1.50 | 1.13 | 1.03 | 0.93 | 1.3 | 1.00 |
| 63 | S063F | 1.26 | 0.91 | 0.73 | 0.88 | 1.0 | 0.77 |
| 63 | S063G | 1.46 | 0.67 | 0.79 | 0.83 | 0.9 | 1.21 |
| 63 | S063H | 1.41 | 0.92 | 0.95 | 0.80 | 1.0 | 0.90 |
| 63 | S063K | 1.62 | 1.03 | 0.75 | 0.89 | 0.3 | 0.82 |
| 63 | S063L | 1.46 | 1.22 | 0.86 | 1.02 | 1.0 | 0.92 |
| 63 | S063M | 1.48 | 1.02 | 0.86 | 1.17 | 0.9 | 1.04 |
| 63 | S063N | 1.42 | 0.97 | 1.00 | 0.97 | 1.2 | 0.85 |
| 63 | S063P | 0.84 | 0.62 | 0.71 | 0.71 | 0.6 | 0.67 |
| 63 | S063Q | 1.55 | 1.36 | 0.81 | 1.05 | 1.0 | 0.98 |
| 63 | S063R | 1.58 | 0.93 | 0.65 | 0.83 | 0.1 | 1.03 |
| 63 | S063T | 1.03 | 1.33 | 1.03 | 1.01 | 1.0 | 0.93 |
| 63 | S063V | 1.19 | 1.13 | 0.88 | 0.98 | 0.7 | 1.35 |
| 63 | S063W | 1.26 | 0.84 | 0.93 | 0.67 | 0.9 | 2.18 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 63 | S063Y | 1.38 | 1.02 | 0.72 | 0.93 | 0.8 | 0.90 |
| 65 | G065Q | 0.23 | 1.74 | 2.07 | 2.34 | 1.2 | 0.66 |
| 66 | T066S | 1.10 | 0.87 | 0.95 | 0.89 | 0.6 | 0.81 |
| 67 | H067A | 1.37 | 0.43 | 0.24 | 0.50 | 0.5 | 0.34 |
| 67 | H067C | 1.36 | 0.46 | 0.24 | 0.52 | 0.3 | 0.29 |
| 67 | H067F | 1.13 | 0.64 | 0.50 | 0.81 | 0.1 | 0.33 |
| 67 | H067I | 1.55 | 0.51 | 0.20 | 0.47 | 0.2 | 0.42 |
| 67 | H067L | 1.31 | 0.37 | 0.14 | 0.47 | 0.3 | 0.16 |
| 67 | H067M | 1.34 | 0.56 | 0.31 | 0.66 | 0.6 | 0.42 |
| 67 | H067N | 1.12 | 0.76 | 0.31 | 0.65 | 0.1 | 0.62 |
| 67 | H067P | 1.01 | 0.43 | 0.18 | 0.50 | 0.8 | 0.51 |
| 67 | H067S | 1.26 | 0.55 | 0.28 | 0.55 | 0.1 | 0.39 |
| 67 | H067T | 1.23 | 0.57 | 0.36 | 0.69 | 0.1 | 0.52 |
| 68 | V068A | 1.00 | 1.79 | 1.11 | 1.30 | 1.0 | 0.06 |
| 68 | V068C | 1.05 | 1.11 | 1.10 | 1.07 | 0.8 | 0.70 |
| 68 | V068I | 1.19 | 1.15 | 0.96 | 0.82 | 1.2 | 0.32 |
| 68 | V068T | 0.94 | 1.17 | 1.06 | 0.79 | 0.6 | 0.42 |
| 69 | A069C | 0.36 | 1.66 | 0.92 | 1.19 | 0.7 | 0.45 |
| 69 | A069G | 0.88 | 0.73 | 0.95 | 1.04 | 0.9 | 1.02 |
| 69 | A069H | 0.17 | 39.03 | 2.24 | 0.72 | 0.7 | 0.08 |
| 69 | A069M | 0.19 | 7.17 | 0.69 | 0.55 | 0.5 | 0.06 |
| 69 | A069S | 0.85 | 0.61 | 0.70 | 1.17 | 0.9 | 0.93 |
| 69 | A069T | 0.48 | 1.58 | 0.96 | 1.02 | 1.0 | 0.72 |
| 71 | T071A | 0.44 | 1.17 | 1.02 | 1.07 | 0.1 | 0.59 |
| 71 | T071D | 0.21 | 41.12 | 34.56 | 1.45 | 0.2 | 0.06 |
| 71 | T071E | 0.25 | 2.16 | 1.92 | 1.03 | 0.1 | 0.33 |
| 71 | T071I | 0.80 | 0.74 | 1.19 | 1.08 | 0.5 | 1.07 |
| 71 | T071K | 0.21 | 37.02 | 45.54 | 2.17 | 0.1 | 0.17 |
| 71 | T071S | 1.14 | 0.87 | 1.17 | 1.17 | 0.5 | 0.89 |
| 71 | T071V | 0.74 | 0.85 | 1.00 | 0.99 | 0.5 | 0.86 |
| 72 | V072A | 0.84 | 0.95 | 1.00 | 0.94 | 0.8 | 0.89 |
| 72 | V072C | 0.71 | 1.13 | 1.02 | 1.10 | 0.8 | 0.88 |
| 72 | V072D | 0.19 | 2.54 | 1.66 | 1.93 | 0.7 | 0.19 |
| 72 | V072E | 0.50 | 1.14 | 1.08 | 1.13 | 0.8 | 0.62 |
| 72 | V072F | 0.71 | 0.96 | 1.06 | 0.96 | 0.7 | 0.74 |
| 72 | V072G | 0.35 | 1.52 | 1.06 | 1.36 | 0.8 | 0.65 |
| 72 | V072H | 0.35 | 1.05 | 1.01 | 1.17 | 0.8 | 0.57 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 72 | V072I | 1.16 | 1.12 | 1.03 | 0.97 | 1.0 | 0.79 |
| 72 | V072K | 0.19 | 2.81 | 2.06 | 2.76 | 1.7 | 0.30 |
| 72 | V072L | 1.00 | 1.16 | 1.07 | 0.97 | 0.9 | 0.67 |
| 72 | V072Q | 0.27 | 1.63 | 1.54 | 1.67 | 0.6 | 0.64 |
| 72 | V072S | 0.36 | 1.36 | 1.41 | 1.34 | 0.8 | 0.91 |
| 72 | V072T | 0.63 | 1.37 | 1.01 | 1.09 | 0.9 | 0.99 |
| 73 | A073C | 0.85 | 1.03 | 1.08 | 1.03 | 0.9 | 0.93 |
| 73 | A073E | 0.47 | 1.62 | 1.22 | 1.13 | 0.2 | 0.78 |
| 73 | A073G | 0.96 | 0.85 | 1.01 | 1.14 | 0.8 | 1.00 |
| 73 | A073I | 0.17 | 22.54 | 15.70 | 6.07 | 0.1 | 0.30 |
| 73 | A073K | 0.18 | 3.45 | 2.48 | 2.56 | 0.1 | 0.27 |
| 73 | A073M | 0.24 | 1.71 | 1.42 | 1.48 | 0.3 | 0.43 |
| 73 | A073Q | 0.55 | 1.31 | 1.33 | 1.14 | 0.5 | 0.93 |
| 73 | A073S | 1.02 | 1.35 | 0.94 | 1.00 | 0.8 | 1.06 |
| 73 | A073T | 0.71 | 1.27 | 1.06 | 1.15 | 0.6 | 1.11 |
| 73 | A073V | 0.38 | 1.42 | 1.19 | 1.46 | 0.1 | 0.83 |
| 74 | A074G | 1.04 | 0.89 | 0.85 | 1.06 | 0.2 | 1.00 |
| 77 | N077C | 0.23 | 0.95 | 0.12 | 0.36 | 0.2 | 0.14 |
| 77 | N077E | 0.20 | 1.17 | 0.13 | 0.35 | 0.2 | 0.07 |
| 77 | N0771 | 0.23 | 0.63 | 0.09 | 0.28 | 0.2 | 0.08 |
| 77 | N077K | 0.18 | 1.28 | 0.06 | 0.36 | 0.2 | 0.12 |
| 77 | N077L | 0.19 | 2.18 | 0.24 | 0.77 | 0.1 | 0.12 |
| 77 | N077M | 0.21 | 0.94 | 0.11 | 0.32 | 0.3 | 0.06 |
| 77 | N077R | 0.18 | 2.49 | 0.10 | 0.93 | 0.1 | 0.16 |
| 78 | S078A | 1.21 | 1.08 | 1.10 | 1.03 | 1.1 | 0.91 |
| 78 | S078C | 1.23 | 1.03 | 0.77 | 0.88 | 1.5 | 0.83 |
| 78 | S078D | 1.33 | 1.10 | 0.83 | 0.88 | 1.7 | 0.90 |
| 78 | S078E | 1.04 | 1.05 | 0.87 | 0.96 | 1.1 | 0.91 |
| 78 | S078F | 1.11 | 0.95 | 0.64 | 0.98 | 0.5 | 0.97 |
| 78 | S078G | 1.28 | 1.14 | 0.83 | 0.92 | 0.6 | 0.88 |
| 78 | S078I | 1.00 | 1.03 | 0.70 | 0.96 | 0.3 | 0.99 |
| 78 | S078K | 1.25 | 0.98 | 0.62 | 0.94 | 0.3 | 0.92 |
| 78 | S078L | 0.68 | 1.01 | 0.78 | 0.92 | 0.8 | 0.96 |
| 78 | S078M | 1.03 | 1.06 | 1.09 | 0.99 | 1.2 | 1.04 |
| 78 | S078N | 1.19 | 1.04 | 0.87 | 1.16 | 1.6 | 1.00 |
| 78 | S078Q | 1.12 | 1.11 | 0.94 | 1.09 | 1.4 | 1.36 |
| 78 | S078R | 1.22 | 1.02 | 0.52 | 0.81 | 0.7 | 1.15 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 78 | S078T | 1.07 | 1.17 | 1.02 | 0.99 | 1.4 | 1.09 |
| 78 | S078V | 1.13 | 1.16 | 0.97 | 0.91 | 0.5 | 0.90 |
| 78 | S078W | 1.00 | 1.31 | 0.67 | 1.02 | 0.3 | 1.08 |
| 78 | S078Y | 1.06 | 0.97 | 0.68 | 1.12 | 0.6 | 1.06 |
| 79 | I079C | 1.22 | 0.73 | 1.12 | 1.02 | 0.2 | 0.91 |
| 79 | I079E | 1.28 | 0.71 | 1.02 | 0.98 | 0.1 | 1.00 |
| 79 | I079F | 1.23 | 0.93 | 1.22 | 1.03 | 0.9 | 0.95 |
| 79 | I079V | 1.10 | 0.68 | 1.15 | 1.02 | 0.1 | 0.97 |
| 79 | I079W | 1.13 | 0.81 | 1.15 | 1.18 | 0.1 | 1.00 |
| 79 | I079Y | 1.19 | 0.76 | 1.26 | 0.92 | 1.0 | 1.25 |
| 80 | G080C | 0.19 | 0.60 | 1.75 | 1.16 | 0.1 | 0.36 |
| 81 | V081D | 0.24 | 1.35 | 0.77 | 0.60 | 0.1 | 0.14 |
| 81 | V081I | 1.15 | 1.09 | 0.82 | 1.05 | 0.3 | 0.88 |
| 81 | V081T | 0.94 | 1.17 | 0.79 | 1.00 | 0.2 | 0.67 |
| 81 | V081Y | 0.88 | 1.22 | 0.77 | 1.08 | 0.1 | 0.84 |
| 82 | L082G | 0.21 | 2.20 | 1.71 | 1.50 | 0.1 | 0.36 |
| 82 | L082V | 0.78 | 1.21 | 1.19 | 1.10 | 0.2 | 1.13 |
| 82 | L082W | 0.17 | 10.29 | 8.50 | 4.18 | 0.7 | 0.37 |
| 84 | V084A | 1.27 | 0.84 | 0.96 | 0.82 | 0.7 | 1.08 |
| 84 | V084C | 1.15 | 0.77 | 0.75 | 0.79 | 0.8 | 0.97 |
| 84 | V084G | 0.29 | 0.75 | 0.84 | 0.86 | 0.5 | 0.66 |
| 84 | V084I | 1.08 | 0.96 | 0.99 | 0.68 | 0.5 | 0.99 |
| 84 | V084L | 0.51 | 0.66 | 0.91 | 1.00 | 0.3 | 1.07 |
| 84 | V084M | 0.89 | 0.91 | 0.68 | 0.87 | 0.3 | 1.20 |
| 84 | V084N | 0.67 | 0.75 | 0.98 | 0.88 | 0.8 | 1.09 |
| 84 | V084S | 0.81 | 0.79 | 0.64 | 0.85 | 0.5 | 1.00 |
| 84 | V084T | 1.06 | 0.95 | 0.42 | 0.70 | 0.7 | 0.92 |
| 85 | A085C | 0.64 | 0.84 | 0.85 | 0.99 | 0.8 | 0.73 |
| 85 | A085G | 0.77 | 0.93 | 0.92 | 0.97 | 0.9 | 0.94 |
| 85 | A085S | 0.90 | 0.94 | 0.94 | 0.97 | 0.9 | 0.93 |
| 85 | A085T | 0.43 | 1.35 | 1.11 | 0.88 | 0.6 | 0.95 |
| 85 | A085V | 0.20 | 2.50 | 2.45 | 2.31 | 0.7 | 0.49 |
| 86 | P086A | 0.45 | 1.30 | 1.13 | 1.29 | 0.7 | 1.08 |
| 86 | P086D | 0.52 | 1.20 | 0.80 | 1.21 | 0.5 | 0.95 |
| 86 | P086E | 0.46 | 0.88 | 1.20 | 1.02 | 0.4 | 1.10 |
| 86 | P086G | 0.37 | 1.41 | 0.91 | 1.28 | 0.1 | 0.76 |
| 86 | P086M | 0.27 | 1.95 | 1.84 | 1.83 | 0.1 | 1.02 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 86 | P086N | 0.54 | 1.25 | 1.13 | 1.14 | 0.7 | 1.07 |
| 86 | P086Q | 0.25 | 1.54 | 1.60 | 1.99 | 0.1 | 0.88 |
| 86 | P086R | 0.19 | 3.54 | 3.82 | 2.73 | 0.7 | 0.72 |
| 86 | P086S | 0.53 | 1.15 | 1.15 | 1.06 | 0.7 | 1.21 |
| 86 | P086T | 0.28 | 1.73 | 1.80 | 1.87 | 0.2 | 1.05 |
| 86 | P086W | 0.42 | 1.35 | 1.52 | 1.25 | 0.4 | 1.41 |
| 86 | P086Y | 0.39 | 1.46 | 1.19 | 1.70 | 0.9 | 1.38 |
| 87 | S087A | 1.11 | 1.20 | 1.08 | 1.01 | 0.9 | 0.82 |
| 87 | S087C | 1.16 | 1.11 | 0.98 | 0.86 | 1.1 | 0.86 |
| 87 | S087D | 1.12 | 1.29 | 1.05 | 0.95 | 1.5 | 0.89 |
| 87 | S087E | 1.19 | 1.03 | 1.04 | 1.00 | 1.4 | 0.96 |
| 87 | S087F | 0.92 | 1.36 | 0.88 | 1.06 | 0.5 | 0.95 |
| 87 | S087G | 1.16 | 1.16 | 0.91 | 1.03 | 0.8 | 0.85 |
| 87 | S087I | 0.56 | 1.40 | 0.81 | 1.01 | 0.4 | 0.90 |
| 87 | S087K | 1.39 | 0.87 | 0.72 | 1.10 | 0.4 | 0.85 |
| 87 | S087L | 1.07 | 1.31 | 0.95 | 1.07 | 0.8 | 0.98 |
| 87 | S087M | 1.02 | 1.34 | 0.99 | 1.08 | 0.7 | 1.04 |
| 87 | S087N | 1.32 | 1.23 | 1.04 | 0.96 | 1.0 | 0.86 |
| 87 | S087Q | 0.81 | 1.34 | 0.93 | 0.96 | 0.8 | 0.87 |
| 87 | S087R | 1.23 | 1.06 | 0.73 | 0.99 | 0.2 | 0.82 |
| 87 | S087T | 1.08 | 1.17 | 1.00 | 1.10 | 0.9 | 1.16 |
| 87 | S087V | 0.92 | 1.33 | 0.95 | 1.17 | 0.9 | 1.15 |
| 87 | S087W | 0.91 | 1.44 | 1.01 | 1.26 | 0.5 | 1.06 |
| 88 | A088C | 0.78 | 1.12 | 1.30 | 1.05 | 0.9 | 1.02 |
| 88 | A088D | 0.27 | 1.44 | 1.63 | 1.25 | 0.7 | 0.68 |
| 88 | A088E | 0.17 | 11.44 | 17.45 | 1.68 | 0.6 | 0.16 |
| 88 | A088G | 0.65 | 1.19 | 1.28 | 1.06 | 0.9 | 1.07 |
| 88 | A088K | 0.21 | 2.38 | 2.98 | 1.68 | 0.8 | 0.67 |
| 88 | A088L | 0.62 | 1.20 | 1.17 | 0.93 | 0.8 | 1.06 |
| 88 | A088M | 0.45 | 1.03 | 1.50 | 0.98 | 0.4 | 0.73 |
| 88 | A088N | 0.67 | 0.97 | 1.11 | 1.08 | 0.6 | 1.17 |
| 88 | A088P | 0.21 | 2.19 | 3.40 | 2.03 | 0.9 | 0.62 |
| 88 | A088Q | 0.22 | 1.86 | 1.99 | 1.23 | 0.5 | 0.52 |
| 88 | A088S | 0.84 | 0.94 | 1.31 | 0.91 | 0.9 | 1.09 |
| 88 | A088T | 0.86 | 1.28 | 1.29 | 0.94 | 1.0 | 1.08 |
| 88 | A088V | 0.87 | 1.22 | 1.41 | 0.94 | 0.9 | 1.11 |
| 89 | S089C | 0.91 | 1.14 | 1.14 | 0.99 | 0.9 | 0.92 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 89 | S089D | 1.20 | 1.35 | 1.04 | 1.09 | 0.8 | 0.96 |
| 89 | S089E | 0.92 | 1.07 | 1.17 | 0.95 | 0.8 | 0.93 |
| 89 | S089F | 0.65 | 1.14 | 1.27 | 0.90 | 0.8 | 1.16 |
| 89 | S089G | 0.88 | 1.13 | 1.10 | 1.13 | 0.8 | 1.04 |
| 89 | S089H | 0.99 | 0.86 | 1.23 | 1.09 | 0.6 | 1.09 |
| 89 | S089K | 0.98 | 0.99 | 1.06 | 1.01 | 0.8 | 0.88 |
| 89 | S089L | 0.41 | 1.08 | 1.52 | 1.40 | 1.3 | 0.93 |
| 89 | S089M | 0.43 | 1.28 | 1.34 | 1.17 | 1.4 | 0.86 |
| 89 | S089R | 0.71 | 0.67 | 1.08 | 1.06 | 0.8 | 0.94 |
| 89 | S089V | 0.66 | 1.31 | 1.27 | 1.17 | 0.7 | 1.34 |
| 89 | S089W | 0.37 | 1.19 | 1.84 | 1.30 | 1.2 | 1.05 |
| 89 | S089Y | 0.75 | 1.22 | 1.34 | 1.00 | 0.7 | 1.13 |
| 90 | L090A | 0.35 | 1.29 | 1.22 | 1.35 | 0.7 | 0.85 |
| 90 | L090D | 0.20 | 2.40 | 2.05 | 2.19 | 0.6 | 0.47 |
| 90 | L090E | 0.23 | 1.61 | 1.38 | 1.49 | 1.0 | 0.52 |
| 90 | L090F | 0.30 | 1.57 | 0.81 | 1.39 | 0.3 | 0.34 |
| 90 | L090G | 0.28 | 0.21 | 0.15 | 0.22 | 0.9 | 0.11 |
| 90 | L090H | 0.23 | 2.00 | 2.18 | 2.26 | 1.0 | 0.88 |
| 90 | L090M | 0.78 | 1.07 | 1.04 | 0.94 | 1.0 | 0.95 |
| 90 | L090P | 0.27 | 1.56 | 1.73 | 1.61 | 0.9 | 0.88 |
| 90 | L090Q | 0.44 | 1.22 | 1.21 | 1.20 | 1.1 | 1.05 |
| 90 | L090S | 0.24 | 1.57 | 1.85 | 1.57 | 0.7 | 0.93 |
| 90 | L090T | 0.21 | 3.87 | 3.31 | 2.49 | 0.8 | 0.96 |
| 90 | L090V | 0.77 | 1.20 | 1.08 | 1.18 | 0.8 | 1.12 |
| 91 | Y091A | 0.25 | 0.60 | 1.29 | 1.22 | 0.9 | 0.67 |
| 91 | Y091C | 0.23 | 0.99 | 2.01 | 1.14 | 1.1 | 0.54 |
| 91 | Y091D | 0.31 | 0.90 | 1.51 | 1.27 | 1.1 | 0.80 |
| 91 | Y091F | 1.07 | 0.60 | 1.21 | 1.23 | 1.1 | 1.00 |
| 91 | Y091H | 0.80 | 0.71 | 1.16 | 1.04 | 1.0 | 0.99 |
| 91 | Y091I | 0.22 | 1.01 | 2.17 | 1.25 | 1.2 | 0.58 |
| 91 | Y091L | 0.18 | 1.59 | 2.82 | 1.67 | 1.0 | 0.49 |
| 91 | Y091M | 0.27 | 1.09 | 1.56 | 1.13 | 1.1 | 0.62 |
| 91 | Y091Q | 0.16 | 7.64 | 26.89 | 1.98 | 1.2 | 0.25 |
| 91 | Y091S | 0.25 | 0.90 | 1.68 | 1.44 | 0.8 | 0.86 |
| 91 | Y091T | 0.17 | 3.68 | 8.06 | 2.67 | 0.9 | 0.61 |
| 91 | Y091V | 0.23 | 0.89 | 1.92 | 1.46 | 1.0 | 0.70 |
| 92 | A092C | 0.29 | 2.61 | 2.00 | 1.71 | 0.6 | 0.33 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 92 | A092G | 0.55 | 1.16 | 1.46 | 1.30 | 0.8 | 0.67 |
| 92 | A092I | 0.33 | 1.73 | 1.53 | 1.55 | 0.7 | 0.31 |
| 92 | A092M | 0.21 | 2.49 | 2.62 | 2.92 | 0.6 | 0.16 |
| 92 | A092N | 0.18 | 6.44 | 7.31 | 5.48 | 0.7 | 0.15 |
| 92 | A092P | 0.26 | 1.62 | 2.34 | 2.13 | 0.9 | 0.47 |
| 92 | A092S | 1.04 | 0.85 | 0.94 | 1.22 | 1.1 | 0.87 |
| 92 | A092T | 0.73 | 1.10 | 1.14 | 0.98 | 0.9 | 0.96 |
| 92 | A092V | 0.30 | 1.40 | 1.31 | 1.73 | 0.6 | 0.50 |
| 93 | V093A | 0.31 | 1.09 | 1.32 | 1.22 | 0.9 | 0.68 |
| 93 | V093C | 0.61 | 1.02 | 1.24 | 1.00 | 0.9 | 0.86 |
| 93 | V093D | 0.21 | 2.38 | 3.40 | 2.01 | 1.1 | 0.67 |
| 93 | V093F | 0.22 | 1.32 | 2.23 | 1.46 | 1.0 | 0.61 |
| 93 | V093G | 0.23 | 0.60 | 1.00 | 0.61 | 1.1 | 0.18 |
| 93 | V093L | 0.58 | 1.39 | 1.12 | 1.01 | 0.8 | 0.98 |
| 93 | V093S | 0.29 | 0.17 | 0.35 | 0.27 | 0.9 | 0.12 |
| 93 | V093T | 0.33 | 1.46 | 1.70 | 1.37 | 1.1 | 1.04 |
| 94 | K094C | 0.22 | 1.33 | 1.37 | 1.05 | 1.0 | 0.17 |
| 94 | K094E | 0.21 | 0.66 | 0.92 | 0.67 | 0.8 | 0.06 |
| 94 | K094L | 0.30 | 0.26 | 0.32 | 0.28 | 1.1 | 0.06 |
| 94 | K094N | 0.28 | 0.69 | 3.31 | 0.56 | 0.8 | 0.08 |
| 94 | K094Q | 0.42 | 1.19 | 1.22 | 0.90 | 1.0 | 0.72 |
| 94 | K094R | 0.41 | 1.36 | 1.48 | 1.32 | 0.8 | 0.39 |
| 94 | K094S | 0.19 | 4.15 | 3.79 | 2.53 | 1.0 | 0.24 |
| 94 | K094V | 0.21 | 0.75 | 1.10 | 0.67 | 0.8 | 0.12 |
| 95 | V095A | 0.44 | 0.85 | 0.53 | 0.71 | 1.0 | 0.87 |
| 95 | V095C | 1.03 | 0.96 | 0.64 | 0.87 | 1.0 | 1.11 |
| 95 | V095G | 0.43 | 0.97 | 0.34 | 0.83 | 1.0 | 0.86 |
| 95 | V095S | 0.91 | 1.07 | 0.82 | 0.96 | 1.1 | 1.11 |
| 95 | V095T | 0.90 | 1.23 | 0.95 | 1.04 | 1.1 | 0.98 |
| 96 | L096F | 0.45 | 2.00 | 0.80 | 1.35 | 0.9 | 0.55 |
| 96 | L096I | 0.76 | 1.64 | 0.85 | 1.02 | 1.2 | 0.35 |
| 96 | L096M | 0.91 | 1.53 | 1.09 | 1.23 | 0.9 | 0.46 |
| 96 | L096V | 0.95 | 1.42 | 0.85 | 1.31 | 1.1 | 0.08 |
| 96 | L096W | 0.20 | 5.01 | 1.57 | 2.17 | 1.0 | 0.33 |
| 97 | G097A | 0.91 | 1.55 | 0.95 | 1.33 | 1.0 | 0.77 |
| 97 | G097C | 1.01 | 1.66 | 1.07 | 1.08 | 1.0 | 0.49 |
| 97 | G097D | 1.55 | 1.43 | 0.79 | 1.20 | 1.0 | 0.76 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 97 | G097E | 1.14 | 1.38 | 0.78 | 1.06 | 1.1 | 0.71 |
| 97 | G097F | 0.29 | 1.93 | 1.01 | 1.36 | 0.9 | 0.29 |
| 97 | G097H | 0.85 | 1.26 | 1.10 | 1.01 | 1.0 | 0.47 |
| 97 | G097K | 1.23 | 1.17 | 0.63 | 1.00 | 1.1 | 0.77 |
| 97 | G097L | 0.77 | 1.43 | 1.11 | 1.24 | 1.1 | 0.70 |
| 97 | G097M | 0.84 | 1.32 | 1.04 | 1.18 | 1.1 | 0.91 |
| 97 | G097P | 0.39 | 2.35 | 1.56 | 1.34 | 1.1 | 0.69 |
| 97 | G097Q | 0.97 | 1.36 | 0.77 | 1.03 | 1.2 | 0.98 |
| 97 | G097R | 1.22 | 0.97 | 0.64 | 1.02 | 1.0 | 0.92 |
| 97 | G097S | 0.98 | 1.45 | 1.12 | 1.07 | 0.9 | 0.95 |
| 97 | G097T | 1.02 | 1.23 | 0.93 | 1.10 | 1.0 | 1.09 |
| 97 | G097V | 0.54 | 1.56 | 1.09 | 1.22 | 1.0 | 0.95 |
| 97 | G097W | 0.23 | 2.95 | 1.54 | 1.49 | 1.0 | 0.34 |
| 97 | G097Y | 0.37 | 1.37 | 0.93 | 1.04 | 0.9 | 0.34 |
| 98 | A098C | 1.10 | 0.86 | 1.07 | 1.06 | 1.2 | 0.94 |
| 98 | A098D | 1.18 | 0.87 | 1.36 | 1.19 | 1.2 | 0.91 |
| 98 | A098E | 1.08 | 0.49 | 1.20 | 1.06 | 0.9 | 1.24 |
| 98 | A098F | 0.73 | 0.54 | 1.00 | 1.02 | 1.0 | 1.16 |
| 98 | A098G | 1.20 | 0.94 | 1.27 | 1.09 | 0.9 | 0.93 |
| 98 | A098H | 1.26 | 0.70 | 1.08 | 0.97 | 1.0 | 0.99 |
| 98 | A098I | 0.98 | 0.93 | 1.00 | 1.08 | 0.9 | 1.39 |
| 98 | A098L | 0.96 | 0.63 | 1.09 | 1.01 | 1.0 | 1.05 |
| 98 | A098P | 1.13 | 0.87 | 1.19 | 1.09 | 1.0 | 0.79 |
| 98 | A098Q | 0.89 | 0.76 | 1.13 | 1.05 | 1.0 | 1.09 |
| 98 | A098R | 1.22 | 0.70 | 1.00 | 1.11 | 1.0 | 0.95 |
| 98 | A098S | 1.20 | 0.85 | 1.12 | 1.12 | 0.8 | 1.01 |
| 98 | A098T | 0.98 | 0.74 | 1.09 | 0.99 | 1.0 | 1.36 |
| 98 | A098V | 0.99 | 0.91 | 1.17 | 0.97 | 0.9 | 1.70 |
| 98 | A098Y | 0.89 | 0.75 | 1.20 | 1.01 | 0.8 | 1.37 |
| 99 | D099A | 0.81 | 1.07 | 0.65 | 1.04 | 0.8 | 0.62 |
| 99 | D099C | 0.71 | 1.49 | 0.78 | 1.08 | 1.1 | 0.62 |
| 99 | D099E | 1.04 | 1.37 | 0.80 | 1.03 | 1.0 | 0.80 |
| 99 | D099F | 0.50 | 1.14 | 0.53 | 1.08 | 1.0 | 0.44 |
| 99 | D099H | 0.79 | 1.17 | 0.53 | 0.98 | 0.9 | 0.65 |
| 99 | D099I | 0.44 | 1.59 | 0.64 | 1.10 | 1.0 | 0.55 |
| 99 | D099K | 0.94 | 0.72 | 0.43 | 1.00 | 1.0 | 0.75 |
| 99 | D099L | 0.34 | 1.20 | 0.47 | 0.93 | 1.1 | 0.57 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 99 | D099M | 0.59 | 1.38 | 0.65 | 1.18 | 0.9 | 0.66 |
| 99 | D099N | 1.06 | 1.09 | 0.74 | 0.99 | 1.1 | 0.88 |
| 99 | D099P | 0.39 | 1.98 | 0.89 | 1.21 | 1.0 | 0.40 |
| 99 | D099Q | 0.98 | 1.08 | 0.77 | 1.15 | 1.0 | 0.65 |
| 99 | D099R | 0.87 | 0.54 | 0.42 | 0.86 | 1.1 | 0.83 |
| 99 | D099S | 0.87 | 1.00 | 0.60 | 0.97 | 0.9 | 0.94 |
| 99 | D099T | 0.70 | 1.08 | 0.70 | 1.10 | 0.9 | 0.97 |
| 99 | D099V | 0.43 | 1.71 | 0.75 | 1.08 | 0.9 | 0.61 |
| 99 | D099W | 0.58 | 1.22 | 0.57 | 0.89 | 0.8 | 0.88 |
| 99 | D099Y | 0.50 | 1.43 | 0.60 | 1.07 | 0.9 | 0.60 |
| 100 | G 100A | 0.94 | 0.95 | 1.13 | 0.96 | 1.0 | 0.35 |
| 100 | G 100D | 0.77 | 1.41 | 1.35 | 1.08 | 0.9 | 0.34 |
| 100 | G100E | 0.54 | 1.66 | 1.46 | 1.25 | 1.0 | 0.30 |
| 100 | G100F | 0.53 | 1.04 | 1.32 | 1.22 | 0.9 | 0.10 |
| 100 | G100H | 0.55 | 1.41 | 1.18 | 1.05 | 0.9 | 0.28 |
| 100 | G100K | 0.65 | 1.46 | 1.00 | 1.22 | 0.9 | 0.31 |
| 100 | G100M | 1.13 | 1.42 | 1.16 | 1.20 | 0.8 | 0.17 |
| 100 | G100N | 1.09 | 1.25 | 1.29 | 1.31 | 1.0 | 0.41 |
| 100 | G100Q | 0.66 | 1.57 | 1.18 | 1.38 | 1.0 | 0.35 |
| 100 | G100R | 0.68 | 1.09 | 0.89 | 1.03 | 0.9 | 0.29 |
| 100 | G100S | 0.79 | 1.28 | 1.35 | 1.15 | 0.9 | 0.35 |
| 100 | G100T | 0.34 | 2.17 | 1.64 | 1.46 | 1.0 | 0.18 |
| 100 | G100V | 0.31 | 1.98 | 1.55 | 1.39 | 1.0 | 0.07 |
| 100 | G100Y | 0.49 | 1.58 | 1.27 | 1.05 | 1.0 | 0.20 |
| 101 | S101A | 1.27 | 1.38 | 1.00 | 1.09 | 1.1 | 1.08 |
| 101 | S101C | 1.24 | 1.14 | 0.67 | 0.90 | 1.2 | 0.61 |
| 101 | S101E | 1.35 | 1.52 | 1.19 | 1.14 | 1.1 | 0.71 |
| 101 | S101F | 0.96 | 1.02 | 0.73 | 0.97 | 1.0 | 1.13 |
| 101 | S101G | 0.38 | 1.30 | 0.46 | 0.67 | 1.1 | 0.21 |
| 101 | S101I | 1.01 | 1.22 | 0.75 | 0.86 | 1.2 | 0.91 |
| 101 | S101K | 1.28 | 1.15 | 0.62 | 1.00 | 1.1 | 0.99 |
| 101 | S101L | 1.10 | 1.21 | 0.80 | 1.00 | 1.0 | 1.21 |
| 101 | S101M | 1.01 | 1.22 | 0.79 | 1.08 | 1.1 | 1.21 |
| 101 | S101N | 1.24 | 1.46 | 0.99 | 1.10 | 1.1 | 1.03 |
| 101 | S101P | 0.25 | 3.64 | 1.39 | 1.83 | 1.1 | 0.30 |
| 101 | S101Q | 0.59 | 1.56 | 0.79 | 1.05 | 1.0 | 1.24 |
| 101 | S101R | 1.20 | 1.04 | 0.65 | 0.90 | 1.0 | 0.94 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 101 | S101T | 0.94 | 1.34 | 0.68 | 1.16 | 1.0 | 1.12 |
| 101 | S101V | 0.92 | 1.39 | 0.74 | 1.18 | 0.9 | 1.19 |
| 101 | S101Y | 0.72 | 1.04 | 0.48 | 0.92 | 1.0 | 1.54 |
| 102 | G102A | 1.12 | 1.46 | 1.31 | 1.19 | 1.0 | 0.31 |
| 102 | G102S | 0.77 | 1.61 | 1.10 | 1.04 | 1.1 | 0.11 |
| 103 | Q103A | 1.09 | 0.93 | 0.93 | 0.89 | 1.0 | 0.92 |
| 103 | Q103C | 1.19 | 1.10 | 1.05 | 0.97 | 1.0 | 0.63 |
| 103 | Q103E | 1.21 | 1.38 | 1.15 | 1.05 | 1.0 | 0.64 |
| 103 | Q103F | 1.02 | 0.82 | 0.57 | 0.83 | 0.9 | 0.91 |
| 103 | Q103G | 0.39 | 1.48 | 1.06 | 1.34 | 1.1 | 0.43 |
| 103 | Q103H | 0.89 | 1.35 | 0.89 | 0.93 | 0.9 | 0.65 |
| 103 | Q103I | 1.06 | 0.88 | 0.89 | 0.87 | 1.1 | 0.82 |
| 103 | Q103K | 0.17 | 2.63 | 1.36 | 1.25 | 1.0 | 0.45 |
| 103 | Q103L | 0.44 | 0.87 | 0.68 | 0.81 | 1.1 | 0.93 |
| 103 | Q103M | 0.89 | 1.06 | 0.76 | 0.99 | 0.9 | 1.01 |
| 103 | Q103N | 1.44 | 1.44 | 1.23 | 0.88 | 1.0 | 0.74 |
| 103 | Q103R | 1.09 | 0.47 | 0.57 | 0.88 | 1.1 | 1.04 |
| 103 | Q103S | 1.21 | 1.03 | 0.96 | 0.91 | 1.0 | 1.07 |
| 103 | Q103T | 0.84 | 1.01 | 0.94 | 0.95 | 1.0 | 0.89 |
| 103 | Q103V | 0.94 | 1.13 | 1.03 | 0.97 | 1.0 | 0.75 |
| 103 | Q103W | 0.98 | 0.65 | 0.63 | 0.73 | 1.1 | 0.76 |
| 104 | Y104C | 0.34 | 1.24 | 1.33 | 1.31 | 1.1 | 0.06 |
| 104 | Y104F | 0.90 | 0.88 | 0.78 | 1.03 | 1.0 | 0.67 |
| 104 | Y104H | 0.75 | 0.95 | 1.78 | 0.95 | 1.1 | 0.30 |
| 104 | Y104I | 0.30 | 1.10 | 1.10 | 1.48 | 1.3 | 0.08 |
| 104 | Y104M | 0.31 | 1.53 | 1.50 | 1.27 | 1.2 | 0.08 |
| 104 | Y104N | 0.46 | 1.42 | 1.78 | 1.04 | 1.1 | 0.10 |
| 104 | Y104T | 0.42 | 1.33 | 1.69 | 1.35 | 2.0 | 0.08 |
| 104 | Y104W | 0.62 | 0.82 | 0.89 | 1.17 | 1.2 | 1.38 |
| 105 | S105A | 0.67 | 1.06 | 0.97 | 1.01 | 1.0 | 1.16 |
| 105 | S105C | 0.32 | 0.91 | 1.10 | 0.81 | 1.1 | 0.52 |
| 105 | S105D | 0.39 | 1.33 | 1.03 | 1.21 | 1.1 | 0.90 |
| 105 | S105E | 0.54 | 0.98 | 0.79 | 0.93 | 1.2 | 0.76 |
| 105 | S105G | 0.68 | 1.12 | 0.98 | 0.98 | 0.9 | 0.93 |
| 105 | S105I | 0.19 | 1.60 | 1.06 | 0.72 | 1.0 | 0.26 |
| 105 | S105K | 0.21 | 0.13 | 0.10 | 0.13 | 1.2 | 0.09 |
| 105 | S105L | 0.17 | 0.60 | 9.45 | 0.53 | 1.2 | 0.12 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 105 | S105M | 0.18 | 1.09 | 1.67 | 1.11 | 0.9 | 0.19 |
| 105 | S105N | 0.32 | 1.10 | 0.97 | 1.17 | 1.1 | 0.81 |
| 105 | S105Q | 0.24 | 0.70 | 0.76 | 0.72 | 1.0 | 0.40 |
| 105 | S105R | 0.16 | 3.02 | 3.37 | 0.95 | 1.2 | 0.19 |
| 105 | S105T | 0.87 | 1.19 | 1.11 | 1.21 | 1.0 | 1.07 |
| 105 | S105V | 0.18 | 2.82 | 3.06 | 2.81 | 1.0 | 0.75 |
| 106 | W106A | 0.38 | 1.42 | 1.15 | 1.06 | 0.9 | 0.36 |
| 106 | W106C | 0.37 | 1.38 | 1.10 | 0.99 | 0.9 | 0.23 |
| 106 | W106E | 0.39 | 1.73 | 1.34 | 1.15 | 1.1 | 0.35 |
| 106 | W106F | 0.81 | 1.33 | 0.80 | 1.29 | 1.0 | 0.40 |
| 106 | W106G | 0.24 | 1.40 | 1.28 | 1.21 | 0.9 | 0.12 |
| 106 | W106H | 0.78 | 1.02 | 0.97 | 1.16 | 0.9 | 0.72 |
| 106 | W106I | 0.30 | 1.51 | 1.13 | 1.34 | 1.1 | 0.40 |
| 106 | W106L | 0.31 | 1.56 | 1.11 | 1.40 | 1.1 | 0.26 |
| 106 | W106M | 0.41 | 1.36 | 1.11 | 1.13 | 0.9 | 0.30 |
| 106 | W106N | 0.51 | 1.16 | 1.02 | 1.23 | 1.0 | 0.51 |
| 106 | W106R | 0.34 | 0.94 | 1.24 | 1.26 | 1.0 | 0.34 |
| 106 | W106S | 0.37 | 1.41 | 0.93 | 1.19 | 2.0 | 0.18 |
| 106 | W106T | 0.39 | 1.41 | 1.15 | 1.25 | 0.9 | 0.30 |
| 106 | W106V | 0.33 | 1.48 | 1.20 | 1.19 | 0.9 | 0.49 |
| 106 | W106Y | 0.90 | 1.19 | 1.05 | 1.22 | 0.9 | 0.43 |
| 107 | I107E | 0.47 | 1.07 | 1.99 | 1.00 | 1.0 | 0.95 |
| 107 | I107F | 0.54 | 0.87 | 0.79 | 1.16 | 1.0 | 0.06 |
| 107 | I107L | 0.84 | 0.57 | 0.33 | 0.48 | 1.0 | 0.95 |
| 107 | I107M | 0.86 | 1.18 | 2.50 | 0.93 | 1.0 | 0.17 |
| 107 | I107R | 0.23 | 2.33 | 2.34 | 1.86 | 1.2 | 0.06 |
| 107 | I107S | 0.46 | 1.41 | 0.88 | 1.22 | 1.0 | 0.08 |
| 107 | I107T | 0.70 | 1.35 | 2.33 | 0.93 | 1.0 | 0.13 |
| 107 | I107V | 0.82 | 0.71 | 0.73 | 0.78 | 0.9 | 1.33 |
| 108 | I108A | 0.38 | 1.15 | 1.22 | 1.17 | 0.8 | 0.70 |
| 108 | I108C | 0.79 | 1.09 | 1.19 | 1.00 | 0.9 | 0.76 |
| 108 | I108E | 0.21 | 0.23 | 1.19 | 0.59 | 0.9 | 0.13 |
| 108 | I108L | 0.73 | 1.29 | 1.29 | 1.05 | 1.0 | 0.41 |
| 108 | I108M | 0.80 | 1.10 | 1.21 | 1.10 | 0.7 | 0.73 |
| 108 | I108Q | 0.20 | 0.33 | 0.24 | 0.07 | 0.7 | 0.06 |
| 108 | I108S | 0.16 | 2.38 | 8.60 | 1.30 | 0.9 | 0.13 |
| 108 | I108T | 0.30 | 1.75 | 1.66 | 1.51 | 0.9 | 0.77 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 108 | I108V | 1.11 | 1.29 | 1.09 | 1.16 | 1.0 | 0.93 |
| 109 | N109A | 0.62 | 1.05 | 1.01 | 1.09 | 1.1 | 1.03 |
| 109 | N109C | 0.90 | 0.82 | 0.73 | 0.89 | 1.1 | 0.85 |
| 109 | N109E | 0.66 | 0.94 | 1.01 | 1.05 | 1.1 | 1.09 |
| 109 | N109F | 0.52 | 1.17 | 0.79 | 1.20 | 1.1 | 0.97 |
| 109 | N109G | 0.66 | 1.13 | 0.93 | 1.01 | 1.1 | 1.12 |
| 109 | N109H | 0.93 | 1.01 | 0.64 | 1.04 | 1.1 | 0.92 |
| 109 | N109L | 0.81 | 0.80 | 0.99 | 1.18 | 1.1 | 1.17 |
| 109 | N109P | 0.59 | 1.13 | 0.94 | 1.20 | 1.0 | 0.79 |
| 109 | N109Q | 1.04 | 0.63 | 0.55 | 0.99 | 1.2 | 0.93 |
| 109 | N109R | 0.71 | 0.58 | 0.57 | 1.03 | 1.0 | 1.07 |
| 109 | N109S | 1.03 | 0.93 | 0.90 | 1.13 | 1.0 | 1.05 |
| 109 | N109T | 0.94 | 1.26 | 0.82 | 1.02 | 0.9 | 1.09 |
| 109 | N109V | 0.59 | 0.91 | 0.91 | 1.17 | 1.0 | 1.08 |
| 109 | N109W | 0.63 | 0.92 | 0.76 | 1.15 | 0.9 | 0.92 |
| 109 | N109Y | 0.54 | 0.88 | 0.89 | 1.08 | 0.9 | 0.96 |
| 110 | G110A | 0.79 | 1.28 | 0.75 | 1.05 | 0.9 | 0.78 |
| 110 | G110S | 0.25 | 2.17 | 1.37 | 1.69 | 0.9 | 0.51 |
| 110 | G110T | 0.20 | 4.57 | 3.95 | 2.82 | 0.9 | 0.53 |
| 111 | I111A | 0.24 | 3.80 | 2.39 | 1.92 | 0.9 | 0.30 |
| 111 | I111C | 0.36 | 1.88 | 1.45 | 1.48 | 0.9 | 0.88 |
| 111 | I111L | 1.00 | 1.30 | 0.96 | 1.10 | 1.0 | 0.74 |
| 111 | I111M | 0.80 | 1.37 | 0.98 | 1.04 | 1.1 | 0.74 |
| 111 | I111T | 0.31 | 3.10 | 1.56 | 1.67 | 1.0 | 0.85 |
| 111 | I111V | 0.73 | 1.59 | 0.98 | 1.32 | 1.0 | 0.83 |
| 112 | E112A | 0.33 | 1.05 | 0.84 | 1.31 | 1.1 | 0.77 |
| 112 | E112C | 0.49 | 0.40 | 0.28 | 0.28 | 1.1 | 0.20 |
| 112 | E112D | 0.97 | 1.20 | 0.97 | 1.11 | 1.0 | 0.91 |
| 112 | E112G | 0.37 | 0.87 | 0.64 | 0.90 | 1.0 | 0.56 |
| 112 | E112I | 0.22 | 7.24 | 2.74 | 2.71 | 1.1 | 0.63 |
| 112 | E112L | 0.21 | 19.65 | 11.59 | 3.83 | 1.1 | 0.57 |
| 112 | E112M | 0.35 | 0.98 | 0.74 | 1.07 | 1.0 | 0.71 |
| 112 | E112N | 0.38 | 1.24 | 0.85 | 1.19 | 1.0 | 0.93 |
| 112 | E112Q | 0.39 | 1.25 | 1.01 | 1.27 | 1.0 | 0.97 |
| 112 | E112S | 0.38 | 1.22 | 0.98 | 1.05 | 1.0 | 0.85 |
| 112 | E112T | 0.25 | 2.89 | 2.14 | 1.98 | 1.0 | 0.90 |
| 113 | W113C | 0.19 | 0.36 | 1.25 | 1.25 | 1.0 | 0.19 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 113 | W113D | 0.18 | 0.52 | 1.67 | 0.60 | 0.9 | 0.16 |
| 113 | W113E | 0.19 | 0.26 | 1.58 | 0.31 | 0.9 | 0.16 |
| 113 | W113F | 0.50 | 0.97 | 1.02 | 1.03 | 0.9 | 0.96 |
| 113 | W113H | 0.22 | 1.45 | 1.69 | 1.49 | 1.1 | 0.57 |
| 113 | W113M | 0.19 | 0.40 | 1.19 | 0.61 | 0.9 | 0.18 |
| 113 | W113N | 0.17 | 0.74 | 2.31 | 0.88 | 0.9 | 0.22 |
| 113 | W113T | 0.17 | 0.12 | 1.00 | 0.51 | 1.1 | 0.10 |
| 113 | W113Y | 0.91 | 0.82 | 1.25 | 1.02 | 1.1 | 0.89 |
| 114 | A114C | 0.87 | 1.18 | 1.38 | 1.17 | 1.0 | 1.19 |
| 114 | A114G | 0.72 | 1.15 | 1.41 | 1.31 | 1.1 | 1.14 |
| 114 | A114I | 0.19 | 3.35 | 2.63 | 2.54 | 0.9 | 0.42 |
| 114 | A114S | 0.33 | 1.15 | 1.39 | 1.63 | 1.0 | 0.98 |
| 114 | A114T | 0.59 | 1.03 | 1.49 | 1.53 | 0.9 | 1.46 |
| 114 | A114V | 0.31 | 1.57 | 2.18 | 1.56 | 0.9 | 0.97 |
| 115 | I115A | 0.40 | 1.58 | 0.99 | 1.09 | 0.9 | 0.79 |
| 115 | I115C | 0.69 | 1.23 | 0.81 | 1.25 | 1.0 | 1.10 |
| 115 | I115E | 0.28 | 2.29 | 1.67 | 1.56 | 0.9 | 0.69 |
| 115 | I115F | 0.30 | 1.99 | 1.22 | 1.37 | 0.9 | 0.61 |
| 115 | I115H | 0.24 | 4.85 | 2.46 | 2.39 | 0.9 | 0.52 |
| 115 | I115L | 0.83 | 1.04 | 0.96 | 1.08 | 1.1 | 1.06 |
| 115 | I115M | 0.89 | 1.46 | 1.03 | 1.09 | 1.0 | 0.94 |
| 115 | I115N | 0.21 | 8.31 | 8.40 | 2.63 | 1.0 | 0.37 |
| 115 | I115Q | 0.58 | 1.30 | 0.80 | 1.24 | 1.0 | 1.00 |
| 115 | I115R | 0.38 | 1.37 | 1.00 | 1.21 | 1.0 | 0.86 |
| 115 | I115S | 0.24 | 4.20 | 2.44 | 2.22 | 0.9 | 0.70 |
| 115 | I115T | 0.59 | 1.46 | 0.89 | 1.08 | 0.9 | 0.91 |
| 115 | I115V | 0.84 | 1.44 | 0.99 | 1.25 | 0.9 | 1.58 |
| 115 | I115Y | 0.24 | 4.81 | 4.07 | 2.48 | 0.9 | 0.79 |
| 116 | A116C | 1.15 | 0.85 | 1.06 | 0.90 | 1.0 | 0.95 |
| 116 | A116D | 1.24 | 1.02 | 0.81 | 1.03 | 1.0 | 1.11 |
| 116 | A116E | 1.16 | 1.21 | 1.09 | 0.89 | 1.0 | 1.08 |
| 116 | A116F | 0.95 | 1.06 | 0.92 | 0.94 | 1.0 | 1.01 |
| 116 | A116G | 0.66 | 0.94 | 1.01 | 0.97 | 1.0 | 1.08 |
| 116 | A116H | 1.07 | 1.09 | 0.92 | 1.13 | 1.0 | 1.30 |
| 116 | A116I | 0.95 | 0.82 | 1.04 | 1.13 | 1.0 | 1.05 |
| 116 | A116K | 1.18 | 0.74 | 0.79 | 1.13 | 1.0 | 0.94 |
| 116 | A116L | 0.82 | 1.05 | 0.89 | 0.83 | 1.0 | 0.98 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 116 | A116M | 1.11 | 0.86 | 1.14 | 0.85 | 1.0 | 0.98 |
| 116 | A116N | 0.99 | 0.97 | 0.88 | 1.10 | 1.0 | 1.15 |
| 116 | A116Q | 1.07 | 0.85 | 0.71 | 1.03 | 1.0 | 1.15 |
| 116 | A116R | 1.15 | 0.86 | 0.73 | 1.09 | 1.0 | 1.15 |
| 116 | A116S | 1.14 | 1.07 | 0.98 | 1.17 | 0.9 | 1.11 |
| 116 | A116T | 1.03 | 1.01 | 1.12 | 1.06 | 0.9 | 1.17 |
| 116 | A116V | 0.88 | 1.03 | 1.03 | 0.96 | 1.0 | 1.09 |
| 116 | A116W | 0.86 | 1.24 | 0.71 | 0.97 | 1.0 | 1.17 |
| 116 | A116Y | 0.88 | 0.89 | 1.03 | 0.93 | 0.9 | 1.19 |
| 117 | N117C | 0.60 | 1.00 | 0.98 | 1.07 | 1.0 | 1.06 |
| 117 | N117E | 0.52 | 0.97 | 1.03 | 1.06 | 1.0 | 1.18 |
| 117 | N117F | 0.40 | 1.04 | 1.21 | 1.17 | 0.9 | 1.33 |
| 117 | N117G | 0.67 | 1.18 | 0.85 | 1.29 | 1.1 | 0.96 |
| 117 | N117K | 0.22 | 1.91 | 1.91 | 1.80 | 1.0 | 0.88 |
| 117 | N117L | 0.30 | 1.13 | 1.22 | 1.39 | 1.0 | 1.13 |
| 117 | N117M | 0.57 | 1.09 | 1.21 | 1.12 | 1.0 | 1.24 |
| 117 | N117Q | 1.04 | 0.69 | 0.95 | 0.99 | 1.1 | 1.10 |
| 117 | N117R | 0.80 | 0.98 | 0.78 | 0.98 | 1.0 | 0.90 |
| 117 | N117S | 0.53 | 1.18 | 1.24 | 1.06 | 1.3 | 1.14 |
| 117 | N117T | 0.80 | 1.05 | 1.07 | 1.15 | 1.0 | 1.13 |
| 117 | N117V | 0.17 | 5.36 | 4.11 | 2.10 | 1.0 | 0.46 |
| 117 | N117W | 0.25 | 2.09 | 1.77 | 1.63 | 1.0 | 1.05 |
| 117 | N117Y | 0.45 | 0.99 | 0.89 | 1.22 | 1.0 | 0.98 |
| 118 | N118A | 0.71 | 0.58 | 0.84 | 0.95 | 1.0 | 1.14 |
| 118 | N118C | 0.74 | 0.61 | 1.01 | 0.63 | 1.1 | 0.94 |
| 118 | N118D | 1.12 | 0.67 | 1.05 | 0.86 | 1.2 | 1.01 |
| 118 | N118E | 0.88 | 0.71 | 0.70 | 0.69 | 1.1 | 1.16 |
| 118 | N118F | 0.34 | 1.02 | 1.31 | 0.93 | 1.0 | 1.07 |
| 118 | N118G | 1.07 | 0.83 | 0.50 | 0.79 | 0.9 | 0.92 |
| 118 | N118H | 1.18 | 0.75 | 0.88 | 0.92 | 1.0 | 1.07 |
| 118 | N118I | 0.17 | 4.85 | 5.90 | 1.96 | 1.1 | 0.62 |
| 118 | N118K | 1.17 | 0.80 | 0.81 | 0.81 | 1.1 | 0.95 |
| 118 | N118L | 0.23 | 1.45 | 1.85 | 1.32 | 1.0 | 1.13 |
| 118 | N118M | 0.49 | 0.87 | 0.99 | 1.06 | 1.0 | 1.17 |
| 118 | N118Q | 1.03 | 0.62 | 1.12 | 0.90 | 1.1 | 1.09 |
| 118 | N118R | 1.35 | 0.82 | 0.62 | 0.80 | 1.0 | 1.01 |
| 118 | N118S | 0.89 | 0.77 | 0.81 | 0.76 | 0.9 | 1.07 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 118 | N118T | 0.64 | 0.72 | 0.86 | 1.08 | 0.9 | 1.21 |
| 118 | N118V | 0.23 | 1.35 | 1.92 | 1.53 | 1.0 | 0.99 |
| 118 | N118W | 0.27 | 1.16 | 1.36 | 1.41 | 1.1 | 1.03 |
| 118 | N118Y | 0.84 | 0.98 | 0.72 | 0.70 | 1.0 | 1.13 |
| 119 | M119A | 0.75 | 1.01 | 1.16 | 1.01 | 0.8 | 0.85 |
| 119 | M119C | 0.86 | 1.01 | 1.16 | 0.95 | 1.0 | 0.99 |
| 119 | M119F | 0.29 | 1.64 | 1.87 | 1.60 | 1.3 | 0.78 |
| 119 | M119G | 0.26 | 0.33 | 0.48 | 0.28 | 1.0 | 0.11 |
| 119 | M119H | 0.30 | 1.19 | 1.46 | 1.22 | 1.1 | 0.67 |
| 119 | M119I | 0.74 | 1.20 | 1.16 | 0.94 | 1.1 | 0.97 |
| 119 | M119L | 0.65 | 1.17 | 1.19 | 0.82 | 1.0 | 0.98 |
| 119 | M119N | 0.23 | 1.95 | 2.59 | 1.79 | 1.1 | 0.83 |
| 119 | M119S | 0.53 | 1.19 | 1.30 | 0.87 | 0.8 | 1.04 |
| 119 | M119T | 0.46 | 1.25 | 1.32 | 1.03 | 1.0 | 1.43 |
| 119 | M119V | 0.75 | 0.95 | 1.13 | 0.91 | 0.7 | 1.14 |
| 120 | D120A | 1.01 | 1.11 | 0.84 | 0.80 | 1.0 | 0.85 |
| 120 | D120C | 0.67 | 0.84 | 0.95 | 0.76 | 1.0 | 0.92 |
| 120 | D120E | 1.04 | 0.60 | 0.87 | 1.04 | 1.1 | 0.99 |
| 120 | D120F | 0.24 | 0.50 | 1.07 | 0.78 | 1.0 | 0.50 |
| 120 | D120G | 1.00 | 0.99 | 1.04 | 0.97 | 0.8 | 0.92 |
| 120 | D120H | 1.13 | 1.07 | 1.16 | 0.88 | 1.1 | 0.93 |
| 120 | D120I | 0.20 | 0.92 | 1.69 | 1.26 | 1.0 | 0.55 |
| 120 | D120K | 1.15 | 0.88 | 1.01 | 0.95 | 1.1 | 0.81 |
| 120 | D120L | 0.33 | 0.65 | 1.25 | 1.12 | 1.1 | 0.88 |
| 120 | D120M | 0.73 | 0.87 | 0.97 | 0.95 | 0.7 | 0.93 |
| 120 | D120N | 1.11 | 1.01 | 1.00 | 0.92 | 1.1 | 0.90 |
| 120 | D120P | 0.22 | 1.12 | 1.61 | 1.14 | 1.1 | 0.53 |
| 120 | D120Q | 1.05 | 1.21 | 1.09 | 0.96 | 1.1 | 0.87 |
| 120 | D120R | 1.03 | 0.86 | 0.97 | 1.10 | 0.9 | 0.93 |
| 120 | D120S | 1.01 | 0.94 | 1.16 | 0.86 | 1.0 | 0.97 |
| 120 | D120T | 0.77 | 1.08 | 1.09 | 1.00 | 1.0 | 1.06 |
| 120 | D120V | 0.32 | 1.13 | 1.21 | 0.64 | 1.1 | 0.81 |
| 120 | D120W | 0.32 | 1.10 | 1.44 | 1.19 | 1.1 | 0.95 |
| 120 | D120Y | 0.22 | 1.30 | 1.73 | 1.29 | 1.0 | 0.70 |
| 121 | V121C | 0.76 | 0.98 | 0.99 | 0.78 | 1.1 | 0.92 |
| 121 | V121E | 0.19 | 0.45 | 1.16 | 0.85 | 0.7 | 0.21 |
| 121 | V121F | 0.18 | 0.12 | 1.14 | 0.47 | 0.7 | 0.12 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 121 | V121I | 1.15 | 0.83 | 0.88 | 0.81 | 1.0 | 0.89 |
| 121 | V121L | 0.81 | 0.95 | 0.91 | 0.88 | 0.8 | 0.94 |
| 121 | V121T | 0.26 | 1.28 | 1.66 | 1.24 | 1.1 | 0.85 |
| 122 | I122A | 0.92 | 1.06 | 1.04 | 1.00 | 0.8 | 0.88 |
| 122 | I122C | 0.78 | 1.17 | 1.22 | 1.09 | 0.8 | 0.98 |
| 122 | I122F | 0.37 | 1.16 | 1.45 | 1.19 | 1.4 | 0.66 |
| 122 | I122L | 0.73 | 0.99 | 1.28 | 1.09 | 0.8 | 0.97 |
| 122 | I122M | 0.93 | 1.17 | 1.05 | 1.23 | 0.8 | 0.96 |
| 122 | I122S | 0.22 | 6.46 | 6.12 | 2.56 | 1.9 | 0.43 |
| 122 | I122T | 0.34 | 1.78 | 1.79 | 1.13 | 1.4 | 0.90 |
| 122 | I122V | 0.80 | 1.14 | 1.39 | 1.13 | 0.7 | 1.09 |
| 123 | N123C | 0.95 | 1.53 | 1.35 | 1.43 | 0.5 | 0.09 |
| 123 | N123G | 0.47 | 1.81 | 1.59 | 1.34 | 0.4 | 0.13 |
| 123 | N123Q | 0.87 | 1.22 | 1.30 | 1.03 | 0.7 | 0.09 |
| 123 | N123S | 0.92 | 1.31 | 1.15 | 1.08 | 0.2 | 0.09 |
| 123 | N123T | 0.94 | 1.20 | 1.41 | 0.93 | 0.4 | 0.13 |
| 124 | M124A | 0.61 | 1.44 | 1.02 | 0.97 | 1.0 | 0.69 |
| 124 | M124C | 0.26 | 0.86 | 0.54 | 0.63 | 1.3 | 0.07 |
| 124 | M124F | 1.13 | 1.02 | 0.86 | 1.06 | 0.9 | 0.34 |
| 124 | M124I | 1.36 | 1.12 | 1.08 | 1.12 | 1.0 | 0.34 |
| 124 | M124L | 0.86 | 0.69 | 1.01 | 1.03 | 1.2 | 0.92 |
| 124 | M124S | 0.28 | 2.79 | 1.44 | 1.52 | 0.9 | 0.12 |
| 124 | M124T | 0.49 | 1.82 | 1.32 | 1.35 | 0.9 | 0.23 |
| 124 | M124V | 1.07 | 1.37 | 1.15 | 1.06 | 0.9 | 0.31 |
| 125 | S125A | 0.92 | 1.90 | 1.40 | 1.17 | 1.1 | 0.06 |
| 126 | L126I | 1.11 | 1.17 | 1.03 | 1.17 | 1.1 | 0.11 |
| 126 | L126V | 1.09 | 1.26 | 1.34 | 1.32 | 1.0 | 0.08 |
| 126 | L126W | 0.89 | 0.71 | 0.79 | 0.78 | 0.9 | 0.06 |
| 128 | G128A | 1.21 | 1.24 | 1.28 | 1.21 | 1.0 | 0.16 |
| 128 | G128S | 1.35 | 1.17 | 1.12 | 1.09 | 1.2 | 0.26 |
| 129 | P129A | 1.04 | 1.02 | 0.98 | 0.99 | 0.7 | 0.80 |
| 129 | P129C | 1.37 | 1.09 | 1.11 | 0.91 | 0.9 | 0.56 |
| 129 | P129D | 1.48 | 1.03 | 1.08 | 0.84 | 1.1 | 0.67 |
| 129 | P129E | 1.42 | 1.24 | 1.04 | 1.10 | 1.0 | 0.87 |
| 129 | P129F | 0.65 | 1.10 | 0.71 | 1.00 | 0.7 | 1.27 |
| 129 | P129G | 0.78 | 0.82 | 0.60 | 0.81 | 0.8 | 0.56 |
| 129 | P129H | 0.66 | 0.92 | 0.73 | 0.84 | 0.9 | 0.81 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 129 | P129I | 1.14 | 0.95 | 0.99 | 1.11 | 0.9 | 0.40 |
| 129 | P129K | 1.13 | 0.72 | 0.64 | 1.01 | 0.9 | 1.22 |
| 129 | P129L | 1.12 | 1.13 | 1.21 | 1.06 | 0.9 | 0.53 |
| 129 | P129M | 1.20 | 1.08 | 0.92 | 1.20 | 0.9 | 0.88 |
| 129 | P129N | 0.90 | 1.00 | 1.04 | 0.98 | 0.9 | 0.85 |
| 129 | P129Q | 1.10 | 1.22 | 0.90 | 1.01 | 1.0 | 0.98 |
| 129 | P129R | 0.74 | 0.73 | 0.75 | 0.87 | 0.9 | 1.01 |
| 129 | P129S | 1.07 | 0.99 | 0.87 | 0.98 | 0.9 | 0.84 |
| 129 | P129T | 1.13 | 1.29 | 1.17 | 1.09 | 0.9 | 1.06 |
| 129 | P129V | 0.72 | 1.41 | 1.26 | 1.09 | 0.8 | 0.75 |
| 129 | P129W | 0.38 | 0.97 | 0.79 | 1.18 | 0.4 | 1.18 |
| 129 | P129Y | 0.71 | 1.01 | 0.90 | 1.04 | 0.8 | 1.56 |
| 130 | S 130A | 1.14 | 0.92 | 0.76 | 0.86 | 1.0 | 0.92 |
| 130 | S130C | 1.24 | 0.75 | 0.95 | 0.66 | 1.1 | 0.78 |
| 130 | S130E | 1.36 | 0.73 | 1.29 | 1.03 | 1.1 | 0.88 |
| 130 | S130F | 1.17 | 0.89 | 0.79 | 0.84 | 1.0 | 1.02 |
| 130 | S130G | 0.93 | 0.95 | 0.99 | 0.98 | 0.9 | 0.80 |
| 130 | S130H | 1.33 | 0.76 | 0.97 | 1.05 | 1.1 | 1.03 |
| 130 | S130I | 1.22 | 0.82 | 0.97 | 0.91 | 1.1 | 0.95 |
| 130 | S 130K | 1.27 | 0.63 | 0.70 | 0.67 | 1.1 | 1.12 |
| 130 | S130L | 1.04 | 0.90 | 0.79 | 0.80 | 1.1 | 0.98 |
| 130 | S 130M | 1.19 | 0.97 | 0.91 | 0.89 | 0.9 | 0.92 |
| 130 | S130P | 0.60 | 1.32 | 1.32 | 1.00 | 0.7 | 0.07 |
| 130 | S130Q | 1.19 | 0.98 | 0.95 | 0.93 | 1.1 | 1.06 |
| 130 | S130R | 1.12 | 0.47 | 0.63 | 0.77 | 1.1 | 1.11 |
| 130 | S130T | 1.16 | 1.08 | 1.10 | 0.95 | 0.9 | 1.19 |
| 130 | S130V | 1.13 | 0.98 | 0.96 | 0.91 | 1.0 | 1.07 |
| 130 | S130W | 0.73 | 0.60 | 0.81 | 0.86 | 0.8 | 0.89 |
| 130 | S130Y | 1.24 | 0.73 | 0.94 | 0.95 | 1.0 | 1.04 |
| 131 | G131A | 1.21 | 0.53 | 1.00 | 0.84 | 1.1 | 1.02 |
| 131 | G131C | 0.55 | 0.59 | 0.82 | 0.83 | 0.6 | 0.87 |
| 131 | G131D | 0.93 | 0.50 | 0.80 | 0.78 | 0.9 | 1.32 |
| 131 | G131F | 0.23 | 0.42 | 0.55 | 0.67 | 0.2 | 0.43 |
| 131 | G131K | 1.00 | 0.48 | 1.00 | 0.89 | 1.0 | 1.39 |
| 131 | G131M | 0.53 | 0.47 | 0.89 | 0.75 | 0.6 | 0.90 |
| 131 | G131N | 0.46 | 0.23 | 0.49 | 0.54 | 0.8 | 1.11 |
| 131 | G131P | 0.22 | 0.77 | 1.29 | 1.21 | 0.1 | 0.50 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 131 | G131Q | 0.28 | 0.49 | 1.15 | 0.95 | 0.3 | 0.70 |
| 131 | G131R | 0.19 | 0.97 | 2.04 | 1.64 | 0.1 | 0.59 |
| 131 | G131S | 0.21 | 0.12 | 0.47 | 0.83 | 0.2 | 0.45 |
| 131 | G131T | 0.86 | 0.52 | 1.16 | 0.89 | 1.0 | 1.03 |
| 131 | G131V | 0.51 | 0.50 | 0.83 | 0.94 | 0.7 | 0.99 |
| 131 | G131Y | 0.30 | 0.26 | 0.40 | 0.71 | 0.9 | 1.04 |
| 132 | S132A | 0.60 | 1.01 | 1.03 | 1.03 | 0.5 | 0.77 |
| 132 | S132C | 1.25 | 1.06 | 1.10 | 0.83 | 1.0 | 0.64 |
| 132 | S132E | 0.33 | 1.04 | 1.40 | 1.01 | 0.9 | 0.50 |
| 132 | S132G | 0.83 | 0.83 | 0.14 | 0.94 | 1.0 | 0.41 |
| 132 | S132I | 0.25 | 1.62 | 1.82 | 1.25 | 0.8 | 0.57 |
| 132 | S132K | 0.19 | 0.09 | 0.60 | 0.16 | 0.7 | 0.11 |
| 132 | S132L | 0.25 | 1.27 | 1.65 | 1.22 | 0.8 | 0.78 |
| 132 | S132M | 0.35 | 1.01 | 1.05 | 1.05 | 0.6 | 0.72 |
| 132 | S132N | 0.88 | 1.38 | 1.35 | 1.17 | 1.0 | 0.87 |
| 132 | S132P | 0.17 | 9.36 | 11.70 | 2.50 | 1.0 | 0.23 |
| 132 | S132Q | 0.34 | 1.35 | 1.28 | 1.18 | 0.7 | 0.88 |
| 132 | S 132V | 0.23 | 3.29 | 2.39 | 2.07 | 0.8 | 0.58 |
| 133 | A133C | 0.98 | 0.87 | 1.06 | 0.79 | 1.1 | 0.84 |
| 133 | A133D | 0.91 | 0.90 | 0.79 | 0.81 | 1.0 | 0.62 |
| 133 | A133E | 1.07 | 0.93 | 0.96 | 0.84 | 1.0 | 0.99 |
| 133 | A133F | 0.83 | 0.95 | 0.65 | 0.93 | 0.9 | 1.11 |
| 133 | A133G | 0.78 | 0.95 | 0.95 | 0.92 | 1.0 | 1.12 |
| 133 | A133I | 0.95 | 0.88 | 0.88 | 0.84 | 1.0 | 1.49 |
| 133 | A133K | 1.11 | 0.75 | 0.65 | 0.72 | 1.1 | 1.02 |
| 133 | A133L | 0.93 | 0.78 | 0.83 | 0.88 | 1.0 | 1.15 |
| 133 | A133M | 0.95 | 0.99 | 0.71 | 0.85 | 1.0 | 1.00 |
| 133 | A133P | 1.10 | 1.21 | 0.74 | 0.83 | 1.2 | 0.94 |
| 133 | A133Q | 0.85 | 1.25 | 0.98 | 0.79 | 1.1 | 1.07 |
| 133 | A133R | 0.93 | 0.51 | 0.41 | 0.65 | 1.1 | 0.97 |
| 133 | A133S | 1.05 | 1.00 | 0.94 | 0.82 | 1.0 | 1.06 |
| 133 | A133T | 0.96 | 1.03 | 0.78 | 0.82 | 1.1 | 1.14 |
| 133 | A133V | 0.98 | 0.95 | 0.89 | 0.87 | 1.1 | 1.07 |
| 133 | A133W | 0.73 | 0.86 | 0.42 | 0.73 | 1.1 | 1.09 |
| 133 | A133Y | 0.84 | 0.71 | 0.75 | 0.65 | 1.1 | 1.12 |
| 134 | A134C | 0.78 | 0.83 | 1.13 | 0.80 | 1.0 | 0.82 |
| 134 | A134F | 0.26 | 0.99 | 0.97 | 0.88 | 0.9 | 0.86 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 134 | A134G | 0.63 | 1.14 | 0.59 | 0.93 | 1.2 | 0.74 |
| 134 | A134I | 0.26 | 1.60 | 1.22 | 1.29 | 0.9 | 0.93 |
| 134 | A134L | 0.19 | 1.85 | 2.41 | 1.75 | 1.1 | 0.61 |
| 134 | A134M | 0.21 | 1.40 | 1.20 | 1.29 | 1.1 | 0.53 |
| 134 | A134P | 0.78 | 1.06 | 0.86 | 0.95 | 1.0 | 1.25 |
| 134 | A134Q | 0.19 | 0.12 | 0.36 | 0.32 | 1.1 | 0.10 |
| 134 | A134S | 0.97 | 1.04 | 0.91 | 0.92 | 1.1 | 1.12 |
| 134 | A134T | 0.94 | 1.03 | 0.86 | 1.04 | 1.0 | 1.04 |
| 134 | A134V | 0.39 | 1.27 | 1.04 | 0.84 | 0.9 | 1.14 |
| 135 | L135A | 0.23 | 1.24 | 0.90 | 1.12 | 1.0 | 0.07 |
| 135 | L135E | 0.43 | 1.19 | 1.03 | 0.88 | 1.1 | 0.82 |
| 135 | L135I | 0.34 | 1.41 | 0.97 | 1.06 | 0.7 | 0.16 |
| 135 | L135M | 1.03 | 1.01 | 1.19 | 0.96 | 1.1 | 0.59 |
| 135 | L135T | 0.19 | 3.36 | 2.29 | 2.06 | 0.6 | 0.06 |
| 135 | L135V | 0.34 | 2.12 | 1.12 | 1.33 | 0.6 | 0.19 |
| 135 | L135W | 0.27 | 2.14 | 1.96 | 1.47 | 0.8 | 0.23 |
| 136 | K136A | 0.57 | 0.80 | 1.04 | 0.83 | 0.9 | 0.80 |
| 136 | K136C | 0.51 | 1.02 | 1.04 | 0.74 | 0.9 | 0.81 |
| 136 | K136D | 0.21 | 1.62 | 1.47 | 0.92 | 0.4 | 0.47 |
| 136 | K136E | 1.03 | 0.92 | 0.73 | 0.82 | 1.2 | 1.03 |
| 136 | K136F | 0.27 | 1.35 | 1.57 | 1.15 | 0.6 | 0.82 |
| 136 | K136G | 0.44 | 1.09 | 1.12 | 0.91 | 0.7 | 0.82 |
| 136 | K136H | 0.89 | 0.99 | 1.00 | 0.68 | 1.1 | 1.08 |
| 136 | K136I | 0.20 | 2.18 | 2.48 | 1.44 | 0.4 | 0.47 |
| 136 | K136L | 0.65 | 1.05 | 0.71 | 0.90 | 1.0 | 1.16 |
| 136 | K136M | 0.69 | 1.02 | 0.87 | 0.87 | 0.9 | 1.09 |
| 136 | K136N | 0.60 | 1.08 | 0.88 | 0.81 | 1.0 | 0.88 |
| 136 | K136R | 0.82 | 1.02 | 0.82 | 0.69 | 1.1 | 1.01 |
| 136 | K136S | 0.39 | 1.08 | 0.87 | 0.92 | 0.7 | 0.80 |
| 136 | K136V | 0.21 | 2.18 | 2.41 | 1.96 | 0.5 | 0.83 |
| 136 | K136W | 0.44 | 1.03 | 0.93 | 0.80 | 0.6 | 0.91 |
| 136 | K136Y | 0.25 | 1.78 | 1.41 | 1.21 | 0.4 | 0.94 |
| 137 | A137C | 1.00 | 0.91 | 0.95 | 0.71 | 1.1 | 1.08 |
| 137 | A137D | 1.14 | 1.04 | 1.00 | 0.81 | 1.0 | 1.06 |
| 137 | A137E | 0.75 | 0.91 | 1.10 | 0.88 | 1.1 | 1.03 |
| 137 | A137F | 0.59 | 1.01 | 0.78 | 0.90 | 1.0 | 1.41 |
| 137 | A137G | 0.72 | 1.01 | 0.91 | 0.78 | 1.1 | 1.24 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 137 | A137H | 1.02 | 0.95 | 0.78 | 0.91 | 0.9 | 1.03 |
| 137 | A137K | 0.90 | 0.96 | 0.52 | 0.90 | 1.0 | 1.17 |
| 137 | A137L | 0.84 | 0.85 | 0.82 | 0.89 | 1.1 | 1.07 |
| 137 | A137M | 0.93 | 1.09 | 0.89 | 0.85 | 1.0 | 1.06 |
| 137 | A137N | 1.01 | 1.02 | 0.62 | 0.77 | 1.0 | 1.38 |
| 137 | A137P | 0.18 | 3.85 | 3.42 | 1.76 | 0.2 | 0.10 |
| 137 | A137Q | 1.05 | 1.05 | 0.60 | 0.92 | 1.0 | 1.18 |
| 137 | A137R | 0.65 | 0.65 | 0.49 | 0.70 | 1.0 | 1.17 |
| 137 | A137S | 0.98 | 0.91 | 0.92 | 1.08 | 0.9 | 1.11 |
| 137 | A137T | 0.96 | 0.94 | 0.77 | 0.96 | 1.0 | 1.07 |
| 137 | A137V | 0.62 | 1.27 | 0.85 | 1.06 | 0.8 | 1.23 |
| 137 | A137W | 0.69 | 0.81 | 0.55 | 0.62 | 0.9 | 1.50 |
| 137 | A137Y | 0.83 | 1.05 | 0.73 | 0.66 | 0.9 | 1.33 |
| 138 | A138C | 0.85 | 1.04 | 1.10 | 1.14 | 0.9 | 1.07 |
| 138 | A138D | 0.17 | 1.47 | 4.55 | 2.27 | 0.3 | 0.16 |
| 138 | A138E | 0.36 | 1.44 | 0.96 | 1.13 | 0.9 | 0.83 |
| 138 | A138F | 0.18 | 2.55 | 2.71 | 1.96 | 0.8 | 0.31 |
| 138 | A138G | 0.52 | 1.27 | 1.35 | 1.07 | 0.9 | 1.16 |
| 138 | A138H | 0.20 | 2.04 | 2.24 | 1.84 | 0.7 | 0.52 |
| 138 | A138I | 0.78 | 0.93 | 0.98 | 1.11 | 0.9 | 1.18 |
| 138 | A138L | 0.27 | 1.23 | 1.19 | 1.42 | 0.9 | 0.70 |
| 138 | A138M | 0.71 | 0.88 | 0.89 | 1.00 | 1.0 | 0.91 |
| 138 | A138Q | 0.17 | 11.86 | 16.19 | 4.99 | 0.9 | 0.75 |
| 138 | A138S | 0.86 | 1.05 | 0.99 | 1.02 | 0.7 | 1.22 |
| 138 | A138T | 0.51 | 1.17 | 1.27 | 1.18 | 0.9 | 1.07 |
| 138 | A138V | 0.76 | 1.08 | 1.11 | 1.05 | 0.9 | 1.12 |
| 138 | A138Y | 0.17 | 5.98 | 9.05 | 3.30 | 0.6 | 0.51 |
| 139 | V139A | 0.69 | 1.06 | 1.06 | 0.97 | 0.8 | 0.94 |
| 139 | V139C | 1.19 | 1.03 | 1.09 | 0.95 | 1.0 | 1.30 |
| 139 | V139G | 0.21 | 0.40 | 0.60 | 0.65 | 0.5 | 0.25 |
| 139 | V139H | 0.25 | 0.75 | 0.74 | 0.73 | 0.8 | 0.06 |
| 139 | V139I | 0.85 | 0.84 | 0.73 | 1.02 | 1.0 | 0.43 |
| 139 | V139L | 0.75 | 0.98 | 0.84 | 1.09 | 0.8 | 0.28 |
| 139 | V139M | 0.61 | 0.97 | 0.67 | 1.01 | 0.6 | 0.49 |
| 139 | V139N | 0.47 | 0.83 | 0.76 | 1.14 | 0.9 | 1.20 |
| 139 | V139S | 0.49 | 0.78 | 1.11 | 1.11 | 0.9 | 0.98 |
| 139 | V139T | 0.42 | 1.27 | 0.83 | 1.19 | 1.0 | 0.63 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 140 | D140A | 0.45 | 0.70 | 0.72 | 0.81 | 0.2 | 0.61 |
| 140 | D140C | 0.56 | 0.90 | 1.02 | 0.89 | 0.8 | 0.88 |
| 140 | D140E | 1.02 | 1.20 | 0.98 | 0.92 | 0.9 | 1.01 |
| 140 | D140G | 0.52 | 0.80 | 0.79 | 0.85 | 0.7 | 0.86 |
| 140 | D140H | 0.50 | 0.47 | 0.53 | 0.67 | 0.7 | 0.72 |
| 140 | D140K | 0.48 | 0.25 | 0.53 | 0.47 | 0.8 | 0.85 |
| 140 | D140L | 0.42 | 0.54 | 0.78 | 0.84 | 0.1 | 0.97 |
| 140 | D140M | 0.48 | 0.77 | 0.81 | 0.82 | 0.3 | 0.95 |
| 140 | D140N | 0.88 | 0.86 | 0.84 | 0.83 | 0.9 | 0.97 |
| 140 | D140Q | 0.61 | 0.87 | 0.80 | 0.96 | 0.7 | 1.31 |
| 140 | D140R | 0.41 | 0.22 | 0.28 | 0.50 | 0.7 | 0.98 |
| 140 | D140S | 0.55 | 0.81 | 0.77 | 0.92 | 0.4 | 0.83 |
| 140 | D140T | 0.60 | 0.85 | 0.80 | 0.89 | 0.5 | 0.87 |
| 140 | D140V | 0.42 | 0.98 | 1.00 | 0.96 | 0.2 | 1.07 |
| 140 | D140W | 0.23 | 0.74 | 0.78 | 0.85 | 0.4 | 0.95 |
| 140 | D140Y | 0.50 | 0.79 | 0.74 | 0.83 | 0.6 | 1.05 |
| 141 | K141A | 0.87 | 0.87 | 1.11 | 0.93 | 1.0 | 1.06 |
| 141 | K141C | 0.78 | 0.73 | 1.11 | 1.04 | 1.0 | 1.11 |
| 141 | K141D | 0.82 | 0.95 | 1.23 | 1.04 | 1.0 | 1.14 |
| 141 | K141E | 1.18 | 0.80 | 1.10 | 1.00 | 1.0 | 0.97 |
| 141 | K141F | 0.70 | 0.84 | 1.27 | 1.04 | 1.0 | 1.13 |
| 141 | K141G | 0.94 | 0.84 | 1.15 | 1.07 | 1.1 | 1.09 |
| 141 | K141H | 1.00 | 1.03 | 1.09 | 1.04 | 1.2 | 1.08 |
| 141 | K141I | 0.60 | 0.94 | 1.23 | 1.00 | 1.1 | 1.25 |
| 141 | K141L | 0.66 | 0.87 | 1.29 | 1.01 | 1.1 | 1.14 |
| 141 | K141M | 0.79 | 1.00 | 1.17 | 1.02 | 1.0 | 1.24 |
| 141 | K141N | 1.05 | 0.90 | 1.30 | 1.03 | 1.1 | 1.17 |
| 141 | K141Q | 1.06 | 0.96 | 1.26 | 1.09 | 1.1 | 1.10 |
| 141 | K141R | 1.09 | 0.87 | 1.13 | 1.07 | 1.0 | 0.97 |
| 141 | K141S | 0.95 | 0.96 | 1.06 | 1.22 | 1.0 | 1.15 |
| 141 | K141V | 0.60 | 1.20 | 1.28 | 1.02 | 1.0 | 1.36 |
| 141 | K141W | 0.70 | 1.14 | 1.32 | 1.18 | 1.2 | 1.27 |
| 141 | K141Y | 0.91 | 1.16 | 1.07 | 1.06 | 1.1 | 1.16 |
| 142 | A142C | 0.79 | 1.03 | 0.92 | 0.89 | 1.1 | 1.17 |
| 142 | A142G | 0.46 | 1.32 | 1.20 | 0.96 | 1.0 | 1.18 |
| 142 | A142I | 0.25 | 1.60 | 1.39 | 1.46 | 0.6 | 0.81 |
| 142 | A142L | 0.29 | 1.24 | 1.40 | 1.19 | 0.6 | 0.70 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 142 | A142M | 0.22 | 1.21 | 1.62 | 1.31 | 0.6 | 0.38 |
| 142 | A142S | 0.68 | 1.01 | 0.98 | 1.23 | 1.0 | 1.18 |
| 142 | A142T | 0.31 | 1.32 | 1.55 | 1.46 | 0.8 | 1.11 |
| 142 | A142V | 0.37 | 1.49 | 1.19 | 1.23 | 0.8 | 1.12 |
| 143 | V143A | 1.05 | 0.81 | 1.04 | 0.90 | 0.9 | 0.88 |
| 143 | V143C | 0.87 | 1.00 | 1.14 | 0.89 | 1.0 | 1.08 |
| 143 | V143D | 0.95 | 0.95 | 1.03 | 0.97 | 1.0 | 1.05 |
| 143 | V143E | 1.12 | 0.87 | 1.05 | 1.02 | 1.0 | 1.08 |
| 143 | V143F | 0.88 | 0.76 | 1.00 | 0.81 | 0.9 | 0.81 |
| 143 | V143G | 0.71 | 0.90 | 1.16 | 1.15 | 0.9 | 1.11 |
| 143 | V143K | 0.84 | 0.61 | 0.72 | 0.96 | 1.1 | 1.00 |
| 143 | V143L | 0.78 | 0.78 | 1.13 | 0.83 | 0.9 | 1.11 |
| 143 | V143M | 0.83 | 0.94 | 1.05 | 1.11 | 0.9 | 0.99 |
| 143 | V143N | 0.91 | 0.90 | 1.00 | 1.20 | 1.0 | 1.13 |
| 143 | V143Q | 0.88 | 0.82 | 1.11 | 1.04 | 1.1 | 1.04 |
| 143 | V143R | 0.77 | 0.64 | 0.70 | 0.92 | 1.0 | 0.94 |
| 143 | V143S | 1.03 | 0.94 | 0.97 | 0.93 | 1.0 | 1.02 |
| 143 | V143T | 0.83 | 0.90 | 0.93 | 0.99 | 1.0 | 1.17 |
| 143 | V143W | 0.65 | 1.36 | 0.84 | 1.02 | 0.9 | 0.88 |
| 144 | A144C | 1.17 | 0.98 | 0.85 | 0.88 | 1.0 | 1.02 |
| 144 | A144D | 1.29 | 1.18 | 1.11 | 0.99 | 1.0 | 0.96 |
| 144 | A144E | 1.17 | 1.08 | 0.76 | 1.00 | 1.0 | 1.13 |
| 144 | A144F | 0.96 | 1.20 | 0.73 | 0.94 | 1.0 | 1.16 |
| 144 | A144G | 1.09 | 1.08 | 0.98 | 1.02 | 1.0 | 0.99 |
| 144 | A144I | 0.97 | 1.29 | 0.79 | 0.97 | 1.1 | 1.06 |
| 144 | A144K | 1.28 | 1.00 | 0.57 | 0.77 | 1.1 | 0.90 |
| 144 | A144L | 1.11 | 1.18 | 0.79 | 1.08 | 1.0 | 1.03 |
| 144 | A144M | 1.06 | 1.08 | 0.84 | 1.07 | 1.0 | 1.05 |
| 144 | A144P | 0.18 | 1.75 | 2.60 | 1.24 | 0.4 | 0.30 |
| 144 | A144R | 1.23 | 0.96 | 0.45 | 0.90 | 1.1 | 1.00 |
| 144 | A144S | 1.04 | 1.24 | 0.93 | 0.85 | 1.0 | 1.05 |
| 144 | A144T | 1.08 | 1.16 | 0.89 | 1.04 | 1.1 | 1.14 |
| 144 | A144V | 0.80 | 1.11 | 1.13 | 0.86 | 1.0 | 1.21 |
| 144 | A144W | 0.87 | 1.23 | 0.64 | 0.88 | 1.0 | 1.63 |
| 145 | S145A | 1.09 | 0.97 | 1.13 | 0.94 | 1.0 | 0.99 |
| 145 | S145C | 1.10 | 0.72 | 1.03 | 1.00 | 1.1 | 1.03 |
| 145 | S145D | 1.24 | 1.03 | 1.06 | 1.08 | 1.1 | 1.01 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 145 | S145E | 1.24 | 0.90 | 1.20 | 0.90 | 1.1 | 0.95 |
| 145 | S145F | 0.84 | 0.81 | 1.15 | 1.12 | 1.1 | 1.22 |
| 145 | S145G | 1.06 | 0.77 | 1.13 | 1.06 | 1.0 | 1.11 |
| 145 | S145H | 1.21 | 0.93 | 1.04 | 1.05 | 1.1 | 1.02 |
| 145 | S145I | 0.79 | 0.42 | 1.24 | 1.03 | 1.1 | 1.24 |
| 145 | S145L | 0.87 | 1.02 | 1.25 | 1.08 | 1.1 | 1.13 |
| 145 | S145M | 1.00 | 0.86 | 1.06 | 1.04 | 1.0 | 1.08 |
| 145 | S145Q | 1.10 | 0.74 | 1.18 | 1.08 | 1.0 | 1.06 |
| 145 | S145R | 1.21 | 0.78 | 1.01 | 1.13 | 1.1 | 1.06 |
| 145 | S145T | 1.04 | 0.92 | 1.17 | 1.04 | 1.0 | 1.07 |
| 145 | S145V | 0.83 | 0.87 | 1.25 | 1.06 | 1.0 | 1.17 |
| 145 | S145W | 0.81 | 1.15 | 1.10 | 1.01 | 0.9 | 1.21 |
| 145 | S145Y | 0.48 | 0.88 | 1.28 | 1.13 | 0.9 | 1.29 |
| 146 | G146A | 0.48 | 1.04 | 1.18 | 1.08 | 1.0 | 1.02 |
| 146 | G146C | 0.50 | 1.01 | 1.15 | 0.98 | 1.1 | 0.97 |
| 146 | G146D | 1.08 | 0.86 | 1.02 | 1.00 | 1.1 | 1.07 |
| 146 | G146E | 0.82 | 0.79 | 1.12 | 0.98 | 1.0 | 1.04 |
| 146 | G146F | 0.50 | 0.88 | 1.03 | 1.06 | 1.0 | 1.02 |
| 146 | G146H | 0.92 | 0.86 | 1.17 | 1.06 | 0.9 | 1.09 |
| 146 | G146K | 0.89 | 0.72 | 1.09 | 0.98 | 0.9 | 1.07 |
| 146 | G146L | 0.18 | 2.22 | 3.81 | 1.88 | 1.1 | 0.59 |
| 146 | G146M | 0.34 | 1.18 | 1.21 | 1.22 | 1.0 | 1.09 |
| 146 | G146P | 0.18 | 0.49 | 0.60 | 0.25 | 0.2 | 0.08 |
| 146 | G146Q | 0.85 | 0.95 | 1.16 | 1.05 | 1.0 | 1.45 |
| 146 | G146R | 0.81 | 0.79 | 1.08 | 1.04 | 0.9 | 1.02 |
| 146 | G146S | 0.76 | 0.92 | 1.11 | 0.91 | 0.9 | 1.06 |
| 146 | G146T | 0.25 | 1.52 | 1.80 | 1.57 | 0.7 | 1.12 |
| 146 | G146Y | 0.21 | 1.66 | 2.23 | 1.62 | 0.9 | 0.88 |
| 147 | V147A | 1.13 | 1.04 | 1.09 | 0.92 | 1.1 | 0.90 |
| 147 | V147C | 0.87 | 1.19 | 1.25 | 0.99 | 1.1 | 0.90 |
| 147 | V147D | 0.21 | 2.29 | 3.05 | 2.09 | 1.2 | 0.53 |
| 147 | V147E | 0.53 | 0.98 | 1.17 | 0.93 | 1.0 | 1.12 |
| 147 | V147G | 0.64 | 1.25 | 1.42 | 1.04 | 1.0 | 0.93 |
| 147 | V147H | 0.70 | 1.23 | 1.30 | 0.94 | 1.2 | 0.93 |
| 147 | V147I | 0.98 | 1.25 | 0.94 | 0.94 | 1.1 | 1.04 |
| 147 | V147L | 0.70 | 1.16 | 1.29 | 1.15 | 1.0 | 0.97 |
| 147 | V147M | 0.97 | 1.00 | 1.07 | 0.90 | 1.0 | 1.14 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 147 | V147P | 0.31 | 1.71 | 2.12 | 1.52 | 0.8 | 1.13 |
| 147 | V147Q | 0.84 | 1.23 | 1.09 | 1.03 | 1.2 | 1.01 |
| 147 | V147R | 0.71 | 1.16 | 1.07 | 0.88 | 1.0 | 0.96 |
| 147 | V147S | 0.87 | 1.06 | 1.22 | 0.87 | 1.0 | 0.98 |
| 147 | V147T | 0.80 | 1.24 | 1.06 | 1.02 | 0.9 | 1.16 |
| 147 | V147W | 0.25 | 1.97 | 2.16 | 1.32 | 0.9 | 0.93 |
| 147 | V147Y | 0.26 | 2.34 | 2.31 | 1.50 | 0.9 | 0.78 |
| 148 | V148A | 0.56 | 1.06 | 0.76 | 1.03 | 0.8 | 0.74 |
| 148 | V148E | 0.21 | 0.62 | 0.99 | 0.67 | 0.9 | 0.14 |
| 148 | V148F | 0.73 | 0.96 | 1.18 | 1.06 | 0.9 | 0.85 |
| 148 | V148G | 0.23 | 0.70 | 1.12 | 0.98 | 0.9 | 0.26 |
| 148 | V148H | 0.30 | 1.07 | 1.68 | 1.39 | 0.8 | 0.74 |
| 148 | V148I | 0.70 | 0.98 | 1.20 | 1.02 | 1.1 | 0.91 |
| 148 | V148L | 0.98 | 1.05 | 1.11 | 1.07 | 1.1 | 0.80 |
| 148 | V148M | 0.64 | 0.94 | 1.06 | 1.01 | 0.8 | 0.74 |
| 148 | V148N | 0.41 | 1.38 | 1.35 | 1.11 | 0.8 | 1.11 |
| 148 | V148P | 0.27 | 0.61 | 0.87 | 0.76 | 1.0 | 0.21 |
| 148 | V148Q | 0.25 | 1.08 | 1.73 | 1.58 | 1.0 | 0.82 |
| 148 | V148S | 0.52 | 1.09 | 1.20 | 1.10 | 1.0 | 0.90 |
| 148 | V148T | 0.55 | 1.05 | 1.41 | 1.13 | 1.0 | 0.99 |
| 148 | V148Y | 0.22 | 1.60 | 1.45 | 1.60 | 0.6 | 0.37 |
| 149 | V149A | 0.80 | 0.96 | 1.10 | 0.99 | 1.0 | 0.99 |
| 149 | V149C | 0.86 | 1.11 | 0.96 | 0.99 | 1.2 | 0.89 |
| 149 | V149D | 0.22 | 0.83 | 1.23 | 1.11 | 1.3 | 0.32 |
| 149 | V149E | 0.18 | 2.65 | 3.71 | 2.50 | 1.0 | 0.40 |
| 149 | V149F | 0.92 | 1.15 | 1.31 | 1.07 | 1.0 | 0.23 |
| 149 | V149G | 0.24 | 0.90 | 0.72 | 1.05 | 1.0 | 0.28 |
| 149 | V149H | 0.25 | 0.54 | 0.75 | 0.70 | 1.2 | 0.26 |
| 149 | V149I | 0.84 | 0.96 | 1.08 | 0.85 | 1.1 | 0.80 |
| 149 | V149L | 0.69 | 1.02 | 1.11 | 1.11 | 1.1 | 0.98 |
| 149 | V149M | 0.82 | 1.09 | 1.11 | 1.07 | 1.1 | 0.82 |
| 149 | V149P | 0.46 | 0.94 | 1.23 | 1.04 | 1.2 | 1.25 |
| 149 | V149S | 0.37 | 1.12 | 1.42 | 1.31 | 1.1 | 1.18 |
| 149 | V149T | 0.66 | 0.88 | 1.16 | 1.09 | 1.1 | 1.11 |
| 149 | V149W | 0.25 | 0.44 | 0.50 | 0.59 | 1.3 | 0.12 |
| 149 | V149Y | 0.33 | 1.18 | 1.52 | 1.33 | 1.0 | 0.18 |
| 150 | V150A | 0.54 | 0.85 | 1.21 | 1.04 | 0.9 | 0.67 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 150 | V150C | 0.80 | 0.86 | 1.07 | 1.06 | 1.0 | 0.80 |
| 150 | V150F | 0.47 | 0.89 | 1.07 | 1.07 | 0.8 | 0.86 |
| 150 | V150I | 0.83 | 1.14 | 1.05 | 1.07 | 1.0 | 1.15 |
| 150 | V150L | 0.75 | 0.97 | 0.96 | 1.02 | 0.9 | 0.95 |
| 150 | V150M | 0.39 | 0.97 | 1.26 | 1.27 | 0.9 | 0.77 |
| 150 | V150N | 0.28 | 0.27 | 0.26 | 0.21 | 1.1 | 0.06 |
| 150 | V150Q | 0.31 | 1.25 | 1.67 | 1.46 | 1.0 | 1.05 |
| 150 | V150S | 0.33 | 1.08 | 1.22 | 1.17 | 0.8 | 0.52 |
| 150 | V150T | 0.61 | 0.71 | 1.28 | 1.09 | 1.0 | 0.72 |
| 151 | A151C | 0.74 | 1.20 | 0.94 | 0.98 | 0.9 | 0.91 |
| 151 | A151G | 0.49 | 0.85 | 1.24 | 1.16 | 0.8 | 0.74 |
| 151 | A151M | 0.24 | 1.15 | 1.42 | 1.13 | 1.0 | 0.32 |
| 151 | A151N | 0.21 | 0.93 | 1.88 | 1.11 | 0.7 | 0.13 |
| 151 | A151S | 0.79 | 0.96 | 1.26 | 1.08 | 1.0 | 0.51 |
| 151 | A151T | 0.46 | 1.36 | 1.20 | 1.18 | 0.6 | 0.56 |
| 151 | A151V | 0.38 | 0.95 | 1.08 | 1.45 | 0.6 | 0.61 |
| 152 | A152S | 1.11 | 0.74 | 1.00 | 0.85 | 0.9 | 0.32 |
| 153 | A153C | 0.54 | 1.02 | 1.03 | 0.96 | 0.5 | 0.67 |
| 153 | A153G | 0.51 | 1.03 | 1.06 | 1.03 | 0.5 | 0.64 |
| 153 | A153S | 0.65 | 1.05 | 1.03 | 1.04 | 0.6 | 0.62 |
| 153 | A153V | 0.28 | 1.34 | 1.24 | 1.39 | 0.3 | 0.88 |
| 154 | G154S | 0.22 | 0.58 | 0.78 | 0.76 | 0.2 | 0.06 |
| 156 | E156A | 1.12 | 0.69 | 0.60 | 0.84 | 1.0 | 0.87 |
| 156 | E156C | 1.10 | 0.61 | 0.70 | 0.83 | 1.0 | 0.85 |
| 156 | E156F | 1.11 | 0.41 | 0.57 | 0.89 | 1.0 | 1.12 |
| 156 | E156I | 0.43 | 0.59 | 0.64 | 0.86 | 0.5 | 0.86 |
| 156 | E156K | 0.99 | 0.42 | 0.41 | 0.78 | 0.5 | 1.30 |
| 156 | E156L | 0.82 | 0.55 | 0.60 | 0.83 | 0.8 | 1.31 |
| 156 | E156M | 1.10 | 0.46 | 0.57 | 0.86 | 0.9 | 1.31 |
| 156 | E156N | 1.17 | 0.38 | 0.48 | 0.72 | 1.0 | 0.91 |
| 156 | E156Q | 0.93 | 0.51 | 0.51 | 0.75 | 0.9 | 0.94 |
| 156 | E156R | 1.10 | 0.23 | 0.41 | 0.66 | 0.4 | 1.35 |
| 156 | E156S | 0.93 | 0.62 | 0.52 | 0.91 | 0.8 | 1.01 |
| 156 | E156T | 0.72 | 0.73 | 0.65 | 1.00 | 0.7 | 1.44 |
| 156 | E156V | 0.54 | 0.60 | 0.60 | 0.98 | 0.6 | 1.13 |
| 156 | E156W | 0.79 | 0.24 | 0.21 | 0.60 | 0.7 | 0.79 |
| 156 | E156Y | 1.10 | 0.35 | 0.43 | 0.72 | 0.9 | 1.09 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 157 | G157A | 0.48 | 0.83 | 0.87 | 0.92 | 0.6 | 0.62 |
| 157 | G157C | 0.60 | 0.63 | 0.79 | 0.95 | 0.4 | 0.18 |
| 157 | G157D | 0.49 | 0.68 | 0.87 | 0.79 | 0.4 | 0.20 |
| 157 | G157E | 0.58 | 0.62 | 0.82 | 0.69 | 0.2 | 0.12 |
| 157 | G157F | 0.34 | 0.38 | 0.60 | 0.68 | 0.2 | 0.19 |
| 157 | G157I | 0.34 | 0.34 | 0.53 | 0.54 | 0.1 | 0.06 |
| 157 | G157L | 0.40 | 0.33 | 0.54 | 0.60 | 0.1 | 0.06 |
| 157 | G157M | 0.43 | 0.58 | 0.72 | 0.66 | 0.2 | 0.11 |
| 157 | G157P | 0.45 | 0.32 | 0.42 | 0.58 | 0.1 | 0.09 |
| 157 | G157Q | 0.41 | 0.55 | 0.80 | 0.71 | 0.2 | 0.13 |
| 157 | G157R | 0.31 | 0.28 | 0.52 | 0.43 | 0.1 | 0.15 |
| 157 | G157S | 0.53 | 1.03 | 0.96 | 0.88 | 0.8 | 0.72 |
| 157 | G157T | 0.37 | 0.51 | 0.81 | 0.97 | 0.2 | 0.12 |
| 157 | G157V | 0.33 | 0.43 | 0.74 | 0.63 | 0.1 | 0.09 |
| 157 | G157W | 0.40 | 0.59 | 0.70 | 0.88 | 0.2 | 0.18 |
| 157 | G157Y | 0.29 | 0.74 | 0.80 | 0.80 | 0.3 | 0.27 |
| 158 | T158A | 0.94 | 0.98 | 0.98 | 0.92 | 0.8 | 1.10 |
| 158 | T158D | 0.68 | 1.09 | 1.09 | 1.04 | 1.1 | 0.94 |
| 158 | T158E | 1.10 | 1.09 | 1.03 | 0.98 | 1.2 | 0.95 |
| 158 | T158G | 0.77 | 1.11 | 0.47 | 1.06 | 0.8 | 0.75 |
| 158 | T158H | 0.85 | 1.04 | 0.95 | 0.92 | 0.9 | 0.87 |
| 158 | T158I | 1.02 | 1.08 | 0.75 | 0.91 | 1.0 | 0.96 |
| 158 | T158K | 0.83 | 1.05 | 0.53 | 0.85 | 0.8 | 0.97 |
| 158 | T158L | 0.71 | 1.06 | 0.65 | 0.83 | 0.8 | 0.73 |
| 158 | T158M | 0.80 | 0.97 | 0.71 | 0.97 | 0.7 | 0.71 |
| 158 | T158N | 0.79 | 1.16 | 0.69 | 0.85 | 1.0 | 0.80 |
| 158 | T158P | 0.58 | 1.12 | 0.88 | 0.86 | 0.7 | 0.66 |
| 158 | T158Q | 0.82 | 1.04 | 0.89 | 0.81 | 1.0 | 0.81 |
| 158 | T158R | 0.56 | 0.84 | 0.54 | 0.83 | 0.5 | 1.02 |
| 158 | T158S | 1.31 | 1.09 | 1.04 | 0.93 | 0.9 | 0.94 |
| 158 | T158V | 0.93 | 1.20 | 0.83 | 0.86 | 0.9 | 0.95 |
| 158 | T158W | 0.61 | 1.01 | 0.75 | 0.89 | 0.6 | 0.82 |
| 158 | T158Y | 0.75 | 1.01 | 0.53 | 1.04 | 0.7 | 0.84 |
| 159 | S159A | 0.92 | 0.93 | 0.90 | 1.07 | 0.7 | 0.78 |
| 159 | S159C | 1.16 | 1.08 | 1.18 | 0.87 | 1.1 | 0.93 |
| 159 | S159D | 1.41 | 1.26 | 1.15 | 1.05 | 1.1 | 0.92 |
| 159 | S159E | 1.07 | 1.04 | 1.13 | 1.07 | 1.0 | 0.90 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 159 | S159F | 0.73 | 1.14 | 0.93 | 1.06 | 0.4 | 0.74 |
| 159 | S159G | 0.88 | 1.14 | 1.13 | 0.91 | 0.6 | 0.86 |
| 159 | S159H | 1.10 | 0.95 | 0.85 | 0.88 | 1.0 | 1.05 |
| 159 | S159I | 0.90 | 1.03 | 1.02 | 0.92 | 0.4 | 0.63 |
| 159 | S159K | 1.24 | 1.37 | 0.92 | 1.00 | 1.0 | 1.08 |
| 159 | S159L | 0.77 | 1.09 | 1.15 | 1.25 | 0.4 | 0.77 |
| 159 | S159M | 0.81 | 1.02 | 1.22 | 0.92 | 0.5 | 0.70 |
| 159 | S159P | 0.72 | 1.20 | 0.94 | 1.05 | 0.3 | 0.62 |
| 159 | S159Q | 1.10 | 1.06 | 1.16 | 1.11 | 1.1 | 1.08 |
| 159 | S159R | 1.09 | 0.78 | 0.87 | 0.93 | 0.9 | 1.07 |
| 159 | S159T | 0.65 | 1.02 | 1.05 | 1.03 | 0.8 | 0.96 |
| 159 | S159V | 0.70 | 1.14 | 1.14 | 1.05 | 0.5 | 0.66 |
| 159 | S159W | 0.43 | 0.93 | 1.04 | 1.06 | 0.2 | 0.54 |
| 159 | S159Y | 0.37 | 1.07 | 1.11 | 1.27 | 0.5 | 0.90 |
| 160 | G160A | 0.51 | 0.83 | 0.96 | 0.81 | 0.6 | 0.63 |
| 160 | G160C | 0.85 | 0.75 | 1.06 | 0.83 | 0.9 | 0.86 |
| 160 | G160D | 1.17 | 0.71 | 1.28 | 0.88 | 1.0 | 0.90 |
| 160 | G160E | 1.05 | 0.85 | 1.31 | 0.92 | 1.0 | 0.92 |
| 160 | G160K | 0.79 | 0.75 | 0.79 | 0.88 | 0.4 | 0.89 |
| 160 | G160L | 0.36 | 0.92 | 1.00 | 1.00 | 0.5 | 0.52 |
| 160 | G160M | 0.41 | 0.70 | 0.86 | 0.87 | 0.4 | 0.46 |
| 160 | G160N | 0.92 | 1.05 | 1.21 | 0.93 | 0.9 | 1.00 |
| 160 | G160P | 0.51 | 0.82 | 1.15 | 0.69 | 0.7 | 0.63 |
| 160 | G160Q | 0.75 | 0.72 | 1.15 | 0.92 | 0.8 | 0.83 |
| 160 | G160R | 0.54 | 0.69 | 0.83 | 0.87 | 0.3 | 0.78 |
| 160 | G160S | 0.80 | 0.82 | 1.12 | 0.89 | 0.7 | 0.84 |
| 160 | G160T | 0.71 | 0.70 | 1.21 | 1.08 | 0.6 | 0.93 |
| 160 | G160V | 0.48 | 1.12 | 1.25 | 0.88 | 0.5 | 0.60 |
| 160 | G160W | 0.37 | 0.93 | 0.90 | 0.99 | 0.3 | 0.61 |
| 160 | G160Y | 0.46 | 0.90 | 1.11 | 1.06 | 0.4 | 0.61 |
| 161 | S161A | 1.08 | 1.15 | 1.21 | 0.95 | 0.8 | 1.00 |
| 161 | S161C | 1.31 | 0.93 | 1.12 | 0.82 | 1.2 | 1.04 |
| 161 | S161E | 1.35 | 1.12 | 1.21 | 1.00 | 1.2 | 0.92 |
| 161 | S161F | 0.58 | 0.96 | 1.18 | 1.02 | 0.9 | 1.17 |
| 161 | S161G | 1.37 | 1.13 | 1.14 | 0.96 | 1.1 | 0.93 |
| 161 | S161I | 1.01 | 1.18 | 1.10 | 0.94 | 0.9 | 0.86 |
| 161 | S161K | 0.97 | 0.88 | 1.00 | 1.02 | 0.9 | 1.04 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 161 | S161L | 1.12 | 1.25 | 0.96 | 0.87 | 1.0 | 0.96 |
| 161 | S161M | 1.07 | 1.08 | 1.03 | 0.99 | 1.0 | 0.95 |
| 161 | S161P | 1.30 | 1.26 | 1.19 | 1.07 | 1.0 | 1.04 |
| 161 | S161Q | 1.10 | 1.16 | 1.13 | 0.98 | 1.1 | 1.15 |
| 161 | S161R | 0.84 | 0.94 | 0.84 | 0.88 | 0.8 | 1.16 |
| 161 | S161T | 1.13 | 1.09 | 1.21 | 1.00 | 1.0 | 1.41 |
| 161 | S161V | 0.93 | 1.29 | 1.07 | 1.05 | 0.9 | 1.10 |
| 161 | S161W | 0.43 | 0.96 | 1.12 | 1.05 | 0.7 | 0.83 |
| 161 | S161Y | 0.70 | 1.05 | 1.05 | 0.95 | 0.8 | 0.99 |
| 162 | S162A | 0.75 | 1.09 | 1.00 | 0.96 | 1.0 | 0.92 |
| 162 | S162C | 1.24 | 0.94 | 0.95 | 0.89 | 1.1 | 0.93 |
| 162 | S162E | 1.41 | 1.16 | 0.93 | 0.89 | 1.1 | 0.99 |
| 162 | S162F | 0.45 | 0.94 | 0.96 | 0.82 | 0.9 | 0.77 |
| 162 | S162G | 0.76 | 1.16 | 0.94 | 0.91 | 1.0 | 1.03 |
| 162 | S162H | 1.29 | 0.95 | 0.71 | 1.02 | 1.0 | 1.02 |
| 162 | S162I | 0.53 | 1.06 | 0.84 | 0.89 | 1.0 | 0.98 |
| 162 | S162K | 1.38 | 1.05 | 0.81 | 0.94 | 1.0 | 1.09 |
| 162 | S162L | 0.66 | 1.25 | 0.55 | 0.91 | 1.0 | 0.84 |
| 162 | S162M | 0.73 | 1.09 | 0.64 | 1.03 | 0.9 | 1.00 |
| 162 | S162N | 1.39 | 0.89 | 0.87 | 1.00 | 1.1 | 1.09 |
| 162 | S162P | 0.56 | 1.06 | 0.87 | 0.89 | 0.7 | 0.66 |
| 162 | S162Q | 1.28 | 1.06 | 1.06 | 0.88 | 1.1 | 1.07 |
| 162 | S162R | 0.90 | 0.80 | 0.69 | 0.89 | 0.9 | 1.26 |
| 162 | S162T | 0.89 | 1.04 | 0.69 | 0.76 | 0.9 | 0.98 |
| 162 | S162V | 0.61 | 1.13 | 0.81 | 0.92 | 0.9 | 1.03 |
| 162 | S162W | 0.50 | 1.03 | 0.55 | 0.68 | 0.7 | 0.71 |
| 163 | S163A | 0.32 | 0.71 | 0.78 | 0.89 | 0.2 | 0.30 |
| 163 | S163C | 0.35 | 0.88 | 0.55 | 0.85 | 0.2 | 0.26 |
| 163 | S163D | 0.56 | 0.92 | 0.90 | 0.86 | 0.1 | 0.45 |
| 163 | S163G | 1.10 | 1.02 | 0.86 | 0.93 | 0.6 | 0.85 |
| 163 | S163H | 0.25 | 0.97 | 0.70 | 0.98 | 0.1 | 0.29 |
| 163 | S163I | 0.31 | 0.73 | 0.72 | 0.93 | 0.1 | 0.20 |
| 163 | S163P | 0.99 | 1.05 | 0.78 | 0.95 | 0.4 | 0.63 |
| 163 | S163R | 0.27 | 0.67 | 0.55 | 0.77 | 0.1 | 0.24 |
| 163 | S163T | 0.45 | 0.95 | 1.01 | 1.06 | 0.7 | 0.60 |
| 163 | S163W | 0.21 | 1.05 | 0.96 | 0.95 | 0.1 | 0.20 |
| 164 | T164A | 0.38 | 0.40 | 0.53 | 0.76 | 0.1 | 0.21 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 164 | T164C | 0.29 | 0.51 | 0.70 | 0.74 | 0.1 | 0.11 |
| 164 | T164E | 0.39 | 0.79 | 1.27 | 1.14 | 0.8 | 0.43 |
| 164 | T164G | 0.40 | 0.51 | 0.76 | 0.70 | 0.1 | 0.26 |
| 164 | T164I | 0.19 | 0.44 | 0.67 | 0.86 | 0.3 | 0.07 |
| 164 | T164L | 0.22 | 0.90 | 1.06 | 1.33 | 0.1 | 0.18 |
| 164 | T164M | 0.30 | 0.40 | 0.66 | 0.81 | 0.4 | 0.19 |
| 164 | T164N | 0.36 | 0.71 | 0.78 | 0.92 | 0.4 | 0.51 |
| 164 | T164S | 0.32 | 0.80 | 0.98 | 0.87 | 0.4 | 0.45 |
| 164 | T164V | 0.26 | 0.55 | 0.80 | 0.88 | 0.1 | 0.19 |
| 164 | T164W | 0.18 | 1.58 | 2.34 | 1.67 | 0.1 | 0.20 |
| 164 | T164Y | 0.20 | 1.10 | 1.76 | 1.62 | 0.1 | 0.19 |
| 165 | V165C | 0.22 | 0.53 | 0.65 | 0.92 | 0.4 | 0.23 |
| 165 | V165I | 0.37 | 0.89 | 1.17 | 1.15 | 1.1 | 0.87 |
| 165 | V165L | 0.28 | 0.62 | 0.66 | 1.12 | 0.8 | 0.54 |
| 165 | V165M | 0.30 | 0.61 | 0.84 | 1.10 | 0.7 | 0.53 |
| 165 | V165P | 0.46 | 0.87 | 0.80 | 0.84 | 0.8 | 0.70 |
| 165 | V165T | 0.19 | 1.50 | 1.95 | 1.86 | 0.1 | 0.21 |
| 166 | G166A | 0.27 | 0.47 | 0.77 | 0.97 | 1.1 | 0.26 |
| 166 | G166C | 0.86 | 0.61 | 0.78 | 0.79 | 1.1 | 0.33 |
| 166 | G166F | 1.09 | 0.54 | 0.46 | 0.82 | 1.3 | 0.17 |
| 166 | G166H | 1.04 | 0.80 | 0.77 | 0.78 | 1.2 | 0.41 |
| 166 | G166K | 0.82 | 0.32 | 0.33 | 0.58 | 0.9 | 0.43 |
| 166 | G166L | 1.36 | 0.41 | 0.48 | 0.61 | 1.2 | 0.20 |
| 166 | G166M | 1.13 | 0.56 | 0.59 | 0.66 | 1.1 | 0.40 |
| 166 | G166N | 1.08 | 0.90 | 0.94 | 1.10 | 1.1 | 0.47 |
| 166 | G166P | 0.37 | 0.79 | 0.83 | 0.88 | 0.1 | 0.19 |
| 166 | G166Q | 1.21 | 0.66 | 0.81 | 0.96 | 1.1 | 0.57 |
| 166 | G166R | 0.87 | 0.41 | 0.43 | 0.88 | 0.9 | 0.55 |
| 166 | G166S | 1.09 | 0.80 | 1.04 | 0.75 | 1.0 | 0.26 |
| 166 | G166W | 1.10 | 0.65 | 0.95 | 0.99 | 1.1 | 0.06 |
| 166 | G166Y | 0.99 | 0.69 | 0.45 | 0.92 | 1.0 | 0.19 |
| 167 | Y167A | 0.68 | 1.35 | 1.13 | 1.28 | 0.7 | 0.57 |
| 167 | Y167C | 0.42 | 1.20 | 1.06 | 1.31 | 0.6 | 0.30 |
| 167 | Y167D | 0.27 | 1.36 | 1.64 | 1.29 | 0.1 | 0.07 |
| 167 | Y167E | 0.37 | 1.46 | 1.71 | 1.31 | 0.5 | 0.08 |
| 167 | Y167F | 1.01 | 0.96 | 1.11 | 0.99 | 0.9 | 0.83 |
| 167 | Y167H | 0.33 | 0.93 | 1.06 | 1.15 | 0.5 | 0.47 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 167 | Y167I | 0.66 | 0.98 | 1.15 | 1.14 | 0.9 | 0.34 |
| 167 | Y167L | 0.23 | 1.16 | 1.53 | 1.29 | 0.1 | 0.17 |
| 167 | Y167M | 0.23 | 1.69 | 1.28 | 1.35 | 0.4 | 0.22 |
| 167 | Y167S | 0.30 | 1.37 | 1.27 | 1.32 | 0.2 | 0.30 |
| 167 | Y167T | 0.33 | 1.47 | 1.25 | 1.39 | 0.3 | 0.35 |
| 167 | Y167V | 0.60 | 0.96 | 1.06 | 1.07 | 0.8 | 0.44 |
| 167 | Y167W | 0.41 | 1.21 | 1.10 | 1.04 | 0.3 | 0.54 |
| 169 | G169A | 1.32 | 0.86 | 0.90 | 0.89 | 1.0 | 1.23 |
| 169 | G169C | 0.19 | 3.00 | 2.18 | 2.35 | 1.0 | 0.46 |
| 169 | G169S | 0.38 | 1.02 | 0.83 | 1.03 | 0.9 | 0.63 |
| 170 | K170A | 1.04 | 1.16 | 1.09 | 0.98 | 1.1 | 1.23 |
| 170 | K170C | 0.81 | 1.19 | 1.03 | 0.95 | 0.9 | 0.73 |
| 170 | K170F | 0.50 | 1.09 | 1.09 | 0.95 | 0.9 | 0.69 |
| 170 | K170G | 0.51 | 1.21 | 0.92 | 1.00 | 0.9 | 0.65 |
| 170 | K170H | 0.57 | 1.10 | 1.10 | 0.98 | 0.8 | 0.86 |
| 170 | K170I | 0.47 | 1.19 | 1.14 | 0.93 | 0.7 | 0.43 |
| 170 | K170L | 0.36 | 1.27 | 1.15 | 0.97 | 0.7 | 0.33 |
| 170 | K170M | 0.39 | 1.04 | 0.96 | 0.99 | 0.6 | 0.38 |
| 170 | K170N | 0.34 | 1.59 | 1.37 | 1.18 | 0.5 | 0.58 |
| 170 | K170P | 0.22 | 2.48 | 2.58 | 1.55 | 0.9 | 0.65 |
| 170 | K170Q | 0.51 | 1.32 | 1.22 | 1.05 | 0.8 | 0.63 |
| 170 | K170R | 1.13 | 1.12 | 1.08 | 1.05 | 1.1 | 1.14 |
| 170 | K170S | 0.51 | 1.30 | 1.13 | 0.90 | 0.9 | 0.75 |
| 170 | K170T | 0.29 | 1.48 | 1.50 | 1.06 | 0.5 | 0.48 |
| 170 | K170V | 0.62 | 1.13 | 1.17 | 1.08 | 0.8 | 0.78 |
| 170 | K170W | 0.30 | 1.08 | 1.13 | 0.97 | 0.8 | 0.48 |
| 170 | K170Y | 0.38 | 1.39 | 1.22 | 1.04 | 0.9 | 0.66 |
| 171 | Y171C | 0.17 | 12.33 | 8.58 | 1.82 | 0.8 | 0.12 |
| 171 | Y171F | 0.47 | 1.24 | 0.88 | 0.93 | 0.7 | 0.68 |
| 171 | Y171L | 0.18 | 4.11 | 3.35 | 2.44 | 0.3 | 0.25 |
| 171 | Y171W | 0.49 | 1.37 | 1.02 | 1.02 | 0.6 | 0.40 |
| 172 | P172A | 0.83 | 1.13 | 1.08 | 1.09 | 1.0 | 1.14 |
| 172 | P172C | 0.69 | 1.22 | 1.17 | 0.77 | 0.9 | 1.06 |
| 172 | P172E | 0.80 | 1.30 | 1.16 | 1.01 | 1.2 | 1.01 |
| 172 | P172F | 0.40 | 1.25 | 0.99 | 1.14 | 0.8 | 0.92 |
| 172 | P172G | 0.34 | 1.36 | 1.22 | 1.23 | 1.0 | 0.87 |
| 172 | P172H | 0.52 | 1.09 | 0.91 | 1.08 | 0.9 | 1.03 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 172 | P172I | 0.46 | 1.38 | 1.11 | 1.11 | 0.9 | 1.07 |
| 172 | P172K | 0.66 | 0.99 | 0.61 | 0.90 | 1.0 | 1.05 |
| 172 | P172L | 0.52 | 1.50 | 1.08 | 1.14 | 1.0 | 1.02 |
| 172 | P172M | 0.55 | 1.23 | 1.14 | 1.08 | 1.0 | 1.02 |
| 172 | P172N | 0.67 | 1.09 | 0.96 | 1.01 | 0.9 | 1.05 |
| 172 | P172Q | 0.75 | 1.17 | 1.02 | 0.90 | 1.0 | 1.19 |
| 172 | P172R | 0.52 | 0.84 | 0.51 | 0.80 | 1.0 | 0.99 |
| 172 | P172S | 0.64 | 1.17 | 1.02 | 1.20 | 1.0 | 1.06 |
| 172 | P172T | 0.58 | 1.27 | 1.04 | 1.15 | 0.8 | 1.24 |
| 172 | P172V | 0.54 | 1.37 | 1.18 | 1.24 | 0.8 | 1.15 |
| 172 | P172Y | 0.29 | 1.67 | 1.29 | 1.41 | 0.8 | 0.88 |
| 173 | S173A | 0.89 | 1.16 | 0.72 | 1.02 | 0.9 | 0.93 |
| 173 | S 173C | 0.81 | 1.19 | 0.89 | 1.05 | 0.9 | 0.98 |
| 173 | S173E | 0.96 | 0.90 | 0.78 | 0.98 | 1.0 | 0.95 |
| 173 | S173F | 0.51 | 1.07 | 1.17 | 0.87 | 0.6 | 0.82 |
| 173 | S 173G | 0.64 | 0.93 | 0.65 | 0.91 | 0.8 | 0.59 |
| 173 | S173H | 0.49 | 1.35 | 0.88 | 1.18 | 0.8 | 0.92 |
| 173 | S173I | 0.44 | 1.16 | 1.05 | 1.30 | 0.8 | 1.10 |
| 173 | S173K | 0.70 | 0.66 | 0.57 | 1.03 | 0.7 | 0.95 |
| 173 | S173L | 0.56 | 1.10 | 1.02 | 1.35 | 0.9 | 0.84 |
| 173 | S173M | 0.69 | 1.11 | 1.16 | 1.00 | 0.8 | 1.01 |
| 173 | S173P | 0.45 | 1.14 | 1.02 | 1.22 | 0.9 | 0.89 |
| 173 | S173Q | 0.67 | 1.28 | 0.84 | 1.21 | 0.9 | 1.03 |
| 173 | S173R | 0.45 | 0.98 | 0.65 | 1.14 | 0.6 | 0.93 |
| 173 | S173T | 0.82 | 1.08 | 0.81 | 1.15 | 1.0 | 1.00 |
| 173 | S173V | 0.79 | 1.09 | 0.79 | 1.10 | 1.0 | 1.09 |
| 173 | S173W | 0.33 | 1.89 | 1.29 | 1.50 | 0.6 | 0.90 |
| 173 | S173Y | 0.39 | 1.71 | 1.01 | 1.38 | 0.7 | 0.87 |
| 174 | V174A | 0.45 | 1.44 | 1.21 | 1.10 | 0.8 | 1.00 |
| 174 | V174C | 0.80 | 1.14 | 1.08 | 1.21 | 0.9 | 1.19 |
| 174 | V174I | 0.37 | 1.48 | 0.86 | 1.14 | 0.6 | 0.58 |
| 174 | V174L | 0.21 | 92.96 | 66.79 | 4.24 | 0.5 | 0.34 |
| 174 | V174S | 0.41 | 1.38 | 1.04 | 1.21 | 0.9 | 0.82 |
| 174 | V174T | 0.78 | 1.29 | 0.82 | 1.13 | 0.9 | 1.11 |
| 175 | I175A | 0.21 | 6.10 | 9.32 | 1.84 | 0.4 | 0.31 |
| 175 | I175C | 0.61 | 1.10 | 1.06 | 1.16 | 0.9 | 0.92 |
| 175 | I175F | 0.32 | 1.69 | 1.23 | 1.30 | 0.6 | 0.81 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 175 | I175L | 0.90 | 1.27 | 0.89 | 1.04 | 0.8 | 1.10 |
| 175 | I175M | 0.92 | 1.17 | 0.82 | 1.05 | 1.0 | 0.98 |
| 175 | I175T | 0.31 | 1.96 | 1.59 | 1.44 | 0.5 | 0.93 |
| 175 | I175V | 0.33 | 1.96 | 1.32 | 1.36 | 0.9 | 1.06 |
| 176 | A176C | 1.04 | 0.99 | 0.79 | 1.00 | 1.1 | 1.15 |
| 176 | A176G | 0.24 | 0.55 | 0.86 | 1.10 | 0.5 | 0.37 |
| 176 | A176S | 0.78 | 0.89 | 1.00 | 1.12 | 1.0 | 0.71 |
| 176 | A176T | 0.58 | 1.19 | 1.12 | 1.24 | 1.1 | 0.77 |
| 177 | V177A | 0.67 | 0.50 | 0.83 | 0.93 | 0.4 | 0.82 |
| 177 | V177C | 0.49 | 0.77 | 0.95 | 1.10 | 0.7 | 0.83 |
| 177 | V177I | 0.37 | 0.89 | 1.25 | 1.25 | 0.5 | 0.89 |
| 177 | V177S | 0.34 | 0.87 | 0.97 | 1.16 | 0.1 | 0.72 |
| 177 | V177T | 0.56 | 0.75 | 0.73 | 1.09 | 0.8 | 1.00 |
| 177 | V177W | 0.18 | 3.77 | 5.40 | 4.51 | 0.2 | 0.53 |
| 179 | A179G | 0.92 | 1.06 | 0.88 | 1.19 | 0.4 | 0.72 |
| 179 | A179M | 0.21 | 0.36 | 0.57 | 0.65 | 0.1 | 0.13 |
| 179 | A179S | 0.63 | 1.04 | 0.74 | 1.16 | 0.4 | 0.74 |
| 180 | V180A | 0.70 | 0.98 | 0.76 | 1.05 | 0.1 | 0.78 |
| 180 | V180C | 0.99 | 1.03 | 0.73 | 1.08 | 1.0 | 0.91 |
| 180 | V180H | 0.17 | 2.27 | 2.90 | 1.90 | 0.1 | 0.14 |
| 180 | V180L | 1.08 | 0.98 | 0.64 | 1.06 | 0.9 | 1.05 |
| 180 | V180S | 0.90 | 0.96 | 0.94 | 1.04 | 0.3 | 1.01 |
| 180 | V180T | 1.07 | 0.93 | 0.87 | 1.01 | 0.9 | 1.13 |
| 181 | D181C | 0.80 | 0.88 | 0.79 | 0.89 | 0.5 | 0.84 |
| 181 | D181E | 0.86 | 1.01 | 0.71 | 1.22 | 0.3 | 0.86 |
| 181 | D181N | 1.07 | 0.79 | 0.62 | 0.93 | 0.2 | 1.06 |
| 182 | S182A | 0.96 | 0.97 | 1.09 | 1.02 | 0.9 | 0.94 |
| 182 | S182C | 1.10 | 1.03 | 1.06 | 0.85 | 1.1 | 1.00 |
| 182 | S182D | 0.92 | 1.22 | 1.07 | 1.01 | 1.1 | 1.06 |
| 182 | S182F | 0.88 | 1.14 | 1.07 | 1.00 | 0.4 | 1.20 |
| 182 | S182G | 1.16 | 1.09 | 1.03 | 1.00 | 0.9 | 0.81 |
| 182 | S182H | 1.09 | 1.13 | 1.04 | 0.93 | 0.8 | 0.91 |
| 182 | S182I | 0.87 | 1.13 | 1.17 | 1.15 | 0.7 | 1.15 |
| 182 | S182L | 0.88 | 0.88 | 1.07 | 0.98 | 0.4 | 1.00 |
| 182 | S182M | 1.00 | 0.96 | 1.22 | 0.95 | 0.8 | 1.05 |
| 182 | S182P | 1.26 | 0.77 | 1.17 | 1.00 | 0.9 | 0.77 |
| 182 | S182Q | 0.94 | 1.28 | 1.04 | 0.98 | 1.0 | 1.08 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 182 | S182T | 0.95 | 0.82 | 1.19 | 0.92 | 0.8 | 0.98 |
| 182 | S182V | 0.79 | 1.02 | 1.31 | 1.07 | 0.8 | 1.19 |
| 182 | S182W | 0.32 | 1.48 | 1.36 | 1.36 | 0.3 | 1.04 |
| 182 | S182Y | 0.86 | 1.07 | 1.05 | 0.98 | 0.5 | 1.22 |
| 183 | S183A | 1.09 | 0.88 | 1.12 | 1.03 | 0.9 | 0.99 |
| 183 | S183C | 1.09 | 1.22 | 1.01 | 0.91 | 1.1 | 0.84 |
| 183 | S183E | 1.06 | 0.84 | 1.01 | 0.93 | 1.2 | 1.04 |
| 183 | S183F | 0.97 | 0.91 | 1.35 | 0.83 | 0.9 | 1.06 |
| 183 | S183G | 0.98 | 0.77 | 0.97 | 1.04 | 1.0 | 0.84 |
| 183 | S183H | 1.10 | 1.10 | 0.84 | 1.00 | 1.0 | 0.85 |
| 183 | S183I | 1.11 | 1.07 | 1.02 | 1.01 | 0.9 | 1.02 |
| 183 | S183K | 1.08 | 0.68 | 0.67 | 0.95 | 0.2 | 0.93 |
| 183 | S183L | 1.02 | 0.84 | 0.85 | 1.00 | 1.0 | 1.01 |
| 183 | S183M | 1.05 | 1.03 | 1.23 | 1.05 | 1.0 | 0.87 |
| 183 | S183N | 1.08 | 0.85 | 1.02 | 1.02 | 1.1 | 0.95 |
| 183 | S183Q | 0.99 | 0.66 | 1.23 | 0.98 | 1.1 | 0.95 |
| 183 | S183R | 1.06 | 0.67 | 0.70 | 0.95 | 0.1 | 0.93 |
| 183 | S183T | 0.92 | 1.20 | 1.25 | 1.02 | 1.0 | 1.12 |
| 183 | S183V | 0.90 | 1.20 | 1.10 | 0.93 | 1.0 | 1.11 |
| 183 | S183W | 0.71 | 1.03 | 1.00 | 1.06 | 0.7 | 1.26 |
| 183 | S183Y | 0.95 | 0.89 | 0.91 | 1.12 | 1.0 | 1.11 |
| 184 | N184A | 0.27 | 1.40 | 1.89 | 1.40 | 0.7 | 0.71 |
| 184 | N184C | 0.90 | 1.16 | 1.02 | 1.06 | 1.0 | 1.10 |
| 184 | N184D | 0.90 | 1.03 | 1.20 | 0.96 | 1.1 | 0.96 |
| 184 | N184E | 0.83 | 1.18 | 1.09 | 0.90 | 0.9 | 1.06 |
| 184 | N184F | 0.84 | 0.86 | 0.99 | 1.14 | 0.5 | 1.03 |
| 184 | N184G | 0.99 | 0.91 | 1.19 | 0.93 | 0.8 | 0.91 |
| 184 | N184H | 0.94 | 1.06 | 1.11 | 1.02 | 0.6 | 0.97 |
| 184 | N184I | 0.75 | 1.04 | 1.07 | 0.98 | 0.2 | 1.03 |
| 184 | N184L | 0.95 | 1.36 | 0.87 | 1.09 | 0.7 | 0.93 |
| 184 | N184M | 1.05 | 0.99 | 1.06 | 0.97 | 0.7 | 0.97 |
| 184 | N184P | 0.78 | 0.78 | 1.02 | 0.79 | 0.1 | 0.82 |
| 184 | N184Q | 0.82 | 0.78 | 1.10 | 1.10 | 0.6 | 1.05 |
| 184 | N184S | 0.88 | 1.02 | 0.97 | 1.06 | 0.7 | 1.04 |
| 184 | N184T | 0.80 | 1.16 | 1.14 | 0.90 | 0.4 | 1.19 |
| 184 | N184V | 0.72 | 1.30 | 1.07 | 1.02 | 0.3 | 1.02 |
| 184 | N184W | 0.80 | 0.92 | 1.28 | 0.99 | 0.4 | 1.26 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 184 | N184Y | 0.80 | 0.93 | 1.06 | 1.16 | 0.6 | 1.10 |
| 185 | Q185A | 1.01 | 0.84 | 1.15 | 1.06 | 0.9 | 1.08 |
| 185 | Q185C | 0.95 | 1.15 | 1.09 | 0.92 | 1.1 | 1.00 |
| 185 | Q185D | 0.29 | 2.02 | 1.65 | 1.42 | 0.6 | 0.56 |
| 185 | Q185E | 0.77 | 1.37 | 1.07 | 0.91 | 1.2 | 1.03 |
| 185 | Q185F | 0.84 | 1.05 | 1.09 | 0.94 | 0.6 | 1.14 |
| 185 | Q185G | 0.74 | 0.79 | 1.29 | 1.00 | 0.7 | 1.02 |
| 185 | Q185H | 1.00 | 0.88 | 0.91 | 1.02 | 0.9 | 1.09 |
| 185 | Q185I | 0.87 | 1.46 | 0.98 | 0.95 | 0.9 | 1.24 |
| 185 | Q185K | 0.98 | 0.68 | 0.73 | 0.85 | 0.7 | 0.97 |
| 185 | Q185L | 0.87 | 0.99 | 0.96 | 1.04 | 0.7 | 1.00 |
| 185 | Q185M | 0.93 | 0.91 | 1.09 | 1.05 | 1.0 | 1.01 |
| 185 | Q185N | 0.97 | 1.14 | 1.11 | 1.08 | 1.1 | 1.05 |
| 185 | Q185R | 0.91 | 0.75 | 0.80 | 0.99 | 0.4 | 1.07 |
| 185 | Q185S | 0.83 | 1.38 | 1.04 | 1.06 | 0.8 | 1.06 |
| 185 | Q185T | 0.82 | 1.36 | 1.28 | 1.13 | 1.0 | 1.17 |
| 185 | Q185V | 0.94 | 1.04 | 1.30 | 0.99 | 0.8 | 1.16 |
| 185 | Q185W | 0.81 | 0.85 | 0.85 | 0.87 | 0.1 | 1.14 |
| 185 | Q185Y | 0.74 | 0.95 | 1.05 | 1.26 | 0.7 | 1.28 |
| 186 | R186A | 0.38 | 1.03 | 1.51 | 1.09 | 0.7 | 0.45 |
| 186 | R186C | 0.36 | 1.91 | 1.61 | 1.05 | 0.6 | 0.47 |
| 186 | R186E | 0.21 | 23.12 | 23.90 | 1.79 | 0.1 | 0.11 |
| 186 | R186F | 0.33 | 1.32 | 1.44 | 0.88 | 0.2 | 0.29 |
| 186 | R186G | 0.31 | 2.21 | 1.88 | 1.19 | 0.2 | 0.60 |
| 186 | R186H | 0.50 | 1.13 | 1.34 | 1.17 | 0.7 | 0.73 |
| 186 | R186I | 0.38 | 1.34 | 2.12 | 1.26 | 0.9 | 0.80 |
| 186 | R186K | 0.62 | 0.95 | 1.19 | 1.00 | 0.6 | 0.92 |
| 186 | R186L | 0.54 | 1.58 | 1.44 | 1.20 | 0.9 | 0.89 |
| 186 | R186N | 0.25 | 2.46 | 3.74 | 1.52 | 0.3 | 0.43 |
| 186 | R186P | 0.24 | 2.96 | 2.29 | 0.95 | 0.1 | 0.19 |
| 186 | R186Q | 0.37 | 1.48 | 2.05 | 1.32 | 0.8 | 0.85 |
| 186 | R186S | 0.27 | 2.44 | 1.91 | 1.39 | 0.3 | 0.40 |
| 186 | R186T | 0.22 | 8.56 | 6.97 | 2.69 | 0.4 | 0.36 |
| 186 | R186V | 0.42 | 2.00 | 2.04 | 1.42 | 0.9 | 0.75 |
| 186 | R186W | 0.63 | 1.54 | 1.71 | 1.12 | 1.1 | 0.98 |
| 186 | R186Y | 0.38 | 1.50 | 1.98 | 1.37 | 0.8 | 0.71 |
| 187 | A187C | 0.77 | 0.75 | 1.06 | 0.84 | 0.2 | 0.31 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 187 | A187D | 0.93 | 1.51 | 0.99 | 1.03 | 0.4 | 0.17 |
| 187 | A187E | 0.72 | 1.41 | 1.49 | 1.00 | 0.2 | 0.34 |
| 187 | A187F | 0.73 | 1.02 | 1.07 | 0.93 | 0.1 | 0.43 |
| 187 | A187G | 0.90 | 1.04 | 1.12 | 0.93 | 0.6 | 1.05 |
| 187 | A187N | 0.74 | 1.11 | 1.39 | 1.03 | 0.5 | 1.22 |
| 187 | A187P | 1.08 | 1.15 | 1.12 | 0.92 | 0.7 | 0.69 |
| 187 | A187S | 0.68 | 1.14 | 1.13 | 1.06 | 0.4 | 0.94 |
| 187 | A187W | 0.93 | 0.95 | 0.99 | 1.07 | 0.7 | 0.41 |
| 187 | A187Y | 1.02 | 1.05 | 1.25 | 0.79 | 0.5 | 0.40 |
| 188 | S188A | 1.14 | 0.93 | 1.08 | 0.96 | 0.9 | 0.92 |
| 188 | S188C | 1.24 | 1.07 | 1.27 | 0.81 | 1.0 | 0.85 |
| 188 | S188D | 1.08 | 1.03 | 1.33 | 0.94 | 1.2 | 0.86 |
| 188 | S188E | 1.03 | 1.18 | 1.29 | 0.98 | 1.1 | 1.00 |
| 188 | S188F | 0.91 | 0.98 | 1.08 | 0.86 | 0.8 | 0.92 |
| 188 | S188G | 1.06 | 0.99 | 1.09 | 0.94 | 0.9 | 0.89 |
| 188 | S188H | 1.01 | 0.92 | 1.00 | 0.91 | 0.9 | 1.01 |
| 188 | S188I | 0.94 | 0.79 | 1.24 | 1.01 | 0.9 | 0.96 |
| 188 | S 188K | 1.14 | 0.64 | 0.89 | 1.00 | 0.5 | 1.05 |
| 188 | S188L | 1.01 | 1.07 | 1.16 | 0.82 | 0.8 | 0.98 |
| 188 | S188M | 0.98 | 0.73 | 1.28 | 0.90 | 0.8 | 0.98 |
| 188 | S188P | 1.05 | 1.05 | 1.23 | 1.01 | 1.0 | 0.90 |
| 188 | S188Q | 0.97 | 1.01 | 1.17 | 1.00 | 0.9 | 1.03 |
| 188 | S 188R | 0.48 | 0.91 | 1.28 | 1.13 | 0.5 | 1.00 |
| 188 | S188T | 0.82 | 0.95 | 1.37 | 1.01 | 0.9 | 1.08 |
| 188 | S188V | 0.84 | 0.97 | 1.25 | 0.96 | 0.8 | 1.15 |
| 188 | S188W | 0.80 | 0.63 | 1.04 | 0.70 | 0.8 | 0.81 |
| 188 | S188Y | 0.77 | 0.90 | 1.11 | 0.86 | 0.9 | 0.94 |
| 189 | F189C | 1.16 | 0.82 | 0.77 | 0.86 | 0.3 | 0.15 |
| 189 | F189D | 2.06 | 1.03 | 0.88 | 0.94 | 0.4 | 0.28 |
| 189 | F189M | 1.67 | 1.01 | 0.89 | 1.02 | 0.1 | 0.40 |
| 189 | F189N | 1.31 | 0.83 | 0.77 | 0.82 | 0.1 | 0.83 |
| 189 | F189S | 0.93 | 0.91 | 1.08 | 1.06 | 0.1 | 0.81 |
| 189 | F189T | 0.74 | 1.01 | 1.10 | 0.99 | 0.1 | 0.92 |
| 189 | F189W | 0.59 | 0.57 | 0.71 | 0.81 | 1.0 | 0.54 |
| 189 | F189Y | 0.66 | 0.62 | 0.86 | 0.96 | 0.5 | 0.53 |
| 190 | S190A | 0.35 | 1.19 | 1.27 | 0.80 | 0.1 | 0.57 |
| 190 | S190C | 0.62 | 0.82 | 1.11 | 0.69 | 0.6 | 0.68 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 190 | S190G | 0.27 | 1.50 | 2.17 | 1.30 | 0.1 | 0.56 |
| 190 | S190N | 0.23 | 1.60 | 2.04 | 1.06 | 0.1 | 0.12 |
| 190 | S190T | 0.31 | 1.03 | 1.36 | 1.15 | 0.3 | 0.49 |
| 191 | Q191A | 0.93 | 0.67 | 1.11 | 0.98 | 0.9 | 0.66 |
| 191 | Q191C | 0.44 | 0.72 | 0.91 | 0.96 | 0.3 | 0.32 |
| 191 | Q191G | 0.77 | 0.75 | 0.73 | 0.74 | 0.7 | 0.33 |
| 191 | Q191N | 0.72 | 0.70 | 1.34 | 1.14 | 0.1 | 0.56 |
| 191 | Q191T | 0.32 | 1.03 | 0.92 | 0.82 | 0.1 | 0.10 |
| 191 | Q191V | 0.33 | 0.98 | 0.62 | 0.74 | 0.1 | 0.09 |
| 192 | Y192A | 0.86 | 0.94 | 1.08 | 0.97 | 0.8 | 1.08 |
| 192 | Y192D | 0.24 | 1.91 | 2.19 | 1.17 | 0.1 | 0.11 |
| 192 | Y192F | 0.51 | 0.70 | 0.98 | 0.87 | 0.4 | 0.55 |
| 192 | Y192G | 0.43 | 0.66 | 0.98 | 0.99 | 0.2 | 0.47 |
| 192 | Y192H | 0.45 | 0.85 | 0.66 | 0.82 | 0.2 | 0.49 |
| 192 | Y192M | 0.41 | 0.90 | 0.93 | 0.84 | 0.1 | 0.39 |
| 192 | Y192N | 0.33 | 1.16 | 1.47 | 1.01 | 0.1 | 0.66 |
| 192 | Y192P | 0.34 | 0.88 | 0.38 | 0.60 | 0.1 | 0.13 |
| 192 | Y192Q | 0.36 | 2.14 | 1.27 | 1.17 | 0.2 | 0.56 |
| 192 | Y192S | 0.63 | 0.82 | 1.15 | 0.93 | 0.7 | 0.88 |
| 192 | Y192T | 0.72 | 0.73 | 1.08 | 0.98 | 0.9 | 0.93 |
| 194 | P194A | 0.69 | 1.09 | 0.94 | 0.95 | 0.8 | 1.16 |
| 194 | P194C | 0.75 | 1.19 | 1.18 | 1.19 | 1.0 | 1.41 |
| 194 | P194E | 0.70 | 1.52 | 1.09 | 1.03 | 1.0 | 1.25 |
| 194 | P194G | 0.48 | 1.23 | 0.92 | 1.12 | 0.7 | 0.81 |
| 194 | P194H | 0.76 | 1.13 | 1.00 | 1.15 | 1.0 | 1.03 |
| 194 | P194I | 0.48 | 1.63 | 1.09 | 1.21 | 0.9 | 1.12 |
| 194 | P194K | 0.72 | 0.79 | 0.90 | 0.90 | 0.6 | 0.98 |
| 194 | P194L | 0.58 | 1.58 | 0.87 | 1.29 | 0.9 | 1.09 |
| 194 | P194M | 0.60 | 1.36 | 1.09 | 1.06 | 0.8 | 1.47 |
| 194 | P194N | 0.52 | 1.82 | 1.00 | 1.23 | 0.1 | 1.69 |
| 194 | P194Q | 0.60 | 1.24 | 1.02 | 1.16 | 0.8 | 1.09 |
| 194 | P194S | 0.69 | 1.19 | 0.94 | 1.13 | 0.8 | 1.10 |
| 194 | P194T | 0.58 | 1.32 | 1.37 | 1.10 | 0.9 | 1.21 |
| 194 | P194V | 0.48 | 1.67 | 1.08 | 1.18 | 0.8 | 1.36 |
| 194 | P194W | 0.57 | 1.27 | 1.17 | 1.10 | 0.9 | 1.16 |
| 195 | E195A | 0.26 | 0.78 | 0.71 | 0.64 | 0.1 | 0.26 |
| 195 | E195C | 0.25 | 1.37 | 0.92 | 0.57 | 0.4 | 0.22 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 195 | E195D | 0.34 | 1.09 | 0.84 | 0.86 | 0.7 | 0.38 |
| 195 | E195G | 0.71 | 0.95 | 0.44 | 0.77 | 0.3 | 0.52 |
| 195 | E195H | 0.26 | 0.67 | 0.38 | 0.48 | 0.1 | 0.08 |
| 195 | E195K | 0.22 | 3.69 | 2.57 | 2.17 | 0.3 | 0.47 |
| 195 | E195Q | 0.30 | 1.29 | 0.69 | 1.02 | 0.2 | 0.69 |
| 195 | E195W | 0.22 | 4.29 | 1.26 | 1.32 | 0.1 | 0.10 |
| 196 | L196I | 0.38 | 1.68 | 1.15 | 1.17 | 0.8 | 1.03 |
| 196 | L196M | 0.37 | 1.52 | 1.04 | 1.06 | 0.2 | 0.72 |
| 196 | L196T | 0.24 | 3.86 | 2.91 | 2.68 | 1.0 | 0.69 |
| 196 | L196V | 0.34 | 1.95 | 1.28 | 1.45 | 0.7 | 0.79 |
| 197 | D197A | 0.45 | 0.74 | 1.09 | 1.05 | 0.5 | 0.66 |
| 197 | D197C | 0.38 | 1.02 | 1.11 | 0.97 | 0.3 | 0.55 |
| 197 | D197G | 0.37 | 1.06 | 1.07 | 1.18 | 0.3 | 0.60 |
| 197 | D197M | 0.18 | 1.52 | 0.68 | 1.10 | 0.1 | 0.09 |
| 197 | D197N | 0.86 | 0.93 | 1.16 | 0.88 | 0.5 | 0.85 |
| 197 | D197S | 0.28 | 0.18 | 0.28 | 0.35 | 0.9 | 0.11 |
| 197 | D197T | 0.53 | 0.94 | 1.20 | 1.14 | 0.5 | 0.88 |
| 198 | V198A | 0.58 | 0.88 | 0.86 | 1.11 | 0.2 | 0.96 |
| 198 | V198C | 0.84 | 0.78 | 1.11 | 1.00 | 0.8 | 0.94 |
| 198 | V198D | 0.20 | 1.23 | 1.82 | 1.45 | 1.1 | 0.34 |
| 198 | V198F | 0.76 | 0.78 | 0.72 | 1.00 | 0.9 | 0.76 |
| 198 | V198H | 0.20 | 0.57 | 1.01 | 0.96 | 0.2 | 0.18 |
| 198 | V198I | 0.83 | 0.41 | 0.94 | 0.98 | 1.0 | 0.87 |
| 198 | V198L | 0.90 | 0.67 | 0.73 | 0.94 | 1.0 | 1.13 |
| 198 | V198M | 0.75 | 0.78 | 1.03 | 1.08 | 0.8 | 0.96 |
| 198 | V198T | 0.42 | 0.99 | 1.05 | 1.06 | 0.2 | 1.05 |
| 198 | V198Y | 0.53 | 0.67 | 1.19 | 1.12 | 0.8 | 1.00 |
| 199 | M199A | 0.49 | 1.04 | 0.94 | 1.10 | 0.1 | 0.79 |
| 199 | M199C | 0.64 | 0.88 | 0.85 | 0.80 | 0.7 | 0.66 |
| 199 | M199F | 0.17 | 28.65 | 28.81 | 2.58 | 0.1 | 0.23 |
| 199 | M199G | 0.18 | 1.09 | 0.55 | 0.93 | 0.1 | 0.12 |
| 199 | M199Q | 0.23 | 2.11 | 1.65 | 1.69 | 0.8 | 0.63 |
| 199 | M199S | 0.54 | 1.12 | 0.88 | 1.00 | 0.3 | 0.93 |
| 199 | M199T | 0.53 | 1.22 | 1.06 | 1.02 | 0.3 | 0.87 |
| 199 | M199V | 0.66 | 1.14 | 0.97 | 0.96 | 0.9 | 1.05 |
| 200 | A200C | 0.70 | 1.32 | 0.86 | 1.16 | 0.6 | 1.17 |
| 200 | A200G | 0.83 | 1.19 | 1.05 | 1.08 | 0.6 | 1.08 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 200 | A200N | 0.47 | 1.33 | 1.20 | 1.17 | 0.7 | 1.28 |
| 200 | A200S | 0.72 | 1.23 | 0.80 | 1.07 | 0.5 | 0.91 |
| 200 | A200T | 0.35 | 1.73 | 0.95 | 1.35 | 0.2 | 0.71 |
| 201 | P201A | 0.65 | 0.61 | 0.87 | 1.08 | 0.9 | 0.79 |
| 201 | P201C | 0.47 | 1.07 | 0.95 | 1.30 | 0.2 | 1.06 |
| 201 | P201G | 0.54 | 0.42 | 1.08 | 1.21 | 0.6 | 0.91 |
| 201 | P201H | 0.40 | 0.65 | 1.21 | 1.15 | 0.1 | 0.90 |
| 201 | P201I | 0.18 | 1.05 | 0.64 | 1.28 | 0.1 | 0.12 |
| 201 | P201N | 0.41 | 1.05 | 1.26 | 1.38 | 0.1 | 0.75 |
| 201 | P201S | 0.43 | 0.66 | 1.21 | 1.35 | 0.1 | 1.06 |
| 201 | P201Y | 0.58 | 0.73 | 0.91 | 1.11 | 0.7 | 1.17 |
| 203 | V203A | 0.51 | 1.07 | 1.26 | 1.24 | 0.3 | 1.48 |
| 203 | V203C | 0.93 | 1.10 | 1.10 | 0.83 | 1.0 | 0.66 |
| 203 | V203D | 0.54 | 1.12 | 1.62 | 1.23 | 1.1 | 1.09 |
| 203 | V203E | 0.57 | 0.88 | 1.05 | 1.13 | 1.1 | 0.88 |
| 203 | V203G | 0.17 | 8.72 | 7.71 | 4.86 | 0.1 | 0.33 |
| 203 | V203H | 0.59 | 0.87 | 0.81 | 0.89 | 0.1 | 0.30 |
| 203 | V203I | 0.81 | 0.63 | 1.05 | 0.93 | 0.8 | 0.77 |
| 203 | V203M | 0.77 | 0.98 | 1.14 | 1.00 | 0.6 | 0.99 |
| 203 | V203N | 0.47 | 1.05 | 1.38 | 1.22 | 0.1 | 0.99 |
| 203 | V203Q | 0.62 | 0.90 | 1.18 | 1.13 | 0.5 | 1.19 |
| 203 | V203S | 0.55 | 0.93 | 1.10 | 1.04 | 0.2 | 1.07 |
| 203 | V203T | 0.65 | 0.90 | 1.31 | 1.07 | 0.6 | 1.01 |
| 203 | V203Y | 0.43 | 0.94 | 1.21 | 1.05 | 0.1 | 0.97 |
| 204 | S204A | 0.97 | 0.86 | 1.00 | 0.81 | 1.2 | 1.06 |
| 204 | S204C | 0.90 | 1.00 | 1.05 | 0.89 | 1.4 | 0.85 |
| 204 | S204F | 0.86 | 0.39 | 0.60 | 0.82 | 0.7 | 1.06 |
| 204 | S204G | 0.88 | 1.01 | 1.19 | 0.75 | 1.1 | 1.50 |
| 204 | S204H | 0.68 | 0.72 | 0.81 | 0.95 | 1.0 | 1.21 |
| 204 | S204I | 0.66 | 0.79 | 0.99 | 0.83 | 0.7 | 0.91 |
| 204 | S204K | 0.90 | 0.74 | 0.64 | 0.87 | 0.1 | 0.93 |
| 204 | S204L | 0.77 | 0.85 | 0.82 | 0.93 | 0.9 | 0.90 |
| 204 | S204M | 0.90 | 0.75 | 1.10 | 0.95 | 0.8 | 0.90 |
| 204 | S204Q | 0.87 | 0.84 | 1.06 | 0.94 | 1.3 | 0.96 |
| 204 | S204T | 0.92 | 0.78 | 0.86 | 0.80 | 0.9 | 1.09 |
| 204 | S204V | 0.82 | 0.71 | 0.79 | 0.85 | 0.7 | 1.20 |
| 204 | S204W | 0.74 | 0.39 | 0.67 | 0.79 | 0.6 | 0.97 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 204 | S204Y | 0.75 | 0.52 | 1.04 | 0.87 | 0.7 | 1.03 |
| 205 | I205T | 0.77 | 1.60 | 1.20 | 1.11 | 0.8 | 1.29 |
| 205 | I205V | 0.95 | 1.25 | 1.08 | 1.11 | 1.3 | 1.03 |
| 206 | Q206A | 1.09 | 0.84 | 1.24 | 1.05 | 0.7 | 0.72 |
| 206 | Q206C | 1.10 | 1.13 | 1.03 | 1.12 | 1.4 | 0.94 |
| 206 | Q206D | 1.02 | 1.23 | 1.04 | 1.01 | 1.3 | 0.84 |
| 206 | Q206E | 1.01 | 1.26 | 1.14 | 1.16 | 1.4 | 0.97 |
| 206 | Q206H | 1.06 | 1.00 | 0.97 | 1.11 | 1.0 | 0.92 |
| 206 | Q206I | 1.12 | 1.00 | 0.99 | 1.04 | 0.5 | 0.87 |
| 206 | Q206K | 1.11 | 0.84 | 0.75 | 1.13 | 0.1 | 0.86 |
| 206 | Q206L | 1.03 | 0.89 | 0.93 | 0.94 | 0.7 | 1.02 |
| 206 | Q206M | 0.77 | 1.09 | 0.93 | 1.06 | 0.8 | 0.95 |
| 206 | Q206N | 1.03 | 1.07 | 1.08 | 1.06 | 1.1 | 0.91 |
| 206 | Q206P | 0.94 | 1.01 | 1.00 | 1.02 | 0.9 | 0.86 |
| 206 | Q206S | 0.92 | 1.21 | 0.98 | 1.00 | 0.9 | 0.97 |
| 206 | Q206T | 0.99 | 0.91 | 0.95 | 1.10 | 0.8 | 0.92 |
| 206 | Q206V | 1.01 | 1.04 | 1.19 | 1.11 | 0.6 | 1.07 |
| 206 | Q206Y | 1.04 | 0.96 | 0.96 | 1.18 | 0.9 | 0.93 |
| 208 | T208A | 0.89 | 1.28 | 1.15 | 1.12 | 0.1 | 1.02 |
| 208 | T208C | 0.82 | 1.48 | 1.13 | 1.24 | 0.8 | 1.14 |
| 208 | T208D | 0.27 | 0.78 | 0.73 | 0.35 | 0.2 | 0.20 |
| 208 | T208M | 0.22 | 5.66 | 6.03 | 1.79 | 0.4 | 0.37 |
| 208 | T208S | 0.81 | 1.20 | 1.16 | 1.01 | 0.2 | 1.24 |
| 209 | L209A | 1.06 | 0.51 | 0.27 | 0.77 | 0.6 | 0.43 |
| 209 | L209C | 0.82 | 1.41 | 1.04 | 1.05 | 0.9 | 1.53 |
| 209 | L209E | 0.98 | 0.69 | 0.36 | 0.76 | 0.8 | 0.71 |
| 209 | L209F | 0.72 | 1.26 | 1.05 | 1.15 | 0.2 | 2.25 |
| 209 | L209G | 1.00 | 0.51 | 0.33 | 0.60 | 0.3 | 0.26 |
| 209 | L209H | 0.64 | 0.45 | 0.25 | 0.62 | 0.6 | 0.14 |
| 209 | L209K | 0.88 | 0.81 | 0.69 | 1.05 | 0.3 | 1.20 |
| 209 | L209M | 0.71 | 1.26 | 1.09 | 1.16 | 0.8 | 1.83 |
| 209 | L209Q | 0.84 | 0.97 | 0.44 | 0.89 | 0.7 | 0.99 |
| 209 | L209S | 0.99 | 0.52 | 0.27 | 0.63 | 0.5 | 0.36 |
| 209 | L209T | 0.81 | 0.77 | 0.35 | 0.82 | 0.5 | 0.88 |
| 209 | L209V | 0.83 | 1.20 | 1.01 | 1.00 | 0.6 | 0.82 |
| 209 | L209W | 0.78 | 1.43 | 0.96 | 1.11 | 0.3 | 1.27 |
| 209 | L209Y | 0.62 | 0.95 | 0.64 | 1.01 | 0.1 | 1.47 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 210 | P210A | 0.66 | 1.12 | 0.81 | 0.89 | 0.3 | 0.93 |
| 210 | P210C | 0.42 | 1.52 | 0.90 | 0.96 | 0.2 | 0.75 |
| 210 | P210D | 0.32 | 1.40 | 0.78 | 1.18 | 0.1 | 0.26 |
| 210 | P210E | 0.37 | 1.67 | 1.08 | 1.31 | 0.4 | 0.69 |
| 210 | P210F | 0.21 | 10.28 | 3.47 | 2.00 | 0.1 | 0.11 |
| 210 | P210S | 0.58 | 1.35 | 1.10 | 1.15 | 0.4 | 1.25 |
| 210 | P210T | 0.55 | 1.15 | 1.10 | 1.34 | 0.3 | 1.10 |
| 210 | P210V | 0.49 | 1.36 | 1.24 | 1.13 | 0.2 | 0.75 |
| 211 | G211A | 0.90 | 1.54 | 1.09 | 1.04 | 1.0 | 0.92 |
| 211 | G211C | 1.13 | 0.68 | 1.18 | 0.94 | 1.1 | 0.86 |
| 211 | G211D | 0.95 | 1.52 | 1.15 | 0.99 | 1.4 | 0.95 |
| 211 | G211E | 0.92 | 1.31 | 1.08 | 1.10 | 1.3 | 0.90 |
| 211 | G211F | 0.85 | 1.21 | 1.09 | 1.15 | 0.6 | 1.08 |
| 211 | G211K | 0.90 | 1.05 | 0.90 | 0.95 | 0.4 | 1.03 |
| 211 | G211L | 0.67 | 1.22 | 1.18 | 1.12 | 0.7 | 0.86 |
| 211 | G211M | 0.88 | 1.22 | 1.21 | 0.99 | 0.9 | 0.96 |
| 211 | G211N | 0.92 | 1.29 | 1.12 | 1.00 | 1.0 | 0.99 |
| 211 | G211P | 0.86 | 1.32 | 1.66 | 1.01 | 0.6 | 0.99 |
| 211 | G211Q | 0.77 | 1.26 | 1.01 | 1.18 | 1.0 | 1.07 |
| 211 | G211R | 0.76 | 0.67 | 0.81 | 0.96 | 0.2 | 0.92 |
| 211 | G211S | 0.95 | 1.25 | 1.18 | 1.01 | 0.9 | 1.01 |
| 211 | G211T | 0.78 | 1.37 | 1.35 | 1.15 | 0.7 | 1.07 |
| 211 | G211V | 0.76 | 1.33 | 1.20 | 0.92 | 0.5 | 0.93 |
| 211 | G211W | 0.80 | 1.33 | 1.08 | 1.18 | 0.2 | 1.04 |
| 211 | G211Y | 0.86 | 1.01 | 1.27 | 1.16 | 0.8 | 1.04 |
| 212 | N212A | 1.18 | 0.98 | 1.29 | 0.99 | 0.8 | 0.67 |
| 212 | N212C | 1.06 | 1.04 | 1.20 | 0.92 | 0.9 | 1.13 |
| 212 | N212E | 0.97 | 1.44 | 1.12 | 0.93 | 1.2 | 0.75 |
| 212 | N212F | 1.08 | 0.91 | 1.01 | 0.95 | 0.5 | 0.75 |
| 212 | N212G | 0.80 | 1.14 | 1.32 | 0.98 | 0.9 | 0.92 |
| 212 | N212H | 0.95 | 1.22 | 1.11 | 0.94 | 0.6 | 1.21 |
| 212 | N212I | 0.49 | 0.41 | 0.41 | 0.24 | 0.5 | 0.20 |
| 212 | N212K | 0.79 | 0.82 | 0.75 | 0.99 | 0.1 | 0.85 |
| 212 | N212L | 0.83 | 0.63 | 0.69 | 0.76 | 0.2 | 0.75 |
| 212 | N212M | 1.06 | 1.08 | 1.04 | 1.09 | 0.5 | 0.87 |
| 212 | N212P | 0.99 | 0.96 | 0.85 | 1.00 | 0.3 | 0.78 |
| 212 | N212Q | 0.95 | 0.97 | 0.80 | 0.89 | 0.9 | 0.98 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 212 | N212R | 0.83 | 0.74 | 0.79 | 1.06 | 0.2 | 0.83 |
| 212 | N212S | 1.09 | 1.12 | 0.96 | 1.03 | 0.8 | 0.77 |
| 212 | N212T | 0.23 | 6.10 | 4.36 | 2.96 | 0.6 | 1.31 |
| 212 | N212V | 0.77 | 0.97 | 0.99 | 1.08 | 0.6 | 0.83 |
| 212 | N212Y | 0.93 | 1.05 | 0.85 | 0.96 | 0.4 | 0.98 |
| 213 | K213A | 1.05 | 1.23 | 1.22 | 0.95 | 1.4 | 0.91 |
| 213 | K213C | 0.96 | 1.03 | 1.03 | 0.86 | 1.4 | 0.99 |
| 213 | K213D | 1.01 | 1.13 | 1.20 | 0.89 | 1.4 | 0.86 |
| 213 | K213E | 0.96 | 0.99 | 1.09 | 0.89 | 1.5 | 0.84 |
| 213 | K213F | 0.92 | 1.20 | 1.26 | 0.98 | 1.1 | 0.93 |
| 213 | K213H | 1.01 | 1.29 | 1.07 | 0.97 | 1.3 | 1.09 |
| 213 | K213I | 1.02 | 1.01 | 1.14 | 0.94 | 1.2 | 0.96 |
| 213 | K213L | 1.03 | 1.13 | 1.17 | 1.03 | 1.3 | 1.17 |
| 213 | K213M | 1.06 | 1.13 | 1.17 | 1.04 | 1.5 | 1.03 |
| 213 | K213N | 1.02 | 1.26 | 1.17 | 1.00 | 1.7 | 0.95 |
| 213 | K213Q | 1.04 | 1.36 | 1.01 | 0.97 | 1.5 | 1.04 |
| 213 | K213R | 0.78 | 1.24 | 1.15 | 0.83 | 1.2 | 0.98 |
| 213 | K213S | 0.82 | 1.17 | 1.07 | 0.96 | 1.5 | 0.85 |
| 213 | K213T | 0.91 | 1.35 | 1.21 | 0.85 | 1.5 | 0.94 |
| 213 | K213V | 0.85 | 1.16 | 1.16 | 1.06 | 1.1 | 0.99 |
| 213 | K213W | 0.80 | 1.11 | 1.10 | 0.98 | 0.9 | 1.13 |
| 213 | K213Y | 0.85 | 1.15 | 1.01 | 0.97 | 1.3 | 0.96 |
| 214 | Y214A | 0.17 | 2.41 | 1.57 | 0.71 | 0.2 | 0.07 |
| 214 | Y214D | 0.18 | 3.22 | 3.18 | 1.71 | 0.2 | 0.12 |
| 214 | Y214N | 0.19 | 1.65 | 1.59 | 1.29 | 0.7 | 0.18 |
| 214 | Y214S | 0.18 | 2.22 | 1.06 | 1.50 | 0.2 | 0.10 |
| 215 | G215A | 0.99 | 1.16 | 0.99 | 0.98 | 0.6 | 0.58 |
| 215 | G215C | 1.00 | 1.03 | 1.06 | 0.82 | 0.8 | 0.70 |
| 215 | G215D | 0.89 | 1.33 | 1.10 | 1.06 | 1.0 | 0.70 |
| 215 | G215E | 1.04 | 1.22 | 1.16 | 0.84 | 1.0 | 0.75 |
| 215 | G215H | 0.64 | 1.16 | 0.76 | 1.05 | 0.1 | 0.72 |
| 215 | G215M | 0.72 | 1.23 | 1.00 | 0.82 | 0.1 | 0.59 |
| 215 | G215Q | 0.80 | 1.37 | 0.96 | 0.97 | 0.1 | 0.67 |
| 215 | G215S | 0.84 | 1.12 | 0.94 | 0.93 | 0.1 | 0.72 |
| 215 | G215V | 0.36 | 1.52 | 0.90 | 1.15 | 0.1 | 0.51 |
| 215 | G215W | 0.48 | 1.00 | 0.88 | 0.91 | 0.1 | 0.71 |
| 216 | A216C | 0.97 | 1.21 | 1.11 | 0.92 | 1.2 | 0.89 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 216 | A216D | 0.76 | 1.22 | 1.28 | 1.19 | 1.2 | 0.95 |
| 216 | A216E | 1.20 | 1.36 | 0.92 | 1.03 | 1.4 | 0.75 |
| 216 | A216F | 1.22 | 1.20 | 1.21 | 0.87 | 1.2 | 0.91 |
| 216 | A216G | 1.00 | 0.99 | 0.78 | 0.88 | 0.8 | 0.90 |
| 216 | A216H | 1.03 | 0.86 | 1.01 | 0.93 | 0.9 | 0.79 |
| 216 | A216I | 1.17 | 0.92 | 0.94 | 1.01 | 1.0 | 0.81 |
| 216 | A216K | 0.94 | 0.78 | 0.98 | 0.87 | 1.0 | 0.55 |
| 216 | A216L | 0.96 | 0.90 | 0.78 | 0.83 | 0.9 | 1.05 |
| 216 | A216M | 0.82 | 0.85 | 1.07 | 0.90 | 1.1 | 0.82 |
| 216 | A216N | 1.03 | 0.97 | 1.11 | 1.07 | 1.1 | 0.72 |
| 216 | A216P | 0.90 | 1.09 | 0.94 | 1.11 | 1.2 | 1.00 |
| 216 | A216Q | 1.03 | 0.96 | 1.14 | 1.09 | 0.9 | 0.94 |
| 216 | A216R | 1.12 | 0.54 | 0.62 | 0.92 | 0.1 | 0.90 |
| 216 | A216S | 0.73 | 1.00 | 1.09 | 1.22 | 0.9 | 1.03 |
| 216 | A216V | 0.92 | 1.13 | 0.93 | 0.94 | 0.9 | 1.15 |
| 216 | A216W | 0.96 | 0.55 | 0.86 | 0.77 | 0.9 | 0.73 |
| 216 | A216Y | 0.81 | 0.76 | 0.90 | 0.96 | 1.1 | 0.88 |
| 217 | Y217C | 1.03 | 0.98 | 0.86 | 0.95 | 1.6 | 1.34 |
| 217 | Y217D | 1.01 | 1.13 | 1.07 | 1.21 | 1.1 | 0.34 |
| 217 | Y217E | 1.00 | 1.40 | 0.99 | 1.18 | 1.3 | 0.54 |
| 217 | Y217F | 1.04 | 0.99 | 1.03 | 0.97 | 1.0 | 1.35 |
| 217 | Y217G | 0.85 | 0.81 | 0.72 | 0.95 | 1.0 | 0.97 |
| 217 | Y217H | 0.96 | 0.97 | 0.90 | 1.15 | 1.1 | 0.76 |
| 217 | Y217I | 0.84 | 1.16 | 1.24 | 1.14 | 0.7 | 1.53 |
| 217 | Y217K | 1.02 | 0.80 | 0.58 | 0.97 | 1.0 | 1.06 |
| 217 | Y217L | 0.98 | 1.34 | 0.97 | 1.07 | 1.4 | 3.46 |
| 217 | Y217M | 0.93 | 1.31 | 1.22 | 1.07 | 1.5 | 1.80 |
| 217 | Y217N | 0.78 | 0.94 | 0.65 | 0.91 | 1.0 | 1.21 |
| 217 | Y217P | 0.28 | 0.54 | 0.18 | 0.57 | 0.1 | 0.18 |
| 217 | Y217Q | 0.92 | 1.19 | 1.29 | 1.11 | 1.2 | 1.05 |
| 217 | Y217R | 1.00 | 0.80 | 0.54 | 1.01 | 0.9 | 0.87 |
| 217 | Y217S | 0.83 | 1.11 | 0.95 | 1.01 | 1.0 | 1.63 |
| 217 | Y217T | 0.80 | 1.03 | 0.63 | 1.10 | 0.8 | 1.15 |
| 217 | Y217V | 0.75 | 1.10 | 0.98 | 1.22 | 0.7 | 1.03 |
| 217 | Y217W | 0.80 | 0.76 | 0.68 | 0.79 | 1.0 | 0.71 |
| 218 | N218A | 0.99 | 0.94 | 0.92 | 0.97 | 0.7 | 1.13 |
| 218 | N218C | 1.05 | 1.06 | 0.86 | 0.83 | 1.1 | 0.76 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 218 | N218E | 0.90 | 1.03 | 1.09 | 0.85 | 1.1 | 0.59 |
| 218 | N218F | 0.77 | 0.62 | 0.55 | 0.78 | 0.2 | 0.97 |
| 218 | N218G | 0.88 | 1.06 | 1.07 | 1.01 | 0.5 | 1.23 |
| 218 | N218H | 0.94 | 1.02 | 0.93 | 0.91 | 0.9 | 0.61 |
| 218 | N218I | 0.52 | 0.45 | 0.43 | 0.57 | 0.1 | 0.36 |
| 218 | N218M | 0.76 | 0.95 | 0.74 | 0.75 | 0.4 | 0.94 |
| 218 | N218P | 0.29 | 1.56 | 0.82 | 1.19 | 0.2 | 0.60 |
| 218 | N218S | 1.00 | 1.00 | 0.99 | 1.07 | 1.2 | 1.07 |
| 218 | N218T | 0.93 | 0.62 | 0.73 | 0.73 | 0.9 | 0.58 |
| 218 | N218V | 0.48 | 0.51 | 0.64 | 0.50 | 0.2 | 0.77 |
| 218 | N218W | 0.70 | 0.65 | 0.59 | 0.65 | 0.3 | 0.68 |
| 218 | N218Y | 0.70 | 0.62 | 0.75 | 0.70 | 0.2 | 1.08 |
| 219 | G219C | 0.53 | 0.83 | 0.71 | 0.65 | 1.0 | 0.60 |
| 219 | G219L | 0.54 | 0.68 | 0.70 | 0.70 | 0.2 | 0.89 |
| 219 | G219T | 0.22 | 0.28 | 0.34 | 0.08 | 0.8 | 0.58 |
| 220 | T220S | 0.68 | 1.16 | 1.13 | 0.97 | 0.6 | 0.49 |
| 222 | M222A | 1.49 | 0.94 | 0.68 | 0.92 | 1.0 | 0.48 |
| 222 | M222C | 1.15 | 0.66 | 0.55 | 0.80 | 1.6 | 1.32 |
| 222 | M222F | 0.86 | 0.62 | 0.73 | 1.00 | 1.4 | 0.06 |
| 222 | M222H | 1.07 | 0.52 | 0.56 | 0.78 | 2.0 | 0.06 |
| 222 | M222L | 0.98 | 0.48 | 0.60 | 0.76 | 0.6 | 0.13 |
| 222 | M222N | 1.14 | 0.76 | 0.74 | 0.93 | 0.9 | 0.27 |
| 222 | M222P | 1.22 | 0.47 | 0.24 | 0.54 | 0.3 | 0.14 |
| 222 | M222Q | 0.78 | 0.97 | 0.87 | 1.19 | 1.4 | 0.12 |
| 222 | M222S | 1.15 | 0.77 | 0.62 | 0.87 | 1.0 | 0.95 |
| 222 | M222T | 1.12 | 0.64 | 0.46 | 0.72 | 1.0 | 0.34 |
| 222 | M222V | 1.20 | 0.69 | 0.50 | 0.80 | 0.9 | 0.12 |
| 222 | M222W | 1.89 | 0.36 | 0.21 | 0.74 | 0.7 | 0.09 |
| 223 | A223G | 0.62 | 1.00 | 0.60 | 0.86 | 0.2 | 0.66 |
| 223 | A223S | 1.05 | 0.95 | 1.00 | 0.91 | 0.9 | 0.72 |
| 223 | A223T | 0.23 | 1.22 | 0.73 | 0.91 | 0.2 | 0.17 |
| 224 | S224A | 1.14 | 1.24 | 1.14 | 1.15 | 0.9 | 1.16 |
| 224 | S224C | 1.34 | 1.23 | 1.23 | 1.22 | 1.1 | 0.65 |
| 224 | S224G | 0.90 | 1.23 | 1.15 | 1.13 | 0.3 | 0.63 |
| 224 | S224N | 0.35 | 1.77 | 1.55 | 1.26 | 0.6 | 0.24 |
| 224 | S224T | 0.66 | 1.16 | 1.22 | 1.07 | 0.9 | 0.37 |
| 224 | S224V | 0.97 | 0.89 | 0.90 | 1.05 | 0.9 | 0.06 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 225 | P225S | 1.13 | 0.37 | 0.40 | 0.55 | 0.9 | 0.07 |
| 227 | V227A | 1.03 | 0.99 | 1.26 | 0.83 | 1.0 | 0.81 |
| 227 | V227C | 0.95 | 1.01 | 1.29 | 1.01 | 0.9 | 0.81 |
| 227 | V227F | 0.30 | 0.87 | 1.35 | 1.00 | 0.5 | 0.74 |
| 227 | V227G | 0.32 | 1.55 | 1.43 | 1.06 | 0.9 | 0.69 |
| 227 | V227I | 0.97 | 1.12 | 1.06 | 0.82 | 0.7 | 0.95 |
| 227 | V227L | 0.50 | 1.36 | 1.30 | 1.04 | 0.4 | 0.92 |
| 227 | V227M | 0.36 | 0.76 | 1.18 | 0.87 | 0.7 | 0.75 |
| 227 | V227S | 0.16 | 14.70 | 19.48 | 4.22 | 0.5 | 0.53 |
| 227 | V227T | 0.63 | 1.04 | 1.44 | 1.01 | 0.7 | 0.87 |
| 228 | A228G | 0.38 | 1.14 | 1.11 | 1.31 | 1.0 | 0.66 |
| 228 | A228I | 0.23 | 1.27 | 0.90 | 1.50 | 0.2 | 0.23 |
| 228 | A228M | 0.22 | 0.86 | 0.93 | 1.15 | 0.4 | 0.20 |
| 228 | A228S | 0.88 | 0.86 | 1.10 | 1.17 | 1.1 | 0.80 |
| 228 | A228T | 0.78 | 1.04 | 1.04 | 1.07 | 1.0 | 0.68 |
| 228 | A228V | 0.37 | 1.18 | 1.37 | 1.39 | 0.9 | 0.51 |
| 229 | G229A | 1.20 | 0.82 | 1.12 | 0.99 | 0.8 | 0.99 |
| 229 | G229S | 0.76 | 0.75 | 0.96 | 1.03 | 0.6 | 1.03 |
| 230 | A230C | 1.06 | 0.88 | 1.21 | 1.12 | 0.8 | 1.06 |
| 230 | A230E | 0.77 | 0.74 | 1.09 | 1.06 | 0.6 | 0.75 |
| 230 | A230F | 0.50 | 0.52 | 1.30 | 1.06 | 1.1 | 0.94 |
| 230 | A230G | 0.99 | 0.86 | 1.16 | 0.95 | 1.0 | 0.98 |
| 230 | A230H | 0.16 | 10.35 | 16.17 | 3.82 | 0.1 | 0.38 |
| 230 | A230N | 0.19 | 1.80 | 2.19 | 1.72 | 0.6 | 0.58 |
| 230 | A230Q | 0.39 | 0.81 | 1.14 | 1.03 | 0.3 | 0.86 |
| 230 | A230S | 1.00 | 0.86 | 1.00 | 1.00 | 0.9 | 1.04 |
| 230 | A230T | 0.89 | 0.94 | 0.94 | 1.01 | 0.7 | 1.14 |
| 230 | A230V | 0.56 | 0.85 | 0.95 | 1.08 | 0.2 | 1.17 |
| 231 | A231C | 0.84 | 0.99 | 1.15 | 1.09 | 0.8 | 0.79 |
| 231 | A231F | 0.33 | 1.16 | 1.34 | 1.33 | 0.5 | 0.57 |
| 231 | A231G | 0.42 | 1.10 | 1.04 | 1.35 | 1.1 | 0.86 |
| 231 | A231I | 0.86 | 1.08 | 1.16 | 1.12 | 0.8 | 0.71 |
| 231 | A231L | 0.45 | 1.07 | 1.27 | 1.32 | 0.7 | 0.83 |
| 231 | A231M | 0.41 | 1.15 | 1.26 | 1.30 | 0.7 | 0.73 |
| 231 | A231S | 0.55 | 0.96 | 1.28 | 1.14 | 1.0 | 1.03 |
| 231 | A231T | 0.57 | 1.22 | 1.31 | 1.09 | 0.8 | 0.98 |
| 231 | A231V | 0.71 | 1.07 | 1.04 | 1.12 | 0.8 | 0.99 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 231 | A231W | 0.18 | 4.36 | 5.85 | 4.42 | 0.3 | 0.16 |
| 231 | A231Y | 0.25 | 1.52 | 2.14 | 1.63 | 0.2 | 0.44 |
| 232 | A232C | 0.92 | 1.09 | 1.13 | 1.00 | 0.9 | 0.99 |
| 232 | A232E | 0.28 | 0.25 | 0.21 | 0.37 | 1.0 | 0.14 |
| 232 | A232L | 0.37 | 1.15 | 1.29 | 1.42 | 1.1 | 0.87 |
| 232 | A232M | 0.66 | 1.02 | 1.04 | 0.98 | 1.0 | 0.91 |
| 232 | A232N | 0.20 | 1.64 | 2.16 | 1.52 | 1.1 | 0.44 |
| 232 | A232S | 0.81 | 0.96 | 1.27 | 0.93 | 0.9 | 0.94 |
| 232 | A232T | 0.73 | 0.97 | 1.02 | 1.03 | 1.0 | 1.04 |
| 232 | A232V | 0.77 | 1.00 | 1.35 | 0.99 | 1.0 | 1.09 |
| 233 | L233D | 0.71 | 0.89 | 0.91 | 0.92 | 1.1 | 0.71 |
| 233 | L233I | 0.85 | 0.89 | 1.11 | 1.03 | 1.0 | 0.90 |
| 233 | L233M | 0.67 | 0.92 | 0.99 | 1.01 | 0.9 | 0.94 |
| 233 | L233S | 0.61 | 1.05 | 1.16 | 0.96 | 0.9 | 0.78 |
| 233 | L233T | 0.78 | 0.95 | 1.25 | 0.90 | 1.0 | 0.89 |
| 233 | L233V | 0.94 | 1.15 | 1.09 | 0.89 | 1.1 | 0.91 |
| 234 | I234A | 1.16 | 0.86 | 0.96 | 0.88 | 0.8 | 1.12 |
| 234 | I234C | 1.21 | 1.02 | 0.92 | 0.96 | 1.1 | 0.88 |
| 234 | I234D | 0.21 | 0.70 | 1.52 | 0.91 | 0.6 | 0.44 |
| 234 | I234E | 0.88 | 1.05 | 1.01 | 0.86 | 0.8 | 1.01 |
| 234 | I234F | 0.72 | 1.03 | 1.02 | 0.72 | 0.1 | 1.02 |
| 234 | I234G | 0.33 | 0.93 | 0.96 | 1.15 | 0.7 | 0.86 |
| 234 | I234H | 0.37 | 0.98 | 0.98 | 0.97 | 0.3 | 0.94 |
| 234 | I234K | 0.30 | 0.89 | 0.92 | 1.00 | 0.1 | 0.84 |
| 234 | I234L | 1.22 | 0.82 | 0.84 | 1.08 | 1.1 | 1.07 |
| 234 | I234M | 1.27 | 1.02 | 0.91 | 0.98 | 1.0 | 1.18 |
| 234 | I234N | 0.42 | 1.08 | 0.98 | 1.03 | 0.9 | 0.91 |
| 234 | I234Q | 0.87 | 0.72 | 0.94 | 0.93 | 0.9 | 0.96 |
| 234 | I234S | 0.81 | 0.92 | 0.93 | 0.93 | 0.9 | 0.98 |
| 234 | I234T | 1.14 | 0.87 | 0.92 | 1.02 | 1.1 | 0.99 |
| 234 | 1234V | 1.20 | 1.02 | 1.19 | 0.93 | 1.1 | 1.14 |
| 234 | I234W | 0.17 | 4.77 | 6.83 | 2.41 | 0.1 | 0.19 |
| 235 | L235A | 0.67 | 1.05 | 0.95 | 1.05 | 1.0 | 1.15 |
| 235 | L235C | 1.08 | 0.96 | 1.24 | 1.03 | 0.9 | 1.07 |
| 235 | L235D | 0.20 | 0.19 | 1.17 | 0.45 | 1.1 | 0.14 |
| 235 | L235E | 0.42 | 0.81 | 1.03 | 0.94 | 1.1 | 0.25 |
| 235 | L235F | 1.25 | 1.08 | 1.01 | 0.94 | 1.0 | 0.80 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 235 | L235G | 0.27 | 1.09 | 0.76 | 1.11 | 1.1 | 0.62 |
| 235 | L235I | 1.31 | 0.92 | 0.83 | 1.10 | 1.1 | 0.94 |
| 235 | L235K | 1.32 | 0.86 | 1.21 | 0.96 | 1.1 | 0.85 |
| 235 | L235M | 1.34 | 0.99 | 0.99 | 1.03 | 1.1 | 0.89 |
| 235 | L235N | 0.25 | 1.32 | 1.54 | 1.19 | 1.2 | 0.69 |
| 235 | L235Q | 1.01 | 0.92 | 0.84 | 1.03 | 1.1 | 1.24 |
| 235 | L235R | 1.29 | 0.79 | 0.86 | 0.95 | 1.0 | 0.90 |
| 235 | L235S | 0.69 | 1.05 | 0.86 | 0.76 | 1.0 | 1.00 |
| 235 | L235T | 0.65 | 1.10 | 0.80 | 0.86 | 1.1 | 1.07 |
| 235 | L235V | 1.17 | 0.92 | 1.07 | 0.83 | 1.1 | 1.24 |
| 235 | L235W | 1.09 | 0.98 | 0.63 | 1.13 | 1.1 | 0.94 |
| 235 | L235Y | 0.99 | 0.99 | 0.98 | 1.04 | 1.1 | 1.02 |
| 236 | S236A | 1.21 | 1.02 | 1.06 | 0.94 | 0.9 | 1.03 |
| 236 | S236C | 1.28 | 0.89 | 0.95 | 0.96 | 1.0 | 0.90 |
| 236 | S236D | 1.22 | 1.02 | 0.95 | 0.98 | 1.2 | 1.07 |
| 236 | S236E | 1.26 | 0.90 | 0.88 | 1.09 | 1.2 | 1.55 |
| 236 | S236G | 1.20 | 1.06 | 1.09 | 0.97 | 0.9 | 0.99 |
| 236 | S236H | 1.12 | 0.84 | 0.87 | 0.92 | 1.2 | 1.07 |
| 236 | S236K | 0.31 | 0.96 | 0.80 | 1.18 | 0.7 | 0.55 |
| 236 | S236L | 0.19 | 0.42 | 0.92 | 0.66 | 0.8 | 0.13 |
| 236 | S236N | 1.24 | 1.03 | 1.18 | 0.86 | 0.6 | 1.13 |
| 236 | S236Q | 1.18 | 0.85 | 0.69 | 0.95 | 1.1 | 1.00 |
| 236 | S236R | 0.34 | 0.96 | 0.72 | 1.13 | 0.7 | 0.60 |
| 236 | S236T | 0.59 | 0.99 | 0.97 | 1.04 | 0.8 | 1.16 |
| 236 | S236V | 0.91 | 0.97 | 0.86 | 1.06 | 0.9 | 1.21 |
| 236 | S236W | 0.18 | 2.11 | 3.43 | 2.28 | 0.8 | 0.50 |
| 236 | S236Y | 0.48 | 1.10 | 0.66 | 0.91 | 0.8 | 1.09 |
| 237 | K237A | 1.13 | 1.11 | 1.31 | 0.81 | 1.0 | 1.03 |
| 237 | K237C | 0.19 | 1.21 | 1.50 | 0.91 | 1.1 | 0.33 |
| 237 | K237E | 1.03 | 0.86 | 0.84 | 0.85 | 1.0 | 1.22 |
| 237 | K237F | 1.05 | 0.80 | 0.99 | 0.92 | 1.0 | 1.21 |
| 237 | K237G | 1.05 | 0.90 | 1.08 | 1.06 | 1.0 | 0.94 |
| 237 | K237H | 1.05 | 0.91 | 0.97 | 0.93 | 1.1 | 0.88 |
| 237 | K237I | 1.02 | 1.03 | 0.69 | 0.70 | 1.0 | 1.05 |
| 237 | K237L | 1.04 | 0.96 | 0.89 | 1.10 | 1.0 | 1.12 |
| 237 | K237M | 1.17 | 0.89 | 1.09 | 0.86 | 0.9 | 1.20 |
| 237 | K237N | 1.09 | 1.00 | 0.99 | 0.94 | 1.1 | 0.93 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 237 | K237P | 0.19 | 0.50 | 0.92 | 0.59 | 1.1 | 0.15 |
| 237 | K237Q | 1.12 | 0.74 | 0.87 | 0.84 | 1.1 | 1.01 |
| 237 | K237R | 1.13 | 0.82 | 0.95 | 1.02 | 1.0 | 1.08 |
| 237 | K237S | 1.17 | 0.97 | 1.07 | 0.86 | 1.0 | 1.24 |
| 237 | K237T | 1.07 | 0.81 | 0.75 | 0.82 | 1.0 | 0.94 |
| 237 | K237V | 1.08 | 0.85 | 1.09 | 1.10 | 0.9 | 1.09 |
| 237 | K237W | 0.81 | 0.96 | 1.08 | 0.95 | 0.9 | 0.87 |
| 237 | K237Y | 1.00 | 1.10 | 0.98 | 1.08 | 0.9 | 1.10 |
| 238 | H238A | 0.28 | 1.34 | 1.44 | 1.12 | 1.1 | 0.46 |
| 238 | H238C | 0.62 | 1.07 | 1.09 | 0.99 | 1.1 | 0.97 |
| 238 | H238D | 0.35 | 0.93 | 1.18 | 1.19 | 1.1 | 0.78 |
| 238 | H238E | 0.61 | 0.91 | 1.00 | 1.00 | 1.1 | 1.08 |
| 238 | H238F | 0.74 | 0.69 | 1.05 | 0.95 | 1.1 | 1.09 |
| 238 | H238G | 0.22 | 5.21 | 5.98 | 2.44 | 1.0 | 0.44 |
| 238 | H238I | 0.22 | 7.74 | 7.02 | 3.64 | 1.1 | 0.52 |
| 238 | H238K | 0.87 | 0.82 | 1.07 | 0.89 | 1.0 | 1.24 |
| 238 | H238M | 0.58 | 1.00 | 1.15 | 0.89 | 1.0 | 0.93 |
| 238 | H238R | 0.79 | 0.96 | 0.76 | 1.04 | 0.9 | 1.17 |
| 238 | H238S | 0.55 | 1.29 | 1.03 | 0.82 | 1.0 | 0.99 |
| 238 | H238W | 0.41 | 1.14 | 0.92 | 1.16 | 1.1 | 1.10 |
| 239 | P239A | 0.57 | 0.98 | 0.94 | 0.88 | 1.0 | 0.86 |
| 239 | P239C | 0.78 | 0.96 | 0.85 | 0.83 | 1.0 | 1.03 |
| 239 | P239D | 0.89 | 1.00 | 0.81 | 0.98 | 1.1 | 0.87 |
| 239 | P239E | 0.80 | 1.16 | 0.81 | 0.97 | 1.1 | 0.96 |
| 239 | P239F | 0.66 | 1.15 | 0.67 | 1.03 | 1.0 | 1.02 |
| 239 | P239G | 0.76 | 0.71 | 0.48 | 0.95 | 0.2 | 0.79 |
| 239 | P239H | 0.89 | 1.41 | 1.02 | 1.12 | 1.1 | 1.09 |
| 239 | P239L | 0.65 | 1.04 | 0.90 | 0.95 | 1.1 | 1.09 |
| 239 | P239M | 0.79 | 1.12 | 0.70 | 0.98 | 1.1 | 0.89 |
| 239 | P239N | 0.82 | 1.08 | 0.83 | 1.01 | 1.2 | 0.94 |
| 239 | P239Q | 0.91 | 1.14 | 0.80 | 1.10 | 1.2 | 0.97 |
| 239 | P239R | 0.92 | 1.21 | 0.67 | 1.27 | 1.0 | 1.04 |
| 239 | P239S | 0.87 | 1.31 | 0.96 | 0.99 | 1.1 | 1.33 |
| 239 | P239T | 0.80 | 0.97 | 1.07 | 1.11 | 1.2 | 1.03 |
| 239 | P239V | 0.71 | 1.10 | 1.22 | 1.06 | 1.2 | 1.32 |
| 239 | P239W | 0.65 | 1.06 | 0.75 | 1.11 | 1.0 | 1.28 |
| 239 | P239Y | 0.76 | 0.90 | 0.94 | 1.20 | 1.2 | 1.22 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 240 | N240A | 0.80 | 0.96 | 0.99 | 0.96 | 1.0 | 0.91 |
| 240 | N240C | 1.06 | 1.04 | 0.83 | 0.89 | 1.1 | 0.95 |
| 240 | N240D | 1.05 | 1.07 | 0.88 | 1.03 | 1.2 | 0.93 |
| 240 | N240E | 1.03 | 1.27 | 0.83 | 1.00 | 1.2 | 1.05 |
| 240 | N240F | 1.02 | 0.94 | 0.84 | 0.93 | 1.1 | 0.92 |
| 240 | N240G | 1.03 | 1.21 | 0.87 | 0.90 | 1.0 | 1.01 |
| 240 | N240K | 1.09 | 1.04 | 1.04 | 0.99 | 1.1 | 1.02 |
| 240 | N240L | 0.78 | 1.27 | 0.86 | 1.08 | 1.0 | 1.05 |
| 240 | N240M | 0.94 | 1.03 | 0.76 | 0.92 | 1.0 | 0.94 |
| 240 | N240P | 0.39 | 0.75 | 0.72 | 0.89 | 0.9 | 0.54 |
| 240 | N240Q | 0.91 | 1.09 | 0.88 | 1.11 | 1.1 | 1.04 |
| 240 | N240R | 0.75 | 0.85 | 0.59 | 1.14 | 1.0 | 0.97 |
| 240 | N240S | 1.00 | 1.19 | 1.04 | 1.11 | 1.1 | 0.95 |
| 240 | N240T | 0.90 | 1.02 | 0.77 | 1.14 | 1.1 | 1.02 |
| 240 | N240V | 0.90 | 1.06 | 0.92 | 1.06 | 1.0 | 1.10 |
| 240 | N240W | 1.05 | 1.05 | 0.66 | 1.01 | 1.0 | 1.00 |
| 240 | N240Y | 0.92 | 1.01 | 0.80 | 1.22 | 1.0 | 1.43 |
| 241 | W241A | 0.84 | 0.94 | 0.65 | 1.02 | 1.1 | 0.86 |
| 241 | W241C | 0.63 | 0.96 | 0.72 | 0.98 | 1.1 | 1.09 |
| 241 | W241D | 0.44 | 1.06 | 1.03 | 1.05 | 1.2 | 0.89 |
| 241 | W241E | 0.34 | 1.05 | 0.81 | 1.06 | 1.1 | 0.63 |
| 241 | W241F | 0.81 | 0.94 | 0.86 | 0.75 | 1.0 | 0.87 |
| 241 | W241G | 0.31 | 1.35 | 1.05 | 0.92 | 1.0 | 0.64 |
| 241 | W241H | 0.65 | 1.08 | 0.99 | 1.12 | 1.1 | 1.05 |
| 241 | W241I | 0.75 | 1.11 | 0.99 | 1.13 | 1.0 | 1.15 |
| 241 | W241K | 0.83 | 1.00 | 0.88 | 1.22 | 1.0 | 1.11 |
| 241 | W241L | 0.85 | 0.78 | 1.00 | 1.03 | 1.0 | 0.99 |
| 241 | W241M | 0.91 | 1.01 | 0.82 | 1.04 | 1.1 | 1.07 |
| 241 | W241N | 0.35 | 1.14 | 0.92 | 1.07 | 1.2 | 0.64 |
| 241 | W241Q | 0.65 | 1.40 | 1.00 | 1.14 | 1.1 | 1.14 |
| 241 | W241R | 0.59 | 0.93 | 0.88 | 1.12 | 0.9 | 1.37 |
| 241 | W241S | 0.66 | 1.14 | 0.68 | 1.04 | 0.9 | 1.06 |
| 241 | W241T | 0.61 | 1.06 | 0.86 | 1.16 | 1.0 | 1.11 |
| 241 | W241V | 0.61 | 1.20 | 1.01 | 1.09 | 1.0 | 1.35 |
| 241 | W241Y | 0.81 | 1.17 | 0.84 | 1.08 | 1.0 | 1.38 |
| 242 | T242A | 1.24 | 0.92 | 1.10 | 0.95 | 1.1 | 0.75 |
| 242 | T242C | 1.10 | 0.89 | 1.21 | 0.84 | 1.1 | 0.86 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 242 | T242E | 1.03 | 1.08 | 1.32 | 0.87 | 1.0 | 0.90 |
| 242 | T242F | 0.71 | 0.86 | 0.97 | 0.81 | 0.9 | 0.99 |
| 242 | T242G | 0.88 | 0.74 | 1.02 | 1.03 | 1.0 | 0.95 |
| 242 | T242H | 0.95 | 1.14 | 0.98 | 0.90 | 1.1 | 0.92 |
| 242 | T242I | 0.84 | 0.82 | 1.26 | 1.11 | 1.1 | 0.93 |
| 242 | T242K | 0.99 | 0.77 | 1.07 | 0.93 | 1.1 | 0.94 |
| 242 | T242L | 0.97 | 0.99 | 1.12 | 1.01 | 1.1 | 0.89 |
| 242 | T242M | 1.00 | 0.83 | 1.03 | 0.97 | 1.0 | 0.87 |
| 242 | T242N | 1.02 | 0.65 | 1.14 | 0.96 | 1.1 | 0.92 |
| 242 | T242P | 0.93 | 0.83 | 1.22 | 1.06 | 1.1 | 0.97 |
| 242 | T242Q | 0.91 | 0.87 | 1.34 | 0.95 | 1.1 | 1.23 |
| 242 | T242R | 0.91 | 1.04 | 1.18 | 0.91 | 1.1 | 1.05 |
| 242 | T242S | 1.05 | 1.00 | 1.16 | 1.18 | 1.1 | 0.95 |
| 242 | T242V | 0.80 | 0.89 | 1.20 | 0.99 | 1.0 | 1.05 |
| 242 | T242W | 0.62 | 0.72 | 0.97 | 0.91 | 1.1 | 0.96 |
| 242 | T242Y | 0.69 | 0.94 | 0.97 | 1.01 | 1.1 | 1.02 |
| 243 | N243C | 1.05 | 0.88 | 1.22 | 0.98 | 1.0 | 0.93 |
| 243 | N243E | 1.14 | 0.94 | 1.22 | 0.98 | 1.1 | 0.79 |
| 243 | N243F | 0.69 | 0.75 | 1.03 | 1.01 | 0.6 | 0.79 |
| 243 | N243G | 0.97 | 1.16 | 0.94 | 1.00 | 1.2 | 0.72 |
| 243 | N2431 | 0.94 | 0.79 | 1.61 | 0.79 | 0.8 | 0.91 |
| 243 | N243K | 0.78 | 0.97 | 1.05 | 1.11 | 0.9 | 0.85 |
| 243 | N243L | 0.83 | 0.92 | 1.24 | 1.03 | 0.8 | 0.74 |
| 243 | N243Q | 0.93 | 0.74 | 1.28 | 1.03 | 1.0 | 0.94 |
| 243 | N243R | 0.73 | 0.79 | 0.92 | 0.90 | 0.8 | 1.09 |
| 243 | N243S | 0.97 | 1.05 | 1.13 | 0.86 | 1.0 | 0.93 |
| 243 | N243T | 0.92 | 0.97 | 1.01 | 0.92 | 1.0 | 0.98 |
| 243 | N243V | 0.89 | 0.87 | 1.13 | 1.13 | 1.0 | 1.29 |
| 243 | N243W | 0.59 | 0.90 | 1.02 | 1.01 | 0.6 | 1.05 |
| 243 | N243Y | 0.81 | 0.82 | 1.20 | 0.82 | 0.9 | 0.92 |
| 244 | T244A | 1.53 | 0.66 | 0.97 | 1.07 | 1.0 | 0.84 |
| 244 | T244D | 1.13 | 0.97 | 1.23 | 1.13 | 1.0 | 1.11 |
| 244 | T244E | 0.69 | 0.89 | 1.12 | 0.90 | 1.0 | 0.92 |
| 244 | T244F | 0.83 | 0.97 | 0.97 | 1.01 | 1.0 | 0.96 |
| 244 | T244G | 1.05 | 0.83 | 1.06 | 1.01 | 1.0 | 0.96 |
| 244 | T244H | 1.06 | 0.90 | 1.29 | 1.01 | 1.1 | 0.94 |
| 244 | T244K | 1.09 | 0.76 | 0.95 | 1.00 | 1.0 | 0.97 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 244 | T244L | 0.99 | 1.07 | 1.13 | 0.92 | 1.1 | 1.00 |
| 244 | T244M | 1.15 | 0.94 | 1.05 | 1.07 | 1.0 | 0.96 |
| 244 | T244N | 1.03 | 1.20 | 1.10 | 1.03 | 1.1 | 0.95 |
| 244 | T244P | 0.74 | 0.89 | 1.25 | 1.00 | 1.0 | 1.00 |
| 244 | T244Q | 1.04 | 0.87 | 1.23 | 0.94 | 1.0 | 1.08 |
| 244 | T244R | 0.96 | 0.54 | 1.04 | 0.91 | 0.9 | 1.10 |
| 244 | T244S | 1.04 | 1.02 | 1.23 | 1.07 | 1.0 | 0.99 |
| 244 | T244V | 0.90 | 0.69 | 1.07 | 1.04 | 1.0 | 1.06 |
| 244 | T244W | 0.88 | 0.70 | 1.29 | 0.94 | 1.0 | 1.13 |
| 244 | T244Y | 0.87 | 0.38 | 1.03 | 1.01 | 1.0 | 1.10 |
| 245 | Q245A | 1.00 | 0.92 | 1.10 | 0.93 | 1.1 | 0.92 |
| 245 | Q245C | 0.80 | 0.79 | 1.15 | 0.93 | 1.1 | 0.89 |
| 245 | Q245D | 1.00 | 0.73 | 1.19 | 0.88 | 1.1 | 0.90 |
| 245 | Q245E | 1.03 | 1.00 | 1.15 | 0.79 | 1.2 | 0.89 |
| 245 | Q245F | 0.80 | 0.89 | 1.21 | 1.00 | 1.0 | 0.98 |
| 245 | Q245G | 0.76 | 1.12 | 1.17 | 0.99 | 1.1 | 0.99 |
| 245 | Q245H | 0.93 | 1.01 | 1.04 | 0.97 | 1.1 | 0.94 |
| 245 | Q245I | 0.83 | 0.97 | 1.12 | 1.01 | 1.1 | 0.99 |
| 245 | Q245K | 0.99 | 0.83 | 0.82 | 0.88 | 1.1 | 0.96 |
| 245 | Q245L | 0.88 | 0.77 | 1.18 | 0.85 | 1.1 | 0.97 |
| 245 | Q245M | 1.06 | 0.81 | 1.19 | 1.10 | 1.1 | 0.92 |
| 245 | Q245N | 0.63 | 0.91 | 0.96 | 0.79 | 1.0 | 0.82 |
| 245 | Q245P | 0.27 | 0.47 | 0.63 | 0.50 | 1.0 | 0.25 |
| 245 | Q245R | 0.76 | 0.57 | 0.89 | 0.97 | 1.0 | 1.07 |
| 245 | Q245S | 0.81 | 0.93 | 1.19 | 0.97 | 0.9 | 1.14 |
| 245 | Q245T | 0.82 | 0.90 | 1.18 | 1.03 | 1.0 | 1.37 |
| 245 | Q245V | 0.72 | 0.96 | 1.19 | 1.06 | 1.0 | 1.09 |
| 245 | Q245W | 0.50 | 0.94 | 1.00 | 0.90 | 0.9 | 1.08 |
| 245 | Q245Y | 1.06 | 0.66 | 1.08 | 0.99 | 1.0 | 1.06 |
| 246 | V246A | 0.53 | 1.06 | 1.22 | 1.01 | 0.9 | 0.92 |
| 246 | V246C | 0.64 | 0.95 | 1.05 | 0.92 | 1.1 | 1.02 |
| 246 | V246F | 0.31 | 1.18 | 1.59 | 1.59 | 0.8 | 0.81 |
| 246 | V246I | 1.00 | 1.00 | 1.11 | 0.81 | 1.1 | 0.95 |
| 246 | V246L | 0.67 | 1.05 | 1.10 | 0.88 | 1.0 | 0.94 |
| 246 | V246N | 0.20 | 0.32 | 0.16 | 0.32 | 1.2 | 0.08 |
| 246 | V246S | 0.23 | 0.60 | 0.54 | 0.81 | 0.7 | 0.33 |
| 246 | V246T | 0.74 | 0.95 | 1.23 | 1.04 | 1.0 | 1.10 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 247 | R247A | 0.55 | 0.86 | 1.05 | 0.74 | 0.2 | 0.70 |
| 247 | R247C | 0.36 | 1.07 | 1.40 | 1.04 | 0.4 | 0.64 |
| 247 | R247D | 0.23 | 0.43 | 0.88 | 0.46 | 0.2 | 0.15 |
| 247 | R247E | 0.38 | 0.77 | 1.02 | 0.88 | 0.3 | 0.59 |
| 247 | R247F | 0.32 | 0.96 | 1.09 | 0.86 | 0.3 | 0.65 |
| 247 | R247H | 0.31 | 1.31 | 1.51 | 1.28 | 0.3 | 0.68 |
| 247 | R247I | 0.37 | 1.16 | 1.37 | 1.11 | 0.2 | 0.88 |
| 247 | R247K | 0.50 | 1.01 | 1.17 | 1.02 | 0.5 | 0.75 |
| 247 | R247L | 0.30 | 1.65 | 1.54 | 1.25 | 0.1 | 0.55 |
| 247 | R247M | 0.42 | 1.07 | 1.22 | 0.83 | 0.2 | 0.63 |
| 247 | R247S | 0.37 | 1.06 | 1.56 | 0.93 | 0.4 | 0.74 |
| 247 | R247T | 0.44 | 1.18 | 1.33 | 1.11 | 0.5 | 0.97 |
| 247 | R247V | 0.40 | 0.84 | 1.31 | 1.01 | 0.4 | 0.76 |
| 247 | R247W | 0.32 | 1.35 | 1.39 | 0.95 | 0.3 | 0.68 |
| 247 | R247Y | 0.27 | 1.53 | 2.24 | 1.48 | 0.2 | 0.90 |
| 248 | S248A | 1.06 | 1.03 | 1.21 | 1.00 | 1.0 | 1.25 |
| 248 | S248C | 1.02 | 0.82 | 1.09 | 0.96 | 1.0 | 0.91 |
| 248 | S248E | 1.21 | 0.61 | 1.11 | 0.86 | 1.0 | 1.02 |
| 248 | S248F | 0.98 | 0.97 | 1.11 | 0.96 | 0.9 | 1.52 |
| 248 | S248G | 0.93 | 1.11 | 0.97 | 0.96 | 0.9 | 1.10 |
| 248 | S248H | 0.78 | 1.16 | 1.10 | 0.98 | 1.0 | 0.99 |
| 248 | S248I | 1.00 | 1.01 | 1.23 | 1.13 | 0.9 | 1.20 |
| 248 | S248K | 1.07 | 0.54 | 0.99 | 1.02 | 0.9 | 0.98 |
| 248 | S248L | 1.00 | 0.82 | 1.08 | 1.01 | 0.9 | 1.18 |
| 248 | S248M | 1.00 | 0.75 | 1.31 | 0.96 | 1.0 | 1.05 |
| 248 | S248N | 1.08 | 0.96 | 1.19 | 1.05 | 1.0 | 1.01 |
| 248 | S248P | 0.57 | 1.06 | 1.39 | 1.07 | 0.9 | 1.00 |
| 248 | S248Q | 1.10 | 1.01 | 1.14 | 0.84 | 1.0 | 0.93 |
| 248 | S248R | 1.10 | 0.47 | 0.80 | 0.87 | 0.9 | 0.87 |
| 248 | S248T | 0.95 | 0.93 | 1.19 | 0.96 | 1.0 | 1.07 |
| 248 | S248V | 0.80 | 1.16 | 1.13 | 1.01 | 0.8 | 1.31 |
| 248 | S248W | 0.85 | 0.87 | 1.24 | 1.02 | 1.1 | 0.99 |
| 248 | S248Y | 1.31 | 0.89 | 1.17 | 0.94 | 1.0 | 0.98 |
| 249 | S249A | 1.07 | 0.92 | 1.25 | 1.02 | 1.0 | 0.95 |
| 249 | S249C | 1.14 | 0.97 | 1.21 | 0.92 | 1.0 | 0.86 |
| 249 | S249D | 0.97 | 0.84 | 1.17 | 0.90 | 0.9 | 1.27 |
| 249 | S249F | 1.14 | 0.75 | 0.97 | 0.95 | 0.8 | 0.89 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 249 | S249G | 0.72 | 0.83 | 1.17 | 0.98 | 0.9 | 1.04 |
| 249 | S249H | 1.10 | 0.95 | 0.87 | 1.12 | 0.9 | 0.87 |
| 249 | S249I | 0.92 | 0.76 | 1.30 | 0.99 | 0.9 | 0.93 |
| 249 | S249K | 1.08 | 0.91 | 0.83 | 0.87 | 0.8 | 0.93 |
| 249 | S249L | 0.99 | 0.89 | 1.19 | 0.91 | 0.8 | 1.21 |
| 249 | S249M | 1.07 | 0.99 | 1.24 | 1.11 | 1.0 | 1.00 |
| 249 | S249N | 1.00 | 1.08 | 1.35 | 1.06 | 1.0 | 0.92 |
| 249 | S249Q | 1.00 | 0.73 | 1.06 | 1.17 | 0.8 | 1.12 |
| 249 | S249R | 1.03 | 0.78 | 0.87 | 0.95 | 0.9 | 1.00 |
| 249 | S249T | 1.08 | 0.78 | 1.17 | 0.95 | 0.9 | 1.13 |
| 249 | S249V | 0.97 | 0.82 | 1.18 | 1.05 | 0.9 | 0.99 |
| 249 | S249W | 0.95 | 0.92 | 1.09 | 1.17 | 0.8 | 1.06 |
| 249 | S249Y | 0.94 | 0.80 | 1.07 | 0.91 | 0.9 | 1.01 |
| 250 | L250C | 0.37 | 1.06 | 1.50 | 1.21 | 0.9 | 1.21 |
| 250 | L250F | 0.61 | 1.05 | 1.44 | 0.90 | 0.7 | 0.96 |
| 250 | L250I | 0.98 | 0.88 | 1.22 | 1.00 | 0.9 | 1.13 |
| 250 | L250M | 0.90 | 1.09 | 1.13 | 1.20 | 1.0 | 1.05 |
| 250 | L250T | 0.25 | 1.49 | 2.25 | 1.69 | 0.8 | 0.81 |
| 250 | L250V | 0.80 | 0.64 | 1.06 | 1.02 | 0.9 | 0.98 |
| 251 | E251A | 0.93 | 0.92 | 0.67 | 0.98 | 0.6 | 0.70 |
| 251 | E251C | 0.66 | 0.99 | 1.04 | 1.14 | 0.8 | 0.69 |
| 251 | E251D | 0.76 | 1.08 | 1.07 | 1.12 | 0.8 | 0.95 |
| 251 | E251F | 0.68 | 0.96 | 0.65 | 1.04 | 0.7 | 0.66 |
| 251 | E251G | 0.65 | 1.07 | 0.83 | 1.17 | 0.6 | 0.95 |
| 251 | E251H | 0.64 | 1.03 | 0.74 | 1.04 | 0.5 | 0.74 |
| 251 | E251I | 0.58 | 0.93 | 0.91 | 0.93 | 0.5 | 0.81 |
| 251 | E251K | 0.62 | 0.54 | 0.41 | 0.89 | 0.1 | 0.65 |
| 251 | E251L | 0.60 | 0.85 | 0.54 | 1.04 | 0.7 | 0.72 |
| 251 | E251M | 0.65 | 0.78 | 0.62 | 0.87 | 0.6 | 0.62 |
| 251 | E251N | 0.69 | 0.96 | 0.78 | 1.09 | 0.6 | 0.85 |
| 251 | E251Q | 0.61 | 1.12 | 0.91 | 1.10 | 0.5 | 0.77 |
| 251 | E251R | 0.43 | 0.66 | 0.74 | 1.23 | 0.2 | 0.79 |
| 251 | E251S | 0.52 | 0.81 | 0.99 | 0.96 | 0.6 | 0.81 |
| 251 | E251T | 0.69 | 1.02 | 0.91 | 1.00 | 0.7 | 0.93 |
| 251 | E251V | 0.61 | 1.05 | 0.78 | 1.19 | 0.6 | 0.88 |
| 251 | E251W | 0.50 | 1.21 | 0.90 | 1.07 | 0.3 | 0.73 |
| 251 | E251Y | 0.58 | 0.92 | 0.57 | 0.97 | 0.4 | 0.74 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 252 | N252A | 1.02 | 1.03 | 1.28 | 0.97 | 1.0 | 0.93 |
| 252 | N252C | 1.01 | 0.91 | 0.88 | 1.01 | 1.1 | 0.83 |
| 252 | N252E | 0.77 | 0.94 | 0.93 | 1.01 | 1.1 | 0.95 |
| 252 | N252F | 0.27 | 1.07 | 0.77 | 1.10 | 0.9 | 0.49 |
| 252 | N252G | 0.88 | 1.23 | 0.86 | 1.08 | 1.1 | 0.97 |
| 252 | N252H | 0.94 | 1.17 | 0.88 | 1.09 | 1.1 | 0.97 |
| 252 | N252I | 0.81 | 1.07 | 1.11 | 0.94 | 1.0 | 1.10 |
| 252 | N252K | 1.08 | 0.87 | 0.86 | 0.93 | 1.0 | 0.90 |
| 252 | N252L | 0.96 | 1.09 | 0.85 | 1.08 | 1.0 | 1.02 |
| 252 | N252M | 0.89 | 0.98 | 1.11 | 1.10 | 1.1 | 1.03 |
| 252 | N252Q | 1.37 | 1.32 | 1.08 | 1.09 | 1.1 | 0.98 |
| 252 | N252R | 0.95 | 0.84 | 0.90 | 1.08 | 0.9 | 0.99 |
| 252 | N252S | 0.95 | 1.05 | 1.13 | 1.03 | 1.0 | 1.16 |
| 252 | N252T | 0.81 | 1.23 | 1.11 | 1.18 | 1.1 | 1.05 |
| 252 | N252V | 0.74 | 0.99 | 0.99 | 1.28 | 1.0 | 1.24 |
| 252 | N252W | 0.80 | 1.12 | 1.04 | 1.10 | 1.0 | 1.21 |
| 252 | N252Y | 0.82 | 1.06 | 0.93 | 1.14 | 1.0 | 1.09 |
| 253 | T253A | 1.02 | 0.94 | 1.16 | 0.98 | 0.9 | 0.82 |
| 253 | T253C | 0.81 | 0.89 | 1.19 | 0.95 | 1.0 | 0.86 |
| 253 | T253D | 0.77 | 1.10 | 1.02 | 1.03 | 0.8 | 0.78 |
| 253 | T253E | 0.91 | 1.10 | 1.08 | 0.92 | 1.0 | 0.87 |
| 253 | T253F | 1.00 | 1.10 | 0.88 | 1.01 | 1.0 | 0.82 |
| 253 | T253G | 0.97 | 0.97 | 1.04 | 1.05 | 0.8 | 0.86 |
| 253 | T253H | 1.25 | 0.99 | 0.94 | 1.04 | 1.0 | 0.89 |
| 253 | T253I | 0.43 | 1.14 | 0.91 | 0.90 | 0.1 | 0.66 |
| 253 | T253K | 0.94 | 0.89 | 0.83 | 1.09 | 0.6 | 0.88 |
| 253 | T253L | 0.86 | 1.15 | 0.85 | 1.08 | 0.8 | 1.00 |
| 253 | T253M | 1.04 | 0.88 | 1.05 | 1.19 | 0.8 | 0.84 |
| 253 | T253N | 0.92 | 1.10 | 1.14 | 1.03 | 1.0 | 0.88 |
| 253 | T253Q | 0.88 | 1.05 | 1.09 | 1.08 | 0.8 | 1.27 |
| 253 | T253R | 0.83 | 0.70 | 0.73 | 1.08 | 0.4 | 0.92 |
| 253 | T253S | 1.00 | 1.13 | 1.23 | 1.08 | 1.0 | 0.94 |
| 253 | T253V | 0.49 | 1.28 | 1.10 | 1.09 | 0.2 | 1.10 |
| 253 | T253Y | 0.21 | 3.47 | 2.00 | 1.61 | 0.2 | 0.10 |
| 254 | T254A | 1.14 | 0.93 | 1.13 | 1.00 | 1.0 | 0.76 |
| 254 | T254C | 1.01 | 0.99 | 1.08 | 1.14 | 1.1 | 0.83 |
| 254 | T254E | 0.25 | 1.23 | 1.03 | 1.06 | 0.1 | 0.39 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 254 | T254G | 0.55 | 1.00 | 1.16 | 1.13 | 0.8 | 0.85 |
| 254 | T254I | 0.48 | 1.08 | 1.07 | 1.16 | 0.2 | 0.81 |
| 254 | T254M | 0.27 | 1.06 | 0.92 | 1.30 | 0.1 | 0.34 |
| 254 | T254P | 0.83 | 1.16 | 0.97 | 1.07 | 0.7 | 0.88 |
| 254 | T254S | 0.93 | 0.95 | 1.03 | 0.90 | 1.0 | 0.92 |
| 254 | T254V | 1.00 | 1.19 | 0.97 | 1.07 | 0.8 | 1.05 |
| 257 | L257A | 0.72 | 1.00 | 1.27 | 0.91 | 0.6 | 0.99 |
| 257 | L257C | 0.78 | 1.09 | 1.04 | 1.05 | 0.8 | 0.83 |
| 257 | L257D | 0.37 | 1.17 | 1.22 | 0.83 | 0.4 | 0.68 |
| 257 | L257E | 0.50 | 1.05 | 1.27 | 0.92 | 0.6 | 0.81 |
| 257 | L257F | 0.67 | 0.60 | 1.05 | 0.91 | 0.2 | 0.98 |
| 257 | L257G | 0.67 | 1.01 | 0.64 | 1.20 | 0.7 | 0.90 |
| 257 | L257H | 0.56 | 0.83 | 1.00 | 1.01 | 0.4 | 0.87 |
| 257 | L257I | 0.83 | 0.89 | 1.04 | 0.97 | 0.9 | 1.06 |
| 257 | L257M | 0.84 | 1.06 | 0.93 | 0.86 | 0.8 | 0.83 |
| 257 | L257N | 0.52 | 1.16 | 1.00 | 1.00 | 0.5 | 0.87 |
| 257 | L257P | 0.45 | 0.69 | 0.96 | 0.91 | 0.3 | 0.73 |
| 257 | L257R | 0.59 | 0.64 | 0.76 | 1.04 | 0.1 | 0.88 |
| 257 | L257S | 0.62 | 0.93 | 1.10 | 0.97 | 0.5 | 1.03 |
| 257 | L257T | 0.62 | 1.05 | 1.29 | 1.00 | 0.7 | 1.06 |
| 257 | L257V | 0.76 | 0.91 | 0.90 | 0.97 | 0.9 | 0.90 |
| 257 | L257Y | 0.62 | 1.17 | 1.00 | 0.98 | 0.4 | 0.84 |
| 258 | G258A | 0.59 | 1.05 | 1.13 | 1.14 | 0.1 | 0.61 |
| 258 | G258C | 0.53 | 0.87 | 1.15 | 1.03 | 0.4 | 0.62 |
| 258 | G258E | 0.57 | 0.98 | 1.11 | 1.12 | 0.9 | 0.71 |
| 258 | G258I | 0.29 | 1.26 | 1.39 | 1.41 | 0.3 | 0.53 |
| 258 | G258M | 0.43 | 1.02 | 1.05 | 1.29 | 0.1 | 0.63 |
| 258 | G258Q | 0.52 | 1.05 | 1.26 | 1.12 | 0.4 | 0.80 |
| 258 | G258V | 0.38 | 1.26 | 1.57 | 1.45 | 0.6 | 0.81 |
| 261 | F261A | 0.55 | 0.82 | 0.95 | 0.91 | 0.5 | 0.59 |
| 261 | F261C | 0.57 | 0.80 | 1.08 | 0.95 | 0.7 | 0.68 |
| 261 | F261E | 0.65 | 0.65 | 1.05 | 0.94 | 0.7 | 0.68 |
| 261 | F261G | 0.46 | 0.75 | 0.82 | 0.80 | 0.2 | 0.55 |
| 261 | F261H | 0.74 | 0.92 | 1.04 | 0.99 | 0.8 | 0.77 |
| 261 | F261L | 0.77 | 0.84 | 0.77 | 0.95 | 0.8 | 0.62 |
| 261 | F261M | 0.73 | 0.79 | 0.90 | 0.76 | 0.7 | 0.73 |
| 261 | F261N | 0.80 | 0.92 | 0.88 | 0.92 | 0.8 | 0.72 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 261 | F261P | 0.45 | 0.87 | 0.86 | 0.95 | 0.3 | 0.60 |
| 261 | F261Q | 0.57 | 0.78 | 1.10 | 0.95 | 0.7 | 0.73 |
| 261 | F261R | 0.58 | 0.71 | 0.48 | 0.93 | 0.2 | 0.66 |
| 261 | F261S | 0.54 | 0.89 | 0.87 | 0.98 | 0.5 | 0.68 |
| 261 | F261T | 0.56 | 0.98 | 1.26 | 1.25 | 0.7 | 0.88 |
| 261 | F261V | 0.57 | 0.92 | 1.09 | 0.87 | 0.7 | 0.78 |
| 261 | F261W | 0.89 | 0.70 | 0.98 | 1.18 | 1.1 | 0.80 |
| 261 | F261Y | 0.67 | 0.63 | 0.94 | 0.97 | 0.9 | 0.79 |
| 262 | Y262A | 0.83 | 0.81 | 0.96 | 0.84 | 0.5 | 0.70 |
| 262 | Y262C | 1.10 | 0.91 | 0.98 | 0.83 | 0.9 | 0.78 |
| 262 | Y262D | 0.92 | 0.99 | 0.91 | 0.97 | 0.8 | 0.61 |
| 262 | Y262E | 0.86 | 1.01 | 1.11 | 1.06 | 0.9 | 0.77 |
| 262 | Y262F | 1.11 | 0.77 | 1.10 | 0.93 | 0.9 | 0.93 |
| 262 | Y262G | 0.55 | 0.94 | 0.60 | 1.03 | 0.2 | 0.56 |
| 262 | Y262H | 0.93 | 0.81 | 0.77 | 1.01 | 0.9 | 0.87 |
| 262 | Y262I | 0.72 | 0.59 | 0.86 | 0.90 | 0.6 | 0.53 |
| 262 | Y262K | 0.84 | 0.53 | 0.67 | 0.95 | 0.1 | 0.96 |
| 262 | Y262L | 0.92 | 0.96 | 1.06 | 0.90 | 0.9 | 0.81 |
| 262 | Y262M | 1.00 | 0.60 | 0.88 | 1.02 | 0.9 | 0.75 |
| 262 | Y262N | 0.82 | 1.00 | 1.13 | 0.86 | 0.7 | 0.77 |
| 262 | Y262Q | 0.82 | 0.86 | 0.96 | 0.91 | 0.8 | 0.72 |
| 262 | Y262S | 0.79 | 1.08 | 0.91 | 1.00 | 0.7 | 0.80 |
| 262 | Y262T | 0.65 | 0.94 | 0.95 | 0.86 | 0.7 | 0.71 |
| 262 | Y262V | 0.51 | 1.01 | 0.90 | 0.94 | 0.6 | 0.69 |
| 262 | Y262W | 0.83 | 0.81 | 0.99 | 1.03 | 0.8 | 0.89 |
| 263 | Y263A | 0.56 | 0.84 | 0.93 | 0.93 | 0.1 | 0.76 |
| 263 | Y263C | 0.98 | 0.86 | 0.98 | 0.93 | 0.6 | 0.90 |
| 263 | Y263D | 0.31 | 1.61 | 1.44 | 1.22 | 0.5 | 0.71 |
| 263 | Y263E | 0.47 | 1.02 | 0.98 | 0.86 | 0.5 | 0.80 |
| 263 | Y263F | 1.03 | 1.14 | 0.97 | 0.89 | 0.9 | 0.80 |
| 263 | Y263K | 0.22 | 2.39 | 2.76 | 1.86 | 0.1 | 0.34 |
| 263 | Y263L | 0.84 | 0.89 | 0.83 | 0.95 | 0.2 | 1.00 |
| 263 | Y263M | 0.96 | 0.99 | 1.08 | 0.97 | 0.6 | 0.74 |
| 263 | Y263R | 0.21 | 8.02 | 4.08 | 1.72 | 0.1 | 0.26 |
| 263 | Y263S | 0.47 | 1.06 | 0.82 | 0.95 | 0.2 | 0.77 |
| 263 | Y263T | 0.91 | 0.84 | 0.97 | 1.10 | 0.7 | 0.97 |
| 263 | Y263V | 0.66 | 0.80 | 0.89 | 0.94 | 0.4 | 1.07 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 263 | Y263W | 0.35 | 0.96 | 0.90 | 0.95 | 0.1 | 0.64 |
| 264 | G264A | 0.55 | 0.81 | 0.96 | 0.93 | 0.3 | 0.79 |
| 265 | K265A | 1.18 | 1.03 | 1.24 | 0.86 | 0.8 | 0.96 |
| 265 | K265C | 0.92 | 0.96 | 1.54 | 1.11 | 0.9 | 0.93 |
| 265 | K265D | 0.59 | 1.05 | 1.24 | 0.85 | 0.9 | 0.77 |
| 265 | K265E | 0.70 | 1.00 | 1.32 | 0.90 | 1.0 | 0.94 |
| 265 | K265G | 0.89 | 1.22 | 1.38 | 1.04 | 0.7 | 0.97 |
| 265 | K265H | 0.69 | 1.16 | 1.06 | 0.92 | 1.1 | 0.88 |
| 265 | K265L | 0.59 | 1.11 | 1.44 | 1.11 | 1.2 | 0.97 |
| 265 | K265M | 0.78 | 1.04 | 1.19 | 0.99 | 1.0 | 0.88 |
| 265 | K265N | 1.05 | 1.07 | 1.31 | 1.00 | 1.2 | 0.85 |
| 265 | K265P | 0.25 | 1.61 | 1.92 | 1.59 | 1.0 | 0.80 |
| 265 | K265Q | 0.76 | 1.16 | 1.19 | 1.03 | 1.3 | 0.82 |
| 265 | K265R | 1.16 | 1.11 | 0.88 | 1.17 | 1.1 | 0.92 |
| 265 | K265S | 1.06 | 1.13 | 1.15 | 1.04 | 1.0 | 0.88 |
| 265 | K265T | 0.80 | 1.08 | 1.20 | 1.01 | 1.1 | 0.87 |
| 265 | K265V | 0.51 | 1.12 | 1.33 | 0.96 | 0.9 | 0.87 |
| 265 | K265W | 0.75 | 1.28 | 1.35 | 1.08 | 1.0 | 1.20 |
| 265 | K265Y | 0.85 | 1.07 | 1.25 | 1.02 | 1.0 | 1.26 |
| 267 | L267A | 0.82 | 1.07 | 1.22 | 1.07 | 0.9 | 1.10 |
| 267 | L267C | 0.68 | 1.04 | 1.22 | 1.19 | 0.9 | 1.14 |
| 267 | L267E | 0.58 | 1.17 | 1.35 | 1.10 | 1.1 | 1.19 |
| 267 | L267F | 0.72 | 0.98 | 1.11 | 1.13 | 0.2 | 0.81 |
| 267 | L267G | 0.62 | 0.71 | 1.13 | 1.16 | 0.3 | 0.97 |
| 267 | L267H | 0.63 | 1.07 | 1.21 | 1.13 | 0.1 | 1.05 |
| 267 | L267I | 0.91 | 1.06 | 1.25 | 1.07 | 1.1 | 0.99 |
| 267 | L267M | 0.77 | 0.94 | 1.22 | 0.95 | 0.9 | 1.00 |
| 267 | L267Q | 0.73 | 1.14 | 1.32 | 1.11 | 0.9 | 1.27 |
| 267 | L267S | 0.71 | 1.03 | 1.07 | 1.16 | 0.5 | 1.15 |
| 267 | L267T | 0.63 | 1.19 | 1.25 | 1.33 | 0.5 | 1.33 |
| 267 | L267V | 0.73 | 1.12 | 1.08 | 1.24 | 0.8 | 1.06 |
| 268 | I268A | 0.86 | 0.85 | 0.88 | 0.95 | 0.7 | 0.68 |
| 268 | I268C | 0.95 | 0.94 | 0.73 | 1.18 | 0.9 | 0.78 |
| 268 | I268E | 0.22 | 0.83 | 1.00 | 0.50 | 0.5 | 0.07 |
| 268 | I268L | 0.99 | 0.85 | 1.01 | 1.08 | 0.7 | 0.91 |
| 268 | I268M | 0.88 | 1.03 | 1.06 | 1.04 | 0.3 | 1.08 |
| 268 | I268P | 0.69 | 0.69 | 0.76 | 0.82 | 0.4 | 0.60 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 268 | I268T | 0.25 | 1.90 | 1.96 | 1.76 | 0.8 | 0.44 |
| 268 | I268V | 0.93 | 0.95 | 0.76 | 1.13 | 1.0 | 0.90 |
| 269 | N269A | 0.48 | 1.02 | 1.00 | 1.07 | 0.3 | 0.82 |
| 269 | N269D | 1.25 | 1.14 | 0.89 | 1.01 | 1.3 | 0.90 |
| 269 | N269E | 0.83 | 1.26 | 1.08 | 1.05 | 1.1 | 1.01 |
| 269 | N269G | 0.53 | 0.88 | 0.95 | 1.03 | 0.1 | 0.89 |
| 269 | N2691 | 0.69 | 1.00 | 1.06 | 1.04 | 0.6 | 1.00 |
| 269 | N269K | 0.64 | 0.75 | 0.81 | 1.01 | 0.5 | 0.89 |
| 269 | N269L | 0.39 | 0.88 | 0.96 | 1.17 | 0.1 | 0.84 |
| 269 | N269M | 0.81 | 0.88 | 0.86 | 0.91 | 0.5 | 0.87 |
| 269 | N269Q | 0.84 | 1.00 | 0.85 | 1.19 | 1.0 | 0.96 |
| 269 | N269S | 0.94 | 1.02 | 1.15 | 1.12 | 0.8 | 1.00 |
| 269 | N269T | 0.56 | 0.85 | 0.91 | 0.91 | 0.3 | 0.88 |
| 269 | N269V | 0.44 | 1.01 | 0.97 | 1.24 | 0.5 | 0.95 |
| 270 | V270A | 1.21 | 0.98 | 1.07 | 1.02 | 1.0 | 0.77 |
| 270 | V270C | 1.09 | 0.99 | 1.05 | 1.03 | 1.1 | 0.90 |
| 270 | V270F | 0.17 | 2.38 | 2.92 | 1.13 | 0.2 | 0.08 |
| 270 | V270G | 0.41 | 0.98 | 0.92 | 1.09 | 0.8 | 0.84 |
| 270 | V270I | 0.88 | 0.96 | 1.09 | 1.01 | 1.0 | 0.89 |
| 270 | V270L | 0.90 | 1.02 | 1.01 | 0.87 | 0.7 | 0.86 |
| 270 | V270M | 0.81 | 0.92 | 1.01 | 0.94 | 0.4 | 0.77 |
| 270 | V270N | 0.64 | 0.94 | 1.31 | 0.89 | 0.7 | 0.93 |
| 270 | V270P | 0.50 | 0.91 | 1.08 | 0.93 | 0.8 | 0.80 |
| 270 | V270S | 1.08 | 0.99 | 0.95 | 0.78 | 1.0 | 0.84 |
| 270 | V270T | 0.94 | 0.96 | 1.07 | 1.01 | 0.8 | 1.06 |
| 271 | Q271A | 1.18 | 0.94 | 1.12 | 1.04 | 1.1 | 0.83 |
| 271 | Q271C | 0.99 | 1.02 | 0.98 | 1.08 | 1.2 | 0.86 |
| 271 | Q271D | 1.00 | 1.03 | 0.84 | 1.00 | 1.1 | 0.89 |
| 271 | Q271E | 1.01 | 0.93 | 1.16 | 0.95 | 1.2 | 1.02 |
| 271 | Q271F | 1.09 | 1.04 | 0.80 | 1.05 | 1.0 | 0.89 |
| 271 | Q271G | 0.92 | 0.97 | 0.86 | 1.06 | 0.7 | 0.96 |
| 271 | Q271H | 0.92 | 1.01 | 0.85 | 1.09 | 1.0 | 1.04 |
| 271 | Q271I | 1.07 | 1.04 | 0.88 | 0.96 | 1.0 | 0.86 |
| 271 | Q271K | 1.03 | 0.60 | 0.57 | 1.09 | 0.5 | 1.01 |
| 271 | Q271L | 1.16 | 0.90 | 0.94 | 1.01 | 1.0 | 0.88 |
| 271 | Q271M | 1.15 | 0.89 | 1.19 | 1.11 | 1.0 | 0.96 |
| 271 | Q271N | 0.99 | 0.91 | 0.91 | 1.09 | 0.9 | 1.01 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 271 | Q271P | 1.03 | 0.75 | 1.12 | 1.06 | 1.1 | 0.76 |
| 271 | Q271R | 1.23 | 0.63 | 1.09 | 1.05 | 0.2 | 0.95 |
| 271 | Q271S | 0.97 | 0.85 | 1.02 | 0.98 | 1.0 | 0.96 |
| 271 | Q271T | 0.88 | 0.97 | 0.91 | 1.11 | 0.8 | 0.99 |
| 271 | Q271V | 0.92 | 1.06 | 0.84 | 1.11 | 0.9 | 0.97 |
| 271 | Q271W | 1.00 | 0.90 | 0.94 | 1.14 | 0.9 | 1.07 |
| 271 | Q271Y | 0.96 | 0.94 | 0.95 | 0.98 | 0.9 | 0.97 |
| 272 | A272E | 1.08 | 0.82 | 1.09 | 0.96 | 1.1 | 0.79 |
| 272 | A272F | 0.88 | 1.02 | 1.08 | 0.99 | 0.9 | 0.91 |
| 272 | A272G | 1.07 | 0.79 | 1.23 | 1.03 | 0.8 | 0.91 |
| 272 | A272H | 1.12 | 0.90 | 1.19 | 1.00 | 0.9 | 0.82 |
| 272 | A272I | 0.76 | 0.72 | 1.23 | 0.98 | 1.0 | 0.94 |
| 272 | A272K | 1.00 | 0.61 | 1.02 | 0.95 | 0.9 | 0.89 |
| 272 | A272L | 0.99 | 0.87 | 1.12 | 0.95 | 1.1 | 0.81 |
| 272 | A272M | 0.98 | 0.77 | 0.98 | 1.08 | 0.9 | 0.86 |
| 272 | A272P | 0.92 | 0.93 | 1.05 | 0.95 | 0.8 | 0.92 |
| 272 | A272Q | 1.19 | 0.86 | 1.18 | 1.02 | 1.0 | 0.88 |
| 272 | A272R | 0.99 | 0.47 | 0.99 | 0.81 | 0.8 | 1.00 |
| 272 | A272S | 1.00 | 1.04 | 1.19 | 1.09 | 1.0 | 0.97 |
| 272 | A272T | 0.88 | 0.97 | 0.99 | 1.04 | 1.0 | 1.19 |
| 272 | A272V | 0.80 | 1.01 | 1.14 | 0.99 | 1.0 | 1.00 |
| 272 | A272W | 0.91 | 0.52 | 1.04 | 1.07 | 0.9 | 1.21 |
| 272 | A272Y | 0.95 | 0.95 | 1.28 | 1.00 | 1.0 | 0.93 |
| 273 | A273E | 0.59 | 0.92 | 0.99 | 1.05 | 0.2 | 0.57 |
| 273 | A273G | 0.87 | 0.93 | 1.21 | 1.22 | 0.8 | 0.94 |
| 273 | A273H | 0.73 | 1.05 | 0.98 | 1.09 | 0.4 | 0.75 |
| 273 | A273L | 0.70 | 0.88 | 1.13 | 1.08 | 0.3 | 0.71 |
| 273 | A273N | 0.41 | 1.13 | 1.13 | 1.15 | 0.1 | 0.66 |
| 273 | A273Q | 0.44 | 1.09 | 1.19 | 1.42 | 0.1 | 0.75 |
| 273 | A273S | 0.87 | 1.06 | 1.35 | 1.15 | 0.7 | 0.98 |
| 273 | A273T | 0.53 | 0.79 | 1.23 | 1.29 | 0.2 | 0.91 |
| 273 | A273V | 0.53 | 1.41 | 1.24 | 1.15 | 0.3 | 0.84 |
| 274 | A274C | 0.94 | 1.10 | 0.99 | 0.96 | 1.0 | 0.97 |
| 274 | A274D | 0.94 | 0.92 | 0.87 | 0.89 | 0.7 | 1.05 |
| 274 | A274F | 0.80 | 0.82 | 0.84 | 0.96 | 0.6 | 0.74 |
| 274 | A274G | 0.85 | 0.94 | 0.88 | 0.92 | 0.9 | 0.96 |
| 274 | A274H | 0.79 | 1.17 | 0.89 | 0.95 | 0.5 | 0.98 |

(continued)

| Position | BPN' variant | TCA PI | PI BMI pH 8 16C | PI BMI pH 7 16C | PI BMI pH 8 32C | LAS-EDTA PI | AAPF PI |
|---|---|---|---|---|---|---|---|
| 274 | A274I | 0.98 | 0.99 | 0.92 | 1.07 | 1.0 | 1.03 |
| 274 | A274K | 0.54 | 0.94 | 0.61 | 0.95 | 0.2 | 0.76 |
| 274 | A274L | 0.78 | 1.21 | 0.92 | 1.03 | 1.1 | 0.83 |
| 274 | A274M | 0.84 | 0.87 | 0.72 | 0.98 | 1.0 | 0.72 |
| 274 | A274P | 0.54 | 0.99 | 0.70 | 1.02 | 0.4 | 0.63 |
| 274 | A274Q | 0.77 | 1.05 | 0.90 | 1.07 | 0.8 | 0.93 |
| 274 | A274R | 0.41 | 0.85 | 0.58 | 1.12 | 0.1 | 1.12 |
| 274 | A274S | 1.12 | 0.92 | 0.88 | 1.05 | 1.0 | 0.97 |
| 274 | A274T | 0.91 | 1.15 | 0.83 | 1.06 | 1.0 | 1.08 |
| 274 | A274V | 0.96 | 1.14 | 0.76 | 1.19 | 1.0 | 1.10 |
| 274 | A274W | 0.37 | 1.32 | 0.74 | 1.09 | 0.5 | 0.70 |
| 274 | A274Y | 0.57 | 1.06 | 0.86 | 1.05 | 0.6 | 0.93 |
| 275 | Q275A | 0.70 | 0.95 | 1.23 | 0.99 | 1.0 | 0.84 |
| 275 | Q275C | 0.41 | 1.04 | 1.23 | 0.91 | 1.1 | 0.83 |
| 275 | Q275D | 1.04 | 1.21 | 1.03 | 1.01 | 1.1 | 1.08 |
| 275 | Q275E | 1.17 | 1.01 | 1.20 | 1.23 | 1.1 | 0.95 |
| 275 | Q275F | 0.70 | 0.86 | 0.97 | 0.89 | 0.9 | 1.01 |
| 275 | Q275G | 0.90 | 0.90 | 0.81 | 1.05 | 1.1 | 0.77 |
| 275 | Q275H | 1.06 | 0.79 | 0.96 | 1.01 | 1.1 | 0.88 |
| 275 | Q275K | 1.02 | 0.70 | 0.80 | 1.06 | 0.9 | 0.92 |
| 275 | Q275L | 0.86 | 0.78 | 1.01 | 0.97 | 1.0 | 1.00 |
| 275 | Q275M | 0.96 | 1.16 | 0.96 | 0.97 | 1.0 | 0.81 |
| 275 | Q275P | 0.74 | 1.10 | 1.00 | 1.00 | 1.1 | 0.96 |
| 275 | Q275R | 1.05 | 0.81 | 0.80 | 1.03 | 0.9 | 0.97 |
| 275 | Q275S | 0.91 | 1.12 | 1.13 | 1.01 | 1.0 | 0.97 |
| 275 | Q275T | 0.85 | 1.06 | 1.01 | 1.05 | 1.0 | 0.98 |
| 275 | Q275V | 0.70 | 1.06 | 1.09 | 0.97 | 1.1 | 1.07 |
| 275 | Q275W | 0.96 | 1.02 | 1.08 | 1.20 | 1.0 | 1.09 |

## EXAMPLE 7

### Comparative Evaluation of Variant Protease Data

In this example, results of experiments conducted to determine cleaning performance, LAS stability, AAPF activity and protein content (tests of properties of interest) of BPN', GG36 (GCI-P036) and variant proteases are compared. As described throughout functionality of the variant proteases is quantified as a performance index (PI), which is the ratio of performance of a variant to a reference protease. PI classifications used herein include: Up mutations (PI > 1.0); Neutral mutations (PI > 0.5); Non-deleterious mutations (PI > 0.05); and Deleterious mutations (PI ≤ 0.05).

Productive sites are those having at least one Up mutation for any property. Productive, non-restrictive sites are those having ≥20% neutral mutations (PI > 0.5) and at least one Up mutation (PI > 1.0) for any property tested (besides protein expression). In Table 7-1 below, the results for variants that meet the definition of a productive, non-restrictive site are shown as a percentage (%) of variants test that meet the definition of an Up mutation (PI > 1).

**Table 7-1 Productive, Non-Restrictive Sites for BPN' and GG36**

| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
|---|---|---|---|---|---|---|---|---|---|---|---|---|---|---|---|
| 1 | **A** | 19 | 63 | 21 | 47 | 32 | 26 | 32 | **A** | 16 | 44 | 38 | 6 | 94 | 88 |
| 2 | **Q** | 19 | 11 | 32 | 26 | 58 | 0 | 0 | **Q** | 15 | 20 | 73 | 0 | 100 | 100 |
| 3 | **S** | 19 | 84 | 21 | 32 | 32 | 21 | 26 | **S** | 15 | 13 | 20 | 20 | 100 | 100 |
| 4 | **V** | 19 | 26 | 26 | 74 | 26 | 5 | 42 | **V** | 15 | 33 | 53 | 0 | 93 | 93 |
| 5 | **P** | 19 | 0 | 42 | 37 | 42 | 0 | 11 | **P** | 16 | 56 | 56 | 0 | 38 | 44 |
| 6 | **Y** | 19 | 58 | 63 | 37 | 11 | 11 | 11 | **W** | 13 | 31 | 0 | 0 | 0 | 0 |
| 7 | **G** | 15 | 0 | 7 | 7 | 27 | 0 | 0 | **G** | 18 | 22 | 28 | 0 | 39 | 39 |
| 8 | **V** | 16 | 0 | 38 | 13 | 75 | 0 | 0 | **I** | 14 | 29 | 36 | 7 | 43 | 43 |
| 9 | **S** | 19 | 74 | 11 | 11 | 5 | 16 | 42 | **S** | 16 | 75 | 63 | 19 | 88 | 63 |
| 10 | **Q** | 19 | 74 | 11 | 21 | 11 | 5 | 26 | **R** | 17 | 88 | 47 | 29 | 41 | 35 |
| 11 | **I** | 19 | 16 | 37 | 26 | 21 | 5 | 16 | **V** | 15 | 53 | 33 | 0 | 27 | 27 |
| 12 | **K** | 19 | 79 | 11 | 16 | 16 | 79 | 16 | **Q** | 16 | 69 | 63 | 25 | 69 | 69 |
| 13 | **A** | 19 | 11 | 37 | 21 | 16 | 11 | 11 | **A** | 13 | 31 | 31 | 0 | 31 | 23 |
| 14 | **P** | 19 | 68 | 0 | 63 | 16 | 0 | 79 | **P** | 15 | 73 | 100 | 7 | 73 | 53 |
| 15 | **A** | 19 | 79 | 0 | 0 | 26 | 42 | 21 | **A** | 13 | 23 | 31 | 54 | 100 | 100 |
| 16 | **L** | 19 | 32 | 5 | 79 | 58 | 11 | 11 | **A** | 15 | 60 | 33 | 7 | 47 | 47 |
| 17 | **H** | 19 | 26 | 11 | 42 | 47 | 0 | 5 | **H** | 16 | 56 | 56 | 13 | 63 | 31 |
| 18 | **S** | 19 | 89 | 0 | 84 | 37 | 32 | 5 | **N** | 19 | 47 | 58 | 32 | 95 | 89 |

**Table 7-1 Productive, Non-Restrictive Sites for BPN' and GG36**

| Position (BPN' #) | BPN' WT Residue | # BPN' Variants | BPN' TCA | BPN' BMI pH7/16°C | BPN' BMI pH8/16 °C | BPN' BMI pH8/32 ° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
|---|---|---|---|---|---|---|---|---|---|---|---|---|---|---|---|
| 19 | **Q** | 19 | 21 | 42 | 26 | 11 | 21 | 42 | **R** | 18 | 89 | 44 | 89 | 83 | 67 |
| 20 | **G** | 19 | 16 | 0 | 84 | 58 | 21 | 26 | **G** | 16 | 19 | 13 | 50 | 100 | 94 |
| 21 | **Y** | 17 | 41 | 0 | 6 | 35 | 35 | 12 | **L** | 17 | 29 | 18 | 12 | 94 | 82 |
| 22 | **T** | 18 | 78 | 44 | 0 | 78 | 83 | 39 | **T** | 15 | 33 | 40 | 53 | 93 | 93 |
| 24 | **S** | 16 | 56 | 75 | 13 | 81 | 88 | 50 | **S** | 16 | 31 | 56 | 75 | 94 | 94 |
| 25 | **N** | 19 | 47 | 0 | 84 | 63 | 37 | 37 | **G** | 15 | 80 | 80 | 67 | 87 | 47 |
| 26 | **V** | 17 | 6 | 35 | 12 | 76 | 59 | 18 | **V** | 12 | 33 | 42 | 17 | 83 | 75 |
| 27 | **K** | 19 | 21 | 11 | 84 | 42 | 58 | 42 | **K** | 17 | 65 | 29 | 47 | 82 | 65 |
| 28 | **V** | 16 | 13 | 38 | 25 | 56 | 44 | 6 | **V** | 13 | 54 | 15 | 31 | 23 | 23 |
| 29 | **A** | 17 | 0 | 35 | 29 | 35 | 18 | 12 | **A** | 16 | 38 | 25 | 6 | 13 | 0 |
| 30 | **V** | 18 | 11 | 50 | 50 | 50 | 17 | 0 | **V** | 14 | 50 | 21 | 29 | 0 | 29 |
| 31 | **I** | 19 | 11 | 68 | 58 | 58 | 79 | 53 | **L** | 13 | 31 | 31 | 15 | 46 | 38 |
| 33 | **S** | 15 | 0 | 60 | 0 | 20 | 7 | 7 | **T** | 18 | 61 | 28 | 11 | 6 | 28 |
| 34 | **G** | 19 | 0 | 5 | 11 | 5 | 0 | 0 | **G** | 15 | 13 | 0 | 0 | 0 | 0 |
| 35 | **I** | 19 | 0 | 5 | 53 | 47 | 0 | 11 | **I** | 11 | 82 | 27 | 9 | 27 | 27 |
| 36 | **D** | 18 | 11 | 17 | 11 | 22 | 0 | 0 | **S** | 17 | 47 | 24 | 12 | 65 | 76 |
| 37 | **S** | 19 | 47 | 58 | 26 | 5 | 16 | 37 | **-** | - | - | - | - | - | - |
| 38 | **S** | 19 | 74 | 42 | 53 | 74 | 68 | 16 | **T** | 14 | 29 | 29 | 43 | 93 | 79 |
| 39 | **H** | 19 | 5 | 11 | 53 | 47 | 0 | 26 | **H** | 17 | 53 | 6 | 0 | 6 | 0 |
| 40 | **P** | 19 | 47 | 74 | 74 | 84 | 16 | 89 | **P** | 17 | 53 | 100 | 29 | 100 | 82 |
| 41 | **D** | 19 | 0 | 37 | 16 | 26 | 0 | 0 | **D** | 18 | 17 | 6 | 0 | 6 | 6 |
| 42 | **L** | 19 | 5 | 16 | 16 | 26 | 0 | 5 | **L** | 18 | 50 | 17 | 0 | 39 | 28 |
| 43 | **K** | 19 | 16 | 0 | 74 | 0 | 74 | 79 | **N** | 16 | 31 | 25 | 63 | 100 | 81 |
| 44 | **V** | 19 | 0 | 21 | 74 | 68 | 0 | 16 | **I** | 17 | 24 | 24 | 47 | 88 | 94 |
| 45 | **A** | 16 | 81 | 31 | 6 | 31 | 81 | 94 | **R** | 18 | 78 | 22 | 89 | 89 | 89 |
| 46 | **G** | 17 | 0 | 59 | 18 | 59 | 18 | 41 | **G** | 17 | 71 | 18 | 94 | 65 | 71 |
| 47 | **G** | 19 | 0 | 26 | 42 | 68 | 5 | 0 | **G** | 15 | 80 | 53 | 0 | 13 | 20 |
| 48 | **A** | 17 | 65 | 35 | 12 | 29 | 65 | 24 | **A** | 17 | 76 | 35 | 76 | 88 | 94 |
| 49 | **S** | 18 | 0 | 22 | 22 | 39 | 6 | 0 | **S** | 12 | 92 | 33 | 25 | 8 | 8 |
| 50 | **M** | 17 | 6 | 82 | 6 | 41 | 65 | 65 | **F** | 11 | 64 | 45 | 73 | 36 | 45 |
| 51 | **V** | 19 | 0 | 42 | 68 | 63 | 26 | 0 | **V** | 11 | 73 | 9 | 18 | 55 | 55 |
| 52 | **P** | 19 | 0 | 74 | 95 | 89 | 5 | 0 | **P** | 16 | 75 | 100 | 38 | 94 | 69 |
| 53 | **S** | 19 | 74 | 37 | 63 | 26 | 53 | 16 | **G** | 17 | 53 | 12 | 12 | 76 | 82 |
| 54 | **E** | 19 | 0 | 53 | 53 | 47 | 0 | 0 | **E** | 18 | 28 | 17 | 22 | 39 | 78 |
| 55 | **T** | 19 | 53 | 21 | 42 | 0 | 79 | 47 | **P** | 18 | 56 | 22 | 33 | 72 | 83 |
| 56 | **N** | 19 | 5 | 68 | 74 | 63 | 5 | 0 | **S** | 15 | 67 | 40 | 33 | 60 | 73 |
| 57 | **P** | 19 | 5 | 68 | 84 | 63 | 0 | 0 | **T** | 18 | 44 | 44 | 39 | 100 | 83 |

**Table 7-1 Productive, Non-Restrictive Sites for BPN' and GG36**

| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
|---|---|---|---|---|---|---|---|---|---|---|---|---|---|---|---|
| 58 | **F** | 19 | 11 | 63 | 89 | 32 | 11 | 16 | **-** | - | - | - | - | - | - |
| 59 | **Q** | 19 | 68 | 68 | 84 | 53 | 47 | 16 | **Q** | 18 | 44 | 22 | 11 | 78 | 78 |
| 60 | **D** | 19 | 5 | 5 | 58 | 32 | 0 | 0 | **D** | 17 | 88 | 53 | 0 | 0 | 0 |
| 61 | **N** | 19 | 100 | 53 | 53 | 21 | 68 | 68 | **G** | 15 | 47 | 27 | 87 | 100 | 73 |
| 62 | **N** | 19 | 74 | 58 | 42 | 26 | 74 | 16 | **N** | 16 | 75 | 69 | 31 | 75 | 94 |
| 63 | **S** | 19 | 89 | 47 | 21 | 21 | 32 | 32 | **G** | 16 | 81 | 31 | 0 | 31 | 50 |
| 66 | **T** | 19 | 5 | 0 | 0 | 0 | 0 | 0 | **T** | 14 | 71 | 7 | 7 | 7 | 7 |
| 67 | **H** | 14 | 79 | 0 | 0 | 0 | 0 | 0 | **H** | 17 | 18 | 0 | 0 | 0 | 53 |
| 68 | **V** | 19 | 26 | 42 | 26 | 26 | 11 | 0 | **V** | 19 | 26 | 5 | 0 | 0 | 53 |
| 69 | **A** | 19 | 0 | 58 | 11 | 26 | 5 | 5 | **A** | 18 | 67 | 17 | 0 | 11 | 6 |
| 71 | **T** | 19 | 5 | 32 | 47 | 47 | 0 | 5 | **T** | 18 | 67 | 39 | 0 | 11 | 17 |
| 72 | **V** | 17 | 6 | 65 | 71 | 53 | 6 | 0 | **I** | 16 | 50 | 38 | 13 | 31 | 31 |
| 73 | **A** | 17 | 6 | 59 | 59 | 65 | 0 | 18 | **A** | 14 | 57 | 71 | 7 | 71 | 36 |
| 74 | **A** | 19 | 11 | 68 | 5 | 11 | 0 | 0 | **A** | 15 | 13 | 13 | 0 | 13 | 13 |
| 75 | **L** | 19 | 32 | 11 | 11 | 63 | 0 | 68 | **L** | 17 | 100 | 88 | 0 | 76 | 24 |
| 76 | **N** | 19 | 32 | 11 | 5 | 42 | 5 | 58 | **N** | 16 | 38 | 63 | 6 | 75 | 63 |
| 77 | **N** | 19 | 0 | 63 | 11 | 21 | 0 | 0 | **N** | 17 | 88 | 6 | 0 | 6 | 0 |
| 78 | **S** | 19 | 84 | 74 | 16 | 26 | 42 | 32 | **S** | 18 | 67 | 89 | 44 | 94 | 72 |
| 79 | **I** | 19 | 74 | 0 | 74 | 42 | 5 | 21 | **I** | 17 | 71 | 71 | 0 | 94 | 53 |
| 80 | **G** | 19 | 0 | 11 | 21 | 32 | 0 | 0 | **G** | 12 | 92 | 0 | 0 | 0 | 0 |
| 81 | **V** | 19 | 16 | 79 | 5 | 74 | 0 | 0 | **V** | 18 | 72 | 44 | 0 | 22 | 39 |
| 82 | **L** | 18 | 0 | 78 | 72 | 83 | 0 | 17 | **L** | 16 | 56 | 69 | 0 | 44 | 38 |
| 84 | **V** | 19 | 21 | 0 | 0 | 5 | 0 | 26 | **V** | 16 | 56 | 31 | 0 | 44 | 38 |
| 85 | **A** | 15 | 0 | 20 | 13 | 7 | 0 | 0 | **A** | 11 | 18 | 18 | 0 | 0 | 0 |
| 86 | **P** | 14 | 0 | 79 | 71 | 93 | 0 | 57 | **P** | 13 | 77 | 62 | 15 | 46 | 15 |
| 87 | **S** | 16 | 69 | 94 | 38 | 69 | 25 | 25 | **S** | 14 | 50 | 50 | 14 | 93 | 86 |
| 88 | **A** | 16 | 0 | 69 | 88 | 50 | 6 | 44 | **A** | 14 | 71 | 43 | 0 | 43 | 7 |
| 89 | **S** | 19 | 5 | 58 | 74 | 53 | 16 | 32 | **E** | 17 | 71 | 76 | 12 | 71 | 41 |
| 90 | **L** | 14 | 0 | 79 | 71 | 71 | 7 | 14 | **L** | 16 | 56 | 31 | 13 | 38 | 31 |
| 91 | **Y** | 19 | 5 | 26 | 74 | 63 | 58 | 5 | **Y** | 15 | 93 | 67 | 47 | 47 | 33 |
| 92 | **A** | 15 | 7 | 53 | 53 | 60 | 7 | 0 | **A** | 17 | 94 | 35 | 12 | 18 | 24 |
| 93 | **V** | 15 | 0 | 40 | 40 | 40 | 20 | 7 | **V** | 16 | 38 | 19 | 56 | 19 | 19 |
| 94 | **K** | 16 | 0 | 25 | 44 | 19 | 13 | 0 | **K** | 18 | 67 | 11 | 0 | 0 | 11 |
| 95 | **V** | 18 | 6 | 17 | 6 | 11 | 17 | 11 | **V** | 18 | 56 | 11 | 22 | 11 | 22 |
| 96 | **L** | 17 | 0 | 71 | 29 | 53 | 12 | 0 | **L** | 16 | 13 | 25 | 31 | 13 | 75 |
| 97 | **G** | 17 | 35 | 94 | 53 | 94 | 59 | 6 | **G** | 18 | 50 | 50 | 67 | 61 | 89 |
| 98 | **A** | 19 | 42 | 0 | 63 | 63 | 32 | 47 | **A** | 15 | 60 | 53 | 60 | 93 | 73 |

**Table 7-1 Productive, Non-Restrictive Sites for BPN' and GG36**

| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
|---|---|---|---|---|---|---|---|---|---|---|---|---|---|---|---|
| 99 | **D** | 18 | 11 | 83 | 0 | 61 | 50 | 0 | **S** | 14 | 50 | 36 | 50 | 71 | 79 |
| 100 | **G** | 19 | 16 | 79 | 68 | 79 | 21 | 0 | **G** | 16 | 19 | 25 | 56 | 19 | 81 |
| 101 | **S** | 16 | 56 | 100 | 19 | 50 | 75 | 56 | **S** | 17 | 47 | 76 | 88 | 76 | 88 |
| 102 | **G** | 14 | 7 | 29 | 14 | 14 | 14 | 0 | **G** | 16 | 50 | 19 | 19 | 0 | 19 |
| 103 | **Q** | 16 | 50 | 63 | 38 | 19 | 69 | 19 | **S** | 16 | 50 | 50 | 56 | 56 | 63 |
| 104 | **Y** | 17 | 0 | 47 | 59 | 65 | 47 | 6 | **V** | 15 | 40 | 47 | 60 | 53 | 53 |
| 105 | **S** | 19 | 0 | 47 | 42 | 32 | 53 | 11 | **S** | 19 | 53 | 26 | 37 | 63 | 53 |
| 106 | **W** | 19 | 0 | 74 | 63 | 74 | 26 | 0 | **S** | 14 | 64 | 57 | 71 | 71 | 57 |
| 107 | **I** | 15 | 0 | 40 | 33 | 27 | 13 | 7 | **I** | 18 | 11 | 61 | 6 | 11 | 56 |
| 108 | **I** | 19 | 5 | 37 | 42 | 32 | 5 | 0 | **A** | 16 | 19 | 38 | 0 | 13 | 19 |
| 109 | **N** | 19 | 11 | 32 | 11 | 68 | 58 | 42 | **Q** | 18 | 50 | 78 | 39 | 78 | 39 |
| 110 | **G** | 16 | 0 | 19 | 25 | 19 | 0 | 0 | **G** | 17 | 6 | 6 | 0 | 6 | 6 |
| 111 | **I** | 19 | 5 | 37 | 21 | 42 | 11 | 0 | **L** | 16 | 44 | 25 | 6 | 0 | 13 |
| 112 | **E** | 19 | 0 | 42 | 21 | 53 | 63 | 0 | **E** | 17 | 76 | 29 | 24 | 18 | 12 |
| 113 | **W** | 19 | 0 | 5 | 42 | 21 | 26 | 0 | **W** | 13 | 0 | 0 | 46 | 0 | 0 |
| 114 | **A** | 18 | 0 | 33 | 33 | 33 | 11 | 17 | **A** | 11 | 18 | 18 | 9 | 9 | 9 |
| 115 | **I** | 19 | 0 | 79 | 42 | 79 | 26 | 16 | **G** | 17 | 53 | 76 | 18 | 94 | 82 |
| 116 | **A** | 19 | 53 | 47 | 42 | 47 | 26 | 74 | **N** | 14 | 86 | 43 | 29 | 100 | 79 |
| 117 | **N** | 19 | 5 | 53 | 47 | 68 | 58 | 47 | **N** | 11 | 64 | 73 | 73 | 73 | 45 |
| 118 | **N** | 19 | 32 | 26 | 42 | 32 | 58 | 68 | **G** | 17 | 76 | 88 | 76 | 47 | 47 |
| 119 | **M** | 18 | 0 | 50 | 56 | 28 | 33 | 17 | **M** | 13 | 46 | 23 | 69 | 23 | 15 |
| 120 | **D** | 19 | 47 | 47 | 68 | 37 | 68 | 5 | **H** | 13 | 69 | 92 | 69 | 100 | 85 |
| 121 | **V** | 19 | 5 | 5 | 16 | 5 | 21 | 0 | **V** | 15 | 80 | 33 | 27 | 53 | 40 |
| 122 | **I** | 19 | 0 | 42 | 42 | 47 | 32 | 5 | **A** | 12 | 58 | 50 | 0 | 25 | 33 |
| 123 | **N** | 19 | 0 | 58 | 58 | 47 | 0 | 0 | **N** | 12 | 17 | 8 | 0 | 0 | 42 |
| 124 | **M** | 18 | 17 | 50 | 44 | 39 | 11 | 0 | **L** | 12 | 50 | 25 | 0 | 8 | 17 |
| 126 | **L** | 14 | 21 | 57 | 36 | 43 | 14 | 0 | **L** | 18 | 0 | 11 | 22 | 0 | 83 |
| 127 | **G** | 17 | 53 | 24 | 0 | 0 | 0 | 0 | **G** | 12 | 8 | 42 | 8 | 0 | 67 |
| 128 | **G** | 17 | 12 | 41 | 35 | 29 | 6 | 0 | **S** | 17 | 18 | 24 | 82 | 59 | 94 |
| 129 | **P** | 19 | 58 | 63 | 37 | 53 | 16 | 32 | **P** | 14 | 21 | 7 | 7 | 100 | 93 |
| 130 | **S** | 19 | 74 | 11 | 16 | 11 | 53 | 42 | **S** | 10 | 30 | 40 | 40 | 80 | 90 |
| 131 | **G** | 19 | 5 | 0 | 32 | 11 | 16 | 32 | **P** | 12 | 58 | 17 | 17 | 58 | 83 |
| 132 | **S** | 19 | 5 | 53 | 53 | 47 | 16 | 0 | **S** | 12 | 75 | 75 | 8 | 58 | 25 |
| 133 | **A** | 19 | 21 | 21 | 5 | 0 | 63 | 58 | **A** | 10 | 60 | 30 | 50 | 80 | 80 |
| 134 | **A** | 19 | 0 | 42 | 26 | 26 | 58 | 21 | **T** | 12 | 67 | 25 | 25 | 17 | 8 |
| 135 | **L** | 13 | 8 | 62 | 46 | 46 | 15 | 0 | **L** | 13 | 54 | 8 | 8 | 15 | 15 |
| 136 | **K** | 19 | 5 | 68 | 42 | 26 | 21 | 26 | **E** | 17 | 12 | 47 | 0 | 41 | 35 |

| Table 7-1 Productive, Non-Restrictive Sites for BPN' and GG36 | | | | | | | | | | | | | | | |
|---|---|---|---|---|---|---|---|---|---|---|---|---|---|---|---|
| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
| 137 | **A** | 19 | 21 | 47 | 16 | 16 | 53 | 89 | **Q** | 13 | 62 | 69 | 38 | 77 | 69 |
| 138 | **A** | 19 | 0 | 63 | 53 | 74 | 0 | 32 | **A** | 14 | 43 | 29 | 14 | 0 | 7 |
| 139 | **V** | 19 | 5 | 16 | 16 | 32 | 11 | 11 | **V** | 15 | 73 | 27 | 0 | 13 | 13 |
| 140 | **D** | 19 | 5 | 5 | 11 | 0 | 0 | 21 | **N** | 17 | 82 | 53 | 6 | 76 | 65 |
| 141 | **K** | 19 | 21 | 21 | 89 | 79 | 74 | 79 | **S** | 13 | 54 | 54 | 46 | 69 | 77 |
| 142 | **A** | 19 | 0 | 42 | 32 | 32 | 5 | 26 | **A** | 19 | 58 | 37 | 0 | 21 | 11 |
| 143 | **V** | 19 | 16 | 11 | 47 | 37 | 26 | 47 | **T** | 15 | 73 | 33 | 7 | 80 | 60 |
| 144 | **A** | 19 | 53 | 68 | 16 | 26 | 42 | 58 | **S** | 18 | 50 | 39 | 33 | 78 | 67 |
| 145 | **S** | 19 | 47 | 16 | 84 | 74 | 53 | 74 | **R** | 17 | 82 | 65 | 53 | 41 | 35 |
| 146 | **G** | 19 | 5 | 32 | 74 | 47 | 21 | 58 | **G** | 17 | 88 | 29 | 6 | 12 | 6 |
| 147 | **V** | 16 | 6 | 88 | 94 | 50 | 56 | 38 | **V** | 12 | 42 | 8 | 8 | 33 | 33 |
| 148 | **V** | 17 | 0 | 47 | 65 | 65 | 18 | 6 | **L** | 18 | 56 | 39 | 0 | 56 | 39 |
| 149 | **V** | 16 | 0 | 44 | 69 | 63 | 88 | 19 | **V** | 17 | 71 | 18 | 24 | 24 | 6 |
| 150 | **V** | 18 | 0 | 17 | 44 | 50 | 28 | 11 | **V** | 14 | 64 | 29 | 0 | 29 | 14 |
| 151 | **A** | 17 | 0 | 18 | 35 | 35 | 6 | 0 | **A** | 13 | 15 | 8 | 0 | 8 | 31 |
| 152 | **A** | 15 | 40 | 7 | 7 | 13 | 0 | 0 | **A** | 13 | 8 | 8 | 0 | 0 | 23 |
| 153 | **A** | 19 | 0 | 26 | 26 | 21 | 0 | 0 | **S** | 13 | 23 | 0 | 0 | 15 | 31 |
| 154 | **G** | 19 | 0 | 0 | 0 | 0 | 0 | 0 | **G** | 16 | 0 | 6 | 0 | 0 | 0 |
| 155 | **N** | 17 | 35 | 0 | 0 | 0 | 0 | 0 | **N** | 14 | 0 | 0 | 0 | 0 | 79 |
| 156 | **E** | 16 | 44 | 0 | 0 | 6 | 13 | 56 | **S** | 18 | 33 | 11 | 0 | 11 | 61 |
| 157 | **G** | 19 | 0 | 5 | 0 | 0 | 0 | 0 | **G** | 14 | 0 | 0 | 0 | 0 | 14 |
| 158 | **T** | 19 | 16 | 74 | 16 | 16 | 16 | 11 | **A** | 14 | 29 | 21 | 36 | 86 | 93 |
| 159 | **S** | 19 | 37 | 74 | 63 | 63 | 32 | 21 | **-** | - | - | - | - | - | - |
| 160 | **G** | 19 | 11 | 11 | 53 | 11 | 5 | 5 | **-** | - | - | - | - | - | - |
| 161 | **S** | 19 | 53 | 58 | 74 | 32 | 37 | 47 | **-** | - | - | - | - | - | - |
| 162 | **S** | 19 | 32 | 63 | 11 | 16 | 37 | 37 | **-** | - | - | - | - | - | - |
| 163 | **S** | 19 | 5 | 21 | 5 | 5 | 0 | 0 | **G** | 17 | 35 | 29 | 18 | 47 | 47 |
| 164 | **T** | 17 | 0 | 12 | 29 | 29 | 0 | 0 | **S** | 15 | 27 | 27 | 53 | 87 | 87 |
| 165 | **V** | 15 | 0 | 7 | 13 | 33 | 7 | 0 | **I** | 17 | 6 | 12 | 6 | 6 | 59 |
| 166 | **G** | 16 | 63 | 0 | 6 | 6 | 69 | 0 | **S** | 16 | 38 | 38 | 19 | 6 | 75 |
| 167 | **Y** | 19 | 5 | 58 | 84 | 74 | 0 | 0 | **Y** | 19 | 32 | 0 | 0 | 11 | 47 |
| 169 | **G** | 18 | 6 | 11 | 6 | 11 | 6 | 6 | **A** | 19 | 11 | 16 | 0 | 0 | 11 |
| 170 | **K** | 18 | 11 | 100 | 83 | 50 | 11 | 11 | **R** | 14 | 93 | 21 | 29 | 21 | 21 |
| 171 | **Y** | 18 | 0 | 33 | 22 | 33 | 0 | 0 | **Y** | 19 | 63 | 32 | 0 | 16 | 11 |
| 172 | **P** | 17 | 0 | 88 | 71 | 76 | 24 | 76 | **A** | 16 | 56 | 56 | 25 | 94 | 81 |
| 173 | **S** | 19 | 0 | 68 | 37 | 74 | 5 | 26 | **N** | 19 | 32 | 37 | 16 | 63 | 79 |
| 174 | **V** | 19 | 0 | 32 | 21 | 42 | 0 | 16 | **A** | 18 | 17 | 17 | 6 | 17 | 22 |

**Table 7-1 Productive, Non-Restrictive Sites for BPN' and GG36**

| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
|---|---|---|---|---|---|---|---|---|---|---|---|---|---|---|---|
| 175 | **I** | 19 | 0 | 42 | 26 | 37 | 5 | 11 | **M** | 16 | 50 | 38 | 0 | 50 | 38 |
| 176 | **A** | 19 | 5 | 11 | 5 | 21 | 11 | 5 | **A** | 16 | 19 | 13 | 0 | 13 | 38 |
| 177 | **V** | 19 | 0 | 5 | 11 | 26 | 0 | 0 | **V** | 17 | 18 | 12 | 0 | 24 | 24 |
| 178 | **G** | 17 | 0 | 0 | 6 | 12 | 0 | 0 | **G** | 16 | 6 | 6 | 0 | 6 | 6 |
| 179 | **A** | 19 | 0 | 11 | 5 | 16 | 0 | 0 | **A** | 14 | 14 | 7 | 0 | 0 | 0 |
| 180 | **V** | 19 | 11 | 32 | 16 | 53 | 0 | 16 | **T** | 15 | 47 | 7 | 0 | 20 | 20 |
| 181 | **D** | 19 | 5 | 5 | 5 | 32 | 0 | 16 | **D** | 12 | 0 | 33 | 0 | 8 | 8 |
| 182 | **S** | 19 | 32 | 53 | 79 | 42 | 16 | 53 | **Q** | 18 | 17 | 33 | 11 | 100 | 100 |
| 183 | **S** | 19 | 53 | 42 | 58 | 42 | 32 | 42 | **N** | 17 | 18 | 29 | 6 | 94 | 88 |
| 184 | **N** | 19 | 5 | 53 | 74 | 47 | 11 | 53 | **N** | 18 | 61 | 11 | 6 | 6 | 6 |
| 185 | **Q** | 19 | 11 | 47 | 63 | 53 | 16 | 84 | **N** | 17 | 41 | 24 | 24 | 94 | 88 |
| 186 | **R** | 19 | 0 | 89 | 95 | 79 | 5 | 0 | **R** | 17 | 53 | 12 | 24 | 12 | 6 |
| 187 | **A** | 19 | 11 | 68 | 74 | 26 | 0 | 11 | **A** | 19 | 5 | 16 | 0 | 5 | 37 |
| 188 | **S** | 19 | 47 | 32 | 84 | 26 | 11 | 32 | **S** | 16 | 6 | 0 | 19 | 100 | 100 |
| 189 | **F** | 17 | 29 | 24 | 12 | 29 | 0 | 0 | **F** | 16 | 6 | 0 | 0 | 0 | 88 |
| 190 | **S** | 19 | 0 | 21 | 26 | 16 | 0 | 0 | **S** | 19 | 0 | 0 | 0 | 0 | 84 |
| 191 | **Q** | 19 | 5 | 5 | 11 | 11 | 0 | 0 | **Q** | 16 | 6 | 0 | 13 | 0 | 75 |
| 192 | **Y** | 19 | 0 | 32 | 53 | 26 | 0 | 5 | **Y** | 17 | 6 | 12 | 6 | 0 | 53 |
| 193 | **G** | 19 | 0 | 21 | 11 | 0 | 0 | 0 | **G** | 15 | 0 | 0 | 0 | 0 | 87 |
| 194 | **P** | 19 | 0 | 74 | 47 | 68 | 0 | 68 | **A** | 17 | 18 | 35 | 35 | 94 | 94 |
| 195 | **E** | 19 | 0 | 32 | 11 | 21 | 0 | 0 | **G** | 16 | 56 | 44 | 13 | 13 | 19 |
| 196 | **L** | 19 | 0 | 21 | 21 | 26 | 5 | 5 | **L** | 14 | 21 | 14 | 0 | 7 | 43 |
| 197 | **D** | 15 | 0 | 27 | 40 | 47 | 0 | 0 | **D** | 18 | 83 | 50 | 0 | 44 | 39 |
| 198 | **V** | 18 | 0 | 6 | 33 | 33 | 11 | 17 | **I** | 17 | 41 | 12 | 0 | 59 | 59 |
| 199 | **M** | 18 | 0 | 50 | 28 | 33 | 0 | 6 | **V** | 17 | 47 | 29 | 12 | 29 | 29 |
| 200 | **A** | 19 | 0 | 42 | 16 | 32 | 0 | 16 | **A** | 11 | 36 | 27 | 0 | 18 | 18 |
| 201 | **P** | 18 | 0 | 44 | 44 | 78 | 0 | 22 | **P** | 16 | 31 | 13 | 0 | 0 | 0 |
| 203 | **V** | 18 | 0 | 33 | 78 | 67 | 17 | 50 | **V** | 16 | 75 | 19 | 13 | 19 | 25 |
| 204 | **S** | 16 | 0 | 13 | 38 | 0 | 31 | 44 | **N** | 14 | 14 | 29 | 7 | 93 | 93 |
| 205 | **I** | 19 | 0 | 16 | 11 | 32 | 5 | 11 | **V** | 15 | 33 | 13 | 13 | 7 | 13 |
| 206 | **Q** | 19 | 68 | 53 | 32 | 84 | 26 | 16 | **Q** | 18 | 67 | 28 | 17 | 94 | 44 |
| 207 | **S** | 19 | 0 | 5 | 5 | 0 | 0 | 0 | **S** | 18 | 11 | 11 | 0 | 6 | 0 |
| 208 | **T** | 19 | 0 | 37 | 37 | 37 | 0 | 21 | **T** | 18 | 39 | 22 | 0 | 22 | 11 |
| 209 | **L** | 19 | 5 | 32 | 21 | 37 | 0 | 32 | **Y** | 18 | 83 | 67 | 0 | 89 | 44 |
| 210 | **P** | 19 | 0 | 74 | 42 | 68 | 0 | 11 | **P** | 17 | 100 | 100 | 29 | 94 | 24 |
| 211 | **G** | 19 | 5 | 79 | 79 | 53 | 21 | 37 | **G** | 15 | 53 | 67 | 20 | 93 | 93 |
| 212 | **N** | 19 | 26 | 42 | 42 | 32 | 5 | 16 | **S** | 13 | 46 | 46 | 38 | 92 | 92 |

| Table 7-1 Productive, Non-Restrictive Sites for BPN' and GG36 | | | | | | | | | | | | | | | | |
|---|---|---|---|---|---|---|---|---|---|---|---|---|---|---|---|---|
| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
| 213 | **K** | 17 | 47 | 94 | 100 | 18 | 94 | 29 | **T** | 18 | 22 | 39 | 6 | 94 | 94 |
| 214 | **Y** | 18 | 0 | 72 | 50 | 44 | 0 | 0 | **Y** | 18 | 100 | 39 | 0 | 22 | 17 |
| 215 | **G** | 19 | 11 | 68 | 26 | 32 | 11 | 0 | **A** | 17 | 71 | 59 | 41 | 100 | 94 |
| 216 | **A** | 19 | 42 | 32 | 42 | 37 | 53 | 21 | **S** | 18 | 56 | 72 | 33 | 100 | 100 |
| 217 | **Y** | 19 | 32 | 47 | 26 | 58 | 53 | 58 | **L** | 16 | 50 | 81 | 25 | 6 | 19 |
| 218 | **N** | 19 | 5 | 26 | 11 | 16 | 16 | 32 | **N** | 17 | 76 | 76 | 24 | 88 | 35 |
| 219 | **G** | 19 | 5 | 0 | 0 | 0 | 16 | 0 | **G** | 16 | 0 | 0 | 0 | 0 | 6 |
| 220 | **T** | 19 | 0 | 5 | 5 | 5 | 0 | 0 | **T** | 15 | 7 | 7 | 0 | 0 | 33 |
| 222 | **M** | 18 | 67 | 0 | 0 | 17 | 28 | 6 | **M** | 17 | 6 | 12 | 12 | 0 | 94 |
| 223 | **A** | 19 | 5 | 11 | 0 | 0 | 0 | 0 | **A** | 18 | 11 | 6 | 0 | 6 | 17 |
| 224 | **S** | 19 | 11 | 37 | 26 | 42 | 5 | 5 | **T** | 17 | 29 | 18 | 12 | 18 | 35 |
| 225 | **P** | 19 | 16 | 0 | 0 | 0 | 0 | 0 | **P** | 18 | 0 | 6 | 11 | 0 | 33 |
| 226 | **H** | 19 | 0 | 58 | 53 | 63 | 0 | 16 | **H** | 16 | 75 | 44 | 0 | 6 | 6 |
| 227 | **V** | 19 | 5 | 32 | 47 | 26 | 5 | 0 | **V** | 16 | 50 | 25 | 0 | 44 | 44 |
| 228 | **A** | 16 | 0 | 25 | 25 | 38 | 6 | 0 | **A** | 17 | 18 | 6 | 12 | 29 | 24 |
| 229 | **G** | 19 | 5 | 0 | 5 | 5 | 0 | 5 | **G** | 15 | 20 | 13 | 0 | 13 | 7 |
| 230 | **A** | 19 | 11 | 21 | 47 | 58 | 11 | 21 | **A** | 16 | 81 | 50 | 6 | 69 | 44 |
| 231 | **A** | 19 | 0 | 47 | 58 | 58 | 5 | 5 | **A** | 16 | 19 | 25 | 6 | 50 | 50 |
| 232 | **A** | 13 | 0 | 38 | 54 | 23 | 38 | 15 | **A** | 10 | 60 | 30 | 20 | 30 | 20 |
| 233 | **L** | 11 | 0 | 18 | 36 | 18 | 27 | 0 | **L** | 16 | 94 | 81 | 6 | 75 | 31 |
| 234 | **I** | 19 | 32 | 37 | 32 | 26 | 21 | 32 | **V** | 12 | 58 | 33 | 8 | 75 | 75 |
| 235 | **L** | 19 | 47 | 32 | 37 | 47 | 68 | 32 | **K** | 16 | 88 | 63 | 56 | 88 | 88 |
| 236 | **S** | 19 | 42 | 32 | 21 | 32 | 26 | 42 | **Q** | 16 | 94 | 88 | 13 | 75 | 50 |
| 237 | **K** | 19 | 79 | 26 | 37 | 26 | 37 | 58 | **K** | 17 | 94 | 71 | 65 | 88 | 82 |
| 238 | **H** | 19 | 0 | 37 | 47 | 42 | 58 | 26 | **N** | 17 | 71 | 71 | 47 | 94 | 88 |
| 239 | **P** | 19 | 0 | 58 | 16 | 47 | 84 | 53 | **P** | 17 | 71 | 82 | 65 | 100 | 82 |
| 240 | **N** | 19 | 42 | 68 | 11 | 47 | 79 | 42 | **S** | 14 | 71 | 93 | 14 | 93 | 79 |
| 241 | **W** | 19 | 0 | 68 | 21 | 79 | 63 | 58 | **W** | 19 | 42 | 63 | 63 | 95 | 84 |
| 242 | **T** | 19 | 32 | 16 | 74 | 32 | 89 | 21 | **S** | 15 | 33 | 33 | 47 | 100 | 73 |
| 243 | **N** | 19 | 11 | 11 | 63 | 37 | 21 | 16 | **N** | 18 | 39 | 50 | 39 | 94 | 89 |
| 244 | **T** | 19 | 47 | 16 | 74 | 53 | 68 | 37 | **V** | 16 | 56 | 75 | 63 | 94 | 81 |
| 245 | **Q** | 19 | 16 | 11 | 79 | 26 | 84 | 32 | **Q** | 13 | 62 | 77 | 46 | 100 | 77 |
| 246 | **V** | 17 | 6 | 18 | 35 | 18 | 18 | 12 | **I** | 17 | 76 | 24 | 29 | 35 | 35 |
| 247 | **R** | 19 | 0 | 53 | 74 | 42 | 0 | 0 | **R** | 19 | 53 | 68 | 21 | 84 | 79 |
| 248 | **S** | 19 | 47 | 37 | 79 | 42 | 32 | 58 | **N** | 16 | 69 | 56 | 31 | 94 | 88 |
| 249 | **S** | 19 | 42 | 5 | 68 | 37 | 5 | 42 | **H** | 18 | 17 | 28 | 39 | 94 | 83 |
| 250 | **L** | 19 | 0 | 26 | 32 | 26 | 0 | 16 | **L** | 17 | 65 | 12 | 0 | 29 | 24 |

| Table 7-1 Productive, Non-Restrictive Sites for BPN' and GG36 | | | | | | | | | | | | | | | |
|---|---|---|---|---|---|---|---|---|---|---|---|---|---|---|---|
| Position (BPN' #) | BPN' WT Residue | # BPN' Variants | BPN' TCA | BPN' BMI pH7/16°C | BPN' BMI pH8/16°C | BPN' BMI pH8/32° | BPN' LAS /EDTA | BPN' AAPF | GG36 WT Residue | # GG36 Variants | GG36 BMI pH8/32°C | GG36 Egg | GG36 LAS/EDTA | GG36 AAPF | GG36 TCA |
| 251 | **E** | 19 | 0 | 37 | 11 | 63 | 0 | 0 | **K** | 14 | 64 | 50 | 50 | 86 | 64 |
| 252 | **N** | 19 | 21 | 58 | 37 | 74 | 53 | 42 | **N** | 16 | 38 | 81 | 63 | 88 | 75 |
| 253 | **T** | 19 | 16 | 53 | 58 | 68 | 11 | 16 | **T** | 14 | 36 | 29 | 7 | 93 | 79 |
| 254 | **T** | 19 | 16 | 53 | 47 | 63 | 11 | 5 | **A** | 18 | 39 | 22 | 0 | 22 | 11 |
| 255 | **T** | 19 | 26 | 58 | 53 | 37 | 0 | 42 | **T** | 17 | 18 | 29 | 35 | 94 | 94 |
| 256 | **K** | 19 | 11 | 89 | 95 | 58 | 0 | 68 | **S** | 17 | 47 | 82 | 35 | 100 | 65 |
| 257 | **L** | 19 | 0 | 47 | 53 | 32 | 0 | 16 | **L** | 15 | 87 | 40 | 0 | 40 | 13 |
| 258 | **G** | 17 | 0 | 47 | 82 | 94 | 0 | 0 | **G** | 17 | 82 | 59 | 0 | 82 | 53 |
| 259 | **D** | 19 | 21 | 5 | 0 | 11 | 0 | 89 | **S** | 12 | 83 | 100 | 8 | 100 | 25 |
| 260 | **S** | 19 | 26 | 63 | 63 | 37 | 0 | 47 | **T** | 14 | 64 | 86 | 21 | 100 | 79 |
| 261 | **F** | 19 | 0 | 0 | 32 | 11 | 5 | 0 | **N** | 16 | 56 | 88 | 63 | 100 | 75 |
| 262 | **Y** | 19 | 11 | 26 | 26 | 37 | 0 | 0 | **L** | 17 | 71 | 71 | 35 | 94 | 88 |
| 263 | **Y** | 19 | 5 | 37 | 32 | 26 | 0 | 5 | **Y** | 15 | 93 | 33 | 0 | 13 | 7 |
| 264 | **G** | 19 | 0 | 5 | 5 | 16 | 0 | 0 | **G** | 14 | 79 | 7 | 0 | 7 | 0 |
| 265 | **K** | 19 | 21 | 84 | 84 | 58 | 47 | 11 | **S** | 18 | 83 | 83 | 6 | 72 | 44 |
| 266 | **G** | 19 | 0 | 0 | 5 | 5 | 0 | 0 | **G** | 17 | 6 | 0 | 0 | 0 | 0 |
| 267 | **L** | 18 | 0 | 61 | 83 | 89 | 11 | 56 | **L** | 17 | 76 | 41 | 0 | 41 | 6 |
| 268 | **I** | 19 | 0 | 26 | 37 | 37 | 0 | 5 | **V** | 15 | 53 | 13 | 0 | 53 | 47 |
| 269 | **N** | 19 | 5 | 32 | 26 | 63 | 11 | 5 | **N** | 13 | 85 | 85 | 8 | 100 | 69 |
| 270 | **V** | 18 | 17 | 11 | 50 | 33 | 17 | 6 | **A** | 17 | 88 | 71 | 0 | 65 | 35 |
| 271 | **Q** | 19 | 53 | 32 | 32 | 79 | 47 | 26 | **E** | 14 | 29 | 100 | 0 | 100 | 64 |
| 272 | **A** | 19 | 26 | 16 | 68 | 32 | 37 | 16 | **A** | 16 | 69 | 63 | 25 | 100 | 69 |
| 273 | **A** | 13 | 0 | 69 | 85 | 100 | 0 | 0 | **A** | 15 | 100 | 73 | 7 | 73 | 27 |
| 274 | **A** | 19 | 5 | 42 | 0 | 53 | 16 | 26 | **T** | 15 | 87 | 80 | 13 | 87 | 60 |
| 275 | **Q** | 19 | 26 | 47 | 53 | 47 | 58 | 26 | **R** | 14 | 86 | 50 | 79 | 71 | 50 |

Highly productive sites are those having ≥20% Up mutations (PI > 1) for at least one property other than protein expression (TCA assay). In Table 7-2 below, the results for variants that meet the definition of a highly productive site are shown as a percentage (%) of variants tested that meet the definition of an Up mutation (PI > 1).

| Table 7-2 Highly Productive Sites for BPN' and GG36 | | | | | | | | | | | | | | | |
|---|---|---|---|---|---|---|---|---|---|---|---|---|---|---|---|
| Position (BPN' #) | BPN' WT Residue | # BPN' Variants | BPN' TCA | BPN' BMI pH7/16°C | BPN' BMI pH8/16°C | BPN' BMI pH8/32° | BPN' LAS | BPN' AAPF | GG36 WT Residue | # GG36 Variants | GG36 BMI pH8/32°C | GG36 Egg | GG36 LAS/EDTA | GG36 AAPF | GG36 TCA |
| 1 | **A** | 19 | 63 | 21 | 47 | 32 | 26 | 32 | **A** | 16 | 44 | 38 | 6 | 94 | 88 |
| 2 | **Q** | 19 | 11 | 32 | 26 | 58 | 0 | 0 | **Q** | 15 | 20 | 73 | 0 | 100 | 100 |
| 3 | **S** | 19 | 84 | 21 | 32 | 32 | 21 | 26 | **S** | 15 | 13 | 20 | 20 | 100 | 100 |
| 4 | **V** | 19 | 26 | 26 | 74 | 26 | 5 | 42 | **V** | 15 | 33 | 53 | 0 | 93 | 93 |
| 5 | **P** | 19 | 0 | 42 | 37 | 42 | 0 | 11 | **P** | 16 | 56 | 56 | 0 | 38 | 44 |
| 6 | **Y** | 19 | 58 | 63 | 37 | 11 | 11 | 11 | **W** | 13 | 31 | 0 | 0 | 0 | 0 |
| 7 | **G** | 15 | 0 | 7 | 7 | 27 | 0 | 0 | **G** | 18 | 22 | 28 | 0 | 39 | 39 |
| 8 | **V** | 16 | 0 | 38 | 13 | 75 | 0 | 0 | **I** | 14 | 29 | 36 | 7 | 43 | 43 |
| 9 | **S** | 19 | 74 | 11 | 11 | 5 | 16 | 42 | **S** | 16 | 75 | 63 | 19 | 88 | 63 |
| 10 | **Q** | 19 | 74 | 11 | 21 | 11 | 5 | 26 | **R** | 17 | 88 | 47 | 29 | 41 | 35 |
| 11 | **I** | 19 | 16 | 37 | 26 | 21 | 5 | 16 | **V** | 15 | 53 | 33 | 0 | 27 | 27 |
| 12 | **K** | 19 | 79 | 11 | 16 | 16 | 79 | 16 | **Q** | 16 | 69 | 63 | 25 | 69 | 69 |
| 13 | **A** | 19 | 11 | 37 | 21 | 16 | 11 | 11 | **A** | 13 | 31 | 31 | 0 | 31 | 23 |
| 14 | **P** | 19 | 68 | 0 | 63 | 16 | 0 | 79 | **P** | 15 | 73 | 100 | 7 | 73 | 53 |
| 15 | **A** | 19 | 79 | 0 | 0 | 26 | 42 | 21 | **A** | 13 | 23 | 31 | 54 | 100 | 100 |
| 16 | **L** | 19 | 32 | 5 | 79 | 58 | 11 | 11 | **A** | 15 | 60 | 33 | 7 | 47 | 47 |
| 17 | **H** | 19 | 26 | 11 | 42 | 47 | 0 | 5 | **H** | 16 | 56 | 56 | 13 | 63 | 31 |
| 18 | **S** | 19 | 89 | 0 | 84 | 37 | 32 | 5 | **N** | 19 | 47 | 58 | 32 | 95 | 89 |
| 19 | **Q** | 19 | 21 | 42 | 26 | 11 | 21 | 42 | **R** | 18 | 89 | 44 | 89 | 83 | 67 |
| 20 | **G** | 19 | 16 | 0 | 84 | 58 | 21 | 26 | **G** | 16 | 19 | 13 | 50 | 100 | 94 |
| 21 | **Y** | 17 | 41 | 0 | 6 | 35 | 35 | 12 | **L** | 17 | 29 | 18 | 12 | 94 | 82 |
| 22 | **T** | 18 | 78 | 44 | 0 | 78 | 83 | 39 | **T** | 15 | 33 | 40 | 53 | 93 | 93 |
| 24 | **S** | 16 | 56 | 75 | 13 | 81 | 88 | 50 | **S** | 16 | 31 | 56 | 75 | 94 | 94 |
| 25 | **N** | 19 | 47 | 0 | 84 | 63 | 37 | 37 | **G** | 15 | 80 | 80 | 67 | 87 | 47 |
| 26 | **V** | 17 | 6 | 35 | 12 | 76 | 59 | 18 | **V** | 12 | 33 | 42 | 17 | 83 | 75 |
| 27 | **K** | 19 | 21 | 11 | 84 | 42 | 58 | 42 | **K** | 17 | 65 | 29 | 47 | 82 | 65 |
| 28 | **V** | 16 | 13 | 38 | 25 | 56 | 44 | 6 | **V** | 13 | 54 | 15 | 31 | 23 | 23 |
| 29 | **A** | 17 | 0 | 35 | 29 | 35 | 18 | 12 | **A** | 16 | 38 | 25 | 6 | 13 | 0 |
| 30 | **V** | 18 | 11 | 50 | 50 | 50 | 17 | 0 | **V** | 14 | 50 | 21 | 29 | 0 | 29 |
| 31 | **I** | 19 | 11 | 68 | 58 | 58 | 79 | 53 | **L** | 13 | 31 | 31 | 15 | 46 | 38 |
| 33 | **S** | 15 | 0 | 60 | 0 | 20 | 7 | 7 | **T** | 18 | 61 | 28 | 11 | 6 | 28 |
| 35 | **I** | 19 | 0 | 5 | 53 | 47 | 0 | 11 | **I** | 11 | 82 | 27 | 9 | 27 | 27 |
| 36 | **D** | 18 | 11 | 17 | 11 | 22 | 0 | 0 | **S** | 17 | 47 | 24 | 12 | 65 | 76 |
| 37 | **S** | 19 | 47 | 58 | 26 | 5 | 16 | 37 | **-** | - | - | - | - | - | - |
| 38 | **S** | 19 | 74 | 42 | 53 | 74 | 68 | 16 | **T** | 14 | 29 | 29 | 43 | 93 | 79 |

| Table 7-2 Highly Productive Sites for BPN' and GG36 | | | | | | | | | | | | | | | |
|---|---|---|---|---|---|---|---|---|---|---|---|---|---|---|---|
| 39 | **H** | 19 | 5 | 11 | 53 | 47 | 0 | 26 | **H** | 17 | 53 | 6 | 0 | 6 | 0 |
| 40 | **P** | 19 | 47 | 74 | 74 | 84 | 16 | 89 | **P** | 17 | 53 | 100 | 29 | 100 | 82 |
| 41 | **D** | 19 | 0 | 37 | 16 | 26 | 0 | 0 | **D** | 18 | 17 | 6 | 0 | 6 | 6 |
| 42 | **L** | 19 | 5 | 16 | 16 | 26 | 0 | 5 | **L** | 18 | 50 | 17 | 0 | 39 | 28 |
| 43 | **K** | 19 | 16 | 0 | 74 | 0 | 74 | 79 | **N** | 16 | 31 | 25 | 63 | 100 | 81 |
| 44 | **V** | 19 | 0 | 21 | 74 | 68 | 0 | 16 | **I** | 17 | 24 | 24 | 47 | 88 | 94 |
| 45 | **A** | 16 | 81 | 31 | 6 | 31 | 81 | 94 | **R** | 18 | 78 | 22 | 89 | 89 | 89 |
| 46 | **G** | 17 | 0 | 59 | 18 | 59 | 18 | 41 | **G** | 17 | 71 | 18 | 94 | 65 | 71 |
| 47 | **G** | 19 | 0 | 26 | 42 | 68 | 5 | 0 | **G** | 15 | 80 | 53 | 0 | 13 | 20 |
| 48 | **A** | 17 | 65 | 35 | 12 | 29 | 65 | 24 | **A** | 17 | 76 | 35 | 76 | 88 | 94 |
| 49 | **S** | 18 | 0 | 22 | 22 | 39 | 6 | 0 | **S** | 12 | 92 | 33 | 25 | 8 | 8 |
| 50 | **M** | 17 | 6 | 82 | 6 | 41 | 65 | 65 | **F** | 11 | 64 | 45 | 73 | 36 | 45 |
| 51 | **V** | 19 | 0 | 42 | 68 | 63 | 26 | 0 | **V** | 11 | 73 | 9 | 18 | 55 | 55 |
| 52 | **P** | 19 | 0 | 74 | 95 | 89 | 5 | 0 | **P** | 16 | 75 | 100 | 38 | 94 | 69 |
| 53 | **S** | 19 | 74 | 37 | 63 | 26 | 53 | 16 | **G** | 17 | 53 | 12 | 12 | 76 | 82 |
| 54 | **E** | 19 | 0 | 53 | 53 | 47 | 0 | 0 | **E** | 18 | 28 | 17 | 22 | 39 | 78 |
| 55 | **T** | 19 | 53 | 21 | 42 | 0 | 79 | 47 | **P** | 18 | 56 | 22 | 33 | 72 | 83 |
| 56 | **N** | 19 | 5 | 68 | 74 | 63 | 5 | 0 | **S** | 15 | 67 | 40 | 33 | 60 | 73 |
| 57 | **P** | 19 | 5 | 68 | 84 | 63 | 0 | 0 | **T** | 18 | 44 | 44 | 39 | 100 | 83 |
| 58 | **F** | 19 | 11 | 63 | 89 | 32 | 11 | 16 | **-** | - | - | - | - | - | - |
| 59 | **Q** | 19 | 68 | 68 | 84 | 53 | 47 | 16 | **Q** | 18 | 44 | 22 | 11 | 78 | 78 |
| 60 | **D** | 19 | 5 | 5 | 58 | 32 | 0 | 0 | **D** | 17 | 88 | 53 | 0 | 0 | 0 |
| 61 | **N** | 19 | 100 | 53 | 53 | 21 | 68 | 68 | **G** | 15 | 47 | 27 | 87 | 100 | 73 |
| 62 | **N** | 19 | 74 | 58 | 42 | 26 | 74 | 16 | **N** | 16 | 75 | 69 | 31 | 75 | 94 |
| 63 | **S** | 19 | 89 | 47 | 21 | 21 | 32 | 32 | **G** | 16 | 81 | 31 | 0 | 31 | 50 |
| 66 | **T** | 19 | 5 | 0 | 0 | 0 | 0 | 0 | **T** | 14 | 71 | 7 | 7 | 7 | 7 |
| 67 | **H** | 14 | 79 | 0 | 0 | 0 | 0 | 0 | **H** | 17 | 18 | 0 | 0 | 0 | 53 |
| 68 | **V** | 19 | 26 | 42 | 26 | 26 | 11 | 0 | **V** | 19 | 26 | 5 | 0 | 0 | 53 |
| 69 | **A** | 19 | 0 | 58 | 11 | 26 | 5 | 5 | **A** | 18 | 67 | 17 | 0 | 11 | 6 |
| 71 | **T** | 19 | 5 | 32 | 47 | 47 | 0 | 5 | **T** | 18 | 67 | 39 | 0 | 11 | 17 |
| 72 | **V** | 17 | 6 | 65 | 71 | 53 | 6 | 0 | **I** | 16 | 50 | 38 | 13 | 31 | 31 |
| 73 | **A** | 17 | 6 | 59 | 59 | 65 | 0 | 18 | **A** | 14 | 57 | 71 | 7 | 71 | 36 |
| 74 | **A** | 19 | 11 | 68 | 5 | 11 | 0 | 0 | **A** | 15 | 13 | 13 | 0 | 13 | 13 |
| 75 | **L** | 19 | 32 | 11 | 11 | 63 | 0 | 68 | **L** | 17 | 100 | 88 | 0 | 76 | 24 |
| 76 | **N** | 19 | 32 | 11 | 5 | 42 | 5 | 58 | **N** | 16 | 38 | 63 | 6 | 75 | 63 |
| 77 | **N** | 19 | 0 | 63 | 11 | 21 | 0 | 0 | **N** | 17 | 88 | 6 | 0 | 6 | 0 |
| 78 | **S** | 19 | 84 | 74 | 16 | 26 | 42 | 32 | **S** | 18 | 67 | 89 | 44 | 94 | 72 |
| 79 | **I** | 19 | 74 | 0 | 74 | 42 | 5 | 21 | **I** | 17 | 71 | 71 | 0 | 94 | 53 |
| 80 | **G** | 19 | 0 | 11 | 21 | 32 | 0 | 0 | **G** | 12 | 92 | 0 | 0 | 0 | 0 |
| 81 | **V** | 19 | 16 | 79 | 5 | 74 | 0 | 0 | **V** | 18 | 72 | 44 | 0 | 22 | 39 |
| 82 | **L** | 18 | 0 | 78 | 72 | 83 | 0 | 17 | **L** | 16 | 56 | 69 | 0 | 44 | 38 |
| 84 | **V** | 19 | 21 | 0 | 0 | 5 | 0 | 26 | **V** | 16 | 56 | 31 | 0 | 44 | 38 |
| 85 | **A** | 15 | 0 | 20 | 13 | 7 | 0 | 0 | **A** | 11 | 18 | 18 | 0 | 0 | 0 |
| 86 | **P** | 14 | 0 | 79 | 71 | 93 | 0 | 57 | **P** | 13 | 77 | 62 | 15 | 46 | 15 |
| 87 | **S** | 16 | 69 | 94 | 38 | 69 | 25 | 25 | **S** | 14 | 50 | 50 | 14 | 93 | 86 |
| 88 | **A** | 16 | 0 | 69 | 88 | 50 | 6 | 44 | **A** | 14 | 71 | 43 | 0 | 43 | 7 |

**Table 7-2 Highly Productive Sites for BPN' and GG36**

| | | | | | | | | | | | | | | | |
|---|---|---|---|---|---|---|---|---|---|---|---|---|---|---|---|
| 89 | **S** | 19 | 5 | 58 | 74 | 53 | 16 | 32 | **E** | 17 | 71 | 76 | 12 | 71 | 41 |
| 90 | **L** | 14 | 0 | 79 | 71 | 71 | 7 | 14 | **L** | 16 | 56 | 31 | 13 | 38 | 31 |
| 91 | **Y** | 19 | 5 | 26 | 74 | 63 | 58 | 5 | **Y** | 15 | 93 | 67 | 47 | 47 | 33 |
| 92 | **A** | 15 | 7 | 53 | 53 | 60 | 7 | 0 | **A** | 17 | 94 | 35 | 12 | 18 | 24 |
| 93 | **V** | 15 | 0 | 40 | 40 | 40 | 20 | 7 | **V** | 16 | 38 | 19 | 56 | 19 | 19 |
| 94 | **K** | 16 | 0 | 25 | 44 | 19 | 13 | 0 | **K** | 18 | 67 | 11 | 0 | 0 | 11 |
| 95 | **V** | 18 | 6 | 17 | 6 | 11 | 17 | 11 | **V** | 18 | 56 | 11 | 22 | 11 | 22 |
| 96 | **L** | 17 | 0 | 71 | 29 | 53 | 12 | 0 | **L** | 16 | 13 | 25 | 31 | 13 | 75 |
| 97 | **G** | 17 | 35 | 94 | 53 | 94 | 59 | 6 | **G** | 18 | 50 | 50 | 67 | 61 | 89 |
| 98 | **A** | 19 | 42 | 0 | 63 | 63 | 32 | 47 | **A** | 15 | 60 | 53 | 60 | 93 | 73 |
| 99 | **D** | 18 | 11 | 83 | 0 | 61 | 50 | 0 | **S** | 14 | 50 | 36 | 50 | 71 | 79 |
| 100 | **G** | 19 | 16 | 79 | 68 | 79 | 21 | 0 | **G** | 16 | 19 | 25 | 56 | 19 | 81 |
| 101 | **S** | 16 | 56 | 100 | 19 | 50 | 75 | 56 | **S** | 17 | 47 | 76 | 88 | 76 | 88 |
| 102 | **G** | 14 | 7 | 29 | 14 | 14 | 14 | 0 | **G** | 16 | 50 | 19 | 19 | 0 | 19 |
| 103 | **Q** | 16 | 50 | 63 | 38 | 19 | 69 | 19 | **S** | 16 | 50 | 50 | 56 | 56 | 63 |
| 104 | **Y** | 17 | 0 | 47 | 59 | 65 | 47 | 6 | **V** | 15 | 40 | 47 | 60 | 53 | 53 |
| 105 | **S** | 19 | 0 | 47 | 42 | 32 | 53 | 11 | **S** | 19 | 53 | 26 | 37 | 63 | 53 |
| 106 | **W** | 19 | 0 | 74 | 63 | 74 | 26 | 0 | **S** | 14 | 64 | 57 | 71 | 71 | 57 |
| 107 | **I** | 15 | 0 | 40 | 33 | 27 | 13 | 7 | **I** | 18 | 11 | 61 | 6 | 11 | 56 |
| 108 | **I** | 19 | 5 | 37 | 42 | 32 | 5 | 0 | **A** | 16 | 19 | 38 | 0 | 13 | 19 |
| 109 | **N** | 19 | 11 | 32 | 11 | 68 | 58 | 42 | **Q** | 18 | 50 | 78 | 39 | 78 | 39 |
| 110 | **G** | 16 | 0 | 19 | 25 | 19 | 0 | 0 | **G** | 17 | 6 | 6 | 0 | 6 | 6 |
| 111 | **I** | 19 | 5 | 37 | 21 | 42 | 11 | 0 | **L** | 16 | 44 | 25 | 6 | 0 | 13 |
| 112 | **E** | 19 | 0 | 42 | 21 | 53 | 63 | 0 | **E** | 17 | 76 | 29 | 24 | 18 | 12 |
| 113 | **W** | 19 | 0 | 5 | 42 | 21 | 26 | 0 | **W** | 13 | 0 | 0 | 46 | 0 | 0 |
| 114 | **A** | 18 | 0 | 33 | 33 | 33 | 11 | 17 | **A** | 11 | 18 | 18 | 9 | 9 | 9 |
| 115 | **I** | 19 | 0 | 79 | 42 | 79 | 26 | 16 | **G** | 17 | 53 | 76 | 18 | 94 | 82 |
| 116 | **A** | 19 | 53 | 47 | 42 | 47 | 26 | 74 | **N** | 14 | 86 | 43 | 29 | 100 | 79 |
| 117 | **N** | 19 | 5 | 53 | 47 | 68 | 58 | 47 | **N** | 11 | 64 | 73 | 73 | 73 | 45 |
| 118 | **N** | 19 | 32 | 26 | 42 | 32 | 58 | 68 | **G** | 17 | 76 | 88 | 76 | 47 | 47 |
| 119 | **M** | 18 | 0 | 50 | 56 | 28 | 33 | 17 | **M** | 13 | 46 | 23 | 69 | 23 | 15 |
| 120 | **D** | 19 | 47 | 47 | 68 | 37 | 68 | 5 | **H** | 13 | 69 | 92 | 69 | 100 | 85 |
| 121 | **V** | 19 | 5 | 5 | 16 | 5 | 21 | 0 | **V** | 15 | 80 | 33 | 27 | 53 | 40 |
| 122 | **I** | 19 | 0 | 42 | 42 | 47 | 32 | 5 | **A** | 12 | 58 | 50 | 0 | 25 | 33 |
| 123 | **N** | 19 | 0 | 58 | 58 | 47 | 0 | 0 | **N** | 12 | 17 | 8 | 0 | 0 | 42 |
| 124 | **M** | 18 | 17 | 50 | 44 | 39 | 11 | 0 | **L** | 12 | 50 | 25 | 0 | 8 | 17 |
| 126 | **L** | 14 | 21 | 57 | 36 | 43 | 14 | 0 | **L** | 18 | 0 | 11 | 22 | 0 | 83 |
| 127 | **G** | 17 | 53 | 24 | 0 | 0 | 0 | 0 | **G** | 12 | 8 | 42 | 8 | 0 | 67 |
| 128 | **G** | 17 | 12 | 41 | 35 | 29 | 6 | 0 | **S** | 17 | 18 | 24 | 82 | 59 | 94 |
| 129 | **P** | 19 | 58 | 63 | 37 | 53 | 16 | 32 | **P** | 14 | 21 | 7 | 7 | 100 | 93 |
| 130 | **S** | 19 | 74 | 11 | 16 | 11 | 53 | 42 | **S** | 10 | 30 | 40 | 40 | 80 | 90 |
| 131 | **G** | 19 | 5 | 0 | 32 | 11 | 16 | 32 | **P** | 12 | 58 | 17 | 17 | 58 | 83 |
| 132 | **S** | 19 | 5 | 53 | 53 | 47 | 16 | 0 | **S** | 12 | 75 | 75 | 8 | 58 | 25 |
| 133 | **A** | 19 | 21 | 21 | 5 | 0 | 63 | 58 | **A** | 10 | 60 | 30 | 50 | 80 | 80 |
| 134 | **A** | 19 | 0 | 42 | 26 | 26 | 58 | 21 | **T** | 12 | 67 | 25 | 25 | 17 | 8 |
| 135 | **L** | 13 | 8 | 62 | 46 | 46 | 15 | 0 | **L** | 13 | 54 | 8 | 8 | 15 | 15 |

| Table 7-2 Highly Productive Sites for BPN' and GG36 | | | | | | | | | | | | | | | |
|---|---|---|---|---|---|---|---|---|---|---|---|---|---|---|---|
| 136 | **K** | 19 | 5 | 68 | 42 | 26 | 21 | 26 | **E** | 17 | 12 | 47 | 0 | 41 | 35 |
| 137 | **A** | 19 | 21 | 47 | 16 | 16 | 53 | 89 | **Q** | 13 | 62 | 69 | 38 | 77 | 69 |
| 138 | **A** | 19 | 0 | 63 | 53 | 74 | 0 | 32 | **A** | 14 | 43 | 29 | 14 | 0 | 7 |
| 139 | **V** | 19 | 5 | 16 | 16 | 32 | 11 | 11 | **V** | 15 | 73 | 27 | 0 | 13 | 13 |
| 140 | **D** | 19 | 5 | 5 | 11 | 0 | 0 | 21 | **N** | 17 | 82 | 53 | 6 | 76 | 65 |
| 141 | **K** | 19 | 21 | 21 | 89 | 79 | 74 | 79 | **S** | 13 | 54 | 54 | 46 | 69 | 77 |
| 142 | **A** | 19 | 0 | 42 | 32 | 32 | 5 | 26 | **A** | 19 | 58 | 37 | 0 | 21 | 11 |
| 143 | **V** | 19 | 16 | 11 | 47 | 37 | 26 | 47 | **T** | 15 | 73 | 33 | 7 | 80 | 60 |
| 144 | **A** | 19 | 53 | 68 | 16 | 26 | 42 | 58 | **S** | 18 | 50 | 39 | 33 | 78 | 67 |
| 145 | **S** | 19 | 47 | 16 | 84 | 74 | 53 | 74 | **R** | 17 | 82 | 65 | 53 | 41 | 35 |
| 146 | **G** | 19 | 5 | 32 | 74 | 47 | 21 | 58 | **G** | 17 | 88 | 29 | 6 | 12 | 6 |
| 147 | **V** | 16 | 6 | 88 | 94 | 50 | 56 | 38 | **V** | 12 | 42 | 8 | 8 | 33 | 33 |
| 148 | **V** | 17 | 0 | 47 | 65 | 65 | 18 | 6 | **L** | 18 | 56 | 39 | 0 | 56 | 39 |
| 149 | **V** | 16 | 0 | 44 | 69 | 63 | 88 | 19 | **V** | 17 | 71 | 18 | 24 | 24 | 6 |
| 150 | **V** | 18 | 0 | 17 | 44 | 50 | 28 | 11 | **V** | 14 | 64 | 29 | 0 | 29 | 14 |
| 151 | **A** | 17 | 0 | 18 | 35 | 35 | 6 | 0 | **A** | 13 | 15 | 8 | 0 | 8 | 31 |
| 152 | **A** | 15 | 40 | 7 | 7 | 13 | 0 | 0 | **A** | 13 | 8 | 8 | 0 | 0 | 23 |
| 153 | **A** | 19 | 0 | 26 | 26 | 21 | 0 | 0 | **S** | 13 | 23 | 0 | 0 | 15 | 31 |
| 155 | **N** | 17 | 35 | 0 | 0 | 0 | 0 | 0 | **N** | 14 | 0 | 0 | 0 | 0 | 79 |
| 156 | **E** | 16 | 44 | 0 | 0 | 6 | 13 | 56 | **S** | 18 | 33 | 11 | 0 | 11 | 61 |
| 158 | **T** | 19 | 16 | 74 | 16 | 16 | 16 | 11 | **A** | 14 | 29 | 21 | 36 | 86 | 93 |
| 159 | **S** | 19 | 37 | 74 | 63 | 63 | 32 | 21 | **-** | - | - | - | - | - | - |
| 160 | **G** | 19 | 11 | 11 | 53 | 11 | 5 | 5 | **-** | - | - | - | - | - | - |
| 161 | **S** | 19 | 53 | 58 | 74 | 32 | 37 | 47 | **-** | - | - | - | - | - | - |
| 162 | **S** | 19 | 32 | 63 | 11 | 16 | 37 | 37 | **-** | - | - | - | - | - | - |
| 163 | **S** | 19 | 5 | 21 | 5 | 5 | 0 | 0 | **G** | 17 | 35 | 29 | 18 | 47 | 47 |
| 164 | **T** | 17 | 0 | 12 | 29 | 29 | 0 | 0 | **S** | 15 | 27 | 27 | 53 | 87 | 87 |
| 165 | **V** | 15 | 0 | 7 | 13 | 33 | 7 | 0 | **I** | 17 | 6 | 12 | 6 | 6 | 59 |
| 166 | **G** | 16 | 63 | 0 | 6 | 6 | 69 | 0 | **S** | 16 | 38 | 38 | 19 | 6 | 75 |
| 167 | **Y** | 19 | 5 | 58 | 84 | 74 | 0 | 0 | **Y** | 19 | 32 | 0 | 0 | 11 | 47 |
| 170 | **K** | 18 | 11 | 100 | 83 | 50 | 11 | 11 | **R** | 14 | 93 | 21 | 29 | 21 | 21 |
| 171 | **Y** | 18 | 0 | 33 | 22 | 33 | 0 | 0 | **Y** | 19 | 63 | 32 | 0 | 16 | 11 |
| 172 | **P** | 17 | 0 | 88 | 71 | 76 | 24 | 76 | **A** | 16 | 56 | 56 | 25 | 94 | 81 |
| 173 | **S** | 19 | 0 | 68 | 37 | 74 | 5 | 26 | **N** | 19 | 32 | 37 | 16 | 63 | 79 |
| 174 | **V** | 19 | 0 | 32 | 21 | 42 | 0 | 16 | **A** | 18 | 17 | 17 | 6 | 17 | 22 |
| 175 | **I** | 19 | 0 | 42 | 26 | 37 | 5 | 11 | **M** | 16 | 50 | 38 | 0 | 50 | 38 |
| 176 | **A** | 19 | 5 | 11 | 5 | 21 | 11 | 5 | **A** | 16 | 19 | 13 | 0 | 13 | 38 |
| 177 | **V** | 19 | 0 | 5 | 11 | 26 | 0 | 0 | **V** | 17 | 18 | 12 | 0 | 24 | 24 |
| 180 | **V** | 19 | 11 | 32 | 16 | 53 | 0 | 16 | **T** | 15 | 47 | 7 | 0 | 20 | 20 |
| 181 | **D** | 19 | 5 | 5 | 5 | 32 | 0 | 16 | **D** | 12 | 0 | 33 | 0 | 8 | 8 |
| 182 | **S** | 19 | 32 | 53 | 79 | 42 | 16 | 53 | **Q** | 18 | 17 | 33 | 11 | 100 | 100 |
| 183 | **S** | 19 | 53 | 42 | 58 | 42 | 32 | 42 | **N** | 17 | 18 | 29 | 6 | 94 | 88 |
| 184 | **N** | 19 | 5 | 53 | 74 | 47 | 11 | 53 | **N** | 18 | 61 | 11 | 6 | 6 | 6 |
| 185 | **Q** | 19 | 11 | 47 | 63 | 53 | 16 | 84 | **N** | 17 | 41 | 24 | 24 | 94 | 88 |
| 186 | **R** | 19 | 0 | 89 | 95 | 79 | 5 | 0 | **R** | 17 | 53 | 12 | 24 | 12 | 6 |
| 187 | **A** | 19 | 11 | 68 | 74 | 26 | 0 | 11 | **A** | 19 | 5 | 16 | 0 | 5 | 37 |

| Table 7-2 Highly Productive Sites for BPN' and GG36 | | | | | | | | | | | | | | | |
|---|---|---|---|---|---|---|---|---|---|---|---|---|---|---|---|
| 188 | **S** | 19 | 47 | 32 | 84 | 26 | 11 | 32 | **S** | 16 | 6 | 0 | 19 | 100 | 100 |
| 189 | **F** | 17 | 29 | 24 | 12 | 29 | 0 | 0 | **F** | 16 | 6 | 0 | 0 | 0 | 88 |
| 190 | **S** | 19 | 0 | 21 | 26 | 16 | 0 | 0 | **S** | 19 | 0 | 0 | 0 | 0 | 84 |
| 191 | **Q** | 19 | 5 | 5 | 11 | 11 | 0 | 0 | **Q** | 16 | 6 | 0 | 13 | 0 | 75 |
| 192 | **Y** | 19 | 0 | 32 | 53 | 26 | 0 | 5 | **Y** | 17 | 6 | 12 | 6 | 0 | 53 |
| 193 | **G** | 19 | 0 | 21 | 11 | 0 | 0 | 0 | **G** | 15 | 0 | 0 | 0 | 0 | 87 |
| 194 | **P** | 19 | 0 | 74 | 47 | 68 | 0 | 68 | **A** | 17 | 18 | 35 | 35 | 94 | 94 |
| 195 | **E** | 19 | 0 | 32 | 11 | 21 | 0 | 0 | **G** | 16 | 56 | 44 | 13 | 13 | 19 |
| 196 | **L** | 19 | 0 | 21 | 21 | 26 | 5 | 5 | **L** | 14 | 21 | 14 | 0 | 7 | 43 |
| 197 | **D** | 15 | 0 | 27 | 40 | 47 | 0 | 0 | **D** | 18 | 83 | 50 | 0 | 44 | 39 |
| 198 | **V** | 18 | 0 | 6 | 33 | 33 | 11 | 17 | **I** | 17 | 41 | 12 | 0 | 59 | 59 |
| 199 | **M** | 18 | 0 | 50 | 28 | 33 | 0 | 6 | **V** | 17 | 47 | 29 | 12 | 29 | 29 |
| 200 | **A** | 19 | 0 | 42 | 16 | 32 | 0 | 16 | **A** | 11 | 36 | 27 | 0 | 18 | 18 |
| 201 | **P** | 18 | 0 | 44 | 44 | 78 | 0 | 22 | **P** | 16 | 31 | 13 | 0 | 0 | 0 |
| 203 | **V** | 18 | 0 | 33 | 78 | 67 | 17 | 50 | **V** | 16 | 75 | 19 | 13 | 19 | 25 |
| 204 | **S** | 16 | 0 | 13 | 38 | 0 | 31 | 44 | **N** | 14 | 14 | 29 | 7 | 93 | 93 |
| 205 | **I** | 19 | 0 | 16 | 11 | 32 | 5 | 11 | **V** | 15 | 33 | 13 | 13 | 7 | 13 |
| 206 | **Q** | 19 | 68 | 53 | 32 | 84 | 26 | 16 | **Q** | 18 | 67 | 28 | 17 | 94 | 44 |
| 208 | **T** | 19 | 0 | 37 | 37 | 37 | 0 | 21 | **T** | 18 | 39 | 22 | 0 | 22 | 11 |
| 209 | **L** | 19 | 5 | 32 | 21 | 37 | 0 | 32 | **Y** | 18 | 83 | 67 | 0 | 89 | 44 |
| 210 | **P** | 19 | 0 | 74 | 42 | 68 | 0 | 11 | **P** | 17 | 100 | 100 | 29 | 94 | 24 |
| 211 | **G** | 19 | 5 | 79 | 79 | 53 | 21 | 37 | **G** | 15 | 53 | 67 | 20 | 93 | 93 |
| 212 | **N** | 19 | 26 | 42 | 42 | 32 | 5 | 16 | **S** | 13 | 46 | 46 | 38 | 92 | 92 |
| 213 | **K** | 17 | 47 | 94 | 100 | 18 | 94 | 29 | **T** | 18 | 22 | 39 | 6 | 94 | 94 |
| 214 | **Y** | 18 | 0 | 72 | 50 | 44 | 0 | 0 | **Y** | 18 | 100 | 39 | 0 | 22 | 17 |
| 215 | **G** | 19 | 11 | 68 | 26 | 32 | 11 | 0 | **A** | 17 | 71 | 59 | 41 | 100 | 94 |
| 216 | **A** | 19 | 42 | 32 | 42 | 37 | 53 | 21 | **S** | 18 | 56 | 72 | 33 | 100 | 100 |
| 217 | **Y** | 19 | 32 | 47 | 26 | 58 | 53 | 58 | **L** | 16 | 50 | 81 | 25 | 6 | 19 |
| 218 | **N** | 19 | 5 | 26 | 11 | 16 | 16 | 32 | **N** | 17 | 76 | 76 | 24 | 88 | 35 |
| 220 | **T** | 19 | 0 | 5 | 5 | 5 | 0 | 0 | **T** | 15 | 7 | 7 | 0 | 0 | 33 |
| 222 | **M** | 18 | 67 | 0 | 0 | 17 | 28 | 6 | **M** | 17 | 6 | 12 | 12 | 0 | 94 |
| 224 | **S** | 19 | 11 | 37 | 26 | 42 | 5 | 5 | **T** | 17 | 29 | 18 | 12 | 18 | 35 |
| 225 | **P** | 19 | 16 | 0 | 0 | 0 | 0 | 0 | **P** | 18 | 0 | 6 | 11 | 0 | 33 |
| 226 | **H** | 19 | 0 | 58 | 53 | 63 | 0 | 16 | **H** | 16 | 75 | 44 | 0 | 6 | 6 |
| 227 | **V** | 19 | 5 | 32 | 47 | 26 | 5 | 0 | **V** | 16 | 50 | 25 | 0 | 44 | 44 |
| 228 | **A** | 16 | 0 | 25 | 25 | 38 | 6 | 0 | **A** | 17 | 18 | 6 | 12 | 29 | 24 |
| 229 | **G** | 19 | 5 | 0 | 5 | 5 | 0 | 5 | **G** | 15 | 20 | 13 | 0 | 13 | 7 |
| 230 | **A** | 19 | 11 | 21 | 47 | 58 | 11 | 21 | **A** | 16 | 81 | 50 | 6 | 69 | 44 |
| 231 | **A** | 19 | 0 | 47 | 58 | 58 | 5 | 5 | **A** | 16 | 19 | 25 | 6 | 50 | 50 |
| 232 | **A** | 13 | 0 | 38 | 54 | 23 | 38 | 15 | **A** | 10 | 60 | 30 | 20 | 30 | 20 |
| 233 | **L** | 11 | 0 | 18 | 36 | 18 | 27 | 0 | **L** | 16 | 94 | 81 | 6 | 75 | 31 |
| 234 | **I** | 19 | 32 | 37 | 32 | 26 | 21 | 32 | **V** | 12 | 58 | 33 | 8 | 75 | 75 |
| 235 | **L** | 19 | 47 | 32 | 37 | 47 | 68 | 32 | **K** | 16 | 88 | 63 | 56 | 88 | 88 |
| 236 | **S** | 19 | 42 | 32 | 21 | 32 | 26 | 42 | **Q** | 16 | 94 | 88 | 13 | 75 | 50 |
| 237 | **K** | 19 | 79 | 26 | 37 | 26 | 37 | 58 | **K** | 17 | 94 | 71 | 65 | 88 | 82 |
| 238 | **H** | 19 | 0 | 37 | 47 | 42 | 58 | 26 | **N** | 17 | 71 | 71 | 47 | 94 | 88 |

**Table 7-2 Highly Productive Sites for BPN' and GG36**

| | | | | | | | | | | | | | | | |
|---|---|---|---|---|---|---|---|---|---|---|---|---|---|---|---|
| 239 | **P** | 19 | 0 | 58 | 16 | 47 | 84 | 53 | **P** | 17 | 71 | 82 | 65 | 100 | 82 |
| 240 | **N** | 19 | 42 | 68 | 11 | 47 | 79 | 42 | **S** | 14 | 71 | 93 | 14 | 93 | 79 |
| 241 | **W** | 19 | 0 | 68 | 21 | 79 | 63 | 58 | **W** | 19 | 42 | 63 | 63 | 95 | 84 |
| 242 | **T** | 19 | 32 | 16 | 74 | 32 | 89 | 21 | **S** | 15 | 33 | 33 | 47 | 100 | 73 |
| 243 | **N** | 19 | 11 | 11 | 63 | 37 | 21 | 16 | **N** | 18 | 39 | 50 | 39 | 94 | 89 |
| 244 | **T** | 19 | 47 | 16 | 74 | 53 | 68 | 37 | **V** | 16 | 56 | 75 | 63 | 94 | 81 |
| 245 | **Q** | 19 | 16 | 11 | 79 | 26 | 84 | 32 | **Q** | 13 | 62 | 77 | 46 | 100 | 77 |
| 246 | **V** | 17 | 6 | 18 | 35 | 18 | 18 | 12 | **I** | 17 | 76 | 24 | 29 | 35 | 35 |
| 247 | **R** | 19 | 0 | 53 | 74 | 42 | 0 | 0 | **R** | 19 | 53 | 68 | 21 | 84 | 79 |
| 248 | **S** | 19 | 47 | 37 | 79 | 42 | 32 | 58 | **N** | 16 | 69 | 56 | 31 | 94 | 88 |
| 249 | **S** | 19 | 42 | 5 | 68 | 37 | 5 | 42 | **H** | 18 | 17 | 28 | 39 | 94 | 83 |
| 250 | **L** | 19 | 0 | 26 | 32 | 26 | 0 | 16 | **L** | 17 | 65 | 12 | 0 | 29 | 24 |
| 251 | **E** | 19 | 0 | 37 | 11 | 63 | 0 | 0 | **K** | 14 | 64 | 50 | 50 | 86 | 64 |
| 252 | **N** | 19 | 21 | 58 | 37 | 74 | 53 | 42 | **N** | 16 | 38 | 81 | 63 | 88 | 75 |
| 253 | **T** | 19 | 16 | 53 | 58 | 68 | 11 | 16 | **T** | 14 | 36 | 29 | 7 | 93 | 79 |
| 254 | **T** | 19 | 16 | 53 | 47 | 63 | 11 | 5 | **A** | 18 | 39 | 22 | 0 | 22 | 11 |
| 255 | **T** | 19 | 26 | 58 | 53 | 37 | 0 | 42 | **T** | 17 | 18 | 29 | 35 | 94 | 94 |
| 256 | **K** | 19 | 11 | 89 | 95 | 58 | 0 | 68 | **S** | 17 | 47 | 82 | 35 | 100 | 65 |
| 257 | **L** | 19 | 0 | 47 | 53 | 32 | 0 | 16 | **L** | 15 | 87 | 40 | 0 | 40 | 13 |
| 258 | **G** | 17 | 0 | 47 | 82 | 94 | 0 | 0 | **G** | 17 | 82 | 59 | 0 | 82 | 53 |
| 259 | **D** | 19 | 21 | 5 | 0 | 11 | 0 | 89 | **S** | 12 | 83 | 100 | 8 | 100 | 25 |
| 260 | **S** | 19 | 26 | 63 | 63 | 37 | 0 | 47 | **T** | 14 | 64 | 86 | 21 | 100 | 79 |
| 261 | **F** | 19 | 0 | 0 | 32 | 11 | 5 | 0 | **N** | 16 | 56 | 88 | 63 | 100 | 75 |
| 262 | **Y** | 19 | 11 | 26 | 26 | 37 | 0 | 0 | **L** | 17 | 71 | 71 | 35 | 94 | 88 |
| 263 | **Y** | 19 | 5 | 37 | 32 | 26 | 0 | 5 | **Y** | 15 | 93 | 33 | 0 | 13 | 7 |
| 264 | **G** | 19 | 0 | 5 | 5 | 16 | 0 | 0 | **G** | 14 | 79 | 7 | 0 | 7 | 0 |
| 265 | **K** | 19 | 21 | 84 | 84 | 58 | 47 | 11 | **S** | 18 | 83 | 83 | 6 | 72 | 44 |
| 267 | **L** | 18 | 0 | 61 | 83 | 89 | 11 | 56 | **L** | 17 | 76 | 41 | 0 | 41 | 6 |
| 268 | **I** | 19 | 0 | 26 | 37 | 37 | 0 | 5 | **V** | 15 | 53 | 13 | 0 | 53 | 47 |
| 269 | **N** | 19 | 5 | 32 | 26 | 63 | 11 | 5 | **N** | 13 | 85 | 85 | 8 | 100 | 69 |
| 270 | **V** | 18 | 17 | 11 | 50 | 33 | 17 | 6 | **A** | 17 | 88 | 71 | 0 | 65 | 35 |
| 271 | **Q** | 19 | 53 | 32 | 32 | 79 | 47 | 26 | **E** | 14 | 29 | 100 | 0 | 100 | 64 |
| 272 | **A** | 19 | 26 | 16 | 68 | 32 | 37 | 16 | **A** | 16 | 69 | 63 | 25 | 100 | 69 |
| 273 | **A** | 13 | 0 | 69 | 85 | 100 | 0 | 0 | **A** | 15 | 100 | 73 | 7 | 73 | 27 |
| 274 | **A** | 19 | 5 | 42 | 0 | 53 | 16 | 26 | **T** | 15 | 87 | 80 | 13 | 87 | 60 |
| 275 | **Q** | 19 | 26 | 47 | 53 | 47 | 58 | 26 | **R** | 14 | 86 | 50 | 79 | 71 | 50 |

Restrictive sites are those having less than 20% neutral mutations for activity and stability. In Table 7-3 below, the results for variants that meet the definition of a restrictive site are shown as a percentage (%) of variants evaluated that meet definition of a neutral mutation (PI > 0.5).

| Table 7-3 Restrictive Sites for BPN' and GG36 | | | | | | | | | | | | | |
|---|---|---|---|---|---|---|---|---|---|---|---|---|---|
| Position (BPN' #) | BPN' WT Residue | # BPN' Variants | BPN' BMI pH7/16°C | BPN' BMI pH8/16°C | BPN' BMI pH8/32° | BPN' LAS | BPN' AAPF | GG36 WT Residue | # GG36 Variants | GG36 BMI pH8/32°C | GG36 Egg | GG36 LAS/EDTA | GG36 AAPF |
| 23 | **G** | 18 | 11 | 17 | 11 | 11 | 6 | **G** | 13 | 15 | 15 | 15 | 15 |
| 32 | **D** | 16 | 0 | 0 | 0 | 0 | 0 | **D** | 17 | 12 | 18 | 0 | 0 |
| 64 | **H** | 13 | 0 | 0 | 0 | 0 | 0 | **H** | 14 | 0 | 0 | 7 | 0 |
| 65 | **G** | 17 | 6 | 12 | 12 | 6 | 6 | **G** | 10 | 0 | 0 | 0 | 0 |
| 70 | **G** | 16 | 0 | 0 | 0 | 0 | 0 | **G** | 12 | 0 | 0 | 0 | 0 |
| 83 | **G** | 19 | 5 | 5 | 5 | 5 | 5 | **G** | 10 | 10 | 10 | 0 | 10 |
| 125 | **S** | 19 | 16 | 5 | 5 | 5 | 0 | **S** | 15 | 13 | 7 | 13 | 0 |
| 168 | **P** | 19 | 16 | 11 | 0 | 0 | 0 | **P** | 18 | 6 | 6 | 0 | 0 |
| 202 | **G** | 19 | 0 | 0 | 11 | 0 | 0 | **G** | 18 | 6 | 0 | 0 | 0 |
| 221 | **S** | 16 | 0 | 0 | 0 | 0 | 0 | **S** | 16 | 0 | 0 | 0 | 0 |

In short as determined during development of the present invention, 10 positions in the mature region of two reference subtilisins are restrictive positions for activity and stability. Thus the remaining 265 positions in the mature region of two reference subtilisins are nonrestrictive positions (≥ 20% neutral mutations) for activity and stability

## EXAMPLE 8

### Protease Production in *B. subtilis*

In this Example, experiments conducted to produce various proteases in *B. subtilis* are described. In particular, the methods used in the transformation of *B. subtilis* with expression vectors for GG36, and BPN'-Y217L are provided. Transformation was performed as known in the art (*See e.g.,* WO 02/14490).

### GG36 Protease Production

In this Example, experiments conducted to produce GG36 (also referred to herein as *B. lentus* subtilisin) in *B. subtilis* are provided. The expression plasmid pAC-GG36ci was assembled using the GG36 codon-improved gene fused at the eighth codon of the aprE signal sequence under the control of the consensus aprE promoter and the BPN' transcriptional terminator. In the sequence provided below, bold and italicized font indicates the consensus aprE promoter, standard font indicates the signal sequence, underlined font indicates the pro sequence, bold font indicates DNA that encodes the GG36 mature protease, and underlined italicized font indicates the BPN' terminator. The coding region of the GG36 mature protease is flanked by KpnI and XhoI restriction sites for cloning purposes:

***atctcaaaaaaatgggtctactaaaatattactccatctattataataaattcacagaatagtcttttaagtaagtctactctgaatttttttaaaaggaga***
***gggtaaaga***gtgagaagcaaaaaattgtggatcgtcgcgtcgaccgcattgctgatttctgttgcttttagctcatccatcgcatccgctgctgaagaagc
aaaagaaaaatatttaattggctttaatgagcaggaagctgtcagtgagtttgtagaacaagttgaggcaaatgacgaggtagccattctctctgaggaag
aggaagtcgaaattgaattgcttcatgaatttgaaacgattcctgttctgtccgttgagttaagcccagaagatgtggacgcgttagagctcgatccagctat
ttcttatattgaagaggatgcagaagtaactacaatg**gcgcaatcggtaccatggggaattagcagagtacaagccccagctgcacataaccgtgg**
**attgacaggttctggtgtaaaagttgctgtccttgataccggtatttccactcatccagacttaaatattcgtggtggagctagctttgtaccaggg**
**gaaccatccactcaagatggcaatggacatggcactcatgttgccggcacaatcgcggctcttaacaattcaattggtgttcttggcgtagcgcc**
**aagcgcagaactatacgctgttaaagtattaggagcaagcggttcaggctctgtcagctctattgcccaaggattggaatggcagggaacaa**
**tggcatgcacgttgctaatcttagtttaggatctccttcgccaagtgccacacttgagcaagctgttaatagcgcgacttctagaggcgttcttgtt**
**gtagcggcctctggaaattcaggtgcaggctcaatcagctatccggcccgttatgcgaacgctatggcagtcggagctactgaccaaaacaac**
**aaccgcgccagcttttcacagtatggcgcagggcttgacattgtcgcaccaggtgtaaacgtgcagagcacttacccaggttcaacatatgcca**
**gcttaaacggtacatcaatggctactcctcatgttgcaggtgcggctgcacttgttaaacaaaagaacccatcttggtccaatgtacaaatccgc**
**aatcatcttaagaatacggcaactagcttaggaagcacaaacttgtatggaagcggacttgtcaatgcagaagctgcaactcgttaa***aagctta*
*actcgagataaaaaaccggccttggccccgccggtttttat* (SEQ ID NO:560)

The amino acid sequence of the GG36 precursor protein is provided below. In this sequence, bold indicates the mature GG36 protease:

MRSKKLWIVASTALLISVAFSSSIASAAEEAKEKYLIGFNEQEAVSEFVEQVEANDEVAILSEEEE
VEIELLHEFETIPVLSVELSPEDVDALELDPAISYIEEDAEVTTM**AQSVPWGISRVQAPAAHNRG**
**LTGSGVKVAVLDTGISTHPDLNIRGGASFVPGEPSTQDGNGHGTHVAGTIAALNNSIGVLG**
**VAPSAELYAVKVLGASGSGSVSSIAQGLEWAGNNGMHVANLSLGSPSPSATLEQAVNSATS**
**RGVLVVAASGNSGAGSISYPARYANAMAVGATDQNNNRASFSQYGAGLDIVAPGVNVQST**
**YPGSTYASLNGTSMATPHVAGAAALVKQKNPSWSNVQIRNHLKNTATSLGSTNLYGSGLV**
**NAEAATR** (SEQ ID NO:561).

The amino acid sequence of the mature GG36 protease (SEQ ID NO:562) was used as the basis for making the variant libraries described herein:

**AQSVPWGISRVQAPAAHNRGLTGSGVKVAVLDTGISTHPDLNIRGGASFVPGEPSTQDGNG**
**HGTHVAGTIAALNNSIGVLGVAPSAELYAVKVLGASGSGSVSSIAQGLEWAGNNGMHVAN**
**LSLGSPSPSATLEQAVNSATSRGVLVVAASGNSGAGSISYPARYANAMAVGATDQNNNRAS**
**FSQYGAGLDIVAPGVNVQSTYPGSTYASLNGTSMATPHVAGAAALVKQKNPSWSNVQIRN**
**HLKNTATSLGSTNLYGSGLVNAEAATR** (SEQ ID NO:562).

Elements of plasmid pAC-GG36ci include: pUB110 = DNA fragment from plasmid pUB110 (McKenzie et al., Plasmid 15:93-103 [1986]), pBR322 = DNA fragment from plasmid pBR322 (Bolivar et al., Gene 2:95-113 [1977]), pC194 = DNA fragment from plasmid pC194 (Horinouchi et al., J Bacteriol, 150:815-825 [1982]). The plasmid features as follows: Ori for *B. subtilis* = origin of replication from pUB110, CAT = chloramphenicol resistance gene from pC194, pMB1 origin = origin of replication from pBR322, bla = beta-lactamase from pBR322, Short aprE promoter = consensus transcriptional promoter, Signal Peptide= signal peptide, Pro Peptide = GG36 pro region, GG36ci Mature Peptide = mature GG36 (replaced by the coding regions for each variant expressed in this study), BPN' Terminator = transcriptional terminator from subtilisin BPN'.

**BPN'-Y217L (PURAFECT® PRIME Subtilisin) Protease Production**

In this Example, experiments conducted to produce *B. amyloliquefaciens* subtilisin BPN'-Y217L (commercially available as PURAFECT® PRIME subtilisin; also referred to as "FNA") in *B. subtilis* are described. The expression plasmid pAC-FNAre was assembled using the BPN'-Y217L gene, fused at the eighth codon of the aprE signal sequence under the control of the consensus aprE promoter and BPN' transcriptional terminator. In the sequence provided below, bold and italicized font indicates the consensus aprE promoter, standard font indicates the signal sequence, underlined font indicates the pro sequence, bold font indicates DNA that encodes the BPN'-Y217L mature protease, and underlined italicized font indicates the BPN' terminator. The coding region of the BPN'-Y217L mature protease contains the *Kpn*I and *Xho*I restriction sites for cloning purposes:

***gaattcatctcaaaaaaatgggtctactaaaatattattccatctattataataaattcacagaatagtcttttaagtaagtctactctgaatttttttaaaa***
***ggagagggtaaagagtgagaagcaaaaaatt***gtgagaagcaaaaaattgtggatcagtttgctgtttgctttagcgttaatctttacgatggcgttcggc
agcacatccagcgcgcaggctgcagggaaatcaaacggggaaaagaaatatattgtcgggtttaaacagacaatgagcacgatgagcgccgctaaga
agaaagacgtcatttctgaaaaaggcgggaaagtgcaaaagcaattcaaatatgtagacgcagctagcgctacattaaacgaaaaagctgtaaaagaat
tgaaaaaagacccgagcgtcgcttacgttgaagaagatcacgtagcacacgcgtac**gcgcagtccgtgccatatggcgtatcacaaattaaagccc**
**ctgctctgcactctcaaggctacaccggttcaaatgttaaagtagcggttatcgacagcggtatcgattcttctcatccagatcttaaagtagcag**
**gcggagccagcatggttccttctgaaacaaatcctttccaagacaacaactctcacggaacacacgttgctggtaccgttgcggctcttaataac**
**tcaatcggtgtattaggcgttgcgccaagcgcatcactttacgctgtaaaagttctcggcgccgacggttccggccaatacagctggatcattaa**
**cggaatcgagtgggcgatcgcaaacaatatggacgttattaacatgagcctcggcggaccgtccggttctgctgctttaaaagcggcagttgat**
**aaagccgttgcatccggcgtcgtagtcgttgcggcagccggcaacgaaggcacttccggcagctcaagcacagtgggctaccctggtaaatac**
**ccttctgtcattgcagtaggcgctgtcgacagcagcaaccaaagagcatctttctcaagcgtaggacctgagctcgatgtcatggcacctggcgt**
**atctatccaaagcacgcttcctggaaacaaatacggcgcgttgaacggtacatcaatggcatctccgcacgttgccggagccgcggctttgatt**
**ctttctaagcacccgaactggacaaacactcaagtccgcagctctctagaaaacaccactacaaaacttggtgattctttctactatggaaaag**
**ggctgatcaatgtacaggcggcagctcagtaa***aactcgagataaaaaaccggccttggccccgccggtttttat* (SEQ ID NO:563).

The amino acid sequence of the BPN'-Y217L precursor protein is provided below. In this sequence, bold indicates the mature BPN'-Y217L protease:

MRSKKLWISLLFALALIFTMAFGSTSSAQAAGKSNGEKKYIVGFKQTMSTMSAAKKKDVISEKG
GKVQKQFKYVDAASATLNEKAVKELKKDPSVAYVEEDHVAHAY**AQSVPYGVSQIKAPALHS**
**QGYTGSNVKVAVIDSGIDSSHPDLKVAGGASMVPSETNPFQDNNSHGTHVAGTVAALNNSI**
**GVLGVAPSASLYAVKVLGADGSGQYSWIINGIEWAIANNMDVINMSLGGPSGSAALKAAVD**
**KAVASGVVVVAAAGNEGTSGSSSTVGYPGKYPSVIAVGAVDSSNQRASFSSVGPELDVMAP**
**GVSIQSTLPGNKYGALNGTSMASPHVAGAAALILSKHPNWTNTQVRSSLENTTTKLGDSFY**
**YGKGLINVQAAAQ** (SEQ ID NO:564).

The amino acid sequence of the mature BPN'-Y217L protease was used as the basis for making the variant libraries described herein:

**AQSVPYGVSQIKAPALHSQGYTGSNVKVAVIDSGIDSSHPDLKVAGGASMVPSETNPFQDN**
**NSHGTHVAGTVAALNNSIGVLGVAPSASLYAVKVLGADGSGQYSWIINGIEWAIANNMDVI**

**NMSLGGPSGSAALKAAVDKAVASGVVVVAAAGNEGTSGSSSTVGYPGKYPSVIAVGAVDSS**
**NQRASFSSVGPELDVMAPGVSIQSTLPGNKYGALNGTSMASPHVAGAAALILSKHPNWTNT**
**QVRSSLENTTTKLGDSFYYGKGLINVQAAAQ** (SEQ ID NO:565).

Elements of plasmid pAC-FNAre include: pUB110 = DNA fragment from plasmid pUB110 (McKenzie et al., Plasmid 15:93-103, [1986]), pBR322 = DNA fragment from plasmid pBR322 (Bolivar et al., Gene 2:95-113 [1977]), pC194 = DNA fragment from plasmid pC194 (Horinouchi et al., J. Bacteriol 150:815-825 [1982]). Plasmid features are as follows: Ori for *B. subtilis* = origin of replication from pUB110, CAT = chloramphenicol resistance gene from pC194, pMB1 origin = origin of replication from pBR322, bla = beta-lactamase from pBR322, Short aprE promoter = consensus transcriptional promoter, Signal Peptide= signal peptide, Pro Peptide = BPN'-Y217L pro region, BPN'-Y217L Mature Peptide = mature BPN'-Y217L (replaced by the coding regions for each variant expressed in this study), BPN' Terminator = transcriptional terminator from subtilisin BPN'.

## EXAMPLE 9

### Expression of Enzyme Variants

This Example describes the methods used to express various recombinant enzymes of transformed *B. subtilis.*

### Subtilisins - 2 ml scale

*B. subtilis* clones containing BPN'-Y217L or GG36 expression vectors were replicated with a steel 96-well replicator from glycerol stocks into 96-well culture plates (BD, 353075) containing 200 μl of LB media + 25 μg/ml chloramphenicol, grown overnight at 37 °C, 220 rpm in a humidified enclosure. A 200 μl aliquot from the overnight culture was used to inoculate 2000 μl defined media + 25μg/ml chloramphenicol in 5ml plastic culture tubes. The cultivation media was an enriched semi-defined media based on MOPS buffer, with urea as major nitrogen source, glucose as the main carbon source, and supplemented with 1% soytone for robust cell growth. Culture tubes were incubated at 37°C, 220 rpm, for 60 hours. Following this incubation, the culture broths were centrifuged at greater than 8000 x RCF. The supernatant solution was decanted into 15ml polypropylene conical tubes for storage. No further purification or concentration was performed. Supernatant stocks were formulated to 40% propylene glycol final concentration for long-term stability and stored at 4°C.

## EXAMPLE 10

### Production of Enzyme Variants

This Example describes the production of enzyme charge ladders and combinatorial charge libraries.

### Enzyme Charge Ladders

Multiple protein variants spanning a range of physical properties of interest are selected from existing libraries or are generated by site-directed mutagenesis techniques as known in the art (*See e.g.,* US Pat. Appln. Ser. Nos., 10/576,331, 11/581,102, and 11/583,334). This defined set of probe proteins is then assayed in a test of interest.

Exemplary protease charge ladder variants are shown in the following tables and assayed as described herein. In these tables, the charge change is relative to the wild-type enzyme.

**Table 10-1. BPN'-Y217L Charge Ladder Variants**

| BPN'-Y217L Variant (BPN' numbering) | Charge Change |
|---|---|
| S87D-N109D-S188D-S248D | -4 |
| S87D-N109D-S 188D | -3 |
| S87D-N109D | -2 |
| N109D | -1 |
| (BPN'-Y217L) | 0 |
| N109R | +1 |
| S87R-N109R | +2 |
| S87R-N109R-S188R | +3 |
| S87R-N109R-S188R-S248R | +4 |

**Table 10-2. GG36 Charge Ladder Variants**

| GG36 Variant (GG36 numbering) | GG36 Variant (BPN' numbering) | Charge Change |
|---|---|---|
| S85D-Q107D-S182D-N242D | S87D-Q109D-S188D-N248D | -4 |
| S85D-Q107D-S182D | S87D-Q109D-S188D | -3 |
| S85D-Q107D | S87D-Q109D | -2 |
| Q107D | Q109D | -1 |
| (GG36) | (GG36) | 0 |
| Q107R | Q109R | +1 |
| S85R-Q107R | S87R-Q109R | +2 |
| S85R-Q107R-S182R | S87R-Q109R-S188R | +3 |

(continued)

| Table 10-2. GG36 Charge Ladder Variants | | |
|---|---|---|
| **GG36 Variant (GG36 numbering)** | **GG36 Variant (BPN' numbering)** | **Charge Change** |
| S85R-Q107R-S182R-N242R | S87R-Q109R-S188R-N248R | +4 |

**Enzyme Combinatorial Charge Libraries**

**Generation of *B. lentus* subtilisin (=GG36) Combinatorial Charge Libraries**

The pAC-GG36ci plasmid containing the codon-improved GG36 gene was sent to DNA 2.0, for the generation of combinatorial charge libraries (CCL). They were also provided with the *Bacillus subtilis* strain (genotype: Δ*aprE,* Δ*nprE,* Δ*spoIIE, amyE*::*xylRPxylAcomK-phleo*) for transformations. In addition a request was made to DNA2.0 Inc. for the generation of positional libraries at each of the four sites in GG36 protease that are shown in Table 10-3. Variants were supplied as glycerol stocks in 96-well plates.

The GG36 CCL was designed by identifying four well-distributed, surface-exposed, uncharged polar amino-acid residues outside the active site. These residues are Ser-85, Gln-107, Ser-182, and Asn-242 (residues 87, 109, 188, and 248 in BPN' numbering). An 81-member combinatorial library (G-1 to G-81) was created by making all combinations of three possibilities at each site: wild-type, arginine, or aspartic acid.

| Table 10-3. GG36 CCL Variants | | | | | |
|---|---|---|---|---|---|
| **Variant #** | **S 85** | **Q 107** | **S 182** | **N 242** | **Charge Change** |
| G-01 | - | - | - | - | 0 |
| G-02 | - | - | - | D | -1 |
| G-03 | - | - | - | R | +1 |
| G-04 | - | - | D | - | -1 |
| G-05 | - | - | D | D | -2 |
| G-06 | - | - | D | R | 0 |
| G-07 | - | - | R | - | +1 |
| G-08 | - | - | R | D | 0 |
| G-09 | - | - | R | R | +2 |
| G-10 | - | D | - | - | -1 |
| G-11 | - | D | - | D | -2 |
| G-12 | - | D | - | R | 0 |
| G-13 | - | D | D | - | -2 |
| G-14 | - | D | D | D | -3 |
| G-15 | - | D | D | R | -1 |
| G-16 | - | D | R | - | 0 |
| G-17 | - | D | R | D | -1 |
| G-18 | - | D | R | R | +1 |
| G-19 | - | R | - | - | +1 |
| G-20 | - | R | - | D | 0 |
| G-21 | - | R | - | R | +2 |
| G-22 | - | R | D | - | 0 |
| G-23 | - | R | D | D | -1 |

(continued)

| Table 10-3. GG36 CCL Variants | | | | | |
|---|---|---|---|---|---|
| Variant # | S 85 | Q 107 | S 182 | N 242 | Charge Change |
| G-24 | - | R | D | R | +1 |
| G-25 | - | R | R | - | +2 |
| G-26 | - | R | R | D | +1 |
| G-27 | - | R | R | R | +3 |
| G-28 | D | - | - | - | -1 |
| G-29 | D | - | - | D | -2 |
| G-30 | D | - | - | R | 0 |
| G-31 | D | - | D | - | -2 |
| G-32 | D | - | D | D | -3 |
| G-33 | D | - | D | R | -1 |
| G-34 | D | - | R | - | 0 |
| G-35 | D | - | R | D | -1 |
| G-36 | D | - | R | R | +1 |
| G-37 | D | D | - | - | -2 |
| G-38 | D | D | - | D | -3 |
| G-39 | D | D | - | R | -1 |
| G-40 | D | D | D | - | -3 |
| G-41 | D | D | D | D | -4 |
| G-42 | D | D | D | R | -2 |
| G-43 | D | D | R | - | -1 |
| G-44 | D | D | R | D | -2 |
| G-45 | D | D | R | R | 0 |
| G-46 | D | R | - | - | 0 |
| G-47 | D | R | - | D | -1 |
| G-48 | D | R | - | R | +1 |
| G-49 | D | R | D | - | -1 |
| G-50 | D | R | D | D | -2 |
| G-51 | D | R | D | R | 0 |
| G-52 | D | R | R | - | +1 |
| G-53 | D | R | R | D | 0 |
| G-54 | D | R | R | R | +2 |
| G-55 | R | - | - | - | +1 |
| G-56 | R | - | - | D | 0 |
| G-57 | R | - | - | R | +2 |
| G-58 | R | - | D | - | 0 |
| G-59 | R | - | D | D | -1 |
| G-60 | R | - | D | R | +1 |

(continued)

| Table 10-3. GG36 CCL Variants | | | | | |
|---|---|---|---|---|---|
| Variant # | S 85 | Q 107 | S 182 | N 242 | Charge Change |
| G-61 | R | - | R | - | +2 |
| G-62 | R | - | R | D | +1 |
| G-63 | R | - | R | R | +3 |
| G-64 | R | D | - | - | 0 |
| G-65 | R | D | - | D | -1 |
| G-66 | R | D | - | R | +1 |
| G-67 | R | D | D | - | -1 |
| G-68 | R | D | D | D | -2 |
| G-69 | R | D | D | R | 0 |
| G-70 | R | D | R | - | +1 |
| G-71 | R | D | R | D | 0 |
| G-72 | R | D | R | R | +2 |
| G-73 | R | R | - | - | +2 |
| G-74 | R | R | - | D | +1 |
| G-75 | R | R | - | R | +3 |
| G-76 | R | R | D | - | +1 |
| G-77 | R | R | D | D | 0 |
| G-78 | R | R | D | R | +2 |
| G-79 | R | R | R | - | +3 |
| G-80 | R | R | R | D | +2 |
| G-81 | R | R | R | R | +4 |

**Generation of *B. amyloliquefaciens* subtilisin BPN'-Y217L CCL**

The pAC-FNAre plasmid containing the BPN'-Y217L gene was sent to DNA 2.0 Inc. (Menlo Park, CA) for the generation of CCL. They were also provided with the *Bacillus subtilis* strain (genotype: Δ*aprE,* Δ*nprE,* Δ*spoIIE, amyE*::*xylRPxylAcomK-phleo*) for transformations. A request was made to DNA 2.0 Inc. for the generation of positional libraries at each of the four BPN'-Y217L protease sites that are shown in Table 10-4. Variants were supplied as glycerol stocks in 96-well plates.

The subtilisin BPN'-Y217L combinatorial charge library was designed by identifying four well-distributed, surface-exposed, uncharged polar amino-acid residues outside the active site. These residues are Ser-87, Asn-109, Ser-188, and Ser-248. An 81-member combinatorial library (F-1 to F-81) was created by making all combinations of three possibilities at each site: wild-type, arginine, or aspartic acid.

| Table 10-4. BPN'-Y217L CCL Variants | | | | | |
|---|---|---|---|---|---|
| Variant # | S 87 | N 109 | S 188 | S 248 | Charge Change |
| F-01 | - | - | - | - | 0 |
| F-02 | - | - | - | D | -1 |
| F-03 | - | - | - | R | +1 |
| F-04 | - | - | D | - | -1 |

(continued)

| Table 10-4. BPN'-Y217L CCL Variants | | | | | |
|---|---|---|---|---|---|
| Variant # | S 87 | N 109 | S 188 | S 248 | Charge Change |
| F-05 | - | - | D | D | -2 |
| F-06 | - | - | D | R | 0 |
| F-07 | - | - | R | - | +1 |
| F-08 | - | - | R | D | 0 |
| F-09 | - | - | R | R | +2 |
| F-10 | - | D | - | - | -1 |
| F-11 | - | D | - | D | -2 |
| F-12 | - | D | - | R | 0 |
| F-13 | - | D | D | - | -2 |
| F-14 | - | D | D | D | -3 |
| F-15 | - | D | D | R | -1 |
| F-16 | - | D | R | - | 0 |
| F-17 | - | D | R | D | -1 |
| F-18 | - | D | R | R | +1 |
| F-19 | - | R | - | - | +1 |
| F-20 | - | R | - | D | 0 |
| F-21 | - | R | - | R | +2 |
| F-22 | - | R | D | - | 0 |
| F-23 | - | R | D | D | -1 |
| F-24 | - | R | D | R | +1 |
| F-25 | - | R | R | - | +2 |
| F-26 | - | R | R | D | +1 |
| F-27 | - | R | R | R | +3 |
| F-28 | D | - | - | - | -1 |
| F-29 | D | - | - | D | -2 |
| F-30 | D | - | - | R | 0 |
| F-31 | D | - | D | - | -2 |
| F-32 | D | - | D | D | -3 |
| F-33 | D | - | D | R | -1 |
| F-34 | D | - | R | - | 0 |
| F-35 | D | - | R | D | -1 |
| F-36 | D | - | R | R | +1 |
| F-37 | D | D | - | - | -2 |
| F-38 | D | D | - | D | -3 |
| F-39 | D | D | - | R | -1 |
| F-40 | D | D | D | - | -3 |
| F-41 | D | D | D | D | -4 |

(continued)

| Table 10-4. BPN'-Y217L CCL Variants | | | | | |
|---|---|---|---|---|---|
| Variant # | S 87 | N 109 | S 188 | S 248 | Charge Change |
| F-42 | D | D | D | R | -2 |
| F-43 | D | D | R | - | -1 |
| F-44 | D | D | R | D | -2 |
| F-45 | D | D | R | R | 0 |
| F-46 | D | R | - | - | 0 |
| F-47 | D | R | - | D | -1 |
| F-48 | D | R | - | R | +1 |
| F-49 | D | R | D | - | -1 |
| F-50 | D | R | D | D | -2 |
| F-51 | D | R | D | R | 0 |
| F-52 | D | R | R | - | +1 |
| F-53 | D | R | R | D | 0 |
| F-54 | D | R | R | R | +2 |
| F-55 | R | - | - | - | +1 |
| F-56 | R | - | - | D | 0 |
| F-57 | R | - | - | R | +2 |
| F-58 | R | - | D | - | 0 |
| F-59 | R | - | D | D | -1 |
| F-60 | R | - | D | R | +1 |
| F-61 | R | - | R | - | +2 |
| F-62 | R | - | R | D | +1 |
| F-63 | R | - | R | R | +3 |
| F-64 | R | D | - | - | 0 |
| F-65 | R | D | - | D | -1 |
| F-66 | R | D | - | R | +1 |
| F-67 | R | D | D | - | -1 |
| F-68 | R | D | D | D | -2 |
| F-69 | R | D | D | R | 0 |
| F-70 | R | D | R | - | +1 |
| F-71 | R | D | R | D | 0 |
| F-72 | R | D | R | R | +2 |
| F-73 | R | R | - | - | +2 |
| F-74 | R | R | - | D | +1 |
| F-75 | R | R | - | R | +3 |
| F-76 | R | R | D | - | +1 |
| F-77 | R | R | D | D | 0 |
| F-78 | R | R | D | R | +2 |

(continued)

| Table 10-4. BPN'-Y217L CCL Variants | | | | | |
|---|---|---|---|---|---|
| Variant # | S 87 | N 109 | S 188 | S 248 | Charge Change |
| F-79 | R | R | R | - | +3 |
| F-80 | R | R | R | D | +2 |
| F-81 | R | R | R | R | +4 |

## EXAMPLE 11

### Variant BPN'-Y217L and GG36 Subtilisin Performance

This Example describes the testing of BPN'-Y217L and GG36 variants in BMI microswatch and baked egg assays in detergents representing various market geographies (e.g., differing pH, T, and/or water hardness), in both laundry and automatic dishwashing applications, as described in Example 1.

The above observations hold for other serine proteases such as the subtilisins BPN'-Y217L and GG36. For instance FIG. 2A and 2B shows an optimum charge for BPN'-Y217L and GG36 respectively, in cleaning performance under North American laundry conditions using off-the-shelf, heat-inactivated TIDE® 2X detergent. The left Y-axes shows microswatch cleaning performance, where a higher number indicates superior BMI stain removal. The right Y-axes shows the performance index defined as cleaning performance of variants (filled symbols) relative to the parent molecule (unfilled symbols). The horizontal lines indicate a performance index at either 2 or 3 standard deviations above the noise of the assay. The BPN'-Y217L charge combinatorial library (CCL) exhibits a charge optimum at zero charge changes with respect to the parent BPN'-Y217L while the GG36 CCL exhibits an optimum at negative two charges relative to the GG36 parent.

FIG. 3A, 3B, 4A, 4B, 5A and 5B demonstrate that the location of the charge optimum is a function of the solution environment determined by detergent formulation, pH, temperature and ionic strength due to water hardness and detergent concentration. For instance the charge optimum for BPN'-Y217L CCL shifts dramatically from zero under North American laundry conditions to more positive charges under Western European and Japanese conditions. Moreover the charge optimum is observed for both liquid and granular (powder) laundry detergent formulations. Similarly, a charge optimum was observed for both BPN'-Y217L and GG36 CCL in automatic dish washing (ADW) detergent against (e.g., Reckitt Benckiser Calgonit 40°C, 12 gpg, pH 10) baked egg as the enzyme substrate.

As demonstrated during development of the present invention, the cleaning performance of protease charge variants (e.g., GG36, BPN'-Y217L, etc) in different detergents is largely dominated by the working solution pH and conductivity. Final conductivity is a measure of ionic strength and is due to water hardness, detergent concentration and composition. For instance, there is a correlation between cleaning performance of GG36 and BPN'-Y217L variants against baked egg stains under European and North American ADW detergent when carried out at pH 10.6 and conductivity of 3.0 mS/cm. In particular, cleaning performance of charge variants is well correlated provided pH and conductivity are the same. This finding makes it possible to screen enzyme performance using a given detergent, for extrapolation of those results to another detergent of matching pH and conductivity. Likewise it is possible to screen enzyme performance in a buffer of matching pH and conductivity, for extrapolation of those results to a detergent exhibiting similar working pH and conductivity.

| Table 11-1 Variants | Net Charge | PI NA Laundry | PI WE Laundry | PI Dish baked egg |
|---|---|---|---|---|
| **GG36** | 0 | 1.000 | 1.000 | 1.000 |
| G-2 | -1 | 0.950 | 0.939 | 1.450 |
| G-3 | 1 | 0.578 | 0.759 | 1.231 |
| G-4 | -1 | 1.219 | 1.539 | 1.467 |
| G-5 | -2 | 1.261 | 1.194 | 1.508 |
| G-6 | 0 | 0.936 | 0.999 | 1.563 |

| Table 11-1 Variants | Net Charge | PI NA Laundry | PI WE Laundry | PI Dish baked egg |
|---|---|---|---|---|
| G-7 | 1 | 0.568 | 0.834 | 0.712 |
| G-8 | 0 | 0.043 | 0.151 | -0.033 |
| G-9 | 2 | 0.350 | 0.601 | 0.708 |
| G-10 | -1 | 1.266 | 1.089 | 1.022 |
| G-11 | -2 | 1.280 | 1.209 | 0.788 |
| G-12 | 0 | 0.810 | 1.074 | 0.977 |
| G-13 | -2 | 1.317 | 1.411 | 1.300 |
| G-14 | -3 | 0.080 | 0.144 | -0.007 |
| G-15 | -1 | 0.917 | 1.254 | 1.393 |
| G-16 | 0 | 0.750 | 1.081 | 0.742 |
| G-17 | -1 | 0.815 | 0.894 | 0.909 |
| G-18 | 1 | 0.675 | 0.931 | 0.867 |
| G-19 | 1 | 0.713 | 0.856 | 1.310 |
| G-20 | 0 | 0.071 | 0.129 | -0.015 |
| G-21 | 2 | 0.434 | 0.834 | 1.098 |
| G-22 | 0 | 0.782 | 1.014 | 1.447 |
| G-23 | -1 | 0.964 | 0.939 | 1.396 |
| G-24 | 1 | 0.466 | 0.729 | 1.368 |
| G-25 | 2 | 0.322 | 0.744 | 0.638 |
| G-26 | 1 | 0.517 | 0.984 | 0.694 |
| G-27 | 3 | 0.303 | 1.074 | 0.971 |
| G-28 | -1 | 1.126 | 1.141 | 1.023 |
| G-29 | -2 | 1.126 | 0.991 | 1.037 |
| G-30 | 0 | 0.945 | 1.149 | 1.006 |
| G-31 | -2 | 1.331 | 1.149 | 1.412 |
| G-32 | -3 | 1.345 | 0.999 | 1.303 |
| G-33 | -1 | 0.950 | 1.036 | 1.420 |
| G-34 | 0 | 0.671 | 0.999 | 0.673 |
| G-35 | -1 | 0.694 | 1.021 | 1.026 |
| G-36 | 1 | 0.415 | 0.774 | 0.704 |
| G-37 | -2 | 1.410 | 1.554 | -0.011 |
| G-38 | -3 | 0.457 | 0.759 | 1.081 |
| G-39 | -1 | 0.936 | 1.186 | 0.940 |
| G-40 | -3 | 0.043 | 0.106 | -0.006 |
| G-41 | -4 | 1.163 | 0.496 | 0.988 |
| G-42 | -2 | 1.359 | 1.276 | 1.165 |
| G-43 | -1 | 0.782 | 1.119 | 0.740 |
| G-44 | -2 | 0.926 | 1.051 | 0.748 |
| G-45 | 0 | 0.503 | 0.961 | 0.619 |
| G-46 | 0 | 0.759 | 0.916 | 1.035 |
| G-47 | -1 | 0.871 | 1.051 | 1.057 |
| G-48 | 1 | 0.452 | 0.864 | 1.060 |
| G-49 | -1 | 0.885 | 0.909 | 1.239 |
| G-50 | -2 | 0.912 | 0.909 | 1.613 |
| G-51 | 0 | 0.638 | 1.006 | 1.723 |
| G-52 | 1 | 0.396 | 0.909 | 0.820 |

| Table 11-1 Variants | Net Charge | PI NA Laundry | PI WE Laundry | PI Dish baked egg |
|---|---|---|---|---|
| G-53 | 0 | 0.568 | 0.909 | 0.806 |
| G-54 | 2 | 0.345 | 0.766 | 0.641 |
| G-55 | 1 | 0.689 | 1.036 | 1.230 |
| G-56 | 0 | 0.675 | 1.134 | 0.818 |
| G-57 | 2 | 0.452 | 0.766 | 0.922 |
| G-58 | 0 | 1.024 | 1.216 | 1.444 |
| G-59 | -1 | 1.131 | 1.306 | 1.473 |
| G-60 | 1 | 0.699 | 0.946 | 1.520 |
| G-61 | 2 | 0.457 | 0.886 | 0.680 |
| G-62 | 1 | 0.759 | 1.059 | 1.169 |
| G-63 | 3 | 0.327 | 0.669 | 0.687 |
| G-64 | 0 | 0.847 | 1.119 | 1.001 |
| G-65 | -1 | 0.601 | 0.879 | 1.014 |
| G-66 | 1 | 1.001 | 1.261 | 1.042 |
| G-67 | -1 | 1.196 | 1.411 | 1.489 |
| G-68 | -2 | 1.131 | 1.179 | 1.163 |
| G-69 | 0 | 0.768 | 0.999 | 1.488 |
| G-70 | 1 | 0.647 | 1.809 | 0.229 |
| G-71 | 0 | 0.620 | 1.081 | 0.631 |
| G-72 | 2 | 0.364 | 0.819 | 0.634 |
| G-73 | 2 | 0.387 | 0.729 | 0.997 |
| G-74 | 1 | 0.638 | 0.939 | 1.105 |
| G-75 | 3 | 0.657 | 0.856 | 1.081 |
| G-76 | 1 | 0.071 | 0.136 | -0.018 |
| G-77 | 0 | 0.866 | 0.969 | 1.400 |
| G-78 | 2 | 0.434 | 0.789 | 1.175 |
| G-79 | 3 | 0.327 | 0.789 | 0.874 |
| G-80 | 2 | 0.355 | 0.781 | 0.833 |
| G-81 | 4 | 0.229 | 0.466 | 0.653 |

| Table 11-2 Variants | Net Charge | PI NA Laundry | PI WE Laundry | PI JPN Laundry | PI dish baked egg |
|---|---|---|---|---|---|
| **BPN'-Y217L** | 0 | 1.000 | 1.000 | 1.000 | 1.000 |
| F-2 | -1 | 0.828 | 0.794 | 1.020 | 1.572 |
| F-3 | 1 | 0.866 | 1.687 | 1.712 | 1.322 |
| F-4 | -1 | 0.814 | 0.868 | 0.810 | 1.211 |
| F-5 | -2 | 0.753 | 0.988 | 0.458 | 1.395 |
| F-6 | 0 | 1.032 | 1.479 | 1.529 | 1.273 |
| F-7 | 1 | 0.805 | 1.792 | 1.359 | 0.969 |
| F-8 | 0 | 0.909 | 1.360 | 1.576 | 0.855 |
| F-9 | 2 | 0.705 | 1.360 | 1.529 | 0.777 |
| F-10 | -1 | 1.102 | 0.749 | 0.607 | 1.073 |
| F-11 | -2 | 0.904 | 0.600 | 0.410 | 1.004 |

| Table 11-2 Variants | Net Charge | PI NA Laundry | PI WE Laundry | PI JPN Laundry | PI dish baked egg |
|---|---|---|---|---|---|
| F-12 | 0 | 1.221 | 1.911 | 1.583 | 1.030 |
| F-13 | -2 | 0.838 | 0.690 | 0.234 | 0.832 |
| F-14 | -3 | 0.549 | 0.705 | 0.139 | 0.665 |
| F-15 | -1 | 1.093 | 1.107 | 0.654 | 1.063 |
| F-16 | 0 | 1.268 | 1.524 | 1.258 | 0.571 |
| F-17 | -1 | 0.819 | 0.764 | 0.722 | 0.710 |
| F-18 | 1 | 1.098 | 1.226 | 1.705 | 0.742 |
| F-19 | 1 | 1.003 | 1.196 | 1.590 | 1.298 |
| F-20 | 0 | 0.875 | 1.047 | 1.542 | 1.328 |
| F-21 | 2 | 0.866 | 1.568 | 1.976 | 1.327 |
| F-22 | 0 | 1.017 | 1.211 | 1.569 | 1.736 |
| F-23 | -1 | 0.771 | 1.241 | 0.973 | 1.335 |
| F-24 | 1 | 0.970 | 2.045 | 1.942 | 1.668 |
| F-25 | 2 | 0.738 | 1.315 | 1.664 | 1.015 |
| F-26 | 1 | 0.937 | 1.434 | 1.624 | 0.919 |
| F-27 | 3 | 0.535 | 1.122 | 1.990 | 1.283 |
| F-28 | -1 | 0.743 | 0.854 | 0.776 | 0.946 |
| F-29 | -2 | 0.691 | 0.749 | 0.451 | 0.707 |
| F-30 | 0 | 1.183 | 1.464 | 1.549 | 0.979 |
| F-31 | -2 | 0.738 | 0.720 | 0.519 | 1.185 |
| F-32 | -3 | 0.497 | 0.645 | 0.376 | 0.908 |
| F-33 | -1 | 1.107 | 1.062 | 1.332 | 0.989 |
| F-34 | 0 | 0.809 | 1.241 | 1.393 | 0.640 |
| F-35 | -1 | 0.672 | 0.824 | 0.844 | 0.928 |
| F-36 | 1 | 0.823 | 1.211 | 1.420 | 1.130 |
| F-37 | -2 | 0.880 | 0.720 | 0.227 | 0.678 |
| F-38 | -3 | 0.597 | 0.779 | 0.139 | 0.865 |
| F-39 | -1 | 0.890 | 1.017 | 0.661 | 0.760 |
| F-40 | -3 | 0.469 | 0.660 | 0.132 | 0.568 |
| F-41 | -4 | 0.322 | 0.541 | 0.064 | 0.464 |
| F-42 | -2 | 1.022 | 0.720 | 0.247 | 0.948 |
| F-43 | -1 | 0.852 | 1.092 | 0.980 | 0.605 |
| F-44 | -2 | 0.530 | 0.794 | 0.369 | 0.804 |
| F-45 | 0 | 0.980 | 1.419 | 1.122 | 0.730 |
| F-46 | 0 | 0.819 | 1.002 | 1.380 | 0.999 |
| F-47 | -1 | 0.984 | 0.928 | 1.102 | 1.172 |
| F-48 | 1 | 0.956 | 1.419 | 2.010 | 1.377 |
| F-49 | -1 | 0.913 | 0.898 | 0.925 | 1.263 |
| F-50 | -2 | 0.743 | 0.779 | 0.756 | 1.084 |
| F-51 | 0 | 1.008 | 1.300 | 1.481 | 1.588 |
| F-52 | 1 | 0.800 | 1.360 | 1.481 | 0.851 |
| F-53 | 0 | 0.705 | 1.077 | 1.271 | 0.792 |
| F-54 | 2 | 0.677 | 1.270 | 1.468 | 1.172 |
| F-55 | 1 | 1.050 | 1.538 | 1.515 | 0.996 |
| F-56 | 0 | 1.112 | 1.122 | 1.088 | 0.721 |
| F-57 | 2 | 0.866 | 1.330 | 2.173 | 1.014 |

| Table 11-2 Variants | Net Charge | PI NA Laundry | PI WE Laundry | PI JPN Laundry | PI dish baked egg |
|---|---|---|---|---|---|
| F-58 | 0 | 1.036 | 1.107 | 1.183 | 1.341 |
| F-59 | -1 | 1.117 | 1.077 | 0.898 | 0.878 |
| F-60 | 1 | 0.928 | 1.449 | 1.976 | 1.483 |
| F-61 | 2 | 0.942 | 1.687 | 1.542 | 0.785 |
| F-62 | 1 | 1.017 | 1.494 | 1.651 | 1.010 |
| F-63 | 3 | 0.630 | 1.613 | 1.522 | 0.910 |
| F-64 | 0 | 1.131 | 1.241 | 0.912 | 0.907 |
| F-65 | -1 | 0.994 | 0.958 | 1.136 | 0.582 |
| F-66 | 1 | 1.216 | 1.464 | 2.308 | 0.985 |
| F-67 | -1 | 1.126 | 0.854 | 0.485 | 0.855 |
| F-68 | -2 | 0.748 | 0.705 | 0.261 | 0.794 |
| F-69 | 0 | 1.249 | 1.479 | 1.041 | 0.925 |
| F-70 | 1 | 1.050 | 1.911 | 1.631 | 0.878 |
| F-71 | 0 | 1.136 | 1.687 | 1.298 | 0.998 |
| F-72 | 2 | 0.871 | 2.893 | 1.949 | 0.850 |
| F-73 | 2 | 0.998 | 1.717 | 1.990 | 1.276 |
| F-74 | 1 | 1.102 | 1.509 | 2.281 | 1.186 |
| F-75 | 3 | 0.672 | 1.687 | 2.044 | 1.351 |
| F-76 | 1 | 0.946 | 1.449 | 1.807 | 1.647 |
| F-77 | 0 | 1.050 | 1.509 | 1.793 | 1.287 |
| F-78 | 2 | 0.975 | 1.256 | 2.207 | 1.786 |
| F-79 | 3 | 0.606 | 1.285 | 1.508 | 0.954 |
| F-80 | 2 | 0.795 | 2.655 | 1.800 | 0.896 |
| F-81 | 4 | 0.611 | 1.419 | 1.759 | 0.965 |

## EXAMPLE 12

### LAS and Chelant Stability

This Example describes determining the relationship between protein charge and stability in a reaction medium containing one or both of an anionic surfactant and a chelant. For the determination of protease activity of the stressed and unstressed samples, the suc-AAPF-pNA assay was used. For determination of the alpha-amylase activity of the stressed and unstressed samples, the BODIPY-starch assay was used. Residual LAS and EDTA from the stress plates do not affect the suc-AAPF-pNA or BODIPY-starch assays.

### LAS / EDTA Stability

Reagents used included: control buffer: 50 mM HEPES, 0.005% Tween-80, pH 8.0; and stress buffer 50 mM HEPES, 0.1% (w/v) LAS (dodecylbenzene-sulfonate, sodium salt, Sigma D-2525), 10 mM EDTA, pH 8.0. Enzyme variants (20 ppm) were diluted 1:20 into 96-well non-binding flat-bottom plate containing either control or stress buffer and mixed. The control plate was incubated at room temperature while the stress plate was immediately placed at 37°C for 30-60 min (depending on the stability of the enzyme being tested). Following incubation, enzyme activity was measured using suc-AAPF-pNA assay for proteases. The fraction of remaining or residual activity was equal to the reaction rate of the stressed sample divided by the reaction rate of the control sample. The parent enzymes and variants were found to be stable for 60 min in the control buffer.

FIG. 6 depicts LAS/EDTA stability as a function of net charge change relative to parent BPN'-Y217L, for a library containing 80 variants. This library was designed and constructed according to the methods described in Example 2, to span several net charges relative to the parent BPN'-Y217L molecule. As evidenced from the Figure, accumulation of negative charges (up to -4) relative to parent BPN'-Y217L, are beneficial for combined LAS/chelant stability. This is an example of optimizing a protein physical property, in this case net charge, for improving protein stability in a complex liquid laundry environment.

**EXAMPLE 13**

**Stain Removal Performance of BPN' Multiple Mutation Library (MML) Variants**

BPN' multiple mutation libraries (or combinatorial libraries) were produced by Geneart or DNA 2.0, as described previously herein, using BPN' as the parent protein. The BPN' variant proteins were also produced as described earlier. Protein concentration of culture supernatants was determined by TCA precipitation as described in Example 1. The stain removal performance of the variants was tested in laundry applications on EMPA 116 swatches (BMI stain, CFT) at pH 8/16°C, pH 7/16°C and pH 8/32°C using methods described in Example 1, with the following modifications. The test detergent used was heat inactivated TIDE® 2X Cold detergent (Procter & Gamble). Heat inactivation of commercial detergent formulas serves to destroy the endogenous enzymatic activity of any protein components while retaining the properties of nonenzymatic components. Heat inactivation of the detergents was performed by placing pre-weighed amounts of liquid detergent (in a glass bottle) in a water bath at 95°C for 2 hours. The detergent was purchased from local supermarket stores. Both unheated and heated detergents were assayed within 5 minutes of dissolving the detergent, in order to accurately determine percentage deactivated. Enzyme activity was tested by AAPF assay. Functionality of BPN' variants was quantified as a performance index (*Pi*) (*i.e.*, the ratio of performance of a variant relative to parent BPN'). Results are shown in Table 13-1. BPN' variants showing a Pi value greater than or equal to 0.5 for one or more BMI stain removal performance tests and/or TCA precipitation showed improved cleaning benefits and/or expression. Performance indices less than or equal to 0.05 were fixed to 0.05 and indicated in bold italics in the table. For every variant with a TCA protein performance index less than or equal to 0.05, all values were fixed at 0.05. In this Table, "ND" indicates "not determined."

**Table 13-1. $P_i$ Values of BPN' Variants Tested for Expression (TCA) and Stain Removal Performance (BMI pH 8/16°C, BMI pH 7/16°C, and BMI pH 8/32°C)**

| Variant Code | TCA | BMI pH 8/16°C | BMI pH 7/16°C | BMI pH 8/32°C |
|---|---|---|---|---|
| Parent BPN' | 1.00 | 1.00 | 1.00 | 1.00 |
| BPN'Y217L | 1.01 | 1.12 | 1.08 | 1.08 |
| A92G | 0.52 | 1.04 | ND | ND |
| A92G-G100T | 0.58 | 0.37 | ND | ND |
| A92G-G128A | 0.49 | 0.83 | ND | ND |
| A92G-G97A | 0.77 | 1.14 | ND | ND |
| A92G-I111 V | 0.6 | 1.02 | ND | ND |
| A92G-L126A | 0.68 | 1.14 | ND | ND |
| A92G-L96T | 0.68 | 0.69 | ND | ND |
| A92G-M124V | 0.55 | 1.07 | ND | ND |
| A92G-N123G | 0.46 | 0.42 | ND | ND |
| A92G-V227T | 0.52 | 0.54 | ND | ND |
| A92G-W106F | 0.44 | 0.4 | ND | ND |
| A92G-Y104N | 0.65 | 0.41 | ND | ND |
| A92G-Y167A | 0.44 | 0.56 | ND | ND |
| A92G-Y217Q | 0.56 | 1.2 | ND | ND |
| BPN'-Y217L | 1.01 | 1.12 | 1.08 | 1.08 |
| G100T | 0.71 | 1.14 | ND | ND |
| G100T-G128A | 0.46 | 0.14 | ND | ND |
| G100T-I111V | 0.46 | 0.77 | ND | ND |
| G100T-L126A | 0.59 | 0.1 | ND | ND |

(continued)

| Table 13-1. $P_i$ Values of BPN' Variants Tested for Expression (TCA) and Stain Removal Performance (BMI pH 8/16°C, BMI pH 7/16°C, and BMI pH 8/32°C) | | | | |
|---|---|---|---|---|
| **Variant Code** | **TCA** | **BMI pH 8/16°C** | **BMI pH 7/16°C** | **BMI pH 8/32°C** |
| G100T-M124V | 0.57 | ***0.05*** | ND | ND |
| G100T-N123G | 0.56 | ***0.05*** | ND | ND |
| G100T-V227T | 0.37 | 0.42 | ND | ND |
| G100T-W106F | 0.62 | ***0.05*** | ND | ND |
| G100T-Y104N | 0.68 | ***0.05*** | ND | ND |
| G100T-Y167A | 0.63 | 0.28 | ND | ND |
| G100T-Y217Q | 0.7 | 1.11 | ND | ND |
| G128A | 1.5 | 1.17 | 1.11 | 1.14 |
| G128A-V227T | 0.48 | 0.86 | ND | ND |
| G128A-Y167A | 0.57 | 0.78 | ND | ND |
| G128A-Y217Q | 1.19 | 1.38 | 1.12 | 1.16 |
| G97A | 1.37 | 1.12 | 1.03 | 1.1 |
| G97A-G100T | 0.6 | 0.99 | ND | ND |
| G97A-G128A | 1.17 | 1.19 | 1.06 | 1.02 |
| G97A-I111 V | 1 | 1.22 | ND | ND |
| G97A-L126A | 1.36 | 1.24 | 1.1 | 1.11 |
| G97A-M124V | 1.33 | 1.29 | 1.12 | 1.12 |
| G97A-N123G | 0.91 | 1.22 | 1.19 | 1.12 |
| G97A-V227T | 0.71 | 1.07 | ND | ND |
| G97A-W106F | 0.69 | 1.1 | ND | ND |
| G97A-Y104N | 0.69 | 1.1 | ND | ND |
| G97A-Y167A | 0.66 | 1.02 | ND | ND |
| G97A-Y217Q | 1.42 | 1.27 | 1.08 | 1.14 |
| I111V | 1.07 | 1.04 | ND | ND |
| I111V-G128A | 1.13 | 1.1 | ND | ND |
| I111 V-L126A | 1.09 | 1.07 | ND | ND |
| I111V-M124V | 0.93 | 1.3 | ND | ND |
| I111V-N123G | 0.67 | 1.09 | ND | ND |
| I111V-V227T | 0.56 | 1.01 | ND | ND |
| I111V-Y167A | 0.66 | 0.97 | ND | ND |
| I111V-Y217Q | 1.06 | 1.28 | ND | ND |
| L126A | 1.56 | 1.16 | 1.1 | 1.08 |
| L126A-G128A | 0.76 | 0.83 | 0.9 | 0.92 |
| L126A-V227T | 0.73 | 0.8 | ND | ND |
| L126A-Y167A | 0.73 | 0.76 | ND | ND |

(continued)

| Table 13-1. $P_i$ Values of BPN' Variants Tested for Expression (TCA) and Stain Removal Performance (BMI pH 8/16°C, BMI pH 7/16°C, and BMI pH 8/32°C) | | | | |
|---|---|---|---|---|
| **Variant Code** | **TCA** | **BMI pH 8/16°C** | **BMI pH 7/16°C** | **BMI pH 8/32°C** |
| L126A-Y217Q | 1.7 | 1.28 | 1.11 | 1.11 |
| L96T | 0.84 | 1.17 | 1.04 | 1.13 |
| L96T-G100T | 0.53 | 0.14 | ND | ND |
| L96T-G128A | 0.59 | 0.77 | 0.77 | 0.84 |
| L96T-G97A | 0.81 | 1.24 | 1.11 | 1.14 |
| L96T-I111V | 0.49 | 0.94 | ND | ND |
| L96T-L126A | 0.76 | 0.43 | 0.37 | 0.6 |
| L96T-M124V | 0.87 | 1.12 | 0.93 | 1.01 |
| L96T-N123G | 0.58 | 0.3 | 0.22 | 0.56 |
| L96T-V227T | 0.5 | 0.3 | ND | ND |
| L96T-W106F | 0.57 | 0.41 | ND | ND |
| L96T-Y104N | 0.43 | ***0.05*** | ND | ND |
| L96T-Y167A | 0.36 | 0.63 | ND | ND |
| L96T-Y217Q | 0.79 | 1.28 | 1 | 1.12 |
| M124V | 1.61 | 1.22 | 1.12 | 1.15 |
| M124V-G128A | 0.96 | 1.17 | 1.11 | 1.12 |
| M124V-L126A | 1.74 | 1.13 | 1.12 | 1.11 |
| M124V-V227T | 0.65 | 0.96 | ND | ND |
| M124V-Y167A | 0.56 | 1.17 | ND | ND |
| M124V-Y217Q | 1.47 | 1.37 | 1.09 | 1.19 |
| N123G | 1 | 1.16 | 1.12 | 1.03 |
| N123G-G128A | 0.5 | 0.75 | 0.88 | 0.85 |
| N123G-L126A | 0.72 | 0.62 | 0.48 | 0.65 |
| N123G-M124V | 0.59 | 0.6 | 0.54 | 0.66 |
| N123G-V227T | 0.65 | ***0.05*** | ND | ND |
| N123G-Y167A | 0.59 | 0.34 | ND | ND |
| N123G-Y217Q | 0.82 | 1.25 | 1.18 | 1.13 |
| N62Q | 1.63 | 1.29 | 1.19 | 1.06 |
| N62Q-A92G | 0.59 | 1.17 | ND | ND |
| N62Q-G100T | 0.57 | 0.96 | ND | ND |
| N62Q-G128A | 1.09 | 1.21 | 1.15 | 1.12 |
| N62Q-G97A | 1.52 | 1.32 | 1.22 | 1.17 |
| N62Q-I111V | 1.4 | 1.25 | ND | ND |
| N62Q-L126A | 1.46 | 1.07 | 0.99 | 1.03 |
| N62Q-L96T | 0.73 | 1.02 | 0.97 | 0.99 |

(continued)

| Table 13-1. $P_i$ Values of BPN' Variants Tested for Expression (TCA) and Stain Removal Performance (BMI pH 8/16°C, BMI pH 7/16°C, and BMI pH 8/32°C) | | | | |
|---|---|---|---|---|
| **Variant Code** | **TCA** | **BMI pH 8/16°C** | **BMI pH 7/16°C** | **BMI pH 8/32°C** |
| N62Q-M124V | 1.29 | 1.23 | 1.06 | 1.15 |
| N62Q-N123G | 0.57 | 1.06 | 0.97 | 1 |
| N62Q-S89Y | 0.6 | 1.17 | ND | ND |
| N62Q-V227T | 0.57 | 1.06 | ND | ND |
| N62Q-V68A | 1.86 | 1.08 | ND | ND |
| N62Q-Y104N | 0.59 | 0.66 | ND | ND |
| N62Q-Y167A | 0.79 | 1.15 | ND | ND |
| N62Q-Y217Q | 1.35 | 1.28 | 1.13 | 1.21 |
| P52L | 0.5 | 0.59 | ND | ND |
| P52L-A92G | 0.69 | 0.2 | ND | ND |
| P52L-G100T | 0.59 | 0.39 | ND | ND |
| P52L-G128A | 0.55 | 0.51 | ND | ND |
| P52L-G97A | 0.54 | 0.78 | ND | ND |
| P52L-I111V | 0.43 | 0.68 | ND | ND |
| P52L-L126A | 0.69 | 0.6 | ND | ND |
| P52L-L96T | 0.67 | 0.34 | ND | ND |
| P52L-M124V | 0.56 | 0.72 | ND | ND |
| P52L-N123G | 0.48 | 0.34 | ND | ND |
| P52L-N62Q | 0.54 | 0.77 | ND | ND |
| P52L-S89Y | 0.51 | 0.42 | ND | ND |
| P52L-V227T | 0.38 | 0.27 | ND | ND |
| P52L-V68A | 0.6 | 1.2 | ND | ND |
| P52L-W106F | 0.43 | 0.52 | ND | ND |
| P52L-Y104N | 0.64 | 0.32 | ND | ND |
| P52L-Y167A | 0.38 | 0.4 | ND | ND |
| P52L-Y217Q | 0.34 | 0.77 | ND | ND |
| S89Y | 0.94 | 1.07 | ND | ND |
| S89Y-A92G | 0.5 | 0.9 | ND | ND |
| S89Y-G100T | 0.65 | 0.67 | ND | ND |
| S89Y-G128A | 0.98 | 1.1 | ND | ND |
| S89Y-G97A | 1.26 | 1.06 | ND | ND |
| S89Y-I111V | 0.83 | 1.07 | ND | ND |
| S89Y-L126A | 0.93 | 1.17 | ND | ND |
| S89Y-L96T | 0.77 | 0.84 | ND | ND |
| S89Y-M124V | 1.1 | 1.31 | ND | ND |

(continued)

Table 13-1. $P_i$ Values of BPN' Variants Tested for Expression (TCA) and Stain Removal Performance (BMI pH 8/16°C, BMI pH 7/16°C, and BMI pH 8/32°C)

| Variant Code | TCA | BMI pH 8/16°C | BMI pH 7/16°C | BMI pH 8/32°C |
|---|---|---|---|---|
| S89Y-N123G | 0.47 | 0.85 | ND | ND |
| S89Y-V227T | 0.66 | 0.87 | ND | ND |
| S89Y-W106F | 0.7 | 0.98 | ND | ND |
| S89Y-Y104N | 0.74 | 0.64 | ND | ND |
| S89Y-Y167A | 0.55 | 0.91 | ND | ND |
| S89Y-Y217Q | 1.32 | 1.17 | ND | ND |
| V227T | 0.65 | 1.09 | ND | ND |
| V68A | 2.02 | 1.33 | ND | ND |
| V68A-A92G | 0.81 | 1.42 | ND | ND |
| V68A-G100T | 0.87 | 0.31 | ND | ND |
| V68A-G128A | 1.16 | 1.28 | ND | ND |
| V68A-G97A | 2.12 | 1.33 | ND | ND |
| V68A-I111V | 2.06 | 1.31 | ND | ND |
| V68A-L126A | 1.66 | 0.58 | ND | ND |
| V68A-L96T | 0.68 | 0.86 | ND | ND |
| V68A-M124V | 1.25 | 0.96 | ND | ND |
| V68A-N123G | 0.92 | 0.69 | ND | ND |
| V68A-S89Y | 1.65 | 1.37 | ND | ND |
| V68A-V227T | 1 | 1.35 | ND | ND |
| V68A-W106F | 0.7 | 1.29 | ND | ND |
| V68A-Y104N | 0.71 | 0.78 | ND | ND |
| V68A-Y167A | 0.87 | 1.19 | ND | ND |
| V68A-Y217Q | 2.34 | 1.32 | ND | ND |
| W106F | 0.78 | 1.17 | ND | ND |
| W106F-G128A | 0.68 | 0.86 | ND | ND |
| W106F-I111V | 0.61 | 1.06 | ND | ND |
| W106F-L126A | 0.67 | 0.82 | ND | ND |
| W106F-M124V | 0.56 | 0.96 | ND | ND |
| W106F-N123G | 0.47 | 0.29 | ND | ND |
| W106F-V227T | 0.45 | 0.76 | ND | ND |
| W106F-Y167A | 0.75 | 0.66 | ND | ND |
| W106F-Y217Q | 0.87 | 1.35 | ND | ND |
| Y104N | 0.88 | 1.03 | ND | ND |
| Y104N-G128A | 0.83 | 1.21 | ND | ND |
| Y104N-I111V | 0.6 | 0.94 | ND | ND |

(continued)

| Table 13-1. $P_i$ Values of BPN' Variants Tested for Expression (TCA) and Stain Removal Performance (BMI pH 8/16°C, BMI pH 7/16°C, and BMI pH 8/32°C) | | | | |
|---|---|---|---|---|
| **Variant Code** | **TCA** | **BMI pH 8/16°C** | **BMI pH 7/16°C** | **BMI pH 8/32°C** |
| Y104N-L126A | 0.59 | 0.17 | ND | ND |
| Y104N-M124V | 0.51 | 0.43 | ND | ND |
| Y104N-N123G | 0.58 | 0.21 | ND | ND |
| Y104N-V227T | 0.4 | 0.32 | ND | ND |
| Y104N-W106F | 0.52 | 0.46 | ND | ND |
| Y104N-Y167A | 0.74 | 0.51 | ND | ND |
| Y104N-Y217Q | 0.68 | 1.27 | ND | ND |
| Y167A | 0.77 | 0.89 | ND | ND |
| Y167A-V227T | 0.45 | 0.43 | ND | ND |
| Y167A-Y217Q | 0.64 | 1.21 | ND | ND |
| Y217Q | 1.37 | 1.26 | ND | ND |
| Y217Q-V227T | 0.87 | 1.08 | ND | ND |

**EXAMPLE 14**

**Stain Removal Performance of BPN' Two Site Variants**

Using BPN' as the parent protein, two site variants were generated at positions 217 and 222 (BPN' numbering) using fusion PCR. The BPN' variant proteins were produced as described before. Protein concentration of culture supernatants was determined by TCA precipitation as described in Example 1. The stain removal performance of the variants was tested in laundry applications on EMPA 116 swatches (BMI stain, CFT) at pH 8/16°C in heat-inactivated TIDE® 2X Cold (Procter & Gamble) using methods described in Example 1. Functionality of BPN' variants was quantified as a performance index (*Pi*) (*i.e.*, the ratio of performance of a variant relative to BPN'-Y217L. Results are shown in Table 14-1. BPN' variants showing a Pi value greater than or equal to 0.5 for the BMI stain removal performance tests and/or TCA precipitation showed improved cleaning benefits and/or expression.

| Table 14-1: Stain Removal Performance and Expression of BPN' Two Site Variants | | |
|---|---|---|
| Variant | BMI | TCA |
| Y217L-M222Q | 0.88 | 1.27 |
| Y217Q-M222Q | 1.08 | 1.18 |
| Y217V-M222Q | 1.02 | 0.76 |

**EXAMPLE 15**

**ASP Protease Production in *B. subtilis***

Experiments conducted to produce 69B4 protease (also referred to herein as "ASP," "Asp," and "ASP protease," and "Asp protease") in *B. subtilis* are described in U.S. Pat. Appln. Ser. No. 10/576,331, incorporated herein by reference in its entirety. Briefly, the DNA sequence (synthetic ASP DNA sequence) provided below, with codon usage adapted for *Bacillus* species, encodes the wild type ASP precursor protein:

ATGACACCACGAAACTGTCACAAGAGCTCTGGCTGTGGCAACAGCAGCTGCTACACTCTTGG
CTGGGGGTATGGCAGCACAAGCTAACGAACCGGCTCCTCCAGGATCTGCATCAGCCCCTCC
ACGATTAGCTGAAAAACTTGACCCTGACTTACTTGAAGCAATGGAACGCGATCTGGGGTTA
GATGCAGAGGAAGCAGCTGCAACGTTAGCTTTTCAGCATGACGCAGCTGAAACGGGAGAGG
CTCTTGCTGAGGAACTCGACGAAGATTTCGCGGGCACGTGGGTTGAAGATGATGTGCTGTAT
GTTGCAACCACTGATGAAGATGCTGTTGAAGAAGTCGAAGGCGAAGGAGCAACTGCTGTGA
CTGTTGAGCATTCTCTTGCTGATTTAGAGGCGTGGAAGACGGTTTTGGATGCTGCGCTGGAG
GGTCATGATGATGTGCCTACGTGGTACGTCGACGTGCCTACGAATTCGGTAGTCGTTGCTGT
AAAGGCAGGAGCGCAGGATGTAGCTGCAGGACTTGTGGAAGGCGCTGATGTGCCATCAGAT
GCGGTCACTTTTGTAGAAACGGACGAAACGCCTAGAACGATG**TTCGACGTAATTGGAGGC**
**AACGCATATACTATTGGCGGCCGGTCTAGATGTTCTATCGGATTCGCAGTAAACGGTG**
**GCTTCATTACTGCCGGTCACTGCGGAAGAACAGGAGCCACTACTGCCAATCCGACTGG**
**CACATTTGCAGGTAGCTCGTTTCCGGGAAATGATTATGCATTCGTCCGAACAGGGGCA**
**GGAGTAAATTTGCTTGCCCAAGTCAATAACTACTCGGGCGGCAGAGTCCAAGTAGCAG**
**GACATACGGCCGCACCAGTTGGATCTGCTGTATGCCGCTCAGGTAGCACTACAGGTTG**
**GCATTGCGGAACTATCACGGCGCTGAATTCGTCTGTCACGTATCCAGAGGGAACAGTC**
**CGAGGACTTATCCGCACGACGGTTTGTGCCGAACCAGGTGATAGCGGAGGTAGCCTTT**
**TAGCGGGAAATCAAGCCCAAGGTGTCACGTCAGGTGGTTCTGGAAATTGTCGGACGG**
**GGGGAACAACATTCTTTCAACCAGTCAACCCGATTTTGCAGGCTTACGGCCTGAGAAT**
**GATTACGACTGACTCTGGAAGTTCCCCT**GCTCCAGCACCTACATCATGTACAGGCTACGC
AAGAACGTTCACAGGAACCCTCGCAGCAGGAAGAGCAGCAGCTCAACCGAACGGTAGCTAT
GTTCAGGTCAACCGGAGCGGTACACATTCCGTCTGTCTCAATGGACCTAGCGGTGCGGACTT
TGATTTGTATGTGCAGCGATGGAATGGCAGTAGCTGGGTAACCGTCGCTCAATCGACATCGC
CGGGAAGCAATGAAACCATTACGTACCGCGGAAATGCTGGATATTATCGCTACGTGGTTAA
CGCTGCGTCAGGATCAGGAGCTTACACAATGGGACTCACCCTCCCCTGA (SEQ ID NO:566)

In the above sequence, bold indicates the DNA that encodes the mature protease, standard font indicates the leader sequence, and the underline indicates the N-terminal and C-terminal prosequences. The mature serine protease enzyme derived from *Cellulomonas strain 69B4* (DSM 983316035) is 189 amino acids long, with a catalytic triad consisting of His32, Asp56, and Ser137, as shown below (with the catalytic triad indicated in bold and underline):

FDVIGGNAYT IGGRSRCSIG FAVNGGFITA G**H**CGRTGATT ANPTGTFAGS
SFPGN**D**YAFV RTGAGVNLLA QVNNYSGGRV QVAGHTAAPV GSAVCRSGST
TGWHCGTITA LNSSVTYPEG TVRGLIRTTV CAEPGD**S**GGS LLAGNQAQGV
TSGGSGNCRT GGTTFFQPVN PILQAYGLRM ITTDSGSSP (SEQ ID NO:567)

## EXAMPLE 16

### Production of ASP Combinatorial Mutants and Multiple Mutation Libraries

In this Example, methods used to construct combinatorial mutants and multiple mutation libraries of ASP are described. Construction of combinatorial mutants of ASP is described in U.S. Patent Appln. Ser. No. 10/576,331.

### Multiple Mutation Library Construction

The multiple mutation library was constructed as outlined in the Stratagene QCMS kit, with the exception of the primer concentration used in the reactions. Specifically, 1 μL of the methylated, purified pUC18-ASP plasmid (about 70 ng) was mixed with 15μL of sterile distilled water, 1.5μL of dNTP, 2.5μL of 10x buffer, 1μL of the enzyme blend and 1.0μL mutant primer mix (for a total of 100 pmol of primers). The primer mix was prepared using 1.0μL of each of the eighteen mutant primers (100 pmol/μL); adding 50ng of each primer for the library as recommended by Stratagene, resulted in fewer mutations in a previous round of mutagenesis. Thus, the protocol was modified in the present round of mutagenesis to include a total of 100 pmol of primers in each reaction. The cycling conditions were 95°C for 1 min, followed by 30 cycles of 95°C for 1 min, 55°C for 1 min, and 65°C for 12 min, in an MJ Research PTC2-200 thermocycler using thin-walled 0.2mL PCR tubes. The reaction product was digested with 1μL of *Dpn*I from the QCMS kit by incubating at 37°C overnight. An additional 0.5μL of *Dpn*I was added, and the reaction was incubated for 1 hour.

Subsequently, the library DNA (mutagenized single stranded pUC18-ASP product) was electroporated into electrocompetent *E.coli* cells (Invitrogen®, Catalog No. C4040-52, One Shot® TOP10 Electrocomp™ *E. coli, dam*+) and growth of transformed cells was selected on agar plates containing 100 mg/L ampicillin resulting in the ASP multiple mutation library in *E.coli* cells. Colonies (tens of thousands) were harvested and the Qiagen spin miniprep DNA kit (Catalog No. 27106) was used for preparing the plasmid DNA by the steps outlined by the manufacturer. The miniprep DNA was eluted with 50μL of Qiagen buffer EB provided in the kit.

Miniprep DNA was digested using the *Pst*I and *Hind*III DNA restriction enzymes. The ASP library fragment mix (*Pst*I x *Hind*III) was gel purified and cloned in the 4154 basepair *Hind*III **x** *Pst*I pHPLT vector fragment by a ligase reaction using Invitrogen® T4 DNA Ligase (Catalog No. 15224-025) as recommended by the manufacturer for cloning cohesive ends). In another approach, synthetic ASP library fragments were produced by GeneArt. These ASP library fragments were also digested with *Pst*I and *Hind*III, purified and cloned in the 4154 basepair *Hind*III x *Pst*I pHPLT vector fragment by a ligase reaction.

To transform the ligation reaction mix directly into *Bacillus* cells, the library DNA (ASP library fragment mix cloned in pHPLT) was amplified using the TempliPhi kit (Amersham Catalog No. 25-6400). For this purpose, 1μL of the ligation reaction mix was mixed with 5μL of sample buffer from the TempliPhi kit and heated for 3 minutes at 95°C to denature the DNA. The reaction was placed on ice to cool for 2 minutes and then spun down briefly. Next, 5μL of reaction buffer and 0.2μL of phi29 polymerase from the TempliPhi kit were added, and the reactions were incubated at 30°C in an MJ Research PCR machine for 4 hours. The phi29 enzyme was heat inactivated in the reactions by incubation at 65°C for 10 min in the PCR machine.

For transformation of the libraries into *Bacillus,* 0.1 μL of the TempliPhi amplification reaction product was mixed with 500 μL of competent *B. subtilis* cells (Δ*aprE,* Δ*nprE, oppA,* Δ*spoIIE, degUHy32,* Δ*amyE*::(*xylR,pxylA-comK*) followed by vigorous shaking at 37°C for 1 hour and 100 and 500 μL was plated on HI-agar plates containing 20 ppm neomycin sulfate (Sigma, Catalog No. N-1876; contains 732 μg neomycin per mg) and 0.5% skim milk. Ninety-five clones from the library were picked for sequencing.

The mutagenesis worked well, in that only 14% of the clones were equal to the backbone (parent) sequence (ASP with R014I-A064K-T086K-T116E-R123F), and about 3% of clones had extra mutations. The remaining sequenced clones (72%) were all mutants and of these about 94% were unique mutants. The sequencing results for the library are provided below in Table 16-1.

| **Table 16-1. Variants of ASP with R014I-A064K-T086K-T116E-R123F** | | | | | | |
|---|---|---|---|---|---|---|
| G54D | | | | | | |
| N24A | | | | | | |
| N24Q | | | | | | |
| N24T | | | | | | |
| N67S | | | | | | |
| R127K | | | | | | |
| R159F | | | | | | |
| R159K | | | | | | |
| R159K | | | | | | |
| R159N | | | | | | |
| R159N | | | | | | |
| G78D | R159F | | | | | |
| N24Q | R35E | | | | | |
| N67S | R159E | | | | | |
| R127K | R159E | | | | | |
| R127K | R159K | | | | | |
| R127K | R159N | | | | | |
| R127Q | R159K | | | | | |
| R35D | R159E | | | | | |

(continued)

| Table 16-1. Variants of ASP with R014I-A064K-T086K-T116E-R123F | | | | | | |
|---|---|---|---|---|---|---|
| R35D | R159K | | | | | |
| R35E | R159K | | | | | |
| G54D | R127K | R159K | | | | |
| G78D | R127K | R159K | | | | |
| G78D | R127K | R159E | | | | |
| G78D | R127Q | R159K | | | | |
| N24A | N67A | R159K | | | | |
| N24A | N67S | R159K | | | | |
| N24E | R35D | G78D | | | | |
| N24T | N67S | R159E | | | | |
| N67L | G78D | R159K | | | | |
| R35D | G78D | R159K | | | | |
| N24A | R35E | G78D | R159N | | | |
| N24D | R35D | G78D | R159F | | | |
| N24E | G54D | G78D | R159K | | | |
| N24E | R35D | G78D | R127K | R159N | | |
| N24Q | G54D | G78D | R159N | | | |
| N24Q | N67L | G78D | R159E | | | |
| N24Q | R35D | R127K | R159K | | | |
| N24T | R35D | G78D | R159K | | | |
| N24T | R35D | G78D | R159K | | | |
| N67S | G78D | R127K | R159K | | | |
| R35D | G78D | R127K | R159E | | | |
| R35D | G78D | R127K | R159N | | | |
| R35D | G78D | R127Q | R159K | | | |
| R35E | G54D | N67A | R159F | | | |
| R35E | N67S | G78D | R127Q | | | |
| N24A | G54D | N67S | G78D | R159F | | |
| N24A | R35D | N67A | G78D | R159F | | |
| N24Q | R35D | N67L | G78D | R159K | | |
| N24Q | R35D | N67L | G78D | R159N | | |
| N24Q | R35D | N67S | R127K | R159E | | |
| N24Q | R35E | N67A | R127K | R159E | | |
| N24Q | R35E | N67A | G78D | R159E | | |
| N24T | N67A | G78D | R127Q | R159N | | |
| N24T | R35E | N67A | G78D | R127Q | | |
| R35E | G54D | N67S | G78D | R159K | | |
| N24A | G54D | N67S | G78D | R127K | R159K | |

(continued)

| Table 16-1. Variants of ASP with R014I-A064K-T086K-T116E-R123F | | | | | | |
|---|---|---|---|---|---|---|
| N24A | R35E | N67S | G78D | R127K | R159K | |
| N24E | R35E | G54D | N67S | R127K | R159N | |
| N24Q | R35D | N67S | G78D | R127K | R159F | |
| N24T | G54D | N67S | G78D | R127Y | R159E | |
| N24E | R35E | G54D | N67S | G78D | R127K | R159K |

## EXAMPLE 17

### Correlation of Deleterious Mutations for Multiple Properties

In this Example, the principle that deleterious mutations for any property are correlated with deleterious mutations for every other property, regardless of correlations of the properties is exemplified. As indicated herein, only a small number of positions (5-10%) have mutations that are bad for all properties. These positions define the protein fold and are conserved in evolution. The implication of this is that although identification of beneficial mutations for any property requires a truly predictive screen for that property, identification of mutations likely to be deleterious for any property can be accomplished using any screen, including but not limited to the methods provided herein.

The variant enzymes were produced as described herein and within US Pat. Appln. Ser. Nos. 10/576,331, 10/581,014, 11/581,102, and 11/583,334, all of which are incorporated by reference in their entirety. The tables below provide pair-wise comparisons of the numbers of variants with more than 5% wt activity and less than 5% activity for each of two properties, along with correlation coefficients for the two properties. The assay systems used in this Example are also provided in the above referenced applications. The properties used herein were casein activity (CAS), keratin activity (KER), AAPF activity (AAPF), LAS stability (LAS) and thermal stability for ASP; and peracid formation (PAF) and peracid degradation (PAD) for ACT.

### Keratin Hydrolysis Assay

In this assay system, the chemical and reagent solutions used were:

| | |
|---|---|
| **Keratin** | **ICN 902111** |
| Detergent | 1.6 g. detergent was dissolved in 1000 ml water (pH = 8.2) 0.6 ml. CaCl2/MgCl2 of 10,000 gpg was also added, as well as 1190 mg HEPES, giving a hardness and buffer strength of 6 gpg and 5 mM respectively. |
| | The pH was adjusted to 8.2 with NaOH. |
| | Picrylsulfonic acid (TNBS) |
| | Sigma P-2297 (5% solution in water) |
| Reagent A | 45.4 g $Na_2B_4O_7$.10 H2O (Merck 6308) and 15 ml of 4N NaOH were dissolved together to a final volume of 1000 ml (by heating if needed) |
| Reagent B | 35.2 g $NaH_2PO_4$.$1H_2O$ (Merck 6346) and 0.6 g $Na_2SO_3$ (Merck 6657) were dissolved together to a final volume of 1000 ml. |

### Method:

Prior to the incubations, keratin was sieved on a 100 μm sieve in small portions at a time. Then, 10 g of the < 100 μm keratin was stirred in detergent solution for at least 20 minutes at room temperature with regular adjustment of the pH to 8.2. Finally, the suspension was centrifuged for 20 minutes at room temperature (Sorvall, GSA rotor, 13,000 rpm). This procedure was then repeated. Finally, the wet sediment was suspended in detergent to a total volume of 200 ml, and the suspension was kept stirred during pipetting. Prior to incubation, MTPs were filled with 200 μl substrate per well with a Biohit multichannel pipette and 1200 μl tip (6 dispenses of 200 μl and dispensed as fast as possible to avoid settling of keratin in the tips). Then, 10μl of the filtered culture was added to the substrate containing MTPs. The plates were covered with tape, placed in an incubator and incubated at 20 °C for 3 hours at 350 rpm (New Brunswick Innova 4330). Following incubation, the plates were centrifuged for 3 minutes at 3000 rpm (Sigma 6K 15 centrifuge). About 15 minutes before removal of the first plate from the incubator, the TNBS reagent was prepared by mixing 1 ml TNBS solution per 50 ml of reagent A.

MTPs were filled with 60 µl TNBS reagent A per well. From the incubated plates, 10 µl was transferred to the MTPs with TNBS reagent A. The plates were covered with tape and shaken for 20 minutes in a bench shaker (BMG Thermostar) at room temperature and 500 rpm. Finally, 200 µl of reagent B was added to the wells, mixed for 1 minute on a shaker, and the absorbance at 405 nm was measured with the MTP-reader.

**Calculation of the Keratin Hydrolyzing Activity**

The obtained absorbance value was corrected for the blank value (substrate without enzyme). The resulting absorbance provides a measure for the hydrolytic activity. For each sample (variant) the performance index was calculated. The performance index compares the performance of the variant (actual value) and the standard enzyme (theoretical value) at the same protein concentration. In addition, the theoretical values can be calculated, using the parameters of the Langmuir equation of the standard enzyme. A performance index (PI) that is greater than 1 (PI>1) identifies a better variant as compared to the standard (e.g., wild-type), while a PI of 1 (PI=1) identifies a variant that performs the same as the standard, and a PI that is less than 1 (PI<1) identifies a variant that performs worse than the standard. Thus, the PI identifies winners, as well as variants that are less desirable for use under certain circumstances.

**Dimethylcasein Hydrolysis Assay (96 wells)**

In this assay system, the chemical and reagent solutions used were:

| | |
|---|---|
| Dimethylcasein (DMC): Sigma C-9801 | |
| TWEEN®-80: | Sigma P-8074 |
| PIPES buffer (free acid): | Sigma P-1851; 15.1 g was dissolved in about 960 ml water; pH is adjusted : to 7.0 with 4N NaOH, 1 ml 5% TWEEN®-80 was added and the volume brought up to 1000 ml. The final concentration of PIPES and TWEEN®-80 is 50 mM and 0.005% respectively. |
| Picrylsulfonic acid (TNBS): | Sigma P-2297 (5% solution in water) |
| Reagent A: | 45.4 g $Na_2B_4O_7$.10 H2O (Merck 6308) and 15 ml of 4N NaOH dissolved together to a final volume of 1000 ml (by heating if needed) |
| Reagent B: | 35.2 g $NaH_2PO_4$.$1H_2O$ (Merck 6346) and 0.6 g $Na_2SO_3$ (Merck 6657) dissolved together to a final volume of 1000 ml. |

**Method:**

To prepare the substrate, 4 g DMC were dissolved in 400 ml PIPES buffer. The filtered culture supernatants were diluted with PIPES buffer; the final concentration of the controls in the growth plate was 20 ppm. Then, 10 µl of each diluted supernatant were added to 200 µl substrate in the wells of a MTP. The MTP plate was covered with tape, shaken for a few seconds and placed in an oven at 37°C for 2 hours without agitation.

About 15 minutes before removal of the first plate from the oven, the TNBS reagent was prepared by mixing 1 ml TNBS solution per 50 ml of reagent A. MTPs were filled with 60 µl TNBS reagent A per well. The incubated plates were shaken for a few seconds, after which 10 µl were transferred to the MTPs with TNBS reagent A. The plates were covered with tape and shaken for 20 minutes in a bench shaker (BMG Thermostar) at room temperature and 500 rpm. Finally, 200 µl of reagent B were added to the wells, mixed for 1 minute on a shaker, and the absorbance at 405 nm was determined using an MTP reader.

**Calculation of Dimethylcasein Hydrolyzing Activity:**

The obtained absorbance value was corrected for the blank value (substrate without enzyme). The resulting absorbance is a measure for the hydrolytic activity. The (arbitrary) specific activity of a sample was calculated by dividing the absorbance and the determined protein concentration.

**Thermostability Assay**

This assay is based on the dimethylcasein (DMC) hydrolysis, before and after heating of the buffered culture supernatant. The same chemical and reagent solutions were used as described in the DMC hydrolysis assay.

**Method:**

The filtered culture supernatants were diluted to 20 ppm in PIPES buffer (based on the concentration of the controls in the growth plates). Then, 50 μl of each diluted supernatant were placed in the empty wells of a MTP. The MTP plate was incubated in an iEMS incubator/shaker HT (Thermo Labsystems) for 90 minutes at 60°C and 400 rpm. The plates were cooled on ice for 5 minutes. Then, 10 μl of the solution was added to a fresh MTP containing 200 μl DMC substrate/well. This MTP was covered with tape, shaken for a few seconds and placed in an oven at 37 °C for 2 hours without agitation. The same detection method as used for the DMC hydrolysis assay was employed.

**Calculation of Thermostability:**

The residual activity of a sample was expressed as the ratio of the final absorbance and the initial absorbance, both corrected for blanks.

As indicated in the following Tables, the only properties that were found to be correlated (correlation coefficients > 0.5) were CAS, KER and AAPF for ASP. All of the others were not correlated (correlation coefficient <0.3). In spite of the fact that the properties were not correlated, the probability that a mutation would be deleterious for the two properties is much higher than expected by chance. Table 17-1 provides the calculated ratios of observed numbers of variants, and expected numbers of variants based on chance. Numbers that are greater than 1 indicate positive correlations, and numbers that are less than 1 indicate negative correlations.

**Table 17-1. CAS and KER Comparison Results for ASP**

| | Observed | | | Expected | | |
|---|---|---|---|---|---|---|
| **Value** | **CAS** | **KER** | | **CAS** | **KER** | **Observed/Expected** |
| <=5% | 892 | 674 | | 31% | 24% | |
| >5% | 1959 | 2177 | | 69% | 76% | |
| | | | | | | |
| both >5% | 1877 | 66% | | 1496 | 52% | **1.25** |
| one >5% | 382 | 13% | | 1144 | 40% | **0.33** |
| Both <=5% | 592 | 21% | | 211 | 7% | **2.81** |
| at least one >5% | 2259 | 79% | | 2640 | 93% | **0.86** |

**Table 17-2. CAS and AAPF Comparison Results for ASP**

| | Observed | | | Expected | | |
|---|---|---|---|---|---|---|
| **Value** | **CAS** | **AAPF** | | **CAS** | **AAPF** | **Observed/Expected** |
| <=5% | 892 | 1263 | | 31% | 44% | |
| >5% | 1959 | 1588 | | 69% | 56% | |
| | | | | | | |
| both >5% | 1576 | 55% | | 1091 | 38% | **1.44** |
| one >5% | 395 | 14% | | 1365 | 48% | **0.29** |
| Both <=5% | 880 | 31% | | 395 | 14% | **2.23** |
| at least one >5% | 1971 | 69% | | 2456 | 86% | **0.80** |

| Table 17-3. CAS and LAS Comparison Results for ASP | | | | | | |
|---|---|---|---|---|---|---|
| | Observed | | | Expected | | |
| Value | CAS | LAS | | CAS | LAS | Observed/Expected |
| <=5% | 892 | 1450 | | 31% | 51% | |
| >5% | 1959 | 1401 | | 69% | 49% | |
| | | | | | | |
| both >5% | 1393 | 49% | | 963 | 34% | **1.45** |
| one >5% | 574 | 20% | | 1435 | 50% | **0.40** |
| Both <=5% | 884 | 31% | | 454 | 16% | **1.95** |
| at least one >5% | 1967 | 69% | | 2397 | 84% | **0.82** |

| Table 17-4. CAS and Thermal Stability Comparison Results for ASP | | | | | | |
|---|---|---|---|---|---|---|
| | Observed | | | Expected | | |
| Value | CAS | THER | | CAS | THER | Observed/Expected |
| <=5% | 892 | 1198 | | 31% | 42% | |
| >5% | 1959 | 1653 | | 69% | 58% | |
| | | | | | | |
| both >5% | 1508 | 53% | | 1136 | 40% | **1.33** |
| one >5% | 596 | 21% | | 1340 | 47% | **0.44** |
| Both <=5% | 747 | 26% | | 375 | 13% | **1.99** |
| at least one >5% | 2104 | 74% | | 2476 | 87% | **0.85** |

| Table 17-5. KER and AAPF Comparison Results for ASP | | | | | | |
|---|---|---|---|---|---|---|
| | Observed | | | Expected | | |
| Value | KER | AAPF | | KER | AAPF | Observed/Expected |
| <=5% | 674 | 1263 | | 24% | 44% | |
| >5% | 2177 | 1588 | | 76% | 56% | |
| | | | | | | |
| both >5% | 1566 | 55% | | 1213 | 43% | **1.29** |
| one >5% | 633 | 22% | | 1340 | 47% | **0.47** |
| Both <=5% | 652 | 23% | | 299 | 10% | **2.18** |
| at least one >5% | 2199 | 77% | | 2552 | 90% | **0.86** |

| Table 17-6. PAF and PAD Comparison Results for ACT | | | | | |
|---|---|---|---|---|---|
| | Observed | | Expected | | |
| Value | PAF | PAD | PAF | PAD | Observed/Expected |
| <=5% | 541 | 751 | 19% | 26% | |
| >5% | 2536 | 2326 | 89% | 82% | |
| both >5% | 2187 | 77% | 2069 | 73% | **1.06** |

(continued)

| Table 17-6. PAF and PAD Comparison Results for ACT | | | | | |
|---|---|---|---|---|---|
| | Observed | | Expected | | |
| Value | PAF | PAD | PAF | PAD | Observed/Expected |
| one >5% | 488 | 17% | 639 | 22% | **0.76** |
| Both <=5% | 402 | 14% | 143 | 5% | **2.82** |
| at least one >5% | 2675 | 94% | 2708 | 95% | **0.99** |

The following numbered paragraphs (paras.) contain statements of broad combinations of the inventive technical features herein disclosed :-

1. An isolated subtilisin variant of a *Bacillus* subtilisin, wherein said subtilisin variant is a mature form having proteolytic activity and comprising a substitution at one or more positions selected from: 1, 2, 3, 4, 5, 6, 7, 8, 9, 10, 11, 12, 13, 14, 15, 16, 17, 18, 19, 20, 21, 21, 22, 23, 24, 25, 26, 27, 28, 29, 30, 31, 33, 35, 36, 37, 39, 40, 41, 42, 43, 44, 45, 46, 47, 48, 49, 50, 51, 52, 53, 54, 55, 56, 57, 58, 59, 60, 64, 65, 66, 67, 71, 72, 73, 76, 77, 78, 79, 81, 82, 83, 84, 85, 86, 87, 88, 89, 90, 91, 92, 93, 94, 95, 96, 97, 98, 99, 101, 102, 103, 104, 105, 106, 107, 109, 110, 111, 112, 113, 114, 115, 116, 117, 118, 119, 122, 124, 125, 126, 127, 128, 129, 130, 131, 132, 133, 134, 135, 136, 137, 139, 141, 143, 144, 145, 146, 147, 148, 149, 150, 151, 152, 153, 156, 157, 158, 159, 160, 162, 163, 164, 165, 166, 167, 169, 170, 171, 172, 173, 174, 175, 176, 177, 178, 179, 180, 181, 182, 183, 184, 185, 186, 187, 188, 189, 190, 191, 192, 194, 195, 196, 197, 198, 199, 200, 201, 203, 204, 206, 207, 209, 210, 212, 213, 214, 215, 216, 217, 218, 219, 220, 222, 223, 224, 225, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 267, 268, 269, 270, 271, 272, 273 and 274, wherein said positions correspond to the amino acid sequence of *B. amyloliquefaciens* subtilisin BPN' set forth as SEQ ID NO:2.

2. The isolated subtilisin variant of para. 1, wherein said one or more positions is selected from: 1, 2, 5, 6, 7, 8, 9, 10, 11, 12, 13, 14, 15, 16, 17, 18, 19, 20, 21, 21, 22, 23, 24, 25, 26, 28, 29, 30, 31, 33, 35, 36, 37, 39, 40, 41, 42, 43, 44, 45, 46, 47, 48, 49, 50, 51, 52, 53, 54, 55, 56, 57, 58, 59, 60, 64, 65, 66, 67, 71, 72, 73, 77, 78, 79, 81, 82, 83, 84, 85, 86, 87, 88, 89, 90, 91, 92, 93, 94, 95, 96, 97, 98, 99, 101, 102, 103, 104, 105, 106, 107, 109, 110, 111, 112, 113, 114, 115, 116, 118, 119, 122, 124, 125, 126, 127, 128, 129, 131, 133, 134, 135, 136, 137, 139, 141, 143, 144, 145, 146, 147, 148, 149, 150, 151, 152, 153, 156, 157, 158, 159, 160, 162, 163, 164, 165, 166, 167, 169, 170, 171, 172, 173, 174, 175, 176, 177, 178, 179, 180, 181, 182, 183, 184, 185, 186, 187, 188, 189, 190, 191, 192, 194, 195, 196, 197, 198, 199, 200, 201, 203, 204, 206, 207, 212, 213, 214, 215, 216, 217, 218, 219, 220, 222, 223, 224, 225, 228, 229, 230, 231, 232, 233, 234, 235, 236, 237, 238, 239, 240, 241, 242, 243, 244, 245, 246, 247, 248, 249, 250, 251, 253, 254, 255, 256, 257, 258, 259, 260, 261, 262, 263, 264, 265, 267, 268, 269, 270, 271, 272, 273 and 274, wherein said positions correspond to the amino acid sequence of *B. amyloliquefaciens* subtilisin BPN' set forth as SEQ ID NO:2.

3. The subtilisin variant of para. 1, wherein said one or more positions are selected from 2, 3, 5, 6, 7, 8, 9, 10, 11, 12, 13, 14, 15, 16, 17, 19, 20, 21, 21, 22, 23, 25, 26, 28, 29, 30, 31, 33, 35, 36, 37, 39, 40, 41, 42, 44, 46, 47, 48, 49, 50, 51, 53, 54, 55, 56, 57, 58, 59, 60, 64, 65, 66, 67, 71, 73, 77, 78, 79, 81, 82, 83, 84, 85, 86, 88, 89, 90, 91, 92, 93, 94, 95, 96, 102, 105, 106, 110, 111, 112, 113, 114, 115, 116, 122, 124, 125, 128, 129, 131, 133, 134, 135, 136, 137, 139, 141, 143, 145, 146, 147, 148, 149, 150, 151, 152, 153, 156, 157, 158, 159, 160, 162, 165, 166, 167, 169, 170, 171, 173, 174, 175, 176, 177, 178, 179, 180, 181, 182, 183, 184, 186, 187, 189, 190, 191, 192, 194, 195, 196, 197, 198, 199, 200, 201, 203, 204, 206, 207, 212, 214, 216, 217, 218, 219, 220, 222, 223, 224, 225, 228, 229, 230, 231, 232, 233, 234, 235, 236, 237, 238, 239, 240, 241, 242, 243, 244, 245, 246, 247, 249, 250, 251, 253, 254, 255, 257, 258, 259, 260, 261, 262, 263, 264, 265, 267, 268, 269, 270, 271, 272, 273, and 274, wherein said positions correspond to the amino acid sequence of *B. amyloliquefaciens* subtilisin BPN' set forth as SEQ ID NO:2.

4. The subtilisin variant of para. 1, wherein said subtilisin variant further comprises a substitution at one or more positions selected from the group consisting of: 18, 52, 72, 117, 119, 127, 144, 185, 209 and 213, wherein said positions correspond to the amino acid sequence of *B. amyloliquefaciens* subtilisin BPN' set forth as SEQ ID NO:2.

5. The subtilisin variant of para. 1, wherein said substitution comprises one or more of: A001C, A001 D, A001E, A001 F, A001 G, A0 para. 001I, A001 K, A001 L, A001 M, A001N, A001Q, A001R, A001S, A001V, A001W, Q002A, Q002D, Q002E, Q002G, Q002S, S003A, S003D, S003F, S003G, S003I, S003K, S003L, S003M, S003N, S003P, S003Q, S003R, S003T, S003V, S003W, S003Y, P005C, P005E, P005G, P005N, P005Q, P005T, Y006A, Y006C, Y006E, Y006F, Y006G, Y006K, Y006M, Y006N, Y006P, Y006Q, Y006R, Y006T, Y006V, Y006W, S009A, S009C, S009E, S009F, S009G, S009H, S009K, S009L, S009M, S009P, S009Q, S009R, S009T, S009V, S009W, Q010A, Q010C, Q010E, Q010F, Q010G, Q010H, Q010I, Q010K, Q010L, Q010M, Q010R, Q010S, Q010T, Q010V, Q010W, IO11C, I011L, I011T, I011V, I011Y, K012A, K012C, K012D, K012E, K012F, K012G, K012H, K012I, K012N, K012Q, K012S, K012T, K012V, K012W, K012Y, A013C, A013F, A013G, A013H, A013L, A013R, A013S, A013T, A013V, P014A, P014C, P014D, P014E, P014F, P014G, P014I, P014K, P014L, P014M, P014N, P014Q, P014R, P014S, P014T, P014V, P014W, P014Y, A015C, A015D, A015E, A015F, A015G, A015H, A015I, A015K, A015L, A015M, A015P, A015Q, A015R, A015T, A015V, A015Y, L016A, L016C, L016I, L016M, L016Q, L016S, L016T, H017F, H017I, H017L, H017M, H017V, H017W, H017Y, S018A, S018E, S018F, S018G, S018H, S018I, S018K, S018L, S018M, S018N, S018P, S018Q, S018R, S018T, S018V, S018W, S018Y, Q019A, Q019C, Q019D, Q019E, Q019G, Q019I, Q019K, Q019M, Q019R, Q019T, Q019V, G020A, G020C, G020D, G020E, G020F, G020H, G020K, G020M, G020N, G020P, G020Q, G020R, G020S, G020W, Y021A, Y021C, Y021D, Y021E, Y021F, Y021G, Y021H, Y021K, Y021M, Y021R, Y021S, Y021T, Y021V, Y021W, T022A, T022C, T022E, T022F, T022G, T022H, T022I, T022K, T022L, T022M, T022N, T022Q, T022S, T022V, T022W, T022Y, S024C, S024F, S024G, S024H, S024I, S024K, S024L, S024M, S024N, S024Q, S024R, S024T, S024W, S024Y, N025C, N025E, N025F, N025G, N025H, N0251, N025K, N025L, N025M, N025P, N025R, N025S, N025T, N025V, N025W, K027A, K027D, K027E, K027F, K027G, K027H, K027L, K027M, K027P, K027R, K027S, K027W, K027Y, D032A, D032C, D032E, D032G, D032I, D032K, D032L, D032M, D032N, D032R, D032S, D032T, D032V, D032Y, S033A, S033F, S033G, S033H, S033P, S033T, S033W, I035A, 1035C, 1035D, 1035R, I035S, 1035T, 1035V, D036A, D036C, D036E, D036Q, D036S, D036W, S037A, S037C, S037E, S037F, S037G , S037H, S037K, S037L, S037M, S037P, S037Q, S037R, S037T, S037W, H039C, H039Q, H039T, H039V, P040A, P040C, P040E, P040F, P040G, P040G, P040H, P040I, P040K, P040L, P040M, P040N, P040Q, P040R, P040S, P040T, P040V, P040W, D041E, L042I, L042M, L042V, K043A, K043C, K043D, K043E, K043F, K043G, K043I, K043L, K043M, K043N, K043R, K043S, K043T, K043V, K043W, K043Y, V044A, V044C, V044I, V044L, V044M, V044P, V044S, V044T, A045C, A045E, A045F, A045H, A045I, A045K, A045L, A045M, A045N, A045P, A045Q, A045S, A045T, A045V, A045Y, G046A, G046C, G046E, G046H, G046K, G046M, G046N, G046Q, G046T, G046W, G046Y, A048C, A048D, A048E, A048F, A048H, A048I, A048K, A048L, A048M, A048Q, A048R, A048S, A048T, A048V, A048W, A048Y, M050A, M050C, M050F, M050H, M050I, M050K, M050L, M050Q, M050R, M050S, M050T, M050V, M050W, M050Y, V0S1A, V0S1C, V051D, V051E, V051H, V051I, V051L, V051P, V051Q, P052C, P052D, P052E, P052F, P052H, P052I, P052K, P052L, P052Q, P052R, P052S, P052T, P052V, P052W, P052Y, S053E, S053F, S053G, S053H, S053I, S053K, S053L, S053M, S053N, S053Q, S053R, S053T, S053V, S053W, E054A, E054N, E054Q, E054S, T055A, T055C, T055D, T055F, T055G, T055H, T055I, T055K, T055M, T055P, T055Q, T055R, T055S, T055V, T055W, T055Y, N056D, N056M, N056P, N056Q, N056S, N056T, N056V, P057N, P057Q, P057T, P057V, P057W, F058C, F058E, F058G, F058H, F058L, F058M, F058N, F058Q, F058S, F058V, F058Y, Q059A, Q059C, Q059D, Q059E, Q059F, Q059G, Q059H, Q059K, Q059L, Q059M, Q059N, Q059P, Q059R, Q059S, Q059T, Q059V, Q059W, Q059Y, D060E, D060G, H064C, H064D, H064F, H064I, H064L, H064M, H064Q, H064R, H064S, H064T, H064V, H064Y, T066S, T066W, T066Y, H067A, H067C, H067F, H067I, H067L, H067M, H067N, H067P, H067R, H067S, H067T, T071I, T071Y, N077S, S078A, S078C, S078D, S078F, S078G, S078I, S078K, S078L, S078M, S078N, S078P, S078Q, S078R, S078T, S078V, S078W, S078Y, I079A, 1079C, 1079E, 1079F, 1079G, 1079H, 1079K, 1079L, 1079N, I079Q, 1079R, I079S, 1079T, 1079V, 1079W, 1079Y, V081F, V081I, V081L, V081M, V081T, G083F, G083P, Q084A, Q084C, Q084H, Q084I, Q084N, Q084T, A085C, A085F, A085G, A085R, A085S, P086A, P086D, P086E, P086G, P086M, P086N, P086Q, P086R, P086S, P086T, P086W, P086Y, S089C, S089D, S089E, S089F, S089G, S089H, S089K, S089L, S089V, S089W, S089Y, L090A, L090D, L090E, L090G, L090H, L090M, L090Q, L090T, L090V, Y091A, Y091C, Y091D, Y091E, Y091F, Y091H, Y091I, Y091L, Y091M, Y091Q, Y091S, Y091T, Y091V, L096F, L096H, L096I, L096K, L096M, L096T, L096V, G097A, G097C, G097D, G097E, G097H, G097K, G097L, G097M, G097P, G097Q, G097R, G097S, G097T, G097V, A098C, A098D, A098F, A098G, A098H, A098I, A098L, A098P, A098Q, A098R, A098S, A098T, A098V, A098Y, D099C, D099E, D099I, D099K, D099L, D099N, D099P, D099Q, D099R, D099S, D099T, S101A, S101C, S101E, S101F, S101G, S101I, S101K, S101L, S101M, S101N, S101P, S101Q, S101R, S101T, S101V, S101Y, G102A, G102C, G102E, G102F, G102I, G102N, G102S, G102V, G102Y, Q103A, Q103C, Q103E, Q103F, Q103G, Q103I, Q103K, Q103L, Q103M, Q103N, Q103R, Q103S, Q103T, Q103V, Q103W, S105A, S105C, S105D, S105E, S105F, S105G, S105I, S105K, S105L, S105M, S105N, S105P, S105R, S105V, S105W, W106A, W106C, W106E, W106F, W106G, W106H, W106I, W106L, W106M, W106N, W106R, W106S, W106T, W106V, W106Y, N109A, N109C, N109E, N109F, N109G, N109H, N109L, N109M, N109P, N109Q, N109R, N109S, N109T, N109V, N109W, N109Y, I111C, I111F, I111L, I111M, I111N, I111P, I111T, I111V, I111W, E112A, E112C, E112D, E112F, E112G, E112I, E112L, E112M, E112N, E112Q, E112R, E112S, E112T, E112V, E112W, E112Y, W113D, W113E, W113F, W113N, W113P, W113Q, W113S, W113V, W113Y, I115E, I115H, I115N, I115R, I115T, I115V, I115W, I115Y, A116C, A116D, A116E, A116F, A116G, A116H, A116I, A116K, A116L, A116M, A116N, A116P, A116Q, A116R, A116S, A116T, A116V, A116W, A116Y, N118A, N118C, N118D, N118E, N118F, N118G, N118H, N118I, N118K, N118L, N118M, N118Q, N118R, N118S, N118T, N118V, N118W, N118Y, I122A, I122C, I122H, I122K, I122L, I122M, I122N, I122P, M124C, M124D, M124I, M124L, M124S, M124T, M124V, M124W, S125A, S125C, S125E, S125F, S125I, S125K, S125M, S125N, S125P, S125Q, S125R, S125T, S125W, L126A, L126C, L126I, L126K, L126N, L126R, L126S, L126T, L126V, L126W, L126Y, G127A, G127C, G127E, G127F, G127I, G127K, G127M, G127N, G127P, G127Q, G127S, G127T, G127V, G127W, G127Y, G128A, G128D, G128F, G128H, G128L, G128N, G128P, G128S, G128T, G128V, G128W, G128Y, P129A, P129C, P129D, P129E, P129F, P129G, P129I, P129K, P129L, P129M, P129N, P129Q, P129S, P129T, P129V, P129Y, G131A, G131C, G131E, G131F, G131K, G131L, G131M, G131N, G131P, G131Q, G131R, G131S, G131T, G131V, G131W, G131Y, A133C, A133D, A133E, A133F, A133G, A133I, A133K, A133L, A133M, A133P, A133R, A133S, A133T, A133V, A133W, A133Y, A134D, A134E, A134F, A134G, A134I, A134K, A134L, A134M, A134P, A134Q, A134R, A134S, A134T, A134V, A134W, L135D, L135E, L135M, L135R, L135W, L135Y, K136E, K136H, K136L, K136M, K136N, K136R, K136S, K136T, K136V, K136W, K136Y, A137C, A137E, A137F, A137H, A137K, A137L, A137M, A137N, A137P, A137Q, A137R, A137S, A137T, A137V, A137W, A137Y, V139C, V139I, V139L, V139N, V139S, V139T, K141A, K141C, K141D, K141E, K141F, K141G, K141H, K141I, K141L, K141M, K141N , K141Q, K141R, K141S, K141V, K141W, K141Y, V143A, V143C, V143D, V143E, V143F, V143G, V143K, V143L, V143M, V143N, V143Q, V143R, V143S, V143T, V143W, A144C, A144D, A144E, A144G, A144I, A144K, A144L, A144M, A144R, A144S, A144T, A144V, A144W, S145A, S145C, S145D, S145E, S145F, S145G, S145H, S145I, S145L, S145M, S145Q, S145R, S145T, S145V, S145W, S145Y, G146A, G146C, G146D, G146E, G146M, G146Q, G146R, G146S, G146T, G146Y, A153S, G154L, G154P, G154T, E156A, E156C, E156F, E156K, E156L, E156N, E156Q, E156R, E156S, E156T, E156V, E156W, E156Y, T158A, T158D, T158E, T158G, T158H, T158I, T158K, T158L, T158M, T158N, T158Q, T158S, T158V, T158Y, S159A, S159C, S159D, S159E, S159G, S159H, S159I, S159K, S159M, S159Q, S159R, S159T, G160D, G160E, G160K, G160N, G160P, G160Q, G160S, G160T, S162A, S162C, S162E, S162H, S162K, S162L, S162M, S162N, S162Q, S162R, S162T, S162V, S163G, S163P, Y167A, Y167F, Y167H, Y167I, P168D, P168G, P168I, P168M, P168S, P168Y, G169A, G169E, G169F, G169H, G169I, G169M, G169N, G169R, G169T, G169V, G169Y, K170A, K170C, K170F, K170G, K170H, K170R, K170V, K170W, K170Y, Y171G, Y171K, Y171P, P172A, P172C, P172E, P172K, P172L, P172M, P172N, P172Q, P172R, P172S, P172T, P172V, P172Y, S173A, S173C, S173E, S173I, S173T, S173V, V174C, V174F, V174H, V174R, V174T, I175G, I175L, I175M, I175R, I175T, I175V, A176C, A176E, A176F, A176K, A176M, A176S, A176Y, A179G , V180A, V180C, V180L, V180N, V180S, V180T, D181C, D181E, D181G, D181H, D181M, D181N, D181S, D181T, D181W, S182A, S182C, S182D, S182F, S182G, S182H, S182I, S182K, S182M, S182P, S182Q, S182R, S182V, S182Y, S183A, S183C, S183E, S183F, S183G, S183H, S183I, S183K, S183L, S183M, S183N, S183Q, S183R, S183T, S183V, S183W, S183Y, N184C, N184D, N184E, N184G, N184H, N184K, N184L, N184M, N184Q, N184S, N184T, N184V, N184W, Q185A, Q185C, Q185E, Q185F, Q185G, Q185H, Q185I, Q185K, Q185L, Q185M, Q185N, Q185S, Q185T, Q185V, Q185W, R186I, R186L, R186W, A187C, A187D, A187E, A187F, A187G, A187P, A187S, A187W, A187Y, S188A, S188C, S188D, S188E, S188F, S188G, S188H, S188I, S188K, S188L, S188M, S188P, S188Q, S188T, S188V, S188W, S188Y, S190F, S190H, S190I, S190K, S191A, S191G, S191N, S191P, V192A, V192S, V192T, V192Y, G193F, G193H, G193I, G193N, G193P, G193R , G193T, P194C, P194E, P194H, P194I, P194K, P194L, P194M, P194Q, P194T, P194V, P194W, L196A, M199P, M199S, A200C, A200G, A200K, A200Y, G202D, G202E, G202F, G202L, G202P, G202V, G202Y, Q206A, Q206C, Q206D, Q206E, Q206F, Q206G, Q206H, Q206I, Q206L, Q206M, Q206N, Q206P, Q206R, Q206S, Q206T, Q206V, Q206W, Q206Y, S207D, S207E, S207K, S207Q, S207T, S207V, P210A, P210C, P210S, P210T, N212A, N212C, N212E, N212F, N212G, N212H, N212K, N212L, N212M, N212P, N212Q, N212R, N212S, N212V, K213A, K213C, K213D, K213E, K213F, K213H, K213I, K213L, K213M, K213N, K213Q, K213R, K213S, K213T, K213V, K213Y, Y214W, G215A, G215C, G215D, G215E, G215I, G215M, G215N, G215Q, G215S, G215T, G215V, A216C, A216D, A216E, A216G, A216K, A216L, A216M, A216N, A216P, A216Q, A216R, A216S, A216V, A216W, Y217A, Y217C, Y217D, Y217E, Y217F, Y217G, Y217H, Y217I, Y217K, Y217L, Y217M, Y217N, Y217Q, Y217R, Y217S, Y217T, Y217V, Y217W, N218A, N218C, N218E, N218F, N218G, N218H, N218K, N218M, N218R, N218S, N218T, N218W, N218Y, G219A, G219C, G219D, G219H, G219I, G219M, G219P, G219Q, G219R, G219S, G219T, G219V, G219W, T220D, T220E, T220F, T220G, T220K, T220M, T220S, T220Y, A223E, A223F, A223L, A223M, A223R, A223S, A223V, A223W, A223Y, S224A, S224C, S224F, S224G, S224H, S224M, S224N, S224Q, S224R, S224T, P225A, P225C, P225F, P225G, P225H, P225I, P225K, P225L, P225M, P225R, P225S, P225T, P225V, P225Y, A228P, A228R, A228S, A228T, A228W, G229A, G229H, G229I, G229S, A230C, A230E, A230G , A230Q, A230R, A230S, A230T, A230V, A231C, A231I, A231P, A231R, I234A, I234C, I234L, I234M, I234N, I234P, I234Q, I234S, I234T, I234V, L235A, L235C, L235G, L235I, L235K, L235M, L235N, L235Q, L235R, L235S, L235T, L235V, L235W, L235Y, S236A, S236C, S236D, S236E, S236G, S236H, S236N, S236Q, S236T, S236V, S236Y, K237A, K237E , K237F, K237G, K237H, K237I, K237L, K237M, K237N, K237Q, K237R, K237S, K237T, K237V, K237W, K237Y, H238C, H238D, H238E, H238F, H238M, H238R, H238S, P239C, P239D, P239E, P239F, P239H, P239L, P239M, P239N, P239Q, P239R, P239S, P239T, P239V, P239W, P239Y, N240A, N240C, N240D, N240F, N240G, N240K, N240L, N240Q, N240R, N240S, N240T, N240V, N240W, N240Y, W241A, W241F, W241G, W241H, W241I, W241K, W241M, W241Q, W241T, W241V, T242A, T242C, T242E, T242F, T242G, T242K, T242M, T242N, T242P, T242R, T242S, T242W, T242Y, N243C, N243E, N243F, N243G, N2431, N243Q, N243S, N243T, N243V, N243W, N243Y, T244A, T244D, T244F, T244G, T244H, T244K, T244L, T244M, T244N, T244P, T244Q, T244R, T244S, T244V, T244W, T244Y, Q245A, Q245C, Q245D, Q245E, Q245H, Q245I, Q245K, Q245L, Q245M, Q245R, Q245T, Q245V, Q245Y, R247W, S248A, S248E, S248F, S248G, S248H, S248I, S248L, S248M, S248N, S248Q, S248R, S248T, S248V, S248W, S248Y, S249C, S249D, S249H, S249I, S249K, S249L, S249M, S249N, S249Q, S249R, S249T, S249V, S249W, S249Y, L250D, L250F, L250H, L250I, L250M, L250T, L250V, E251A, E251T, E251W, N252A, N252C, N252E, N252G, N252H, N2521, N252L, N252M, N252Q, N252R, N252S, N252T, N252V, N252Y, T253A, T253C, T253E, T253G, T253H, T253K, T253M, T253S, T253V, T254A, T254C, T254L, T254M, T254R, T254S, T254V, T255A, T255C, T255D, T255E, T255F, T255G, T255H, T255I, T255K, T255L, T255M, T255R, T255S, T255V, K256A, K256C, K256D, K256E, K256F, K256H, K256I, K256L, K256M, K256N, K256P, K256Q, K256R, K256S, K256T, K256V, K256W, K256Y, L257A, L257C, L257F, L257G, L257H, L257I, L257K, L257M, L257N, L257R, L257S, L257T, L257V, L257W, L257Y, G258Q, D259A, D259E, D259F, D259G, D259L, D259N, D259P, D259Q, D259R, D259S, D259T, D259V, D259W, D259Y, S260A, S260C, S260D, S260E, S260F, S260G, S260H, S260L, S260M, S260N, S260P, S260R, S260V, S260W, S260Y, F261H, F261W, Y262A, Y262C, Y262E, Y262F, Y262H, Y262L, Y262M, Y262N, Y263F, Y263M, Y263T, G264F, G264I, G264L, K265A, K265C, K265E, K265G, K265H, K265L, K265M, K265N, K265Q, K265R, K265S, K265W, K265Y, G266C, G266F, G266L, G266M, G266P, G266R, G266V, G266W, G266Y, L267A, L267C, L267E, L267G, L267H, L267I, L267M, L267N, L267Q, L267S, L267T, L267V, I268A, I268C, I268K, I268L, I268M, I268P, I268R, I268V, N269D, N269E, N269K, N269L, N269P, N269Q, N269S, Q271A, Q271C, Q271D, Q271E, Q271F, Q271G, Q271H, Q271I, Q271K, Q271L, Q271M, Q271N, Q271P, Q271R, Q271S, Q271T, Q271V, Q271W, Q271Y, A272E, A272F, A272G, A272H, A272K, A272L, A272M, A272N, A272Q, A272R, A272S, A272T, A272V, A272W, A272Y, A274C, A274D, A274F, A274G, A274H, A274I, A274K, A274L, A274Q, A274S, A274T, and A274V, wherein said positions correspond to the amino acid sequence *of B. amyloliquefaciens* subtilisin BPN' set forth as SEQ ID NO:2.

6. The subtilisin variant of para. 1, wherein said substitution comprises a combination of substitutions selected from: Y021H-Y217E, Y021H-Y217Q, S101E-Y217L, Y021H-Y217L, K213I-Y217Q, A045I-Y217Q, M119F-Y217Q, A045V-Y217L, Y021H-Y217L, K213I-Y217E, A092G-A114G, Y021W-S101E, V26Q-K213I, and Y021H-S101N, wherein said positions correspond to the amino acid sequence *of B. amyloliquefaciens* subtilisin BPN' set forth as SEQ ID NO:2.

7. The subtilisin variant of para. 1, wherein said substitution comprises a combination selected from: S101N-K213I, S101N-M119H, K213L, M119N-K213N, K213N, K213I-Y217E, M119N-K213I, K213N-Y217Q, M119H-K213N, S101P-K213N, M119H-Y217Q, K213I-Y217Q, M119F-K213L, Y217Q, M119F-Y217Q, A048E-K213L, A048H-K213L, K213N, V026N-K213L, V026N-K213L, V026Y-K213N, A048D-K213N, A048H-K213N, A048H-K213L, K213L, K213N, V147D-K213L, K213I, A048E-K213L, A048D-K213N, K213I, A048H-K213N, V147D-K213N, Y021H-Y217L, Y021H-Y217Q, A045V-Y217L, S101E-Y217L, Y021H-Y217E, A045I-Y217Q, Y021H-Y217L, Y021H-Y217E, wherein said positions correspond to the amino acid sequence of *B. amyloliquefaciens* subtilisin BPN' set forth as SEQ ID NO:2.

8. The subtilisin variant of para. 1, wherein said substitution comprises a combination selected from: S101N-M119H, Y217L, M119N-K213N, S101E-Y217L, A045I-Y217Q, wherein said positions correspond to the amino acid sequence *of B. amyloliquefaciens* subtilisin BPN' set forth as SEQ ID NO:2.

9. An isolated nucleic acid encoding the subtilisin variant set forth in para. 1.

10. An expression vector comprising the nucleic acid of para. 9.

11. A host cell comprising the expression vector set forth para. 10.

12. A cleaning composition comprising the subtilisin variant of para. 1.

13. Wherein said cleaning composition is a laundry detergent.

14. Wherein said laundry detergent is a cold water detergent, a low pH detergent, or a compact detergent.

15. A methods for producing a subtilisin variant of a *Bacillus* subtilisin, comprising the steps:

a) transforming a host cell with an expression vector comprising a nucleic acid encoding the subtilisin variant of para. 1; and
b) cultivating the transformed host cell under conditions suitable for the production of the subtilisin variant, to produce a subtilisin variant.

16. The method of para. 15, further comprising the step of harvesting the produced subtilisin variant.

17. The method of para. 15, wherein said host cell is a *Bacillus* species.

18. The method of para. 17, wherein said *Bacillus* species is *B. subtilis.*

19. A method of cleaning, comprising the step of contacting a surface and/or an article comprising a fabric with a cleaning composition comprising an isolated subtilisin variant of any of paras. 1-8.

20. A method for protease engineering comprising:

a) providing a plurality of site evaluation libraries (SELs) each comprising a plurality of protease variants having distinct substitutions at an identical amino acid position of said protease;
b) testing said protease variants of said SELs and a standard protease in a test of a property of interest;
c) determining a performance index (PI) for each of said protease variants for said test;
d) identifying two or more of said amino acid positions as non-restrictive positions, wherein at least one of said plurality of protease variants in each of two of said SELs has a PI greater than 0.5; and
f) providing a multiple mutation library comprising a plurality of multiply-substituted protease variants each comprising substitutions in said two or more non-restrictive positions.

21. The method of para. 20, wherein said test comprises two or more different assays selected from the group consisting of stain removal assays (microswatch), LAS stability, EDTA stability, detergent stability assays, and specific activity assays.

22. The method of either of paras. 20 and 21, wherein said protease is a bacterial subtilisin.

23. A method for producing a multiply substituted subtilisin variant of a *Bacillus* subtilisin, comprising:

a) testing a plurality of singly-substituted subtilisin variants in a first test of a first property and a second test of a second property, wherein the property of a parent subtilisin is given a value of 1.0 in each test, a favorable first or second property has a value greater than 1.0, and an unduly unfavorable first or second property has a value less than about 0.80 or in some preferred embodiments, less than about 0.60;
b) identifying a substitution in at least one of the singly-substituted subtilisin variants that is associated with a favorable first property and which is not associated with an unduly unfavorable second property;
c) identifying a substitution in at least one of the singly-substituted subtilisin variants that is associated with a favorable second property and which is not associated with an unduly unfavorable first property;
d) introducing the substitution from the previous steps into a subtilisin to yield a multiply-substituted subtilisin variant.

24. The method of para. 23, further comprising testing the multiply-substituted subtilisin variant in the first test and the second test, wherein an improved subtilisin variant achieves a value of greater than 1.0 in both of said first and second tests, or a value of greater than 1.0 in the first test and a value of 0.80 to 1.0 in the second test.

25. The method of para. 24, further comprising producing the improved subtilisin variant(s).

26. The method of para. 25, wherein said first and second properties are negatively correlated.

27. The method of para. 26, wherein a favorable first or second property has a value greater than about 1.2.

28. The method of para. 24, wherein an unduly unfavorable first or second property has a value less than about 0.40.

29. The method of para. 24, wherein the first property is stability, and the second property is wash performance.

30. The method of para. 29, wherein said stability comprises stability in detergent and said wash performance comprises blood milk ink (BMI) wash performance in detergent.

31. The method of para. 24, wherein wash performance is tested in a powder or liquid detergent composition having a pH of between 5 and 12.0.

32. The method of para. 24, wherein the parent bacterial subtilisin is a wild type mature form of a *B. amyloliquefaciens* subtilisin BPN' having an amino acid sequence set forth as SEQ ID NO:2.

33. The method of para. 24, wherein the positions are in a parent subtilisin having a solvent accessible surface (SAS) of greater than about 50%.

34. The method of para. 33, wherein said one or more positions in a parent subtilisin are positions having a solvent accessible surface (SAS) of greater than about 65%.

## Claims

1. An isolated subtilisin variant of a *Bacillus* subtilisin comprising the combination of modifications G128A-Y217Q, wherein said positions correspond to the amino acid sequence *of B. amyloliquefaciens* subtilisin BPN' set forth as SEQ ID NO:2.

2. An isolated nucleic acid encoding a subtilisin variant according to claim 1.

3. An expression vector comprising a nucleic acid according to claim 2.

4. A host cell comprising an expression vector according to claim 10.

5. A cleaning composition comprising a subtilisin variant according to claim 1.

6. A cleaning composition according to claim 5 wherein said cleaning composition is a laundry detergent.

7. A cleaning composition according to claim 6 wherein said laundry detergent is a cold water detergent, a low pH detergent, or a compact detergent.

8. A method for producing a subtilisin variant of a *Bacillus* subtilisin, comprising the steps:

   a) transforming a host cell with an expression vector comprising a nucleic acid encoding a subtilisin variant according to claim 1; and
   b) cultivating the transformed host cell under conditions suitable for the production of the subtilisin variant, to produce a subtilisin variant.

9. A method according to claim 8, further comprising the step of harvesting the produced subtilisin variant.

10. A method according to claim 8, wherein said host cell is a *Bacillus* species.

11. A method according to claim 10, wherein said *Bacillus* species is *B. subtidis.*

12. A method of cleaning, comprising the step of contacting a surface and/or an article comprising a fabric with a cleaning composition comprising an isolated subtilisin variant according to claim 1.

Fig. 1A

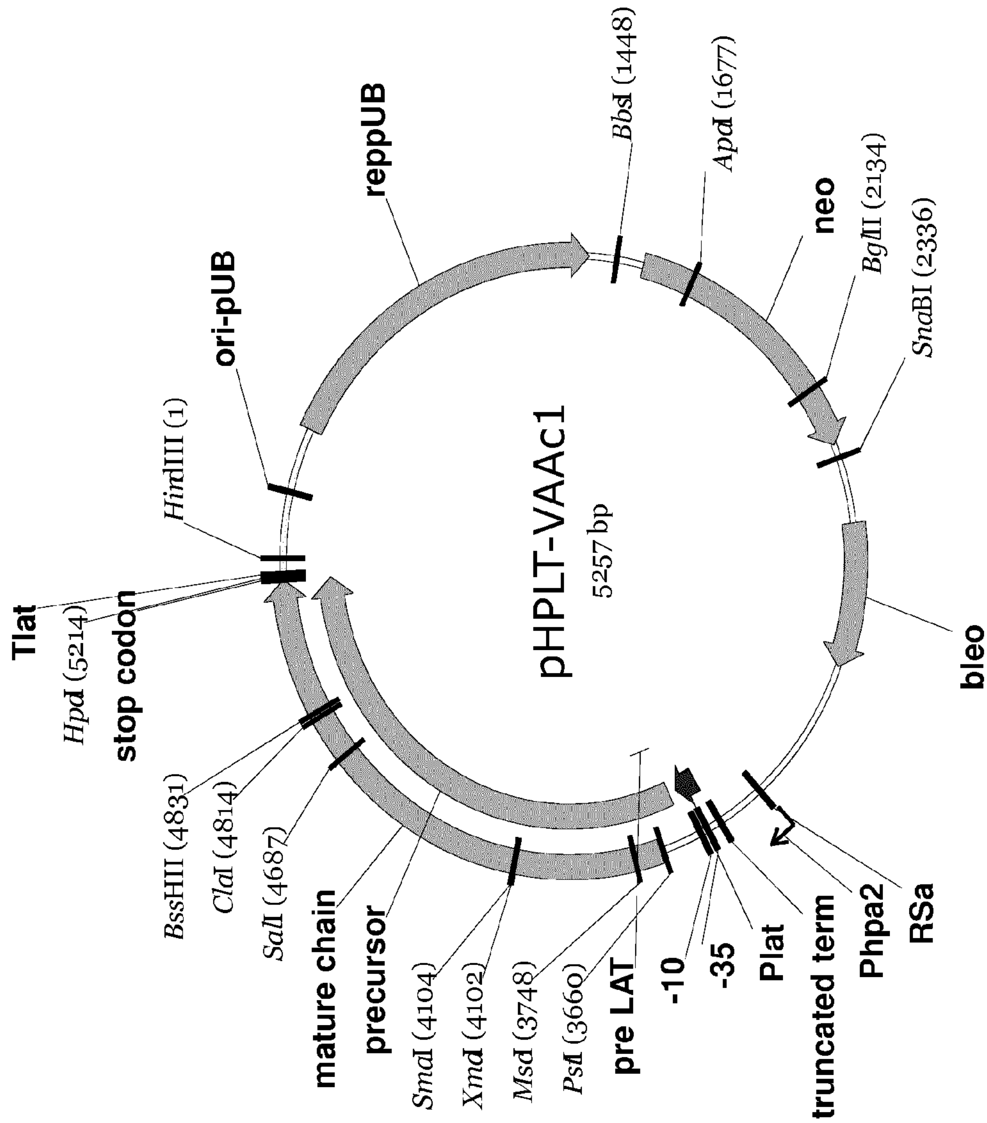
pHPLT-VAAc1
5257 bp
reppUB
BbsI (1448)
ApaI (1677)
neo
BglII (2134)
SnaBI (2336)
bleo
RSa
Phpa2
truncated term
Plat
-35
-10
pre LAT
PstI (3660)
MscI (3748)
XmaI (4102)
SmaI (4104)
precursor
mature chain
SalI (4687)
ClaI (4814)
BssHII (4831)
stop codon
HpaI (5214)
Tlat
HindIII (1)
ori-pUB

Fig. 1B

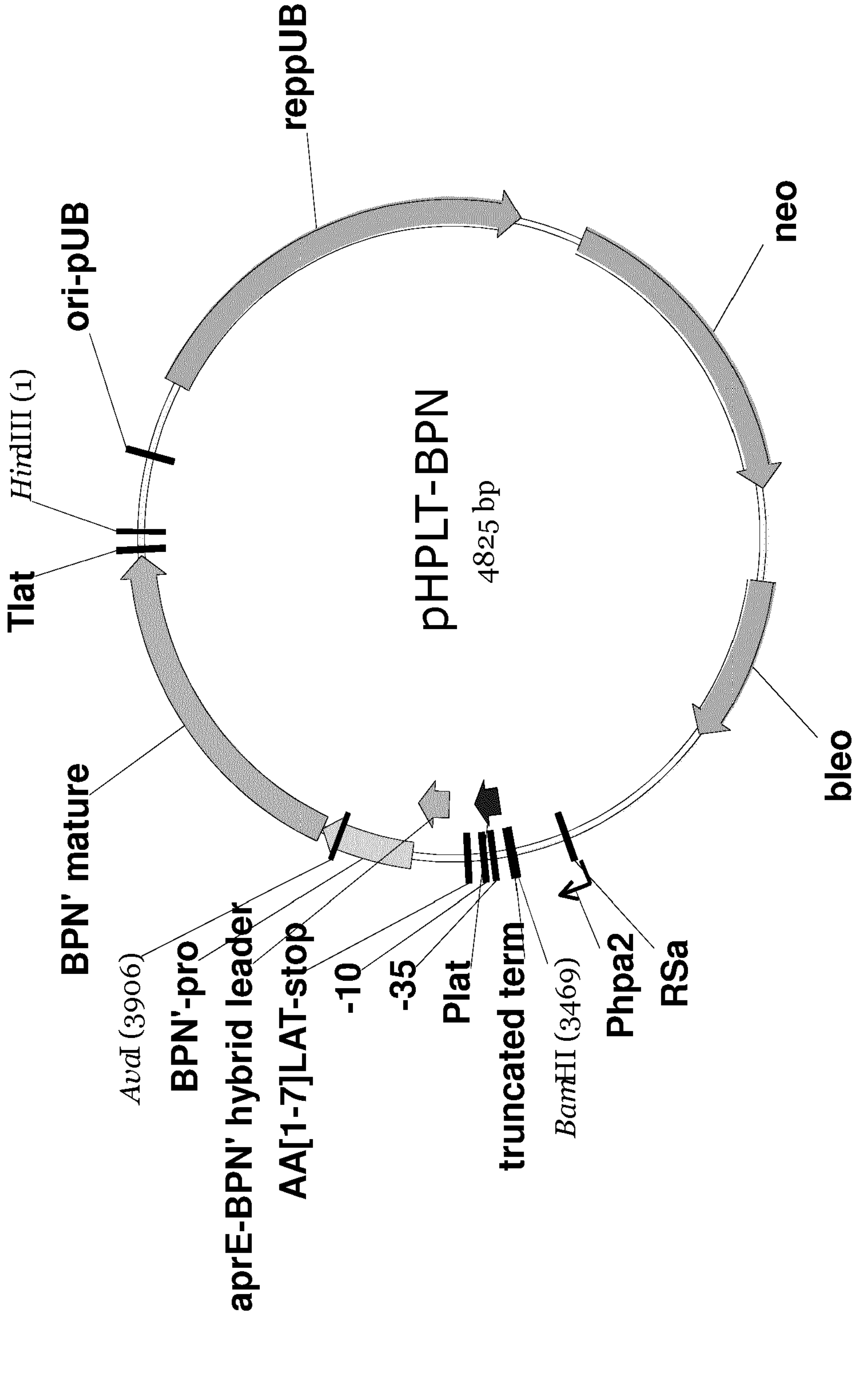
pHPLT-BPN
4825 bp
reppUB
neo
bleo
ori-pUB
HindIII (1)
Tlat
BPN' mature
AvaI (3906)
BPN'-pro
aprE-BPN' hybrid leader
AA[1-7]LAT-stop
-10
-35
Plat
truncated term
BamHI (3469)
Phpa2
RSa

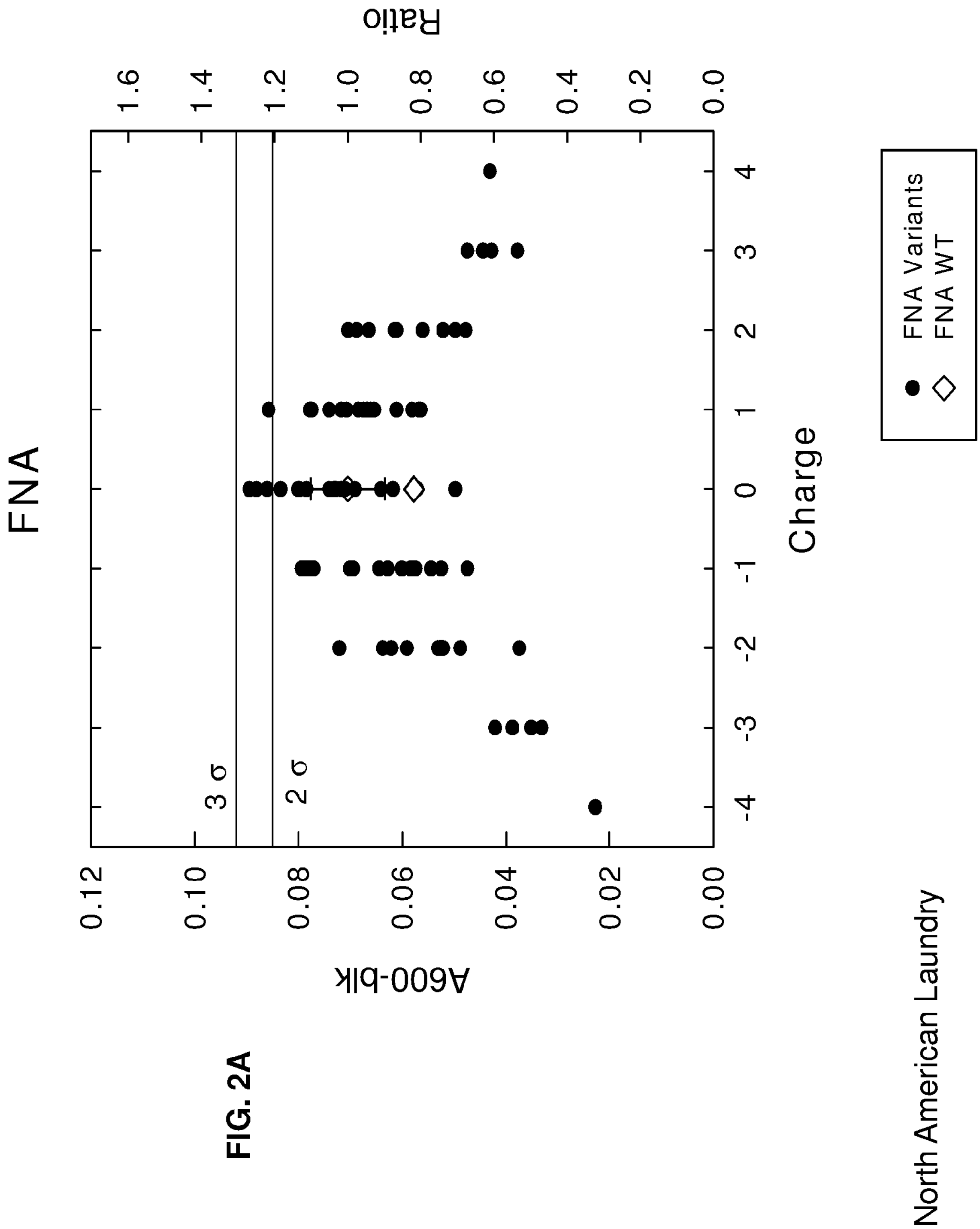


**FIG. 2A**

North American Laundry

FIG. 2B

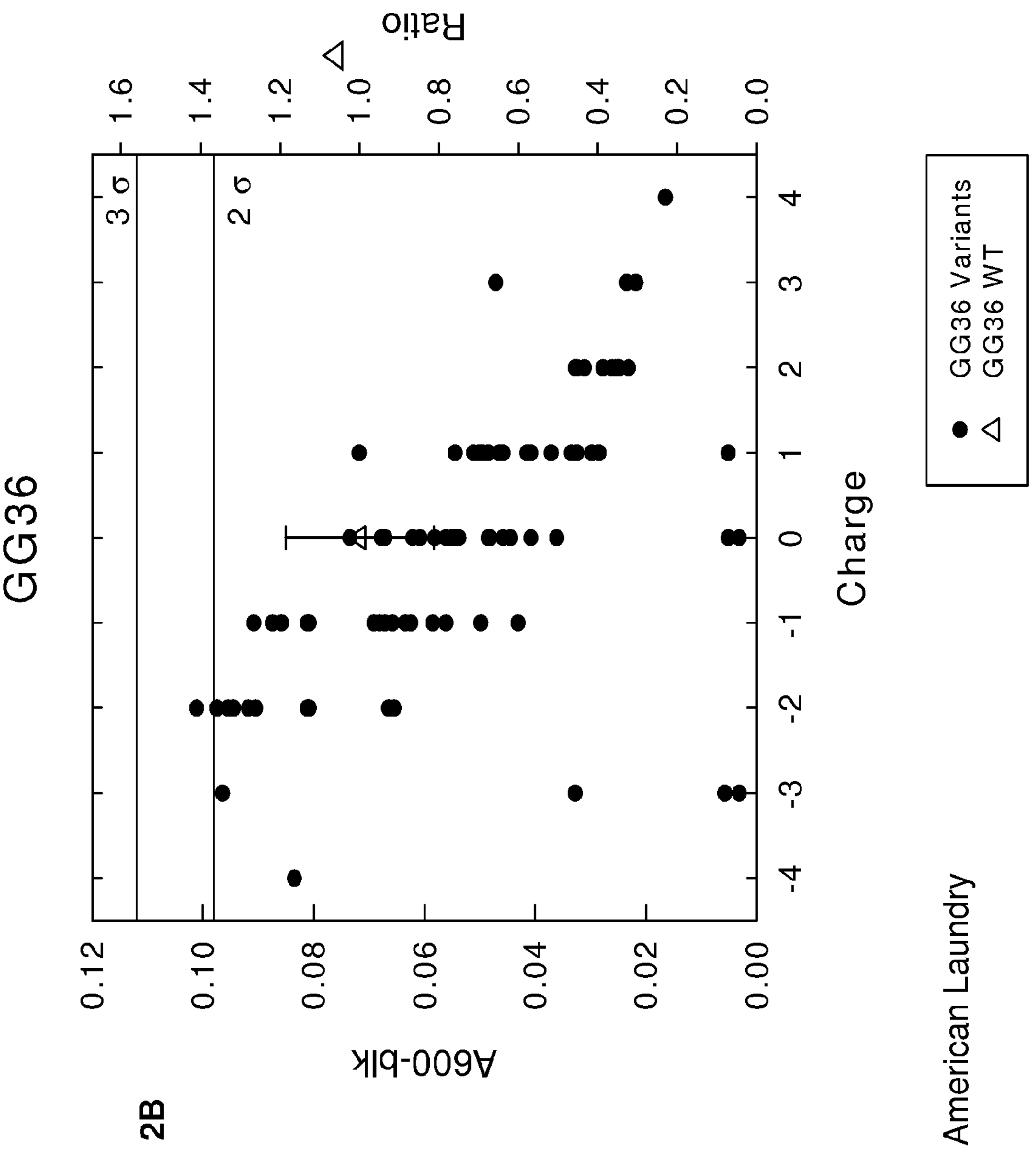


North American Laundry

FIG. 3A

FNA

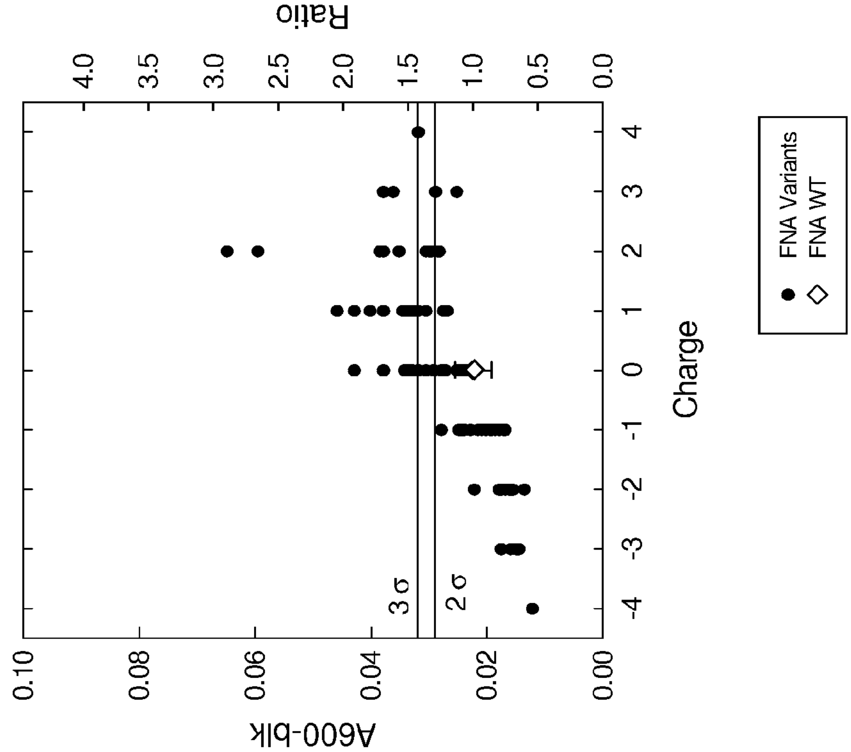


FIG. 3B

GG36

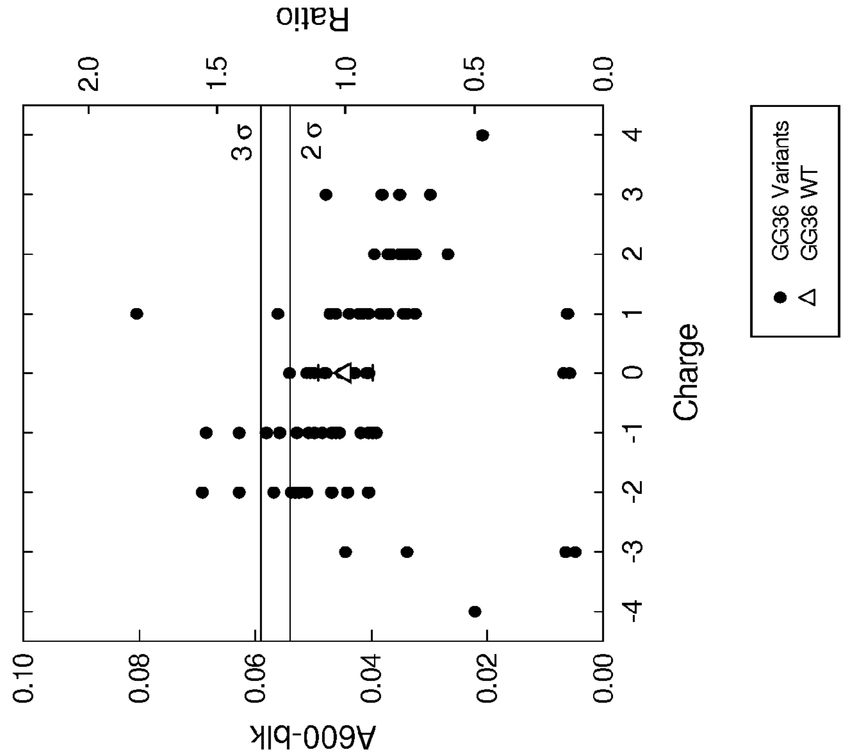


Western European Liquid Laundry

FIG. 4A

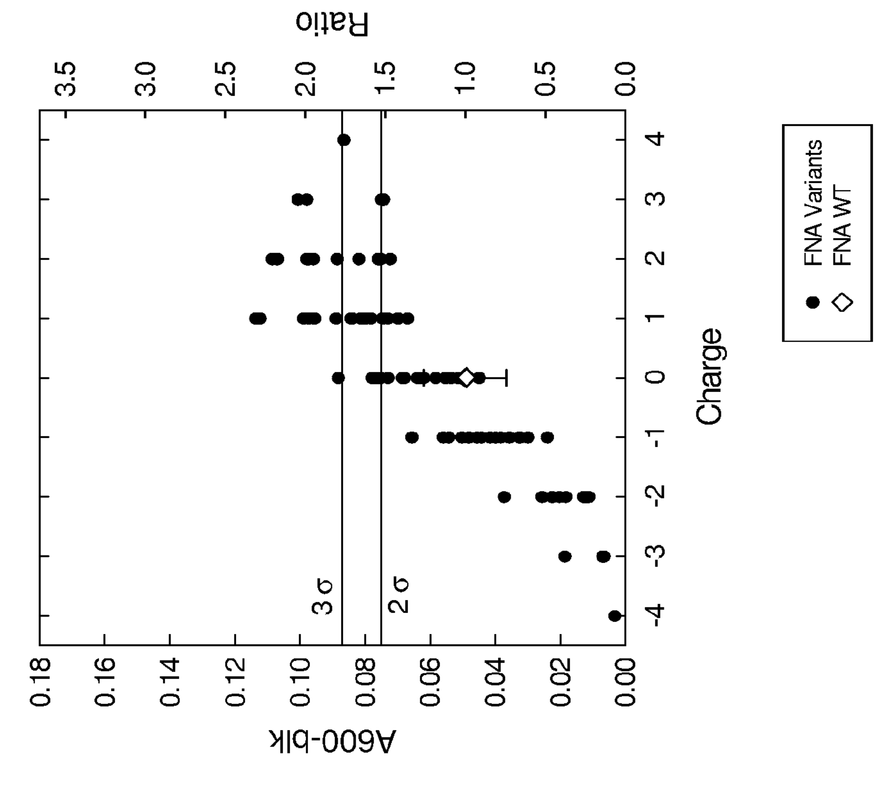


FIG. 4B

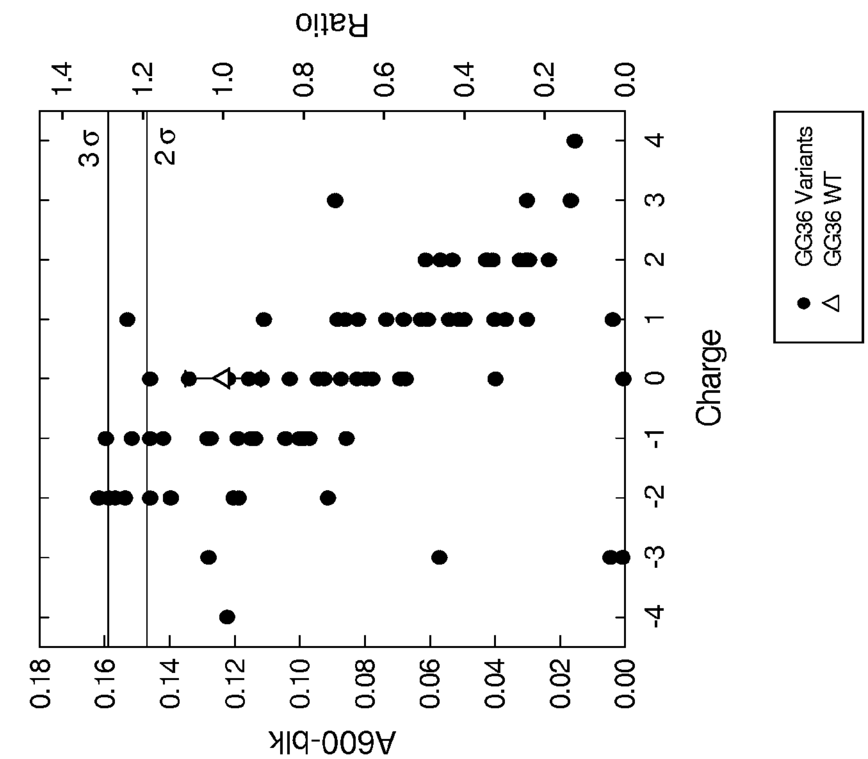


Japanese Laundry Powder

**FIG. 5A**

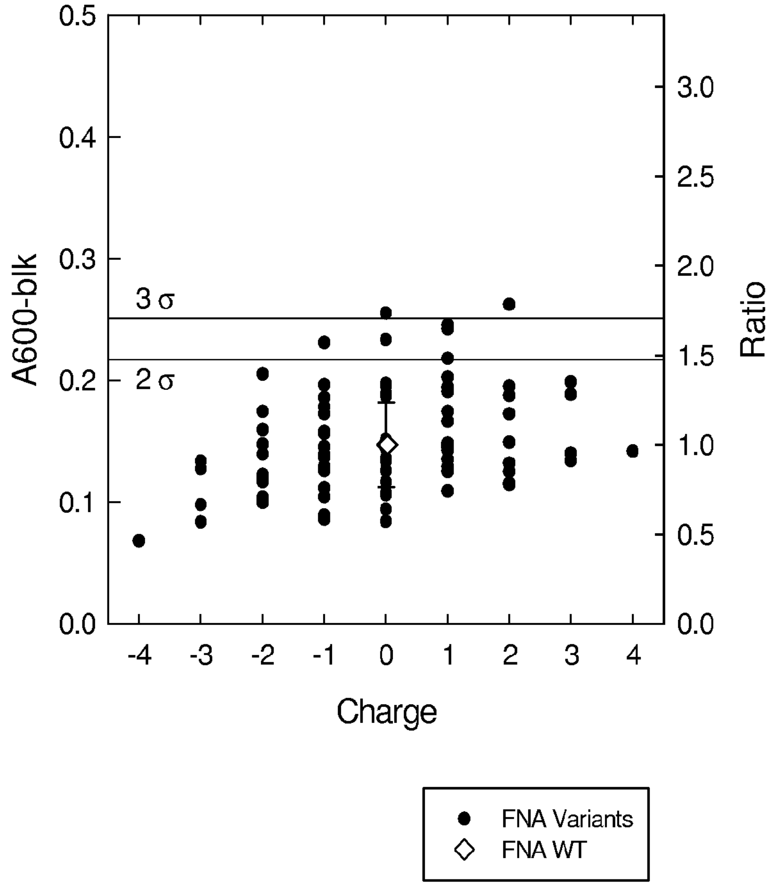


**FIG. 5B**

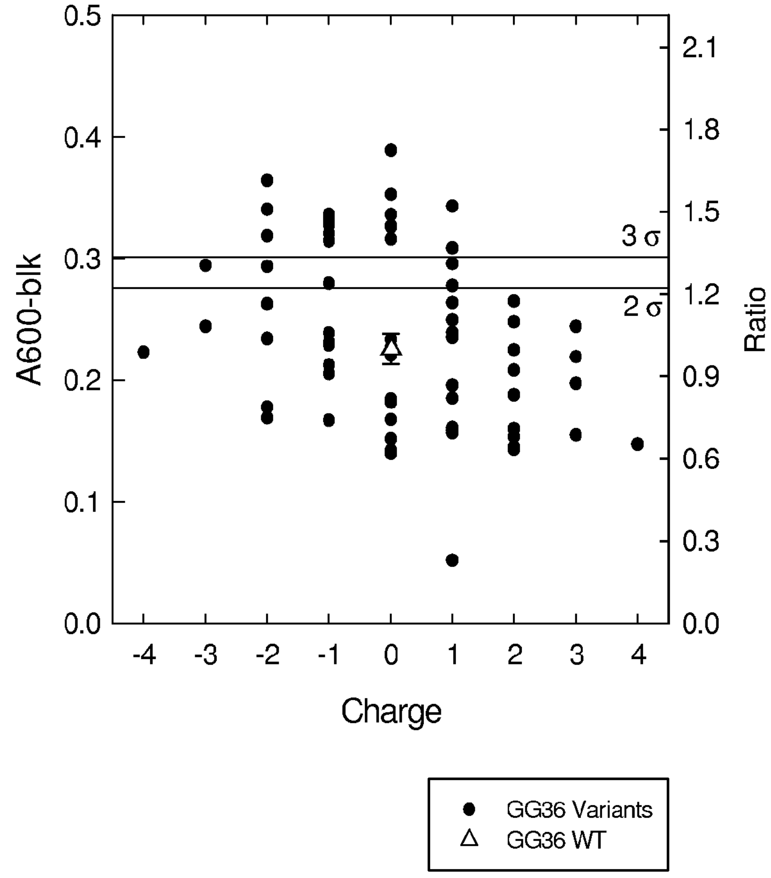


Automatic Dish Washing Detergent

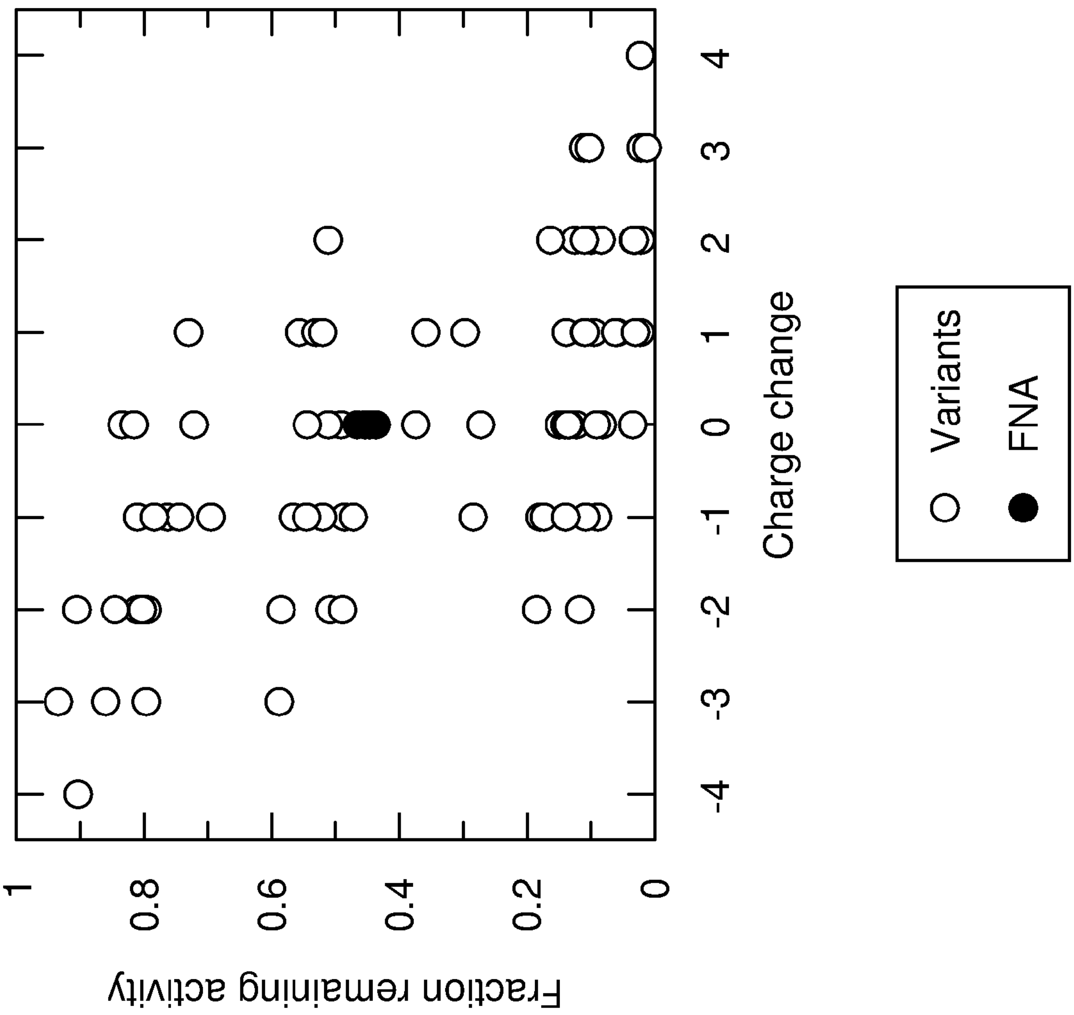
Fraction remaining activity
0
0.2
0.4
0.6
0.8
1
Charge change
-4
-3
-2
-1
0
1
2
3
4
Variants
FNA

FIG. 6

Europäisches Patentamt
European Patent Office
Office européen des brevets

# EUROPEAN SEARCH REPORT

Application Number
EP 12 18 3920

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE Geneseq [Online]<br><br>13 January 2000 (2000-01-13), "Wild type subtilisin BPN'.", XP002544118, retrieved from EBI accession no. GSP:AAY43221<br>Database accession no. AAY43221<br>* the whole document *<br>----- | 1-12 | INV.<br>C12N9/54<br>A23K1/165 |
| Y | WO 02/077187 A2 (GENENCOR INT [US]; PROCTER & GAMBLE [US]; ESTELL DAVID A [US]; GANSHAW) 3 October 2002 (2002-10-03)<br>* claims 7-10 *<br>----- | 1-12 | |
| Y | ROLLENCE M L ET AL: "ENGINEERING THERMOSTABILITY IN SUBTILISIN BPN' IN VITRO MUTAGENESIS",<br>CRC CRITICAL REVIEWS IN BIOTECHNOLOGY, CRC PRESS, BOCA RATON, FL, US,<br>vol. 8, no. 3, 1 January 1988 (1988-01-01), pages 217-224, XP000603226,<br>ISSN: 0738-8551<br>* the whole document *<br>----- | 1-12 | TECHNICAL FIELDS SEARCHED (IPC)<br>C12N<br>C11D |
| Y | WO 03/062381 A (GENENCOR INT [US]; BOTT RICHARD R [US]; ESTELL DAVID A [US]; KELLIS JR) 31 July 2003 (2003-07-31)<br>* the whole document *<br>----- | 1-12 | |
| Y | US 6 312 936 B1 (POULOSE AYROOKARAN J [US] ET AL) 6 November 2001 (2001-11-06)<br>* the whole document *<br>----- | 1-12 | |

The present search report has been drawn up for all claims

1

| Place of search | Date of completion of the search | Examiner |
|---|---|---|
| Munich | 1 March 2013 | Offermann, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 18 3920

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2013

| Patent document cited in search report | Publication date | Patent family member(s) | Publication date |
|---|---|---|---|
| WO 02077187 A2 | 03-10-2002 | AT 527276 T | 15-10-2011 |
| | | AU 2002254374 A1 | 08-10-2002 |
| | | CA 2441595 A1 | 03-10-2002 |
| | | CN 1512998 A | 14-07-2004 |
| | | DK 1373296 T3 | 09-01-2012 |
| | | EP 1373296 A2 | 02-01-2004 |
| | | HK 1061566 A1 | 10-08-2012 |
| | | PL 364138 A1 | 13-12-2004 |
| | | RU 2311458 C2 | 27-11-2007 |
| | | US 2003118605 A1 | 26-06-2003 |
| | | US 2005202551 A1 | 15-09-2005 |
| | | US 2005202552 A1 | 15-09-2005 |
| | | WO 02077187 A2 | 03-10-2002 |
| WO 03062381 A | 31-07-2003 | AT 450606 T | 15-12-2009 |
| | | AU 2003210552 A1 | 02-09-2003 |
| | | BR 0306955 A | 19-12-2006 |
| | | CA 2472725 A1 | 31-07-2003 |
| | | DK 1523553 T3 | 19-04-2010 |
| | | EP 1523553 A2 | 20-04-2005 |
| | | ES 2336092 T3 | 08-04-2010 |
| | | JP 4703113 B2 | 15-06-2011 |
| | | JP 2005519592 A | 07-07-2005 |
| | | JP 2011041571 A | 03-03-2011 |
| | | MX PA04006809 A | 11-10-2004 |
| | | PT 1523553 E | 03-03-2010 |
| | | US 2005221461 A1 | 06-10-2005 |
| | | US 2008124783 A1 | 29-05-2008 |
| | | US 2008176313 A1 | 24-07-2008 |
| | | US 2009011489 A1 | 08-01-2009 |
| | | US 2011086412 A1 | 14-04-2011 |
| | | US 2011091958 A1 | 21-04-2011 |
| | | US 2011091959 A1 | 21-04-2011 |
| | | WO 03062381 A2 | 31-07-2003 |
| US 6312936 B1 | 06-11-2001 | AR 013718 A1 | 10-01-2001 |
| | | AR 013719 A1 | 10-01-2001 |
| | | AR 015977 A1 | 30-05-2001 |
| | | AR 016969 A1 | 01-08-2001 |
| | | AR 052281 A2 | 07-03-2007 |
| | | AR 052282 A2 | 07-03-2007 |
| | | AR 052353 A2 | 14-03-2007 |
| | | AR 052354 A2 | 14-03-2007 |
| | | AR 052355 A2 | 14-03-2007 |
| | | AR 052453 A2 | 21-03-2007 |
| | | AT 292179 T | 15-04-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 18 3920

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2013

| Patent document cited in search report | Publication date | Patent family member(s) | Publication date |
|---|---|---|---|
| | | AT 314478 T | 15-01-2006 |
| | | AT 384799 T | 15-02-2008 |
| | | AT 399469 T | 15-07-2008 |
| | | AT 416624 T | 15-12-2008 |
| | | AT 417099 T | 15-12-2008 |
| | | AT 445002 T | 15-10-2009 |
| | | AT 511540 T | 15-06-2011 |
| | | AT 513479 T | 15-07-2011 |
| | | AU 742573 B2 | 10-01-2002 |
| | | AU 742632 B2 | 10-01-2002 |
| | | AU 742694 B2 | 10-01-2002 |
| | | AU 758371 B2 | 20-03-2003 |
| | | AU 1199699 A | 10-05-1999 |
| | | AU 1276299 A | 10-05-1999 |
| | | AU 8733701 A | 03-01-2002 |
| | | BR 9813115 A | 15-08-2000 |
| | | BR 9813260 A | 22-08-2000 |
| | | BR 9813266 A | 20-11-2001 |
| | | BR 9813879 A | 26-09-2000 |
| | | BR 9814097 A | 03-10-2000 |
| | | BR 9815230 A | 02-10-2001 |
| | | CA 2306794 A1 | 29-04-1999 |
| | | CA 2306894 A1 | 29-04-1999 |
| | | CA 2307638 A1 | 29-04-1999 |
| | | CA 2307640 A1 | 29-04-1999 |
| | | CA 2308113 A1 | 29-04-1999 |
| | | CA 2308206 A1 | 29-04-1999 |
| | | CA 2618389 A1 | 29-04-1999 |
| | | CA 2672492 A1 | 29-04-1999 |
| | | CA 2672500 A1 | 29-04-1999 |
| | | CN 1279720 A | 10-01-2001 |
| | | CN 1279721 A | 10-01-2001 |
| | | CN 1286723 A | 07-03-2001 |
| | | CN 1286724 A | 07-03-2001 |
| | | CN 1306577 A | 01-08-2001 |
| | | CN 1327476 A | 19-12-2001 |
| | | CN 1597898 A | 23-03-2005 |
| | | CN 1597899 A | 23-03-2005 |
| | | CN 102021159 A | 20-04-2011 |
| | | CZ 20001477 A3 | 12-12-2001 |
| | | CZ 20001478 A3 | 12-12-2001 |
| | | CZ 20001479 A3 | 16-01-2002 |
| | | CZ 20001493 A3 | 14-11-2001 |
| | | CZ 20001503 A3 | 13-12-2000 |
| | | CZ 20001504 A3 | 14-03-2001 |
| | | DE 69829577 D1 | 04-05-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 18 3920

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2013

| Patent document cited in search report | Publication date | Patent family member(s) | Publication date |
|---|---|---|---|
| | | DE 69829577 T2 | 02-02-2006 |
| | | DE 69833015 T2 | 10-08-2006 |
| | | DE 69839057 T2 | 12-03-2009 |
| | | DK 1025194 T3 | 06-04-2009 |
| | | DK 1025239 T3 | 02-06-2008 |
| | | DK 1025240 T3 | 22-05-2006 |
| | | DK 1025241 T3 | 08-08-2005 |
| | | DK 1027418 T3 | 10-11-2008 |
| | | DK 1398367 T3 | 06-04-2009 |
| | | DK 1571199 T3 | 26-09-2011 |
| | | DK 1612271 T3 | 12-09-2011 |
| | | DK 1624050 T3 | 15-02-2010 |
| | | EG 21711 A | 27-02-2002 |
| | | EG 22075 A | 31-07-2002 |
| | | EP 1025194 A1 | 09-08-2000 |
| | | EP 1025239 A2 | 09-08-2000 |
| | | EP 1025240 A2 | 09-08-2000 |
| | | EP 1025241 A2 | 09-08-2000 |
| | | EP 1027418 A1 | 16-08-2000 |
| | | EP 1082404 A2 | 14-03-2001 |
| | | EP 1398367 A1 | 17-03-2004 |
| | | EP 1571199 A2 | 07-09-2005 |
| | | EP 1612271 A2 | 04-01-2006 |
| | | EP 1624050 A2 | 08-02-2006 |
| | | ES 2241180 T3 | 16-10-2005 |
| | | ES 2255189 T3 | 16-06-2006 |
| | | ES 2301214 T3 | 16-06-2008 |
| | | ES 2310014 T3 | 16-12-2008 |
| | | ES 2319401 T3 | 07-05-2009 |
| | | ES 2319470 T3 | 07-05-2009 |
| | | ES 2332915 T3 | 15-02-2010 |
| | | HK 1039159 A1 | 09-05-2008 |
| | | HU 0100859 A2 | 28-06-2001 |
| | | HU 0103080 A2 | 28-12-2001 |
| | | HU 0104539 A2 | 29-04-2002 |
| | | ID 25637 A | 19-10-2000 |
| | | ID 25897 A | 09-11-2000 |
| | | ID 26501 A | 11-01-2001 |
| | | ID 28256 A | 10-05-2001 |
| | | JP 4346237 B2 | 21-10-2009 |
| | | JP 4511025 B2 | 28-07-2010 |
| | | JP 2001520044 A | 30-10-2001 |
| | | JP 2001520045 A | 30-10-2001 |
| | | JP 2001520046 A | 30-10-2001 |
| | | JP 2001520305 A | 30-10-2001 |
| | | JP 2001520307 A | 30-10-2001 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 18 3920

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2013

| Patent document cited in search report | Publication date | Patent family member(s) | Publication date |
|---|---|---|---|
| | | JP 2001520308 A | 30-10-2001 |
| | | JP 2012183078 A | 27-09-2012 |
| | | MA 24811 A1 | 31-12-1999 |
| | | MA 25044 A1 | 01-10-2000 |
| | | NO 20001899 A | 19-06-2000 |
| | | NO 20001900 A | 15-06-2000 |
| | | NO 20001901 A | 19-06-2000 |
| | | NZ 519204 A | 27-02-2004 |
| | | NZ 520770 A | 28-05-2004 |
| | | NZ 520771 A | 25-06-2004 |
| | | PL 340600 A1 | 12-02-2001 |
| | | PT 1025194 E | 17-03-2009 |
| | | PT 1025239 E | 30-04-2008 |
| | | PT 1025241 E | 31-08-2005 |
| | | PT 1027418 E | 14-10-2008 |
| | | PT 1398367 E | 12-03-2009 |
| | | PT 1571199 E | 27-09-2011 |
| | | PT 1612271 E | 01-09-2011 |
| | | PT 1624050 E | 13-01-2010 |
| | | TR 200001111 T2 | 23-12-2002 |
| | | TR 200001112 T2 | 21-01-2002 |
| | | TR 200001113 T2 | 21-05-2002 |
| | | TR 200002013 T2 | 22-01-2001 |
| | | TR 200002014 T2 | 21-12-2000 |
| | | TR 200002057 T2 | 21-03-2001 |
| | | TR 200101199 T2 | 21-05-2002 |
| | | TR 200101861 T2 | 21-06-2002 |
| | | TW 562859 B | 21-11-2003 |
| | | TW 585912 B | 01-05-2004 |
| | | US 6312936 B1 | 06-11-2001 |
| | | US 6482628 B1 | 19-11-2002 |
| | | US 6673590 B1 | 06-01-2004 |
| | | US 2003073222 A1 | 17-04-2003 |
| | | US 2003119690 A1 | 26-06-2003 |
| | | WO 9920723 A2 | 29-04-1999 |
| | | WO 9920726 A1 | 29-04-1999 |
| | | WO 9920769 A2 | 29-04-1999 |
| | | WO 9920770 A2 | 29-04-1999 |
| | | WO 9920771 A2 | 29-04-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9711151 A
- EP 0922499 A
- US 4977252 A
- US 5354559 A
- US 5935826 A
- US RE34606 E
- US 5955340 A
- US 5700676 A
- US 6312936 B
- US 6482628 B
- WO 8906270 A
- WO 9523221 A
- WO 9221760 A
- US 5801039 A
- US 5340735 A
- US 5500364 A
- US 5855625 A
- EP 258068 A
- EP 305216 A
- EP 238023 A
- EP 214761 A
- EP 218272 A
- EP 331376 A
- GB 1372034 A
- JP 64744992 B
- WO 9116422 A
- WO 8809367 A
- WO 9009446 A
- GB 1296839 A
- US 4435307 A
- EP 0495257 A
- US 5874276 A
- US 6566114 B
- US 6602842 B
- US 6440991 B
- WO 9412621 A
- WO 9501426 A
- WO 05056782 A
- US 6610642 B
- US 6605458 B
- US 5705464 A
- US 5710115 A
- US 5698504 A
- US 5695679 A
- US 5686014 A
- US 5646101 A
- US 6376450 B
- US 5879584 A
- US 5691297 A
- US 5574005 A
- US 5569645 A
- US 5565422 A
- US 5516448 A
- US 5489392 A
- US 5486303 A
- US 5576282 A
- US 6306812 B
- US 6326348 B
- US 4430243 A
- US 5597936 A
- US 5595967 A
- WO 00332601 A
- US 6225464 B
- WO 9934011 A
- US 4683195 A
- US 4683202 A
- US 4965188 A
- US 5322770 A
- US 69925000 A
- US 6312936 A
- US 6482628 A
- WO 9707770 A
- WO 0214490 A
- US 576331 A
- US 11581102 A
- US 11583334 A
- US 10581014 A

### Non-patent literature cited in the description

- **PIERCE ; WINFREE.** *Protein Engineer.,* 2002, vol. 15, 779-782
- **ESTELL et al.** *World Biotech Report,* 1984, vol. 2, 181-187
- **WELLS et al.** *Gene,* 1985, vol. 34, 315-323
- **REETZ et al.** *Agnew. Chem. Int. Edn.,* 2005, vol. 44, 4192-4196
- **KATO et al.** *J. Mol. Biol.,* 2005, vol. 351, 683-692
- **SANDBERG et al.** *Proc. Natl. Acad. Sci.,* 1993, vol. 90, 8367-8371

- **BLOOM et al.** *Curr. Opin. Struct. Biol.,* 2005, vol. 15, 447-452
- **BLOOM et al.** *Proc. Natl. Acad. Sci. USA,* 2006, vol. 103, 5869-5874
- **GUO et al.** *Proc. Natl. Acad. Sci. USA,* 2004, vol. 101, 9205-9210
- **DARTOIS et al.** *Biochem. Biophys. Acta,* 1993, vol. 1131, 253-260
- **YAMAGUCHI et al.** *Gene,* 1991, vol. 103, 61-67
- **SCHIMADA et al.** *J. Biochem.,* 1989, vol. 106, 383-388
- **HASS et al.** *Gene,* 1991, vol. 109, 117-113
- **KUGIMIYA et al.** *Biosci. Biotech. Biochem.,* 1992, vol. 56, 716-719
- **SAMBROOK et al.** Molecular Cloning: A Laboratory Manual. Cold Spring Harbor, 1989
- **AUSUBEL et al.** *Current Protocols in Molecular Biology,* 1987
- Microbial Proteinases. **KALISZ.** Advances in Biochemical Engineering/Biotechnology. 1988
- **DEL MAR et al.** *Anal. Biochem.,* 1979, vol. 99, 316-320
- Genetics. **FERRARI et al.** Bacillus. Plenum Publishing Corp, 1989, 57-72
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *Proc. Natl. Acad. Sci. USA,* 1993, vol. 90, 5873-5787
- **ALTSCHUL et al.** *Meth. Enzymol.,* 1996, vol. 266, 460-480
- **DYNAN ; TIJAN.** *Nature,* 1985, vol. 316, 774-78
- **HEINZ et al.** *Biochemistry,* 1992, vol. 31, 8755-66
- **FERRARI et al.** *J Bacteriol,* 1983, vol. 154, 1513-5
- **NEIDHARDT et al.** *J. Bacteriol.,* 1974, vol. 119, 736-747
- **MCKENZIE et al.** *Plasmid,* 1986, vol. 15, 93-103
- **BOLIVAR et al.** *Gene,* 1977, vol. 2, 95-113
- **HORINOUCHI et al.** *J Bacteriol,* 1982, vol. 150, 815-825
- **HORINOUCHI et al.** *J. Bacteriol,* 1982, vol. 150, 815-825

SEQUENCE LISTING

<110> Danisco US Inc.

<120> Compositions and Methods Comprising Variant Microbial Proteases

<130> GRF/FP6850465

<140> Divisional application of 09759359.4
<141> 2009-06-03

<150> 09759359.4
<151> 2009-06-03

<150> PCT/US09/46156
<151> 2009-06-03

<150> US 61/059,695
<151> 2008-06-06

<160> 568

<170> PatentIn version 3.5

<210> 1
<211> 1149
<212> DNA
<213> Bacillus amyloliquefaciens

<400> 1
```
gtgagaagca aaaaattgtg gatcagtttg ctgtttgctt tagcgttaat ctttacgatg      60
gcgttcggca gcacatcctc tgcccaggcg gcagggaaat caaacgggga aaagaaatat     120
attgtcgggt ttaaacagac aatgagcacg atgagcgccg ctaagaagaa agatgtcatt     180
tctgaaaaag gcgggaaagt gcaaaagcaa ttcaaatatg tagacgcagc ttcagctaca     240
ttaaacgaaa aagctgtaaa agaattgaaa aaagacccga gcgtcgctta cgttgaagaa     300
gatcacgtag cacatgcgta cgcgcagtcc gtgccttacg gcgtatcaca aattaaagcc     360
cctgctctgc actctcaagg ctacactgga tcaaatgtta aagtagcggt tatcgacagc     420
ggtatcgatt cttctcatcc tgatttaaag gtagcaggcg gagccagcat ggttccttct     480
gaaacaaatc ctttccaaga caacaactct cacggaactc acgttgccgg cacagttgcg     540
gctcttaata actcaatcgg tgtattaggc gttgcgccaa gcgcatcact ttacgctgta     600
aaagttctcg gtgctgacgg ttccggccaa tacagctgga tcattaacgg aatcgagtgg     660
gcgatcgcaa acaatatgga cgttattaac atgagcctcg gcggaccttc tggttctgct     720
gctttaaaag cggcagttga taaagccgtt gcatccggcg tcgtagtcgt tgcggcagcc     780
ggtaacgaag gcacttccgg cagctcaagc acagtgggct accctggtaa ataccettct     840
gtcattgcag taggcgctgt tgacagcagc aaccaaagag catctttctc aagcgtagga     900
cctgagcttg atgtcatggc acctggcgta tctatccaaa gcacgcttcc tggaaacaaa     960
tacggggcgt acaacggtac gtcaatggca tctccgcacg ttgccggagc ggctgctttg    1020
attctttcta agcacccgaa ctggacaaac actcaagtcc gcagcagttt agaaaacacc    1080
actacaaaac ttggtgattc tttctactat ggaaaagggc tgatcaacgt acaggcggca    1140
gctcagtaa                                                            1149
```

<210> 2
<211> 382
<212> PRT
<213> Bacillus amyloliquefaciens

<400> 2
```
Val Arg Ser Lys Lys Leu Trp Ile Ser Leu Leu Phe Ala Leu Ala Leu
1               5                   10                  15
Ile Phe Thr Met Ala Phe Gly Ser Thr Ser Ser Ala Gln Ala Ala Gly
            20                  25                  30
Lys Ser Asn Gly Glu Lys Lys Tyr Ile Val Gly Phe Lys Gln Thr Met
        35                  40                  45
Ser Thr Met Ser Ala Ala Lys Lys Lys Asp Val Ile Ser Glu Lys Gly
    50                  55                  60
```

```
Gly Lys Val Gln Lys Gln Phe Lys Tyr Val Asp Ala Ala Ser Ala Thr
65                  70                  75                  80
Leu Asn Glu Lys Ala Val Lys Glu Leu Lys Lys Asp Pro Ser Val Ala
                85                  90                  95
Tyr Val Glu Glu Asp His Val Ala His Ala Tyr Ala Gln Ser Val Pro
            100                 105                 110
Tyr Gly Val Ser Gln Ile Lys Ala Pro Ala Leu His Ser Gln Gly Tyr
        115                 120                 125
Thr Gly Ser Asn Val Lys Val Ala Val Ile Asp Ser Gly Ile Asp Ser
    130                 135                 140
Ser His Pro Asp Leu Lys Val Ala Gly Gly Ala Ser Met Val Pro Ser
145                 150                 155                 160
Glu Thr Asn Pro Phe Gln Asp Asn Asn Ser His Gly Thr His Val Ala
                165                 170                 175
Gly Thr Val Ala Ala Leu Asn Asn Ser Ile Gly Val Leu Gly Val Ala
            180                 185                 190
Pro Ser Ala Ser Leu Tyr Ala Val Lys Val Leu Gly Ala Asp Gly Ser
        195                 200                 205
Gly Gln Tyr Ser Trp Ile Ile Asn Gly Ile Glu Trp Ala Ile Ala Asn
    210                 215                 220
Asn Met Asp Val Ile Asn Met Ser Leu Gly Gly Pro Ser Gly Ser Ala
225                 230                 235                 240
Ala Leu Lys Ala Ala Val Asp Lys Ala Val Ala Ser Gly Val Val Val
                245                 250                 255
Val Ala Ala Ala Gly Asn Glu Gly Thr Ser Gly Ser Ser Ser Thr Val
            260                 265                 270
Gly Tyr Pro Gly Lys Tyr Pro Ser Val Ile Ala Val Gly Ala Val Asp
        275                 280                 285
Ser Ser Asn Gln Arg Ala Ser Phe Ser Ser Val Gly Pro Glu Leu Asp
    290                 295                 300
Val Met Ala Pro Gly Val Ser Ile Gln Ser Thr Leu Pro Gly Asn Lys
305                 310                 315                 320
Tyr Gly Ala Tyr Asn Gly Thr Ser Met Ala Ser Pro His Val Ala Gly
                325                 330                 335
Ala Ala Ala Leu Ile Leu Ser Lys His Pro Asn Trp Thr Asn Thr Gln
            340                 345                 350
Val Arg Ser Ser Leu Glu Asn Thr Thr Thr Lys Leu Gly Asp Ser Phe
        355                 360                 365
Tyr Tyr Gly Lys Gly Leu Ile Asn Val Gln Ala Ala Ala Gln
    370                 375                 380
```

```
<210>  3
<211>  2013
<212>  DNA
<213>  Artificial

<220>
<223>  synthetic sequence

<400>  3
gaattcctcc atttcttct gctatcaaaa taacagactc gtgattttcc aaacgagctt      60
tcaaaaaagc ctctgcccct tgcaaatcgg atgcctgtct ataaaattcc cgatattggc    120
ttaaacagcg gcgcaatggc ggccgcatct gatgtctttg cttggcgaat gttcatctta    180
tttcttcctc cctctcaata atttttcat tctatccctt ttctgtaaag tttattttc     240
agaatacttt tatcatcatg ctttgaaaaa atatcacgat aatatccatt gttctcacgg    300
aagcacacgc aggtcatttg aacgaatttt ttcgacagga atttgccggg actcaggagc    360
atttaaccta aaaaagcatg acatttcagc ataatgaaca tttactcatg tctattttcg    420
ttcttttctg tatgaaaata gttatttcga gtctctacgg aaatagcgag agatgatata    480
cctaaataga gataaaatca tctcaaaaaa atgggtctac taaaatatta ttccatctat    540
tacaataaat tcacagaata gtcttttaag taagtctact ctgaattttt ttaaaaggag    600
agggtaaaga gtgagaagca aaaaattgtg gatcagtttg ctgtttgctt tagcgttaat    660
ctttacgatg gcgttcggca gcacatcctc tgcccaggcg gcagggaaat caaacgggga    720
aaagaaatat attgtcgggt ttaaacagac aatgagcacg atgagcgccg ctaagaagaa    780
agatgtcatt tctgaaaaag gcgggaaagt gcaaaagcaa ttcaaatatg tagacgcagc    840
ttcagctaca ttaaacgaaa aagctgtaaa agaattgaaa aaagacccga gcgtcgctta    900
```

cgttgaagaa gatcacgtag cacatgcgta cgcgcagtcc gtgccttacg gcgtatcaca 960
aattaaagcc cctgctctgc actctcaagg ctacactgga tcaaatgtta aagtagcggt 1020
tatcgacagc ggtatcgatt cttctcatcc tgatttaaag gtagcaggcg gagccagcat 1080
ggttccttct gaaacaaatc ctttccaaga caacaactct cacggaactc acgttgccgg 1140
cacagttgcg gctcttaata actcaatcgg tgtattaggc gttgcgccaa gcgcatcact 1200
ttacgctgta aaagttctcg gtgctgacgg ttccggccaa tacagctgga tcattaacgg 1260
aatcgagtgg gcgatcgcaa acaatatgga cgttattaac atgagcctcg gcggaccttc 1320
tggttctgct gctttaaaag cggcagttga taaagccgtt gcatccggcg tcgtagtcgt 1380
tgcggcagcc ggtaacgaag gcacttccgg cagctcaagc acagtgggct accctggtaa 1440
atacccttct gtcattgcag taggcgctgt tgacagcagc aaccaaagag catctttctc 1500
aagcgtagga cctgagcttg atgtcatggc acctggcgta tctatccaaa gcacgcttcc 1560
tggaaacaaa tacggggcgt acaacggtac gtcaatggca tctccgcacg ttgccggagc 1620
ggctgctttg attctttcta agcacccgaa ctggacaaac actcaagtcc gcagcagttt 1680
agaaaacacc actacaaaac ttggtgattc tttctactat ggaaaagggc tgatcaacgt 1740
acaggcggca gctcagtaaa acataaaaaa ccggccttgg ccccgccggt tttttattat 1800
ttttcttcct ccgcatgttc aatccgctcc ataatcgacg gatggctccc tctgaaaatt 1860
ttaacgagaa acggcgggtt gacccggctc agtcccgtaa cggccaagtc ctgaaacgtc 1920
tcaatcgccg cttcccggtt tccggtcagc tcaatgccgt aacggtcggc ggcgttttcc 1980
tgataccggg agacggcatt cgtaatcgga tcc 2013

<210> 4
<211> 34
<212> DNA
<213> Artificial

<220>
<223> synthetic primer

<400> 4
gtcctctgtt aacttactga gctgccgcct gtac 34

<210> 5
<211> 42
<212> DNA
<213> Artificial

<220>
<223> synthetic primer

<400> 5
ttatgcgagg ctagcaaaag gagagggtaa agagtgagaa gc 42

<210> 6
<211> 29
<212> DNA
<213> Artificial

<220>
<223> synthetic primer

<400> 6
gcaatcagat cttccttcag gttatgacc 29

<210> 7
<211> 30
<212> DNA
<213> Artificial

<220>
<223> synthetic primer

<400> 7
gcatcgaaga tctgattgct taactgcttc 30

<210> 8

<211> 39
<212> DNA
<213> Artificial

<220>
<223> synthetic primer

<220>
<221> misc_feature
<222> (19)..(20)
<223> n is a, c, g, or t

<400> 8
cacgtagcac atgcatacnn scagtccgtg ccttacggc 39

<210> 9
<211> 39
<212> DNA
<213> Artificial

<220>
<223> synthetic primer

<220>
<221> misc_feature
<222> (19)..(20)
<223> n is a, c, g, or t

<400> 9
gtagcacatg catacgcgnn stccgtgcct tacggcgta 39

<210> 10
<211> 39
<212> DNA
<213> Artificial

<220>
<223> synthetic primer

<220>
<221> misc_feature
<222> (19)..(20)
<223> n is a, c, g, or t

<400> 10
gcacatgcat acgcgcagnn sgtgccttac ggcgtatca 39

<210> 11
<211> 39
<212> DNA
<213> Artificial

<220>
<223> synthetic primer

<220>
<221> misc_feature
<222> (19)..(20)
<223> n is a, c, g, or t

<400> 11
catgcatacg cgcagtccnn sccttacggc gtatcacaa 39

<210> 12
<211> 39

<212> DNA
<213> Artificial

<220>
<223> synthetic primer

<220>
<221> misc_feature
<222> (19)..(20)
<223> n is a, c, g, or t

<400> 12
gcatacgcgc agtccgtgnn stacggcgta tcacaaatt 39

<210> 13
<211> 39
<212> DNA
<213> Artificial

<220>
<223> synthetic primer

<220>
<221> misc_feature
<222> (19)..(20)
<223> n is a, c, g, or t

<400> 13
tacgcgcagt ccgtgcctnn sggcgtatca caaattaaa 39

<210> 14
<211> 39
<212> DNA
<213> Artificial

<220>
<223> synthetic primer

<220>
<221> misc_feature
<222> (19)..(20)
<223> n is a, c, g, or t

<400> 14
gcgcagtccg tgccttacnn sgtatcacaa attaaagcc 39

<210> 15
<211> 39
<212> DNA
<213> Artificial

<220>
<223> synthetic primer

<220>
<221> misc_feature
<222> (19)..(20)
<223> n is a, c, g, or t

<400> 15
cagtccgtgc cttacggcnn stcacaaatt aaagcccct 39

<210> 16
<211> 39
<212> DNA

<213> Artificial

<220>
<223> synthetic primer

<220>
<221> misc_feature
<222> (19)..(20)
<223> n is a, c, g, or t

<400> 16
tccgtgcctt acggcgtann scaaattaaa gcccctgct 39

<210> 17
<211> 39
<212> DNA
<213> Artificial

<220>
<223> synthetic primer

<220>
<221> misc_feature
<222> (19)..(20)
<223> n is a, c, g, or t

<400> 17
gtgccttacg gcgtatcann sattaaagcc cctgctctg 39

<210> 18
<211> 39
<212> DNA
<213> Artificial

<220>
<223> synthetic primer

<220>
<221> misc_feature
<222> (19)..(20)
<223> n is a, c, g, or t

<400> 18
ccttacggcg tatcacaann saaagcccct gctctgcac 39

<210> 19
<211> 39
<212> DNA
<213> Artificial

<220>
<223> synthetic primer

<220>
<221> misc_feature
<222> (19)..(20)
<223> n is a, c, g, or t

<400> 19
tacggcgtat cacaaattnn sgcccctgct ctgcactct 39

<210> 20
<211> 39
<212> DNA
<213> Artificial

<220>
<223> synthetic primer

<220>
<221> misc_feature
<222> (19)..(20)
<223> n is a, c, g, or t

<400> 20
ggcgtatcac aaattaaann scctgctctg cactctcaa 39

<210> 21
<211> 39
<212> DNA
<213> Artificial

<220>
<223> synthetic primer

<220>
<221> misc_feature
<222> (19)..(20)
<223> n is a, c, g, or t

<400> 21
gtatcacaaa ttaaagccnn sgctctgcac tctcaaggc 39

<210> 22
<211> 39
<212> DNA
<213> Artificial

<220>
<223> synthetic primer

<220>
<221> misc_feature
<222> (19)..(20)
<223> n is a, c, g, or t

<400> 22
tcacaaatta aagcccctnn sctgcactct caaggctac 39

<210> 23
<211> 39
<212> DNA
<213> Artificial

<220>
<223> synthetic primer

<220>
<221> misc_feature
<222> (19)..(20)
<223> n is a, c, g, or t

<400> 23
caaattaaag cccctgctnn scactctcaa ggctacact 39

<210> 24
<211> 39
<212> DNA
<213> Artificial

<220>
<223> synthetic primer

<220>
<221> misc_feature
<222> (19)..(20)
<223> n is a, c, g, or t

<400> 24
attaaagccc ctgctctgnn stctcaaggc tacactgga 39

<210> 25
<211> 39
<212> DNA
<213> Artificial

<220>
<223> synthetic primer

<220>
<221> misc_feature
<222> (19)..(20)
<223> n is a, c, g, or t

<400> 25
aaagcccctg ctctgcacnn scaaggctac actggatca 39

<210> 26
<211> 39
<212> DNA
<213> Artificial

<220>
<223> synthetic primer

<220>
<221> misc_feature
<222> (19)..(20)
<223> n is a, c, g, or t

<400> 26
gcccctgctc tgcactctnn sggctacact ggatcaaat 39

<210> 27
<211> 39
<212> DNA
<213> Artificial

<220>
<223> synthetic primer

<220>
<221> misc_feature
<222> (19)..(20)
<223> n is a, c, g, or t

<400> 27
cctgctctgc actctcaann stacactgga tcaaatgtt 39

<210> 28
<211> 39
<212> DNA
<213> Artificial

<220>

<223> synthetic primer

<220>
<221> misc_feature
<222> (19)..(20)
<223> n is a, c, g, or t

<400> 28
gctctgcact ctcaaggcnn sactggatca aatgttaaa 39

<210> 29
<211> 39
<212> DNA
<213> Artificial

<220>
<223> synthetic primer

<220>
<221> misc_feature
<222> (19)..(20)
<223> n is a, c, g, or t

<400> 29
ctgcactctc aaggctacnn sggatcaaat gttaaagta 39

<210> 30
<211> 39
<212> DNA
<213> Artificial

<220>
<223> synthetic primer

<220>
<221> misc_feature
<222> (19)..(20)
<223> n is a, c, g, or t

<400> 30
cactctcaag gctacactnn stcaaatgtt aaagtagcg 39

<210> 31
<211> 39
<212> DNA
<213> Artificial

<220>
<223> synthetic primer

<220>
<221> misc_feature
<222> (19)..(20)
<223> n is a, c, g, or t

<400> 31
tctcaaggct acactggann saatgttaaa gtagcggtt 39

<210> 32
<211> 39
<212> DNA
<213> Artificial

<220>
<223> synthetic primer

<220>
<221> misc_feature
<222> (19)..(20)
<223> n is a, c, g, or t

<400> 32
caaggctaca ctggatcann sgttaaagta gcggttatc 39

<210> 33
<211> 39
<212> DNA
<213> Artificial

<220>
<223> synthetic primer

<220>
<221> misc_feature
<222> (19)..(20)
<223> n is a, c, g, or t

<400> 33
ggctacactg gatcaaatnn saaagtagcg gttatcgac 39

<210> 34
<211> 39
<212> DNA
<213> Artificial

<220>
<223> synthetic primer

<220>
<221> misc_feature
<222> (19)..(20)
<223> n is a, c, g, or t

<400> 34
tacactggat caaatgttnn sgtagcggtt atcgacagc 39

<210> 35
<211> 39
<212> DNA
<213> Artificial

<220>
<223> synthetic primer

<220>
<221> misc_feature
<222> (19)..(20)
<223> n is a, c, g, or t

<400> 35
actggatcaa atgttaaann sgcggttatc gacagcggt 39

<210> 36
<211> 39
<212> DNA
<213> Artificial

<220>
<223> synthetic primer

<220>
<221> misc_feature
<222> (19)..(20)
<223> n is a, c, g, or t

<400> 36
ggatcaaatg ttaaagtann sgttatcgac agcggtatc 39

<210> 37
<211> 39
<212> DNA
<213> Artificial

<220>
<223> synthetic primer

<220>
<221> misc_feature
<222> (19)..(20)
<223> n is a, c, g, or t

<400> 37
tcaaatgtta aagtagcgnn satcgacagc ggtatcgat 39

<210> 38
<211> 39
<212> DNA
<213> Artificial

<220>
<223> synthetic primer

<220>
<221> misc_feature
<222> (19)..(20)
<223> n is a, c, g, or t

<400> 38
aatgttaaag tagcggttnn sgacagcggt atcgattct 39

<210> 39
<211> 39
<212> DNA
<213> Artificial

<220>
<223> synthetic primer

<220>
<221> misc_feature
<222> (19)..(20)
<223> n is a, c, g, or t

<400> 39
gttaaagtag cggttatcnn sagcggtatc gattcttct 39

<210> 40
<211> 39
<212> DNA
<213> Artificial

<220>
<223> synthetic primer

<220>

<221> misc_feature
<222> (19)..(20)
<223> n is a, c, g, or t

<400> 40
aaagtagcgg ttatcgacnn sggtatcgat tcttctcat 39

<210> 41
<211> 39
<212> DNA
<213> Artificial

<220>
<223> synthetic primer

<220>
<221> misc_feature
<222> (19)..(20)
<223> n is a, c, g, or t

<400> 41
gtagcggtta tcgacagcnn satcgattct tctcatcct 39

<210> 42
<211> 39
<212> DNA
<213> Artificial

<220>
<223> synthetic primer

<220>
<221> misc_feature
<222> (19)..(20)
<223> n is a, c, g, or t

<400> 42
gcggttatcg acagcggtnn sgattcttct catcctgat 39

<210> 43
<211> 39
<212> DNA
<213> Artificial

<220>
<223> synthetic primer

<220>
<221> misc_feature
<222> (19)..(20)
<223> n is a, c, g, or t

<400> 43
gttatcgaca gcggtatcnn stcttctcat cctgattta 39

<210> 44
<211> 39
<212> DNA
<213> Artificial

<220>
<223> synthetic primer

<220>
<221> misc_feature

<222> (19)..(20)
<223> n is a, c, g, or t

<400> 44
atcgacagcg gtatcgatnn stctcatcct gatttaaag 39

<210> 45
<211> 39
<212> DNA
<213> Artificial

<220>
<223> synthetic primer

<220>
<221> misc_feature
<222> (19)..(20)
<223> n is a, c, g, or t

<400> 45
gacagcggta tcgattctnn scatcctgat ttaaaggta 39

<210> 46
<211> 39
<212> DNA
<213> Artificial

<220>
<223> synthetic primer

<220>
<221> misc_feature
<222> (19)..(20)
<223> n is a, c, g, or t

<400> 46
agcggtatcg attcttctnn scctgattta aaggtagca 39

<210> 47
<211> 39
<212> DNA
<213> Artificial

<220>
<223> synthetic primer

<220>
<221> misc_feature
<222> (19)..(20)
<223> n is a, c, g, or t

<400> 47
ggtatcgatt cttctcatnn sgatttaaag gtagcaggc 39

<210> 48
<211> 39
<212> DNA
<213> Artificial

<220>
<223> synthetic primer

<220>
<221> misc_feature
<222> (19)..(20)

<223> n is a, c, g, or t

<400> 48
atcgattctt ctcatcctnn sttaaaggta gcaggcgga 39

<210> 49
<211> 39
<212> DNA
<213> Artificial

<220>
<223> synthetic primer

<220>
<221> misc_feature
<222> (19)..(20)
<223> n is a, c, g, or t

<400> 49
gattcttctc atcctgatnn saaggtagca ggcggagcc 39

<210> 50
<211> 39
<212> DNA
<213> Artificial

<220>
<223> synthetic primer

<220>
<221> misc_feature
<222> (19)..(20)
<223> n is a, c, g, or t

<400> 50
tcttctcatc ctgatttann sgtagcaggc ggagccagc 39

<210> 51
<211> 39
<212> DNA
<213> Artificial

<220>
<223> synthetic primer

<220>
<221> misc_feature
<222> (19)..(20)
<223> n is a, c, g, or t

<400> 51
tctcatcctg atttaaagnn sgcaggcgga gccagcatg 39

<210> 52
<211> 39
<212> DNA
<213> Artificial

<220>
<223> synthetic primer

<220>
<221> misc_feature
<222> (19)..(20)
<223> n is a, c, g, or t

<400> 52
catcctgatt taaaggtann sggcggagcc agcatggtt 39

<210> 53
<211> 39
<212> DNA
<213> Artificial

<220>
<223> synthetic primer

<220>
<221> misc_feature
<222> (19)..(20)
<223> n is a, c, g, or t

<400> 53
cctgatttaa aggtagcann sggagccagc atggttcct 39

<210> 54
<211> 39
<212> DNA
<213> Artificial

<220>
<223> synthetic primer

<220>
<221> misc_feature
<222> (19)..(20)
<223> n is a, c, g, or t

<400> 54
gatttaaagg tagcaggcnn sgccagcatg gttccttct 39

<210> 55
<211> 39
<212> DNA
<213> Artificial

<220>
<223> synthetic primer

<220>
<221> misc_feature
<222> (19)..(20)
<223> n is a, c, g, or t

<400> 55
ttaaaggtag caggcggann sagcatggtt ccttctgaa 39

<210> 56
<211> 39
<212> DNA
<213> Artificial

<220>
<223> synthetic primer

<220>
<221> misc_feature
<222> (19)..(20)
<223> n is a, c, g, or t

<400> 56
aaggtagcag gcggagccnn satggttcct tctgaaaca 39

<210> 57
<211> 39
<212> DNA
<213> Artificial

<220>
<223> synthetic primer

<220>
<221> misc_feature
<222> (19)..(20)
<223> n is a, c, g, or t

<400> 57
gtagcaggcg gagccagcnn sgttccttct gaaacaaat 39

<210> 58
<211> 39
<212> DNA
<213> Artificial

<220>
<223> synthetic primer

<220>
<221> misc_feature
<222> (19)..(20)
<223> n is a, c, g, or t

<400> 58
gcaggcggag ccagcatgnn sccttctgaa acaaatcct 39

<210> 59
<211> 39
<212> DNA
<213> Artificial

<220>
<223> synthetic primer

<220>
<221> misc_feature
<222> (19)..(20)
<223> n is a, c, g, or t

<400> 59
ggcggagcca gcatggttnn stctgaaaca aatcctttc 39

<210> 60
<211> 39
<212> DNA
<213> Artificial

<220>
<223> synthetic primer

<220>
<221> misc_feature
<222> (19)..(20)
<223> n is a, c, g, or t

<400> 60

ggagccagca tggttcctnn sgaaacaaat cctttccaa 39

<210> 61
<211> 39
<212> DNA
<213> Artificial

<220>
<223> synthetic primer

<220>
<221> misc_feature
<222> (19)..(20)
<223> n is a, c, g, or t

<400> 61
gccagcatgg ttccttctnn sacaaatcct ttccaagac 39

<210> 62
<211> 39
<212> DNA
<213> Artificial

<220>
<223> synthetic primer

<220>
<221> misc_feature
<222> (19)..(20)
<223> n is a, c, g, or t

<400> 62
agcatggttc cttctgaann saatcctttc caagacaac 39

<210> 63
<211> 39
<212> DNA
<213> Artificial

<220>
<223> synthetic primer

<220>
<221> misc_feature
<222> (19)..(20)
<223> n is a, c, g, or t

<400> 63
atggttcctt ctgaaacann scctttccaa gacaacaac 39

<210> 64
<211> 39
<212> DNA
<213> Artificial

<220>
<223> synthetic primer

<220>
<221> misc_feature
<222> (19)..(20)
<223> n is a, c, g, or t

<400> 64
gttccttctg aaacaaatnn sttccaagac aacaactct 39

<210> 65
<211> 39
<212> DNA
<213> Artificial

<220>
<223> synthetic primer

<220>
<221> misc_feature
<222> (19)..(20)
<223> n is a, c, g, or t

<400> 65
cottctgaaa caaatcctnn scaagacaac aactctcac 39

<210> 66
<211> 39
<212> DNA
<213> Artificial

<220>
<223> synthetic primer

<220>
<221> misc_feature
<222> (19)..(20)
<223> n is a, c, g, or t

<400> 66
tctgaaacaa atcctttcnn sgacaacaac tctcacgga 39

<210> 67
<211> 39
<212> DNA
<213> Artificial

<220>
<223> synthetic primer

<220>
<221> misc_feature
<222> (19)..(20)
<223> n is a, c, g, or t

<400> 67
gaaacaaatc ctttccaann saacaactct cacggaact 39

<210> 68
<211> 39
<212> DNA
<213> Artificial

<220>
<223> synthetic primer

<220>
<221> misc_feature
<222> (19)..(20)
<223> n is a, c, g, or t

<400> 68
acaaatcctt tccaagacnn saactctcac ggaactcac 39

<210> 69
<211> 39
<212> DNA
<213> Artificial

<220>
<223> synthetic primer

<220>
<221> misc_feature
<222> (19)..(20)
<223> n is a, c, g, or t

<400> 69
aatcctttcc aagacaacnn stctcacgga actcacgtt 39

<210> 70
<211> 39
<212> DNA
<213> Artificial

<220>
<223> synthetic primer

<220>
<221> misc_feature
<222> (19)..(20)
<223> n is a, c, g, or t

<400> 70
cctttccaag acaacaacnn scacggaact cacgttgcc 39

<210> 71
<211> 39
<212> DNA
<213> Artificial

<220>
<223> synthetic primer

<220>
<221> misc_feature
<222> (19)..(20)
<223> n is a, c, g, or t

<400> 71
ttccaagaca acaactctnn sggaactcac gttgccggc 39

<210> 72
<211> 39
<212> DNA
<213> Artificial

<220>
<223> synthetic primer

<220>
<221> misc_feature
<222> (19)..(20)
<223> n is a, c, g, or t

<400> 72
caagacaaca actctcacnn sactcacgtt gccggcaca 39

<210> 73

<211> 39
<212> DNA
<213> Artificial

<220>
<223> synthetic primer

<220>
<221> misc_feature
<222> (19)..(20)
<223> n is a, c, g, or t

<400> 73
gacaacaact ctcacggann scacgttgcc ggcacagtt 39

<210> 74
<211> 39
<212> DNA
<213> Artificial

<220>
<223> synthetic primer

<220>
<221> misc_feature
<222> (19)..(20)
<223> n is a, c, g, or t

<400> 74
aacaactctc acggaactnn sgttgccggc acagttgcg 39

<210> 75
<211> 39
<212> DNA
<213> Artificial

<220>
<223> synthetic primer

<220>
<221> misc_feature
<222> (19)..(20)
<223> n is a, c, g, or t

<400> 75
aactctcacg gaactcacnn sgccggcaca gttgcggct 39

<210> 76
<211> 39
<212> DNA
<213> Artificial

<220>
<223> synthetic primer

<220>
<221> misc_feature
<222> (19)..(20)
<223> n is a, c, g, or t

<400> 76
tctcacggaa ctcacgttnn sggcacagtt gcggctctt 39

<210> 77
<211> 39

<212> DNA
<213> Artificial

<220>
<223> synthetic primer

<220>
<221> misc_feature
<222> (19)..(20)
<223> n is a, c, g, or t

<400> 77
cacggaactc acgttgccnn sacagttgcg gctcttaat 39

<210> 78
<211> 39
<212> DNA
<213> Artificial

<220>
<223> synthetic primer

<220>
<221> misc_feature
<222> (19)..(20)
<223> n is a, c, g, or t

<400> 78
ggaactcacg ttgccggcnn sgttgcggct cttaataac 39

<210> 79
<211> 39
<212> DNA
<213> Artificial

<220>
<223> synthetic primer

<220>
<221> misc_feature
<222> (19)..(20)
<223> n is a, c, g, or t

<400> 79
actcacgttg ccggcacann sgcggctctt aataactca 39

<210> 80
<211> 39
<212> DNA
<213> Artificial

<220>
<223> synthetic primer

<220>
<221> misc_feature
<222> (19)..(20)
<223> n is a, c, g, or t

<400> 80
cacgttgccg gcacagttnn sgctcttaat aactcaatc 39

<210> 81
<211> 39
<212> DNA

<213> Artificial

<220>
<223> synthetic primer

<220>
<221> misc_feature
<222> (19)..(20)
<223> n is a, c, g, or t

<400> 81
gttgccggca cagttgcgnn scttaataac tcaatcggt 39

<210> 82
<211> 39
<212> DNA
<213> Artificial

<220>
<223> synthetic primer

<220>
<221> misc_feature
<222> (19)..(20)
<223> n is a, c, g, or t

<400> 82
gccggcacag ttgcggctnn saataactca atcggtgta 39

<210> 83
<211> 39
<212> DNA
<213> Artificial

<220>
<223> synthetic primer

<220>
<221> misc_feature
<222> (19)..(20)
<223> n is a, c, g, or t

<400> 83
ggcacagttg cggctcttnn saactcaatc ggtgtatta 39

<210> 84
<211> 39
<212> DNA
<213> Artificial

<220>
<223> synthetic primer

<220>
<221> misc_feature
<222> (19)..(20)
<223> n is a, c, g, or t

<400> 84
acagttgcgg ctcttaatnn stcaatcggt gtattaggc 39

<210> 85
<211> 39
<212> DNA
<213> Artificial

<220>
<223> synthetic primer

<220>
<221> misc_feature
<222> (19)..(20)
<223> n is a, c, g, or t

<400> 85
gttgcggctc ttaataacnn satcggtgta ttaggcgtt 39

<210> 86
<211> 39
<212> DNA
<213> Artificial

<220>
<223> synthetic primer

<220>
<221> misc_feature
<222> (19)..(20)
<223> n is a, c, g, or t

<400> 86
gcggctctta ataactcann sggtgtatta ggcgttgcg 39

<210> 87
<211> 39
<212> DNA
<213> Artificial

<220>
<223> synthetic primer

<220>
<221> misc_feature
<222> (19)..(20)
<223> n is a, c, g, or t

<400> 87
gctcttaata actcaatcnn sgtattaggc gttgcgcca 39

<210> 88
<211> 39
<212> DNA
<213> Artificial

<220>
<223> synthetic primer

<220>
<221> misc_feature
<222> (19)..(20)
<223> n is a, c, g, or t

<400> 88
cttaataact caatcggtnn sttaggcgtt gcgccaagc 39

<210> 89
<211> 39
<212> DNA
<213> Artificial

<220>
<223> synthetic primer

<220>
<221> misc_feature
<222> (19)..(20)
<223> n is a, c, g, or t

<400> 89
aataactcaa tcggtgtann sggcgttgcg ccaagcgca 39

<210> 90
<211> 39
<212> DNA
<213> Artificial

<220>
<223> synthetic primer

<220>
<221> misc_feature
<222> (19)..(20)
<223> n is a, c, g, or t

<400> 90
aactcaatcg gtgtattann sgttgcgcca agcgcatca 39

<210> 91
<211> 39
<212> DNA
<213> Artificial

<220>
<223> synthetic primer

<220>
<221> misc_feature
<222> (19)..(20)
<223> n is a, c, g, or t

<400> 91
tcaatcggtg tattaggcnn sgcgccaagc gcatcactt 39

<210> 92
<211> 39
<212> DNA
<213> Artificial

<220>
<223> synthetic primer

<220>
<221> misc_feature
<222> (19)..(20)
<223> n is a, c, g, or t

<400> 92
atcggtgtat taggcgttnn sccaagcgca tcactttac 39

<210> 93
<211> 39
<212> DNA
<213> Artificial

<220>

<223> synthetic primer

<220>
<221> misc_feature
<222> (19)..(20)
<223> n is a, c, g, or t

<400> 93
ggtgtattag gcgttgcgnn sagcgcatca ctttacgct 39

<210> 94
<211> 39
<212> DNA
<213> Artificial

<220>
<223> synthetic primer

<220>
<221> misc_feature
<222> (19)..(20)
<223> n is a, c, g, or t

<400> 94
gtattaggcg ttgcgccann sgcatcactt tacgctgta 39

<210> 95
<211> 39
<212> DNA
<213> Artificial

<220>
<223> synthetic primer

<220>
<221> misc_feature
<222> (19)..(20)
<223> n is a, c, g, or t

<400> 95
ttaggcgttg cgccaagcnn stcactttac gctgtaaaa 39

<210> 96
<211> 39
<212> DNA
<213> Artificial

<220>
<223> synthetic primer

<220>
<221> misc_feature
<222> (19)..(20)
<223> n is a, c, g, or t

<400> 96
ggcgttgcgc caagcgcann sctttacgct gtaaaagtt 39

<210> 97
<211> 39
<212> DNA
<213> Artificial

<220>
<223> synthetic primer

<220>
<221> misc_feature
<222> (19)..(20)
<223> n is a, c, g, or t

<400> 97
gttgcgccaa gcgcatcann stacgctgta aaagttctc 39

<210> 98
<211> 39
<212> DNA
<213> Artificial

<220>
<223> synthetic primer

<220>
<221> misc_feature
<222> (19)..(20)
<223> n is a, c, g, or t

<400> 98
gcgccaagcg catcacttnn sgctgtaaaa gttctcggt 39

<210> 99
<211> 39
<212> DNA
<213> Artificial

<220>
<223> synthetic primer

<220>
<221> misc_feature
<222> (19)..(20)
<223> n is a, c, g, or t

<400> 99
ccaagcgcat cactttacnn sgtaaaagtt ctcggtgct 39

<210> 100
<211> 39
<212> DNA
<213> Artificial

<220>
<223> synthetic primer

<220>
<221> misc_feature
<222> (19)..(20)
<223> n is a, c, g, or t

<400> 100
agcgcatcac tttacgctnn saaagttctc ggtgctgac 39

<210> 101
<211> 39
<212> DNA
<213> Artificial

<220>
<223> synthetic primer

<220>
<221> misc_feature
<222> (19)..(20)
<223> n is a, c, g, or t

<400> 101
gcatcacttt acgctgtann sgttctcggt gctgacggt 39

<210> 102
<211> 39
<212> DNA
<213> Artificial

<220>
<223> synthetic primer

<220>
<221> misc_feature
<222> (19)..(20)
<223> n is a, c, g, or t

<400> 102
tcactttacg ctgtaaaann sctcggtgct gacggttcc 39

<210> 103
<211> 39
<212> DNA
<213> Artificial

<220>
<223> synthetic primer

<220>
<221> misc_feature
<222> (19)..(20)
<223> n is a, c, g, or t

<400> 103
ctttacgctg taaaagttnn sggtgctgac ggttccggc 39

<210> 104
<211> 39
<212> DNA
<213> Artificial

<220>
<223> synthetic primer

<220>
<221> misc_feature
<222> (19)..(20)
<223> n is a, c, g, or t

<400> 104
tacgctgtaa aagttctcnn sgctgacggt tccggccaa 39

<210> 105
<211> 39
<212> DNA
<213> Artificial

<220>
<223> synthetic primer

<220>

<221>  misc_feature
<222>  (19)..(20)
<223>  n is a, c, g, or t

<400>  105
gctgtaaaag ttctcggtnn sgacggttcc ggccaatac    39

<210>  106
<211>  39
<212>  DNA
<213>  Artificial

<220>
<223>  synthetic primer

<220>
<221>  misc_feature
<222>  (19)..(20)
<223>  n is a, c, g, or t

<400>  106
gtaaaagttc tcggtgctnn sggttccggc caatacagc    39

<210>  107
<211>  39
<212>  DNA
<213>  Artificial

<220>
<223>  synthetic primer

<220>
<221>  misc_feature
<222>  (19)..(20)
<223>  n is a, c, g, or t

<400>  107
aaagttctcg gtgctgacnn stccggccaa tacagctgg    39

<210>  108
<211>  39
<212>  DNA
<213>  Artificial

<220>
<223>  synthetic primer

<220>
<221>  misc_feature
<222>  (19)..(20)
<223>  n is a, c, g, or t

<400>  108
gttctcggtg ctgacggtnn sggccaatac agctggatc    39

<210>  109
<211>  39
<212>  DNA
<213>  Artificial

<220>
<223>  synthetic primer

<220>
<221>  misc_feature

<222> (19)..(20)
<223> n is a, c, g, or t

<400> 109
ctcggtgctg acggttccnn scaatacagc tggatcatt 39

<210> 110
<211> 39
<212> DNA
<213> Artificial

<220>
<223> synthetic primer

<220>
<221> misc_feature
<222> (19)..(20)
<223> n is a, c, g, or t

<400> 110
ggtgctgacg gttccggcnn stacagctgg atcattaac 39

<210> 111
<211> 39
<212> DNA
<213> Artificial

<220>
<223> synthetic primer

<220>
<221> misc_feature
<222> (19)..(20)
<223> n is a, c, g, or t

<400> 111
gctgacggtt ccggccaann sagctggatc attaacgga 39

<210> 112
<211> 39
<212> DNA
<213> Artificial

<220>
<223> synthetic primer

<220>
<221> misc_feature
<222> (19)..(20)
<223> n is a, c, g, or t

<400> 112
gacggttccg gccaatacnn stggatcatt aacggaatc 39

<210> 113
<211> 39
<212> DNA
<213> Artificial

<220>
<223> synthetic primer

<220>
<221> misc_feature
<222> (19)..(20)

<223>  n is a, c, g, or t

<400>  113
ggttccggcc aatacagcnn satcattaac ggaatcgag  39

<210>  114
<211>  39
<212>  DNA
<213>  Artificial

<220>
<223>  synthetic primer

<220>
<221>  misc_feature
<222>  (19)..(20)
<223>  n is a, c, g, or t

<400>  114
tccggccaat acagctggnn sattaacgga atcgagtgg  39

<210>  115
<211>  39
<212>  DNA
<213>  Artificial

<220>
<223>  synthetic primer

<220>
<221>  misc_feature
<222>  (19)..(20)
<223>  n is a, c, g, or t

<400>  115
ggccaataca gctggatcnn saacggaatc gagtgggcg  39

<210>  116
<211>  39
<212>  DNA
<213>  Artificial

<220>
<223>  synthetic primer

<220>
<221>  misc_feature
<222>  (19)..(20)
<223>  n is a, c, g, or t

<400>  116
caatacagct ggatcattnn sggaatcgag tgggcgatc  39

<210>  117
<211>  39
<212>  DNA
<213>  Artificial

<220>
<223>  synthetic primer

<220>
<221>  misc_feature
<222>  (19)..(20)
<223>  n is a, c, g, or t

<400> 117
tacagctgga tcattaacnn satcgagtgg gcgatcgca 39

<210> 118
<211> 39
<212> DNA
<213> Artificial

<220>
<223> synthetic primer

<220>
<221> misc_feature
<222> (19)..(20)
<223> n is a, c, g, or t

<400> 118
agctggatca ttaacggann sgagtgggcg atcgcaaac 39

<210> 119
<211> 39
<212> DNA
<213> Artificial

<220>
<223> synthetic primer

<220>
<221> misc_feature
<222> (19)..(20)
<223> n is a, c, g, or t

<400> 119
tggatcatta acggaatcnn stgggcgatc gcaaacaat 39

<210> 120
<211> 39
<212> DNA
<213> Artificial

<220>
<223> synthetic primer

<220>
<221> misc_feature
<222> (19)..(20)
<223> n is a, c, g, or t

<400> 120
atcattaacg gaatcgagnn sgcgatcgca aacaatatg 39

<210> 121
<211> 39
<212> DNA
<213> Artificial

<220>
<223> synthetic primer

<220>
<221> misc_feature
<222> (19)..(20)
<223> n is a, c, g, or t

<400> 121
attaacggaa tcgagtggnn satcgcaaac aatatggac 39

<210> 122
<211> 39
<212> DNA
<213> Artificial

<220>
<223> synthetic primer

<220>
<221> misc_feature
<222> (19)..(20)
<223> n is a, c, g, or t

<400> 122
aacggaatcg agtgggcgnn sgcaaacaat atggacgtt 39

<210> 123
<211> 39
<212> DNA
<213> Artificial

<220>
<223> synthetic primer

<220>
<221> misc_feature
<222> (19)..(20)
<223> n is a, c, g, or t

<400> 123
ggaatcgagt gggcgatcnn saacaatatg gacgttatt 39

<210> 124
<211> 39
<212> DNA
<213> Artificial

<220>
<223> synthetic primer

<220>
<221> misc_feature
<222> (19)..(20)
<223> n is a, c, g, or t

<400> 124
atcgagtggg cgatcgcann saatatggac gttattaac 39

<210> 125
<211> 39
<212> DNA
<213> Artificial

<220>
<223> synthetic primer

<220>
<221> misc_feature
<222> (19)..(20)
<223> n is a, c, g, or t

<400> 125

gagtgggcga tcgcaaacnn satggacgtt attaacatg 39

<210> 126
<211> 39
<212> DNA
<213> Artificial

<220>
<223> synthetic primer

<220>
<221> misc_feature
<222> (19)..(20)
<223> n is a, c, g, or t

<400> 126
tgggcgatcg caaacaatnn sgacgttatt aacatgagc 39

<210> 127
<211> 39
<212> DNA
<213> Artificial

<220>
<223> synthetic primer

<220>
<221> misc_feature
<222> (19)..(20)
<223> n is a, c, g, or t

<400> 127
gcgatcgcaa acaatatgnn sgttattaac atgagcctc 39

<210> 128
<211> 39
<212> DNA
<213> Artificial

<220>
<223> synthetic primer

<220>
<221> misc_feature
<222> (19)..(20)
<223> n is a, c, g, or t

<400> 128
atcgcaaaca atatggacnn sattaacatg agcctcggc 39

<210> 129
<211> 39
<212> DNA
<213> Artificial

<220>
<223> synthetic primer

<220>
<221> misc_feature
<222> (19)..(20)
<223> n is a, c, g, or t

<400> 129
gcaaacaata tggacgttnn saacatgagc ctcggcgga 39

<210> 130
<211> 39
<212> DNA
<213> Artificial

<220>
<223> synthetic primer

<220>
<221> misc_feature
<222> (19)..(20)
<223> n is a, c, g, or t

<400> 130
aacaatatgg acgttattnn satgagcctc ggcggacct 39

<210> 131
<211> 39
<212> DNA
<213> Artificial

<220>
<223> synthetic primer

<220>
<221> misc_feature
<222> (19)..(20)
<223> n is a, c, g, or t

<400> 131
aatatggacg ttattaacnn sagcctcggc ggaccttct 39

<210> 132
<211> 39
<212> DNA
<213> Artificial

<220>
<223> synthetic primer

<220>
<221> misc_feature
<222> (19)..(20)
<223> n is a, c, g, or t

<400> 132
atggacgtta ttaacatgnn sctcggcgga ccttctggt 39

<210> 133
<211> 39
<212> DNA
<213> Artificial

<220>
<223> synthetic primer

<220>
<221> misc_feature
<222> (19)..(20)
<223> n is a, c, g, or t

<400> 133
gacgttatta acatgagcnn sggcggacct tctggttct 39

<210> 134
<211> 39
<212> DNA
<213> Artificial

<220>
<223> synthetic primer

<220>
<221> misc_feature
<222> (19)..(20)
<223> n is a, c, g, or t

<400> 134
gttattaaca tgagcctcnn sggaccttct ggttctgct 39

<210> 135
<211> 39
<212> DNA
<213> Artificial

<220>
<223> synthetic primer

<220>
<221> misc_feature
<222> (19)..(20)
<223> n is a, c, g, or t

<400> 135
attaacatga gcctcggcnn sccttctggt tctgctgct 39

<210> 136
<211> 39
<212> DNA
<213> Artificial

<220>
<223> synthetic primer

<220>
<221> misc_feature
<222> (19)..(20)
<223> n is a, c, g, or t

<400> 136
aacatgagcc tcggcggann stctggttct gctgcttta 39

<210> 137
<211> 39
<212> DNA
<213> Artificial

<220>
<223> synthetic primer

<220>
<221> misc_feature
<222> (19)..(20)
<223> n is a, c, g, or t

<400> 137
atgagcctcg gcggacctnn sggttctgct gctttaaaa 39

<210> 138

<211> 39
<212> DNA
<213> Artificial

<220>
<223> synthetic primer

<220>
<221> misc_feature
<222> (19)..(20)
<223> n is a, c, g, or t

<400> 138
agcctcggcg gaccttctnn stctgctgct ttaaaagcg 39

<210> 139
<211> 39
<212> DNA
<213> Artificial

<220>
<223> synthetic primer

<220>
<221> misc_feature
<222> (19)..(20)
<223> n is a, c, g, or t

<400> 139
ctcggcggac cttctggtnn sgctgcttta aaagcggca 39

<210> 140
<211> 39
<212> DNA
<213> Artificial

<220>
<223> synthetic primer

<220>
<221> misc_feature
<222> (19)..(20)
<223> n is a, c, g, or t

<400> 140
ggcggacctt ctggttctnn sgctttaaaa gcggcagtt 39

<210> 141
<211> 39
<212> DNA
<213> Artificial

<220>
<223> synthetic primer

<220>
<221> misc_feature
<222> (19)..(20)
<223> n is a, c, g, or t

<400> 141
ggaccttctg gttctgctnn sttaaaagcg gcagttgat 39

<210> 142
<211> 39

<212> DNA
<213> Artificial

<220>
<223> synthetic primer

<220>
<221> misc_feature
<222> (19)..(20)
<223> n is a, c, g, or t

<400> 142
ccttctggtt ctgctgctnn saaagcggca gttgataaa 39

<210> 143
<211> 39
<212> DNA
<213> Artificial

<220>
<223> synthetic primer

<220>
<221> misc_feature
<222> (19)..(20)
<223> n is a, c, g, or t

<400> 143
tctggttctg ctgctttann sgcggcagtt gataaagcc 39

<210> 144
<211> 39
<212> DNA
<213> Artificial

<220>
<223> synthetic primer

<220>
<221> misc_feature
<222> (19)..(20)
<223> n is a, c, g, or t

<400> 144
ggttctgctg ctttaaaann sgcagttgat aaagccgtt 39

<210> 145
<211> 39
<212> DNA
<213> Artificial

<220>
<223> synthetic primer

<220>
<221> misc_feature
<222> (19)..(20)
<223> n is a, c, g, or t

<400> 145
tctgctgctt taaaagcgnn sgttgataaa gccgttgca 39

<210> 146
<211> 39
<212> DNA

<213> Artificial

<220>
<223> synthetic primer

<220>
<221> misc_feature
<222> (19)..(20)
<223> n is a, c, g, or t

<400> 146
gctgctttaa aagcggcann sgataaagcc gttgcatcc 39

<210> 147
<211> 39
<212> DNA
<213> Artificial

<220>
<223> synthetic primer

<220>
<221> misc_feature
<222> (19)..(20)
<223> n is a, c, g, or t

<400> 147
gctttaaaag cggcagttnn saaagccgtt gcatccggc 39

<210> 148
<211> 39
<212> DNA
<213> Artificial

<220>
<223> synthetic primer

<220>
<221> misc_feature
<222> (19)..(20)
<223> n is a, c, g, or t

<400> 148
ttaaaagcgg cagttgatnn sgccgttgca tccggcgtc 39

<210> 149
<211> 39
<212> DNA
<213> Artificial

<220>
<223> synthetic primer

<220>
<221> misc_feature
<222> (19)..(20)
<223> n is a, c, g, or t

<400> 149
aaagcggcag ttgataaann sgttgcatcc ggcgtcgta 39

<210> 150
<211> 39
<212> DNA
<213> Artificial

<220>
<223> synthetic primer

<220>
<221> misc_feature
<222> (19)..(20)
<223> n is a, c, g, or t

<400> 150
gcggcagttg ataaagccnn sgcatccggc gtcgtagtc 39

<210> 151
<211> 39
<212> DNA
<213> Artificial

<220>
<223> synthetic primer

<220>
<221> misc_feature
<222> (19)..(20)
<223> n is a, c, g, or t

<400> 151
gcagttgata aagccgttnn stccggcgtc gtagtcgtt 39

<210> 152
<211> 39
<212> DNA
<213> Artificial

<220>
<223> synthetic primer

<220>
<221> misc_feature
<222> (19)..(20)
<223> n is a, c, g, or t

<400> 152
gttgataaag ccgttgcann sggcgtcgta gtcgttgcg 39

<210> 153
<211> 39
<212> DNA
<213> Artificial

<220>
<223> synthetic primer

<220>
<221> misc_feature
<222> (19)..(20)
<223> n is a, c, g, or t

<400> 153
gataaagccg ttgcatccnn sgtcgtagtc gttgcggca 39

<210> 154
<211> 39
<212> DNA
<213> Artificial

<220>
<223> synthetic primer

<220>
<221> misc_feature
<222> (19)..(20)
<223> n is a, c, g, or t

<400> 154
aaagccgttg catccggcnn sgtagtcgtt gcggcagcc 39

<210> 155
<211> 39
<212> DNA
<213> Artificial

<220>
<223> synthetic primer

<220>
<221> misc_feature
<222> (19)..(20)
<223> n is a, c, g, or t

<400> 155
gccgttgcat ccggcgtcnn sgtcgttgcg gcagccggt 39

<210> 156
<211> 39
<212> DNA
<213> Artificial

<220>
<223> synthetic primer

<220>
<221> misc_feature
<222> (19)..(20)
<223> n is a, c, g, or t

<400> 156
gttgcatccg gcgtcgtann sgttgcggca gccggtaac 39

<210> 157
<211> 39
<212> DNA
<213> Artificial

<220>
<223> synthetic primer

<220>
<221> misc_feature
<222> (19)..(20)
<223> n is a, c, g, or t

<400> 157
gcatccggcg tcgtagtcnn sgcggcagcc ggtaacgaa 39

<210> 158
<211> 39
<212> DNA
<213> Artificial

<220>

<223> synthetic primer

<220>
<221> misc_feature
<222> (19)..(20)
<223> n is a, c, g, or t

<400> 158
tccggcgtcg tagtcgttnn sgcagccggt aacgaaggc 39

<210> 159
<211> 39
<212> DNA
<213> Artificial

<220>
<223> synthetic primer

<220>
<221> misc_feature
<222> (19)..(20)
<223> n is a, c, g, or t

<400> 159
ggcgtcgtag tcgttgcgnn sgccggtaac gaaggcact 39

<210> 160
<211> 39
<212> DNA
<213> Artificial

<220>
<223> synthetic primer

<220>
<221> misc_feature
<222> (19)..(20)
<223> n is a, c, g, or t

<400> 160
gtcgtagtcg ttgcggcann sggtaacgaa ggcacttcc 39

<210> 161
<211> 39
<212> DNA
<213> Artificial

<220>
<223> synthetic primer

<220>
<221> misc_feature
<222> (19)..(20)
<223> n is a, c, g, or t

<400> 161
gtagtcgttg cggcagccnn saacgaaggc acttccggc 39

<210> 162
<211> 39
<212> DNA
<213> Artificial

<220>
<223> synthetic primer

<220>
<221> misc_feature
<222> (19)..(20)
<223> n is a, c, g, or t

<400> 162
gtcgttgcgg cagccggtnn sgaaggcact tccggcagc 39

<210> 163
<211> 39
<212> DNA
<213> Artificial

<220>
<223> synthetic primer

<220>
<221> misc_feature
<222> (19)..(20)
<223> n is a, c, g, or t

<400> 163
gttgcggcag ccggtaacnn sggcacttcc ggcagctca 39

<210> 164
<211> 39
<212> DNA
<213> Artificial

<220>
<223> synthetic primer

<220>
<221> misc_feature
<222> (19)..(20)
<223> n is a, c, g, or t

<400> 164
gcggcagccg gtaacgaann sacttccggc agctcaagc 39

<210> 165
<211> 39
<212> DNA
<213> Artificial

<220>
<223> synthetic primer

<220>
<221> misc_feature
<222> (19)..(20)
<223> n is a, c, g, or t

<400> 165
gcagccggta acgaaggcnn stccggcagc tcaagcaca 39

<210> 166
<211> 39
<212> DNA
<213> Artificial

<220>
<223> synthetic primer

<220>
<221> misc_feature
<222> (19)..(20)
<223> n is a, c, g, or t

<400> 166
gccggtaacg aaggcactnn sggcagctca agcacagtg 39

<210> 167
<211> 39
<212> DNA
<213> Artificial

<220>
<223> synthetic primer

<220>
<221> misc_feature
<222> (19)..(20)
<223> n is a, c, g, or t

<400> 167
ggtaacgaag gcacttccnn sagctcaagc acagtgggc 39

<210> 168
<211> 39
<212> DNA
<213> Artificial

<220>
<223> synthetic primer

<220>
<221> misc_feature
<222> (19)..(20)
<223> n is a, c, g, or t

<400> 168
aacgaaggca cttccggcnn stcaagcaca gtgggctac 39

<210> 169
<211> 39
<212> DNA
<213> Artificial

<220>
<223> synthetic primer

<220>
<221> misc_feature
<222> (19)..(20)
<223> n is a, c, g, or t

<400> 169
gaaggcactt ccggcagcnn sagcacagtg ggctaccct 39

<210> 170
<211> 39
<212> DNA
<213> Artificial

<220>
<223> synthetic primer

<220>

<221> misc_feature
<222> (19)..(20)
<223> n is a, c, g, or t

<400> 170
ggcacttccg gcagctcann sacagtgggc taccctggt 39

<210> 171
<211> 39
<212> DNA
<213> Artificial

<220>
<223> synthetic primer

<220>
<221> misc_feature
<222> (19)..(20)
<223> n is a, c, g, or t

<400> 171
acttccggca gctcaagcnn sgtgggctac cctggtaaa 39

<210> 172
<211> 39
<212> DNA
<213> Artificial

<220>
<223> synthetic primer

<220>
<221> misc_feature
<222> (19)..(20)
<223> n is a, c, g, or t

<400> 172
tccggcagct caagcacann sggctaccct ggtaaatac 39

<210> 173
<211> 39
<212> DNA
<213> Artificial

<220>
<223> synthetic primer

<220>
<221> misc_feature
<222> (19)..(20)
<223> n is a, c, g, or t

<400> 173
ggcagctcaa gcacagtgnn staccctggt aaatacect 39

<210> 174
<211> 39
<212> DNA
<213> Artificial

<220>
<223> synthetic primer

<220>
<221> misc_feature

<222> (19)..(20)
<223> n is a, c, g, or t

<400> 174
agctcaagca cagtgggcnn scctggtaaa tacccttct 39

<210> 175
<211> 39
<212> DNA
<213> Artificial

<220>
<223> synthetic primer

<220>
<221> misc_feature
<222> (19)..(20)
<223> n is a, c, g, or t

<400> 175
tcaagcacag tgggctacnn sggtaaatac ccttctgtc 39

<210> 176
<211> 39
<212> DNA
<213> Artificial

<220>
<223> synthetic primer

<220>
<221> misc_feature
<222> (19)..(20)
<223> n is a, c, g, or t

<400> 176
agcacagtgg gctaccctnn saaataccct tctgtcatt 39

<210> 177
<211> 39
<212> DNA
<213> Artificial

<220>
<223> synthetic primer

<220>
<221> misc_feature
<222> (19)..(20)
<223> n is a, c, g, or t

<400> 177
acagtgggct accctggtnn stacccttct gtcattgca 39

<210> 178
<211> 39
<212> DNA
<213> Artificial

<220>
<223> synthetic primer

<220>
<221> misc_feature
<222> (19)..(20)

<223> n is a, c, g, or t

<400> 178
gtgggctacc ctggtaaann scctttctgtc attgcagta 39

<210> 179
<211> 39
<212> DNA
<213> Artificial

<220>
<223> synthetic primer

<220>
<221> misc_feature
<222> (19)..(20)
<223> n is a, c, g, or t

<400> 179
ggctaccctg gtaaatacnn stctgtcatt gcagtaggc 39

<210> 180
<211> 39
<212> DNA
<213> Artificial

<220>
<223> synthetic primer

<220>
<221> misc_feature
<222> (19)..(20)
<223> n is a, c, g, or t

<400> 180
taccctggta aataccctnn sgtcattgca gtaggcgct 39

<210> 181
<211> 39
<212> DNA
<213> Artificial

<220>
<223> synthetic primer

<220>
<221> misc_feature
<222> (19)..(20)
<223> n is a, c, g, or t

<400> 181
cctggtaaat acccttctnn sattgcagta ggcgctgtt 39

<210> 182
<211> 39
<212> DNA
<213> Artificial

<220>
<223> synthetic primer

<220>
<221> misc_feature
<222> (19)..(20)
<223> n is a, c, g, or t

<400> 182
ggtaaatacc cttctgtcnn sgcagtaggc gctgttgac 39

<210> 183
<211> 39
<212> DNA
<213> Artificial

<220>
<223> synthetic primer

<220>
<221> misc_feature
<222> (19)..(20)
<223> n is a, c, g, or t

<400> 183
aaataccctt ctgtcattnn sgtaggcgct gttgacagc 39

<210> 184
<211> 39
<212> DNA
<213> Artificial

<220>
<223> synthetic primer

<220>
<221> misc_feature
<222> (19)..(20)
<223> n is a, c, g, or t

<400> 184
taccettctg tcattgcann sggcgctgtt gacagcagc 39

<210> 185
<211> 39
<212> DNA
<213> Artificial

<220>
<223> synthetic primer

<220>
<221> misc_feature
<222> (19)..(20)
<223> n is a, c, g, or t

<400> 185
ccttctgtca ttgcagtann sgctgttgac agcagcaac 39

<210> 186
<211> 39
<212> DNA
<213> Artificial

<220>
<223> synthetic primer

<220>
<221> misc_feature
<222> (19)..(20)
<223> n is a, c, g, or t

<400> 186
tctgtcattg cagtaggcnn sgttgacagc agcaaccaa 39

<210> 187
<211> 39
<212> DNA
<213> Artificial

<220>
<223> synthetic primer

<220>
<221> misc_feature
<222> (19)..(20)
<223> n is a, c, g, or t

<400> 187
gtcattgcag taggcgctnn sgacagcagc aaccaaaga 39

<210> 188
<211> 39
<212> DNA
<213> Artificial

<220>
<223> synthetic primer

<220>
<221> misc_feature
<222> (19)..(20)
<223> n is a, c, g, or t

<400> 188
attgcagtag gcgctgttnn sagcagcaac caaagagca 39

<210> 189
<211> 39
<212> DNA
<213> Artificial

<220>
<223> synthetic primer

<220>
<221> misc_feature
<222> (19)..(20)
<223> n is a, c, g, or t

<400> 189
gcagtaggcg ctgttgacnn sagcaaccaa agagcatct 39

<210> 190
<211> 39
<212> DNA
<213> Artificial

<220>
<223> synthetic primer

<220>
<221> misc_feature
<222> (19)..(20)
<223> n is a, c, g, or t

<400> 190

gtaggcgctg ttgacagcnn saaccaaaga gcatctttc 39

<210> 191
<211> 39
<212> DNA
<213> Artificial

<220>
<223> synthetic primer

<220>
<221> misc_feature
<222> (19)..(20)
<223> n is a, c, g, or t

<400> 191
ggcgctgttg acagcagcnn scaaagagca tctttctca 39

<210> 192
<211> 39
<212> DNA
<213> Artificial

<220>
<223> synthetic primer

<220>
<221> misc_feature
<222> (19)..(20)
<223> n is a, c, g, or t

<400> 192
gctgttgaca gcagcaacnn sagagcatct ttctcaagc 39

<210> 193
<211> 39
<212> DNA
<213> Artificial

<220>
<223> synthetic primer

<220>
<221> misc_feature
<222> (19)..(20)
<223> n is a, c, g, or t

<400> 193
gttgacagca gcaaccaann sgcatctttc tcaagcgta 39

<210> 194
<211> 39
<212> DNA
<213> Artificial

<220>
<223> synthetic primer

<220>
<221> misc_feature
<222> (19)..(20)
<223> n is a, c, g, or t

<400> 194
gacagcagca accaaagann stctttctca agcgtagga 39

<210> 195
<211> 39
<212> DNA
<213> Artificial

<220>
<223> synthetic primer

<220>
<221> misc_feature
<222> (19)..(20)
<223> n is a, c, g, or t

<400> 195
agcagcaacc aaagagcann sttctcaagc gtaggacct 39

<210> 196
<211> 39
<212> DNA
<213> Artificial

<220>
<223> synthetic primer

<220>
<221> misc_feature
<222> (19)..(20)
<223> n is a, c, g, or t

<400> 196
agcaaccaaa gagcatctnn stcaagcgta ggacctgag 39

<210> 197
<211> 39
<212> DNA
<213> Artificial

<220>
<223> synthetic primer

<220>
<221> misc_feature
<222> (19)..(20)
<223> n is a, c, g, or t

<400> 197
aaccaaagag catctttcnn sagcgtagga cctgagctt 39

<210> 198
<211> 39
<212> DNA
<213> Artificial

<220>
<223> synthetic primer

<220>
<221> misc_feature
<222> (19)..(20)
<223> n is a, c, g, or t

<400> 198
caaagagcat ctttctcann sgtaggacct gagcttgat 39

<210> 199
<211> 39
<212> DNA
<213> Artificial

<220>
<223> synthetic primer

<220>
<221> misc_feature
<222> (19)..(20)
<223> n is a, c, g, or t

<400> 199
agagcatctt tctcaagcnn sggacctgag cttgatgtc 39

<210> 200
<211> 39
<212> DNA
<213> Artificial

<220>
<223> synthetic primer

<220>
<221> misc_feature
<222> (19)..(20)
<223> n is a, c, g, or t

<400> 200
gcatctttct caagcgtann scctgagctt gatgtcatg 39

<210> 201
<211> 39
<212> DNA
<213> Artificial

<220>
<223> synthetic primer

<220>
<221> misc_feature
<222> (19)..(20)
<223> n is a, c, g, or t

<400> 201
tctttctcaa gcgtaggann sgagcttgat gtcatggca 39

<210> 202
<211> 39
<212> DNA
<213> Artificial

<220>
<223> synthetic primer

<220>
<221> misc_feature
<222> (19)..(20)
<223> n is a, c, g, or t

<400> 202
ttctcaagcg taggacctnn scttgatgtc atggcacct 39

<210> 203

<211> 39
<212> DNA
<213> Artificial

<220>
<223> synthetic primer

<220>
<221> misc_feature
<222> (19)..(20)
<223> n is a, c, g, or t

<400> 203
tcaagcgtag gacctgagnn sgatgtcatg gcacctggc 39

<210> 204
<211> 39
<212> DNA
<213> Artificial

<220>
<223> synthetic primer

<220>
<221> misc_feature
<222> (19)..(20)
<223> n is a, c, g, or t

<400> 204
agcgtaggac ctgagcttnn sgtcatggca cctggcgta 39

<210> 205
<211> 39
<212> DNA
<213> Artificial

<220>
<223> synthetic primer

<220>
<221> misc_feature
<222> (19)..(20)
<223> n is a, c, g, or t

<400> 205
gtaggacctg agcttgatnn satggcacct ggcgtatct 39

<210> 206
<211> 39
<212> DNA
<213> Artificial

<220>
<223> synthetic primer

<220>
<221> misc_feature
<222> (19)..(20)
<223> n is a, c, g, or t

<400> 206
ggacctgagc ttgatgtcnn sgcacctggc gtatctatc 39

<210> 207
<211> 39

<212> DNA
<213> Artificial

<220>
<223> synthetic primer

<220>
<221> misc_feature
<222> (19)..(20)
<223> n is a, c, g, or t

<400> 207
cctgagcttg atgtcatgnn scctggcgta tctatccaa 39

<210> 208
<211> 39
<212> DNA
<213> Artificial

<220>
<223> synthetic primer

<220>
<221> misc_feature
<222> (19)..(20)
<223> n is a, c, g, or t

<400> 208
gagcttgatg tcatggcann sggcgtatct atccaaagc 39

<210> 209
<211> 39
<212> DNA
<213> Artificial

<220>
<223> synthetic primer

<220>
<221> misc_feature
<222> (19)..(20)
<223> n is a, c, g, or t

<400> 209
cttgatgtca tggcacctnn sgtatctatc caaagcacg 39

<210> 210
<211> 39
<212> DNA
<213> Artificial

<220>
<223> synthetic primer

<220>
<221> misc_feature
<222> (19)..(20)
<223> n is a, c, g, or t

<400> 210
gatgtcatgg cacctggcnn stctatccaa agcacgctt 39

<210> 211
<211> 39
<212> DNA

<213>  Artificial

<220>
<223>  synthetic primer

<220>
<221>  misc_feature
<222>  (19)..(20)
<223>  n is a, c, g, or t

<400>  211
gtcatggcac ctggcgtann satccaaagc acgcttcct    39

<210>  212
<211>  39
<212>  DNA
<213>  Artificial

<220>
<223>  synthetic primer

<220>
<221>  misc_feature
<222>  (19)..(20)
<223>  n is a, c, g, or t

<400>  212
atggcacctg gcgtatctnn scaaagcacg cttcctgga    39

<210>  213
<211>  39
<212>  DNA
<213>  Artificial

<220>
<223>  synthetic primer

<220>
<221>  misc_feature
<222>  (19)..(20)
<223>  n is a, c, g, or t

<400>  213
gcacctggcg tatctatcnn sagcacgctt cctggaaac    39

<210>  214
<211>  39
<212>  DNA
<213>  Artificial

<220>
<223>  synthetic primer

<220>
<221>  misc_feature
<222>  (19)..(20)
<223>  n is a, c, g, or t

<400>  214
cctggcgtat ctatccaann sacgcttcct ggaaacaaa    39

<210>  215
<211>  39
<212>  DNA
<213>  Artificial

<220>
<223> synthetic primer

<220>
<221> misc_feature
<222> (19)..(20)
<223> n is a, c, g, or t

<400> 215
ggcgtatcta tccaaagcnn scttcctgga aacaaatac 39

<210> 216
<211> 39
<212> DNA
<213> Artificial

<220>
<223> synthetic primer

<220>
<221> misc_feature
<222> (19)..(20)
<223> n is a, c, g, or t

<400> 216
gtatctatcc aaagcacgnn scctggaaac aaatacggg 39

<210> 217
<211> 39
<212> DNA
<213> Artificial

<220>
<223> synthetic primer

<220>
<221> misc_feature
<222> (19)..(20)
<223> n is a, c, g, or t

<400> 217
tctatccaaa gcacgcttnn sggaaacaaa tacggggcg 39

<210> 218
<211> 39
<212> DNA
<213> Artificial

<220>
<223> synthetic primer

<220>
<221> misc_feature
<222> (19)..(20)
<223> n is a, c, g, or t

<400> 218
atccaaagca cgcttcctnn saacaaatac ggggcgtac 39

<210> 219
<211> 39
<212> DNA
<213> Artificial

<220>
<223> synthetic primer

<220>
<221> misc_feature
<222> (19)..(20)
<223> n is a, c, g, or t

<400> 219
caaagcacgc ttcctggann saaatacggg gcgtacaac 39

<210> 220
<211> 39
<212> DNA
<213> Artificial

<220>
<223> synthetic primer

<220>
<221> misc_feature
<222> (19)..(20)
<223> n is a, c, g, or t

<400> 220
agcacgcttc ctggaaacnn stacggggcg tacaacggt 39

<210> 221
<211> 39
<212> DNA
<213> Artificial

<220>
<223> synthetic primer

<220>
<221> misc_feature
<222> (19)..(20)
<223> n is a, c, g, or t

<400> 221
acgcttcctg gaaacaaann sggggcgtac aacggtacg 39

<210> 222
<211> 39
<212> DNA
<213> Artificial

<220>
<223> synthetic primer

<220>
<221> misc_feature
<222> (19)..(20)
<223> n is a, c, g, or t

<400> 222
cttcctggaa acaaatacnn sgcgtacaac ggtacgtca 39

<210> 223
<211> 39
<212> DNA
<213> Artificial

<220>

<223> synthetic primer

<220>
<221> misc_feature
<222> (19)..(20)
<223> n is a, c, g, or t

<400> 223
cctggaaaca aatacgggnn stacaacggt acgtcaatg 39

<210> 224
<211> 39
<212> DNA
<213> Artificial

<220>
<223> synthetic primer

<220>
<221> misc_feature
<222> (19)..(20)
<223> n is a, c, g, or t

<400> 224
ggaaacaaat acggggcgnn saacggtacg tcaatggca 39

<210> 225
<211> 39
<212> DNA
<213> Artificial

<220>
<223> synthetic primer

<220>
<221> misc_feature
<222> (19)..(20)
<223> n is a, c, g, or t

<400> 225
aacaaatacg gggcgtacnn sggtacgtca atggcatct 39

<210> 226
<211> 39
<212> DNA
<213> Artificial

<220>
<223> synthetic primer

<220>
<221> misc_feature
<222> (19)..(20)
<223> n is a, c, g, or t

<400> 226
aaatacgggg cgtacaacnn sacgtcaatg gcatctccg 39

<210> 227
<211> 39
<212> DNA
<213> Artificial

<220>
<223> synthetic primer

<220>
<221> misc_feature
<222> (19)..(20)
<223> n is a, c, g, or t

<400> 227
tacggggcgt acaacggtnn stcaatggca tctccgcac 39

<210> 228
<211> 39
<212> DNA
<213> Artificial

<220>
<223> synthetic primer

<220>
<221> misc_feature
<222> (19)..(20)
<223> n is a, c, g, or t

<400> 228
ggggcgtaca acggtacgnn satggcatct ccgcacgtt 39

<210> 229
<211> 39
<212> DNA
<213> Artificial

<220>
<223> synthetic primer

<220>
<221> misc_feature
<222> (19)..(20)
<223> n is a, c, g, or t

<400> 229
gcgtacaacg gtacgtcann sgcatctccg cacgttgcc 39

<210> 230
<211> 39
<212> DNA
<213> Artificial

<220>
<223> synthetic primer

<220>
<221> misc_feature
<222> (19)..(20)
<223> n is a, c, g, or t

<400> 230
tacaacggta cgtcaatgnn stctccgcac gttgccgga 39

<210> 231
<211> 39
<212> DNA
<213> Artificial

<220>
<223> synthetic primer

<220>
<221> misc_feature
<222> (19)..(20)
<223> n is a, c, g, or t

<400> 231
aacggtacgt caatggcann sccgcacgtt gccggagcg 39

<210> 232
<211> 39
<212> DNA
<213> Artificial

<220>
<223> synthetic primer

<220>
<221> misc_feature
<222> (19)..(20)
<223> n is a, c, g, or t

<400> 232
ggtacgtcaa tggcatctnn scacgttgcc ggagcggct 39

<210> 233
<211> 39
<212> DNA
<213> Artificial

<220>
<223> synthetic primer

<220>
<221> misc_feature
<222> (19)..(20)
<223> n is a, c, g, or t

<400> 233
acgtcaatgg catctccgnn sgttgccgga gcggctgct 39

<210> 234
<211> 39
<212> DNA
<213> Artificial

<220>
<223> synthetic primer

<220>
<221> misc_feature
<222> (19)..(20)
<223> n is a, c, g, or t

<400> 234
tcaatggcat ctccgcacnn sgccggagcg gctgctttg 39

<210> 235
<211> 39
<212> DNA
<213> Artificial

<220>
<223> synthetic primer

<220>

<221> misc_feature
<222> (19)..(20)
<223> n is a, c, g, or t

<400> 235
atggcatctc cgcacgttnn sggagcggct gctttgatt 39

<210> 236
<211> 39
<212> DNA
<213> Artificial

<220>
<223> synthetic primer

<220>
<221> misc_feature
<222> (19)..(20)
<223> n is a, c, g, or t

<400> 236
gcatctccgc acgttgccnn sgcggctgct ttgattctt 39

<210> 237
<211> 39
<212> DNA
<213> Artificial

<220>
<223> synthetic primer

<220>
<221> misc_feature
<222> (19)..(20)
<223> n is a, c, g, or t

<400> 237
tctccgcacg ttgccggann sgctgctttg attctttct 39

<210> 238
<211> 39
<212> DNA
<213> Artificial

<220>
<223> synthetic primer

<220>
<221> misc_feature
<222> (19)..(20)
<223> n is a, c, g, or t

<400> 238
ccgcacgttg ccggagcgnn sgctttgatt ctttctaag 39

<210> 239
<211> 39
<212> DNA
<213> Artificial

<220>
<223> synthetic primer

<220>
<221> misc_feature

<222> (19)..(20)
<223> n is a, c, g, or t

<400> 239
cacgttgccg gagcggctnn sttgattctt tctaagcac 39

<210> 240
<211> 39
<212> DNA
<213> Artificial

<220>
<223> synthetic primer

<220>
<221> misc_feature
<222> (19)..(20)
<223> n is a, c, g, or t

<400> 240
gttgccggag cggctgctnn sattctttct aagcacccg 39

<210> 241
<211> 39
<212> DNA
<213> Artificial

<220>
<223> synthetic primer

<220>
<221> misc_feature
<222> (19)..(20)
<223> n is a, c, g, or t

<400> 241
gccggagcgg ctgctttgnn sctttctaag cacccgaac 39

<210> 242
<211> 39
<212> DNA
<213> Artificial

<220>
<223> synthetic primer

<220>
<221> misc_feature
<222> (19)..(20)
<223> n is a, c, g, or t

<400> 242
ggagcggctg ctttgattnn stctaagcac ccgaactgg 39

<210> 243
<211> 39
<212> DNA
<213> Artificial

<220>
<223> synthetic primer

<220>
<221> misc_feature
<222> (19)..(20)

<223> n is a, c, g, or t

<400> 243
gcggctgctt tgattcttnn saagcacccg aactggaca 39

<210> 244
<211> 39
<212> DNA
<213> Artificial

<220>
<223> synthetic primer

<220>
<221> misc_feature
<222> (19)..(20)
<223> n is a, c, g, or t

<400> 244
gctgctttga ttctttctnn scacccgaac tggacaaac 39

<210> 245
<211> 39
<212> DNA
<213> Artificial

<220>
<223> synthetic primer

<220>
<221> misc_feature
<222> (19)..(20)
<223> n is a, c, g, or t

<400> 245
gctttgattc tttctaagnn sccgaactgg acaaacact 39

<210> 246
<211> 39
<212> DNA
<213> Artificial

<220>
<223> synthetic primer

<220>
<221> misc_feature
<222> (19)..(20)
<223> n is a, c, g, or t

<400> 246
ttgattcttt ctaagcacnn saactggaca aacactcaa 39

<210> 247
<211> 39
<212> DNA
<213> Artificial

<220>
<223> synthetic primer

<220>
<221> misc_feature
<222> (19)..(20)
<223> n is a, c, g, or t

<400> 247
attctttcta agcacccgnn stggacaaac actcaagtc 39

<210> 248
<211> 39
<212> DNA
<213> Artificial

<220>
<223> synthetic primer

<220>
<221> misc_feature
<222> (19)..(20)
<223> n is a, c, g, or t

<400> 248
ctttctaagc acccgaacnn sacaaacact caagtccgc 39

<210> 249
<211> 39
<212> DNA
<213> Artificial

<220>
<223> synthetic primer

<220>
<221> misc_feature
<222> (19)..(20)
<223> n is a, c, g, or t

<400> 249
tctaagcacc cgaactggnn saacactcaa gtccgcagc 39

<210> 250
<211> 39
<212> DNA
<213> Artificial

<220>
<223> synthetic primer

<220>
<221> misc_feature
<222> (19)..(20)
<223> n is a, c, g, or t

<400> 250
aagcacccga actggacann sactcaagtc cgcagcagt 39

<210> 251
<211> 39
<212> DNA
<213> Artificial

<220>
<223> synthetic primer

<220>
<221> misc_feature
<222> (19)..(20)
<223> n is a, c, g, or t

<400> 251
cacccgaact ggacaaacnn scaagtccgc agcagttta 39

<210> 252
<211> 39
<212> DNA
<213> Artificial

<220>
<223> synthetic primer

<220>
<221> misc_feature
<222> (19)..(20)
<223> n is a, c, g, or t

<400> 252
ccgaactgga caaacactnn sgtccgcagc agtttagaa 39

<210> 253
<211> 39
<212> DNA
<213> Artificial

<220>
<223> synthetic primer

<220>
<221> misc_feature
<222> (19)..(20)
<223> n is a, c, g, or t

<400> 253
aactggacaa acactcaann scgcagcagt ttagaaaac 39

<210> 254
<211> 39
<212> DNA
<213> Artificial

<220>
<223> synthetic primer

<220>
<221> misc_feature
<222> (19)..(20)
<223> n is a, c, g, or t

<400> 254
tggacaaaca ctcaagtcnn sagcagttta gaaaacacc 39

<210> 255
<211> 39
<212> DNA
<213> Artificial

<220>
<223> synthetic primer

<220>
<221> misc_feature
<222> (19)..(20)
<223> n is a, c, g, or t

<400> 255

acaaacactc aagtccgcnn sagtttagaa aacaccact 39

<210> 256
<211> 39
<212> DNA
<213> Artificial

<220>
<223> synthetic primer

<220>
<221> misc_feature
<222> (19)..(20)
<223> n is a, c, g, or t

<400> 256
aacactcaag tccgcagcnn sttagaaaac accactaca 39

<210> 257
<211> 39
<212> DNA
<213> Artificial

<220>
<223> synthetic primer

<220>
<221> misc_feature
<222> (19)..(20)
<223> n is a, c, g, or t

<400> 257
actcaagtcc gcagcagtnn sgaaaacacc actacaaaa 39

<210> 258
<211> 39
<212> DNA
<213> Artificial

<220>
<223> synthetic primer

<220>
<221> misc_feature
<222> (19)..(20)
<223> n is a, c, g, or t

<400> 258
caagtccgca gcagtttann saacaccact acaaaactt 39

<210> 259
<211> 39
<212> DNA
<213> Artificial

<220>
<223> synthetic primer

<220>
<221> misc_feature
<222> (19)..(20)
<223> n is a, c, g, or t

<400> 259
gtccgcagca gtttagaann saccactaca aaacttggt 39

<210> 260
<211> 39
<212> DNA
<213> Artificial

<220>
<223> synthetic primer

<220>
<221> misc_feature
<222> (19)..(20)
<223> n is a, c, g, or t

<400> 260
cgcagcagtt tagaaaacnn sactacaaaa cttggtgat 39

<210> 261
<211> 39
<212> DNA
<213> Artificial

<220>
<223> synthetic primer

<220>
<221> misc_feature
<222> (19)..(20)
<223> n is a, c, g, or t

<400> 261
agcagtttag aaaacaccnn sacaaaactt ggtgattct 39

<210> 262
<211> 39
<212> DNA
<213> Artificial

<220>
<223> synthetic primer

<220>
<221> misc_feature
<222> (19)..(20)
<223> n is a, c, g, or t

<400> 262
agtttagaaa acaccactnn saaacttggt gattctttc 39

<210> 263
<211> 39
<212> DNA
<213> Artificial

<220>
<223> synthetic primer

<220>
<221> misc_feature
<222> (19)..(20)
<223> n is a, c, g, or t

<400> 263
ttagaaaaca ccactacann scttggtgat tctttctac 39

<210> 264
<211> 39
<212> DNA
<213> Artificial

<220>
<223> synthetic primer

<220>
<221> misc_feature
<222> (19)..(20)
<223> n is a, c, g, or t

<400> 264
gaaaacacca ctacaaaann sggtgattct ttctactat 39

<210> 265
<211> 39
<212> DNA
<213> Artificial

<220>
<223> synthetic primer

<220>
<221> misc_feature
<222> (19)..(20)
<223> n is a, c, g, or t

<400> 265
aacaccacta caaaacttnn sgattctttc tactatgga 39

<210> 266
<211> 39
<212> DNA
<213> Artificial

<220>
<223> synthetic primer

<220>
<221> misc_feature
<222> (19)..(20)
<223> n is a, c, g, or t

<400> 266
accactacaa aacttggtnn stctttctac tatggaaaa 39

<210> 267
<211> 39
<212> DNA
<213> Artificial

<220>
<223> synthetic primer

<220>
<221> misc_feature
<222> (19)..(20)
<223> n is a, c, g, or t

<400> 267
actacaaaac ttggtgatnn sttctactat ggaaaaggg 39

<210> 268

<211> 39
<212> DNA
<213> Artificial

<220>
<223> synthetic primer

<220>
<221> misc_feature
<222> (19)..(20)
<223> n is a, c, g, or t

<400> 268
acaaaacttg gtgattctnn stactatgga aaagggctg 39

<210> 269
<211> 39
<212> DNA
<213> Artificial

<220>
<223> synthetic primer

<220>
<221> misc_feature
<222> (19)..(20)
<223> n is a, c, g, or t

<400> 269
aaacttggtg attctttcnn statggaaaa gggctgatc 39

<210> 270
<211> 39
<212> DNA
<213> Artificial

<220>
<223> synthetic primer

<220>
<221> misc_feature
<222> (19)..(20)
<223> n is a, c, g, or t

<400> 270
cttggtgatt ctttctacnn sggaaaaggg ctgatcaac 39

<210> 271
<211> 39
<212> DNA
<213> Artificial

<220>
<223> synthetic primer

<220>
<221> misc_feature
<222> (19)..(20)
<223> n is a, c, g, or t

<400> 271
ggtgattctt tctactatnn saaagggctg atcaacgta 39

<210> 272
<211> 39

<212> DNA
<213> Artificial

<220>
<223> synthetic primer

<220>
<221> misc_feature
<222> (19)..(20)
<223> n is a, c, g, or t

<400> 272
gattctttct actatggann sgggctgatc aacgtacag 39

<210> 273
<211> 39
<212> DNA
<213> Artificial

<220>
<223> synthetic primer

<220>
<221> misc_feature
<222> (19)..(20)
<223> n is a, c, g, or t

<400> 273
tctttctact atggaaaann sctgatcaac gtacaggcg 39

<210> 274
<211> 39
<212> DNA
<213> Artificial

<220>
<223> synthetic primer

<220>
<221> misc_feature
<222> (19)..(20)
<223> n is a, c, g, or t

<400> 274
ttctactatg gaaaagggnn satcaacgta caggcggca 39

<210> 275
<211> 39
<212> DNA
<213> Artificial

<220>
<223> synthetic primer

<220>
<221> misc_feature
<222> (19)..(20)
<223> n is a, c, g, or t

<400> 275
tactatggaa aagggctgnn saacgtacag gcggcagct 39

<210> 276
<211> 39
<212> DNA

<213> Artificial

<220>
<223> synthetic primer

<220>
<221> misc_feature
<222> (19)..(20)
<223> n is a, c, g, or t

<400> 276
tatggaaaag ggctgatcnn sgtacaggcg gcagctcag 39

<210> 277
<211> 39
<212> DNA
<213> Artificial

<220>
<223> synthetic primer

<220>
<221> misc_feature
<222> (19)..(20)
<223> n is a, c, g, or t

<400> 277
ggaaaagggc tgatcaacnn scaggcggca gctcagtaa 39

<210> 278
<211> 39
<212> DNA
<213> Artificial

<220>
<223> synthetic primer

<220>
<221> misc_feature
<222> (19)..(20)
<223> n is a, c, g, or t

<400> 278
aaagggctga tcaacgtann sgcggcagct cagtaaagc 39

<210> 279
<211> 39
<212> DNA
<213> Artificial

<220>
<223> synthetic primer

<220>
<221> misc_feature
<222> (19)..(20)
<223> n is a, c, g, or t

<400> 279
gggctgatca acgtacagnn sgcagctcag taaagctta 39

<210> 280
<211> 39
<212> DNA
<213> Artificial

<220>
<223> synthetic primer

<220>
<221> misc_feature
<222> (19)..(20)
<223> n is a, c, g, or t

<400> 280
ctgatcaacg tacaggcgnn sgctcagtaa agcttactg 39

<210> 281
<211> 39
<212> DNA
<213> Artificial

<220>
<223> synthetic primer

<220>
<221> misc_feature
<222> (19)..(20)
<223> n is a, c, g, or t

<400> 281
atcaacgtac aggcggcann scagtaaagc ttactggcc 39

<210> 282
<211> 39
<212> DNA
<213> Artificial

<220>
<223> synthetic primer

<220>
<221> misc_feature
<222> (19)..(20)
<223> n is a, c, g, or t

<400> 282
aacgtacagg cggcagctnn staaagctta ctggccgtc 39

<210> 283
<211> 39
<212> DNA
<213> Artificial

<220>
<223> synthetic primer

<220>
<221> misc_feature
<222> (20)..(21)
<223> n is a, c, g, or t

<400> 283
gccgtaaggc acggactgsn ngtatgcatg tgctacgtg 39

<210> 284
<211> 39
<212> DNA
<213> Artificial

<220>
<223> synthetic primer

<220>
<221> misc_feature
<222> (20)..(21)
<223> n is a, c, g, or t

<400> 284
tacgccgtaa ggcacggasn ncgcgtatgc atgtgctac 39

<210> 285
<211> 39
<212> DNA
<213> Artificial

<220>
<223> synthetic primer

<220>
<221> misc_feature
<222> (20)..(21)
<223> n is a, c, g, or t

<400> 285
tgatacgccg taaggcacsn nctgcgcgta tgcatgtgc 39

<210> 286
<211> 39
<212> DNA
<213> Artificial

<220>
<223> synthetic primer

<220>
<221> misc_feature
<222> (20)..(21)
<223> n is a, c, g, or t

<400> 286
ttgtgatacg ccgtaaggsn nggactgcgc gtatgcatg 39

<210> 287
<211> 39
<212> DNA
<213> Artificial

<220>
<223> synthetic primer

<220>
<221> misc_feature
<222> (20)..(21)
<223> n is a, c, g, or t

<400> 287
aatttgtgat acgccgtasn ncacggactg cgcgtatgc 39

<210> 288
<211> 39
<212> DNA
<213> Artificial

<220>

<223> synthetic primer

<220>
<221> misc_feature
<222> (20)..(21)
<223> n is a, c, g, or t

<400> 288
tttaatttgt gatacgccsn naggcacgga ctgcgcgta 39

<210> 289
<211> 39
<212> DNA
<213> Artificial

<220>
<223> synthetic primer

<220>
<221> misc_feature
<222> (20)..(21)
<223> n is a, c, g, or t

<400> 289
ggctttaatt tgtgatacsn ngtaaggcac ggactgcgc 39

<210> 290
<211> 39
<212> DNA
<213> Artificial

<220>
<223> synthetic primer

<220>
<221> misc_feature
<222> (20)..(21)
<223> n is a, c, g, or t

<400> 290
aggggcttta atttgtgasn ngccgtaagg cacggactg 39

<210> 291
<211> 39
<212> DNA
<213> Artificial

<220>
<223> synthetic primer

<220>
<221> misc_feature
<222> (20)..(21)
<223> n is a, c, g, or t

<400> 291
agcaggggct ttaatttgsn ntacgccgta aggcacgga 39

<210> 292
<211> 39
<212> DNA
<213> Artificial

<220>
<223> synthetic primer

<220>
<221> misc_feature
<222> (20)..(21)
<223> n is a, c, g, or t

<400> 292
cagagcaggg gctttaatsn ntgatacgcc gtaaggcac 39

<210> 293
<211> 39
<212> DNA
<213> Artificial

<220>
<223> synthetic primer

<220>
<221> misc_feature
<222> (20)..(21)
<223> n is a, c, g, or t

<400> 293
gtgcagagca ggggctttsn nttgtgatac gccgtaagg 39

<210> 294
<211> 39
<212> DNA
<213> Artificial

<220>
<223> synthetic primer

<220>
<221> misc_feature
<222> (20)..(21)
<223> n is a, c, g, or t

<400> 294
agagtgcaga gcaggggcsn naatttgtga tacgccgta 39

<210> 295
<211> 39
<212> DNA
<213> Artificial

<220>
<223> synthetic primer

<220>
<221> misc_feature
<222> (20)..(21)
<223> n is a, c, g, or t

<400> 295
ttgagagtgc agagcaggsn ntttaatttg tgatacgcc 39

<210> 296
<211> 39
<212> DNA
<213> Artificial

<220>
<223> synthetic primer

<220>
<221> misc_feature
<222> (20)..(21)
<223> n is a, c, g, or t

<400> 296
gccttgagag tgcagagcsn nggctttaat ttgtgatac 39

<210> 297
<211> 39
<212> DNA
<213> Artificial

<220>
<223> synthetic primer

<220>
<221> misc_feature
<222> (20)..(21)
<223> n is a, c, g, or t

<400> 297
gtagccttga gagtgcagsn naggggcttt aatttgtga 39

<210> 298
<211> 39
<212> DNA
<213> Artificial

<220>
<223> synthetic primer

<220>
<221> misc_feature
<222> (20)..(21)
<223> n is a, c, g, or t

<400> 298
agtgtagcct tgagagtgsn nagcaggggc tttaatttg 39

<210> 299
<211> 39
<212> DNA
<213> Artificial

<220>
<223> synthetic primer

<220>
<221> misc_feature
<222> (20)..(21)
<223> n is a, c, g, or t

<400> 299
tccagtgtag ccttgagasn ncagagcagg ggctttaat 39

<210> 300
<211> 39
<212> DNA
<213> Artificial

<220>
<223> synthetic primer

<220>

<221> misc_feature
<222> (20)..(21)
<223> n is a, c, g, or t

<400> 300
tgatccagtg tagccttgsn ngtgcagagc aggggcttt 39

<210> 301
<211> 39
<212> DNA
<213> Artificial

<220>
<223> synthetic primer

<220>
<221> misc_feature
<222> (20)..(21)
<223> n is a, c, g, or t

<400> 301
atttgatcca gtgtagccsn nagagtgcag agcaggggc 39

<210> 302
<211> 39
<212> DNA
<213> Artificial

<220>
<223> synthetic primer

<220>
<221> misc_feature
<222> (20)..(21)
<223> n is a, c, g, or t

<400> 302
aacatttgat ccagtgtasn nttgagagtg cagagcagg 39

<210> 303
<211> 39
<212> DNA
<213> Artificial

<220>
<223> synthetic primer

<220>
<221> misc_feature
<222> (20)..(21)
<223> n is a, c, g, or t

<400> 303
tttaacattt gatccagtsn ngccttgaga gtgcagagc 39

<210> 304
<211> 39
<212> DNA
<213> Artificial

<220>
<223> synthetic primer

<220>
<221> misc_feature

<222> (20)..(21)
<223> n is a, c, g, or t

<400> 304
tactttaaca tttgatccsn ngtagccttg agagtgcag 39

<210> 305
<211> 39
<212> DNA
<213> Artificial

<220>
<223> synthetic primer

<220>
<221> misc_feature
<222> (20)..(21)
<223> n is a, c, g, or t

<400> 305
cgctacttta acatttgasn nagtgtagcc ttgagagtg 39

<210> 306
<211> 39
<212> DNA
<213> Artificial

<220>
<223> synthetic primer

<220>
<221> misc_feature
<222> (20)..(21)
<223> n is a, c, g, or t

<400> 306
aaccgctact ttaacattsn ntccagtgta gccttgaga 39

<210> 307
<211> 39
<212> DNA
<213> Artificial

<220>
<223> synthetic primer

<220>
<221> misc_feature
<222> (20)..(21)
<223> n is a, c, g, or t

<400> 307
gataaccgct actttaacsn ntgatccagt gtagccttg 39

<210> 308
<211> 39
<212> DNA
<213> Artificial

<220>
<223> synthetic primer

<220>
<221> misc_feature
<222> (20)..(21)

<223> n is a, c, g, or t

<400> 308
gtcgataacc gctactttsn natttgatcc agtgtagcc 39

<210> 309
<211> 39
<212> DNA
<213> Artificial

<220>
<223> synthetic primer

<220>
<221> misc_feature
<222> (20)..(21)
<223> n is a, c, g, or t

<400> 309
gctgtcgata accgctacsn naacatttga tccagtgta 39

<210> 310
<211> 39
<212> DNA
<213> Artificial

<220>
<223> synthetic primer

<220>
<221> misc_feature
<222> (20)..(21)
<223> n is a, c, g, or t

<400> 310
accgctgtcg ataaccgcsn ntttaacatt tgatccagt 39

<210> 311
<211> 39
<212> DNA
<213> Artificial

<220>
<223> synthetic primer

<220>
<221> misc_feature
<222> (20)..(21)
<223> n is a, c, g, or t

<400> 311
gataccgctg tcgataacsn ntactttaac atttgatcc 39

<210> 312
<211> 39
<212> DNA
<213> Artificial

<220>
<223> synthetic primer

<220>
<221> misc_feature
<222> (20)..(21)
<223> n is a, c, g, or t

<400> 312
atcgataccg ctgtcgatsn ncgctacttt aacatttga 39

<210> 313
<211> 39
<212> DNA
<213> Artificial

<220>
<223> synthetic primer

<220>
<221> misc_feature
<222> (20)..(21)
<223> n is a, c, g, or t

<400> 313
agaatcgata ccgctgtcsn naaccgctac tttaacatt 39

<210> 314
<211> 39
<212> DNA
<213> Artificial

<220>
<223> synthetic primer

<220>
<221> misc_feature
<222> (20)..(21)
<223> n is a, c, g, or t

<400> 314
agaagaatcg ataccgctsn ngataaccgc tactttaac 39

<210> 315
<211> 39
<212> DNA
<213> Artificial

<220>
<223> synthetic primer

<220>
<221> misc_feature
<222> (20)..(21)
<223> n is a, c, g, or t

<400> 315
atgagaagaa tcgataccsn ngtcgataac cgctacttt 39

<210> 316
<211> 39
<212> DNA
<213> Artificial

<220>
<223> synthetic primer

<220>
<221> misc_feature
<222> (20)..(21)
<223> n is a, c, g, or t

<400> 316
aggatgagaa gaatcgatsn ngctgtcgat aaccgctac 39

<210> 317
<211> 39
<212> DNA
<213> Artificial

<220>
<223> synthetic primer

<220>
<221> misc_feature
<222> (20)..(21)
<223> n is a, c, g, or t

<400> 317
atcaggatga gaagaatcsn naccgctgtc gataaccgc 39

<210> 318
<211> 39
<212> DNA
<213> Artificial

<220>
<223> synthetic primer

<220>
<221> misc_feature
<222> (20)..(21)
<223> n is a, c, g, or t

<400> 318
taaatcagga tgagaagasn ngataccgct gtcgataac 39

<210> 319
<211> 39
<212> DNA
<213> Artificial

<220>
<223> synthetic primer

<220>
<221> misc_feature
<222> (20)..(21)
<223> n is a, c, g, or t

<400> 319
ctttaaatca ggatgagasn natcgatacc gctgtcgat 39

<210> 320
<211> 39
<212> DNA
<213> Artificial

<220>
<223> synthetic primer

<220>
<221> misc_feature
<222> (20)..(21)
<223> n is a, c, g, or t

<400> 320

tacctttaaa tcaggatgsn nagaatcgat accgctgtc 39

<210> 321
<211> 39
<212> DNA
<213> Artificial

<220>
<223> synthetic primer

<220>
<221> misc_feature
<222> (20)..(21)
<223> n is a, c, g, or t

<400> 321
tgctaccttt aaatcaggsn nagaagaatc gataccgct 39

<210> 322
<211> 39
<212> DNA
<213> Artificial

<220>
<223> synthetic primer

<220>
<221> misc_feature
<222> (20)..(21)
<223> n is a, c, g, or t

<400> 322
gcctgctacc tttaaatcsn natgagaaga atcgatacc 39

<210> 323
<211> 39
<212> DNA
<213> Artificial

<220>
<223> synthetic primer

<220>
<221> misc_feature
<222> (20)..(21)
<223> n is a, c, g, or t

<400> 323
tccgcctgct acctttaasn naggatgaga agaatcgat 39

<210> 324
<211> 39
<212> DNA
<213> Artificial

<220>
<223> synthetic primer

<220>
<221> misc_feature
<222> (20)..(21)
<223> n is a, c, g, or t

<400> 324
ggctccgcct gctaccttsn natcaggatg agaagaatc 39

<210> 325
<211> 39
<212> DNA
<213> Artificial

<220>
<223> synthetic primer

<220>
<221> misc_feature
<222> (20)..(21)
<223> n is a, c, g, or t

<400> 325
gctggctccg cctgctacsn ntaaatcagg atgagaaga 39

<210> 326
<211> 39
<212> DNA
<213> Artificial

<220>
<223> synthetic primer

<220>
<221> misc_feature
<222> (20)..(21)
<223> n is a, c, g, or t

<400> 326
catgctggct ccgcctgcsn nctttaaatc aggatgaga 39

<210> 327
<211> 39
<212> DNA
<213> Artificial

<220>
<223> synthetic primer

<220>
<221> misc_feature
<222> (20)..(21)
<223> n is a, c, g, or t

<400> 327
aaccatgctg gctccgccsn ntacctttaa atcaggatg 39

<210> 328
<211> 39
<212> DNA
<213> Artificial

<220>
<223> synthetic primer

<220>
<221> misc_feature
<222> (20)..(21)
<223> n is a, c, g, or t

<400> 328
aggaaccatg ctggctccsn ntgctacctt taaatcagg 39

<210> 329
<211> 39
<212> DNA
<213> Artificial

<220>
<223> synthetic primer

<220>
<221> misc_feature
<222> (20)..(21)
<223> n is a, c, g, or t

<400> 329
agaaggaacc atgctggcsn ngcctgctac ctttaaatc 39

<210> 330
<211> 39
<212> DNA
<213> Artificial

<220>
<223> synthetic primer

<220>
<221> misc_feature
<222> (20)..(21)
<223> n is a, c, g, or t

<400> 330
ttcagaagga accatgctsn ntccgcctgc tacctttaa 39

<210> 331
<211> 39
<212> DNA
<213> Artificial

<220>
<223> synthetic primer

<220>
<221> misc_feature
<222> (20)..(21)
<223> n is a, c, g, or t

<400> 331
tgtttcagaa ggaaccatsn nggctccgcc tgctacctt 39

<210> 332
<211> 39
<212> DNA
<213> Artificial

<220>
<223> synthetic primer

<220>
<221> misc_feature
<222> (20)..(21)
<223> n is a, c, g, or t

<400> 332
atttgtttca gaaggaacsn ngctggctcc gcctgctac 39

<210> 333

<211> 39
<212> DNA
<213> Artificial

<220>
<223> synthetic primer

<220>
<221> misc_feature
<222> (20)..(21)
<223> n is a, c, g, or t

<400> 333
aggatttgtt tcagaaggsn ncatgctggc tccgcctgc 39

<210> 334
<211> 39
<212> DNA
<213> Artificial

<220>
<223> synthetic primer

<220>
<221> misc_feature
<222> (20)..(21)
<223> n is a, c, g, or t

<400> 334
gaaaggattt gtttcagasn naaccatgct ggctccgcc 39

<210> 335
<211> 39
<212> DNA
<213> Artificial

<220>
<223> synthetic primer

<220>
<221> misc_feature
<222> (20)..(21)
<223> n is a, c, g, or t

<400> 335
ttggaaagga tttgtttcsn naggaaccat gctggctcc 39

<210> 336
<211> 39
<212> DNA
<213> Artificial

<220>
<223> synthetic primer

<220>
<221> misc_feature
<222> (20)..(21)
<223> n is a, c, g, or t

<400> 336
gtcttggaaa ggatttgtsn nagaaggaac catgctggc 39

<210> 337
<211> 39

<212> DNA
<213> Artificial

<220>
<223> synthetic primer

<220>
<221> misc_feature
<222> (20)..(21)
<223> n is a, c, g, or t

<400> 337
gttgtcttgg aaaggattsn nttcagaagg aaccatgct 39

<210> 338
<211> 39
<212> DNA
<213> Artificial

<220>
<223> synthetic primer

<220>
<221> misc_feature
<222> (20)..(21)
<223> n is a, c, g, or t

<400> 338
gttgttgtct tggaaaggsn ntgtttcaga aggaaccat 39

<210> 339
<211> 39
<212> DNA
<213> Artificial

<220>
<223> synthetic primer

<220>
<221> misc_feature
<222> (20)..(21)
<223> n is a, c, g, or t

<400> 339
agagttgttg tcttggaasn natttgtttc agaaggaac 39

<210> 340
<211> 39
<212> DNA
<213> Artificial

<220>
<223> synthetic primer

<220>
<221> misc_feature
<222> (20)..(21)
<223> n is a, c, g, or t

<400> 340
gtgagagttg ttgtcttgsn naggatttgt ttcagaagg 39

<210> 341
<211> 39
<212> DNA

<213>  Artificial

<220>
<223>  synthetic primer

<220>
<221>  misc_feature
<222>  (20)..(21)
<223>  n is a, c, g, or t

<400>  341
tccgtgagag ttgttgtcsn ngaaaggatt tgtttcaga 39

<210>  342
<211>  39
<212>  DNA
<213>  Artificial

<220>
<223>  synthetic primer

<220>
<221>  misc_feature
<222>  (20)..(21)
<223>  n is a, c, g, or t

<400>  342
agttccgtga gagttgttsn nttggaaagg atttgtttc 39

<210>  343
<211>  39
<212>  DNA
<213>  Artificial

<220>
<223>  synthetic primer

<220>
<221>  misc_feature
<222>  (20)..(21)
<223>  n is a, c, g, or t

<400>  343
gtgagttccg tgagagttsn ngtcttggaa aggatttgt 39

<210>  344
<211>  39
<212>  DNA
<213>  Artificial

<220>
<223>  synthetic primer

<220>
<221>  misc_feature
<222>  (20)..(21)
<223>  n is a, c, g, or t

<400>  344
aacgtgagtt ccgtgagasn ngttgtcttg gaaaggatt 39

<210>  345
<211>  39
<212>  DNA
<213>  Artificial

<220>
<223> synthetic primer

<220>
<221> misc_feature
<222> (20)..(21)
<223> n is a, c, g, or t

<400> 345
ggcaacgtga gttccgtgsn ngttgttgtc ttggaaagg 39

<210> 346
<211> 39
<212> DNA
<213> Artificial

<220>
<223> synthetic primer

<220>
<221> misc_feature
<222> (20)..(21)
<223> n is a, c, g, or t

<400> 346
gccggcaacg tgagttccsn nagagttgtt gtcttggaa 39

<210> 347
<211> 39
<212> DNA
<213> Artificial

<220>
<223> synthetic primer

<220>
<221> misc_feature
<222> (20)..(21)
<223> n is a, c, g, or t

<400> 347
tgtgccggca acgtgagtsn ngtgagagtt gttgtcttg 39

<210> 348
<211> 39
<212> DNA
<213> Artificial

<220>
<223> synthetic primer

<220>
<221> misc_feature
<222> (20)..(21)
<223> n is a, c, g, or t

<400> 348
aactgtgccg gcaacgtgsn ntccgtgaga gttgttgtc 39

<210> 349
<211> 39
<212> DNA
<213> Artificial

<220>
<223> synthetic primer

<220>
<221> misc_feature
<222> (20)..(21)
<223> n is a, c, g, or t

<400> 349
cgcaactgtg ccggcaacsn nagttccgtg agagttgtt 39

<210> 350
<211> 39
<212> DNA
<213> Artificial

<220>
<223> synthetic primer

<220>
<221> misc_feature
<222> (20)..(21)
<223> n is a, c, g, or t

<400> 350
agccgcaact gtgccggcsn ngtgagttcc gtgagagtt 39

<210> 351
<211> 39
<212> DNA
<213> Artificial

<220>
<223> synthetic primer

<220>
<221> misc_feature
<222> (20)..(21)
<223> n is a, c, g, or t

<400> 351
aagagccgca actgtgccsn naacgtgagt tccgtgaga 39

<210> 352
<211> 39
<212> DNA
<213> Artificial

<220>
<223> synthetic primer

<220>
<221> misc_feature
<222> (20)..(21)
<223> n is a, c, g, or t

<400> 352
attaagagcc gcaactgtsn nggcaacgtg agttccgtg 39

<210> 353
<211> 39
<212> DNA
<213> Artificial

<220>

<223> synthetic primer

<220>
<221> misc_feature
<222> (20)..(21)
<223> n is a, c, g, or t

<400> 353
gttattaaga gccgcaacsn ngccggcaac gtgagttcc 39

<210> 354
<211> 39
<212> DNA
<213> Artificial

<220>
<223> synthetic primer

<220>
<221> misc_feature
<222> (20)..(21)
<223> n is a, c, g, or t

<400> 354
tgagttatta agagccgcsn ntgtgccggc aacgtgagt 39

<210> 355
<211> 39
<212> DNA
<213> Artificial

<220>
<223> synthetic primer

<220>
<221> misc_feature
<222> (20)..(21)
<223> n is a, c, g, or t

<400> 355
gattgagtta ttaagagcsn naactgtgcc ggcaacgtg 39

<210> 356
<211> 39
<212> DNA
<213> Artificial

<220>
<223> synthetic primer

<220>
<221> misc_feature
<222> (20)..(21)
<223> n is a, c, g, or t

<400> 356
accgattgag ttattaagsn ncgcaactgt gccggcaac 39

<210> 357
<211> 39
<212> DNA
<213> Artificial

<220>
<223> synthetic primer

<220>
<221> misc_feature
<222> (20)..(21)
<223> n is a, c, g, or t

<400> 357
tacaccgatt gagttattsn nagccgcaac tgtgccggc 39

<210> 358
<211> 39
<212> DNA
<213> Artificial

<220>
<223> synthetic primer

<220>
<221> misc_feature
<222> (20)..(21)
<223> n is a, c, g, or t

<400> 358
taatacaccg attgagttsn naagagccgc aactgtgcc 39

<210> 359
<211> 39
<212> DNA
<213> Artificial

<220>
<223> synthetic primer

<220>
<221> misc_feature
<222> (20)..(21)
<223> n is a, c, g, or t

<400> 359
gcctaataca ccgattgasn nattaagagc cgcaactgt 39

<210> 360
<211> 39
<212> DNA
<213> Artificial

<220>
<223> synthetic primer

<220>
<221> misc_feature
<222> (20)..(21)
<223> n is a, c, g, or t

<400> 360
aacgcctaat acaccgatsn ngttattaag agccgcaac 39

<210> 361
<211> 39
<212> DNA
<213> Artificial

<220>
<223> synthetic primer

<220>
<221> misc_feature
<222> (20)..(21)
<223> n is a, c, g, or t

<400> 361
cgcaacgcct aatacaccsn ntgagttatt aagagccgc 39

<210> 362
<211> 39
<212> DNA
<213> Artificial

<220>
<223> synthetic primer

<220>
<221> misc_feature
<222> (20)..(21)
<223> n is a, c, g, or t

<400> 362
tggcgcaacg cctaatacsn ngattgagtt attaagagc 39

<210> 363
<211> 39
<212> DNA
<213> Artificial

<220>
<223> synthetic primer

<220>
<221> misc_feature
<222> (20)..(21)
<223> n is a, c, g, or t

<400> 363
gcttggcgca acgcctaasn naccgattga gttattaag 39

<210> 364
<211> 39
<212> DNA
<213> Artificial

<220>
<223> synthetic primer

<220>
<221> misc_feature
<222> (20)..(21)
<223> n is a, c, g, or t

<400> 364
tgcgcttggc gcaacgccsn ntacaccgat tgagttatt 39

<210> 365
<211> 39
<212> DNA
<213> Artificial

<220>
<223> synthetic primer

<220>

<221> misc_feature
<222> (20)..(21)
<223> n is a, c, g, or t

<400> 365
tgatgcgctt ggcgcaacsn ntaatacacc gattgagtt 39

<210> 366
<211> 39
<212> DNA
<213> Artificial

<220>
<223> synthetic primer

<220>
<221> misc_feature
<222> (20)..(21)
<223> n is a, c, g, or t

<400> 366
aagtgatgcg cttggcgcsn ngcctaatac accgattga 39

<210> 367
<211> 39
<212> DNA
<213> Artificial

<220>
<223> synthetic primer

<220>
<221> misc_feature
<222> (20)..(21)
<223> n is a, c, g, or t

<400> 367
gtaaagtgat gcgcttggsn naacgcctaa tacaccgat 39

<210> 368
<211> 39
<212> DNA
<213> Artificial

<220>
<223> synthetic primer

<220>
<221> misc_feature
<222> (20)..(21)
<223> n is a, c, g, or t

<400> 368
agcgtaaagt gatgcgctsn ncgcaacgcc taatacacc 39

<210> 369
<211> 39
<212> DNA
<213> Artificial

<220>
<223> synthetic primer

<220>
<221> misc_feature

<222>  (20)..(21)
<223>  n is a, c, g, or t

<400>  369
tacagcgtaa agtgatgcsn ntggcgcaac gcctaatac    39

<210>  370
<211>  39
<212>  DNA
<213>  Artificial

<220>
<223>  synthetic primer

<220>
<221>  misc_feature
<222>  (20)..(21)
<223>  n is a, c, g, or t

<400>  370
ttttacagcg taaagtgasn ngcttggcgc aacgcctaa    39

<210>  371
<211>  39
<212>  DNA
<213>  Artificial

<220>
<223>  synthetic primer

<220>
<221>  misc_feature
<222>  (20)..(21)
<223>  n is a, c, g, or t

<400>  371
aacttttaca gcgtaaagsn ntgcgcttgg cgcaacgcc    39

<210>  372
<211>  39
<212>  DNA
<213>  Artificial

<220>
<223>  synthetic primer

<220>
<221>  misc_feature
<222>  (20)..(21)
<223>  n is a, c, g, or t

<400>  372
gagaactttt acagcgtasn ntgatgcgct tggcgcaac    39

<210>  373
<211>  39
<212>  DNA
<213>  Artificial

<220>
<223>  synthetic primer

<220>
<221>  misc_feature
<222>  (20)..(21)

<223> n is a, c, g, or t

<400> 373
accgagaact tttacagcsn naagtgatgc gcttggcgc 39

<210> 374
<211> 39
<212> DNA
<213> Artificial

<220>
<223> synthetic primer

<220>
<221> misc_feature
<222> (20)..(21)
<223> n is a, c, g, or t

<400> 374
agcaccgaga acttttacsn ngtaaagtga tgcgcttgg 39

<210> 375
<211> 39
<212> DNA
<213> Artificial

<220>
<223> synthetic primer

<220>
<221> misc_feature
<222> (20)..(21)
<223> n is a, c, g, or t

<400> 375
gtcagcaccg agaactttsn nagcgtaaag tgatgcgct 39

<210> 376
<211> 39
<212> DNA
<213> Artificial

<220>
<223> synthetic primer

<220>
<221> misc_feature
<222> (20)..(21)
<223> n is a, c, g, or t

<400> 376
accgtcagca ccgagaacsn ntacagcgta aagtgatgc 39

<210> 377
<211> 39
<212> DNA
<213> Artificial

<220>
<223> synthetic primer

<220>
<221> misc_feature
<222> (20)..(21)
<223> n is a, c, g, or t

<400> 377
ggaaccgtca gcaccgagsn nttttacagc gtaaagtga 39

<210> 378
<211> 39
<212> DNA
<213> Artificial

<220>
<223> synthetic primer

<220>
<221> misc_feature
<222> (20)..(21)
<223> n is a, c, g, or t

<400> 378
gccggaaccg tcagcaccsn naacttttac agcgtaaag 39

<210> 379
<211> 39
<212> DNA
<213> Artificial

<220>
<223> synthetic primer

<220>
<221> misc_feature
<222> (20)..(21)
<223> n is a, c, g, or t

<400> 379
ttggccggaa ccgtcagcsn ngagaacttt tacagcgta 39

<210> 380
<211> 39
<212> DNA
<213> Artificial

<220>
<223> synthetic primer

<220>
<221> misc_feature
<222> (20)..(21)
<223> n is a, c, g, or t

<400> 380
gtattggccg gaaccgtcsn naccgagaac ttttacagc 39

<210> 381
<211> 39
<212> DNA
<213> Artificial

<220>
<223> synthetic primer

<220>
<221> misc_feature
<222> (20)..(21)
<223> n is a, c, g, or t

<400>  381
gctgtattgg ccggaaccsn nagcaccgag aactttac    39

<210>  382
<211>  39
<212>  DNA
<213>  Artificial

<220>
<223>  synthetic primer

<220>
<221>  misc_feature
<222>  (20)..(21)
<223>  n is a, c, g, or t

<400>  382
ccagctgtat tggccggasn ngtcagcacc gagaacttt    39

<210>  383
<211>  39
<212>  DNA
<213>  Artificial

<220>
<223>  synthetic primer

<220>
<221>  misc_feature
<222>  (20)..(21)
<223>  n is a, c, g, or t

<400>  383
gatccagctg tattggccsn naccgtcagc accgagaac    39

<210>  384
<211>  39
<212>  DNA
<213>  Artificial

<220>
<223>  synthetic primer

<220>
<221>  misc_feature
<222>  (20)..(21)
<223>  n is a, c, g, or t

<400>  384
aatgatccag ctgtattgsn nggaaccgtc agcaccgag    39

<210>  385
<211>  39
<212>  DNA
<213>  Artificial

<220>
<223>  synthetic primer

<220>
<221>  misc_feature
<222>  (20)..(21)
<223>  n is a, c, g, or t

<400>  385

gttaatgatc cagctgtasn ngccggaacc gtcagcacc 39

<210> 386
<211> 39
<212> DNA
<213> Artificial

<220>
<223> synthetic primer

<220>
<221> misc_feature
<222> (20)..(21)
<223> n is a, c, g, or t

<400> 386
tccgttaatg atccagctsn nttggccgga accgtcagc 39

<210> 387
<211> 39
<212> DNA
<213> Artificial

<220>
<223> synthetic primer

<220>
<221> misc_feature
<222> (20)..(21)
<223> n is a, c, g, or t

<400> 387
gattccgtta atgatccasn ngtattggcc ggaaccgtc 39

<210> 388
<211> 39
<212> DNA
<213> Artificial

<220>
<223> synthetic primer

<220>
<221> misc_feature
<222> (20)..(21)
<223> n is a, c, g, or t

<400> 388
ctcgattccg ttaatgatsn ngctgtattg gccggaacc 39

<210> 389
<211> 39
<212> DNA
<213> Artificial

<220>
<223> synthetic primer

<220>
<221> misc_feature
<222> (20)..(21)
<223> n is a, c, g, or t

<400> 389
ccactcgatt ccgttaatsn nccagctgta ttggccgga 39

<210> 390
<211> 39
<212> DNA
<213> Artificial

<220>
<223> synthetic primer

<220>
<221> misc_feature
<222> (20)..(21)
<223> n is a, c, g, or t

<400> 390
cgcccactcg attccgttsn ngatccagct gtattggcc 39

<210> 391
<211> 39
<212> DNA
<213> Artificial

<220>
<223> synthetic primer

<220>
<221> misc_feature
<222> (20)..(21)
<223> n is a, c, g, or t

<400> 391
gatcgcccac tcgattccsn naatgatcca gctgtattg 39

<210> 392
<211> 39
<212> DNA
<213> Artificial

<220>
<223> synthetic primer

<220>
<221> misc_feature
<222> (20)..(21)
<223> n is a, c, g, or t

<400> 392
tgcgatcgcc cactcgatsn ngttaatgat ccagctgta 39

<210> 393
<211> 39
<212> DNA
<213> Artificial

<220>
<223> synthetic primer

<220>
<221> misc_feature
<222> (20)..(21)
<223> n is a, c, g, or t

<400> 393
gtttgcgatc gcccactcsn ntccgttaat gatccagct 39

<210> 394
<211> 39
<212> DNA
<213> Artificial

<220>
<223> synthetic primer

<220>
<221> misc_feature
<222> (20)..(21)
<223> n is a, c, g, or t

<400> 394
attgtttgcg atcgcccasn ngattccgtt aatgatcca 39

<210> 395
<211> 39
<212> DNA
<213> Artificial

<220>
<223> synthetic primer

<220>
<221> misc_feature
<222> (20)..(21)
<223> n is a, c, g, or t

<400> 395
catattgttt gcgatcgcsn nctcgattcc gttaatgat 39

<210> 396
<211> 39
<212> DNA
<213> Artificial

<220>
<223> synthetic primer

<220>
<221> misc_feature
<222> (20)..(21)
<223> n is a, c, g, or t

<400> 396
gtccatattg tttgcgatsn nccactcgat tccgttaat 39

<210> 397
<211> 39
<212> DNA
<213> Artificial

<220>
<223> synthetic primer

<220>
<221> misc_feature
<222> (20)..(21)
<223> n is a, c, g, or t

<400> 397
aacgtccata ttgtttgcsn ncgcccactc gattccgtt 39

<210> 398

<211> 39
<212> DNA
<213> Artificial

<220>
<223> synthetic primer

<220>
<221> misc_feature
<222> (20)..(21)
<223> n is a, c, g, or t

<400> 398
aataacgtcc atattgttsn ngatcgccca ctcgattcc 39

<210> 399
<211> 39
<212> DNA
<213> Artificial

<220>
<223> synthetic primer

<220>
<221> misc_feature
<222> (20)..(21)
<223> n is a, c, g, or t

<400> 399
gttaataacg tccatattsn ntgcgatcgc ccactcgat 39

<210> 400

<400> 400
000

<210> 401
<211> 39
<212> DNA
<213> Artificial

<220>
<223> synthetic primer

<220>
<221> misc_feature
<222> (20)..(21)
<223> n is a, c, g, or t

<400> 401
catgttaata acgtccatsn ngtttgcgat cgcccactc 39

<210> 402
<211> 39
<212> DNA
<213> Artificial

<220>
<223> synthetic primer

<220>
<221> misc_feature
<222> (20)..(21)
<223> n is a, c, g, or t

<400> 402
gctcatgtta ataacgtcsn nattgtttgc gatcgccca 39

<210> 403
<211> 39
<212> DNA
<213> Artificial

<220>
<223> synthetic primer

<220>
<221> misc_feature
<222> (20)..(21)
<223> n is a, c, g, or t

<400> 403
gaggctcatg ttaataacsn ncatattgtt tgcgatcgc 39

<210> 404
<211> 39
<212> DNA
<213> Artificial

<220>
<223> synthetic primer

<220>
<221> misc_feature
<222> (20)..(21)
<223> n is a, c, g, or t

<400> 404
gccgaggctc atgttaatsn ngtccatatt gtttgcgat 39

<210> 405
<211> 39
<212> DNA
<213> Artificial

<220>
<223> synthetic primer

<220>
<221> misc_feature
<222> (20)..(21)
<223> n is a, c, g, or t

<400> 405
tccgccgagg ctcatgttsn naacgtccat attgtttgc 39

<210> 406
<211> 39
<212> DNA
<213> Artificial

<220>
<223> synthetic primer

<220>
<221> misc_feature
<222> (20)..(21)
<223> n is a, c, g, or t

<400> 406

aggtccgccg aggctcatsn naataacgtc catattgtt 39

<210> 407
<211> 39
<212> DNA
<213> Artificial

<220>
<223> synthetic primer

<220>
<221> misc_feature
<222> (20)..(21)
<223> n is a, c, g, or t

<400> 407
agaaggtccg ccgaggctsn ngttaataac gtccatatt 39

<210> 408
<211> 39
<212> DNA
<213> Artificial

<220>
<223> synthetic primer

<220>
<221> misc_feature
<222> (20)..(21)
<223> n is a, c, g, or t

<400> 408
accagaaggt ccgccgagsn ncatgttaat aacgtccat 39

<210> 409
<211> 39
<212> DNA
<213> Artificial

<220>
<223> synthetic primer

<220>
<221> misc_feature
<222> (20)..(21)
<223> n is a, c, g, or t

<400> 409
agaaccagaa ggtccgccsn ngctcatgtt aataacgtc 39

<210> 410
<211> 39
<212> DNA
<213> Artificial

<220>
<223> synthetic primer

<220>
<221> misc_feature
<222> (20)..(21)
<223> n is a, c, g, or t

<400> 410
agcagaacca gaaggtccsn ngaggctcat gttaataac 39

<210> 411
<211> 39
<212> DNA
<213> Artificial

<220>
<223> synthetic primer

<220>
<221> misc_feature
<222> (20)..(21)
<223> n is a, c, g, or t

<400> 411
agcagcagaa ccagaaggsn ngccgaggct catgttaat 39

<210> 412
<211> 39
<212> DNA
<213> Artificial

<220>
<223> synthetic primer

<220>
<221> misc_feature
<222> (20)..(21)
<223> n is a, c, g, or t

<400> 412
taaagcagca gaaccagasn ntccgccgag gctcatgtt 39

<210> 413
<211> 39
<212> DNA
<213> Artificial

<220>
<223> synthetic primer

<220>
<221> misc_feature
<222> (20)..(21)
<223> n is a, c, g, or t

<400> 413
ttttaaagca gcagaaccsn naggtccgcc gaggctcat 39

<210> 414
<211> 39
<212> DNA
<213> Artificial

<220>
<223> synthetic primer

<220>
<221> misc_feature
<222> (20)..(21)
<223> n is a, c, g, or t

<400> 414
cgcttttaaa gcagcagasn nagaaggtcc gccgaggct 39

<210> 415
<211> 39
<212> DNA
<213> Artificial

<220>
<223> synthetic primer

<220>
<221> misc_feature
<222> (20)..(21)
<223> n is a, c, g, or t

<400> 415
tgccgctttt aaagcagcsn naccagaagg tccgccgag 39

<210> 416
<211> 39
<212> DNA
<213> Artificial

<220>
<223> synthetic primer

<220>
<221> misc_feature
<222> (20)..(21)
<223> n is a, c, g, or t

<400> 416
aactgccgct tttaaagcsn nagaaccaga aggtccgcc 39

<210> 417
<211> 39
<212> DNA
<213> Artificial

<220>
<223> synthetic primer

<220>
<221> misc_feature
<222> (20)..(21)
<223> n is a, c, g, or t

<400> 417
atcaactgcc gcttttaasn nagcagaacc agaaggtcc 39

<210> 418
<211> 39
<212> DNA
<213> Artificial

<220>
<223> synthetic primer

<220>
<221> misc_feature
<222> (20)..(21)
<223> n is a, c, g, or t

<400> 418
tttatcaact gccgctttsn nagcagcaga accagaagg 39

<210> 419

<211> 39
<212> DNA
<213> Artificial

<220>
<223> synthetic primer

<220>
<221> misc_feature
<222> (20)..(21)
<223> n is a, c, g, or t

<400> 419
ggctttatca actgccgcsn ntaaagcagc agaaccaga 39

<210> 420
<211> 39
<212> DNA
<213> Artificial

<220>
<223> synthetic primer

<220>
<221> misc_feature
<222> (20)..(21)
<223> n is a, c, g, or t

<400> 420
aacggcttta tcaactgcsn nttttaaagc agcagaacc 39

<210> 421
<211> 39
<212> DNA
<213> Artificial

<220>
<223> synthetic primer

<220>
<221> misc_feature
<222> (20)..(21)
<223> n is a, c, g, or t

<400> 421
tgcaacggct ttatcaacsn ncgcttttaa agcagcaga 39

<210> 422
<211> 39
<212> DNA
<213> Artificial

<220>
<223> synthetic primer

<220>
<221> misc_feature
<222> (20)..(21)
<223> n is a, c, g, or t

<400> 422
ggatgcaacg gctttatcsn ntgccgcttt taaagcagc 39

<210> 423
<211> 39

<212> DNA
<213> Artificial

<220>
<223> synthetic primer

<220>
<221> misc_feature
<222> (20)..(21)
<223> n is a, c, g, or t

<400> 423
gccggatgca acggctttsn naactgccgc ttttaaagc 39

<210> 424
<211> 39
<212> DNA
<213> Artificial

<220>
<223> synthetic primer

<220>
<221> misc_feature
<222> (20)..(21)
<223> n is a, c, g, or t

<400> 424
gacgccggat gcaacggcsn natcaactgc cgcttttaa 39

<210> 425
<211> 39
<212> DNA
<213> Artificial

<220>
<223> synthetic primer

<220>
<221> misc_feature
<222> (20)..(21)
<223> n is a, c, g, or t

<400> 425
tacgacgccg gatgcaacsn ntttatcaac tgccgcttt 39

<210> 426
<211> 39
<212> DNA
<213> Artificial

<220>
<223> synthetic primer

<220>
<221> misc_feature
<222> (20)..(21)
<223> n is a, c, g, or t

<400> 426
gactacgacg ccggatgcsn nggctttatc aactgccgc 39

<210> 427
<211> 39
<212> DNA

<213> Artificial

<220>
<223> synthetic primer

<220>
<221> misc_feature
<222> (20)..(21)
<223> n is a, c, g, or t

<400> 427
aacgactacg acgccggasn naacggcttt atcaactgc 39

<210> 428
<211> 39
<212> DNA
<213> Artificial

<220>
<223> synthetic primer

<220>
<221> misc_feature
<222> (20)..(21)
<223> n is a, c, g, or t

<400> 428
cgcaacgact acgacgccsn ntgcaacggc tttatcaac 39

<210> 429
<211> 39
<212> DNA
<213> Artificial

<220>
<223> synthetic primer

<220>
<221> misc_feature
<222> (20)..(21)
<223> n is a, c, g, or t

<400> 429
tgccgcaacg actacgacsn nggatgcaac ggctttatc 39

<210> 430
<211> 39
<212> DNA
<213> Artificial

<220>
<223> synthetic primer

<220>
<221> misc_feature
<222> (20)..(21)
<223> n is a, c, g, or t

<400> 430
ggctgccgca acgactacsn ngccggatgc aacggcttt 39

<210> 431
<211> 39
<212> DNA

<213> Artificial

<220>
<223> synthetic primer

<220>
<221> misc_feature
<222> (20)..(21)
<223> n is a, c, g, or t

<400> 431
accggctgcc gcaacgacsn ngacgccgga tgcaacggc 39

<210> 432
<211> 39
<212> DNA
<213> Artificial

<220>
<223> synthetic primer

<220>
<221> misc_feature
<222> (20)..(21)
<223> n is a, c, g, or t

<400> 432
gttaccggct gccgcaacsn ntacgacgcc ggatgcaac 39

<210> 433
<211> 39
<212> DNA
<213> Artificial

<220>
<223> synthetic primer

<220>
<221> misc_feature
<222> (20)..(21)
<223> n is a, c, g, or t

<400> 433
ttcgttaccg gctgccgcsn ngactacgac gccggatgc 39

<210> 434
<211> 39
<212> DNA
<213> Artificial

<220>
<223> synthetic primer

<220>
<221> misc_feature
<222> (20)..(21)
<223> n is a, c, g, or t

<400> 434
gccttcgtta ccggctgcsn naacgactac gacgccgga 39

<210> 435
<211> 39
<212> DNA
<213> Artificial

<220>
<223> synthetic primer

<220>
<221> misc_feature
<222> (20)..(21)
<223> n is a, c, g, or t

<400> 435
agtgccttcg ttaccggcsn ncgcaacgac tacgacgcc 39

<210> 436
<211> 39
<212> DNA
<213> Artificial

<220>
<223> synthetic primer

<220>
<221> misc_feature
<222> (20)..(21)
<223> n is a, c, g, or t

<400> 436
ggaagtgcct tcgttaccsn ntgccgcaac gactacgac 39

<210> 437
<211> 39
<212> DNA
<213> Artificial

<220>
<223> synthetic primer

<220>
<221> misc_feature
<222> (20)..(21)
<223> n is a, c, g, or t

<400> 437
gccggaagtg ccttcgttsn nggctgccgc aacgactac 39

<210> 438
<211> 39
<212> DNA
<213> Artificial

<220>
<223> synthetic primer

<220>
<221> misc_feature
<222> (20)..(21)
<223> n is a, c, g, or t

<400> 438
gctgccggaa gtgccttcsn naccggctgc cgcaacgac 39

<210> 439
<211> 39
<212> DNA
<213> Artificial

<220>
<223> synthetic primer

<220>
<221> misc_feature
<222> (20)..(21)
<223> n is a, c, g, or t

<400> 439
tgagctgccg gaagtgccsn ngttaccggc tgccgcaac 39

<210> 440
<211> 39
<212> DNA
<213> Artificial

<220>
<223> synthetic primer

<220>
<221> misc_feature
<222> (20)..(21)
<223> n is a, c, g, or t

<400> 440
gcttgagctg ccggaagtsn nttcgttacc ggctgccgc 39

<210> 441
<211> 39
<212> DNA
<213> Artificial

<220>
<223> synthetic primer

<220>
<221> misc_feature
<222> (20)..(21)
<223> n is a, c, g, or t

<400> 441
tgtgcttgag ctgccggasn ngccttcgtt accggctgc 39

<210> 442
<211> 39
<212> DNA
<213> Artificial

<220>
<223> synthetic primer

<220>
<221> misc_feature
<222> (20)..(21)
<223> n is a, c, g, or t

<400> 442
cactgtgctt gagctgccsn nagtgccttc gttaccggc 39

<210> 443
<211> 39
<212> DNA
<213> Artificial

<220>

<223> synthetic primer

<220>
<221> misc_feature
<222> (20)..(21)
<223> n is a, c, g, or t

<400> 443
gcccactgtg cttgagctsn nggaagtgcc ttcgttacc 39

<210> 444
<211> 39
<212> DNA
<213> Artificial

<220>
<223> synthetic primer

<220>
<221> misc_feature
<222> (20)..(21)
<223> n is a, c, g, or t

<400> 444
gtagcccact gtgcttgasn ngccggaagt gccttcgtt 39

<210> 445
<211> 39
<212> DNA
<213> Artificial

<220>
<223> synthetic primer

<220>
<221> misc_feature
<222> (20)..(21)
<223> n is a, c, g, or t

<400> 445
agggtagccc actgtgctsn ngctgccgga agtgccttc 39

<210> 446
<211> 39
<212> DNA
<213> Artificial

<220>
<223> synthetic primer

<220>
<221> misc_feature
<222> (20)..(21)
<223> n is a, c, g, or t

<400> 446
accagggtag cccactgtsn ntgagctgcc ggaagtgcc 39

<210> 447
<211> 39
<212> DNA
<213> Artificial

<220>
<223> synthetic primer

<220>
<221> misc_feature
<222> (20)..(21)
<223> n is a, c, g, or t

<400> 447
tttaccaggg tagcccacsn ngcttgagct gccggaagt 39

<210> 448
<211> 39
<212> DNA
<213> Artificial

<220>
<223> synthetic primer

<220>
<221> misc_feature
<222> (20)..(21)
<223> n is a, c, g, or t

<400> 448
gtatttacca gggtagccsn ntgtgcttga gctgccgga 39

<210> 449
<211> 39
<212> DNA
<213> Artificial

<220>
<223> synthetic primer

<220>
<221> misc_feature
<222> (20)..(21)
<223> n is a, c, g, or t

<400> 449
agggtattta ccagggtasn ncactgtgct tgagctgcc 39

<210> 450
<211> 39
<212> DNA
<213> Artificial

<220>
<223> synthetic primer

<220>
<221> misc_feature
<222> (20)..(21)
<223> n is a, c, g, or t

<400> 450
agaagggtat ttaccaggsn ngcccactgt gcttgagct 39

<210> 451
<211> 39
<212> DNA
<213> Artificial

<220>
<223> synthetic primer

<220>
<221> misc_feature
<222> (20)..(21)
<223> n is a, c, g, or t

<400> 451
gacagaaggg tatttaccsn ngtagcccac tgtgcttga 39

<210> 452
<211> 39
<212> DNA
<213> Artificial

<220>
<223> synthetic primer

<220>
<221> misc_feature
<222> (20)..(21)
<223> n is a, c, g, or t

<400> 452
aatgacagaa gggtatttsn nagggtagcc cactgtgct 39

<210> 453
<211> 39
<212> DNA
<213> Artificial

<220>
<223> synthetic primer

<220>
<221> misc_feature
<222> (20)..(21)
<223> n is a, c, g, or t

<400> 453
tgcaatgaca gaagggtasn naccagggta gcccactgt 39

<210> 454
<211> 39
<212> DNA
<213> Artificial

<220>
<223> synthetic primer

<220>
<221> misc_feature
<222> (20)..(21)
<223> n is a, c, g, or t

<400> 454
tactgcaatg acagaaggsn ntttaccagg gtagcccac 39

<210> 455
<211> 39
<212> DNA
<213> Artificial

<220>
<223> synthetic primer

<220>
<221> misc_feature
<222> (20)..(21)
<223> n is a, c, g, or t

<400> 455
gcctactgca atgacagasn ngtatttacc agggtagcc 39

<210> 456
<211> 39
<212> DNA
<213> Artificial

<220>
<223> synthetic primer

<220>
<221> misc_feature
<222> (20)..(21)
<223> n is a, c, g, or t

<400> 456
agcgcctact gcaatgacsn nagggtattt accagggta 39

<210> 457
<211> 39
<212> DNA
<213> Artificial

<220>
<223> synthetic primer

<220>
<221> misc_feature
<222> (20)..(21)
<223> n is a, c, g, or t

<400> 457
aacagcgcct actgcaatsn nagaagggta tttaccagg 39

<210> 458
<211> 39
<212> DNA
<213> Artificial

<220>
<223> synthetic primer

<220>
<221> misc_feature
<222> (20)..(21)
<223> n is a, c, g, or t

<400> 458
gtcaacagcg cctactgcsn ngacagaagg gtatttacc 39

<210> 459
<211> 39
<212> DNA
<213> Artificial

<220>
<223> synthetic primer

<220>

<221> misc_feature
<222> (20)..(21)
<223> n is a, c, g, or t

<400> 459
gctgtcaaca gcgcctacsn naatgacaga agggtattt 39

<210> 460
<211> 39
<212> DNA
<213> Artificial

<220>
<223> synthetic primer

<220>
<221> misc_feature
<222> (20)..(21)
<223> n is a, c, g, or t

<400> 460
gctgctgtca acagcgccsn ntgcaatgac agaagggta 39

<210> 461
<211> 39
<212> DNA
<213> Artificial

<220>
<223> synthetic primer

<220>
<221> misc_feature
<222> (20)..(21)
<223> n is a, c, g, or t

<400> 461
gttgctgctg tcaacagcsn ntactgcaat gacagaagg 39

<210> 462
<211> 39
<212> DNA
<213> Artificial

<220>
<223> synthetic primer

<220>
<221> misc_feature
<222> (20)..(21)
<223> n is a, c, g, or t

<400> 462
ttggttgctg ctgtcaacsn ngcctactgc aatgacaga 39

<210> 463
<211> 39
<212> DNA
<213> Artificial

<220>
<223> synthetic primer

<220>
<221> misc_feature

<222> (20)..(21)
<223> n is a, c, g, or t

<400> 463
tctttggttg ctgctgtcsn nagcgcctac tgcaatgac 39

<210> 464
<211> 39
<212> DNA
<213> Artificial

<220>
<223> synthetic primer

<220>
<221> misc_feature
<222> (20)..(21)
<223> n is a, c, g, or t

<400> 464
tgctctttgg ttgctgctsn naacagcgcc tactgcaat 39

<210> 465
<211> 39
<212> DNA
<213> Artificial

<220>
<223> synthetic primer

<220>
<221> misc_feature
<222> (20)..(21)
<223> n is a, c, g, or t

<400> 465
agatgctctt tggttgctsn ngtcaacagc gcctactgc 39

<210> 466
<211> 39
<212> DNA
<213> Artificial

<220>
<223> synthetic primer

<220>
<221> misc_feature
<222> (20)..(21)
<223> n is a, c, g, or t

<400> 466
gaaagatgct ctttggttsn ngctgtcaac agcgcctac 39

<210> 467
<211> 39
<212> DNA
<213> Artificial

<220>
<223> synthetic primer

<220>
<221> misc_feature
<222> (20)..(21)

<223>  n is a, c, g, or t

<400>  467
tgagaaagat gctctttgsn ngctgctgtc aacagcgcc    39

<210>  468
<211>  39
<212>  DNA
<213>  Artificial

<220>
<223>  synthetic primer

<220>
<221>  misc_feature
<222>  (20)..(21)
<223>  n is a, c, g, or t

<400>  468
gcttgagaaa gatgctctsn ngttgctgct gtcaacagc    39

<210>  469
<211>  39
<212>  DNA
<213>  Artificial

<220>
<223>  synthetic primer

<220>
<221>  misc_feature
<222>  (20)..(21)
<223>  n is a, c, g, or t

<400>  469
tacgcttgag aaagatgcsn nttggttgct gctgtcaac    39

<210>  470
<211>  39
<212>  DNA
<213>  Artificial

<220>
<223>  synthetic primer

<220>
<221>  misc_feature
<222>  (20)..(21)
<223>  n is a, c, g, or t

<400>  470
tcctacgctt gagaaagasn ntctttggtt gctgctgtc    39

<210>  471
<211>  39
<212>  DNA
<213>  Artificial

<220>
<223>  synthetic primer

<220>
<221>  misc_feature
<222>  (20)..(21)
<223>  n is a, c, g, or t

<400> 471
aggtcctacg cttgagaasn ntgctctttg gttgctgct 39

<210> 472
<211> 39
<212> DNA
<213> Artificial

<220>
<223> synthetic primer

<220>
<221> misc_feature
<222> (20)..(21)
<223> n is a, c, g, or t

<400> 472
ctcaggtcct acgcttgasn nagatgctct ttggttgct 39

<210> 473
<211> 39
<212> DNA
<213> Artificial

<220>
<223> synthetic primer

<220>
<221> misc_feature
<222> (20)..(21)
<223> n is a, c, g, or t

<400> 473
aagctcaggt cctacgctsn ngaaagatgc tctttggtt 39

<210> 474
<211> 39
<212> DNA
<213> Artificial

<220>
<223> synthetic primer

<220>
<221> misc_feature
<222> (20)..(21)
<223> n is a, c, g, or t

<400> 474
atcaagctca ggtcctacsn ntgagaaaga tgctctttg 39

<210> 475
<211> 39
<212> DNA
<213> Artificial

<220>
<223> synthetic primer

<220>
<221> misc_feature
<222> (20)..(21)
<223> n is a, c, g, or t

<400> 475
gacatcaagc tcaggtccsn ngcttgagaa agatgctct 39

<210> 476
<211> 39
<212> DNA
<213> Artificial

<220>
<223> synthetic primer

<220>
<221> misc_feature
<222> (20)..(21)
<223> n is a, c, g, or t

<400> 476
catgacatca agctcaggsn ntacgcttga gaaagatgc 39

<210> 477
<211> 39
<212> DNA
<213> Artificial

<220>
<223> synthetic primer

<220>
<221> misc_feature
<222> (20)..(21)
<223> n is a, c, g, or t

<400> 477
tgccatgaca tcaagctcsn ntcctacgct tgagaaaga 39

<210> 478
<211> 39
<212> DNA
<213> Artificial

<220>
<223> synthetic primer

<220>
<221> misc_feature
<222> (20)..(21)
<223> n is a, c, g, or t

<400> 478
aggtgccatg acatcaagsn naggtcctac gcttgagaa 39

<210> 479
<211> 39
<212> DNA
<213> Artificial

<220>
<223> synthetic primer

<220>
<221> misc_feature
<222> (20)..(21)
<223> n is a, c, g, or t

<400> 479

gccaggtgcc atgacatcsn nctcaggtcc tacgcttga 39

<210> 480
<211> 39
<212> DNA
<213> Artificial

<220>
<223> synthetic primer

<220>
<221> misc_feature
<222> (20)..(21)
<223> n is a, c, g, or t

<400> 480
tacgccaggt gccatgacsn naagctcagg tcctacgct 39

<210> 481
<211> 39
<212> DNA
<213> Artificial

<220>
<223> synthetic primer

<220>
<221> misc_feature
<222> (20)..(21)
<223> n is a, c, g, or t

<400> 481
agatacgcca ggtgccatsn natcaagctc aggtcctac 39

<210> 482
<211> 39
<212> DNA
<213> Artificial

<220>
<223> synthetic primer

<220>
<221> misc_feature
<222> (20)..(21)
<223> n is a, c, g, or t

<400> 482
gatagatacg ccaggtgcsn ngacatcaag ctcaggtcc 39

<210> 483
<211> 39
<212> DNA
<213> Artificial

<220>
<223> synthetic primer

<220>
<221> misc_feature
<222> (20)..(21)
<223> n is a, c, g, or t

<400> 483
ttggatagat acgccaggsn ncatgacatc aagctcagg 39

<210> 484
<211> 39
<212> DNA
<213> Artificial

<220>
<223> synthetic primer

<220>
<221> misc_feature
<222> (20)..(21)
<223> n is a, c, g, or t

<400> 484
gctttggata gatacgccsn ntgccatgac atcaagctc 39

<210> 485
<211> 39
<212> DNA
<213> Artificial

<220>
<223> synthetic primer

<220>
<221> misc_feature
<222> (20)..(21)
<223> n is a, c, g, or t

<400> 485
cgtgctttgg atagatacsn naggtgccat gacatcaag 39

<210> 486
<211> 39
<212> DNA
<213> Artificial

<220>
<223> synthetic primer

<220>
<221> misc_feature
<222> (20)..(21)
<223> n is a, c, g, or t

<400> 486
aagcgtgctt tggatagasn ngccaggtgc catgacatc 39

<210> 487
<211> 39
<212> DNA
<213> Artificial

<220>
<223> synthetic primer

<220>
<221> misc_feature
<222> (20)..(21)
<223> n is a, c, g, or t

<400> 487
aggaagcgtg ctttggatsn ntacgccagg tgccatgac 39

<210> 488
<211> 39
<212> DNA
<213> Artificial

<220>
<223> synthetic primer

<220>
<221> misc_feature
<222> (20)..(21)
<223> n is a, c, g, or t

<400> 488
tccaggaagc gtgctttgsn nagatacgcc aggtgccat 39

<210> 489
<211> 39
<212> DNA
<213> Artificial

<220>
<223> synthetic primer

<220>
<221> misc_feature
<222> (20)..(21)
<223> n is a, c, g, or t

<400> 489
gtttccagga agcgtgctsn ngatagatac gccaggtgc 39

<210> 490
<211> 39
<212> DNA
<213> Artificial

<220>
<223> synthetic primer

<220>
<221> misc_feature
<222> (20)..(21)
<223> n is a, c, g, or t

<400> 490
tttgtttcca ggaagcgtsn nttggataga tacgccagg 39

<210> 491
<211> 39
<212> DNA
<213> Artificial

<220>
<223> synthetic primer

<220>
<221> misc_feature
<222> (20)..(21)
<223> n is a, c, g, or t

<400> 491
gtatttgttt ccaggaagsn ngctttggat agatacgcc 39

<210> 492

<211> 39
<212> DNA
<213> Artificial

<220>
<223> synthetic primer

<220>
<221> misc_feature
<222> (20)..(21)
<223> n is a, c, g, or t

<400> 492
cccgtatttg tttccaggsn ncgtgctttg gatagatac 39

<210> 493
<211> 39
<212> DNA
<213> Artificial

<220>
<223> synthetic primer

<220>
<221> misc_feature
<222> (20)..(21)
<223> n is a, c, g, or t

<400> 493
cgccccgtat ttgtttccsn naagcgtgct ttggataga 39

<210> 494
<211> 39
<212> DNA
<213> Artificial

<220>
<223> synthetic primer

<220>
<221> misc_feature
<222> (20)..(21)
<223> n is a, c, g, or t

<400> 494
gtacgccccg tatttgttsn naggaagcgt gctttggat 39

<210> 495
<211> 39
<212> DNA
<213> Artificial

<220>
<223> synthetic primer

<220>
<221> misc_feature
<222> (20)..(21)
<223> n is a, c, g, or t

<400> 495
gttgtacgcc ccgtatttsn ntccaggaag cgtgctttg 39

<210> 496

<211> 39
<212> DNA
<213> Artificial

<220>
<223> synthetic primer

<220>
<221> misc_feature
<222> (20)..(21)
<223> n is a, c, g, or t

<400> 496
accgttgtac gccccgtasn ngtttccagg aagcgtgct 39

<210> 497
<211> 39
<212> DNA
<213> Artificial

<220>
<223> synthetic primer

<220>
<221> misc_feature
<222> (20)..(21)
<223> n is a, c, g, or t

<400> 497
cgtaccgttg tacgccccsn ntttgtttcc aggaagcgt 39

<210> 498
<211> 39
<212> DNA
<213> Artificial

<220>
<223> synthetic primer

<220>
<221> misc_feature
<222> (20)..(21)
<223> n is a, c, g, or t

<400> 498
tgacgtaccg ttgtacgcsn ngtatttgtt tccaggaag 39

<210> 499
<211> 39
<212> DNA
<213> Artificial

<220>
<223> synthetic primer

<220>
<221> misc_feature
<222> (20)..(21)
<223> n is a, c, g, or t

<400> 499
cattgacgta ccgttgtasn ncccgtattt gtttccagg 39

<210> 500
<211> 39

<212> DNA
<213> Artificial

<220>
<223> synthetic primer

<220>
<221> misc_feature
<222> (20)..(21)
<223> n is a, c, g, or t

<400> 500
tgccattgac gtaccgttsn ncgccccgta tttgtttcc 39

<210> 501
<211> 39
<212> DNA
<213> Artificial

<220>
<223> synthetic primer

<220>
<221> misc_feature
<222> (20)..(21)
<223> n is a, c, g, or t

<400> 501
agatgccatt gacgtaccsn ngtacgcccc gtatttgtt 39

<210> 502
<211> 39
<212> DNA
<213> Artificial

<220>
<223> synthetic primer

<220>
<221> misc_feature
<222> (20)..(21)
<223> n is a, c, g, or t

<400> 502
cggagatgcc attgacgtsn ngttgtacgc cccgtattt 39

<210> 503
<211> 39
<212> DNA
<213> Artificial

<220>
<223> synthetic primer

<220>
<221> misc_feature
<222> (20)..(21)
<223> n is a, c, g, or t

<400> 503
gtgcggagat gccattgasn naccgttgta cgccccgta 39

<210> 504
<211> 39
<212> DNA

<213> Artificial

<220>
<223> synthetic primer

<220>
<221> misc_feature
<222> (20)..(21)
<223> n is a, c, g, or t

<400> 504
aacgtgcgga gatgccatsn ncgtaccgtt gtacgcccc 39

<210> 505
<211> 39
<212> DNA
<213> Artificial

<220>
<223> synthetic primer

<220>
<221> misc_feature
<222> (20)..(21)
<223> n is a, c, g, or t

<400> 505
ggcaacgtgc ggagatgcsn ntgacgtacc gttgtacgc 39

<210> 506
<211> 39
<212> DNA
<213> Artificial

<220>
<223> synthetic primer

<220>
<221> misc_feature
<222> (20)..(21)
<223> n is a, c, g, or t

<400> 506
tccggcaacg tgcggagasn ncattgacgt accgttgta 39

<210> 507
<211> 39
<212> DNA
<213> Artificial

<220>
<223> synthetic primer

<220>
<221> misc_feature
<222> (20)..(21)
<223> n is a, c, g, or t

<400> 507
cgctccggca acgtgcggsn ntgccattga cgtaccgtt 39

<210> 508
<211> 39
<212> DNA
<213> Artificial

<220>
<223> synthetic primer

<220>
<221> misc_feature
<222> (20)..(21)
<223> n is a, c, g, or t

<400> 508
agccgctccg gcaacgtgsn nagatgccat tgacgtacc 39

<210> 509
<211> 39
<212> DNA
<213> Artificial

<220>
<223> synthetic primer

<220>
<221> misc_feature
<222> (20)..(21)
<223> n is a, c, g, or t

<400> 509
agcagccgct ccggcaacsn ncggagatgc cattgacgt 39

<210> 510
<211> 39
<212> DNA
<213> Artificial

<220>
<223> synthetic primer

<220>
<221> misc_feature
<222> (20)..(21)
<223> n is a, c, g, or t

<400> 510
caaagcagcc gctccggcsn ngtgcggaga tgccattga 39

<210> 511
<211> 39
<212> DNA
<213> Artificial

<220>
<223> synthetic primer

<220>
<221> misc_feature
<222> (20)..(21)
<223> n is a, c, g, or t

<400> 511
aatcaaagca gccgctccsn naacgtgcgg agatgccat 39

<210> 512
<211> 39
<212> DNA
<213> Artificial

<220>
<223> synthetic primer

<220>
<221> misc_feature
<222> (20)..(21)
<223> n is a, c, g, or t

<400> 512
aagaatcaaa gcagccgcsn nggcaacgtg cggagatgc 39

<210> 513
<211> 39
<212> DNA
<213> Artificial

<220>
<223> synthetic primer

<220>
<221> misc_feature
<222> (20)..(21)
<223> n is a, c, g, or t

<400> 513
agaaagaatc aaagcagcsn ntccggcaac gtgcggaga 39

<210> 514
<211> 39
<212> DNA
<213> Artificial

<220>
<223> synthetic primer

<220>
<221> misc_feature
<222> (20)..(21)
<223> n is a, c, g, or t

<400> 514
cttagaaaga atcaaagcsn ncgctccggc aacgtgcgg 39

<210> 515
<211> 39
<212> DNA
<213> Artificial

<220>
<223> synthetic primer

<220>
<221> misc_feature
<222> (20)..(21)
<223> n is a, c, g, or t

<400> 515
gtgcttagaa agaatcaasn nagccgctcc ggcaacgtg 39

<210> 516
<211> 39
<212> DNA
<213> Artificial

<220>

<223> synthetic primer

<220>
<221> misc_feature
<222> (20)..(21)
<223> n is a, c, g, or t

<400> 516
cgggtgctta gaaagaatsn nagcagccgc tccggcaac 39

<210> 517
<211> 39
<212> DNA
<213> Artificial

<220>
<223> synthetic primer

<220>
<221> misc_feature
<222> (20)..(21)
<223> n is a, c, g, or t

<400> 517
gttcgggtgc ttagaaagsn ncaaagcagc cgctccggc 39

<210> 518
<211> 39
<212> DNA
<213> Artificial

<220>
<223> synthetic primer

<220>
<221> misc_feature
<222> (20)..(21)
<223> n is a, c, g, or t

<400> 518
ccagttcggg tgcttagasn naatcaaagc agccgctcc 39

<210> 519
<211> 39
<212> DNA
<213> Artificial

<220>
<223> synthetic primer

<220>
<221> misc_feature
<222> (20)..(21)
<223> n is a, c, g, or t

<400> 519
tgtccagttc gggtgcttsn naagaatcaa agcagccgc 39

<210> 520
<211> 39
<212> DNA
<213> Artificial

<220>

<223> synthetic primer

<220>
<221> misc_feature
<222> (20)..(21)
<223> n is a, c, g, or t

<400> 520
gtttgtccag ttcgggtgsn nagaaagaat caaagcagc 39

<210> 521
<211> 39
<212> DNA
<213> Artificial

<220>
<223> synthetic primer

<220>
<221> misc_feature
<222> (20)..(21)
<223> n is a, c, g, or t

<400> 521
agtgtttgtc cagttcggsn ncttagaaag aatcaaagc 39

<210> 522
<211> 39
<212> DNA
<213> Artificial

<220>
<223> synthetic primer

<220>
<221> misc_feature
<222> (20)..(21)
<223> n is a, c, g, or t

<400> 522
ttgagtgttt gtccagttsn ngtgcttaga aagaatcaa 39

<210> 523
<211> 39
<212> DNA
<213> Artificial

<220>
<223> synthetic primer

<220>
<221> misc_feature
<222> (20)..(21)
<223> n is a, c, g, or t

<400> 523
gacttgagtg tttgtccasn ncgggtgctt agaaagaat 39

<210> 524
<211> 39
<212> DNA
<213> Artificial

<220>
<223> synthetic primer

<220>
<221> misc_feature
<222> (20)..(21)
<223> n is a, c, g, or t

<400> 524
gcggacttga gtgtttgtsn ngttcgggtg cttagaaag 39

<210> 525
<211> 39
<212> DNA
<213> Artificial

<220>
<223> synthetic primer

<220>
<221> misc_feature
<222> (20)..(21)
<223> n is a, c, g, or t

<400> 525
gctgcggact tgagtgttsn nccagttcgg gtgcttaga 39

<210> 526
<211> 39
<212> DNA
<213> Artificial

<220>
<223> synthetic primer

<220>
<221> misc_feature
<222> (20)..(21)
<223> n is a, c, g, or t

<400> 526
actgctgcgg acttgagtsn ntgtccagtt cgggtgctt 39

<210> 527
<211> 39
<212> DNA
<213> Artificial

<220>
<223> synthetic primer

<220>
<221> misc_feature
<222> (20)..(21)
<223> n is a, c, g, or t

<400> 527
taaactgctg cggacttgsn ngtttgtcca gttcgggtg 39

<210> 528
<211> 39
<212> DNA
<213> Artificial

<220>
<223> synthetic primer

<220>
<221> misc_feature
<222> (20)..(21)
<223> n is a, c, g, or t

<400> 528
ttctaaactg ctgcggacsn nagtgtttgt ccagttcgg 39

<210> 529
<211> 39
<212> DNA
<213> Artificial

<220>
<223> synthetic primer

<220>
<221> misc_feature
<222> (20)..(21)
<223> n is a, c, g, or t

<400> 529
gttttctaaa ctgctgcgsn nttgagtgtt tgtccagtt 39

<210> 530
<211> 39
<212> DNA
<213> Artificial

<220>
<223> synthetic primer

<220>
<221> misc_feature
<222> (20)..(21)
<223> n is a, c, g, or t

<400> 530
ggtgttttct aaactgctsn ngacttgagt gtttgtcca 39

<210> 531
<211> 39
<212> DNA
<213> Artificial

<220>
<223> synthetic primer

<220>
<221> misc_feature
<222> (20)..(21)
<223> n is a, c, g, or t

<400> 531
agtggtgttt tctaaactsn ngcggacttg agtgtttgt 39

<210> 532
<211> 39
<212> DNA
<213> Artificial

<220>
<223> synthetic primer

<220>

<221> misc_feature
<222> (20)..(21)
<223> n is a, c, g, or t

<400> 532
tgtagtggtg ttttctaasn ngctgcggac ttgagtgtt 39

<210> 533
<211> 39
<212> DNA
<213> Artificial

<220>
<223> synthetic primer

<220>
<221> misc_feature
<222> (20)..(21)
<223> n is a, c, g, or t

<400> 533
ttttgtagtg gtgttttcsn nactgctgcg gacttgagt 39

<210> 534
<211> 39
<212> DNA
<213> Artificial

<220>
<223> synthetic primer

<220>
<221> misc_feature
<222> (20)..(21)
<223> n is a, c, g, or t

<400> 534
aagttttgta gtggtgttsn ntaaactgct gcggacttg 39

<210> 535
<211> 39
<212> DNA
<213> Artificial

<220>
<223> synthetic primer

<220>
<221> misc_feature
<222> (20)..(21)
<223> n is a, c, g, or t

<400> 535
accaagtttt gtagtggtsn nttctaaact gctgcggac 39

<210> 536
<211> 39
<212> DNA
<213> Artificial

<220>
<223> synthetic primer

<220>
<221> misc_feature

<222> (20)..(21)
<223> n is a, c, g, or t

<400> 536
atcaccaagt tttgtagtsn ngttttctaa actgctgcg 39

<210> 537
<211> 39
<212> DNA
<213> Artificial

<220>
<223> synthetic primer

<220>
<221> misc_feature
<222> (20)..(21)
<223> n is a, c, g, or t

<400> 537
agaatcacca agttttgtsn nggtgttttc taaactgct 39

<210> 538
<211> 39
<212> DNA
<213> Artificial

<220>
<223> synthetic primer

<220>
<221> misc_feature
<222> (20)..(21)
<223> n is a, c, g, or t

<400> 538
gaaagaatca ccaagtttsn nagtggtgtt ttctaaact 39

<210> 539
<211> 39
<212> DNA
<213> Artificial

<220>
<223> synthetic primer

<220>
<221> misc_feature
<222> (20)..(21)
<223> n is a, c, g, or t

<400> 539
gtagaaagaa tcaccaagsn ntgtagtggt gttttctaa 39

<210> 540
<211> 39
<212> DNA
<213> Artificial

<220>
<223> synthetic primer

<220>
<221> misc_feature
<222> (20)..(21)

<223> n is a, c, g, or t

<400> 540
atagtagaaa gaatcaccsn nttttgtagt ggtgttttc 39

<210> 541
<211> 39
<212> DNA
<213> Artificial

<220>
<223> synthetic primer

<220>
<221> misc_feature
<222> (20)..(21)
<223> n is a, c, g, or t

<400> 541
tccatagtag aaagaatcsn naagttttgt agtggtgtt 39

<210> 542
<211> 39
<212> DNA
<213> Artificial

<220>
<223> synthetic primer

<220>
<221> misc_feature
<222> (20)..(21)
<223> n is a, c, g, or t

<400> 542
ttttccatag tagaaagasn naccaagttt tgtagtggt 39

<210> 543
<211> 39
<212> DNA
<213> Artificial

<220>
<223> synthetic primer

<220>
<221> misc_feature
<222> (20)..(21)
<223> n is a, c, g, or t

<400> 543
cccttttcca tagtagaasn natcaccaag ttttgtagt 39

<210> 544
<211> 39
<212> DNA
<213> Artificial

<220>
<223> synthetic primer

<220>
<221> misc_feature
<222> (20)..(21)
<223> n is a, c, g, or t

<400> 544
cagccctttt ccatagtasn nagaatcacc aagttttgt 39

<210> 545
<211> 39
<212> DNA
<213> Artificial

<220>
<223> synthetic primer

<220>
<221> misc_feature
<222> (20)..(21)
<223> n is a, c, g, or t

<400> 545
gatcagccct tttccatasn ngaaagaatc accaagttt 39

<210> 546
<211> 39
<212> DNA
<213> Artificial

<220>
<223> synthetic primer

<220>
<221> misc_feature
<222> (20)..(21)
<223> n is a, c, g, or t

<400> 546
gttgatcagc ccttttccsn ngtagaaaga atcaccaag 39

<210> 547
<211> 39
<212> DNA
<213> Artificial

<220>
<223> synthetic primer

<220>
<221> misc_feature
<222> (20)..(21)
<223> n is a, c, g, or t

<400> 547
tacgttgatc agcccttttsn natagtagaa agaatcacc 39

<210> 548
<211> 39
<212> DNA
<213> Artificial

<220>
<223> synthetic primer

<220>
<221> misc_feature
<222> (20)..(21)
<223> n is a, c, g, or t

<400> 548
ctgtacgttg atcagcccsn ntccatagta gaaagaatc 39

<210> 549
<211> 39
<212> DNA
<213> Artificial

<220>
<223> synthetic primer

<220>
<221> misc_feature
<222> (20)..(21)
<223> n is a, c, g, or t

<400> 549
cgcctgtacg ttgatcagsn nttttccata gtagaaaga 39

<210> 550
<211> 39
<212> DNA
<213> Artificial

<220>
<223> synthetic primer

<220>
<221> misc_feature
<222> (20)..(21)
<223> n is a, c, g, or t

<400> 550
tgccgcctgt acgttgatsn nccctttcc atagtagaa 39

<210> 551
<211> 39
<212> DNA
<213> Artificial

<220>
<223> synthetic primer

<220>
<221> misc_feature
<222> (20)..(21)
<223> n is a, c, g, or t

<400> 551
agctgccgcc tgtacgttsn ncagcccttt tccatagta 39

<210> 552
<211> 39
<212> DNA
<213> Artificial

<220>
<223> synthetic primer

<220>
<221> misc_feature
<222> (20)..(21)
<223> n is a, c, g, or t

<400> 552

ctgagctgcc gcctgtacsn ngatcagccc ttttccata 39

<210> 553
<211> 39
<212> DNA
<213> Artificial

<220>
<223> synthetic primer

<220>
<221> misc_feature
<222> (20)..(21)
<223> n is a, c, g, or t

<400> 553
ttactgagct gccgcctgsn ngttgatcag cccttttcc 39

<210> 554
<211> 39
<212> DNA
<213> Artificial

<220>
<223> synthetic primer

<220>
<221> misc_feature
<222> (20)..(21)
<223> n is a, c, g, or t

<400> 554
gctttactga gctgccgcsn ntacgttgat cagcccttt 39

<210> 555
<211> 39
<212> DNA
<213> Artificial

<220>
<223> synthetic primer

<220>
<221> misc_feature
<222> (20)..(21)
<223> n is a, c, g, or t

<400> 555
taagctttac tgagctgcsn nctgtacgtt gatcagccc 39

<210> 556
<211> 39
<212> DNA
<213> Artificial

<220>
<223> synthetic primer

<220>
<221> misc_feature
<222> (20)..(21)
<223> n is a, c, g, or t

<400> 556
cagtaagctt tactgagcsn ncgcctgtac gttgatcag 39

<210> 557
<211> 39
<212> DNA
<213> Artificial

<220>
<223> synthetic primer

<220>
<221> misc_feature
<222> (20)..(21)
<223> n is a, c, g, or t

<400> 557
ggccagtaag ctttactgsn ntgccgcctg tacgttgat 39

<210> 558
<211> 39
<212> DNA
<213> Artificial

<220>
<223> synthetic primer

<220>
<221> misc_feature
<222> (20)..(21)
<223> n is a, c, g, or t

<400> 558
gacggccagt aagctttasn nagctgccgc ctgtacgtt 39

<210> 559
<211> 934
<212> DNA
<213> Bacillus amyloliquefaciens

<400> 559
aagacccgag cgtcgcttac gttgaagaag atcacgtagc acatgcgtac gcgcagtccg 60
tgccttacgg cgtatcacaa attaaagccc ctgctctgca ctctcaaggc tacactggat 120
caaatgttaa agtagcggtt atcgacagcg gtatcgattc ttctcatcct gatttaaagg 180
tagcaggcgg agccagcatg gttccttctg aaacaaatcc tttccaagac aacaactctc 240
acggaactca cgttgccggc acagttgcgg ctcttaataa ctcaatcggt gtattaggcg 300
ttgcgccaag cgcatcactt tacgctgtaa aagttctcgg tgctgacggt tccggccaat 360
acagctggat cattaacgga atcgagtggg cgatcgcaaa caatatggac gttattaaca 420
tgagcctcgg cggaccttct ggttctgctg ctttaaaagc ggcagttgat aaagccgttg 480
catccggcgt cgtagtcgtt gcggcagccg gtaacgaagg cacttccggc agctcaagca 540
cagtgggcta ccctggtaaa tacccttctg tcattgcagt aggcgctgtt gacagcagca 600
accaaagagc atctttctca agcgtaggac ctgagcttga tgtcatggca cctggcgtat 660
ctatccaaag cacgcttcct ggaaacaaat acggggcgta caacggtacg tcaatggcat 720
ctccgcacgt tgccggagcg gctgctttga ttctttctaa gcacccgaac tggacaaaca 780
ctcaagtccg cagcagttta gaaaacacca ctacaaaact tggtgattct ttctactatg 840
gaaaagggct gatcaacgta caggcggcag ctcagtaagt taacagagga ggatttcctg 900
aaggaaatcc gtttttttat tttaagcttg gaga 934

<210> 560
<211> 1303
<212> DNA
<213> Artificial

<220>
<223> synthetic sequence

<400> 560

atctcaaaaa aatgggtcta ctaaaatatt actccatcta ttataataaa ttcacagaat 60
agtcttttaa gtaagtctac tctgaatttt tttaaaagga gagggtaaag agtgagaagc 120
aaaaaattgt ggatcgtcgc gtcgaccgca ttgctgattt ctgttgcttt tagctcatcc 180
atcgcatccg ctgctgaaga agcaaaagaa aaatatttaa ttggctttaa tgagcaggaa 240
gctgtcagtg agtttgtaga acaagttgag gcaaatgacg aggtagccat tctctctgag 300
gaagaggaag tcgaaattga attgcttcat gaatttgaaa cgattcctgt tctgtccgtt 360
gagttaagcc cagaagatgt ggacgcgtta gagctcgatc cagctatttc ttatattgaa 420
gaggatgcag aagtaactac aatggcgcaa tcggtaccat ggggaattag cagagtacaa 480
gccccagctg cacataaccg tggattgaca ggttctggtg taaaagttgc tgtccttgat 540
accggtattt ccactcatcc agacttaaat attcgtggtg gagctagctt tgtaccaggg 600
gaaccatcca ctcaagatgg caatggacat ggcactcatg ttgccggcac aatcgcggct 660
cttaacaatt caattggtgt tcttggcgta gcgccaagcg cagaactata cgctgttaaa 720
gtattaggag caagcggttc aggctctgtc agctctattg cccaaggatt ggaatgggca 780
gggaacaatg gcatgcacgt tgctaatctt agtttaggat ctccttcgcc aagtgccaca 840
cttgagcaag ctgttaatag cgcgacttct agaggcgttc ttgttgtagc ggcctctgga 900
aattcaggtg caggctcaat cagctatccg gcccgttatg cgaacgctat ggcagtcgga 960
gctactgacc aaaacaacaa ccgcgccagc ttttcacagt atggcgcagg gcttgacatt 1020
gtcgcaccag gtgtaaacgt gcagagcact tacccaggtt caacatatgc cagcttaaac 1080
ggtacatcaa tggctactcc tcatgttgca ggtgcggctg cacttgttaa acaaaagaac 1140
ccatcttggt ccaatgtaca aatccgcaat catcttaaga atacggcaac tagcttagga 1200
agcacaaact tgtatggaag cggacttgtc aatgcagaag ctgcaactcg ttaaaagctt 1260
aactcgagat aaaaaaccgg ccttggcccc gccggttttt tat 1303

<210> 561
<211> 380
<212> PRT
<213> Bacillus subtilis

<400> 561
Met Arg Ser Lys Lys Leu Trp Ile Val Ala Ser Thr Ala Leu Leu Ile
1 5 10 15
Ser Val Ala Phe Ser Ser Ser Ile Ala Ser Ala Ala Glu Glu Ala Lys
20 25 30
Glu Lys Tyr Leu Ile Gly Phe Asn Glu Gln Glu Ala Val Ser Glu Phe
35 40 45
Val Glu Gln Val Glu Ala Asn Asp Glu Val Ala Ile Leu Ser Glu Glu
50 55 60
Glu Glu Val Glu Ile Glu Leu Leu His Glu Phe Glu Thr Ile Pro Val
65 70 75 80
Leu Ser Val Glu Leu Ser Pro Glu Asp Val Asp Ala Leu Glu Leu Asp
85 90 95
Pro Ala Ile Ser Tyr Ile Glu Glu Asp Ala Glu Val Thr Thr Met Ala
100 105 110
Gln Ser Val Pro Trp Gly Ile Ser Arg Val Gln Ala Pro Ala Ala His
115 120 125
Asn Arg Gly Leu Thr Gly Ser Gly Val Lys Val Ala Val Leu Asp Thr
130 135 140
Gly Ile Ser Thr His Pro Asp Leu Asn Ile Arg Gly Gly Ala Ser Phe
145 150 155 160
Val Pro Gly Glu Pro Ser Thr Gln Asp Gly Asn Gly His Gly Thr His
165 170 175
Val Ala Gly Thr Ile Ala Ala Leu Asn Asn Ser Ile Gly Val Leu Gly
180 185 190
Val Ala Pro Ser Ala Glu Leu Tyr Ala Val Lys Val Leu Gly Ala Ser
195 200 205
Gly Ser Gly Ser Val Ser Ser Ile Ala Gln Gly Leu Glu Trp Ala Gly
210 215 220
Asn Asn Gly Met His Val Ala Asn Leu Ser Leu Gly Ser Pro Ser Pro
225 230 235 240
Ser Ala Thr Leu Glu Gln Ala Val Asn Ser Ala Thr Ser Arg Gly Val
245 250 255
Leu Val Val Ala Ala Ser Gly Asn Ser Gly Ala Gly Ser Ile Ser Tyr
260 265 270
Pro Ala Arg Tyr Ala Asn Ala Met Ala Val Gly Ala Thr Asp Gln Asn
275 280 285

```
Asn Asn Arg Ala Ser Phe Ser Gln Tyr Gly Ala Gly Leu Asp Ile Val
    290                 295                 300
Ala Pro Gly Val Asn Val Gln Ser Thr Tyr Pro Gly Ser Thr Tyr Ala
305                 310                 315                 320
Ser Leu Asn Gly Thr Ser Met Ala Thr Pro His Val Ala Gly Ala Ala
                325                 330                 335
Ala Leu Val Lys Gln Lys Asn Pro Ser Trp Ser Asn Val Gln Ile Arg
            340                 345                 350
Asn His Leu Lys Asn Thr Ala Thr Ser Leu Gly Ser Thr Asn Leu Tyr
        355                 360                 365
Gly Ser Gly Leu Val Asn Ala Glu Ala Ala Thr Arg
    370                 375                 380

<210>  562
<211>  269
<212>  PRT
<213>  Bacillus subtilis

<400>  562
Ala Gln Ser Val Pro Trp Gly Ile Ser Arg Val Gln Ala Pro Ala Ala
1               5                   10                  15
His Asn Arg Gly Leu Thr Gly Ser Gly Val Lys Val Ala Val Leu Asp
            20                  25                  30
Thr Gly Ile Ser Thr His Pro Asp Leu Asn Ile Arg Gly Gly Ala Ser
        35                  40                  45
Phe Val Pro Gly Glu Pro Ser Thr Gln Asp Gly Asn Gly His Gly Thr
    50                  55                  60
His Val Ala Gly Thr Ile Ala Ala Leu Asn Asn Ser Ile Gly Val Leu
65                  70                  75                  80
Gly Val Ala Pro Ser Ala Glu Leu Tyr Ala Val Lys Val Leu Gly Ala
                85                  90                  95
Ser Gly Ser Gly Ser Val Ser Ser Ile Ala Gln Gly Leu Glu Trp Ala
            100                 105                 110
Gly Asn Asn Gly Met His Val Ala Asn Leu Ser Leu Gly Ser Pro Ser
        115                 120                 125
Pro Ser Ala Thr Leu Glu Gln Ala Val Asn Ser Ala Thr Ser Arg Gly
    130                 135                 140
Val Leu Val Val Ala Ala Ser Gly Asn Ser Gly Ala Gly Ser Ile Ser
145                 150                 155                 160
Tyr Pro Ala Arg Tyr Ala Asn Ala Met Ala Val Gly Ala Thr Asp Gln
                165                 170                 175
Asn Asn Asn Arg Ala Ser Phe Ser Gln Tyr Gly Ala Gly Leu Asp Ile
            180                 185                 190
Val Ala Pro Gly Val Asn Val Gln Ser Thr Tyr Pro Gly Ser Thr Tyr
        195                 200                 205
Ala Ser Leu Asn Gly Thr Ser Met Ala Thr Pro His Val Ala Gly Ala
    210                 215                 220
Ala Ala Leu Val Lys Gln Lys Asn Pro Ser Trp Ser Asn Val Gln Ile
225                 230                 235                 240
Arg Asn His Leu Lys Asn Thr Ala Thr Ser Leu Gly Ser Thr Asn Leu
                245                 250                 255
Tyr Gly Ser Gly Leu Val Asn Ala Glu Ala Ala Thr Arg
            260                 265

<210>  563
<211>  1326
<212>  DNA
<213>  Artificial

<220>
<223>  synthetic sequence

<400>  563
gaattcatct caaaaaaatg ggtctactaa aatattattc catctattat aataaattca      60
cagaatagtc ttttaagtaa gtctactctg aatttttta aaaggagagg gtaaagagtg      120
```

```
agaagcaaaa aattgtgaga agcaaaaaat tgtggatcag tttgctgttt gctttagcgt      180
taatctttac gatggcgttc ggcagcacat ccagcgcgca ggctgcaggg aaatcaaacg      240
gggaaaagaa atatattgtc gggtttaaac agacaatgag cacgatgagc gccgctaaga      300
agaaagacgt catttctgaa aaaggcggga aagtgcaaaa gcaattcaaa tatgtagacg      360
cagctagcgc tacattaaac gaaaaagctg taaaagaatt gaaaaaagac ccgagcgtcg      420
cttacgttga agaagatcac gtagcacacg cgtacgcgca gtccgtgcca tatggcgtat      480
cacaaattaa agcccctgct ctgcactctc aaggctacac cggttcaaat gttaaagtag      540
cggttatcga cagcggtatc gattcttctc atccagatct taaagtagca ggcggagcca      600
gcatggttcc ttctgaaaca aatcctttcc aagacaacaa ctctcacgga acacacgttg      660
ctggtaccgt tgcggctctt aataactcaa tcggtgtatt aggcgttgcg ccaagcgcat      720
cactttacgc tgtaaaagtt ctcggcgccg acggttccgg ccaatacagc tggatcatta      780
acggaatcga gtgggcgatc gcaaacaata tggacgttat taacatgagc ctcggcggac      840
cgtccggttc tgctgcttta aaagcggcag ttgataaagc cgttgcatcc ggcgtcgtag      900
tcgttgcggc agccggcaac gaaggcactt ccggcagctc aagcacagtg ggctaccctg      960
gtaaataccc ttctgtcatt gcagtaggcg ctgtcgacag cagcaaccaa agagcatctt     1020
tctcaagcgt aggacctgag ctcgatgtca tggcacctgg cgtatctatc caaagcacgc     1080
ttcctggaaa caaatacggc gcgttgaacg gtacatcaat ggcatctccg cacgttgccg     1140
gagccgcggc tttgattctt tctaagcacc cgaactggac aaacactcaa gtccgcagct     1200
ctctagaaaa caccactaca aaacttggtg attctttcta ctatggaaaa gggctgatca     1260
atgtacaggc ggcagctcag taaaactcga gataaaaaac cggccttggc cccgccggtt     1320
ttttat                                                                1326
```

<210> 564
<211> 382
<212> PRT
<213> Artificial

<220>
<223> synthetic precursor protein

<400> 564

```
Met Arg Ser Lys Lys Leu Trp Ile Ser Leu Leu Phe Ala Leu Ala Leu
1               5                   10                  15
Ile Phe Thr Met Ala Phe Gly Ser Thr Ser Ser Ala Gln Ala Ala Gly
            20                  25                  30
Lys Ser Asn Gly Glu Lys Lys Tyr Ile Val Gly Phe Lys Gln Thr Met
        35                  40                  45
Ser Thr Met Ser Ala Ala Lys Lys Lys Asp Val Ile Ser Glu Lys Gly
    50                  55                  60
Gly Lys Val Gln Lys Gln Phe Lys Tyr Val Asp Ala Ala Ser Ala Thr
65                  70                  75                  80
Leu Asn Glu Lys Ala Val Lys Glu Leu Lys Lys Asp Pro Ser Val Ala
                85                  90                  95
Tyr Val Glu Glu Asp His Val Ala His Ala Tyr Ala Gln Ser Val Pro
            100                 105                 110
Tyr Gly Val Ser Gln Ile Lys Ala Pro Ala Leu His Ser Gln Gly Tyr
        115                 120                 125
Thr Gly Ser Asn Val Lys Val Ala Val Ile Asp Ser Gly Ile Asp Ser
    130                 135                 140
Ser His Pro Asp Leu Lys Val Ala Gly Gly Ala Ser Met Val Pro Ser
145                 150                 155                 160
Glu Thr Asn Pro Phe Gln Asp Asn Asn Ser His Gly Thr His Val Ala
                165                 170                 175
Gly Thr Val Ala Ala Leu Asn Asn Ser Ile Gly Val Leu Gly Val Ala
            180                 185                 190
Pro Ser Ala Ser Leu Tyr Ala Val Lys Val Leu Gly Ala Asp Gly Ser
        195                 200                 205
Gly Gln Tyr Ser Trp Ile Ile Asn Gly Ile Glu Trp Ala Ile Ala Asn
    210                 215                 220
Asn Met Asp Val Ile Asn Met Ser Leu Gly Gly Pro Ser Gly Ser Ala
225                 230                 235                 240
Ala Leu Lys Ala Ala Val Asp Lys Ala Val Ala Ser Gly Val Val Val
                245                 250                 255
Val Ala Ala Ala Gly Asn Glu Gly Thr Ser Gly Ser Ser Ser Thr Val
            260                 265                 270
```

Gly Tyr Pro Gly Lys Tyr Pro Ser Val Ile Ala Val Gly Ala Val Asp
275 280 285
Ser Ser Asn Gln Arg Ala Ser Phe Ser Ser Val Gly Pro Glu Leu Asp
290 295 300
Val Met Ala Pro Gly Val Ser Ile Gln Ser Thr Leu Pro Gly Asn Lys
305 310 315 320
Tyr Gly Ala Leu Asn Gly Thr Ser Met Ala Ser Pro His Val Ala Gly
325 330 335
Ala Ala Ala Leu Ile Leu Ser Lys His Pro Asn Trp Thr Asn Thr Gln
340 345 350
Val Arg Ser Ser Leu Glu Asn Thr Thr Thr Lys Leu Gly Asp Ser Phe
355 360 365
Tyr Tyr Gly Lys Gly Leu Ile Asn Val Gln Ala Ala Ala Gln
370 375 380

<210> 565
<211> 275
<212> PRT
<213> Bacillus amiloliquefaciens

<400> 565
Ala Gln Ser Val Pro Tyr Gly Val Ser Gln Ile Lys Ala Pro Ala Leu
1 5 10 15
His Ser Gln Gly Tyr Thr Gly Ser Asn Val Lys Val Ala Val Ile Asp
20 25 30
Ser Gly Ile Asp Ser Ser His Pro Asp Leu Lys Val Ala Gly Gly Ala
35 40 45
Ser Met Val Pro Ser Glu Thr Asn Pro Phe Gln Asp Asn Asn Ser His
50 55 60
Gly Thr His Val Ala Gly Thr Val Ala Ala Leu Asn Asn Ser Ile Gly
65 70 75 80
Val Leu Gly Val Ala Pro Ser Ala Ser Leu Tyr Ala Val Lys Val Leu
85 90 95
Gly Ala Asp Gly Ser Gly Gln Tyr Ser Trp Ile Ile Asn Gly Ile Glu
100 105 110
Trp Ala Ile Ala Asn Asn Met Asp Val Ile Asn Met Ser Leu Gly Gly
115 120 125
Pro Ser Gly Ser Ala Ala Leu Lys Ala Ala Val Asp Lys Ala Val Ala
130 135 140
Ser Gly Val Val Val Val Ala Ala Ala Gly Asn Glu Gly Thr Ser Gly
145 150 155 160
Ser Ser Ser Thr Val Gly Tyr Pro Gly Lys Tyr Pro Ser Val Ile Ala
165 170 175
Val Gly Ala Val Asp Ser Ser Asn Gln Arg Ala Ser Phe Ser Ser Val
180 185 190
Gly Pro Glu Leu Asp Val Met Ala Pro Gly Val Ser Ile Gln Ser Thr
195 200 205
Leu Pro Gly Asn Lys Tyr Gly Ala Leu Asn Gly Thr Ser Met Ala Ser
210 215 220
Pro His Val Ala Gly Ala Ala Ala Leu Ile Leu Ser Lys His Pro Asn
225 230 235 240
Trp Thr Asn Thr Gln Val Arg Ser Ser Leu Glu Asn Thr Thr Thr Lys
245 250 255
Leu Gly Asp Ser Phe Tyr Tyr Gly Lys Gly Leu Ile Asn Val Gln Ala
260 265 270
Ala Ala Gln
275

<210> 566
<211> 1488
<212> DNA
<213> Artificial

<220>
<223> synthetic Asp sequence

```
<400>  566
atgacaccac gaactgtcac aagagctctg gctgtggcaa cagcagctgc tacactcttg      60
gctgggggta tggcagcaca agctaacgaa ccggctcctc caggatctgc atcagcccct     120
ccacgattag ctgaaaaact tgaccctgac ttacttgaag caatggaacg cgatctgggg     180
ttagatgcag aggaagcagc tgcaacgtta gcttttcagc atgacgcagc tgaaacggga     240
gaggctcttg ctgaggaact cgacgaagat ttcgcgggca cgtgggttga agatgatgtg     300
ctgtatgttg caaccactga tgaagatgct gttgaagaag tcgaaggcga aggagcaact     360
gctgtgactg ttgagcattc tcttgctgat ttagaggcgt ggaagacggt tttggatgct     420
gcgctggagg gtcatgatga tgtgcctacg tggtacgtcg acgtgcctac gaattcggta     480
gtcgttgctg taaaggcagg agcgcaggat gtagctgcag gacttgtgga aggcgctgat     540
gtgccatcag atgcggtcac ttttgtagaa acggacgaaa cgcctagaac gatgttcgac     600
gtaattggag gcaacgcata tactattggc ggccggtcta gatgttctat cggattcgca     660
gtaaacggtg gcttcattac tgccggtcac tgcggaagaa caggagccac tactgccaat     720
ccgactggca catttgcagg tagctcgttt ccgggaaatg attatgcatt cgtccgaaca     780
ggggcaggag taaatttgct tgcccaagtc aataactact cgggcggcag agtccaagta     840
gcaggacata cggccgcacc agttggatct gctgtatgcc gctcaggtag cactacaggt     900
tggcattgcg gaactatcac ggcgctgaat tcgtctgtca cgtatccaga gggaacagtc     960
cgaggactta tccgcacgac ggtttgtgcc gaaccaggtg atagcggagg tagccttttta    1020
gcgggaaatc aagcccaagg tgtcacgtca ggtggttctg gaaattgtcg gacgggggga    1080
acaacattct ttcaaccagt caacccgatt ttgcaggctt acggcctgag aatgattacg    1140
actgactctg gaagttcccc tgctccagca cctacatcat gtacaggcta cgcaagaacg    1200
ttcacaggaa ccctcgcagc aggaagagca gcagctcaac cgaacggtag ctatgttcag    1260
gtcaaccgga gcggtacaca ttccgtctgt ctcaatggac ctagcggtgc ggactttgat    1320
ttgtatgtgc agcgatggaa tggcagtagc tgggtaaccg tcgctcaatc gacatcgccg    1380
ggaagcaatg aaaccattac gtaccgcgga aatgctggat attatcgcta cgtggttaac    1440
gctgcgtcag gatcaggagc ttacacaatg ggactcaccc tcccctga                  1488

<210>  567
<211>  189
<212>  PRT
<213>  Cellulomonas bogoriensis

<400>  567
Phe Asp Val Ile Gly Gly Asn Ala Tyr Thr Ile Gly Gly Arg Ser Arg
1               5                   10                  15
Cys Ser Ile Gly Phe Ala Val Asn Gly Gly Phe Ile Thr Ala Gly His
            20                  25                  30
Cys Gly Arg Thr Gly Ala Thr Thr Ala Asn Pro Thr Gly Thr Phe Ala
        35                  40                  45
Gly Ser Ser Phe Pro Gly Asn Asp Tyr Ala Phe Val Arg Thr Gly Ala
    50                  55                  60
Gly Val Asn Leu Leu Ala Gln Val Asn Asn Tyr Ser Gly Gly Arg Val
65                  70                  75                  80
Gln Val Ala Gly His Thr Ala Ala Pro Val Gly Ser Ala Val Cys Arg
                85                  90                  95
Ser Gly Ser Thr Thr Gly Trp His Cys Gly Thr Ile Thr Ala Leu Asn
            100                 105                 110
Ser Ser Val Thr Tyr Pro Glu Gly Thr Val Arg Gly Leu Ile Arg Thr
        115                 120                 125
Thr Val Cys Ala Glu Pro Gly Asp Ser Gly Gly Ser Leu Leu Ala Gly
    130                 135                 140
Asn Gln Ala Gln Gly Val Thr Ser Gly Gly Ser Gly Asn Cys Arg Thr
145                 150                 155                 160
Gly Gly Thr Thr Phe Phe Gln Pro Val Asn Pro Ile Leu Gln Ala Tyr
                165                 170                 175
Gly Leu Arg Met Ile Thr Thr Asp Ser Gly Ser Ser Pro
            180                 185

<210>  568
<211>  4
<212>  PRT
<213>  Artificial

<220>
```

<223> synthetic reagent

<400> 568
Ala Ala Pro Phe
1